# EUROPEAN PATENT APPLICATION

(11) **EP 1 710 307 A2**
(43) Date of publication of application: **11.10.2006**
(21) Application number: 06075987.5
(22) Date of filing: 20.02.2003
(51) Int. Cl.: C12N 15/11, C12P 19/34, C07H 21/02, C07H 21/04, A61K 31/713

(54) **RNA interference mediated inhibition of gene expression using short interfering nucleic acid (siNA)**

(30) Priority: 20.02.2002 US 358580 P; 11.03.2002 US 363124 P; 06.03.2002 US 386782 P; 29.08.2002 US 406784 P; 05.09.2002 US 408378 P; 09.09.2002 US 409293 P; 15.01.2003 US 440129 P
(62) Divisional of application: 03743684.7
(71) Applicant: Sirna Therapeutics, Inc., Boulder, CO 80301 (US)
(72) Inventor: Mcswiggen, James, Boulder, CO 80301 (US); Beigelman, Leonid, Longmont CO 80503 (US); Chowrira, Bharat, Louisvill CO 80027 (US); Pavco, Pamela, Lafayette CO 80026 (US); Fosnaugh, Kathy, Boulder, Colorado 80305 (US); Jamison, Sharon, Boulder, CO 80301 (US); Usman, Nassim, Lafayette, CO 80026 (US); Thompson, James, Lafayette, CO 80026 (US)
(74) Representative: Maschio, Antonio

(57) **Abstract**

The present invention concerns methods for reagents useful in modulating gene expression in a variety of applications, including use in therapeutic, diagnostic, target validation, and genomic discovery applications. Specifically, the invention relates to small nucleic acid molecules, such as short interfering nucleic acid (siNA), short interfering RNA (siRNA), double-stranded RNA (dsRNA), micro-RNA (miRNA), and short hairpin RNA (shRNA) molecules capable of mediating RNA interference (RNAi) against target nucleic acid sequences. The small nucleic acid molecules are useful in the treatment of any disease or condition that responds to modulation of gene expression or activity in a cell, tissue, or organism.

## Description

This invention claims the benefit of Beigelman USSN 60/358,580 filed February 20, 2002, of Beigelman USSN 60/363,124 filed March 11, 2002, of Beigelman USSN 60/386,782 filed June 6, 2002, of Beigelman USSN 60/406,784 filed August 29, 2002, of Beigelman USSN 60/408,378 filed September 5, 2002, of Beigelman USSN 60/409,293 filed September 9, 2002, and of Beigelman USSN 60/440,129 filed January 15, 2003. These applications are hereby incorporated by reference herein in their entireties, including the drawings.

### Field Of The Invention

The present invention concerns methods and reagents useful in modulating gene expression in a variety of applications, including use in therapeutic, diagnostic, target validation, and genomic discovery applications. Specifically, the invention relates to small nucleic acid molecules, such as short interfering nucleic acid (siNA), short interfering RNA (siRNA), double-stranded RNA (dsRNA), micro-RNA (miRNA), and short hairpin RNA (shRNA) molecules capable of mediating RNA interference (RNAi).

### Background Of The Invention

The following is a discussion of relevant art pertaining to RNAi. The discussion is provided only for understanding of the invention that follows. The summary is not an admission that any of the work described below is prior art to the claimed invention. Applicant demonstrates herein that chemically modified short interfering nucleic acids possess the same capacity to mediate RNAi as do siRNA molecules and are expected to possess improved stability and activity in vivo; therefore, this discussion is not meant to be limiting only to siRNA and can be applied to siNA as a whole.

RNA interference refers to the process of sequence-specific post-transcriptional gene silencing in animals mediated by short interfering RNAs (siRNAs) (Fire et al., 1998, Nature, 391, 806). The corresponding process in plants is commonly referred to as post-transcriptional gene silencing or RNA silencing and is also referred to as quelling in fungi. The process of post-transcriptional gene silencing is thought to be an evolutionarily-conserved cellular defense mechanism used to prevent the expression of foreign genes and is commonly shared by diverse flora and phyla (Fire et al., 1999, Trends Genet., 15, 358). Such protection from foreign gene expression may have evolved in response to the production of double-stranded RNAs (dsRNAs) derived from viral infection or from the random integration of transposon elements into a host genome via a cellular response that specifically destroys homologous single-stranded RNA or viral genomic RNA. The presence of dsRNA in cells triggers the RNAi response though a mechanism that has yet to be fully characterized. This mechanism appears to be different from the interferon response that results from dsRNA-mediated activation of protein kinase PKR and 2',5'-oligoadenylate synthetase resulting in non-specific cleavage of mRNA by ribonuclease L.

The presence of long dsRNAs in cells stimulates the activity of a ribonuclease III enzyme referred to as dicer. Dicer is involved in the processing of the dsRNA into short pieces of dsRNA known as short interfering RNAs (siRNAs) (Berstein et al., 2001, Nature, 409, 363). Short interfering RNAs derived from dicer activity are typically about 21 to about 23 nucleotides in length and comprise about 19 base pair duplexes (Elbashir et al., 2001, Genes Dev., 15, 188). Dicer has also been implicated in the excision of 21-and 22-nucleotide small temporal RNAs (stRNAs) from precursor RNA of conserved structure that are implicated in translational control (Hutvagner et al., 2001, Science, 293, 834). The RNAi response also features an endonuclease complex, commonly referred to as an RNA-induced silencing complex (RISC), which mediates cleavage of single-stranded RNA having sequence complementary to the antisense strand of the siRNA duplex. Cleavage of the target RNA takes place in the middle of the region complementary to the antisense strand of the siRNA duplex (Elbashir et al., 2001, Genes Dev., 15, 188).

RNAi has been studied in a variety of systems. Fire et al., 1998, Nature, 391, 806, were the first to observe RNAi in *C. elegans*. Wianny and Goetz, 1999, Nature Cell Biol., 2, 70, describe RNAi mediated by dsRNA in mouse embryos. Hammond et al., 2000, Nature, 404, 293, describe RNAi in *Drosophila* cells transfected with dsRNA. Elbashir et al., 2001, Nature, 411, 494, describe RNAi induced by introduction of duplexes of synthetic 21-nucleotide RNAs in cultured mammalian cells including human embryonic kidney and HeLa cells. Recent work in Drosophila embryonic lysates (Elbashir et al., 2001, EMBO J., 20, 6877) has revealed certain requirements for siRNA length, structure, chemical composition, and sequence that are essential to mediate efficient RNAi activity. These studies have shown that 21-nucleotide siRNA duplexes are most active when containing 3'-terminal dinucleotide overhangs. Furthermore, complete substitution of one or both siRNA strands with 2'-deoxy (2'-H) or 2'-O-methyl nucleotides abolishes RNAi activity, whereas substitution of the 3'-terminal siRNA overhang nucleotides with 2'-deoxy nucleotides (2'-H) was shown to be tolerated. Single mismatch sequences in the center of the siRNA duplex were also shown to abolish RNAi activity. In addition, these studies also indicate that the position of the cleavage site in the target RNA is defined by the 5'-end of the siRNA guide sequence rather than the 3'-end of the guide sequence (Elbashir et al., 2001, EMBO J., 20, 6877). Other studies have indicated that a 5'-phosphate on the target-complementary strand of a siRNA duplex is required for siRNA activity and that ATP is utilized to maintain the 5'-phosphate moiety on the siRNA (Nykanen et al., 2001, Cell, 107, 309).

Studies have shown that replacing the 3'-terminal nucleotide overhanging segments of a 21-mer siRNA duplex having two -nucleotide 3'-overhangs with deoxyribonucleotides does not have an adverse effect on RNAi activity. Replacing up to four nucleotides on each end of the siRNA with deoxyribonucleotides has been reported to be well tolerated, whereas complete substitution with deoxyribonucleotides results in no RNAi activity (Elbashir et al., 2001, EMBO J., 20, 6877). In addition, Elbashir *et al*., *supra*, also report that substitution of siRNA with 2'-O-methyl nucleotides completely abolishes RNAi activity. Li et al., International PCT Publication No. WO 00/44914, and Beach et al., International PCT Publication No. WO 01/68836 preliminarily suggest that siRNA may include modifications to either the phosphate-sugar backbone or the nucleoside to include at least one of a nitrogen or sulfur heteroatom, however, neither application postulates to what extent such modifications would be tolerated in siRNA molecules, nor provides any further guidance or examples of such modified siRNA. Kreutzer et al., Canadian Patent Application No. 2,359,180, also describe certain chemical modifications for use in dsRNA constructs in order to counteract activation of double-stranded RNA-dependent protein kinase PKR, specifically 2'-amino or 2'-O-methyl nucleotides, and nucleotides containing a 2'-O or 4'-C methylene bridge. However, Kreutzer *et al*. similarly fails to provide examples or guidance as to what extent these modifications would be tolerated in siRNA molecules.

Parrish et al., 2000, Molecular Cell, 6, 1977-1087, tested certain chemical modifications targeting the unc-22 gene in *C. elegans* using long (>25 nt) siRNA transcripts. The authors describe the introduction of thiophosphate residues into these siRNA transcripts by incorporating thiophosphate nucleotide analogs with T7 and T3 RNA polymerase and observed that RNAs with two phosphorothioate modified bases also had substantial decreases in effectiveness as RNAi. Further, Parrish *et al*. reported that phosphorothioate modification of more than two residues greatly destabilized the RNAs *in vitro* such that interference activities could not be assayed. *Id.* at 1081. The authors also tested certain modifications at the 2'-position of the nucleotide sugar in the long siRNA transcripts and found that substituting deoxynucleotides for ribonucleotides produced a substantial decrease in interference activity, especially in the case of Uridine to Thymidine and/or Cytidine to deoxy-Cytidine substitutions. *Id.* In addition, the authors tested certain base modifications, including substituting, in sense and antisense strands of the siRNA, 4-thiouracil, 5-bromouracil, 5-iodouracil, and 3-(aminoallyl)uracil for uracil, and inosine for guanosine. Whereas 4-thiouracil and 5-bromouracil substitution appeared to be tolerated, Parnsh reported that inosine produced a substantial decrease in interference activity when incorporated in either strand. Parrish also reported that incorporation of 5-iodouracil and 3-(aminoallyl)uracil in the antisense strand resulted in a substantial decrease in RNAi activity as well.

The use of longer dsRNA has been described. For example, Beach et al., International PCT Publication No. WO 01/68836, describes specific methods for attenuating gene expression using endogenously-derived dsRNA. Tuschl et al., International PCT Publication No. WO 01/75164, describe a *Drosophila in vitro* RNAi system and the use of specific siRNA molecules for certain functional genomic and certain therapeutic applications; although Tuschl, 2001, Chem. Biochem., 2, 239-245, doubts that RNAi can be used to cure genetic diseases or viral infection due to the danger of activating interferon response. Li et al., International PCT Publication No. WO 00/44914, describe the use of specific dsRNAs for attenuating the expression of certain target genes. Zernicka-Goetz et al., International PCT Publication No. WO 01/36646, describe certain methods for inhibiting the expression of particular genes in mammalian cells using certain dsRNA molecules. Fire et al., International PCT Publication No. WO 99/32619, describe particular methods for introducing certain dsRNA molecules into cells for use in inhibiting gene expression. Plaetinck et al., International PCT Publication No. WO 00/01846, describe certain methods for identifying specific genes responsible for conferring a particular phenotype in a cell using specific dsRNA molecules. Mello et al., International PCT Publication No. WO 01/29058, describe the identification of specific genes involved in dsRNA-mediated RNAi. Deschamps Depaillette et al., International PCT Publication No. WO 99/07409, describe specific compositions consisting of particular dsRNA molecules combined with certain anti-viral agents. Waterhouse et al., International PCT Publication No. 99/53050, describe certain methods for decreasing the phenotypic expression of a nucleic acid in plant cells using certain dsRNAs. Driscoll et al., International PCT Publication No. WO 01/49844, describe specific DNA constructs for use in facilitating gene silencing in targeted organisms.

Others have reported on various RNAi and gene-silencing systems. For example, Parrish et al., 2000, Molecular Cell, 6, 1977-1087, describe specific chemically-modified siRNA constructs targeting the unc-22 gene of *C. elegans.* Grossniklaus, International PCT Publication No. WO 01/38551, describes certain methods for regulating polycomb gene expression in plants using certain dsRNAs. Churikov et al., International PCT Publication No. WO 01/42443, describe certain methods for modifying genetic characteristics of an organism using certain dsRNAs. Cogoni et al., International PCT Publication No. WO 01/53475, describe certain methods for isolating a Neurospora silencing gene and uses thereof. Reed et al., International PCT Publication No. WO 01/68836, describe certain methods for gene silencing in plants. Honer et al., International PCT Publication No. WO 01/70944, describe certain methods of drug screening using transgenic nematodes as Parkinson's Disease models using certain dsRNAs. Deak et al., International PCT Publication No. WO 01/72774, describe certain *Drosophila*-derived gene products that may be related to RNAi in *Drosophila.* Arndt et al., International PCT Publication No. WO 01/92513 describe certain methods for mediating gene suppression by using factors that enhance RNAi. Tuschl et al., International PCT Publication No. WO 02/44321, describe certain synthetic siRNA constructs. Pachuk et al., International PCT Publication No. WO 00/63364, and Satishchandran et al., International PCT Publication No. WO 01/04313, describe certain methods and compositions for inhibiting the function of certain polynucleotide sequences using certain dsRNAs. Echeverri et al., International PCT Publication No. WO 02/38805, describe certain *C. elegans* genes identified via RNAi. Kreutzer et al., International PCT Publications Nos. WO 02/055692, WO 02/055693, and EP 1144623 B1 describes certain methods for inhibiting gene expression using RNAi. Graham et al., International PCT Publications Nos. WO 99/49029 and WO 01/70949, and AU 4037501 describe certain vector expressed siRNA molecules. Fire et al., US 6,506,559, describe certain methods for inhibiting gene expression in vitro using certain long dsRNA (greater than 25 nucleotide) constructs that mediate RNAi.

### SUMMARY OF THE INVENTION

This invention relates to compounds, compositions, and methods useful for modulating RNA function and/or gene expression in a cell. Specifically, the instant invention features synthetic small nucleic acid molecules, such as short interfering nucleic acid (siNA), short interfering RNA (siRNA), double-stranded RNA (dsRNA), micro-RNA (miRNA), and short hairpin RNA (shRNA) molecules capable of modulating gene expression in cells by RNA inference (RNAi). The siRNA of the instant invention can be chemically synthesized, expressed from a vector or enzymatically synthesized. The use of chemically modified siNA can improve various properties of native siRNA molecules through increased resistance to nuclease degradation *in vivo* and/or improved cellular uptake. The chemically modified siNA molecules of the instant invention provide useful reagents and methods for a variety of therapeutic, diagnostic, agricultural, target validation, genomic discovery, genetic engineering and pharmacogenomic applications.

In a non-limiting example, the introduction of chemically modified nucleotides into nucleic acid molecules provides a powerful tool in overcoming potential limitations of *in vivo* stability and bioavailability inherent to native RNA molecules that are delivered exogenously. For example, the use of chemically modified nucleic acid molecules can enable a lower dose of a particular nucleic acid molecule for a given therapeutic effect since chemically modified nucleic acid molecules tend to have a longer half-life in serum. Furthermore, certain chemical modifications can improve the bioavailability of nucleic acid molecules by targeting particular cells or tissues and/or improving cellular uptake of the nucleic acid molecule. Therefore, even if the activity of a chemically modified nucleic acid molecule is reduced as compared to a native nucleic acid molecule, for example when compared to an all RNA nucleic acid molecule, the overall activity of the modified nucleic acid molecule can be greater than the native molecule due to improved stability and/or delivery of the molecule. Unlike native unmodified siRNA, chemically modified siNA can also minimize the possibility of activating interferon activity in humans.

The siRNA molecules of the invention can be designed to inhibit gene expression through RNAi targeting of a variety of RNA molecules. In one embodiment, the siRNA molecules of the invention are used to target various RNAs corresponding to a target gene. Non-limiting examples of such RNAs include messenger RNA (mRNA), alternate RNA splice variants of target gene(s), post-transcriptionally modified RNA of target gene(s), pre-mRNA of target gene(s). If alternate splicing produces a family of transcipts that are distinguished by usage of appropriate exons, the instant invention can be used to inhibit gene expression through the appropriate exons to specifically inhibit or to distinguish among the functions of gene family members. For example, a protein that contains an alternatively spliced transmembrane domain can be expressed in both membrane bound and secreted forms. Use of the invention to target the exon containing the transmembrane domain can be used to determine the functional consequences of pharmaceutical targeting of membrane bound as opposed to the secreted form of the protein. Non-limiting examples of applications of the invention relating to targeting these RNA molecules include therapeutic pharmaceutical applications, pharmaceutical discovery applications, molecular diagnostic and gene function applications, and gene mapping, for example using single nucleotide polymorphism mapping with siRNA molecules of the invention. Such applications can be implemented using known gene sequences or from partial sequences available from an expressed sequence tag (EST).

In another embodiment, the siRNA molecules of the invention are used to target conserved sequences corresponding to a gene family or gene families. As such, siRNA can be used to characterize pathways of gene function in a variety of applications. For example, the present invention can be used to inhibit the activity of target gene(s) in a pathway to determine the function of uncharacterized gene(s) in gene function analysis, mRNA function analysis, or translational analysis. The invention can be used to determine potential target gene pathways involved in various diseases and conditions toward pharmaceutical development. The invention can be used to understand pathways of gene expression involved in development, such as prenatal development, postnatal development and/or aging.

In one embodiment, the invention features a short interfering nucleic acid (siNA) molecule that down-regulates expression of a gene family by RNA interference. The gene family can comprise more than one splice variant of a target gene, more than one post-transcriptionally modified RNA of a target gene, or more than one RNA trascript having shared homology. In one embodiment, the gene family comprises epidermal growth factor (e.g., EGFR, such as HER1, HER2, HER3, and/or HER4) genes, vascular endothelial growth factor and vascular endothelial growth factor receptor (e.g., VEGF, VEGFR1, VEGFR2, or VEGFR3) genes, or viral genes corresponding to different viral strains (e.g., HIV-1 and HIV-2). Such gene families can be established by analysing nucleic acid sequences (e.g., sequences shown by Genbank Accession Nos. in Table V) for homology.

In one embodiment, the invention features a double-stranded short interfering nucleic acid (siNA) molecule that down-regulates expression of an endogenous mammalian target gene (e.g., a human gene), wherein the siNA molecule comprises one or more chemical modifications and each strand of the double-stranded siNA is about 21 nucleotides long.

In one embodiment, a siNA molecule of the invention comprises no ribonucleotides. In another embodiment, a siNA molecule of the invention comprises ribonucleotides.

In one embodiment, the invention features a double-stranded short interfering nucleic acid (siNA) molecule that down-regulates expression of an endogenous mammalian target gene (e.g., a human gene), wherein one of the strands of the double-stranded siNA molecule comprises a nucleotide sequence that is complementary to a nucleotide sequence of the endogenous mammalian target gene or a portion thereof, and wherein the second strand of the double-stranded siNA molecule comprises a nucleotide sequence substantially similar to the nucleotide sequence of the endogenous mammalian target gene or a portion thereof.

In one embodiment, the invention features a double-stranded short interfering nucleic acid (siNA) molecule that down-regulates expression of an endogenous mammalian target gene (e.g., a human gene), wherein each strand of the siNA molecule comprises about 19 to about 23 nucleotides, and wherein each strand comprises about 19 nucleotides that are complementary to the nucleotides of the other strand.

In one embodiment, the invention features a double-stranded short interfering nucleic acid (siNA) molecule that down-regulates expression of an endogenous mammalian target gene (e.g., a human gene), wherein the siNA molecule comprises an antisense region comprising a nucleotide sequence that is complementary to a nucleotide sequence of the endogenous mammalian target gene or a portion thereof, and wherein the siNA further comprises a sense region, wherein the sense region comprises a nucleotide sequence substantially similar to the nucleotide sequence of the endogenous mammalian target gene or a portion thereof.

In one embodiment, the invention features a double-stranded short interfering nucleic acid (siNA) molecule that down-regulates expression of an endogenous mammalian target gene (e.g., a human gene), wherein the antisense region and the sense region each comprise about 19 to about 23 nucleotides, and wherein the antisense region comprises about 19 nucleotides that are complementary to nucleotides of the sense region.

In one embodiment, the invention features a double-stranded short interfering nucleic acid (siNA) molecule that down-regulates expression of an endogenous mammalian target gene (e.g., a human gene), wherein the siNA molecule comprises a sense region and an antisense region and wherein the antisense region comprises a nucleotide sequence that is complementary to a nucleotide sequence of RNA encoded by the endogenous mammalian target gene or a portion thereof and the sense region comprises a nucleotide sequence that is complementary to the antisense region.

In one embodiment, the invention features a double-stranded short interfering nucleic acid (siNA) molecule that down-regulates expression of an endogenous mammalian target gene (e.g., a human gene), wherein the siNA molecule is assembled from two separate oligonucleotide fragments wherein one fragment comprises the sense region and the second fragment comprises the antisense region of the siNA molecule. The sense region can be connected to the antisense region via a linker molecule, such as a polynucleotide linker or a non-nucleotide linker.

In one embodiment, the invention features a double-stranded short interfering nucleic acid (siNA) molecule that down-regulates expression of an endogenous mammalian target gene (e.g., a human gene), wherein the siNA molecule comprises a sense region and an antisense region and wherein the antisense region comprises a nucleotide sequence that is complementary to a nucleotide sequence of RNA encoded by the endogenous mammalian target gene or a portion thereof and the sense region comprises a nucleotide sequence that is complementary to the antisense region, and wherein pyrimidine nucleotides in the sense region are 2'-O-methyl pyrimidine nucleotides, 2'-deoxy nucleotides, and/or 2'-deoxy-2'-fluoro pyrimidine nucleotides.

In one embodiment, the invention features a double-stranded short interfering nucleic acid (siNA) molecule that down-regulates expression of an endogenous mammalian target gene (e.g., a human gene), wherein the siNA molecule is assembled from two separate oligonucleotide fragments wherein one fragment comprises the sense region and the second fragment comprises the antisense region of the siNA molecule, and wherein the fragment comprising the sense region includes a terminal cap moiety at the 5'-end, the 3'-end, or both of the 5' and 3' ends of the fragment comprising the sense region. In another embodiment, the terminal cap moiety is an inverted deoxy abasic moiety or glyceryl moiety. In another embodiment, each of the two fragments of the siNA molecule comprise 21 nucleotides.

In one embodiment, the invention features a double-stranded short interfering nucleic acid (siNA) molecule that down-regulates expression of an endogenous mammalian target gene (e.g., a human gene), wherein the siNA molecule comprises a sense region and an antisense region and wherein the antisense region comprises a nucleotide sequence that is complementary to a nucleotide sequence of RNA encoded by the endogenous mammalian target gene or a portion thereof and the sense region comprises a nucleotide sequence that is complementary to the antisense region, and wherein the purine nucleotides present in the antisense region comprise 2'-deoxy- purine nucleotides. In another embodiment, the antisense region comprises a phosphorothioate internucleotide linkage at the 3' end of the antisense region. In another embodiment, the antisense region comprises a glyceryl modification at the 3' end of the antisense region.

In one embodiment, the invention features a double-stranded short interfering nucleic acid (siNA) molecule that down-regulates expression of an endogenous mammalian target gene (e.g., a human gene), wherein the siNA molecule is assembled from two separate oligonucleotide fragments wherein one fragment comprises the sense region and the second fragment comprises the antisense region of the siNA molecule, and wherein about 19 nucleotides of each fragment of the siNA molecule are base-paired to the complementary nucleotides of the other fragment of the siNA molecule and wherein at least two 3' terminal nucleotides of each fragment of the siNA molecule are not base-paired to the nucleotides of the other fragment of the siNA molecule. In another embodiment, each of the two 3' terminal nucleotides of each fragment of the siNA molecule are 2'-deoxy-pyrimidines, such as 2'-deoxy-thymidine. In another embodiment, all 21 nucleotides of each fragment of the siNA molecule are base-paired to the complementary nucleotides of the other fragment of the siNA molecule. In another embodiment, about 19 nucleotides of the antisense region are base-paired to the nucleotide sequence or a portion thereof of the RNA encoded by the endogenous mammalian target gene. In another embodiment, 21 nucleotides of the antisense region are base-paired to the nucleotide sequence or a portion thereof of the RNA encoded by the endogenous mammalian target gene. In another embodiment, the 5'-end of the fragment comprising said antisense region optionally includes a phosphate group.

In one embodiment, the invention features a double-stranded short interfering nucleic acid (siNA) molecule that inhibits the expression of an endogenous mammalian target RNA sequence (e.g., wherein said target RNA sequence is encoded by a human gene), wherein the siNA molecule comprises no ribonucleotides and wherein each strand of the double-stranded siNA molecule comprises about 21 nucleotides.

In one embodiment, the invention features a double-stranded short interfering nucleic acid (siNA) molecule that inhibits the expression of an endogenous mammalian target gene (e.g., a human gene such as vascular endothelial growth factor, vascular endothelial growth factor receptor (such as VEGFR1, VEGFR2, or VEGFR3), BCL2, HER2/neu, c-Myc, PCNA, REL-A, PTP1B, BACE, CHK1, PKC-alpha, or EGFR), wherein the siNA molecule does not require the presence of a ribonucleotide within the siNA molecule for said inhibition of expression of an endogenous mammalian target gene and wherein each strand of the double-stranded siNA molecule is about 21 nucleotides long.

In one embodiment, the invention features a medicament comprising a siNA molecule of the invention.

In one embodiment, the invention features an active ingredient comprising a siNA molecule of the invention.

In one embodiment, the invention features the use of a double-stranded short interfering nucleic acid (siNA) molecule to down-regulate expression of an endogenous mammalian target gene, wherein the siNA molecule comprises one or more chemical modifications and each strand of the double-stranded siNA is about 21 nucleotides long.

In one embodiment, siRNA molecule(s) and/or methods of the invention are used to inhibit the expression of gene(s) that encode RNA referred to by Genbank Accession number in **Table V.** In another embodiment, siRNA molecule(s) and/or methods of the invention are used to target RNA sequence(s) referred to by Genbank Accession number in **Table V,** or nucleic acid sequences encoding such sequences referred to by Genbank Accession number in **Table V.** Such sequences are readily obtained using the Genbank Accession numbers in **Table V.**

In one embodiment, the invention features a siNA molecule having RNAi activity against an RNA encoding a protein, wherein the siNA molecule comprises a sequence complementary to RNA having protein encoding sequence, such as those sequences having GenBank Accession Nos. shown in **Table V.**

In another embodiment, the invention features a siNA molecule having RNAi activity against a gene, wherein the siNA molecule comprises nucleotide sequence complementary to a nucleotide sequence of the gene, such as genes encoding sequences having GenBank Accession Nos. shown in **Table V.** In another embodiment, a siNA molecule of the invention includes nucleotide sequence that can interact with nucleotide sequence of a gene and thereby mediate silencing of gene expression, for example, wherein the siNA mediates regulation of gene expression by cellular processes that modulate the chromatin structure of the gene and prevent transcription of the gene.

In yet another embodiment, the invention features a siNA molecule comprising a sequence, for example, the antisense sequence of the siNA construct, complementary to a sequence represented by GenBank Accession Nos. shown in **Table V** or a portion of said sequence.

In one embodiment, the nucleic acid molecules of the invention that act as mediators of the RNA interference gene silencing response are chemically modified double stranded nucleic acid molecules. As in their native double stranded RNA counterparts, these siNA molecules typically consist of duplexes containing about 19 base pairs between oligonucleotides comprising about 19 to about 25 nucleotides. The most active siRNA molecules are thought to have such duplexes with overhanging ends of 1-3 nucleotides, for example 21 nucleotide duplexes with 19 base pairs and 2 nucleotide 3'-overhangs. These overhanging segments are readily hydrolyzed by endonucleases *in vivo.* Studies have shown that replacing the 3'-overhanging segments of a 21-mer siRNA duplex having 2 nucleotide 3' overhangs with deoxyribonucleotides does not have an adverse effect on RNAi activity. Replacing up to 4 nucleotides on each end of the siRNA with deoxyribonucleotides has been reported to be well tolerated whereas complete substitution with deoxyribonucleotides results in no RNAi activity (Elbashir et al., 2001, EMBO J., 20, 6877). In addition, Elbashir *et al, supra*, also report that substitution of siRNA with 2'-O-methyl nucleotides completely abolishes RNAi activity. Li et al., International PCT Publication No. WO 00/44914, and Beach et al., International PCT Publication No. WO 01/68836 both suggest that siRNA may include modifications to either the phosphate-sugar back bone or the nucleoside to include at least one of a nitrogen or sulfur heteroatom, however neither application teaches to what extent these modifications are tolerated in siRNA molecules nor provide any examples of such modified siRNA. Kreutzer and Limmer, Canadian Patent Application No. 2,359,180, also describe certain chemical modifications for use in dsRNA constructs in order to counteract activation of double stranded-RNA-dependent protein kinase PKR, specifically 2'-amino or 2'-O-methyl nucleotides, and nucleotides containing a 2'-O or 4'-C methylene bridge. However, Kreutzer and Limmer similarly fail to show to what extent these modifications are tolerated in siRNA molecules nor provide any examples of such modified siRNA.

In one embodiment, the invention features chemically modified siNA constructs having specificity for target nucleic acid molecules in a cell (i.e. target nucleic acid molecules comprising or encoded by seqences referred to herein by Genbank Accession numbers in **Table V**). Non-limiting examples of such chemical modifications include without limitation phosphorothioate internucleotide linkages, 2'-O-methyl ribonucleotides, 2'-deoxy-2'-fluoro ribonucleotides, 2'-deoxy ribonucleotides, "universal base" nucleotides, 5-C-methyl nucleotides, and inverted deoxyabasic residue incorporation. These chemical modifications, when used in various siNA constructs, are shown to preserve RNAi activity in cells while at the same time, dramatically increasing the serum stability of these compounds. Furthermore, contrary to the data published by Parrish *et al., supra*, applicant demonstrates that multiple (greater than one) phosphorothioate substitutions are well-tolerated and confer substantial increases in serum stability for modified siNA constructs.

In one embodiment, a siNA molecule of the invention comprises modified nucleotides while maintaining the ability to mediate RNAi. The modified nucleotides can be used to improve *in vitro* or *in vivo* characteristics such as stability, activity, and/or bioavailability. For example, a siNA molecule of the invention can comprise modified nucleotides as a percentage of the total number of nucleotides present in the siNA molecule. As such, a siNA molecule of the invention can generally comprise modified nucleotides of about 5 to about 100% of the nucleotide positions (e.g., 5%, 10%, 15%, 20%, 25%, 30%, 35%, 40%, 45%, 50%, 55%, 60%, 65%, 70%, 75%, 80%, 85%, 90%, 95% or 100% of the nucleotide positions). The actual percentage of modified nucleotides present in a given siNA molecule depends on the total number of nucleotides present in the siNA. If the siNA molecule is single stranded, the percent modification can be based upon the total number of nucleotides present in the single stranded siNA molecules. Likewise, if the siNA molecule is double stranded, the percent modification can be based upon the total number of nucleotides present in the sense strand, antisense strand, or both the sense and antisense strands. In addition, the actual percentage of modified nucleotides present in a given siNA molecule can also depend on the total number of purine and pyrimidine nucleotides present in the siNA, for example wherein all pyrimidine nucleotides and/or all purine nucleotides present in the siNA molecule are modified.

The antisense region of a siNA molecule of the invention can comprise a phosphorothioate internucleotide linkage at the 3'-end of said antisense region. The antisense region can comprise about one to about five phosphorothioate internucleotide linkages at the 5'-end of said antisense region. The 3'-terminal nucleotide overhangs of a siNA molecule of the invention can comprise ribonucleotides or deoxyribonucleotides that are chemically-modified at a nucleic acid sugar, base, or backbone. The 3'-terminal nucleotide overhangs can comprise one or more universal base ribonucleotides. The 3'-terminal nucleotide overhangs can comprise one or more acyclic nucleotides.

One embodiment of the invention provides an expression vector comprising a nucleic acid sequence encoding at least one siNA molecule of the invention in a manner that allows expression of the nucleic acid molecule. Another embodiment of the invention provides a mammalian cell comprising such an expression vector. The mammalian cell can be a human cell. The siNA molecule of the expression vector can comprise a sense region and an antisense region. The antisense region can comprise sequence complementary to a RNA or DNA sequence encoding a protein and the sense region can comprise sequence complementary to the antisense region. The siNA molecule can comprise two distinct strands having complementary sense and antisense regions. The siNA molecule can comprise a single strand having complementary sense and antisense regions.

In one embodiment, the invention features a chemically-modified short interfering nucleic acid (siNA) molecule capable of mediating RNA interference (RNAi) inside a cell or reconstituted *in vitro* system, wherein the chemical modification comprises one or more (*e.g.,* about 1, 2, 3, 4, 5, 6, 7, 8, 9, 10, or more) nucleotides comprising a backbone modified internucleotide linkage having Formula I: 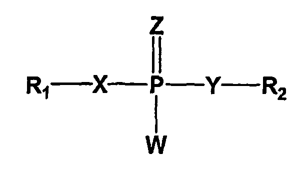 wherein each R1 and R2 is independently any nucleotide, non-nucleotide, or polynucleotide which can be naturally-occurring or chemically-modified, each X and Y is independently O, S, N, alkyl, or substituted alkyl, each Z and W is independently O, S, N, alkyl, substituted alkyl, O-alkyl, S-alkyl, alkaryl, or aralkyl, and wherein W, X, Y, and Z are optionally not all O.

The chemically-modified internucleotide linkages having Formula I, for example, wherein any Z, W, X, and/or Y independently comprises a sulphur atom, can be present in one or both oligonucleotide strands of the siNA duplex, for example, in the sense strand, the antisense strand, or both strands. The siNA molecules of the invention can comprise one or more (*e.g.,* about 1, 2, 3, 4, 5, 6, 7, 8, 9, 10, or more) chemically-modified internucleotide linkages having Formula I at the 3'-end, the 5'-end, or both of the 3' and 5'-ends of the sense strand, the antisense strand, or both strands. For example, an exemplary siNA molecule of the invention can comprise about 1 to about 5 or more (*e.g.,* about 1, 2, 3, 4, 5, or more) chemically-modified internucleotide linkages having Formula I at the 5'-end of the sense strand, the antisense strand, or both strands. In another non-limiting example, an exemplary siNA molecule of the invention can comprise one or more (*e.g.*, about 1, 2, 3, 4, 5, 6, 7, 8, 9, 10, or more) pyrimidine nucleotides with chemically-modified internucleotide linkages having Formula I in the sense strand, the antisense strand, or both strands. In yet another non-limiting example, an exemplary siNA molecule of the invention can comprise one or more (*e.g.,* about 1, 2, 3, 4, 5, 6, 7, 8, 9, 10, or more) purine nucleotides with chemically-modified internucleotide linkages having Formula I in the sense strand, the antisense strand, or both strands. In another embodiment, a siNA molecule of the invention having internucleotide linkage(s) of Formula I also comprises a chemically-modified nucleotide or non-nucleotide having any of Formulae I-VII.

In one embodiment, the invention features a chemically-modified short interfering nucleic acid (siNA) molecule capable of mediating RNA interference (RNAi) inside a cell or reconstituted *in vitro* system, wherein the chemical modification comprises one or more (*e*.*g*., about 1, 2, 3, 4, 5, 6, 7, 8, 9, 10, or more) nucleotides or non-nucleotides having Formula II: 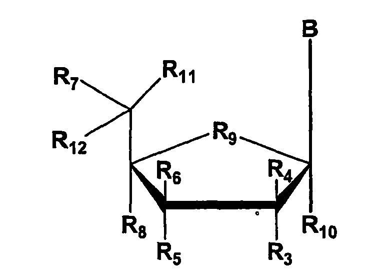 wherein each R3, R4, R5, R6, R7, R8, R10, R11 and R12 is independently H, OH, alkyl, substituted alkyl, alkaryl or aralkyl, F, Cl, Br, CN, CF3, OCF3, OCN, O-alkyl, S-alkyl, N-alkyl, O-alkenyl, S-alkenyl, N-alkenyl, SO-alkyl, alkyl-OSH, alkyl-OH, O-alkyl-OH, O-alkyl-SH, S-alkyl-OH, S-alkyl-SH, alkyl-S-alkyl, alkyl-O-alkyl, ONO2, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalklylamino, substituted silyl, or group having Formula I; R9 is O, S, CH2, S=O, CHF, or CF2, and B is a nucleosidic base such as adenine, guanine, uracil, cytosine, thymine, 2-aminoadenosine, 5-methylcytosine, 2,6-diaminopurine, or any other non-naturally occurring base that can be complementary or non-complementary to target RNA or a non-nucleosidic base such as phenyl, naphthyl, 3-nitropyrrole, 5-nitroindole, nebularine, pyridone, pyridinone, or any other non-naturally occurring universal base that can be complementary or non-complementary to target RNA.

The chemically-modified nucleotide or non-nucleotide of Formula II can be present in one or both oligonucleotide strands of the siNA duplex, for example in the sense strand, the antisense strand, or both strands. The siNA molecules of the invention can comprise one or more chemically-modified nucleotide or non-nucleotide of Formula II at the 3'-end, the 5'-end, or both of the 3' and 5'-ends of the sense strand, the antisense strand, or both strands. For example, an exemplary siNA molecule of the invention can comprise about 1 to about 5 or more *(e.g.,* about 1, 2, 3, 4, 5, or more) chemically-modified nucleotides or non-nucleotides of Formula II at the 5'-end of the sense strand, the antisense strand, or both strands. In anther non-limiting example, an exemplary siNA molecule of the invention can comprise about 1 to about 5 or more *(e.g.,* about 1, 2, 3, 4, 5, or more) chemically-modified nucleotides or non-nucleotides of Formula II at the 3'-end of the sense strand, the antisense strand, or both strands.

In one embodiment, the invention features a chemically-modified short interfering nucleic acid (siNA) molecule capable of mediating RNA interference (RNAi) inside a cell or reconstituted *in vitro* system, wherein the chemical modification comprises one or more (*e.g.,* about 1, 2, 3, 4, 5, 6, 7, 8, 9, 10, or more) nucleotides or non-nucleotides having Formula III: 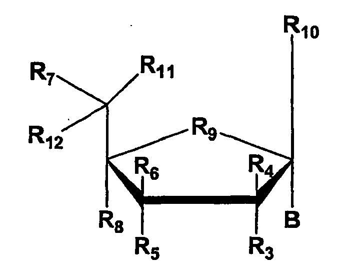 wherein each R3, R4, R5, R6, R7, R8, R10, R11 and R12 is independently H, OH, alkyl, substituted alkyl, alkaryl or aralkyl, F, Cl, Br, CN, CF3, OCF3, OCN, O-alkyl, S-alkyl, N-alkyl, O-alkenyl, S-alkenyl, N-alkenyl, SO-alkyl, alkyl-OSH, alkyl-OH, O-alkyl-OH, O-alkyl-SH, S-alkyl-OH, S-alkyl-SH, alkyl-S-alkyl, alkyl-O-alkyl, ONO2, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalklylamino, substituted silyl, or group having Formula I; R9 is O, S, CH2, S=O, CHF, or CF2, and B is a nucleosidic base such as adenine, guanine, uracil, cytosine, thymine, 2-aminoadenosine, 5-methylcytosine, 2,6-diaminopurine, or any other non-naturally occurring base that can be employed to be complementary or non-complementary to target RNA or a non-nucleosidic base such as phenyl, naphthyl, 3-nitropyrrole, 5-nitroindole, nebularine, pyridone, pyridinone, or any other non-naturally occurring universal base that can be complementary or non-complementary to target RNA.

The chemically-modified nucleotide or non-nucleotide of Formula III can be present in one or both oligonucleotide strands of the siNA duplex, for example, in the sense strand, the antisense strand, or both strands. The siNA molecules of the invention can comprise one or more chemically-modified nucleotide or non-nucleotide of Formula III at the 3'-end, the 5'-end, or both of the 3' and 5'-ends of the sense strand, the antisense strand, or both strands. For example, an exemplary siNA molecule of the invention can comprise about 1 to about 5 or more (*e*.*g*., about 1, 2, 3, 4, 5, or more) chemically-modified nucleotide(s) or non-nucleotide(s) of Formula III at the 5'-end of the sense strand, the antisense strand, or both strands. In anther non-limiting example, an exemplary siNA molecule of the invention can comprise about 1 to about 5 or more (*e.g.,* about 1, 2, 3, 4, 5, or more) chemically-modified nucleotide or non-nucleotide of Formula III at the 3'-end of the sense strand, the antisense strand, or both strands.

In another embodiment, a siNA molecule of the invention comprises a nucleotide having Formula II or III, wherein the nucleotide having Formula II or III is in an inverted configuration. For example, the nucleotide having Formula II or III is connected to the siNA construct in a 3'-3', 3'-2', 2'-3', or 5'-5' configuration, such as at the 3'-end, the 5'-end, or both of the 3' and 5'-ends of one or both siNA strands.

In one embodiment, the invention features a chemically-modified short interfering nucleic acid (siNA) molecule capable of mediating RNA interference (RNAi) inside a cell or reconstituted *in vitro* system, wherein the chemical modification comprises a 5'-terminal phosphate group having Formula IV: 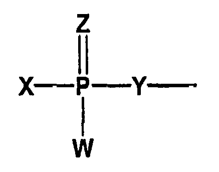 wherein each X and Y is independently O, S, N, alkyl, substituted alkyl, or alkylhalo; wherein each Z and W is independently O, S, N, alkyl, substituted alkyl, O-alkyl, S-alkyl, alkaryl, aralkyl, or alkylhalo; and wherein W, X, Y and Z are not all O.

In one embodiment, the invention features a siNA molecule having a 5'-terminal phosphate group having Formula IV on the target-complementary strand, for example, a strand complementary to a target RNA, wherein the siNA molecule comprises an all RNA siNA molecule. In another embodiment, the invention features a siNA molecule having a 5'-terminal phosphate group having Formula IV on the target-complementary strand wherein the siNA molecule also comprises about 1 to about 3 (*e.g.,* about 1, 2, or 3) nucleotide 3'-terminal nucleotide overhangs having about 1 to about 4 (*e.g.,* about 1, 2, 3, or 4) deoxyribonucleotides on the 3'-end of one or both strands. In another embodiment, a 5'-terminal phosphate group having Formula IV is present on the target-complementary strand of a siNA molecule of the invention, for example a siNA molecule having chemical modifications having any of Formulae I-VII.

In one embodiment, the invention features a chemically-modified short interfering nucleic acid (siNA) molecule capable of mediating RNA interference (RNAi) inside a cell or reconstituted *in vitro* system, wherein the chemical modification comprises one or more phosphorothioate internucleotide linkages. For example, in a non-limiting example, the invention features a chemically-modified short interfering nucleic acid (siNA) having about 1, 2, 3, 4, 5, 6, 7, 8 or more phosphorothioate internucleotide linkages in one siNA strand. In yet another embodiment, the invention features a chemically-modified short interfering nucleic acid (siNA) individually having about 1, 2, 3, 4, 5, 6, 7, 8 or more phosphorothioate internucleotide linkages in both siNA strands. The phosphorothioate internucleotide linkages can be present in one or both oligonucleotide strands of the siNA duplex, for example in the sense strand, the antisense strand, or both strands. The siNA molecules of the invention can comprise one or more phosphorothioate internucleotide linkages at the 3'-end, the 5'-end, or both of the 3'- and 5'-ends of the sense strand, the antisense strand, or both strands. For example, an exemplary siNA molecule of the invention can comprise about 1 to about 5 or more (*e*.*g*., about 1, 2, 3, 4, 5, or more) consecutive phosphorothioate internucleotide linkages at the 5'-end of the sense strand, the antisense strand, or both strands. In another non-limiting example, an exemplary siNA molecule of the invention can comprise one or more (*e.g.,* about 1, 2, 3, 4, 5, 6, 7, 8, 9, 10, or more) pyrimidine phosphorothioate internucleotide linkages in the sense strand, the antisense strand, or both strands. In yet another non-limiting example, an exemplary siNA molecule of the invention can comprise one or more (*e.g.,* about 1, 2, 3, 4, 5, 6, 7, 8, 9, 10, or more) purine phosphorothioate internucleotide linkages in the sense strand, the antisense strand, or both strands.

In one embodiment, the invention features a siNA molecule, wherein the sense strand comprises one or more, for example, about 1, 2, 3, 4, 5, 6, 7, 8, 9, 10, or more phosphorothioate internucleotide linkages, and/or one or more (*e.g.,* about 1, 2, 3, 4, 5, 6, 7, 8, 9, 10 or more) 2'-deoxy, 2'-O-methyl, 2'-deoxy-2'-fluoro, and/or about one or more (*e.g.,* about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the sense strand; and wherein the antisense strand comprises about 1 to about 10 or more, specifically about 1, 2, 3, 4, 5, 6, 7, 8, 9, 10, or more phosphorothioate internucleotide linkages, and/or one or more (*e.g.*, about 1, 2, 3, 4, 5, 6, 7, 8, 9, 10 or more) 2'-deoxy, 2'-O-methyl, 2'-deoxy-2'-fluoro, and/or one or more (*e.g.,* about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In another embodiment, one or more, for example about 1, 2, 3, 4, 5, 6, 7, 8, 9, 10, or more, pyrimidine nucleotides of the sense and/or antisense siNA strand are chemically-modified with 2'-deoxy, 2'-O-methyl and/or 2'-deoxy-2'-fluoro nucleotides, with or without one or more, for example about 1, 2, 3, 4, 5, 6, 7, 8, 9, 10, or more, phosphorothioate internucleotide linkages and/or a terminal cap molecule at the 3'-end, the 5'-end, or both of the 3'- and 5'-ends, being present in the same or different strand.

In another embodiment, the invention features a siNA molecule, wherein the sense strand comprises about 1 to about 5, specifically about 1, 2, 3, 4, or 5 phosphorothioate internucleotide linkages, and/or one or more (*e*.*g*., about 1, 2, 3, 4, 5, or more) 2'-deoxy, 2'-O-methyl, 2'-deoxy-2'-fluoro, and/or one or more (*e*.*g*., about 1, 2, 3, 4, 5, or more) universal base modified nucleotides, and optionally a terminal cap molecule at the 3-end, the 5'-end, or both of the 3'- and 5'-ends of the sense strand; and wherein the antisense strand comprises about 1 to about 5 or more, specifically about 1, 2, 3, 4, 5, or more phosphorothioate internucleotide linkages, and/or one or more (*e.g*., about 1, 2, 3, 4, 5, 6, 7, 8, 9, 10 or more) 2'-deoxy, 2'-O-methyl, 2'-deoxy-2'-fluoro, and/or one or more (*e.g.,* about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In another embodiment, one or more, for example about 1, 2, 3, 4, 5, 6, 7, 8, 9, 10, or more, pyrimidine nucleotides of the sense and/or antisense siNA strand are chemically-modified with 2'-deoxy, 2'-O-methyl and/or 2'-deoxy-2'-fluoro nucleotides, with or without about 1 to about 5 or more, for example about 1, 2, 3, 4, 5, or more phosphorothioate internucleotide linkages and/or a terminal cap molecule at the 3'-end, the 5'-end, or both of the 3'- and 5'-ends, being present in the same or different strand.

In one embodiment, the invention features a siNA molecule, wherein the antisense strand comprises one or more, for example, about 1, 2, 3, 4, 5, 6, 7, 8, 9, 10, or more phosphorothioate internucleotide linkages, and/or about one or more (*e.g.,* about 1, 2, 3, 4, 5, 6, 7, 8, 9, 10 or more) 2'-deoxy, 2'-O-methyl, 2'-deoxy-2'-fluoro, and/or one or more (*e.g.*, about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the sense strand; and wherein the antisense strand comprises about 1 to about 10 or more, specifically about 1, 2, 3, 4, 5, 6, 7, 8, 9, 10 or more phosphorothioate internucleotide linkages, and/or one or more (*e.g*., about 1, 2, 3, 4, 5, 6, 7, 8, 9, 10 or more) 2'-deoxy, 2'-O-methyl, 2'-deoxy-2'-fluoro, and/or one or more (*e*.*g*., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In another embodiment, one or more, for example about 1, 2, 3, 4, 5, 6, 7, 8, 9, 10 or more pyrimidine nucleotides of the sense and/or antisense siNA strand are chemically-modified with 2'-deoxy, 2'-O-methyl and/or 2'-deoxy-2'-fluoro nucleotides, with or without one or more, for example, about 1, 2, 3, 4, 5, 6, 7, 8, 9, 10 or more phosphorothioate internucleotide linkages and/or a terminal cap molecule at the 3'-end, the 5'-end, or both of the 3' and 5'-ends, being present in the same or different strand.

In another embodiment, the invention features a siNA molecule, wherein the antisense strand comprises about 1 to about 5 or more, specifically about 1, 2, 3, 4, 5 or more phosphorothioate internucleotide linkages, and/or one or more (*e.g.*, about 1, 2, 3, 4, 5, 6, 7, 8, 9, 10 or more) 2'-deoxy, 2'-O-methyl, 2'-deoxy-2'-fluoro, and/or one or more (*e.g.*, about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the sense strand; and wherein the antisense strand comprises about 1 to about 5 or more, specifically about 1, 2, 3, 4, 5 or more phosphorothioate internucleotide linkages, and/or one or more (*e.g.,* about 1, 2, 3, 4, 5, 6, 7, 8, 9, 10 or more) 2'-deoxy, 2'-O-methyl, 2'-deoxy-2'-fluoro, and/or one or more (*e*.*g*., about 1, 2, 3, 4, 5, 6, 7, 8, 9, 10 or more) universal base modified nucleotides, and optionally a terminal cap molecule at the 3'-end, the 5'-end, or both of the 3'- and 5'-ends of the antisense strand. In another embodiment, one or more, for example about 1, 2, 3, 4, 5, 6, 7, 8, 9, 10 or more pyrimidine nucleotides of the sense and/or antisense siNA strand are chemically-modified with 2'-deoxy, 2'-O-methyl and/or 2'-deoxy-2'-fluoro nucleotides, with or without about 1 to about 5, for example about 1, 2, 3, 4, 5 or more phosphorothioate internucleotide linkages and/or a terminal cap molecule at the 3'-end, the 5'-end, or both of the 3'- and 5'-ends, being present in the same or different strand.

In one embodiment, the invention features a chemically-modified short interfering nucleic acid (siNA) molecule having about 1 to about 5, specifically about 1, 2, 3, 4, 5 or more phosphorothioate internucleotide linkages in each strand of the siNA molecule.

In another embodiment, the invention features a siNA molecule comprising 2'-5' internucleotide linkages. The 2'-5' internucleotide linkage(s) can be at the 3'-end, the 5'-end, or both of the 3'- and 5'-ends of one or both siNA sequence strands. In addition, the 2'-5' internucleotide linkage(s) can be present at various other positions within one or both siNA sequence strands, for example, about 1, 2, 3, 4, 5, 6, 7, 8, 9, 10, or more including every internucleotide linkage of a pyrimidine nucleotide in one or both strands of the siNA molecule can comprise a 2'-5' internucleotide linkage, or about 1, 2, 3, 4, 5, 6, 7, 8, 9, 10, or more including every internucleotide linkage of a purine nucleotide in one or both strands of the siNA molecule can comprise a 2'-5' internucleotide linkage.

In another embodiment, a chemically-modified siNA molecule of the invention comprises a duplex having two strands, one or both of which can be chemically-modified, wherein each strand is about 18 to about 27 (*e*.*g*., about 18, 19, 20, 21, 22, 23, 24, 25, 26, or 27) nucleotides in length, wherein the duplex has about 18 to about 23 (*e.g.,* about 18, 19, 20, 21, 22, or 23) base pairs, and wherein the chemical modification comprises a structure having any of Formulae I-VII. For example, an exemplary chemically-modified siNA molecule of the invention comprises a duplex having two strands, one or both of which can be chemically-modified with a chemical modification having any of Formulae I-VII or any combination thereof, wherein each strand consists of about 21 nucleotides, each having a 2-nucleotide 3'-terminal nucleotide overhang, and wherein the duplex has about 19 base pairs. In another embodiment, a siNA molecule of the invention comprises a single stranded hairpin structure, wherein the siNA is about 36 to about 70 (*e.g*., about 36, 40, 45, 50, 55, 60, 65, or 70) nucleotides in length having about 18 to about 23 (*e.g.,* about 18, 19, 20, 21, 22, or 23) base pairs, and wherein the siNA can include a chemical modification comprising a structure having any of Formulae I-VII or any combination thereof. For example, an exemplary chemically-modified siNA molecule of the invention comprises a linear oligonucleotide having about 42 to about 50 (*e.g.*, about 42, 43, 44, 45, 46, 47, 48, 49, or 50) nucleotides that is chemically-modified with a chemical modification having any of Formulae I-VII or any combination thereof, wherein the linear oligonucleotide forms a hairpin structure having about 19 base pairs and a 2-nucleotide 3'-terminal nucleotide overhang. In another embodiment, a linear hairpin siNA molecule of the invention contains a stem loop motif, wherein the loop portion of the siNA molecule is biodegradable. For example, a linear hairpin siNA molecule of the invention is designed such that degradation of the loop portion of the siNA molecule *in vivo* can generate a double-stranded siNA molecule with 3'-terminal overhangs, such as 3'-terminal nucleotide overhangs comprising about 2 nucleotides.

In another embodiment, a siNA molecule of the invention comprises a circular nucleic acid molecule, wherein the siNA is about 38 to about 70 (*e.g.,* about 38, 40, 45, 50, 55, 60, 65, or 70) nucleotides in length having about 18 to about 23 (*e*.*g*., about 18, 19, 20, 21, 22, or 23) base pairs, and wherein the siNA can include a chemical modification, which comprises a structure having any of Formulae I-VII or any combination thereof. For example, an exemplary chemically-modified siNA molecule of the invention comprises a circular oligonucleotide having about 42 to about 50 (*e.g.,* about 42, 43, 44, 45, 46, 47, 48, 49, or 50) nucleotides that is chemically-modified with a chemical modification having any of Formulae I-VII or any combination thereof, wherein the circular oligonucleotide forms a dumbbell shaped structure having about 19 base pairs and 2 loops.

In another embodiment, a circular siNA molecule of the invention contains two loop motifs, wherein one or both loop portions of the siNA molecule is biodegradable. For example, a circular siNA molecule of the invention is designed such that degradation of the loop portions of the siNA molecule *in vivo* can generate a double-stranded siNA molecule with 3'-terminal overhangs, such as 3'-terminal nucleotide overhangs comprising about 2 nucleotides.

In one embodiment, a siNA molecule of the invention comprises at least one (*e*.*g*., about 1, 2, 3, 4, 5, 6, 7, 8, 9, 10, or more) abasic moiety, for example a compound having Formula V: 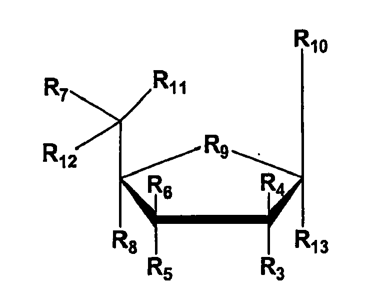 wherein each R3, R4, R5, R6, R7, R8, R10, R11, R12, and R13 is independently H, OH, alkyl, substituted alkyl, alkaryl or aralkyl, F, Cl, Br, CN, CF3, OCF3, OCN, O-alkyl, S-alkyl, N-alkyl, O-alkenyl, S-alkenyl, N-alkenyl, SO-alkyl, alkyl-OSH, alkyl-OH, O-alkyl-OH, O-alkyl-SH, S-alkyl-OH, S-alkyl-SH, alkyl-S-alkyl, alkyl-O-alkyl, ONO2, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalklylamino, substituted silyl, or group having Formula I; R9 is O, S, CH2, S=O, CHF, or CF2.

In one embodiment, a siNA molecule of the invention comprises at least one (*e.g.,* about 1, 2, 3, 4, 5, 6, 7, 8, 9, 10, or more) inverted abasic moiety, for example a compound having Formula VI: 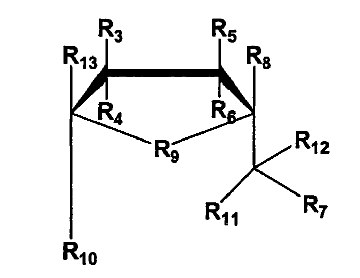 wherein each R3, R4, R5, R6, R7, R8, R10, R11, R12, and R13 is independently H, OH, alkyl, substituted alkyl, alkaryl or aralkyl, F, Cl, Br, CN, CF3, OCF3, OCN, O-alkyl, S-alkyl, N-alkyl, O-alkenyl, S-alkenyl, N-alkenyl, SO-alkyl, alkyl-OSH, alkyl-OH, O-alkyl-OH, O-alkyl-SH, S-alkyl-OH, S-alkyl-SH, alkyl-S-alkyl, alkyl-O-alkyl, ONO2, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalklylamino, substituted silyl, or group having Formula I; R9 is O, S, CH2, S=O, CHF, or CF2, and either R2, R3, R8 or R13 serve as points of attachment to the siNA molecule of the invention.

In another embodiment, a siNA molecule of the invention comprises at least one (*e.g.,* about 1, 2, 3, 4, 5, 6, 7, 8, 9, 10, or more) substituted polyalkyl moieties, for example a compound having Formula VII: 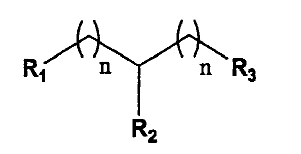 wherein each n is independently an integer from 1 to 12, each R1, R2 and R3 is independently H, OH, alkyl, substituted alkyl, alkaryl or aralkyl, F, Cl, Br, CN, CF3, OCF3, OCN, O-alkyl, S-alkyl, N-alkyl, O-alkenyl, S-alkenyl, N-alkenyl, SO-alkyl, alkyl-OSH, alkyl-OR, O-alkyl-OH, O-alkyl-SH, S-alkyl-OH, S-alkyl-SH, alkyl-S-alkyl, alkyl-O-alkyl, ONO2, NO2, N3, NH2, aminoalkyl, aminoacid, aminoacyl, ONH2, O-aminoalkyl, O-aminoacid, O-aminoacyl, heterocycloalkyl, heterocycloalkaryl, aminoalkylamino, polyalklylamino, substituted silyl, or a group having Formula I, and R1, R2 or R3 serves as points of attachment to the siNA molecule of the invention.

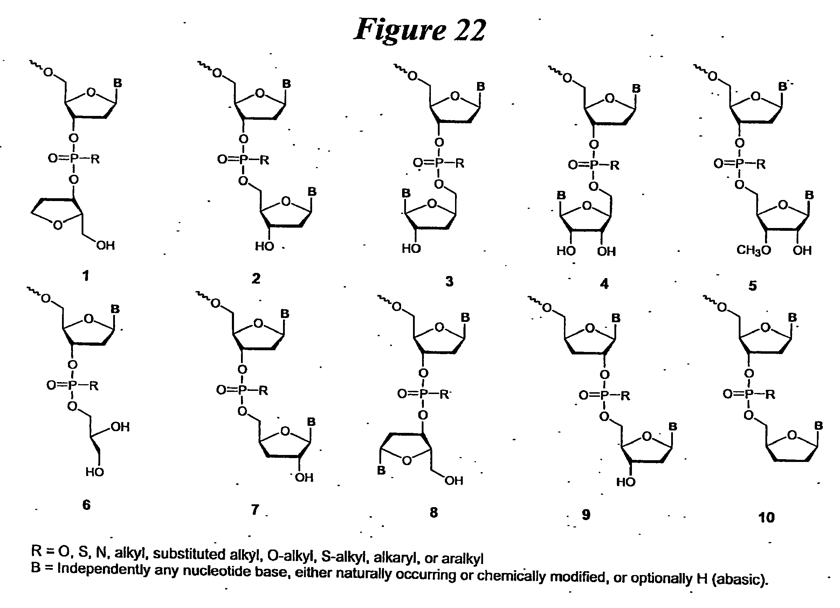

In another embodiment, the invention features a compound having Formula VII, wherein R1 and R2 are hydroxyl (OH) groups, n = 1, and R3 comprises O and is the point of attachment to the 3'-end, the 5'-end, or both of the 3' and 5'-ends of one or both strands of a double-stranded siNA molecule of the invention or to a single-stranded siNA molecule of the invention. This modification is referred to herein as "glyceryl" (for example modification 6 in **Figure 22**).

In another embodiment, a moiety having any of Formula V, VI or VII of the invention is at the 3'-end, the 5'-end, or both of the 3' and 5'-ends of a siNA molecule of the invention. For example, a moiety having Formula V, VI or VII can be present at the 3'-end, the 5'-end, or both of the 3' and 5'-ends of the antisense strand, the sense strand, or both antisense and sense strands of the siNA molecule. In addition, a moiety having Formula VII can be present at the 3'-end or the 5'-end of a hairpin siNA molecule as described herein.

In another embodiment, a siNA molecule of the invention comprises an abasic residue having Formula V or VI, wherein the abasic residue having Formula VI or VI is connected to the siNA construct in a 3'-3', 3'-2', 2'-3', or 5'-5' configuration, such as at the 3'-end, the 5'-end, or both of the 3' and 5'-ends of one or both siNA strands.

In one embodiment, a siNA molecule of the invention comprises one or more (e.g., about 1, 2, 3, 4, 5, 6, 7, 8, 9, 10, or more) locked nucleic acid (LNA) nucleotides, for example at the 5'-end, the 3'-end, both of the 5' and 3'-ends, or any combination thereof, of the siNA molecule.

In another embodiment, a siNA molecule of the invention comprises one or more (*e.g*., about 1, 2, 3, 4, 5, 6, 7, 8, 9, 10, or more) acyclic nucleotides, for example at the 5'-end, the 3'-end, both of the 5' and 3'-ends, or any combination thereof, of the siNA molecule.

In one embodiment, the invention features a chemically-modified short interfering nucleic acid (siNA) molecule of the invention, wherein the chemically-modified siNA comprises a sense region, where any (*e*.*g*., one or more or all) pyrimidine nucleotides present in the sense region are 2'-deoxy-2'-fluoro pyrimidine nucleotides (*e.g.,* wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and where any (*e.g.*, one or more or all) purine nucleotides present in the sense region are 2'-deoxy purine nucleotides (*e.g.,* wherein all purine nucleotides are 2'-deoxy purine nucleotides or alternately a plurality of purine nucleotides are 2'-deoxy purine nucleotides).

In one embodiment, the invention features a chemically-modified short interfering nucleic acid (siNA) molecule of the invention, wherein the chemically-modified siNA comprises a sense region, where any (*e*.*g*., one or more or all) pyrimidine nucleotides present in the sense region are 2'-deoxy-2'-fluoro pyrimidine nucleotides (*e.g.,* wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and where any (*e*.*g*., one or more or all) purine nucleotides present in the sense region are 2'-deoxy purine nucleotides (*e*.*g*., wherein all purine nucleotides are 2'-deoxy purine nucleotides or alternately a plurality of purine nucleotides are 2'-deoxy purine nucleotides), wherein any nucleotides comprising a 3'-terminal nucleotide overhang that are present in said sense region are 2'-deoxy nucleotides.

In one embodiment, the invention features a chemically-modified short interfering nucleic acid (siNA) molecule of the invention, wherein the chemically-modified siNA comprises an antisense region, where any (*e*.*g*., one or more or all) pyrimidine nucleotides present in the antisense region are 2'-deoxy-2'-fluoro pyrimidine nucleotides (*e*.*g*., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any (*e*.*g*., one or more or all) purine nucleotides present in the antisense region are 2'-O-methyl purine nucleotides (*e*.*g*., wherein all purine nucleotides are 2'-O-methyl purine nucleotides or alternately a plurality of purine nucleotides are 2'-O-methyl purine nucleotides).

In one embodiment, the invention features a chemically-modified short interfering nucleic acid (siNA) molecule of the invention, wherein the chemically-modified siNA comprises an antisense region, where any (*e.g.,* one or more or all) pyrimidine nucleotides present in the antisense region are 2'-deoxy-2'-fluoro pyrimidine nucleotides (*e*.*g*., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any (*e.g.,* one or more or all) purine nucleotides present in the antisense region are 2'-O-methyl purine nucleotides (*e.g.,* wherein all purine nucleotides are 2'-O-methyl purine nucleotides or alternately a plurality of purine nucleotides are 2'-O-methyl purine nucleotides), wherein any nucleotides comprising a 3'-terminal nucleotide overhang that are present in said antisense region are 2'-deoxy nucleotides.

In one embodiment, the invention features a chemically-modified short interfering nucleic acid (siNA) molecule of the invention, wherein the chemically-modified siNA comprises an antisense region, where any (*e*.*g*., one or more or all) pyrimidine nucleotides present in the antisense region are 2'-deoxy-2'-fluoro pyrimidine nucleotides (*e.g.,* wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and where any (*e*.*g*., one or more or all) purine nucleotides present in the antisense region are 2'-deoxy purine nucleotides (*e.g.,* wherein all purine nucleotides are 2'-deoxy purine nucleotides or alternately a plurality of purine nucleotides are 2'-deoxy purine nucleotides).

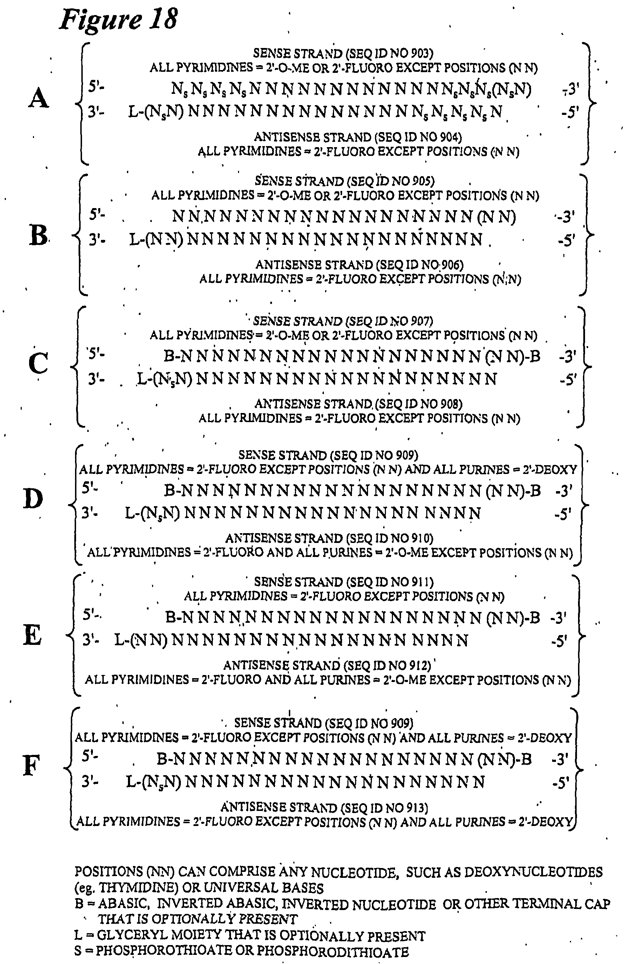

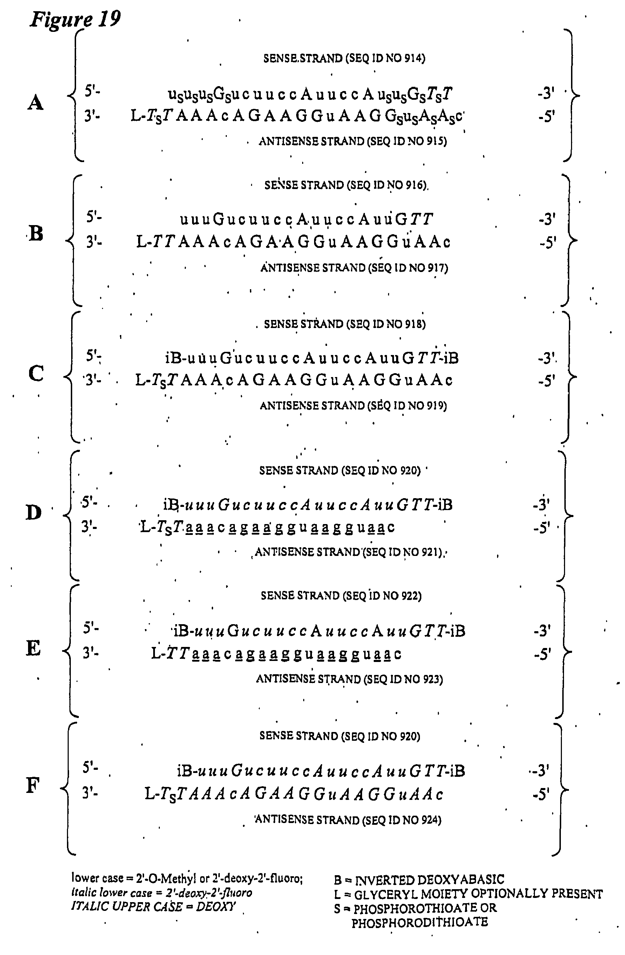

In one embodiment, the invention features a chemically-modified short interfering nucleic acid (siNA) molecule of the invention capable of mediating RNA interference (RNAi) inside a cell or reconstituted *in vitro* system, wherein the chemically-modified siNA comprises a sense region and an antisense region. The sense region comprises one or more 2'-deoxy-2'-fluoro pyrimidine nucleotides (*e.g.*, wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and one or more 2'-deoxy purine nucleotides (*e*.*g*., wherein all purine nucleotides are 2'-deoxy purine nucleotides or alternately a plurality of purine nucleotides are 2'-deoxy purine nucleotides). Inverted deoxy abasic modifications can be optionally present at the 3'-end, the 5'-end, or both of the 3' and 5'-ends of the sense region. The sense region optionally further comprises a 3'-terminal overhang having about 1 to about 4 (e.g., about 1, 2, 3, or 4) 2'-deoxyribonucleotides. The antisense region comprises one or more 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and one or more 2'-O-methyl purine nucleotides (e.g., wherein all purine nucleotides are 2'-O-methyl purine nucleotides or alternately a plurality of purine nucleotides are 2'-O-methyl purine nucleotides). A terminal cap modification, such as any modification described herein or shown in **Figure 22**, is optionally present at the 3'-end, the 5'-end, or both of the 3' and 5'-ends of the antisense sequence. The antisense region optionally further comprises a 3'-terminal nucleotide overhang having about 1 to about 4 (e.g., about 1, 2, 3, or 4) 2'-deoxynucleotides, wherein the overhang nucleotides can further comprise one or more (e.g., 1, 2, 3, or 4) phosphorothioate internucleotide linkages. Non-limiting examples of these chemically-modified siNAs are shown in **Figures 18 and 19** and **Table IV** herein.

In one embodiment, the invention features a chemically-modified short interfering nucleic acid (siNA) molecule of the invention capable of mediating RNA interference (RNAi) inside a cell or reconstituted *in vitro* system, wherein the siNA comprises a sense region and an antisense region, wherein the sense region comprises one or more 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and one or more purine ribonucleotides (e.g., wherein all purine nucleotides are purine ribonucleotides or alternately a plurality of purine nucleotides are purine ribonucleotides) and wherein the antisense region comprises one or more 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and one or more 2'-O-methyl purine nucleotides (e.g., wherein all purine nucleotides are 2'-O-methyl purine nucleotides or alternately a plurality of purine nucleotides are 2'-O-methyl purine nucleotides). Inverted deoxy abasic modifications are optionally present at the 3'-end, the 5'-end, or both of the 3' and 5'-ends of the sense region. The sense region optionally further comprises a 3'-terminal overhang having about 1 to about 4 (e.g., about 1, 2, 3, or 4) 2'-deoxyribonucleotides. A terminal cap modification, such as any modification described herein or shown in **Figure 22,** is optionally present at the 3'-end, the 5'-end, or both of the 3' and 5'-ends of the antisense sequence. The antisense region optionally further comprises a 3'-terminal nucleotide overhang having about 1 to about 4 (e.g., about 1, 2, 3, or 4) 2'-deoxynucleotides, wherein the overhang nucleotides can further comprise one or more (e.g., 1, 2, 3, or 4) phosphorothioate internucleotide linkages. Non-limiting examples of these chemically-modified siNAs are shown in **Figures 18 and 19** and **Table IV** herein.

In one embodiment, the invention features a chemically-modified short interfering nucleic acid (siNA) molecule of the invention capable of mediating RNA interference (RNAi) inside a cell or reconstituted *in vitro* system, wherein the chemically-modified siNA comprises a sense region and an antisense region, wherein the sense region comprises one or 2'-deoxy-2'-fluoro pyrimidine nucleotides (**e.g.**, wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and one or more purine nucleotides selected from the group consisting of 2'-deoxy nucleotides, locked nucleic acid (LNA) nucleotides, 2'-methoxyethyl nucleotides, 4'-thionucleotides, and 2'-O-methyl nucleotides (*e*.*g*., wherein all purine nucleotides are selected from the group consisting of 2'-deoxy nucleotides, locked nucleic acid (LNA) nucleotides, 2'-methoxyethyl nucleotides, 4'-thionucleotides, and 2'-O-methyl nucleotides or alternately a plurality of purine nucleotides are selected from the group consisting of 2'-deoxy nucleotides, locked nucleic acid (LNA) nucleotides, 2'-methoxyethyl nucleotides, 4'-thionucleotides, and 2'-O-methyl nucleotides) and wherein the antisense region comprises one or more 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and one or more purine nucleotides selected from the group consisting of 2'-deoxy nucleotides, locked nucleic acid (LNA) nucleotides, 2'-methoxyethyl nucleotides, 4'-thionucleotides, and 2'-O-methyl nucleotides (e.g., wherein all purine nucleotides are selected from the group consisting of 2'-deoxy nucleotides, locked nucleic acid (LNA) nucleotides, 2'-methoxyethyl nucleotides, 4'-thionucleotides, and 2'-O-methyl nucleotides or alternately a plurality of purine nucleotides are selected from the group consisting of 2'-deoxy nucleotides, locked nucleic acid (LNA) nucleotides, 2'-methoxyethyl nucleotides, 4'-thionucleotides, and 2'-O-methyl nucleotides). Inverted deoxy abasic modifications are optionally present at the 3'-end, the 5'-end, or both of the 3' and 5'-ends of the sense region. The sense region optionally further comprises a 3'-terminal overhang having about 1 to about 4 (e.g., about 1, 2, 3, or 4) 2'-deoxyribonucleotides. A terminal cap modification, such as any modification described herein or shown in **Figure 22,** is optionally present at the 3'-end, the 5'-end, or both of the 3' and 5'-ends of the antisense sequence. The antisense region optionally further comprises a 3'-terminal nucleotide overhang having about 1 to about 4 (e.g., about 1, 2, 3, or 4) 2'-deoxynucleotides, wherein the overhang nucleotides can further comprise one or more (e.g., 1, 2, 3, or 4) phosphorothioate internucleotide linkages.

In another embodiment, any modified nucleotides present in the siNA molecules of the invention, preferably in the antisense strand of the siNA molecules of the invention, but also optionally in the sense and/or both antisense and sense strands, comprise modified nucleotides having properties or characteristics similar to naturally occurring ribonucleotides. For example, the invention features siNA molecules including modified nucleotides having a Northern conformation (e.g., Northern pseudorotation cycle, see for example Saenger, Principles of Nucleic Acid Structure, Springer-Verlag ed., 1984). As such, chemically modified nucleotides present in the siNA molecules of the invention, preferably in the antisense strand of the siNA molecules of the invention, but also optionally in the sense and/or both antisense and sense strands, are resistant to nuclease degradation while at the same time maintaining the capacity to mediate RNAi. Non-limiting examples of nucleotides having a northern configuration include locked nucleic acid (LNA) nucleotides (e.g., 2'-O,4'-C-methylene-(D-ribofuranosyl) nucleotides); 2'-methoxyethoxy (MOB) nucleotides; 2'-methyl-thio-ethyl, 2'-deoxy-2'-fluoro nucleotides, 2'-deoxy-2'-chloro nucleotides, 2'-azido nucleotides, and 2'-O-methyl nucleotides.

In one embodiment, the invention features a chemically-modified short interfering nucleic acid molecule (siNA) capable of mediating RNA interference (RNAi) inside a cell or reconstituted *in vitro* system, wherein the chemical modification comprises a conjugate covalently attached to the chemically-modified siNA molecule. In another embodiment, the conjugate is covalently attached to the chemically-modified siNA molecule via a biodegradable linker. In one embodiment, the conjugate molecule is attached at the 3'-end of either the sense strand, the antisense strand, or both strands of the chemically-modified siNA molecule. In another embodiment, the conjugate molecule is attached at the 5'-end of either the sense strand, the antisense strand, or both strands of the chemically-modified siNA molecule. In yet another embodiment, the conjugate molecule is attached both the 3'-end and 5'-end of either the sense strand, the antisense strand, or both strands of the chemically-modified siNA molecule, or any combination thereof. In one embodiment, a conjugate molecule of the invention comprises a molecule that facilitates delivery of a chemically-modified siNA molecule into a biological system, such as a cell. In another embodiment, the conjugate molecule attached to the chemically-modified siNA molecule is a poly ethylene glycol, human serum albumin, or a ligand for a cellular receptor that can mediate cellular uptake. Examples of specific conjugate molecules contemplated by the instant invention that can be attached to chemically-modified siNA molecules are described in Vargeese et al., U.S. Serial No. 10/201,394, incorporated by reference herein. The type of conjugates used and the extent of conjugation of siNA molecules of the invention can be evaluated for improved pharmacokinetic profiles, bioavailability, and/or stability of siNA constructs while at the same time maintaining the ability of the siNA to mediate RNAi activity. As such, one skilled in the art can screen siNA constructs that are modified with various conjugates to determine whether the siNA conjugate complex possesses improved properties while maintaining the ability to mediate RNAi, for example in animal models as are generally known in the art.

In one embodiment, the invention features a short interfering nucleic acid (siNA) molecule of the invention, wherein the siNA further comprises a nucleotide, non-nucleotide, or mixed nucleotide/non-nucleotide linker that joins the sense region of the siNA to the antisense region of the siNA. In one embodiment, a nucleotide linker of the invention can be a linker of ≥ 2 nucleotides in length, for example 3, 4, 5, 6, 7, 8, 9, or 10 nucleotides in length. In another embodiment, the nucleotide linker can be a nucleic acid aptamer. By "aptamer" or "nucleic acid aptamer" as used herein is meant a nucleic acid molecule that binds specifically to a target molecule wherein the nucleic acid molecule has sequence that comprises a sequence recognized by the target molecule in its natural setting. Alternately, an aptamer can be a nucleic acid molecule that binds to a target molecule where the target molecule does not naturally bind to a nucleic acid. The target molecule can be any molecule of interest. For example, the aptamer can be used to bind to a ligand-binding domain of a protein, thereby preventing interaction of the naturally occurring ligand with the protein. This is a non-limiting example and those in the art will recognize that other embodiments can be readily generated using techniques generally known in the art. (See, for example, Gold et al., 1995, Annu. Rev. Biochem., 64, 763; Brody and Gold, 2000, J. Biotechnol., 74, 5; Sun, 2000, Curr. Opin. Mol. Ther., 2, 100; Kusser, 2000, J. Biotechnol., 74, 27; Hermann and Patel, 2000, Science, 287, 820; and Jayasena, 1999, Clinical Chemistry, 45, 1628.)

In yet another embodiment, a non-nucleotide linker of the invention comprises abasic nucleotide, polyether, polyamine, polyamide, peptide, carbohydrate, lipid, polyhydrocarbon, or other polymeric compounds (e.g. polyethylene glycols such as those having between 2 and 100 ethylene glycol units). Specific examples include those described by Seela and Kaiser, Nucleic Acids Res. 1990, 18:6353 and Nucleic Acids Res. 1987, 15:3113; Cload and Schepartz, J. Am. Chem. Soc. 1991, 113:6324; Richardson and Schepartz, J. Am. Chem. Soc. 1991, 113:5109; Ma et al., Nucleic Acids Res. 1993, 21:2585 and Biochemistry 1993, 32:1751; Durand et al., Nucleic Acids Res. 1990, 18:6353; McCurdy et al., Nucleosides & Nucleotides 1991, 10:287; Jschke et al., Tetrahedron Lett. 1993, 34:301; Ono et al., Biochemistry 1991, 30:9914; Arnold et al., International Publication No. WO 89/02439; Usman et al., International Publication No. WO 95/06731; Dudycz et al., International Publication No. WO 95/11910 and Ferentz and Verdine, J. Am. Chem. Soc. 1991, 113:4000, all hereby incorporated by reference herein. A "non-nucleotide" further means any group or compound that can be incorporated into a nucleic acid chain in the place of one or more nucleotide units, including either sugar and/or phosphate substitutions, and allows the remaining bases to exhibit their enzymatic activity. The group or compound can be abasic in that it does not contain a commonly recognized nucleotide base, such as adenosine, guanine, cytosine, uracil or thymine, for example at the C1 position of the sugar.

In one embodiment, the invention features a short interfering nucleic acid (siNA) molecule capable of mediating RNA interference (RNAi) inside a cell or reconstituted in vitro system, wherein one or both strands of the siNA molecule that are assembled from two separate oligonucleotides do not comprise any ribonucleotides. For example, a siNA molecule can be assembled from a single oligonculeotide where the sense and antisense regions of the siNA comprise separate oligonucleotides not having any ribonucleotides (e.g., nucleotides having a 2'-OH group) present in the oligonucleotides. In another example, a siNA molecule can be assembled from a single oligonculeotide where the sense and antisense regions of the siNA are linked or circularized by a nucleotide or non-nucleotide linker as desrcibed herein, wherein the oligonucleotide does not have any ribonucleotides (e.g., nucleotides having a 2'-OH group) present in the oligonucleotide. Applicant has surprisingly found that the presense of ribonucleotides (e.g., nucleotides having a 2'-hydroxyl group) within the siNA molecule is not required or essential to support RNAi activity. As such, in one embodiment, all positions within the siNA can include chemically modified nucleotides and/or non-nucleotides such as nucleotides and or non-nucleotides having Formula I, II, III, IV, V, VI, or VII or any combination thereof to the extent that the ability of the siNA molecule to support RNAi activity in a cell is maintained.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence. In another embodiment, the single stranded siNA molecule of the invention comprises a 5'-terminal phosphate group. In another embodiment, the single stranded siNA molecule of the invention comprises a 5'-terminal phosphate group and a 3'-terminal phosphate group (e.g., a 2', 3'-cyclic phosphate). In another embodiment, the single stranded siNA molecule of the invention comprises about 19 to about 29 nucleotides. In yet another embodiment, the single stranded siNA molecule of the invention comprises one or more chemically modified nucleotides or non-nucleotides described herein. For example, all the positions within the siNA molecule can include chemically-modified nucleotides such as nucleotides having any of Formulae I-VII, or any combination thereof to the extent that the ability of the siNA molecule to support RNAi activity in a cell is maintained.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence, and wherein one or more pyrimidine nucleotides present in the siNA are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the antisense region are 2'-O-methyl purine nucleotides (e.g., wherein all purine nucleotides are 2'-O-methyl purine nucleotides or alternately a plurality of purine nucleotides are 2'-O-methyl purine nucleotides), and a terminal cap modification, such as any modification described herein or shown in Figure 22, that is optionally present at the 3'-end, the 5'-end, or both of the 3' and 5'-ends of the antisense sequence, the siNA optionally further comprising about 1 to about 4 (e.g., about 1, 2, 3, or 4) terminal 2'-deoxynucleotides at the 3'-end of the siNA molecule, wherein the terminal nucleotides can further comprise one or more (e.g., 1, 2, 3, or 4) phosphorothioate internucleotide linkages, and wherein the siNA optionally further comprises a terminal phosphate group, such as a 5'-terminal phosphate group.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence, and wherein one or more pyrimidine nucleotides present in the siNA are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the antisense region are 2'-deoxy purine nucleotides (e.g., wherein all purine nucleotides are 2'-deoxy purine nucleotides or alternately a plurality of purine nucleotides are 2'-deoxy purine nucleotides), and a terminal cap modification, such as any modification described herein or shown in **Figure 22**, that is optionally present at the 3'-end, the 5'-end, or both of the 3' and 5'-ends of the antisense sequence, the siNA optionally further comprising about 1 to about 4 (e.g., about 1, 2, 3, or 4) terminal 2'-deoxynucleotides at the 3'-end of the siNA molecule, wherein the terminal nucleotides can further comprise one or more (e.g., 1, 2, 3, or 4) phosphorothioate internucleotide linkages, and wherein the siNA optionally further comprises a terminal phosphate group, such as a 5'-terminal phosphate group.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence, and wherein one or more pyrimidine nucleotides present in the siNA are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the antisense region are locked nucleic acid (LNA) nucleotides (e.g., wherein all purine nucleotides are LNA nucleotides or alternately a plurality of purine nucleotides are LNA nucleotides), and a terminal cap modification, such as any modification described herein or shown in Figure 22, that is optionally present at the 3'-end, the 5'-end, or both of the 3' and 5'-ends of the antisense sequence, the siNA optionally further comprising about 1 to about 4 (e.g., about 1, 2, 3, or 4) terminal 2'-deoxynucleotides at the 3'-end of the siNA molecule, wherein the terminal nucleotides can further comprise one or more (e.g., 1, 2, 3, or 4) phosphorothioate internucleotide linkages, and wherein the siNA optionally further comprises a terminal phosphate group, such as a 5'-terminal phosphate group.

In one embodiment, a siNA molecule of the invention is a single stranded siNA molecule that mediates RNAi activity in a cell or reconstituted in vitro system, wherein the siNA molecule comprises a single stranded polynucleotide having complementarity to a target nucleic acid sequence, and wherein one or more pyrimidine nucleotides present in the siNA are 2'-deoxy-2'-fluoro pyrimidine nucleotides (e.g., wherein all pyrimidine nucleotides are 2'-deoxy-2'-fluoro pyrimidine nucleotides or alternately a plurality of pyrimidine nucleotides are 2'-deoxy-2'-fluoro pyrimidine nucleotides), and wherein any purine nucleotides present in the antisense region are 2'-methoxyethyl purine nucleotides (e.g., wherein all purine nucleotides are 2'-methoxyethyl purine nucleotides or alternately a plurality of purine nucleotides are 2'-methoxyethyl purine nucleotides), and a terminal cap modification, such as any modification described herein or shown in **Figure 22**, that is optionally present at the 3'-end, the 5'-end, or both of the 3' and 5'-ends of the antisense sequence, the siNA optionally further comprising about 1 to about 4 (e.g., about 1, 2, 3, or 4) terminal 2'-deoxynucleotides at the 3'-end of the siNA molecule, wherein the terminal nucleotides can further comprise one or more (e.g., 1, 2, 3, or 4) phosphorothioate internucleotide linkages, and wherein the siNA optionally further comprises a terminal phosphate group, such as a 5'-terminal phosphate group.

In another embodiment, any modified nucleotides present in the single stranded siNA molecules of the invention comprise modified nucleotides having properties or characteristics similar to naturally occurring ribonucleotides. For example, the invention features siNA molecules including modified nucleotides having a Northern conformation (e.g., Northern pseudorotation cycle, see for example Saenger, *Principles of Nucleic Acid Structure,* Springer-Verlag ed., 1984). As such, chemically modified nucleotides present in the single stranded siNA molecules of the invention are preferably resistant to nuclease degradation while at the same time maintaining the capacity to mediate RNAi.

In one embodiment, the invention features a method for modulating the expression of a gene within a cell comprising: (a) synthesizing a siNA molecule of the invention, which can be chemically-modified, wherein one of the siNA strands comprises a sequence complementary to RNA of the gene; and (b) introducing the siNA molecule into a cell under conditions suitable to modulate the expression of the gene in the cell.

In one embodiment, the invention features a method for modulating the expression of a gene within a cell comprising: (a) synthesizing a siNA molecule of the invention, which can be chemically-modified, wherein one of the siNA strands comprises a sequence complementary to RNA of the gene and wherein the sense strand sequence of the siNA comprises a sequence substantially similar to the sequence of the target RNA; and (b) introducing the siNA molecule into a cell under conditions suitable to modulate the expression of the gene in the cell.

In another embodiment, the invention features a method for modulating the expression of more than one gene within a cell comprising: (a) synthesizing siNA molecules of the invention, which can be chemically-modified, wherein one of the siNA strands comprises a sequence complementary to RNA of the genes; and (b) introducing the siNA molecules into a cell under conditions suitable to modulate the expression of the genes in the cell.

In another embodiment, the invention features a method for modulating the expression of more than one gene within a cell comprising: (a) synthesizing a siNA molecule of the invention, which can be chemically-modified, wherein one of the siNA strands comprises a sequence complementary to RNA of the gene and wherein the sense strand sequence of the siNA comprises a sequence substantially similar to the sequence of the target RNA; and (b) introducing the siNA molecules into a cell under conditions suitable to modulate the expression of the genes in the cell.

In one embodiment, siNA molecules of the invention are used as reagents in ex vivo applications. For example, siNA reagents are intoduced into tissue or cells that are transplanted into a subject for therapeutic effect. The cells and/or tissue can be derived from an organism or subject that later receives the explant, or can be derived from another organism or subject prior to transplantation. The siNA molecules can be used to modulate the expression of one or more genes in the cells or tissue, such that the cells or tissue obtain a desired phenotype or are able to perform a function when transplanted in vivo. In one embodiment, certain target cells from a patient are extracted. These extracted cells are contacted with siNAs targeteing a specific nucleotide sequence within the cells under conditions suitable for uptake of the siNAs by these cells (e.g. using delivery reagents such as cationic lipids, liposomes and the like or using techniques such as electroporation to facilitate the delivery of siNAs into cells). The cells are then reintroduced back into the same patient or other patients. Non-limiting examples of ex vivo applications include use in organ/tissue transplant, tissue grafting, or treatment of pulmonary disease (e.g., restenosis) or prevent neointimal hyperplasia and atherosclerosis in vein grafts. Such ex vivo applications may also used to treat conditions associated with coronary and peripheral bypass graft failure, for example, such methods can be used in conjunction with peripheral vascular bypass graft surgery and coronary artery bypass graft surgery. Additional applications include transplants to treat CNS lesions or injury, including use in treatment of neurodegenerative conditions such as Alzheimer's disease, Parkinson's Disease, Epilepsy, Dementia, Huntington's disease, or amyotrophic lateral sclerosis (ALS).

In one embodiment, the invention features a method of modulating the expression of a gene in a tissue explant comprising: (a) synthesizing a siNA molecule of the invention, which can be chemically-modified, wherein one of the siNA strands comprises a sequence complementary to RNA of the gene; and (b) introducing the siNA molecule into a cell of the tissue explant derived from a particular organism under conditions suitable to modulate the expression of the gene in the tissue explant. In another embodiment, the method further comprises introducing the tissue explant back into the organism the tissue was derived from or into another organism under conditions suitable to modulate the expression of the gene in that organism.

In one embodiment, the invention features a method of modulating the expression of a gene in a tissue explant comprising: (a) synthesizing a siNA molecule of the invention, which can be chemically-modified, wherein one of the siNA strands comprises a sequence complementary to RNA of the gene and wherein the sense strand sequence of the siNA comprises a sequence substantially similar to the sequence of the target RNA; and (b) introducing the siNA molecule into a cell of the tissue explant derived from a particular organism under conditions suitable to modulate the expression of the gene in the tissue explant. In another embodiment, the method further comprises introducing the tissue explant back into the organism the tissue was derived from or into another organism under conditions suitable to modulate the expression of the gene in that organism.

In another embodiment, the invention features a method of modulating the expression of more than one gene in a tissue explant comprising: (a) synthesizing siNA molecules of the invention, which can be chemically-modified, wherein one of the siNA strands comprises a sequence complementary to RNA of the genes; and (b) introducing the siNA molecules into a cell of the tissue explant derived from a particular organism under conditions suitable to modulate the expression of the genes in the tissue explant. In another embodiment, the method further comprises introducing the tissue explant back into the organism the tissue was derived from or into another organism under conditions suitable to modulate the expression of the genes in that organism.

In one embodiment, the invention features a method of modulating the expression of a gene in an organism comprising: (a) synthesizing a siNA molecule of the invention, which can be chemically-modified, wherein one of the siNA strands comprises a sequence complementary to RNA of the gene; and (b) introducing the siNA molecule into the organism under conditions suitable to modulate the expression of the gene in the organism.

In another embodiment, the invention features a method of modulating the expression of more than one gene in an organism comprising: (a) synthesizing siNA molecules of the invention, which can be chemically-modified, wherein one of the siNA strands comprises a sequence complementary to RNA of the genes; and (b) introducing the siNA molecules into the organism under conditions suitable to modulate the expression of the genes in the organism.

In one embodiment, the invention features a method for modulating the expression of a gene within a cell comprising: (a) synthesizing a siNA molecule of the invention, which can be chemically-modified, wherein the siNA comprises a single stranded sequence having complementarity to RNA of the gene; and (b) introducing the siNA molecule into a cell under conditions suitable to modulate the expression of the gene in the cell.

In another embodiment, the invention features a method for modulating the expression of more than one gene within a cell comprising: (a) synthesizing siNA molecules of the invention, which can be chemically-modified, wherein the siNA comprises a single stranded sequence having complementarity to RNA of the gene; and (b) contacting the siNA molecule with a cell in vitro or in vivo under conditions suitable to modulate the expression of the genes in the cell.

In one embodiment, the invention features a method of modulating the expression of a gene in a tissue explant comprising: (a) synthesizing a siNA molecule of the invention, which can be chemically-modified, wherein the siNA comprises a single stranded sequence having complementarity to RNA of the gene; and (b) contacting the siNA molecule with a cell of the tissue explant derived from a particular organism under conditions suitable to modulate the expression of the gene in the tissue explant. In another embodiment, the method further comprises introducing the tissue explant back into the organism the tissue was derived from or into another organism under conditions suitable to modulate the expression of the gene in that organism.

In another embodiment, the invention features a method of modulating the expression of more than one gene in a tissue explant comprising: (a) synthesizing siNA molecules of the invention, which can be chemically-modified, wherein the siNA comprises a single stranded sequence having complementarity to RNA of the gene; and (b) introducing the siNA molecules into a cell of the tissue explant derived from a particular organism under conditions suitable to modulate the expression of the genes in the tissue explant. In another embodiment, the method further comprises introducing the tissue explant back into the organism the tissue was derived from or into another organism under conditions suitable to modulate the expression of the genes in that organism.

In one embodiment, the invention features a method of modulating the expression of a gene in an organism comprising: (a) synthesizing a siNA molecule of the invention, which can be chemically-modified, wherein the siNA comprises a single stranded sequence having complementarity to RNA of the gene; and (b) introducing the siNA molecule into the organism under conditions suitable to modulate the expression of the gene in the organism.

In another embodiment, the invention features a method of modulating the expression of more than one gene in an organism comprising: (a) synthesizing siNA molecules of the invention, which can be chemically-modified, wherein the siNA comprises a single stranded sequence having complementarity to RNA of the gene; and (b) introducing the siNA molecules into the organism under conditions suitable to modulate the expression of the genes in the organism.

In one embodiment, the invention features a method of modulating the expression of a gene in an organism comprising contacting the organism with a siNA molecule of the invention under conditions suitable to modulate the expression of the gene in the organism.

In another embodiment, the invention features a method of modulating the expression of more than one gene in an organism comprising contacting the organism with one or more siNA molecules of the invention under conditions suitable to modulate the expression of the genes in the organism.

The siNA molecules of the invention can be designed to inhibit target gene expression through RNAi targeting of a variety of RNA molecules. In one embodiment, the siNA molecules of the invention are used to target various RNAs corresponding to a target gene. Non-limiting examples of such RNAs include messenger RNA (mRNA), alternate RNA splice variants of target gene(s), post-transcriptionally modified RNA of target gene(s), pre-mRNA of target gene(s), and/or RNA templates. If alternate splicing produces a family of transcripts that are distinguished by usage of appropriate exons, the instant invention can be used to inhibit gene expression through the appropriate exons to specifically inhibit or to distinguish among the functions of gene family members. For example, a protein that contains an alternatively spliced transmembrane domain can be expressed in both membrane bound and secreted forms. Use of the invention to target the exon containing the transmembrane domain can be used to determine the functional consequences of pharmaceutical targeting of membrane bound as opposed to the secreted form of the protein. Non-limiting examples of applications of the invention relating to targeting these RNA molecules include therapeutic pharmaceutical applications, pharmaceutical discovery applications, molecular diagnostic and gene function applications, and gene mapping, for example using single nucleotide polymorphism mapping with siNA molecules of the invention. Such applications can be implemented using known gene sequences or from partial sequences available from an expressed sequence tag (EST).

In another embodiment, the siNA molecules of the invention are used to target conserved sequences corresponding to a gene family or gene families. As such, siNA molecules targeting multiple gene targets can provide increased therapeutic effect. In addition, siNA can be used to characterize pathways of gene function in a variety of applications. For example, the present invention can be used to inhibit the activity of target gene(s) in a pathway to determine the function of uncharacterized gene(s) in gene function analysis, mRNA function analysis, or translational analysis. The invention can be used to determine potential target gene pathways involved in various diseases and conditions toward pharmaceutical development. The invention can be used to understand pathways of gene expression involved in, for example, in development, such as prenatal development and postnatal development, and/or the progression and/or maintenance of cancer, infectious disease, autoimmunity, inflammation, endocrine disorders, renal disease, pulmonary disease, cardiovascular disease, birth defects, ageing, any other disease or condition related to gene expression.

In one embodiment, the invention features a method comprising: (a) generating a library of siNA constructs having a predetermined complexity; and (b) assaying the siNA constructs of (a) above, under conditions suitable to determine RNAi target sites within the target RNA sequence. In another embodiment, the siNA molecules of (a) have strands of a fixed length, for example, about 23 nucleotides in length. In yet another embodiment, the siNA molecules of (a) are of differing length, for example having strands of about 19 to about 25 (*e*.*g*., about 19, 20, 21, 22, 23, 24, or 25) nucleotides in length. In one embodiment, the assay can comprise a reconstituted *in vitro* siNA assay as described herein. In another embodiment, the assay can comprise a cell culture system in which target RNA is expressed. In another embodiment, fragments of target RNA are analyzed for detectable levels of cleavage, for example by gel electrophoresis, northern blot analysis, or RNAse protection assays, to determine the most suitable target site(s) within the target RNA sequence. The target RNA sequence can be obtained as is known in the art, for example, by cloning and/or transcription for *in vitro* systems, and by cellular expression in *in vivo* systems.

In one embodiment, the invention features a method comprising: (a) generating a randomized library of siNA constructs having a predetermined complexity, such as of 4^{N}, where N represents the number of base paired nucleotides in each of the siNA construct strands (*eg.* for a siNA construct having 21 nucleotide sense and antisense strands with 19 base pairs, the complexity would be 4¹⁹); and (b) assaying the siNA constructs of (a) above, under conditions suitable to determine RNAi target sites within the target RNA sequence. In another embodiment, the siNA molecules of (a) have strands of a fixed length, for example about 23 nucleotides in length. In yet another embodiment, the siNA molecules of (a) are of differing length, for example having strands of about 19 to about 25 (*e.g*., about 19, 20, 21, 22, 23, 24, or 25) nucleotides in length. In one embodiment, the assay can comprise a reconstituted *in vitro* siNA assay as described in Example 7 herein. In another embodiment, the assay can comprise a cell culture system in which target RNA is expressed. In another embodiment, fragments of target RNA are analyzed for detectable levels of cleavage, for example by gel electrophoresis, northern blot analysis, or RNAse protection assays, to determine the most suitable target site(s) within the target RNA sequence. In another embodiment, the target RNA sequence can be obtained as is known in the art, for example, by cloning and/or transcription for *in vitro* systems, and by cellular expression in *in vivo* systems.

In another embodiment, the invention features a method comprising: (a) analyzing the sequence of a RNA target encoded by a target gene; (b) synthesizing one or more sets of siNA molecules having sequence complementary to one or more regions of the RNA of (a); and (c) assaying the siNA molecules of (b) under conditions suitable to determine RNAi targets within the target RNA sequence. In one embodiment, the siNA molecules of (b) have strands of a fixed length, for example about 23 nucleotides in length. In another embodiment, the siNA molecules of (b) are of differing length, for example having strands of about 19 to about 25 (*e*.*g*., about 19, 20, 21, 22, 23, 24, or 25) nucleotides in length. In one embodiment, the assay can comprise a reconstituted *in vitro* siNA assay as described herein. In another embodiment, the assay can comprise a cell culture system in which target RNA is expressed. Fragments of target RNA are analyzed for detectable levels of cleavage, for example by gel electrophoresis, northern blot analysis, or RNAse protection assays, to determine the most suitable target site(s) within the target RNA sequence. The target RNA sequence can be obtained as is known in the art, for example, by cloning and/or transcription for *in vitro* systems, and by expression in *in vivo* systems.

By "target site" is meant a sequence within a target RNA that is "targeted" for cleavage mediated by a siNA construct which contains sequences within its antisense region that are complementary to the target sequence.

By "detectable level of cleavage" is meant cleavage of target RNA (and formation of cleaved product RNAs) to an extent sufficient to discern cleavage products above the background of RNAs produced by random degradation of the target RNA. Production of cleavage products from 1-5% of the target RNA is sufficient to detect above the background for most methods of detection.

In one embodiment, the invention features a composition comprising a siNA molecule of the invention, which can be chemically-modified, in a pharmaceutically acceptable carrier or diluent. In another embodiment, the invention features a pharmaceutical composition comprising siNA molecules of the invention, which can be chemically-modified, targeting one or more genes in a pharmaceutically acceptable carrier or diluent. In another embodiment, the invention features a method for treating or preventing a disease or condition in a subject, comprising administering to the subject a composition of the invention under conditions suitable for the treatment or prevention of the disease or condition in the subject, alone or in conjunction with one or more other therapeutic compounds. In yet another embodiment, the invention features a method for reducing or preventing tissue rejection in a subject comprising administering to the subject a composition of the invention under conditions suitable for the reduction or prevention of tissue rejection in the subject.

In another embodiment, the invention features a method for validating a gene target, comprising: (a) synthesizing a siNA molecule of the invention, which can be chemically-modified, wherein one of the siNA strands includes a sequence complementary to RNA of a target gene; (b) introducing the siNA molecule into a cell, tissue, or organism under conditions suitable for modulating expression of the target gene in the cell, tissue, or organism; and (c) determining the function of the gene by assaying for any phenotypic change in the cell, tissue, or organism.

In another embodiment, the invention features a method for validating a target gene comprising: (a) synthesizing a siNA molecule of the invention, which can be chemically-modified, wherein one of the siNA strands includes a sequence complementary to RNA of a target gene; (b) introducing the siNA molecule into a biological system under conditions suitable for modulating expression of the target gene in the biological system; and (c) determining the function of the gene by assaying for any phenotypic change in the biological system.

By "biological system" is meant, material, in a purified or unpurified form, from biological sources, including but not limited to human, animal, plant, insect, bacterial, viral or other sources, wherein the system comprises the components required for RNAi acitivity. The term "biological system" includes, for example, a cell, tissue, or organism, or extract thereof. The term biological system also includes reconstituted RNAi systems that can be used in an *in vitro* setting.

By "phenotypic change" is meant any detectable change to a cell that occurs in response to contact or treatment with a nucleic acid molecule of the invention (e.g., siNA). Such detectable changes include, but are not limited to, changes in shape, size, proliferation, motility, protein expression or RNA expression or other physical or chemical changes as can be assayed by methods known in the art. The detectable change can also include expression of reporter genes/molecules such as Green Florescent Protein (GFP) or various tags that are used to identify an expressed protein or any other cellular component that can be assayed.

In one embodiment, the invention features a kit containing a siNA molecule of the invention, which can be chemically-modified, that can be used to modulate the expression of a target gene in a cell, tissue, or organism. In another embodiment, the invention features a kit containing more than one siNA molecule of the invention, which can be chemically-modified, that can be used to modulate the expression of more than one target gene in a cell, tissue, or organism.

In one embodiment, the invention features a kit containing a siNA molecule of the invention, which can be chemically-modified, that can be used to modulate the expression of a target gene in a biological system. In another embodiment, the invention features a kit containing more than one siNA molecule of the invention, which can be chemically-modified, that can be used to modulate the expression of more than one target gene in a biological system.

In one embodiment, the invention features a cell containing one or more siNA molecules of the invention, which can be chemically-modified. In another embodiment, the cell containing a siNA molecule of the invention is a mammalian cell. In yet another embodiment, the cell containing a siNA molecule of the invention is a human cell.

In one embodiment, the synthesis of a siNA molecule of the invention, which can be chemically-modified, comprises: (a) synthesis of two complementary strands of the siNA molecule; (b) annealing the two complementary strands together under conditions suitable to obtain a double-stranded siNA molecule. In another embodiment, synthesis of the two complementary strands of the siNA molecule is by solid phase oligonucleotide synthesis. In yet another embodiment, synthesis of the two complementary strands of the siNA molecule is by solid phase tandem oligonucleotide synthesis.

In one embodiment, the invention features a method for synthesizing a siNA duplex molecule comprising: (a) synthesizing a first oligonucleotide sequence strand of the siNA molecule, wherein the first oligonucleotide sequence strand comprises a cleavable linker molecule that can be used as a scaffold for the synthesis of the second oligonucleotide sequence strand of the siNA; (b) synthesizing the second oligonucleotide sequence strand of siNA on the scaffold of the first oligonucleotide sequence strand, wherein the second oligonucleotide sequence strand further comprises a chemical moiety than can be used to purify the siNA duplex; (c) cleaving the linker molecule of (a) under conditions suitable for the two siNA oligonucleotide strands to hybridize and form a stable duplex; and (d) purifying the siNA duplex utilizing the chemical moiety of the second oligonucleotide sequence strand. In one embodiment, cleavage of the linker molecule in (c) above takes place during deprotection of the oligonucleotide, for example under hydrolysis conditions using an alkylamine base such as methylamine. In one embodiment, the method of synthesis comprises solid phase synthesis on a solid support such as controlled pore glass (CPG) or polystyrene, wherein the first sequence of (a) is synthesized on a cleavable linker, such as a succinyl linker, using the solid support as a scaffold. The cleavable linker in (a) used as a scaffold for synthesizing the second strand can comprise similar reactivity as the solid support derivatized linker, such that cleavage of the solid support derivatized linker and the cleavable linker of (a) takes place concomitantly. In another embodiment, the chemical moiety of (b) that can be used to isolate the attached oligonucleotide sequence comprises a trityl group, for example a dimethoxytrityl group, which can be employed in a trityl-on synthesis strategy as described herein. In yet another embodiment, the chemical moiety, such as a dimethoxytrityl group, is removed during purification, for example, using acidic conditions.

In a further embodiment, the method for siNA synthesis is a solution phase synthesis or hybrid phase synthesis wherein both strands of the siNA duplex are synthesized in tandem using a cleavable linker attached to the first sequence which acts a scaffold for synthesis of the second sequence. Cleavage of the linker under conditions suitable for hybridization of the separate siNA sequence strands results in formation of the double-stranded siNA molecule.

In another embodiment, the invention features a method for synthesizing a siNA duplex molecule comprising: (a) synthesizing one oligonucleotide sequence strand of the siNA molecule, wherein the sequence comprises a cleavable linker molecule that can be used as a scaffold for the synthesis of another oligonucleotide sequence; (b) synthesizing a second oligonucleotide sequence having complementarity to the first sequence strand on the scaffold of (a), wherein the second sequence comprises the other strand of the double-stranded siNA molecule and wherein the second sequence further comprises a chemical moiety than can be used to isolate the attached oligonucleotide sequence; (c) purifying the product of (b) utilizing the chemical moiety of the second oligonucleotide sequence strand under conditions suitable for isolating the full-length sequence comprising both siNA oligonucleotide strands connected by the cleavable linker and under conditions suitable for the two siNA oligonucleotide strands to hybridize and form a stable duplex. In one embodiment, cleavage of the linker molecule in (c) above takes place during deprotection of the oligonucleotide, for example under hydrolysis conditions. In another embodiment, cleavage of the linker molecule in (c) above takes place after deprotection of the oligonucleotide. In another embodiment, the method of synthesis comprises solid phase synthesis on a solid support such as controlled pore glass (CPG) or polystyrene, wherein the first sequence of (a) is synthesized on a cleavable linker, such as a succinyl linker, using the solid support as a scaffold. The cleavable linker in (a) used as a scaffold for synthesizing the second strand can comprise similar reactivity or differing reactivity as the solid support derivatized linker, such that cleavage of the solid support derivatized linker and the cleavable linker of (a) takes place either concomitantly or sequentially. In one embodiment, the chemical moiety of (b) that can be used to isolate the attached oligonucleotide sequence comprises a trityl group, for example a dimethoxytrityl group.

In another embodiment, the invention features a method for making a double-stranded siNA molecule in a single synthetic process comprising: (a) synthesizing an oligonucleotide having a first and a second sequence, wherein the first sequence is complementary to the second sequence, and the first oligonucleotide sequence is linked to the second sequence via a cleavable linker, and wherein a terminal 5'-protecting group, for example, a 5'-O-dimethoxytrityl group (5'-O-DMT) remains on the oligonucleotide having the second sequence; (b) deprotecting the oligonucleotide whereby the deprotection results in the cleavage of the linker joining the two oligonucleotide sequences; and (c) purifying the product of (b) under conditions suitable for isolating the double-stranded siNA molecule, for example using a trityl-on synthesis strategy as described herein.

In another embodiment, the method of synthesis of siNA molecules of the invention comprises the teachings of Scaringe et al., US Patent Nos. 5,889,136; 6,008,400; and 6,111,086, incorporated by reference herein in their entirety.

In one embodiment, the invention features siNA constructs that mediate RNAi in a cell or reconstituted system, wherein the siNA construct comprises one or more chemical modifications, for example, one or more chemical modifications having any of Formulae I-VII or any combination thereof that increases the nuclease resistance of the siNA construct.

In another embodiment, the invention features a method for generating siNA molecules with increased nuclease resistance comprising (a) introducing nucleotides having any of Formula I-VII or any combination thereof into a siNA molecule, and (b) assaying the siNA molecule of step (a) under conditions suitable for isolating siNA molecules having increased nuclease resistance.

In one embodiment, the invention features siNA constructs that mediate RNAi against a target gene, wherein the siNA construct comprises one or more chemical modifications described herein that modulates the binding affinity between the sense and antisense strands of the siNA construct.

In another embodiment, the invention features a method for generating siNA molecules with increased binding affinity between the sense and antisense strands of the siNA molecule comprising (a) introducing nucleotides having any of Formula I-VII or any combination thereof into a siNA molecule, and (b) assaying the siNA molecule of step (a) under conditions suitable for isolating siNA molecules having increased binding affinity between the sense and antisense strands of the siNA molecule.

In one embodiment, the invention features siNA constructs that mediate RNAi in a cell or reconstituted system, wherein the siNA construct comprises one or more chemical modifications described herein that modulates the binding affinity between the antisense strand of the siNA construct and a complementary target RNA sequence within a cell.

In one embodiment, the invention features siNA constructs that mediate RNAi in a cell or reconstituted system, wherein the siNA construct comprises one or more chemical modifications described herein that modulates the binding affinity between the antisense strand of the siNA construct and a complementary target DNA sequence within a cell.

In another embodiment, the invention features a method for generating siNA molecules with increased binding affinity between the antisense strand of the siNA molecule and a complementary target RNA sequence comprising (a) introducing nucleotides having any of Formula I-VII or any combination thereof into a siNA molecule, and (b) assaying the siNA molecule of step (a) under conditions suitable for isolating siNA molecules having increased binding affinity between the antisense strand of the siNA molecule and a complementary target RNA sequence.

In another embodiment, the invention features a method for generating siNA molecules with increased binding affinity between the antisense strand of the siNA molecule and a complementary target DNA sequence comprising (a) introducing nucleotides having any of Formula I-VII or any combination thereof into a siNA molecule, and (b) assaying the siNA molecule of step (a) under conditions suitable for isolating siNA molecules having increased binding affinity between the antisense strand of the siNA molecule and a complementary target DNA sequence.

In one embodiment, the invention features siNA constructs that mediate RNAi in a cell or reconstituted system, wherein the siNA construct comprises one or more chemical modifications described herein that modulate the polymerase activity of a cellular polymerase capable of generating additional endogenous siNA molecules having sequence homology to the chemically-modified siNA construct.

In another embodiment, the invention features a method for generating siNA molecules capable of mediating increased polymerase activity of a cellular polymerase capable of generating additional endogenous siNA molecules having sequence homology to a chemically-modified siNA molecule comprising (a) introducing nucleotides having any of Formula I-VII or any combination thereof into a siNA molecule, and (b) assaying the siNA molecule of step (a) under conditions suitable for isolating siNA molecules capable of mediating increased polymerase activity of a cellular polymerase capable of generating additional endogenous siNA molecules having sequence homology to the chemically-modified siNA molecule.

In one embodiment, the invention features chemically-modified siNA constructs that mediate RNAi in a cell or reconstituted system, wherein the chemical modifications do not significantly effect the interaction of siNA with a target RNA molecule, DNA molecule and/or proteins or other factors that are essential for RNAi in a manner that would decrease the efficacy of RNAi mediated by such siNA constructs.

In another embodiment, the invention features a method for generating siNA molecules with improved RNAi activity, comprising (a) introducing nucleotides having any of Formula I-VII or any combination thereof into a siNA molecule, and (b) assaying the siNA molecule of step (a) under conditions suitable for isolating siNA molecules having improved RNAi activity.

In yet another embodiment, the invention features a method for generating siNA molecules with improved RNAi activity against a target RNA comprising (a) introducing nucleotides having any of Formula I-VII or any combination thereof into a siNA molecule, and (b) assaying the siNA molecule of step (a) under conditions suitable for isolating siNA molecules having improved RNAi activity against the target RNA.

In yet another embodiment, the invention features a method for generating siNA molecules with improved RNAi activity against a DNA target comprising (a) introducing nucleotides having any of Formula I-VII or any combination thereof into a siNA molecule, and (b) assaying the siNA molecule of step (a) under conditions suitable for isolating siNA molecules having improved RNAi activity against the DNA target, such as a gene, chromosome, or portion thereof.

In one embodiment, the invention features siNA constructs that mediate RNAi in a cell or reconstituted system, wherein the siNA construct comprises one or more chemical modifications described herein that modulates the cellular uptake of the siNA construct.

In another embodiment, the invention features a method for generating siNA molecules against a target gene with improved cellular uptake comprising (a) introducing nucleotides having any of Formula I-VII or any combination thereof into a siNA molecule, and (b) assaying the siNA molecule of step (a) under conditions suitable for isolating siNA molecules having improved cellular uptake.

In one embodiment, the invention features siNA constructs that mediate RNAi against a target gene, wherein the siNA construct comprises one or more chemical modifications described herein that increases the bioavailability of the siNA construct, for example, by attaching polymeric conjugates such as polyethyleneglycol or equivalent conjugates that improve the pharmacokinetics of the siNA construct, or by attaching conjugates that target specific tissue types or cell types *in vivo.* Non-limiting examples of such conjugates are described in Vargeese et al., U.S. Serial No. 10/201,394 incorporated by reference herein.

In one embodiment, the invention features a method for generating siNA molecules of the invention with improved bioavailability, comprising (a) introducing a conjugate into the structure of a siNA molecule, and (b) assaying the siNA molecule of step (a) under conditions suitable for isolating siNA molecules having improved bioavailability. Such conjugates can include ligands for cellular receptors, such as peptides derived from naturally occurring protein ligands; protein localization sequences, including cellular ZIP code sequences; antibodies; nucleic acid aptamers; vitamins and other co-factors, such as folate and N-acetylgalactosamine; polymers, such as polyethyleneglycol (PEG); phospholipids; polyamines, such as spermine or spermidine; and others.

In another embodiment, the invention features a method for generating siNA molecules of the invention with improved bioavailability comprising (a) introducing an excipient formulation to a siNA molecule, and (b) assaying the siNA molecule of step (a) under conditions suitable for isolating siNA molecules having improved bioavailability. Such excipients include polymers such as cyclodextrins, lipids, cationic lipids, polyamines, phospholipids, and others.

In another embodiment, the invention features a method for generating siNA molecules of the invention with improved bioavailability comprising (a) introducing nucleotides having any of Formulae I-VII or any combination thereof into a siNA molecule, and (b) assaying the siNA molecule of step (a) under conditions suitable for isolating siNA molecules having improved bioavailability.

In another embodiment, polyethylene glycol (PEG) can be covalently attached to siNA compounds of the present invention. The attached PEG can be any molecular weight, preferably from about 2,000 to about 50,000 daltons (Da).

The present invention can be used alone or as a component of a kit having at least one of the reagents necessary to carry out the *in vitro* or *in vivo* introduction of RNA to test samples and/or subjects. For example, preferred components of the kit include a siNA molecule of the invention and a vehicle that promotes introduction of the siNA into cells of interest as described herein (e.g., using lipids and other methods of transfection known in the art, see for example Beigelman et al, US 6,395,713). The kit can be used for target validation, such as in determining gene function and/or activity, or in drug optimization, and in drug discovery (see for example Usman et al., USSN 60/402,996). Such a kit can also include instructions to allow a user of the kit to practice the invention.

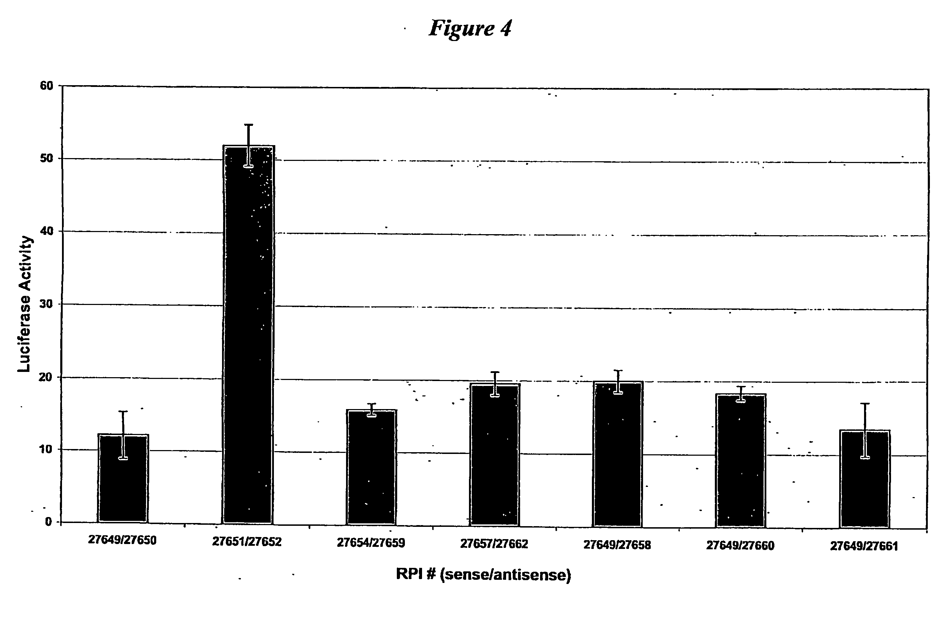

The term "short interfering nucleic acid", "siNA", "short interfering RNA", "siRNA", "short interfering nucleic acid molecule", "short interfering oligonucleotide molecule", or "chemically-modified short interfering nucleic acid molecule" as used herein refers to any nucleic acid molecule capable of inhibiting or down regulating gene expression or viral replication, for example by mediating RNA interference "RNAi" or gene silencing in a sequence-specific manner; see for example Bass, 2001, Nature, 411, 428-429; Elbashir et al., 2001, Nature, 411, 494-498; and Kreutzer et al., International PCT Publication No. WO 00/44895; Zernicka-Goetz et al., International PCT Publication No. WO 01/36646; Fire, International PCT Publication No. WO 99/32619; Plaetinck et al., International PCT Publication No. WO 00/01846; Mello and Fire, International PCT Publication No. WO 01/29058; Deschamps-Depaillette, International PCT Publication No. WO 99/07409; and Li et al., International PCT Publication No. WO 00/44914; Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237; Hutvagner and Zamore, 2002, Science, 297, 2056-60; McManus et al., 2002, RNA, 8, 842-850; Reinhart et al., 2002, Gene & Dev., 16, 1616-1626; and Reinhart & Bartel, 2002, Science, 297,1831). Non limiting examples of siNA molecules of the invention are shown in **Figures 4-6,** and **Tables II, III, and IV** herein. For example the siNA can be a double-stranded polynucleotide molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. The siNA can be assembled from two separate oligonucleotides, where one strand is the sense strand and the other is the antisense strand, wherein the antisense and sense strands are self-complementary (i.e. each strand comprises nucleotide sequence that is complementary to nucleotide sequence in the other strand; such as where the antisense strand and sense strand form a duplex or double stranded structure, for example wherein the double stranded region is about 19 base pairs); the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof. Alternatively, the siNA is assembled from a single oligonucleotide, where the self-complementary sense and antisense regions of the siNA are linked by means of a nucleic acid based or non-nucleic acid-based linker(s). The siNA can be a polynucleotide with a hairpin secondary structure, having self-complementary sense and antisense regions, wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof. The siNA can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either *in vivo* or *in vitro* to generate an active siNA molecule capable of mediating RNAi. The siNA can also comprise a single stranded polynucleotide having nucleotide sequence complementary to nucleotide sequence in a target nucleic acid molecule or a portion thereof (for example, where such siNA molecule does not require the presence within the siNA molecule of nucleotide sequence corresponding to the target nucleic acid sequence or a portion thereof), wherein the single stranded polynucleotide can further comprise a terminal phosphate group, such as a 5'-phosphate (see for example Martinez et al., 2002, Cell., 110, 563-574 and Schwarz et al., 2002, Molecular Cell, 10, 537-568), or 5',3'-diphosphate. In certain embodiment, the siNA molecule of the invention comprises separate sense and antisense sequences or regions, wherein the sense and antisense regions are covalently linked by nucleotide or non-nucleotide linkers molecules as is known in the art, or are alternately non-covalently linked by ionic interactions, hydrogen bonding, van der waals interactions, hydrophobic intercations, and/or stacking interactions. In certain embodiments, the siNA molecules of the invention comprise nucleotide sequence that is complementary to nucleotide sequence of a target gene. In another embodiment, the siNA molecule of the invention interacts with nucleotide sequence of a target gene in a manner that causes inhibition of expression of the target gene. As used herein, siNA molecules need not be limited to those molecules containing only RNA, but further encompasses chemically-modified nucleotides and non-nucleotides. In certain embodiments, the short interfering nucleic acid molecules of the invention lack 2'-hydroxy (2'-OH) containing nucleotides. Applicant describes in certain embodiments short interfering nucleic acids that do not require the presence of nucleotides having a 2'-hydroxy group for mediating RNAi and as such, short interfering nucleic acid molecules of the invention optionally do not include any ribonucleotides (e.g., nucleotides having a 2'-OH group). Such siNA molecules that do not require the presence of ribonucleotides within the siNA molecule to support RNAi can however have an attached linker or linkers or other attached or associated groups, moieties, or chains containing one or more nucleotides with 2'-OH groups. Optionally, siNA molecules can comprise ribonucleotides at about 5, 10, 20, 30, 40, or 50% of the nucleotide positions. The modified short interfering nucleic acid molecules of the invention can also be referred to as short interfering modified oligonucleotides "siMON." As used herein, the term siNA is meant to be equivalent to other terms used to describe nucleic acid molecules that are capable of mediating sequence specific RNAi, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically-modified siRNA, post-transcriptional gene silencing RNA (ptgsRNA), and others. In addition, as used herein, the term RNAi is meant to be equivalent to other terms used to describe sequence specific RNA interference, such as post transcriptional gene silencing, or epigenetics. For example, siNA molecules of the invention can be used to epigenetically silence genes at both the post-transcriptional level or the pre-transcriptional level. In a non-limiting example, epigenetic regulation of gene expression by siNA molecules of the invention can result from siNA mediated modification of chromatin structure to alter gene expression (see, for example, Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237).

By "modulate" is meant that the expression of the gene, or level of RNA molecule or equivalent RNA molecules encoding one or more proteins or protein subunits, or activity of one or more proteins or protein subunits is up regulated or down regulated, such that expression, level, or activity is greater than or less than that observed in the absence of the modulator. For example, the term "modulate" can mean "inhibit," but the use of the word "modulate" is not limited to this definition.

By "inhibit" it is meant that the activity of a gene expression product or level of RNAs or equivalent RNAs encoding one or more gene products is reduced below that observed in the absence of the nucleic acid molecule of the invention. In one embodiment, inhibition with a siNA molecule preferably is below that level observed in the presence of an inactive or attenuated molecule that is unable to mediate an RNAi response. In another embodiment, inhibition of gene expression with the siNA molecule of the instant invention is greater in the presence of the siNA molecule than in its absence.

By "inhibit", "down-regulate", or "reduce", it is meant that the expression of the gene, or level of RNA molecules or equivalent RNA molecules encoding one or more proteins or protein subunits, or activity of one or more proteins or protein subunits, is reduced below that observed in the absence of the nucleic acid molecules (e.g., siNA) of the invention. In one embodiment, inhibition, down-regulation or reduction with an siNA molecule is below that level observed in the presence of an inactive or attenuated molecule. In another embodiment, inhibition, down-regulation, or reduction with siNA molecules is below that level observed in the presence of, for example, an siNA molecule with scrambled sequence or with mismatches. In another embodiment, inhibition, down-regulation, or reduction of gene expression with a nucleic acid molecule of the instant invention is greater in the presence of the nucleic acid molecule than in its absence.

By "gene" or "target gene" is meant, a nucleic acid that encodes an RNA, for example, nucleic acid sequences including, but not limited to, structural genes encoding a polypeptide. The target gene can be a gene derived from a cell, an endogenous gene, a transgene, or exogenous genes such as genes of a pathogen, for example a virus, which is present in the cell after infection thereof. The cell containing the target gene can be derived from or contained in any organism, for example a plant, animal, protozoan, virus, bacterium, or fungus. Non-limiting examples of plants include monocots, dicots, or gymnosperms. Non-limiting examples of animals include vertebrates or invertebrates. Non-limiting examples of fungi include molds or yeasts.

By "endogenous" or "cellular" gene is meant a gene normally found in a cell in its natural location in the genome. For example, HER-2, VEGF, VEGF-R, EGFR, BCL-2, c-MYC, RAS and the like would be considered an endogenous gene. Genes expressed in a cell from a plasmid, viral vector or other vectors or from virus, bacteria, fungi would be considered "foreign" or "heterologous" gene; such genes are not normally found in the host cell, but are introduced by standard gene transfer techniques or as a result of infection by a virus, bacterial or other infectious agent.

By "gene family" is meant a group of more than one nucleic acid molecules that share at least one common characteristic, such as sequence homology, target specificity, mode of action, secondary structure, or the ability to modulate a process or more than one process in a biological system. The gene family can be of viral or cellular origin. The gene family can encode, for example, groups of cytokines, receptors, growth factors, adapter proteins, structural proteins, and other protein epitopes.

By "protein family" is meant a group of more than one proteins, peptides, or polypeptides that share at least one common characteristic, such as sequence homology, target specificity, mode of action, secondary structure, or the ability to modulate a process or more than one process in a biological system. The protein family can be of viral or cellular origin. The protein family can encode, for example, groups of cytokines, receptors, growth factors, adapter proteins, structural proteins, and other protein epitopes.

By "highly conserved sequence region" is meant, a nucleotide sequence of one or more regions in a target gene does not vary significantly from one generation to the other or from one biological system to the other.

By "cancer" is meant a group of diseases characterized by uncontrolled growth and spread of abnormal cells.

By "sense region" is meant a nucleotide sequence of a siNA molecule having complementarity to an antisense region of the siNA molecule. In addition, the sense region of a siNA molecule can comprise a nucleic acid sequence having homology with a target nucleic acid sequence.

By "antisense region" is meant a nucleotide sequence of a siNA molecule having complementarity to a target nucleic acid sequence. In addition, the antisense region of a siNA molecule can optionally comprise a nucleic acid sequence having complementarity to a sense region of the siNA molecule.

By "target nucleic acid" is meant any nucleic acid sequence whose expression or activity is to be modulated. The target nucleic acid can be DNA or RNA.

By "complementarity" is meant that a nucleic acid can form hydrogen bond(s) with another nucleic acid sequence by either traditional Watson-Crick or other non-traditional types. In reference to the nucleic molecules of the present invention, the binding free energy for a nucleic acid molecule with its complementary sequence is sufficient to allow the relevant function of the nucleic acid to proceed, e.g., RNAi activity. Determination of binding free energies for nucleic acid molecules is well known in the art (see, e.g., Turner et al., 1987, CSH Symp. Quant. Biol. LII pp.123-133; Frier et al., 1986, Proc. Nat. Acad. Sci. USA 83:9373-9377; Turner et al., 1987, J. Am. Chem. Soc. 109:3783-3785). A percent complementarity indicates the percentage of contiguous residues in a nucleic acid molecule that can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence.

The siNA molecules of the invention represent a novel therapeutic approach to a broad spectrum of diseases and conditions, including cancer or cancerous disease, infectious disease, cardiovascular disease, neurological disease, prion disease, inflammatory disease, autoimmune disease, pulmonary disease, renal disease, liver disease, mitochondrial disease, endocrine disease, reproduction related diseases and conditions, and any other indications that can respond to the level of an expressed gene product in a cell or organsim.

In one embodiment of the present invention, each sequence of a siNA molecule of the invention is independently about 18 to about 24 nucleotides in length, in specific embodiments about 18, 19, 20, 21, 22, 23, or 24 nucleotides in length. In another embodiment, the siNA duplexes of the invention independently comprise about 17 to about 23 base pairs (*e.g.,* about 17, 18, 19, 20, 21, 22 or 23). In yet another embodiment, siNA molecules of the invention comprising hairpin or circular structures are about 35 to about 55 (e.g., about 35, 40, 45, 50 or 55) nucleotides in length, or about 38 to about 44 (*e.g.*, 38, 39, 40, 41, 42, 43 or 44) nucleotides in length and comprising about 16 to about 22 (*e.g.,* about 16, 17, 18, 19, 20, 21 or 22) base pairs. Exemplary siNA molecules of the invention are shown in **Table II.** Exemplary synthetic siNA molecules of the invention are shown in **Table I** and/or **Figures 18-19**.

As used herein "cell" is used in its usual biological sense, and does not refer to an entire multicellular organism, e.g., specifically does not refer to a human. The cell can be present in an organism, e.g., birds, plants and mammals such as humans, cows, sheep, apes, monkeys, swine, dogs, and cats. The cell can be prokaryotic or eukaryotic (e.g., mammalian or plant cell). The cell can be of somatic or germ line origin, totipotent or pluripotent, dividing or non-dividing. The cell can also be derived from or can comprise a gamete or embryo, a stem cell, or a fully differentiated cell.

The siNA molecules of the invention are added directly, or can be complexed with cationic lipids, packaged within liposomes, or otherwise delivered to target cells or tissues. The nucleic acid or nucleic acid complexes can be locally administered to relevant tissues *ex vivo*, or *in vivo* through injection, infusion pump or stent, with or without their incorporation in biopolymers. In particular embodiments, the nucleic acid molecules of the invention comprise sequences shown in **Tables I-II** and/or **Figures 18-19.** Examples of such nucleic acid molecules consist essentially of sequences defined in these tables and figures. Furthermore, the chemically modified constructs described in **Table IV** can be applied to any siNA sequence of the invention.

In another aspect, the invention provides mammalian cells containing one or more siNA molecules of this invention. The one or more siNA molecules can independently be targeted to the same or different sites.

By "RNA" is meant a molecule comprising at least one ribonucleotide residue. By "ribonucleotide" is meant a nucleotide with a hydroxyl group at the 2' position of a β-D-ribo-furanose moiety. The terms include double-stranded RNA, single-stranded RNA, isolated RNA such as partially purified RNA, essentially pure RNA, synthetic RNA, recombinantly produced RNA, as well as altered RNA that differs from naturally occurring RNA by the addition, deletion, substitution and/or alteration of one or more nucleotides. Such alterations can include addition of non-nucleotide material, such as to the end(s) of the siNA or internally, for example at one or more nucleotides of the RNA. Nucleotides in the RNA molecules of the instant invention can also comprise non-standard nucleotides, such as non-naturally occurring nucleotides or chemically synthesized nucleotides or deoxynucleotides. These altered RNAs can be referred to as analogs or analogs of naturally-occurring RNA.

By "subject" is meant an organism, which is a donor or recipient of explanted cells or the cells themselves. "Subject" also refers to an organism to which the nucleic acid molecules of the invention can be administered. In one embodiment, a subject is a mammal or mammalian cells. In another embodiment, a subject is a human or human cells.

The term "phosphorothioate" as used herein refers to an internucleotide linkage having Formula I, wherein Z and/or W comprise a sulfur atom. Hence, the term phosphorothioate refers to both phosphorothioate and phosphorodithioate internucleotide linkages.

The term "universal base" as used herein refers to nucleotide base analogs that form base pairs with each of the natural DNA/RNA bases with little discrimination between them. Non-limiting examples of universal bases include C-phenyl, C-naphthyl and other aromatic derivatives, inosine, azole carboxamides, and nitroazole derivatives such as 3-nitropyrrole, 4-nitroindole, 5-nitroindole, and 6-nitroindole as known in the art (see for example Loakes, 2001, Nucleic Acids Research, 29, 2437-2447).

The term "acyclic nucleotide" as used herein refers to any nucleotide having an acyclic ribose sugar, for example where any of the ribose carbons (C1, C2, C3, C4, or C5), are independently or in combination absent from the nucleotide.

The nucleic acid molecules of the instant invention, individually, or in combination or in conjunction with other drugs, can be used to treat diseases or conditions discussed herein. For example, to treat a particular disease or condition, the siNA molecules can be administered to a subject or can be administered to other appropriate cells evident to those skilled in the art, individually or in combination with one or more drugs under conditions suitable for the treatment.

In a further embodiment, the siNA molecules can be used in combination with other known treatments to treat conditions or diseases discussed above. For example, the described molecules could be used in combination with one or more known therapeutic agents to treat a disease or condition. Non-limiting examples of other therapeutic agents that can be readily combined with a siNA molecule of the invention are enzymatic nucleic acid molecules, allosteric nucleic acid molecules, antisense, decoy, or aptamer nucleic acid molecules, antibodies such as monoclonal antibodies, small molecules, and other organic and/or inorganic compounds including metals, salts and ions.

In one embodiment, the invention features an expression vector comprising a nucleic acid sequence encoding at least one siNA molecule of the invention, in a manner which allows expression of the siNA molecule. For example, the vector can contain sequence(s) encoding both strands of a siNA molecule comprising a duplex. The vector can also contain sequence(s) encoding a single nucleic acid molecule that is self-complementary and thus forms a siNA molecule. Non-limiting examples of such expression vectors are described in Paul et al., 2002, Nature Biotechnology, 19, 505; Miyagishi and Taira, 2002, Nature Biotechnology, 19, 497; Lee et al., 2002, Nature Biotechnology, 19, 500; and Novina et al., 2002, Nature Medicine, advance online publication doi:10.1038/nm725.

In another embodiment, the invention features a mammalian cell, for example, a human cell, including an expression vector of the invention.

In yet another embodiment, the expression vector of the invention comprises a sequence for a siRNA molecule having complementarity to a RNA molecule referred to by a Genbank Accession number in Table III.

In yet another embodiment, the expression vector of the invention comprises a sequence for a siNA molecule having complementarity to a RNA molecule referred to by a Genbank Accession numbers, for example Genbank Accession Nos. shown in **Table I.**

In one embodiment, an expression vector of the invention comprises a nucleic acid sequence encoding two or more siNA molecules, which can be the same or different.

In another aspect of the invention, siRNA molecules that interact with target RNA molecules and down-regulate gene encoding target RNA molecules (for example target RNA molecules referred to by Genbank Accession number in Table III) are expressed from transcription units inserted into DNA or RNA vectors. The recombinant vectors can be DNA plasmids or viral vectors. siNA expressing viral vectors can be constructed based on, but not limited to, adeno-associated virus, retrovirus, adenovirus, or alphavirus. The recombinant vectors capable of expressing the siNA molecules can be delivered as described herein, and persist in target cells. Alternatively, viral vectors can be used that provide for transient expression of siNA molecules. Such vectors can be repeatedly administered as necessary. Once expressed, the siNA molecules bind and down-regulate gene function or expression via RNA interference (RNAi). Delivery of siNA expressing vectors can be systemic, such as by intravenous or intramuscular administration, by administration to target cells ex-planted from a subject followed by reintroduction into the subject, or by any other means that would allow for introduction into the desired target cell.

By "vectors" is meant any nucleic acid- and/or viral-based technique used to deliver a desired nucleic acid

Other features and advantages of the invention will be apparent from the following description of the preferred embodiments thereof, and from the claims.

### BRIEF DESCRIPTION OF THE DRAWINGS

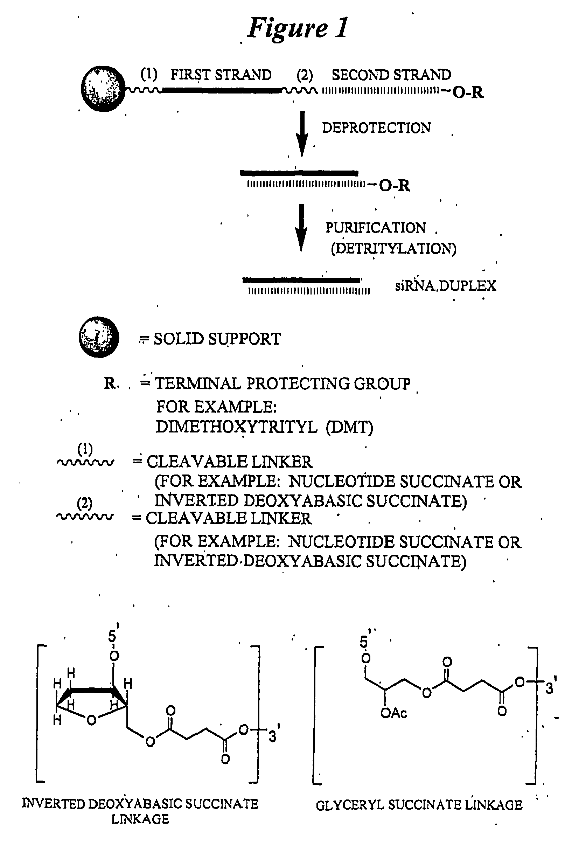

**Figure 1** shows a non-limiting example of a scheme for the synthesis of siNA molecules. The complementary siNA sequence strands, strand 1 and strand 2, are synthesized in tandem and are connected by a cleavable linkage, such as a nucleotide succinate or abasic succinate, which can be the same or different from the cleavable linker used for solid phase synthesis on a solid support. The synthesis can be either solid phase or solution phase, in the example shown, the synthesis is a solid phase synthesis. The synthesis is performed such that a protecting group, such as a dimethoxytrityl group, remains intact on the terminal nucleotide of the tandem oligonucleotide. Upon cleavage and deprotection of the oligonucleotide, the two siNA strands spontaneously hybridize to form a siNA duplex, which allows the purification of the duplex by utilizing the properties of the terminal protecting group, for example by applying a trityl on purification method wherein only duplexes/oligonucleotides with the terminal protecting group are isolated.

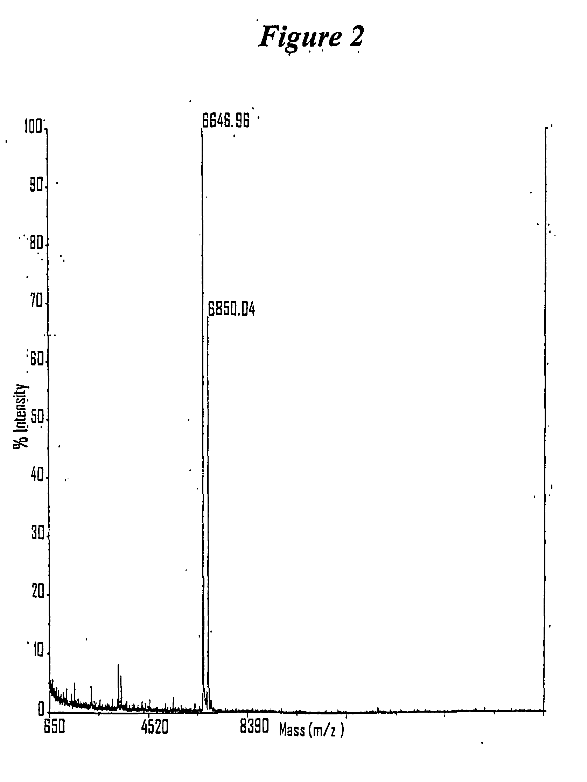

**Figure 2** shows a MALDI-TOV mass spectrum of a purified siNA duplex synthesized by a method of the invention. The two peaks shown correspond to the predicted mass of the separate siNA sequence strands. This result demonstrates that the siNA duplex generated from tandem synthesis can be purified as a single entity using a simple trityl-on purification methodology.

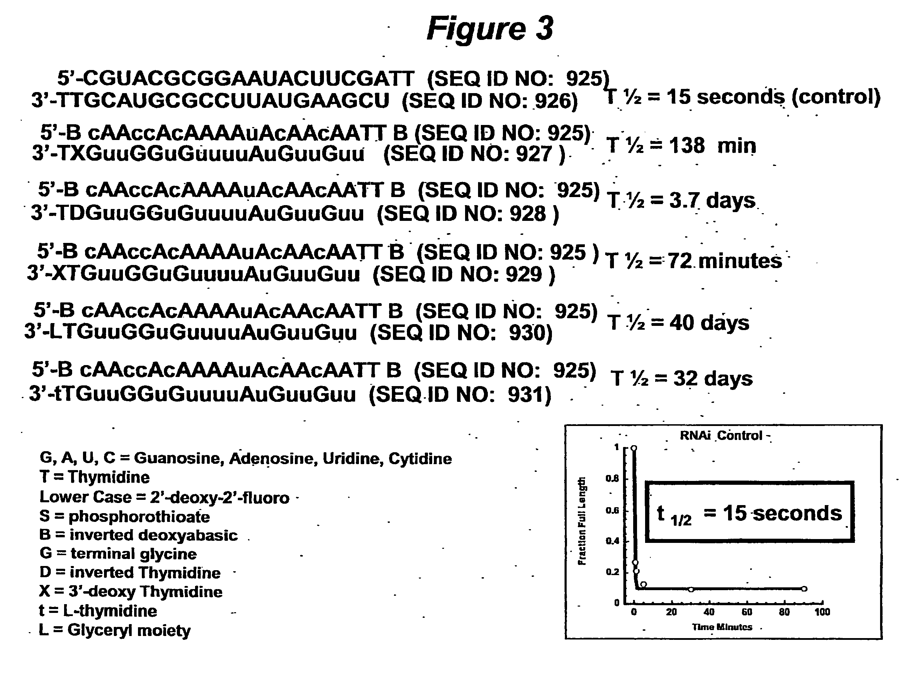

**Figure 3** shows the results of a stability assay used to determine the serum stability of chemically modified siNA constructs compared to a siNA control consisting of all RNA with 3'-TT termini. T ½ values are shown for duplex stability.
**Figure 4** shows the results of an RNAi activity screen of phosphorothioate modified siNA constructs using a luciferase reporter system.

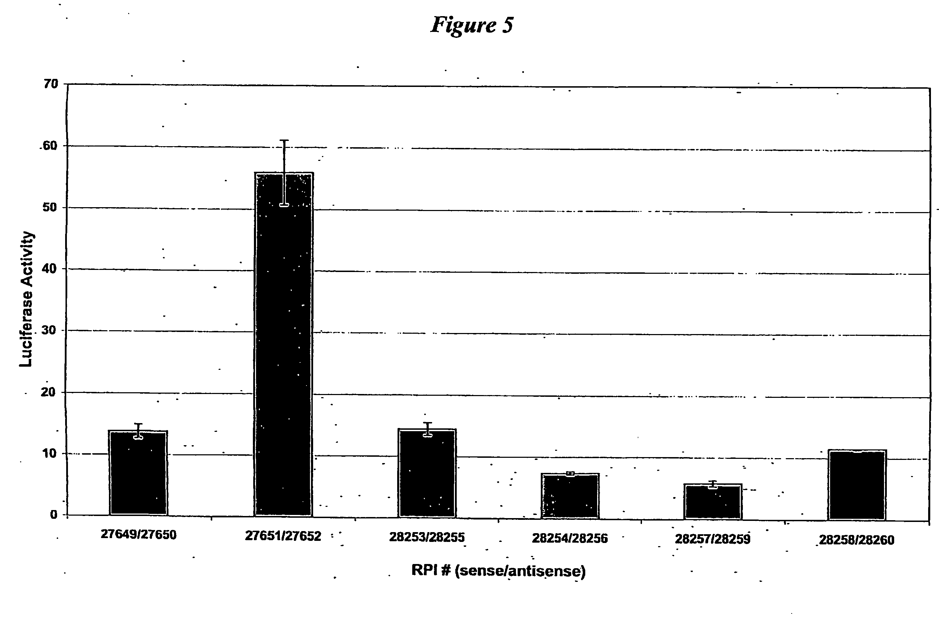

**Figure 5** shows the results of an RNAi activity screen of phosphorothioate and universal base modified siNA constructs using a luciferase reporter system.

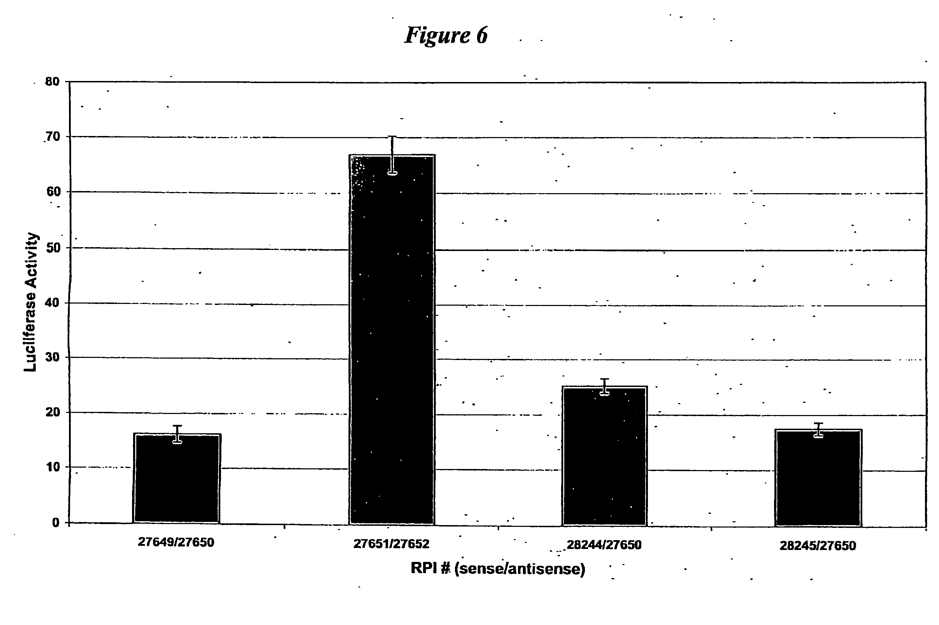

**Figure 6** shows the results of an RNAi activity screen of 2'-O-methyl modified siNA constructs using a luciferase reporter system.

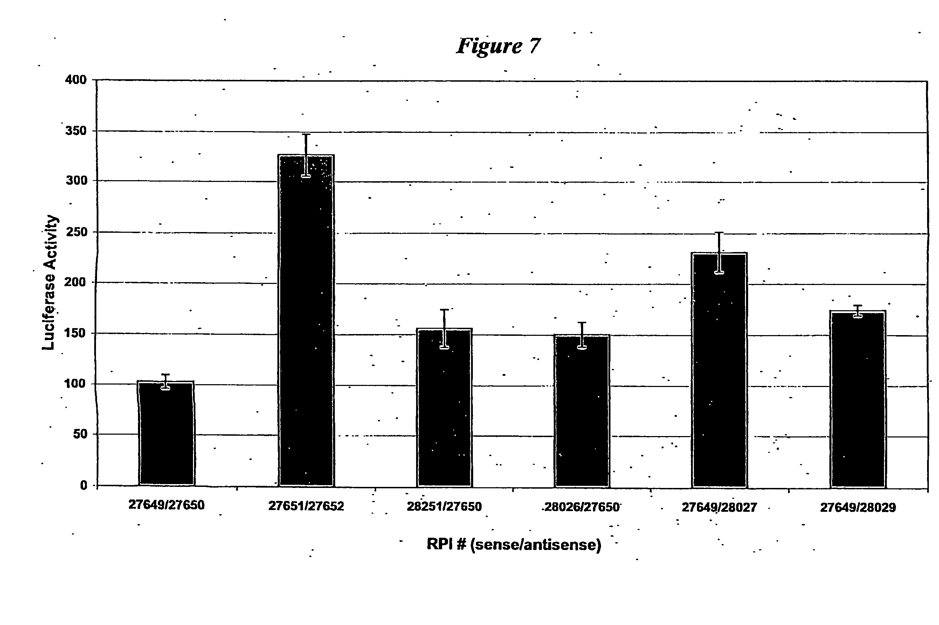

**Figure 7** shows the results of an RNAi activity screen of 2'-O-methyl and 2'-deoxy-2'-fluoro modified siNA constructs using a luciferase reporter system.

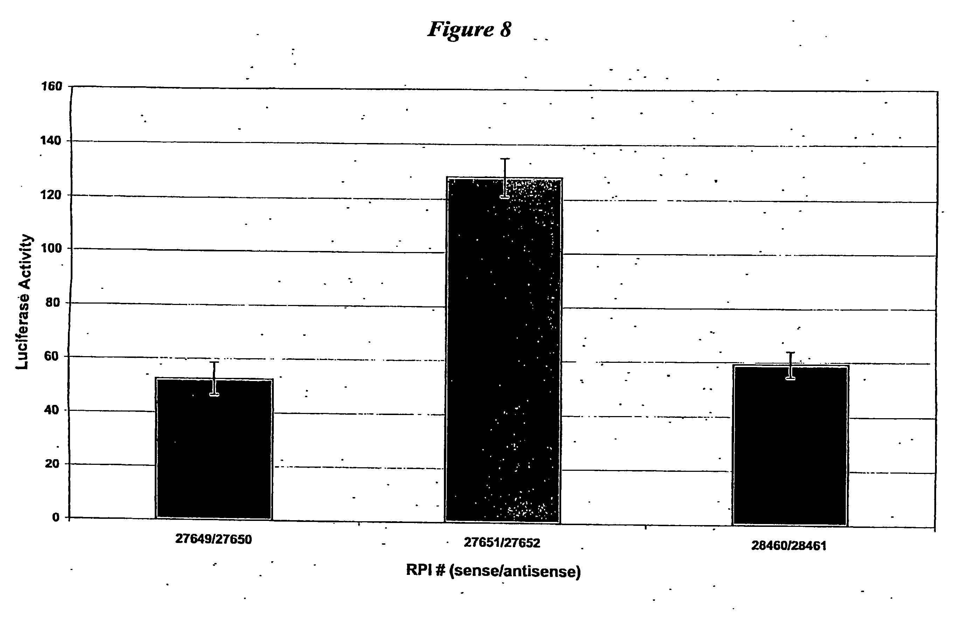

**Figure 8** shows the results of an RNAi activity screen of a phosphorothioate modified siNA construct using a luciferase reporter system.

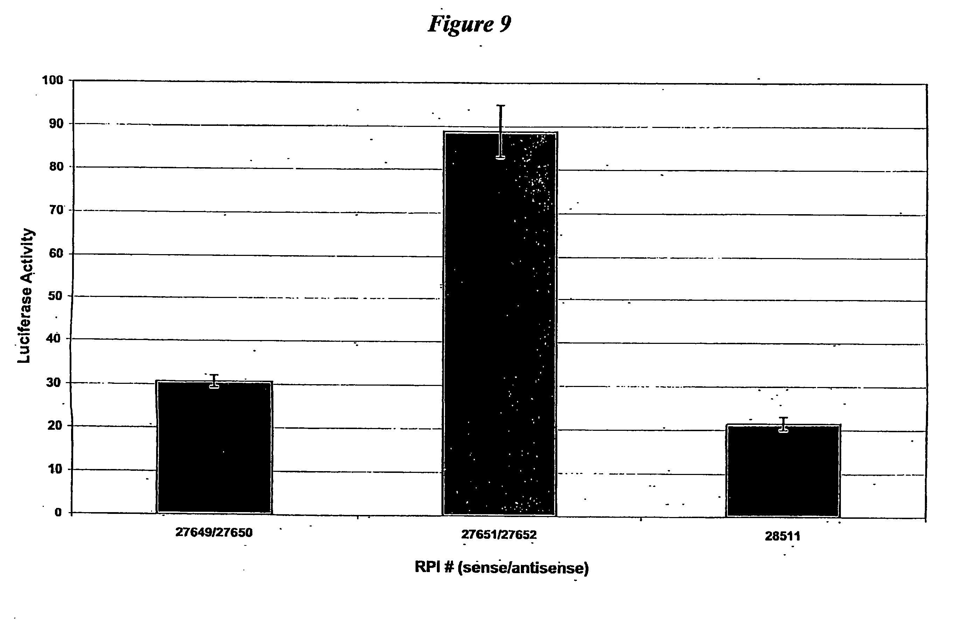

**Figure 9** shows the results of an RNAi activity screen of an inverted deoxyabasic modified siNA construct generated via tandem synthesis using a luciferase reporter system.

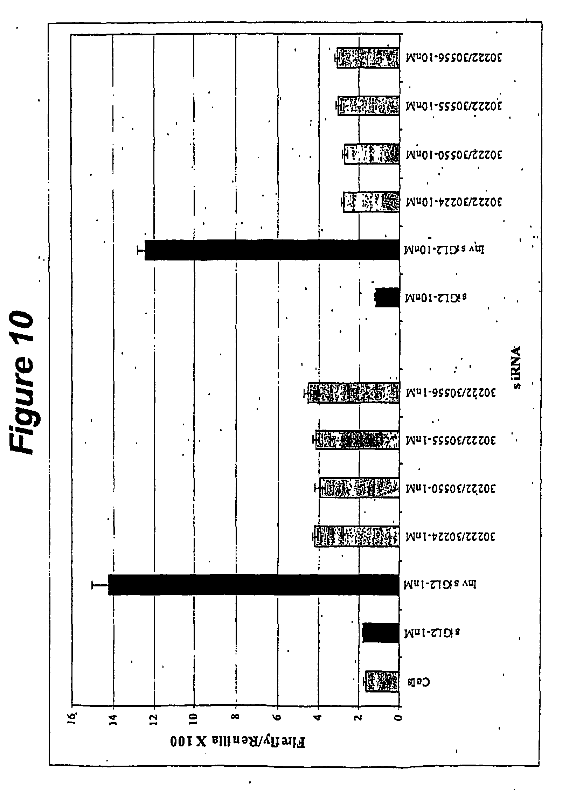

**Figure 10** shows the results of an RNAi activity screen of chemically modifed siNA constructs including 3'-glyceryl modified siNA constructs compared to an all RNA control siNA construct using a luciferase reporter system. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I.

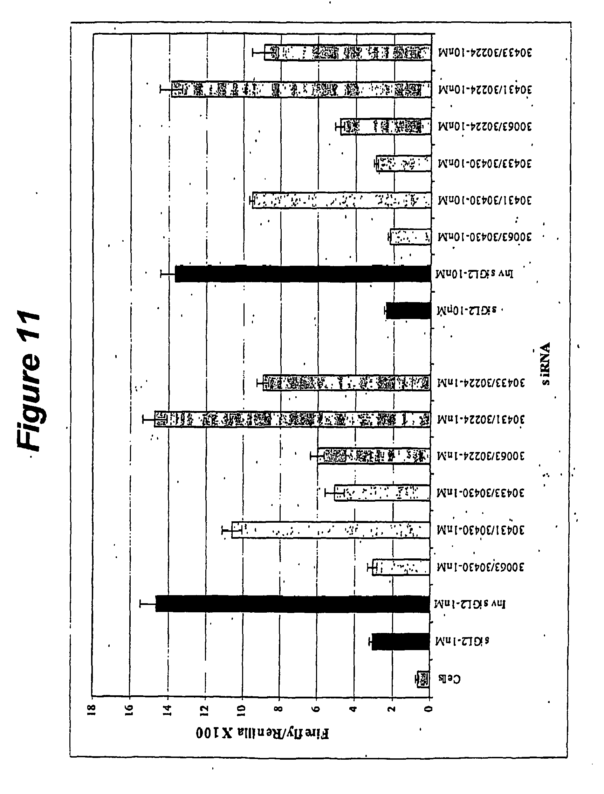

**Figure 11** shows the results of an RNAi activity screen of chemically modifed siNA constructs. The screen compared various combinations of sense strand chemical modifications and antisense strand chemical modifications. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I.

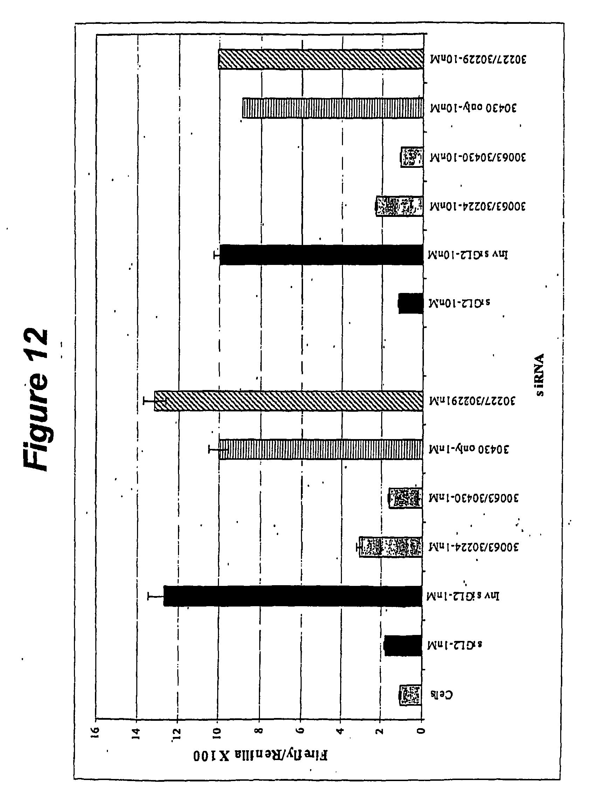

**Figure 12** shows the results of an RNAi activity screen of chemically modifed siNA constructs. The screen compared various combinations of sense strand chemical modifications and antisense strand chemical modifications. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table L In addition, the antisense strand alone (RPI 30430) and an inverted control (RPI 30227/30229, having matched chemistry to RPI 30063/30224) was compared to the siNA duplexes described above.

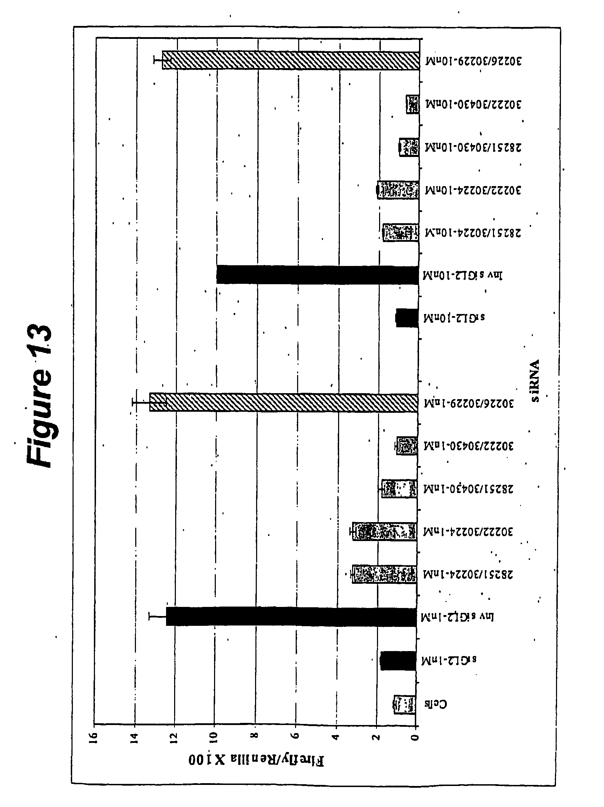

**Figure 13** shows the results of an RNAi activity screen of chemically modifed siNA constructs. The screen compared various combinations of sense strand chemical modifications and antisense strand chemical modifications. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I. In addition, an inverted control (RPI 30226/30229, having matched chemistry to RPI 30222/30224) was compared to the siNA duplexes described above.

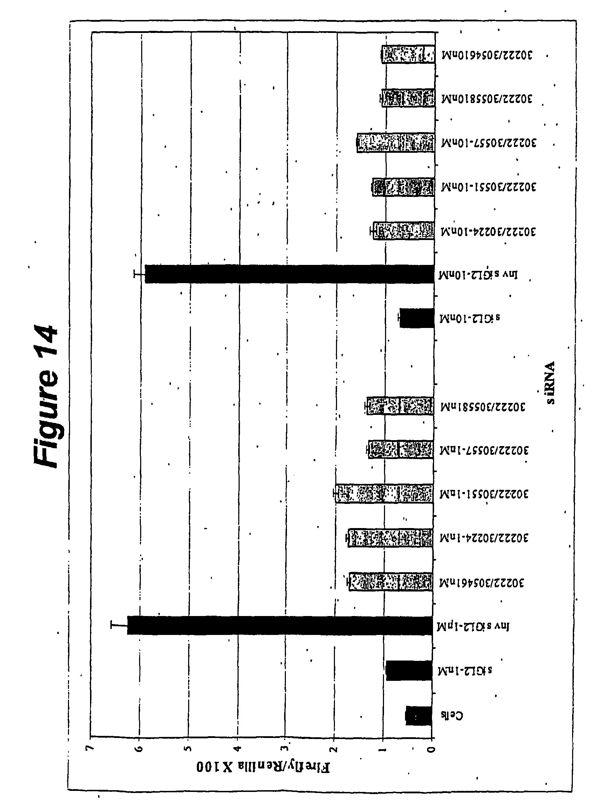

**Figure 14** shows the results of an RNAi activity screen of chemically modifed siNA constructs including various 3'-terminal modified siNA constructs compared to an all RNA control siNA construct using a luciferase reporter system. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I.

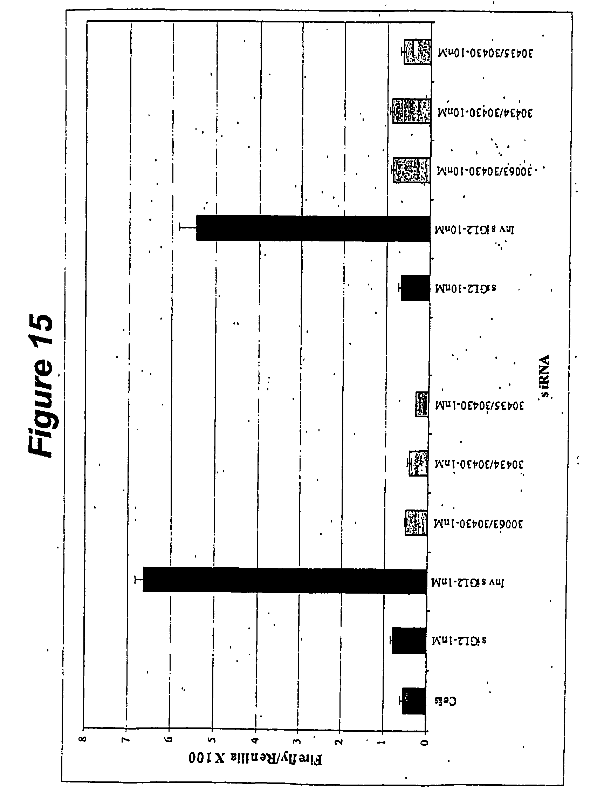

**Figure 15** shows the results of an RNAi activity screen of chemically modifed siNA constructs. The screen compared various combinations of sense strand chemistries compared to a fixed antisense strand chemistry. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I.

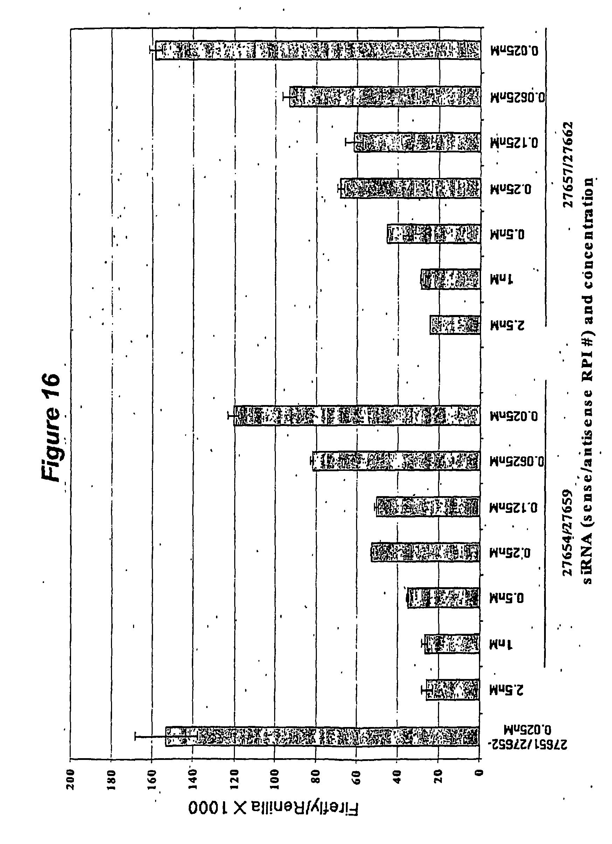

**Figure 16** shows the results of a siNA titration study wherein the RNAi activity of a phosphorothioate modified siNA construct is compared to that of a siNA construct consisting of all ribonucleotides except for two terminal thymidine residues using a luciferase reporter system.

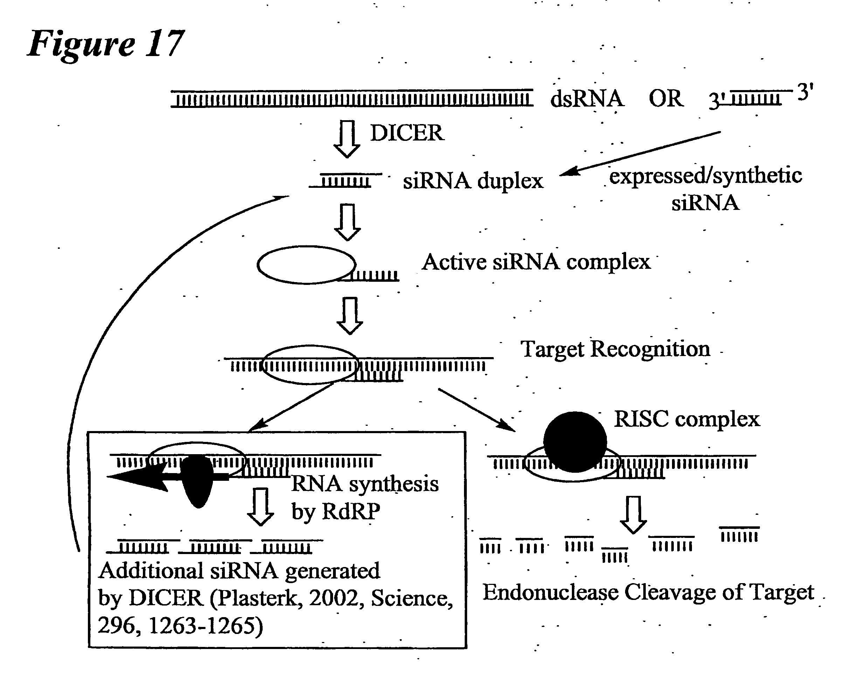

**Figure 17** shows a non-limiting proposed mechanistic representation of target RNA degradation involved in RNAi. Double-stranded RNA (dsRNA), which is generated by RNA-dependent RNA polymerase (RdRP) from foreign single-stranded RNA, for example viral, transposon, or other exogenous RNA, activates the DICER enzyme that in turn generates siNA duplexes. Alternately, synthetic or expressed siNA can be introduced directely into a cell by appropriate means. An active siNA complex forms which recognizes a target RNA, resulting in degradation of the target RNA by the RISC endonuclease complex or in the synthesis of additional RNA by RNA-dependent RNA polymerase (RdRP), which can activate DICER and result in additional siNA molecules, thereby amplifying the RNAi response.
**Figure 18A-F** shows non-limiting examples of chemically-modified siNA constructs of the present invention. In the figure, N stands for any nucleotide (adenosine, guanosine, cytosine, uridine, or optionally thymidine, for example thymidine can be substituted in the overhanging regions designated by parenthesis (N N). Various modifications are shown for the sense and antisense strands of the siNA constructs.
**Figure 18A:** The sense strand comprises 21 nucleotides having four phosphorothioate 5'- and 3'-terminal internucleotide linkages, wherein the two terminal 3'-nucleotides are optionally base paired and wherein all pyrimidine nucleotides that may be present are 2'-O-methyl or 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein the two terminal 3'-nucleotides are optionally complementary to the target RNA sequence, and having one 3'-terminal phosphorothioate internucleotide linkage and four 5'-terminal phosphorothioate internucleotide linkages and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein.
**Figure 18B:** The sense strand comprises 21 nucleotides wherein the two terminal 3'-nucleotides are optionally base paired and wherein all pyrimidine nucleotides that may be present are 2'-O-methyl or 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein.the two terminal 3'-nucleotides are optionally complementary to the target RNA sequence, and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein.
**Figure 18C:** The sense strand comprises 21 nucleotides having 5'- and 3'- terminal cap moieties wherein the two terminal 3'-nucleotides are optionally base paired and wherein all pyrimidine nucleotides that may be present are 2'-O-methyl or 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein the two terminal 3'-nucleotides are optionally complementary to the target RNA sequence, and having one 3'-terminal phosphorothioate internucleotide linkage and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein.
**Figure 18D:** The sense strand comprises 21 nucleotides having 5'- and 3'- terminal cap moieties wherein the two terminal 3'-nucleotides are optionally base paired and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein and wherein and all purine nucleotides that may be present are 2'-deoxy nucleotides. The antisense strand comprises 21 nucleotides, optionally having a 3'-terminal glyceryl moiety and wherein the two terminal 3'-nucleotides are optionally complementary to the target RNA sequence, and having one 3'-terminal phosphorothioate internucleotide linkage and wherein all pyrimidine nucleotides that may be present are 2'-deoxy-2'-fluoro modified nucleotides and all purine nucleotides that may be present are 2'-O-methyl modified nucleotides except for (N N) nucleotides, which can comprise ribonucleotides, deoxynucleotides, universal bases, or other chemical modifications described herein.
**Figure 18E:** The sense strand comprises 21 nucleotides having 5'- and 3'- terminal cap moieties wherein the two terminal 3'-nucleotides are optionally base paired and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein the two terminal 3'-nucleotides are optionally complementary to the target RNA sequence, and wherein all pyrimidine nucleotides that may be present are 2'-deoxy-2'-fluoro modified nucleotides and all purine nucleotides that may be present are 2'-O-methyl modified nucleotides except for (N N) nucleotides, which can comprise ribonucleotides, deoxynucleotides, universal bases, or other chemical modifications described herein.
**Figure 18F:** The sense strand comprises 21 nucleotides having 5'- and 3'- terminal cap moieties wherein the two terminal 3'-nucleotides are optionally base paired and wherein all pyrimidine nucleotides that may be present are 2'-deoxy-2'-fluoro modified nucleotides except for (N N) nucleotides, which can comprise ribonucleotides, deoxynucleotides, universal bases, or other chemical modifications described herein. The antisense strand comprises 21 nucleotides, optionally having a 3'-terminal glyceryl moiety and wherein the two terminal 3'-nucleotides are optionally complementary to the target RNA sequence, and having one 3'-terminal phosphorothioate internucleotide linkage and wherein all pyrimidine nucleotides that may be present are 2'-deoxy-2'-fluoro modified nucleotides and all purine nucleotides that may be present are 2'-deoxy modified nucleotides except for (N N) nucleotides, which can comprise ribonucleotides, deoxynucleotides, universal bases, or other chemical modifications described herein. The antisense strand of constructs A-F comprise sequence complementary to target RNA sequence of the invention.
**Figure 19** shows non-limiting examples of specific chemically modified siNA sequences of the invention. **A-F** applies the chemical modifications described in **Figure 18A-F** to a representative siNA sequence targeting the EGFR (HER1).

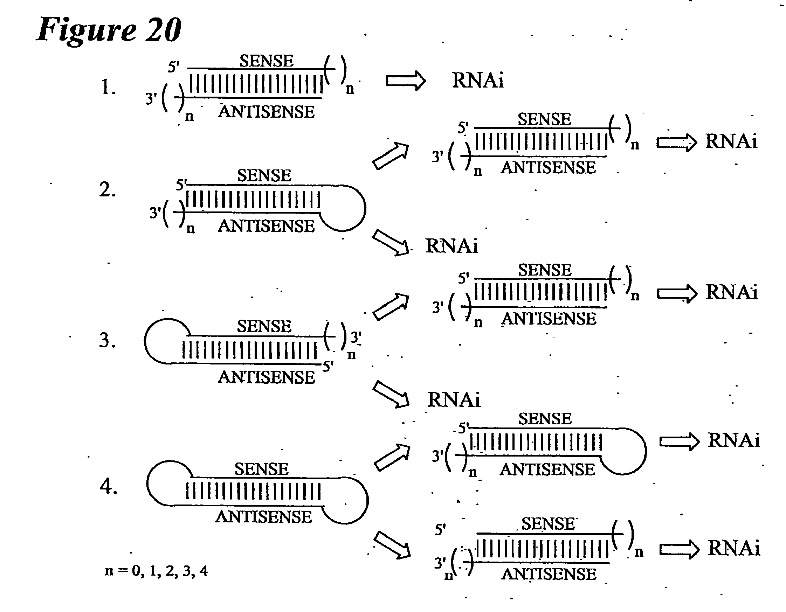

**Figure 20** shows non-limiting examples of different siNA constructs of the invention. The examples shown (constructs 1, 2, and 3) have 19 representative base pairs, however, different embodiments of the invention include any number of base pairs described herein. Bracketed regions represent nucleotide overhangs, for example comprising between about 1, 2, 3, or 4 nucleotides in length, preferably about 2 nucleotides. Constructs 1 and 2 can be used independently for RNAi activity. Construct 2 can comprise a polynucleotide or non-nucleotide linker, which can optionally be designed as a biodegradable linker. In one embodiment, the loop structure shown in construct 2 can comprise a biodegradable linker that results in the formation of construct 1 in vivo and/or in vitro. In another example, construct 3 can be used to generate construct 2 under the same principle wherein a linker is used to generate the active siNA construct 2 in vivo and/or in vitro, which can optionally utilize another biodegradable linker to generate the active siNA construct 1 in vivo and/or in vitro. As such, the stability and/or activity of the siNA constructs can be modulated based on the design of the siNA construct for use in vivo or in vitro and/or in vitro.

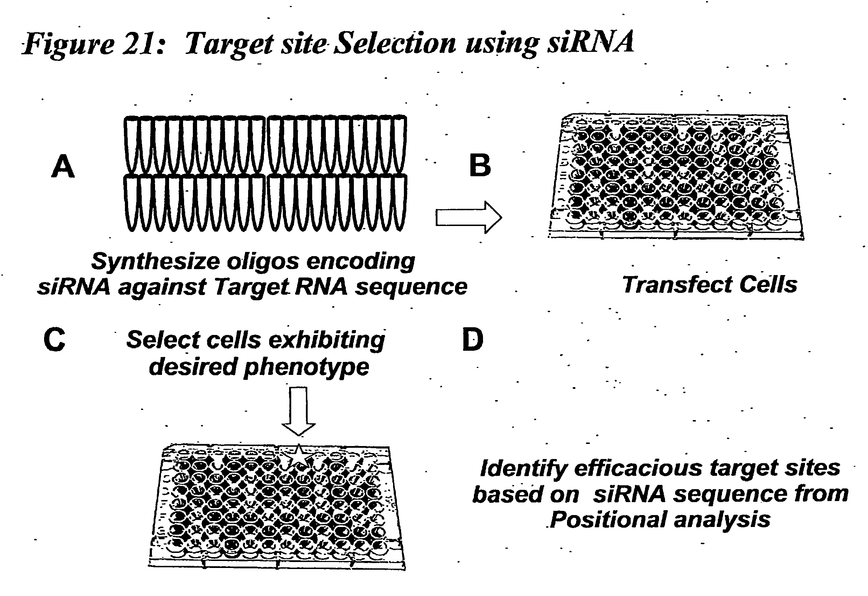

**Figure 21** is a diagrammatic representation of a method used to determine target sites for siNA mediated RNAi within a particular target nucleic acid sequence, such as messenger RNA. (A) A pool of siNA oligonucleotides are synthesized wherein the antisense region of the siNA constructs has complementarity to target sites across the target nucleic acid sequence, and wherein the sense region comprises sequence complementary to the antisense region of the siNA. (B) The sequences are transfected into cells. (C) Cells are selected based on phenotypic change that is associated with modulation of the target nucleic acid sequence. (D) The siNA is isolated from the selected cells and is sequenced to identify efficacious target sites within the target nucleic acid sequence.
**Figure 22** shows non-limiting examples of different stabilization chemistries (1-10) that can be used, for example, to stabilize the 3'-end of siNA sequences of the invention, including **(1)** [3-3']-inverted deoxyribose; **(2)** deoxyribonucleotide; **(3)** [5'-3']-3'-deoxyribonucleotide; **(4)** [5'-3']-ribonucleotide; **(5)** [5'-3']-3'-O-methyl ribonucleotide; **(6)** 3'-glyceryl; **(7)** [3'-5']-3'-deoxyribonucleotide; **(8)** [3'-3']-deoxyribonucleotide; **(9)** [5'-2']-deoxyribonucleotide; and **(10)** [5-3']-dideoxyribonucleotide. In addition to modified and unmodified backbone chemistries indicated in the figure, these chemistries can be combined with different backbone modifications as described herein, for example, backbone modifications having Formula I. In addition, the 2'-deoxy nucleotide shown 5' to the terminal modifications shown can be another modified or unmodified nucleotide or non-nucleotide described herein, for example modifications having any of Formulae I-VII or any combination thereof.

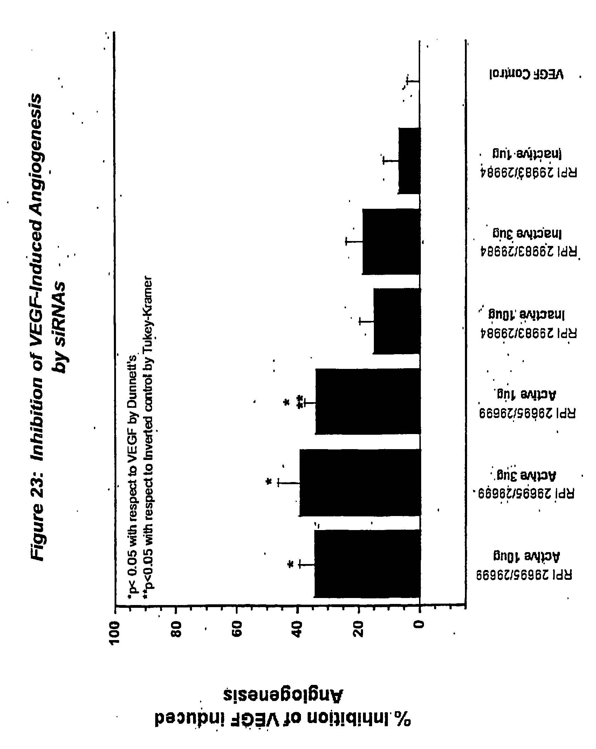

**Figure 23** shows a non-limiting example of siNA mediated inhibition of VEGF-induced angiogenesis using the rat corneal model of angiogenesis. siNA targeting site 2340 of VEGFR1 RNA (shown as RPI No. sense strand/antisense strand) were compared to inverted controls (shown as RPI No. sense strand/antisense strand) at three different concentrations and compared to a VEGF control in which no siNA was administered.

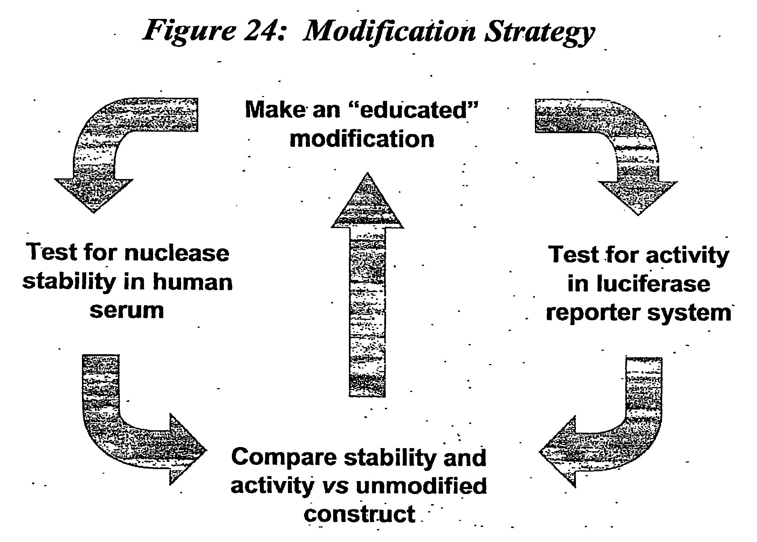

**Figure 24** shows a non-limiting example of a strategy used to identify chemically modified siNA constructs of the invention that are nuclease resistance while preserving the ability to mediate RNAi activity. Chemical modifications are introduced into the siNA construct based on educated design parameters (e.g. introducing 2'-mofications, base modifications, backbone modifications, terminal cap modifications etc). The modified construct in tested in an appropriate system (e.g human serum for nuclease resistance, shown, or an animal model for PK/delivery parameters). In parallel, the siNA construct is tested for RNAi activity, for example in a cell culture system such as a luciferase reporter assay). Lead siNA constructs are then identified which possess a particular characteristic while maintaining RNAi activity, and can be further modified and assayed once again. This same approach can be used to identify siNA-conjugate molecules with improved pharmacokinetic profiles, delivery, and RNAi activity.

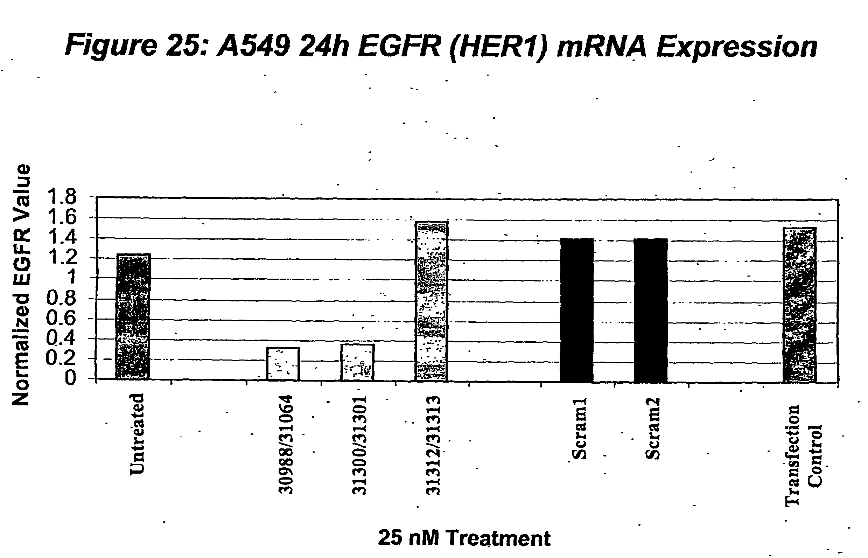

**Figure 25** shows a non-limiting example of reduction of HER2 mRNA in A549 cells mediated by RNA-based and chemically-modified siNAs that target HER2 mRNA sites 2344 and 3706. A549 cells were transfected with 4 ug/ml lipid complexed with 25 nM unmodified siNA with a 3'-terminal dithymidine cap (RPI#28266/28267) or a corresponding inverted control (RPI#28268/28269) for site 2344 and (RPI#28262/28263) and a corresponding inverted control (RPI 28264/28265) for site 3706. In addition, A549 cells were transfected with 4 ug/ml lipid complexed with 25 nM modified siNA (RPI#30442/30443) and a corresponding matched control (RPI#30444/30445) for site 2344 and (RPI#30438/30439) and a corresponding matched control (RPI 30440/30441) for site 3706. As shown in the figures, the modified and unmodified constructs targeting sites 2344 and 3706 all demonstrate significant inhibition of HER2 RNA expression.

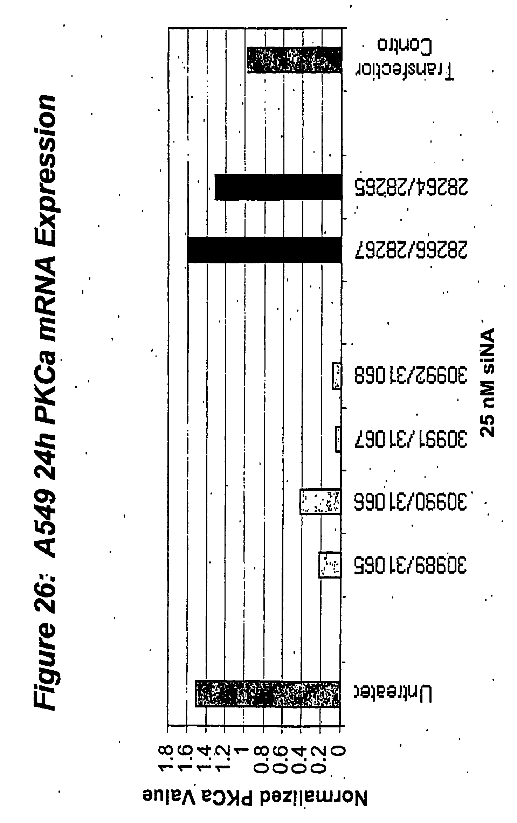

**Figure 26** shows a non-limiting example of reduction of PKC-alpha mRNA in A549 cells mediated by chemically-modified siNAs that target PKC-alpha mRNA. A549 cells were transfected with 0.25 ug/well of lipid complexed with 25 nM siNA. A screen of siNA constructs comprising ribonucleotides and 3'-terminal dithymidine caps was compared to untreated cells, scrambled siNA control constructs (Scram1 and Scram2), and cells transfected with lipid alone (transfection control). As shown in the figure, all of the siNA constructs show significant reduction of PKC-alpha RNA expression.

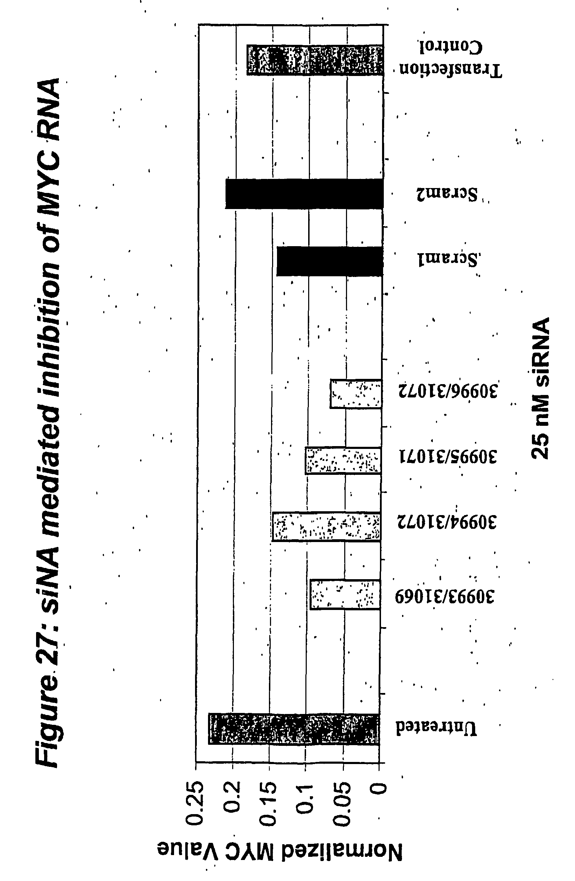

**Figure 27** shows a non-limiting example of reduction of Myc (c-Myc) mRNA in 293T cells mediated by chemically-modified siNAs that target c-Myc mRNA. 293T cells were transfected with 0.25 ug/well of lipid complexed with 25 nM siNA. A screen of siNA constructs comprising ribonucleotides and 3'-terminal dithymidine caps was compared to untreated cells, scrambled siNA control constructs (Scram1 and Scram2), and cells transfected with lipid alone (transfection control). As shown in the figure, three of the siNA constructs (RPI 30993/31069; RPI 30995/31071; and RPI 30996/31072) show significant reduction of c-Myc RNA expression.

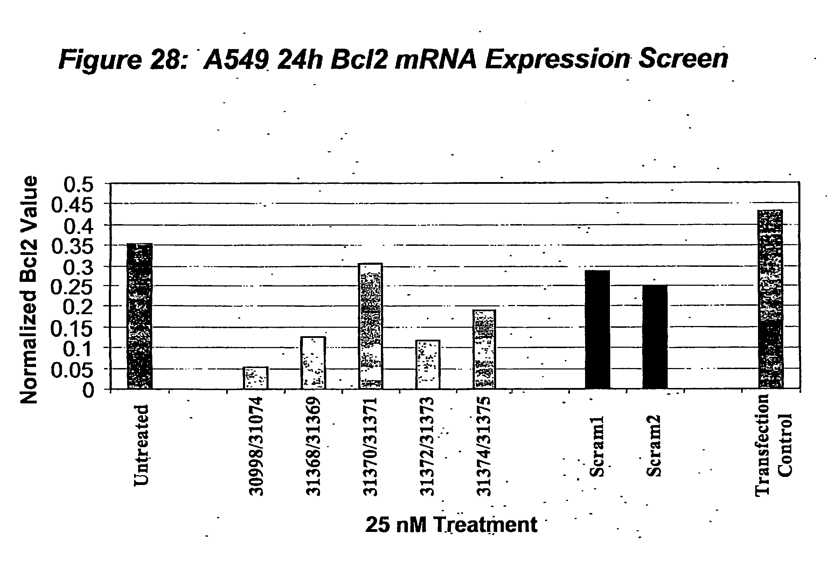

**Figure 28** shows a non-limiting example of reduction of BCL2 mRNA in A549 cells mediated by chemically-modified siNAs that target BCL2 mRNA. A549 cells were transfected with 0.25 ug/well of lipid complexed with 25 nM siNA. A siNA construct comprising ribonucleotides and 3'-terminal dithymidine caps (RPI#30998/31074) was tested along with a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and the antisense strand comprises a 3'-terminal phosphorothioate internucleotide linkage (RPI#31368/31369), which was also compared to a matched chemistry inverted control (RPI#31370/31371) and a chemically modified siNA construct comprising 2'-deoxy-2'-fluoro pyrimidine and 2'-deoxy-2'-fluoro purine nucleotides in which the sense strand of the siNA is further modified with 5' and 3'-terminal inverted deoxyabasic caps and the antisense strand comprises a 3'-terminal phosphorothioate internucleotide linkage (RPI#31372/31373) which was also compared to a matched chemistry inverted control (RPI#31374/31375). In addition, the siNA constructs were also compared to untreated cells, cells transfected with lipid and scrambled siNA constructs (Scram1 and Scram2), and cells transfected with lipid alone (transfection control). As shown in the figure, the siNA constructs show significant reduction of BCL2 RNA expression compared to scrambled, untreated, and transfection controls.

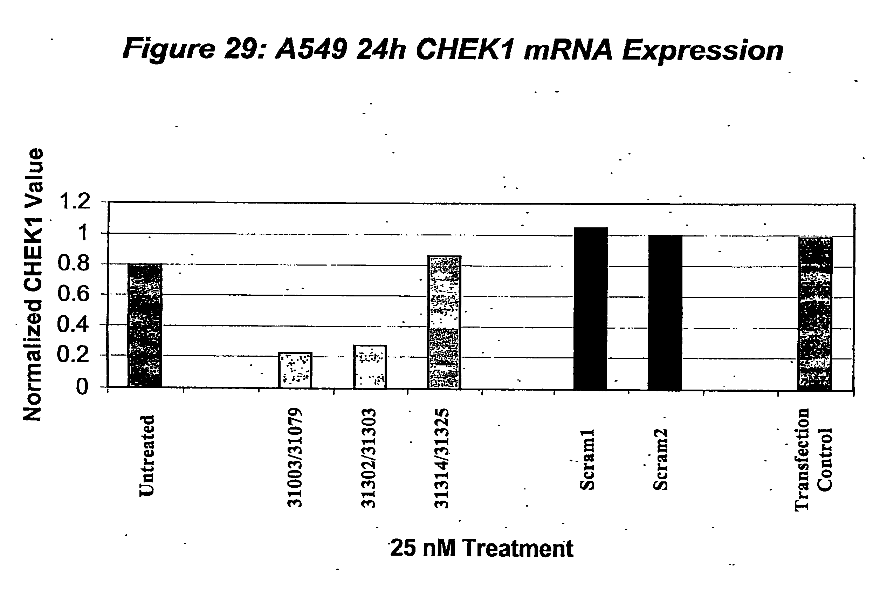

**Figure 29** shows a non-limiting example of reduction of CHK-1 mRNA in A549 cells mediated by chemically-modified siNAs that target CHK-1 mRNA. A549 cells were transfected with 0.25 ug/well of lipid complexed with 25 nM siNA. A siNA construct comprising ribonucleotides and 3'-terminal dithymidine caps (RPI#31003/31079) and a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and in which the antisense strand comprises a 3'-terminal phosphorothioate internucleotide linkage (RPI#31302/31303), were compared to a matched chemistry inverted control (RPI#31314/31325). In addition, the siNA constructs were also compared to untreated cells, cells transfected with lipid and scrambled siNA constructs (Scram1 and Scram2), and cells transfected with lipid alone (transfection control). As shown in the figure, both siNA constructs show significant reduction of CHK-1 RNA expression compared to appropriate controls.

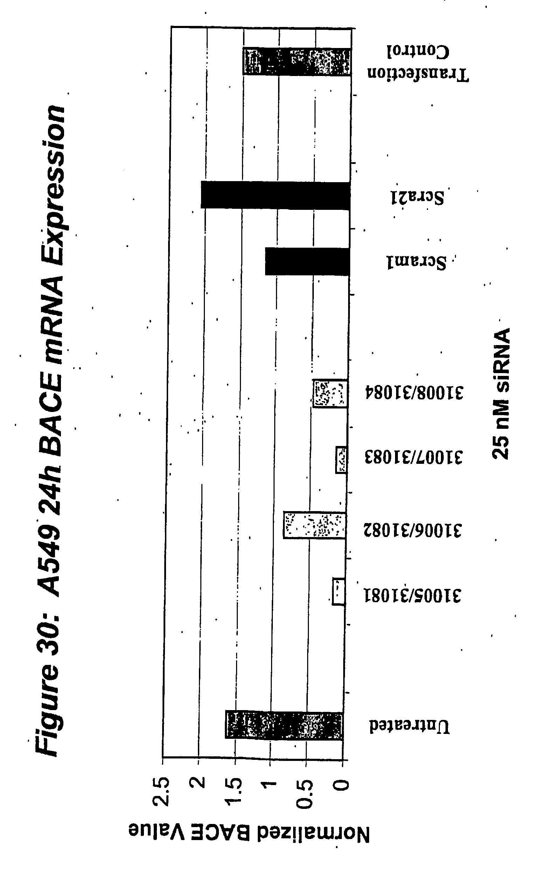

**Figure 30** shows a non-limiting example of reduction of BACE mRNA in A549 cells mediated by siNAs that target BACE mRNA. A549 cells were transfected with 0.25 ug/well of lipid complexed with 25 nM siNA. A screen of siNA constructs comprising ribonucleotides and 3'-terminal dithymidine caps was compared to untreated cells, scrambled siNA control constructs (Scram1 and Scram2), and cells transfected with lipid alone (transfection control). As shown in the figure, all of the siNA constructs show significant reduction of BACE RNA expression.

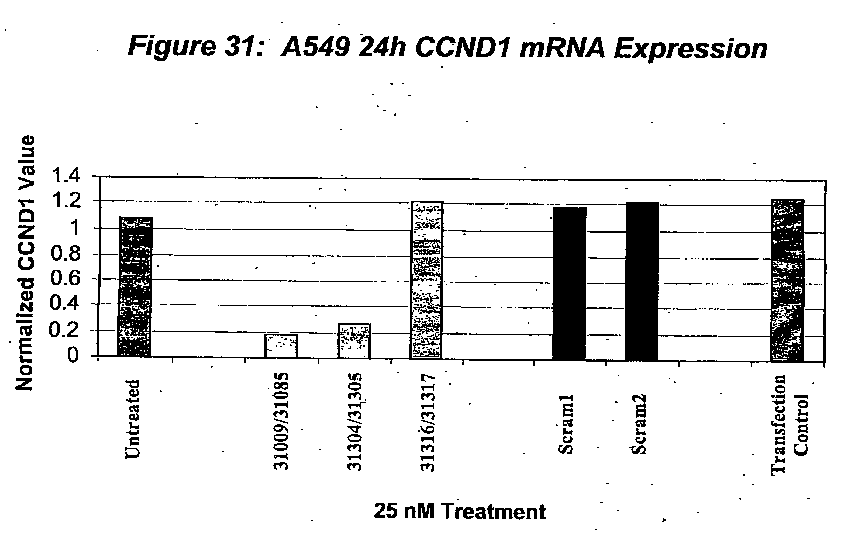

**Figure 31** shows a non-limiting example of reduction of cyclin D1 mRNA in A549 cells mediated by chemically-modified siNAs that target cyclin D 1 mRNA. A549 cells were transfected with 0.25 ug/well of lipid complexed with 25 nM siNA. A siNA construct comprising ribonucleotides and 3'-terminal dithymidine caps (RPI#31009/31085) was compared to a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and the antisense strand comprises a 3'-terminal phosphorothioate internucleotide linkage (RPI#31304/31305), which was also compared to a matched chemistry inverted control (RPI#31316/31317). In addition, the siNA constructs were also compared to untreated cells, cells transfected with lipid and scrambled siNA constructs (Scram1 and Scram2), and cells transfected with lipid alone (transfection control). As shown in the figure, both siNA constructs show significant reduction of cyclin D1 RNA expression.

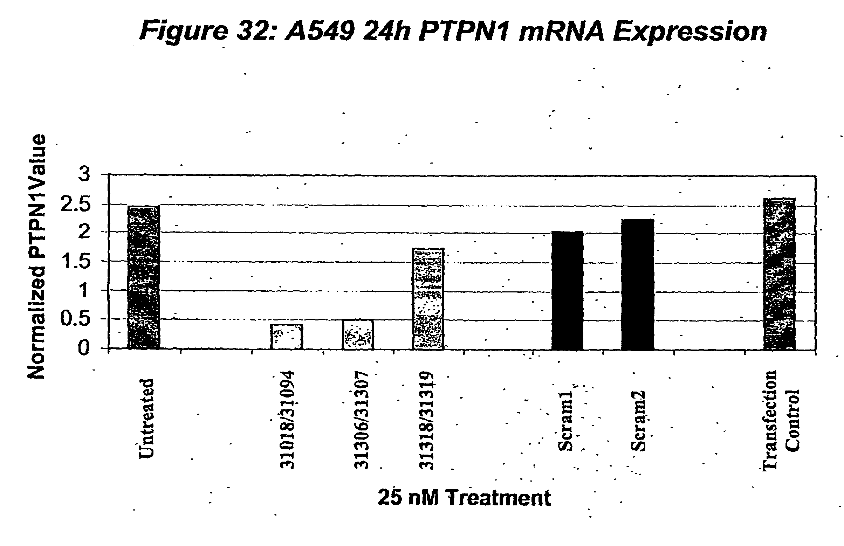

**Figure 32** shows a non-limiting example of reduction of PTP-1B mRNA in A549 cells mediated by chemically-modified siNAs that target PTP-1B mRNA. A549 cells were transfected with 0.25 ug/well of lipid complexed with 25 nM siNA. A siNA construct comprising ribonucleotides and 3'-terminal dithymidine caps (RPI#31018/31307) was compared to a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and the antisense strand comprises a 3'-terminal phosphorothioate internucleotide linkage (RPI#31306/31307), which was also compared to a matched chemistry inverted control (RPI#31318/31319). In addition, the siNA constructs were also compared to untreated cells, cells transfected with lipid and scrambled siNA constructs (Scram1 and Scram2), and cells transfected with lipid alone (transfection control). As shown in the figure, both siNA constructs show significant reduction of PTP-1B RNA expression.

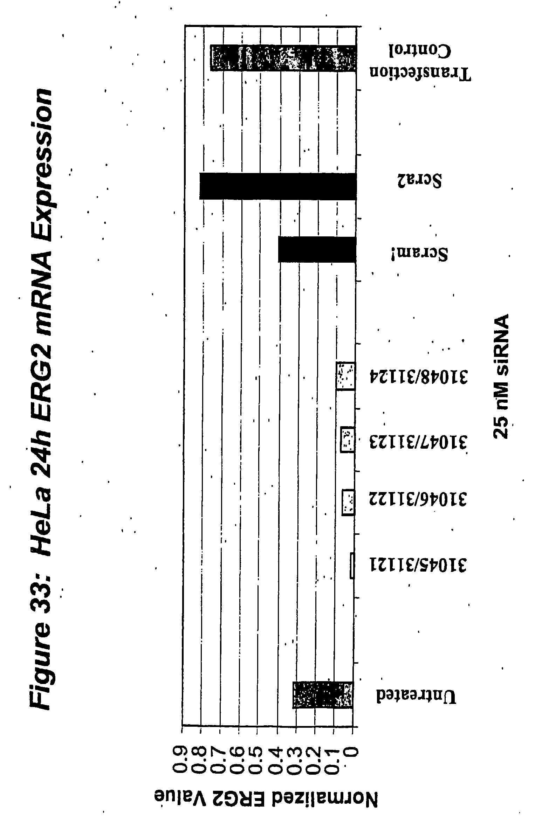

**Figure 33** shows a non-limiting example of reduction of ERG2 mRNA in DLD1 cells mediated by siNAs that target ERG2 mRNA. DLD1 cells were transfected with 0.25 ug/well of lipid complexed with 25 nM siNA. A screen of siNA constructs comprising ribonucleotides and 3'-terminal dithymidine caps was compared to untreated cells, scrambled siNA control constructs (Scram1 and Scram2), and cells transfected with lipid alone (transfection control). As shown in the figure, all of the siNA constructs show significant reduction of ERG2 RNA expression.

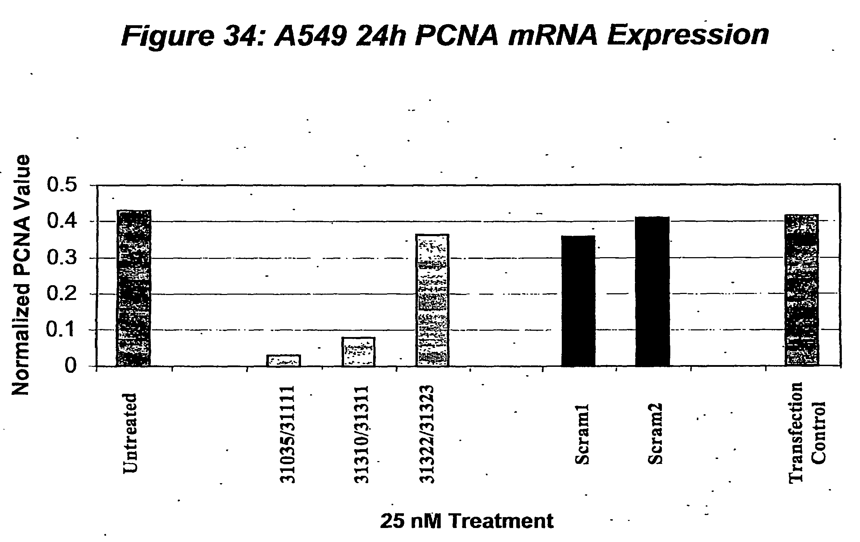

**Figure 34** shows a non-limiting example of reduction of PCNA mRNA in A549 cells mediated by chemically-modified siNAs that target PCNA mRNA. A549 cells were transfected with 0.25 ug/well of lipid complexed with 25 nM siNA. A siNA construct comprising ribonucleotides and 3'-terminal dithymidine caps (RPI#31035/31111) was compared to a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and the antisense strand comprises a 3'-terminal phosphorothioate internucleotide linkage (RPI#31310/31311), which was also compared to a matched chemistry inverted control (RPI#31322/31323). In addition, the siNA constructs were also compared to untreated cells, cells transfected with lipid and scrambled siNA constructs (Scram1 and Scram2), and cells transfected with lipid alone (transfection control). As shown in the figure, both siNA constructs show significant reduction of PCNA RNA expression.

### DETAILED DESCRIPTION OF THE INVENTION

### Mechanism of action of Nucleic Acid Molecules of the Invention

The discussion that follows discusses the proposed mechanism of RNA interference mediated by short interfering RNA as is presently known, and is not meant to be limiting and is not an admission of prior art. Applicant demonstrates herein that chemically-modified short interfering nucleic acids possess similar or improved capacity to mediate RNAi as do siRNA molecules and are expected to possess improved stability and activity *in vivo;* therefore, this discussion is not meant to be limiting only to siRNA and can be applied to siNA as a whole. By "improved capacity to mediate RNAi" or "improved RNAi activity" is meant to include RNAi activity measured *in vitro* and/or *in vivo* where the RNAi activity is a reflection of both the ability of the siNA to mediate RNAi and the stability of the siNAs of the invention. In this invention, the product of these activities can be increased *in vitro* and/or *in vivo* compared to an all RNA siRNA or a siNA containing a plurality of ribonucleotides. In some cases, the activity or stability of the siNA molecule can be decreased (i.e., less than ten-fold), but the overall activity of the siNA molecule is enhanced *in vitro* and/or *in vivo.*

RNA interference refers to the process of sequence specific post-transcriptional gene silencing in animals mediated by short interfering RNAs (siRNAs) (Fire et al., 1998, Nature, 391, 806). The corresponding process in plants is commonly referred to as post-transcriptional gene silencing or RNA silencing and is also referred to as quelling in fungi. The process of post-transcriptional gene silencing is thought to be an evolutionarily-conserved cellular defense mechanism used to prevent the expression of foreign genes which is commonly shared by diverse flora and phyla (Fire *et al*., 1999, *Trends Genet*., 15, 358). Such protection from foreign gene expression may have evolved in response to the production of double-stranded RNAs (dsRNAs) derived from viral infection or the random integration of transposon elements into a host genome via a cellular response that specifically destroys homologous single-stranded RNA or viral genomic RNA. The presence of dsRNA in cells triggers the RNAi response though a mechanism that has yet to be fully characterized. This mechanism appears to be different from the interferon response that results from dsRNA-mediated activation of protein kinase PKR and 2', 5'-oligoadenylate synthetase resulting in non-specific cleavage of mRNA by ribonuclease L.

The presence of long dsRNAs in cells stimulates the activity of a ribonuclease III enzyme referred to as Dicer. Dicer is involved in the processing of the dsRNA into short pieces of dsRNA known as short interfering RNAs (siRNAs) (Berstein et al., 2001, Nature, 409, 363). Short interfering RNAs derived from Dicer activity are typically about 21 to about 23 nucleotides in length and comprise about 19 base pair duplexes. Dicer has also been implicated in the excision of 21- and 22-nucleotide small temporal RNAs (stRNAs) from precursor RNA of conserved structure that are implicated in translational control (Hutvagner et al., 2001, Science, 293, 834). The RNAi response also features an endonuclease complex containing a siRNA, commonly referred to as an RNA-induced silencing complex (RISC), which mediates cleavage of single-stranded RNA having sequence homologous to the siRNA. Cleavage of the target RNA takes place in the middle of the region complementary to the guide sequence of the siRNA duplex (Elbashir et al., 2001, Genes Dev., 15, 188). In addition, RNA interference can also involve small RNA (e.g., micro-RNA or miRNA) mediated gene silencing, presumably though cellular mechanisms that regulate chromatin structure and thereby prevent transcription of target gene sequences (see for example Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237). As such, siNA molecules of the invention can be used to mediate gene silencing via interaction with RNA transcripts or alternately by interaction with particular gene sequences, wherein such interaction results in gene silencing either at the transcriptional level or post-transcriptional level.

RNAi has been studied in a variety of systems. Fire et al., 1998, Nature, 391, 806, were the first to observe RNAi in *C. elegans.* Wianny and Goetz, 1999, Nature Cell Biol., 2, 70, describe RNAi mediated by dsRNA in mouse embryos. Hammond et al., 2000, Nature, 404, 293, describe RNAi in *Drosophila* cells transfected with dsRNA. Elbashir et al., 2001, Nature, 411, 494, describe RNAi induced by introduction of duplexes of synthetic 21-nucleotide RNAs in cultured mammalian cells including human embryonic kidney and HeLa cells. Recent work in Drosophila embryonic lysates has revealed certain requirements for siRNA length, structure, chemical composition, and sequence that are essential to mediate efficient RNAi activity. These studies have shown that 21 nucleotide siRNA duplexes are most active when containing two 2-nucleotide 3'-terminal nucleotide overhangs. Furthermore, substitution of one or both siRNA strands with 2'-deoxy or 2'-O-methyl nucleotides abolishes RNAi activity, whereas substitution of 3'-terminal siRNA nucleotides with deoxy nucleotides was shown to be tolerated. Mismatch sequences in the center of the siRNA duplex were also shown to abolish RNAi activity. In addition, these studies also indicate that the position of the cleavage site in the target RNA is defined by the 5'-end of the siRNA guide sequence rather than the 3'-end (Elbashir et al., 2001, EMBO J., 20, 6877). Other studies have indicated that a 5'-phosphate on the target-complementary strand of a siRNA duplex is required for siRNA activity and that ATP is utilized to maintain the 5'-phosphate moiety on the siRNA (Nykanen et al., 2001, Cell, 107, 309); however, siRNA molecules lacking a 5'-phosphate are active when introduced exogenously, suggesting that 5'-phosphorylation of siRNA constructs may occur *in vivo*.

### Synthesis of Nucleic acid Molecules

Synthesis of nucleic acids greater than 100 nucleotides in length is difficult using automated methods, and the therapeutic cost of such molecules is prohibitive. In this invention, small nucleic acid motifs "small" refers to nucleic acid motifs no more than 100 nucleotides in length, preferably no more than 80 nucleotides in length, and most preferably no more than 50 nucleotides in length; *e.g.,* individual siNA oligonucleotide sequences or siNA sequences synthesized in tandem) are preferably used for exogenous delivery. The simple structure of these molecules increases the ability of the nucleic acid to invade targeted regions of protein and/or RNA structure. Exemplary molecules of the instant invention are chemically synthesized, and others can similarly be synthesized.

Oligonucleotides (*e.g.,* certain modified oligonucleotides or portions of oligonucleotides lacking ribonucleotides) are synthesized using protocols known in the art, for example as described in Caruthers et al., 1992, Methods in Enzymology 211, 3-19, Thompson et al., International PCT Publication No. WO 99/54459, Wincott et al., 1995, Nucleic Acids Res. 23, 2677-2684, Wincott et al., 1997, Methods Mol. Bio., 74, 59, Brennan et al., 1998, Biotechnol Bioeng., 61, 33-45, and Brennan, U.S. Pat. No. 6,001,311. All of these references are incorporated herein by reference. The synthesis of oligonucleotides makes use of common nucleic acid protecting and coupling groups, such as dimethoxyhityl at the 5'-end, and phosphoramidites at the 3'-end. In a non-limiting example, small scale syntheses are conducted on a 394 Applied Biosystems, Inc. synthesizer using a 0.2 µmol scale protocol with a 2.5 min coupling step for 2'-O-methylated nucleotides and a 45 sec coupling step for 2'-deoxy nucleotides or 2'-deoxy-2'-fluoro nucleotides. **Table II** outlines the amounts and the contact times of the reagents used in the synthesis cycle. Alternatively, syntheses at the 0.2 µmol scale can be performed on a 96-well plate synthesizer, such as the instrument produced by Protogene (Palo Alto, CA) with minimal modification to the cycle. A 33-fold excess (60 µL of 0.11 M = 6.6 µmol) of 2'-O-methyl phosphoramidite and a 105-fold excess of S-ethyl tetrazole (60 µL of 0.25 M =15 µmol) can be used in each coupling cycle of 2'-O-methyl residues relative to polymer-bound 5'-hydroxyl. A 22-fold excess (40 µL of 0.11 M = 4.4 µmol) of deoxy phosphoramidite and a 70-fold excess of S-ethyl tetrazole (40 µL of 0.25 M = 10 µmol) can be used in each coupling cycle of deoxy residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer include the following: detritylation solution is 3% TCA in methylene chloride (ABI); capping is performed with 16% *N*-methyl imidazole in THF (ABI) and 10% acetic anhydride/10% 2,6-lutidine in THF (ABI); and.oxidation solution is 16.9 mM I₂, 49 mM pyridine, 9% water in THF (PERSEPTIVE™). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzodithiol-3-one 1,1-dioxide, 0.05 M in acetonitrile) is used.

Deprotection of the DNA-based oligonucleotides is performed as follows: the polymer-bound trityl-on oligoribonucleotide is transferred to a 4 mL glass screw top vial and suspended in a solution of 40% aq. methylamine (1 mL) at 65 °C for 10 min. After cooling to -20 °C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder.

The method of synthesis used for RNA including certain siNA molecules of the invention follows the procedure as described in Usman et al., 1987, J. Am. Chem. Soc., 109, 7845; Scaringe et al., 1990, Nucleic Acids Res., 18, 5433; and Wincott et al., 1995, Nucleic Acids Res. 23, 2677-2684 Wincott et al., 1997, Methods Mol. Bio., 74, 59, and makes use of common nucleic acid protecting and coupling groups, such as dimethoxytrityl at the 5'-end, and phosphoramidites at the 3'-end. In a non-limiting example, small scale syntheses are conducted on a 394 Applied Biosystems, Inc. synthesizer using a 0.2 µmol scale protocol with a 7.5 min coupling step for alkylsilyl protected nucleotides and a 2.5 min coupling step for 2'-O-methylated nucleotides. Table II outlines the amounts and the contact times of the reagents used in the synthesis cycle. Alternatively, syntheses at the 0.2 µmol scale can be done on a 96-well plate synthesizer, such as the instrument produced by Protogene (Palo Alto, CA) with minimal modification to the cycle. A 33-fold excess (60 µL of 0.11 M = 6.6 µmol) of 2'-O-methyl phosphoramidite and a 75-fold excess of S-ethyl tetrazole (60 µL of 0.25 M = 15 µmol) can be used in each coupling cycle of 2'-O-methyl residues relative to polymer-bound 5'-hydroxyl. A 66-fold excess (120 µL of 0.11 M = 13.2 µmol) of alkylsilyl (ribo) protected phosphoramidite and a 150-fold excess of S-ethyl tetrazole (120 µL of 0.25 M = 30 µmol) can be used in each coupling cycle of ribo residues relative to polymer-bound 5'-hydroxyl. Average coupling yields on the 394 Applied Biosystems, Inc. synthesizer, determined by colorimetric quantitation of the trityl fractions, are typically 97.5-99%. Other oligonucleotide synthesis reagents for the 394 Applied Biosystems, Inc. synthesizer include the following: detritylation solution is 3% TCA in methylene chloride (ABI); capping is performed with 16% *N*-methyl imidazole in THF (ABI) and 10% acetic anhydride/10% 2,6-lutidine in THF (ABI); oxidation solution is 16.9 mM I₂, 49 mM pyridine, 9% water in THF (PERSEPTIVE™). Burdick & Jackson Synthesis Grade acetonitrile is used directly from the reagent bottle. S-Ethyltetrazole solution (0.25 M in acetonitrile) is made up from the solid obtained from American International Chemical, Inc. Alternately, for the introduction of phosphorothioate linkages, Beaucage reagent (3H-1,2-Benzodithiol-3-one 1,1-dioxide0.05 M in acetonitrile) is used.

Deprotection of the RNA is performed using either a two-pot or one-pot protocol. For the two-pot protocol, the polymer-bound trityl-on oligoribonucleotide is transferred to a 4 mL glass screw top vial and suspended in a solution of 40% aq. methylamine (1 mL) at 65 °C for 10 min. After cooling to -20°C, the supernatant is removed from the polymer support. The support is washed three times with 1.0 mL of EtOH:MeCN:H2O/3:1:1, vortexed and the supernatant is then added to the first supernatant. The combined supernatants, containing the oligoribonucleotide, are dried to a white powder. The base deprotected oligoribonucleotide is resuspended in anhydrous TEA/HF/NMP solution (300 µL of a solution of 1.5 mL N-methylpyrrolidinone, 750 µL TEA and 1 mL TEA•3HF to provide a 1.4 M HF concentration) and heated to 65 °C. After 1.5 h, the oligomer is quenched with 1.5 M NH₄HCO₃.

Alternatively, for the one-pot protocol, the polymer-bound trityl-on oligoribonucleotide is transferred to a 4 mL glass screw top vial and suspended in a solution of 33% ethanolic methylamine/DMSO: 1/1 (0.8 mL) at 65 °C for 15 min. The vial is brought to rt. TEA•3BF (0.1 mL) is added and the vial is heated at 65 °C for 15 min. The sample is cooled at-20 °C and then quenched with 1.5 M NH₄HCO₃.

For purification of the trityl-on oligomers, the quenched NH₄HCO₃ solution is loaded onto a C-18 containing cartridge that had been prewashed with acetonitrile followed by 50 mM TEAA. After washing the loaded cartridge with water, the RNA is detritylated with 0.5% TFA for 13 min. The cartridge is then washed again with water, salt exchanged with 1 M NaCl and washed with water again. The oligonucleotide is then eluted with 30% acetonitrile.

The average stepwise coupling yields are typically >98% (Wincott et al., 1995 Nucleic Acids Res. 23, 2677-2684). Those of ordinary skill in the art will recognize that the scale of synthesis can be adapted to be larger or smaller than the example described above including but not limited to 96-well format.

Alternatively, the nucleic acid molecules of the present invention can be synthesized separately and joined together post-synthetically, for example, by ligation (Moore et al., 1992, Science 256, 9923; Draper et al., International PCT publication No. WO 93/23569; Shabarova et al., 1991, Nucleic Acids Research 19, 4247; Bellon et al., 1997, Nucleosides & Nucleotides, 16, 951; Bellon et al., 1997, Bioconjugate Chem. 8, 204), or by hybridization following synthesis and/or deprotection.

The siNA molecules of the invention can also be synthesized via a tandem synthesis methodology as described in Example 1 herein, wherein both siNA strands are synthesized as a single contiguous oligonucleotide fragment or strand separated by a cleavable linker which is subsequently cleaved to provide separate siNA fragments or strands that hybridize and permit purification of the siNA duplex. The linker can be a polynucleotide linker or a non-nucleotide linker. The tandem synthesis of siNA as described herein can be readily adapted to both multiwell/multiplate synthesis platforms such as 96 well or similarly larger multi-well platforms. The tandem synthesis of siNA as described herein can also be readily adapted to large scale synthesis platforms employing batch reactors, synthesis columns and the like.

A siNA molecule can also be assembled from two distinct nucleic acid strands or fragments wherein one fragment includes the sense region and the second fragment includes the antisense region of the RNA molecule.

The nucleic acid molecules of the present invention can be modified extensively to enhance stability by modification with nuclease resistant groups, for example, 2'-amino, 2'-*C*-allyl, 2'-fluoro, 2'-*O*-methyl, 2'-H (for a review see Usman and Cedergren, 1992, TIBS 17, 34; Usman et al., 1994, Nucleic Acids Symp. Ser. 31, 163). siNA constructs can be purified by gel electrophoresis using general methods or can be purified by high pressure liquid chromatography (HPLC; see Wincott *et al., supra*, the totality of which is hereby incorporated herein by reference) and re-suspended in water.

In another aspect of the invention, siNA molecules of the invention are expressed from transcription units inserted into DNA or RNA vectors. The recombinant vectors can be DNA plasmids or viral vectors. siNA expressing viral vectors can be constructed based on, but not limited to, adeno-associated virus, retrovirus, adenovirus, or alphavirus. The recombinant vectors capable of expressing the siNA molecules can be delivered as described herein, and persist in target cells. Alternatively, viral vectors can be used that provide for transient expression of siNA molecules.

### Optimizing Activity of the nucleic acid molecule of the invention.

Chemically synthesizing nucleic acid molecules with modifications (base, sugar and/or phosphate) can prevent their degradation by serum ribonucleases, which can increase their potency (see *e.g.,* Eckstein et al., International Publication No. WO 92/07065; Perrault et al., 1990 Nature 344, 565; Pieken et al., 1991, Science 253, 314; Usman and Cedergren, 1992, Trends in Biochem. Sci. 17, 334; Usman et al., International Publication No. WO 93/15187; and Rossi et al., International Publication No. WO 91/03162; Sproat, U.S. Pat. No. 5,334,711; Gold et al., U.S. Pat. No. 6,300,074; and Burgin *et al., supra;* all of which are incorporated by reference herein). All of the above references describe various chemical modifications that can be made to the base, phosphate and/or sugar moieties of the nucleic acid molecules described herein. Modifications that enhance their efficacy in cells, and removal of bases from nucleic acid molecules to shorten oligonucleotide synthesis times and reduce chemical requirements are desired.

There are several examples in the art describing sugar, base and phosphate modifications that can be introduced into nucleic acid molecules with significant enhancement in their nuclease stability and efficacy. For example, oligonucleotides are modified to enhance stability and/or enhance biological activity by modification with nuclease resistant groups, for example, 2'-amino, 2'-*C*-allyl, 2'-fluoro, 2'-*O*-methyl, 2'-O-allyl, 2'-H, nucleotide base modifications (for a review see Usman and Cedergren, 1992, TIBS. 17, 34; Usman et al., 1994, Nucleic Acids Symp. Ser. 31, 163; Burgin et al., 1996, Biochemistry, 35, 14090). Sugar modification of nucleic acid molecules have been extensively described in the art (see Eckstein et al., International Publication PCT No. WO 92/07065; Perrault et al. Nature, 1990, 344, 565-568; Pieken et al. Science, 1991, 253, 314-317; Usman and Cedergren, Trends in Biochem. Sci. , 1992, 17, 334-339; Usman et al. International Publication PCT No. WO 93/15187; Sproat, U.S. Pat. No. 5,334,711 and Beigelman et al., 1995, J. Biol. Chem., 270, 25702; Beigelman et al., International PCT publication No. WO 97/26270; Beigelman et al., U.S. Pat. No. 5,716,824; Usman et al., U.S. Pat. No. 5,627,053; Woolf et al., International PCT Publication No. WO 98/13526; Thompson et al., USSN 60/082,404 which was filed on April 20, 1998; Karpeisky et al., 1998, Tetrahedron Lett., 39, 1131; Earnshaw and Gait, 1998, Biopolymers (Nucleic Acid Sciences), 48, 39-55; Verma and Eckstein, 1998, Annu. Rev. Biochem., 67, 99-134; and Burlina et al., 1997, Bioorg. Med. Chem., 5, 1999-2010; all of the references are hereby incorporated in their totality by reference herein). Such publications describe general methods and strategies to determine the location of incorporation of sugar, base and/or phosphate modifications and the like into nucleic acid molecules without modulating catalysis, and are incorporated by reference herein. In view of such teachings, similar modifications can be used as described herein to modify the siNA nucleic acid molecules of the instant invention so long as the ability of siNA to promote RNAi is cells is not significantly inhibited.

While chemical modification of oligonucleotide internucleotide linkages with phosphorothioate, phosphorodithioate, and/or 5'-methylphosphonate linkages improves stability, excessive modifications can cause some toxicity or decreased activity. Therefore, when designing nucleic acid molecules, the amount of these internucleotide linkages should be minimized. The reduction in the concentration of these linkages should lower toxicity, resulting in increased efficacy and higher specificity of these molecules.

Short interfering nucleic acid (siNA) molecules having chemical modifications that maintain or enhance activity are provided. Such a nucleic acid is also generally more resistant to nucleases than an unmodified nucleic acid. Accordingly, the *in vitro* and/or *in vivo* activity should not be significantly lowered. In cases in which modulation is the goal, therapeutic nucleic acid molecules delivered exogenously should optimally be stable within cells until translation of the target RNA has been modulated long enough to reduce the levels of the undesirable protein. This period of time varies between hours to days depending upon the disease state. Improvements in the chemical synthesis of RNA and DNA (Wincott et al., 1995, Nucleic Acids Res. 23, 2677; Caruthers et al., 1992, Methods in Enzymology 211,3-19 (incorporated by reference herein)) have expanded the ability to modify nucleic acid molecules by introducing nucleotide modifications to enhance their nuclease stability, as described above.

In one embodiment, nucleic acid molecules of the invention include one or more (e.g., about 1, 2, 3, 4, 5, 6, 7, 8, 9, 10, or more) G-clamp nucleotides. A G-clamp nucleotide is a modified cytosine analog wherein the modifications confer the ability to hydrogen bond both Watson-Crick and Hoogsteen faces of a complementary guanine within a duplex, see for example Lin and Matteucci, 1998, J. Am. Chem. Soc., 120, 8531-8532. A single G-clamp analog substitution within an oligonucleotide can result in substantially enhanced helical thermal stability and mismatch discrimination when hybridized to complementary oligonucleotides. The inclusion of such nucleotides in nucleic acid molecules of the invention results in both enhanced affinity and specificity to nucleic acid targets, complementary sequences, or template strands. In another embodiment, nucleic acid molecules of the invention include one or more (*e*.*g*., about 1, 2, 3, 4, 5, 6, 7, 8, 9, 10, or more) LNA "locked nucleic acid" nucleotides such as a 2', 4'-C methylene bicyclo nucleotide (see for example Wengel et al., International PCT Publication No. WO 00/66604 and WO 99/14226).

In another embodiment, the invention features conjugates and/or complexes of siNA molecules of the invention. Such conjugates and/or complexes can be used to facilitate delivery of siNA molecules into a biological system, such as a cell. The conjugates and complexes provided by the instant invention can impart therapeutic activity by transferring therapeutic compounds across cellular membranes, altering the pharmacokinetics, and/or modulating the localization of nucleic acid molecules of the invention. The present invention encompasses the design and synthesis of novel conjugates and complexes for the delivery of molecules, including, but not limited to, small molecules, lipids, phospholipids, nucleosides, nucleotides, nucleic acids, antibodies, toxins, negatively charged polymers and other polymers, for example proteins, peptides, hormones, carbohydrates, polyethylene glycols, or polyamines, across cellular membranes. In general, the transporters described are designed to be used either individually or as part of a multi-component system, with or without degradable linkers. These compounds are expected to improve delivery and/or localization of nucleic acid molecules of the invention into a number of cell types originating from different tissues, in the presence or absence of serum (see Sullenger and Cech, U.S. Pat. No. 5,854,038). Conjugates of the molecules described herein can be attached to biologically active molecules via linkers that are biodegradable, such as biodegradable nucleic acid linker molecules.

The term "biodegradable linker" as used herein, refers to a nucleic acid or non-nucleic acid linker molecule that is designed as a biodegradable linker to connect one molecule to another molecule, for example, a biologically active molecule to a siNA molecule of the invention or the sense and antisense strands of a siNA molecule of the invention. The biodegradable linker is designed such that its stability can be modulated for a particular purpose, such as delivery to a particular tissue or cell type. The stability of a nucleic acid-based biodegradable linker molecule can be modulated by using various chemistries, for example combinations of ribonucleotides, deoxyribonucleotides, and chemically-modified nucleotides, such as 2'-O-methyl, 2'-fluoro, 2'-amino, 2'-O-amino, 2'-C-allyl, 2'-O-allyl, and other 2'-modified or base modified nucleotides. The biodegradable nucleic acid linker molecule can be a dimer, trimer, tetramer or longer nucleic acid molecule, for example, an oligonucleotide of about 2, 3, 4, 5, 6, 7, 8, 9, 10, 11, 12, 13, 14, 15, 16, 17, 18, 19, or 20 nucleotides in length, or can comprise a single nucleotide with a phosphorus-based linkage, for example, a phosphoramidate or phosphodiester linkage. The biodegradable nucleic acid linker molecule can also comprise nucleic acid backbone, nucleic acid sugar, or nucleic acid base modifications.

The term "biodegradable" as used herein, refers to degradation in a biological system, for example enzymatic degradation or chemical degradation.

The term "biologically active molecule" as used herein, refers to compounds or molecules that are capable of eliciting or modifying a biological response in a system. Non-limiting examples of biologically active siNA molecules either alone or in combination with other molecules contemplated by the instant invention include therapeutically active molecules such as antibodies, hormones, antivirals, peptides, proteins, chemotherapeutics, small molecules, vitamins, co-factors, nucleosides, nucleotides, oligonucleotides, enzymatic nucleic acids, antisense nucleic acids, triplex forming oligonucleotides, 2,5-A chimeras, siNA, dsRNA, allozymes, aptamers, decoys and analogs thereof. Biologically active molecules of the invention also include molecules capable of modulating the pharmacokinetics and/or pharmacodynamics of other biologically active molecules, for example, lipids and polymers such as polyamines, polyamides, polyethylene glycol and other polyethers.

The term "phospholipid" as used herein, refers to a hydrophobic molecule comprising at least one phosphorus group. For example, a phospholipid can comprise a phosphorus-containing group and saturated or unsaturated alkyl group, optionally substituted with OH, COOH, oxo, amine, or substituted or unsubstituted aryl groups.

Therapeutic nucleic acid molecules (*e*.*g*., siNA molecules) delivered exogenously optimally are stable within cells until reverse transcription of the RNA has been modulated long enough to reduce the levels of the RNA transcript. The nucleic acid molecules are resistant to nucleases in order to function as effective intracellular therapeutic agents. Improvements in the chemical synthesis of nucleic acid molecules described in the instant invention and in the art have expanded the ability to modify nucleic acid molecules by introducing nucleotide modifications to enhance their nuclease stability as described above.

In yet another embodiment, siNA molecules having chemical modifications that maintain or enhance enzymatic activity of proteins involved in RNAi are provided. Such nucleic acids are also generally more resistant to nucleases than unmodified nucleic acids. Thus, *in vitro* and/or *in vivo* the activity should not be significantly lowered.

Use of the nucleic acid-based molecules of the invention will lead to better treatment of the disease progression by affording the possibility of combination therapies (*e*.*g*., multiple siNA molecules targeted to different genes; nucleic acid molecules coupled with known small molecule modulators; or intermittent treatment with combinations of molecules, including different motifs and/or other chemical or biological molecules). The treatment of subjects with siNA molecules can also include combinations of different types of nucleic acid molecules, such as enzymatic nucleic acid molecules (ribozymes), allozymes, antisense, 2,5-A oligoadenylate, decoys, and aptamers.

In another aspect a siNA molecule of the invention comprises one or more 5' and/or a 3'- cap structure, for example on only the sense siNA strand, the antisense siNA strand, or both siNA strands.

By "cap structure" is meant chemical modifications, which have been incorporated at either terminus of the oligonucleotide (see, for example, Adamic et al., U.S. Pat. No. 5,998,203, incorporated by reference herein). These terminal modifications protect the nucleic acid molecule from exonuclease degradation, and may help in delivery and/or localization within a cell. The cap may be present at the 5'-terminus (5'-cap) or at the 3'-terminal (3'-cap) or may be present on both termini. In non-limiting examples, the 5'-cap is selected from the group consisting of glyceryl, inverted deoxy abasic residue (moiety); 4',5'-methylene nucleotide; 1-(beta-D-erythrofuranosyl) nucleotide, 4'-thio nucleotide; carbocyclic nucleotide; 1,5-anhydrohexitol nucleotide; L-nucleotides; alpha-nucleotides; modified base nucleotide; phosphorodithioate linkage; *threo*-pentofuranosyl nucleotide; acyclic 3',4'-seco nucleotide; acyclic 3,4-dihydroxybutyl nucleotide; acyclic 3,5-dihydroxypentyl nucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety.

In non-limiting examples, the 3'-cap is selected from the group consisting of glyceryl, inverted deoxy abasic residue (moiety), 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate; 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; *threo*-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inverted abasic moiety; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moieties (for more details see Beaucage and Iyer, 1993, Tetrahedron 49, 1925; incorporated by reference herein).

By the term "non-nucleotide" is meant any group or compound which can be incorporated into a nucleic acid chain in the place of one or more nucleotide units, including either sugar and/or phosphate substitutions, and allows the remaining bases to exhibit their enzymatic activity. The group or compound is abasic in that it does not contain a commonly recognized nucleotide base, such as adenosine, guanine, cytosine, uracil or thymine and therefore lacks a base at the 1'-position.

An "alkyl" group refers to a saturated aliphatic hydrocarbon, including straight-chain, branched-chain, and cyclic alkyl groups. Preferably, the alkyl group has 1 to 12 carbons. More preferably, it is a lower alkyl of from 1 to 7 carbons, more preferably 1 to 4 carbons. The alkyl group can be substituted or unsubstituted. When substituted the substituted group(s) is preferably, hydroxyl, cyano, alkoxy, =O, =S, NO₂ or N(CH₃)₂, amino, or SH. The term also includes alkenyl groups that are unsaturated hydrocarbon groups containing at least one carbon-carbon double bond, including straight-chain, branched-chain, and cyclic groups. Preferably, the alkenyl group has 1 to 12 carbons. More preferably, it is a lower alkenyl of from 1 to 7 carbons, more preferably 1 to 4 carbons. The alkenyl group may be substituted or unsubstituted. When substituted the substituted group(s) is preferably, hydroxyl, cyano, alkoxy, =O, =S, NO₂, halogen, N(CH₃)₂, amino, or SH. The term "alkyl" also includes alkynyl groups that have an unsaturated hydrocarbon group containing at least one carbon-carbon triple bond, including straight-chain, branched-chain, and cyclic groups. Preferably, the alkynyl group has 1 to 12 carbons. More preferably, it is a lower alkynyl of from 1 to 7 carbons, more preferably 1 to 4 carbons. The alkynyl group may be substituted or unsubstituted. When substituted the substituted group(s) is preferably, hydroxyl, cyano, alkoxy, =O, =S, NO₂ or N(CH₃)₂, amino or SH.

Such alkyl groups can also include aryl, alkylaryl, carbocyclic aryl, heterocyclic aryl, amide and ester groups. An "aryl" group refers to an aromatic group that has at least one ring having a conjugated pi electron system and includes carbocyclic aryl, heterocyclic aryl and biaryl groups, all of which may be optionally substituted. The preferred substituent(s) of aryl groups are halogen, trihalomethyl, hydroxyl, SH, OH, cyano, alkoxy, alkyl, alkenyl, alkynyl, and amino groups. An "alkylaryl" group refers to an alkyl group (as described above) covalently joined to an aryl group (as described above). Carbocyclic aryl groups are groups wherein the ring atoms on the aromatic ring are all carbon atoms. The carbon atoms are optionally substituted. Heterocyclic aryl groups are groups having from 1 to 3 heteroatoms as ring atoms in the aromatic ring and the remainder of the ring atoms are carbon atoms. Suitable heteroatoms include oxygen, sulfur, and nitrogen, and include furanyl, thienyl, pyridyl, pyrrolyl, N-lower alkyl pyrrolo, pyrimidyl, pyrazinyl, imidazolyl and the like, all optionally substituted. An "amide" refers to an -C(O)-NH-R, where R is either alkyl, aryl, alkylaryl or hydrogen. An "ester" refers to an -C(O)-OR', where R is either alkyl, aryl, alkylaryl or hydrogen.

By "nucleotide" as used herein is as recognized in the art to include natural bases (standard), and modified bases well known in the art. Such bases are generally located at the 1' position of a nucleotide sugar moiety. Nucleotides generally comprise a base, sugar and a phosphate group. The nucleotides can be unmodified or modified at the sugar, phosphate and/or base moiety, (also referred to interchangeably as nucleotide analogs, modified nucleotides, non-natural nucleotides, non-standard nucleotides and other; see, for example, Usman and McSwiggen, *supra*; Eckstein et al., International PCT Publication No. WO 92/07065; Usman et al., International PCT Publication No. WO 93/15187; Uhlman & Peyman, *supra*, all are hereby incorporated by reference herein). There are several examples of modified nucleic acid bases known in the art as summarized by Limbach et al., 1994, Nucleic Acids Res. 22, 2183. Some of the non-limiting examples of base modifications that can be introduced into nucleic acid molecules include, inosine, purine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidines (*e.g.,* 5-methylcytidine), 5-alkyluridines (*e.g.,* ribothymidine), 5-halouridine (*e.g.,* 5-bromouridine) or 6-azapyrimidines or 6-alkylpyrimidines (*e.g.* 6-methyluridine), propyne, and others (Burgin et al., 1996, Biochemistry, 35, 14090; Uhlman & Peyman, *supra*). By "modified bases" in this aspect is meant nucleotide bases other than adenine, guanine, cytosine and uracil at 1' position or their equivalents.

In one embodiment, the invention features modified siNA molecules, with phosphate backbone modifications comprising one or more phosphorothioate, phosphorodithioate, methylphosphonate, phosphotriester, morpholino, amidate carbamate, carboxymethyl, acetamidate, polyamide, sulfonate, sulfonamide, sulfamate, formacetal, thioformacetal, and/or alkylsilyl, substitutions. For a review of oligonucleotide backbone modifications, see Hunziker and Leumann, 1995, Nucleic Acid Analogues: Synthesis and Properties, in Modern Synthetic Methods, VCH, 331-417, and Mesmaeker et al., 1994, Novel Backbone Replacements for Oligonucleotides, in Carbohydrate Modifications in Antisense Research, ACS, 24-39.

By "abasic" is meant sugar moieties lacking a base or having other chemical groups in place of a base at the 1' position, see for example Adamic et al., U.S. Pat. No. 5,998,203.

By "unmodified nucleoside" is meant one of the bases adenine, cytosine, guanine, thymine, or uracil joined to the 1' carbon of β-D-ribo-furanose.

By "modified nucleoside" is meant any nucleotide base which contains a modification in the chemical structure of an unmodified nucleotide base, sugar and/or phosphate. Non-limiting examples of modified nucleotides are shown by Formulae I-VII and/or other modifications described herein.

In connection with 2'-modified nucleotides as described for the present invention, by "amino" is meant 2'-NH₂ or 2'-*O*- NH₂, which can be modified or unmodified. Such modified groups are described, for example, in Eckstein et al., U.S. Pat. No. 5,672,695 and Matulic-Adamic et al., U.S. Pat. No. 6,248,878, which are both incorporated by reference in their entireties.

Various modifications to nucleic acid siNA structure can be made to enhance the utility of these molecules. Such modifications will enhance shelf-life, half-life *in vitro,* stability, and ease of introduction of such oligonucleotides to the target site, *e.g.,* to enhance penetration of cellular membranes, and confer the ability to recognize and bind to targeted cells.

### Administration of Nucleic Acid Molecules

A siNA molecule of the invention can be adapted for use to treat any disease, infection or condition associated with gene expression, and other indications that can respond to the level of gene product in a cell or tissue, alone or in combination with other therapies. For example, a siNA molecule can comprise a delivery vehicle, including liposomes, for administration to a subject, carriers and diluents and their salts, and/or can be present in pharmaceutically acceptable formulations. Methods for the delivery of nucleic acid molecules are described in Akhtar et al., 1992, Trends Cell Bio., 2, 139; Delivery Strategies for Antisense Oligonucleotide Therapeutics, ed. Akhtar, 1995, Maurer et al., 1999, Mol. Membr. Biol., 16, 129-140; Hofland and Huang, 1999, Handb. Exp. Pharmacol., 137, 165-192; and Lee et al., 2000, ACS Symp. Ser., 752, 184-192, all of which are incorporated herein by reference. Beigelman et al., U.S. Pat. No. 6,395,713 and Sullivan et al., PCT WO 94/02595 further describe the general methods for delivery of nucleic acid molecules. These protocols can be utilized for the delivery of virtually any nucleic acid molecule. Nucleic acid molecules can be administered to cells by a variety of methods known to those of skill in the art, including, but not restricted to, encapsulation in liposomes, by iontophoresis, or by incorporation into other vehicles, such as hydrogels, cyclodextrins (see for example Gonzalez et al., 1999, Bioconjugate Chem., 10, 1068-1074), biodegradable nanocapsules, and bioadhesive microspheres, or by proteinaceous vectors (O'Hare and Normand, International PCT Publication No. WO 00/53722). Alternatively, the nucleic acid/vehicle combination is locally delivered by direct injection or by use of an infusion pump. Direct injection of the nucleic acid molecules of the invention, whether subcutaneous, intramuscular, or intradermal, can take place using standard needle and syringe methodologies, or by needle-free technologies such as those described in Conry et al., 1999, Clin. Cancer Res., 5, 2330-2337 and Barry et al., International PCT Publication No. WO 99/31262. Many examples in the art describe CNS delivery methods of oligonucleotides by osmotic pump, (see Chun et al., 1998, Neuroscience Letters, 257, 135-138, D'Aldin et al., 1998, Mol. Brain Research, 55, 151-164, Dryden et al., 1998, J. Endocrinol., 157, 169-175, Ghirnikar et al., 1998, Neuroscience Letters, 247, 21-24) or direct infusion (Broaddus et al., 1997, Neurosurg. Focus, 3, article 4). Other routes of delivery include, but are not limited to oral (tablet or pill form) and/or intrathecal delivery (Gold, 1997, Neuroscience, 76, 1153-1158). More detailed descriptions of nucleic acid delivery and administration are provided in Sullivan *et al*., supra, Draper et al., PCT WO93/23569, Beigelman et al., PCT WO99/05094, and Klimuk et al., PCT WO99/04819 all of which have been incorporated by reference herein. The molecules of the instant invention can be used as pharmaceutical agents. Pharmaceutical agents prevent, modulate the occurrence, or treat (alleviate a symptom to some extent, preferably all of the symptoms) of a disease state in a subject.

In addition, the invention features the use of methods to deliver the nucleic acid molecules of the instant invention to hematopoietic cells, including monocytes and lymphocytes. These methods are described in detail by Hartmann et al., 1998, J. Phamacol. Exp. Ther., 285(2), 920-928; Kronenwett et al., 1998, Blood, 91(3), 852-862; Filion and Phillips, 1997, Biochim. Biophys. Acta., 1329(2), 345-356; Ma and Wei, 1996, Leuk. Res., 20(11/12), 925-930; and Bongartz et al., 1994, Nucleic Acids Research, 22(22), 4681-8. Such methods, as described above, include the use of free oligonucleitide, cationic lipid formulations, liposome formulations including pH sensitive liposomes and immunoliposomes, and bioconjugates including oligonucleotides conjugated to fusogenic peptides, for the transfection of hematopoietic cells with oligonucleotides.

Thus, the invention features a pharmaceutical composition comprising one or more nucleic acid(s) of the invention in an acceptable carrier, such as a stabilizer, buffer, and the like. The polynucleotides of the invention can be administered (e.g., RNA, DNA or protein) and introduced into a subject by any standard means, with or without stabilizers, buffers, and the like, to form a pharmaceutical composition. When it is desired to use a liposome delivery mechanism, standard protocols for formation of liposomes can be followed. The compositions of the present invention can also be formulated and used as tablets, capsules or elixirs for oral administration, suppositories for rectal administration, sterile solutions, suspensions for injectable administration, and the other compositions known in the art.

The present invention also includes pharmaceutically acceptable formulations of the compounds described. These formulations include salts of the above compounds, *e*.*g*., acid addition salts, for example, salts of hydrochloric, hydrobromic, acetic acid, and benzene sulfonic acid

A pharmacological composition or formulation refers to a composition or formulation in a form suitable for administration, e.g., systemic administration, into a cell or subject, including for example a human. Suitable forms, in part, depend upon the use or the route of entry, for example oral, transdermal, or by injection. Such forms should not prevent the composition or formulation from reaching a target cell (*i*.*e*., a cell to which the negatively charged nucleic acid is desirable for delivery). For example, pharmacological compositions injected into the blood stream should be soluble. Other factors are known in the art, and include considerations such as toxicity and forms that prevent the composition or formulation from exerting its effect.

By "systemic administration" is meant *in vivo* systemic absorption or accumulation of drugs in the blood stream followed by distribution throughout the entire body. Administration routes that lead to systemic absorption include, without limitation: intravenous, subcutaneous, intraperitoneal, inhalation, oral, intrapulmonary and intramuscular. Each of these administration routes exposes the siNA molecules of the invention to an accessible diseased tissue. The rate of entry of a drug into the circulation has been shown to be a function of molecular weight or size. The use of a liposome or other drug carrier comprising the compounds of the instant invention can potentially localize the drug, for example, in certain tissue types, such as the tissues of the reticular endothelial system (RES). A liposome formulation that can facilitate the association of drug with the surface of cells, such as, lymphocytes and macrophages is also useful. This approach can provide enhanced delivery of the drug to target cells by taking advantage of the specificity of macrophage and lymphocyte immune recognition of abnormal cells, such as cells producing excess MDR

By "pharmaceutically acceptable formulation" is meant, a composition or formulation that allows for the effective distribution of the nucleic acid molecules of the instant invention in the physical location most suitable for their desired activity. Non-limiting examples of agents suitable for formulation with the nucleic acid molecules of the instant invention include: P-glycoprotein inhibitors (such as Pluronic P85), which can enhance entry of drugs into the CNS (Jolliet-Riant and Tillement, 1999, Fundam. Clin. Pharmacol., 13, 16-26); biodegradable polymers, such as poly (DL-lactide-coglycolide) microspheres for sustained release delivery after intracerebral implantation (Emerich, DF et al, 1999, Cell Transplant, 8, 47-58) (Alkermes, Inc. Cambridge, MA); and loaded nanoparticles, such as those made of polybutylcyanoacrylate, which can deliver drugs across the blood brain barrier and can alter neuronal uptake mechanisms (Prog Neuropsychopharmacol Biol Psychiatry, 23, 941-949, 1999). Other non-limiting examples of delivery strategies for the nucleic acid molecules of the instant invention include material described in Boado et al., 1998, J. Pharm. Sci., 87, 1308-1315; Tyler et al., 1999, FEBS Lett., 421, 280-284; Pardridge et al., 1995, PNAS USA., 92, 5592-5596; Boado, 1995, Adv. Drug Delivery Rev., 15, 73-107; Aldrian-Herrada et al., 1998, Nucleic Acids Res., 26, 4910-4916; and Tyler et al., 1999, PNAS USA., 96, 7053-7058.

The invention also features the use of the composition comprising surface-modified liposomes containing poly (ethylene glycol) lipids (PEG-modified, or long-circulating liposomes or stealth liposomes). These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al. Chem. Rev. 1995, 95, 2601-2627; Ishiwata et al., Chem. Pharm. Bull. 1995, 43, 1005-1011). Such liposomes have been shown to accumulate selectively in tumors, presumably by extravasation and capture in the neovascularized target tissues (Lasic et al., Science 1995, 267, 1275-1276; Oku et al., 1995, Biochim. Biophys. Acta, 1238, 86-90). The long-circulating liposomes enhance the pharmacokinetics and pharmacodynamics of DNA and RNA, particularly compared to conventional cationic liposomes which are known to accumulate in tissues of the MPS (Liu et al., J. Biol. Chem. 1995, 42, 24864-24870; Choi et al., International PCT Publication No. WO 96/10391; Ansell et al., International PCT Publication No. WO 96/10390; Holland et al., International PCT Publication No. WO 96/10392). Long-circulating liposomes are also likely to protect drugs from nuclease degradation to a greater extent compared to cationic liposomes, based on their ability to avoid accumulation in metabolically aggressive MPS tissues such as the liver and spleen.

The present invention also includes compositions prepared for storage or administration that include a pharmaceutically effective amount of the desired compounds in a pharmaceutically acceptable carrier or diluent. Acceptable carriers or diluents for therapeutic use are well known in the pharmaceutical art, and are described, for example, in Remington's Pharmaceutical Sciences, Mack Publishing Co. (A.R. Gennaro edit. 1985), hereby incorporated by reference herein. For example, preservatives, stabilizers, dyes and flavoring agents can be provided. These include sodium benzoate, sorbic acid and esters of *p*-hydroxybenzoic acid In addition, antioxidants and suspending agents can be used.

A pharmaceutically effective dose is that dose required to prevent, inhibit the occurrence, or treat (alleviate a symptom to some extent, preferably all of the symptoms) of a disease state. The pharmaceutically effective dose depends on the type of disease, the composition used, the route of administration, the type of mammal being treated, the physical characteristics of the specific mammal under consideration, concurrent medication, and other factors that those skilled in the medical arts will recognize. Generally, an amount between 0.1 mg/kg and 100 mg/kg body weight/day of active ingredients is administered dependent upon potency of the negatively charged polymer.

The nucleic acid molecules of the invention and formulations thereof can be administered orally, topically, parenterally, by inhalation or spray, or rectally in dosage unit formulations containing conventional non-toxic pharmaceutically acceptable carriers, adjuvants and/or vehicles. The term parenteral as used herein includes percutaneous, subcutaneous, intravascular (*e*.*g*., intravenous), intramuscular, or intrathecal injection or infusion techniques and the like. In addition, there is provided a pharmaceutical formulation comprising a nucleic acid molecule of the invention and a pharmaceutically acceptable carrier. One or more nucleic acid molecules of the invention can be present in association with one or more non-toxic pharmaceutically acceptable carriers and/or diluents and/or adjuvants, and if desired other active ingredients. The pharmaceutical compositions containing nucleic acid molecules of the invention can be in a form suitable for oral use, for example, as tablets, troches, lozenges, aqueous or oily suspensions, dispersible powders or granules, emulsion, hard or soft capsules, or syrups or elixirs.

Compositions intended for oral use can be prepared according to any method known to the art for the manufacture of pharmaceutical compositions and such compositions can contain one or more such sweetening agents, flavoring agents, coloring agents or preservative agents in order to provide pharmaceutically elegant and palatable preparations. Tablets contain the active ingredient in admixture with non-toxic pharmaceutically acceptable excipients that are suitable for the manufacture of tablets. These excipients can be, for example, inert diluents; such as calcium carbonate, sodium carbonate, lactose, calcium phosphate or sodium phosphate; granulating and disintegrating agents, for example, com starch, or alginic acid; binding agents, for example starch, gelatin or acacia; and lubricating agents, for example magnesium stearate, stearic acid or talc. The tablets can be uncoated or they can be coated by known techniques. In some cases such coatings can be prepared by known techniques to delay disintegration and absorption in the gastrointestinal tract and thereby provide a sustained action over a longer period. For example, a time delay material such as glyceryl monosterate or glyceryl distearate can be employed.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in a mixture with excipients suitable for the manufacture of aqueous suspensions. Such excipients are suspending agents, for example sodium carboxymethylcellulose, methylcellulose, hydropropylmethylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents and flavoring agents can be added to provide palatable oral preparations. These compositions can be preserved by the addition of an anti-oxidant such as ascorbic acid

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, can also be present.

Pharmaceutical compositions of the invention can also be in the form of oil-in-water emulsions. The oily phase can be a vegetable oil or a mineral oil or mixtures of these. Suitable emulsifying agents can be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions can also contain sweetening and flavoring agents.

Syrups and elixirs can be formulated with sweetening agents, for example glycerol, propylene glycol, sorbitol, glucose or sucrose. Such formulations can also contain a demulcent, a preservative and flavoring and coloring agents. The pharmaceutical compositions can be in the form of a sterile injectable aqueous or oleaginous suspension. This suspension can be formulated according to the known art using those suitable dispersing or wetting agents and suspending agents that have been mentioned above. The sterile injectable preparation can also be a sterile injectable solution or suspension in a non-toxic parentally acceptable diluent or solvent, for example as a solution in 1,3-butanediol. Among the acceptable vehicles and solvents that can be employed are water, Ringer's solution and isotonic sodium chloride solution. In addition, sterile, fixed oils are conventionally employed as a solvent or suspending medium. For this purpose, any bland fixed oil can be employed including synthetic mono-or diglycerides. In addition, fatty acids such as oleic acid find use in the preparation of injectables.

The nucleic acid molecules of the invention can also be administered in the form of suppositories, e.g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

Nucleic acid molecules of the invention can be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

Dosage levels of the order of from about 0.1 mg to about 140 mg per kilogram of body weight per day are useful in the treatment of the above-indicated conditions (about 0.5 mg to about 7 g per subject per day). The amount of active ingredient that can be combined with the carrier materials to produce a single dosage form varies depending upon the host treated and the particular mode of administration. Dosage unit forms generally contain between from about 1 mg to about 500 mg of an active ingredient.

It is understood that the specific dose level for any particular subject depends upon a variety of factors including the activity of the specific compound employed, the age, body weight, general health, sex, diet, time of administration, route of administration, and rate of excretion, drug combination and the severity of the particular disease undergoing therapy.

For administration to non-human animals, the composition can also be added to the animal feed or drinking water. It can be convenient to formulate the animal feed and drinking water compositions so that the animal takes in a therapeutically appropriate quantity of the composition along with its diet. It can also be convenient to present the composition as a premix for addition to the feed or drinking water.

The nucleic acid molecules of the present invention can also be administered to a subject in combination with other therapeutic compounds to increase the overall therapeutic effect. The use of multiple compounds to treat an indication can increase the beneficial effects while reducing the presence of side effects.

In one embodiment, the invention comprises compositions suitable for administering nucleic acid molecules of the invention to specific cell types. For example, the asialoglycoprotein receptor (ASGPr) (Wu and Wu, 1987, J. Biol. Chem. 262, 4429-4432) is unique to hepatocytes and binds branched galactose-terminal glycoproteins, such as asialoorosomucoid (ASOR). In another example, the folate receptor is overexpressed in many cancer cells. Binding of such glycoproteins, synthetic glycoconjugates, or folates to the receptor takes place with an affinity that strongly depends on the degree of branching of the oligosaccharide chain, for example, triatennary structures are bound with greater affinity than biatenarry or monoatennary chains (Baenziger and Fiete, 1980, Cell, 22, 611-620; Connolly et al., 1982, J. Biol. Chem., 257, 939-945). Lee and Lee, 1987, Glycoconjugate J., 4, 317-328, obtained this high specificity through the use of N-acetyl-D-galactosamine as the carbohydrate moiety, which has higher affinity for the receptor, compared to galactose. This "clustering effect" has also been described for the binding and uptake of mannosyl-terminating glycoproteins or glycoconjugates (Ponpipom et al., 1981, J. Med. Chem., 24, 1388-1395). The use of galactose, galactosamine, or folate based conjugates to transport exogenous compounds across cell membranes can provide a targeted delivery approach to, for example, the treatment of liver disease, cancers of the liver, or other cancers. The use of bioconjugates can also provide a reduction in the required dose of therapeutic compounds required for treatment. Furthermore, therapeutic bioavialability, pharmacodynamics, and pharmacokinetic parameters can be modulated through the use of nucleic acid bioconjugates of the invention. Non-limiting examples of such bioconjugates are described in Vargeese et al., USSN 101201,394, filed August 13, 2001; and Matulic-Adamic et al., USSN 60/362,016, filed March 6, 2002.

Alternatively, certain siNA molecules of the instant invention can be expressed within cells from eukaryotic promoters (*e.g.,* Izant and Weintraub, 1985, Science, 229, 345; McGarry and Lindquist, 1986, Proc. Natl. Acad. Sci., USA 83, 399; Scanlon et al., 1991, Proc. Natl. Acad. Sci. USA, 88, 10591-5; Kashani-Sabet et al., 1992, Antisense Res. Dev., 2, 3-15; Dropulic et al., 1992, J. Virol., 66, 1432-41; Weerasinghe et al., 1991, J. Virol., 65, 5531-4; Ojwang et al., 1992, Proc. Natl. Acad. Sci. USA, 89, 10802-6; Chen et al., 1992, Nucleic Acids Res., 20, 4581-9; Sarver et al., 1990 Science, 247, 1222-1225; Thompson et al., 1995, Nucleic Acids Res., 23, 2259; Good et al., 1997, Gene Therapy, 4, 45. Those skilled in the art realize that any nucleic acid can be expressed in eukaryotic cells from the appropriate DNA/RNA vector. The activity of such nucleic acids can be augmented by their release from the primary transcript by a enzymatic nucleic acid (Draper et al., PCT WO 93/23569, and Sullivan et al., PCT WO 94/02595; Ohkawa et al., 1992, Nucleic Acids Symp. Ser., 27, 15-6; Taira et al., 1991, Nucleic Acids Res., 19, 5125-30; Ventura et al., 1993, Nucleic Acids Res., 21, 3249-55; Chowrira et al., 1994, J. Biol. Chem., 269, 25856.

In another aspect of the invention, RNA molecules of the present invention can be expressed from transcription units (see for example Couture *et al*., 1996, *TIG*., 12, 510) inserted into DNA or RNA vectors. The recombinant vectors can be DNA plasmids or viral vectors. siNA expressing viral vectors can be constructed based on, but not limited to, adeno-associated virus, retrovirus, adenovirus, or alphavirus. In another embodiment, pol III based constructs are used to express nucleic acid molecules of the invention (see for example Thompson, U.S. Pats. Nos. 5,902,880 and 6,146,886). The recombinant vectors capable of expressing the siNA molecules can be delivered as described above, and persist in target cells. Alternatively, viral vectors can be used that provide for transient expression of nucleic acid molecules. Such vectors can be repeatedly administered as necessary. Once expressed, the siNA molecule interacts with the target mRNA and generates an RNAi response. Delivery of siNA molecule expressing vectors can be systemic, such as by intravenous or intra-muscular administration, by administration to target cells ex-planted from a subject followed by reintroduction into the subject, or by any other means that would allow for introduction into the desired target cell (for a review see Couture *et al*., 1996, *TIG*., 12, 510).

In one aspect the invention features an expression vector comprising a nucleic acid sequence encoding at least one siNA molecule of the instant invention. The expression vector can encode one or both strands of a siNA duplex, or a single self-complementary strand that self hybridizes into a siNA duplex. The nucleic acid sequences encoding the siNA molecules of the instant invention can be operably linked in a manner that allows expression of the siNA molecule (see for example Paul et al., 2002, Nature Biotechnology, 19, 505; Miyagishi and Taira, 2002, Nature Biotechnology, 19, 497; Lee et al., 2002, Nature Biotechnology, 19, 500; and Novina et al., 2002, Nature Medicine, advance online publication doi:10.1038/nm725).

In another aspect, the invention features an expression vector comprising: a) a transcription initiation region (*e.g.,* eukaryotic pol I, II or III initiation region); b) a transcription termination region (*e.g.,* eukaryotic pol I, II or III termination region); and c) a nucleic acid sequence encoding at least one of the siNA molecules of the instant invention; wherein said sequence is operably linked to said initiation region and said termination region, in a manner that allows expression and/or delivery of the siNA molecule. The vector can optionally include an open reading frame (ORF) for a protein operably linked on the 5' side or the 3'-side of the sequence encoding the siNA of the invention; and/or an intron (intervening sequences).

Transcription of the siNA molecule sequences can be driven from a promoter for eukaryotic RNA polymerase I (pol I), RNA polymerase II (pol II), or RNA polymerase III (pol III). Transcripts from pol II or pol III promoters are expressed at high levels in all cells; the levels of a given pol II promoter in a given cell type depends on the nature of the gene regulatory sequences (enhancers, silencers, etc.) present nearby. Prokaryotic RNA polymerase promoters are also used, providing that the prokaryotic RNA polymerase enzyme is expressed in the appropriate cells (Elroy-Stein and Moss, 1990, Proc. Natl. Acad. Sci. U S A, 87, 6743-7; Gao and Huang 1993, Nucleic Acids Res., 21, 2867-72; Lieber et al., 1993, Methods Enzymol., 217, 47-66; Zhou et al., 1990, Mol. Cell. Biol., 10, 4529-37). Several investigators have demonstrated that nucleic acid molecules expressed from such promoters can function in mammalian cells (e.g. Kashani-Sabet et al., 1992, Antisense Res. Dev., 2, 3-15; Ojwang et al., 1992, Proc. Natl. Acad. Sci. U S A, 89, 10802-6; Chen et al., 1992, Nucleic Acids Res., 20, 4581-9; Yu et al., 1993, Proc. Natl. Acad. Sci. U S A, 90, 6340-4; L'Huillier et al., 1992, EMBO J., 11, 4.411-8; Lisziewicz et al., 1993, Proc. Natl. Acad Sci. U. S. A, 90, 8000-4; Thompson et al., 1995, Nucleic Acids Res., 23, 2259; Sullenger & Cech, 1993, Science, 262, 1566). More specifically, transcription units such as the ones derived from genes encoding U6 small nuclear (snRNA), transfer RNA (tRNA) and adenovirus VA RNA are useful in generating high concentrations of desired RNA molecules such as siNA in cells (Thompson *et al., supra*; Couture and Stinchcomb, *1996, supra*; Noonberg et al., 1994, Nucleic Acid Res., 22, 2830; Noonberg et al., U.S. Pat. No. 5,624,803; Good et al., 1997, Gene Ther., 4, 45; Beigelman et al., International PCT Publication No. WO 96/18736. The above siNA transcription units can be incorporated into a variety of vectors for introduction into mammalian cells, including but not restricted to, plasmid DNA vectors, viral DNA vectors (such as adenovirus or adeno-associated virus vectors), or viral RNA vectors (such as retroviral or alphavirus vectors) (for a review see Couture and Stinchcomb, *1996, supra)*.

In another aspect the invention features an expression vector comprising a nucleic acid sequence encoding at least one of the siNA molecules of the invention in a manner that allows expression of that siNA molecule. The expression vector comprises in one embodiment; a) a transcription initiation region; b) a transcription termination region; and c) a nucleic acid sequence encoding at least one strand of the siNA molecule, wherein the sequence is operably linked to the initiation region and the termination region in a manner that allows expression and/or delivery of the siNA molecule.

In another embodiment the expression vector comprises: a) a transcription initiation region; b) a transcription termination region; c) an open reading frame; and d) a nucleic acid sequence encoding at least one strand of a siNA molecule, wherein the sequence is operably linked to the 3'-end of the open reading frame and wherein the sequence is operably linked to the initiation region, the open reading frame and the termination region in a manner that allows expression and/or delivery of the siNA molecule. In yet another embodiment, the expression vector comprises: a) a transcription initiation region; b) a transcription termination region; c) an intron; and d) a nucleic acid sequence encoding at least one siNA molecule, wherein the sequence is operably linked to the initiation region, the intron and the termination region in a manner which allows expression and/or delivery of the nucleic acid molecule.

In another embodiment, the expression vector comprises: a) a transcription initiation region; b) a transcription termination region; c) an intron; d) an open reading frame; and e) a nucleic acid sequence encoding at least one strand of a siNA molecule, wherein the sequence is operably linked to the 3'-end of the open reading frame and wherein the sequence is operably linked to the initiation region, the intron, the open reading frame and the termination region in a manner which allows expression and/or delivery of the siNA molecule.

### Examples:

The following are non-limiting examples showing the selection, isolation, synthesis and activity of nucleic acids of the instant invention.

### Example 1: Tandem synthesis of siNA constructs

Exemplary siNA molecules of the invention are synthesized in tandem using a cleavable linker, for example, a succinyl-based linker. Tandem synthesis as described herein is followed by a one-step purification process that provides RNAi molecules in high yield. This approach is highly amenable to siNA synthesis in support of high throughput RNAi screening, and can be readily adapted to multi-column or multi-well synthesis platforms.

After completing a tandem synthesis of a siNA oligo and its complement in which the 5'-terminal dimethoxytrityl (5'-O-DMT) group remains intact (trityl on synthesis), the oligonucleotides are deprotected as described above. Following deprotection, the siNA sequence strands are allowed to spontaneously hybridize. This hybridization yields a duplex in which one strand has retained the 5'-O-DMT group while the complementary strand comprises a terminal 5'-hydroxyl. The newly formed duplex behaves as a single molecule during routine solid-phase extraction purification (Trityl-On purification) even though only one molecule has a dimethoxytrityl group. Because the strands form a stable duplex, this dimethoxytrityl group (or an equivalent group, such as other trityl groups or other hydrophobic moieties) is all that is required to purify the pair of oligos, for example, by using a C18 cartridge.

Standard phosphoramidite synthesis chemistry is used up to the point of introducing a tandem linker, such as an inverted deoxy abasic succinate or glyceryl succinate linker (see **Figure 1**) or an equivalent cleavable linker. A non-limiting example of linker coupling conditions that can be used includes a hindered base such as diisopropylethylamine (DIPA) and/or DMAP in the presence of an activator reagent such as Bromotripyrrolidinophosphoniumhexaflurorophosphate (PyBrOP). After the linker is coupled, standard synthesis chemistry is utilized to complete synthesis of the second sequence leaving the terminal the 5'-O-DMT intact. Following synthesis, the resulting oligonucleotide is deprotected according to the procedures described herein and quenched with a suitable buffer, for example with 50mM NaOAc or 1.5M NH₄H₂CO₃.

Purification of the siNA duplex can be readily accomplished using solid phase extraction, for example using a Waters C18 SepPak 1g cartridge conditioned with 1 column volume (CV) of acetonitrile, 2 CV H2O, and 2 CV 50mM NaOAc. The sample is loaded and then washed with 1 CV H2O or 50mM NaOAc. Failure sequences are eluted with 1 CV 14% ACN (Aqueous with 50mM NaOAc and 50mM NaCl). The column is then washed, for example with 1 CV H2O followed by on-column detritylation, for example by passing 1 CV of 1% aqueous trifluoroacetic acid (TFA) over the column, then adding a second CV of 1% aqueous TFA to the column and allowing to stand for approximately 10 minutes. The remaining TFA solution is removed and the column washed with H20 followed by 1 CV 1M NaCl and additional H2O. The siNA duplex product is then eluted, for example, using 1 CV 20% aqueous CAN.

**Figure 2** provides an example of MALDI-TOV mass spectrometry analysis of a purified siNA construct in which each peak corresponds to the calculated mass of an individual siNA strand of the siNA duplex. The same purified siNA provides three peaks when analyzed by capillary gel electrophoresis (CGE), one peak presumably corresponding to the duplex siNA, and two peaks presumably corresponding to the separate siNA sequence strands. Ion exchange HPLC analysis of the same siNA contract only shows a single peak. Testing of the purified siNA construct using a luciferase reporter assay described below demonstrated the same RNAi activity compared to siNA constructs generated from separately synthesized oligonucleotide sequence strands.

### Example 2: Serum stability of chemically modified siNA constructs

Chemical modifications were introduced into siNA constructs to determine the stability of these constructs compared to native siNA oligonucleotides (containing two thymidine nucleotide overhangs) in human serum. An investigation of the serum stability of RNA duplexes revealed that siNA constructs consisting of all RNA nucleotides containing two thymidine nucleotide overhangs have a half-life in serum of 15 seconds, whereas chemically modified siNA constructs remained stable in serum for 1 to 3 days depending on the extent of modification. RNAi stability tests were performed by internally labeling one strand (strand 1) of siNA and duplexing with 1.5 X the concentration of the complementary siNA strand (strand 2) (to insure all labeled material was in duplex form). Duplexed siNA constructs were then tested for stability by incubating at a final concentration of 2µM siNA (strand 2 concentration) in 90% mouse or human serum for time-points of 30sec, 1min, 5min, 30min, 90min, 4hrs 10min, 16hrs 24min, and 49hrs. Time points were run on a 15% denaturing polyacrylamide gels and analyzed on a phosphoimager.

Internal labeling was performed via kinase reactions with polynucleotide kinase (PNK) and ³²P-γ-ATP, with addition of radiolabeled phosphate at nucleotide 13 of strand 2, counting in from the 3' side. Ligation of the remaining 8-mer fragments with T4 RNA ligase resulted in the full length, 21-mer, strand 2. Duplexing of RNAi was done by adding appropriate concentrations of the siNA oligonucleotides and heating to 95° C for 5min followed by slow cooling to room temperature. Reactions were performed by adding 100% serum to the siNA duplexes and incubating at 37° C, then removing aliquots at desired time-points. Results of this study are summarized in **Figure 3.** As shown in the Figure 3, chemically modified siNA molecules (e.g., SEQ ID NOs: 925/927, 925/928, 925/929, 925/930, and 925/931) have significantly increased serum stability compared to an siNA construct having all ribonucleotides except a 3'-terminal dithymidine (TT) modification (e.g., SEQ ID NOs: 925/926).

### Example 3: Identification of potential siNA target sites in any RNA sequence

The sequence of an RNA target of interest, such as a viral or human mRNA transcript, is screened for target sites, for example by using a computer folding algorithm. In a non-limiting example, the sequence of a gene or RNA gene transcript derived from a database, such as Genbank, is used to generate siNA targets having complementarity to the target. Such sequences can be obtained from a database, or can be determined experimentally as known in the art. Target sites that are known, for example, those target sites determined to be effective target sites based on studies with other nucleic acid molecules, for example ribozymes or antisense, or those targets known to be associated with a disease or condition such as those sites containing mutations or deletions, can be used to design siNA molecules targeting those sites. Various parameters can be used to determine which sites are the most suitable target sites within the target RNA sequence. These parameters include but are not limited to secondary or tertiary RNA structure, the nucleotide base composition of the target sequence, the degree of homology between various regions of the target sequence, or the relative position of the target sequence within the RNA transcript. Based on these determinations, any number of target sites within the RNA transcript can be chosen to screen siNA molecules for efficacy, for example by using *in vitro* RNA cleavage assays, cell culture, or animal models. In a non-limiting example, anywhere from 1 to 1000 target sites are chosen within the transcript based on the size of the siNA construct to be used. High throughput screening assays can be developed for screening siNA molecules using methods known in the art, such as with multi-well or multi-plate assays or combinatorial/siNA library screening assays to determine efficient reduction in target gene expression.

### Example 4: Selection of siNA molecule target sites in a RNA

The following non-limiting steps can be used to carry out the selection of siNAs targeting a given gene sequence or transcript.

The target sequence is parsed in silico into a list of all fragments or subsequences of a particular length, for example 23 nucleotide fragments, contained within the target sequence. This step is typically carried out using a custom Per1 script, but commercial sequence analysis programs such as Oligo, MacVector, or the GCG Wisconsin Package can be employed as well.

In some instances the siNAs correspond to more than one target sequence; such would be the case for example in targeting different transcripts of the same gene, targeting different transcripts of more than one gene, or for targeting both the human gene and an animal homolog. In this case, a subsequence list of a particular length is generated for each of the targets, and then the lists are compared to find matching sequences in each list. The subsequences are then ranked according to the number of target sequences that contain the given subsequence; the goal is to find subsequences that are present in most or all of the target sequences. Alternately, the ranking can identify subsequences that are unique to a target sequence, such as a mutant target sequence. Such an approach would enable the use of siNA to target specifically the mutant sequence and not effect the expression of the normal sequence.

In some instances the siNA subsequences are absent in one or more sequences while present in the desired target sequence; such would be the case if the siNA targets a gene with a paralogous family member that is to remain untargeted. As in case 2 above, a subsequence list of a particular length is generated for each of the targets, and then the lists are compared to find sequences that are present in the target gene but are absent in the untargeted paralog.

The ranked siNA subsequences can be further analyzed and ranked according to GC content. A preference can be given to sites containing 30-70% GC, with a further preference to sites containing 40-60% GC.

The ranked siNA subsequences can be further analyzed and ranked according to self-folding and internal hairpins. Weaker, internal folds are preferred; strong hairpin structures are to be avoided.

The ranked siNA subsequences can be further analyzed and ranked according to whether they have runs of GGG or CCC in the sequence. GGG (or even more Gs) in either strand can make oligonucleotide synthesis problematic and can potentially interfere with RNAi activity, so it is avoided whenever other appropriately suitable sequences are available. CCC is searched in the target strand because that will place GGG in the antisense strand.

The ranked siNA subsequences can be further analyzed and ranked according to whether they have the dinucleotide UU (uridine dinucleotide) on the 3'-end of the sequence, and/or AA on the 5'-end of the sequence (to yield 3' UU on the antisense sequence). These sequences allow one to design siNA molecules with terminal TT thymidine dinucleotides.

Four or five target sites are chosen from the ranked list of subsequences as described above. For example, in subsequences having 23 nucleotides, the right 21 nucleotides of each chosen 23-mer subsequence are then designed and synthesized for the upper (sense) strand of the siNA duplex, while the reverse complement of the left 21 nucleotides of each chosen 23-mer subsequence are then designed and synthesized for the lower (antisense) strand of the siNA duplex (see Tables I). If terminal TT residues are desired for the sequence (as described in paragraph 7), then the two 3' terminal nucleotides of both the sense and antisense strands are replaced by TT prior to synthesizing the oligos.

The siNA molecules are screened in an in vitro, cell culture or animal model system to identify the most active siNA molecule or the most preferred target site within the target RNA sequence.

In an alternate approach, a pool of siNA constructs specific to a target sequence is used to screen for target sites in cells expressing target RNA, such as human HeLa cells. The general strategy used in this approach is shown in **Figure 21.** A non-limiting example of such as pool is a pool comprising sequences having antisense sequences complementary to the target RNA sequence and sense sequences complementary to the antisense sequences. Cells (e.g., HeLa cells) expressing the target gene are transfected with the pool of siNA constructs and cells that demonstrate a phenotype associated with gene silencing are sorted. The pool of siNA constructs can be chemically modified as described herein and synthesized, for example, in a high throughput manner. The siNA from cells demonstrating a positive phenotypic change (e.g., decreased target mRNA levels or target protein expression), are identified, for example by positional analysis within the assay, and are used to determine the most suitable target site(s) within the target RNA sequence based upon the complementary sequence to the corresponding siNA antisense strand identified in the assay.

### Example 5: RNAi activity of chemically modified siNA constructs

Short interfering nucleic acid (siNA) is emerging as a powerful tool for gene regulation. All-ribose siNA duplexes activate the RNAi pathway but have limited utility as therapeutic compounds due to their nuclease sensitivity and short half-life in serum, as shown in Example 2 above. To develop nuclease-resistant siNA constructs for *in vivo* applications, siNAs that target luciferase mRNA and contain stabilizing chemical modifications were tested for activity in HeLa cells. The sequences for the siNA oligonucleotide sequences used in this study are shown in **Table I.** Modifications included phosphorothioate linkages (P=S), 2'-*O*-methyl nucleotides, or 2'-fluoro (F) nucleotides in one or both siNA strands and various 3'-end stabilization chemistries, including 3'-glyceryl, 3'-inverted abasic, 3'-inverted Thymidine, and/or Thymidine. Active siNA containing stabilizing modifications such as described herein should prove useful for *in vivo* applications.

A luciferase reporter system was utilized to test RNAi activity of chemically modified siNA constructs compared to siNA constructs consisting of all RNA nucleotides containing two thymidine nucleotide overhangs. Sense and antisense siNA strands (20 uM each) were annealed by incubation in buffer (100 mM potassium acetate, 30 mM HEPES-KOH, pH 7.4, 2 mM magnesium acetate) for 1 min. at 90°C followed by 1 hour at 37°C. Plasmids encoding firefly luciferase (pGL2) and renilla luciferase (pRLSV40) were purchased from Promega Biotech.

HeLa S3 cells were grown at 37°C in DMEM with 5% FBS and seeded at 15,300 cells in 100 ul media per well of a 96-well plate 24 hours prior to transfection. For transfection, 4 ul Lipofectamine 2000 (Life Technologies) was added to 96 ul OPTI-MEM, vortexed and incubated at room temperature for 5 minutes. The 100 ul diluted lipid was then added to a microtiter tube containing 5 ul pGL2 (200ng/ul), 5 ul pRLSV40 (8 ng/ul) 6 ul siNA (25 nM or 10 nM final), and 84 ul OPTI-MEM, vortexed briefly and incubated at room temperature for 20 minutes. The transfection mix was then mixed briefly and 50 ul was added to each of three wells that contained HeLa S3 cells in 100 ul media. Cells were incubated for 20 hours after transfection and analyzed for luciferase expression using the Dual luciferase assay according to the manufacturer's instructions (Promega Biotech). The results of this study are summarized in **Figures 4-16**. The sequences of the siNA strands used in this study are shown in Table I and are referred to by RPI# in the figures. Normalized luciferase activity is reported as the ratio of firefly luciferase activity to renilla luciferase activity in the same sample. Error bars represent standard deviation of triplicate transfections. As shown in **Figures 4-16,** the RNAi activity of chemically modified constructs is comparable to that of control siNA constructs, which consist of all ribonucleotides at every position except the 3'-terminus which comprises two thymidine nucleotide overhangs. In some instances, the RNAi activity of the chemically modified constructs is greater than the siNA construct consisting of all ribonucleotides at every position except the 3'-terminus which comprises two thymidine nucleotide overhangs. For example, **Figure 4** shows results obtained from a screen using phosphorothioate modified siNA constructs; the RPI 27654/27659 construct contains phosphorothioate substitutions for every pyrimidine nucleotide in both sequences, the RPI 27657/27662 construct contains 5 terminal 3'-phosphorothioate substitutions in each strand, the RPI 27649/27658 construct contains all phosphorothioate substitutions only in the antisense strand, whereas the RPI 27649/27660 and RPI 27649/27661 constructs have unmodified sense strands and varying degrees of phosphorothioate substitutions in the antisense strand. All of these constructs show significant RNAi activity when compared to a scrambled siNA.

**Figure 5** shows results obtained from a screen using phosphorothioate (RPI 28253/28255 and RPI 28254/28256) and universal base substitutions (RPI 28257/28259 and RPI 28258/28260) compared to the same controls described above. As shown, these modifications show equivalent or better RNAi activity when compared to the control siNA construct.

**Figure 6** shows results obtained from a screen using 2'-O-methyl modified siNA constructs in which the sense strand contains either 10 (RPI 28244/27650) or 5 (RPI 28245/27650) 2'-O-methyl substitutions, both with comparable activity to the control siNA construct.

**Figure 7** shows results obtained from a screen using 2'-O-methyl or 2'-deoxy-2'-fluoro modified siNA constructs compared to a control construct consisting of all ribonucleotides at every position except the 3'-terminus which comprises two thymidine nucleotide overhangs.

**Figure 8** compares a siNA construct containing six phosphorothioate substitutions in each strand (RPI 28460/28461), where 5 phosphorothioates are present at the 3' end and a single phosphorothioate is present at the 5' end of each strand. This motif shows very similar activity to the control siNA construct consisting of all ribonucleotides at every position except the 3'-terminus which comprises two thymidine nucleotide overhangs.

**Figure 9** compares a siNA construct synthesized by the method of the invention described in Example 1, wherein an inverted deoxyabasic succinate linker was used to generate a siNA having a 3'-inverted deoxyabasic cap on the antisense strand of the siNA. This construct shows improved activity compared to the control siNA (siGL2) construct consisting of all ribonucleotides at every position except the 3'-terminus which comprises two thymidine nucleotide overhangs.

**Figure 10** shows the results of an RNAi activity screen of chemically modifed siNA constructs including 3'-glyceryl modified siNA constructs compared to an all RNA control siNA construct using a luciferase reporter system. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I. As shown in the Figure, the 3'-terminal modified siNA constructs retain significant RNAi activity compared to the control siNA (siGL2) construct.

**Figure 11** shows the results of an RNAi activity screen of chemically modifed siNA constructs. The screen compared various combinations of sense strand chemical modifications and antisense strand chemical modifications. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table L As shown in the figure, the chemically modified RPI 30063/30430, RPI 30433/30430, and RPI 30063/30224 constructs retain significant RNAi activity compared to the control siNA construct. It should be noted that RPI 30433/30430 is a siNA construct having no ribonucleotides which retains significant RNAi activity compared to the constrol siGL2 construct in vitro, therefore, this construct is expected to have both similar RNAi activity and improved stability compared to siNA constructs having ribonucleotides in vivo.

**Figure 12** shows the results of an RNAi activity screen of chemically modifed siNA constructs. The screen compared various combinations of sense strand chemical modifications and antisense strand chemical modifications. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I. As shown in the figure, the chemically modified RPI 30063/30224 and RPI 30063/30430 constructs retain significant RNAi activity compared to the control siNA (siGL2) construct. In addition, the antisense strand alone (RPI 30430) and an inverted control (RPI 30227/30229, having matched chemistry to RPI 30063/30224) were compared to the siNA duplexes described above. The antisense strand (RPI 30430) alone provides far less inhibition compared to the siNA duplexes using this sequence.

**Figure 13** shows the results of an RNAi activity screen of chemically modifed siNA constructs. The screen compared various combinations of sense strand chemical modifications and antisense strand chemical modifications. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I. In addition, an inverted control (RPI 30226/30229, having matched chemistry to RPI 30222/30224) was compared to the siNA duplexes described above. As shown in the figure, the chemically modified RPI 28251/30430, RPI 28251/30224, and RPI 30222/30224 constructs retain significant RNAi activity compared to the control siNA construct, and the chemically modified RPI 28251/30430 construct demonstrates improved activity compared to the control siNA (siGL2) construct.

**Figure 14** shows the results of an RNAi activity screen of chemically modifed siNA constructs including various 3'-terminal modified siNA constructs compared to an all RNA control siNA construct using a luciferase reporter system. These chemically modified siNAs were compared in the luciferase assay described herein at 1 nM and 10nM concentration using an all RNA siNA control (siGL2) having having 3'-terminal dithymidine (TT) and its corresponding inverted contxol (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I. As shown in the figure, the chemically modified RPI 30222/30546, 30222/30224, 30222/30551, 30222/30557 and 30222/30558 constructs retain significant RNAi activity compared to the control siNA construct.

**Figure 15** shows the results of an RNAi activity screen of chemically modifed siNA constructs. The screen compared various combinations of sense strand chemistries compared to a fixed antisense strand chemistry. These chemically modified siNAs were compared in the luciferase assay described herein at 1 nM and 10nM concentration using an all RNA siNA control (siGL2) having having 3'-terminal dithymidine (TT) and its corresponding inverted control (Inv siGL2). The background level of luciferase expression in the HeLa cells is designated by the "cells" column. Sense and antisense strands of chemically modified siNA constructs are shown by RPI number (sense strand/antisense strand). Sequences correspoding to these RPI numbers are shown in Table I. As shown in the figure, the chemically modified RPI 30063/30430, 30434/30430, and 30435/30430 constructs all demonstrate greater activity compared to the control siNA (siGL2) construct.

### Example 6: RNAi activity titration

A titration assay was performed to determine the lower range of siNA concentration required for RNAi activity both in a control siNA construct consisting of all RNA nucleotides containing two thymidine nucleotide overhangs and a chemically modified siNA construct comprising 5 phosphorothioate internucleotide linkages in both the sense and antisense strands. The assay was performed as described above, however, the siNA constructs were diluted to final concentrations between 2.5 nM and 0.025 nM. Results are shown in **Figure 16.** As shown in **Figure 16,** the chemically modified siNA construct shows a very similar concentration dependent RNAi activity profile to the control siNA construct when compared to an inverted siNA sequence control.

### Example 7: siNA design

siNA target sites were chosen by analyzing sequences of the target RNA and optionally prioritizing the target sites on the basis of folding (structure of any given sequence analyzed to determine siNA accessibility to the target), by using a library of siNA molecules as described in Example 4, or alternately by using an *in vitro* siNA system as described in Example 9 herein. siNA molecules were designed that could bind each target and are optionally individually analyzed by computer folding to assess whether the siNA molecule can interact with the target sequence. Varying the length of the siNA molecules can be chosen to optimize activity. Generally, a sufficient number of complementary nucleotide bases are chosen to bind to, or otherwise interact with, the target RNA, but the degree of complementarity can be modulated to accommodate siNA duplexes or varying length or base composition. By using such methodologies, siNA molecules can be designed to target sites within any known RNA sequence, for example those RNA sequences corresponding to the any gene transcript.

Chemically modified siNA constructs are designed to provide nuclease stability for systemic administration in vivo and/or improved pharmacokinetic, localization, and delivery properties while preserving the ability to mediate RNAi activity. Chemical modifications as described herein are introduced synthetically using synthetic methods described herein and those generally known in the art. The synthetic siNA constructs are then assayed for nuclease stability in serum and/or cellular/tissue extracts (e.g. liver extracts). The synthetic siNA constructs are also tested in parallel for RNAi activity using an appropriate assay, such as a luciferase reporter assay as described herein or another suitable assay that can quantity RNAi activity. Synthetic siNA constructs that possess both nuclease stability and RNAi activity can be further modified and reevaluated in stability and activity assays. The chemical modifications of the stabilized active siNA constructs can then be applied to any siNA sequence targeting any chosen RNA and used, for example, in target screening assays to pick lead siNA compounds for therapeutic development (see for example **Figure 24**).

### Example 8: Chemical Synthesis and Purification of siNA

siNA molecules can be designed to interact with various sites in the RNA message, for example, target sequences within the RNA sequences described herein. The sequence of one strand of the siNA molecule(s) is complementary to the target site sequences described above. The siNA molecules can be chemically synthesized using methods described herein. Inactive siNA molecules that are used as control sequences can be synthesized by scrambling the sequence of the siNA molecules such that it is not complementary to the target sequence. Generally, siNA constructs can by synthesized using solid phase oligonucleotide synthesis methods as described herein (see for example Usman et al., US Patent Nos. 5,804,683; 5,831,071; 5,998,203; 6,117,657; 6,353,098; 6,362,323; 6,437,117; 6,469,158; Scaringe et al., US Patent Nos. 6,111,086; 6,008,400; 6,111,086 all incorporated by reference herein in their entirety).

In a non-limiting example, RNA oligonucleotides are synthesized in a stepwise fashion using the phosphoramidite chemistry as is known in the art. Standard phosphoramidite chemistry involves the use of nucleosides comprising any of 5'-O-dimethoxytrityl, 2'-O-tert-butyldimethylsilyl, 3'-O-2-Cyanoethyl N,N-diisopropylphosphoroamidite groups, and exocyclic amine protecting groups (e.g. N6-benzoyl adenosine, N4 acetyl cytidine, and N2-isobutyryl guanosine). Alternately, 2'-O-Silyl Ethers can be used in conjunction with acid-labile 2'-O-orthoester protecting groups in the synthesis of RNA as described by Scaringe *supra*. Differing 2' chemistries can require different protecting groups, for example 2'-deoxy-2'-amino nucleosides can utilize N-phthaloyl protection as described by Usman et al., US Patent 5,631,360, incorporated by reference herein in its entirety).

During solid phase synthesis, each nucleotide is added sequentially (3'- to 5'-direction) to the solid support-bound oligonucleotide. The first nucleoside at the 3'-end of the chain is covalently attached to a solid support (e.g., controlled pore glass or polystyrene) using various linkers. The nucleotide precursor, a ribonucleoside phosphoramidite, and activator are combined resulting in the coupling of the second nucleoside phosphoramidite onto the 5'-end of the first nucleoside. The support is then washed and any unreacted 5'-hydroxyl groups are capped with a capping reagent such as acetic anhydride to yield inactive 5'-acetyl moieties. The trivalent phosphorus linkage is then oxidized to a more stable phosphate linkage. At the end of the nucleotide addition cycle, the 5'-O-protecting group is cleaved under suitable conditions (e.g., acidic conditions for trityl-based groups and Fluoride for silyl-based groups). The cycle is repeated for each subsequent nucleotide.

Modification of synthesis conditions can be used to optimize coupling efficiency, for example by using differing coupling times, differing reagent/phosphoramidite concentrations, differing contact times, differing solid supports and solid support linker chemistries depending on the particular chemical composition of the siNA to be synthesized. Deprotection and purification of the siNA can be performed as is generally described in Usman et al., US 5,831,071, US 6,353,098, US 6,437,117, and Bellon et al., US 6,054,576, US 6,162,909, US 6,303,773, incorporated by reference herein in their entirety or Scaringe *supra*,. Additionally, deprotection conditions can be modified to provide the best possible yield and purity of siNA constructs. For example, applicant has observed that oligonucleotides comprising 2'-deoxy-2'-fluoro nucleotides can degrade under inappropriate deprotection conditions. Such oligonucleotides are deprotected using aqueous methylamine at about 35°C for 30 minutes. If the 2'-deoxy-2'-fluoro containing oligonucleotide also comprises ribonucleotides, after deprotection with aqueous methylamine at about 35°C for 30 minutes, TEA-HF is added and the reaction maintained at about 65°C for an additional 15 minutes.

### Example 9: RNAi in vitro assay to assess siNA activity

An in vitro assay that recapitulates RNAi in a cell free system is used to evaluate siNA constructs specific to target RNA. The assay comprises the system described by Tuschl et al., 1999, Genes and Development, 13, 3191-3197 and Zamore et al., 2000, Cell, 101, 25-33 adapted for use with target RNA. A Drosophila extract derived from syncytial blastoderm is used to reconstitute RNAi activity *in vitro*. Target RNA is generated via *in vitro* transcription from an appropriate plasmid using T7 RNA polymerase or via chemical synthesis as described herein. Sense and antisense siNA strands (for example 20 uM each) are annealed by incubation in buffer (such as 100 mM potassium acetate, 30 mM HEPES-KOH, pH 7.4, 2 mM magnesium acetate) for 1 min. at 90°C followed by 1 hour at 37°C , then diluted in lysis buffer (for example 100 mM potassium acetate, 30 mM HEPES-KOH at pH 7.4, 2mM magnesium acetate). Annealing can be monitored by gel electrophoresis on an agarose gel in TBE buffer and stained with ethidium bromide. The Drosophila lysate is prepared using zero to two-hour-old embryos from Oregon R flies collected on yeasted molasses agar that are dechorionated and lysed. The lysate is centrifuged and the supernatant isolated. The assay comprises a reaction mixture containing 50% lysate [vol/vol], RNA (10-50 pM final concentration), and 10% [vol/vol] lysis buffer containing siNA (10 nM final concentration). The reaction mixture also contains 10 mM creatine phosphate, 10 ug.ml creatine phosphokinase, 100 um GTP, 100 uM UTP, 100 uM CTP, 500 uM ATP, 5 mM DTT, 0.1 U/uL RNasin (Promega), and 100 uM of each amino acid. The final concentration of potassium acetate is adjusted to 100 mM. The reactions are pre-assembled on ice and preincubated at 25° C for 10 minutes before adding RNA, then incubated at 25° C for an additional 60 minutes. Reactions are quenched with 4 volumes of 1.25 x Passive Lysis Buffer (Promega). Target RNA cleavage is assayed by RT-PCR analysis or other methods known in the art and are compared to control reactions in which siNA is omitted from the reaction.

Alternately, internally-labeled target RNA for the assay is prepared by *in vitro* transcription in the presence of [alpha-³²p] CTP, passed over a G 50 Sephadex column by spin chromatography and used as target RNA without further purification. Optionally, target RNA is 5'-³²P-end labeled using T4 polynucleotide kinase enzyme. Assays are performed as described above and target RNA and the specific RNA cleavage products generated by RNAi are visualized on an autoradiograph of a gel. The percentage of cleavage is determined by Phosphor Imager® quantitation of bands representing intact control RNA or RNA from control reactions without siNA and the cleavage products generated by the assay.

In one embodiment, this assay is used to determine target sites the RNA target for siNA mediated RNAi cleavage, wherein a plurality of siNA constructs are screened for RNAi mediated cleavage of the RNA target, for example, by analyzing the assay reaction by electrophoresis of labeled target RNA, or by northern blotting, as well as by other methodology well known in the art.

### Example 10: Nucleic acid inhibition of target RNA in vivo

siNA molecules targeted to the target RNA are designed and synthesized as described above. These nucleic acid molecules can be tested for cleavage activity *in vivo,* for example, using the following procedure.

Two formats are used to test the efficacy of siNAs targeting a particular gene transcipt. First, the reagents are tested on target expressing cells (e.g., HeLa), to determine the extent of RNA and protein inhibition. siNA reagents are selected against the RNA target. RNA inhibition is measured after delivery of these reagents by a suitable transfection agent to cells. Relative amounts of target RNA are measured versus actin using real-time PCR monitoring of amplification (*eg*., ABI 7700 Taqman®). A comparison is made to a mixture of oligonucleotide sequences made to unrelated targets or to a randomized siNA control with the same overall length and chemistry, but randomly substituted at each position. Primary and secondary lead reagents are chosen for the target and optimization performed. After an optimal transfection agent concentration is chosen, a RNA time-course of inhibition is performed with the lead siNA molecule. In addition, a cell-plating format can be used to determine RNA inhibition.

### Delivery of siNA to Cells

Cells (e.g., HeLa) are seeded, for example, at 1x10⁵ cells per well of a six-well dish in EGM-2 (BioWhittaker) the day before transfection. siNA (final concentration, for example 20nM) and cationic lipid (e.g., final concentration 2µg/ml) are complexed in EGM basal media (Biowhittaker) at 37°C for 30 mins in polystyrene tubes. Following vortexing, the complexed siNA is added to each well and incubated for the times indicated. For initial optimization experiments, cells are seeded, for example, at 1x10³ in 96 well plates and siNA complex added as described. Efficiency of delivery of siNA to cells is determined using a fluorescent siNA complexed with lipid. Cells in 6-well dishes are incubated with siNA for 24 hours, rinsed with PBS and fixed in 2% paraformaldehyde for 15 minutes at room temperature. Uptake of siNA is visualized using a fluorescent microscope.

### Taqman and Lightcycler quantification of mRNA

Total RNA is prepared from cells following siNA delivery, for example, using Qiagen RNA purification kits for 6-well or Rneasy extraction kits for 96-well assays. For Taqman analysis, dual-labeled probes are synthesized with the reporter dye, FAM or JOE, covalently linked at the 5'-end and the quencher dye TAMRA conjugated to the 3'-end. One-step RT-PCR amplifications are performed on, for example, an ABI PRISM 7700 Sequence Detector using 50 µl reactions consisting of 10 µl total RNA, 100 nM forward primer, 900 nM reverse primer, 100 nM probe, 1X TaqMan PCR reaction buffer (PE-Applied Biosystems), 5.5 mM MgCl₂, 300 µM each dATP, dCTP, dGTP, and dTTP, 10U RNase Inhibitor (Promega), 1.25U AmpliTaq Gold (PE-Applied Biosystems) and 10U M-MLV Reverse Transcriptase (Promega). The thermal cycling conditions can consist of 30 min at 48°C, 10 min at 95°C, followed by 40 cycles of 15 sec at 95°C and 1 min at 60°C. Quantitation of mRNA levels is determined relative to standards generated from serially diluted total cellular RNA (300, 100, 33, 11 ng/rxn) and normalizing to β-actin or GAPDH mRNA in parallel TaqMan reactions. For each gene of interest an upper and lower primer and a fluorescently labeled probe are designed. Real time incorporation of SYBR Green I dye into a specific PCR product can be measured in glass capillary tubes using a lightcyler. A standard curve is generated for each primer pair using control cRNA. Values are represented as relative expression to GAPDH in each sample.

### Western blotting

Nuclear extracts can be prepared using a standard micro preparation technique (see for example Andrews and Faller, 1991, Nucleic Acids Research, 19, 2499). Protein extracts from supernatants are prepared, for example using TCA precipitation. An equal volume of 20% TCA is added to the cell supernatant, incubated on ice for 1 hour and pelleted by centrifugation for 5 minutes. Pellets are washed in acetone, dried and resuspended in water. Cellular protein extracts are run on a 10% Bis-Tris NuPage (nuclear extracts) or 4-12% Tris-Glycine (supernatant extracts) polyacrylamide gel and transferred onto nitro-cellulose membranes. Non-specific binding can be blocked by incubation, for example, with 5% non-fat milk for 1 hour followed by primary antibody for 16 hour at 4°C. Following washes, the secondary antibody is applied, for example (1:10,000 dilution) for 1 hour at room temperature and the signal detected with SuperSignal reagent (Pierce).

### Example 11: Animal Models

Various animal models can be used to screen siNA constructs *in vivo* as are known in the art, for example those animal models that are used to evaluate other nucleic acid technologies such as enzymatic nucleic acid molecules (ribozymes) and/or antisense. Such animal models are used to test the efficacy of siNA molecules described herein. In a non-limiting example, siNA molecules that are designed as anti-angiogenic agents can be screened animal models. There are several animal models in which the anti-angiogenesis effect of nucleic acids of the present invention, such as siNA, directed against genes associated with angiogenesis and/or metastais, such as VEGFR (e.g., VEGFR1, VEGFR2, and VEGFR3) genes. Typically a corneal model has been used to study angiogenesis in rat and rabbit since recruitment of vessels can easily be followed in this normally avascular tissue (Pandey et al., 1995 Science 268: 567-569). In these models, a small Teflon or Hydron disk pretreated with an angiogenesis factor (e.g. bFGF or VEGF) is inserted into a pocket surgically created in the cornea. Angiogenesis is monitored 3 to 5 days later. siNA molecules directed against VEGFR mRNAs are delivered in the disk as well, or dropwise to the eye over the time course of the experiment. In another eye model, hypoxia has been shown to cause both increased expression of VEGF and neovascularization in the retina (Pierce et al., 1995 Proc. Natl. Acad. Sci. USA. 92: 905-909; Shweiki et al., 1992 J. Clin. Invest. 91: 2235-2243).

Several animal models exist for screening of anti-angiogenic agents. These include corneal vessel formation following corneal injury (Burger et al., 1985 Cornea 4: 35-41; Lepri, et al., 1994 J. Ocular Pharmacol. 10: 273-280; Ormerod et al., 1990 Am. J. Pathol. 137: 1243-1252) or intracomeal growth factor implant (Grant et al., 1993 Diabetologia 36: 282-291; Pandey *et al*. 1995 *supra;* Zieche et al., 1992 Lab. Invest. 67: 711-715), vessel growth into Matrigel matrix containing growth factors (Passaniti *et al.,* 1992 *supra*), female reproductive organ neovascularization following hormonal manipulation (Shweiki et al., 1993 Clin. Invest. 91: 2235-2243), several models involving inhibition of tumor growth in highly vascularized solid tumors (O'Reilly et al., 1994 Cell 79: 315-328; Senger et al., 1993 Cancer and Metas. Rev. 12: 303-324; Takahasi et al., 1994 Cancer Res. 54: 4233-4237; Kim *et al.,* 1993 *supra*), and transient hypoxia-induced neovascularization in the mouse retina (Pierce et al., 1995 Proc. Natl. Acad. Sci. USA. 92: 905-909).gene

The cornea model, described in Pandey et al. *supra*, is the most common and well characterized anti-angiogenic agent efficacy screening model. This model involves an avascular tissue into which vessels are recruited by a stimulating agent (growth factor, thermal or alkalai burn, endotoxin). The corneal model would utilize the intrastromal corneal implantation of a Teflon pellet soaked in a VEGF-Hydron solution to recruit blood vessels toward the pellet which can be quantitated using standard microscopic and image analysis techniques. To evaluate their anti-angiogenic efficacy, ribozymes are applied topically to the eye or bound within Hydron on the Teflon pellet itself. This avascular cornea as well as the Matrigel model provide for low background assays. While the corneal model has been performed extensively in the rabbit, studies in the rat have also been conducted.

The mouse model (Passaniti et al., *supra*) is a non-tissue model which utilizes Matrigel, an extract of basement membrane (Kleinman et al., 1986) or Millipore® filter disk, which can be impregnated with growth factors and anti-angiogenic agents in a liquid form prior to injection. Upon subcutaneous administration at body temperature, the Matrigel or Millipore® filter disk forms a solid implant. VEGF embedded in the Matrigel or Millipore® filter disk is used to recruit vessels within the matrix of the Matrigel or Millipore® filter disk which can be processed histologically for endothelial cell specific vWF (factor VIII antigen) immunohistochemistry, Trichrome-Masson stain, or hemoglobin content. Like the cornea, the Matrigel or Millipore® filter disk are avascular; however, it is not tissue. In the Matrigel or Millipore® filter disk model, siNA molecules are administered within the matrix of the Matrigel or Millipore® filter disk to test their anti-angiogenic efficacy. Thus, delivery issues in this model, as with delivery of siNA molecules by Hydron- coated Teflon pellets in the rat cornea model, may be less problematic due to the homogeneous presence of the siNA within the respective matrix.

The Lewis lung carcinoma and B-16 murine melanoma models are well accepted models of primary and metastatic cancer and are used for initial screening of anti-cancer agents. These murine models are not dependent upon the use of immunodeficient mice, are relatively inexpensive, and minimize housing concerns. Both the Lewis lung and B-16 melanoma models involve subcutaneous implantation of approximately 10⁶ tumor cells from metastatically aggressive tumor cell lines (Lewis lung lines 3LL or D122, LLc-LN7; B-16-BL6 melanoma) in C57BL/6J mice. Alternatively, the Lewis lung model can be produced by the surgical implantation of tumor spheres (approximately 0.8 mm in diameter). Metastasis also may be modeled by injecting the tumor cells directly *i.v.*. In the Lewis lung model, microscopic metastases can be observed approximately 14 days following implantation with quantifiable macroscopic metastatic tumors developing within 21-25 days. The B-16 melanoma exhibits a similar time course with tumor neovascularization beginning 4 days following implantation. Since both primary and metastatic tumors exist in these models after 21-25 days in the same animal, multiple measurements can be taken as indices of efficacy. Primary tumor volume and growth latency as well as the number of micro- and macroscopic metastatic lung foci or number of animals exhibiting metastases can be quantitated. The percent increase in lifespan can also be measured. Thus, these models provide suitable primary efficacy assays for screening systemically administered siNA molecules and siNA formulations.

In the Lewis lung and B-16 melanoma models, systemic pharmacotherapy with a wide variety of agents usually begins 1-7 days following tumor implantation/inoculation with either continuous or multiple administration regimens. Concurrent pharmacokinetic studies can be performed to determine whether sufficient tissue levels of siNA can be achieved for pharmacodynamic effect to be expected. Furthermore, primary tumors and secondary lung metastases can be removed and subjected to a variety of *in vitro* studies (*i.e.* target RNA reduction).

In utilizing these models to assess siNA activity, VEGFR1, VEGFR2, and/or VEGFR3 protein levels can be measured clinically or experimentally by FACS analysis. VEGFR1, VEGFR2, and/or VEGFR3 encoded mRNA levels will be assessed by Northern analysis, RNase-protection, primer extension analysis and/or quantitative RT-PCR. siNA molecules that block VEGFR1, VEGFR2, and/or VEGFR3 protein encoding mRNAs and therefore result in decreased levels of VEGFR1, VEGFR2, and/or VEGFR3 activity by more than 20% *in vitro* can be thus identified.

### Example 12: siNA-mediated inhibition of angiogenesis in vivo

The purpose of this study was to assess the anti-angiogenic activity of siNA targeted against VEGFR1 in the rat cornea model of VEGF induced angiogenesis (see above). These siNA molecules have matched inverted controls which are inactive since they are not able to interact with the RNA target. The siNA molecules and VEGF were co-delivered using the filter disk method: Nitrocellulose filter disks (Millipore® ) of 0.057 diameter were immersed in appropriate solutions and were surgically implanted in rat cornea as described by Pandey *et al., supra*.

The stimulus for angiogenesis in this study was the treatment of the filter disk with 30 µM VEGF which is implanted within the cornea's stroma. This dose yields reproducible neovascularization stemming from the pericorneal vascular plexus growing toward the disk in a dose-response study 5 days following implant. Filter disks treated only with the vehicle for VEGF show no angiogenic response. The siNA were co-adminstered with VEGF on a disk in two different siNA concentrations. One concern with the simultaneous administration is that the siNA would not be able to inhibit angiogenesis since VEGF receptors can be stimulated. However, Applicant has observed that in low VEGF doses, the neovascular response reverts to normal, suggesting that the VEGF stimulus is essential for maintaining the angiogenic response. Blocking the production of VEGF receptors using simultaneous administration of anti-VEGF-R mRNA siNA could attenuate the normal neovascularization induced by the filter disk treated with VEGF.

### Materials and Methods:

### Test Compounds and Controls

R&D Systems VEGF, carrier free at 75 µM in 82 mM Tris-Cl, pH 6.9
siNA, 1.67 µG/µL, SITE 2340 (SEQ ID NO: 2; SEQ ID NO: 6) sense/antisense
siNA, 1.67 µG/µL, INVERTED CONTROL FOR SITE 2340 (SEQ ID NO: 19; SEQ ID NO: 20) sense/antisense
siNA 1.67 µg/µL, Site 2340 (SEQ ID NO: 419; SEQ ID NO: 420) sense/antisense *Animals*
Harlan Sprague-Dawley Rats, Approximately 225-250g
45 males, 5 animals per group.

### Husbandry

Animals are housed in groups of two. Feed, water, temperature and humidity are determined according to Pharmacology Testing Facility performance standards (SOP's) which are in accordance with the 1996 Guide for the Care and Use of Laboratory Animals (NRC). Animals are acclimated to the facility for at least 7 days prior to experimentation. During this time, animals are observed for overall health and sentinels will be bled for baseline serology.

### Experimental Groups

Each solution (VEGF and siNAs) was prepared as a **1X** solution for final concentrations shown in the experimental groups described in **Table III.**

### siNA Annealing Conditions

siNA sense and antisense strands are annealed for 1 minute in H₂O at 1.67mg/mL/strand followed by a 1 hour incubation at 37°C producing 3.34 mg/mL of duplexed siNA. For the 20µg/eye treatment, 6 µLs of the 3.34 mg/mL duplex is injected into the eye (see below). The 3.34 mg/mL duplex siNA can then be serially diluted for dose response assays.

### Preparation of VEGF Filter Disk

For corneal implantation, 0.57 mm diameter nitrocellulose disks, prepared from 0.45 µm pore diameter nitrocellulose filter membranes (Millipore Corporation), were soaked for 30 min in 1 µL of 75 µM VEGF in 82 mM Tris HCl (pH 6.9) in covered petri dishes on ice. Filter disks soaked only with the vehicle for VEGF (83 mM Tris-Cl pH 6.9) elicit no angiogenic response.

### Corneal surgery

The rat corneal model used in this study was a modified from Koch *et al. Supra* and Pandey *et al., supra*. Briefly, corneas were irrigated with 0.5% povidone iodine solution followed by normal saline and two drops of 2% lidocaine. Under a dissecting microscope (Leica MZ-6), a stromal pocket was created and a presoaked filter disk (see above) was inserted into the pocket such that its edge was 1 mm from the corneal limbus.

### Intraconjunctival injection of test solutions

Immediately after disk insertion, the tip of a 40-50 µm OD injector (constructed in our laboratory) was inserted within the conjunctival tissue 1 mm away from the edge of the corneal limbus that was directly adjacent to the VEGF-soaked filter disk. Six hundred nanoliters of test solution (siNA, inverted control or sterile water vehicle) were dispensed at a rate of 1.2 µL/min using a syringe pump (Kd Scientific). The injector was then removed, serially rinsed in 70% ethanol and sterile water and immersed in sterile water between each injection. Once the test solution was injected, closure of the eyelid was maintained using microaneurism clips until the animal began to recover gross motor activity. Following treatment, animals were warmed on a heating pad at 37°C.

### Quantitation of angiogenic response

Five days after disk implantation, animals were euthanized following im administration of 0.4 mg/kg atropine and corneas were digitally imaged. The neovascular surface area (NSA, expressed in pixels) was measured *postmortem* from blood-filled corneal vessels using computerized morphometry (Image Pro Plus, Media Cybernetics, v2.0). The individual mean NSA was determined in triplicate from three regions of identical size in the area of maximal neovascularization between the filter disk and the limbus. The number of pixels corresponding to the blood-filled corneal vessels in these regions was summated to produce an index of NSA. A group mean NSA was then calculated. Data from each treatment group were normalized to VEGF/siNA vehicle-treated control NSA and finally expressed as percent inhibition of VEGF-induced angiogenesis.

### Statistics

After determining the normality of treatment group means, group mean percent inhibition of VEGF-induced angiogenesis was subjected to a one-way analysis of variance. This was followed by two post-hoc tests for significance including Dunnett's (comparison to VEGF control) and Tukey-Kramer (all other group mean comparisons) at alpha = 0.05. Statistical analyses were performed using JMP v.3.1.6 (SAS Institute).

Results are graphically represented in **Figure 23.** As shown in **Figure 23,** VEGFR1 site 4229 active siNA at three concentrations were effective at inhibiting angiogenesis compared to the inverted siNA control and the VEGF control. A chemically modified version of the VEGFR1 site 4229 active siNA comprising a sense strand having 2'-deoxy-2'-fluoro pyrimidines and ribo purines with 5' and 3' terminal inverted deoxyabasic residues (SEQ ID NO: 419) and an antisense strand having having 2'-deoxy-2'-fluoro pyrimidines and ribo purines with a terminal 3'-phosphorothioate internucleotide linkage (SEQ ID NO: 420), showed similar inhibition. This result shows siNA molecules of differing chemically modified composition of the invention are capable of significantly inhibiting angiogenesis *in vivo.*

### Example 13: RNAi mediated inhibition of EGFR (HER1) RNA expression

siNA constructs (**Table I**) were tested for efficacy in reducing EGFR (HER1) RNA expression in A549 cells. A549 cells were plated approximately 24h before transfection in 96-well plates at 5,000-7,500 cells/well, 100 µl/well, such that at the time of transfection cells are 70-90% confluent. For transfection, annealed siNAs were mixed with the transfection reagent (Lipofectamine 2000, Invitrogen) in a volume of 50 µl/well and incubated for 20 min. at room temperature. The siNA transfection mixtures were added to cells to give a final siNA concentration of 25 nM in a volume of 150 µl. Each siNA transfection mixture was added to 3 wells for triplicate siNA treatments. Cells were incubated at 37°C for 24h in the continued presence of the siNA transfection mixture. At 24h, RNA was prepared from each well of treated cells. The supernatants with the transfection mixtures were first removed and discarded, then the cells wre lysed and RNA prepared from each well. Target gene expression following treatment was evaluated by RT-PCR for the target gene and for a control gene (36B4, an RNA polymerase subunit) for normalization. The triplicate data were averaged and the standard deviations determined for each treatment. Normalized data were graphed and the percent reduction of target mRNA by active siNAs in comparison to their respective inverted control siNAs was determined.

Results of this study are shown in **Figure 25**. A siNA construct comprising ribonucleotides and 3'-terminal dithymidine caps (RPI#30988/31064) was compared to a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and the antisense strand comprises a 3'-terminal phosphorothioate internucleotide linkage (RPI#31300/31301), which was also compared to a matched chemistry inverted control (RPI#31312/31313). In addition, the siNA constructs were also compared to untreated cells, cells transfected with lipid and scrambled siNA constructs (Scram1 and Scram2), and cells transfected with lipid alone (transfection control). As shown in the figure, both siNA constructs significantly reduce EGFR RNA expression. Additional stabilization chemistries as described in **Table IV** are similarly assayed for activity.

### Example 14: RNAi mediated inhibition of PKC-alpha RNA expression

siNA constructs (**Table I**) are tested for efficacy in reducing PKC-alpha RNA expression in, for example in A549 cells. Cells are plated approximately 24h before transfection in 96-well plates at 5,000-7,500 cells/well, 100 µl/well, such that at the time of transfection cells are 70-90% confluent. For transfection, annealed siNAs are mixed with the transfection reagent (Lipofectamine 2000, Invitrogen) in a volume of 50 µl/well and incubated for 20 min. at room temperature. The siNA transfection mixtures are added to cells to give a final siNA concentration of 25 nM in a volume of 150 µl. Each siNA transfection mixture is added to 3 wells for triplicate siNA treatments. Cells are incubated at 37° for 24h in the continued presence of the siNA transfection mixture. At 24h, RNA is prepared from each well of treated cells. The supernatants with the transfection mixtures are first removed and discarded, then the cells are lysed and RNA prepared from each well. Target gene expression following treatment is evaluated by RT-PCR for the target gene and for a control gene (36B4, an RNA polymerase subunit) for normalization. The triplicate data is averaged and the standard deviations determined for each treatment. Normalized data are graphed and the percent reduction of target mRNA by active siNAs in comparison to their respective inverted control siNAs was determined.

In a non-limiting example, siNA constructs were screened for activity (see **Figure 26**) and compared to untreated cells, scrambled siNA control constructs (Scram1 and Scram2), and cells transfected with lipid alone (transfection control). As shown in **Figure 26,** the siNA constructs significantly reduce PKC-alpha RNA expression. Leads generated from such a screen are then further assayed. In a non-limiting example, siNA constructs comprising ribonucleotides and 3'-terminal dithymidine caps are assayed along with a chemically modified siNA construct comprising 2'-deoxy-2'-fluoro pyrimidine nucleotides and purine ribonucleotides, in which the sense strand of the siNA is further modified with 5' and 3'-terminal inverted deoxyabasic caps and the antisense strand comprises a 3'-terminal phosphorothioate internucleotide linkage. Additional stabilization chemistries as described in **Table IV** are similarly assayed for activity. These siNA constructs are compared to appropriate matched chemistry inverted controls. In addition, the siNA constructs are also compared to untreated cells, cells transfected with lipid and scrambled siNA constructs, and cells transfected with lipid alone (transfection control).

### Example 15: RNAi mediated inhibition of Myc RNA expression

siNA constructs **(Table I**) were tested for efficacy in reducing Myc (c-Myc) RNA expression in 293T cells. 293T cells were plated approximately 24h before transfection in 96-well plates at 5,000-7,500 cells/well, 100 µl/well, such that at the time of transfection cells were 70-90% confluent. For transfection, annealed siNAs were mixed with the transfection reagent (Lipofectamine 2000, Invitrogen) in a volume of 50 µl/well and incubated for 20 min. at room temperature. The siNA transfection mixtures were added to cells to give a final siNA concentration of 25 nM in a volume of 150 µl. Each siNA transfection mixture was added to 3 wells for triplicate siNA treatments. Cells were incubated at 37°C for 24h in the continued presence of the siNA transfection mixture. At 24h, RNA was prepared from each well of treated cells. The supernatants with the transfection mixtures were first removed and discarded, then the cells were lysed and RNA prepared from each well. Target gene expression following treatment was evaluated by RT-PCR for the target gene and for a control gene (36B4, an RNA polymerase subunit) for normalization. The triplicate data were averaged and the standard deviations determined for each treatment. Normalized data were graphed and the percent reduction of target mRNA by active siNAs in comparison to their respective inverted control siNAs was determined.

Results of this study are shown in **Figure 27.** A screen of siNA constructs was compared to untreated cells, scrambled siNA control constructs (Scram1 and Scram2), and cells transfected with lipid alone (transfection control). As shown in the figure, three of the siNA constructs (RPI 30993/31069; RPI 30995/31071; and RPI 30996/31072) significantly reduce c-Myc RNA expression. Additional stabilization chemistries as described in **Table IV** are similarly assayed for activity.

### Example 16: RNAi mediated inhibition of BCL2 RNA expression

siNA constructs (**Table I**) are tested for efficacy in reducing BCL2 RNA expression in, for example, A549 cells. Cells are plated approximately 24h before transfection in 96-well plates at 5,000-7,500 cells/well, 100 µl/well, such that at the time of transfection cells are 70-90% confluent. For transfection, annealed siNAs are mixed with the transfection reagent (Lipofectamine 2000, Invitrogen) in a volume of 50 µl/well and incubated for 20 min. at room temperature. The siNA transfection mixtures are added to cells to give a final siNA concentration of 25 nM in a volume of 150 µl. Each siNA transfection mixture is added to 3 wells for triplicate siNA treatments. Cells are incubated at 37° for 24h in the continued presence of the siNA transfection mixture. At 24h, RNA is prepared from each well of treated cells. The supernatants with the transfection mixtures are first removed and discarded, then the cells are lysed and RNA prepared from each well. Target gene expression following treatment is evaluated by RT-PCR for the target gene and for a control gene (36B4, an RNA polymerase subunit) for normalization. The triplicate data is averaged and the standard deviations determined for each treatment. Normalized data are graphed and the percent reduction of target mRNA by active siNAs in comparison to their respective inverted control siNAs is determined.

In a non-limiting example, A549 cells were transfected with 0.25 ug/well of lipid complexed with 25 nM siNA. A siNA construct comprising ribonucleotides and 3'-terminal dithymidine caps (RPI#30998/31074) was tested along with a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and the antisense strand comprises a 3'-terminal phosphorothioate internucleotide linkage (RPI#31368/31369), which was also compared to a matched chemistry inverted control (RPI#31370/31371) and a chemically modified siNA construct comprising 2'-deoxy-2'-fluoro pyrimidine and 2'-deoxy-2'-fluoro purine nucleotides in which the sense strand of the siNA is further modified with 5' and 3'-terminal inverted deoxyabasic caps and the antisense strand comprises a 3'-terminal phosphorothioate internucleotide linkage (RPI#31372/31373) which was also compared to a matched chemistry inverted control (RPI#31374/31375). In addition, the siNA constructs were also compared to untreated cells, cells transfected with lipid and scrambled siNA constructs (Scram1 and Scram2), and cells transfected with lipid alone (transfection control). As shown in **Figure 28**, the siNA constructs significantly reduce BCL2 RNA expression compared to scrambled, untreated, and transfection controls. Additional stabilization chemistries as described in **Table IV** are similarly assayed for activity.

### Example 17: RNAi mediated inhibition of CHK-1 RNA expression

siNA constructs **(Table I)** were tested for efficacy in reducing CHK-1 RNA expression in A549 cells. A549 cells were plated approximately 24h before transfection in 96-well plates at 5,000-7,500 cells/well, 100 µl/well, such that at the time of transfection cells are 70-90% confluent For transfection, annealed siNAs were mixed with the transfection reagent (Lipofectamine 2000, Invitrogen) in a volume of 50 µl/well and incubated for 20 min. at room temperature. The siNA transfection mixtures were added to cells to give a final siNA concentration of 25 nM in a volume of 150 µl. Each siNA transfection mixture was added to 3 wells for triplicate siNA treatments. Cells were incubated at 37° for 24h in the continued presence of the siNA transfection mixture. At 24h, RNA was prepared from each well of treated cells. The supernatants with the transfection mixtures were first removed and discarded, then the cells were lysed and RNA prepared from each well. Target gene expression following treatment was evaluated by RT-PCR for the target gene and for a control gene (36B4, an RNA polymerase subunit) for normalization. The triplicate data were averaged and the standard deviations determined for each treatment. Normalized data were graphed and the percent reduction of target mRNA by active siNAs in comparison to their respective inverted control siNAs was determined.

Results of this study are shown in **Figure 29.** A siNA construct comprising ribonucleotides and 3'-terminal dithymidine caps (RPI#31003/31079) and a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and in which the antisense strand comprises a 3'-terminal phosphorothioate internucleotide linkage (RPI#31302/31303), were compared to a matched chemistry inverted control (RPI#31314/31325). In addition, the siNA constructs were also compared to untreated cells, cells transfected with lipid and scrambled siNA constructs (Scram1 and Scram2), and cells transfected with lipid alone (transfection control). As shown in the figure, both siNA constructs significantly reduce CHK-1 RNA expression compared to appropriate controls. Additional stabilization chemistries as described in **Table IV** are similarly assayed for activity.

### Example 18: RNAi mediated inhibition of BACE RNA expression

siNA constructs (**Table I**) are tested for efficacy in reducing BACE RNA expression in, for example in A549 cells. Cells are plated approximately 24h before transfection in 96-well plates at 5,000-7,500 cells/well, 100 µl/well, such that at the time of transfection cells are 70-90% confluent. For transfection, annealed siNAs are mixed with the transfection reagent (Lipofectamine 2000, Invitrogen) in a volume of 50 µl/well and incubated for 20 min. at room temperature. The siNA transfection mixtures are added to cells to give a final siNA concentration of 25 nM in a volume of 150 µl. Each siNA transfection mixture is added to 3 wells for triplicate siNA treatments. Cells are incubated at 37°C for 24h in the continued presence of the siNA transfection mixture. At 24h, RNA is prepared from each well of treated cells. The supernatants with the transfection mixtures are first removed and discarded, then the cells are lysed and RNA prepared from each well. Target gene expression following treatment is evaluated by RT-PCR for the target gene and for a control gene (36B4, an RNA polymerase subunit) for normalization. The triplicate data is averaged and the standard deviations determined for each treatment. Normalized data are graphed and the percent reduction of target mRNA by active siNAs in comparison to their respective inverted control siNAs was determined.

In a non-limiting example, siNA constructs were screened for activity (see **Figure 30**) and compared to untreated cells, scrambled siNA control constructs (Scram1 and Scram2), and cells transfected with lipid alone (transfection control). As shown in **Figure 30,** the siNA constructs significantly reduce BACE RNA expression. Leads generated from such a screen are then further assayed. In a non-limiting example, siNA constructs comprising ribonucleotides and 3'-terminal dithymidine caps are assayed along with a chemically modified siNA construct comprising 2'-deoxy-2'-fluoro pyrimidine nucleotides and purine ribonucleotides, in which the sense strand of the siNA is further modified with 5' and 3'-terminal inverted deoxyabasic caps and the antisense strand comprises a 3'-terminal phosphorothioate internucleotide linkage. Additional stabilization chemistries as described in **Table IV** are similarly assayed for activity. These siNA constructs are compared to appropriate matched chemistry inverted controls. In addition, the siNA constructs are also compared to untreated cells, cells transfected with lipid and scrambled siNA constructs, and cells transfected with lipid alone (transfection control).

### Example 19: RNAi mediated inhibition of cyclin D 1 RNA expression

siNA constructs (**Table I**) were tested for efficacy in reducing cyclin D1 RNA expression in A549 cells. A549 cells were plated approximately 24h before transfection in 96-well plates at 5,000-7,500 cells/well, 100 µl/well, such that at the time of transfection cells are 70-90% confluent. For transfection, annealed siNAs were mixed with the transfection reagent (Lipofectamine 2000, Invitrogen) in a volume of 50 µl/well and incubated for 20 min. at room temperature. The siNA transfection mixtures were added to cells to give a final siNA concentration of 25 nM in a volume of 150 µl. Each siNA transfection mixture was added to 3 wells for triplicate siNA treatments. Cells were incubated at 37° for 24h in the continued presence of the siNA transfection mixture. At 24h, RNA was prepared from each well of treated cells. The supernatants with the transfection mixtures were first removed and discarded, then the cells were lysed and RNA prepared from each well. Target gene expression following treatment was evaluated by RT-PCR for the target gene and for a control gene (36B4, an RNA polymerase subunit) for normalization. The triplicate data were averaged and the standard deviations determined for each treatment. Normalized data were graphed and the percent reduction of target mRNA by active siNAs in comparison to their respective inverted control siNAs was determined.

Results of this study are shown in **Figure 31.** A siNA construct comprising ribonucleotides and 3'-terminal dithymidine caps (RPI#30988/31064) was assayed along with a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and the antisense strand comprises a 3'-terminal phosphorothioate internucleotide linkage (RPI#31300/3130), which was also compared to a matched chemistry inverted control (RPI#31312/31313). In addition, the siNA constructs were also compared to untreated cells, cells transfected with lipid and scrambled siNA constructs (Scram1 and Scram2), and cells transfected with lipid alone (transfection control). As shown in the figure, both siNA constructs significantly reduce cyclin D1 RNA expression. Additional stabilization chemistries as described in **Table IV** are similarly assayed for activity.

### Example 20: RNAi mediated inhibition of PTP-1B RNA expression

siNA constructs (**Table I**) were tested for efficacy in reducing PTP-1B RNA expression in A549 cells. A549 cells were plated approximately 24h before transfection in 96-well plates at 5,000-7,500 cells/well, 100 µl/well, such that at the time of transfection cells are 70-90% confluent. For transfection, annealed siNAs were mixed with the transfection reagent (Lipofectamine 2000, Invitrogen) in a volume of 50 µl/well and incubated for 20 min. at room temperature. The siNA transfection mixtures were added to cells to give a final siNA concentration of 25 nM in a volume of 150 µl. Each siNA transfection mixture was added to 3 wells for triplicate siNA treatments. Cells were incubated at 37° for 24h in the continued presence of the siNA transfection mixture. At 24h, RNA was prepared from each well of treated cells. The supernatants with the transfection mixtures were first removed and discarded, then the cells were lysed and RNA prepared from each well. Target gene expression following treatment was evaluated by RT-PCR for the target gene and for a control gene (36B4, an RNA polymerase subunit) for normalization. The triplicate data were averaged and the standard deviations determined for each treatment. Normalized data were graphed and the percent reduction of target mRNA by active siNAs in comparison to their respective inverted control siNAs was determined.

Results of this study are shown in **Figure 32.** A siNA construct comprising ribonucleotides and 3'-terminal dithymidine caps (RPI#31018/31094) was assayed along with a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and the antisense strand comprises a 3'-terminal phosphorothioate internucleotide linkage (RPI#31306/31307), which was also compared to a matched chemistry inverted control (RPI#31318/31319). In addition, the siNA constructs were also compared to untreated cells, cells transfected with lipid and scrambled siNA constructs (Scram1 and Scram2), and cells transfected with lipid alone (transfection control). As shown in the figure, both siNA constructs significantly reduce PTP-1B RNA expression. Additional stabilization chemistries as described in **Table IV** are similarly assayed for activity.

### Example 21: RNAi mediated inhibition of ERG2 RNA expression

siNA constructs (**Table I**) are tested for efficacy in reducing ERG2 RNA expression in, for example in DLD1 cells. Cells are plated approximately 24h before transfection in 96-well plates at 5,000-7,500 cells/well, 100 µl/well, such that at the time of transfection cells are 70-90% confluent. For transfection, annealed siNAs are mixed with the transfection reagent (Lipofectamine 2000, Invitrogen) in a volume of 50 µl/well and incubated for 20 min. at room temperature. The siNA transfection mixtures are added to cells to give a final siNA concentration of 25 nM in a volume of 150 µl. Each siNA transfection mixture is added to 3 wells for triplicate siNA treatments. Cells are incubated at 37° for 24h in the continued presence of the siNA transfection mixture. At 24h, RNA is prepared from each well of treated cells. The supernatants with the transfection mixtures are first removed and discarded, then the cells are lysed and RNA prepared from each well. Target gene expression following treatment is evaluated by RT-PCR for the target gene and for a control gene (36B4, an RNA polymerase subunit) for normalization. The triplicate data is averaged and the standard deviations determined for each treatment. Normalized data are graphed and the percent reduction of target mRNA by active siNAs in comparison to their respective inverted control siNAs was determined.

In a non-limiting example, siNA constructs were screened for activity (see **Figure 33**) and compared to untreated cells, scrambled siNA control constructs (Scram1 and Scram2), and cells transfected with lipid alone (transfection control). As shown in **Figure 33,** the siNA constructs significantly reduce of ERG2 RNA expression. Leads generated from such a screen are then further assayed. In a non-limiting example, siNA constructs comprising ribonucleotides and 3'-terminal dithymidine caps are assayed along with a chemically modified siNA construct comprising 2'-deoxy-2'-fluoro pyrimidine nucleotides and purine ribonucleotides, in which the sense strand of the siNA is further modified with 5' and 3'-terminal inverted deoxyabasic caps and the antisense strand comprises a 3'-terminal phosphorothioate internucleotide linkage. Additional stabilization chemistries as described in **Table IV** are similarly assayed for activity. These siNA constructs are compared to appropriate matched chemistry inverted controls. In addition, the siNA constructs are also compared to untreated cells, cells transfected with lipid and scrambled siNA constructs, and cells transfected with lipid alone (transfection control). Additional stabilization chemistries as described in **Table IV** are similarly assayed for activity.

### Example 22: RNAi mediated inhibition of PCNA RNA expression

siNA constructs **(Table I)** were tested for efficacy in reducing PCNA RNA expression in A549 cells. A549 cells were plated approximately 24h before transfection in 96-well plates at 5,000-7,500 cells/well, 100 µl/well, such that at the time of transfection cells are 70-90% confluent. For transfection, annealed siNAs were mixed with the transfection reagent (Lipofectamine 2000, Invitrogen) in a volume of 50 µl/well and incubated for 20 min. at room temperature. The siNA transfection mixtures were added to cells to give a final siNA concentration of 25 nM in a volume of 150 µl. Each siNA transfection mixture was added to 3 wells for triplicate siNA treatments. Cells were incubated at 37° for 24h in the continued presence of the siNA transfection mixture. At 24h, RNA was prepared from each well of treated cells. The supernatants with the transfection mixtures were first removed and discarded, then the cells were lysed and RNA prepared from each well. Target gene expression following treatment was evaluated by RT-PCR for the target gene and for a control gene (36B4, an RNA polymerase subunit) for normalization. The triplicate data were averaged and the standard deviations determined for each treatment. Normalized data were graphed and the percent reduction of target mRNA by active siNAs in comparison to their respective inverted control siNAs was determined.

Results of this study are shown in **Figure 34.** A siNA construct comprising ribonucleotides and 3'-terminal dithymidine caps (RPI#31035/31111) was assayed along with a chemically modified siNA construct comprising 2'-deoxy-2'-fluoro pyrimidine nucleotides and purine ribonucleotides in which the sense strand of the siNA is further modified with 5' and 3'-terminal inverted deoxyabasic caps and the antisense strand comprises a 3'-terminal phosphorothioate internucleotide linkage (RPI#31310/31311), which was also compared to a matched chemistry inverted control (RPI#31322/31323). In addition, the siNA constructs were also compared to untreated cells, cells transfected with lipid and scrambled siNA constructs (Scram1 and Scram2), and cells transfected with lipid alone (transfection control). As shown in the figure, both siNA constructs significant reduce PCNA RNA expression. Additional stabilization chemistries as described in **Table IV** are similarly assayed for activity.

### Example 23: Indications

The siNA molecules of the invention can be used to treat a variety of diseases and conditions through modulation of gene expression. Using the methods described herein, chemically modified siNA molecules can be designed to modulate the expression any number of target genes, including but not limited to genes associated with cancer, metabolic diseases, infectious diseases such as viral, bacterial or fungal infections, neurologic diseases, musculoskeletal diseases, diseases of the immune system, diseases associated with signaling pathways and cellular messengers, and diseases associated with transport systems including molecular pumps and channels.

Non-limiting examples of various viral genes that can be targeted using siRNA molecules of the invention include Hepatitis C Virus (HCV, for example Genbank Accession Nos: D11168, D50483.1, L38318 and S82227), Hepatitis B Virus (HBV, for example GenBank Accession No. AF100308.1), Human Immunodeficiency Virus type 1 (HIV-1, for example GenBank Accession No. U51188), Human Immunodeficiency Virus type 2 (HIV-2, for example GenBank Accession No. X60667), West Nile Virus (WNV for example GenBank accession No. NC_001563), cytomegalovirus (CMV for example GenBank Accession No. NC_001347), respiratory syncytial virus (RSV for example GenBank Accession No. NC_001781), influenza virus (for example example GenBank Accession No. AF037412, rhinovirus (for example, GenBank accession numbers: D00239, X02316, X01087, L24917, M16248, K02121, X01087), papillomavirus (for example GenBank Accession No. NC_001353), Herpes Simplex Virus (HSV for example GenBank Accession No. NC_001345), and other viruses such as HTLV (for example GenBank Accession No. AJ430458). Due to the high sequence variability of many viral genomes, selection of siRNA molecules for broad therapeutic applications would likely involve the conserved regions of the viral genome. Nonlimiting examples of conserved regions of the viral genomes include but are not limited to 5'-Non Coding Regions (NCR), 3'- Non Coding Regions (NCR) and/or internal ribosome entry sites (IRES). siRNA molecules designed against conserved regions of various viral genomes will enable efficient inhibition of viral replication in diverse patient populations and may ensure the effectiveness of the siRNA molecules against viral quasi species which evolve due to mutations in the non-conserved regions of the viral genome.

Non-limiting examples of human genes that can be targeted using siRNA molecules of the invention using methods described herein include any human RNA sequence, for example those commonly referred to by Genbank Accession Number. These RNA sequences can be used to design siRNA molecules that inhibit gene expression and therefore abrogate diseases, conditions, or infections associated with expression of those genes. Such non-limiting examples of human genes that can be targeted using siRNA molecules of the invention include VEGFr (VEGFr-1 for example GenBank Accession No. XM_067723, VEGFr-2 for example GenBank Accession No. AF063658), HER1, HER2, HER3, and HER4 (for example Genbank Accession Nos: NM_005228, NM_004448, NM_001982, and NM_005235 respectively), telomerase (TERT, for example GenBank Accession No. NM_003219), telomerase RNA (for example GenBank Accession No. U86046), NFkappaB, Rel-A (for example GenBank Accession No. NM_005228), NOGO (for example GenBank Accession No. AB020693), NOGOr (for example GenBank Accession No. XM_015620), RAS (for example GenBank Accession No. NM_004283), RAF (for example GenBank Accession No. XM_033884), CD20 (for example GenBank Accession No. X07203), METAP2 (for example GenBank Accession No. NM_003219), CLCA1 (for example GenBank Accession No. NM_001285), phospholamban (for example GenBank Accession No. NM_002667), PTP1B (for example GenBank Accession No. M31724), and others, for example, those shown in Table III.

The siNA molecule of the invention can also be used in a variety of agricultural applications involving modulation of endogenous or exogenous gene expression in plants using siNA, including use as insecticidal, antiviral and anti-fungal agents or modulate plant traits such as oil and starch profiles and stress resistance.

### Example 24: Diagnostic uses

The siNA molecules of the invention can be used in a variety of diagnostic applications, such as in the identification of molecular targets (e.g., RNA) in a variety of applications, for example, in clinical, industrial, environmental, agricultural and/or research settings. Such diagnostic use of siNA molecules involves utilizing reconstituted RNAi systems, for example, using cellular lysates or partially purified cellular lysates. siNA molecules of this invention can be used as diagnostic tools to examine genetic drift and mutations within diseased cells or to detect the presence of endogenous or exogenous, for example viral, RNA in a cell. The close relationship between siNA activity and the structure of the target RNA allows the detection of mutations in any region of the molecule, which alters the base-pairing and three-dimensional structure of the target RNA. By using multiple siNA molecules described in this invention, one can map nucleotide changes, which are important to RNA structure and function *in vitro,* as well as in cells and tissues. Cleavage of target RNAs with siNA molecules can be used to inhibit gene expression and define the role of specified gene products in the progression of disease or infection. In this manner, other genetic targets can be defined as important mediators of the disease. These experiments will lead to better treatment of the disease progression by affording the possibility of combination therapies (e.g., multiple siNA molecules targeted to different genes, siNA molecules coupled with known small molecule inhibitors, or intermittent treatment with combinations siNA molecules and/or other chemical or biological molecules). Other *in vitro* uses of siNA molecules of this invention are well known in the art, and include detection of the presence of mRNAs associated with a disease, infection, or related condition. Such RNA is detected by determining the presence of a cleavage product after treatment with a siNA using standard methodologies, for example, fluorescence resonance emission transfer (FRET).

In a specific example, siNA molecules that cleave only wild-type or mutant forms of the target RNA are used for the assay. The first siNA molecules (*i*.*e*., those that cleave only wild-type forms of target RNA) are used to identify wild-type RNA present in the sample and the second siNA molecules (*i*.*e*., those that cleave only mutant forms of target RNA) are used to identify mutant RNA in the sample. As reaction controls, synthetic substrates of both wild-type and mutant RNA are cleaved by both siNA molecules to demonstrate the relative siNA efficiencies in the reactions and the absence of cleavage of the "non-targeted" RNA species. The cleavage products from the synthetic substrates also serve to generate size markers for the analysis of wild-type and mutant RNAs in the sample population. Thus, each analysis requires two siNA molecules, two substrates and one unknown sample, which is combined into six reactions. The presence of cleavage products is determined using an RNase protection assay so that full-length and cleavage fragments of each RNA can be analyzed in one lane of a polyacrylamide gel. It is not absolutely required to quantify the results to gain insight into the expression of mutant RNAs and putative risk of the desired phenotypic changes in target cells. The expression of mRNA whose protein product is implicated in the development of the phenotype (*i*.*e*., disease related or infection related) is adequate to establish risk. If probes of comparable specific activity are used for both transcripts, then a qualitative comparison of RNA levels is adequate and decreases the cost of the initial diagnosis. Higher mutant form to wild-type ratios are correlated with higher risk whether RNA levels are compared qualitatively or quantitatively.

All patents and publications mentioned in the specification are indicative of the levels of skill of those skilled in the art to which the invention pertains. All references cited in this disclosure are incorporated by reference to the same extent as if each reference had been incorporated by reference in its entirety individually.

One skilled in the art would readily appreciate that the present invention is well adapted to carry out the objects and obtain the ends and advantages mentioned, as well as those inherent therein. The methods and compositions described herein as presently representative of preferred embodiments are exemplary and are not intended as limitations on the scope of the invention. Changes therein and other uses will occur to those skilled in the art, which are encompassed within the spirit of the invention, are defined by the scope of the claims.

It will be readily apparent to one skilled in the art that varying substitutions and modifications can be made to the invention disclosed herein without departing from the scope and spirit of the invention. Thus, such additional embodiments are within the scope of the present invention and the following claims. The present invention teaches one skilled in the art to test various combinations and/or substitutions of chemical modifications described herein toward generating nucleic acid constructs with improved activity for mediating RNAi activity. Such improved activity can comprise improved stability, improved bioavailability, and/or improved activation of cellular responses mediating RNAi. Therefore, the specific embodiments described herein are not limiting and one skilled in the art can readily appreciate that specific combinations of the modifications described herein can be tested without undue experimentation toward identifying siNA molecules with improved RNAi activity.

The invention illustratively described herein suitably can be practiced in the absence of any element or elements, limitation or limitations that are not specifically disclosed herein. Thus, for example, in each instance herein any of the terms "comprising", "consisting essentially of', and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments, optional features, modification and variation of the concepts herein disclosed may be resorted to by those skilled in the art, and that such modifications and variations are considered to be within the scope of this invention as defined by the description and the appended claims.

In addition, where features or aspects of the invention are described in terms of Markush groups or other grouping of alternatives, those skilled in the art will recognize that the invention is also thereby described in terms of any individual member or subgroup of members of the Markush group or other group.

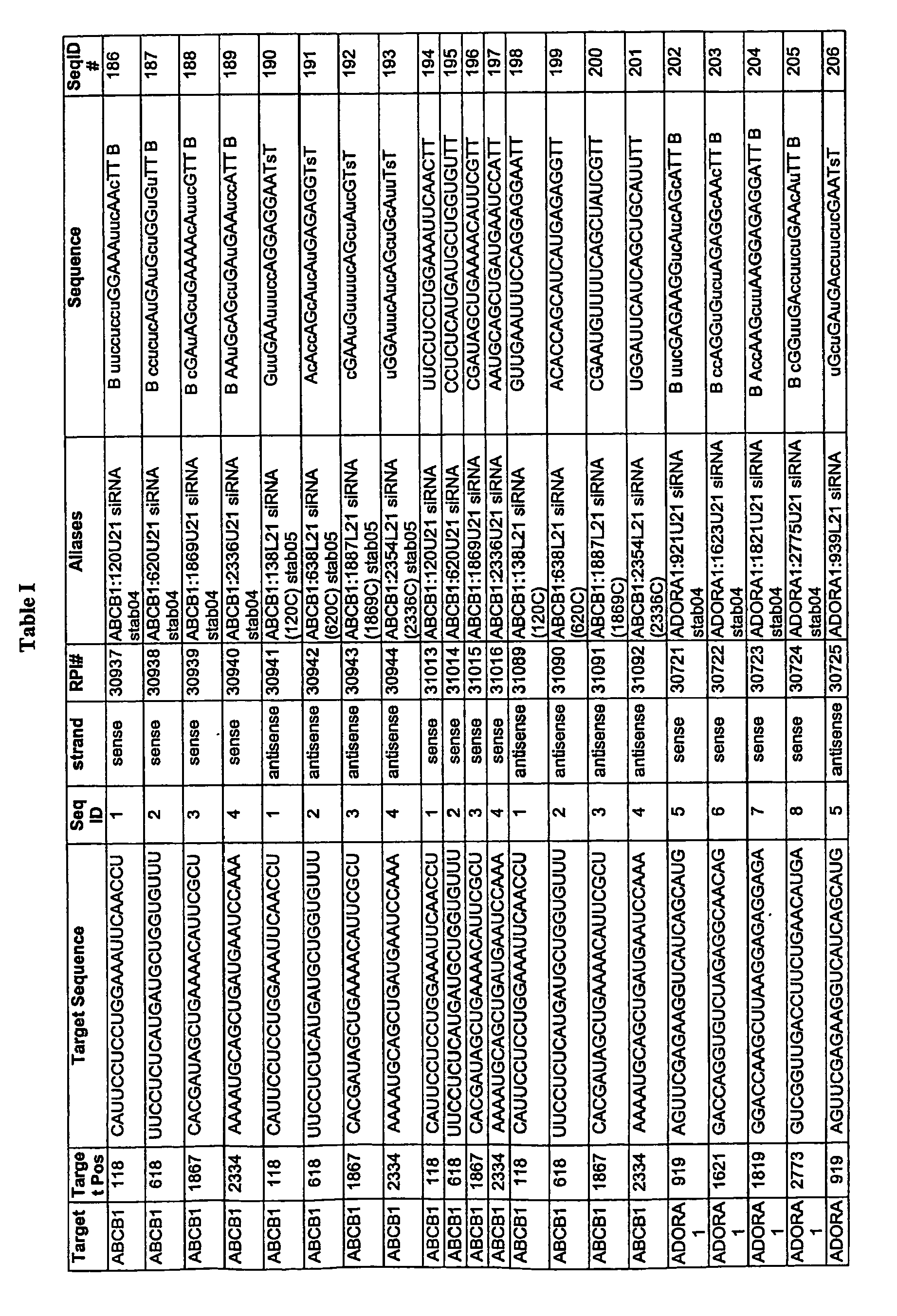

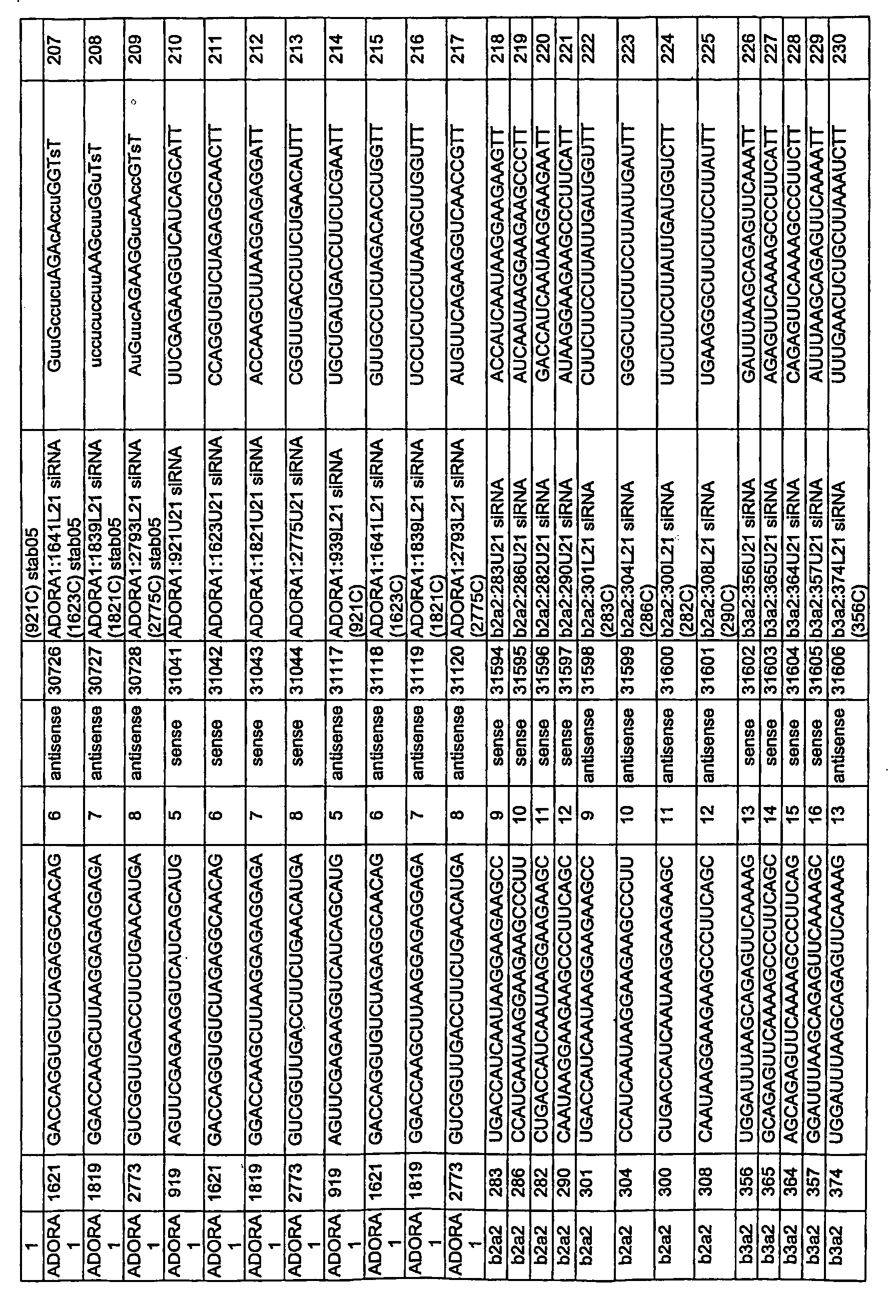

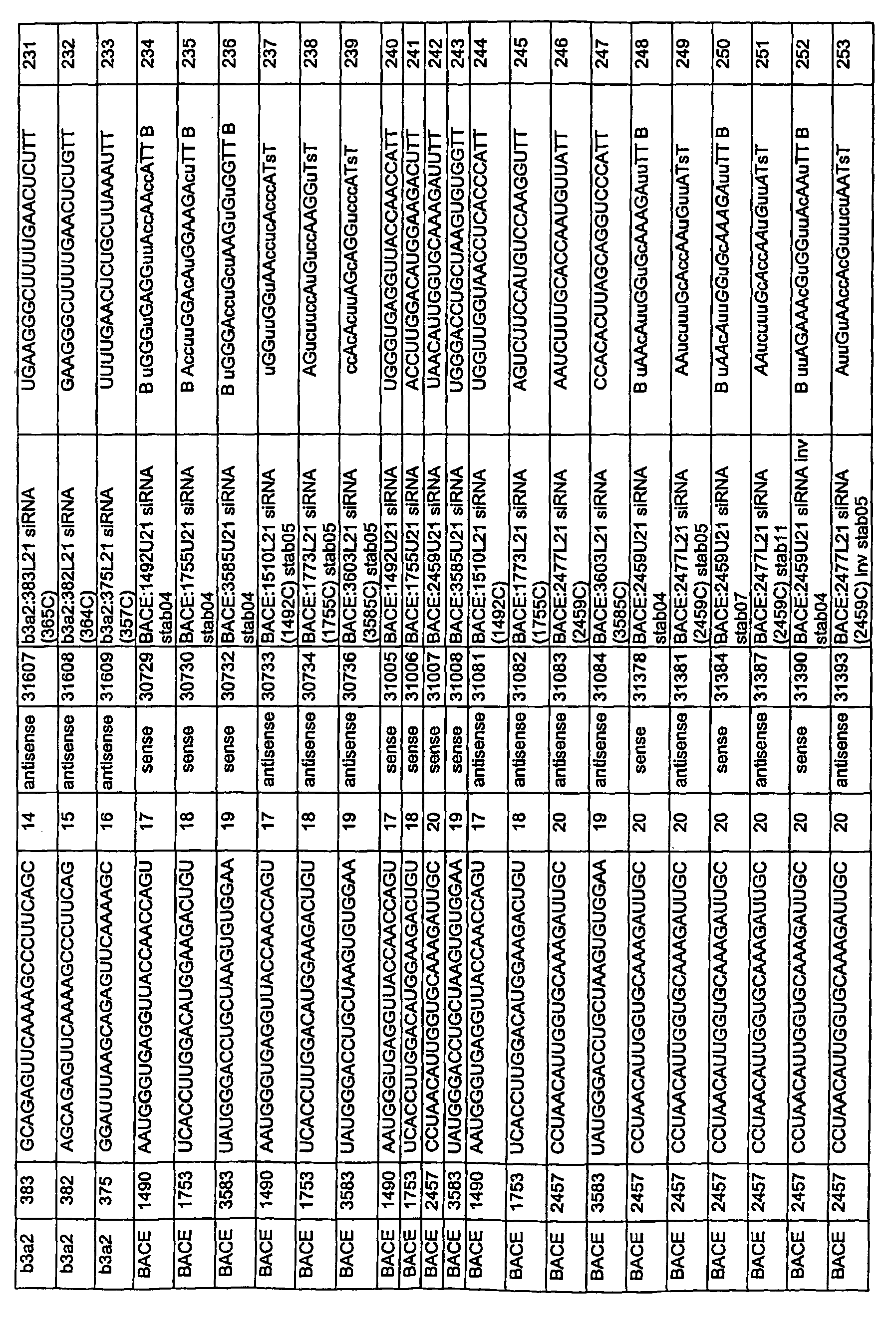

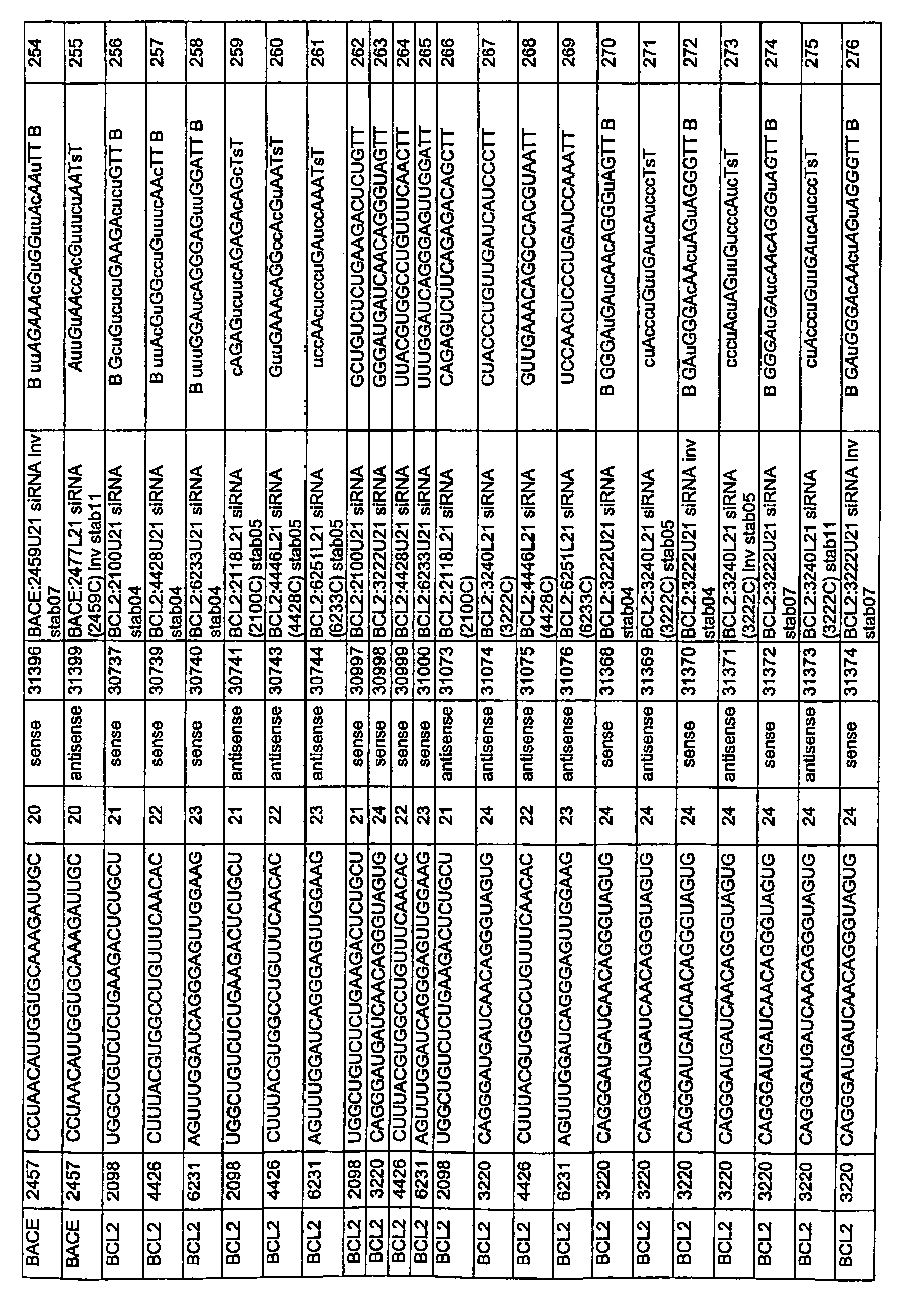

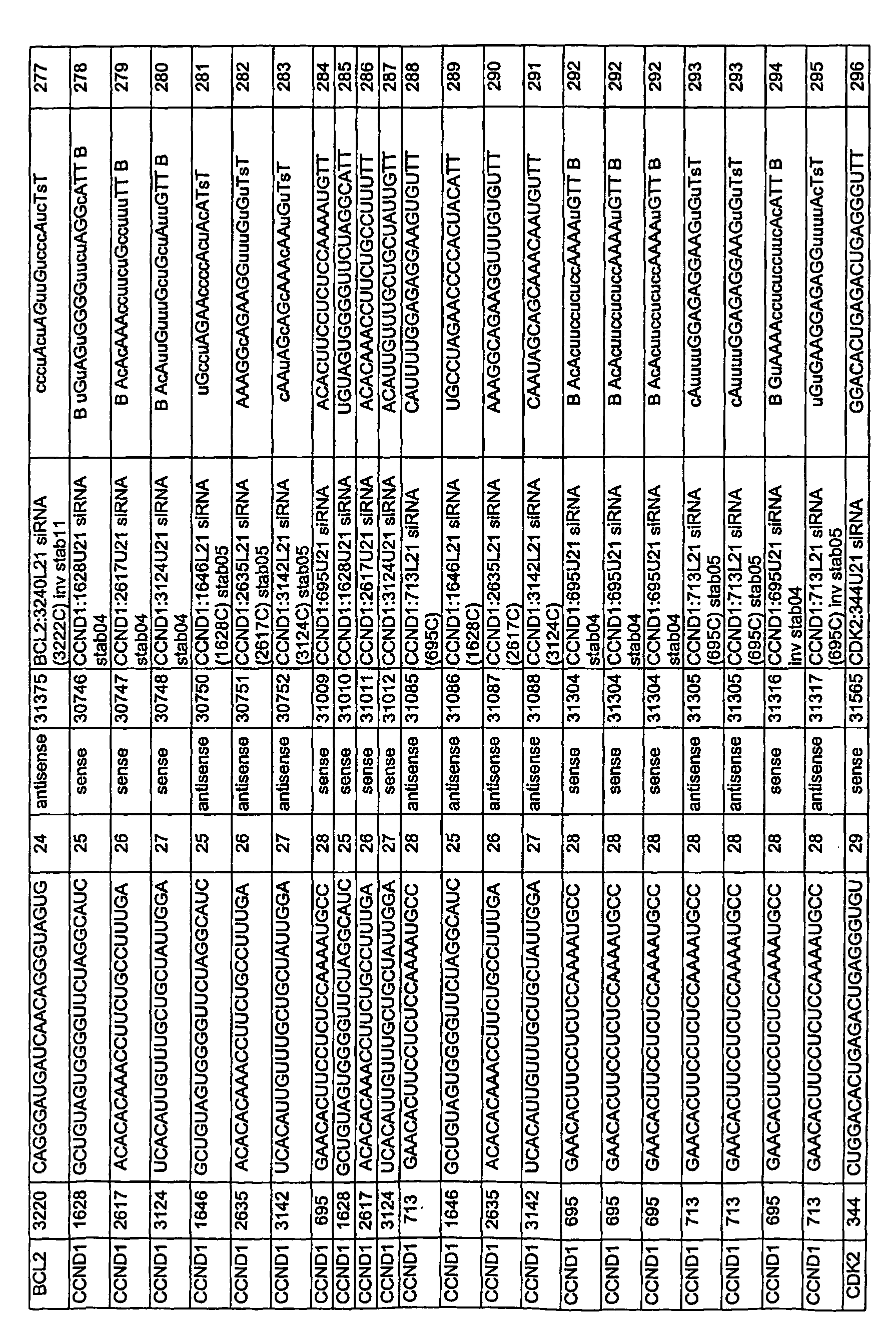

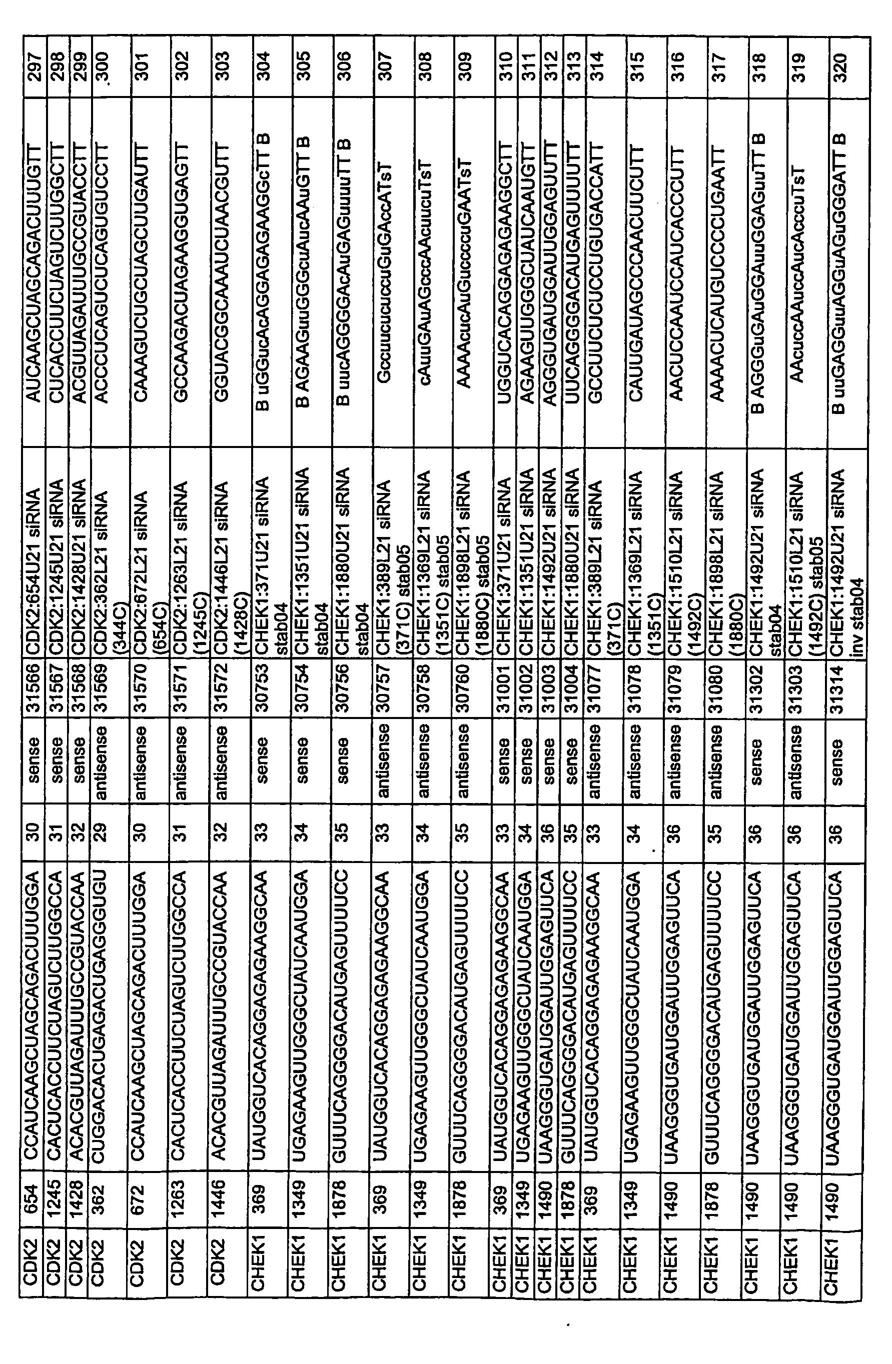

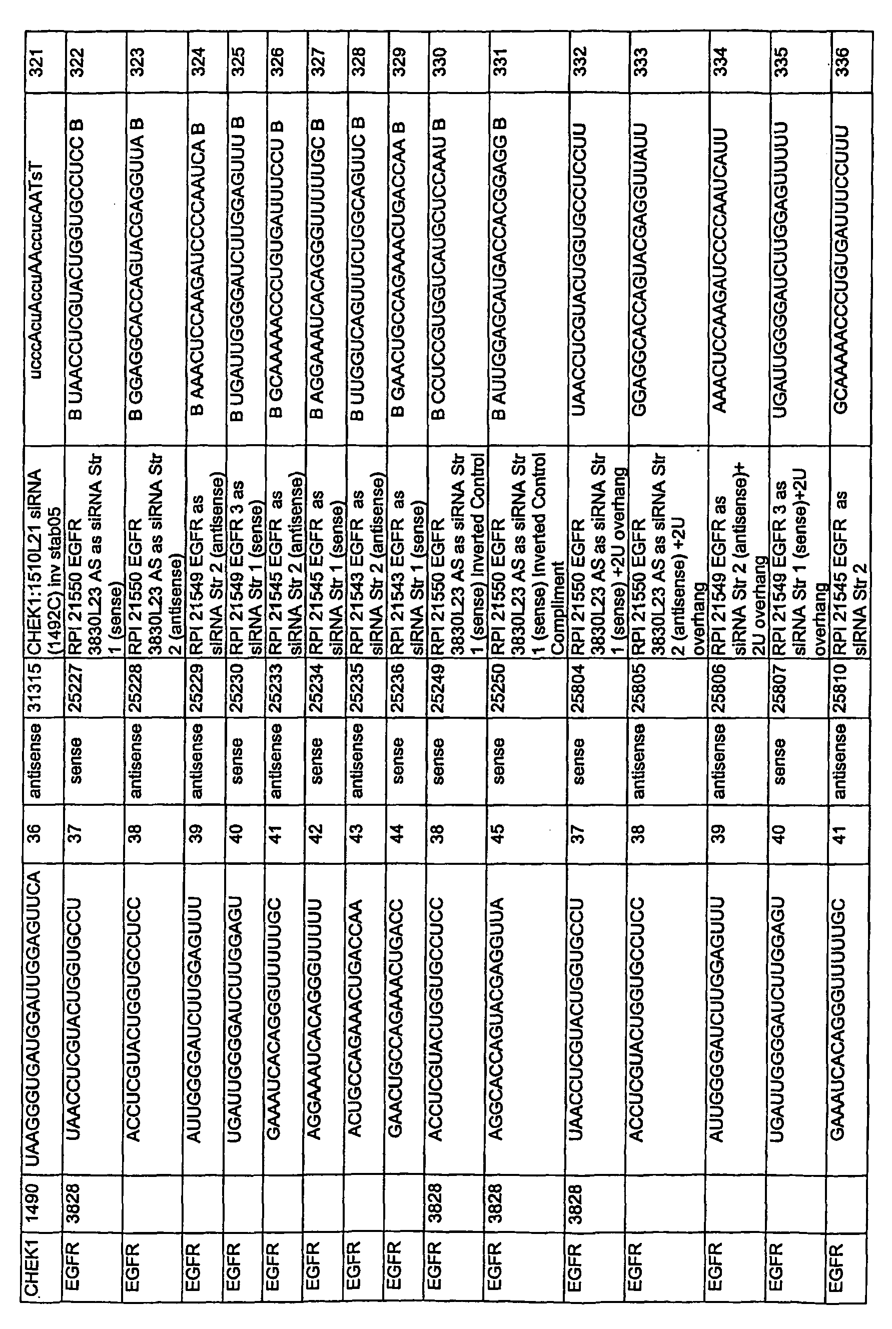

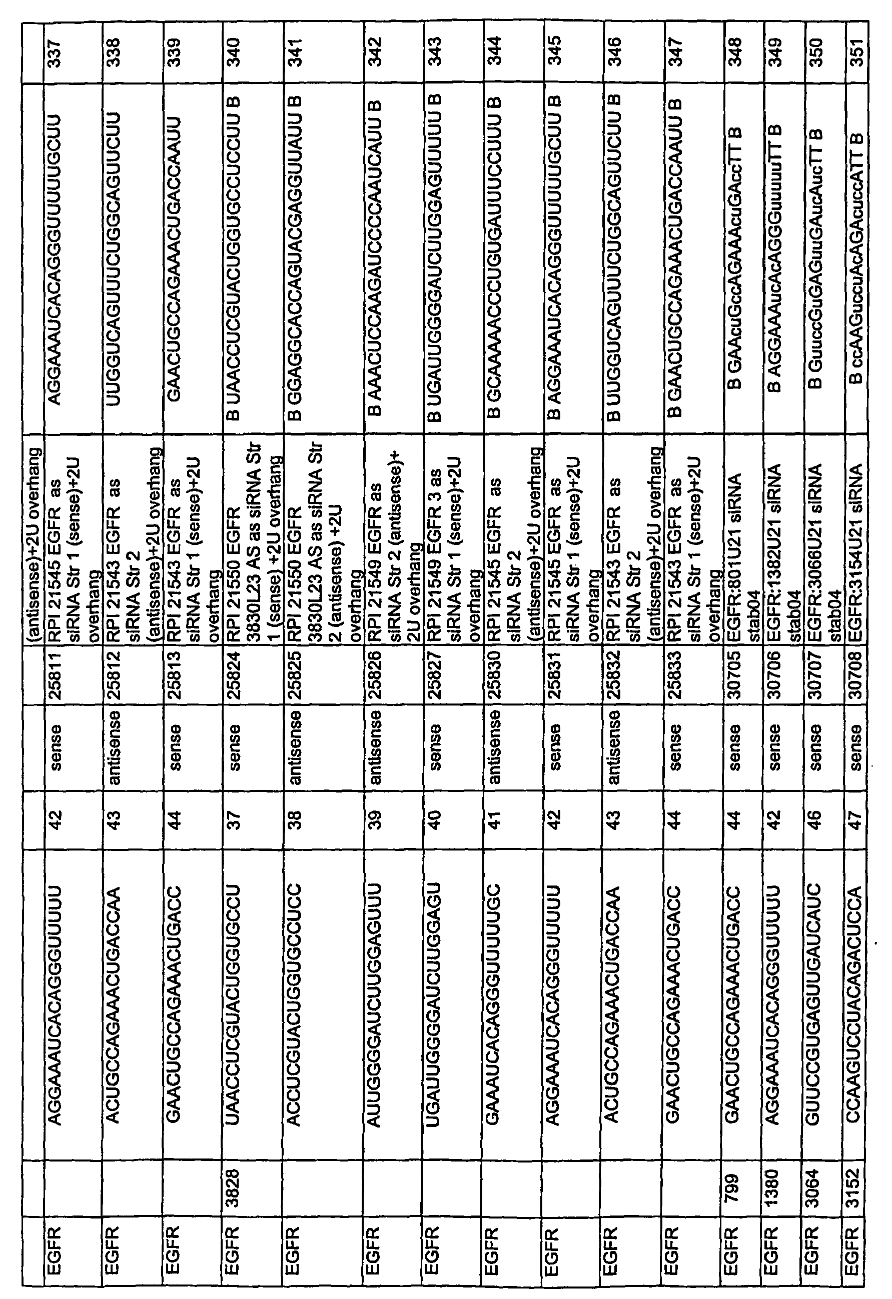

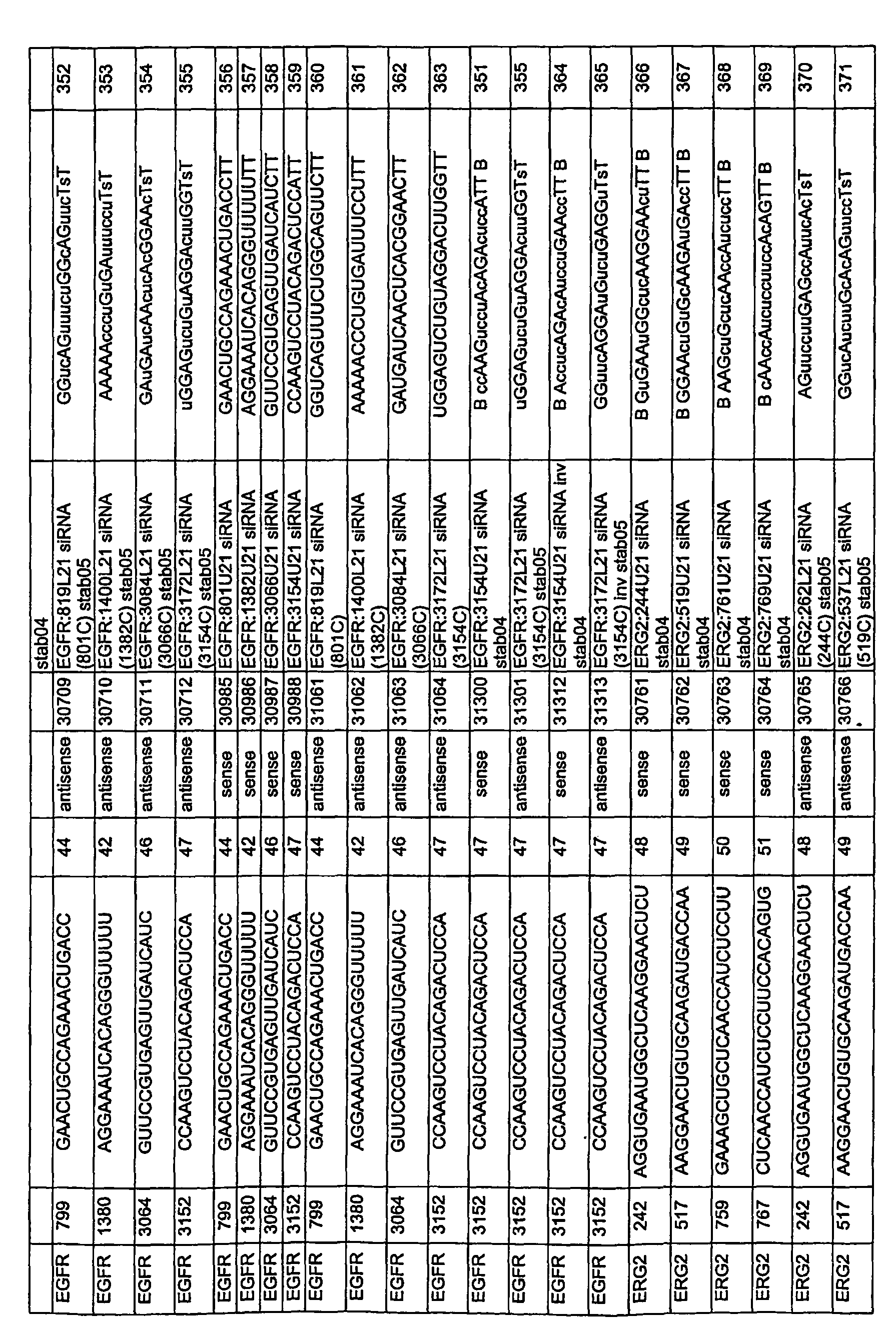

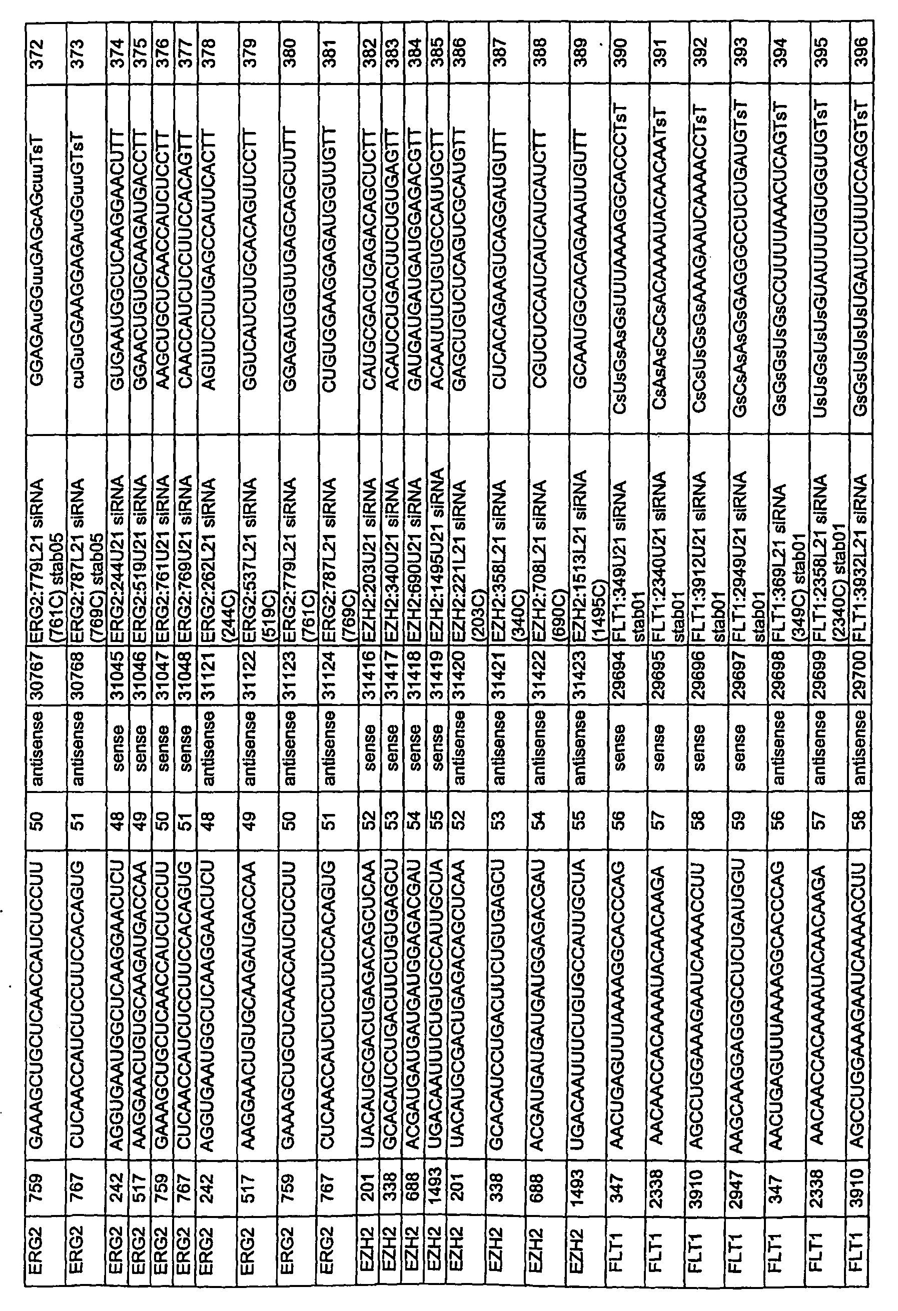

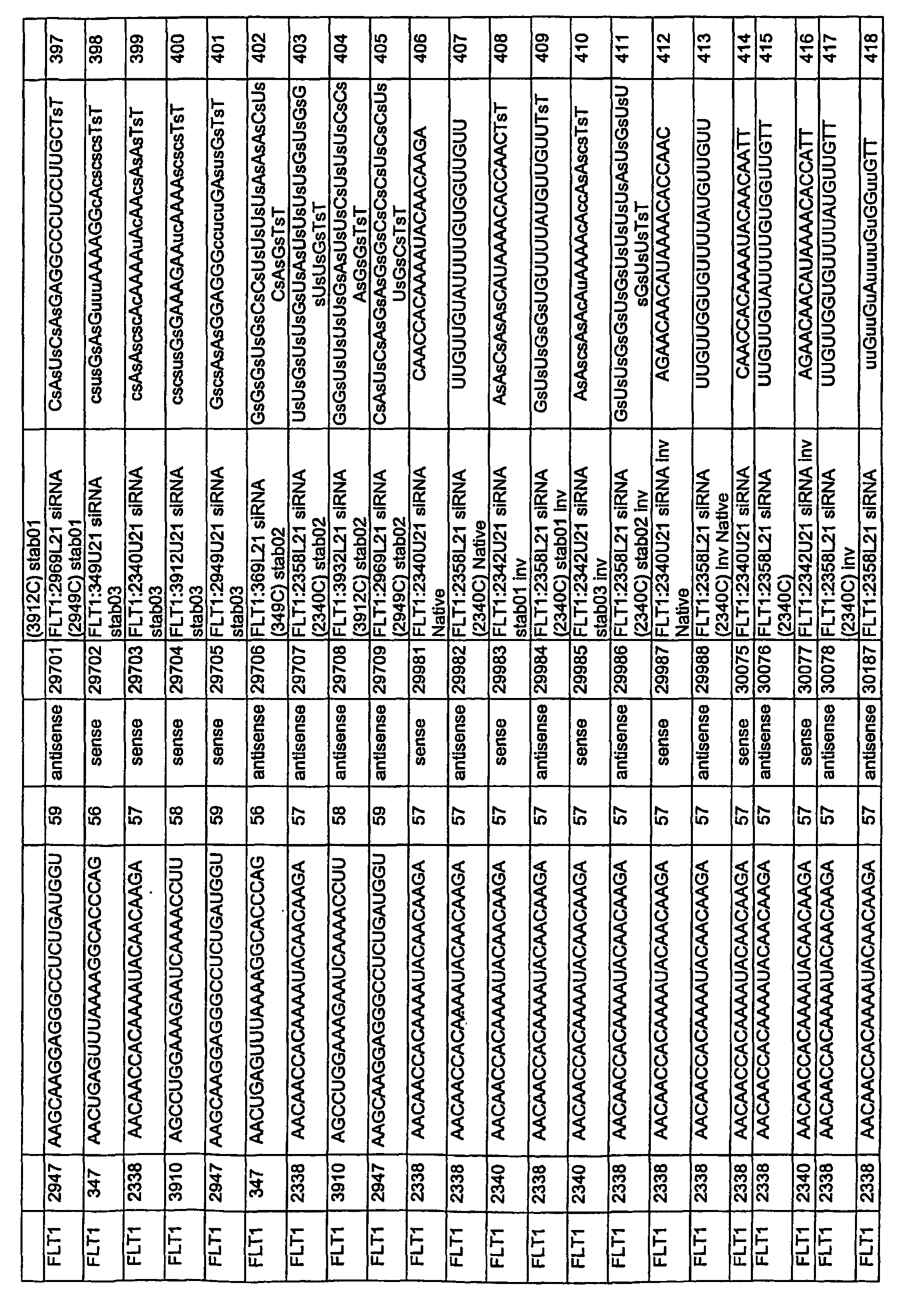

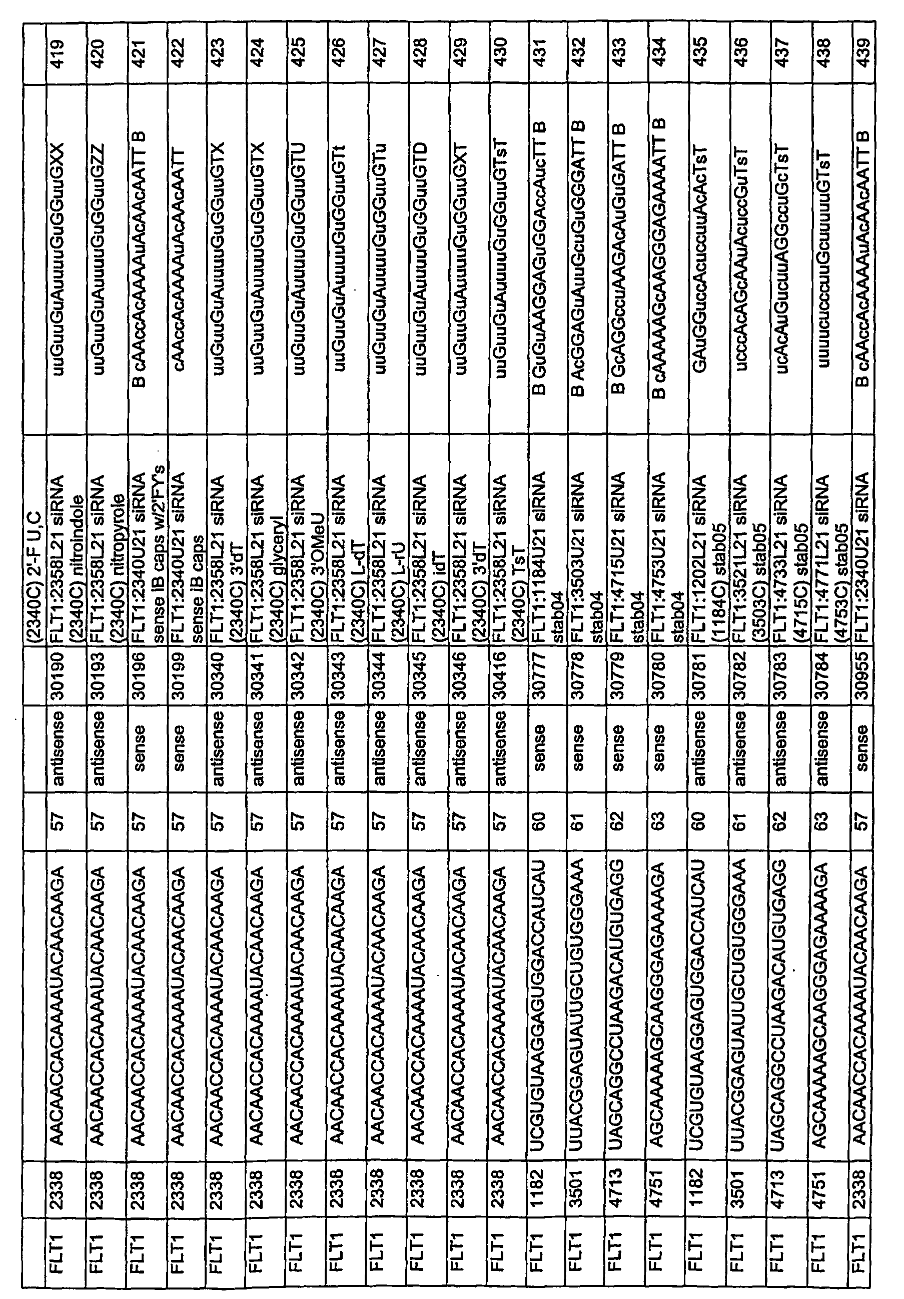

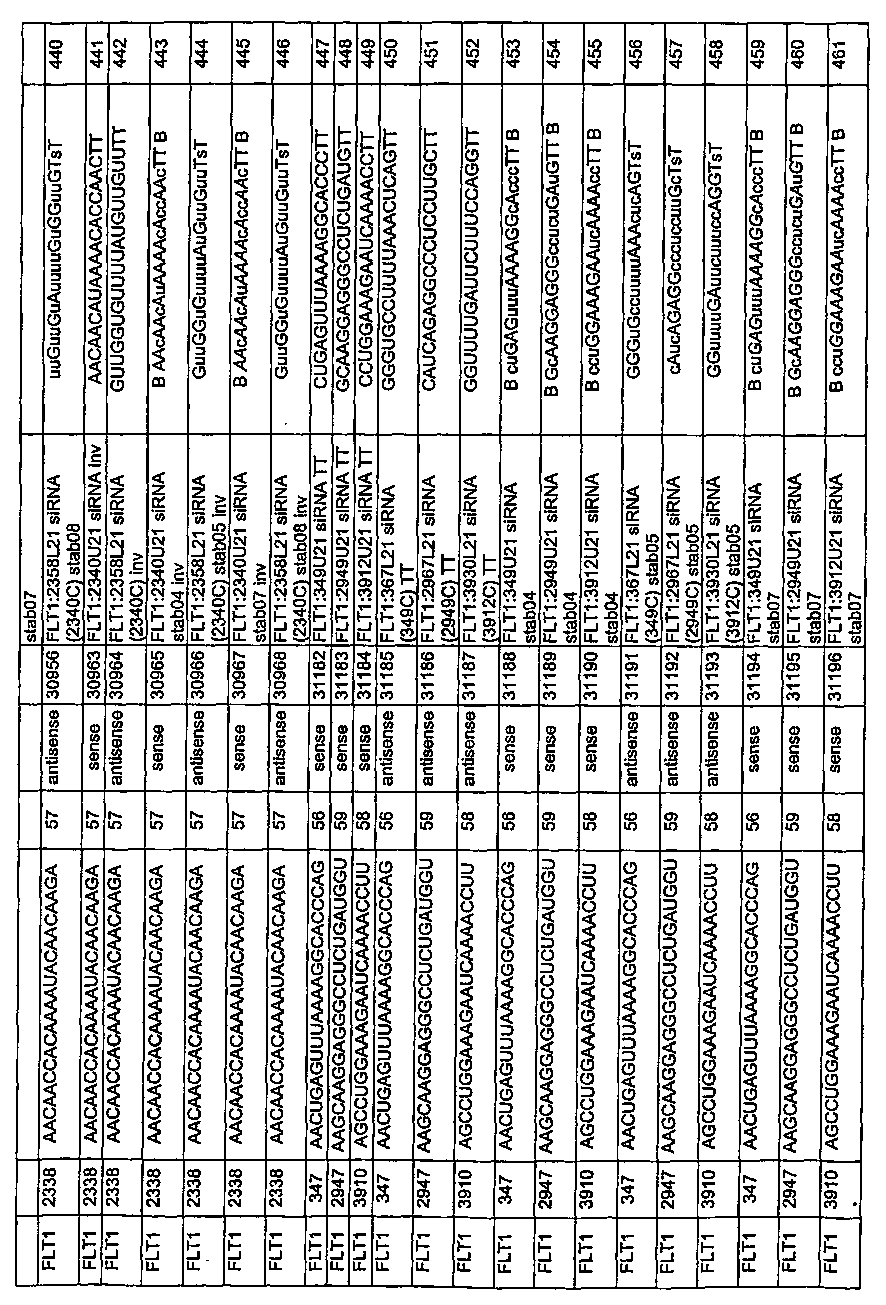

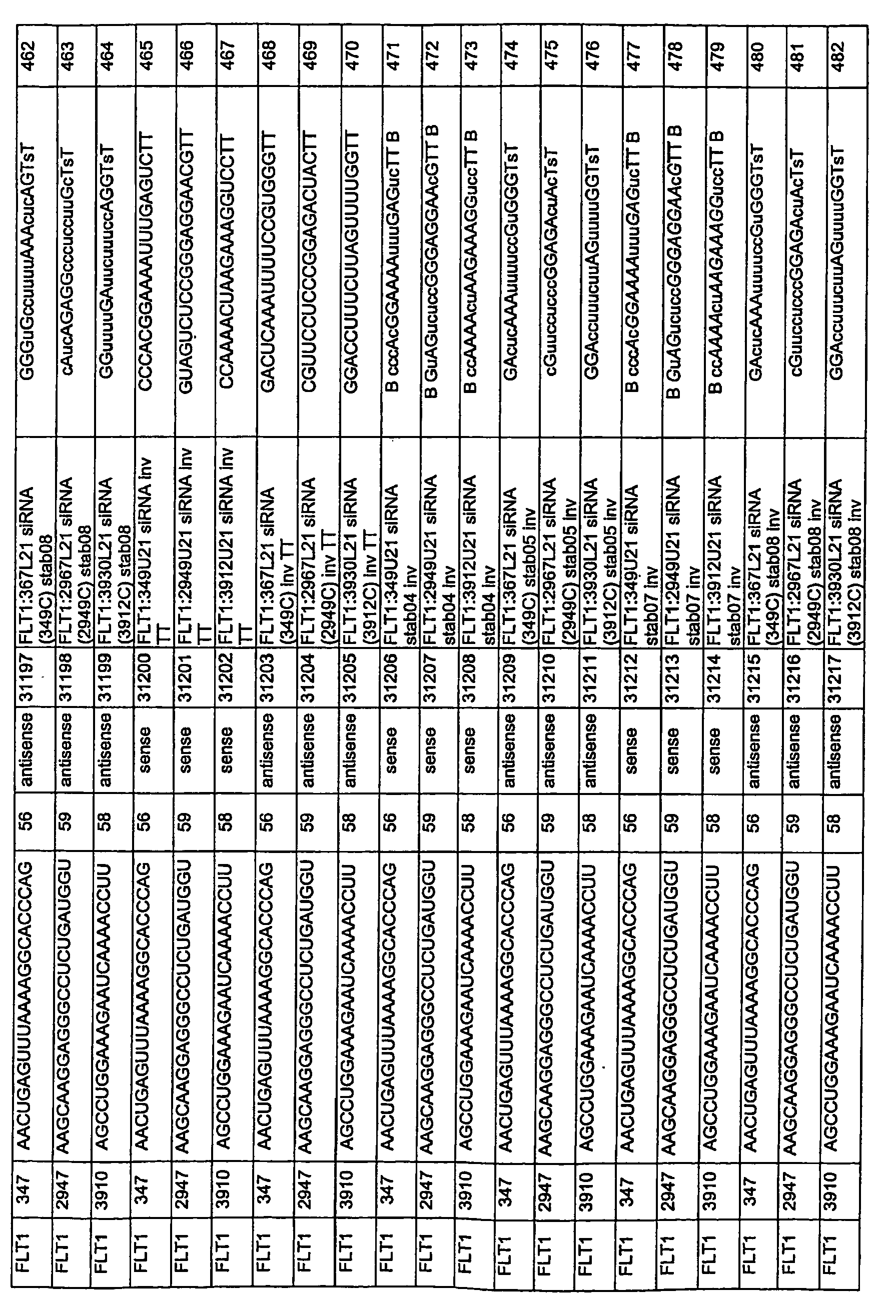

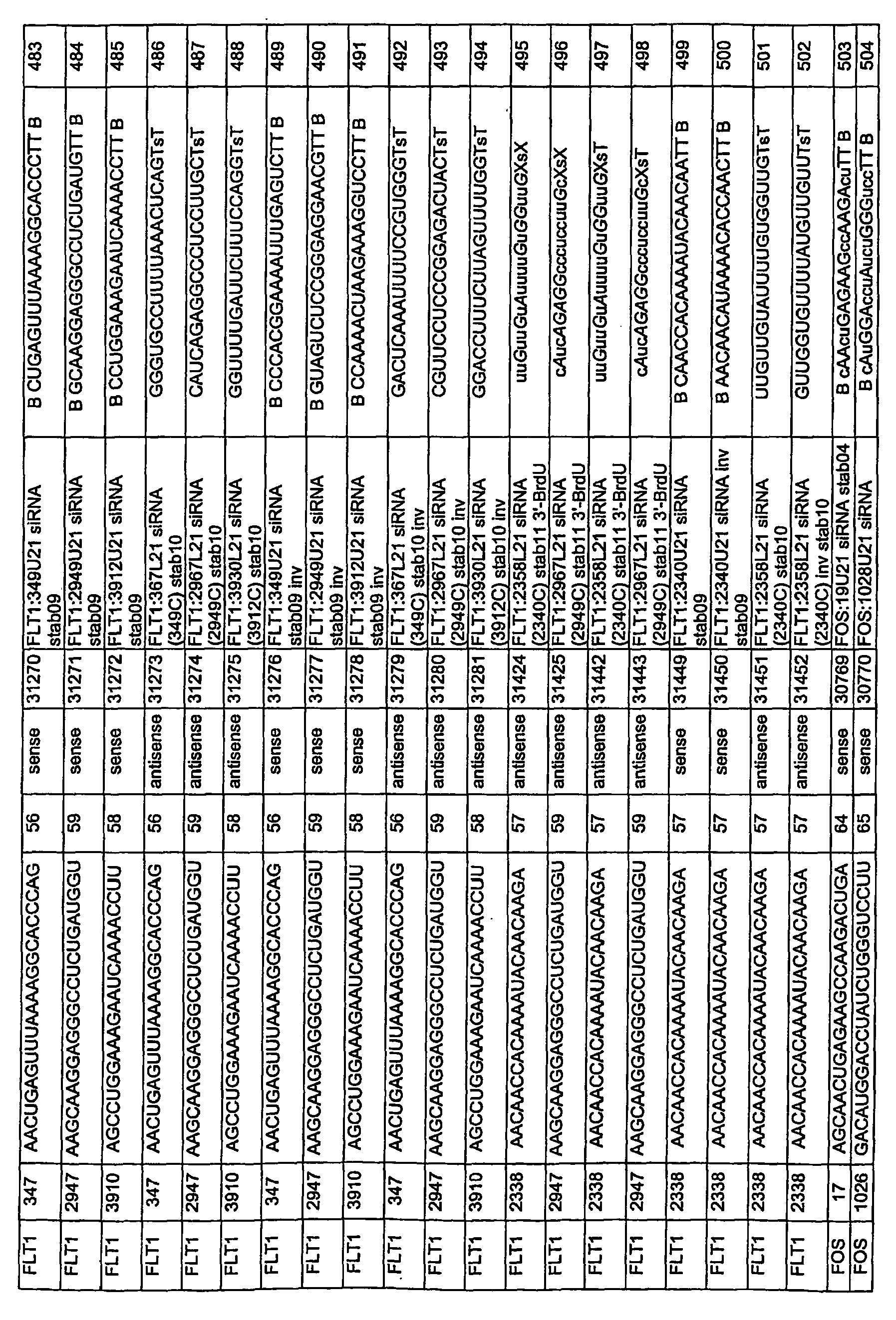

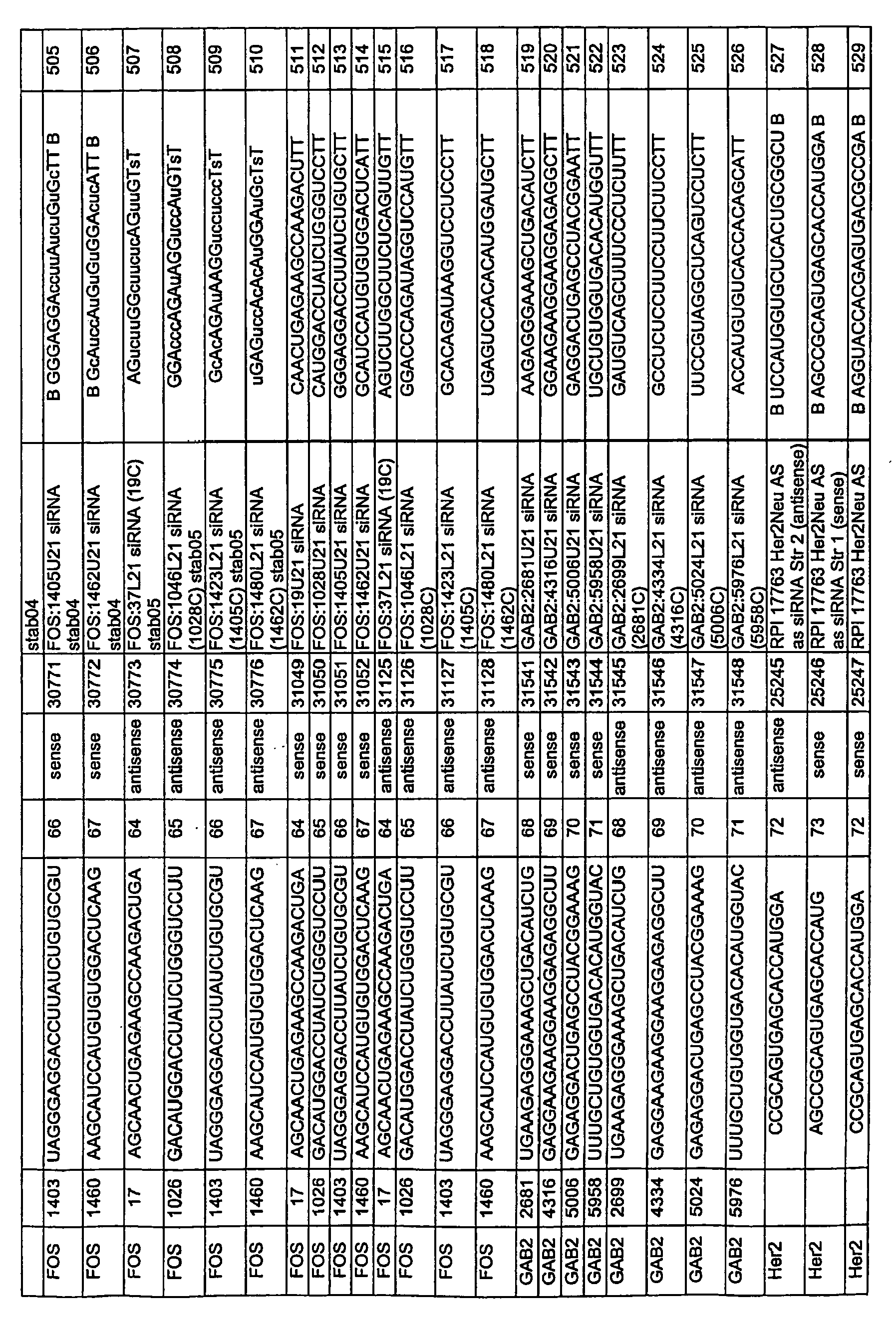

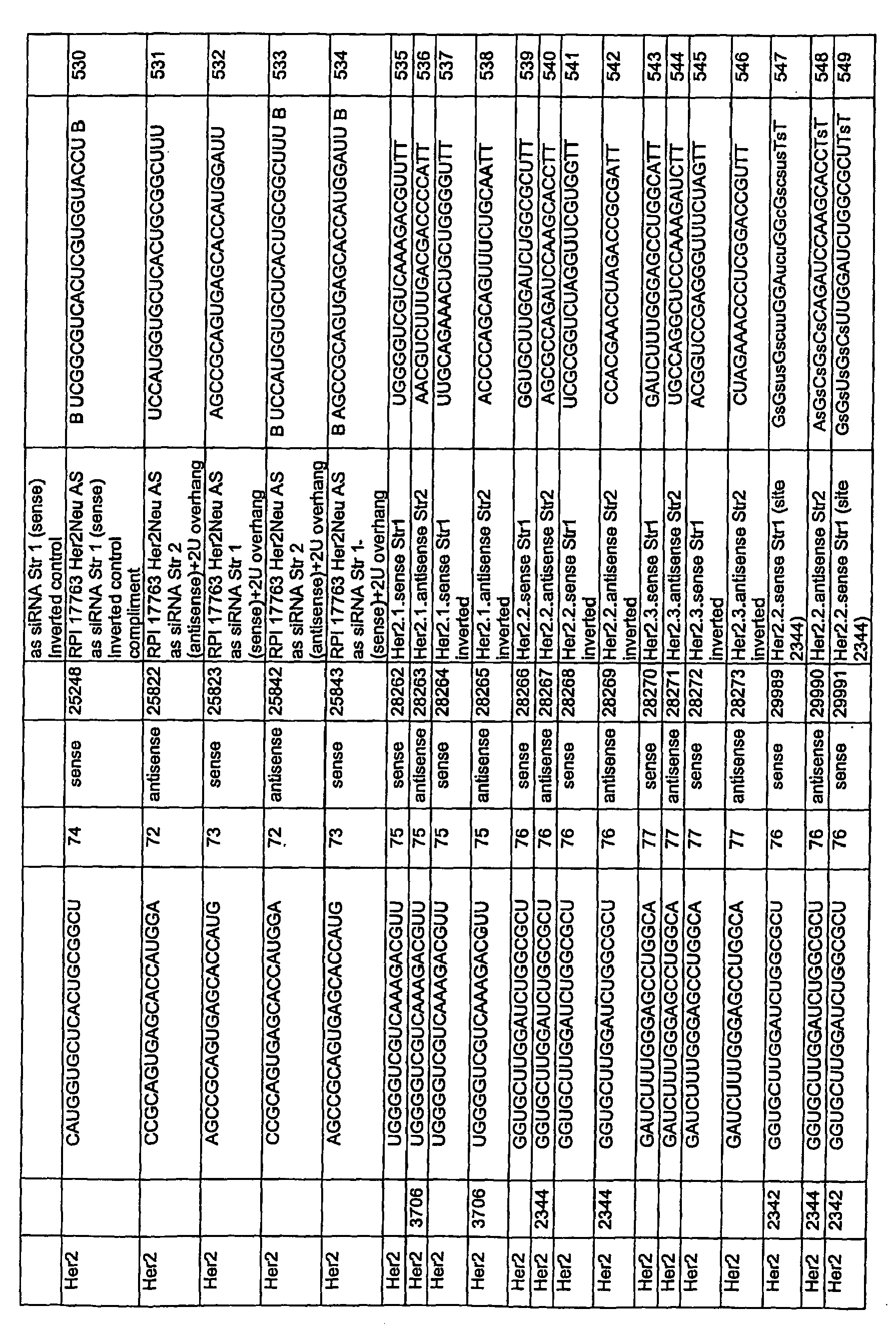

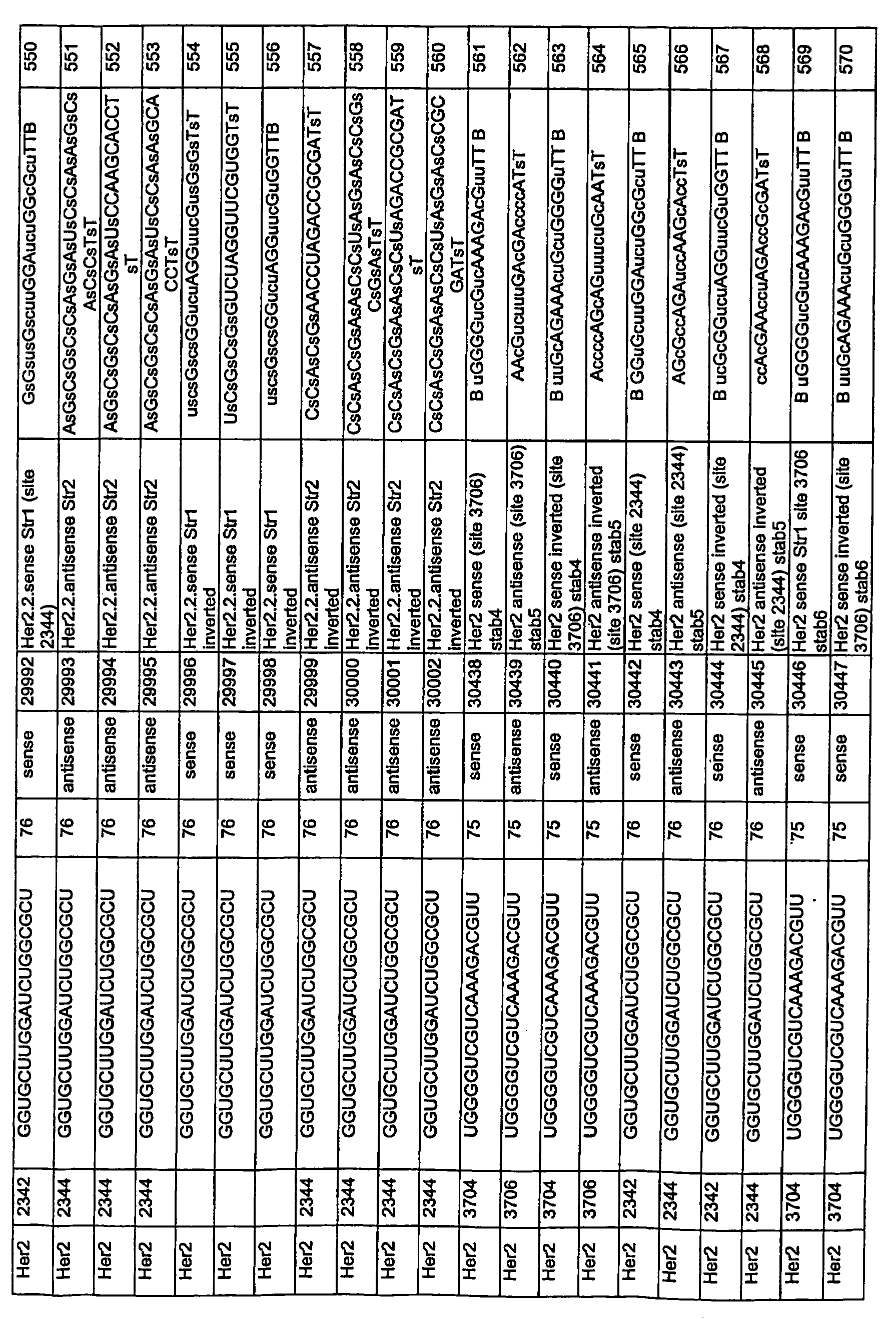

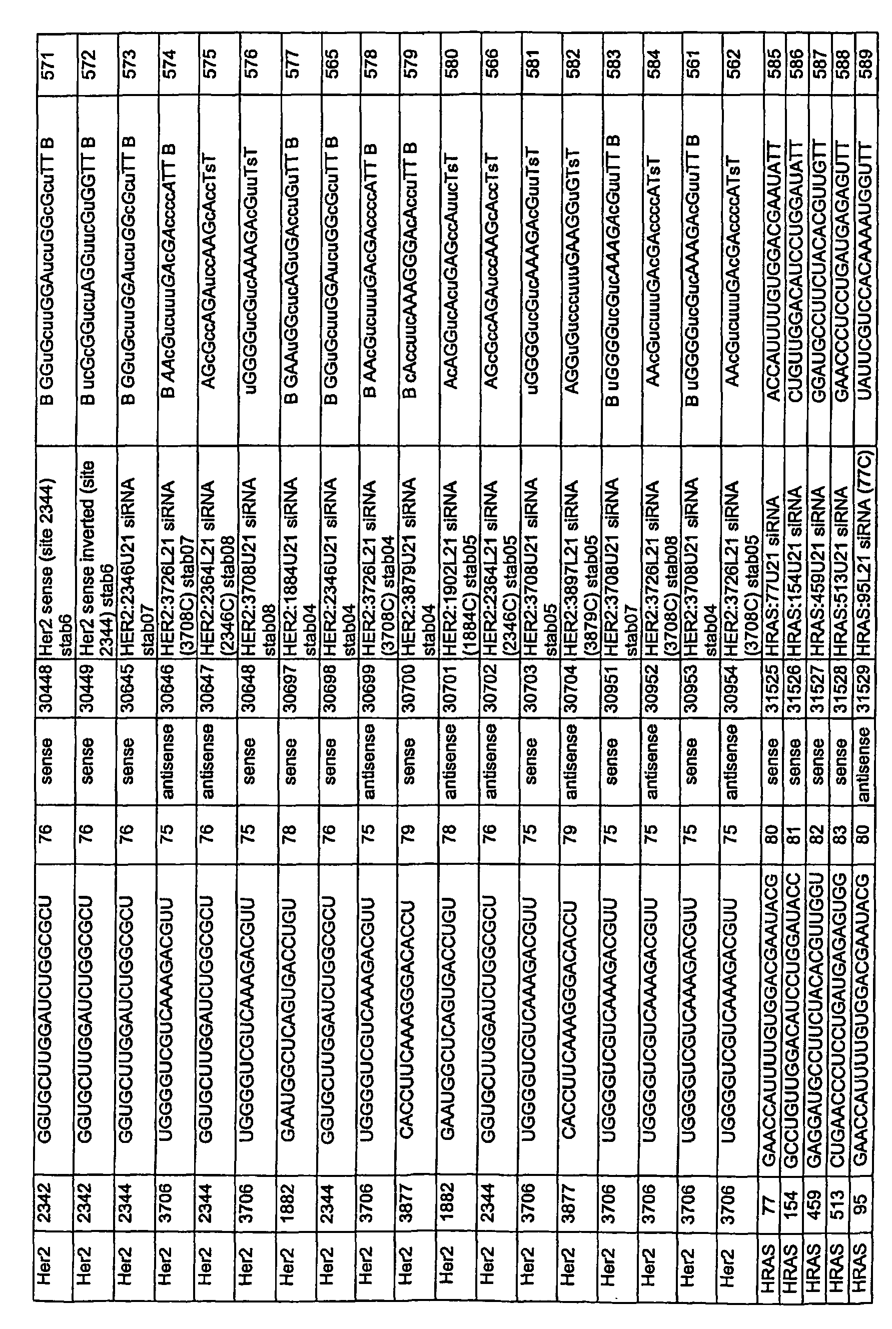

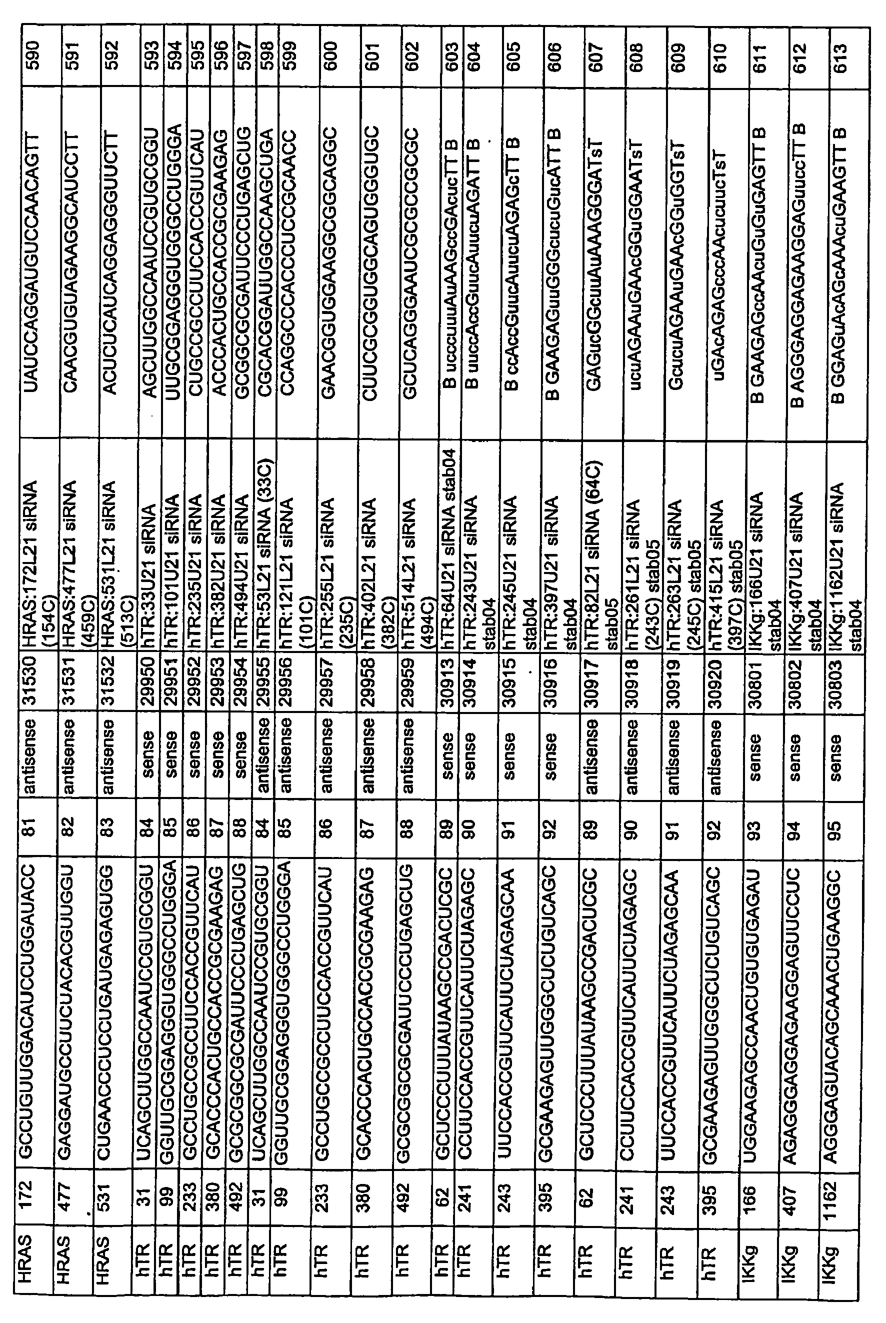

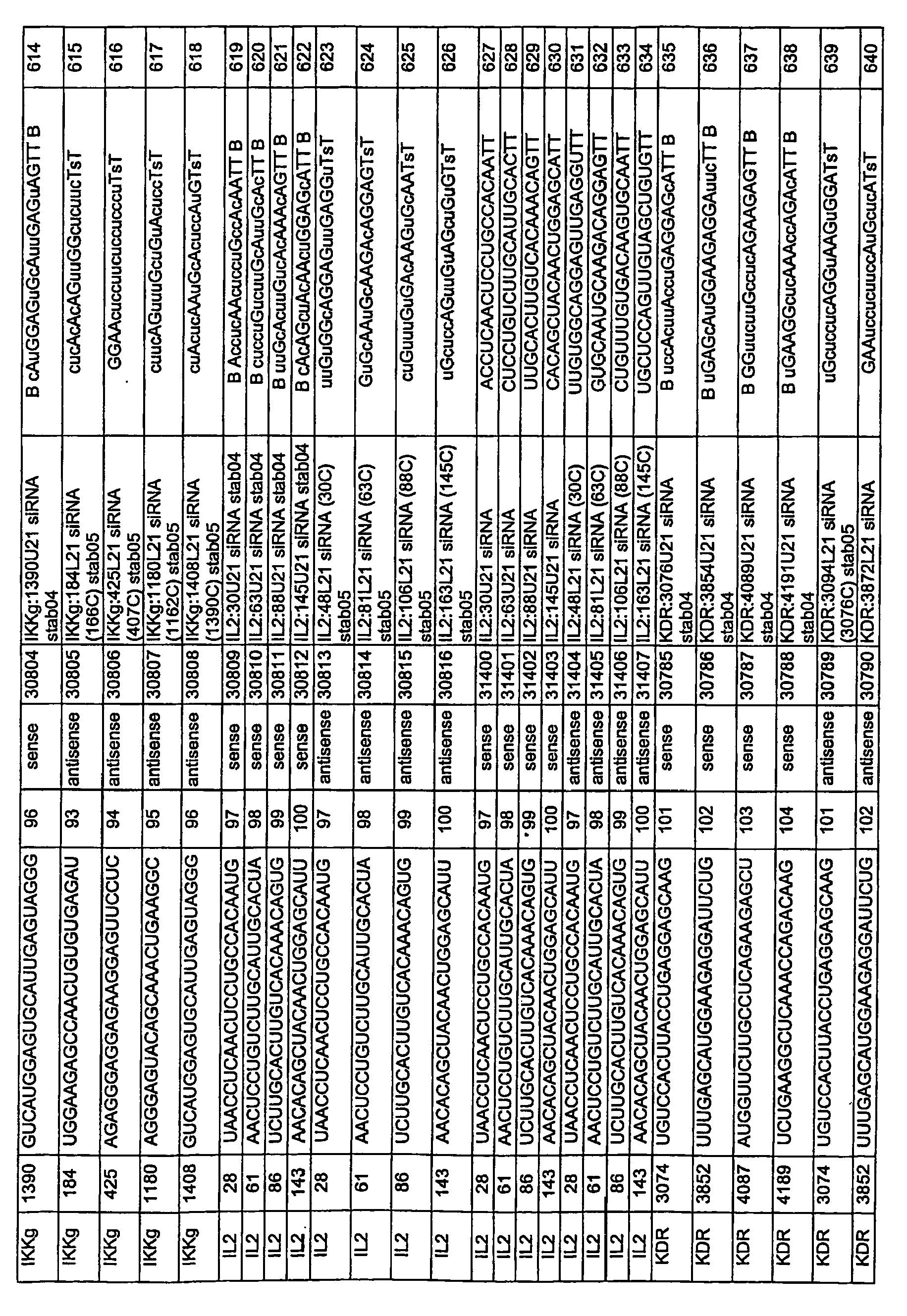

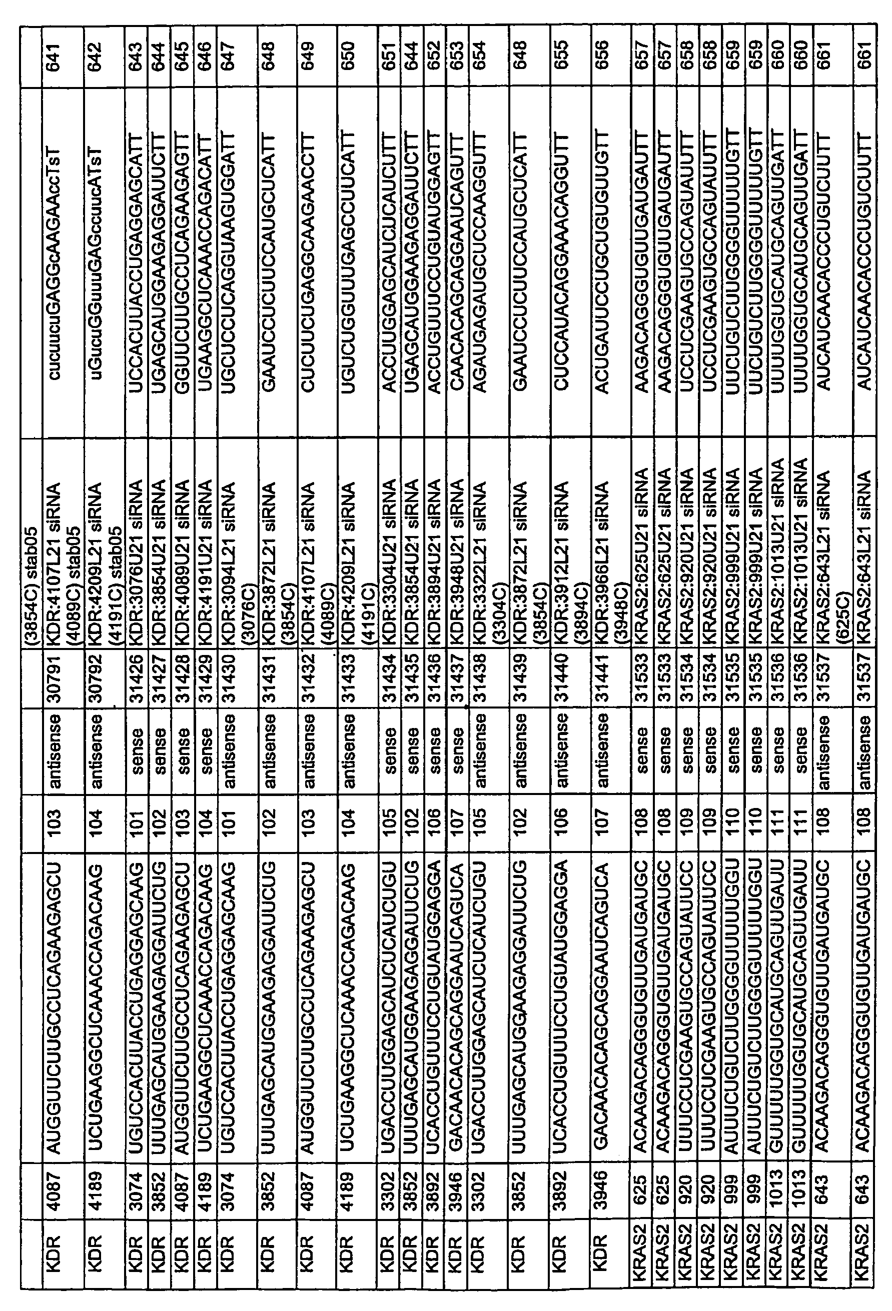

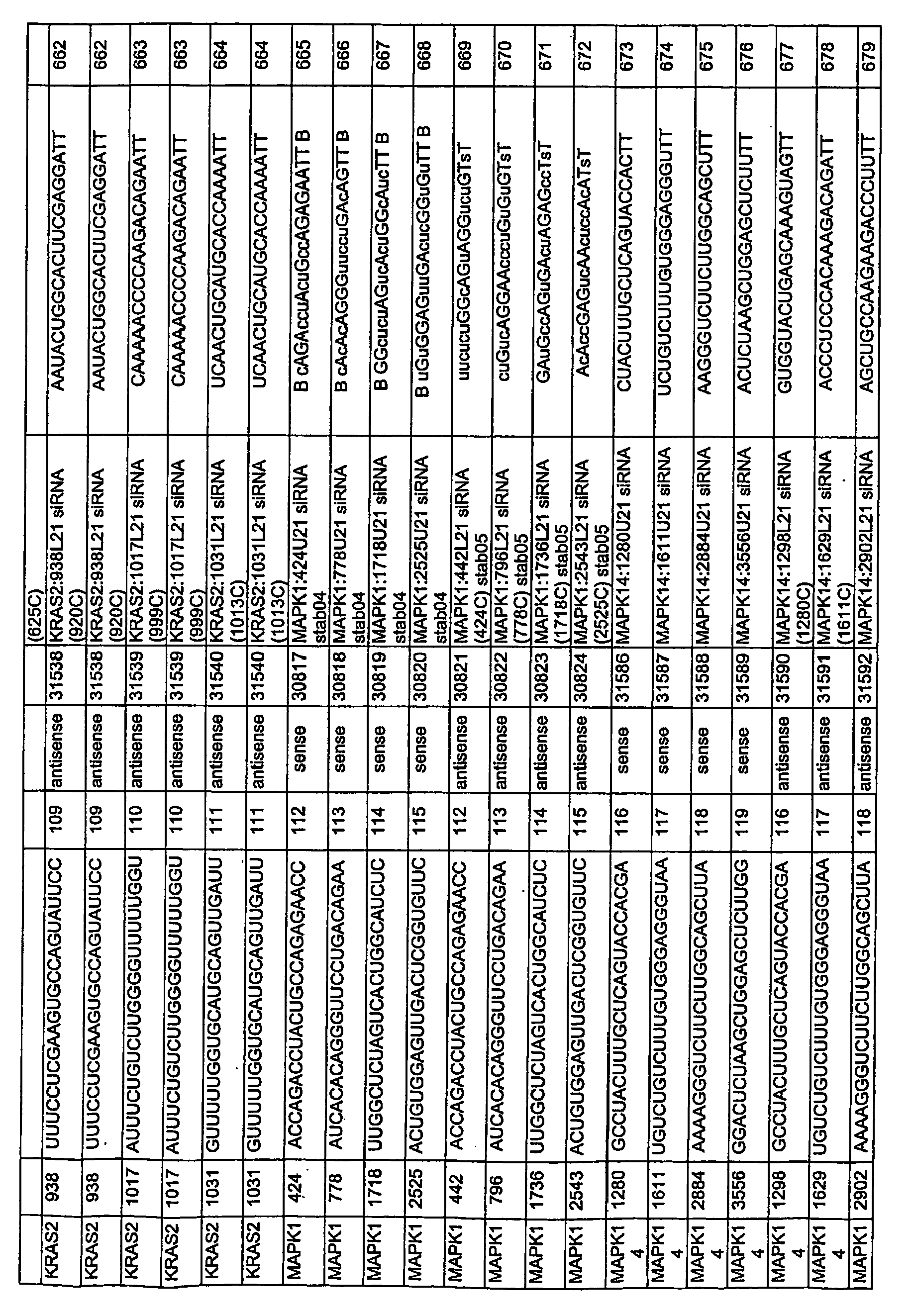

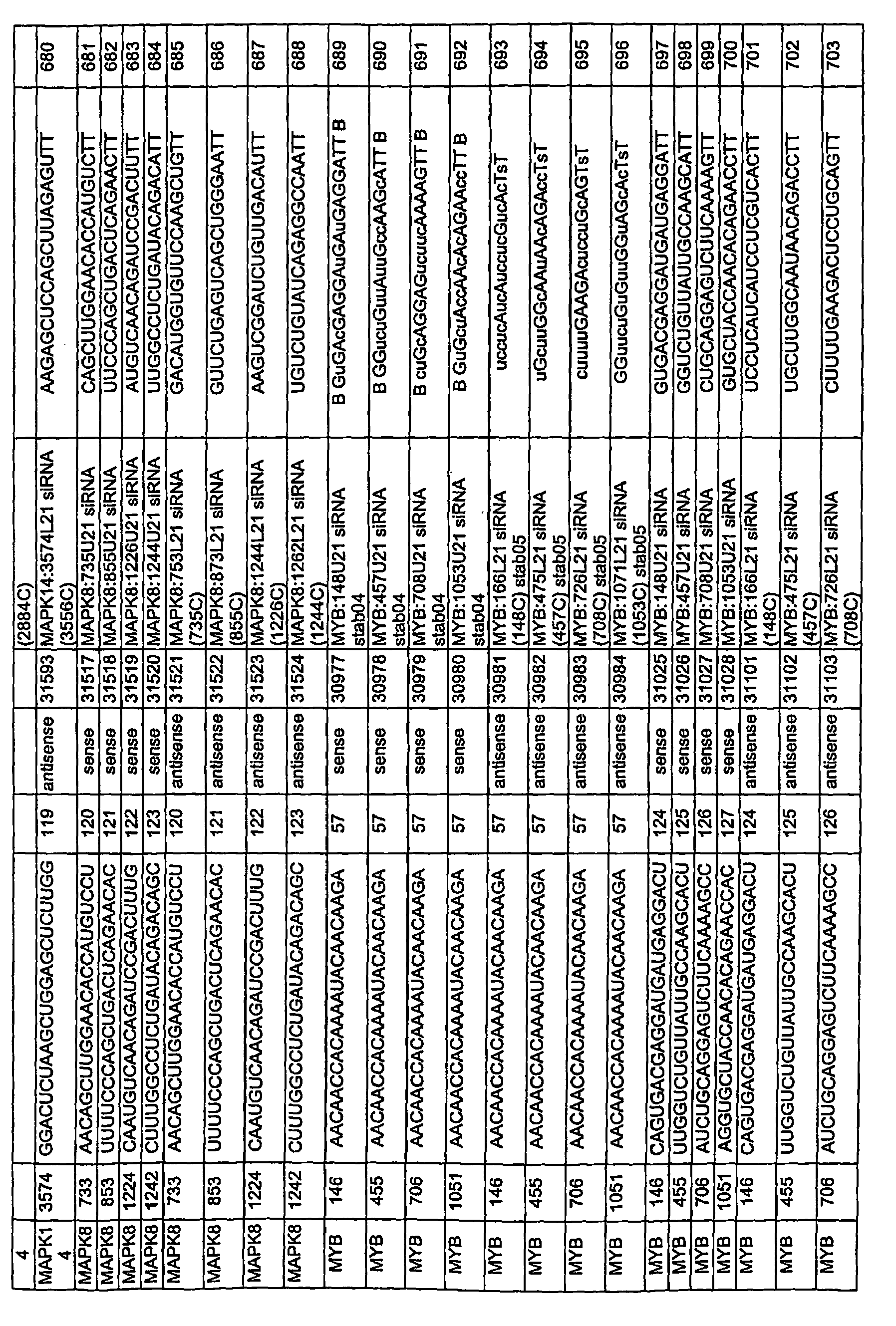

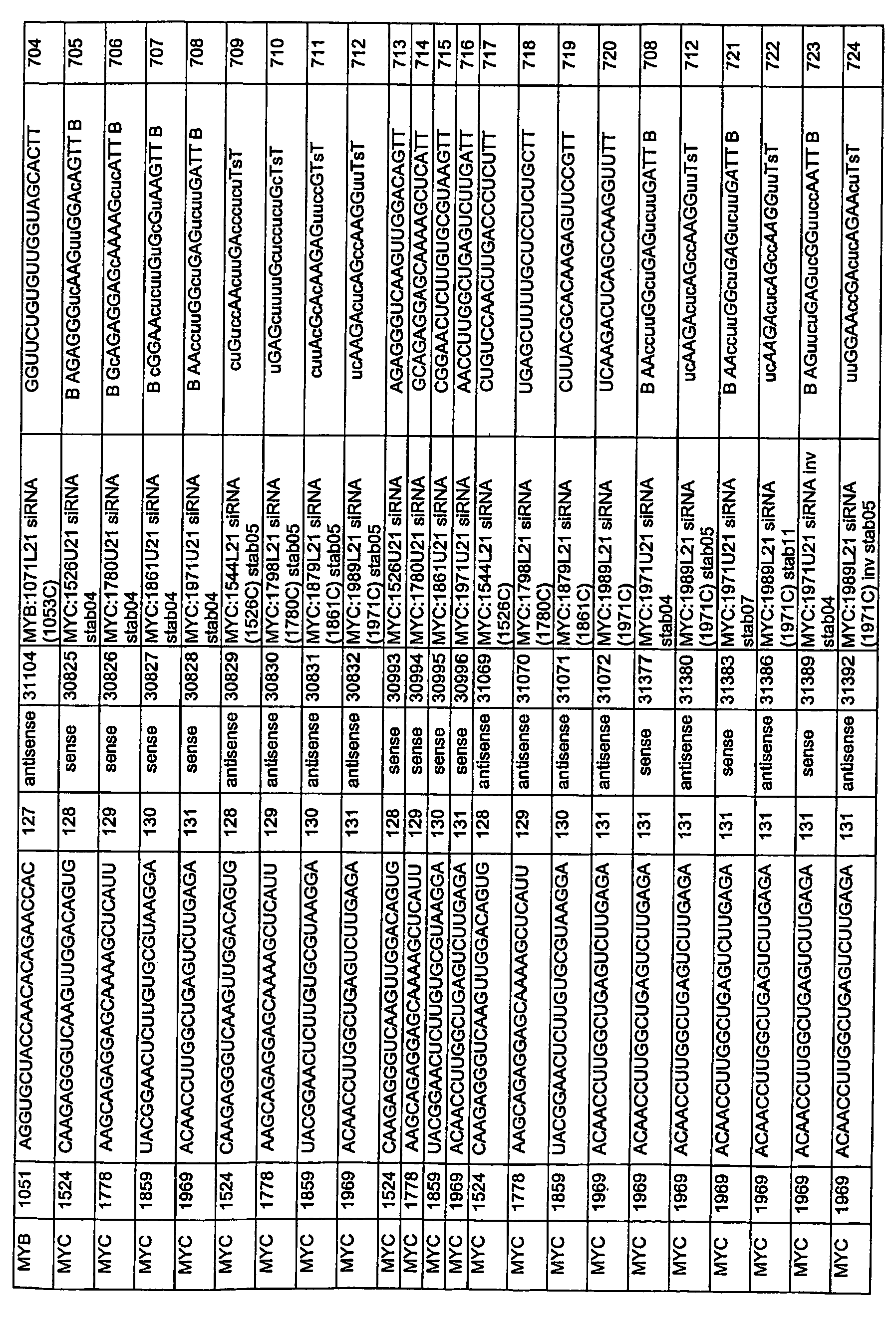

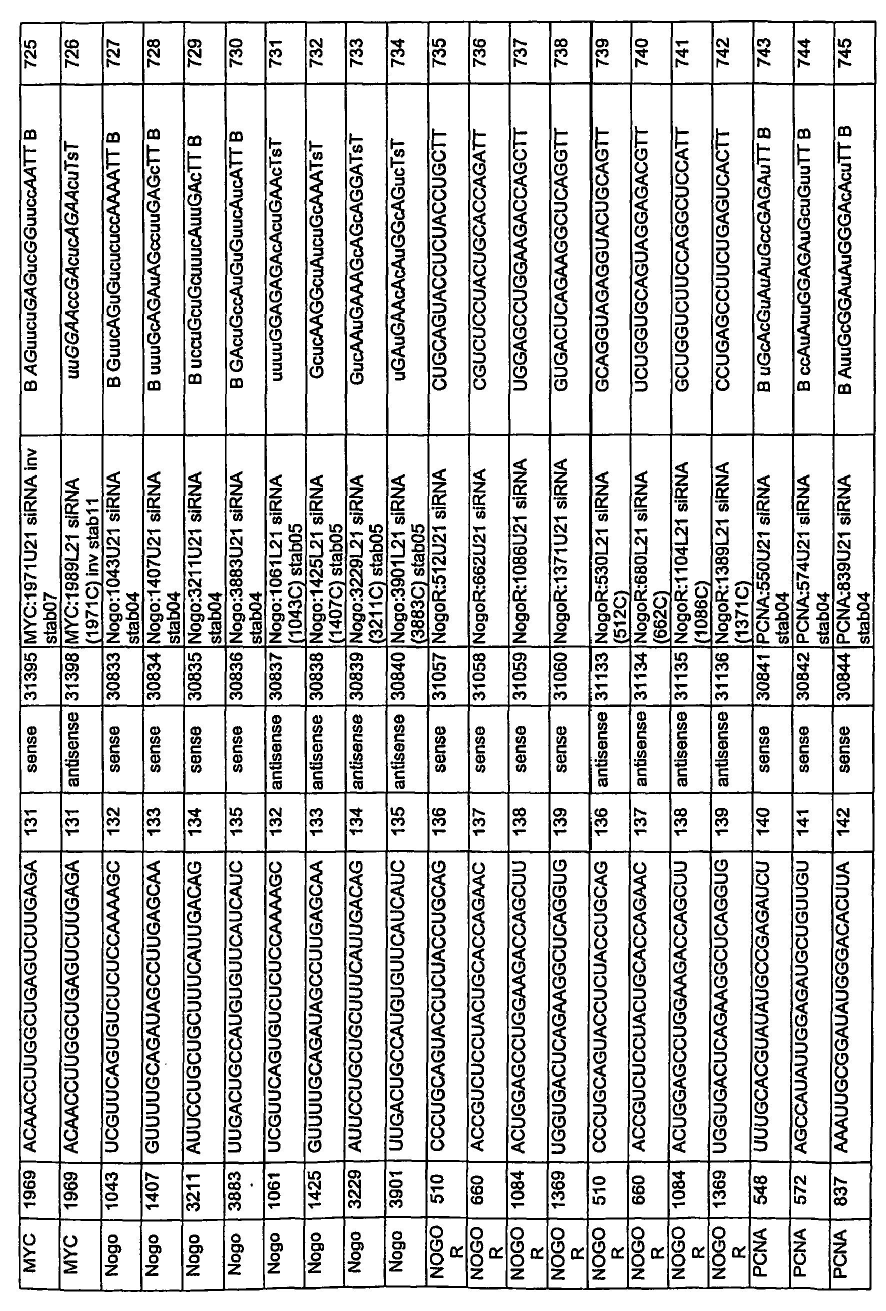

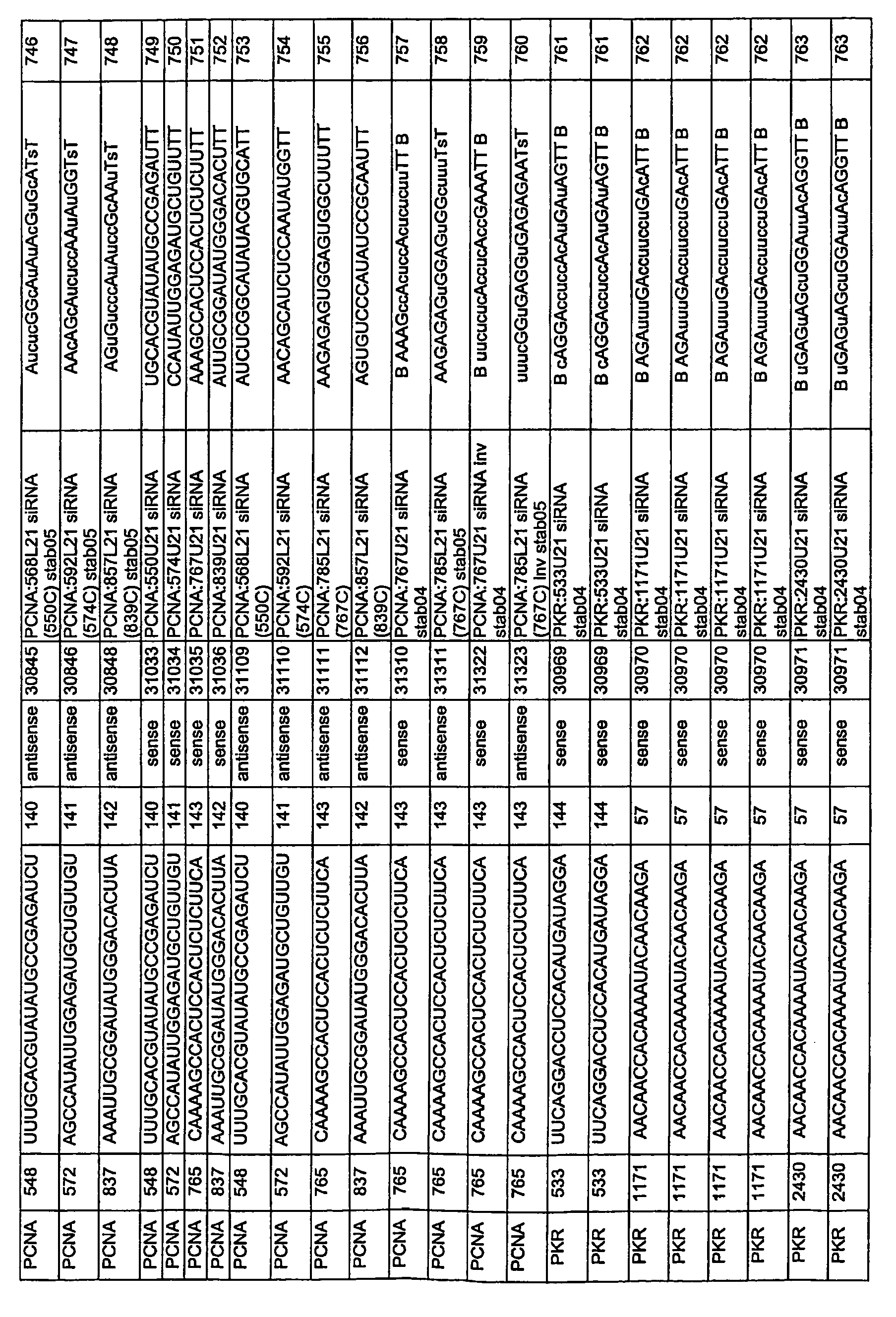

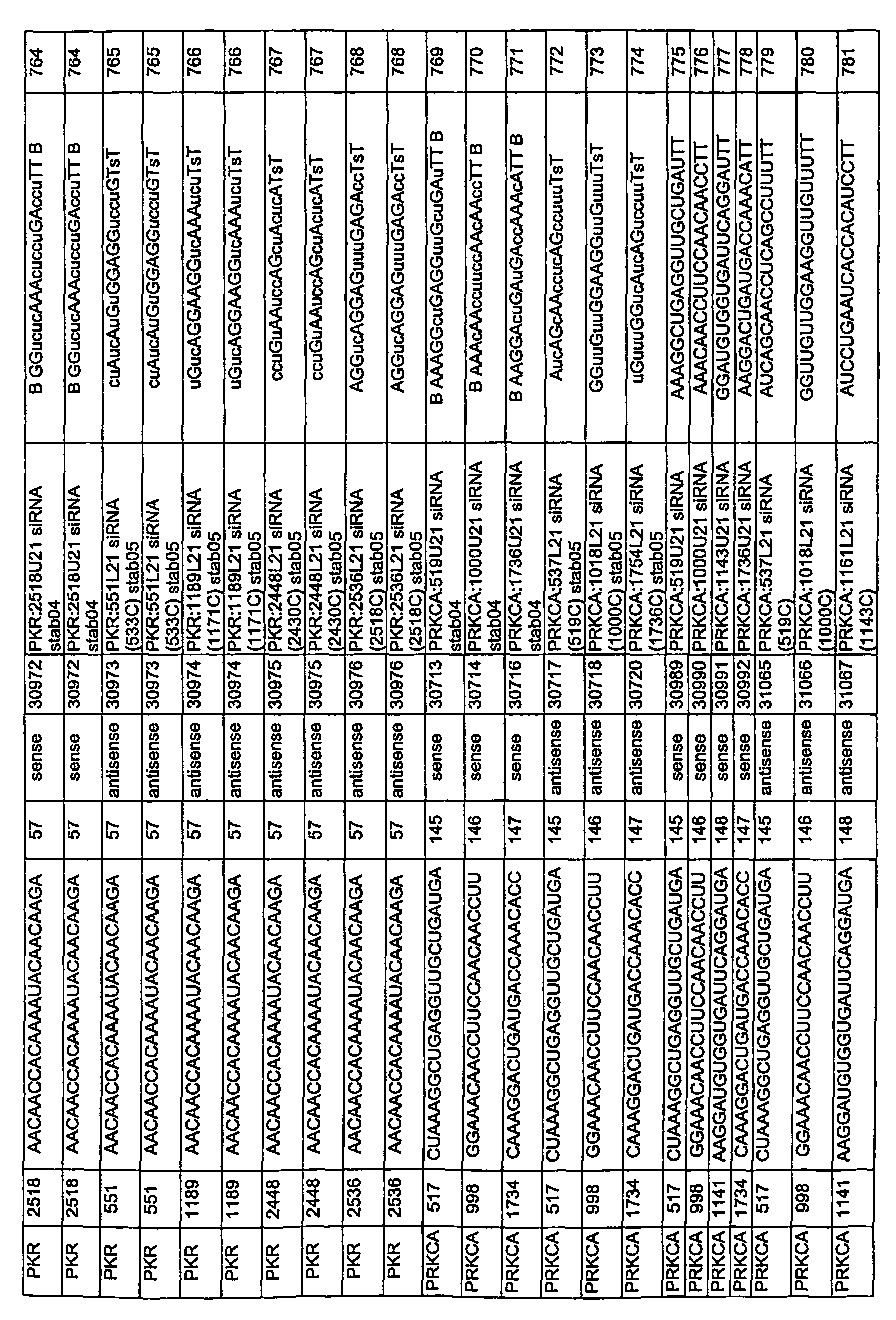

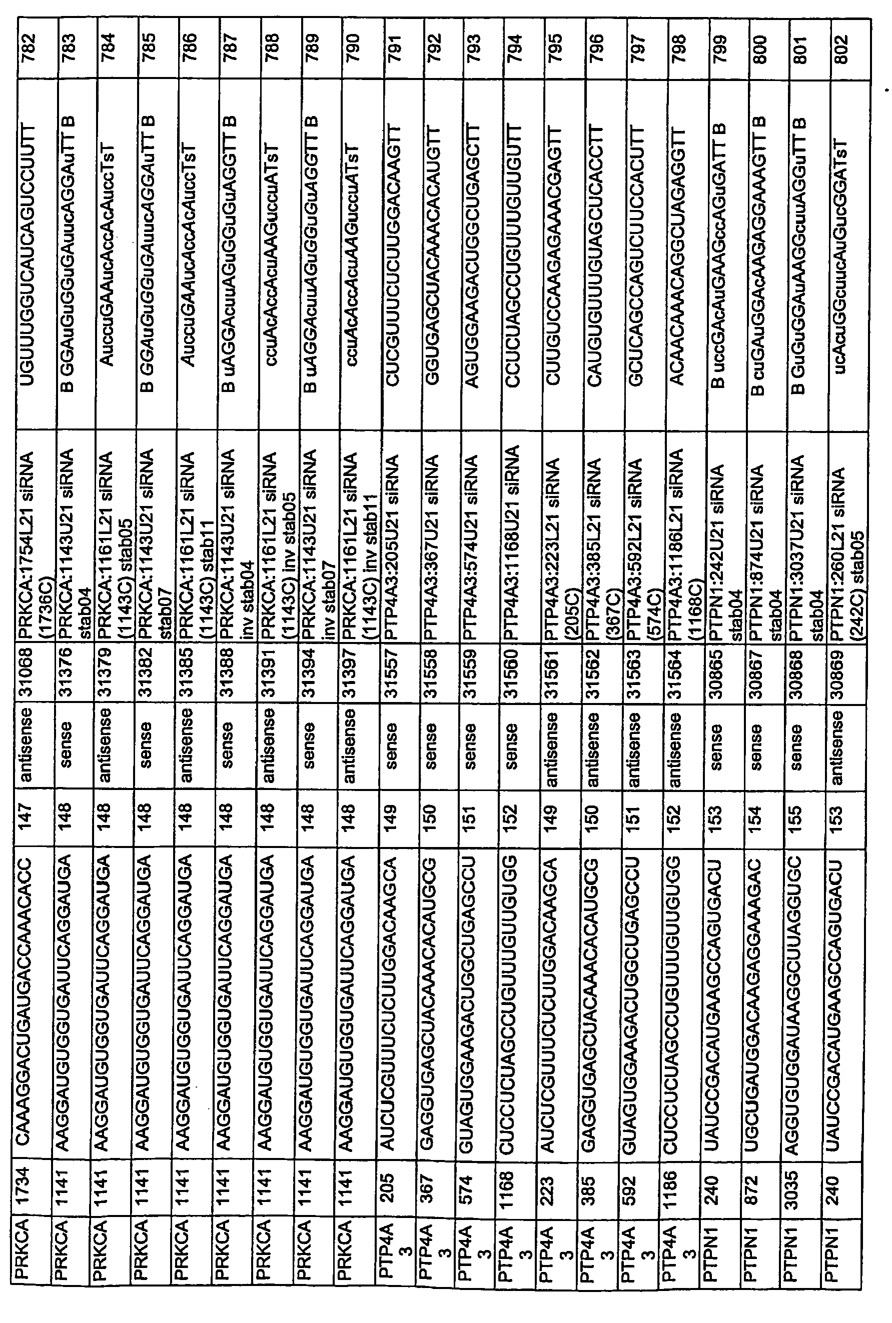

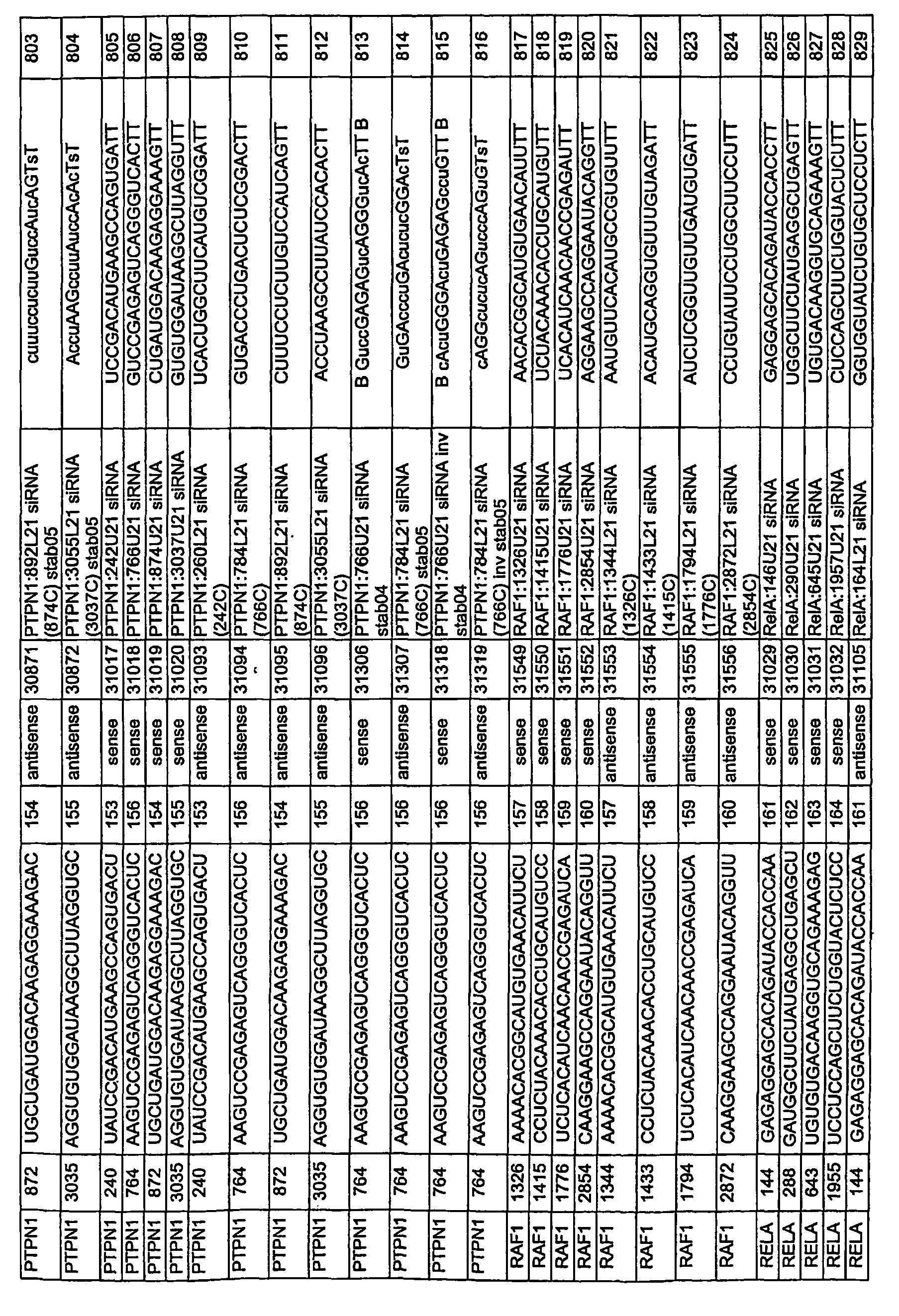

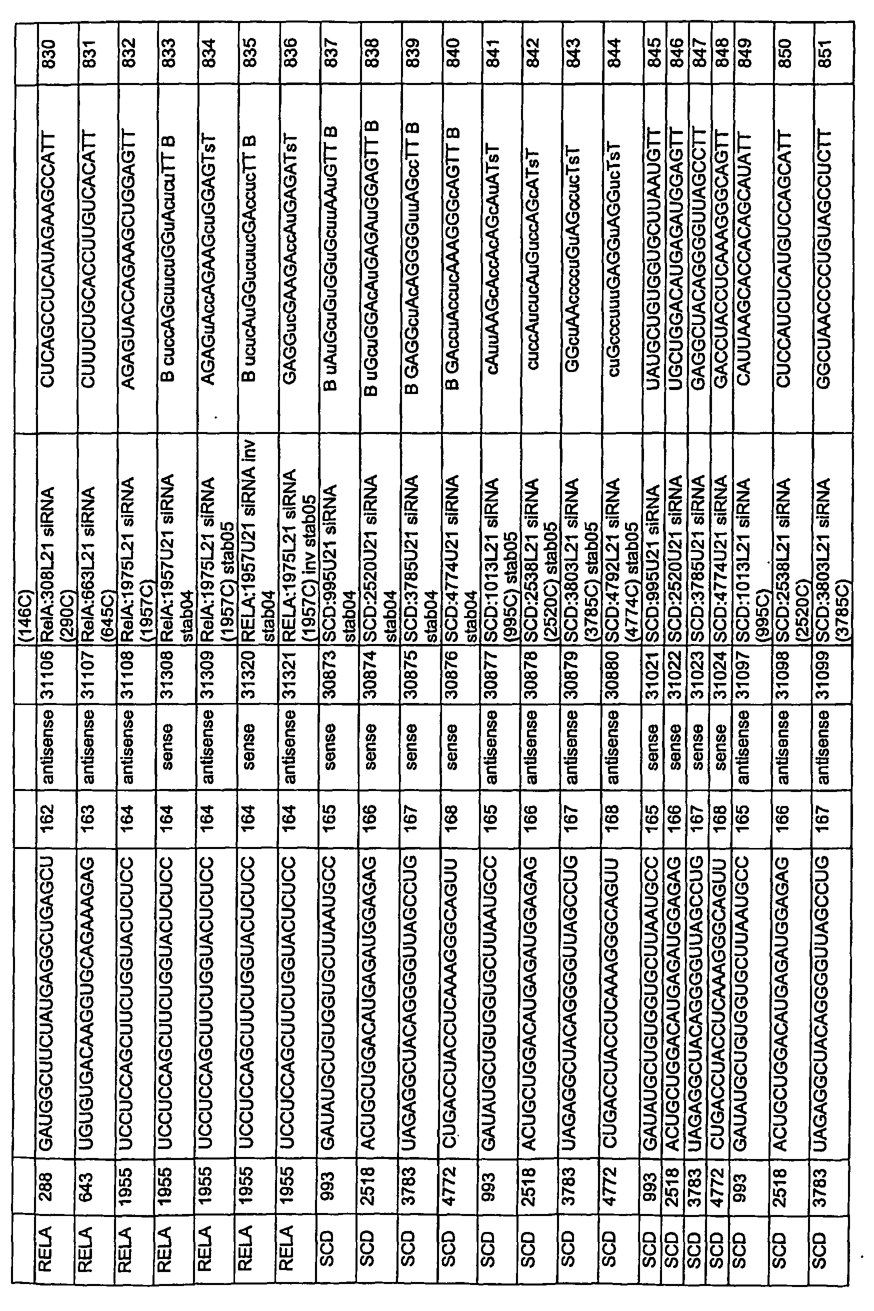

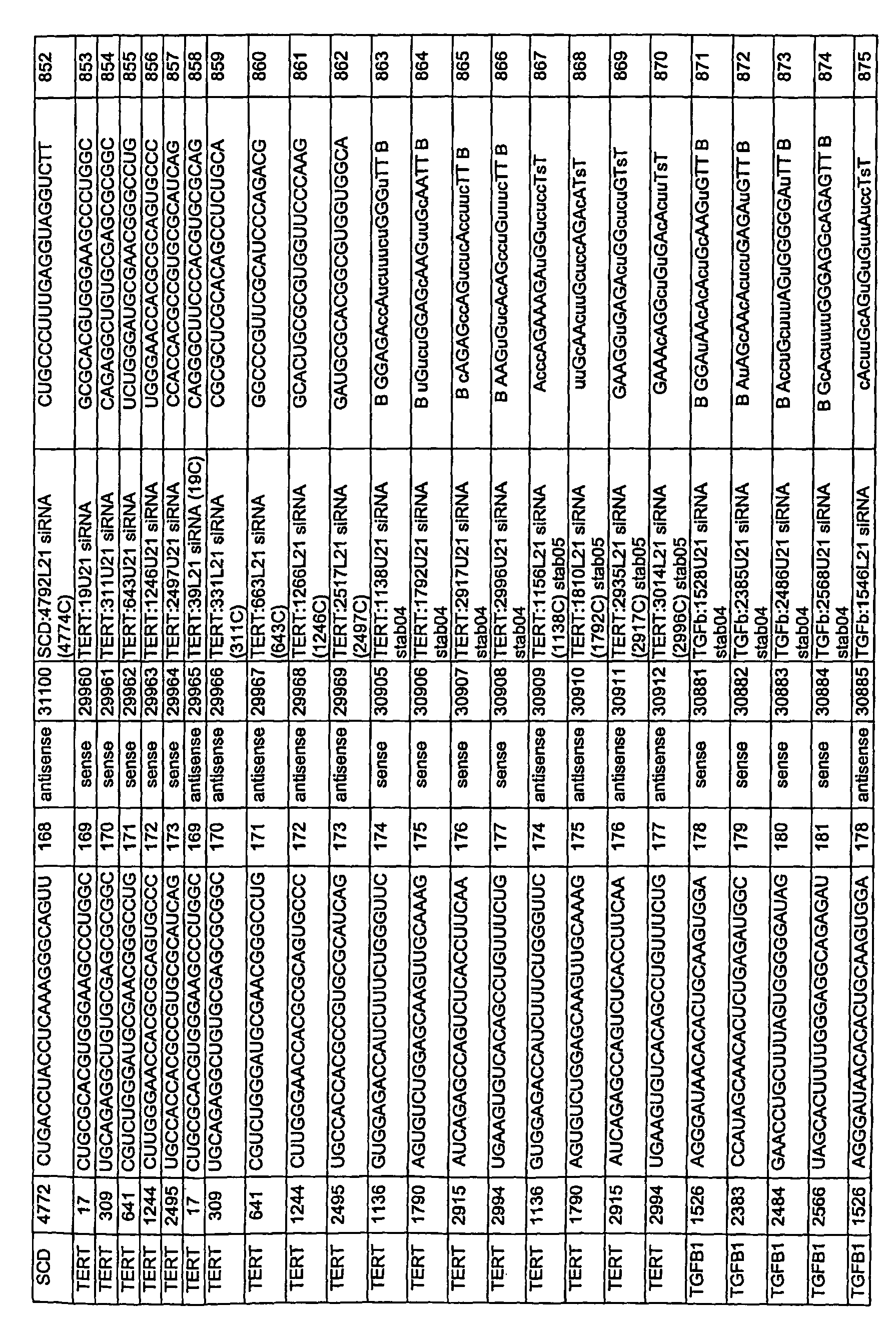

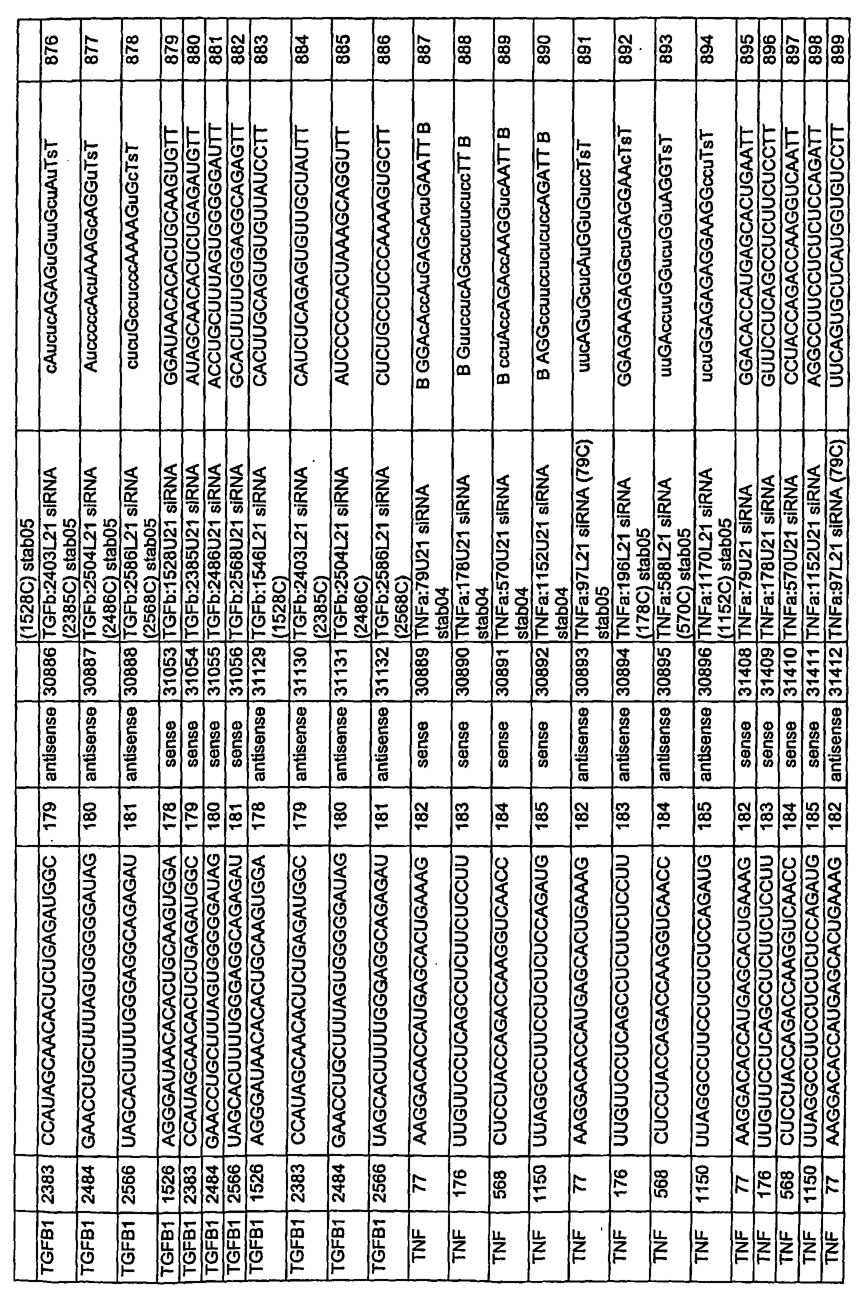

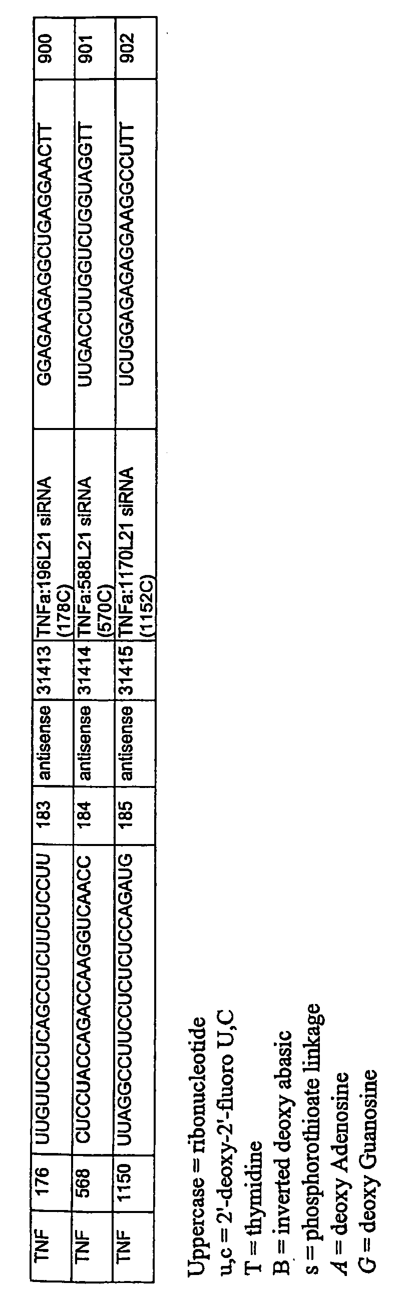

**Table II**

| A. 2.5 µmol Synthesis Cycle ABI 394 Instrument | | | | | |
|---|---|---|---|---|---|
| **Reagent** | **Equivalents** | **Amount** | **Wait Time* DNA** | **Wait Time* 2'-O-methyl** | **Wait Time*RNA** |
| | | | | | |
| Phosphoramidites | 6.5 | 163 µL | 45 sec | 2.5 min | 7.5 min |
| S-Ethyl Tetrazole | 23.8 | 238 µL | 45 sec | 2.5 min | 7.5 min |
| Acetic Anhydride | 100 | 233 µL | 5 sec | 5 sec | 5 sec |
| *N*-Methyl Imidazole | 186 | 233 µL | 5 sec | 5 sec | 5 sec |
| TCA | 176 | 2.3 mL | 21 sec | 21 sec | 21 sec |
| Iodine | 11.2 | 1.7 mL | 45 sec | 45 sec | 45 sec |
| Beaucage | 12.9 | 645 µL | 100 sec | 300 sec | 300 sec |
| Acetonitrile | NA | 6.67 mL | NA | NA | NA |

| B. 0.2 µmol Synthesis Cycle ABI 394 Instrument | | | | | |
|---|---|---|---|---|---|
| **Reagent** | **Equivalents** | **Amount** | **Wait Time* DNA** | **Wait Time* 2'-O-methyl** | **Wait Time*RNA** |
| | | | | | |
| Phosphoramidites | 15 | 31 µL | 45 sec | 233 sec | 465 sec |
| *S*-Ethyl Tetrazole | 38.7 | 31 µL | 45 sec | 233 min | 465 sec |
| Acetic Anhydride | 655 | 124 µL | 5 sec | 5 sec | 5 sec |
| *N*-Methyl Imidazole | 1245 | 124 µL | 5 sec | 5 sec | 5 sec |
| TCA | 700 | 732 µL | 10 sec | 10 sec | 10 sec |
| Iodine | 20.6 | 244 µL | 15 sec | 15 sec | 15 sec |
| Beaucage | 7.7 | 232 µL | 100 sec | 300 sec | 300 sec |
| Acetonftnie | NA | 2.64 mL | NA | NA | NA |

| C. 0.2 µmol Synthesis Cycle 96 well Instrument | | | | | |
|---|---|---|---|---|---|
| **Reagent** | **Equivalents:DNA/ 2'-O-methyl/Ribo** | **Amount: DNA/2'-O-methyl/Ribo** | **Wait Time* DNA** | **Wait Time* 2'-O-methyl** | **Wait Time* Ribo** |
| | | | | | |
| Phosphoramidites | 22/33/66 | 40/60/120 µL | 60 sec | 180 sec | 360sec |
| *S*-Ethyl Tetrazole | 70/105/210 | 40/60/120 µL | 60 sec | 180 min | 360 sec |
| Acetic Anhydride | 265/265/265 | 50/50/50 µL | 10 sec | 10 sec | 10 sec |
| *N*-Methyl Imidazole | 502/502/502 | 50/50/50 µL | 10 sec | 10 sec | 10 sec |
| TCA | 238/475/475 | 250/500/500 µL | 15 sec | 15 sec | 15 sec |
| Iodine | 6.8/6.8/6.8 | 80/80/80 µL | 30 sec | 30 sec | 30 sec |
| Beaucage | 34/51/51 | 80/120/120 | 100 sec | 200 sec | 200 sec |
| Acetonitrile | NA | 1150/1150/1150 µL | NA | NA | NA |

| | | | | | |
|---|---|---|---|---|---|
| • Wait time does not include contact time during delivery. • Tandem synthesis utilizes double coupling of linker molecule | | | | | |

**Table III**

| **Group** | **Solution on Filter (1.0 µL)** | **Stock VEGF concentration** | **Number of Animals** | **Injectate (6.0 µL)** | **Dose** | **Conc. injectate** |
|---|---|---|---|---|---|---|
| 1 | Tris-Cl pH 6.9 | NA | 5 | water | NA | NA |
| 2 | R&D Systems VEGF-carrier free 75 µM | 3.53 µg/µL | 5 | water | NA | NA |
| 3 | R&D Systems VEGF-carrier free 75 µM | 3.53 µg/µL | 5 | Site 2340 Stab1 siRNA | 10 µg/eye | 1.67 µg/µL |
| 4 | R&D Systems VEGF-carrier free 75 µM | 3.53 µg/µL | 5 | Site 2340 Stab1 siRNA | 3 µg/eye | 0.5 µg/µL |
| 5 | R&D Systems VEGF-carrier free 75 µM | 3.53 µg/µL | 5 | Site 2340 Stab1 siRNA | 1 µg/eye | 0.167 µg/µL |
| 6 | R&D Systems VEGF-carrier free 75 µM | 3.53 µg/µL | 5 | Inactive Site 2340 Stab1 siRNA | 10 µg/eye | 1.67 µg/µL |
| 7 | R&D Systems VEGF-carrier free 75 µM | 3.53 µg/µL | 5 | Inactive Site 2340 Stab1 siRNA | 3 µg/eye | 0.5 µg/µL |
| 8 | R&D Systems VEGF-carrier free 75 µM | 3.53 µg/µL | 5 | Inactive Site 2340 Stab1 siRNA | 1 µg/eye | 0.167 µg/µL |

**Table IV Non-limiting examples of Stabilization Chemistries for chemically modified siNA constructs**

| **Chemistry** | **pyrimidine** | **Purine** | **cap** | **p=S** | **Strand** |
|---|---|---|---|---|---|
| **"Stab 1"** | Ribo | Ribo | - | 5 at 5'-end 1 at 3'-end | S/AS |
| **"Stab 2"** | Ribo | Ribo | - | All linkages | Usually AS |
| **"Stab 3"** | 2'-fluoro | Ribo | - | 4 at 5'-end 4 at 3'-end | Usually S |
| **"Stab 4"** | 2'-fluoro | Ribo | 5' and 3'-ends | - | Usually S |
| **"Stab 5"** | 2'-fluoro | Ribo | - | 1 at 3'-end | Usually AS |
| **"Stab 6"** | 2'-O-Methyl | Ribo | 5' and 3'-ends | - | Usually S |
| **"Stab 7"** | 2'-fluoro | 2'-deoxy | 5' and 3'-ends | - | Usually S |
| **"Stab 8"** | 2'-fluoro | 2'-O-Methyl | - | 1 at 3'-end | Usually AS |
| **"Stab 9"** | Ribo | Ribo | 5' and 3'-ends | - | Usually S |
| **"Stab 10"** | Ribo | Ribo | - | 1 at 3'-end | Usually AS |
| **"Stab 11"** | 2'-fluoro | 2'-deoxy | - | 1 at 3'-end | Usually AS |

| | | | | | |
|---|---|---|---|---|---|
| CAP = any terminal cap, see for example **Figure 10.** All Stab 1-11 chemistries can comprise 3'-terminal thymidine (TT) residues All Stab 1-11 chemistries typically comprise 21 nucleotides, but can vary as described herein. S = sense strand AS = antisense strand | | | | | |

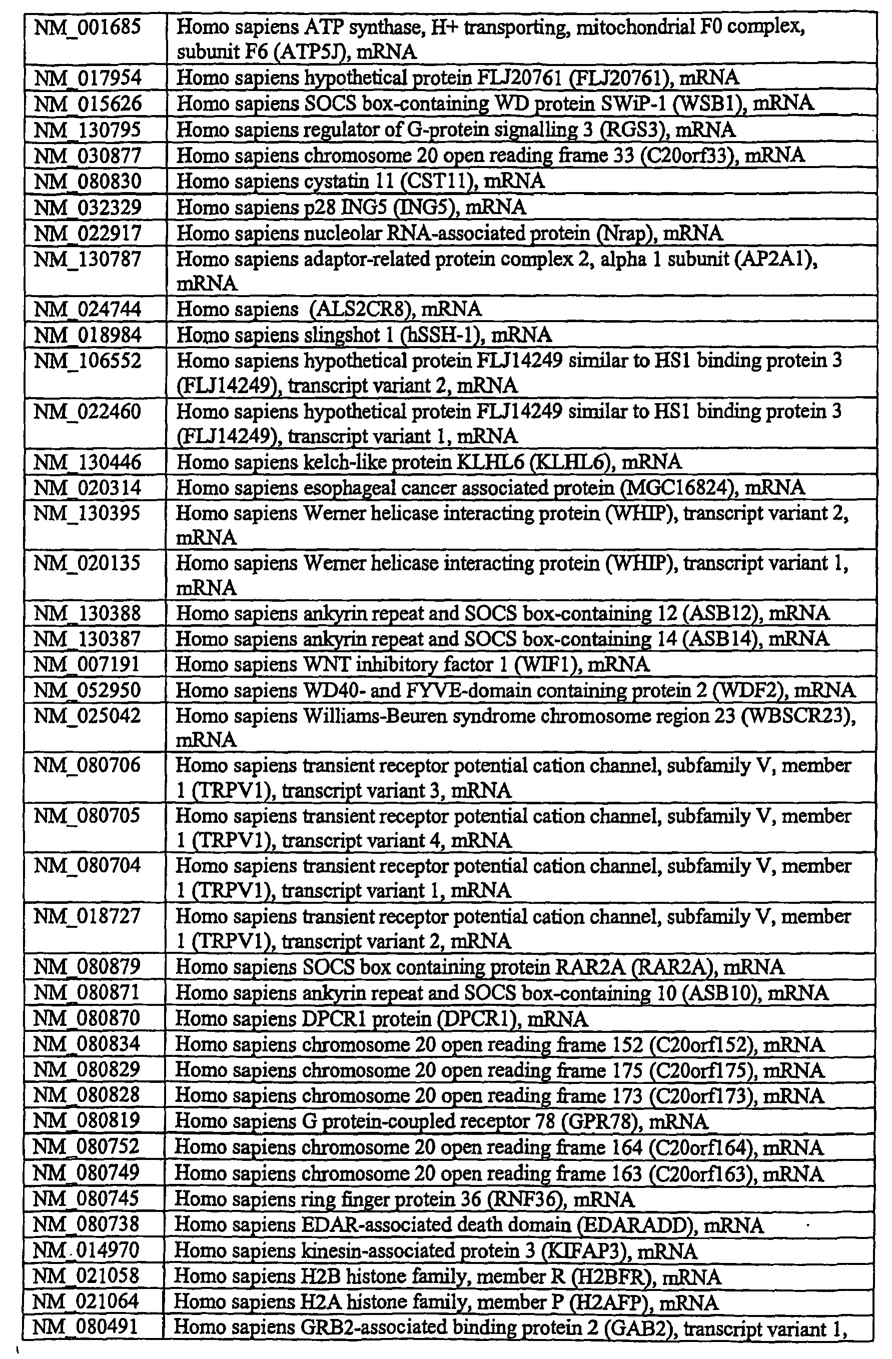

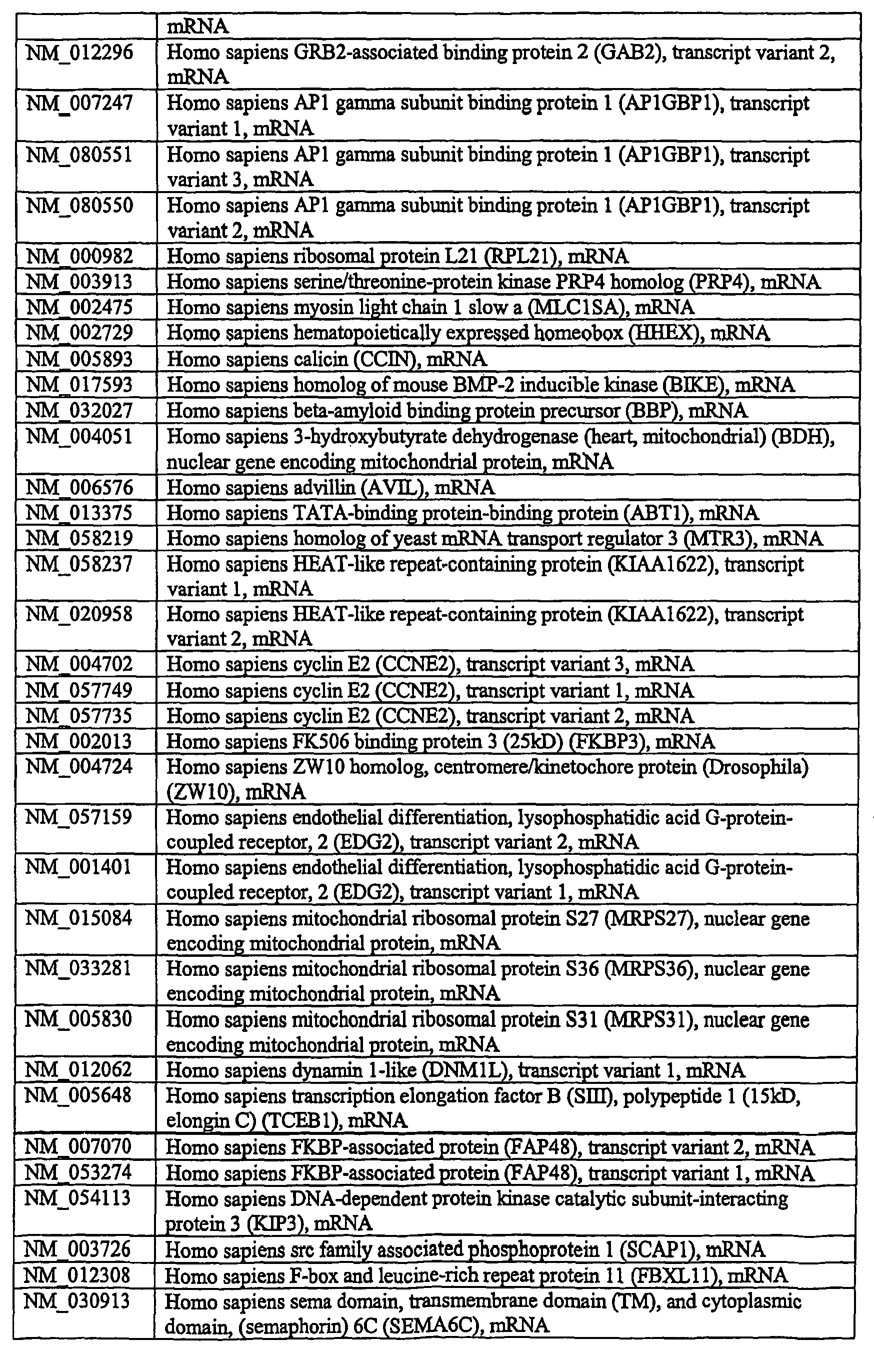

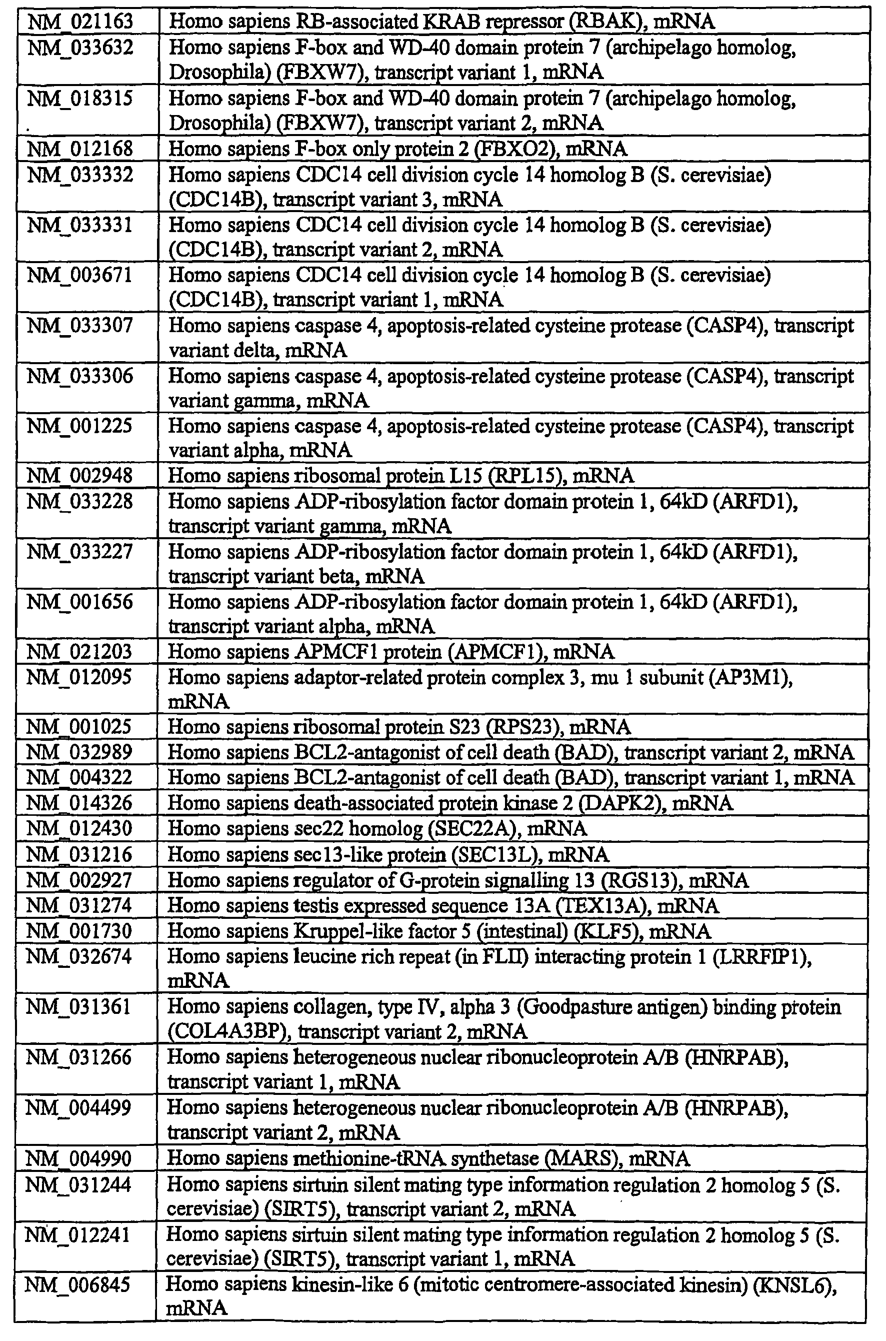

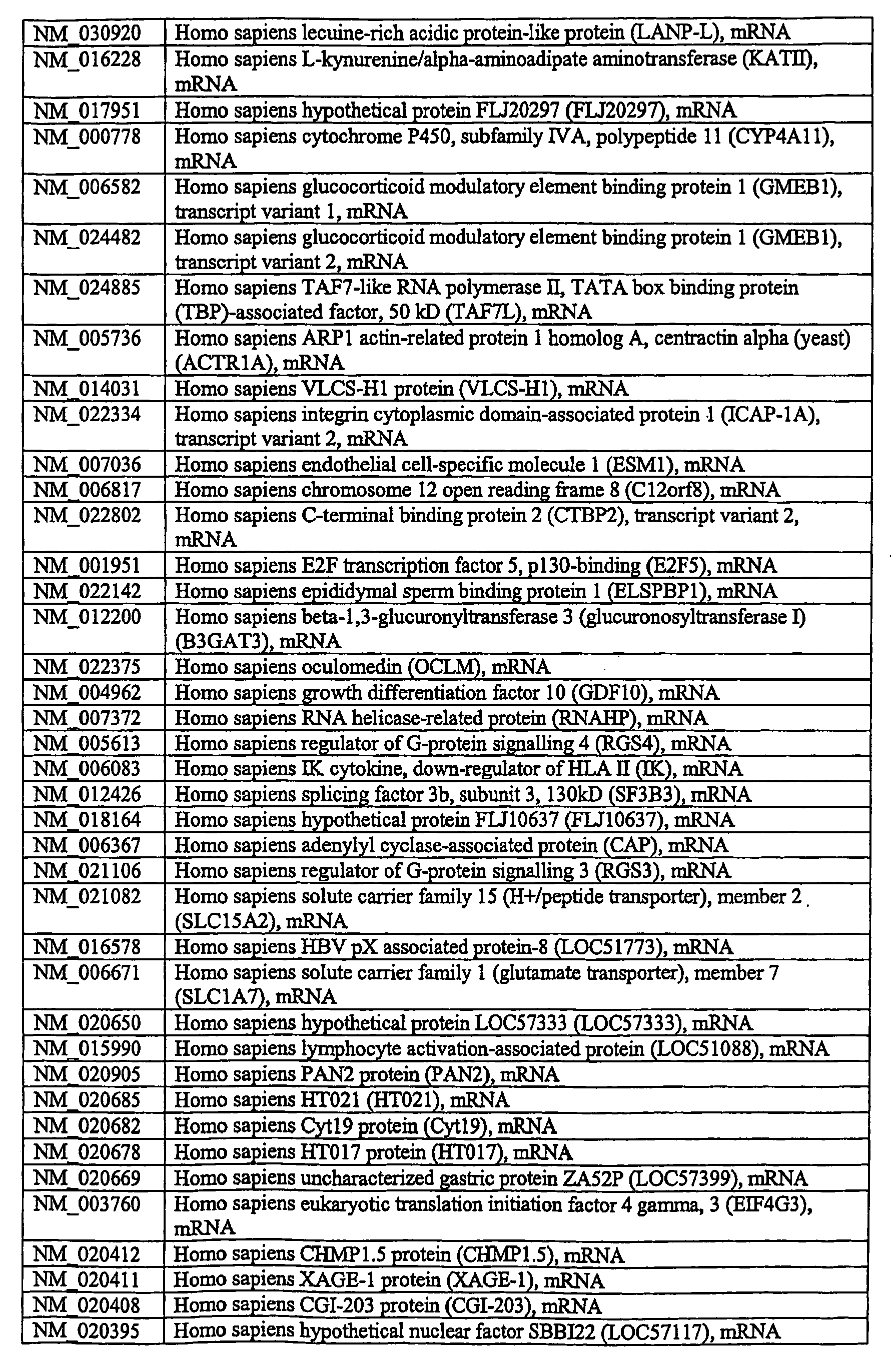

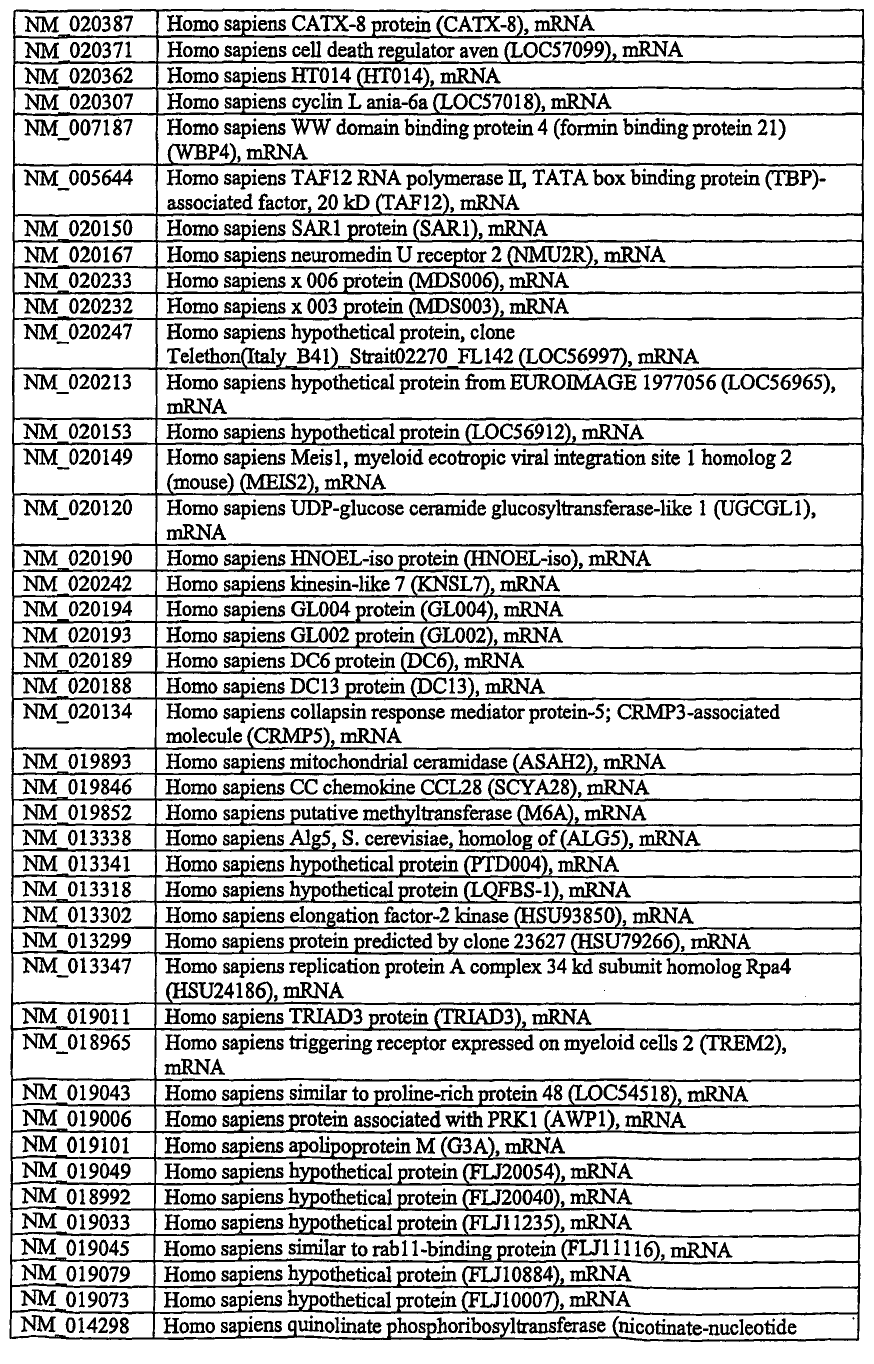

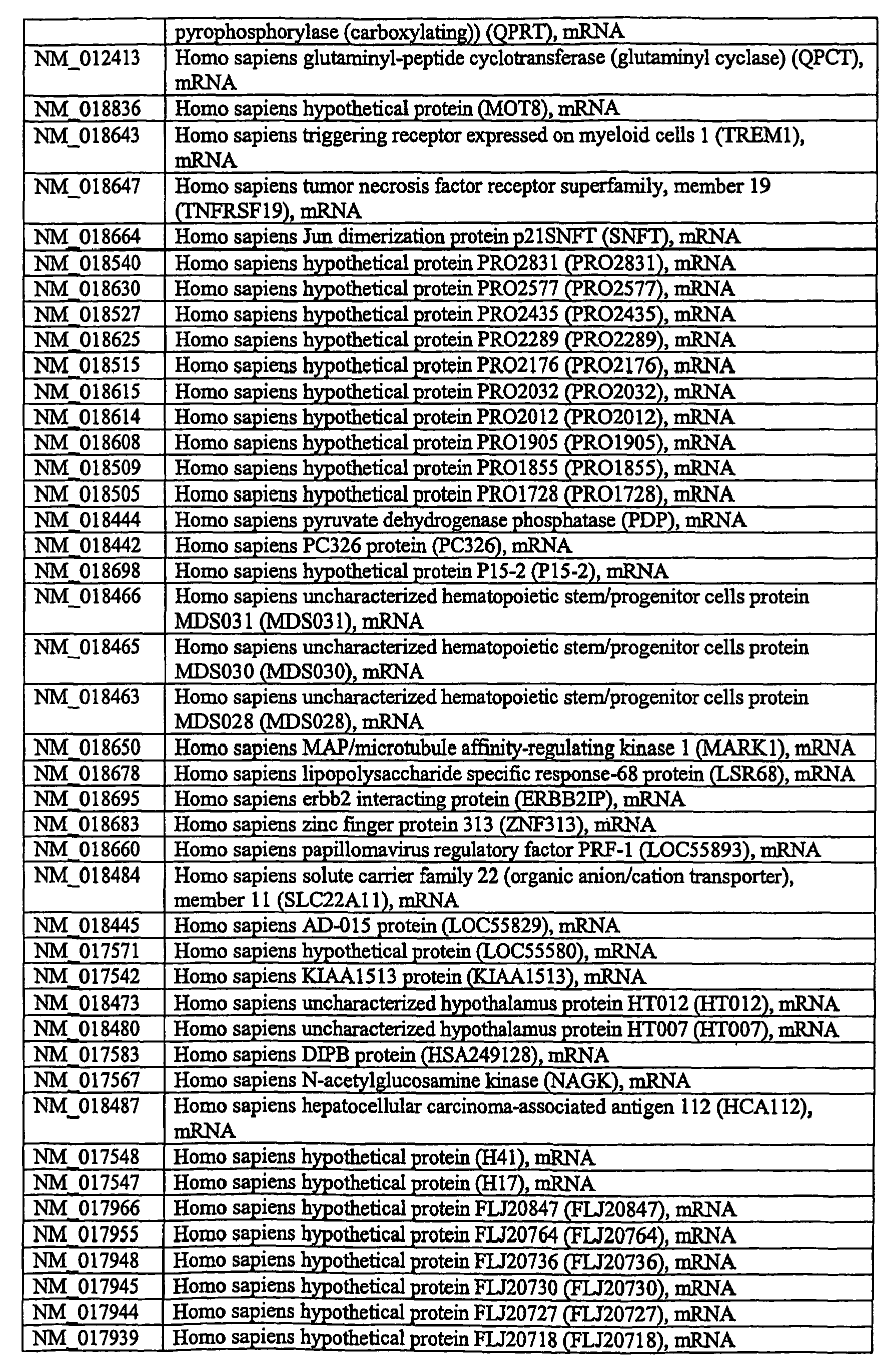

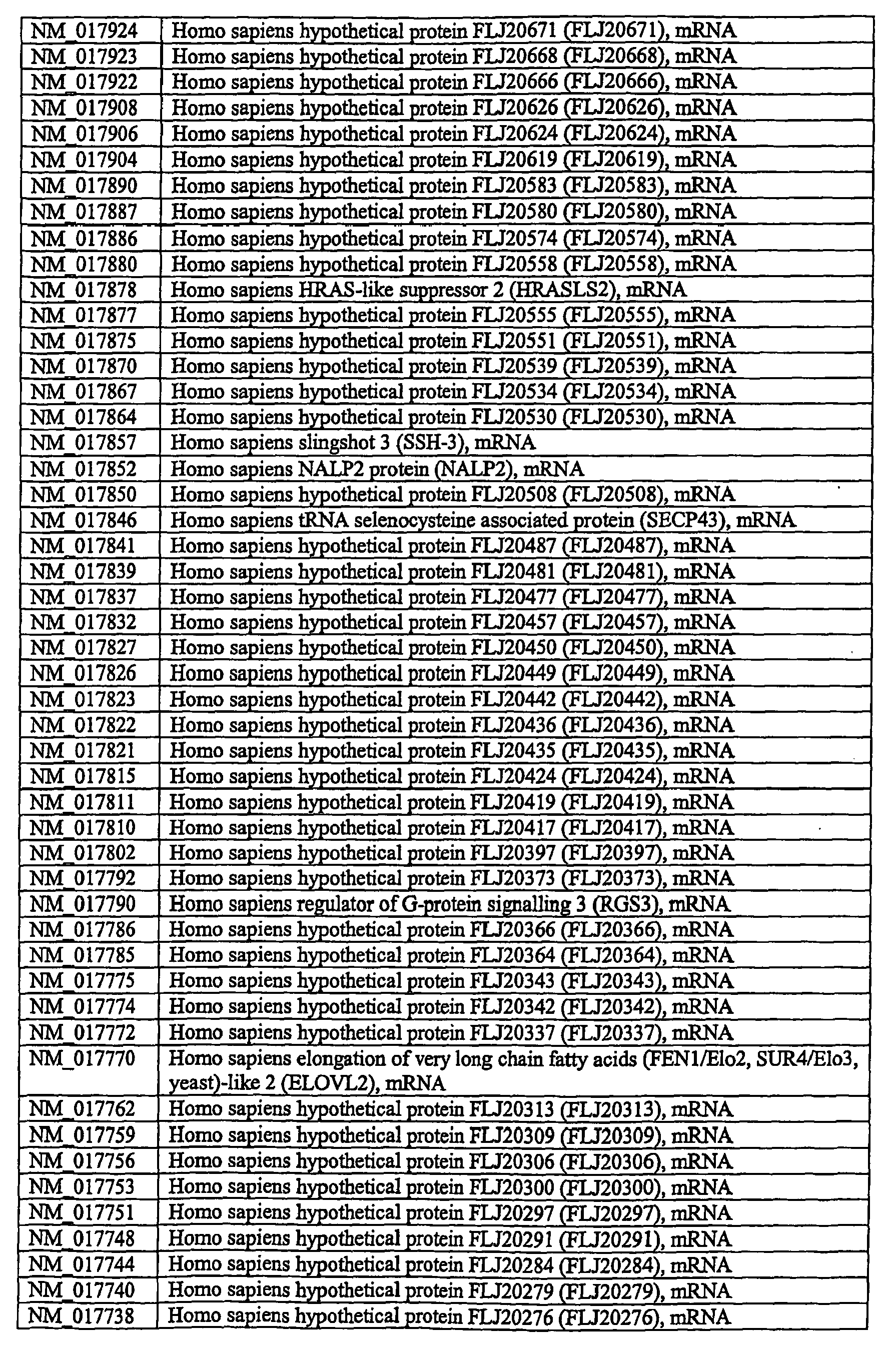

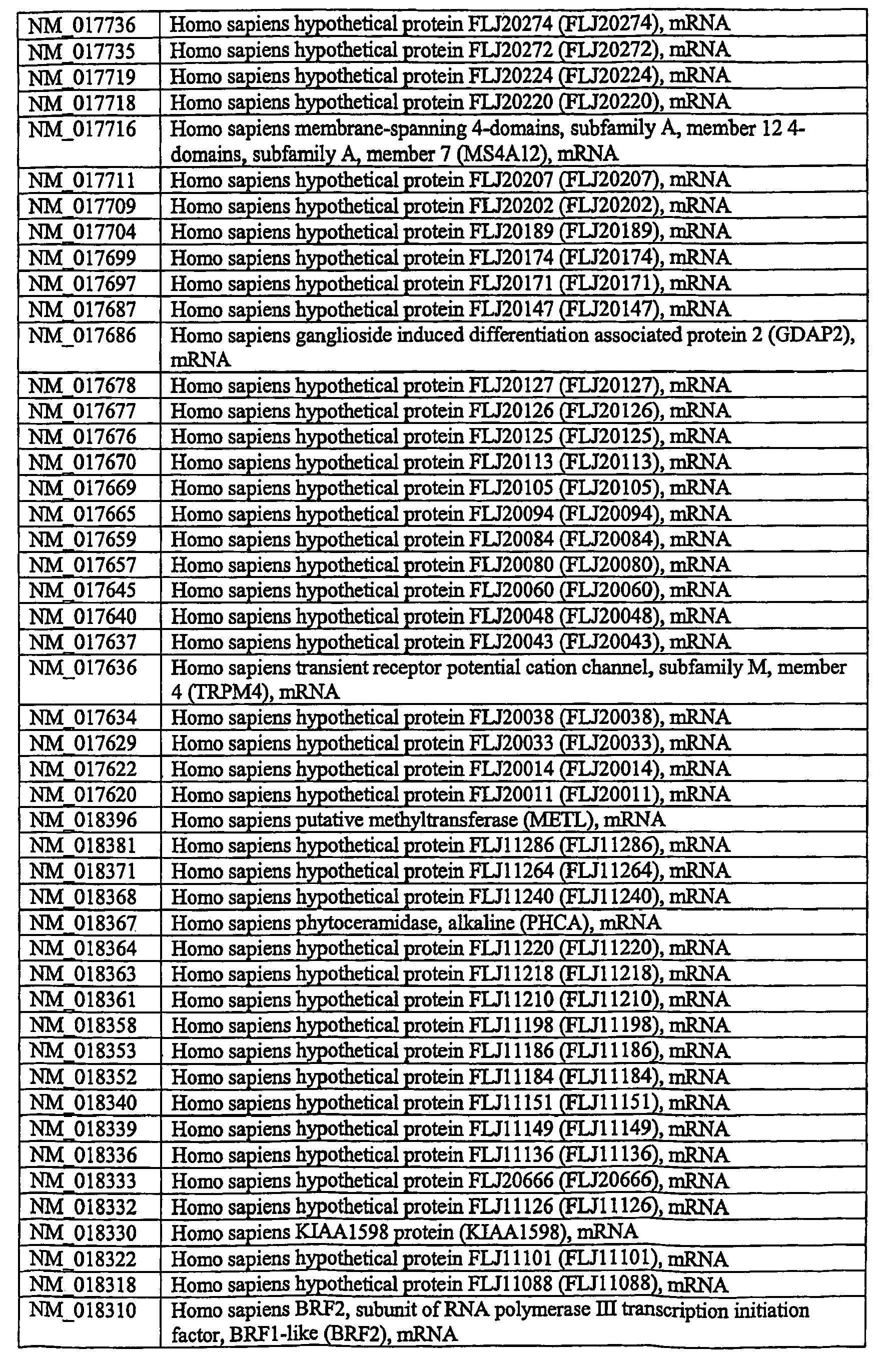

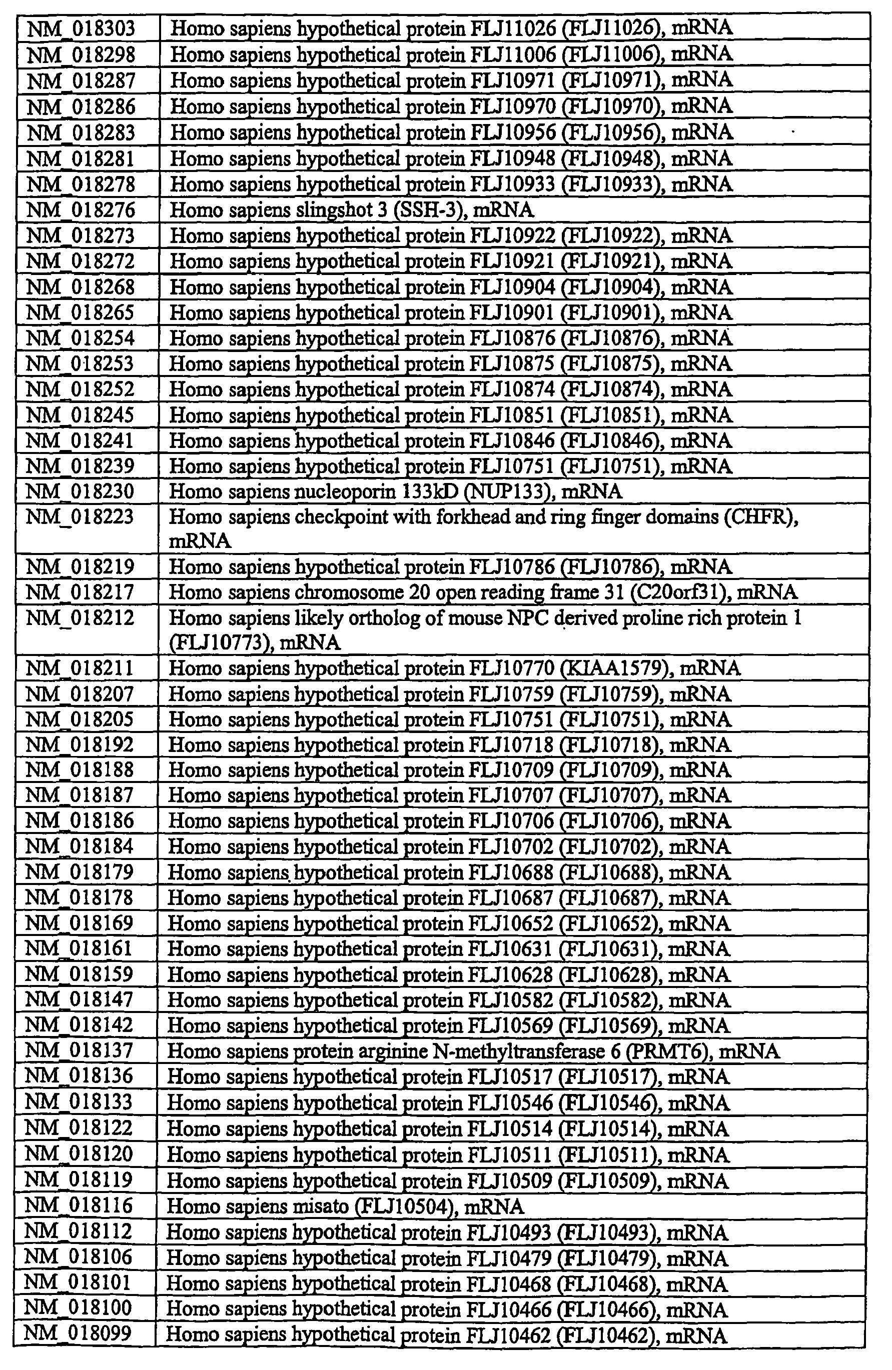

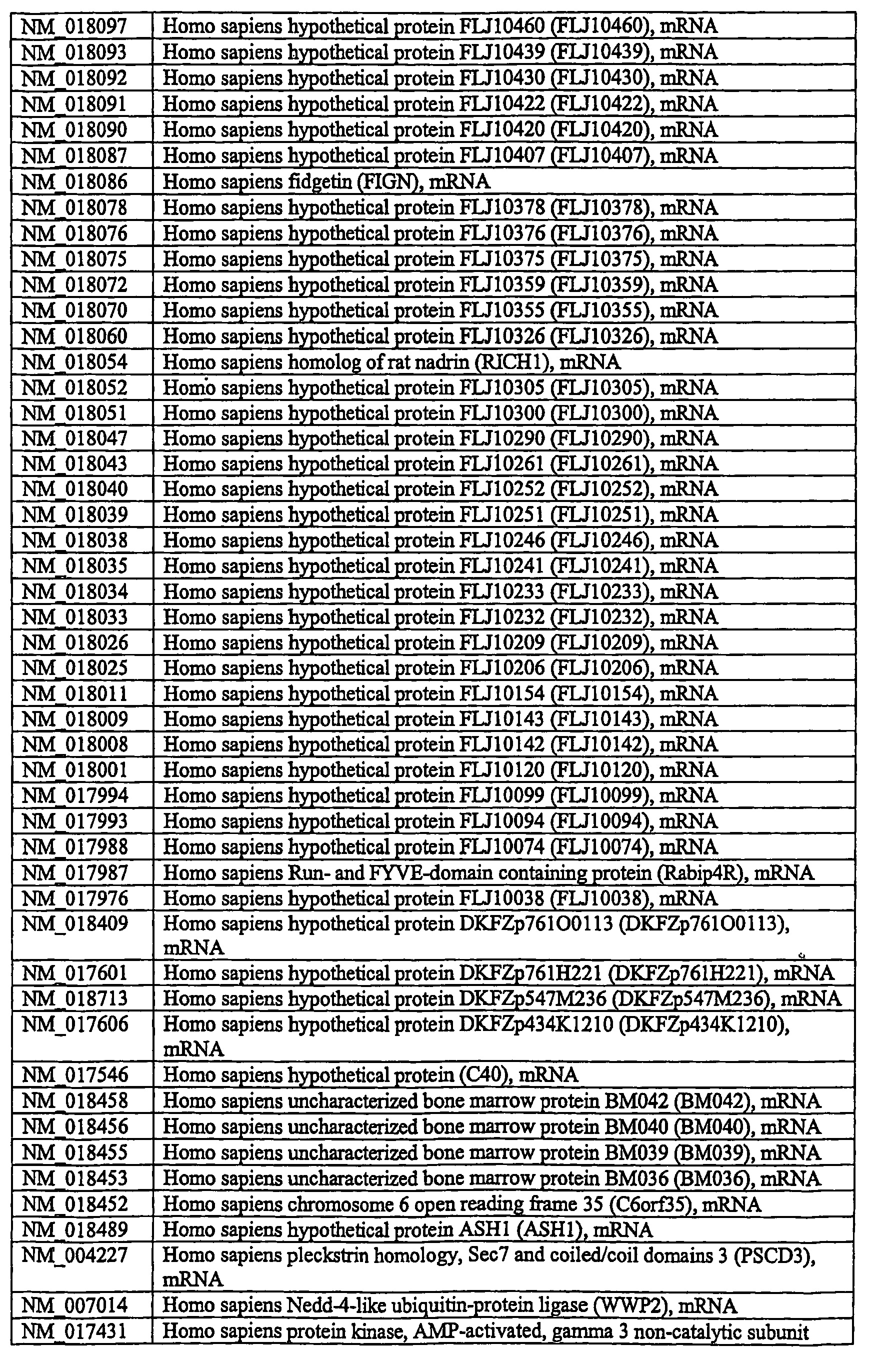

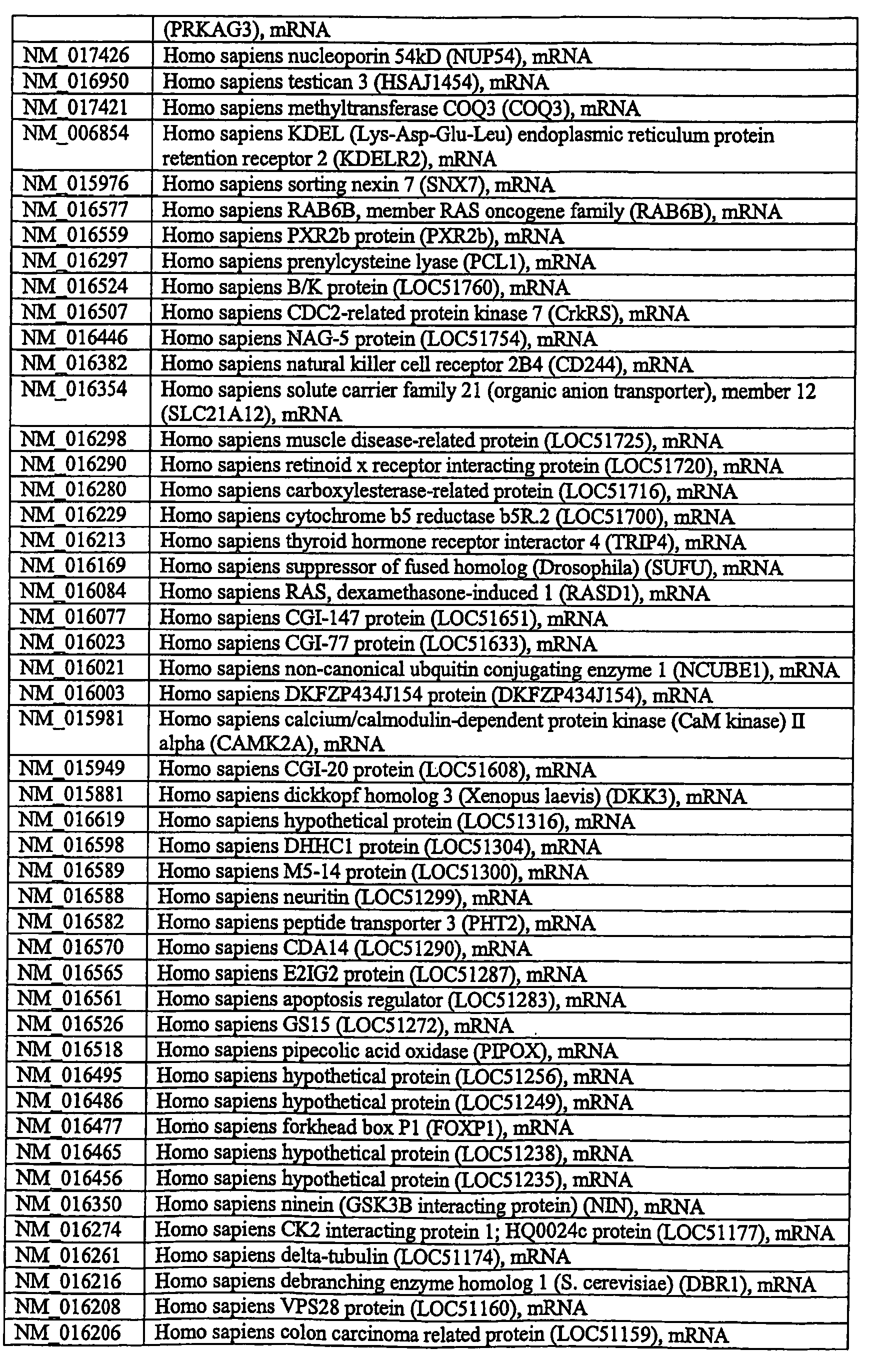

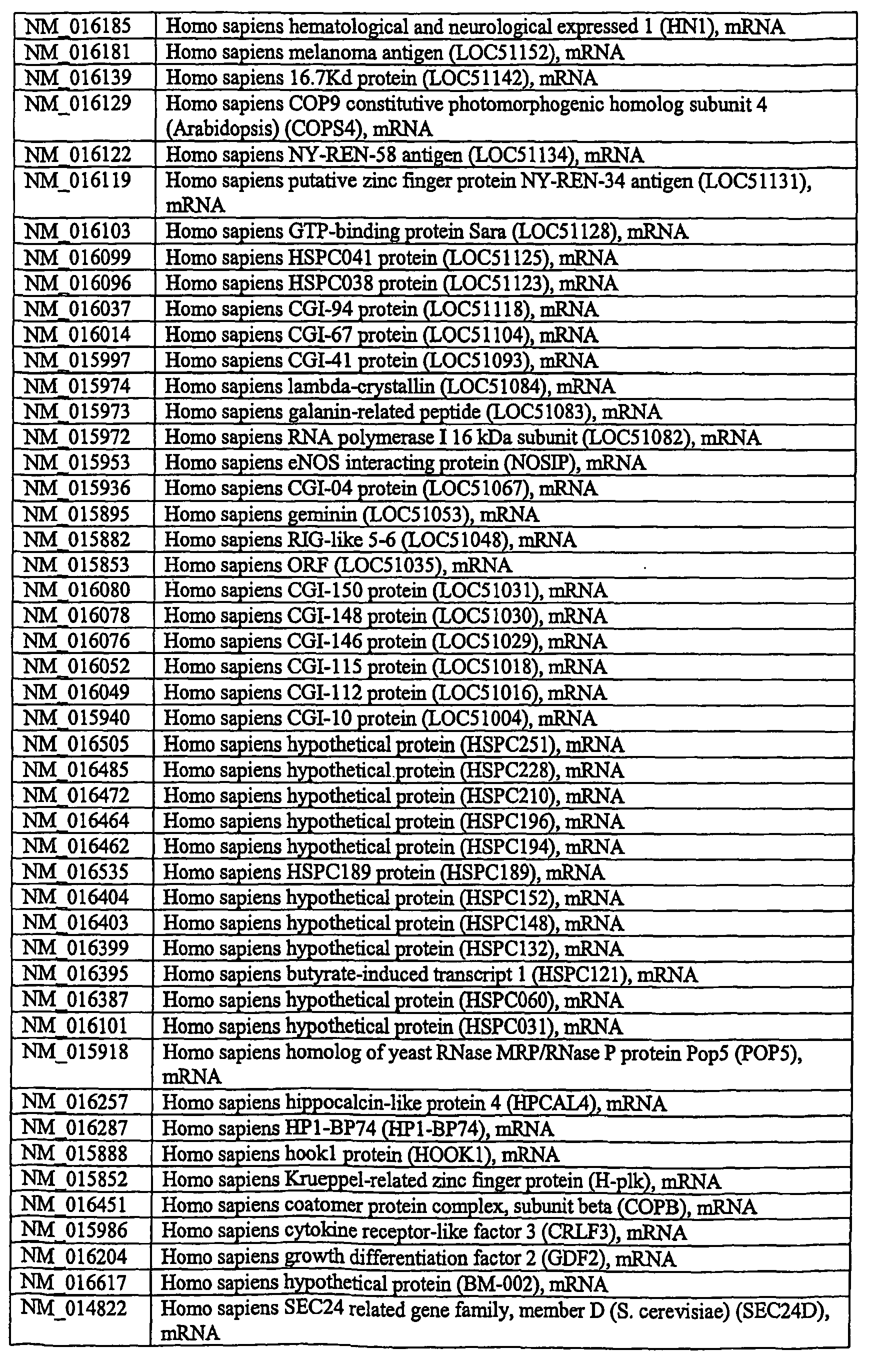

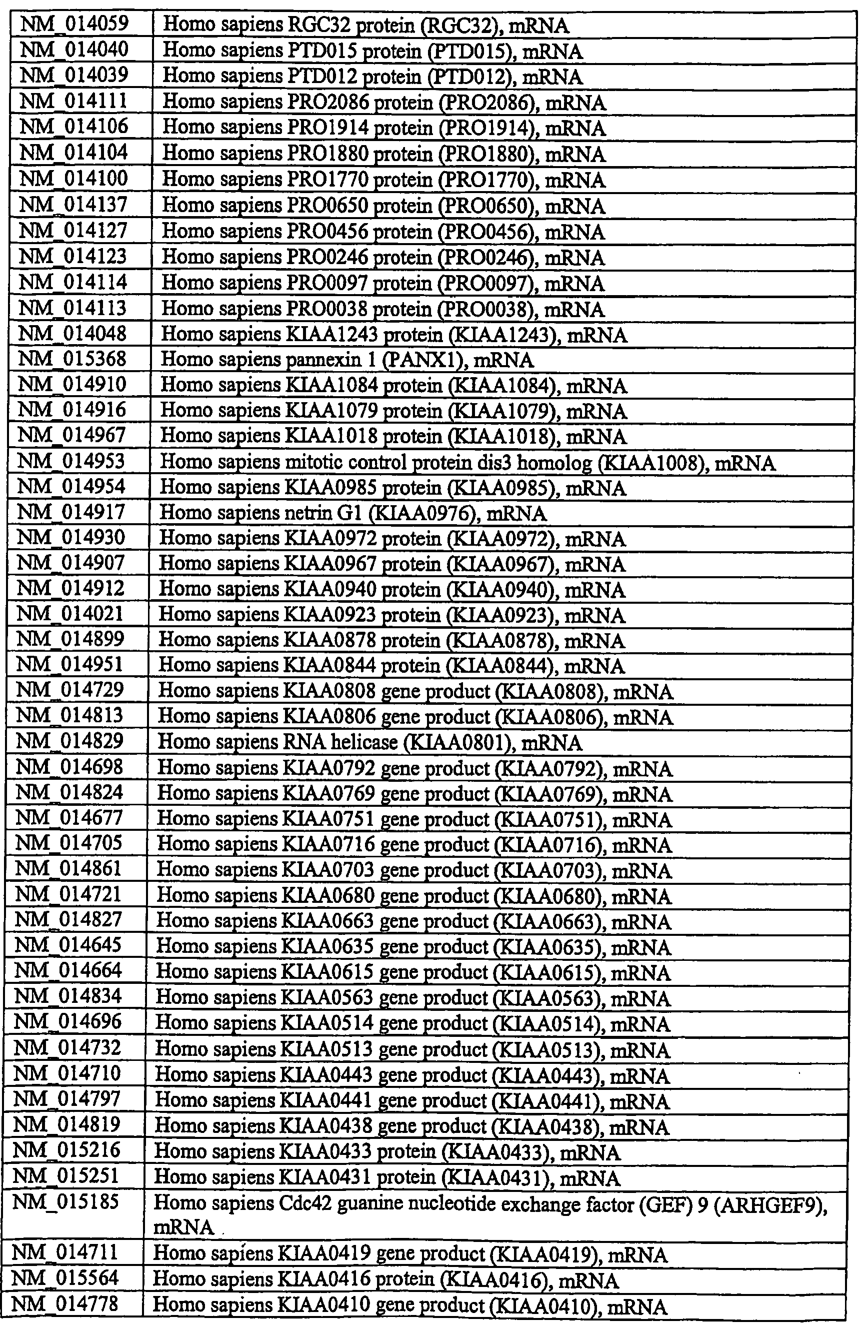

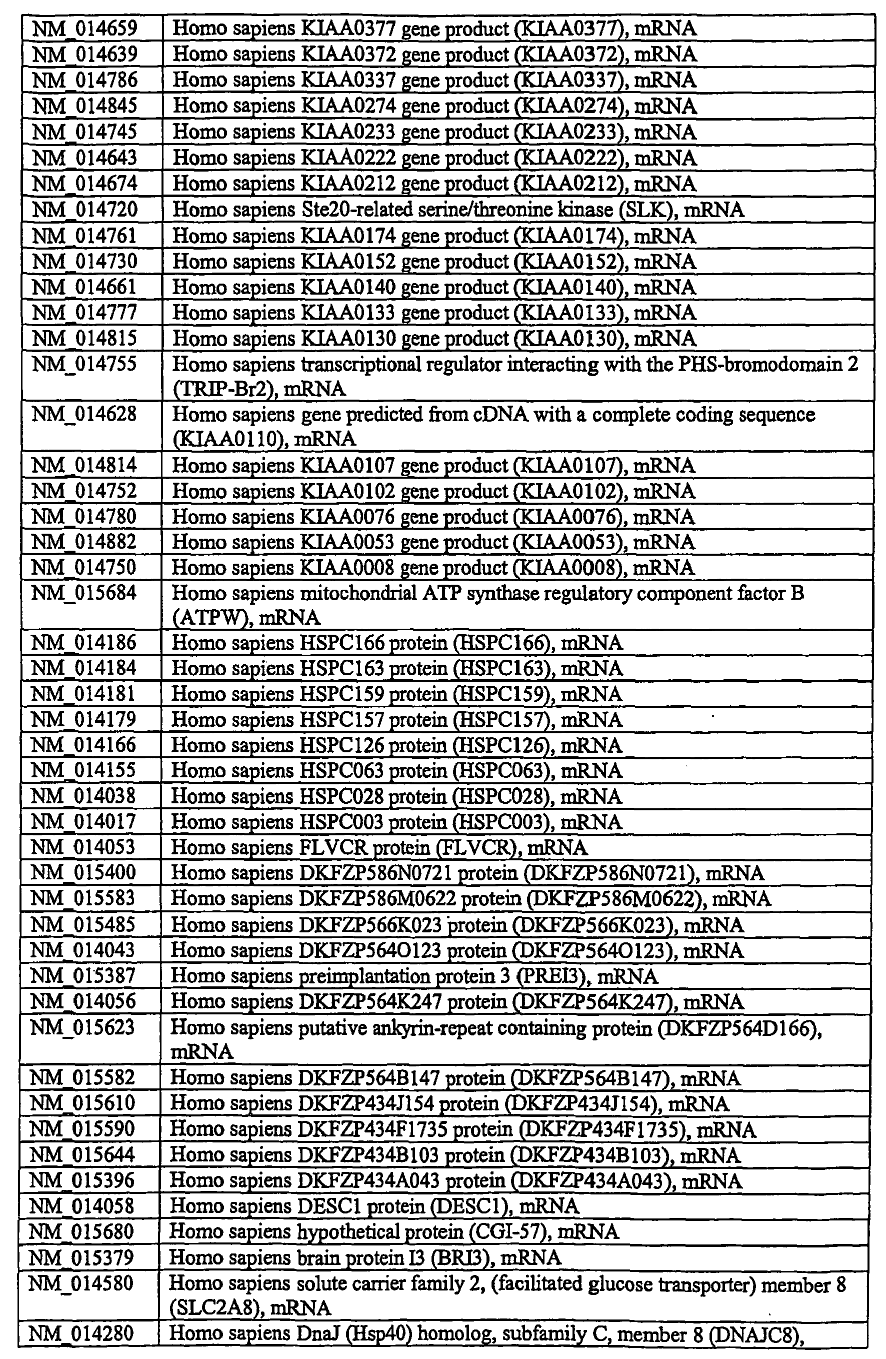

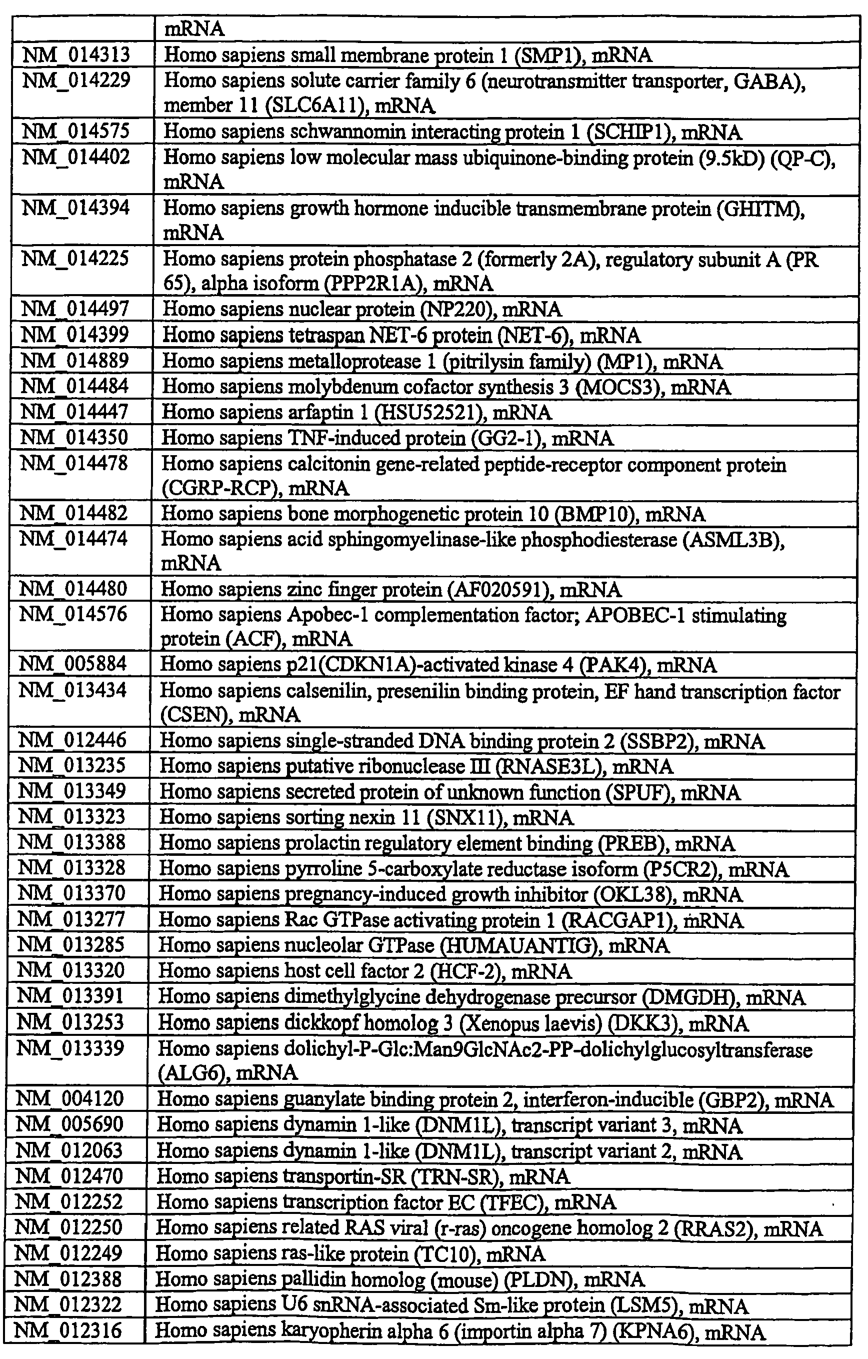

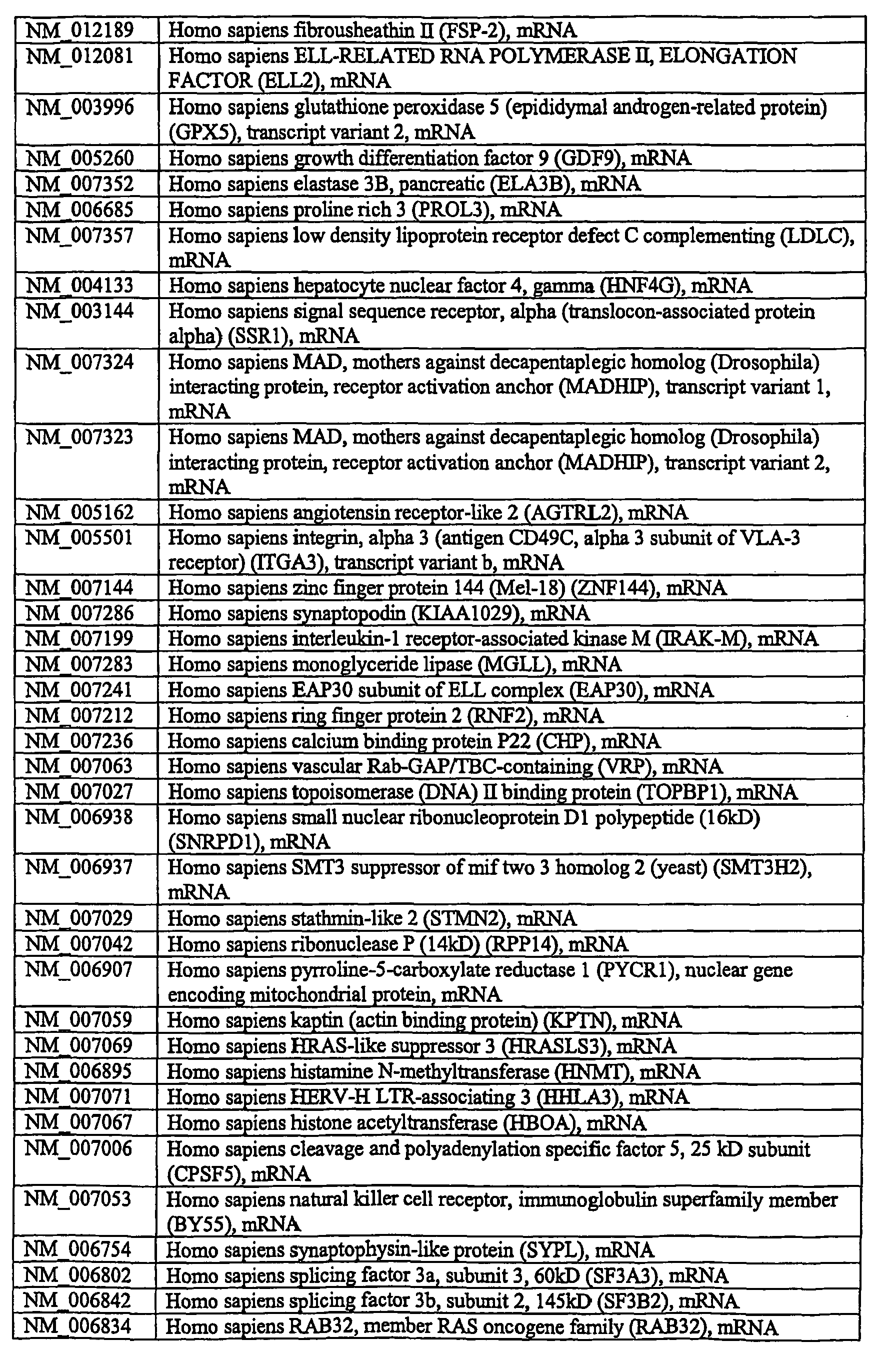

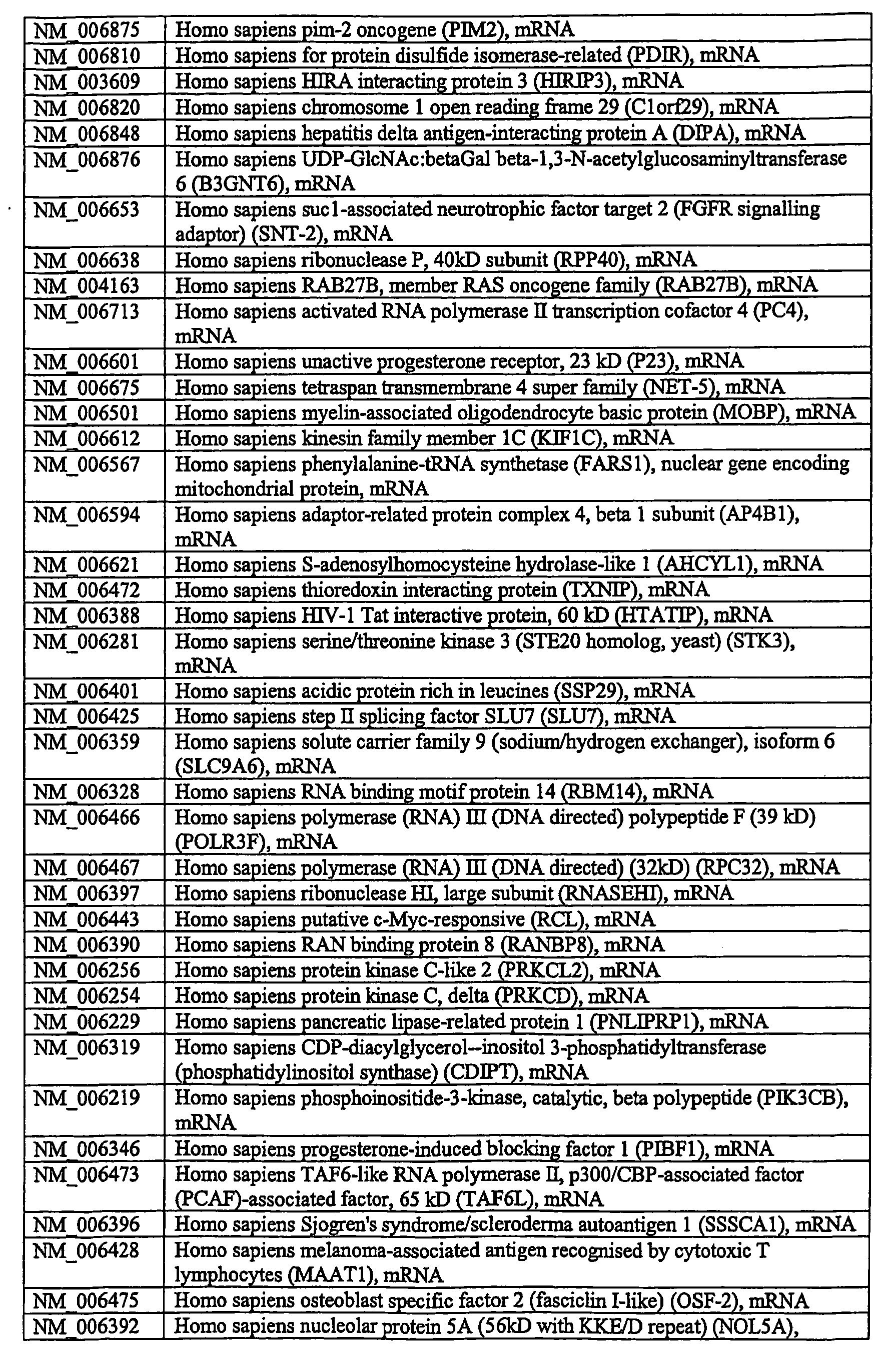

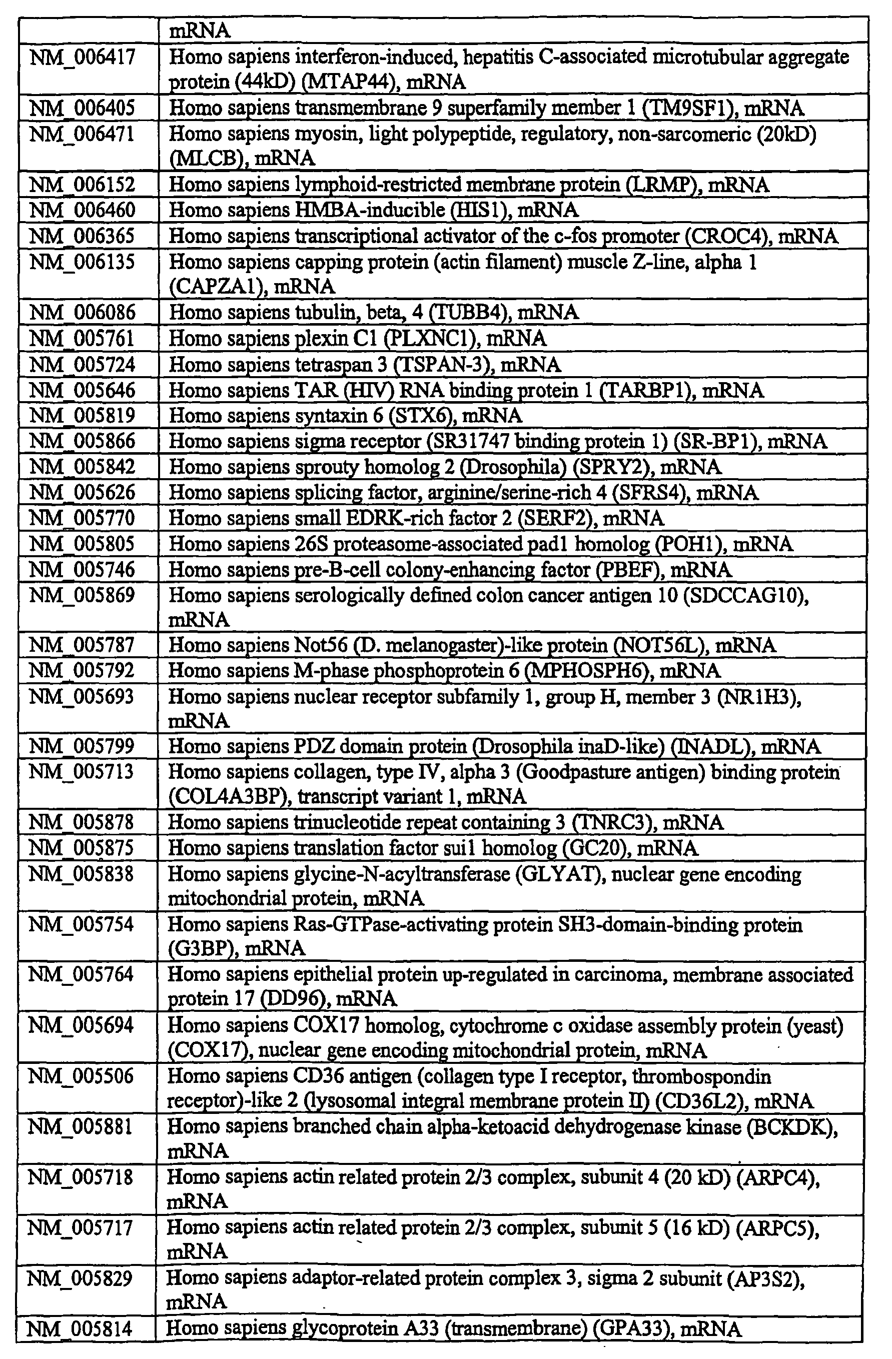

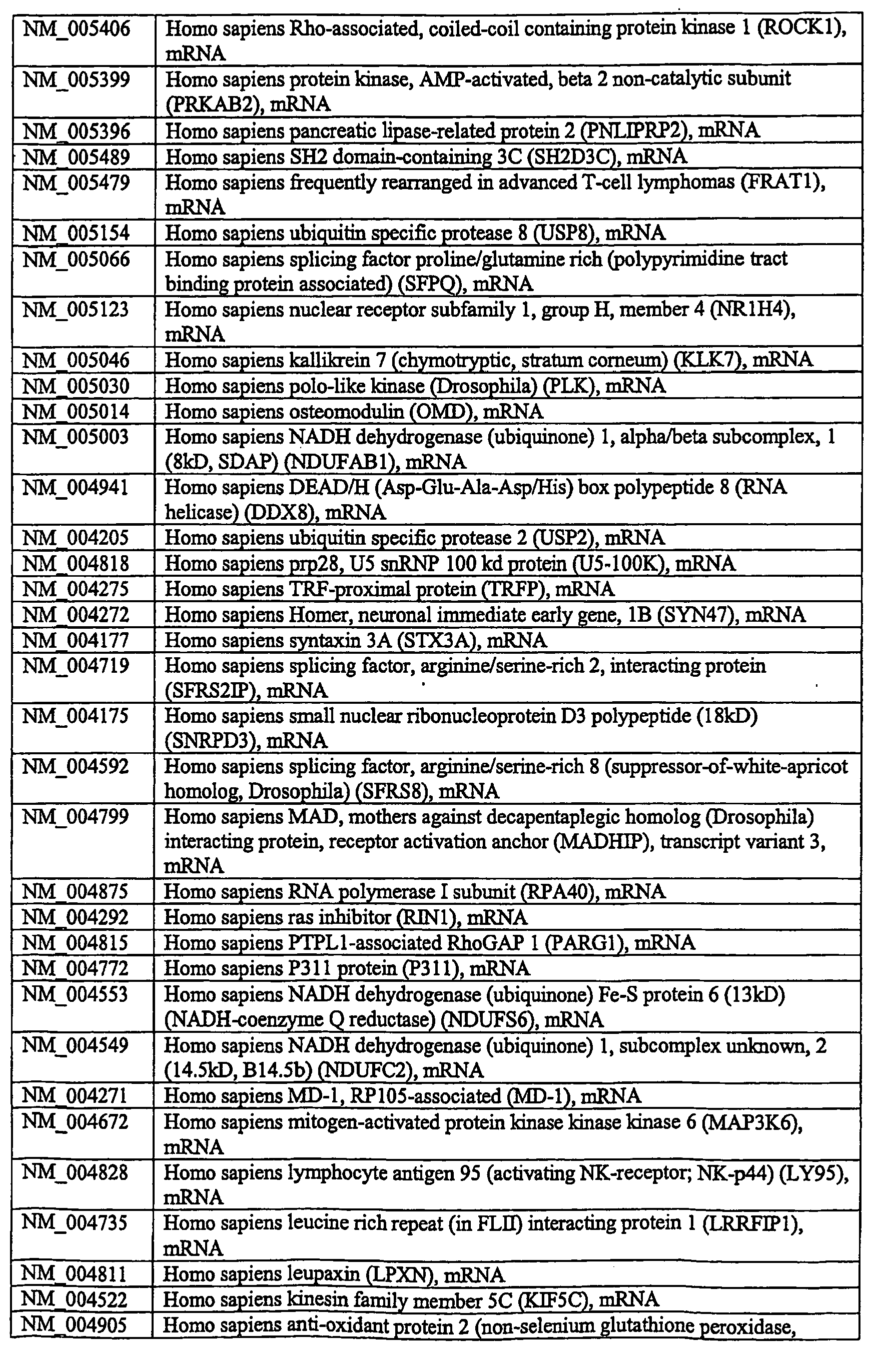

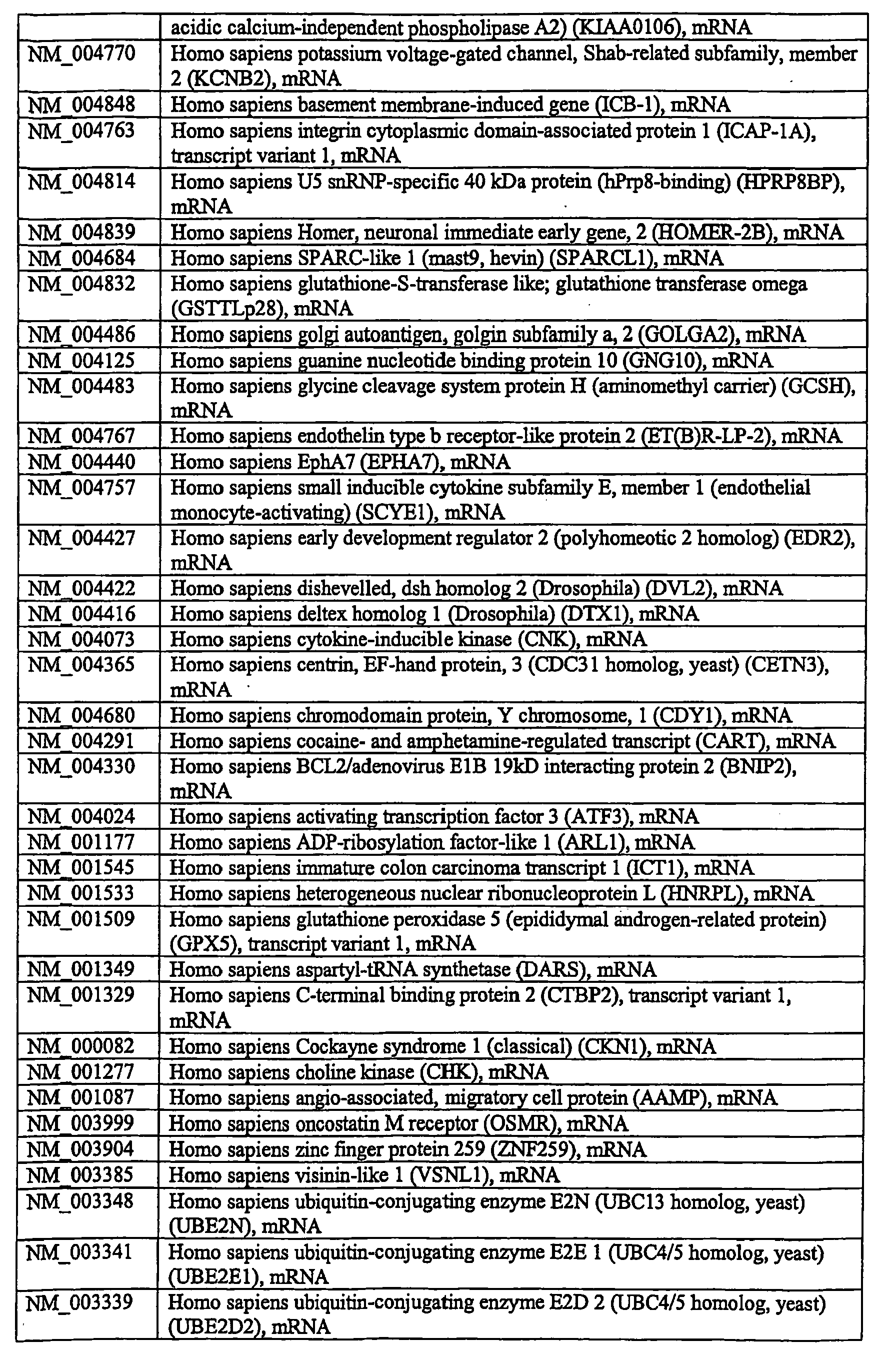

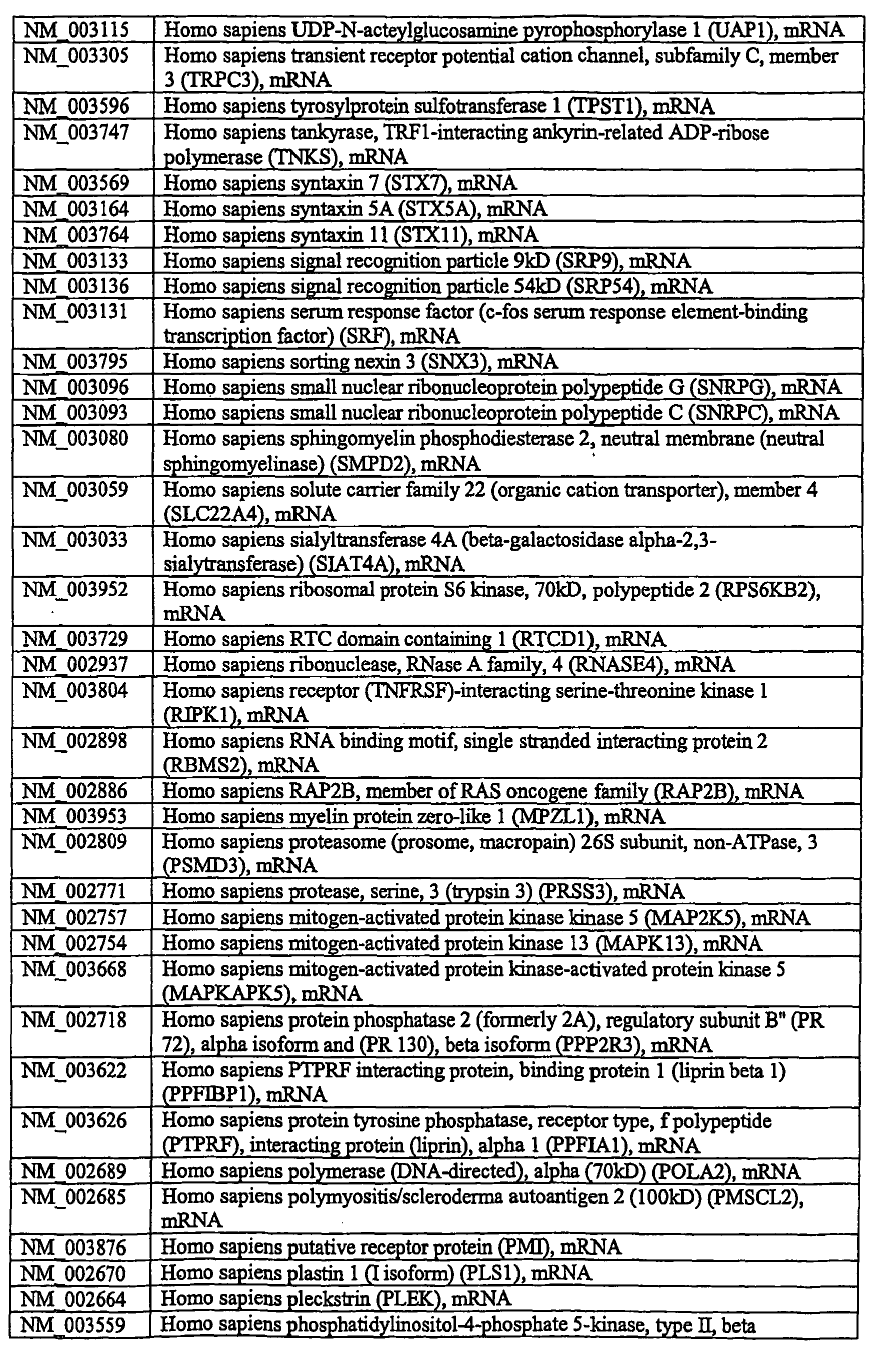

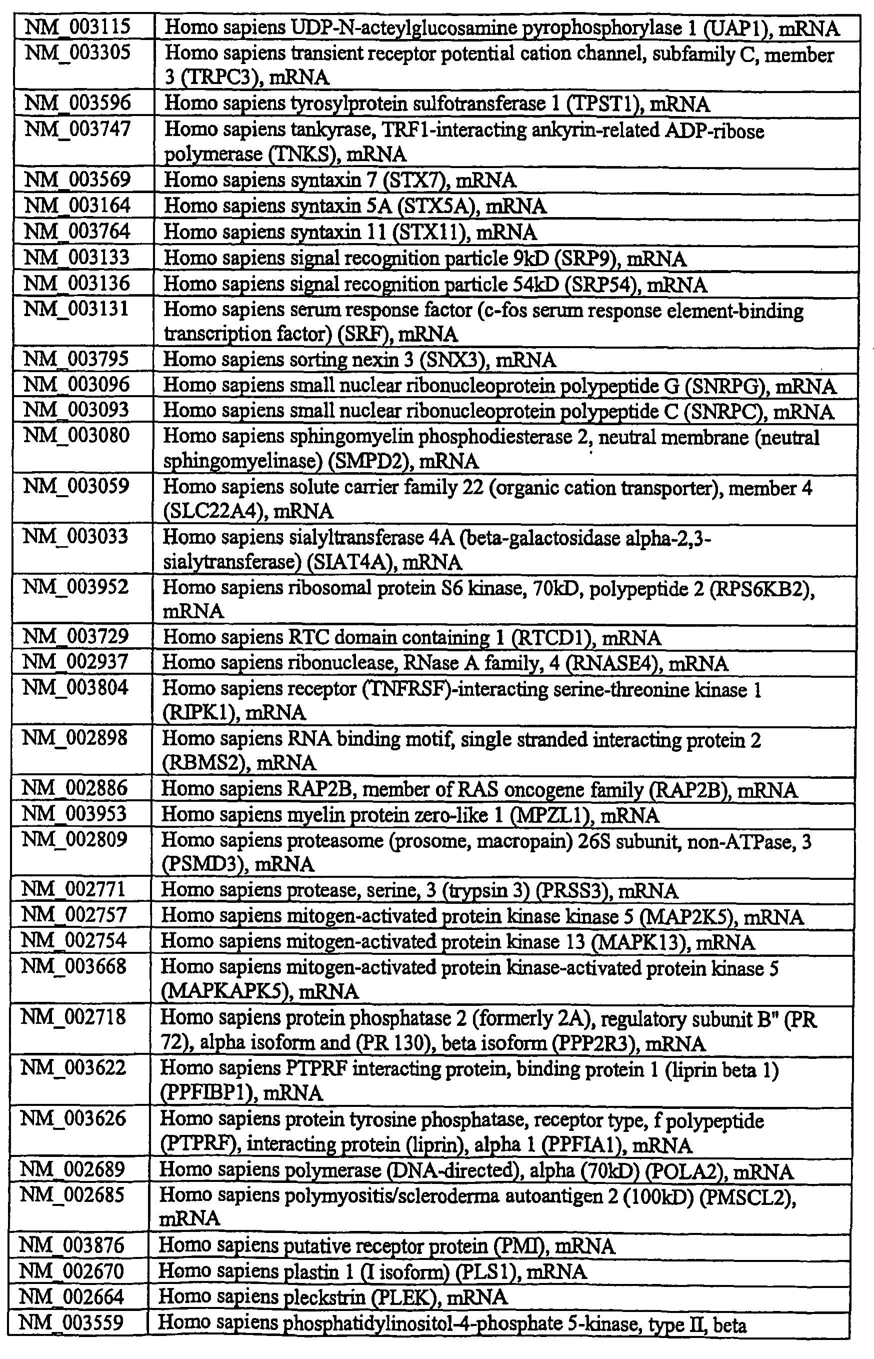

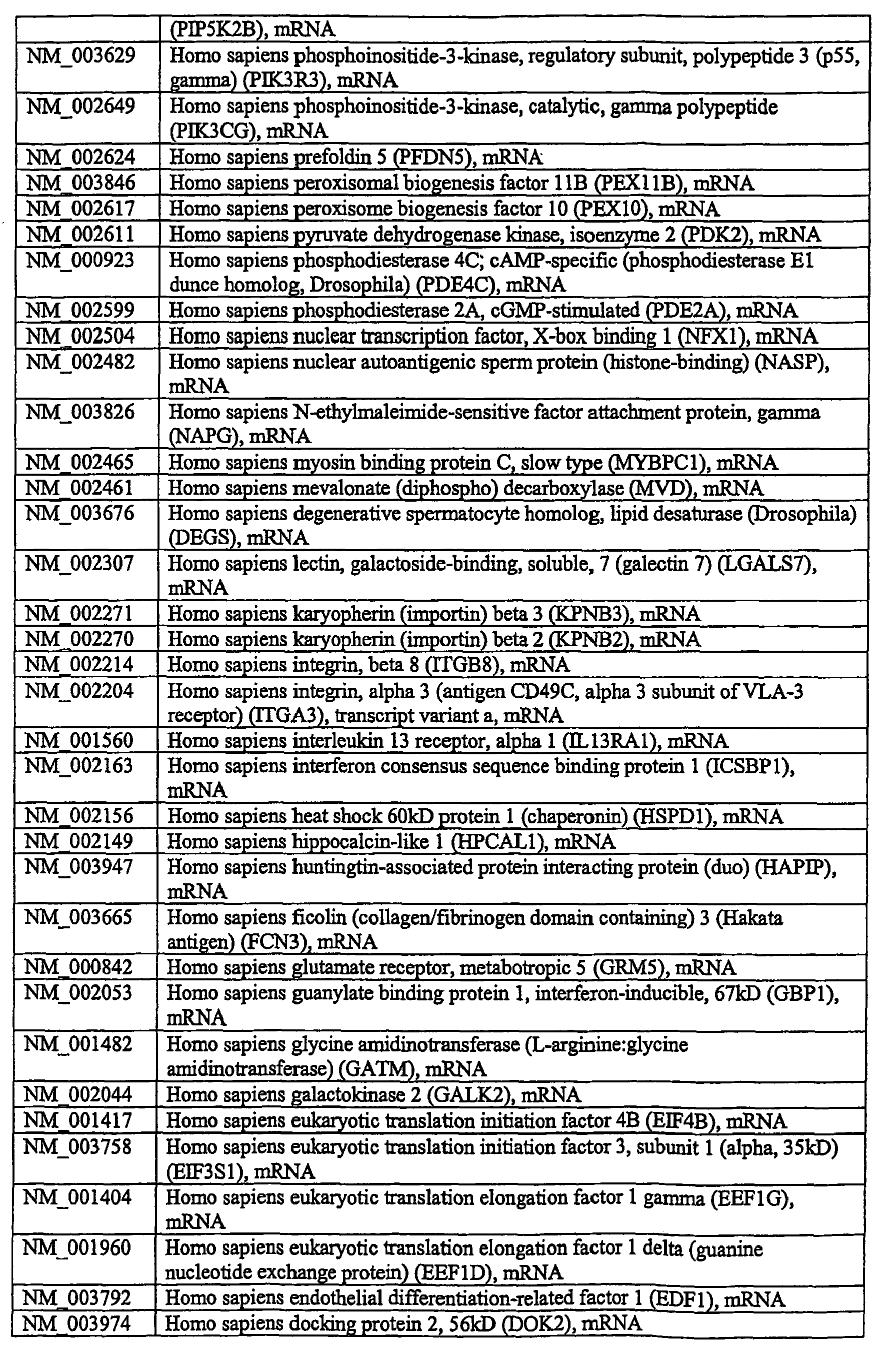

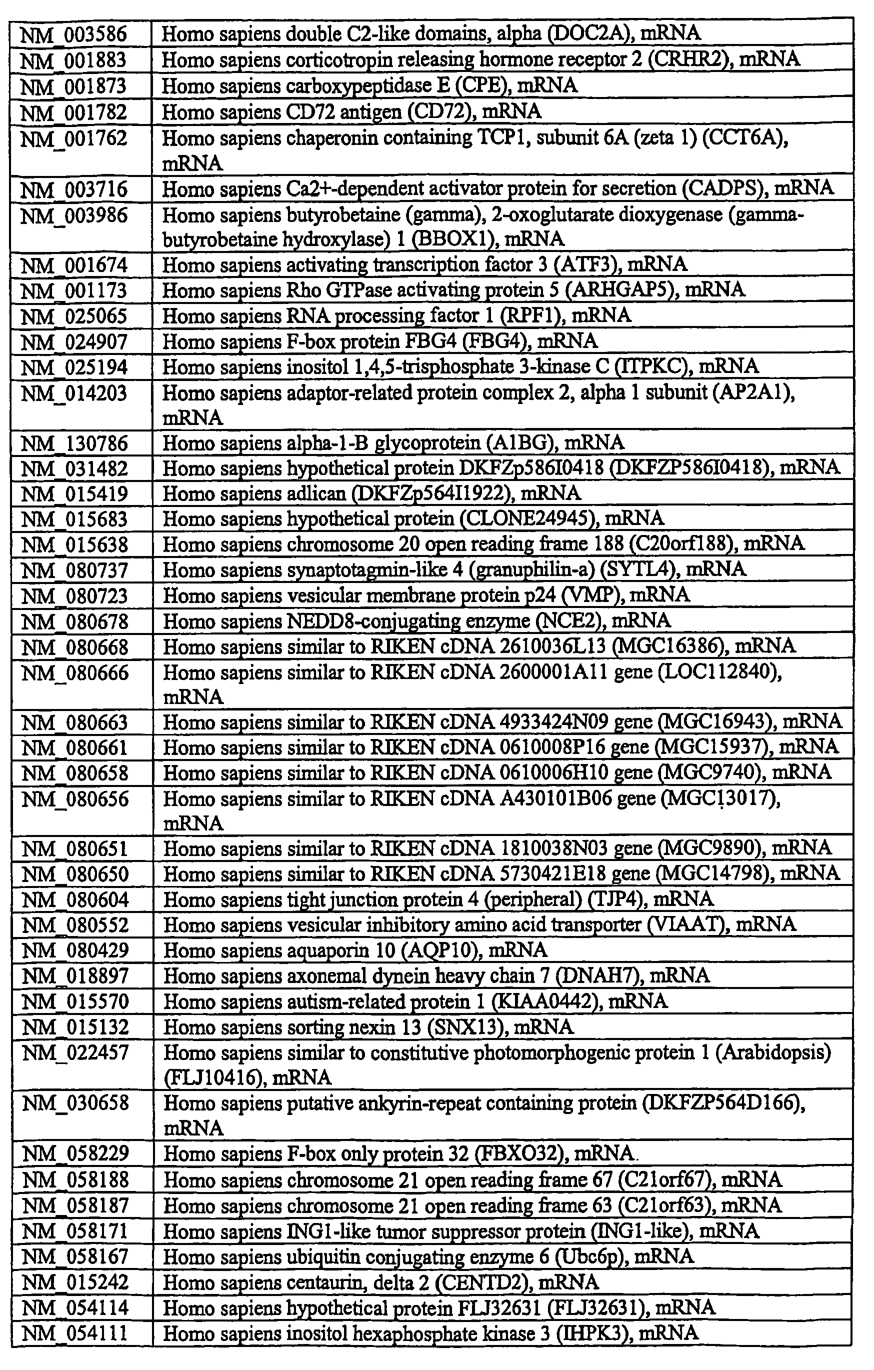

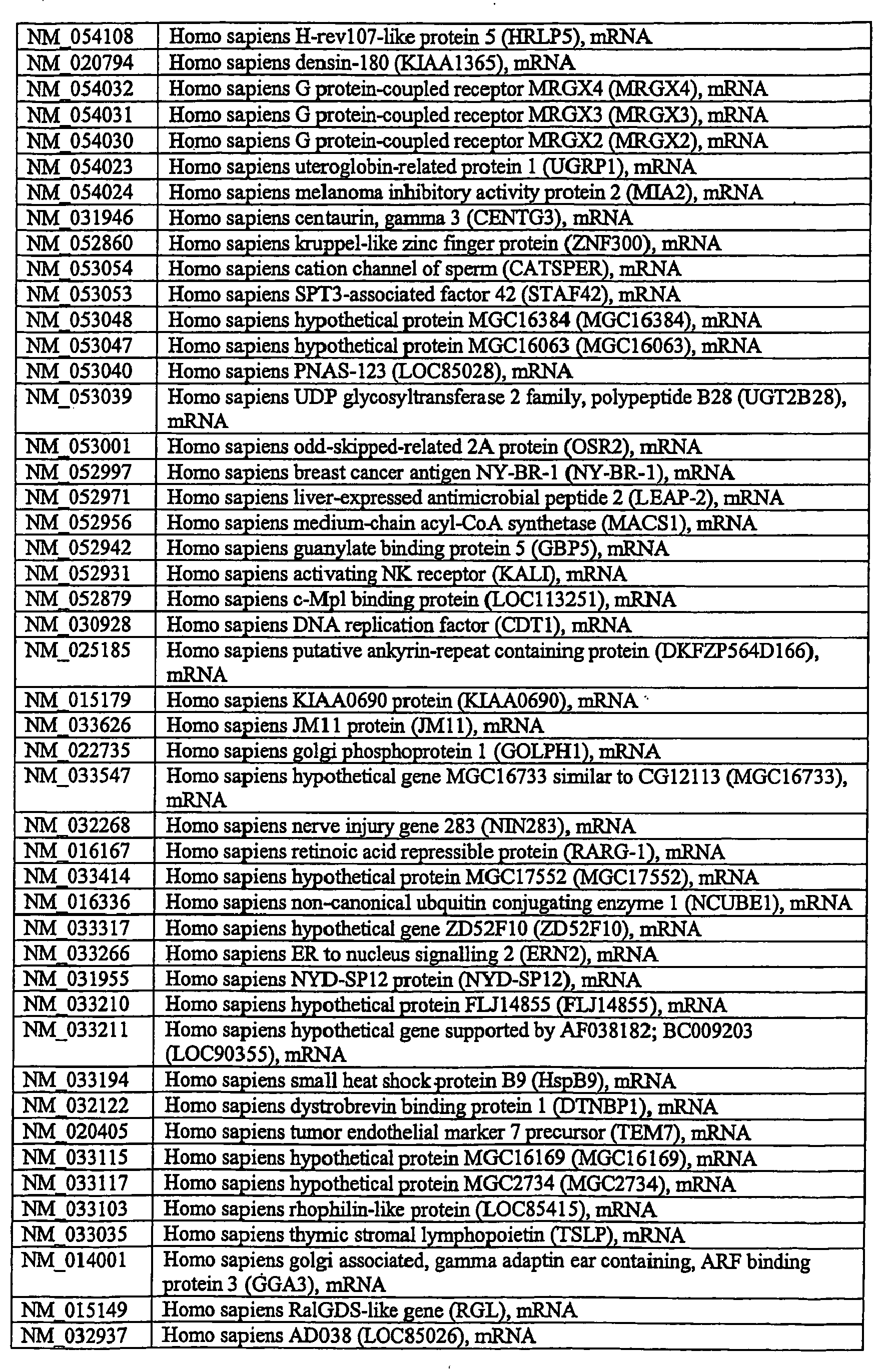

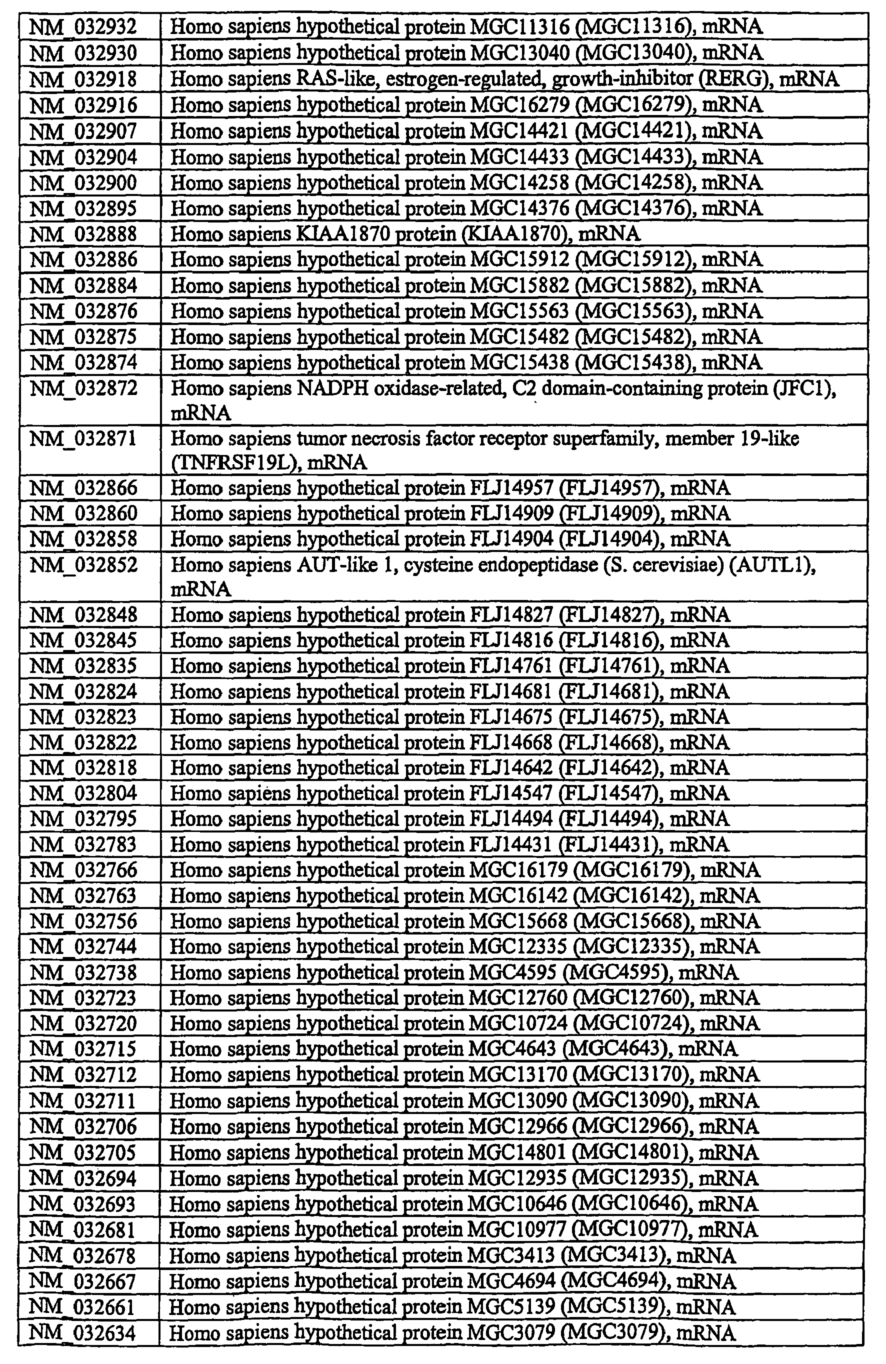

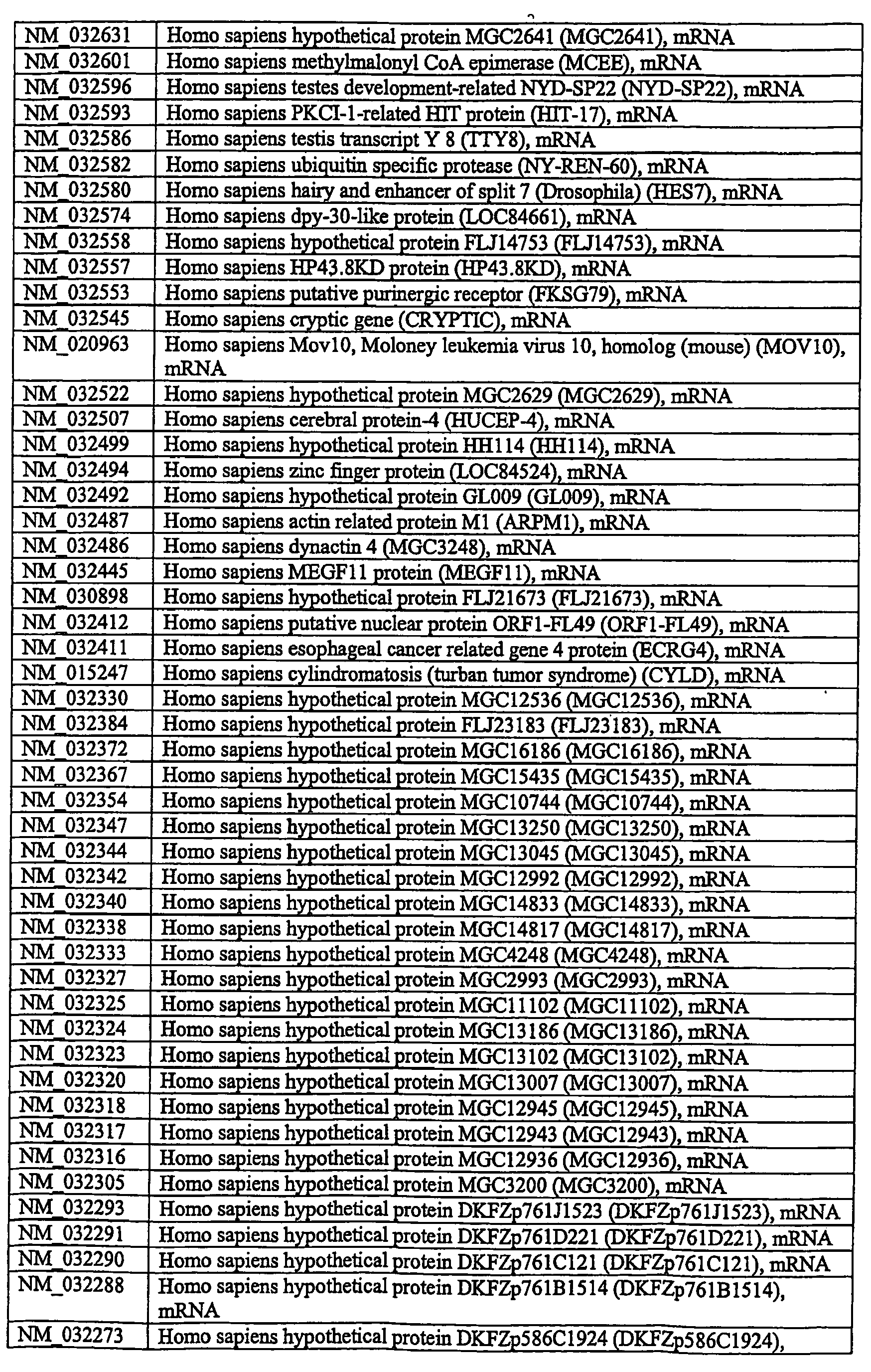

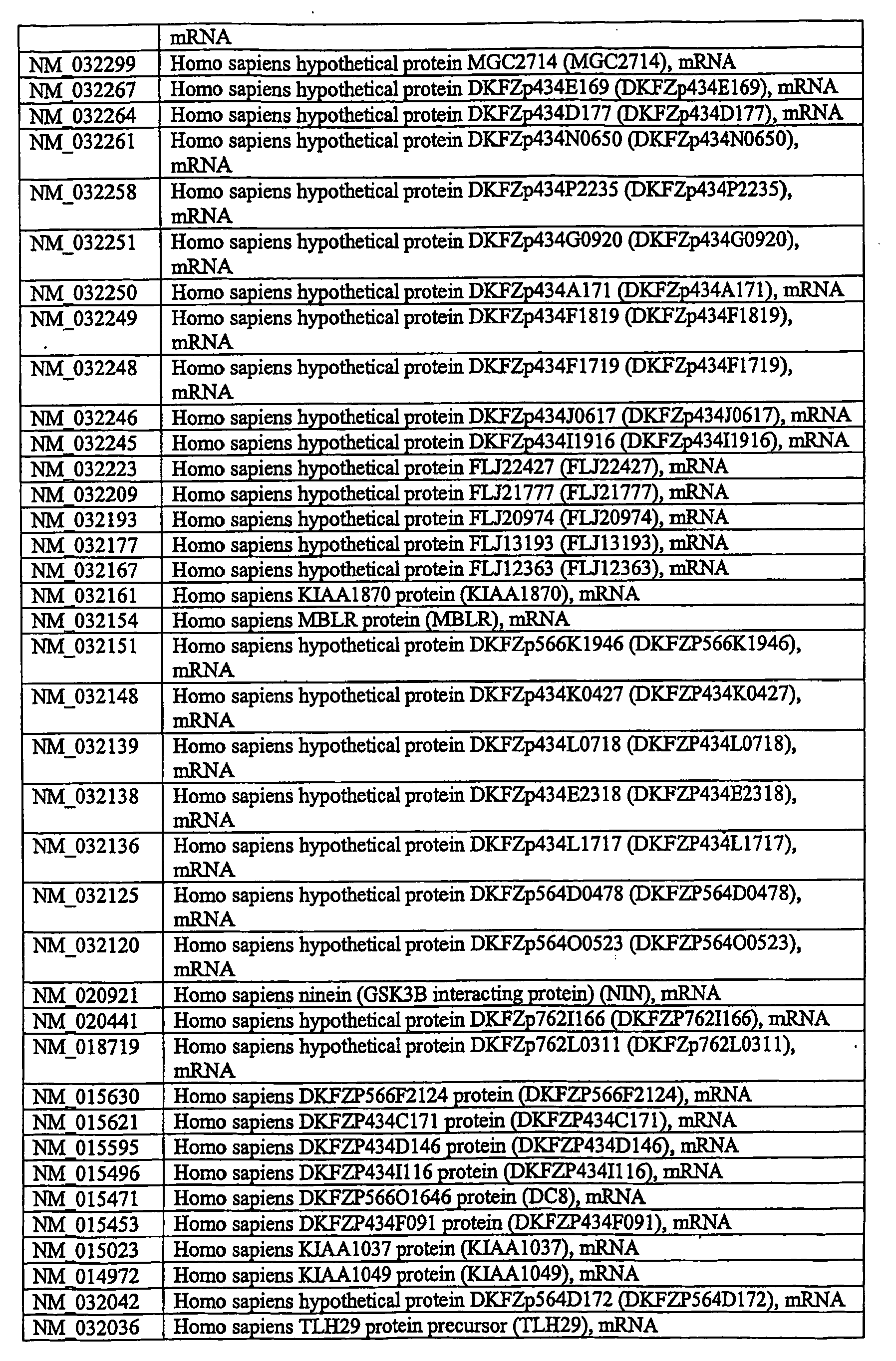

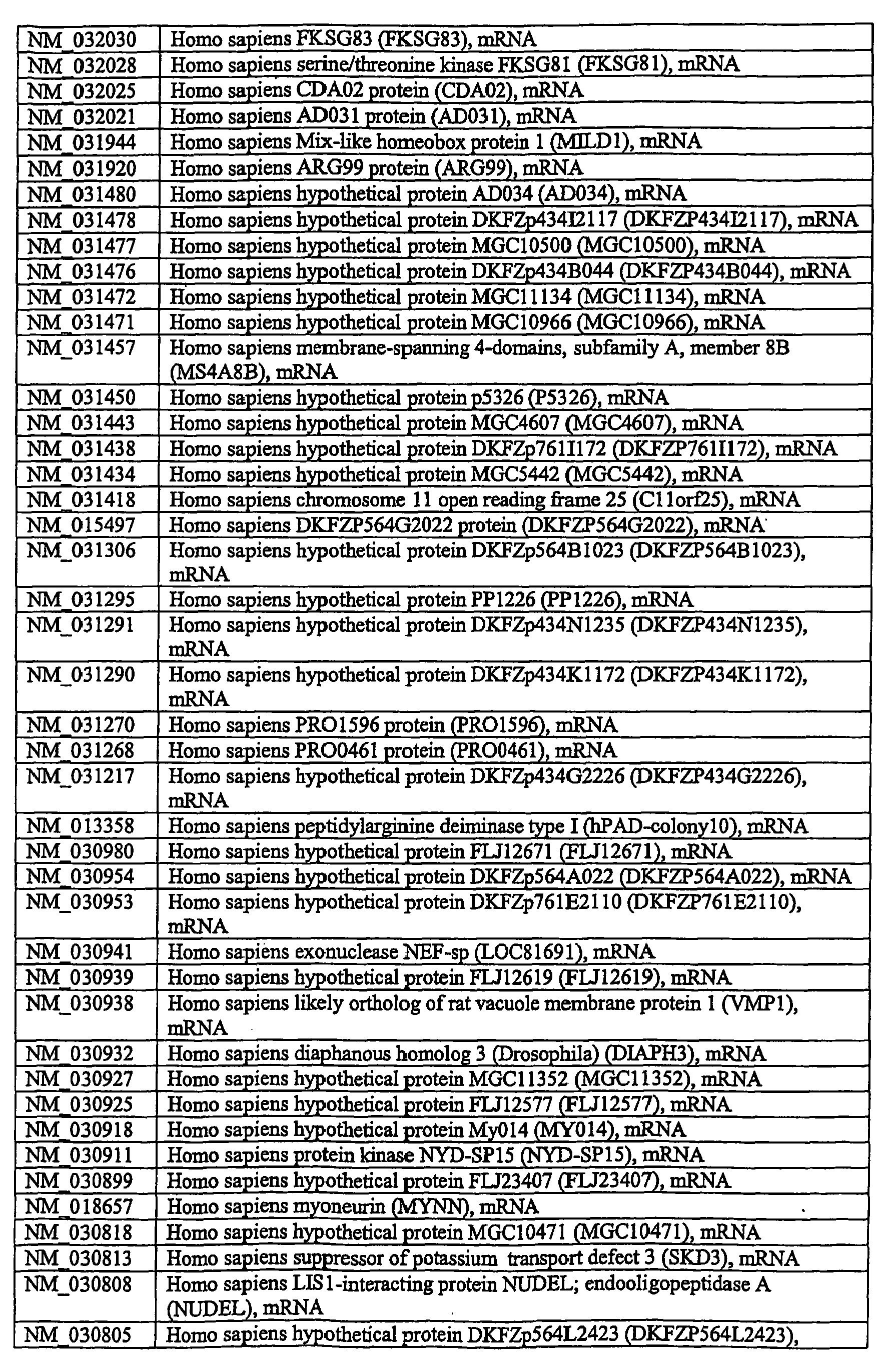

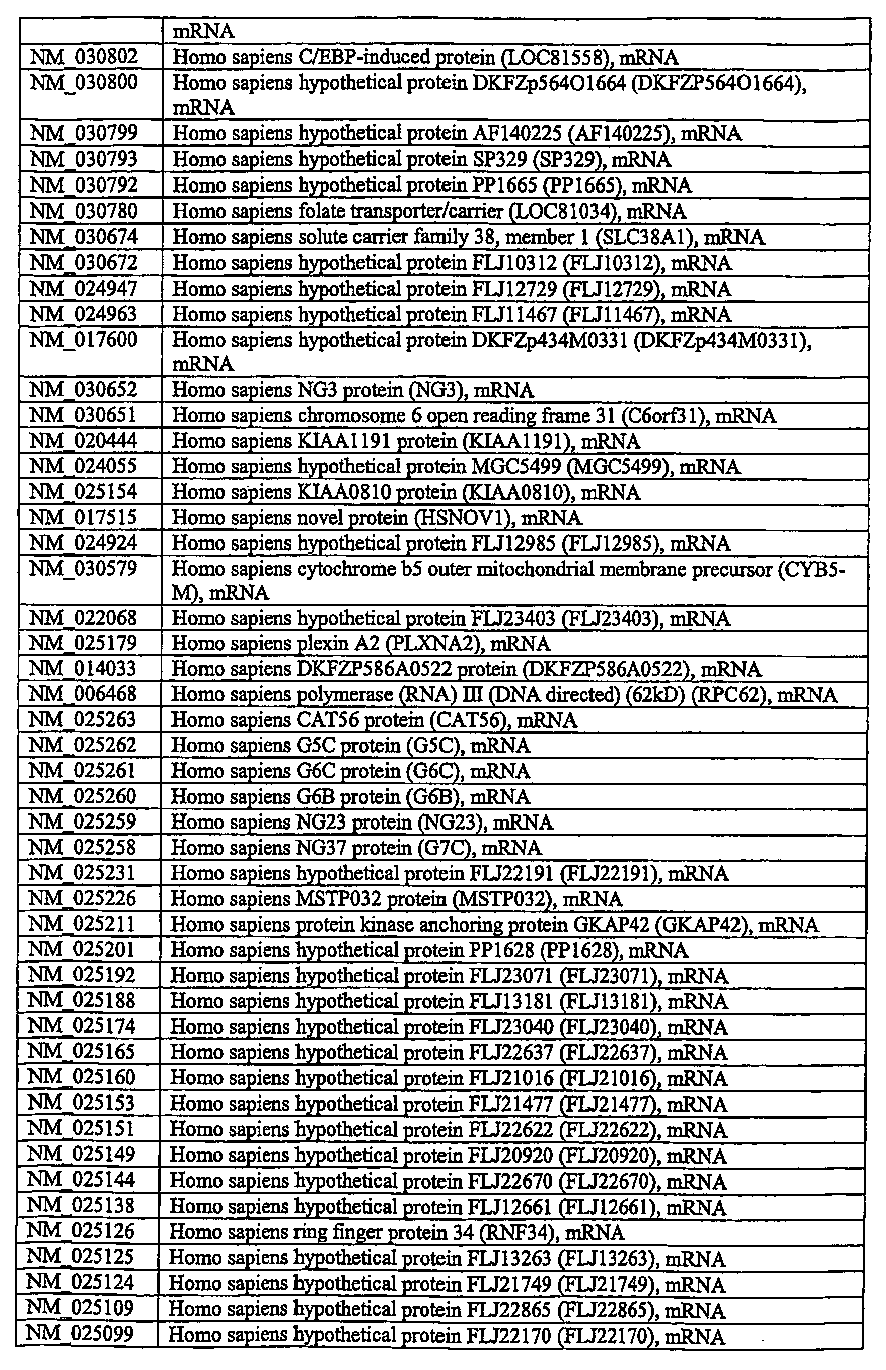

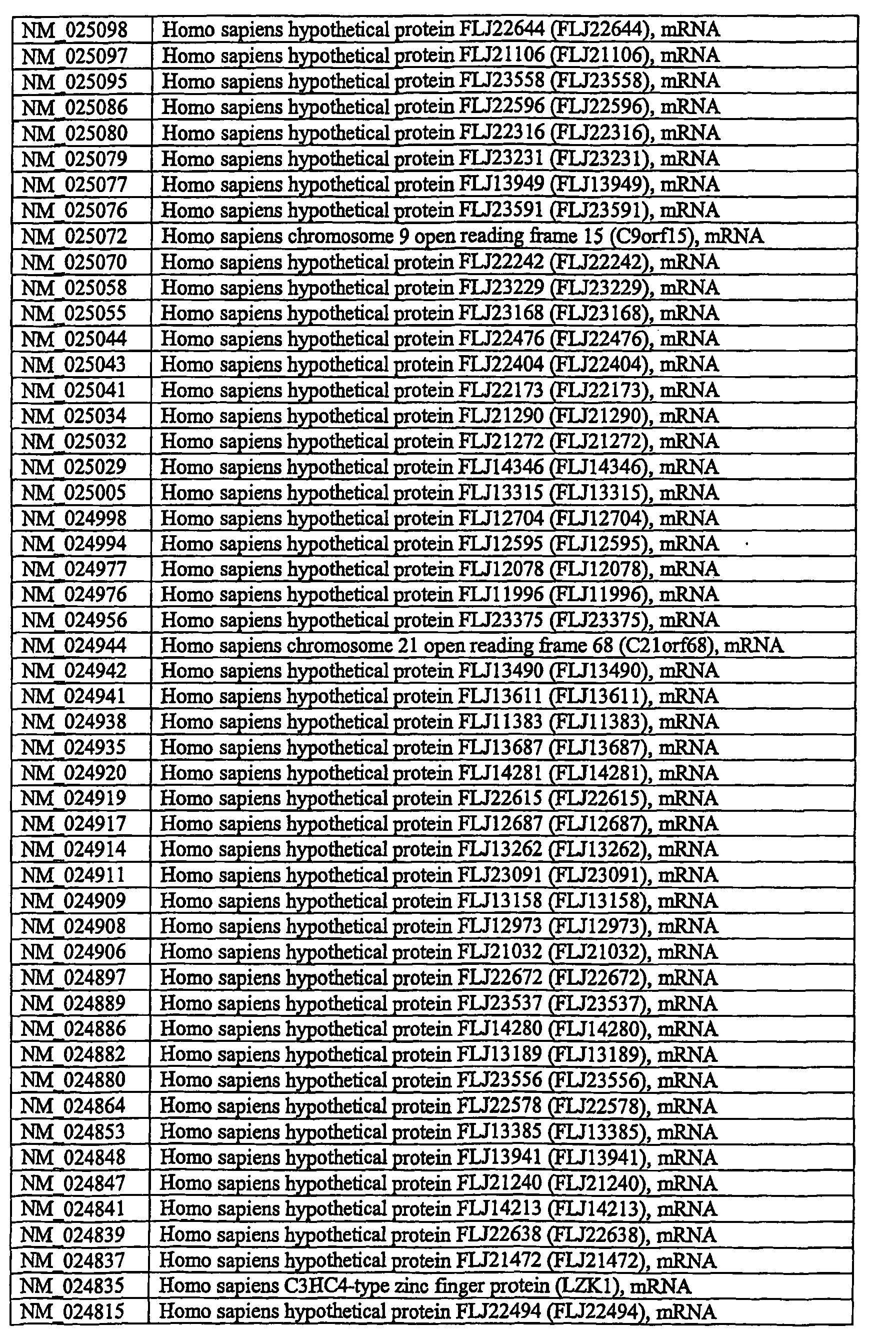

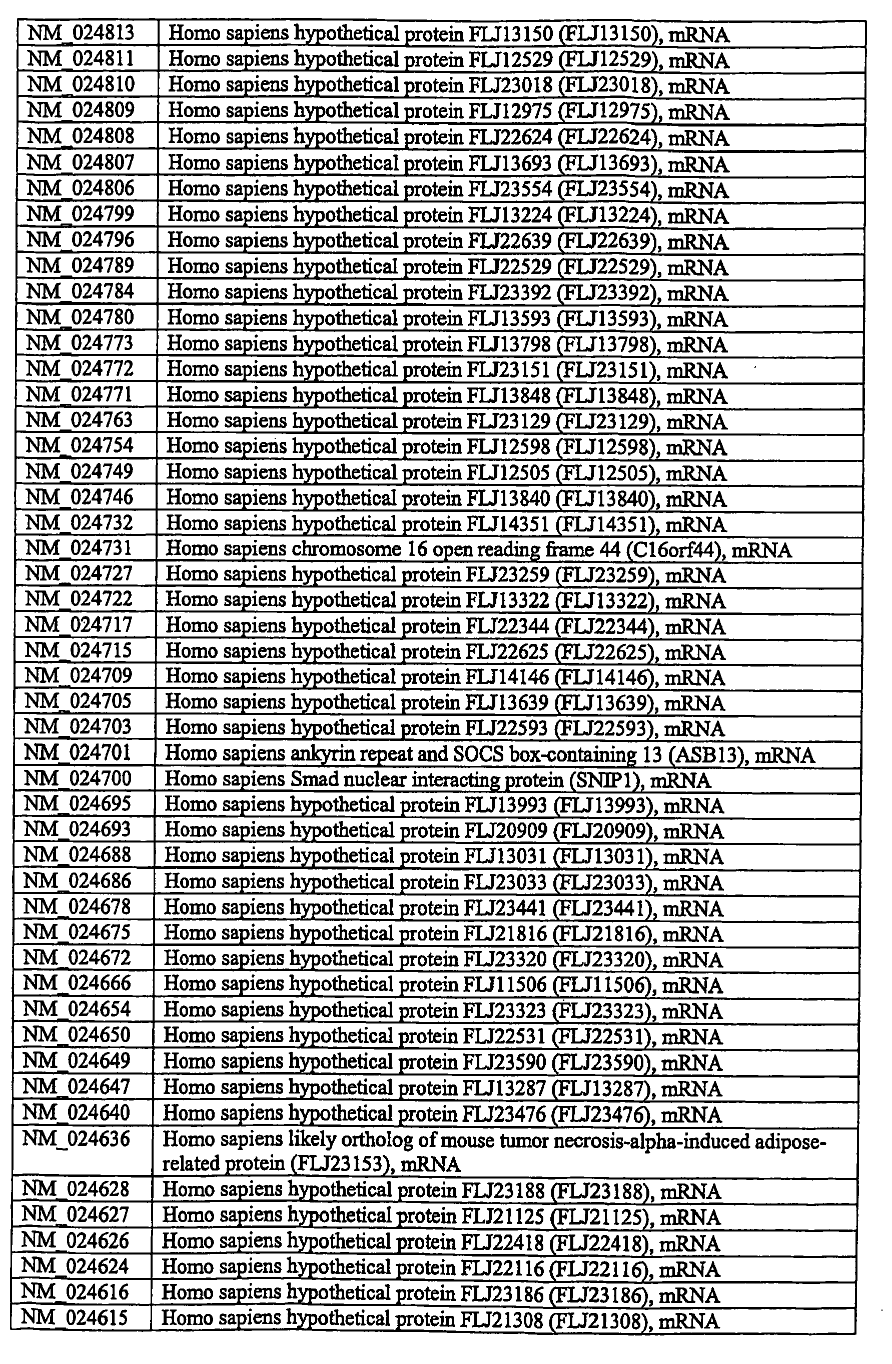

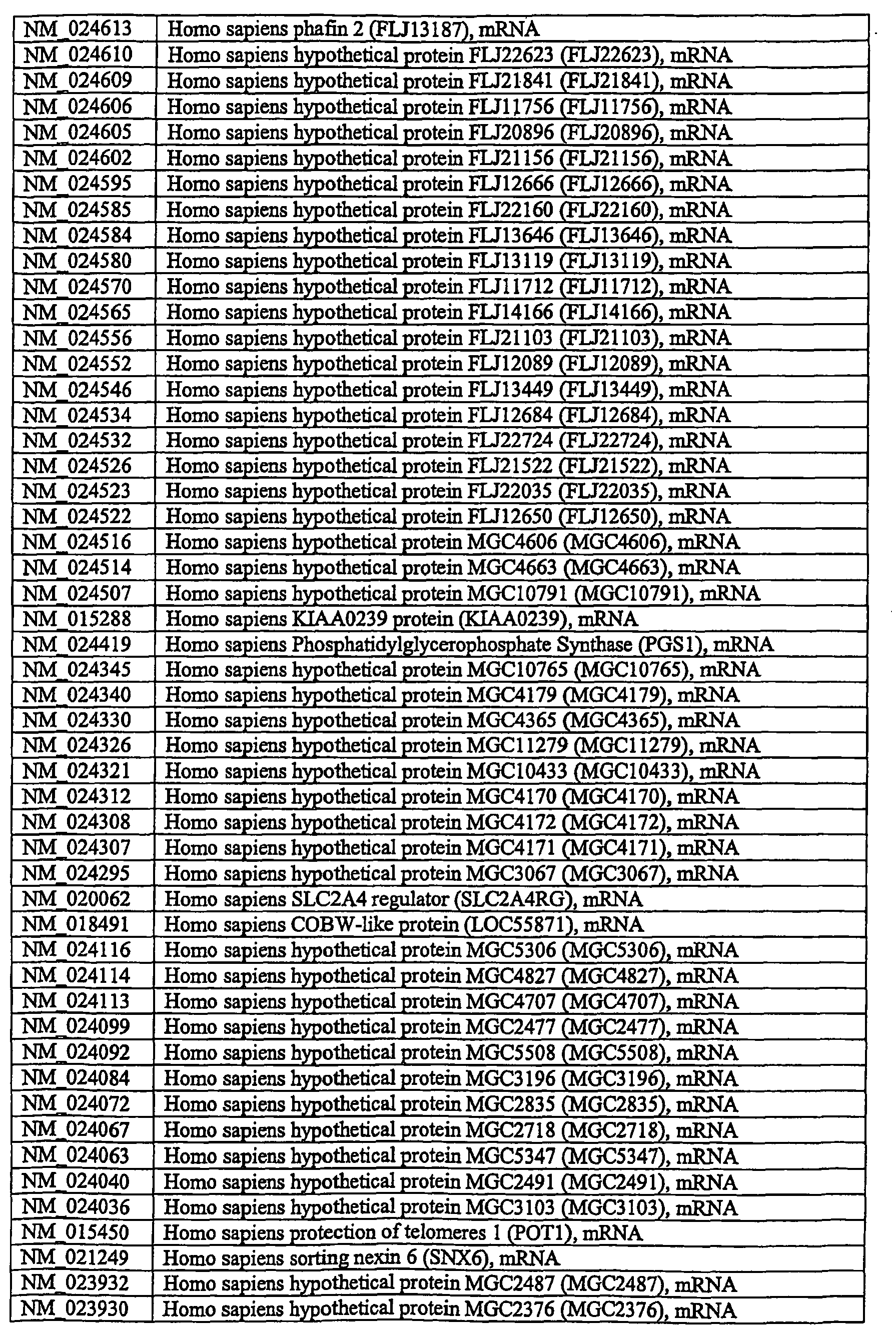

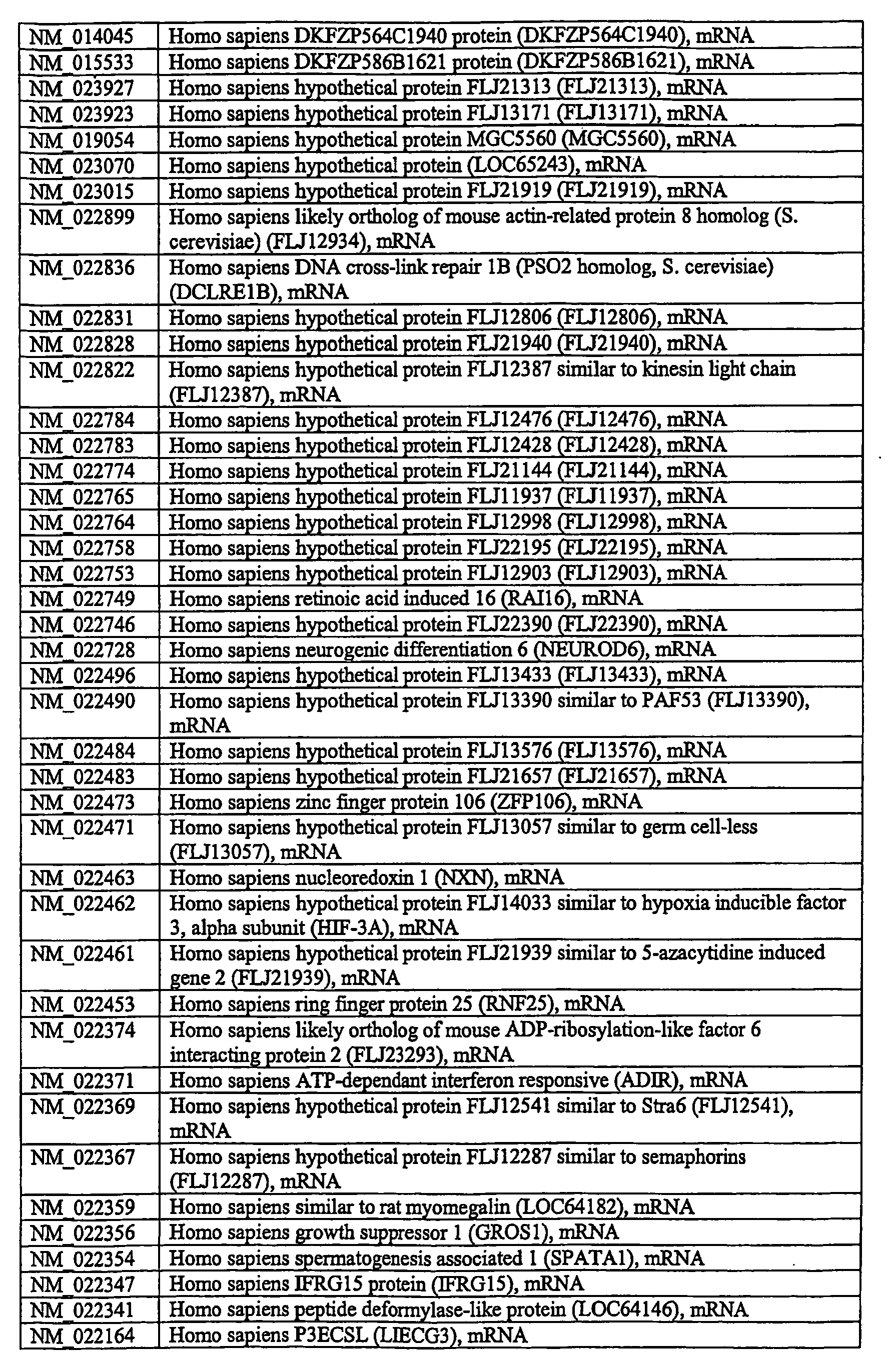

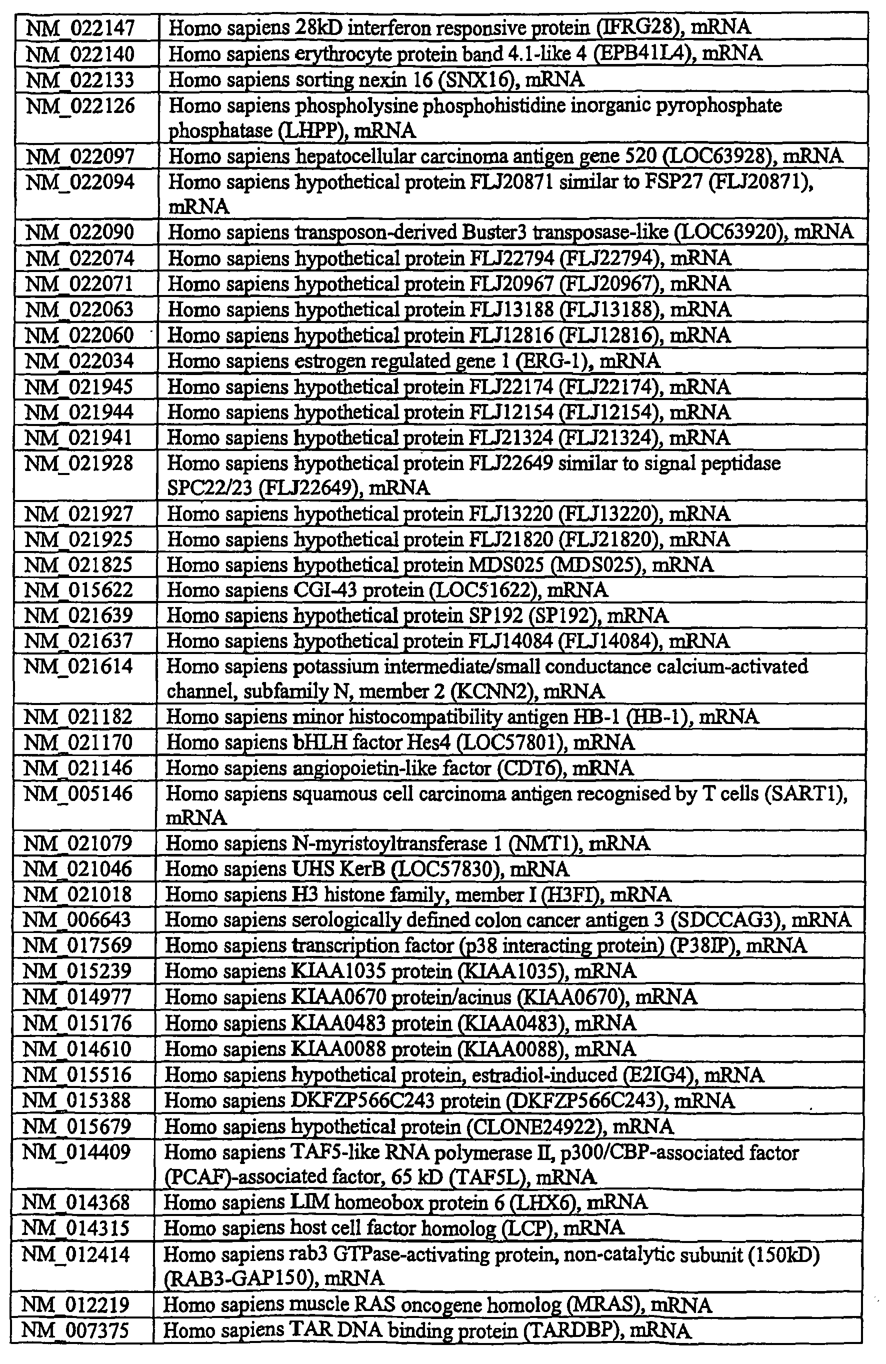

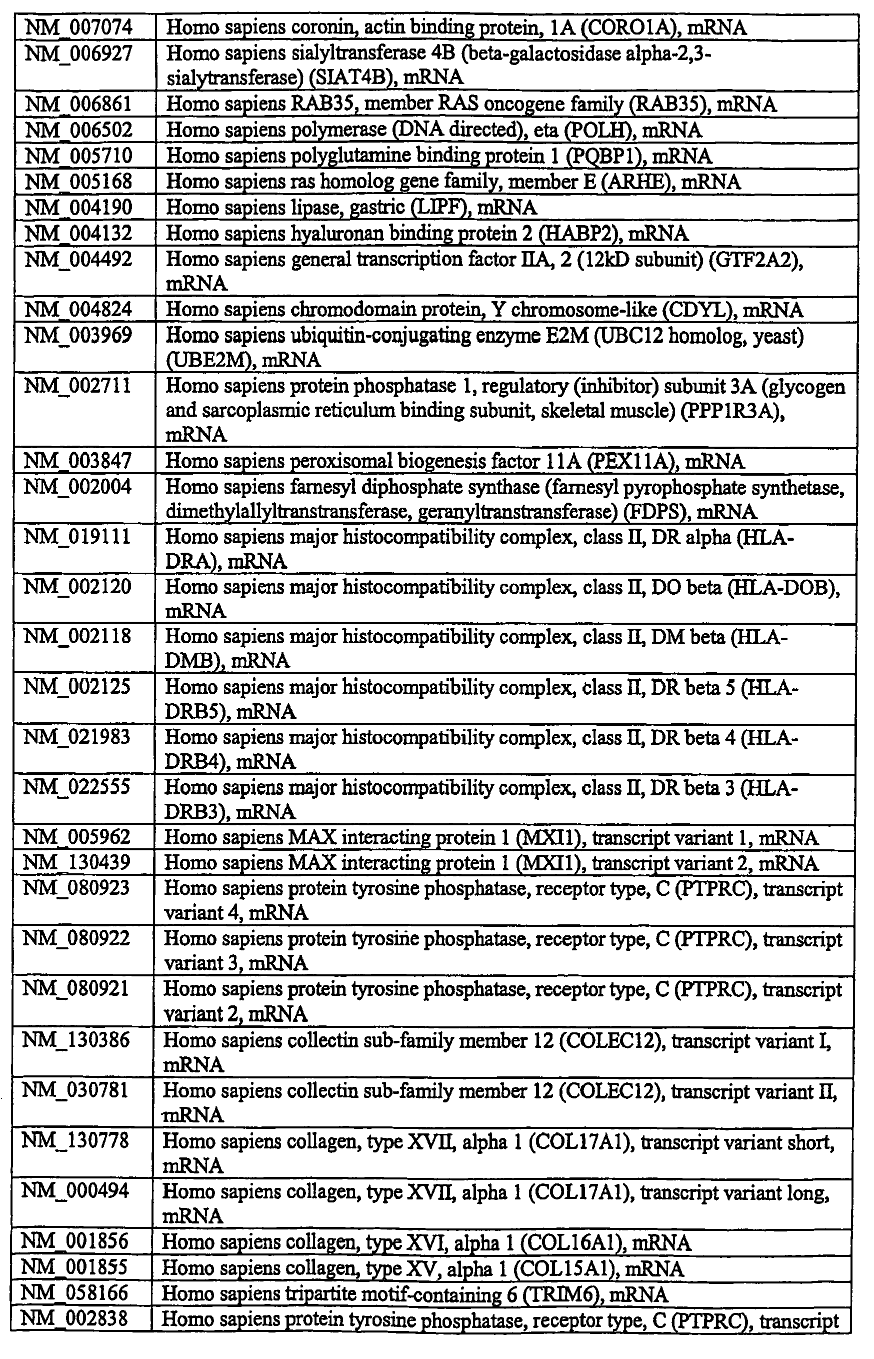

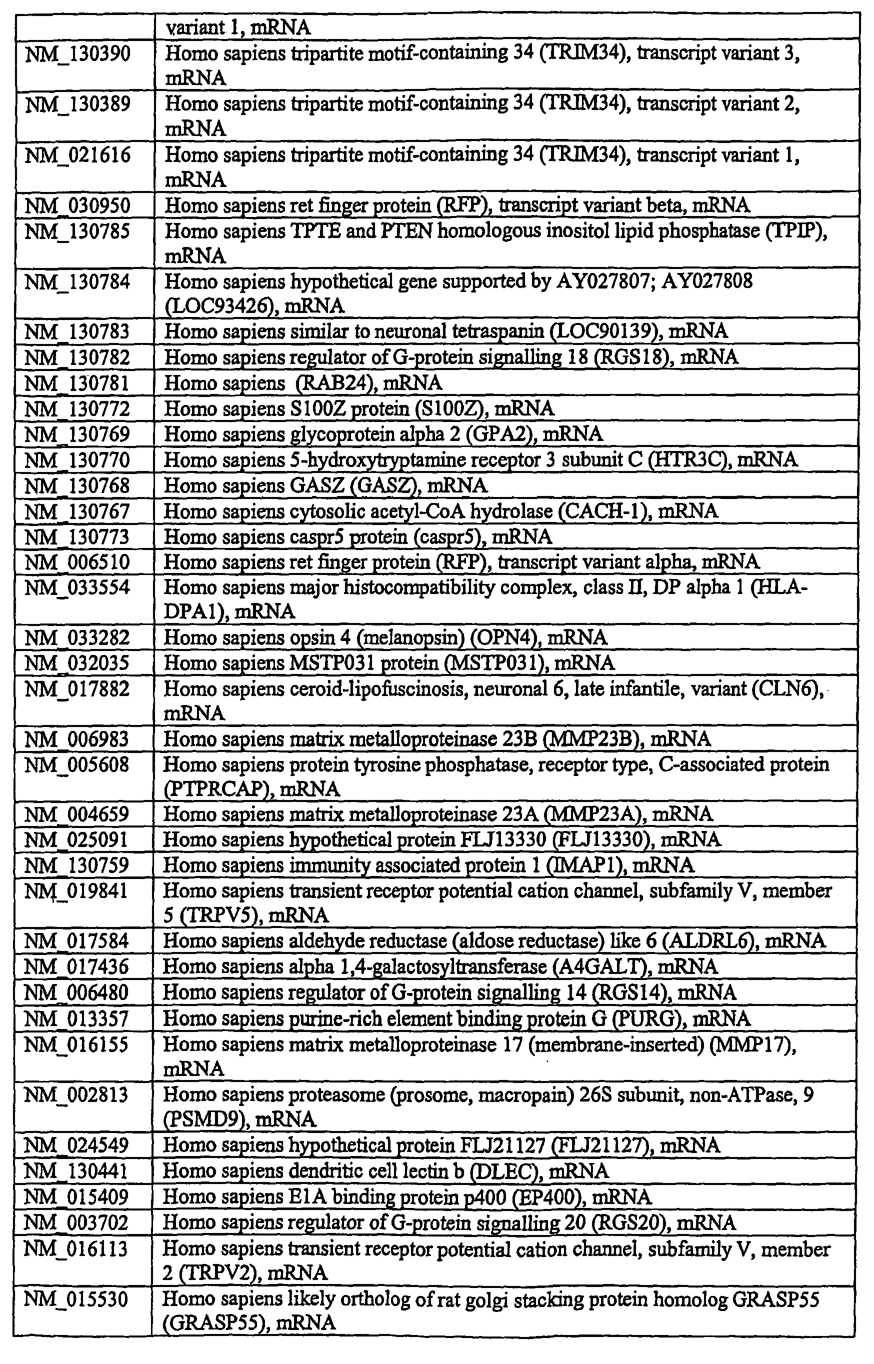

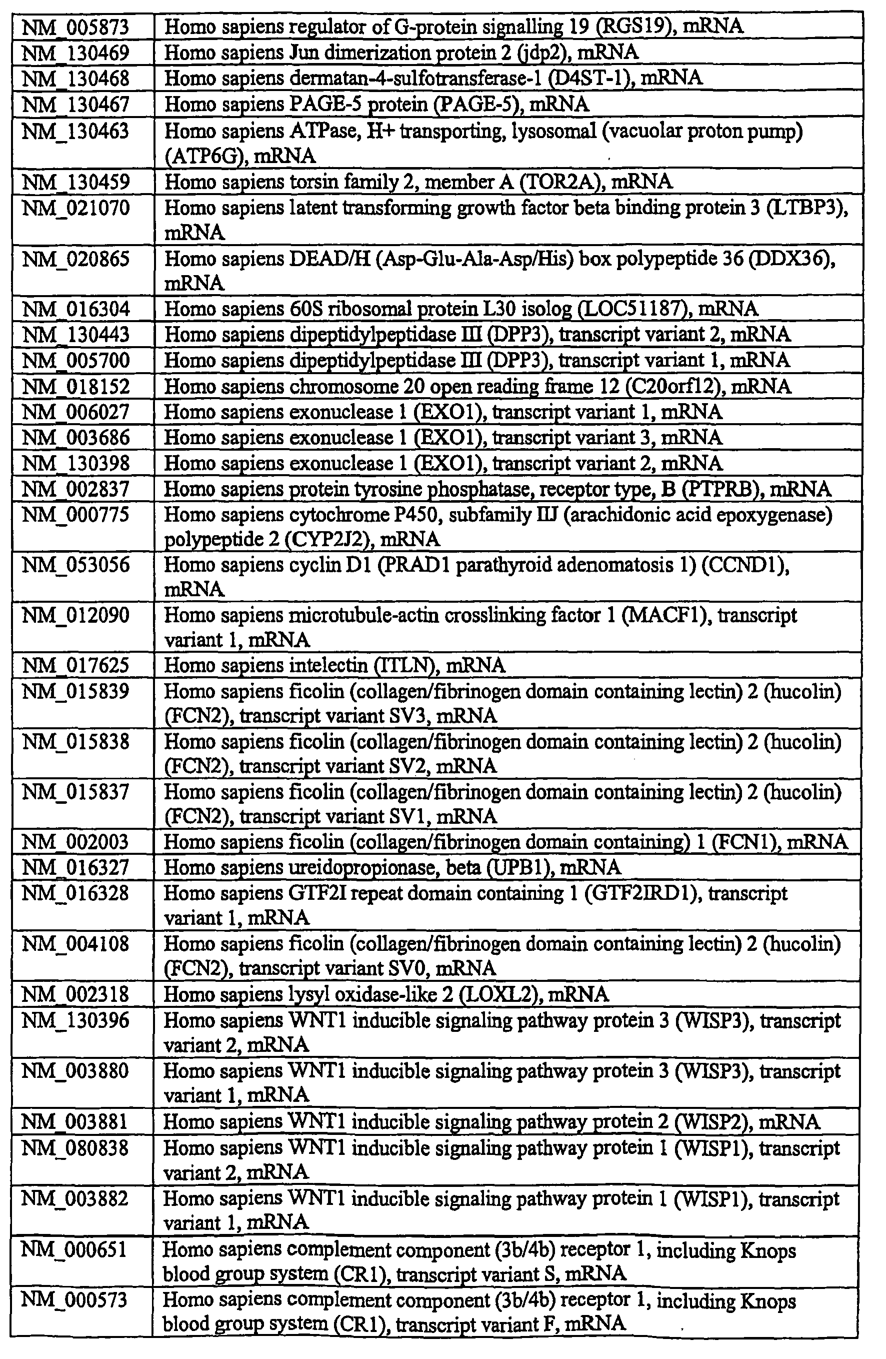

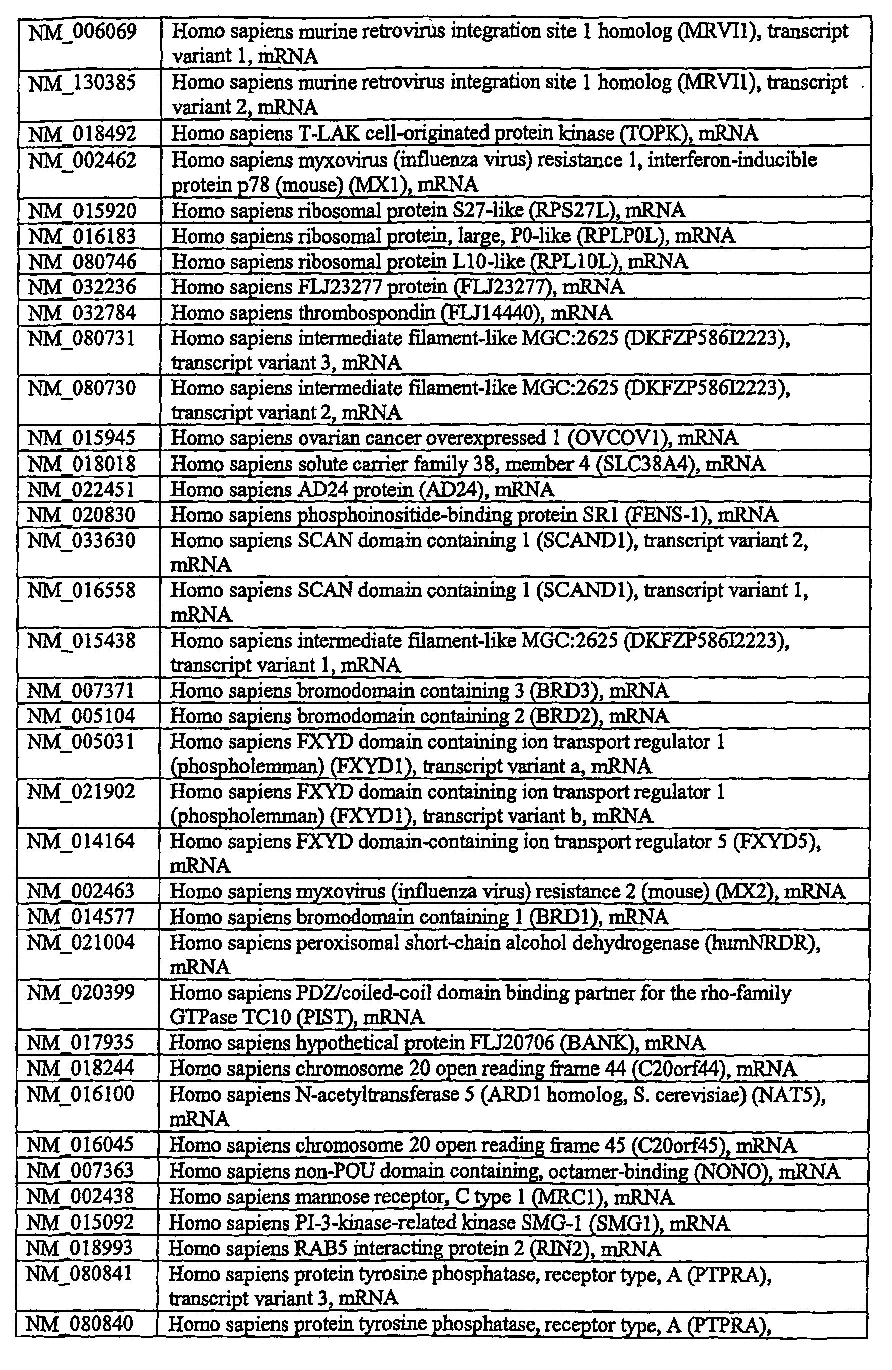

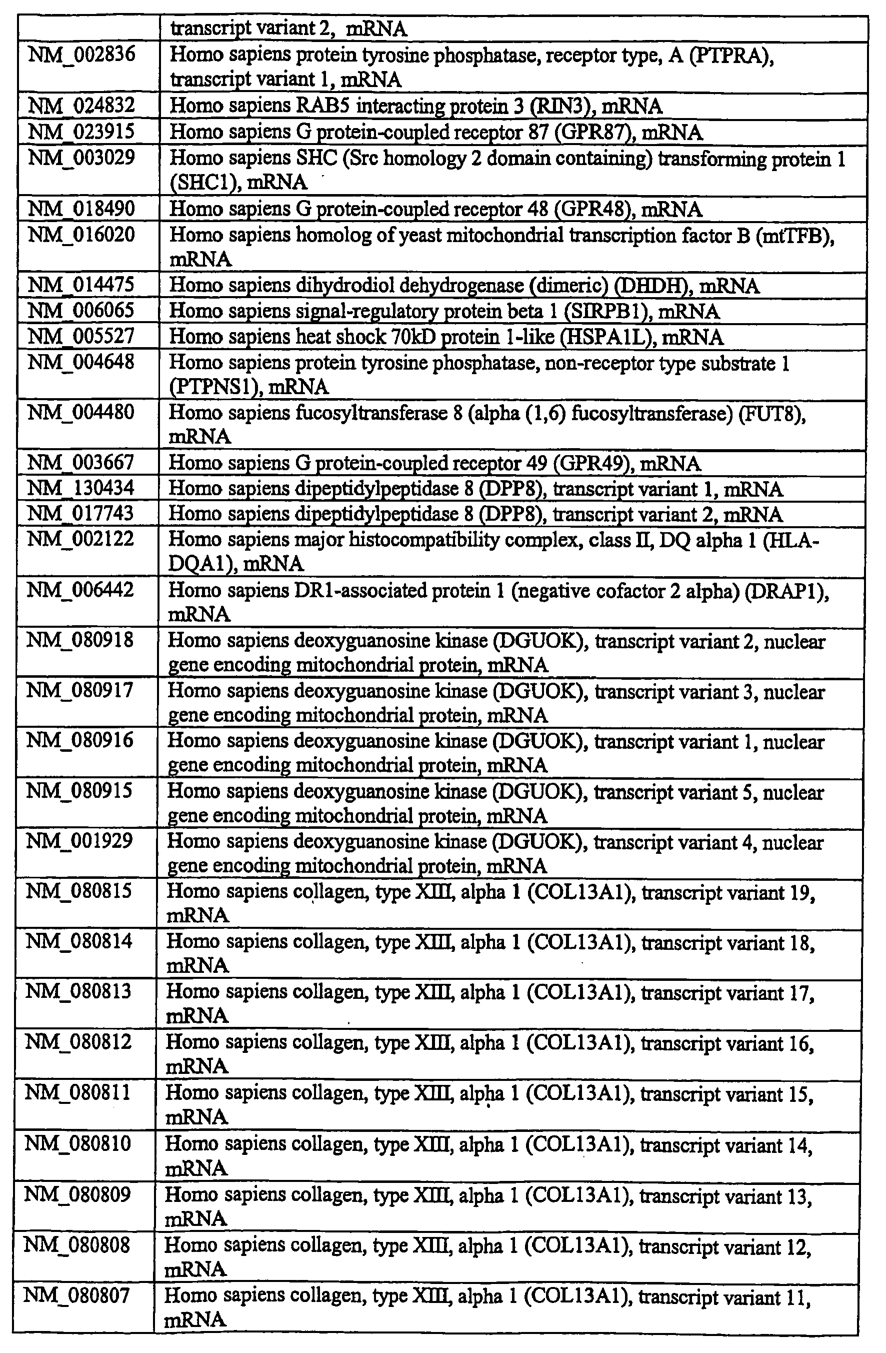

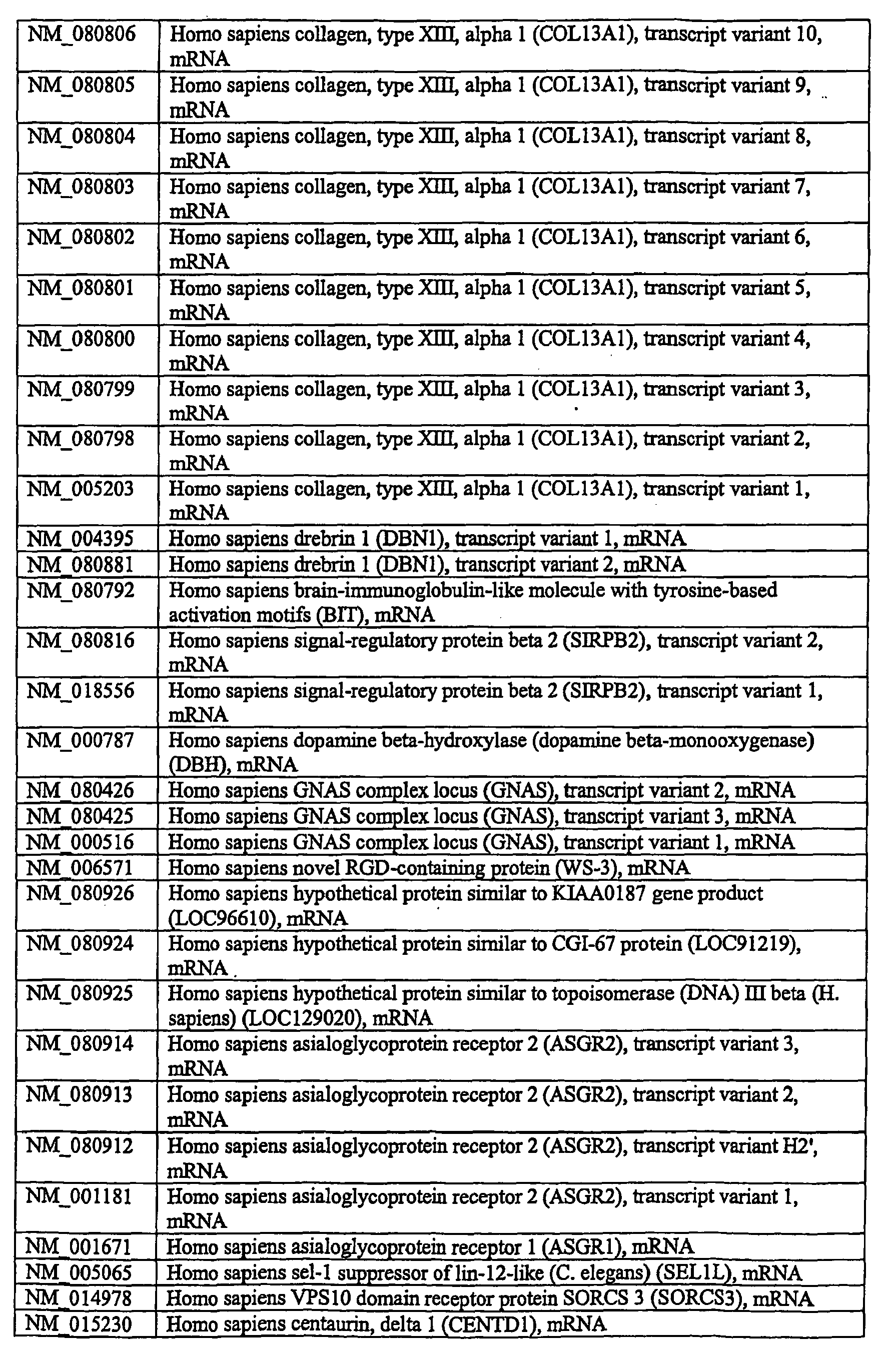

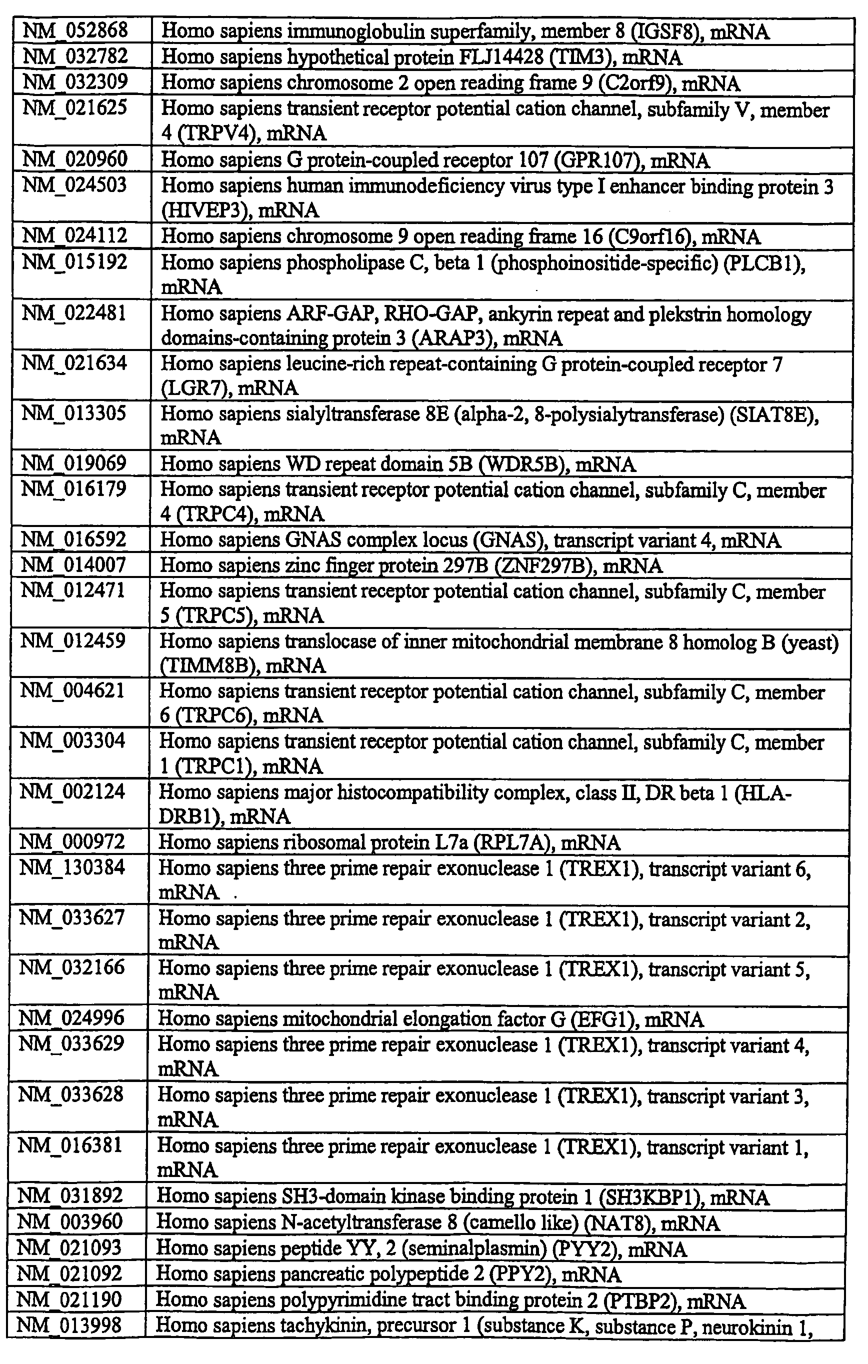

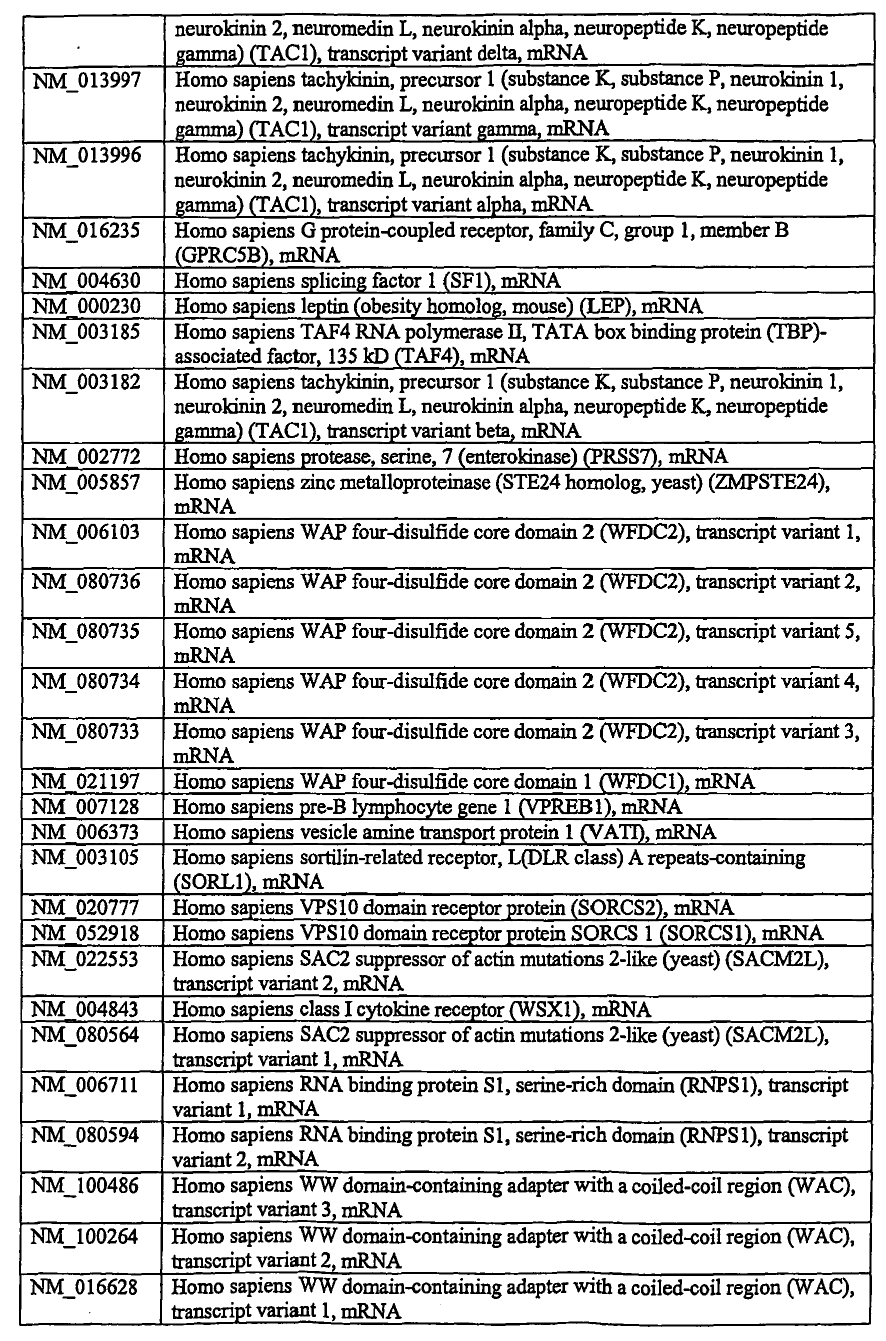

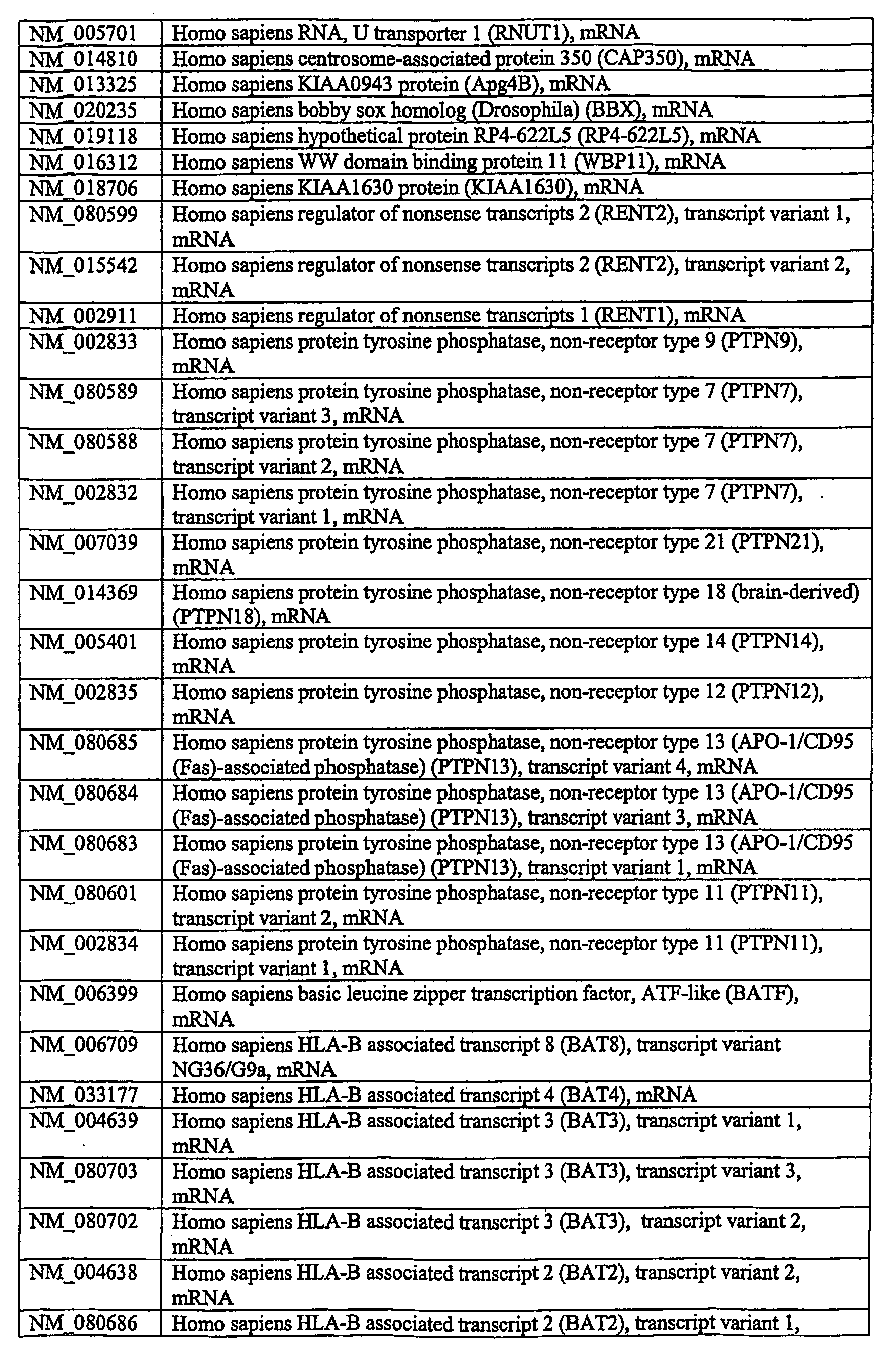

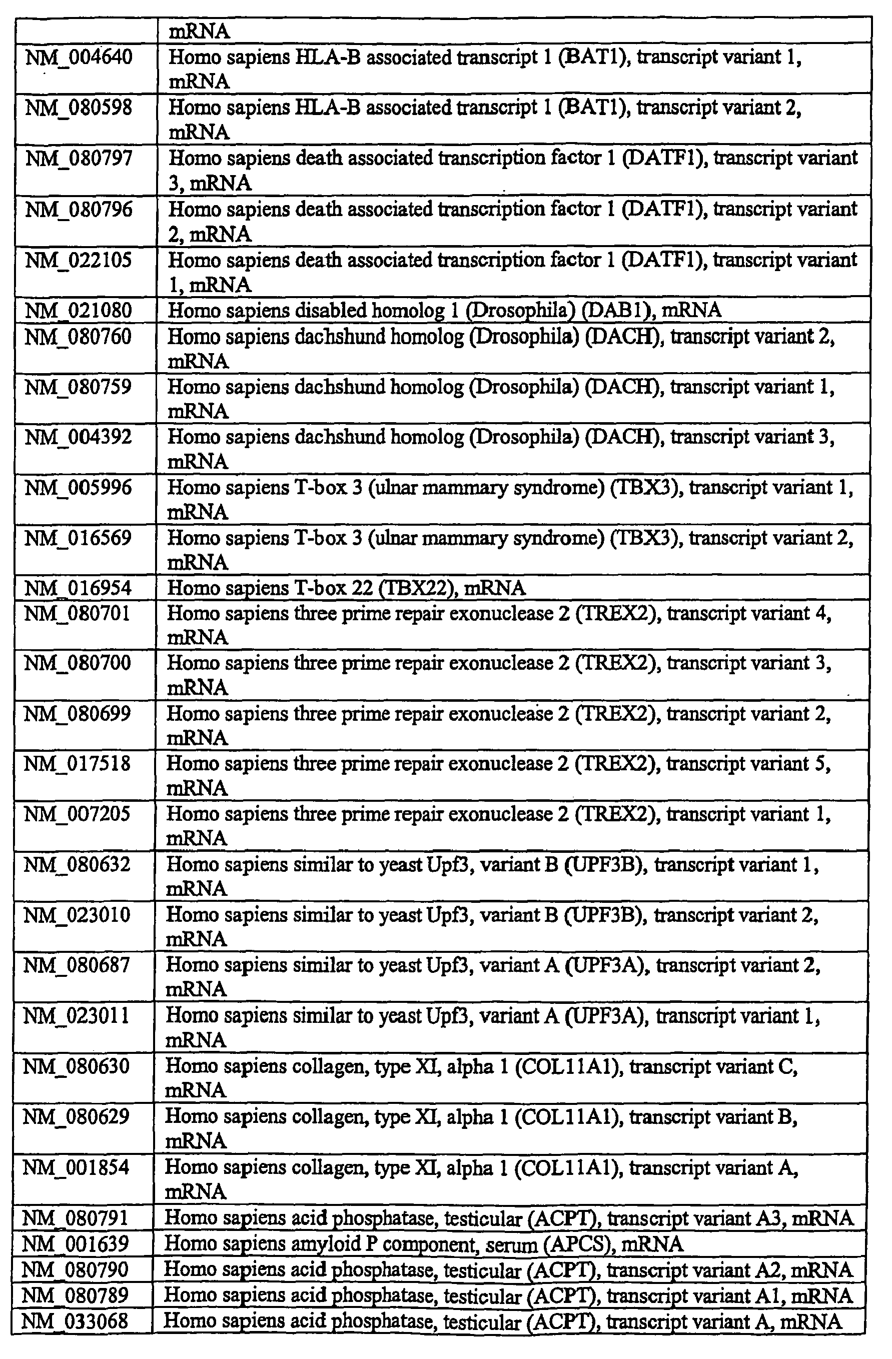

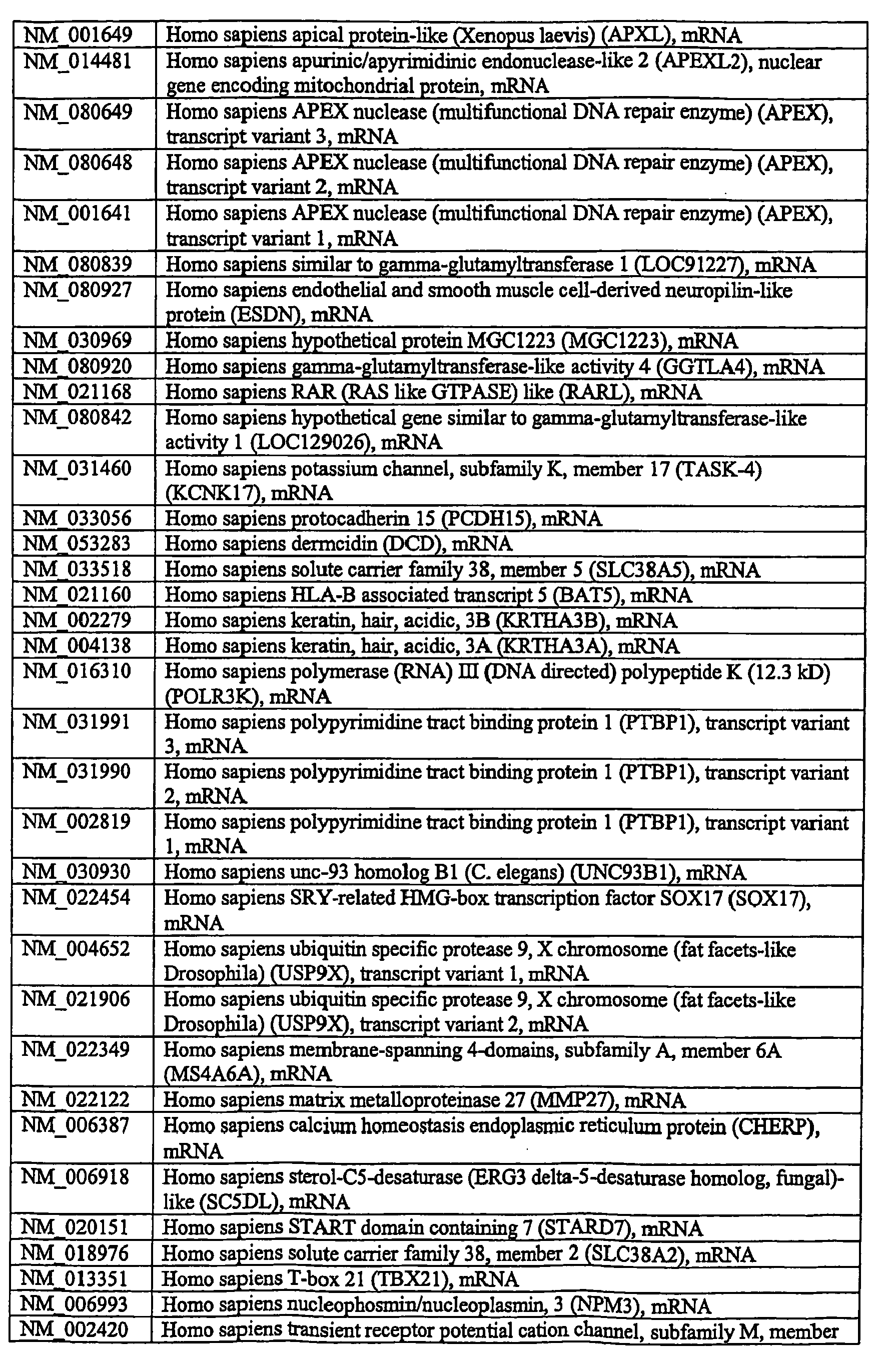

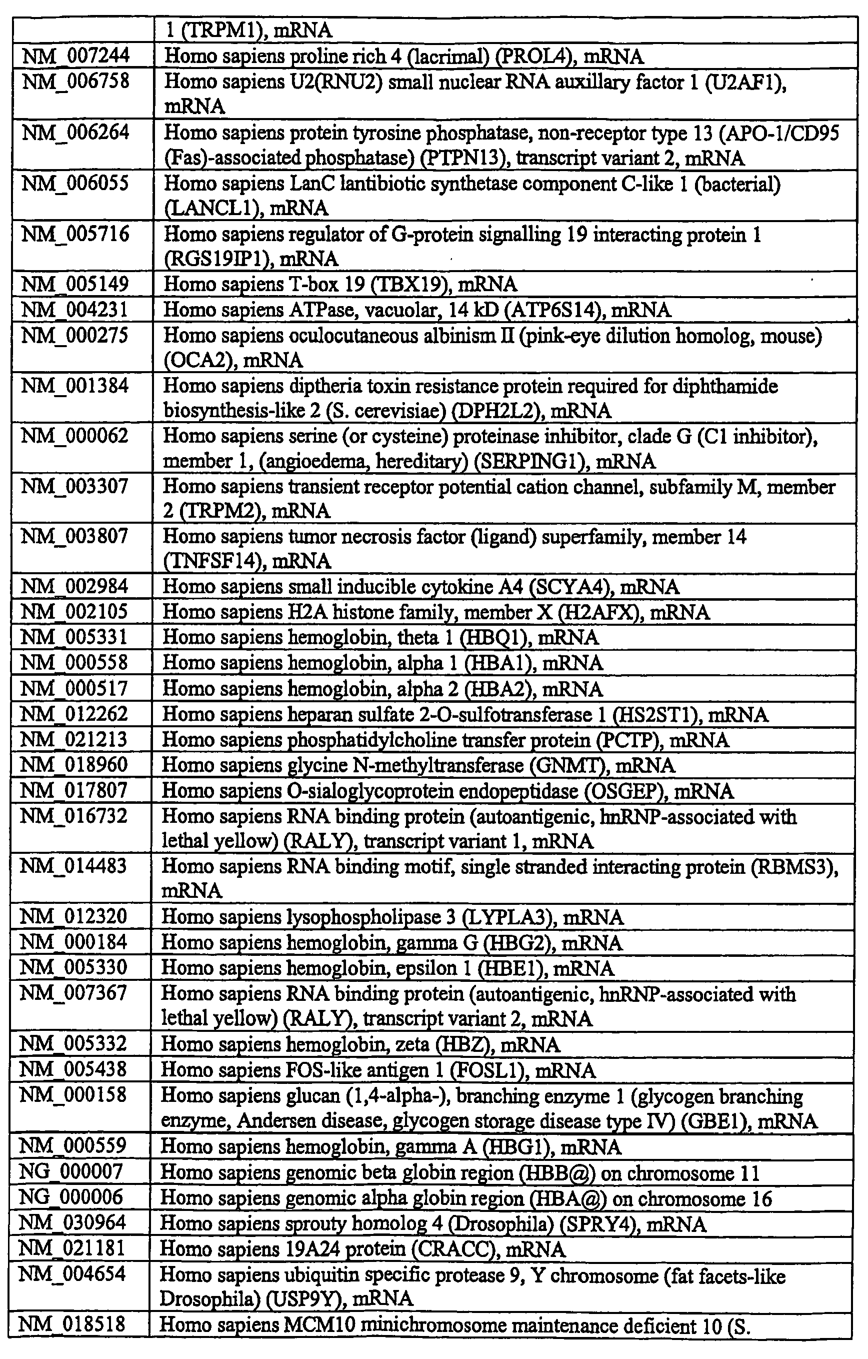

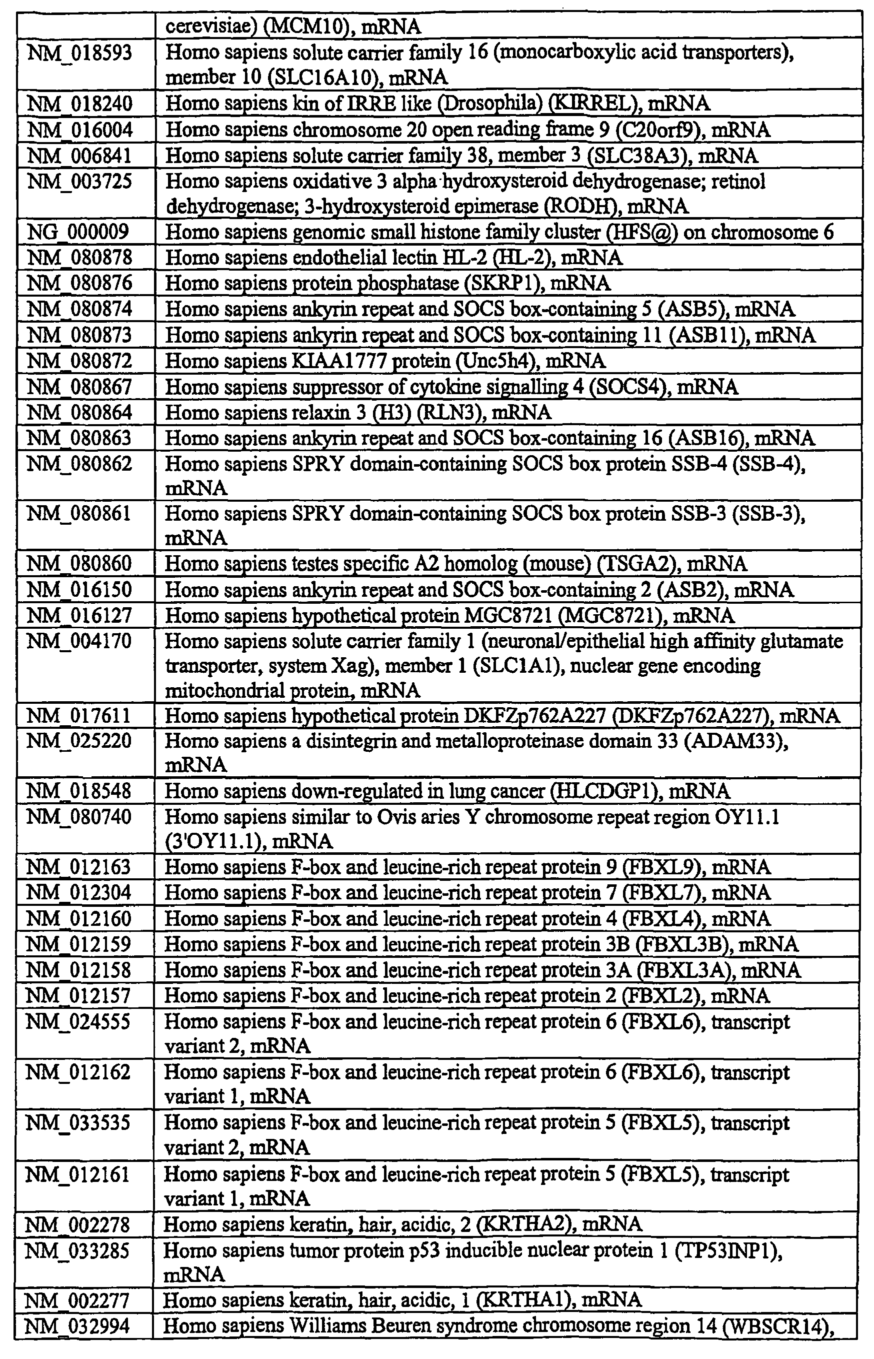

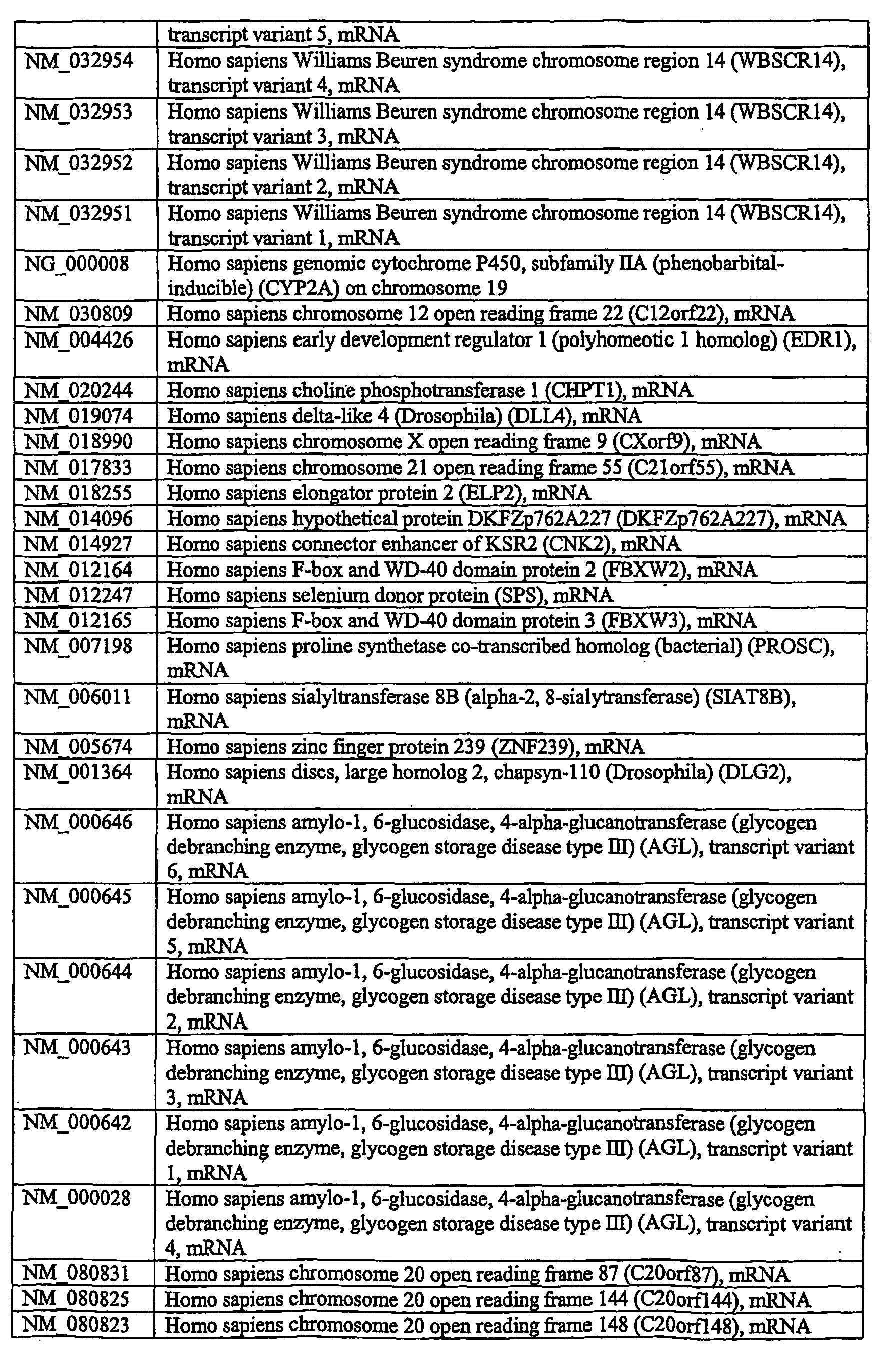

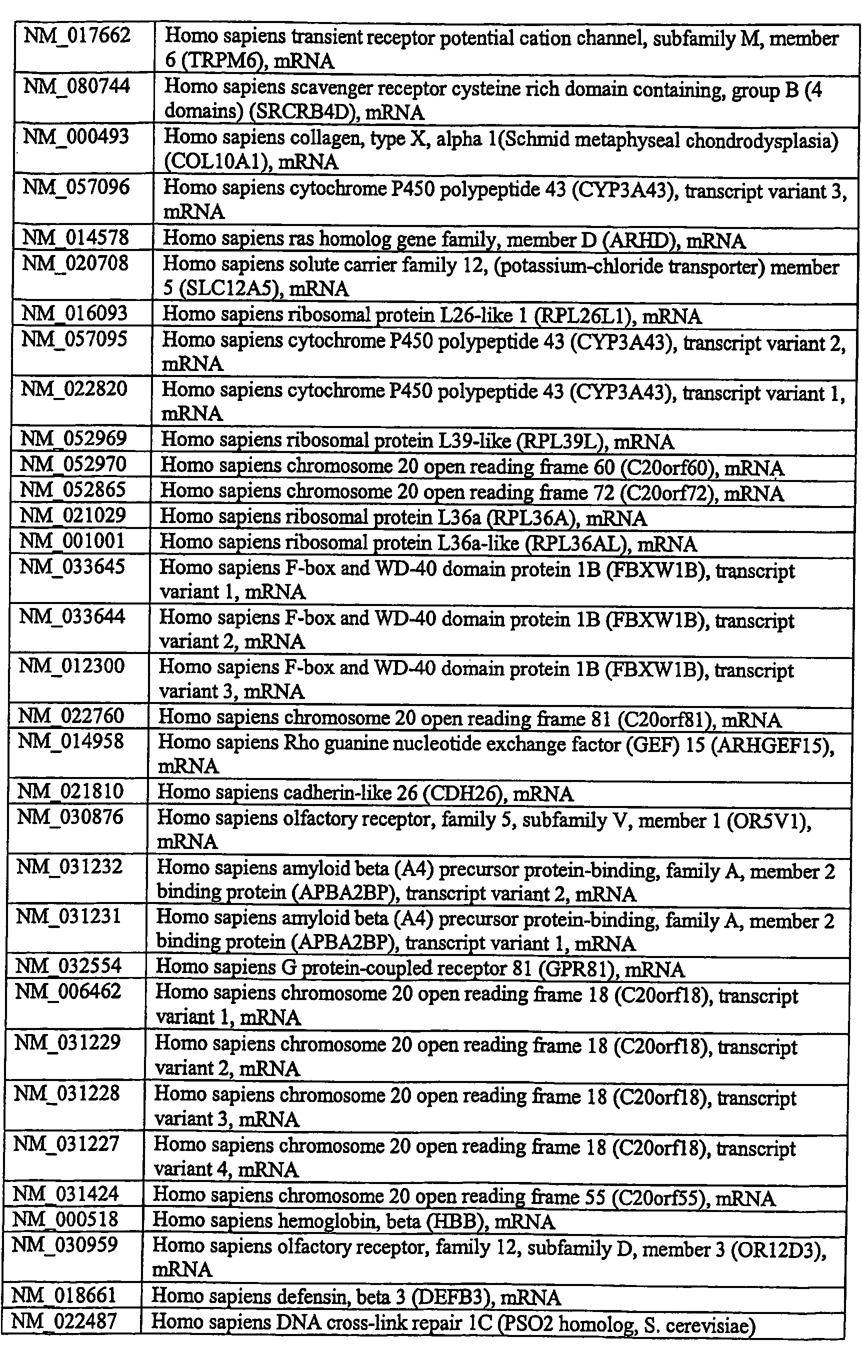

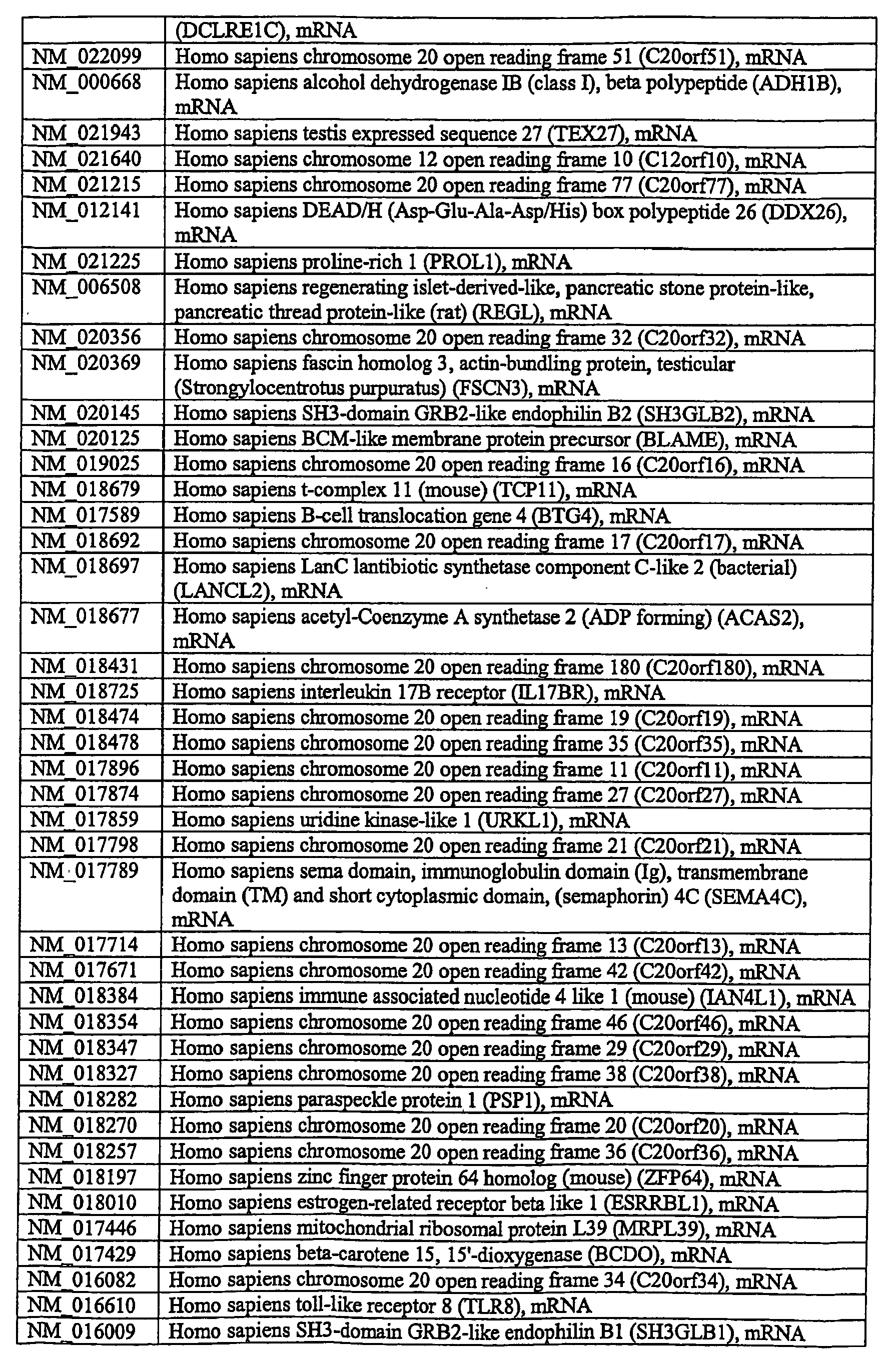

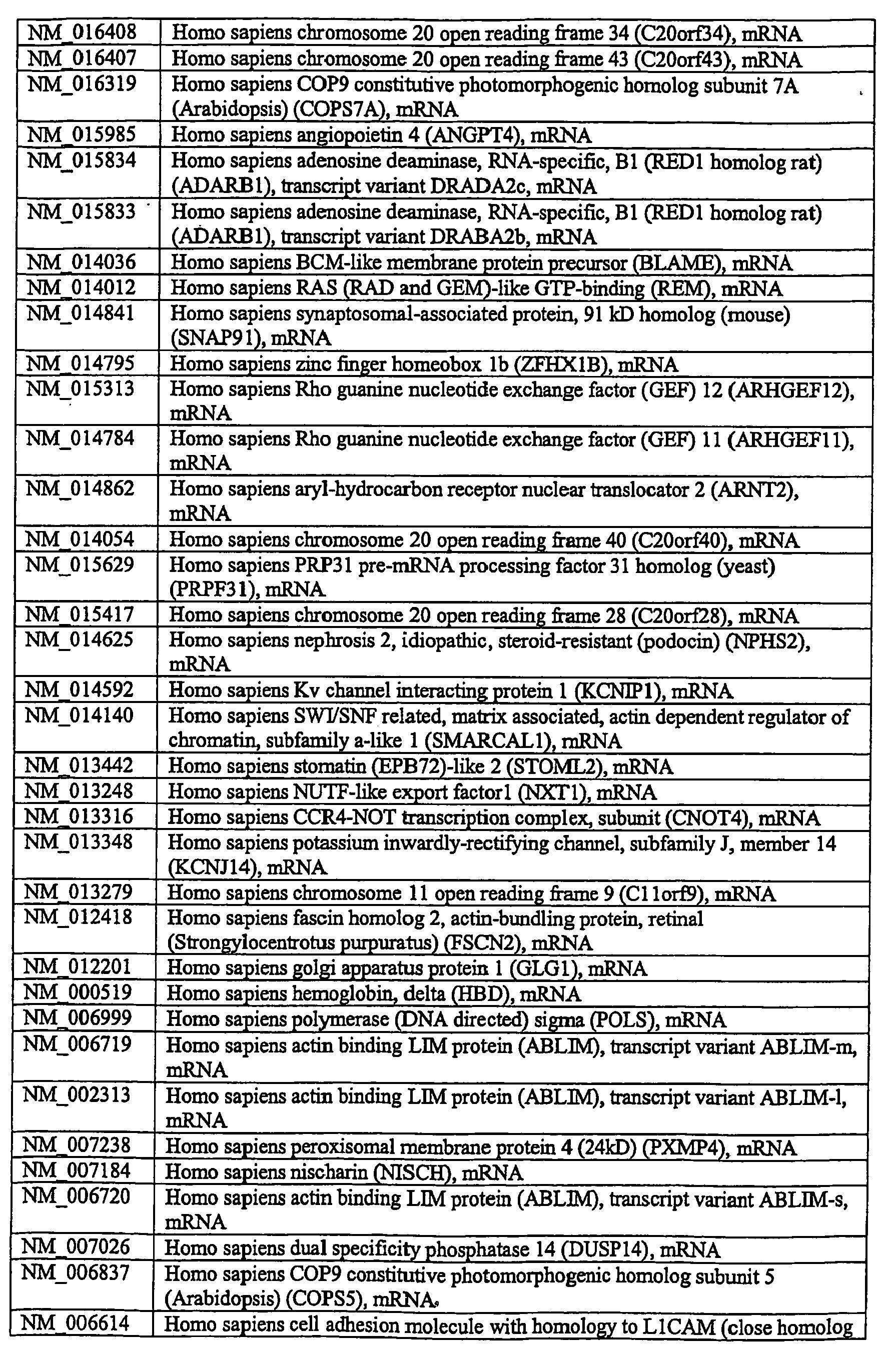

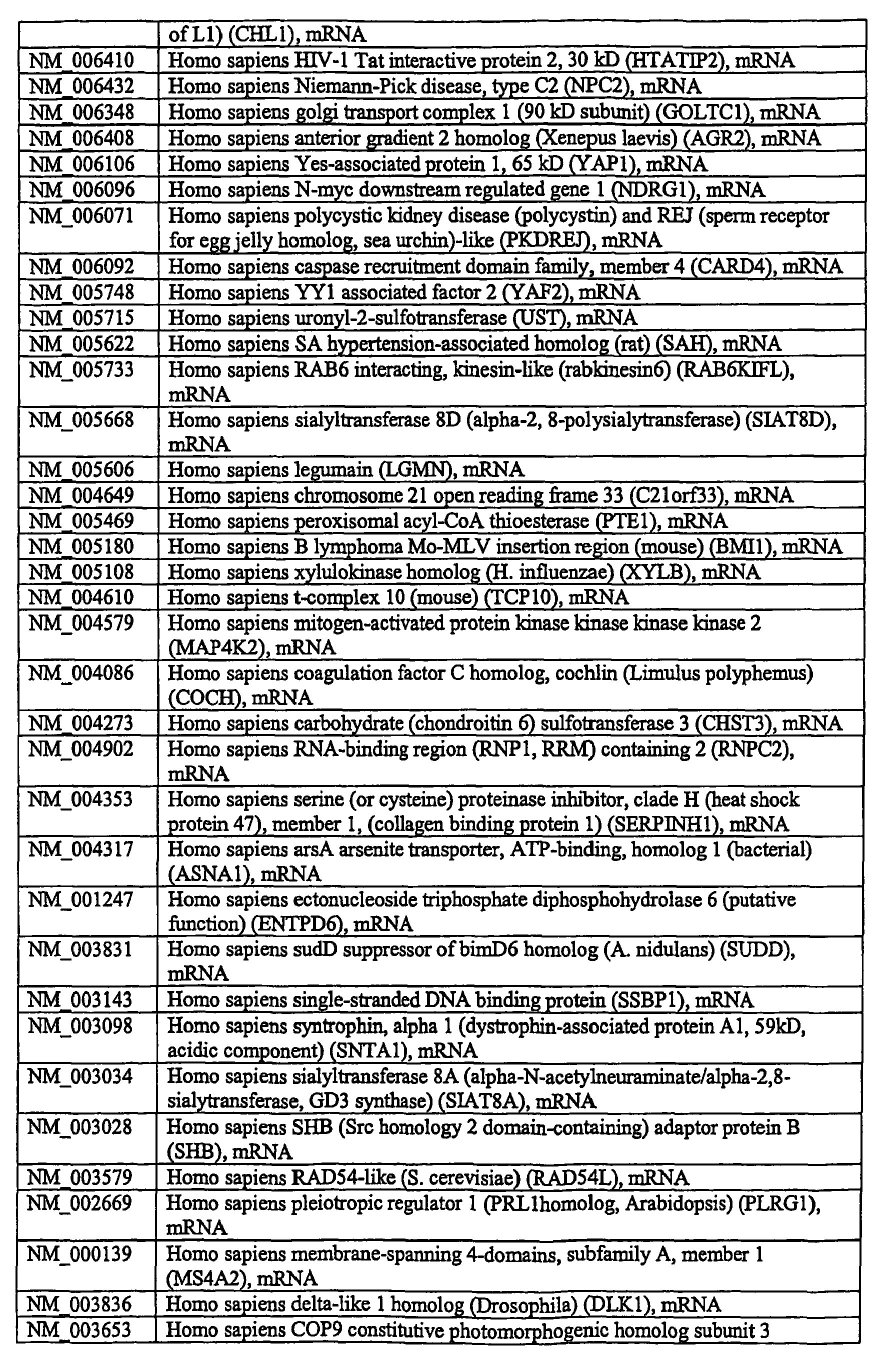

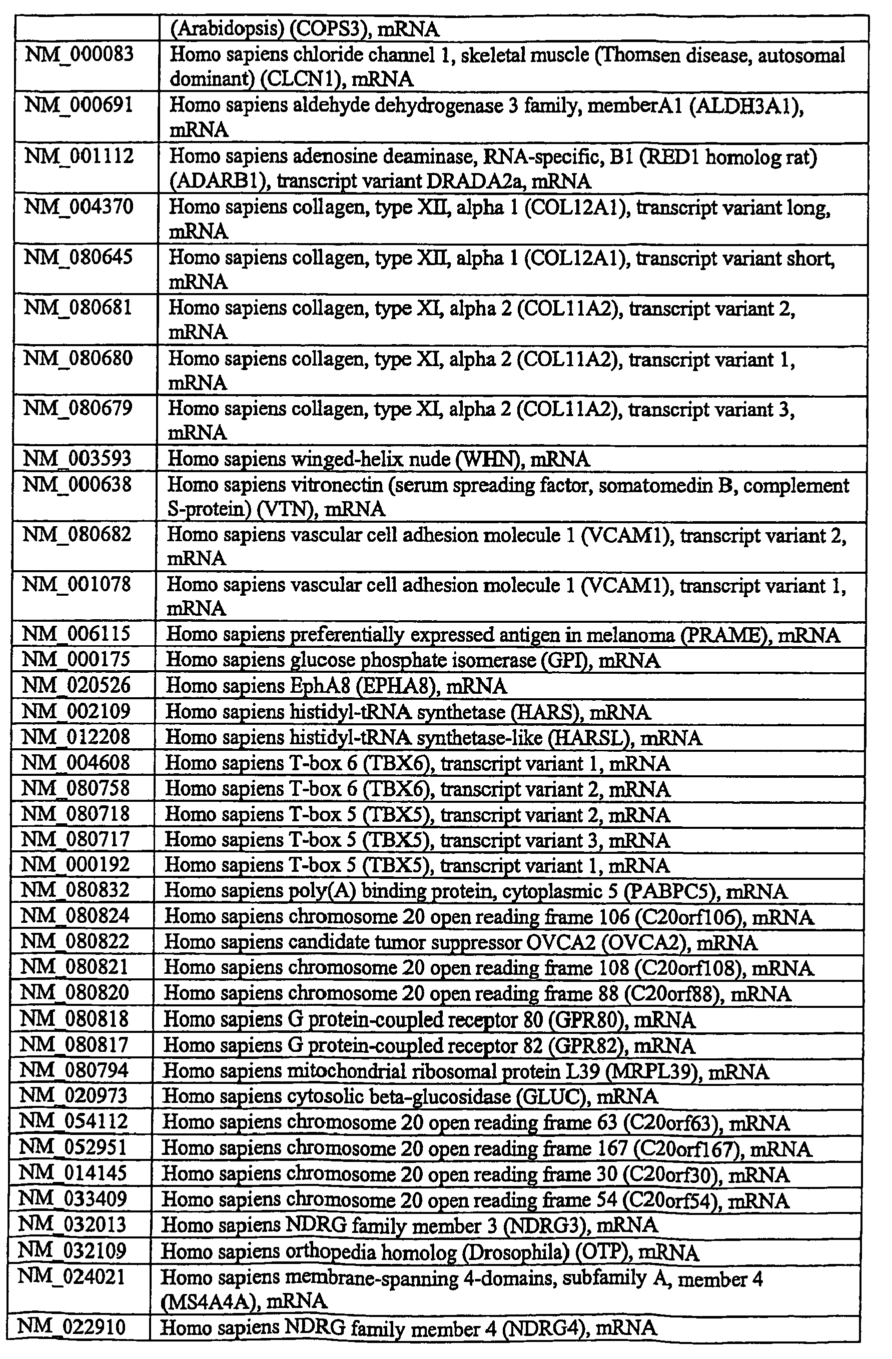

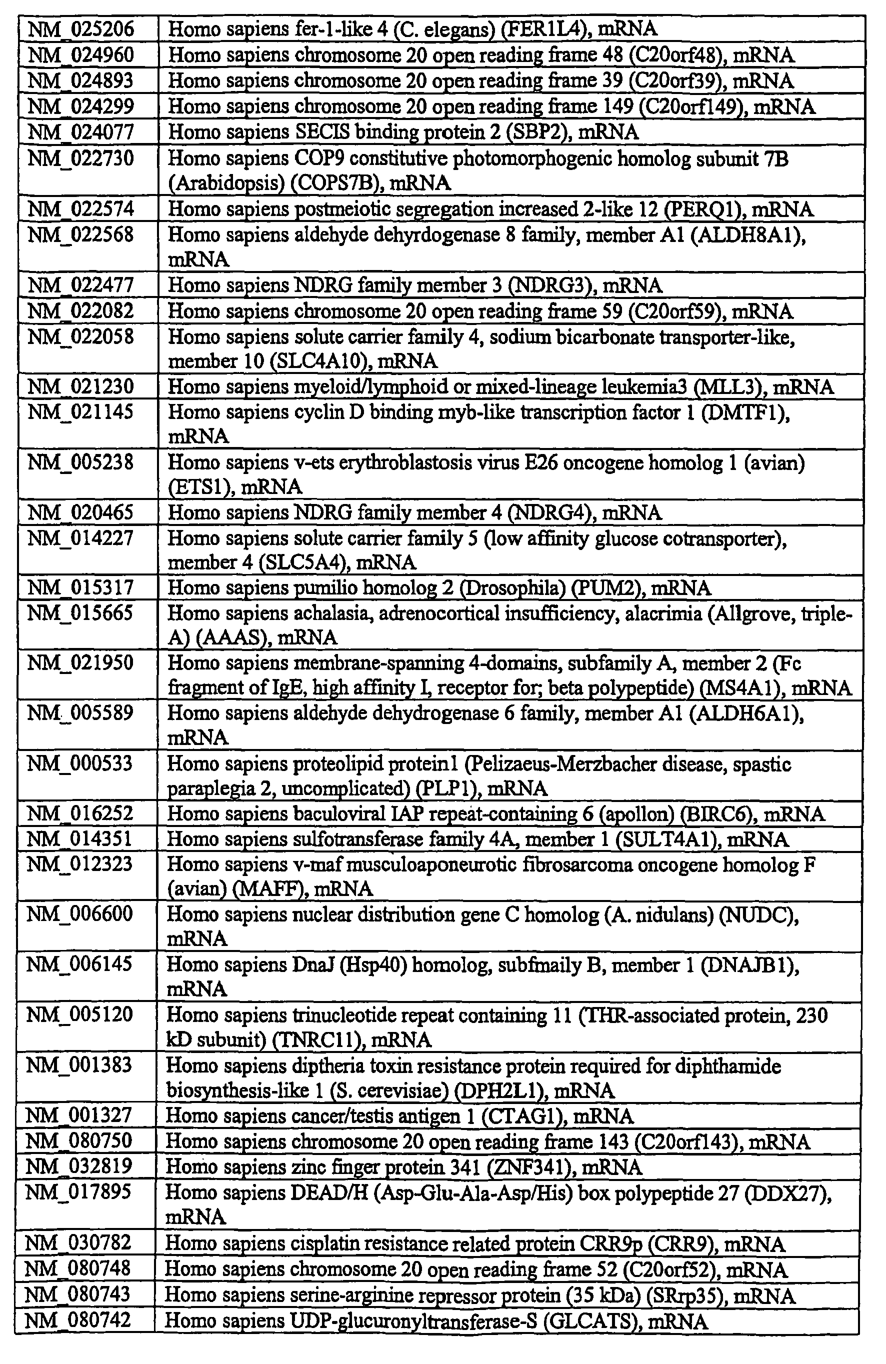

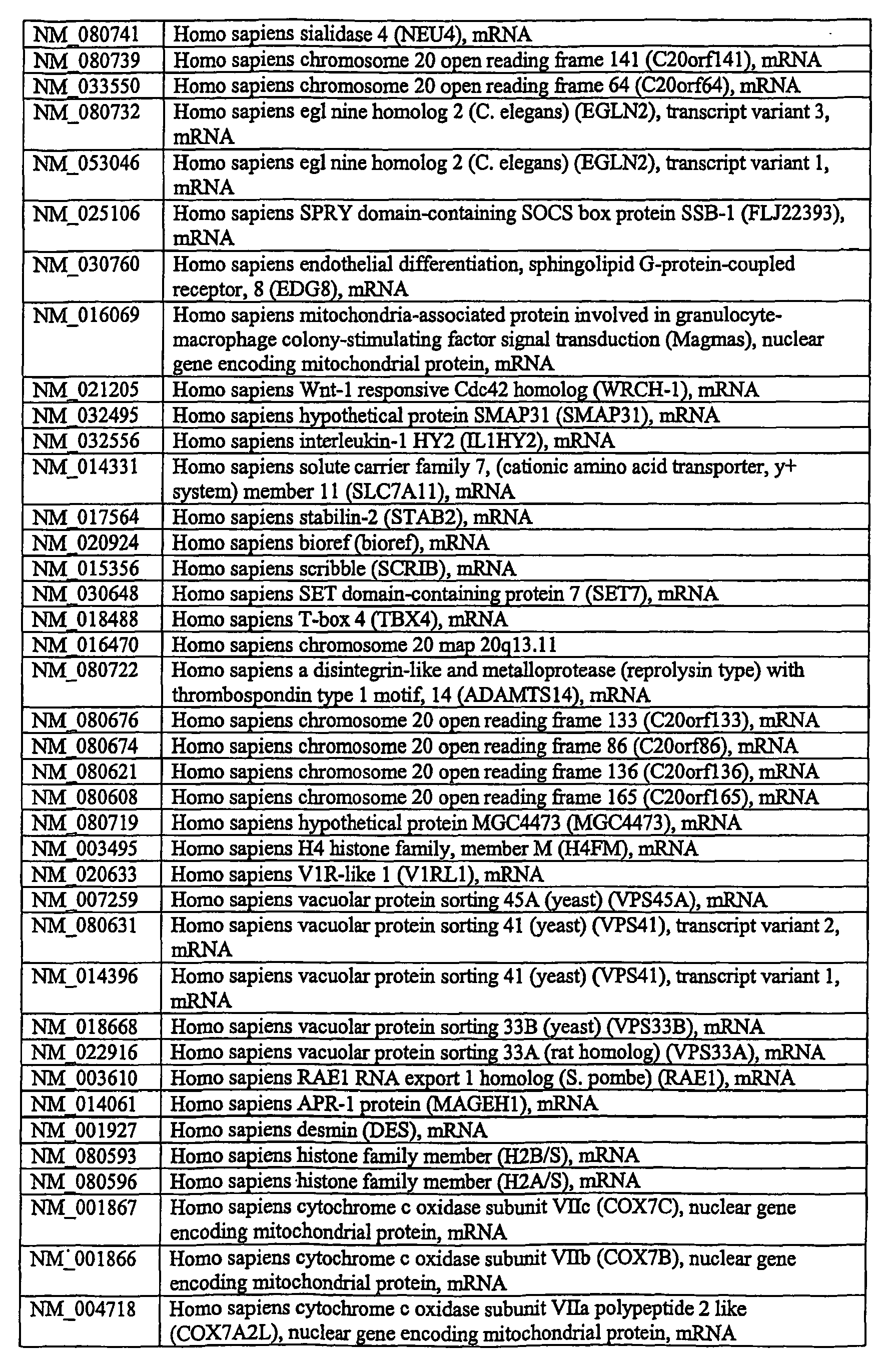

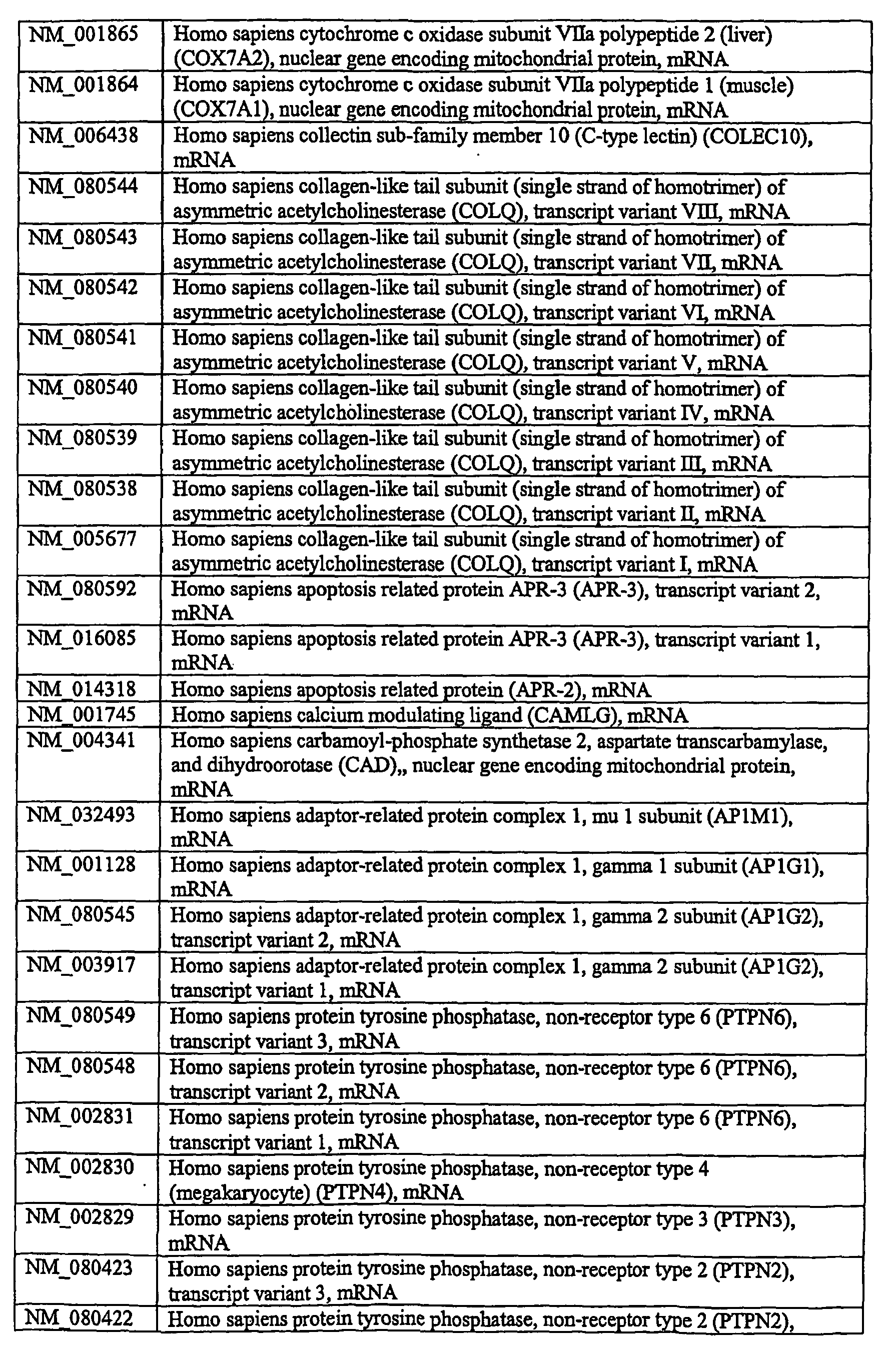

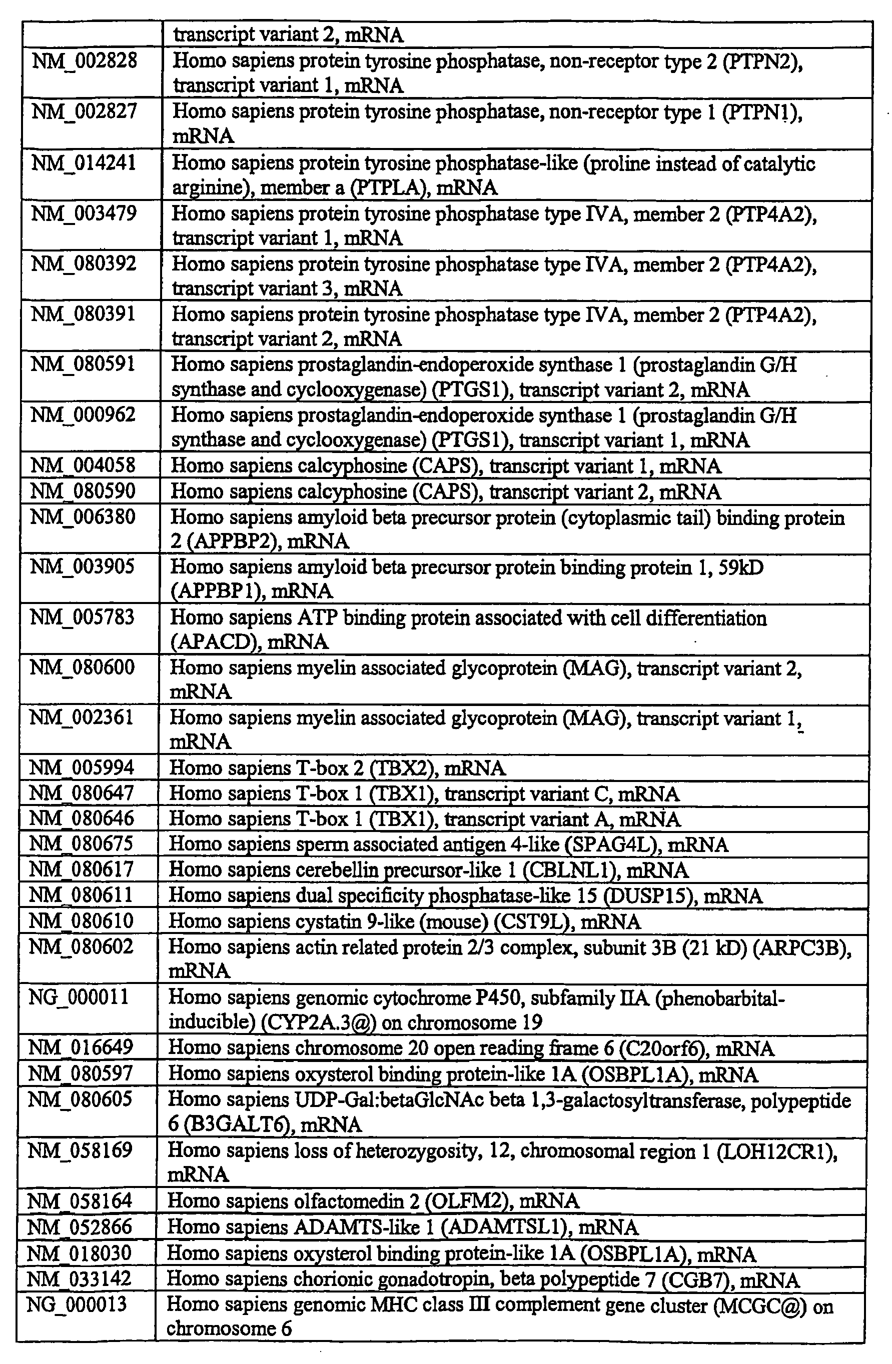

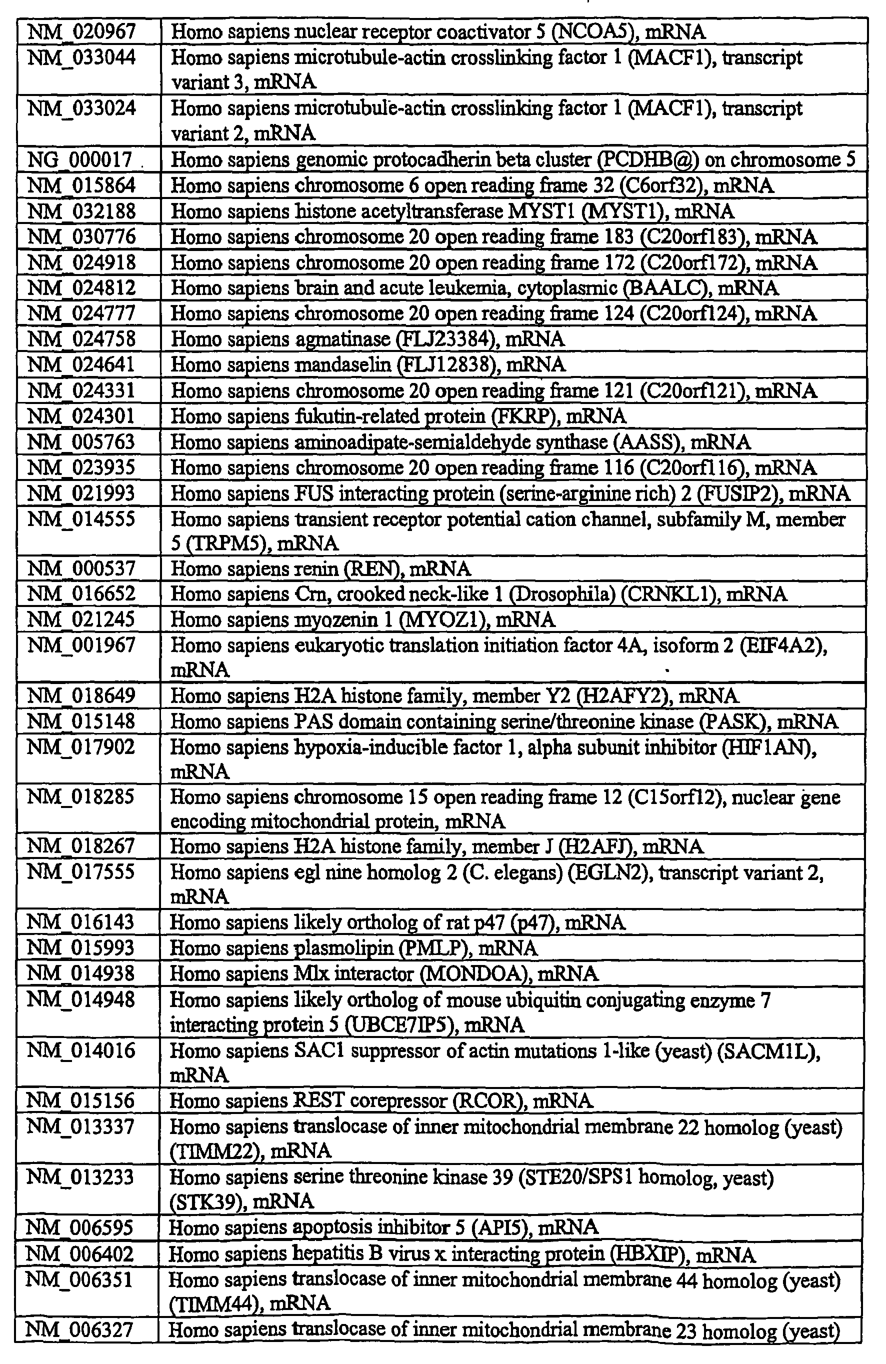

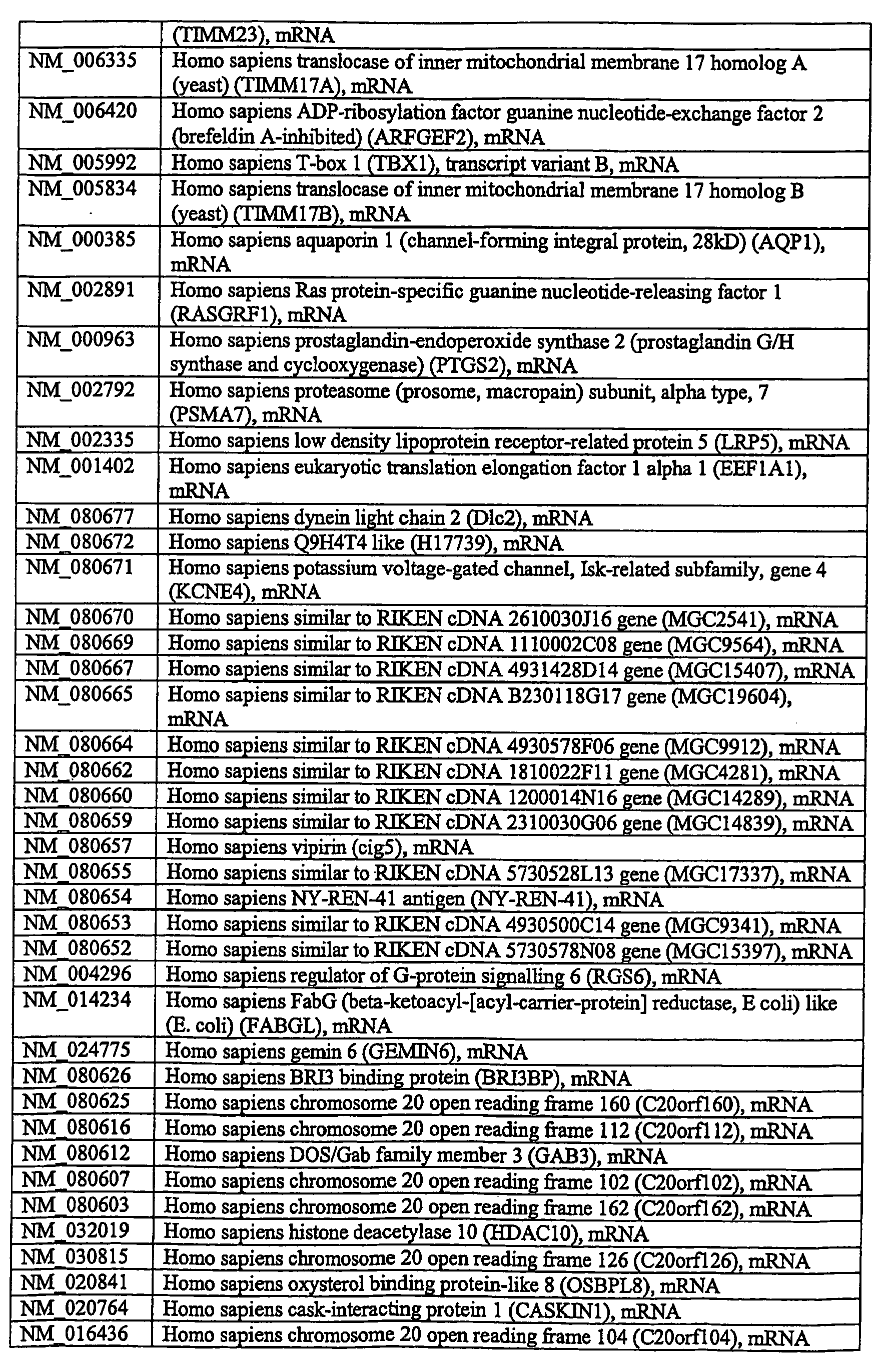

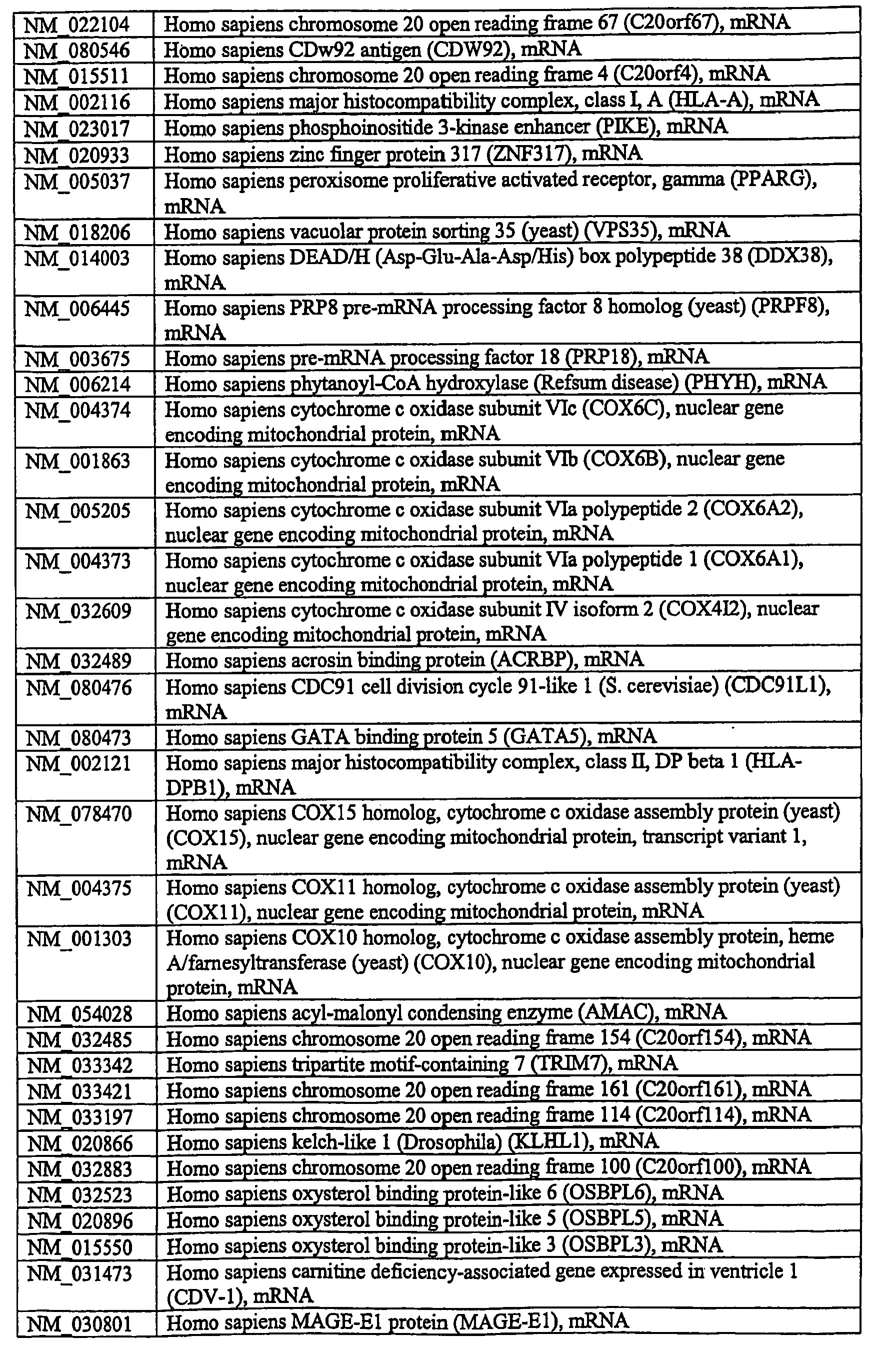

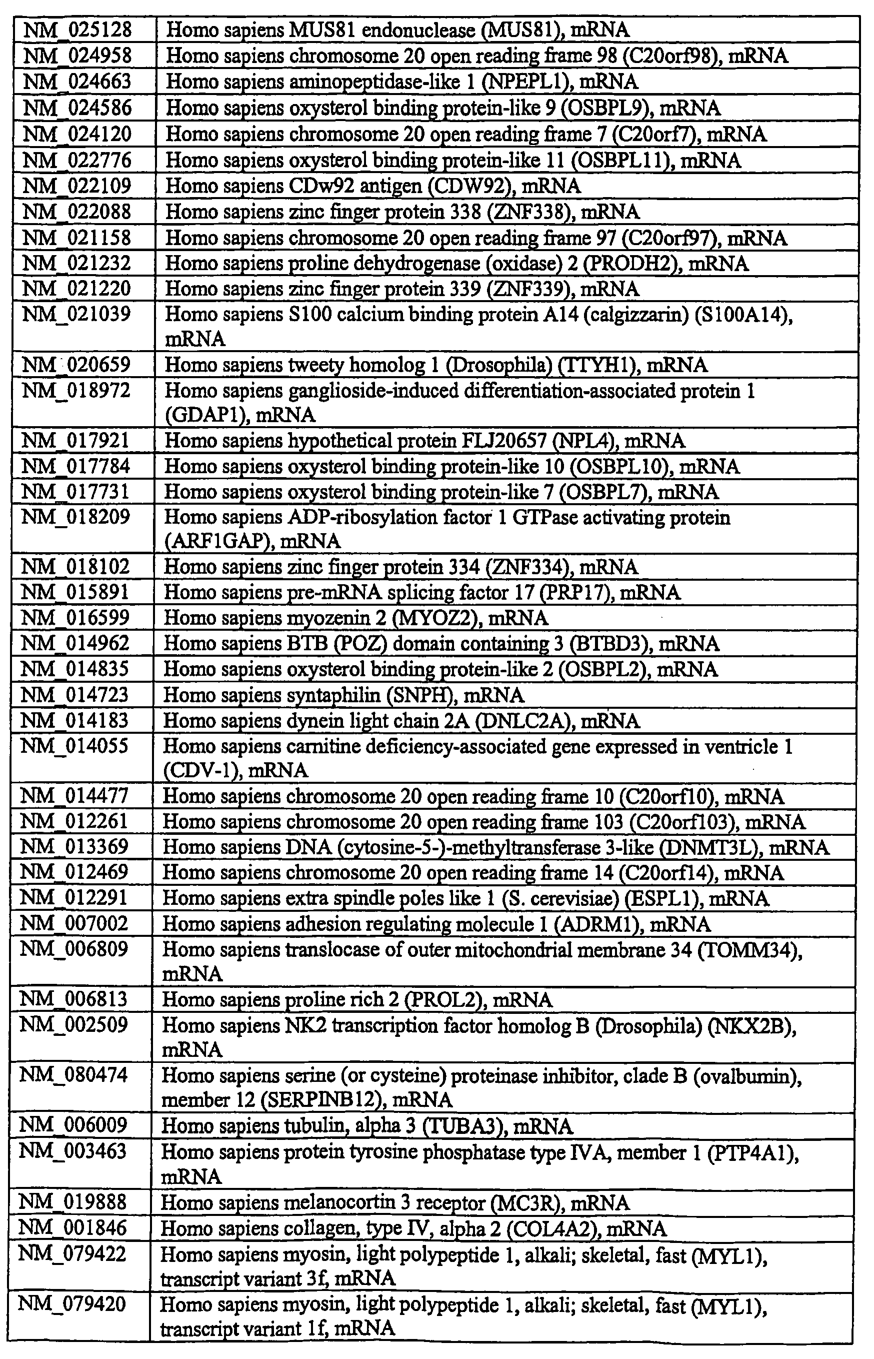

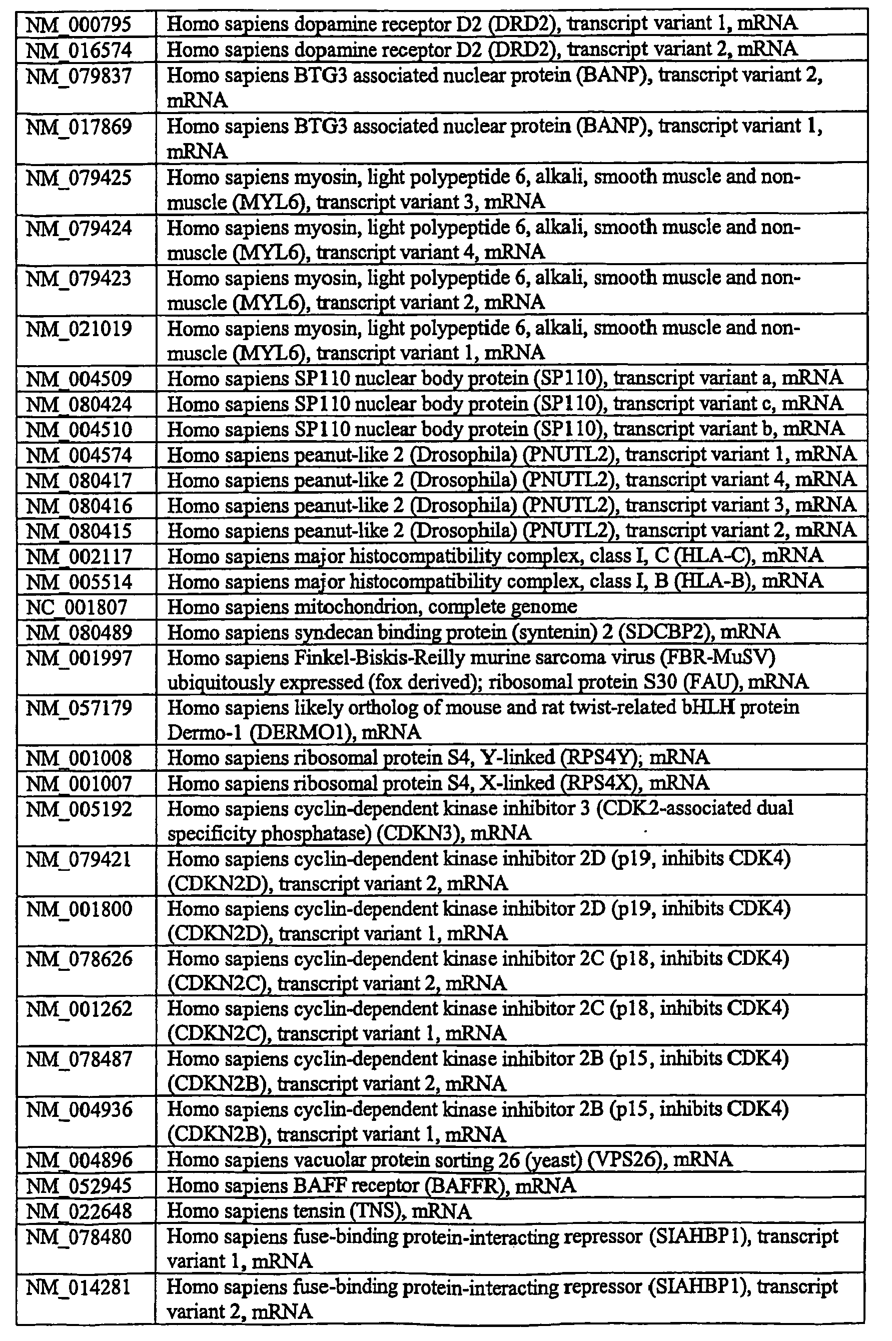

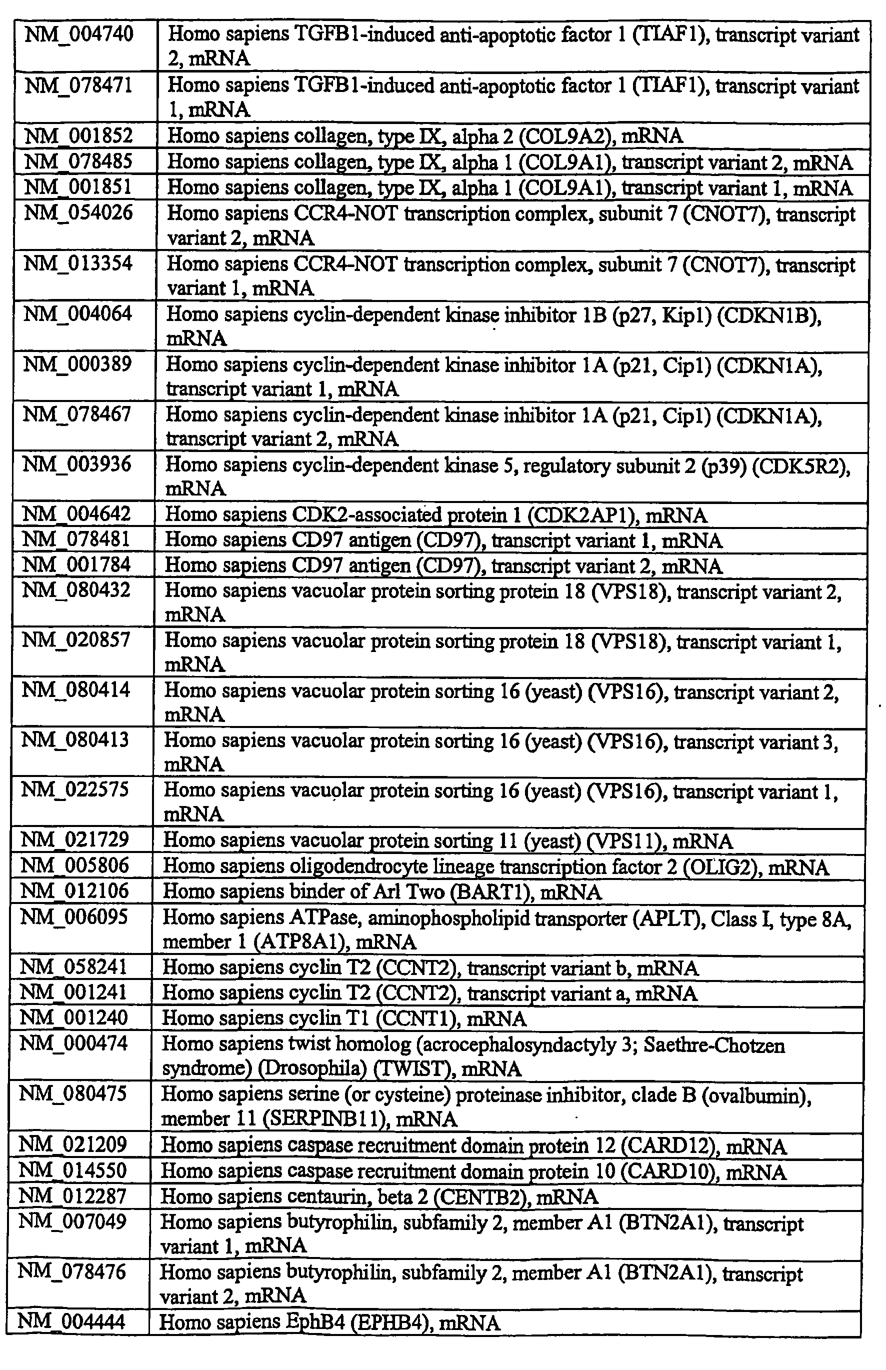

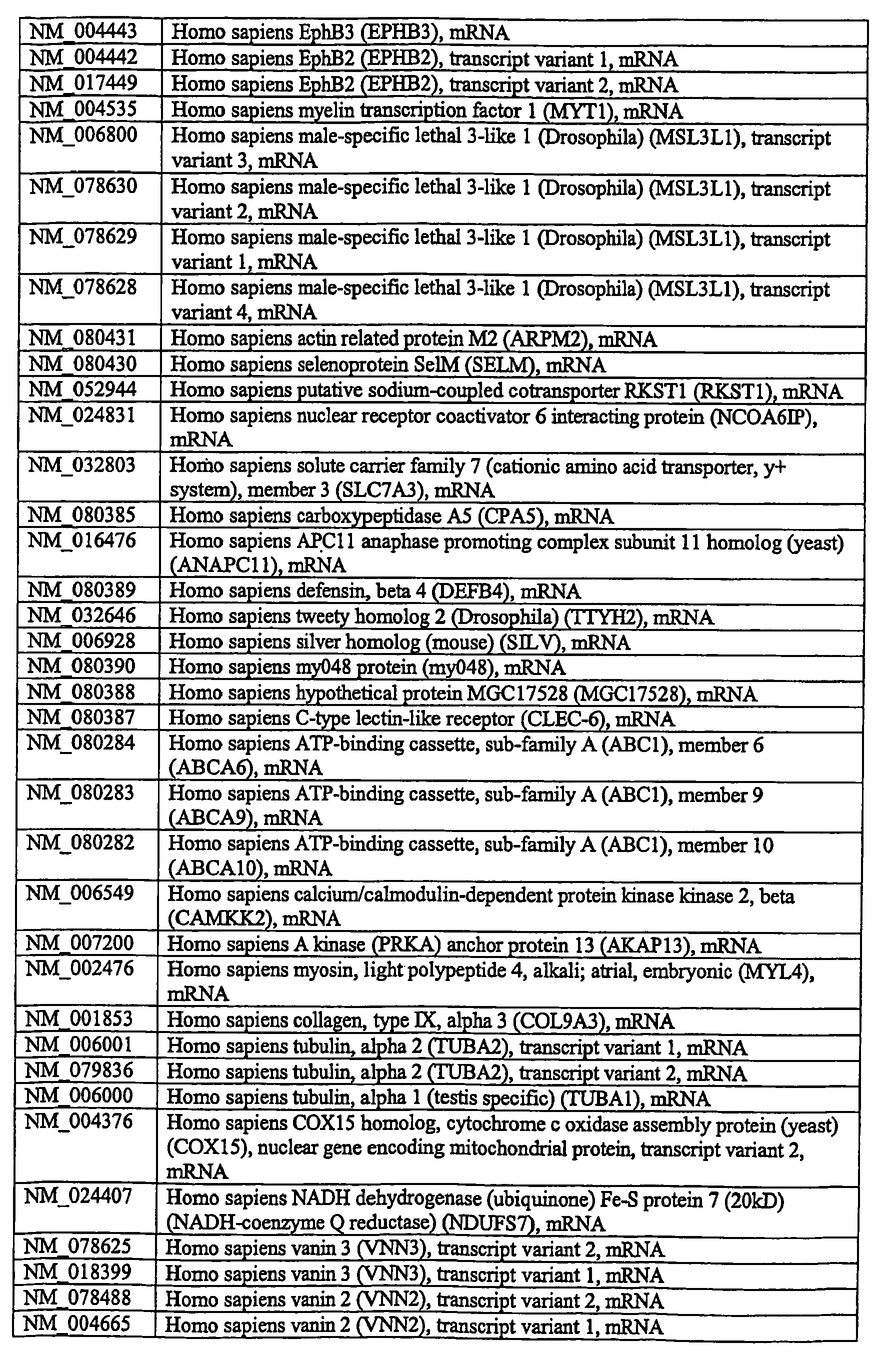

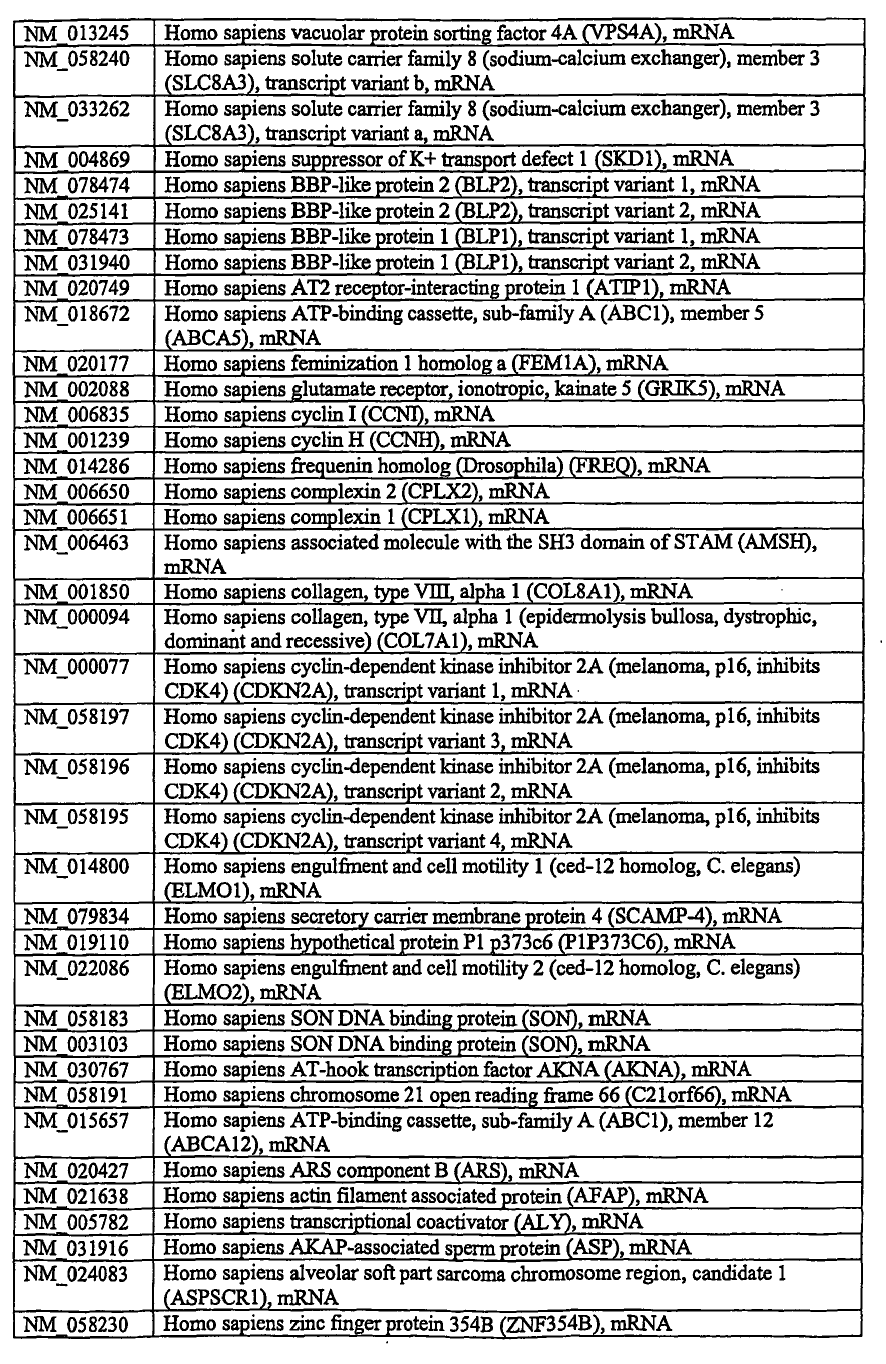

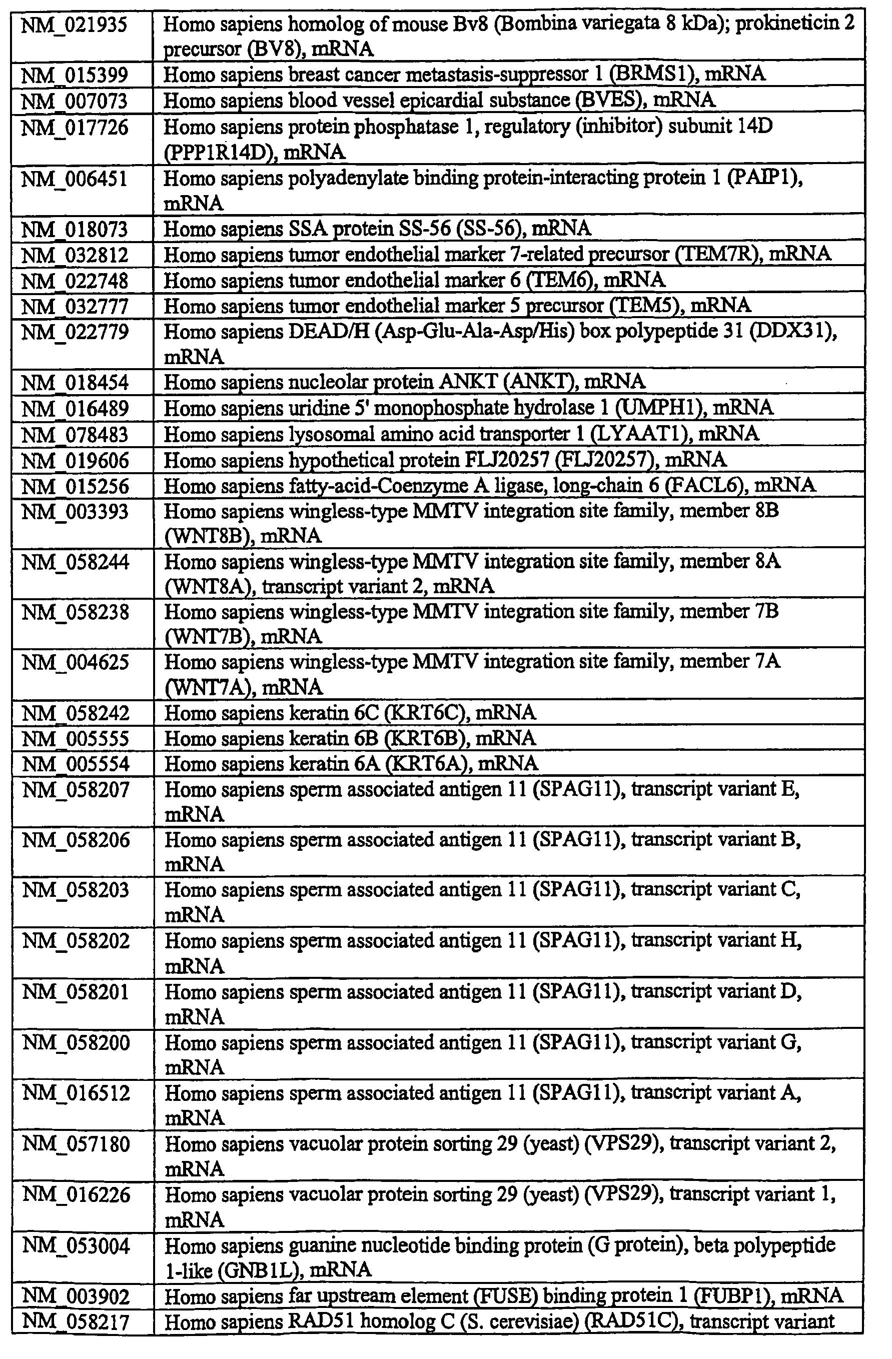

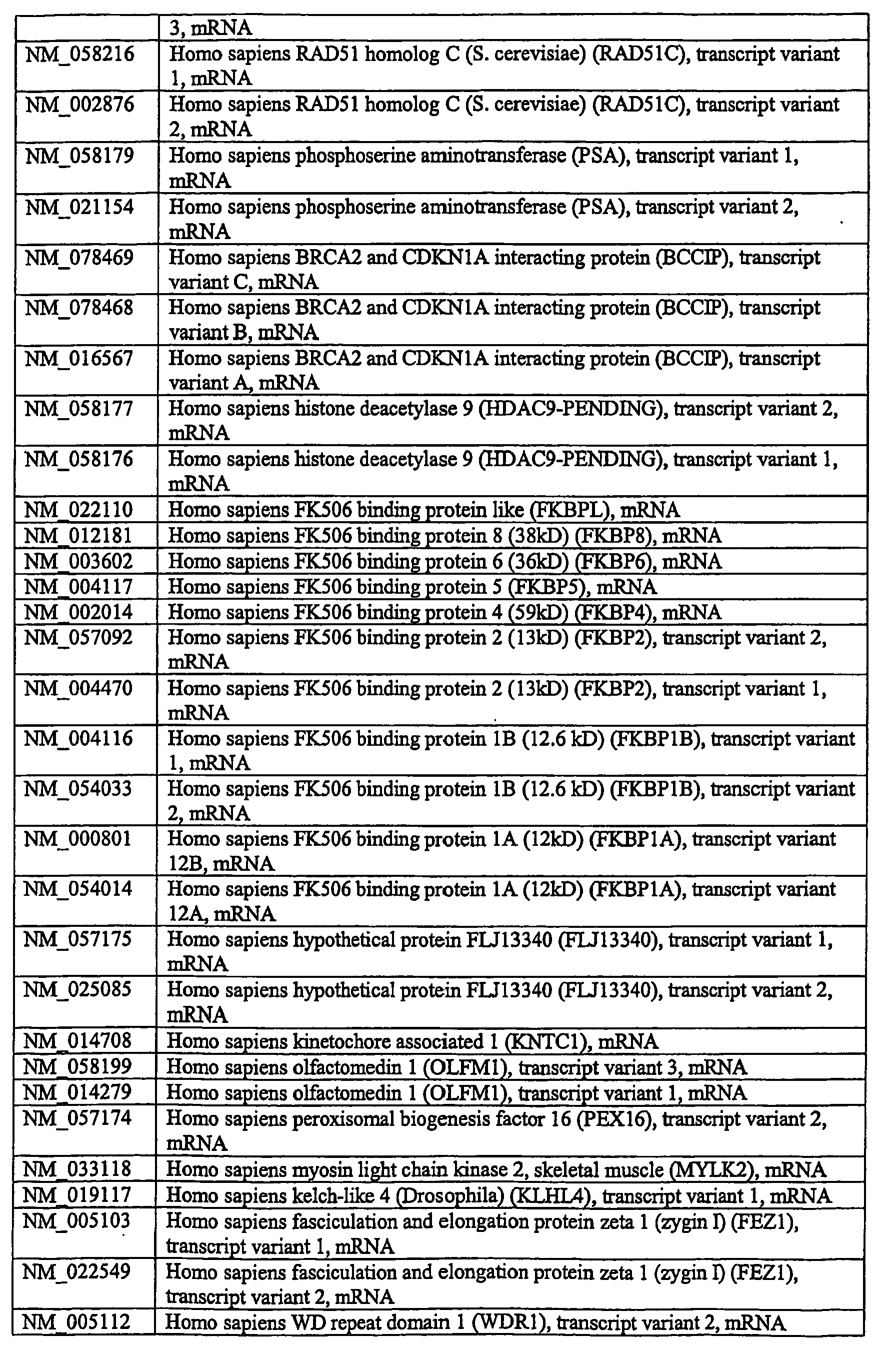

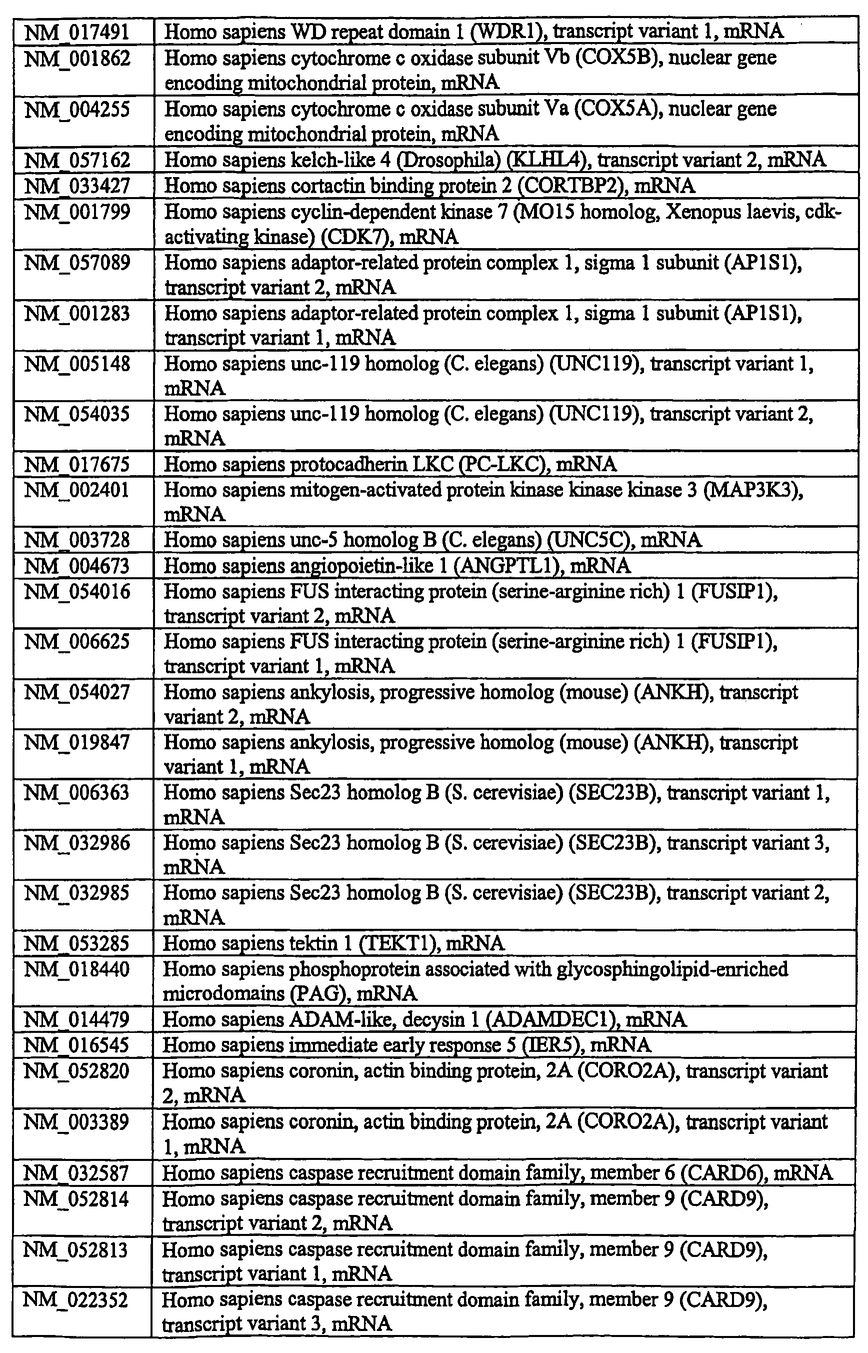

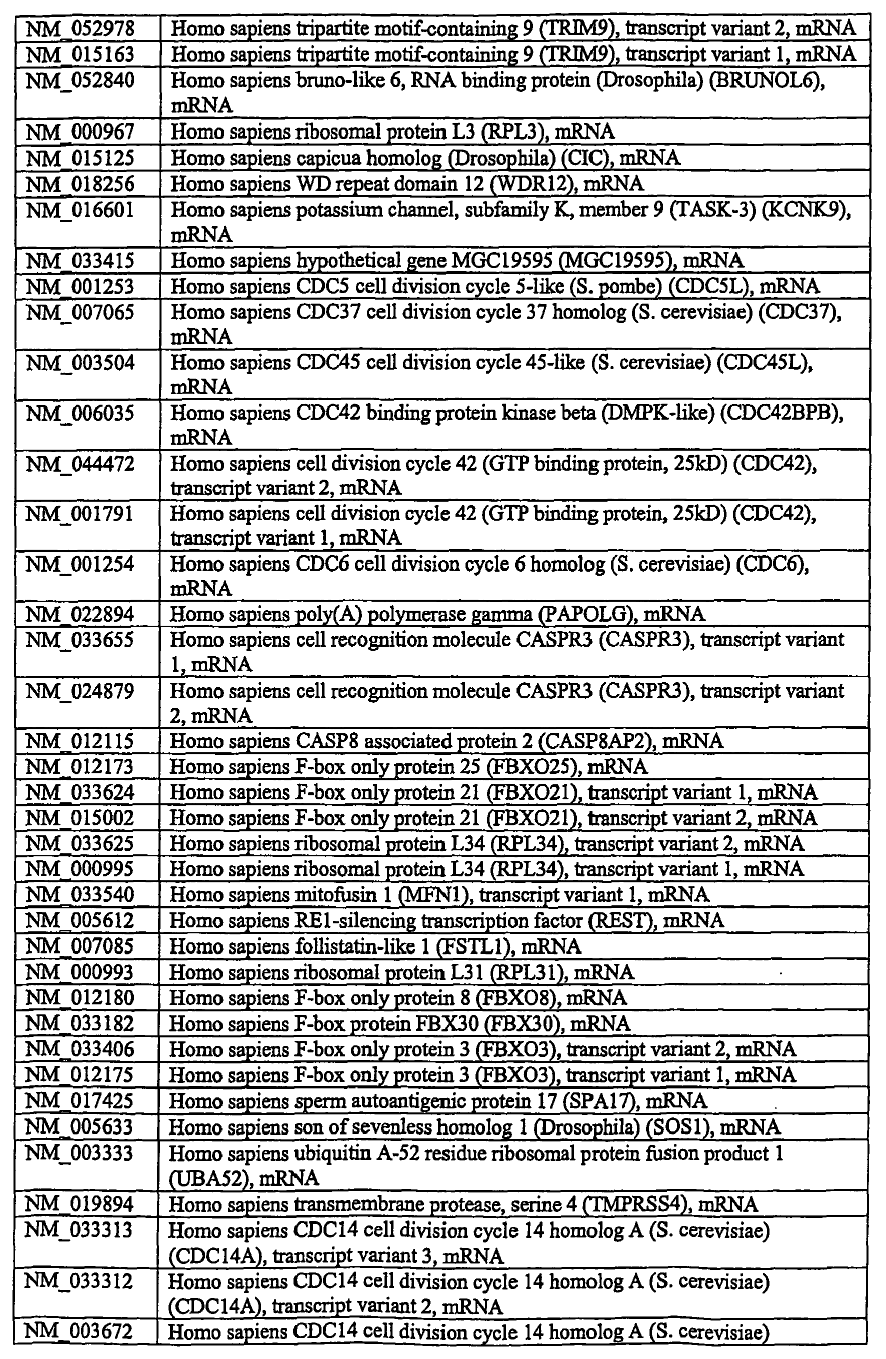

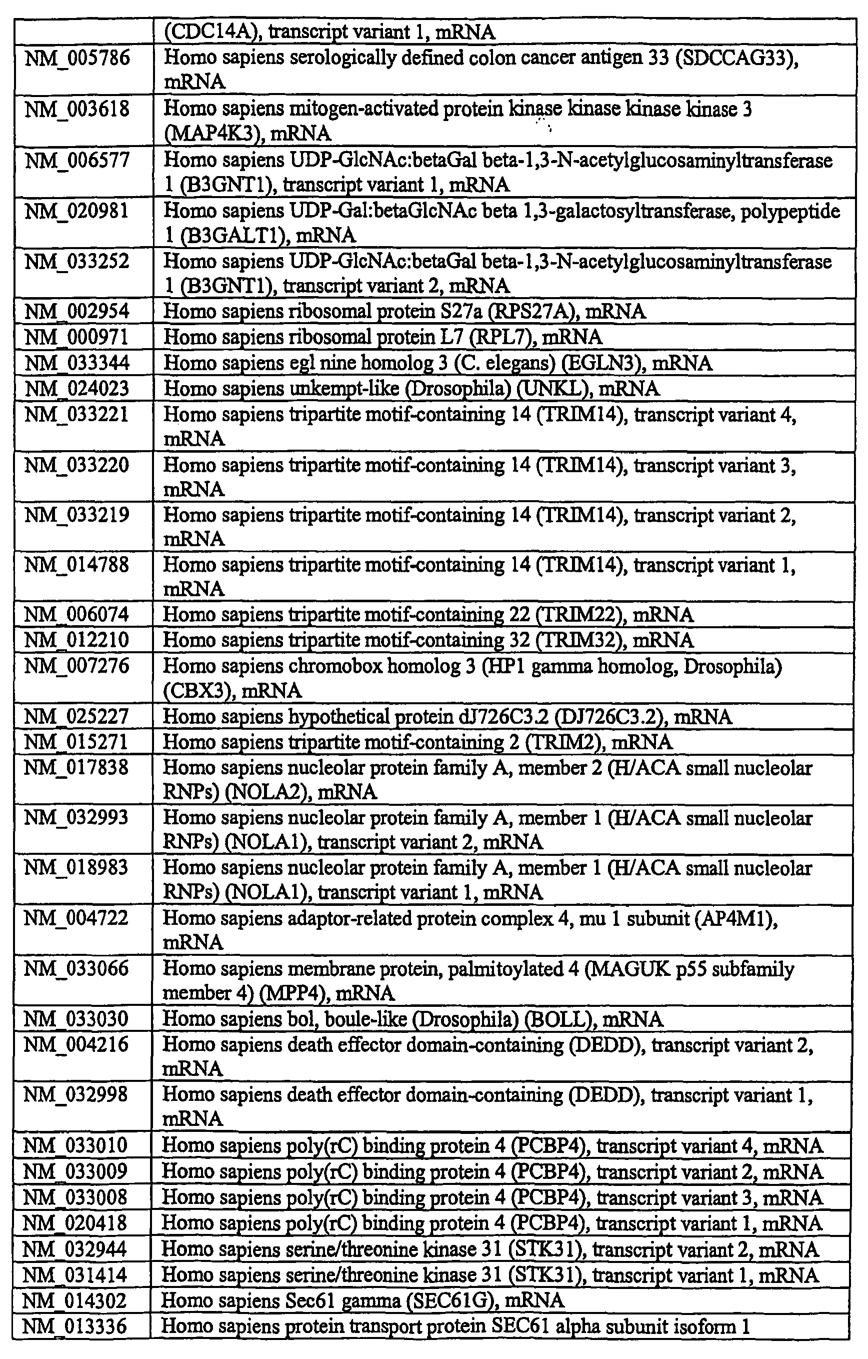

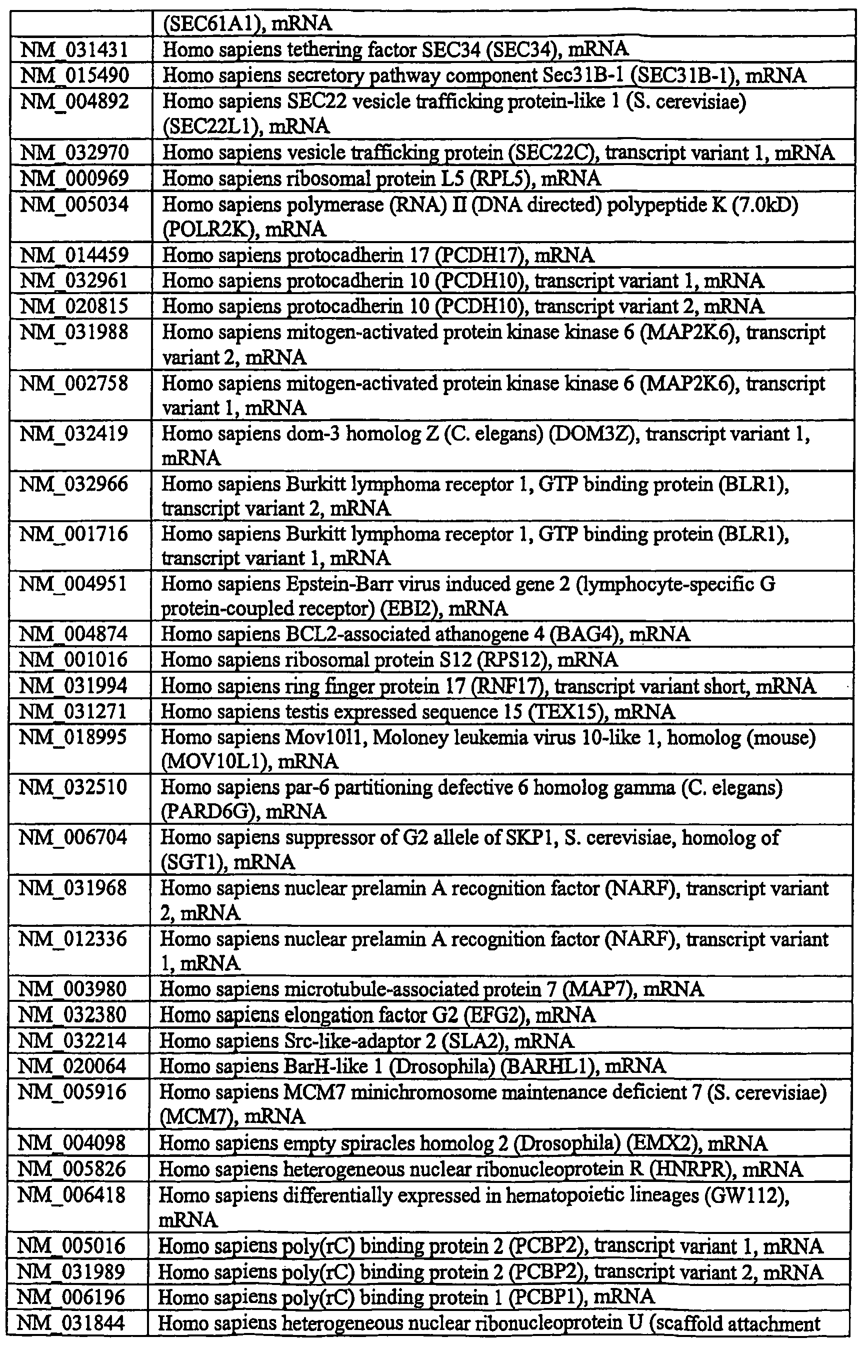

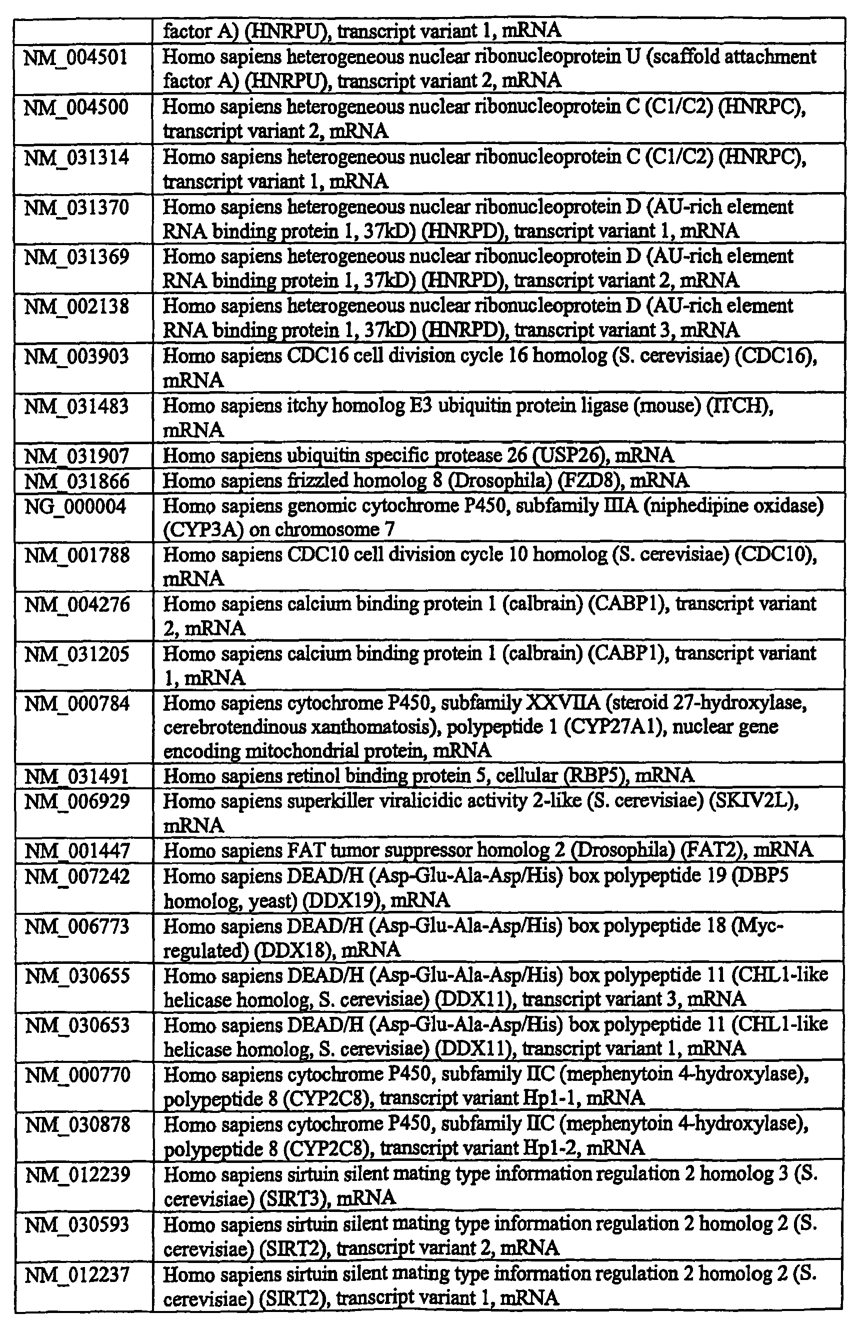

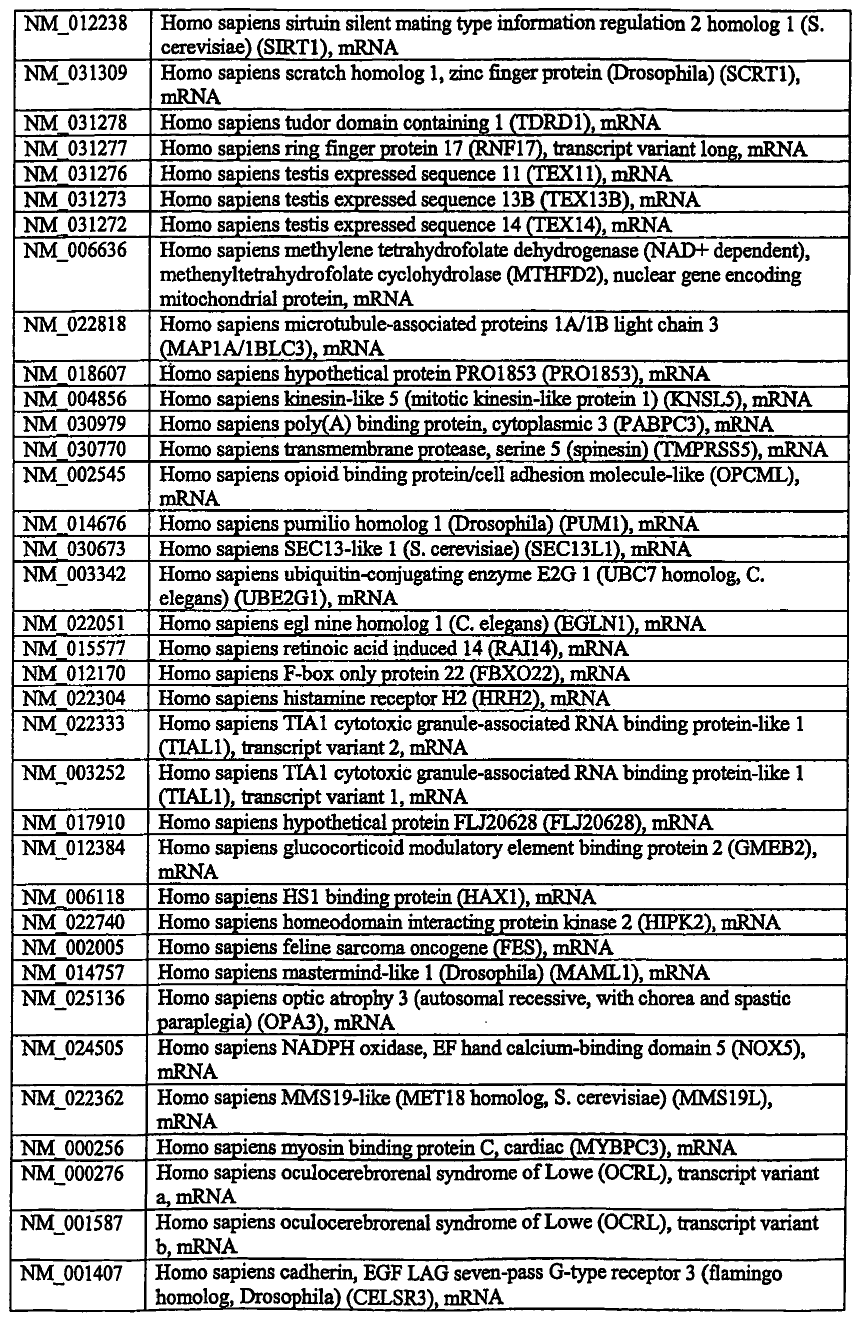

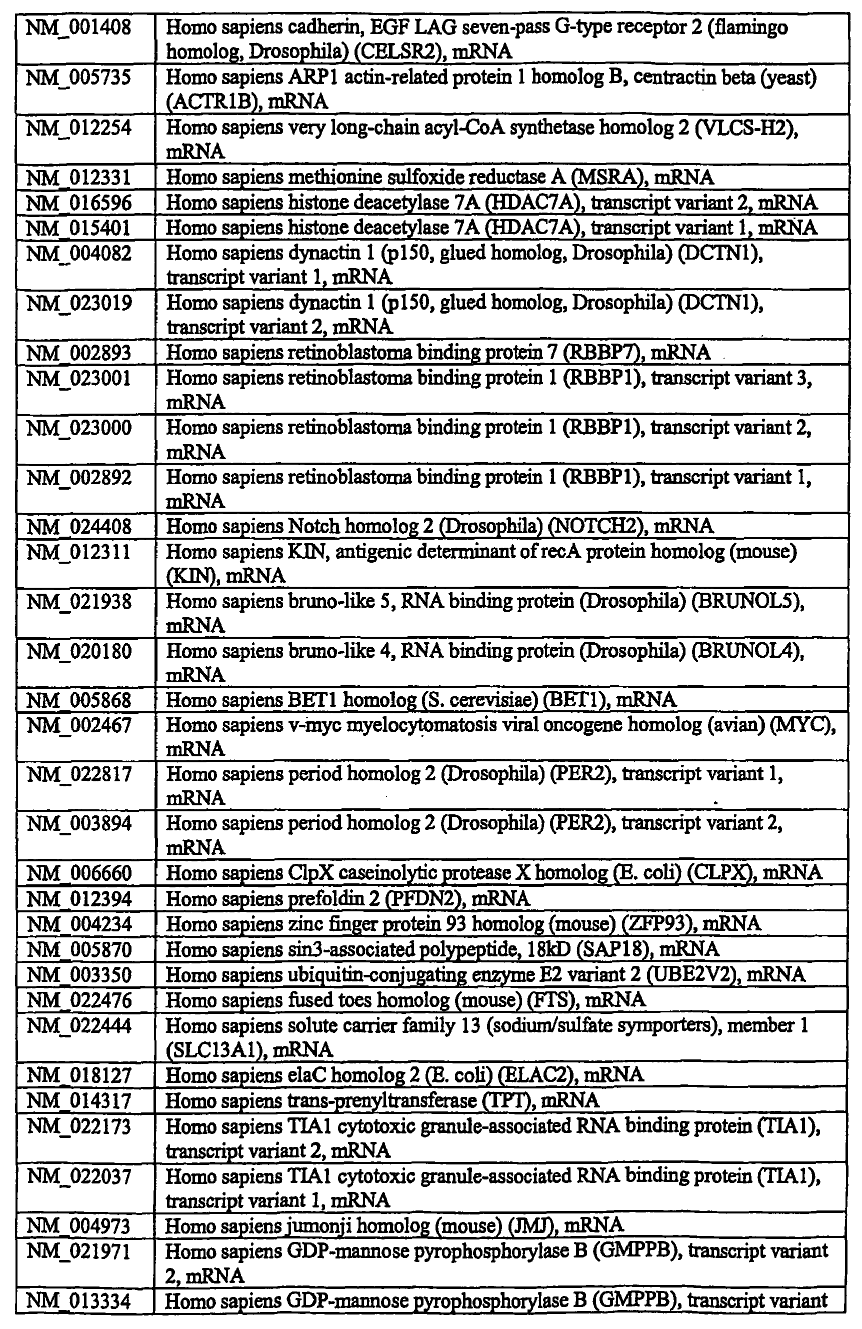

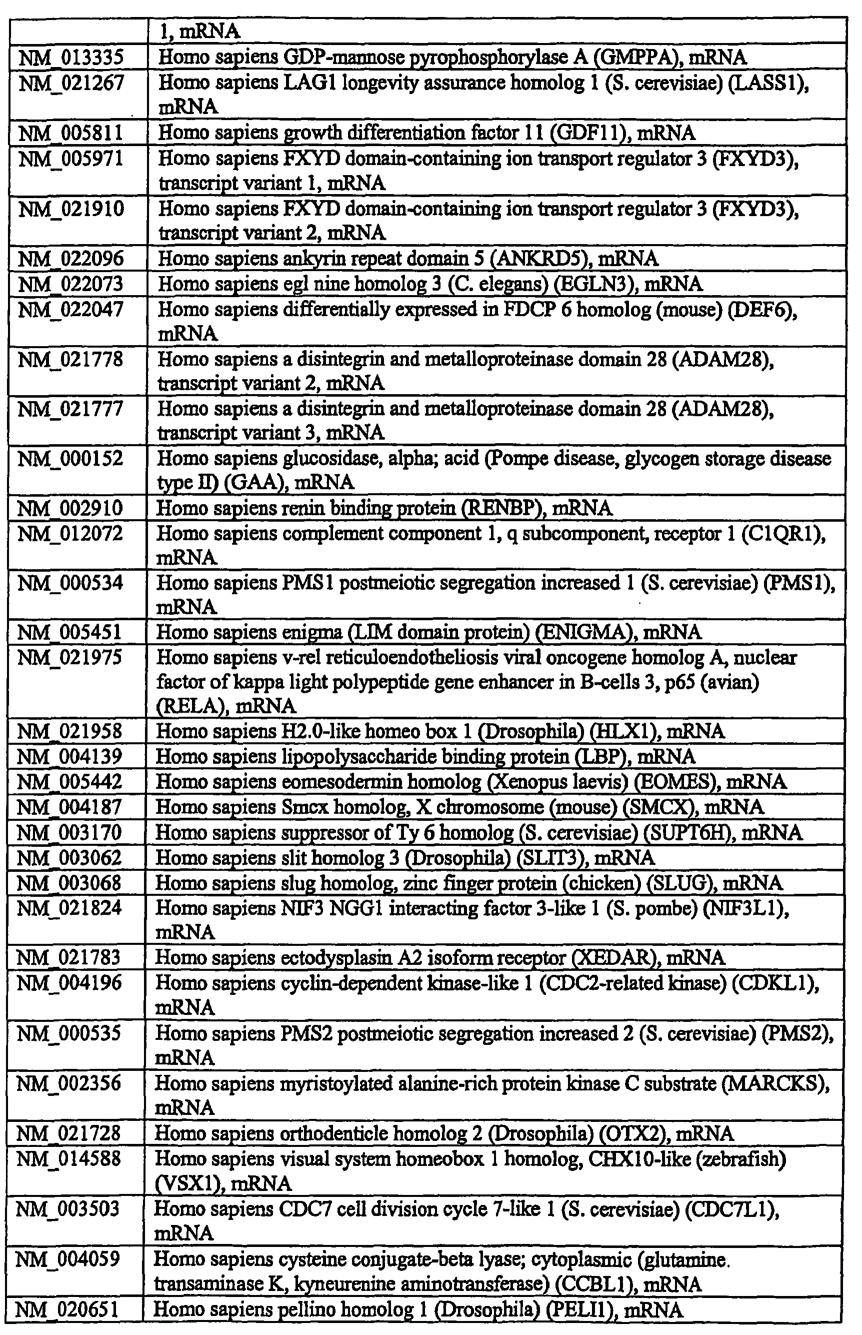

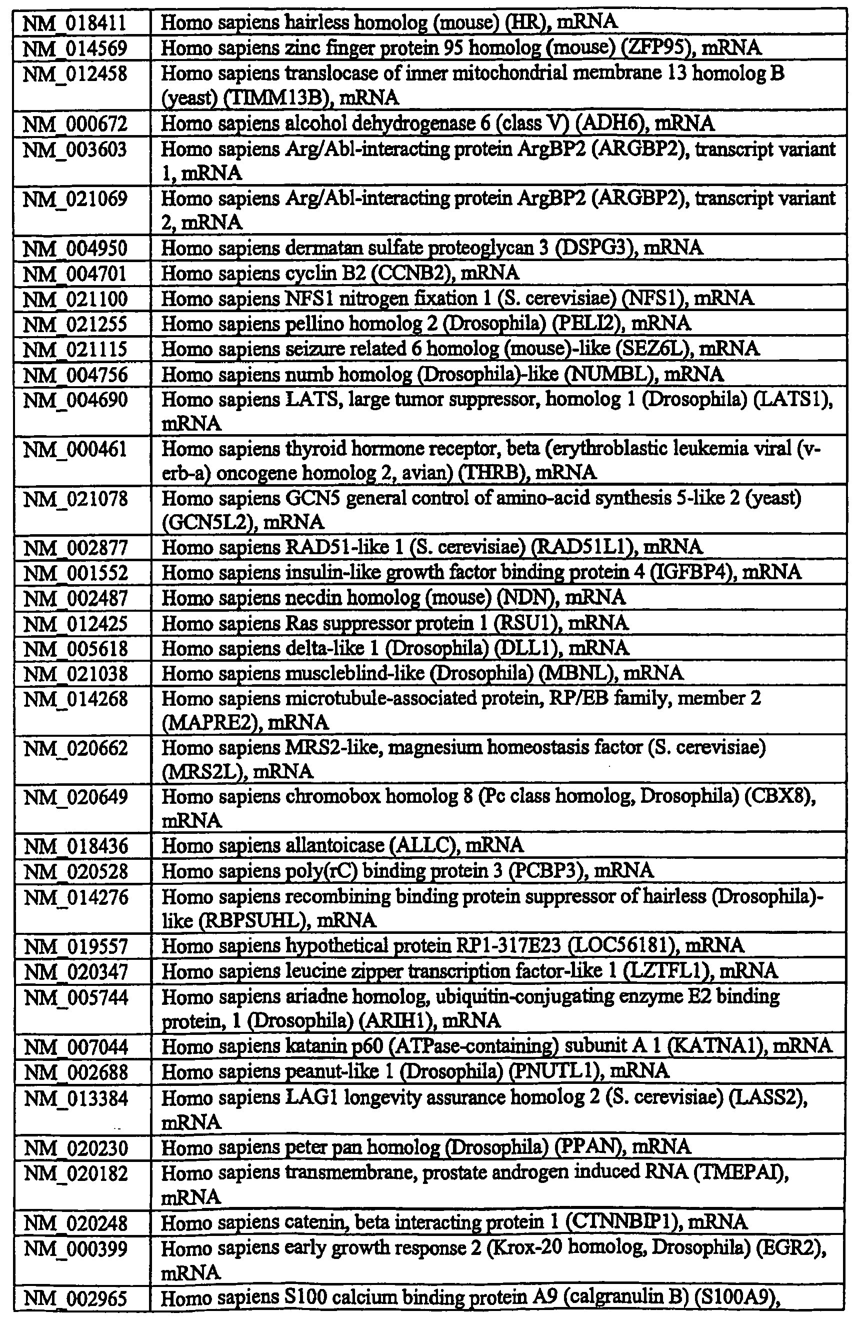

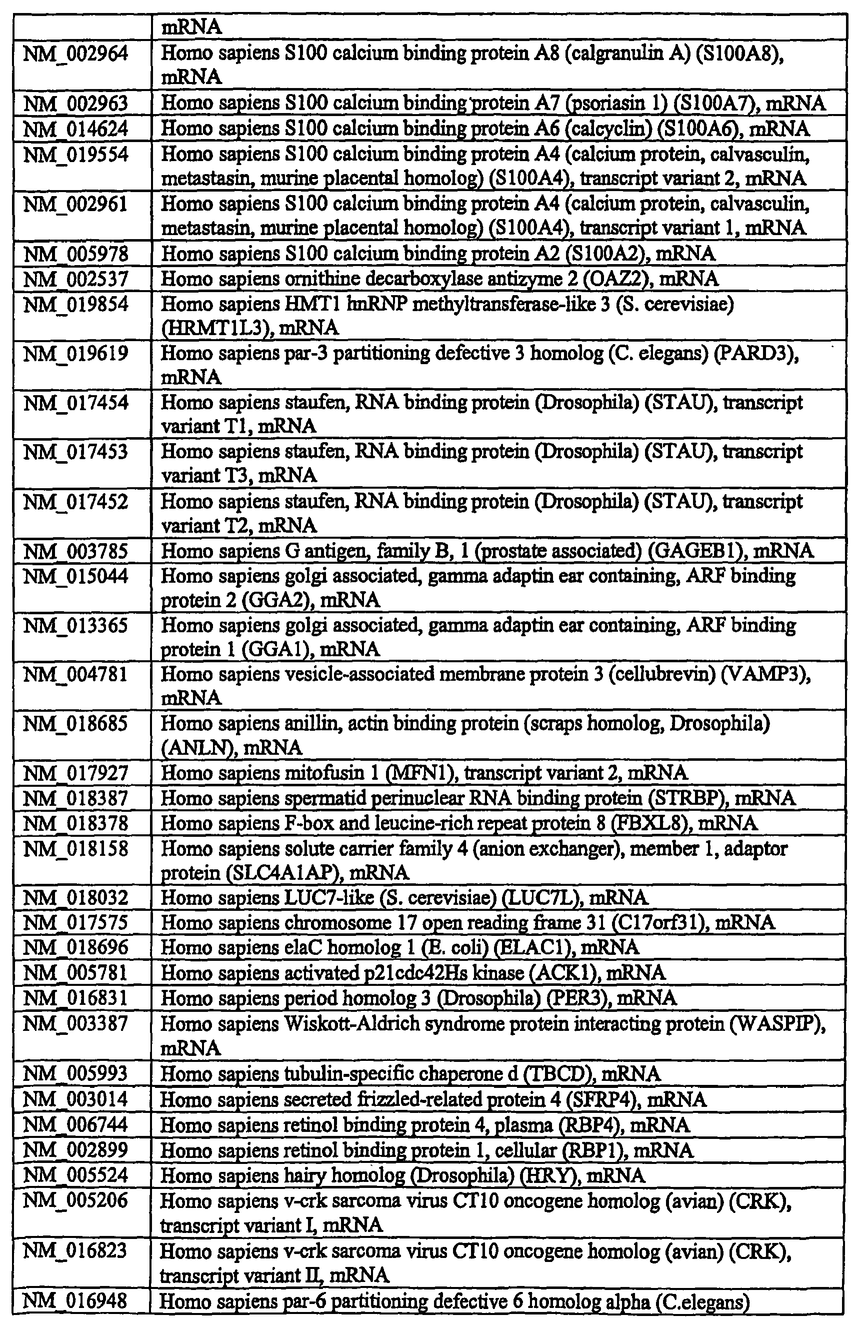

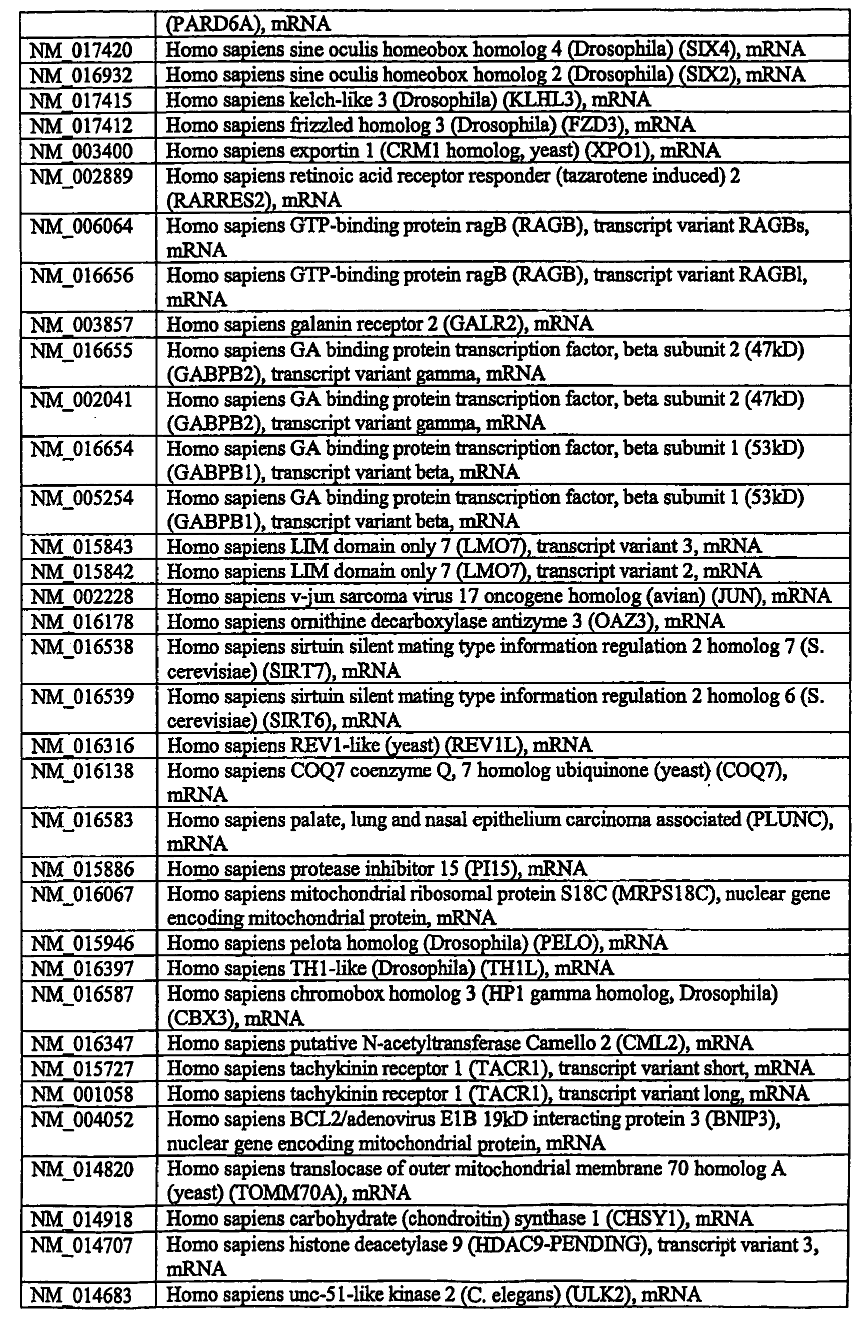

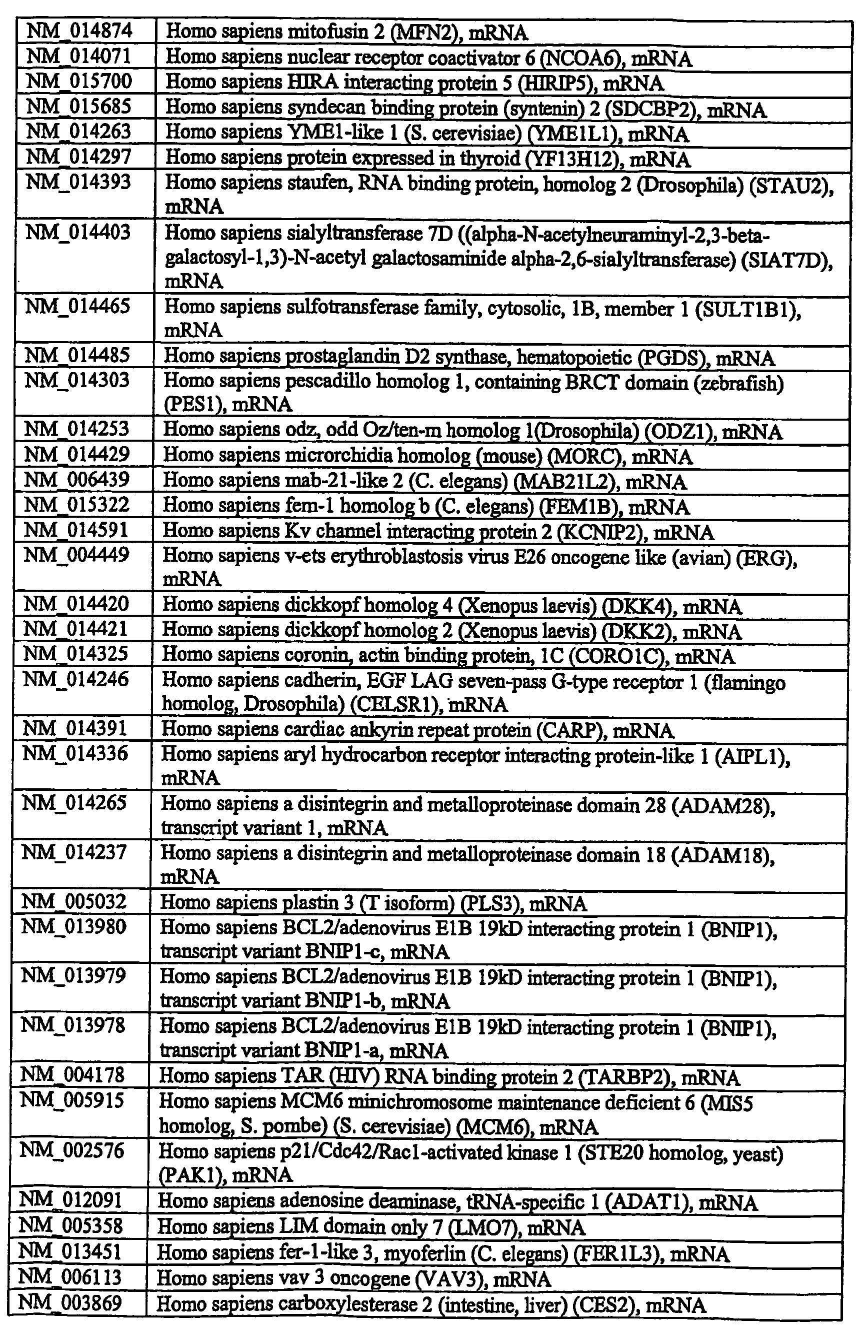

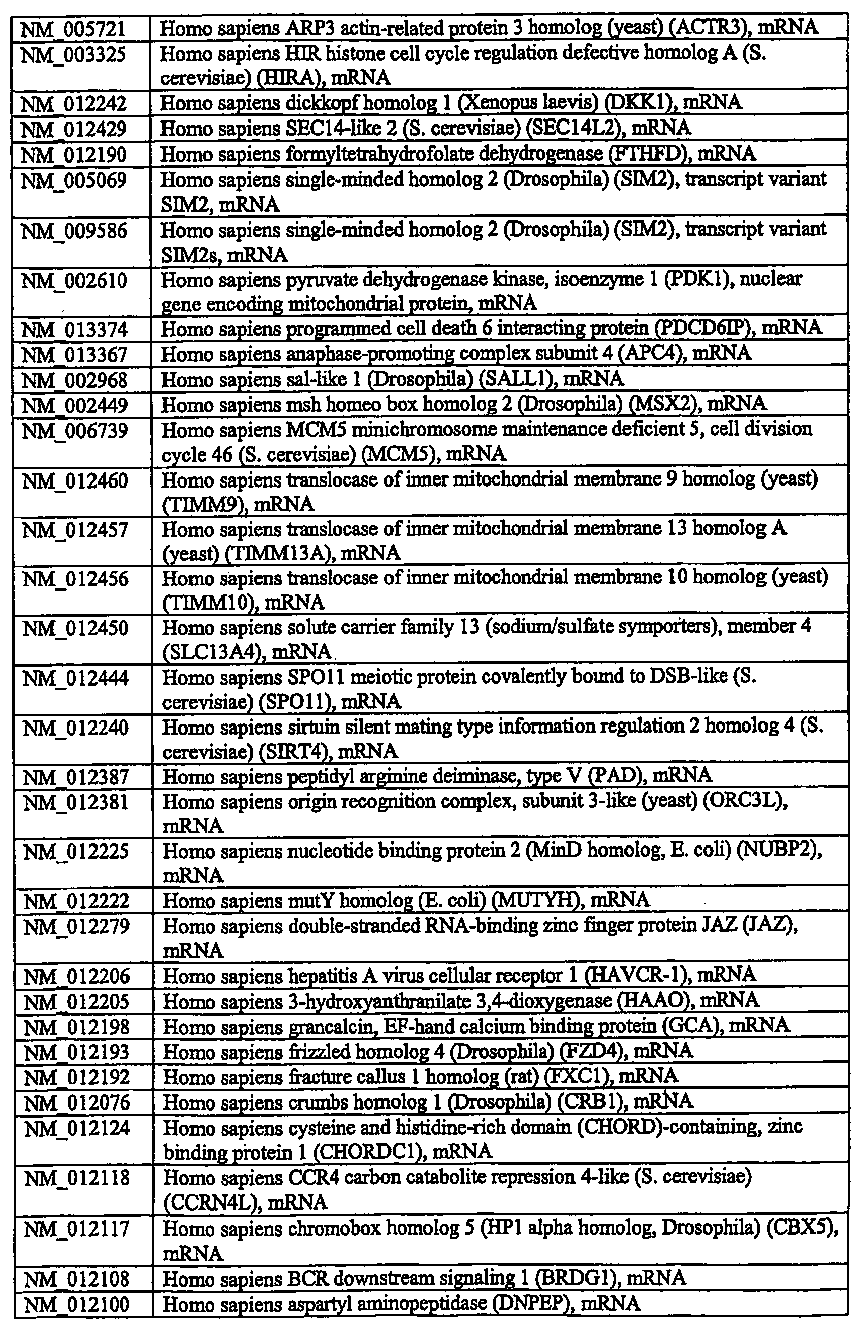

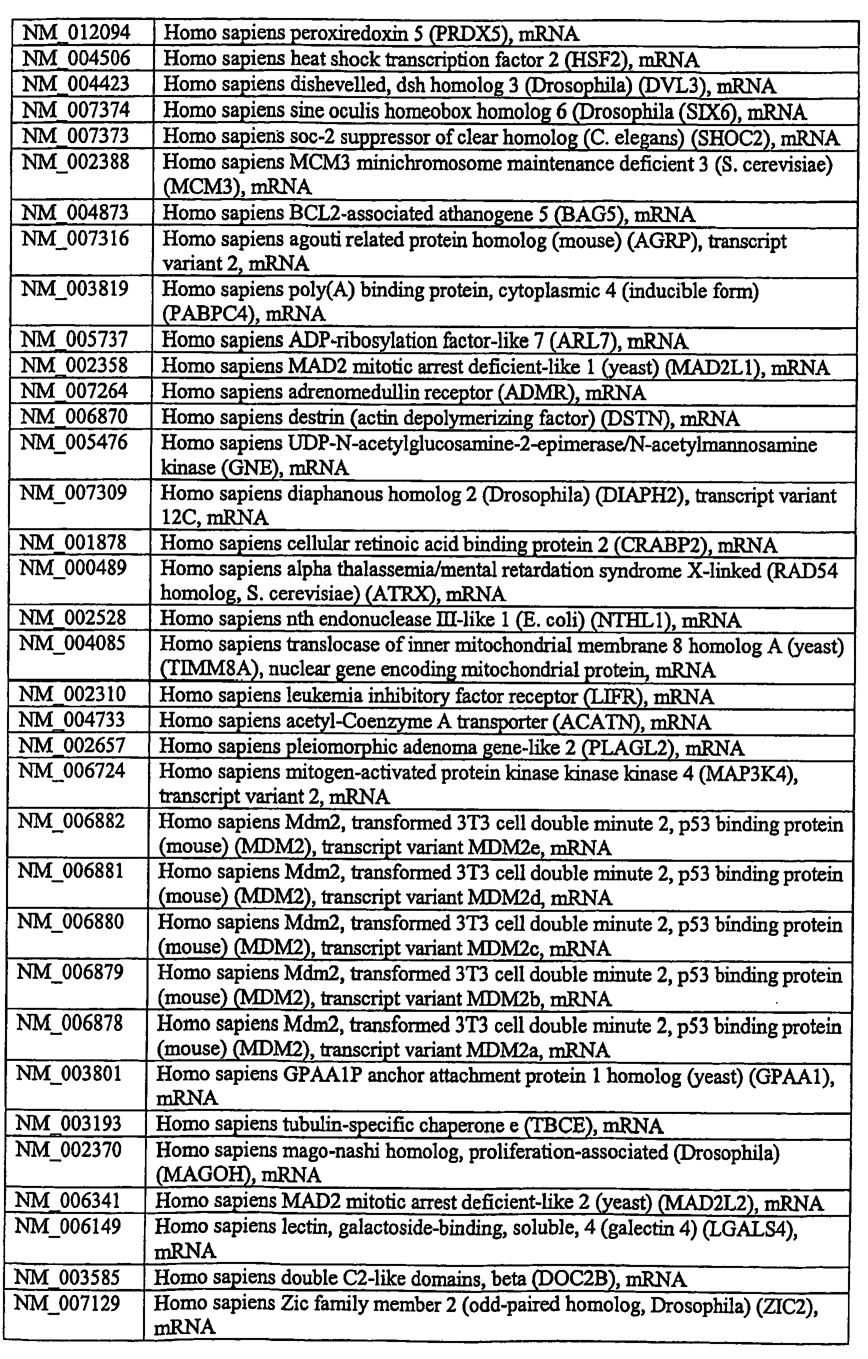

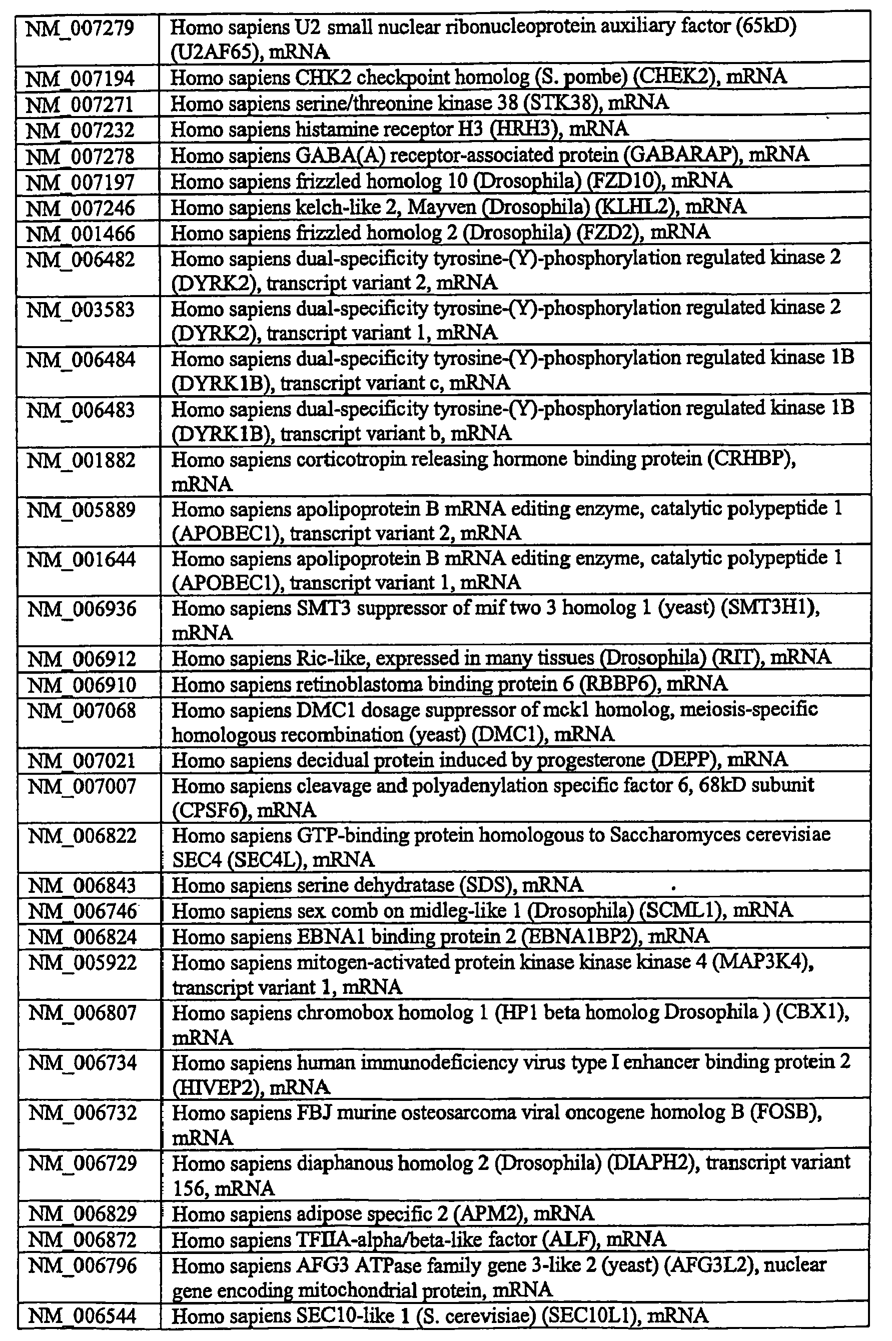

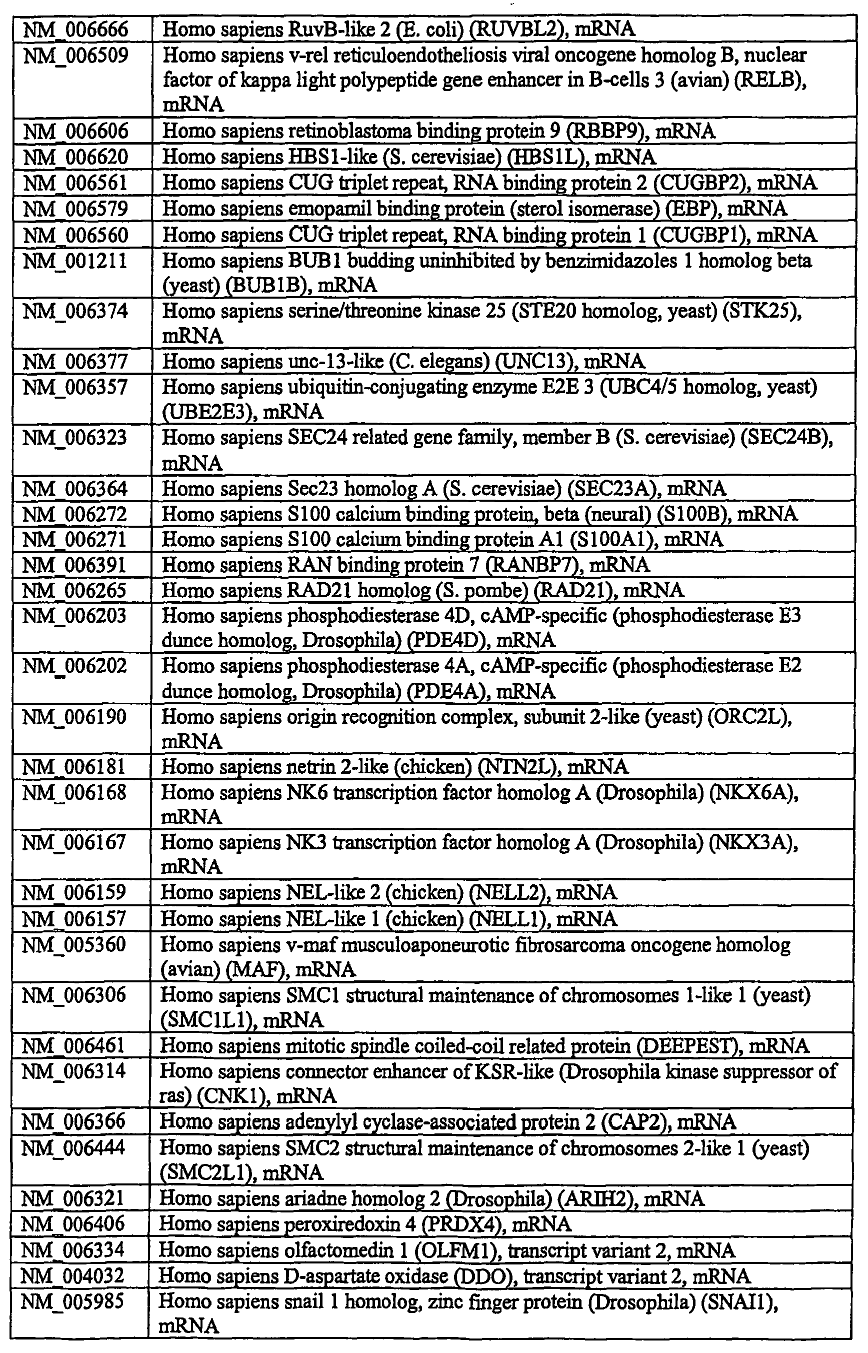

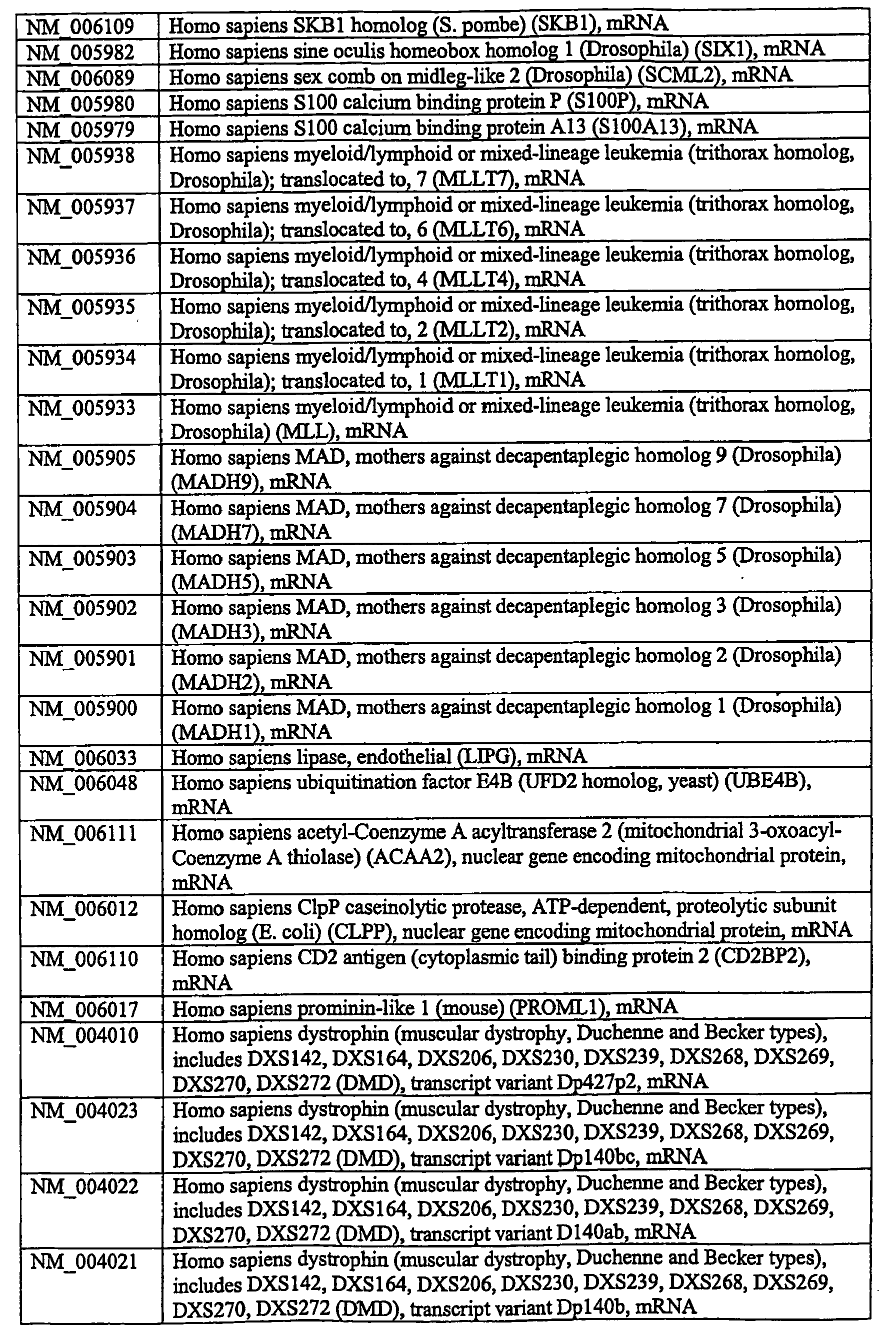

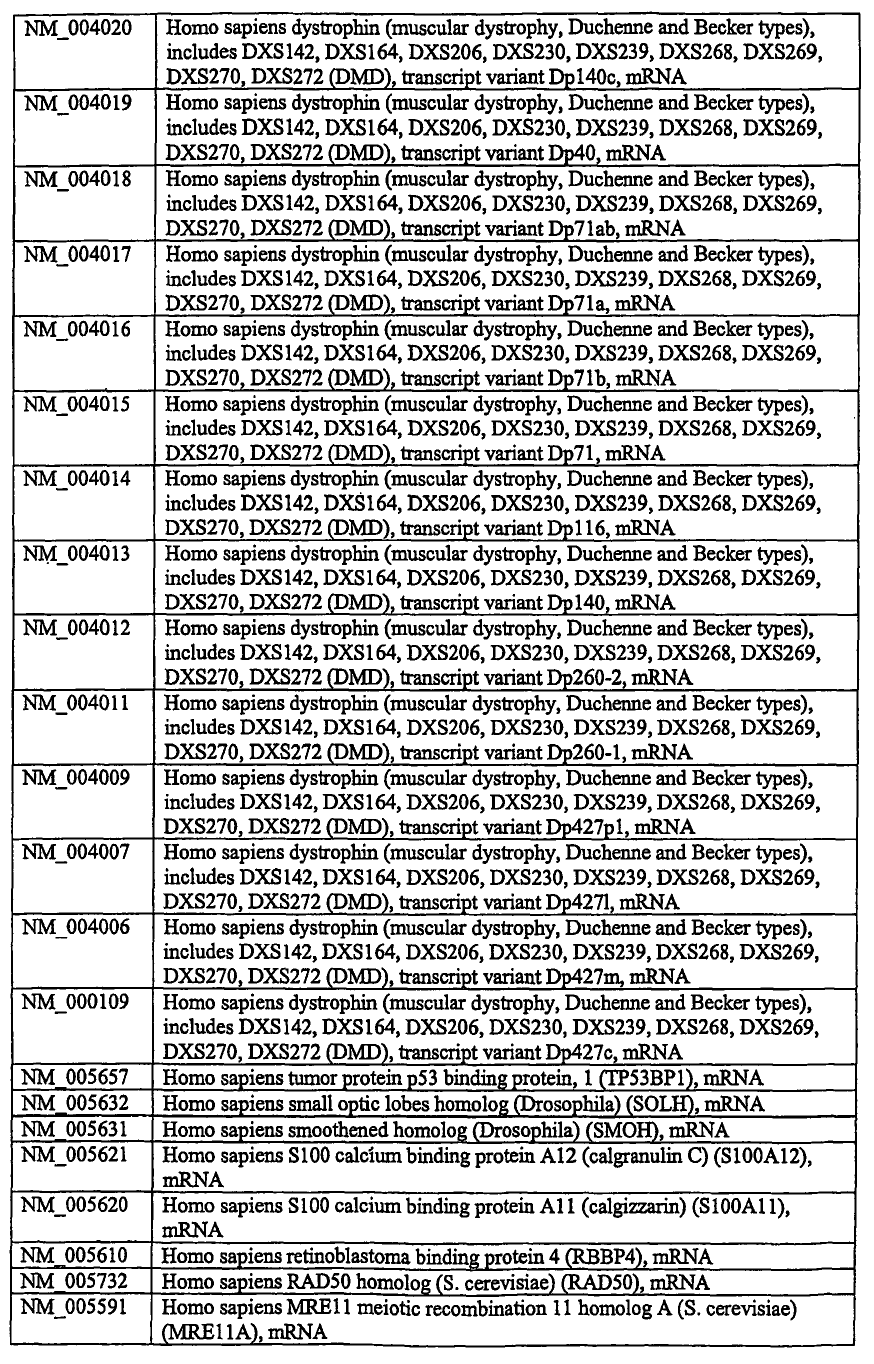

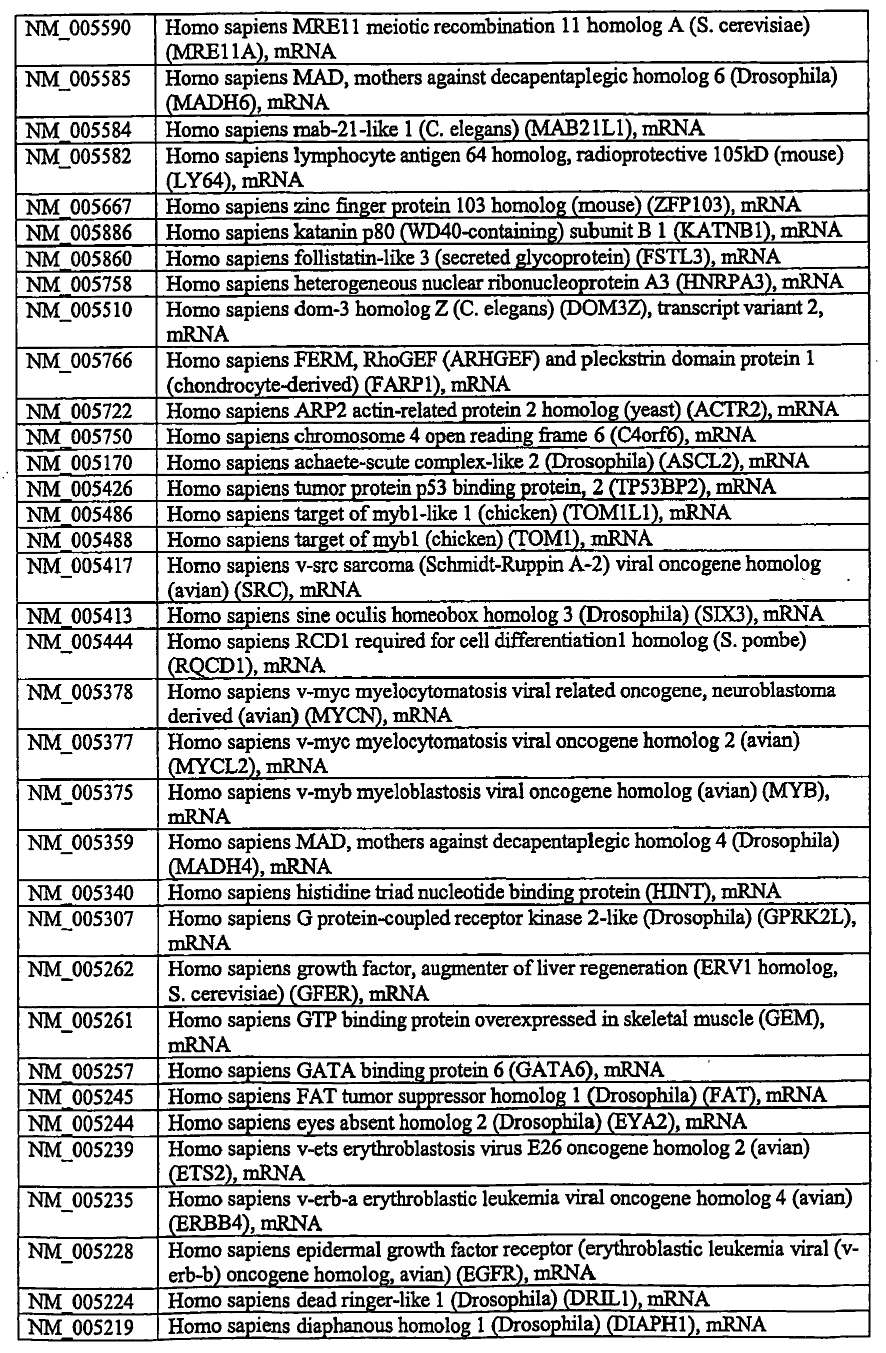

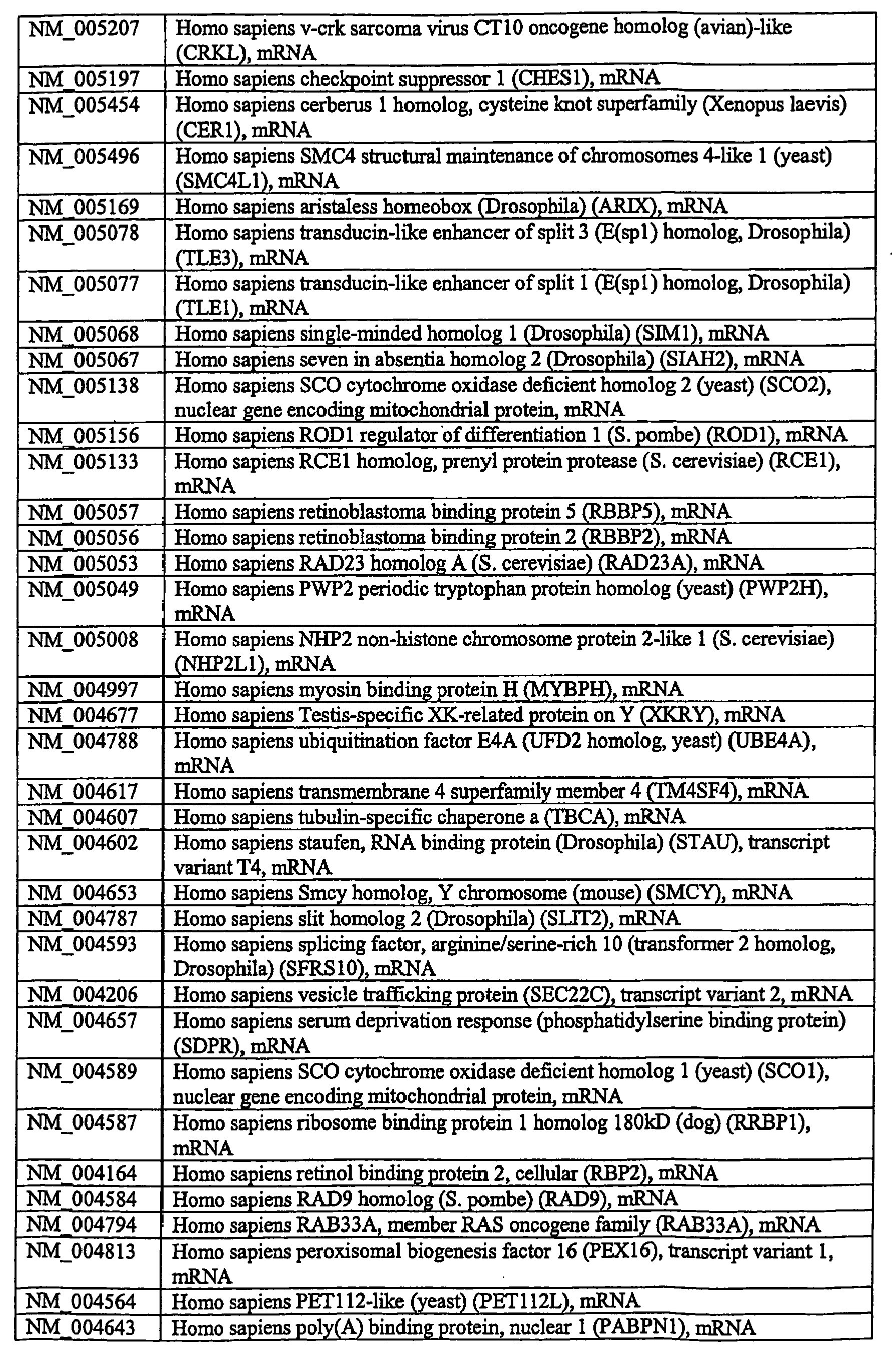

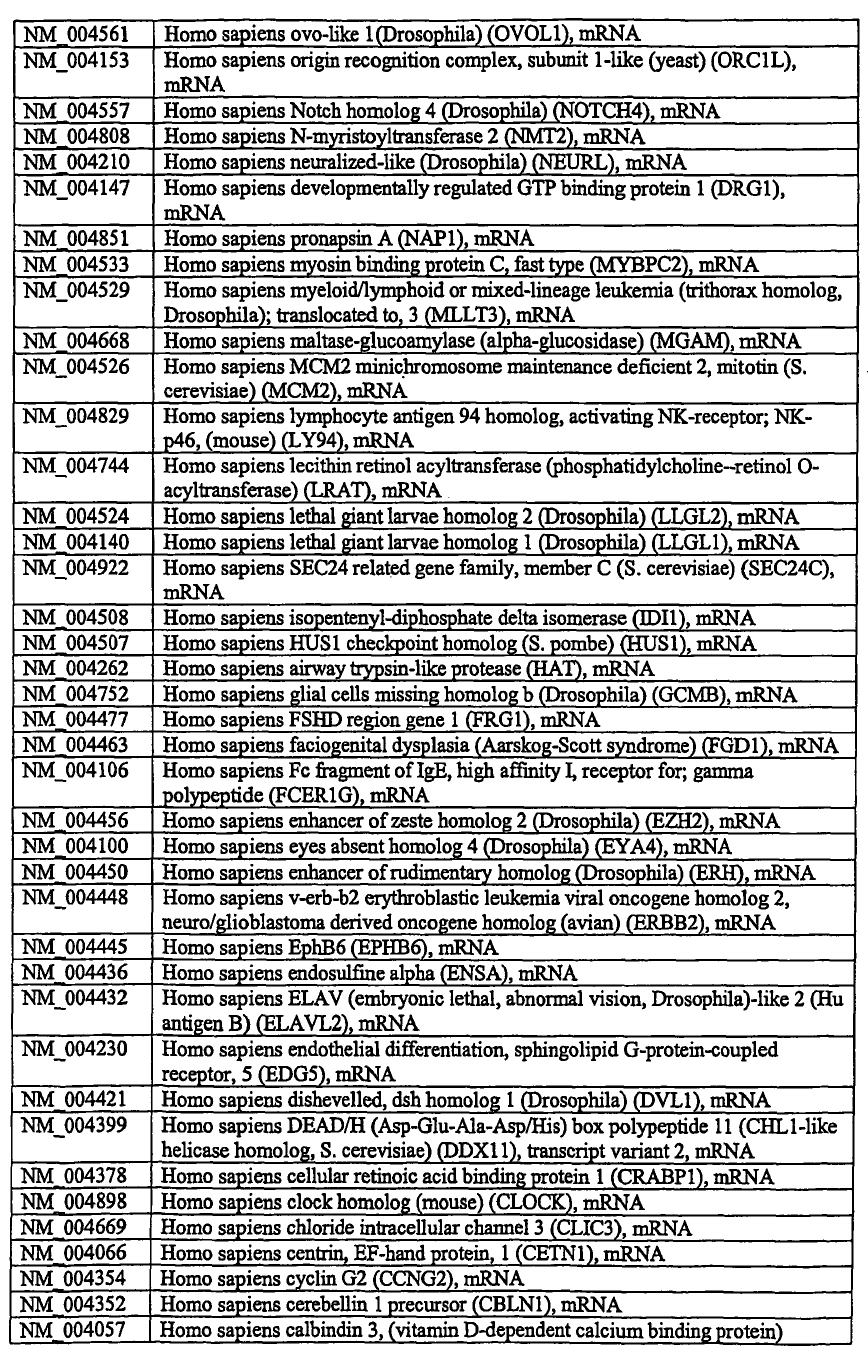

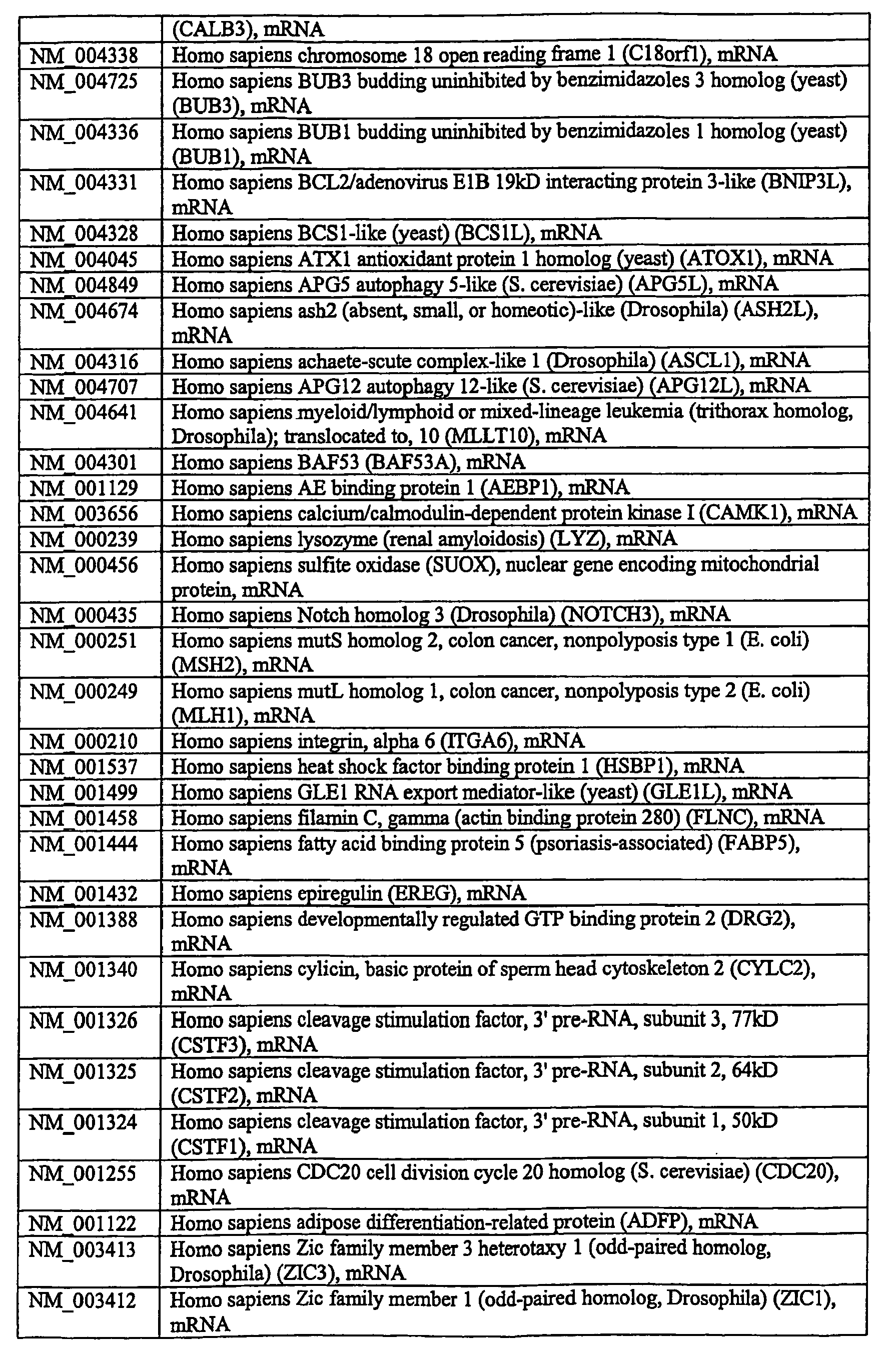

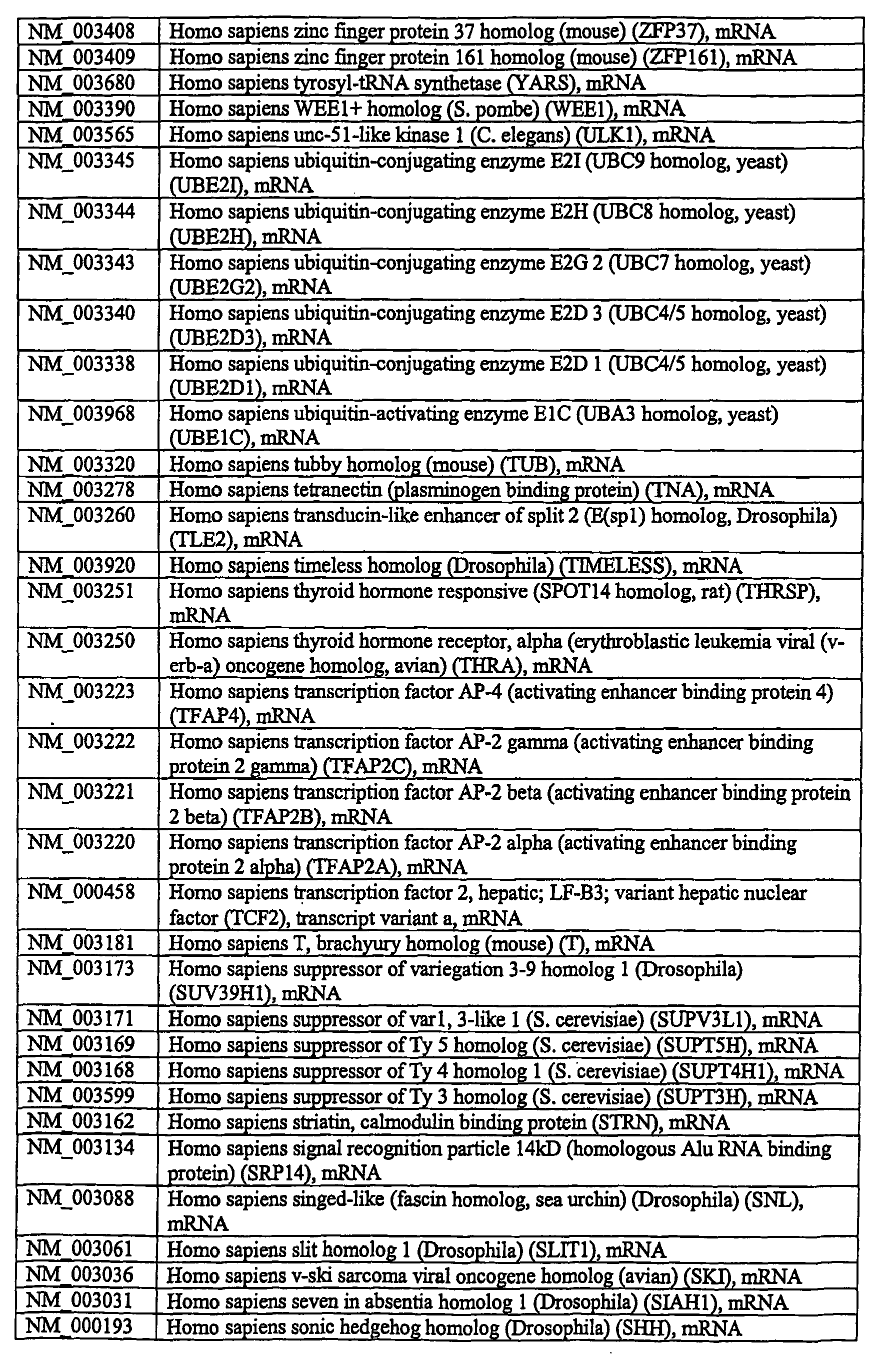

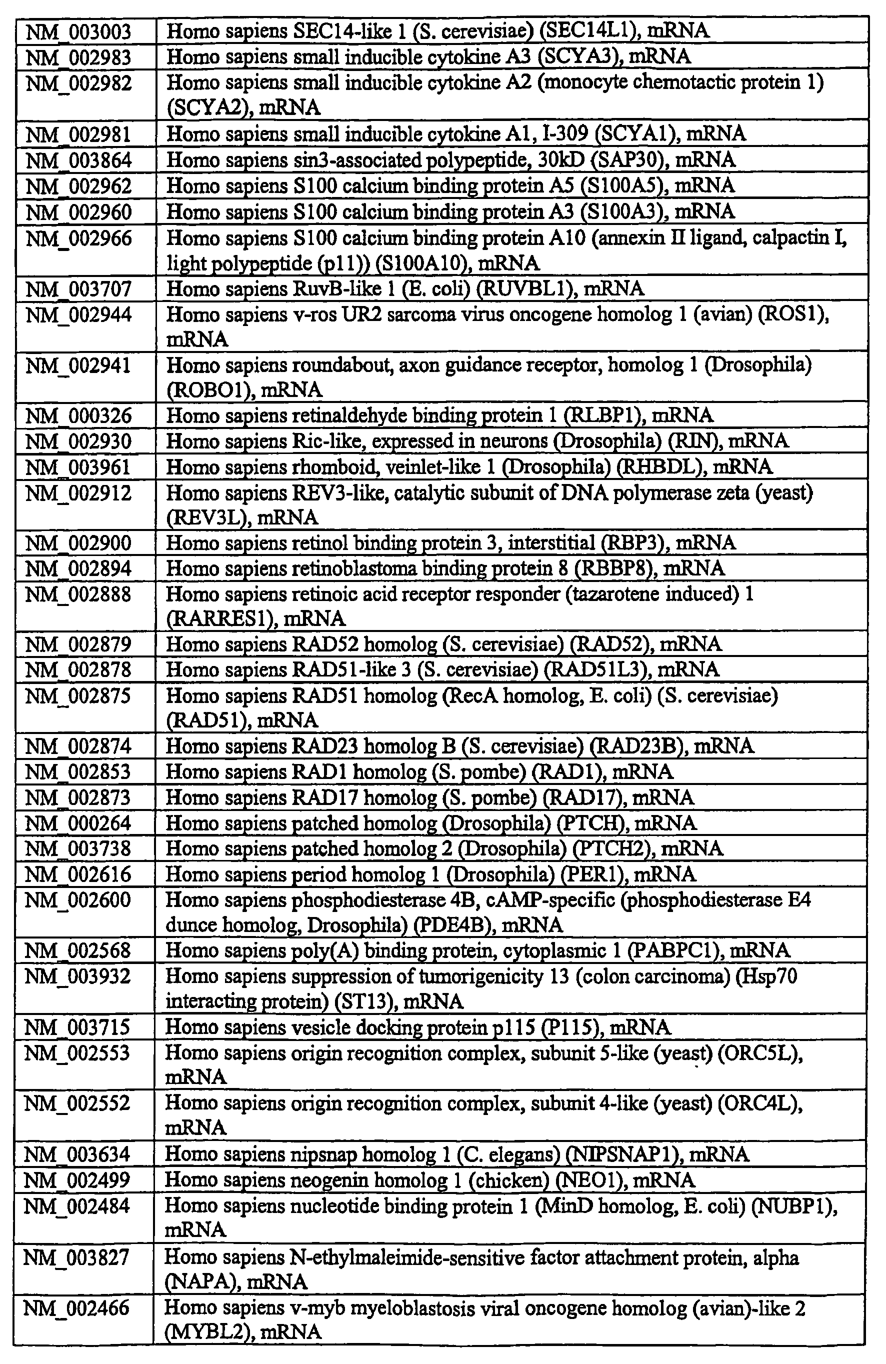

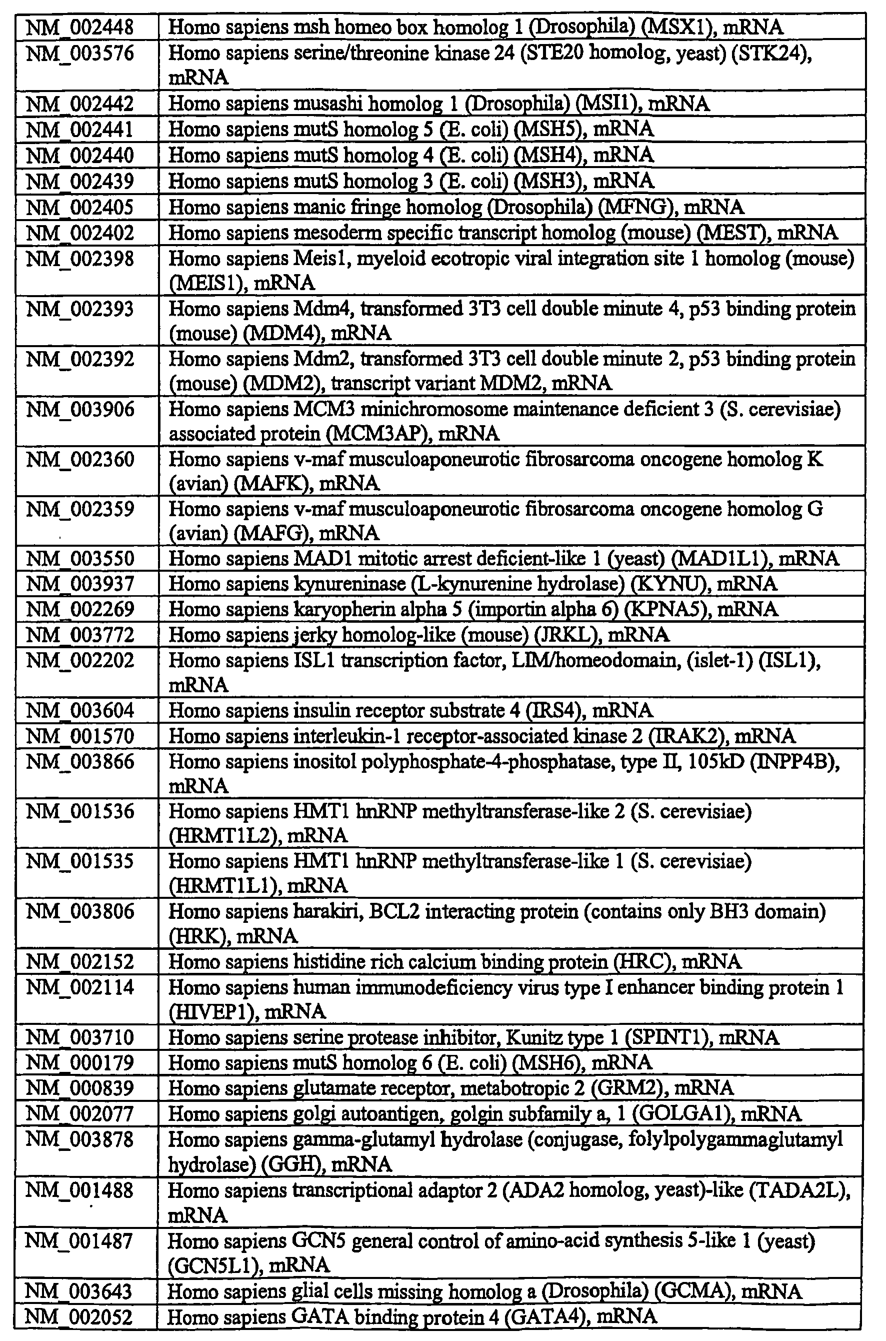

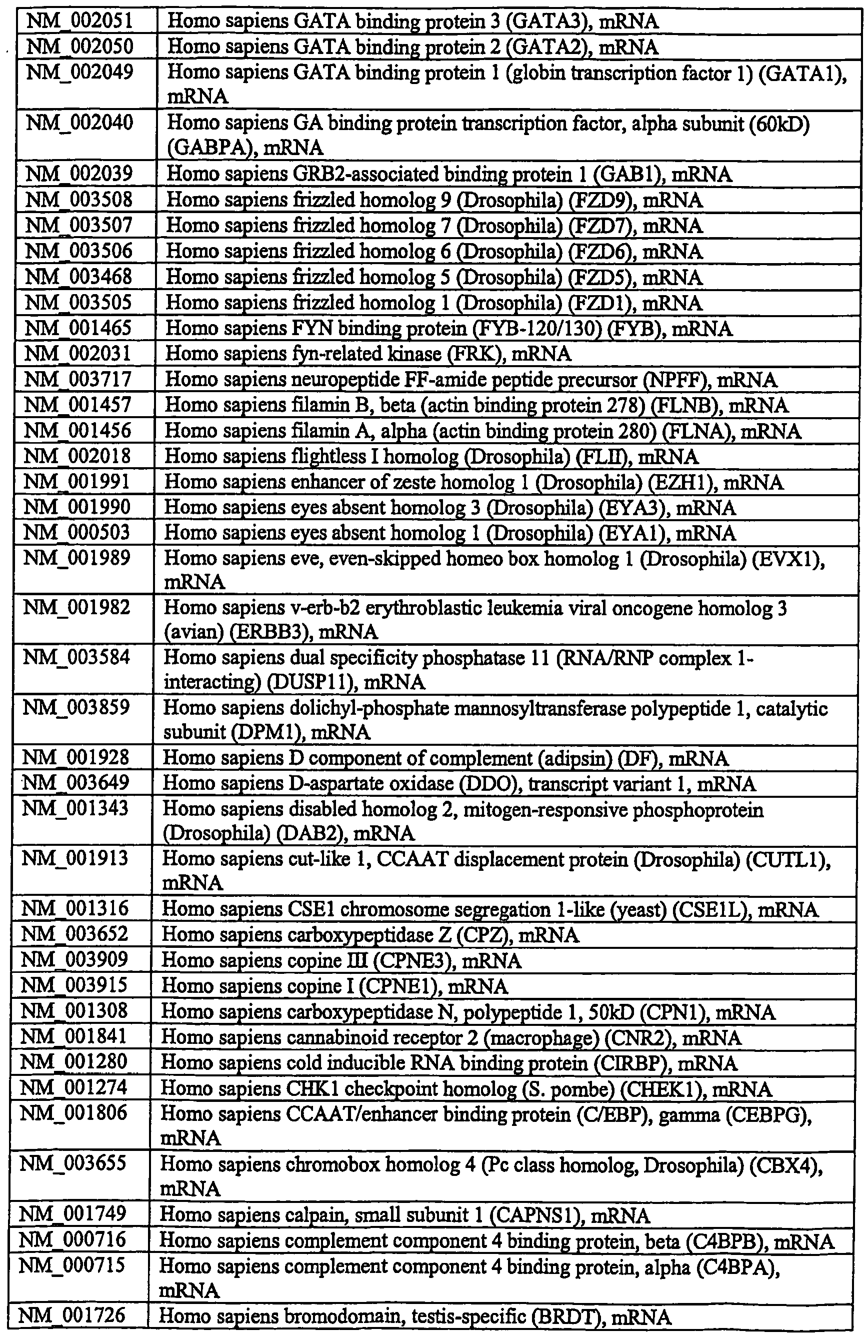

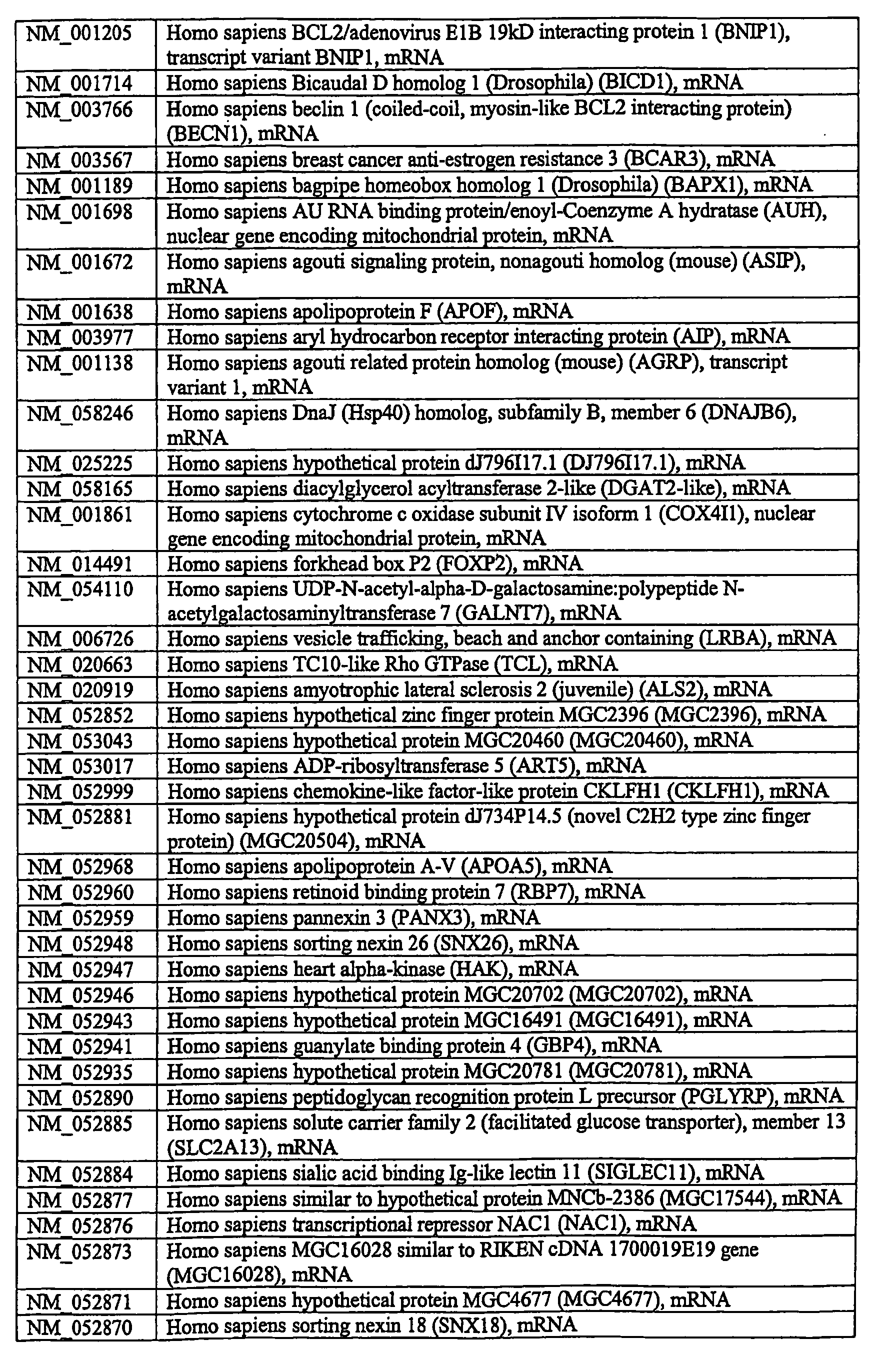

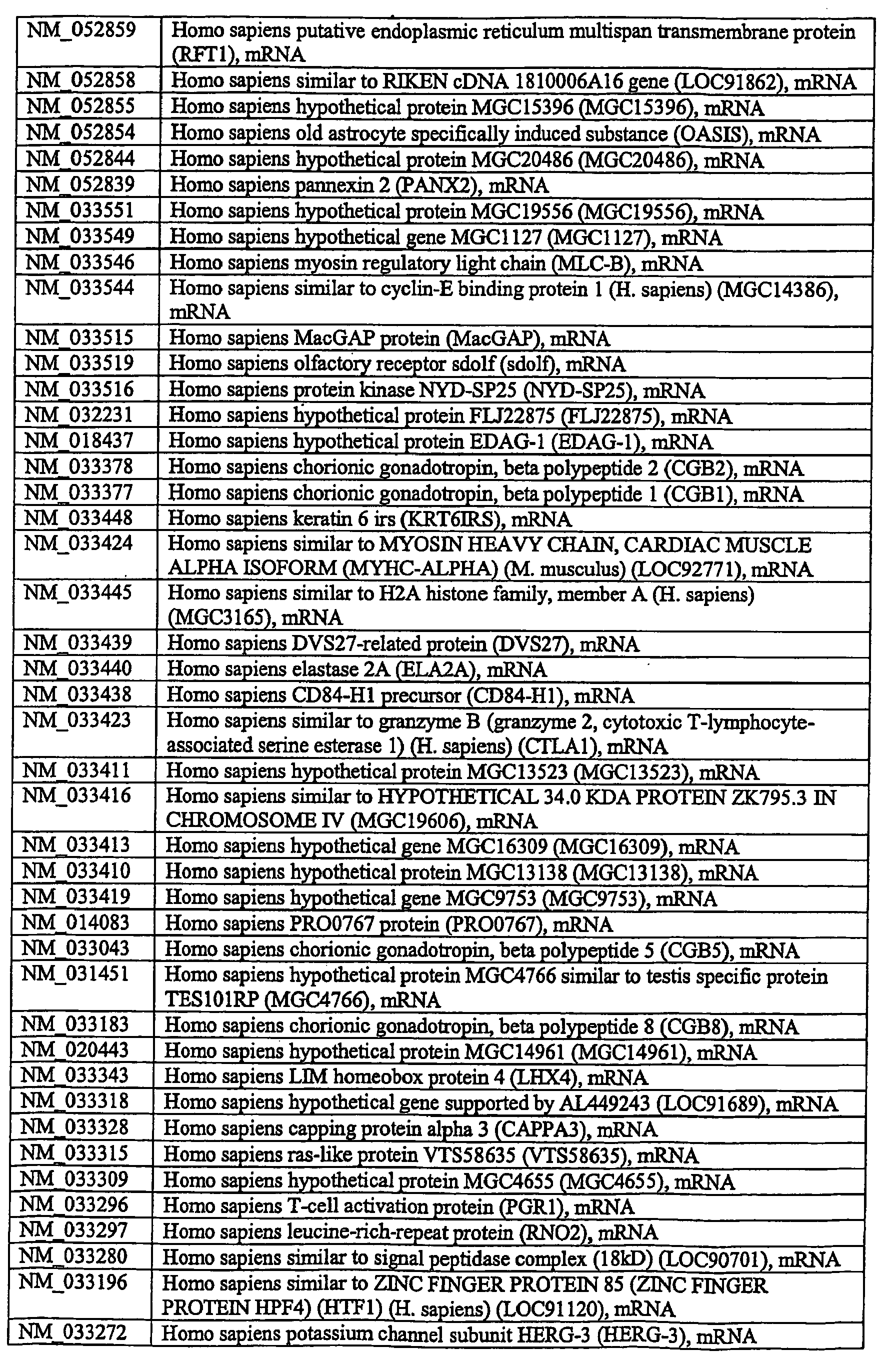

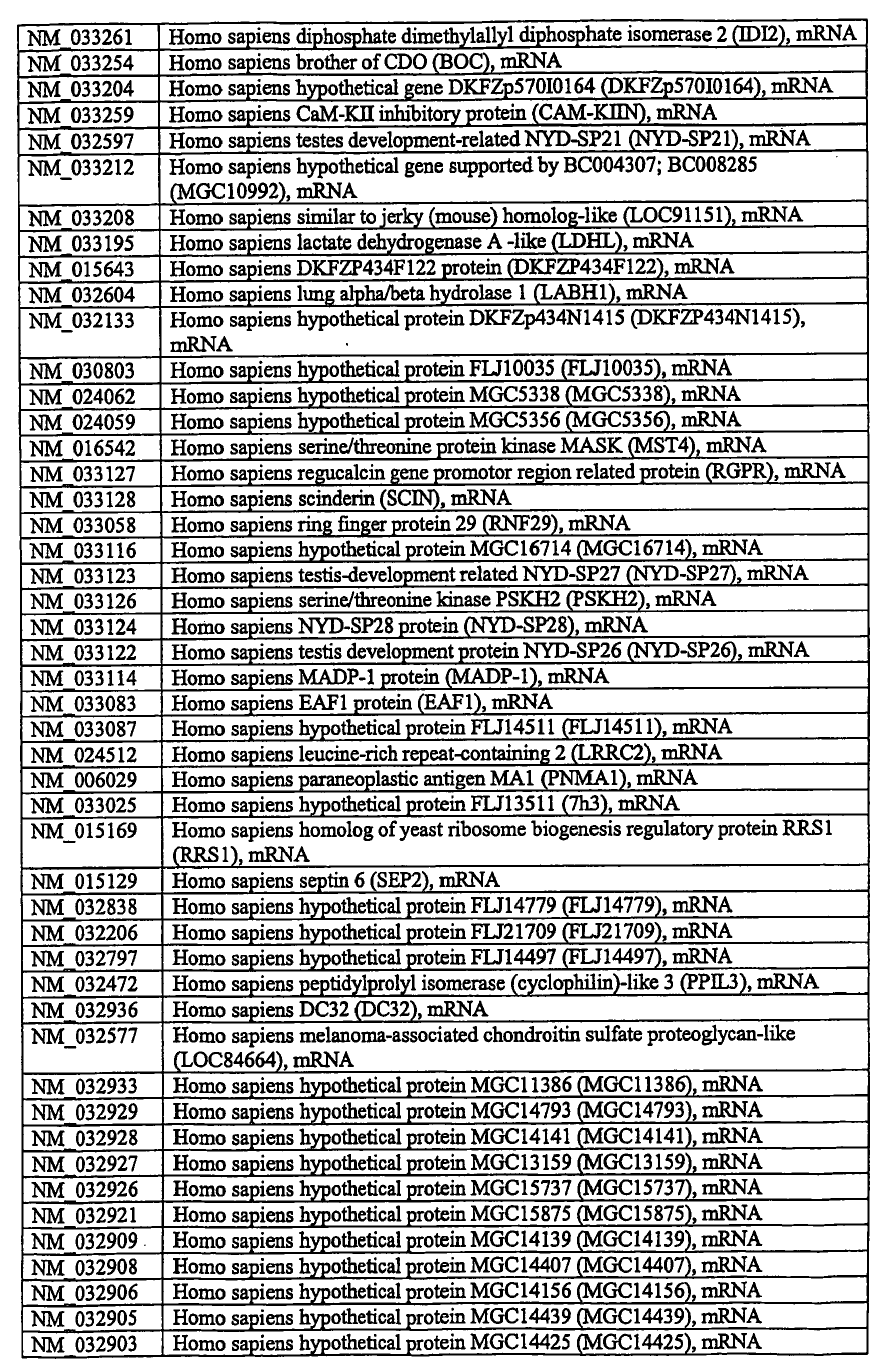

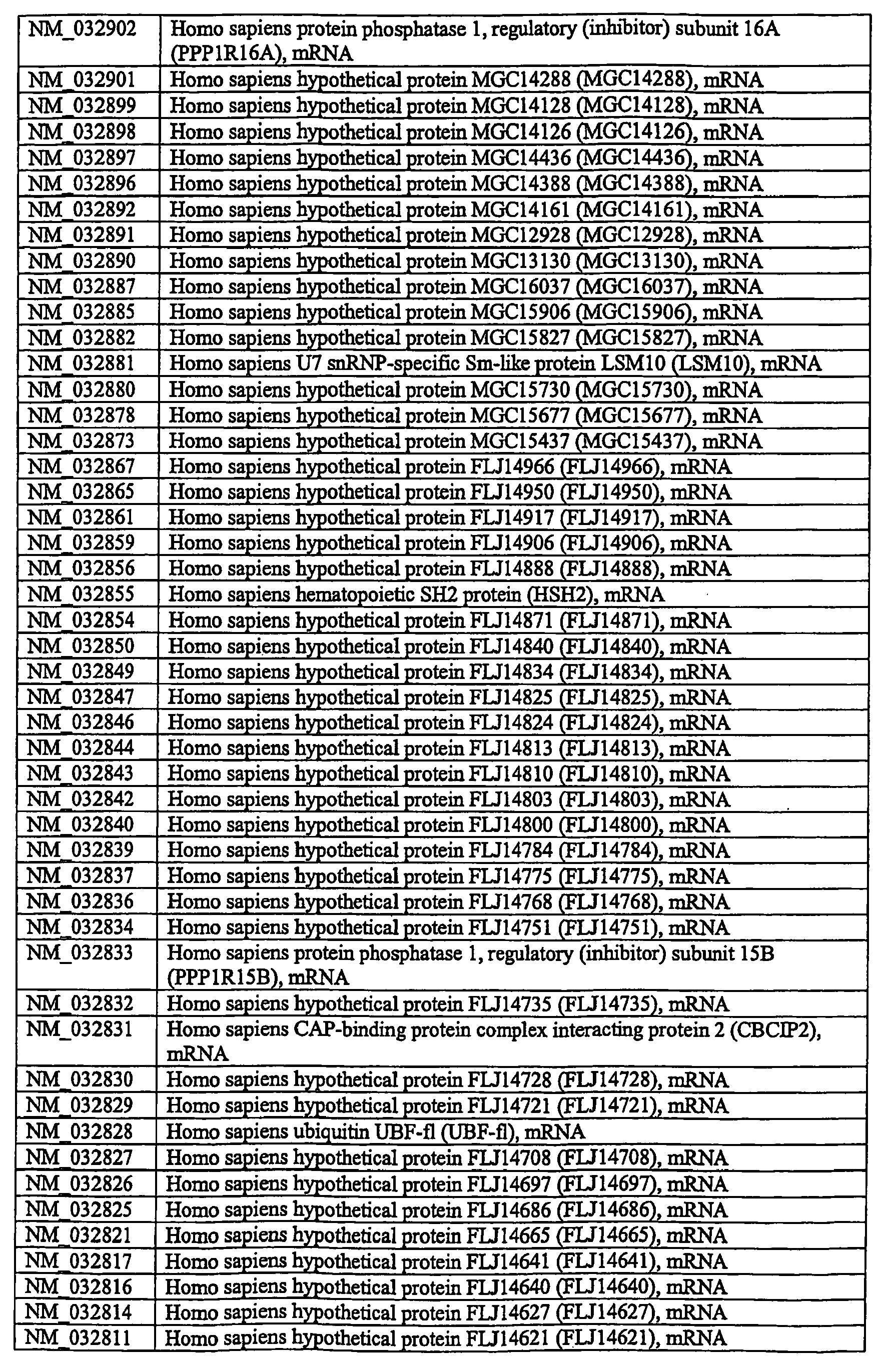

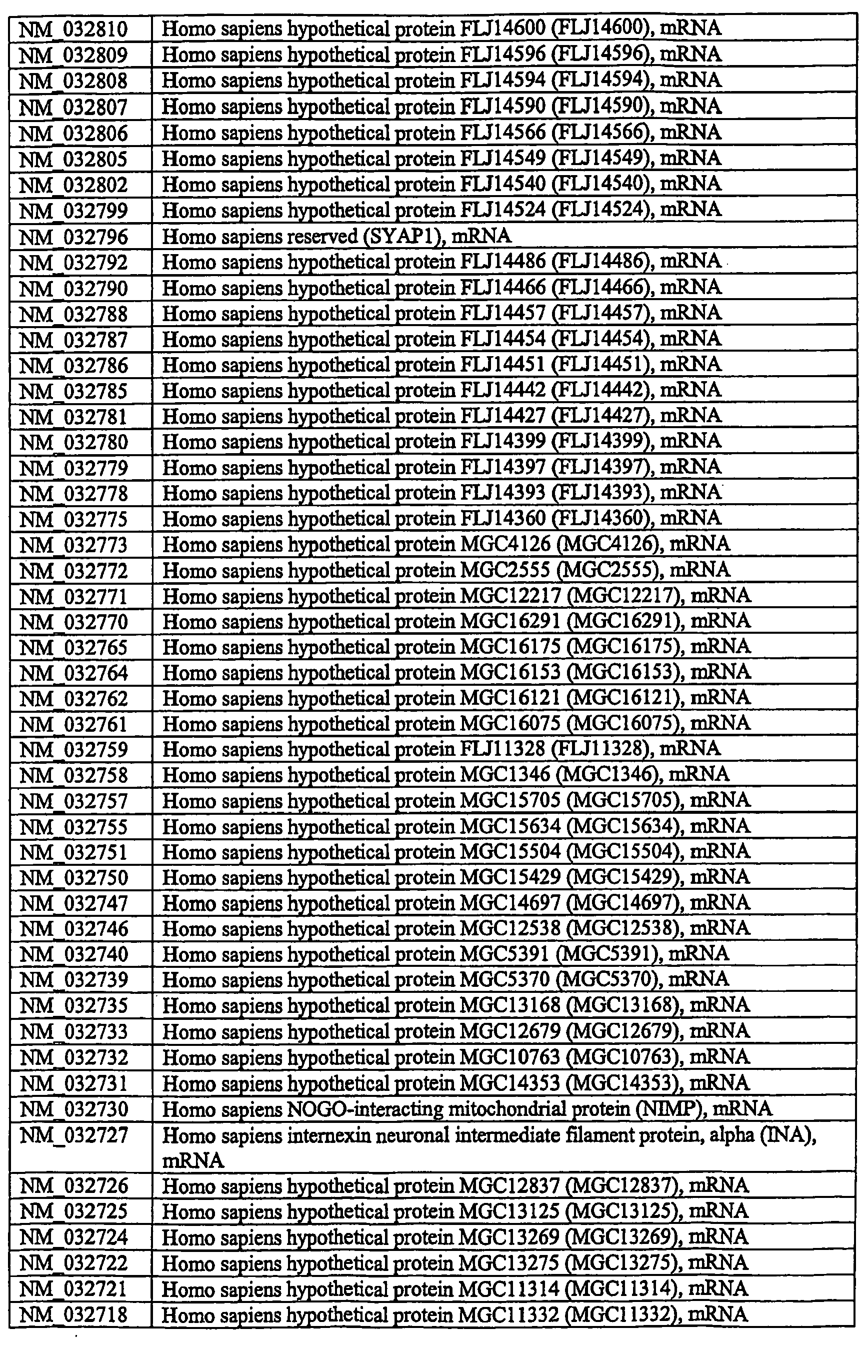

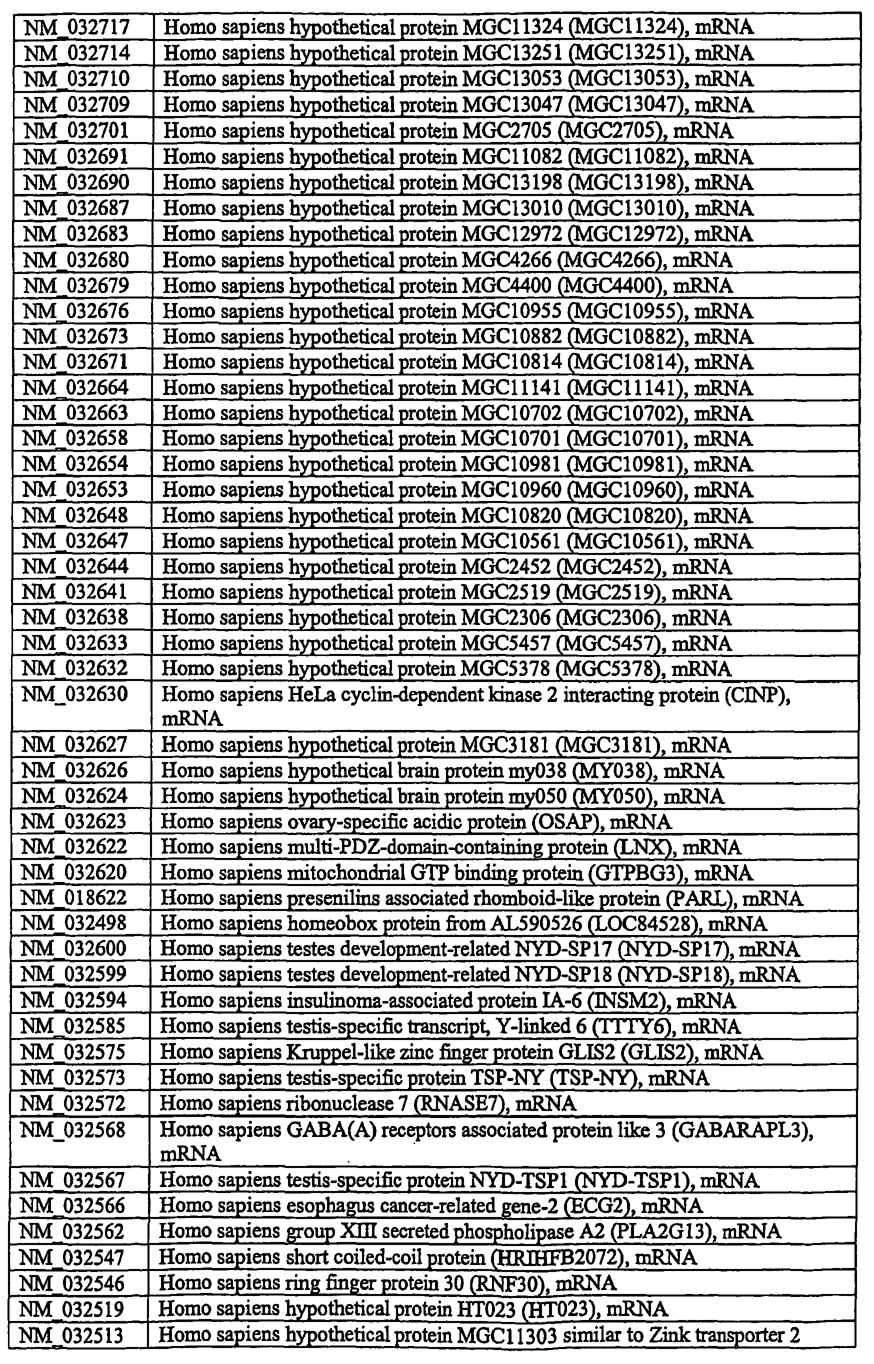

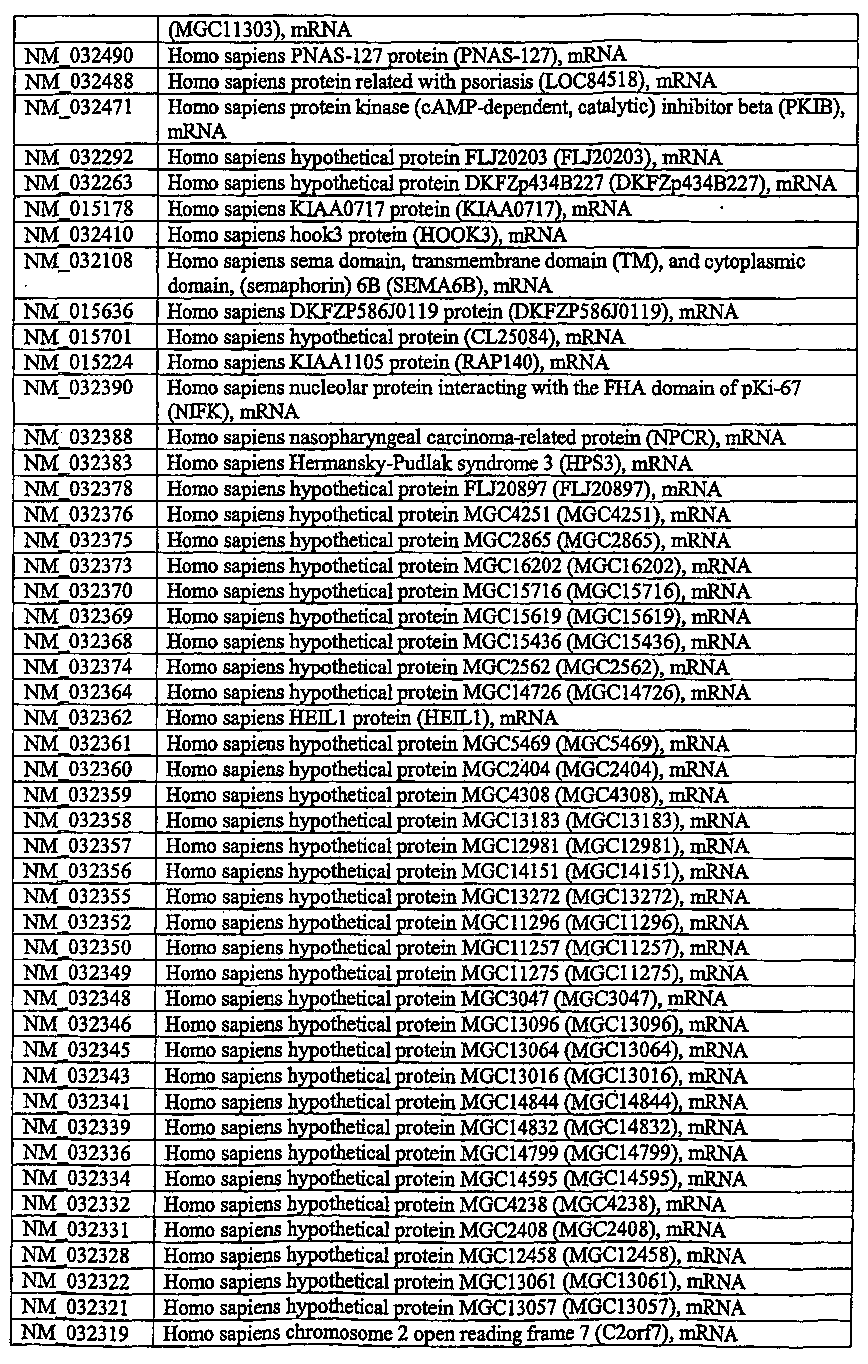

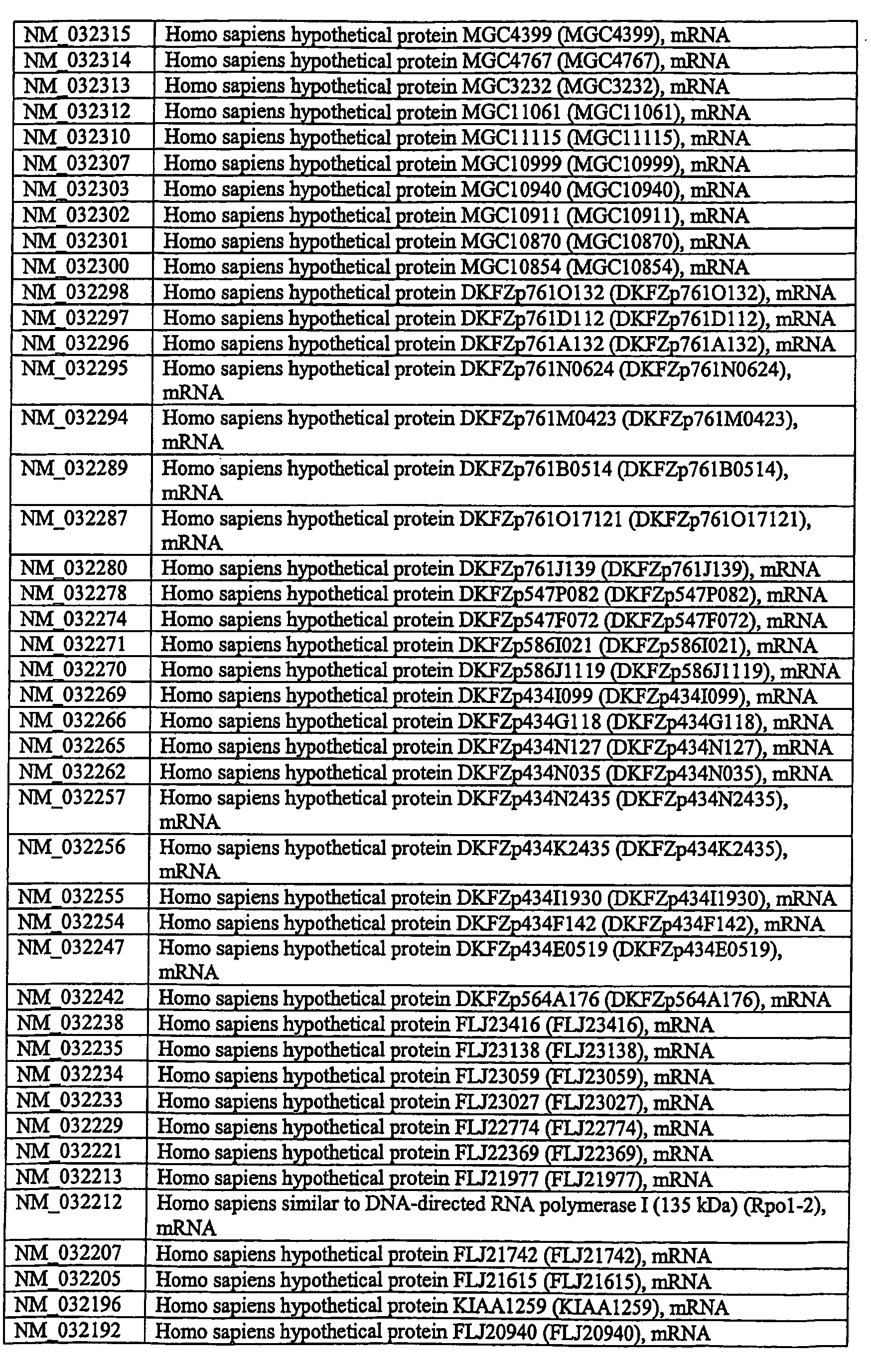

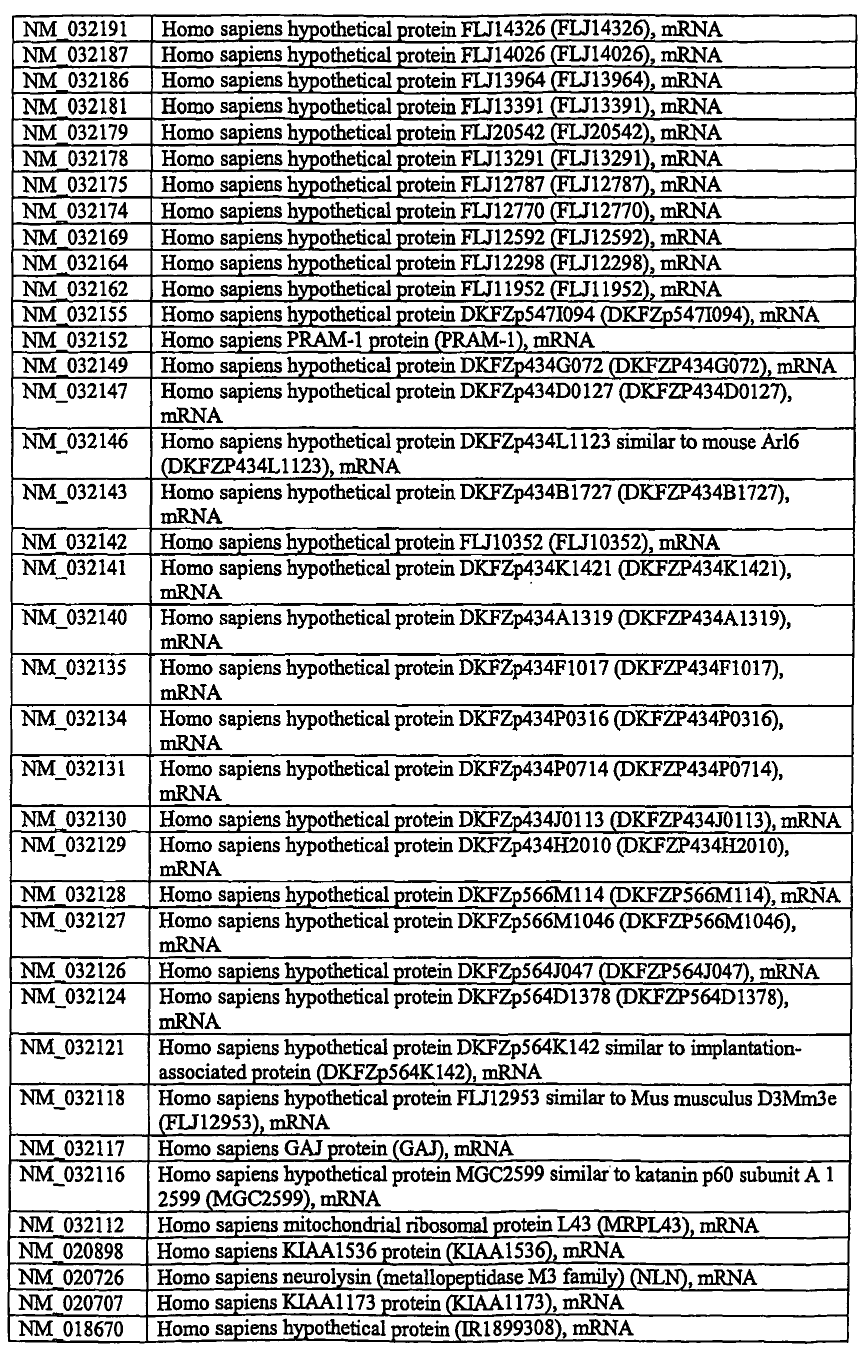

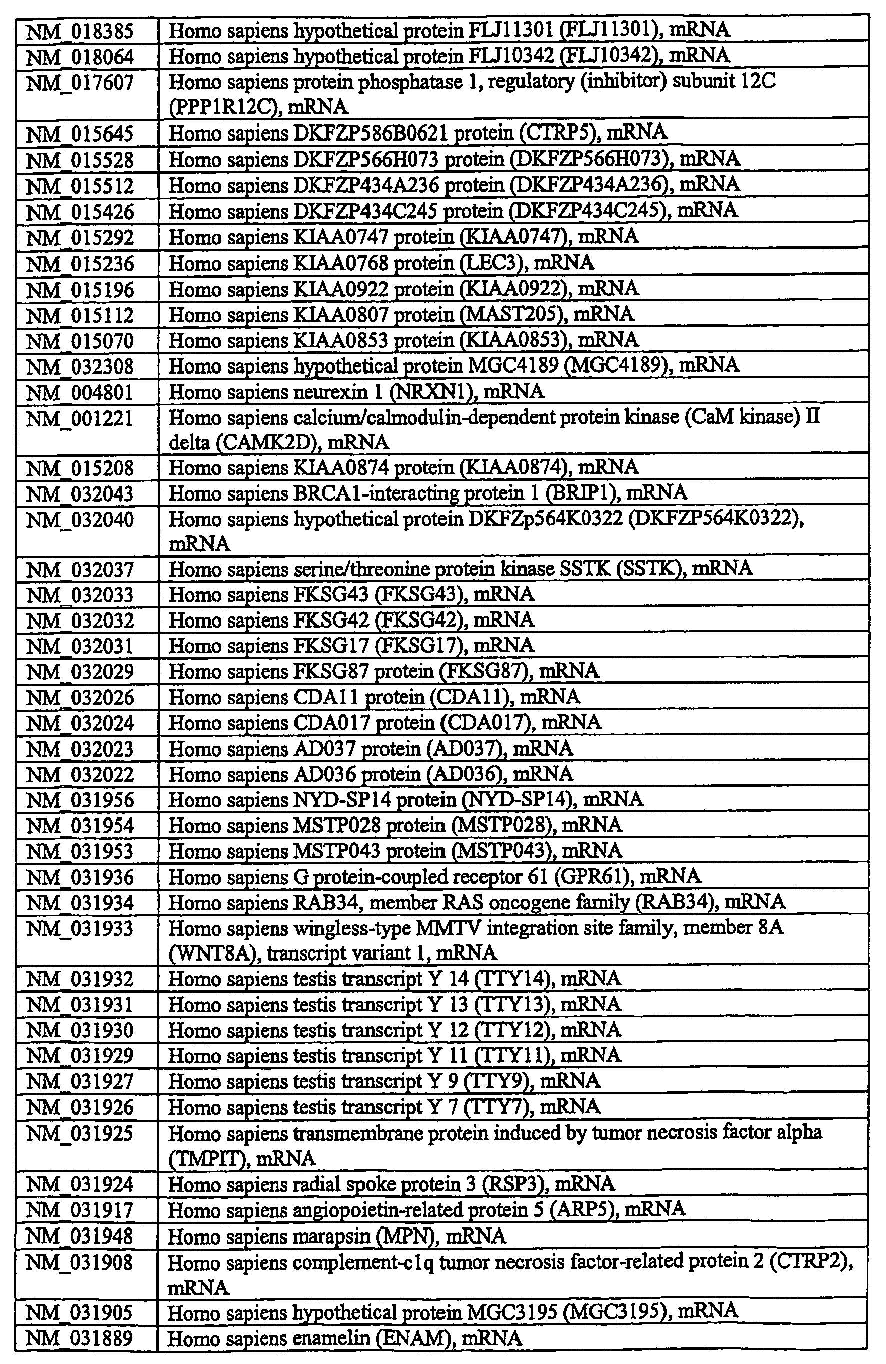

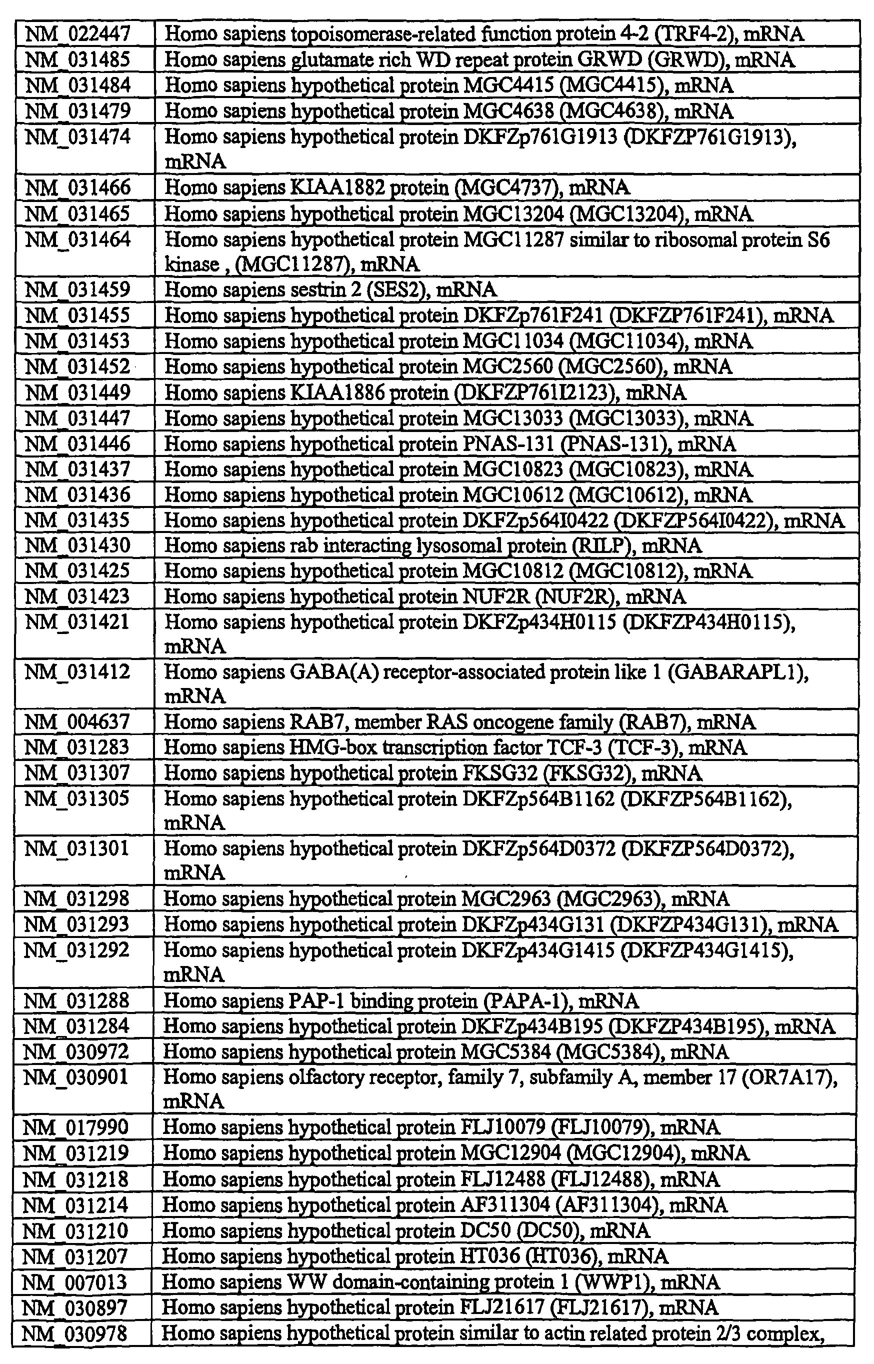

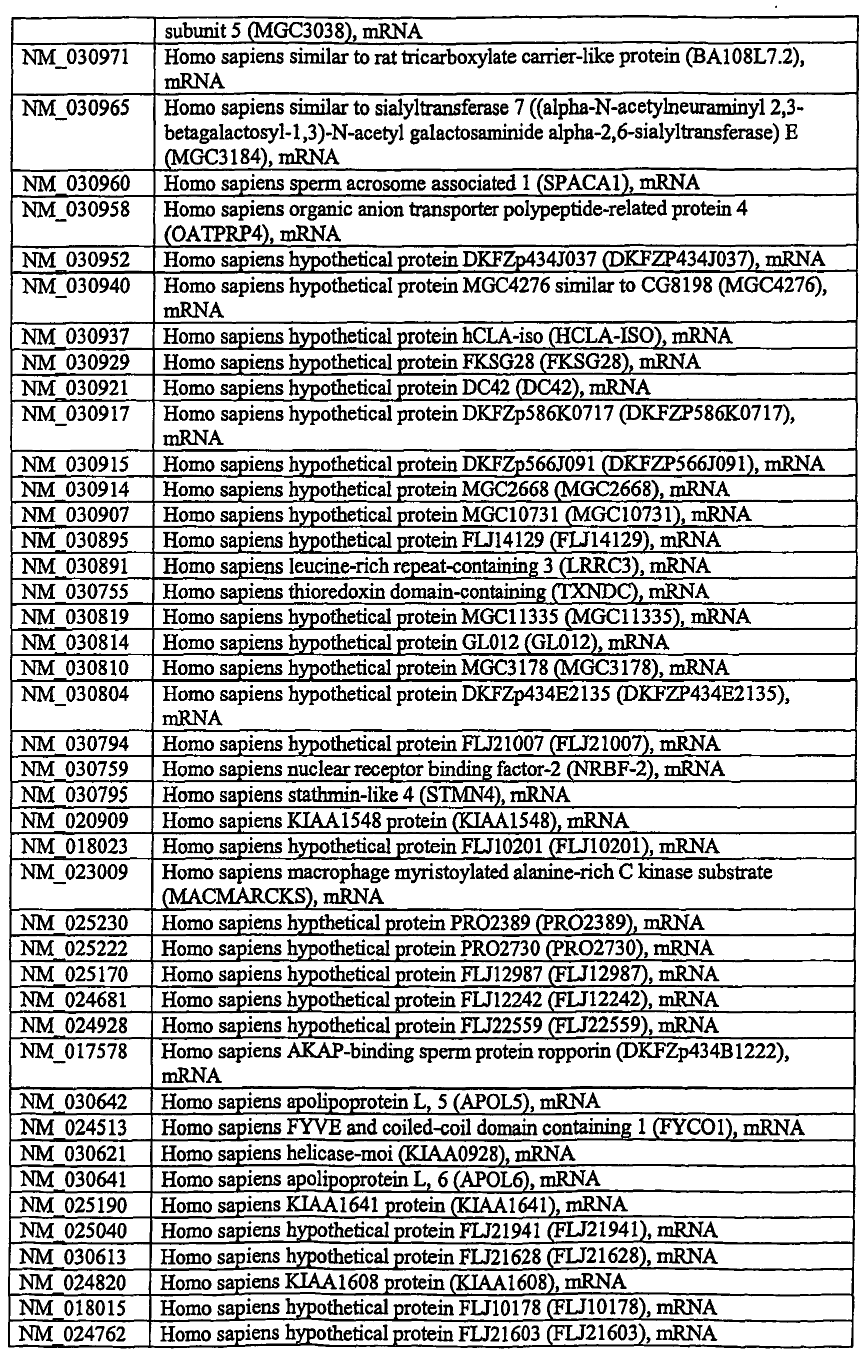

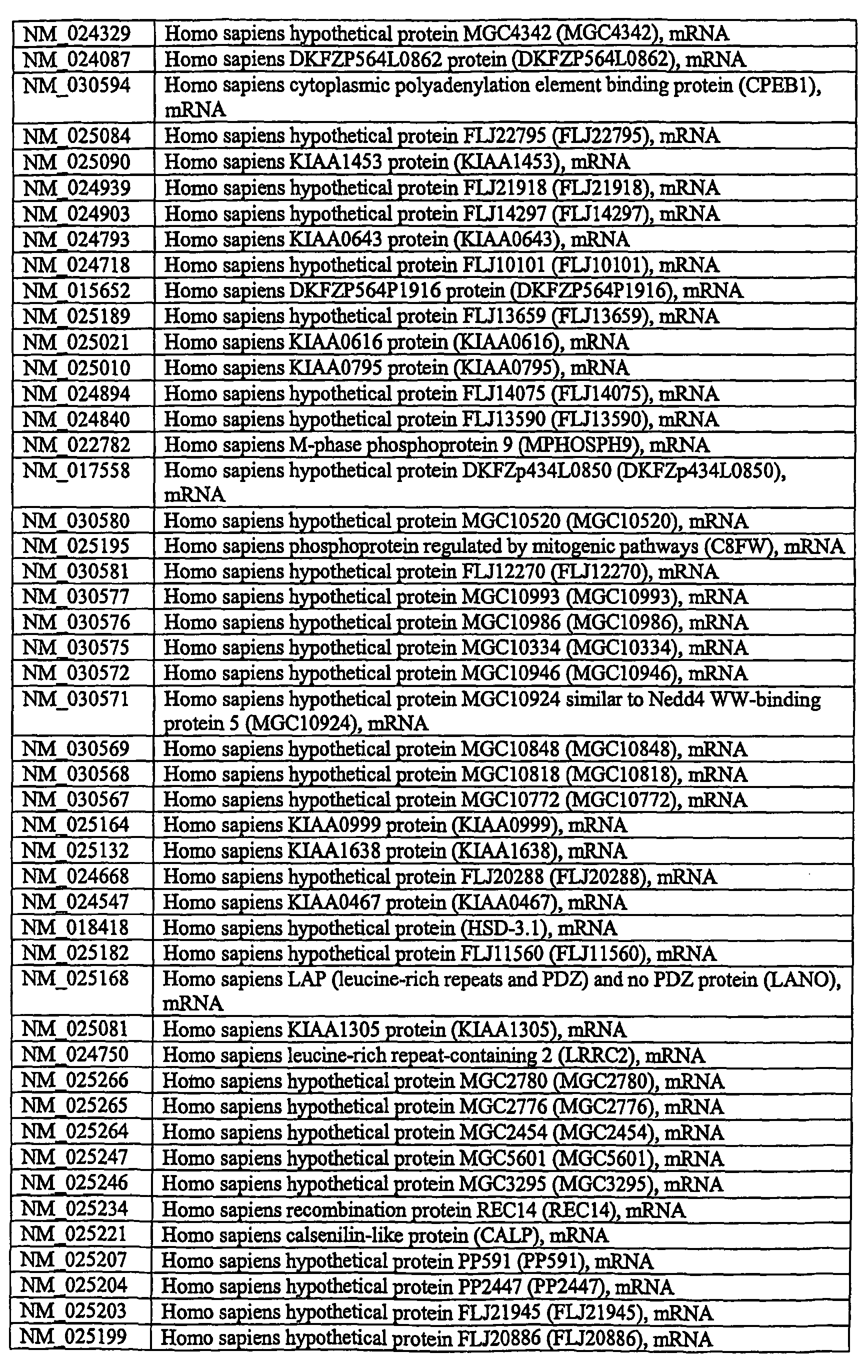

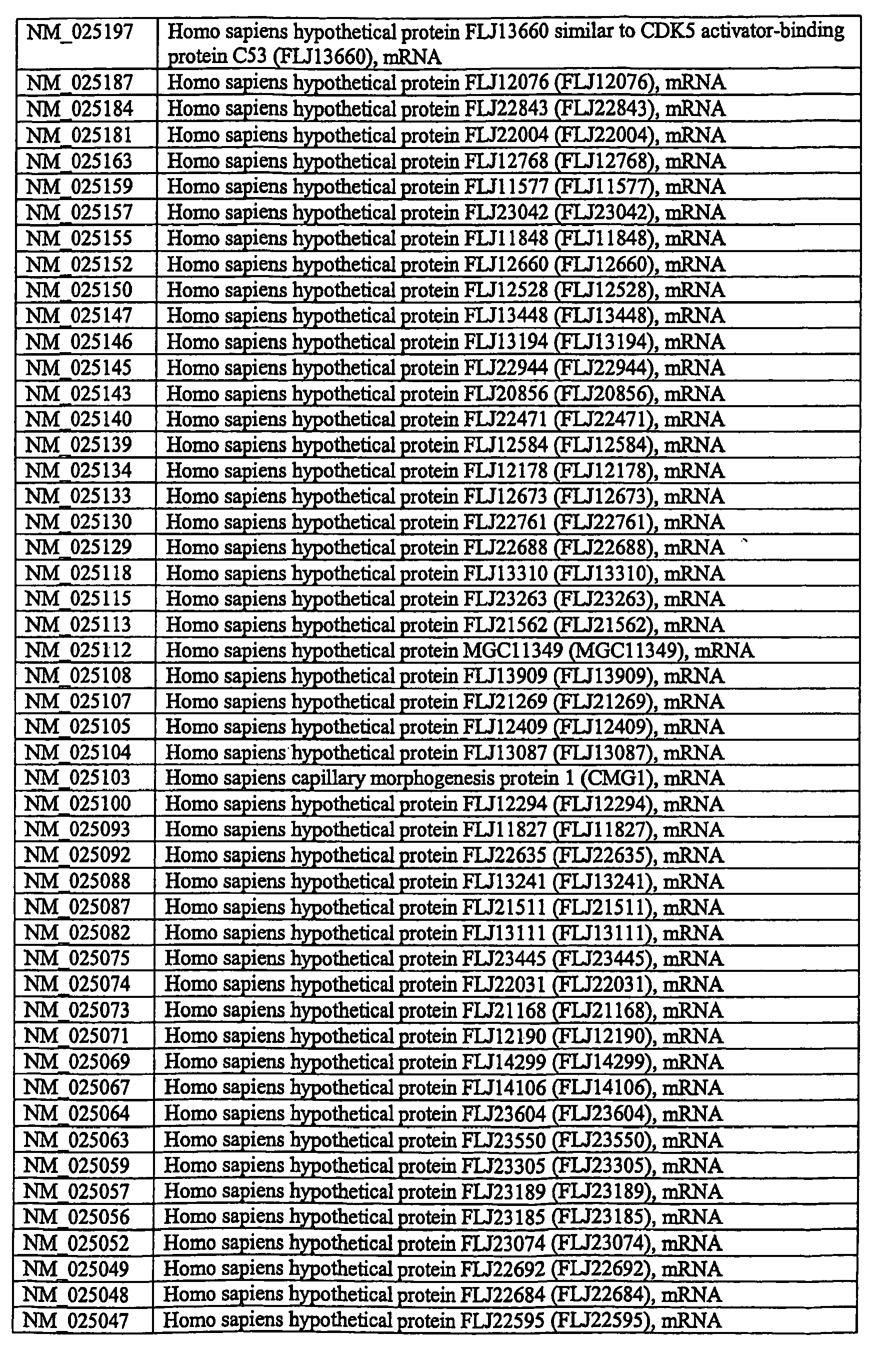

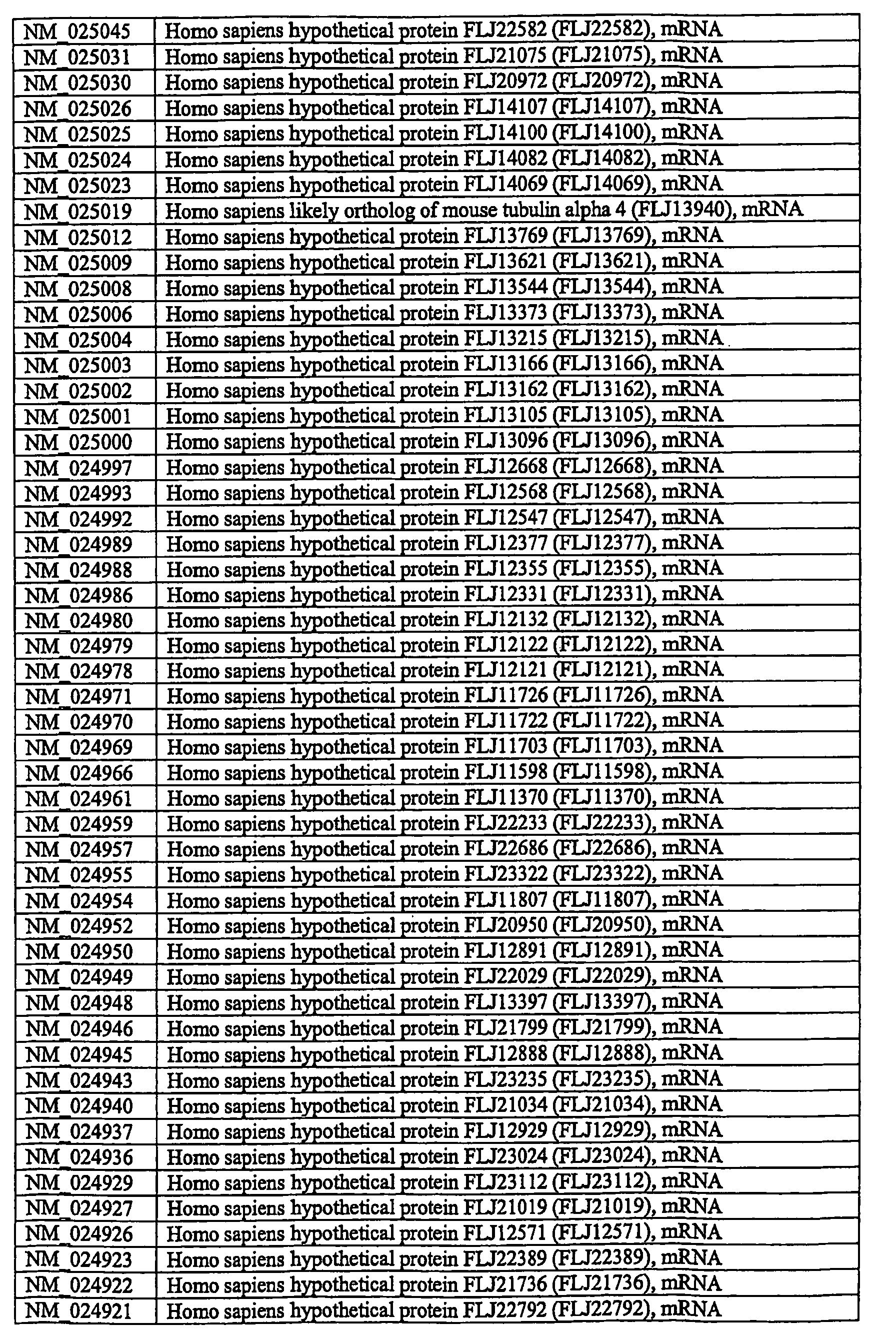

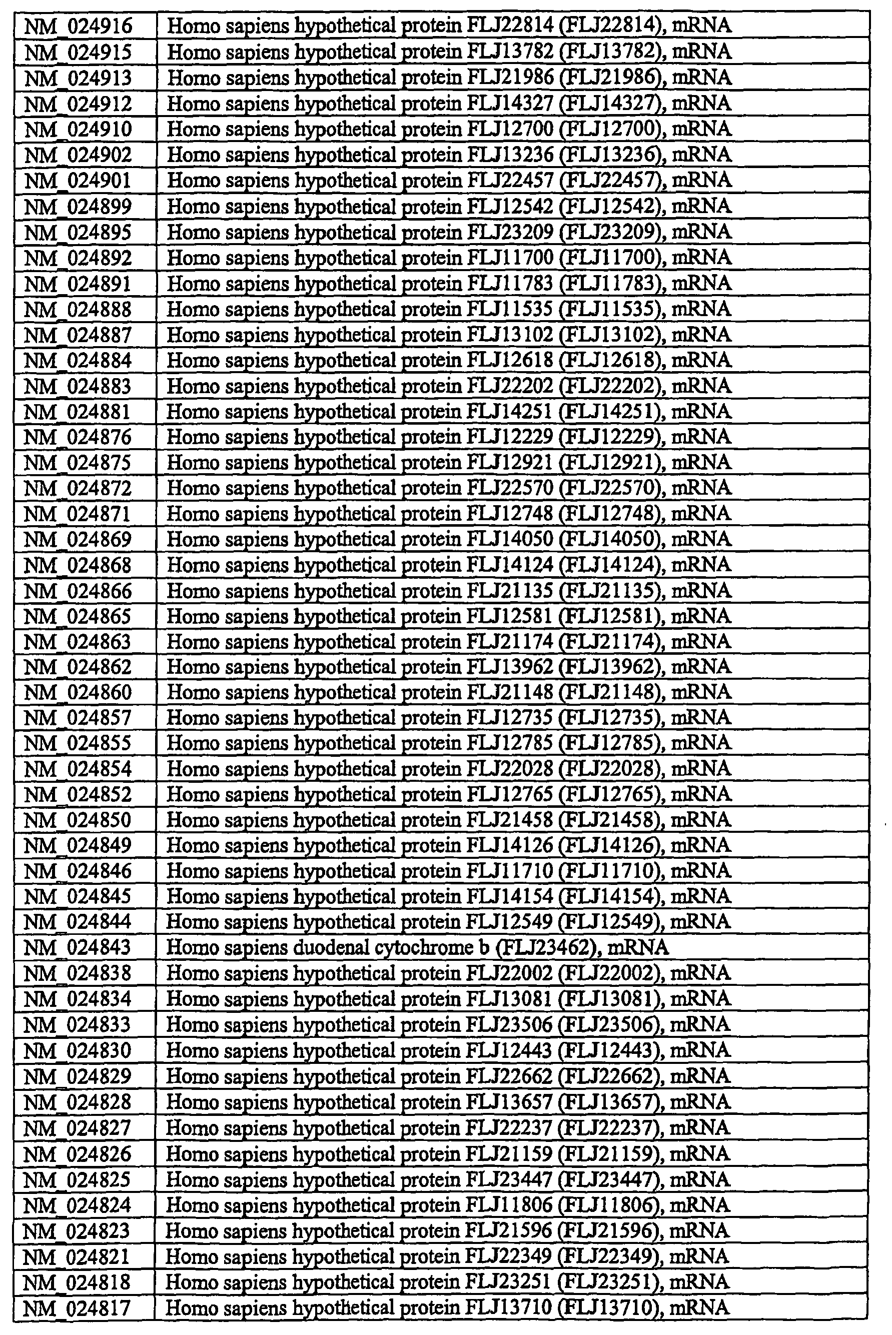

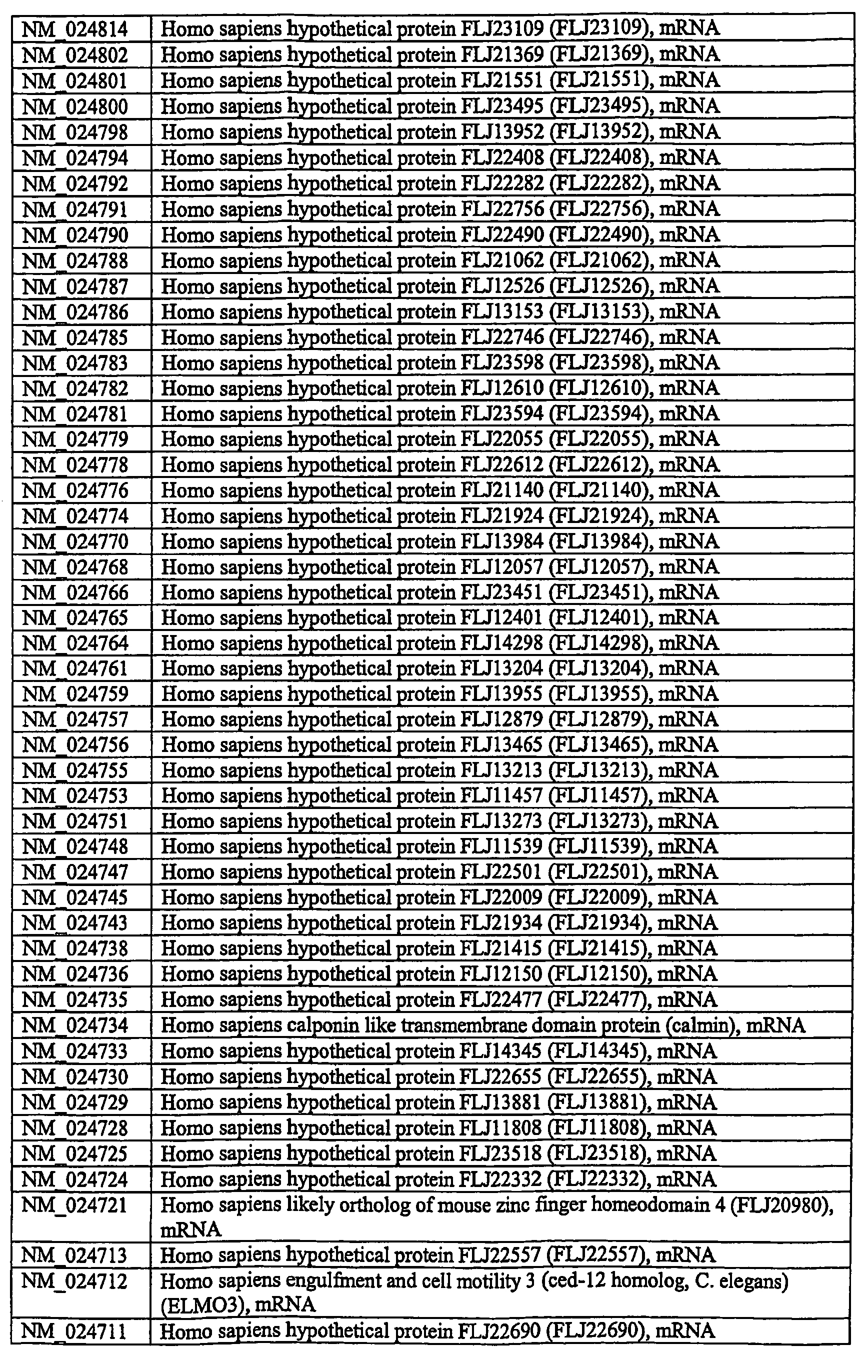

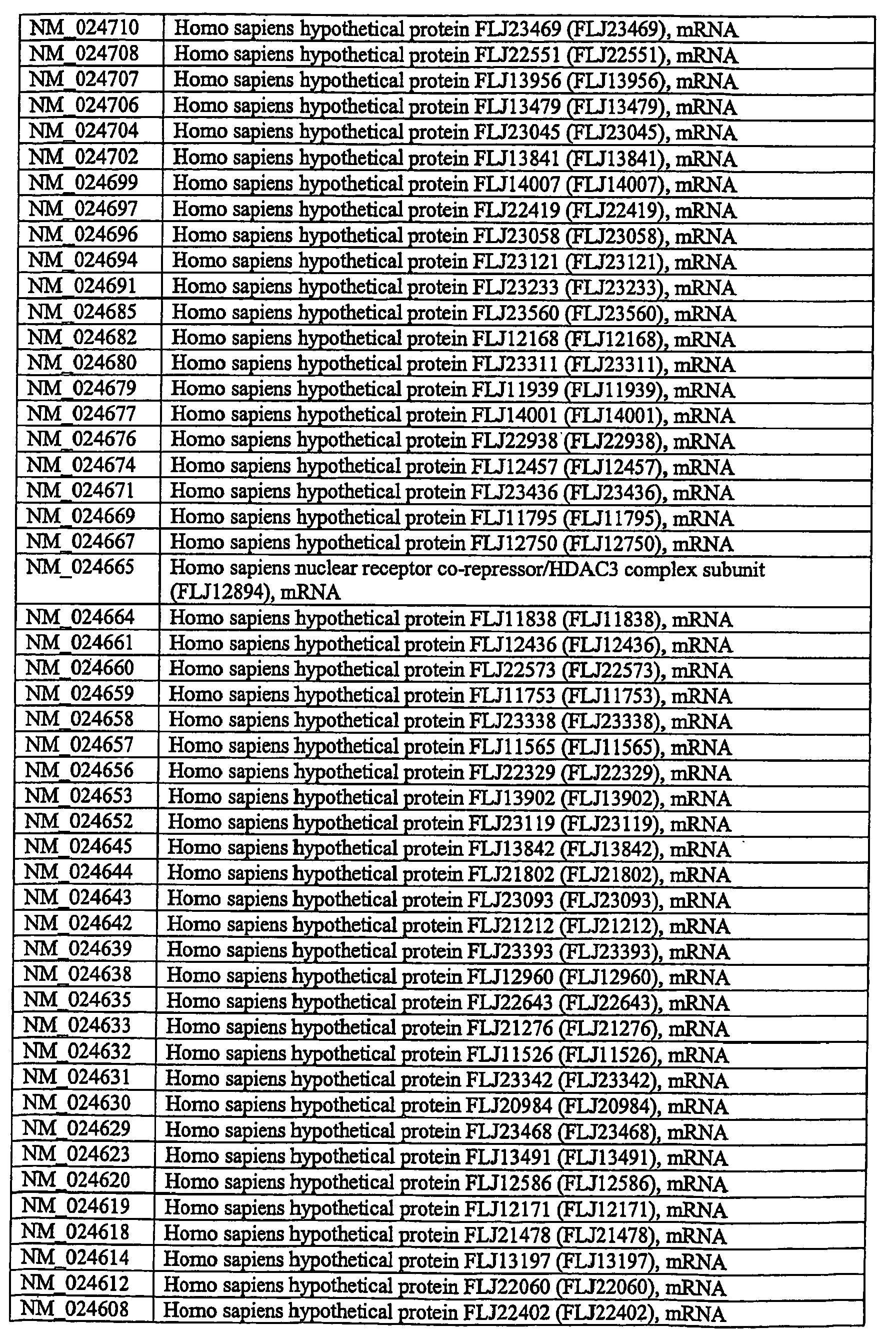

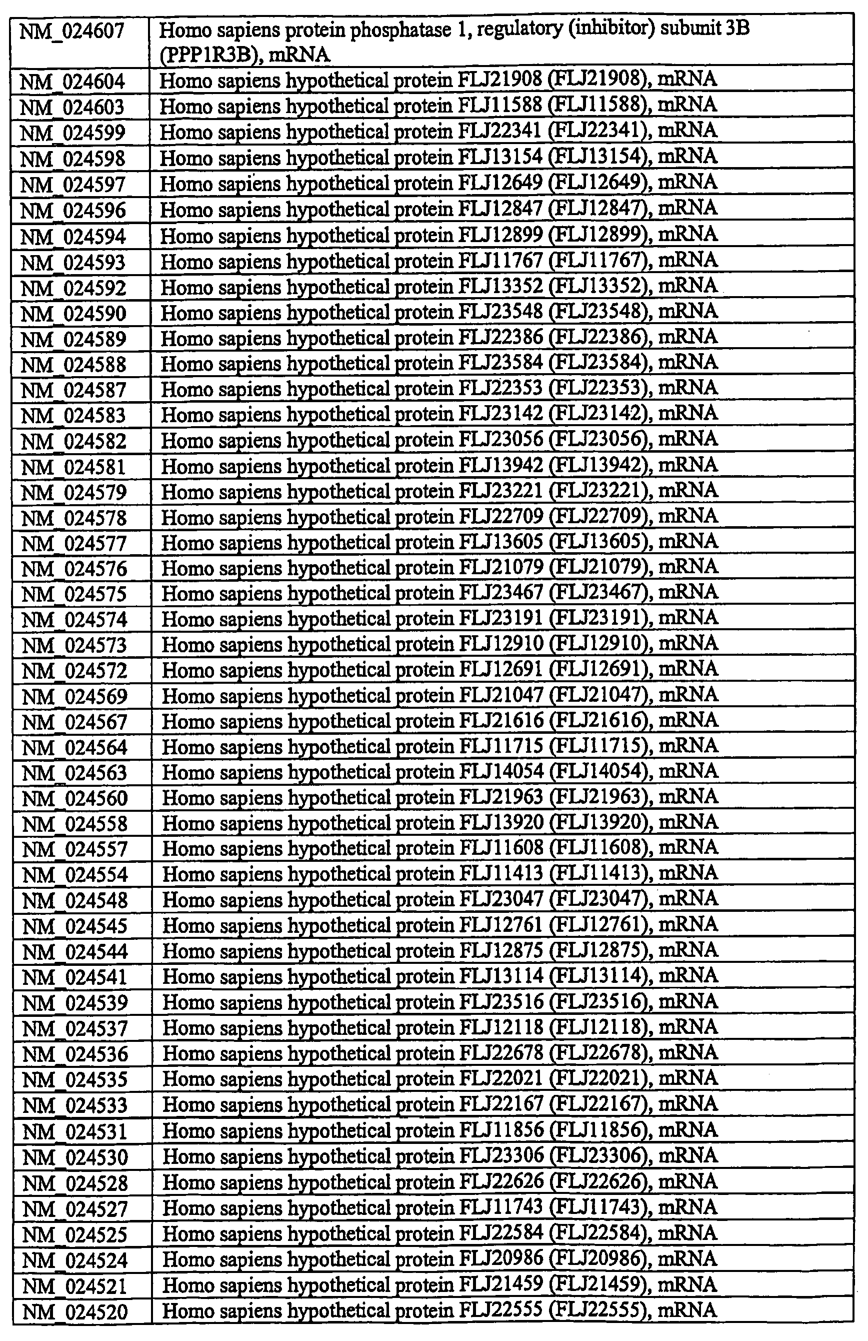

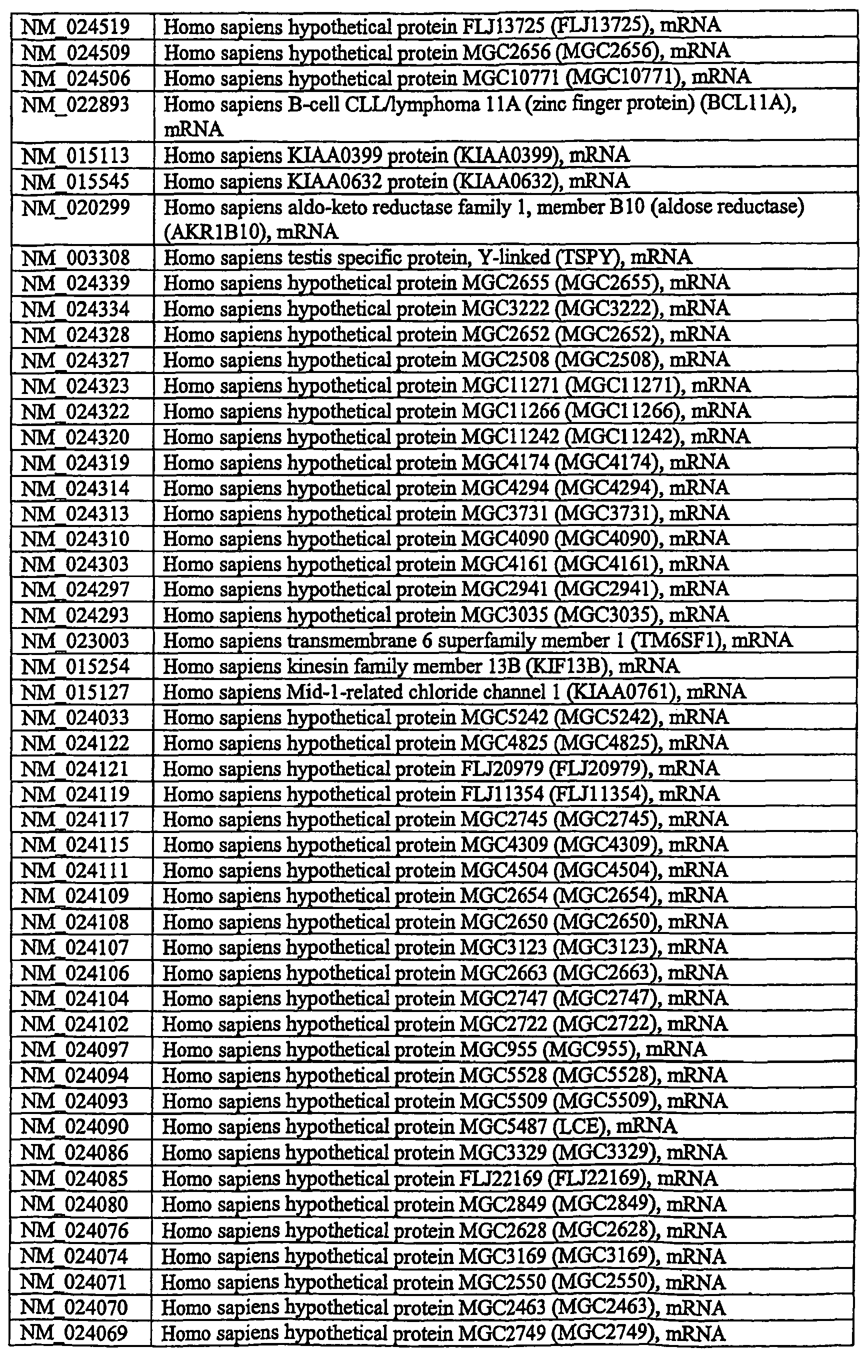

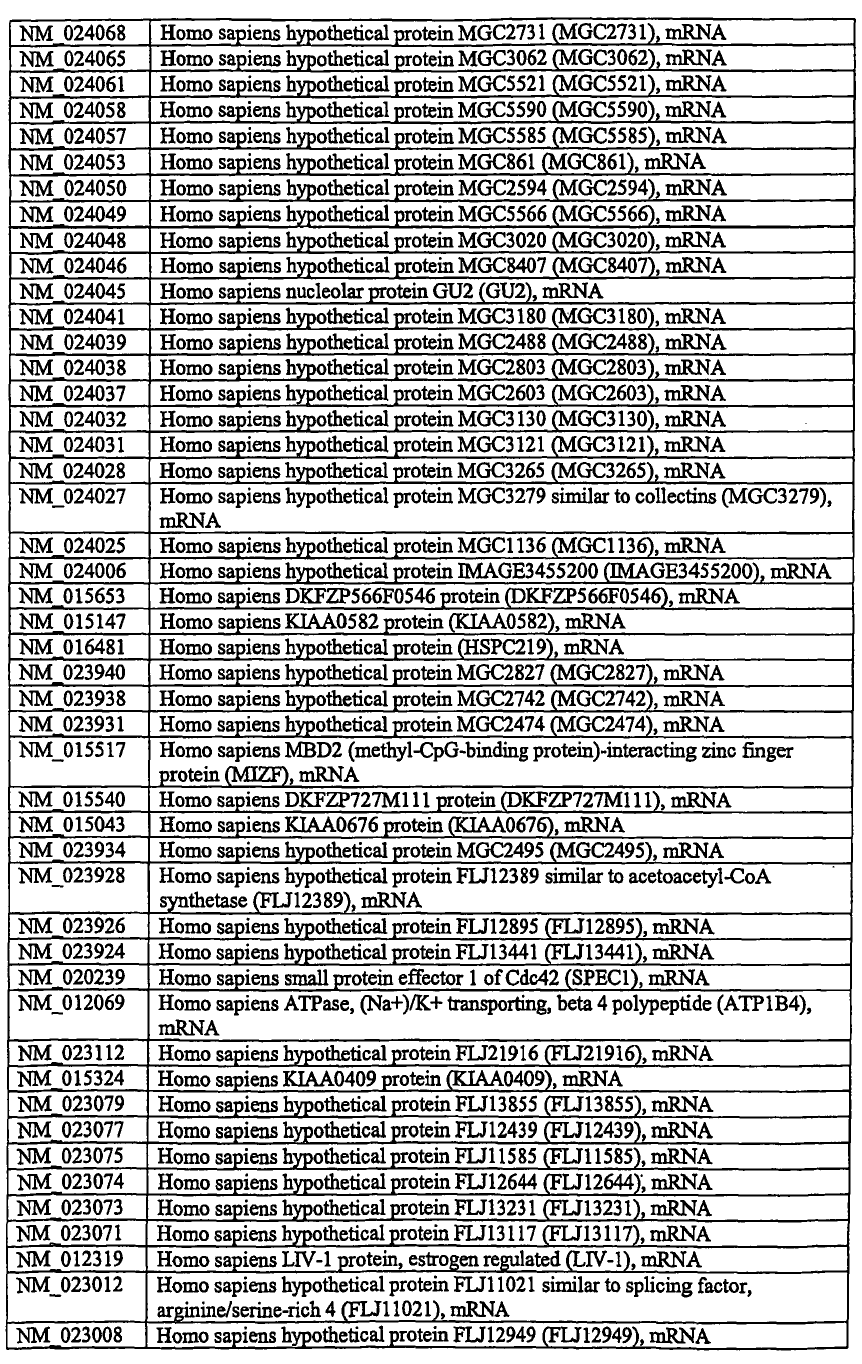

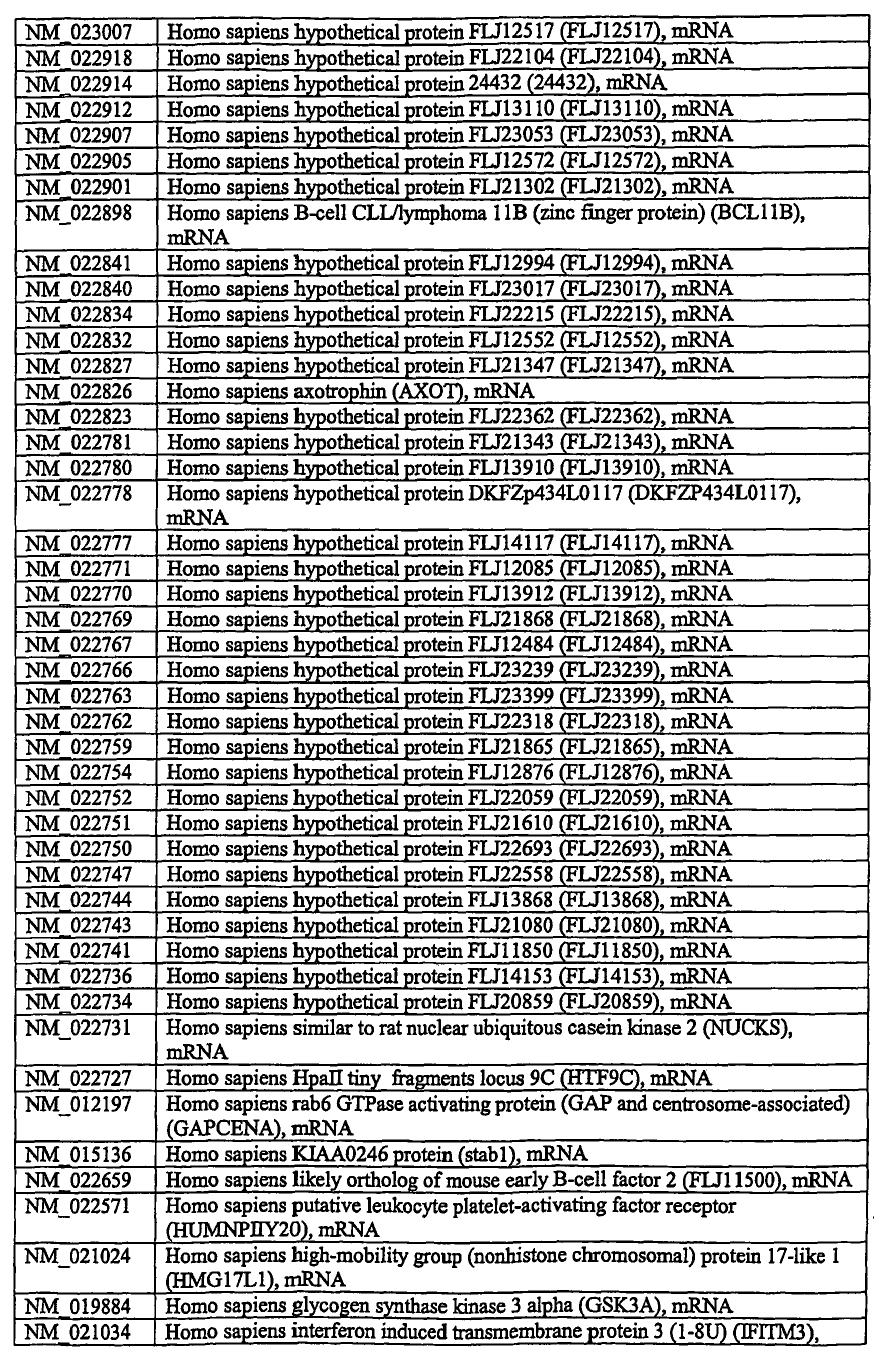

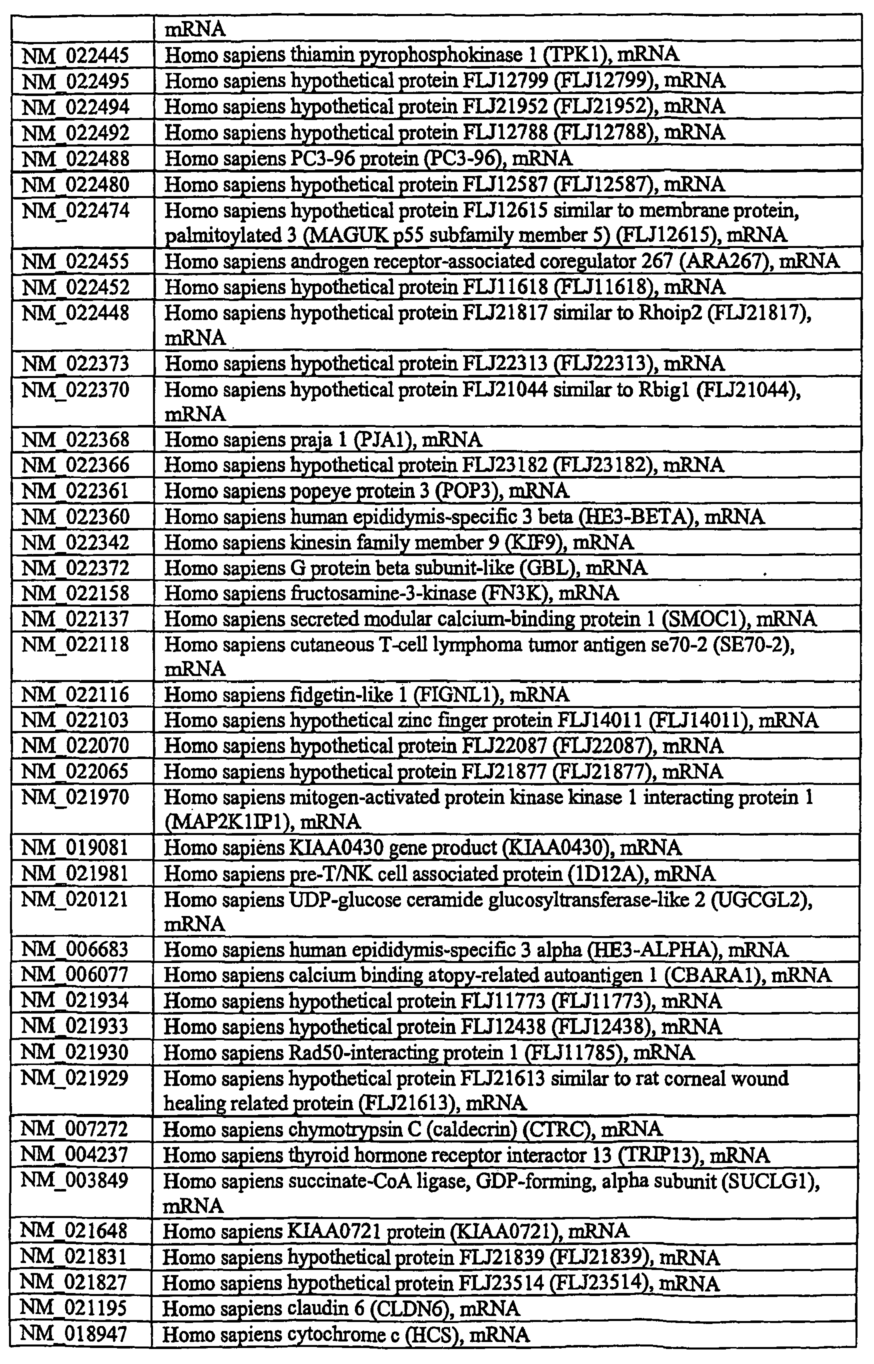

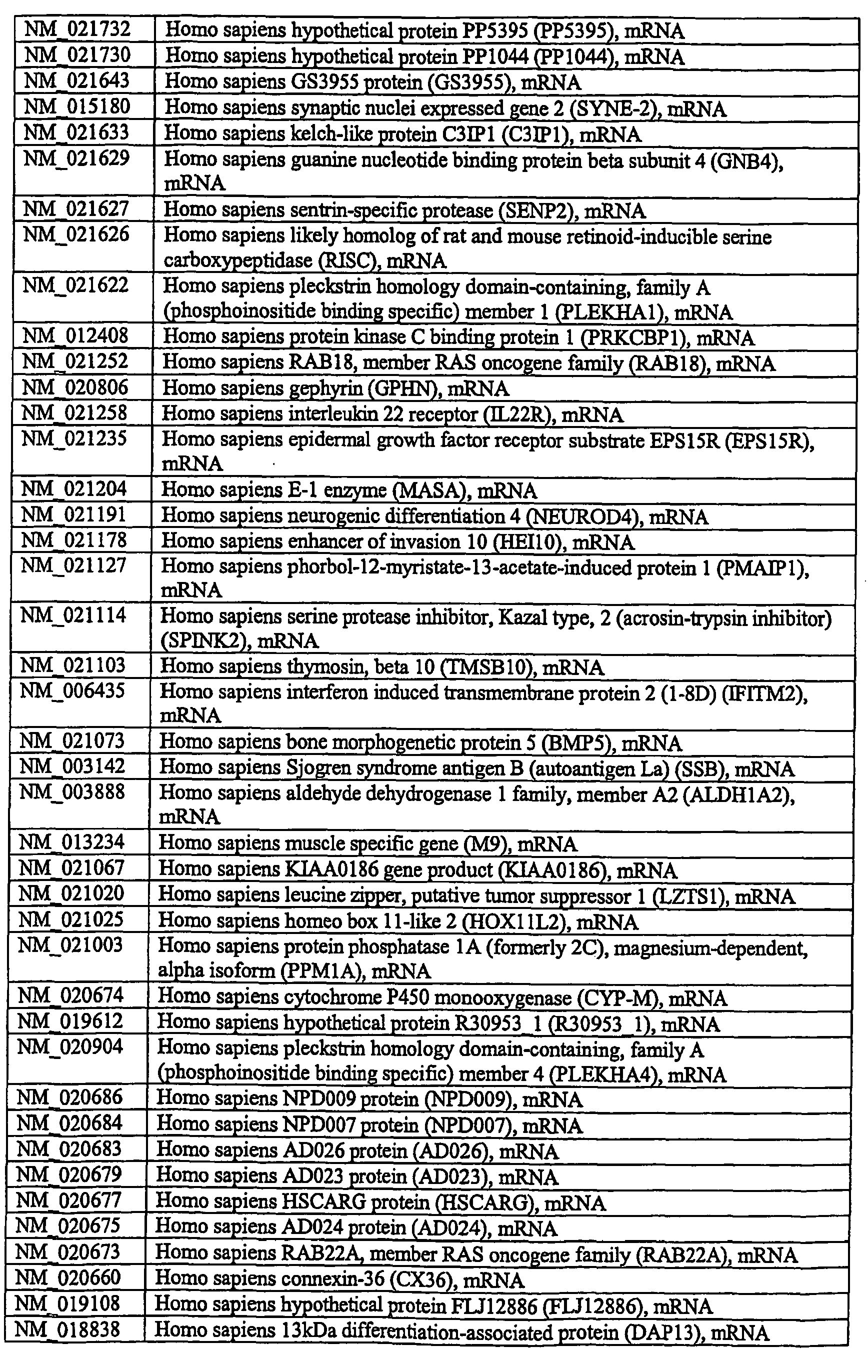

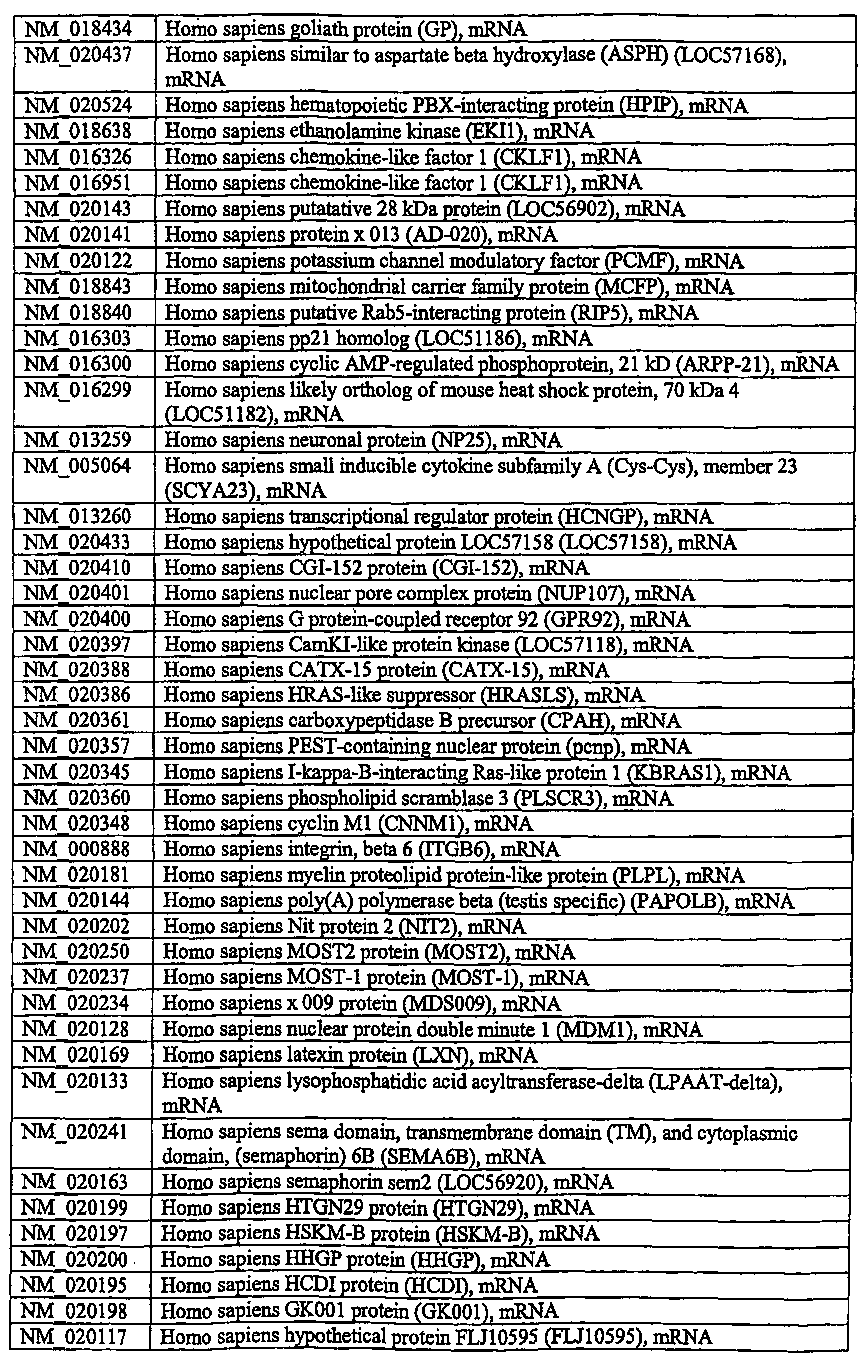

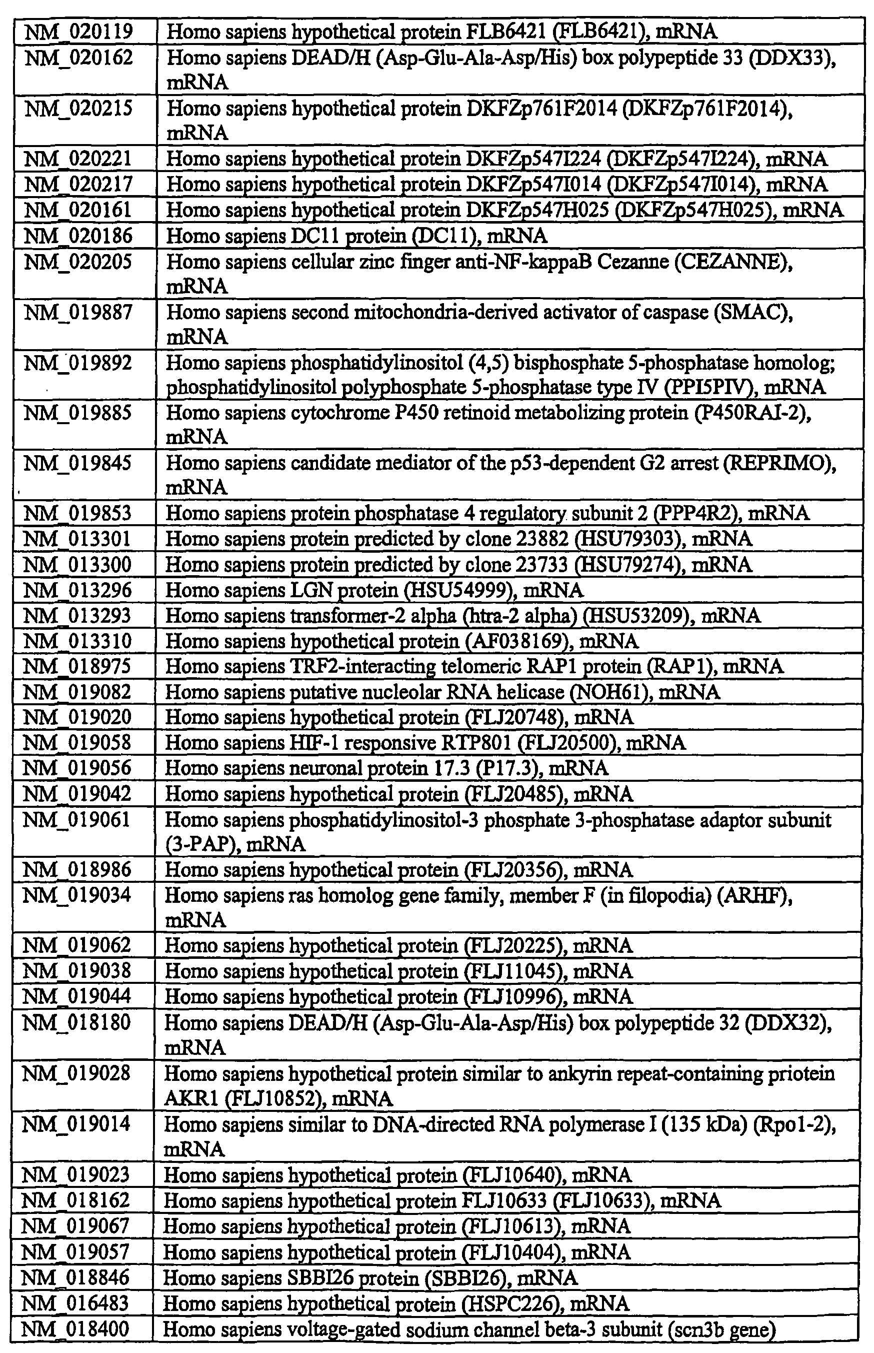

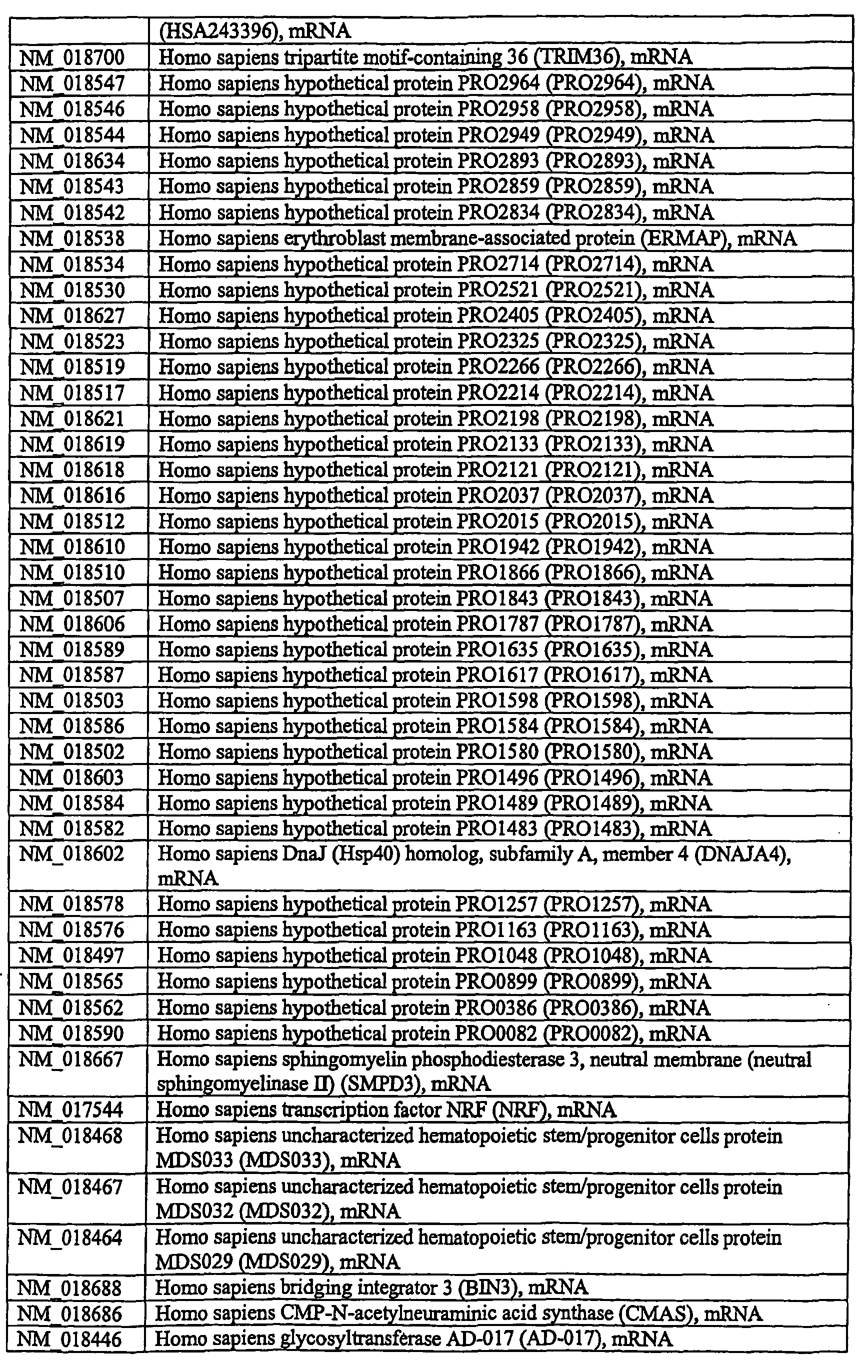

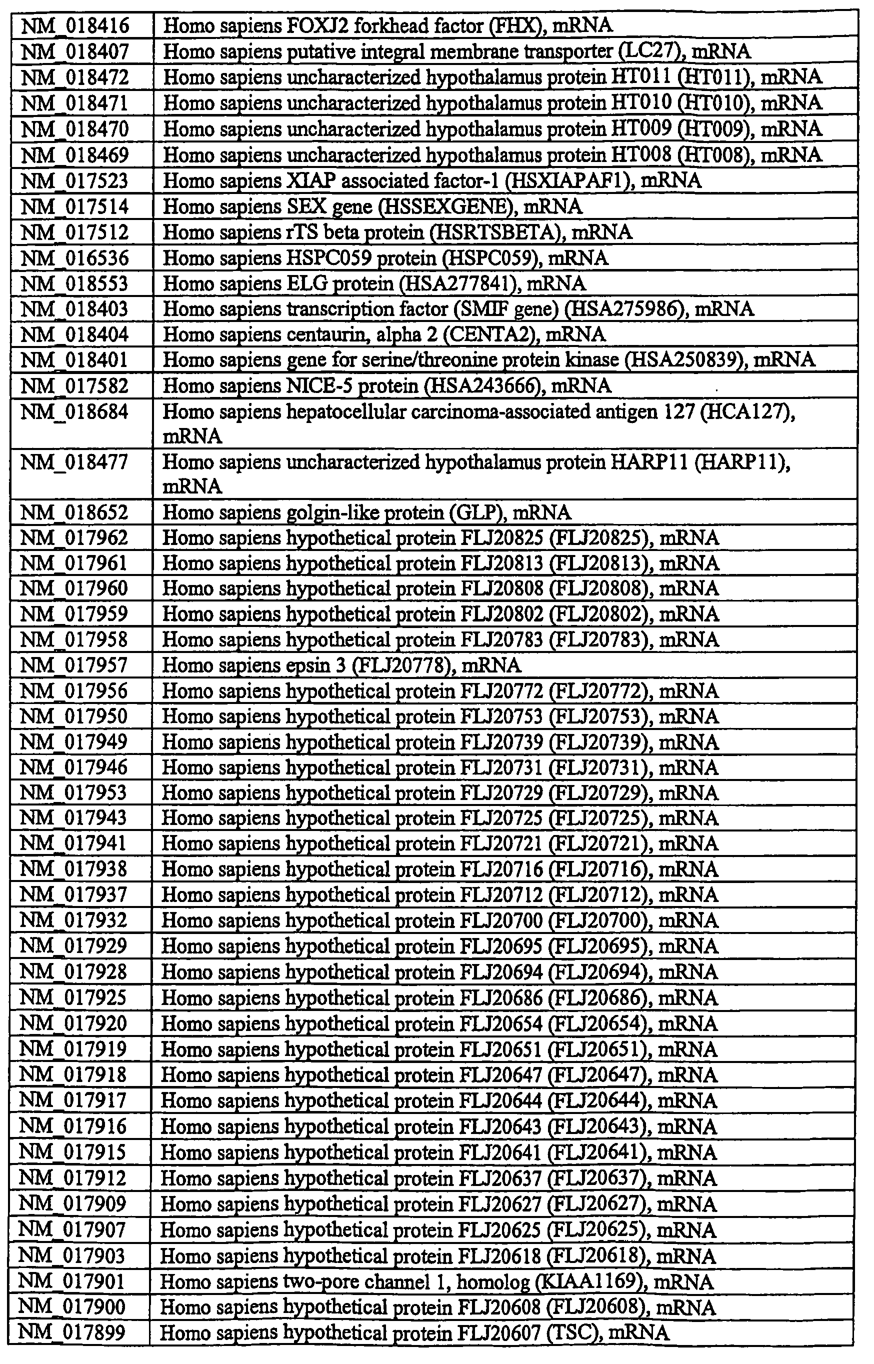

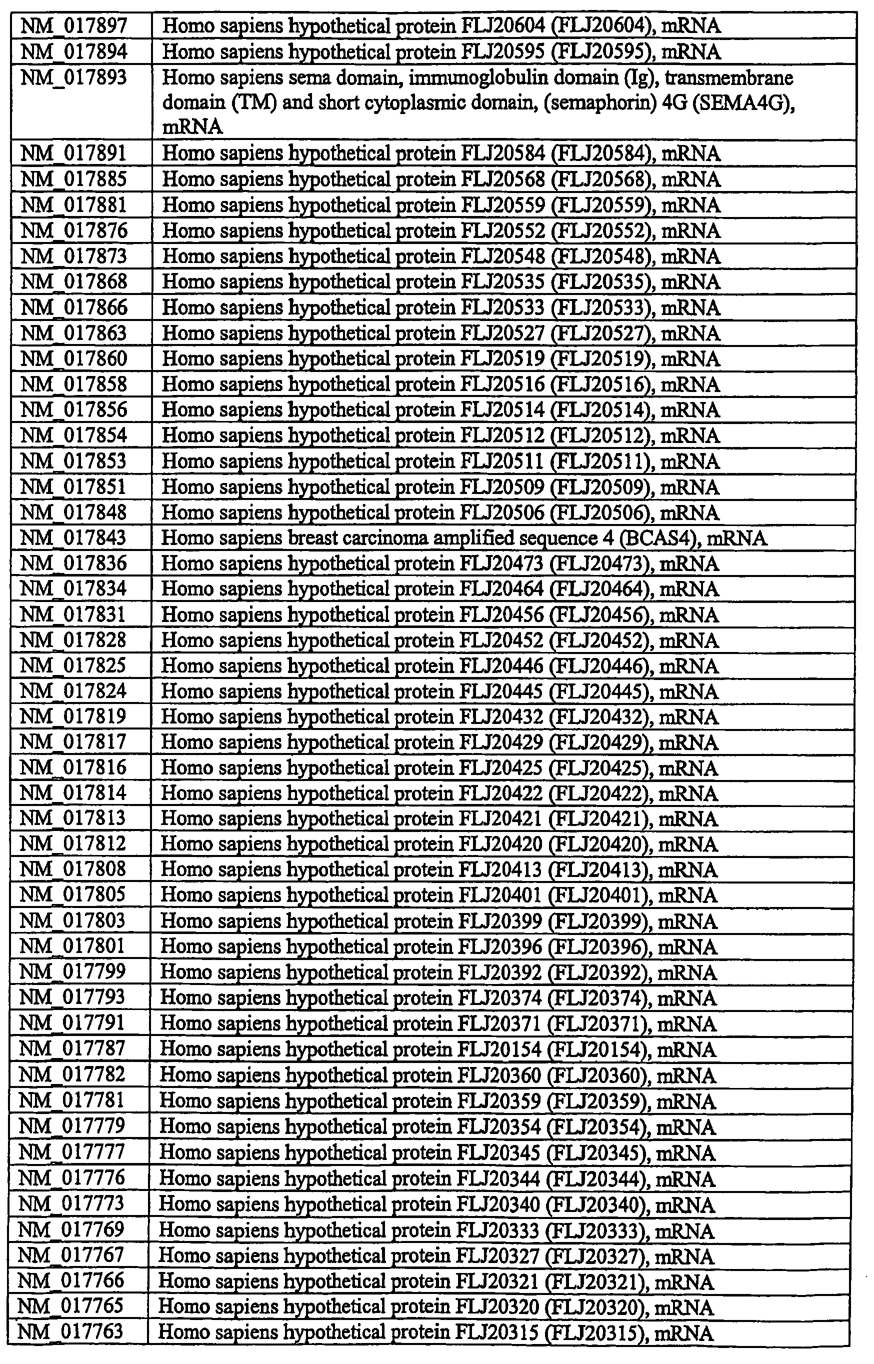

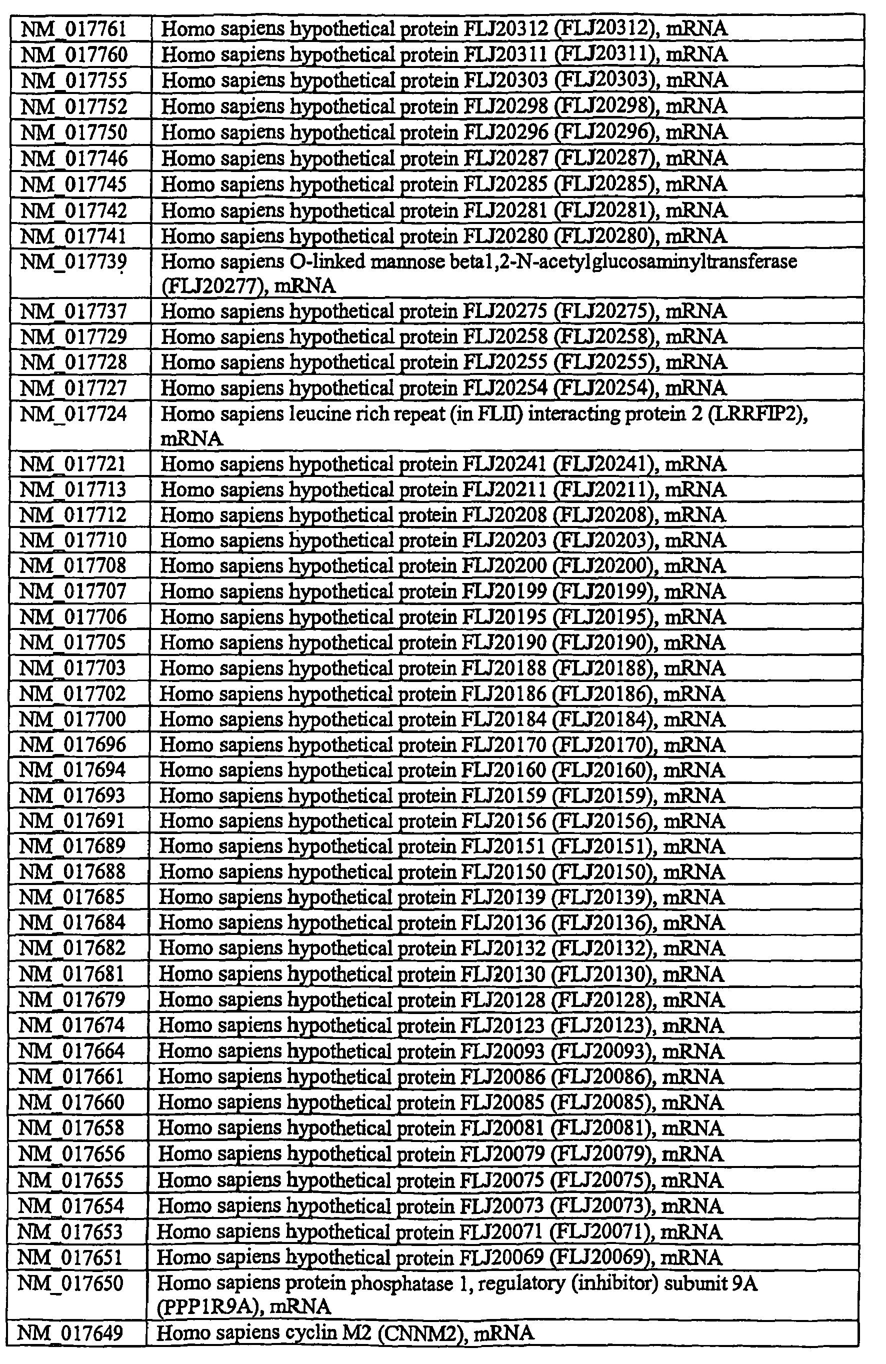

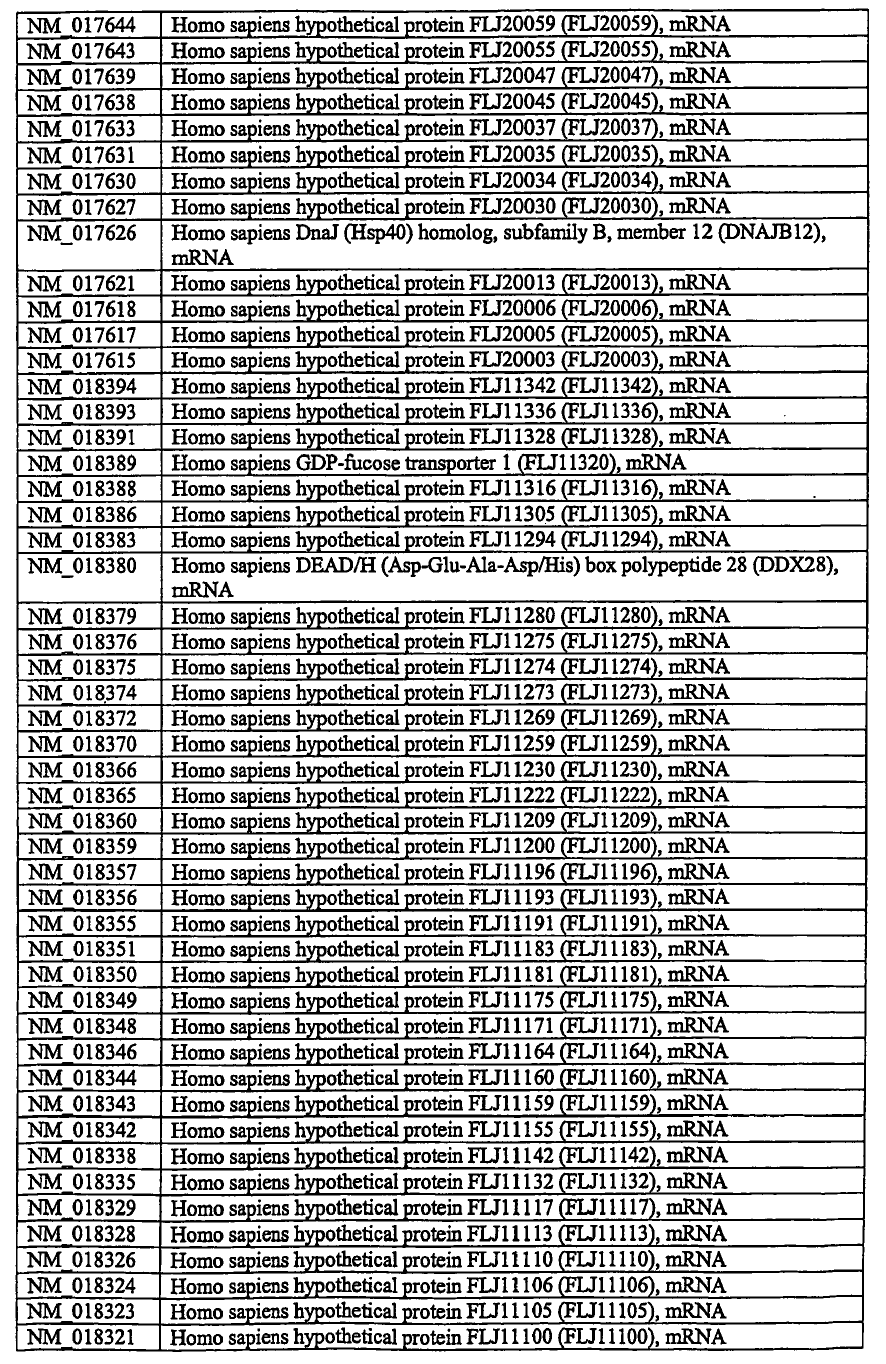

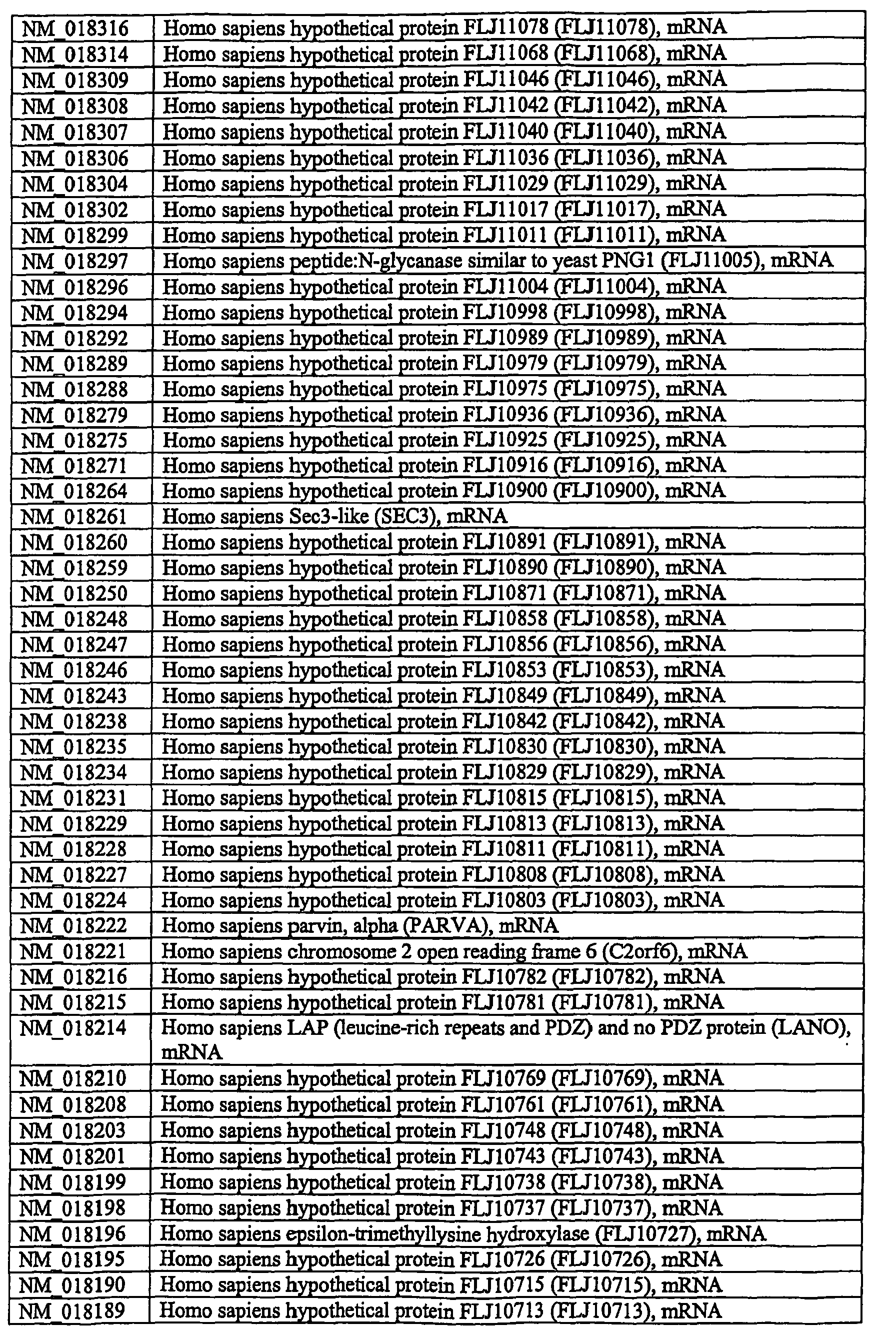

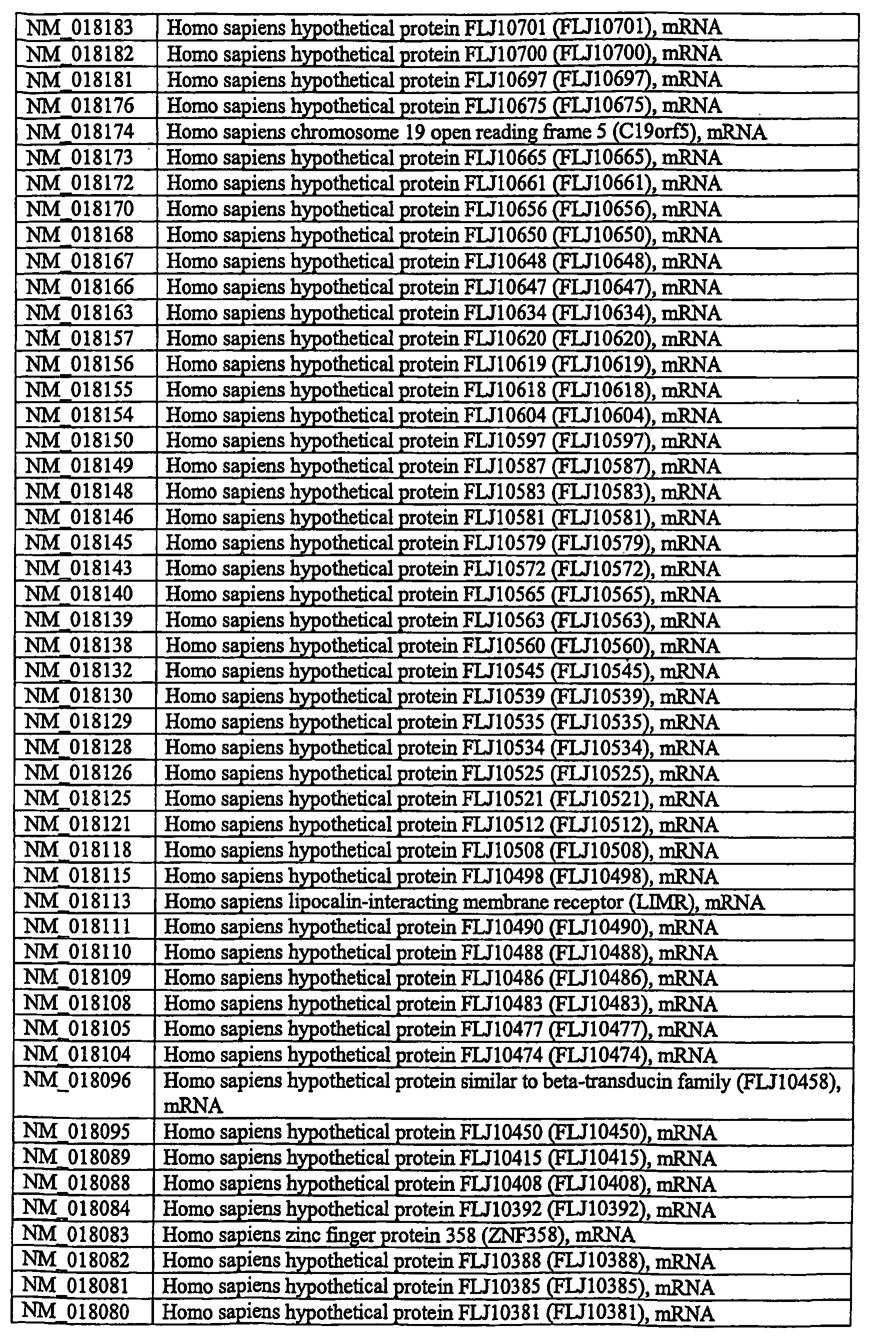

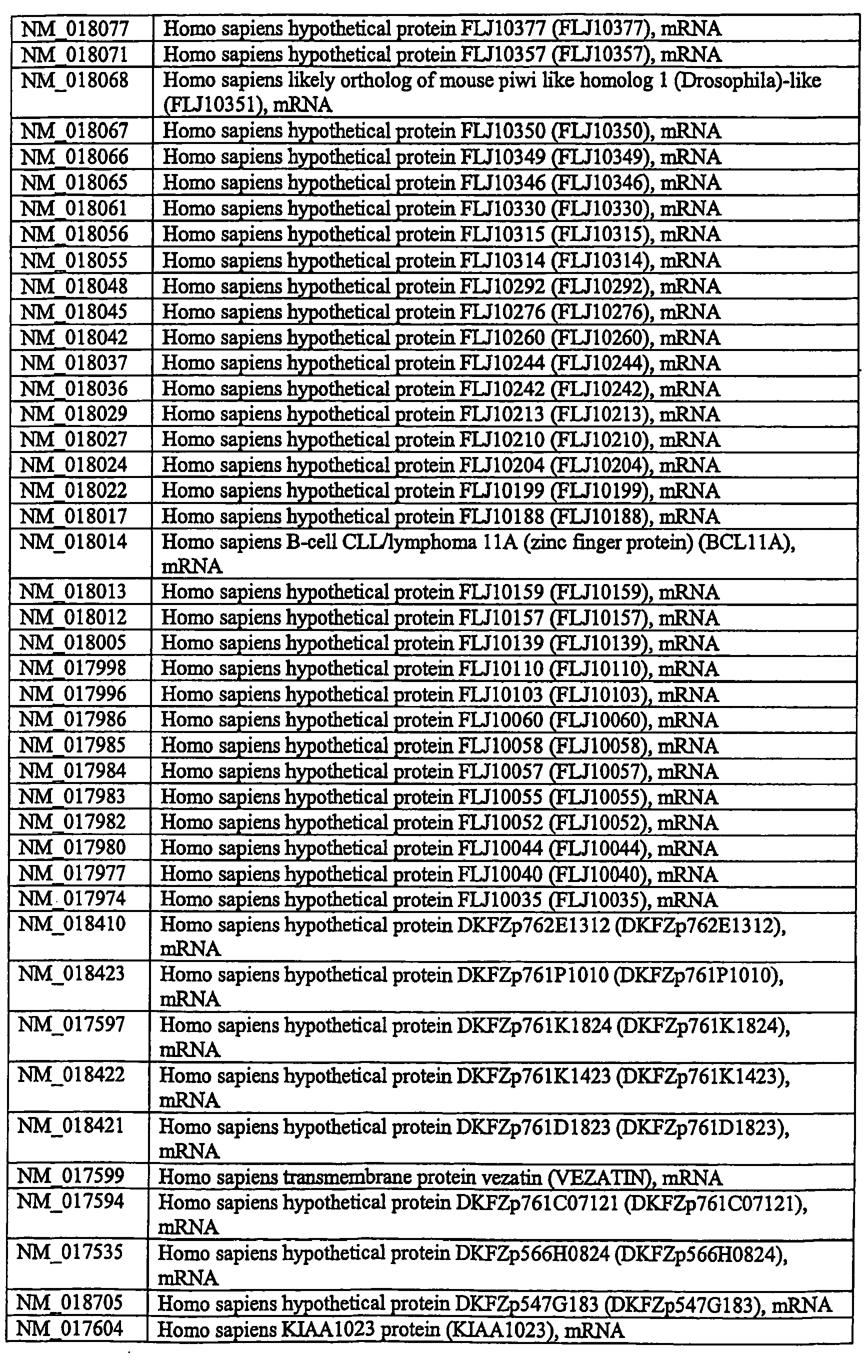

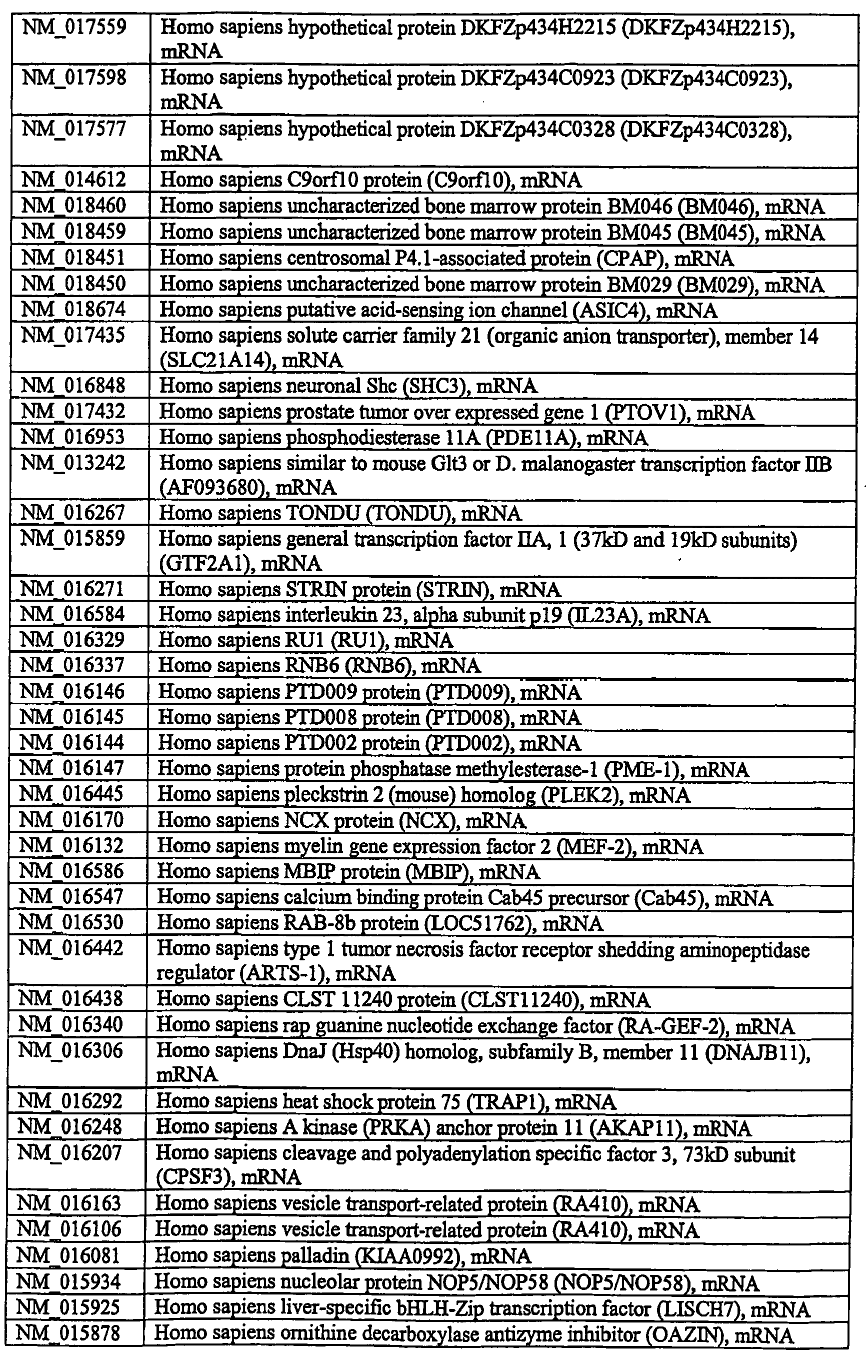

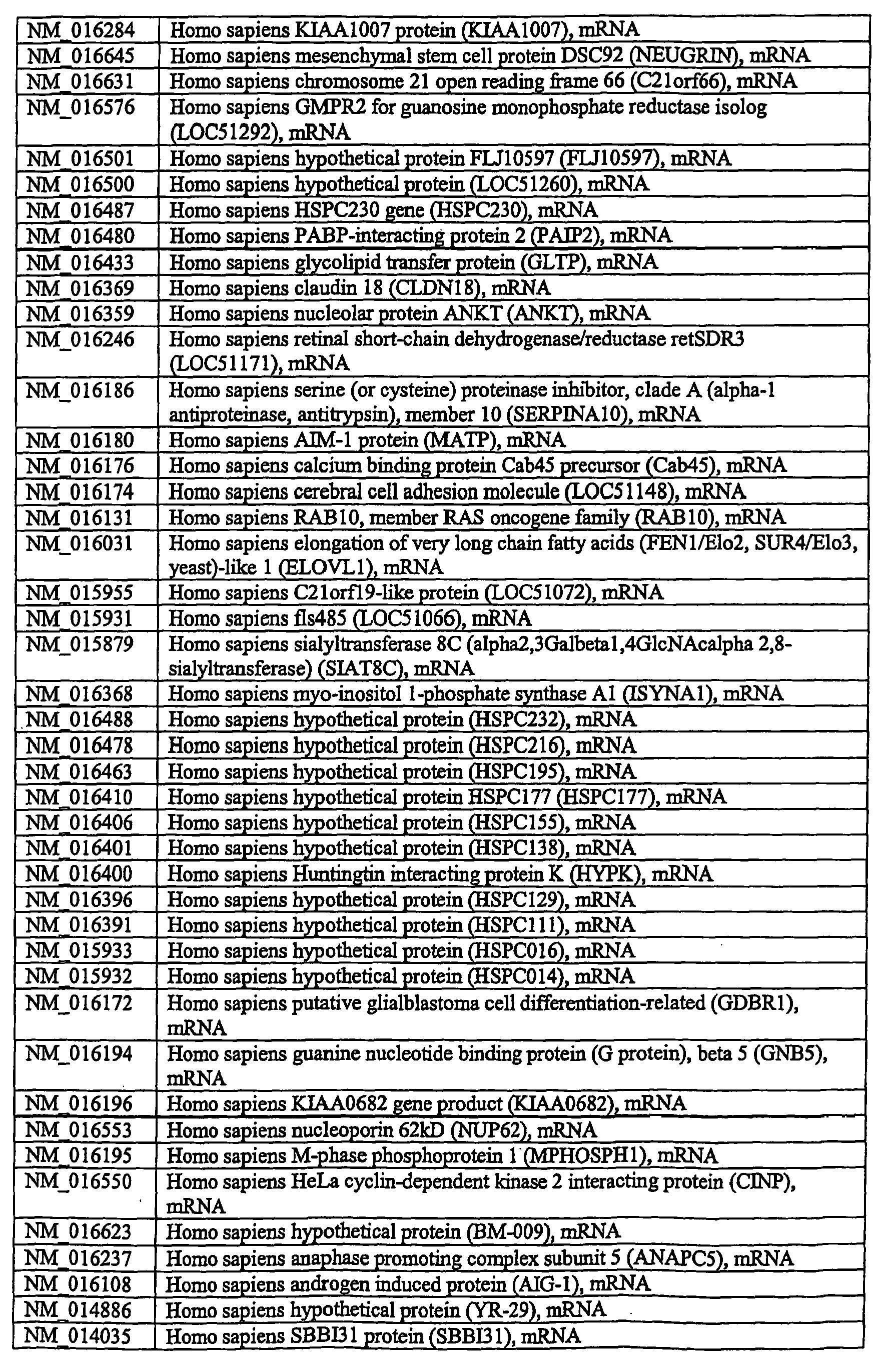

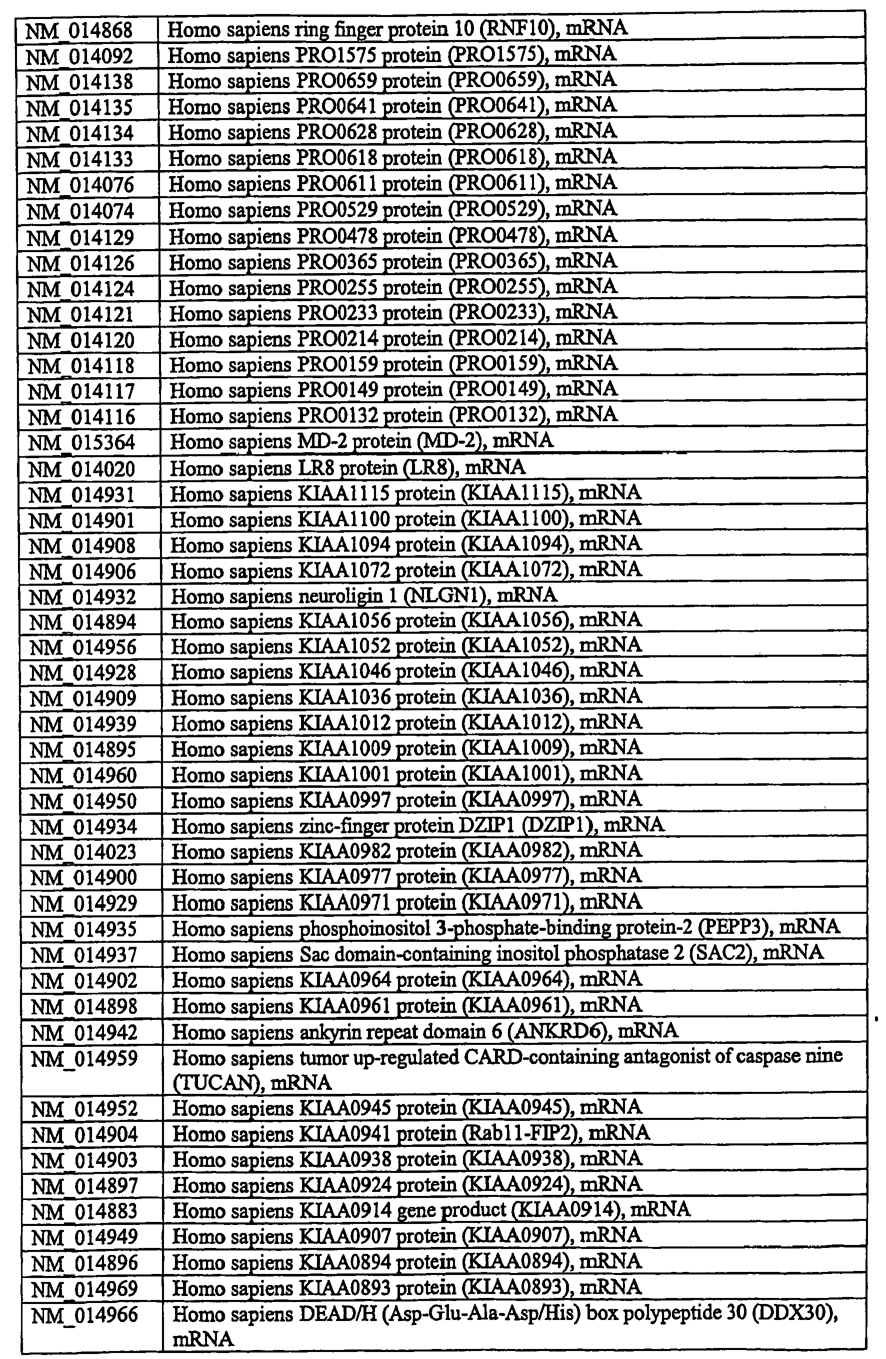

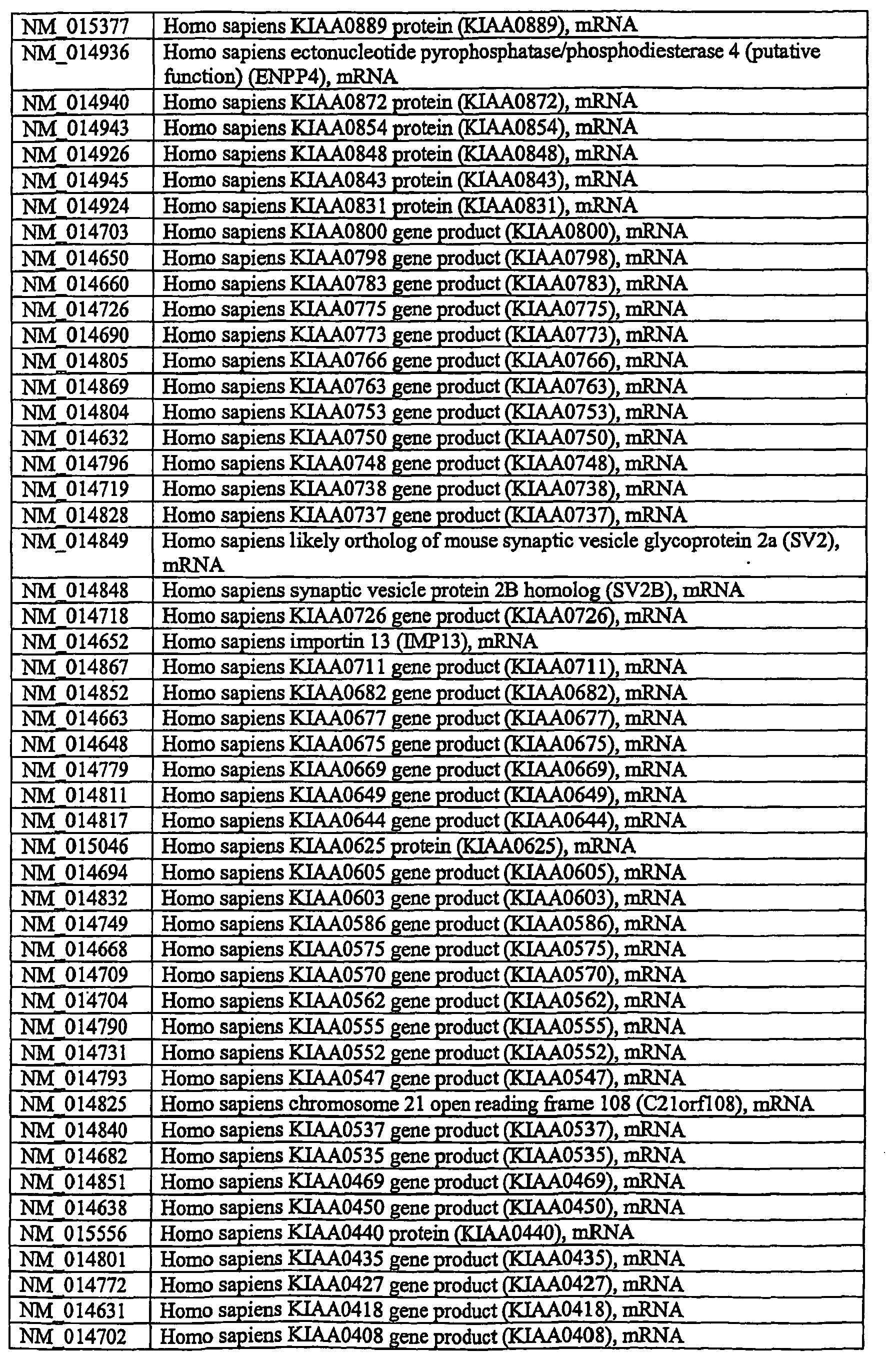

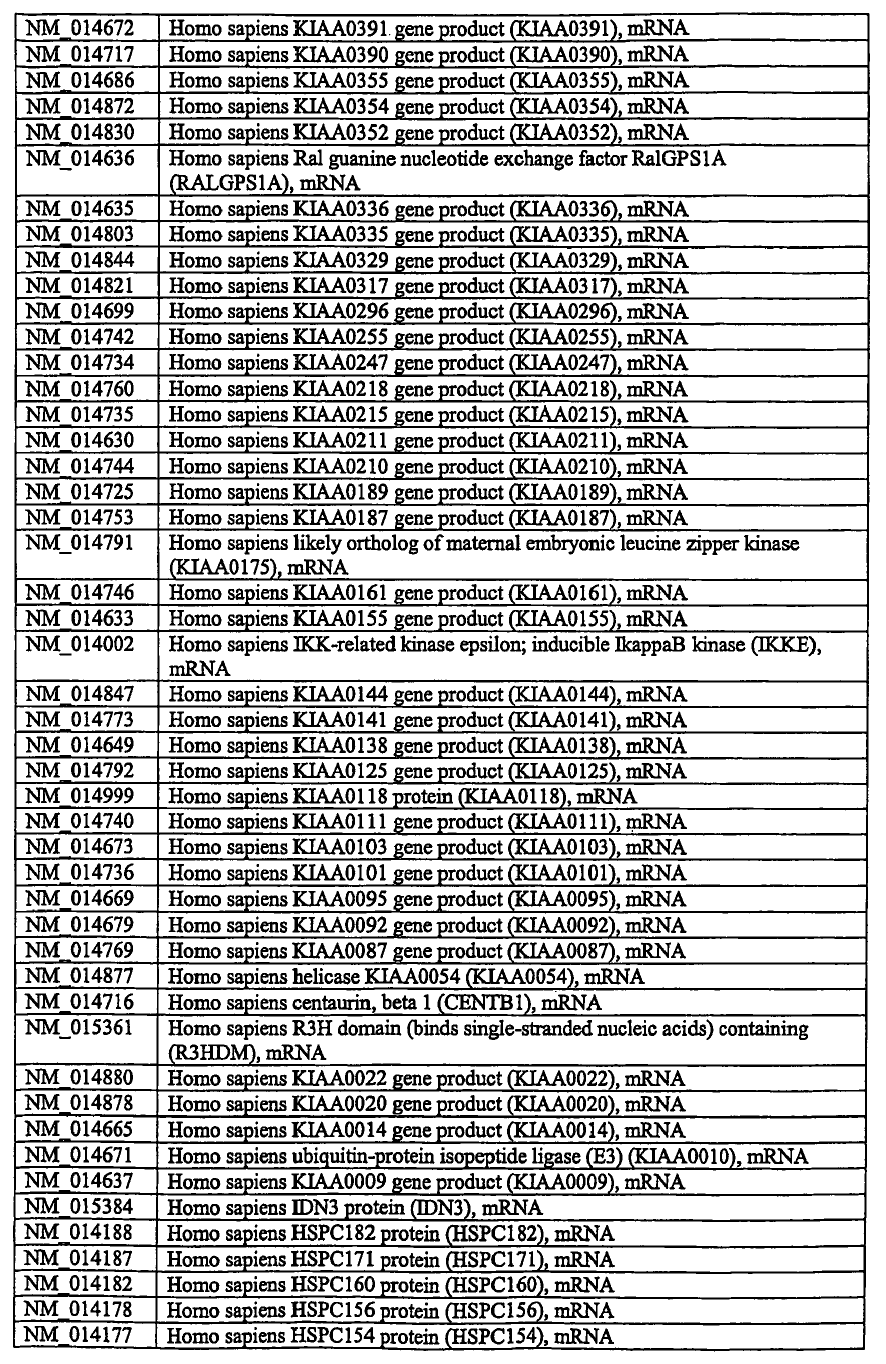

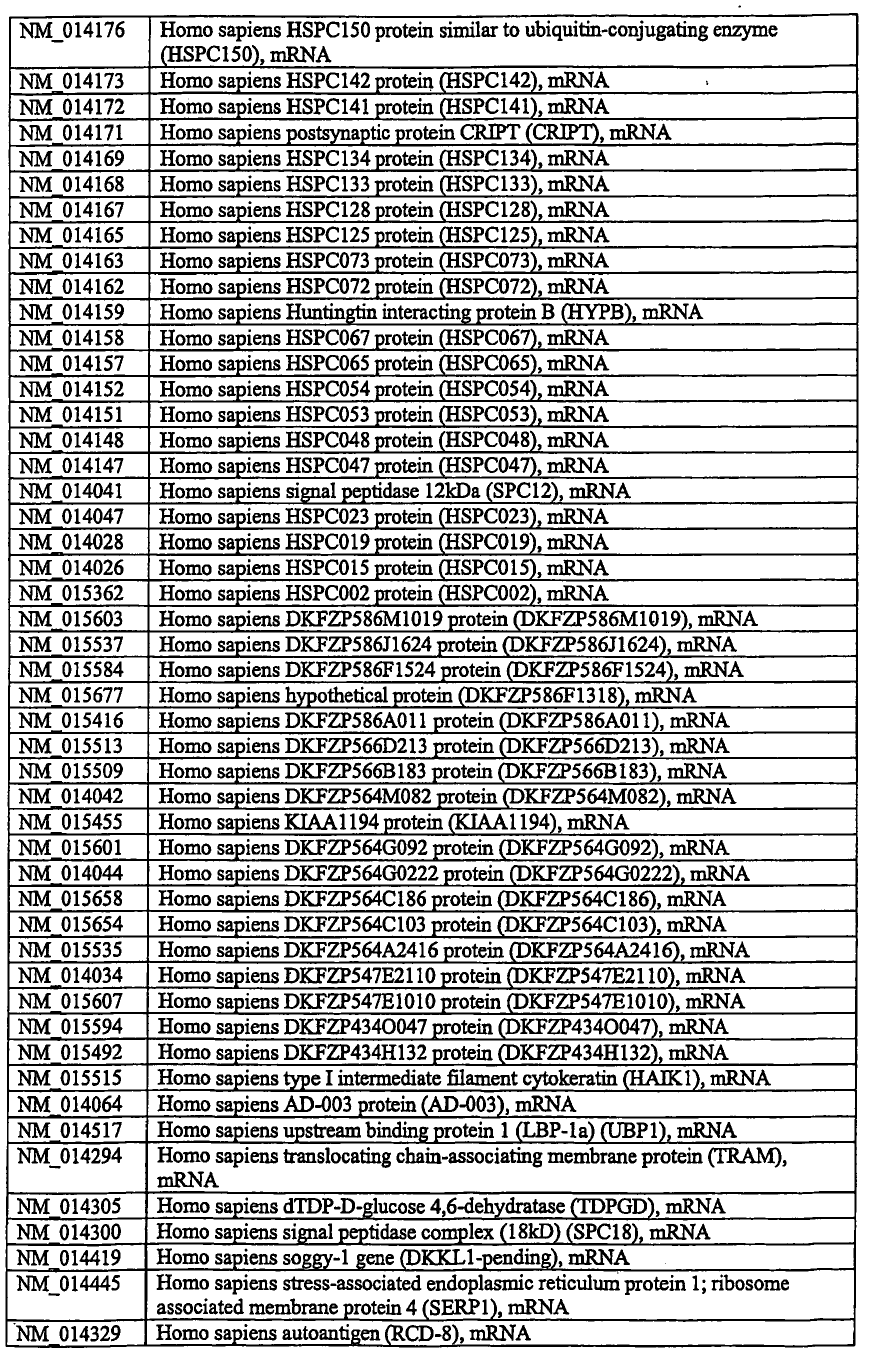

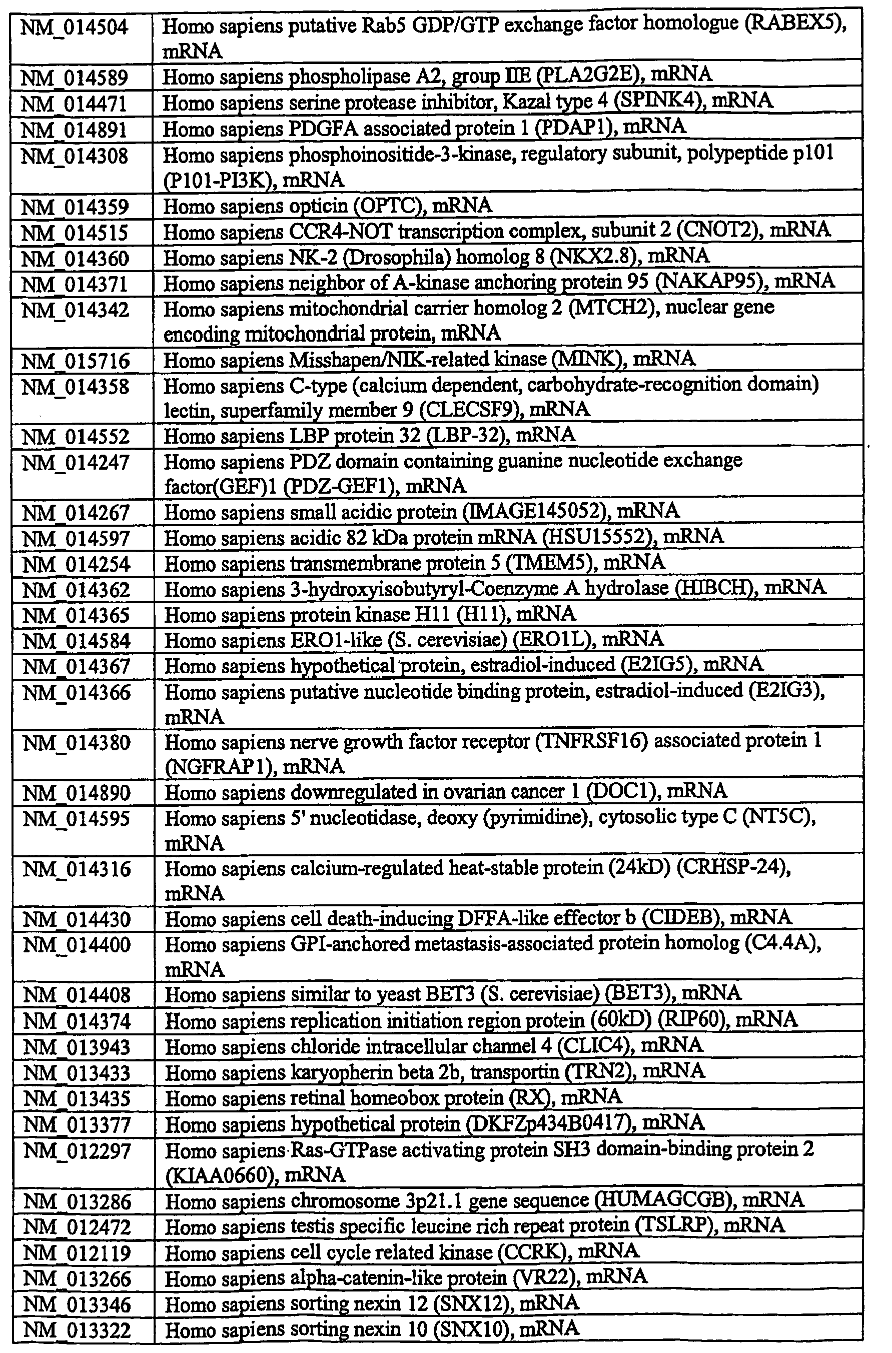

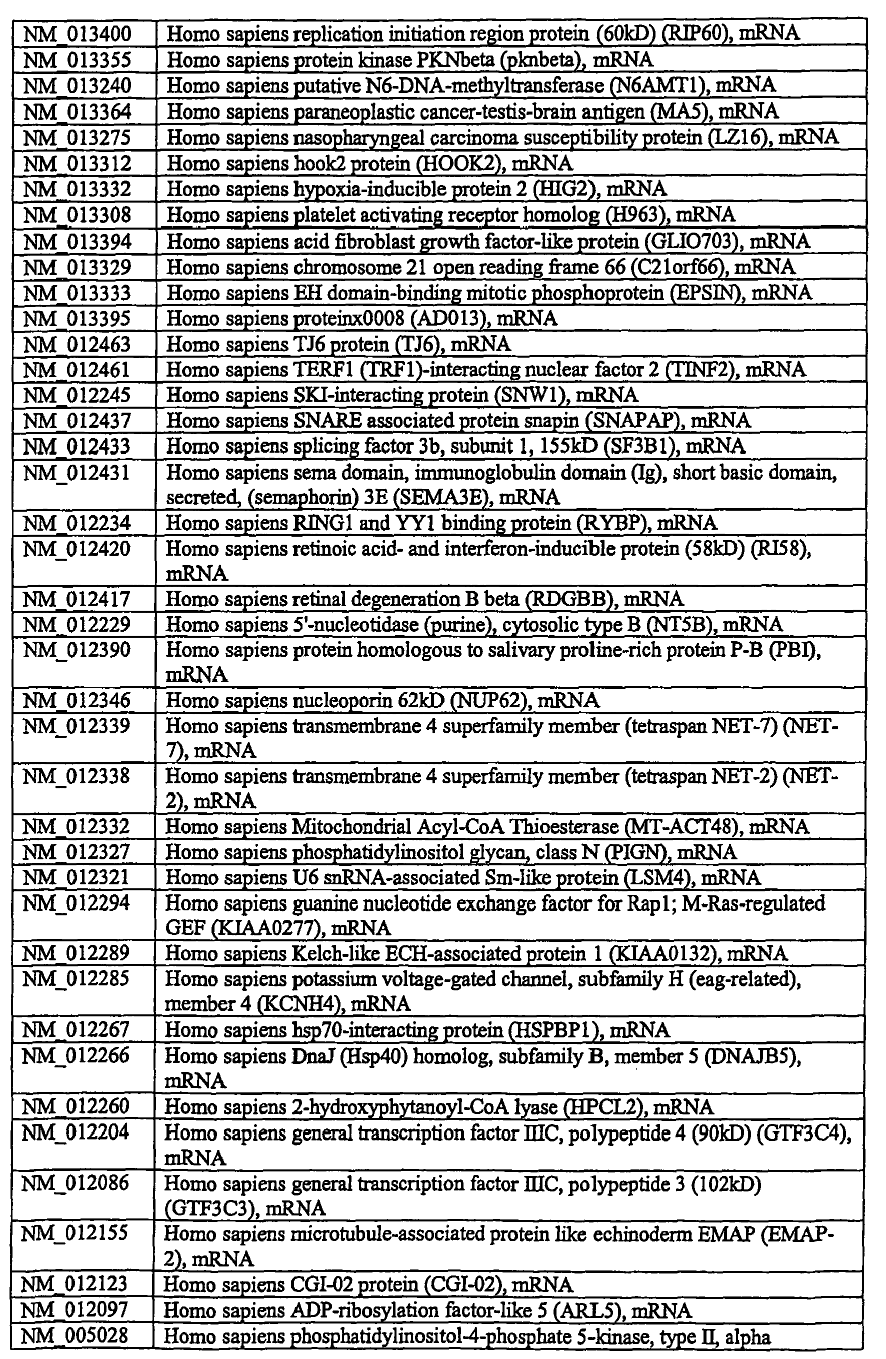

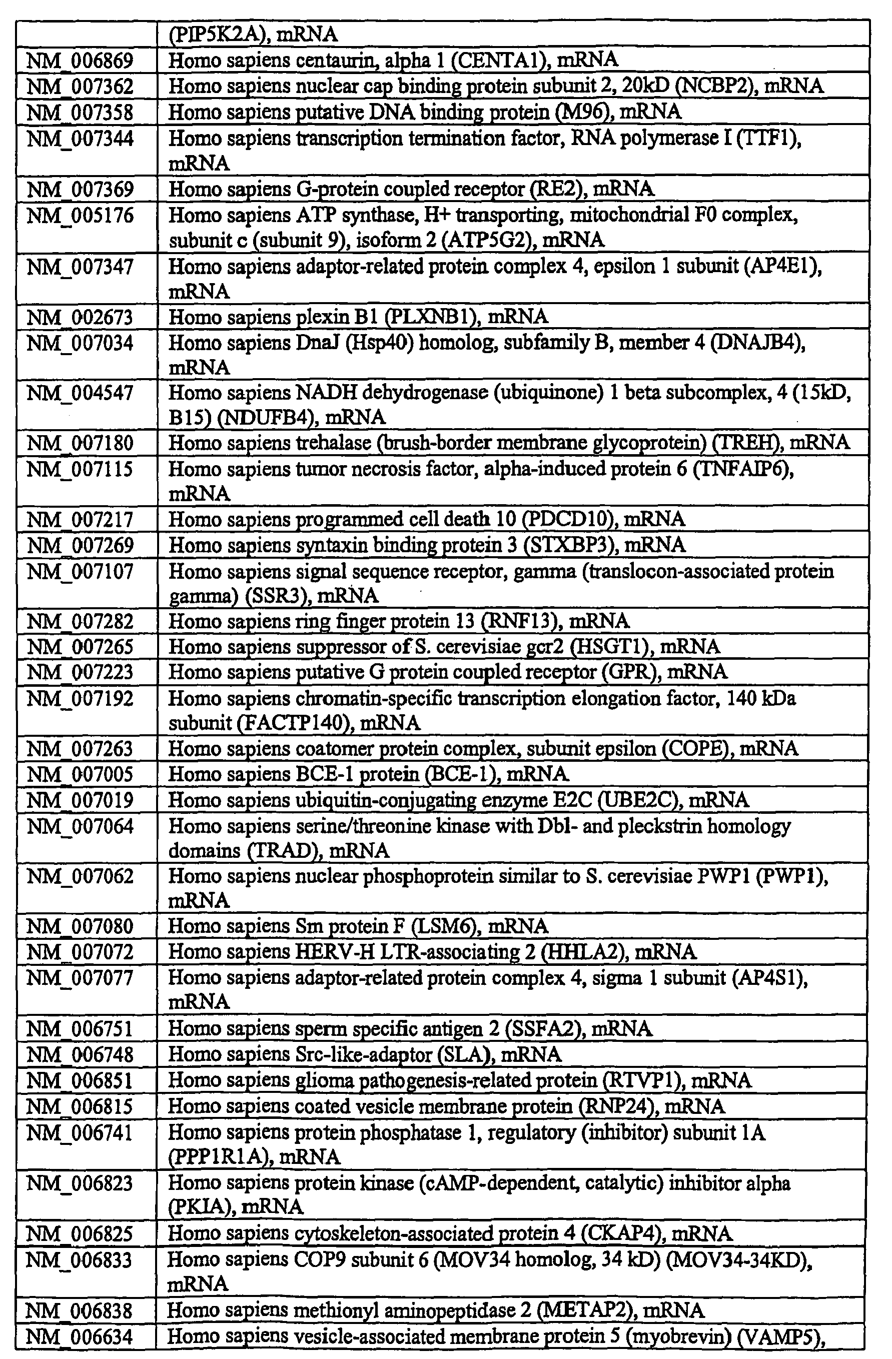

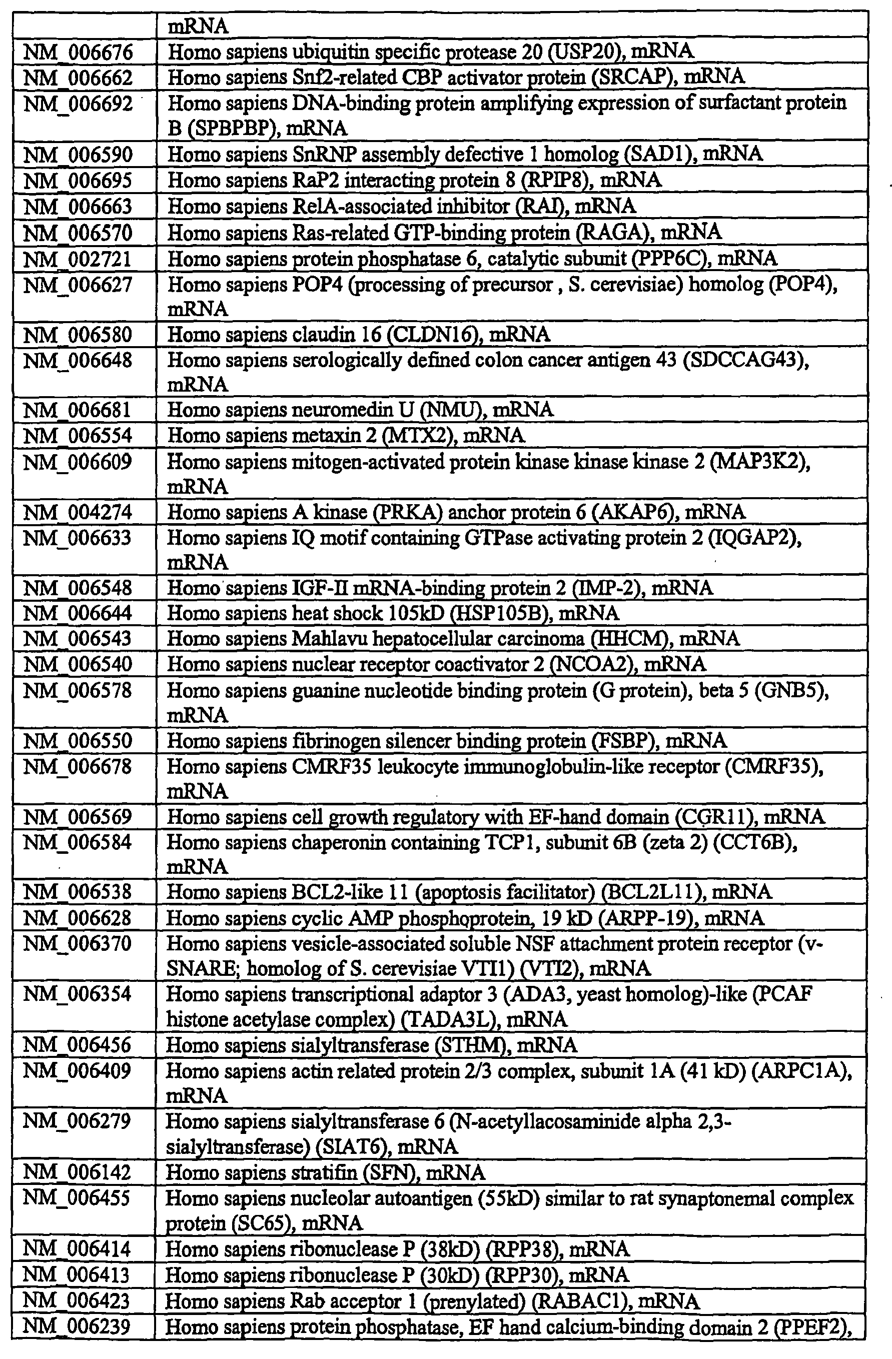

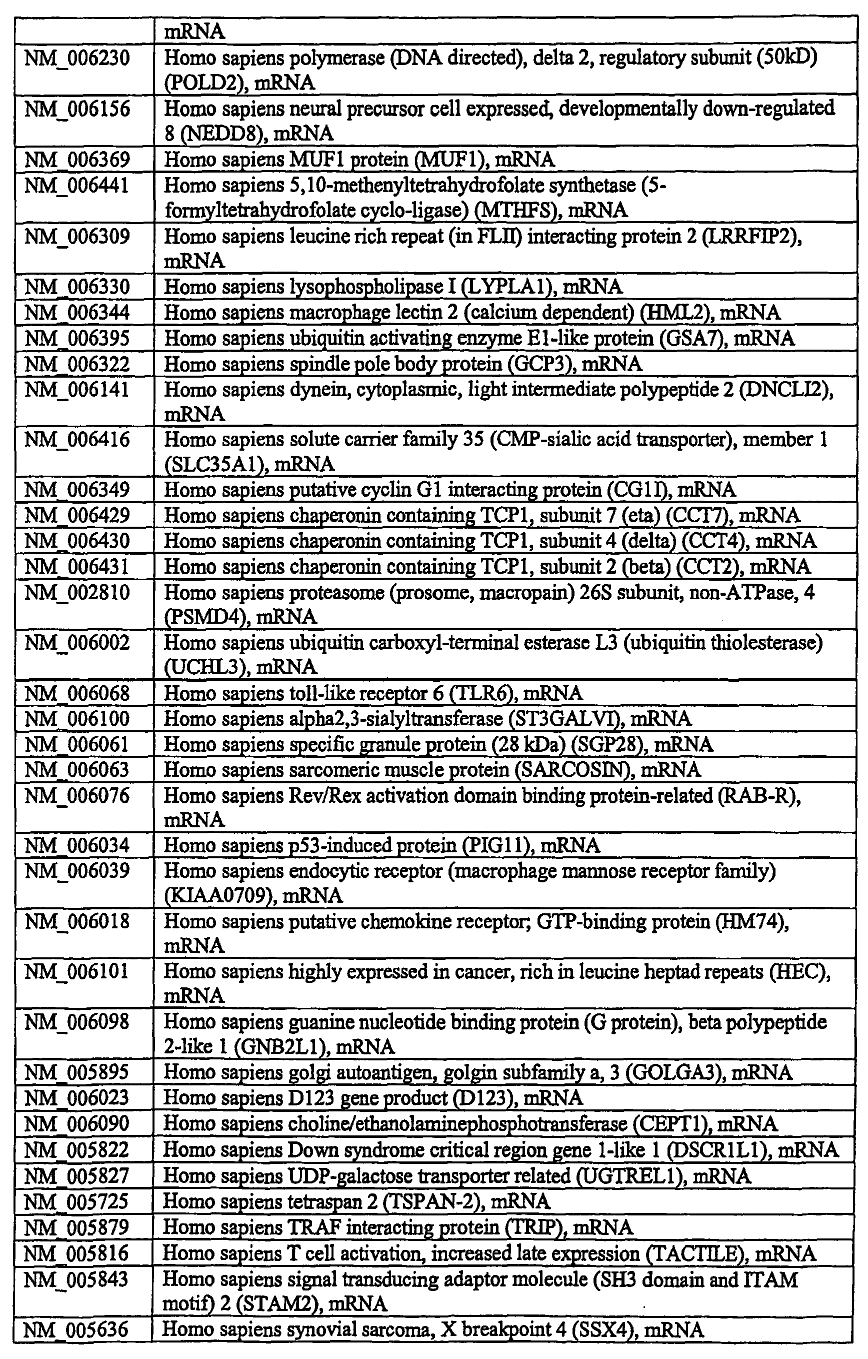

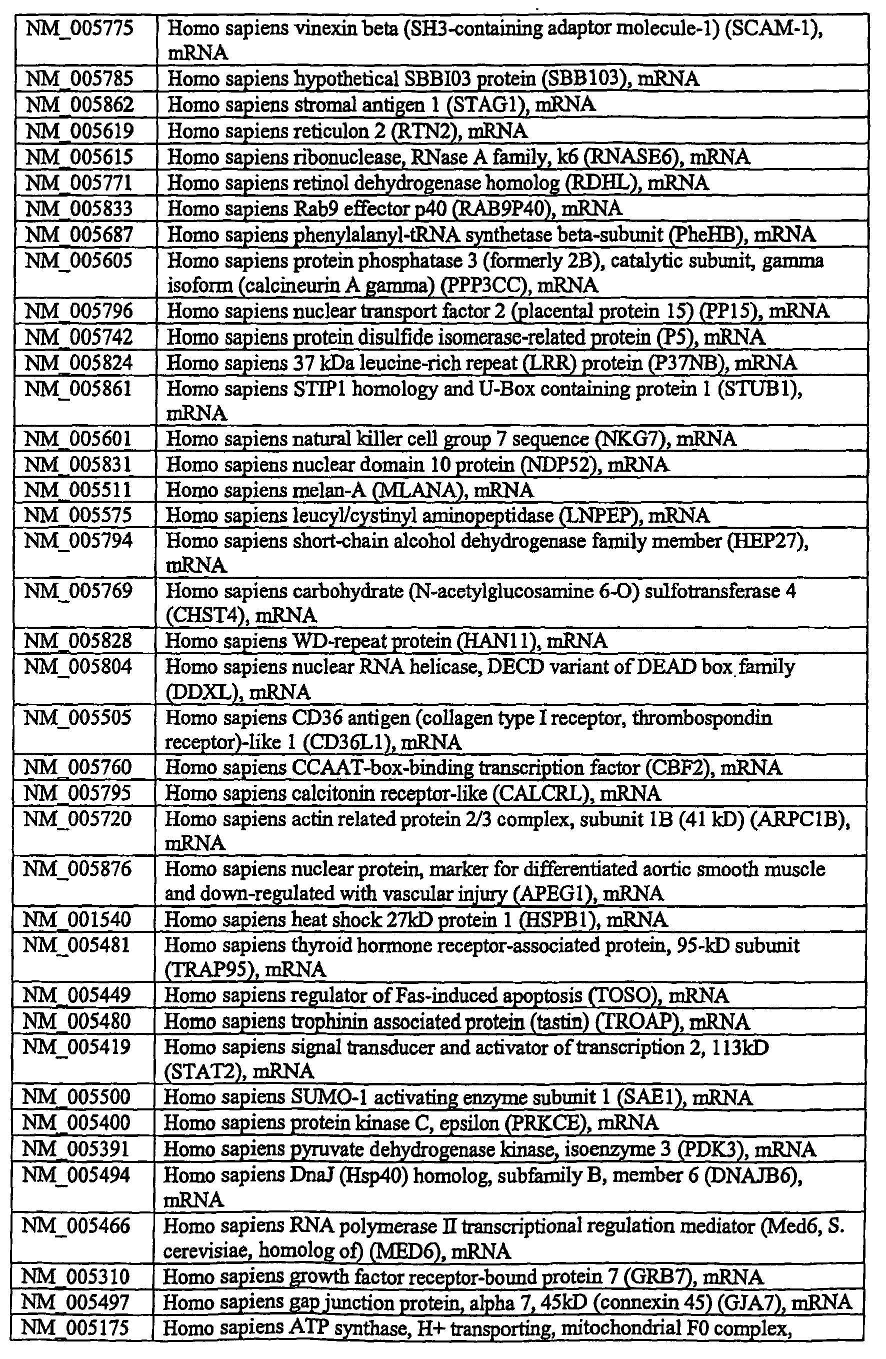

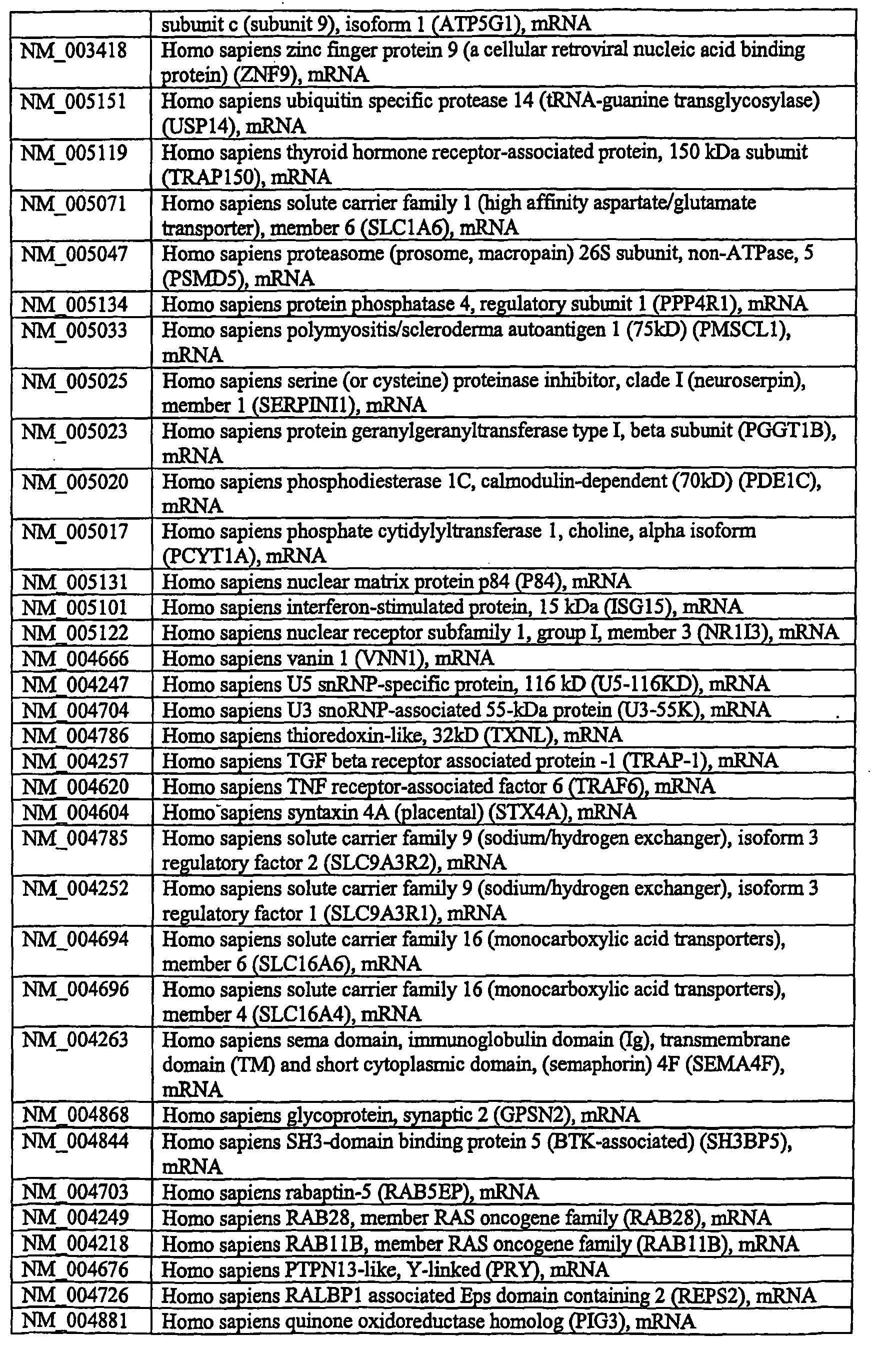

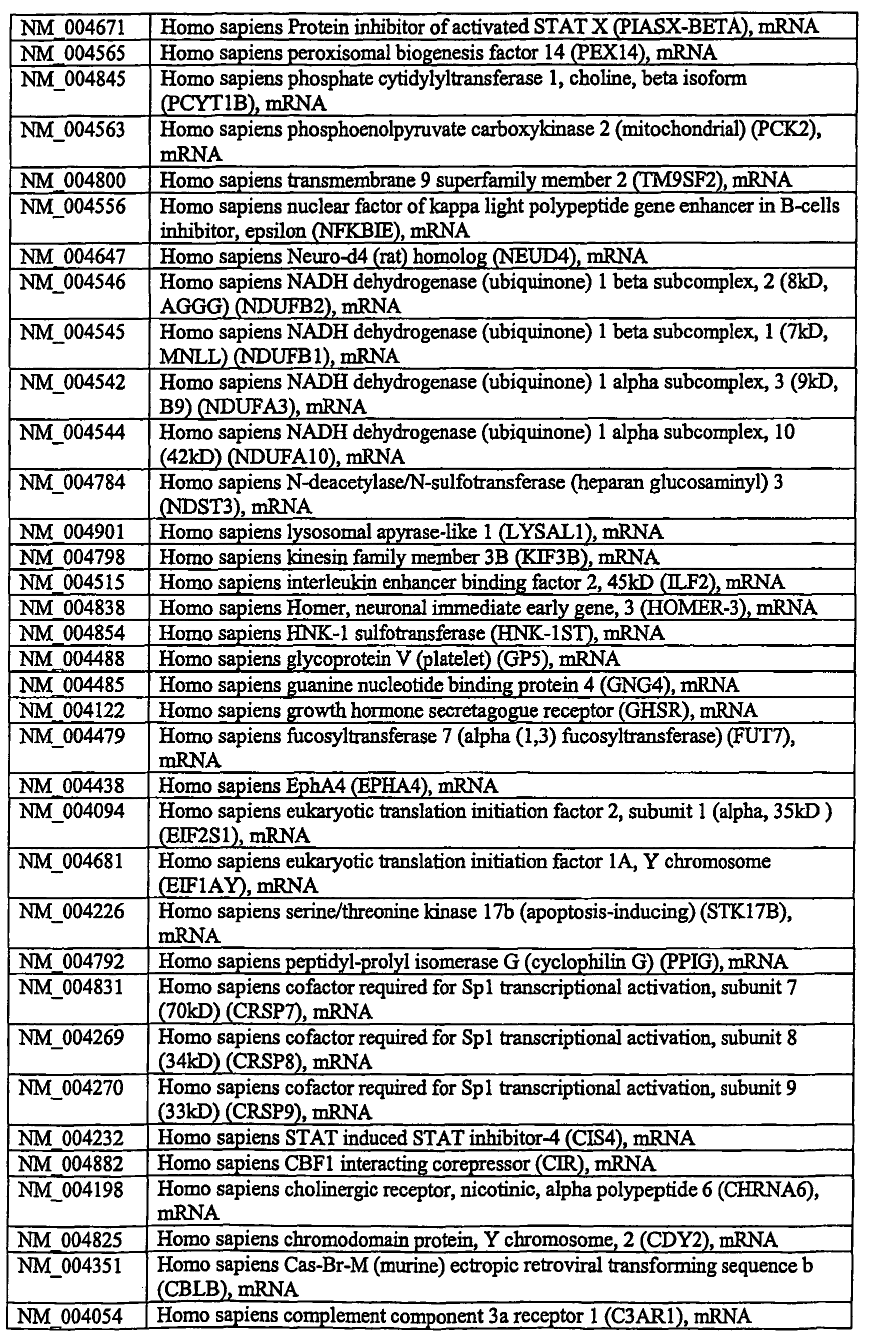

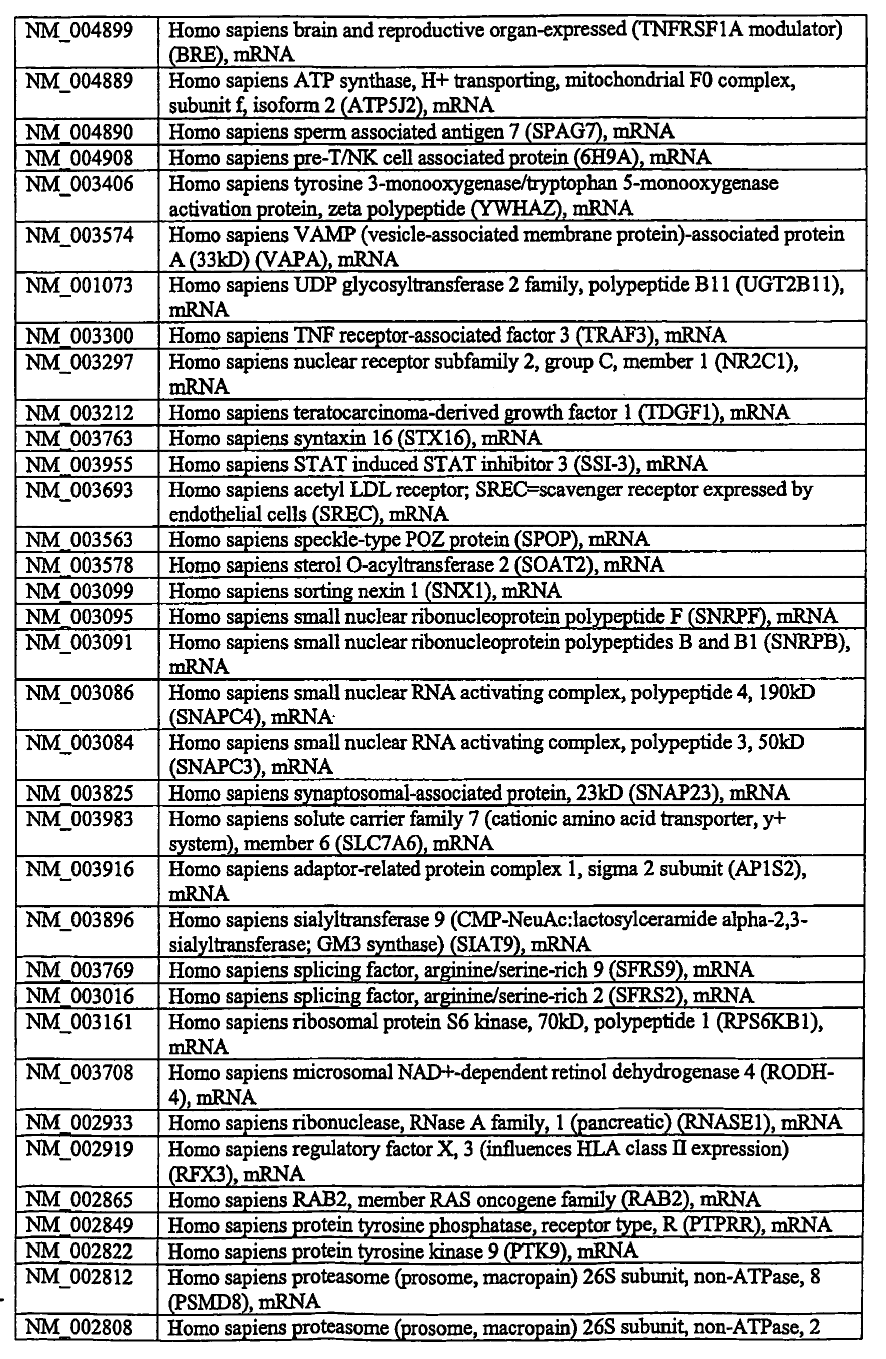

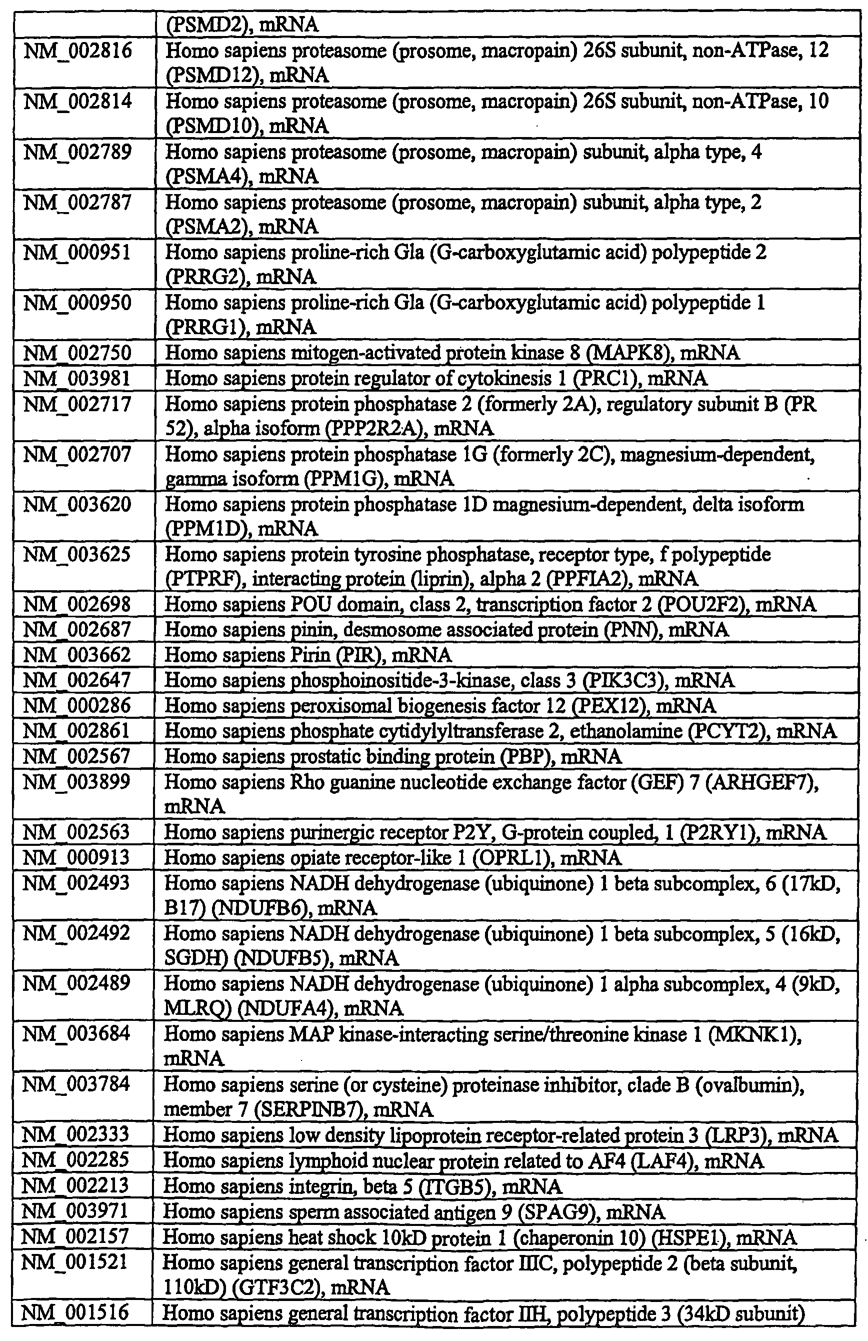

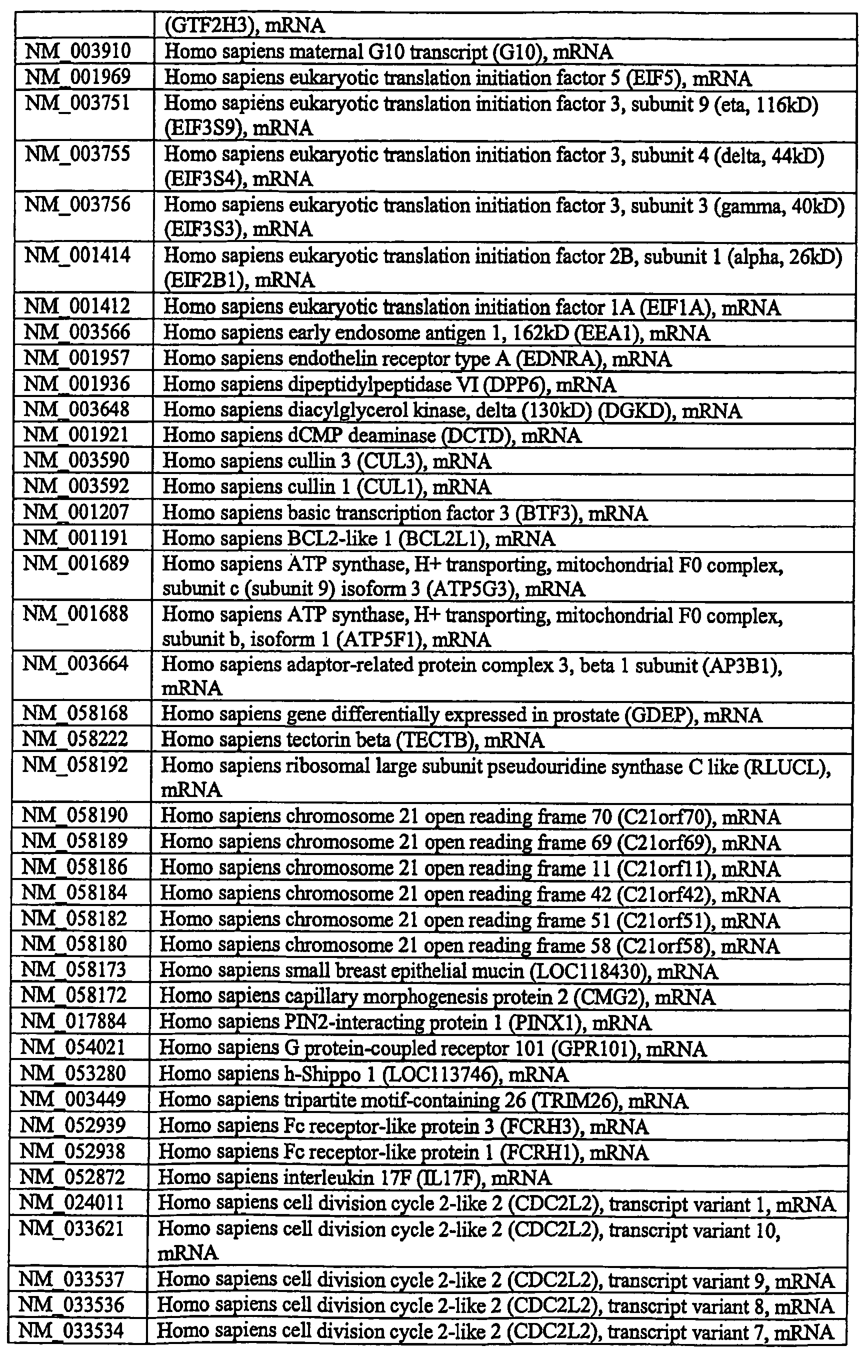

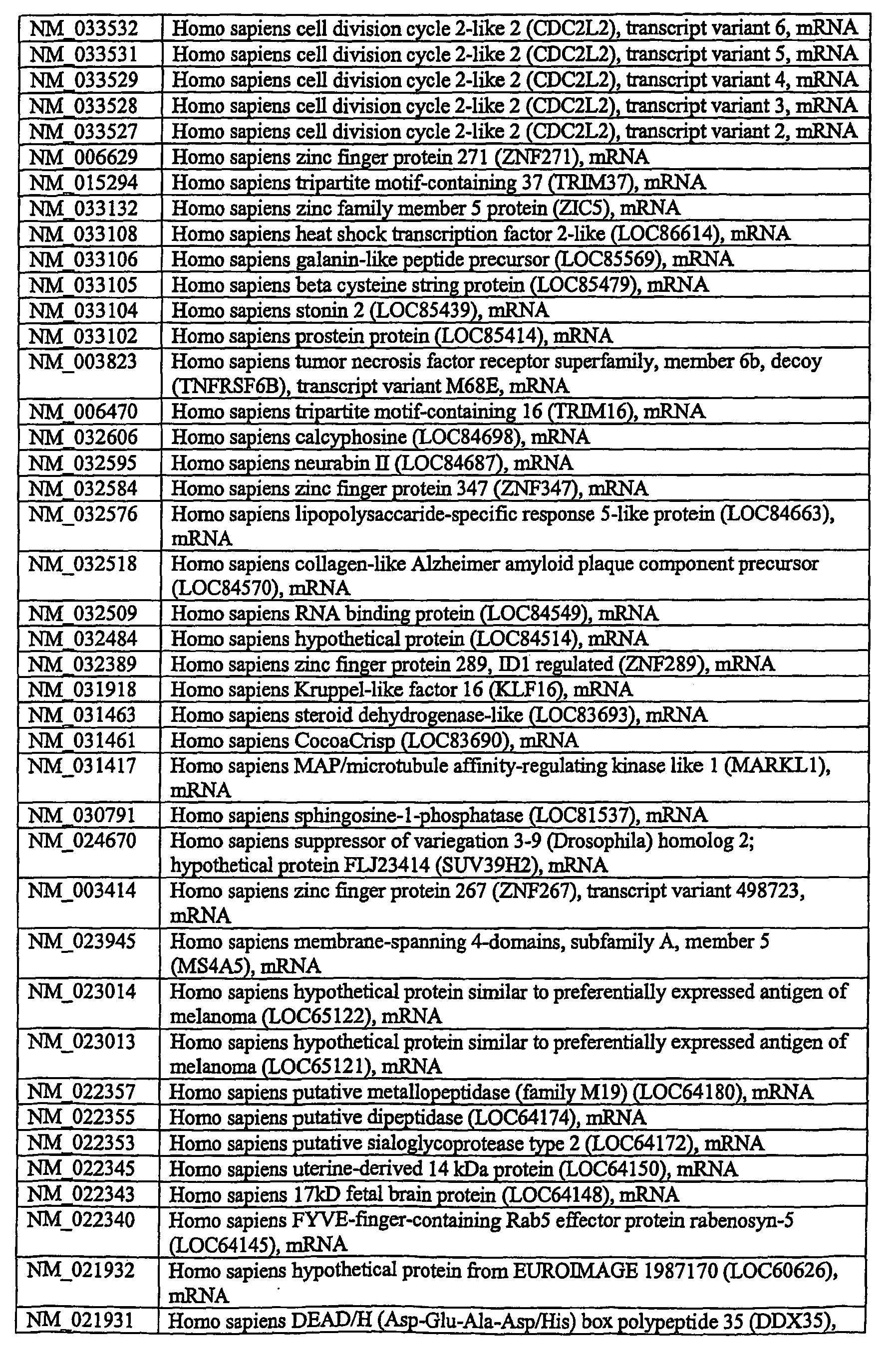

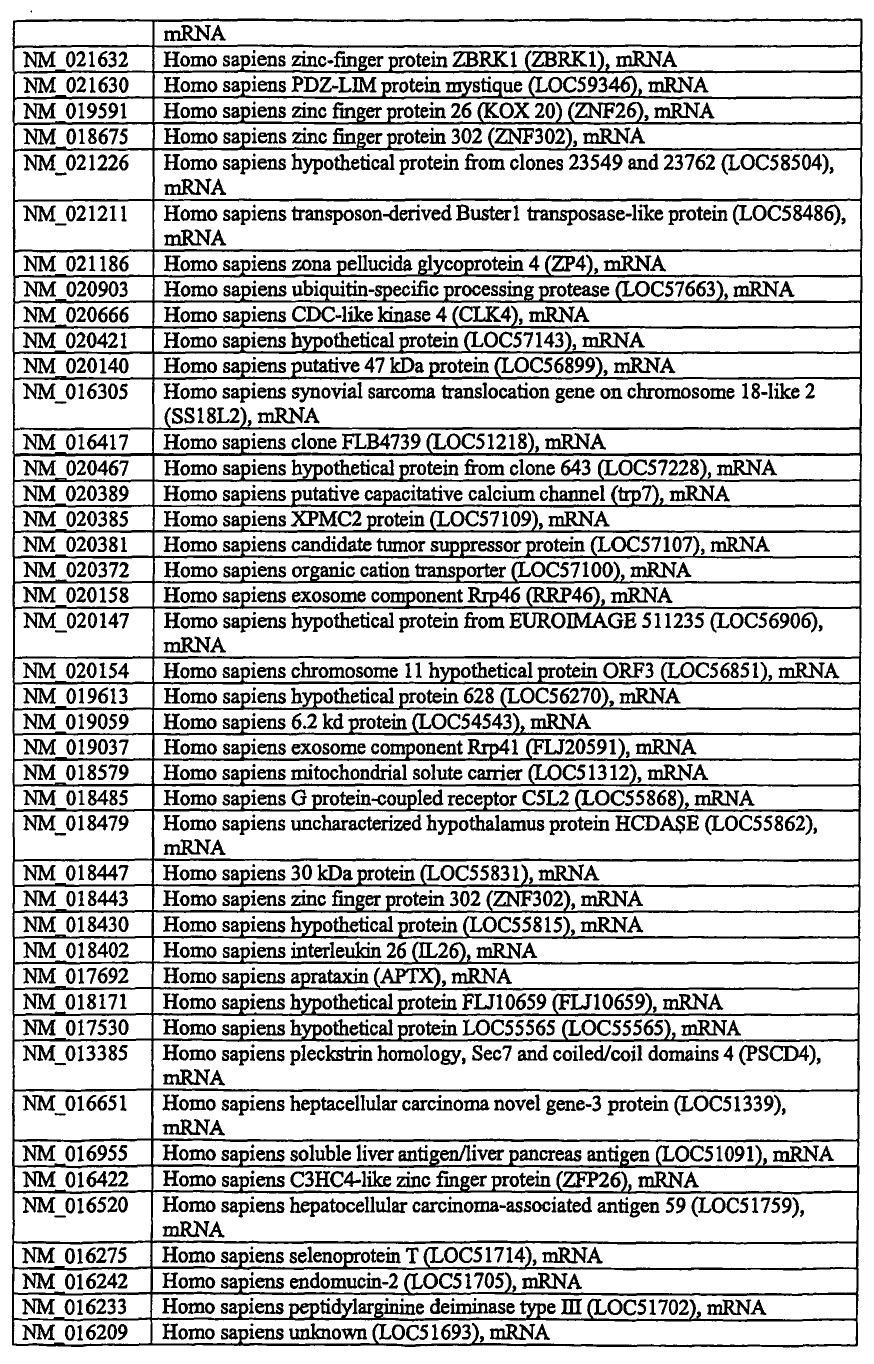

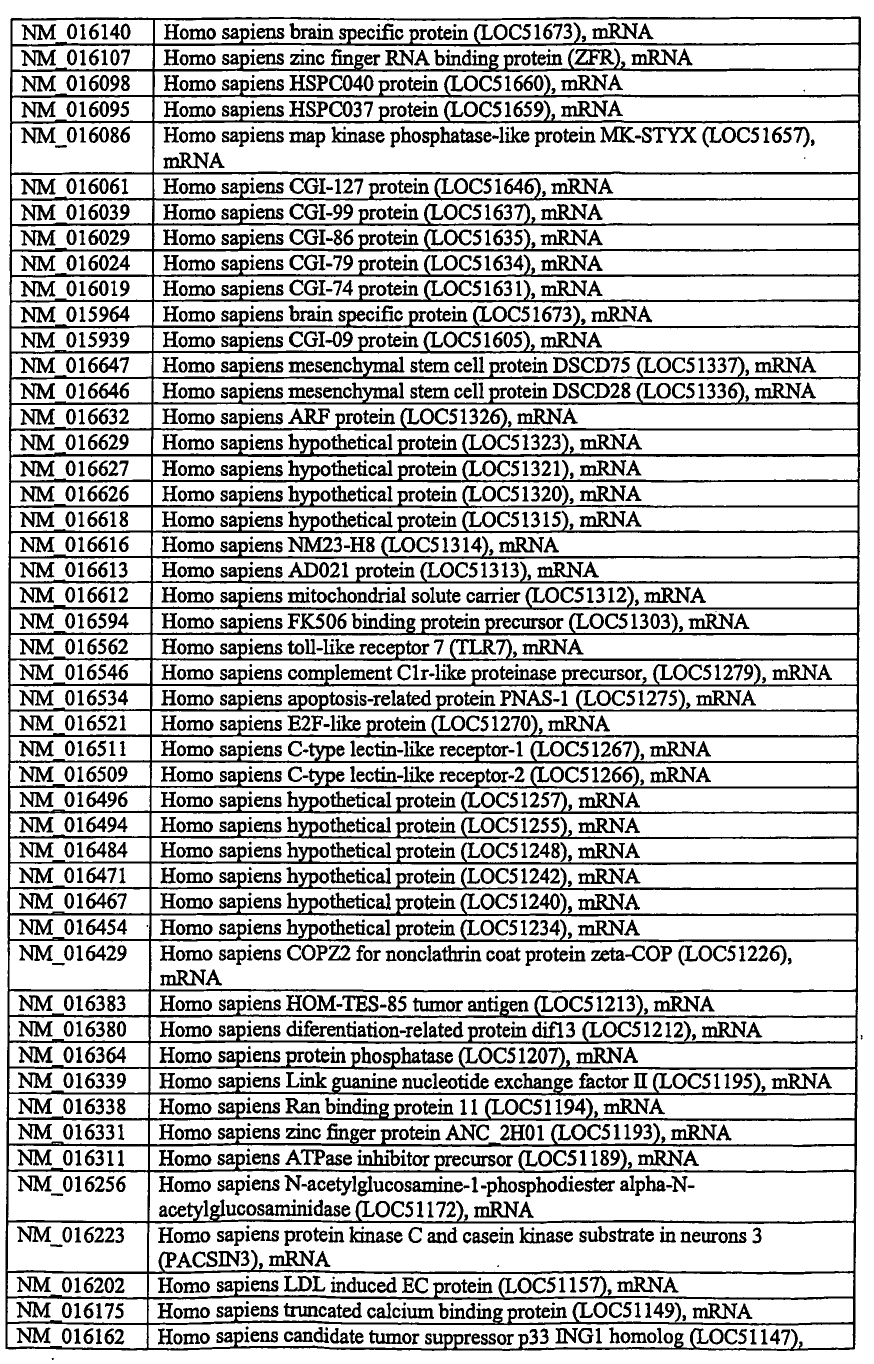

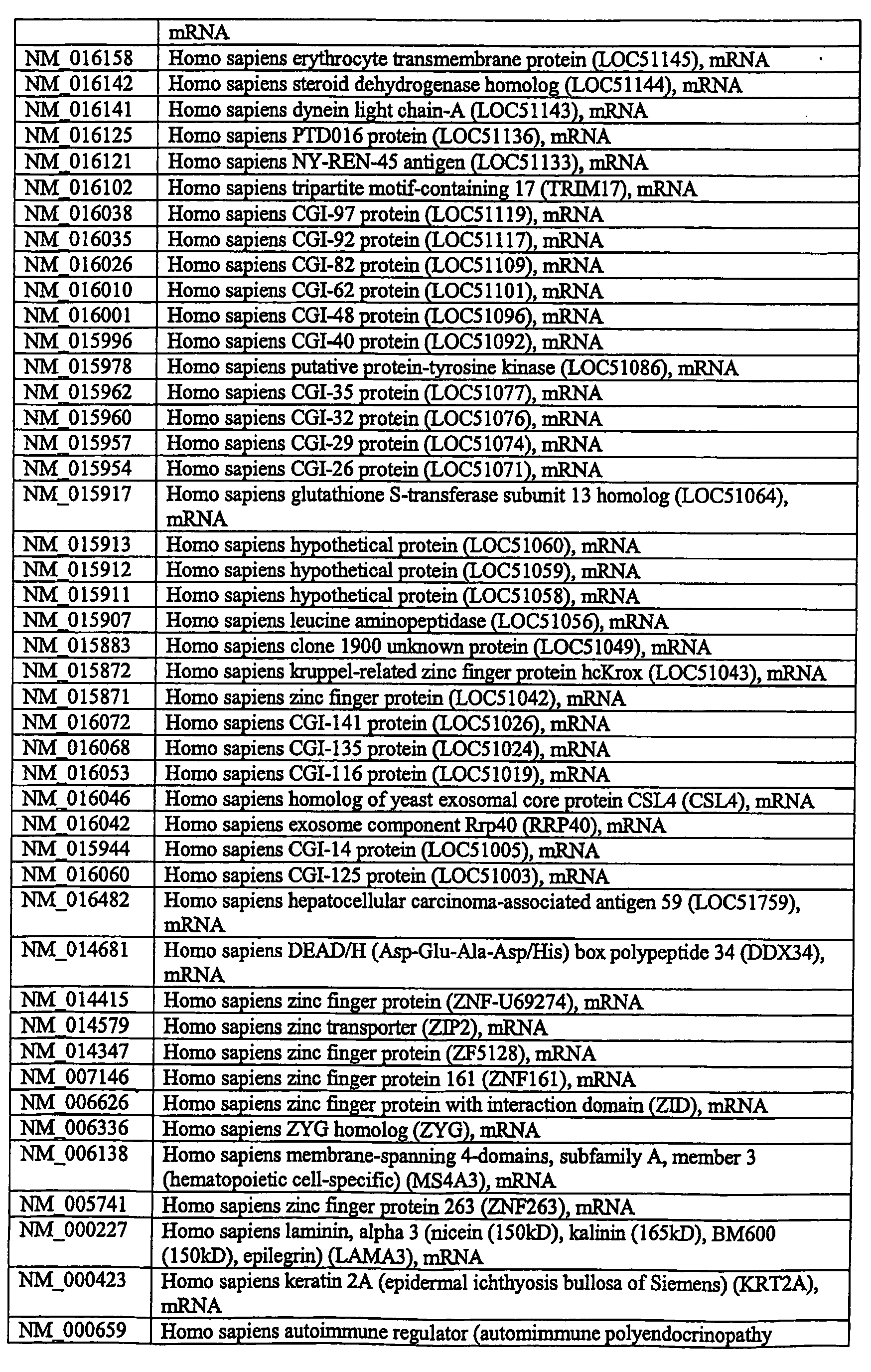

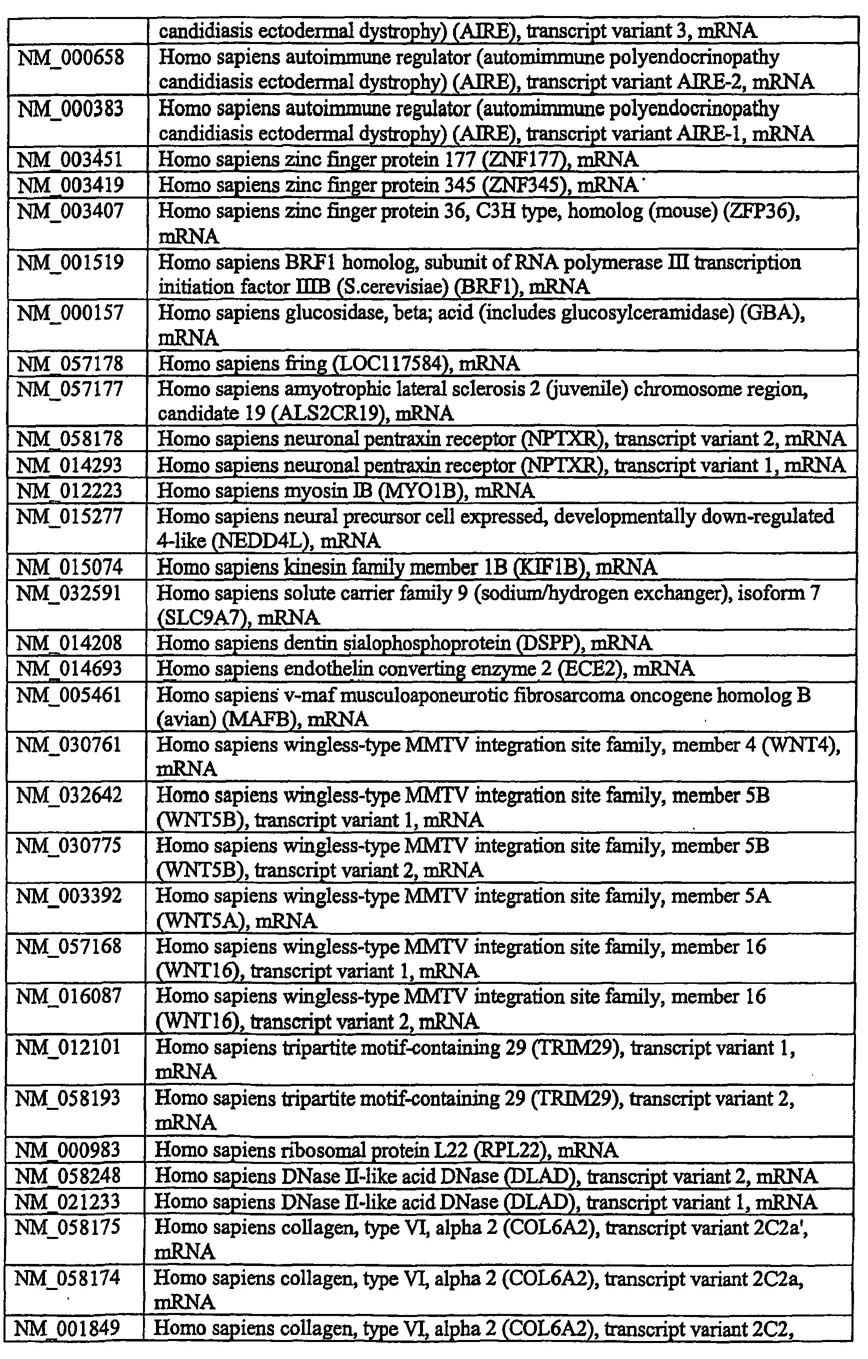

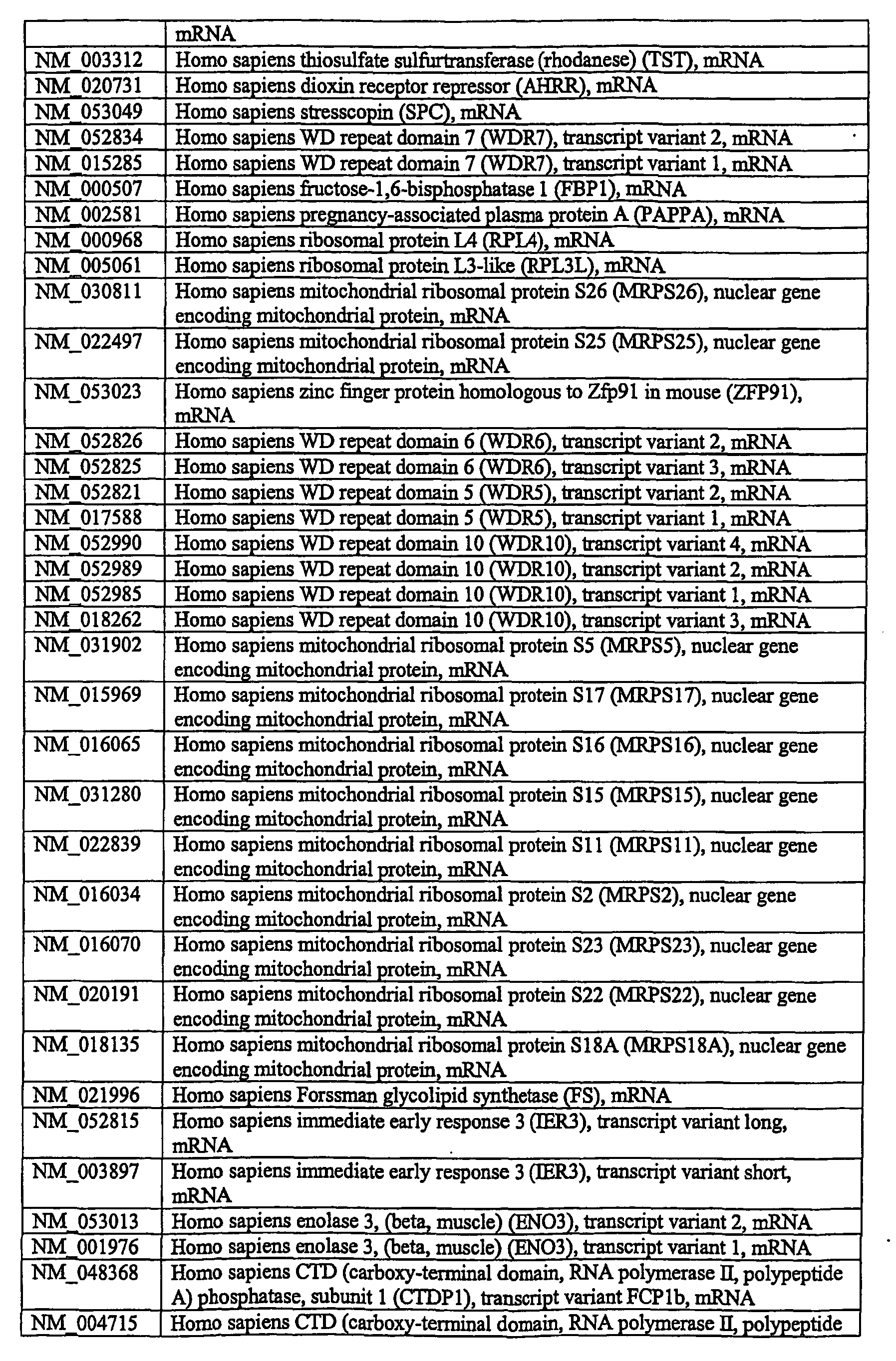

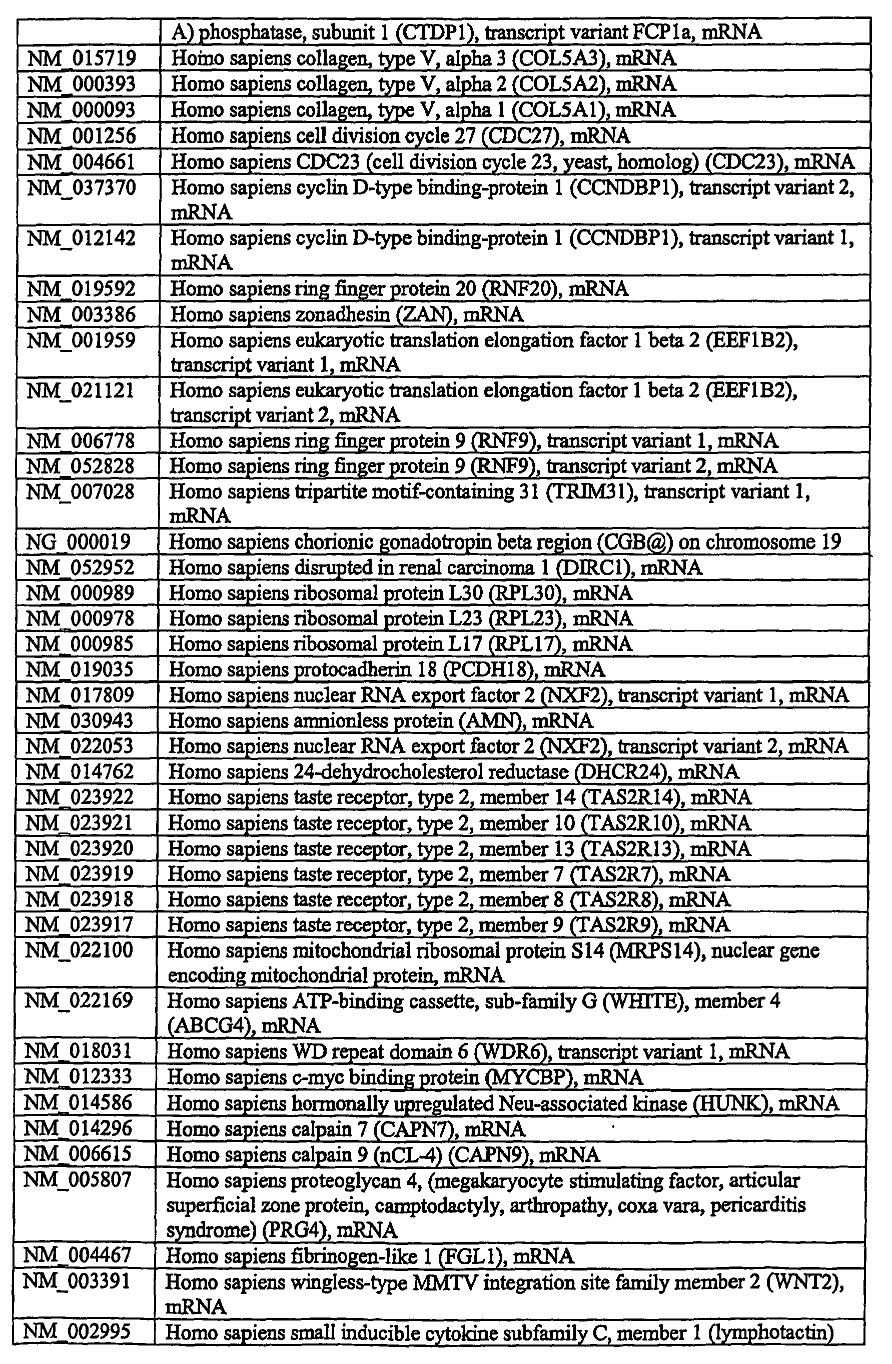

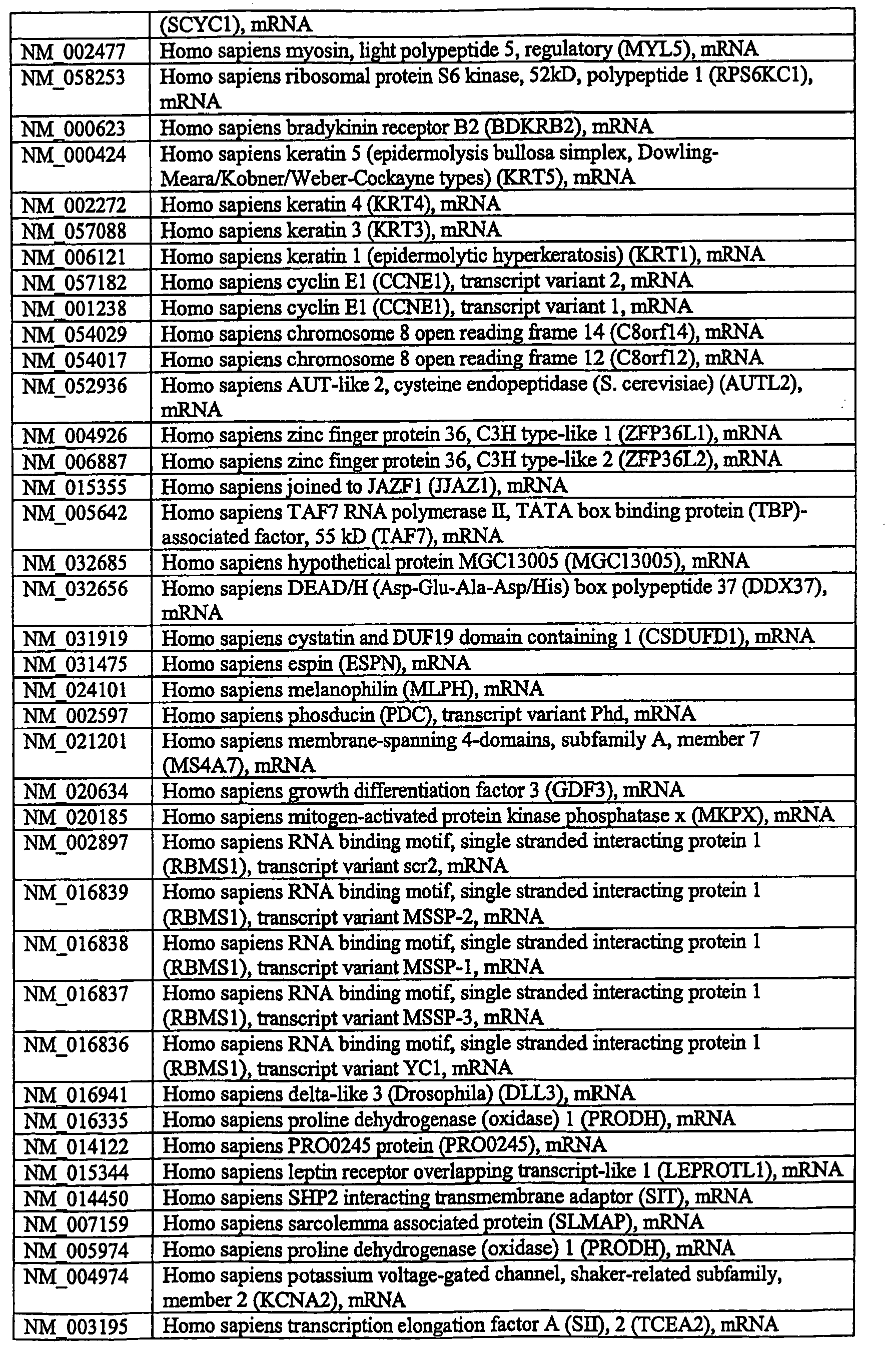

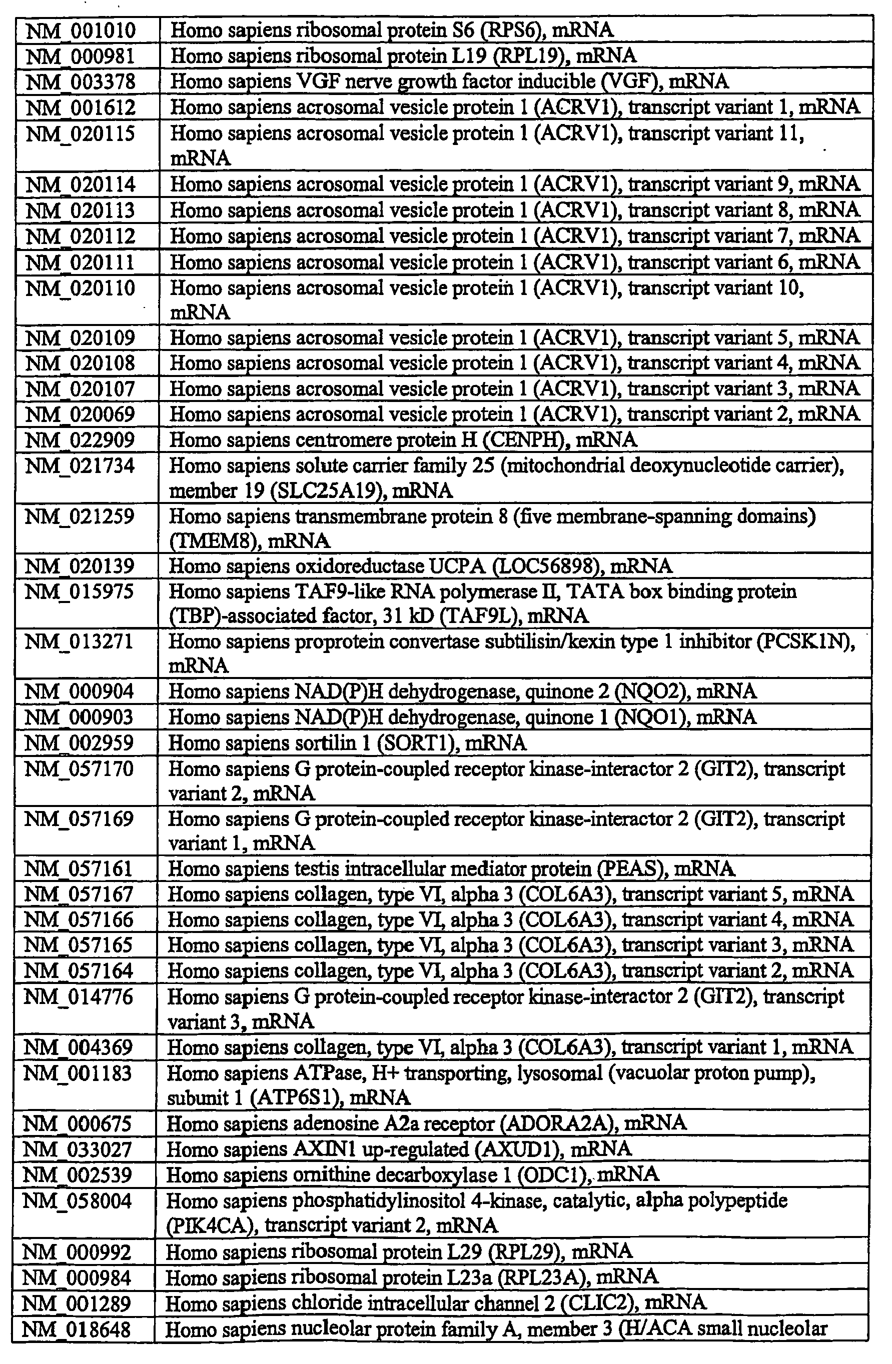

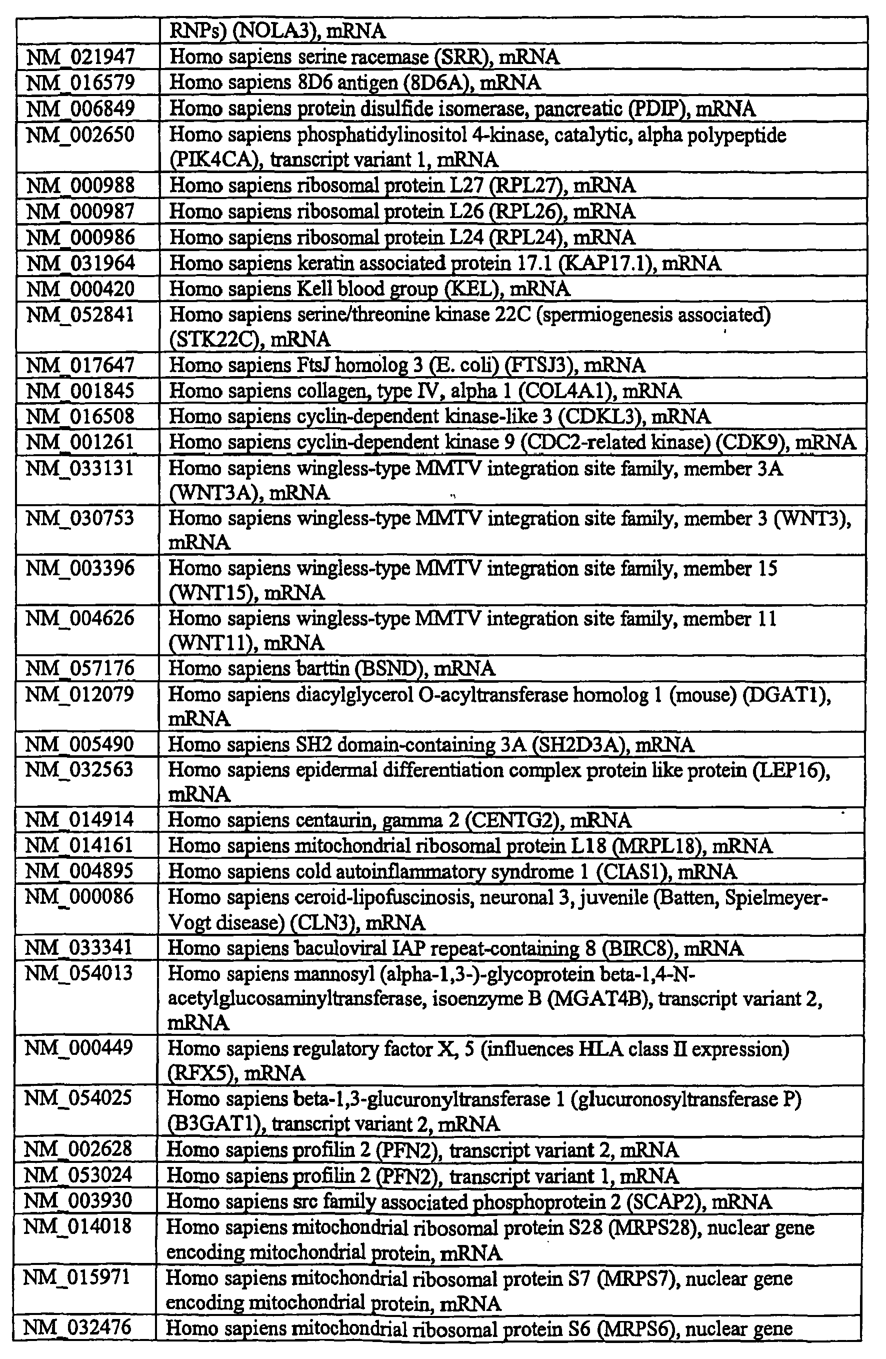

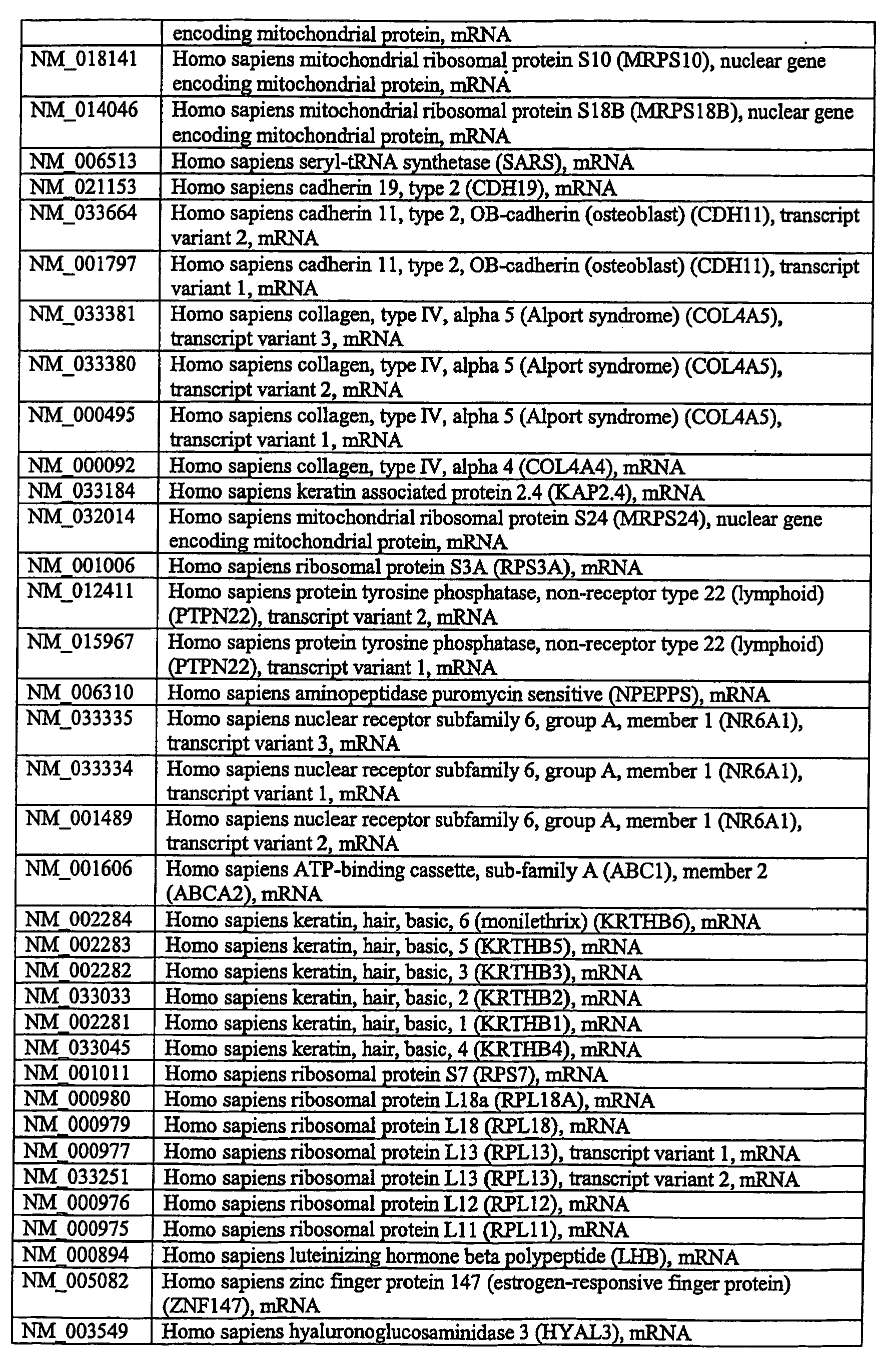

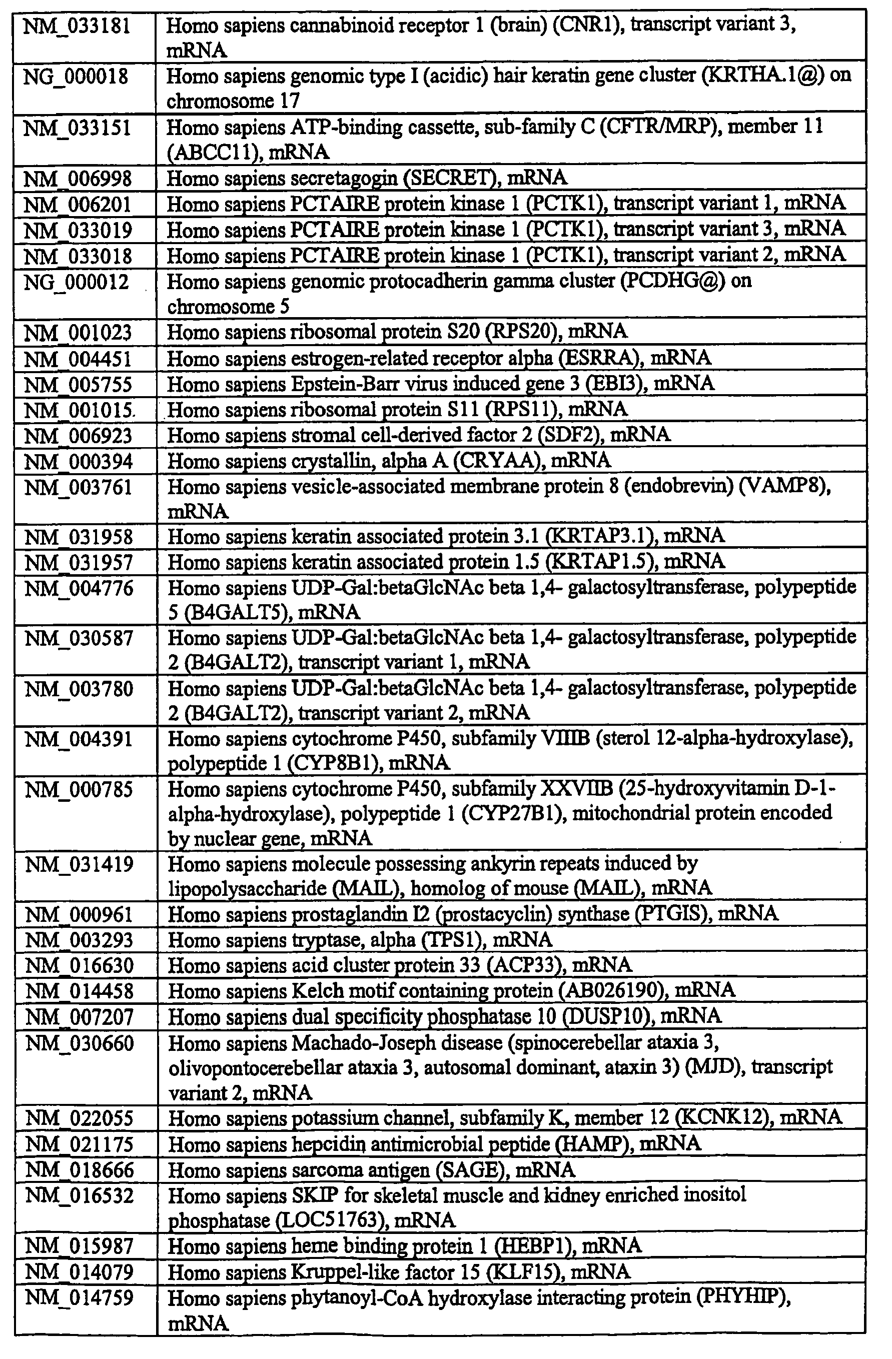

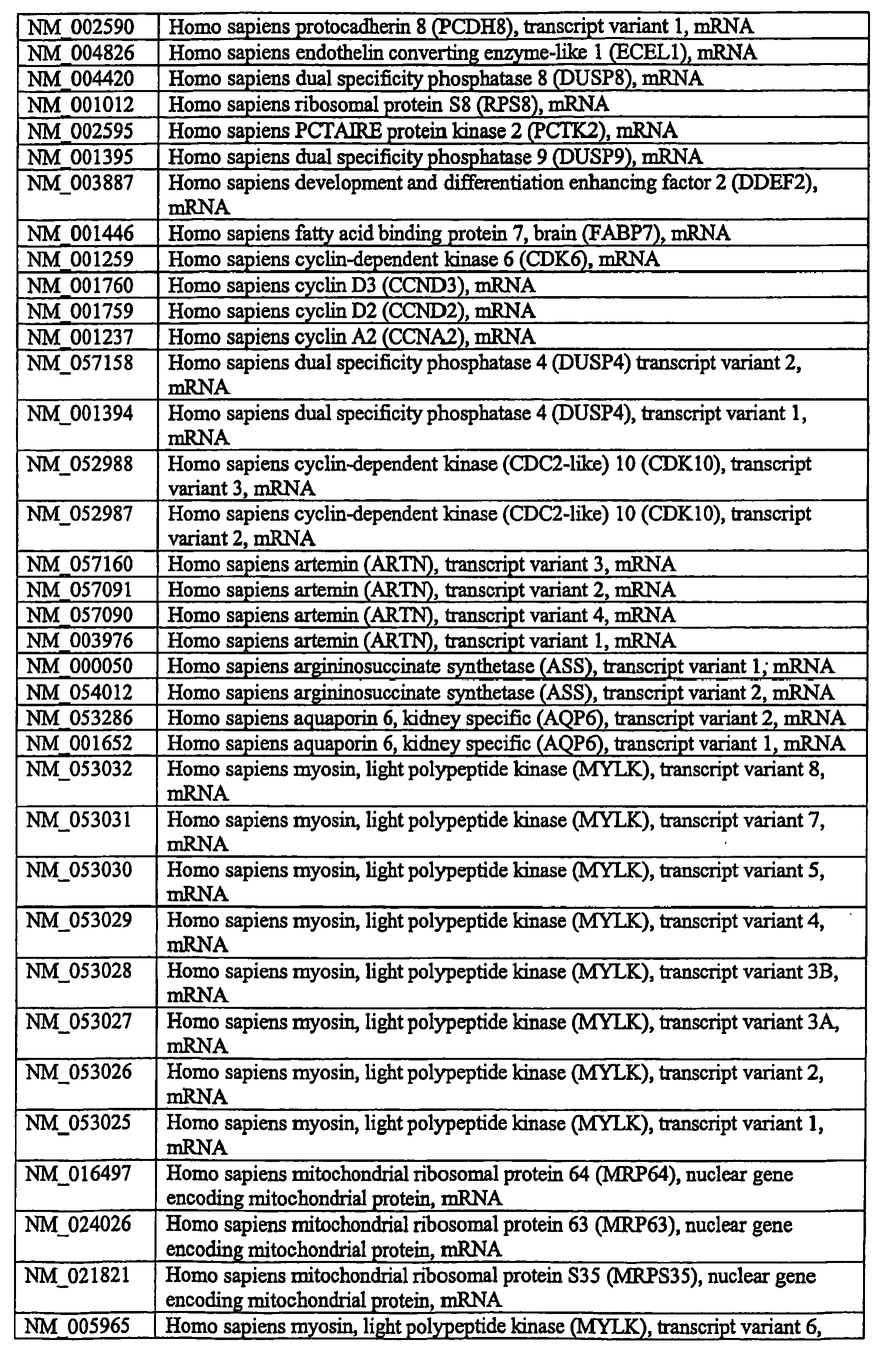

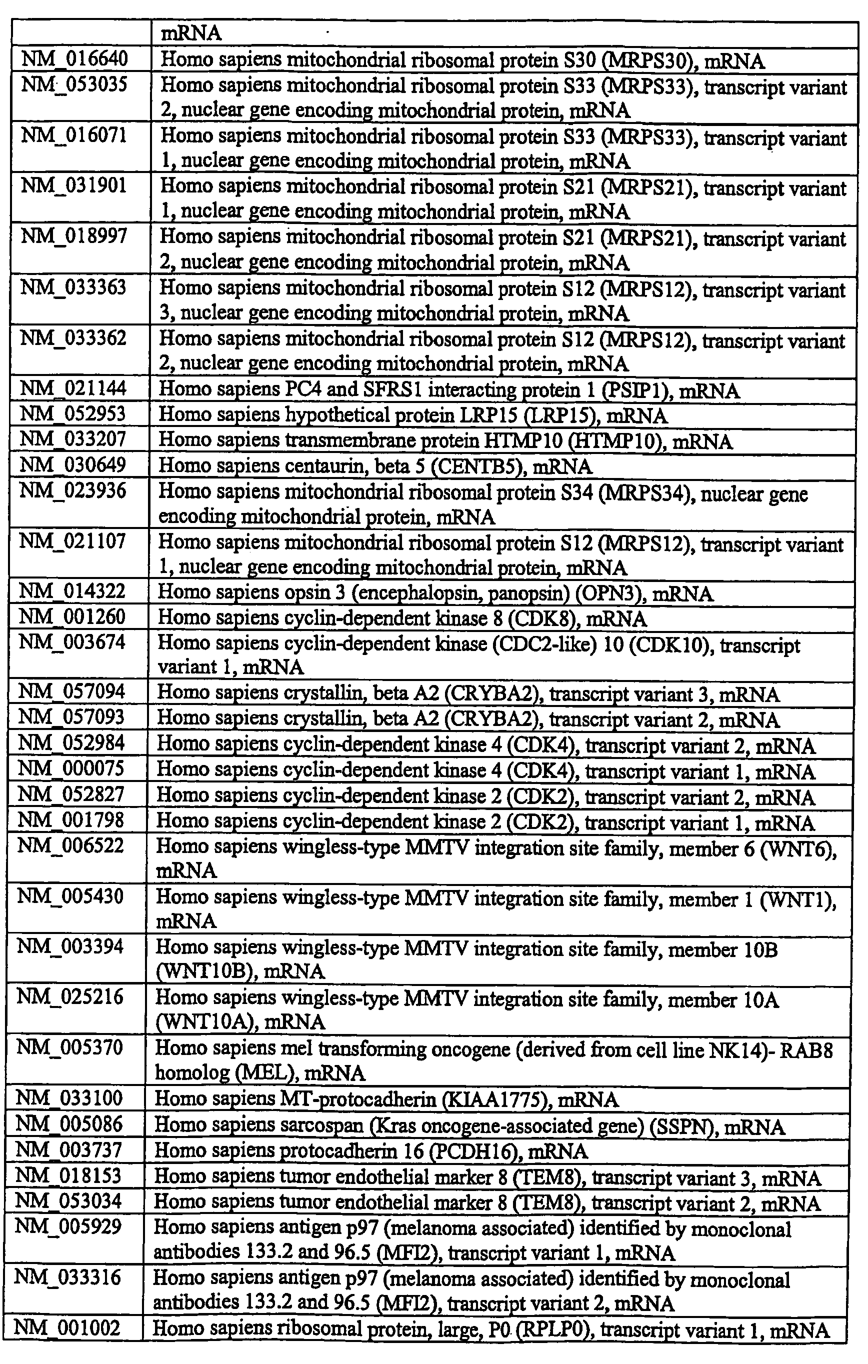

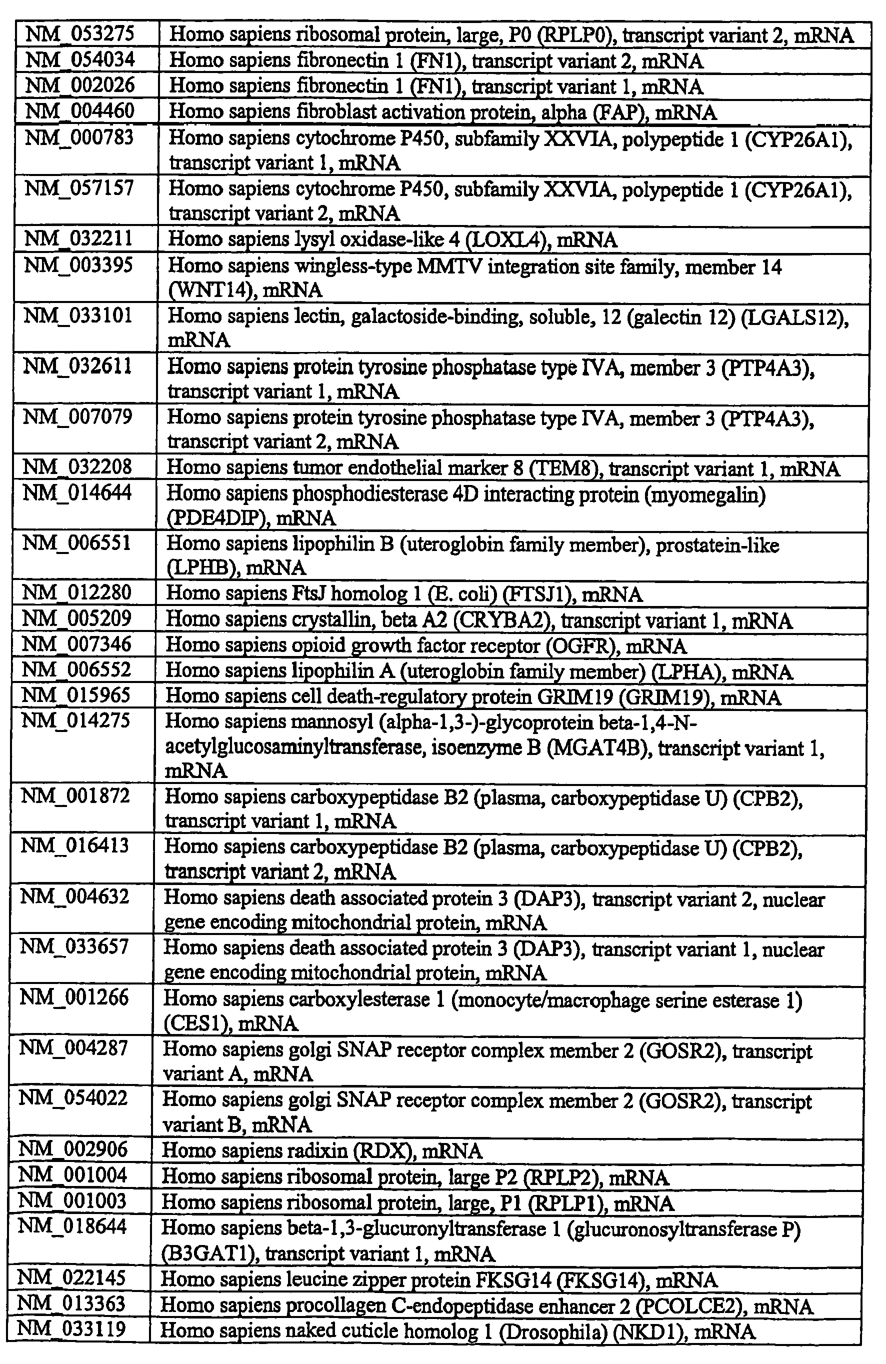

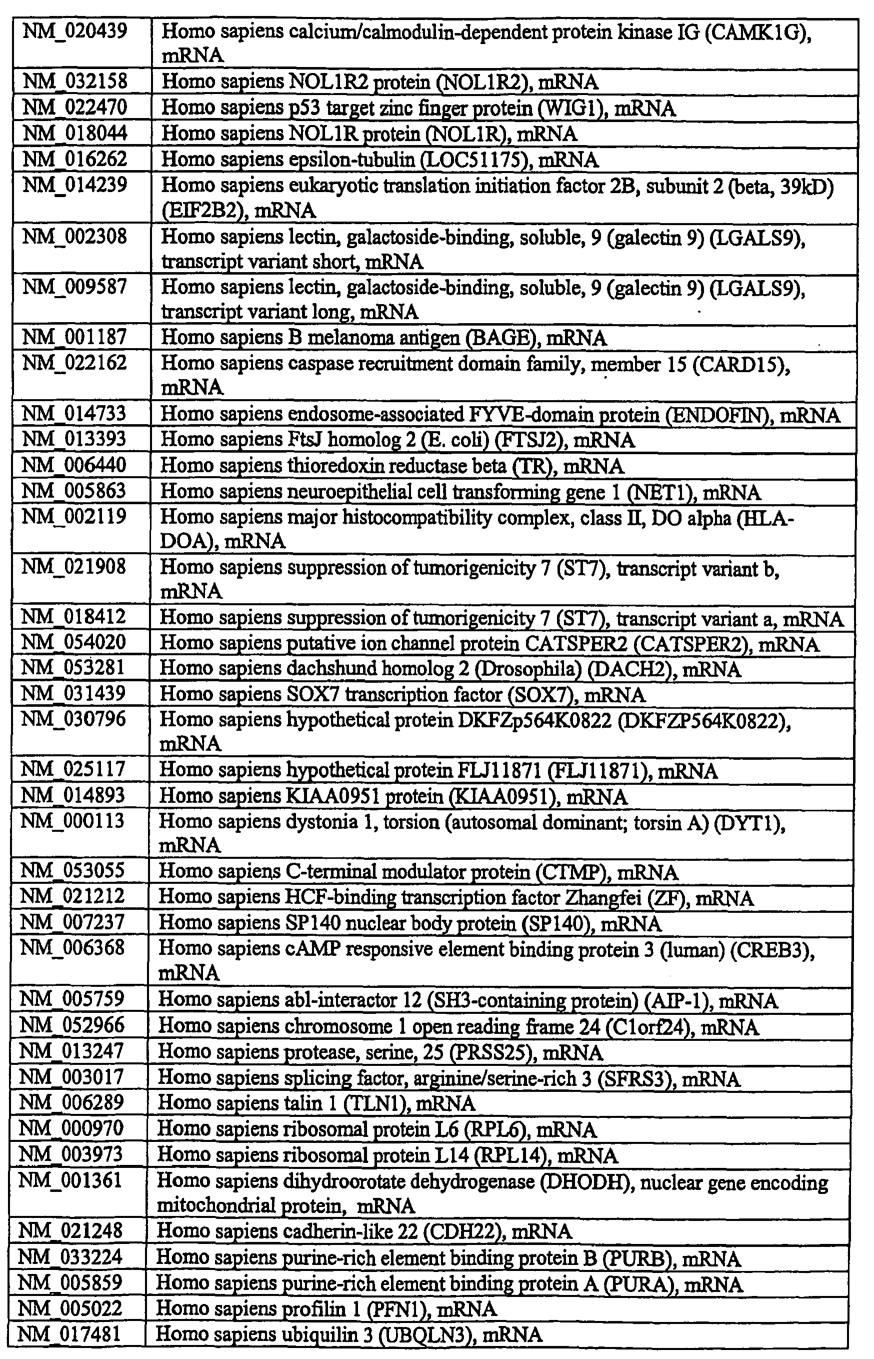

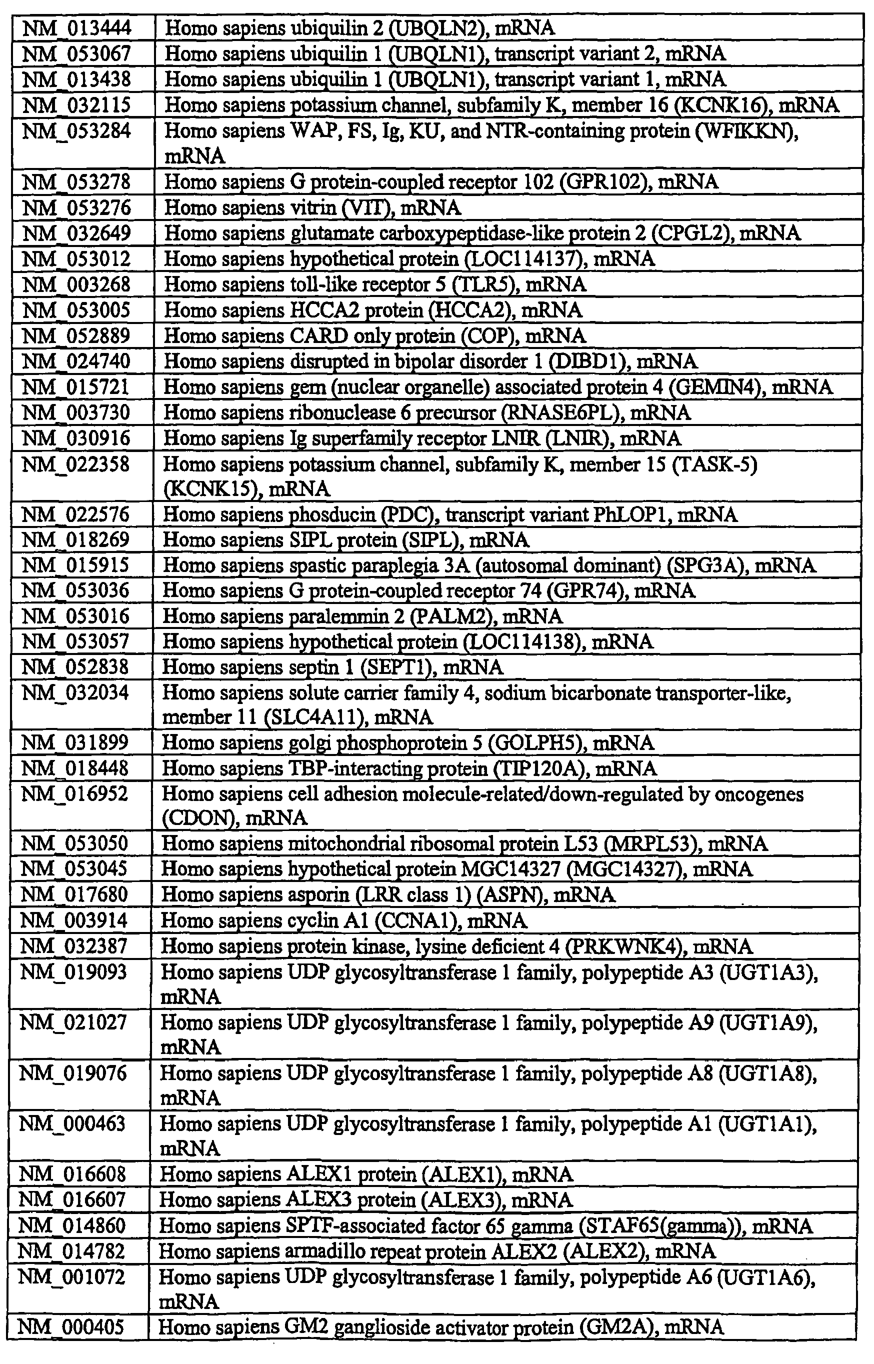

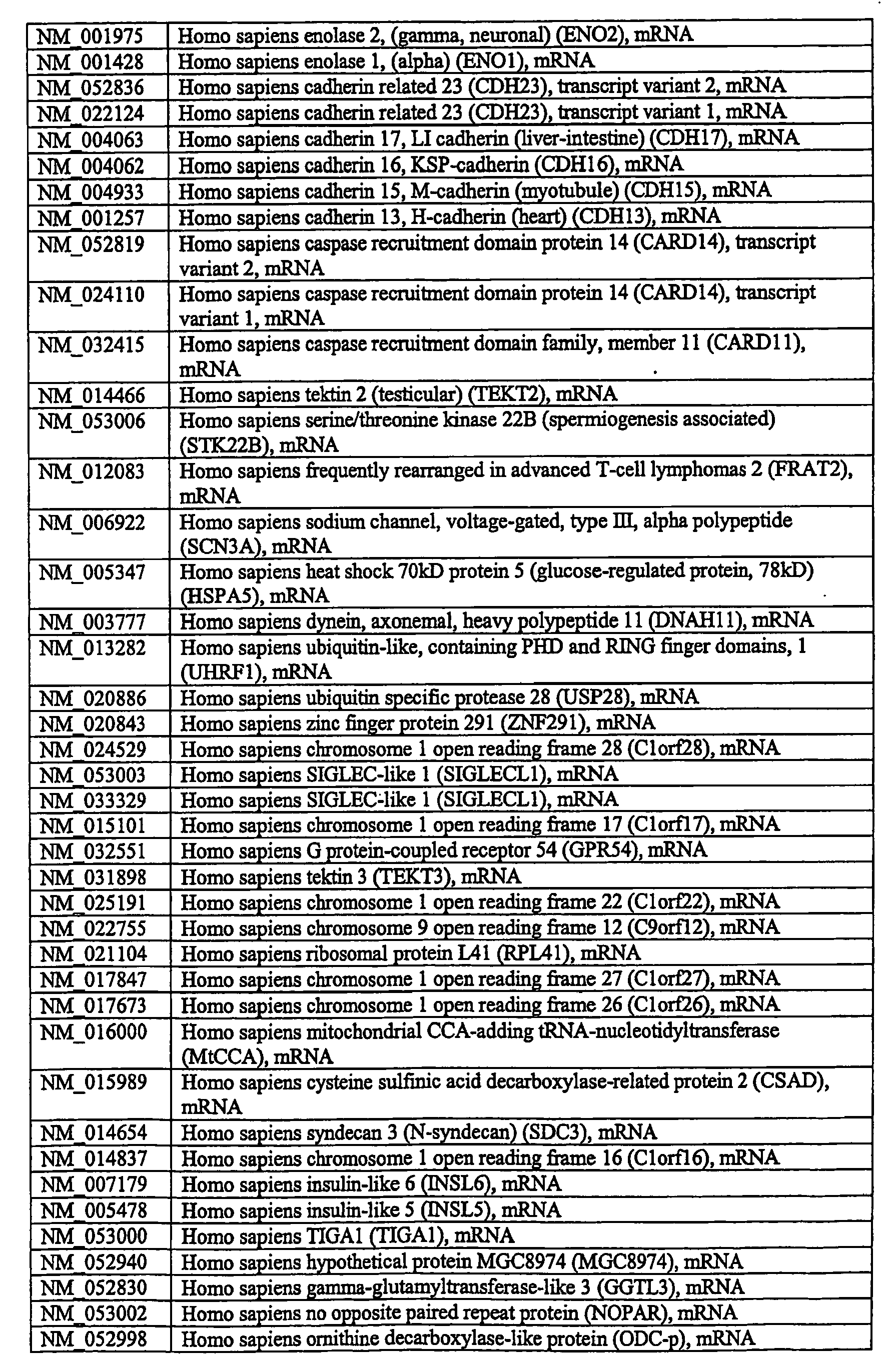

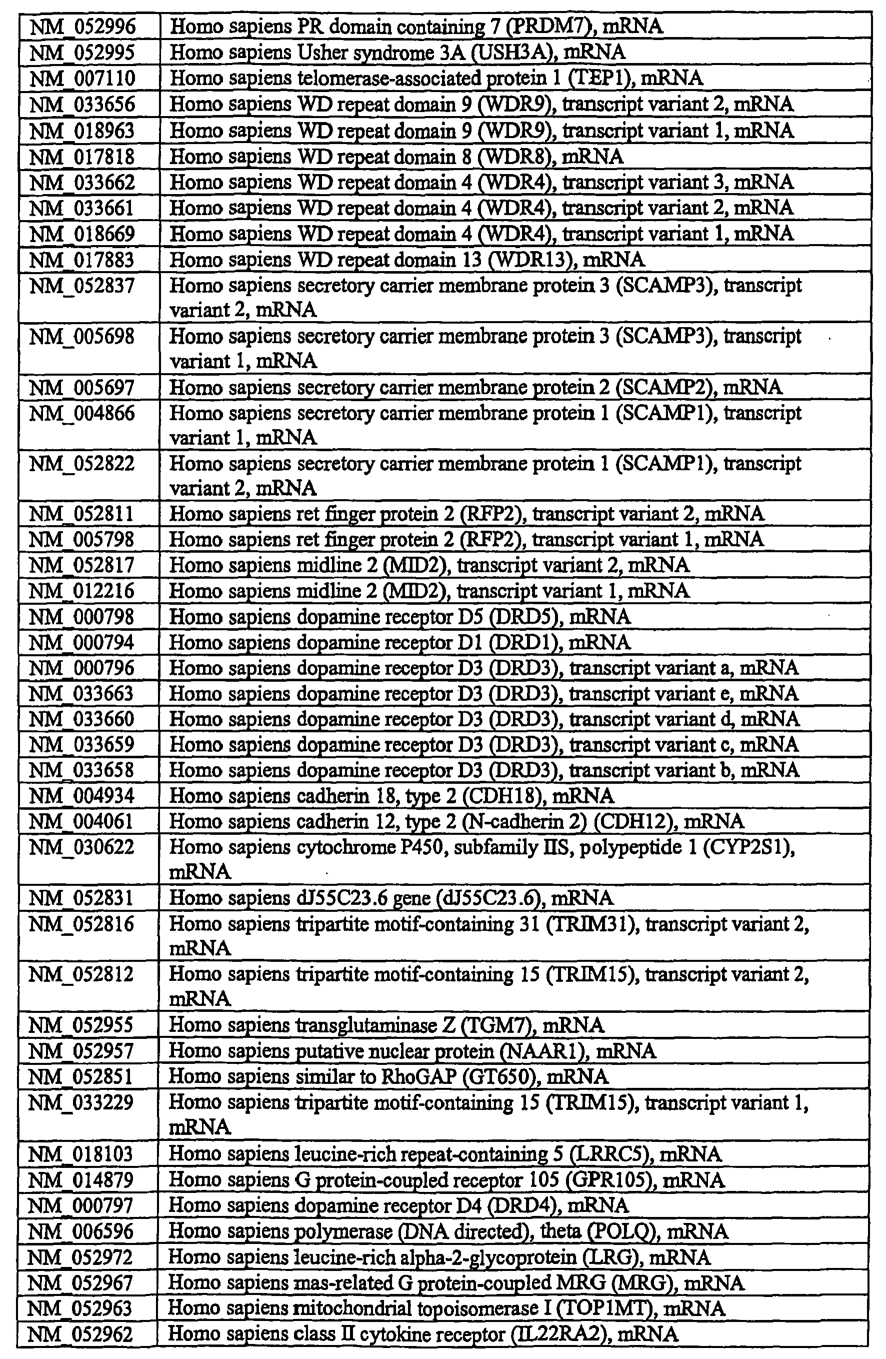

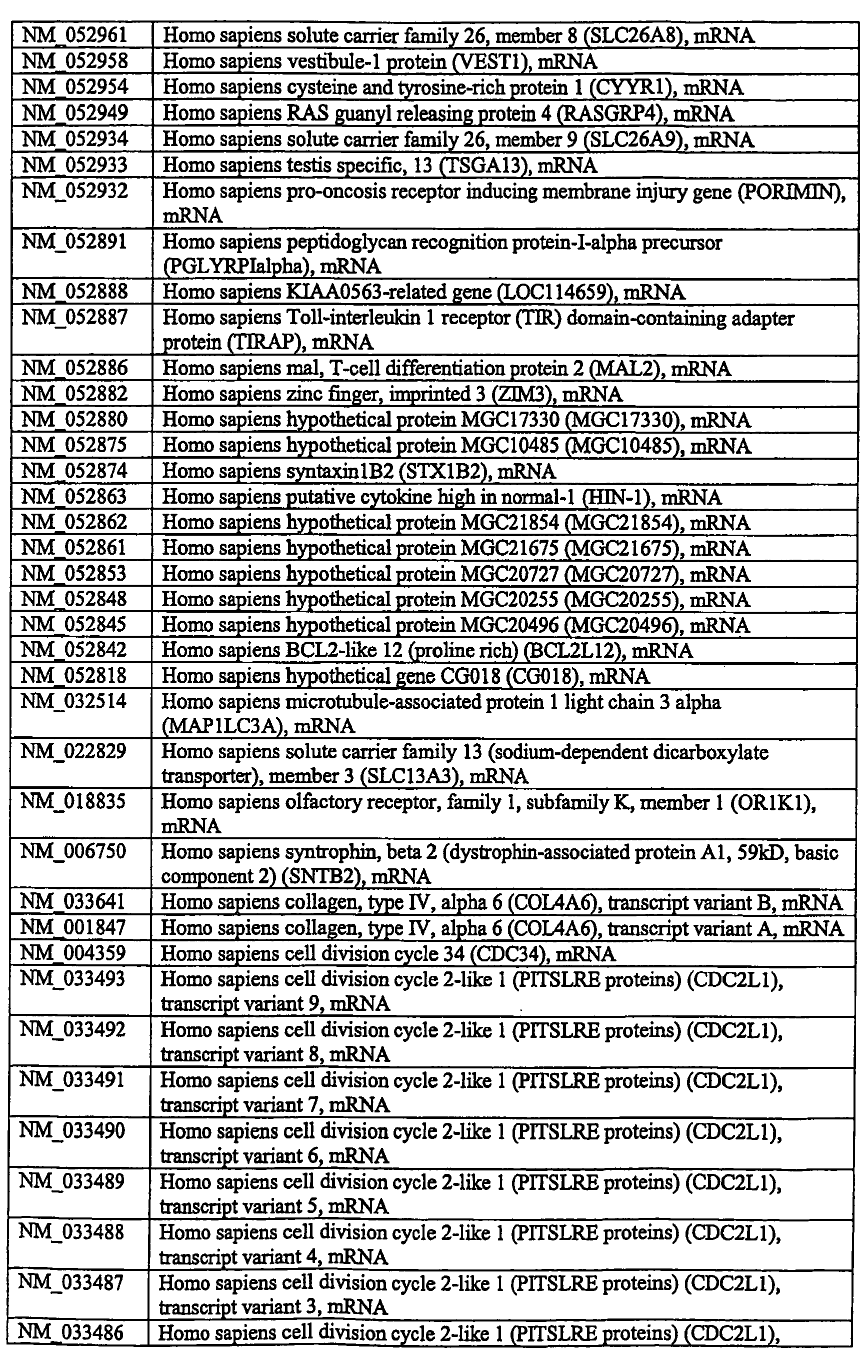

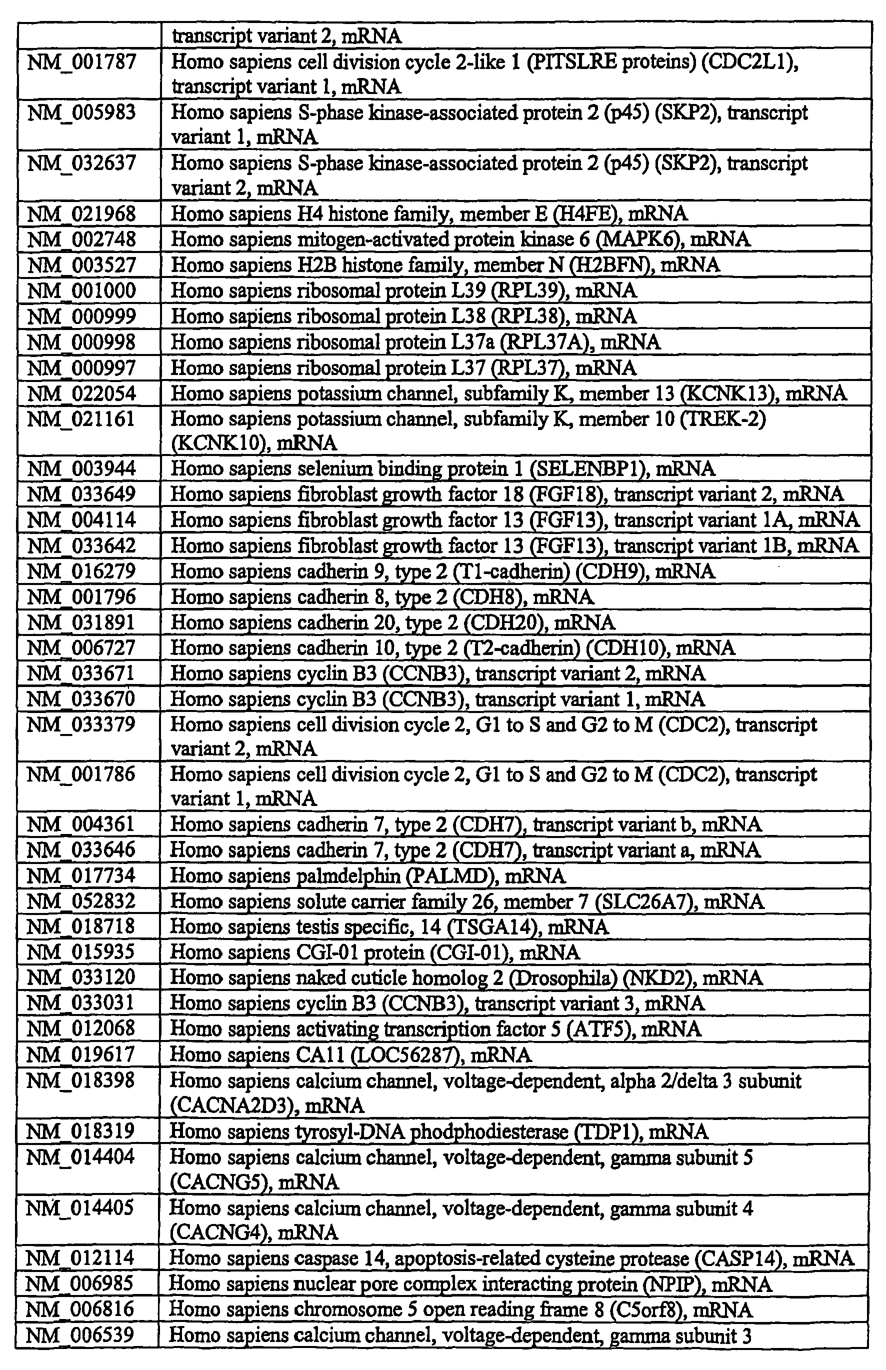

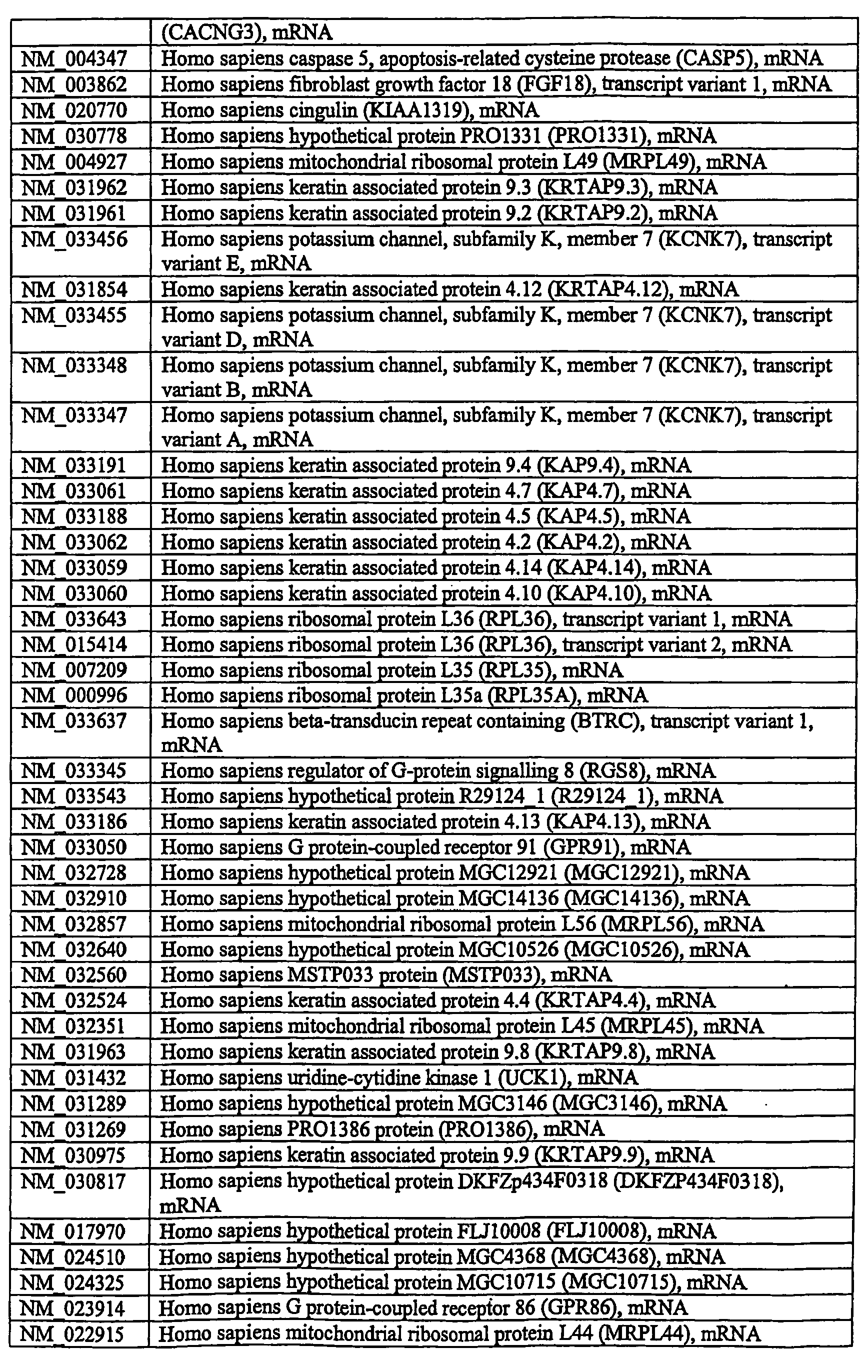

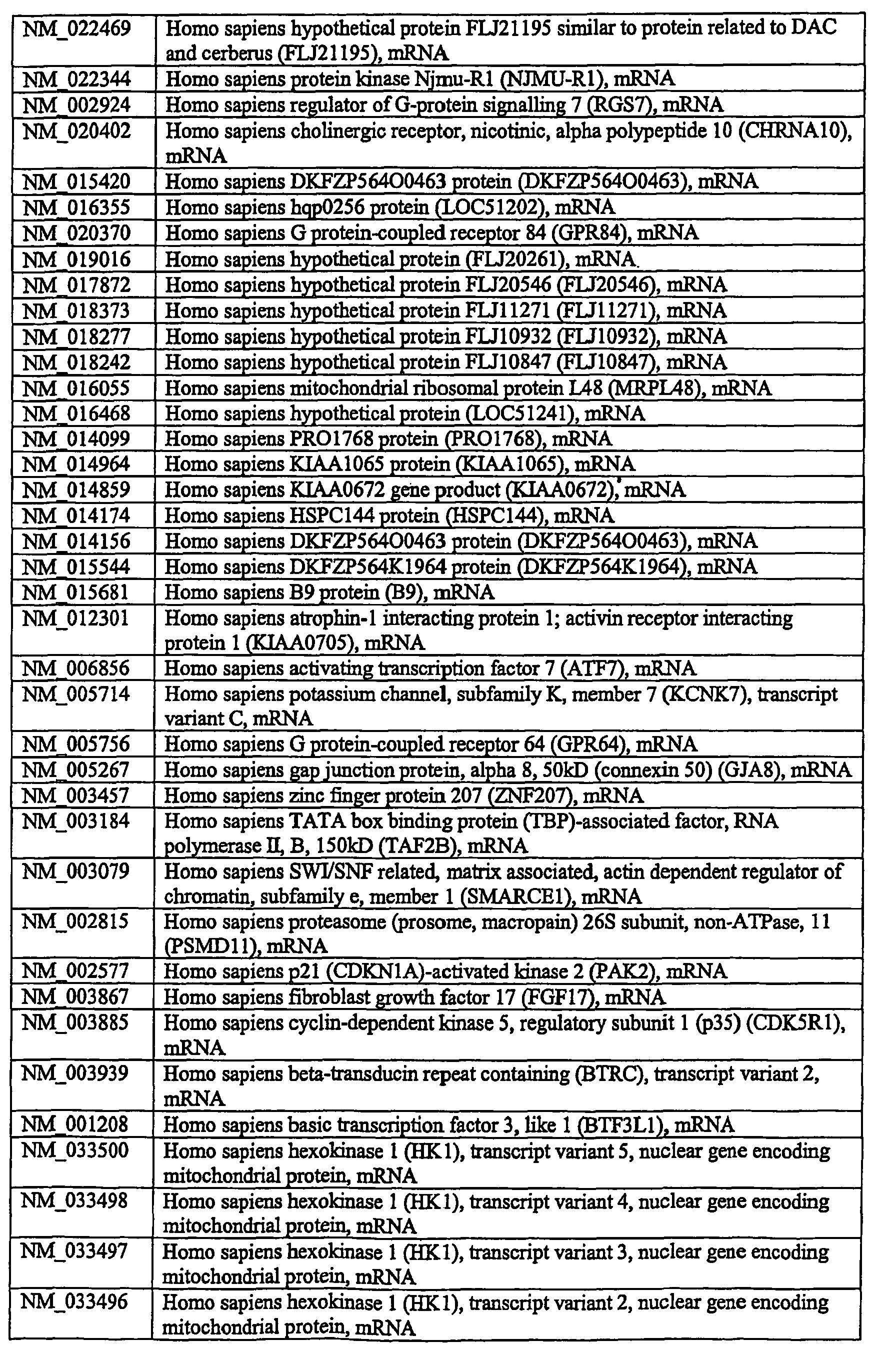

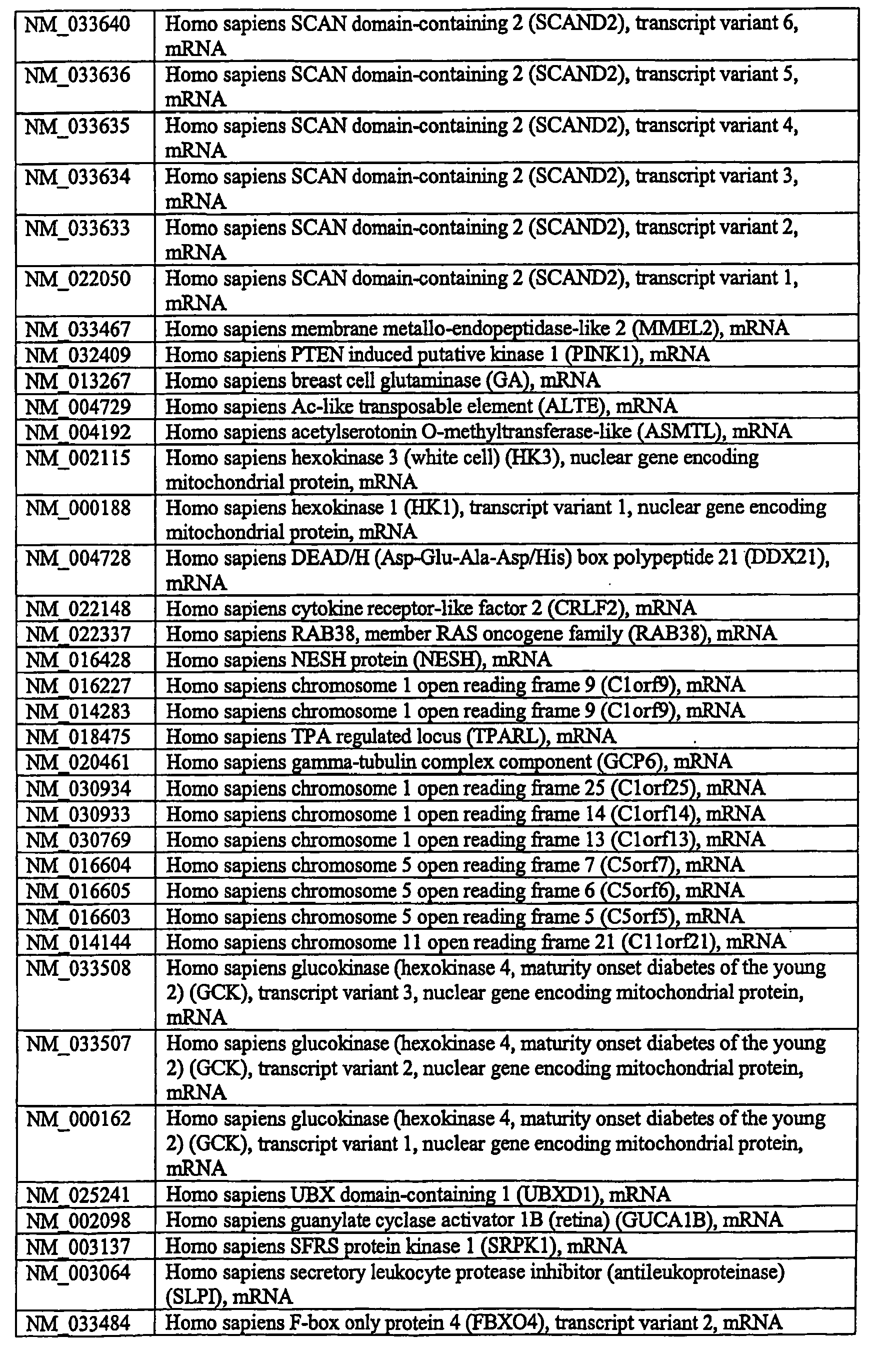

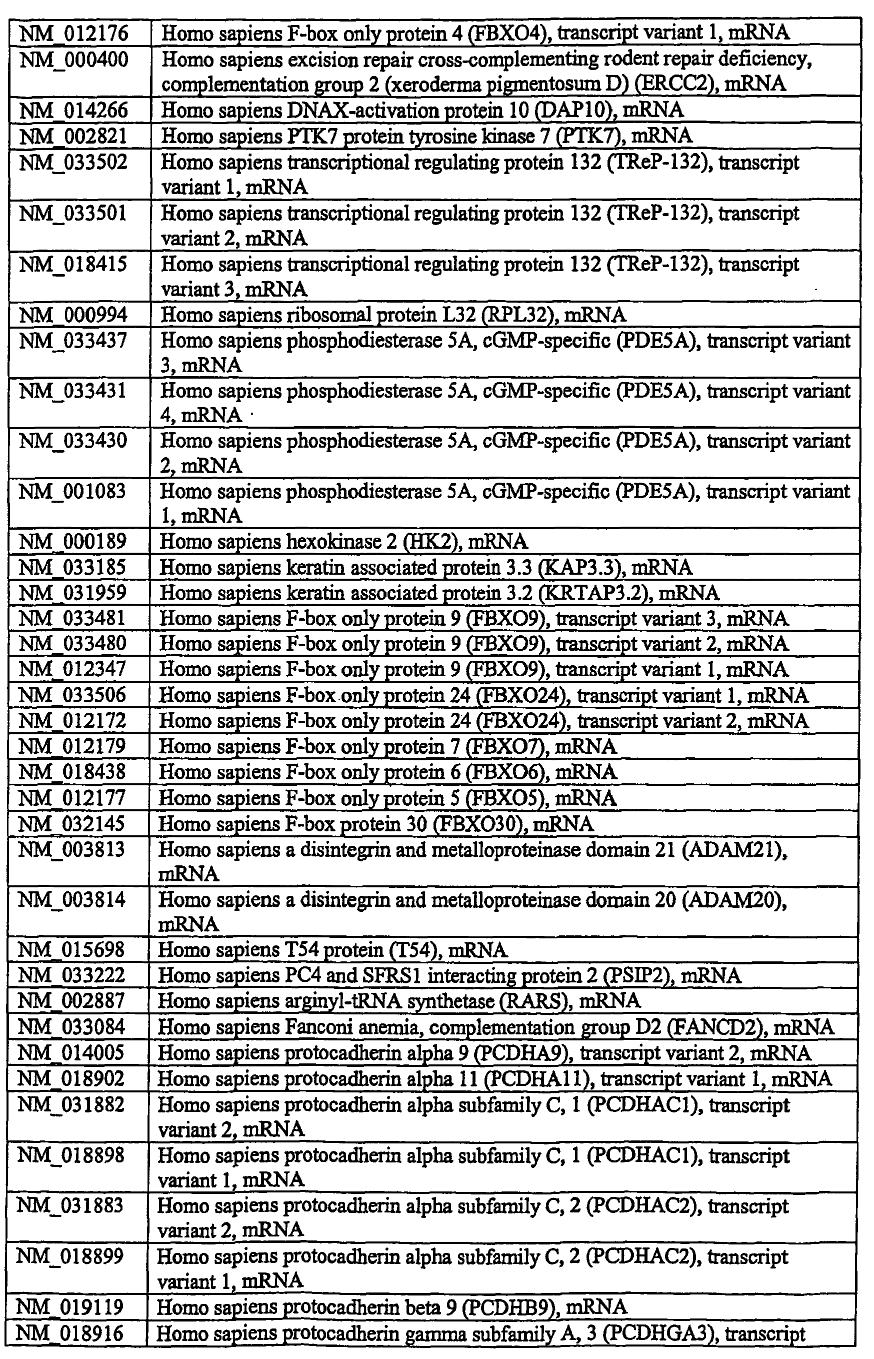

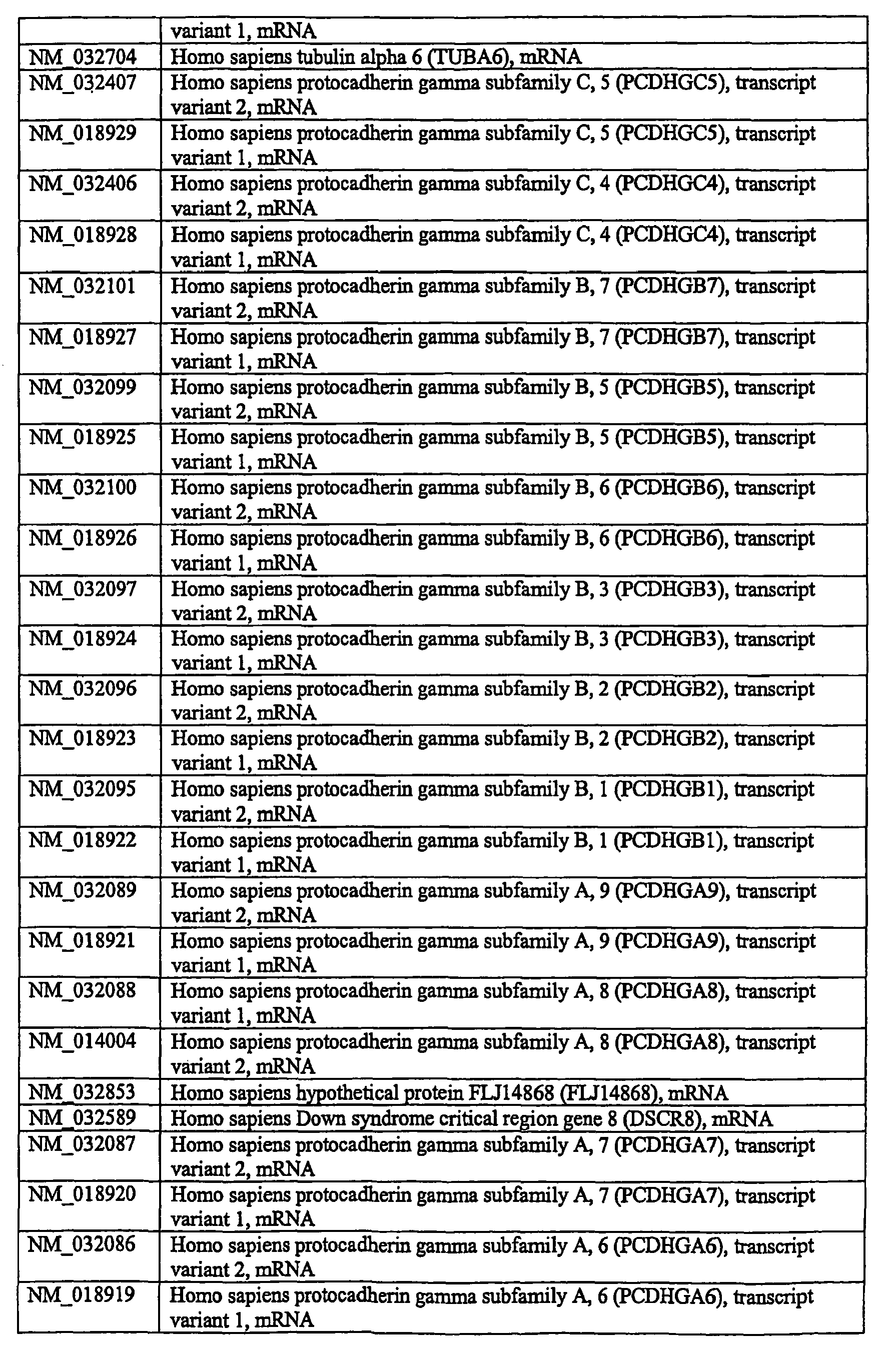

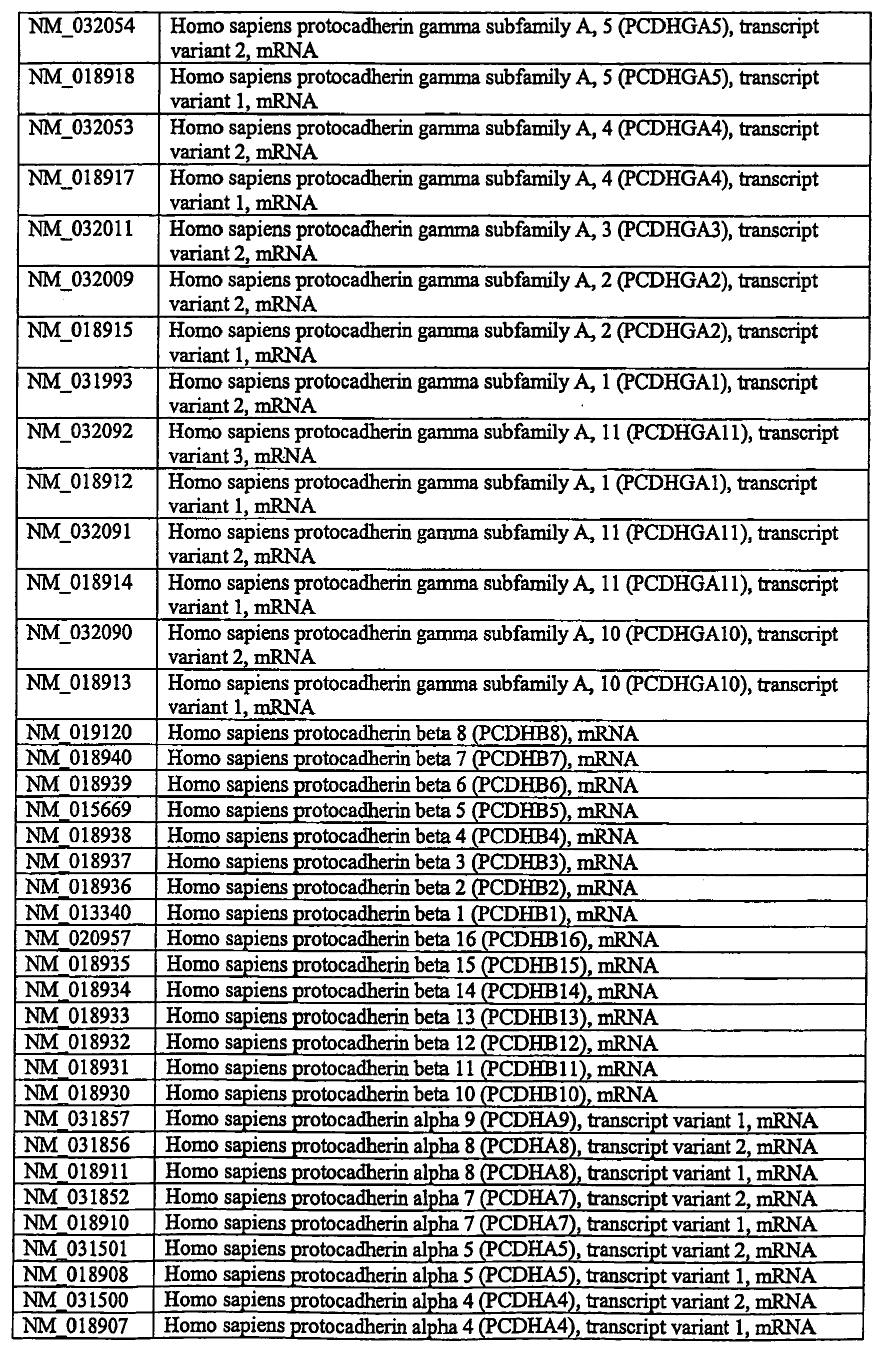

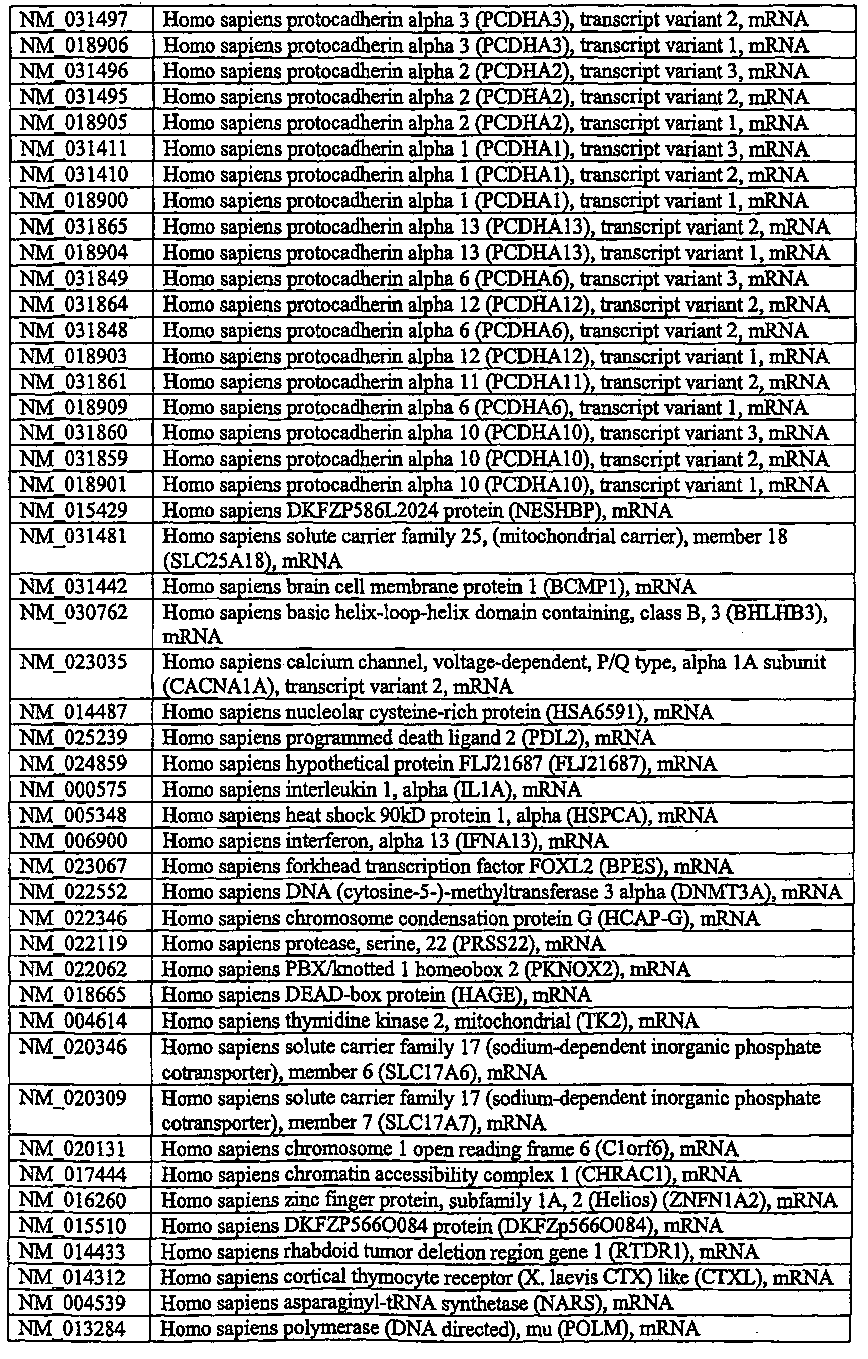

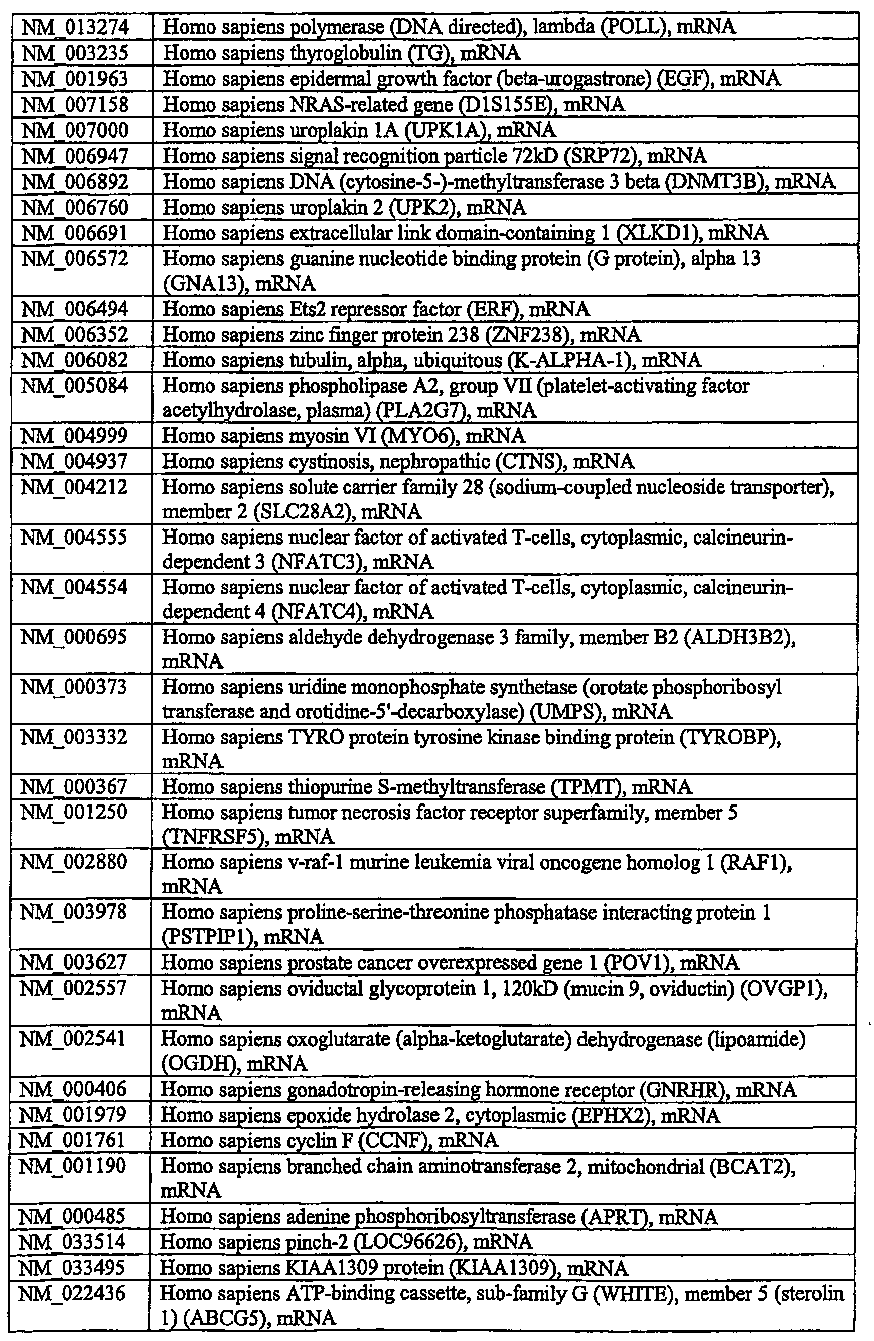

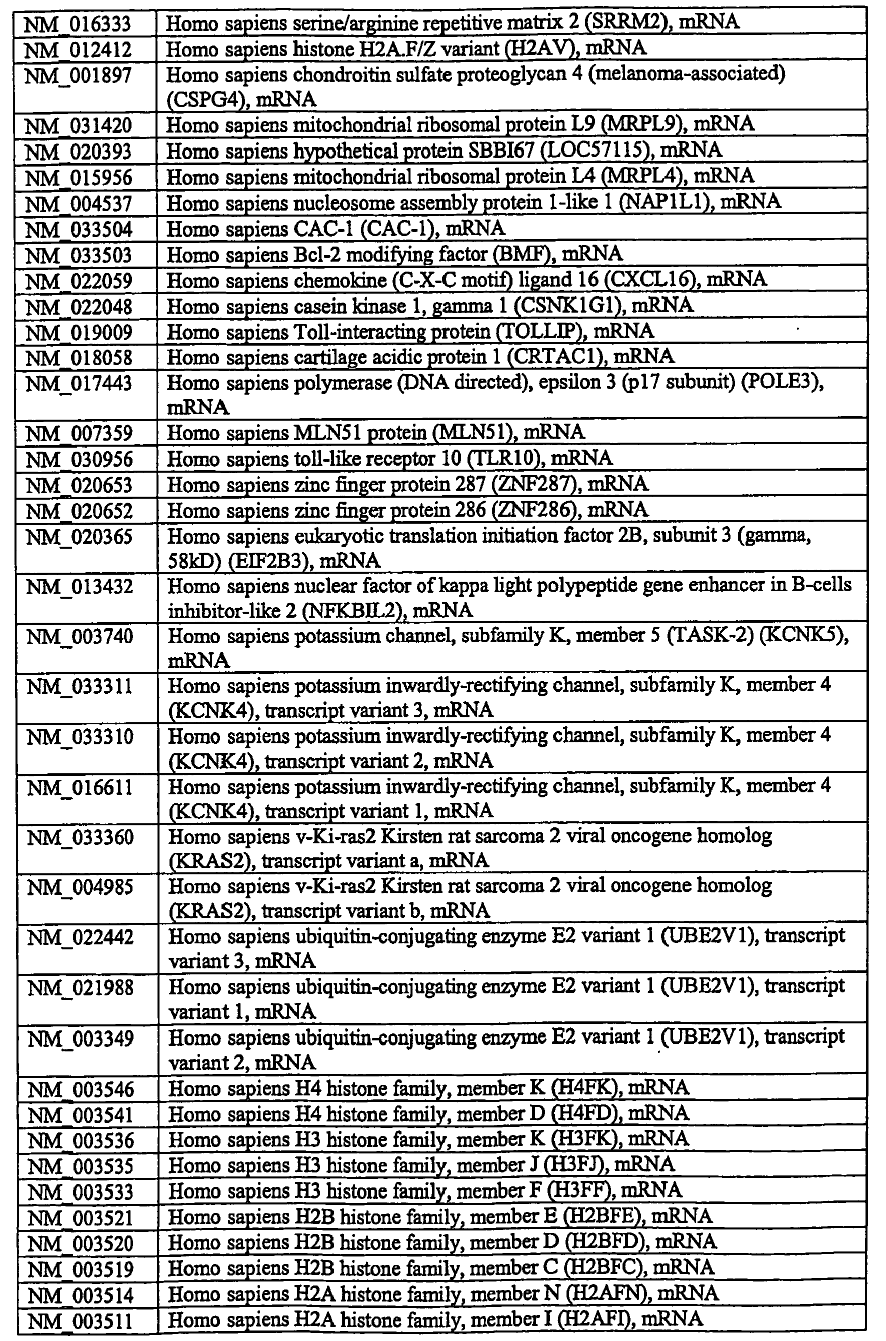

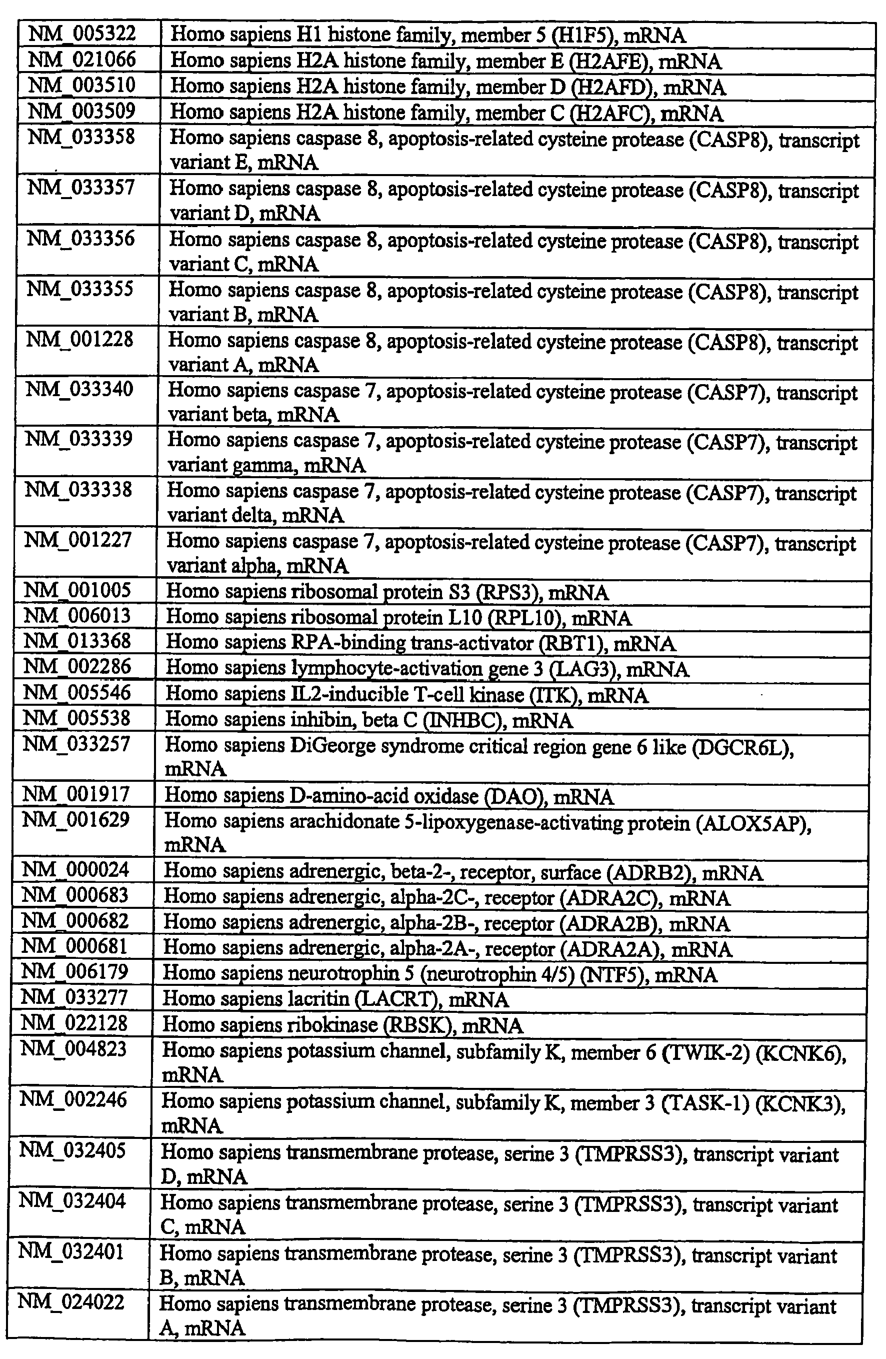

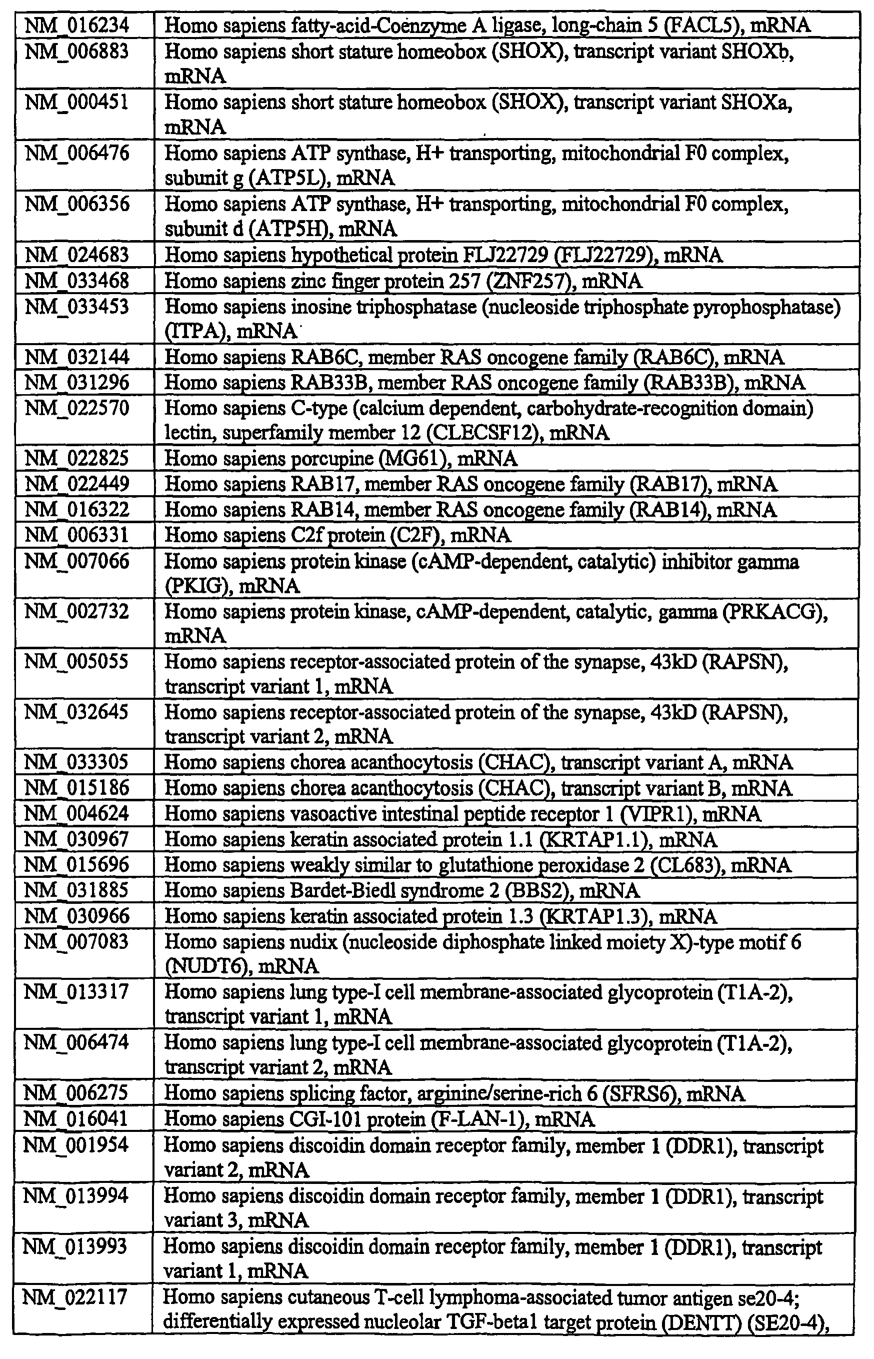

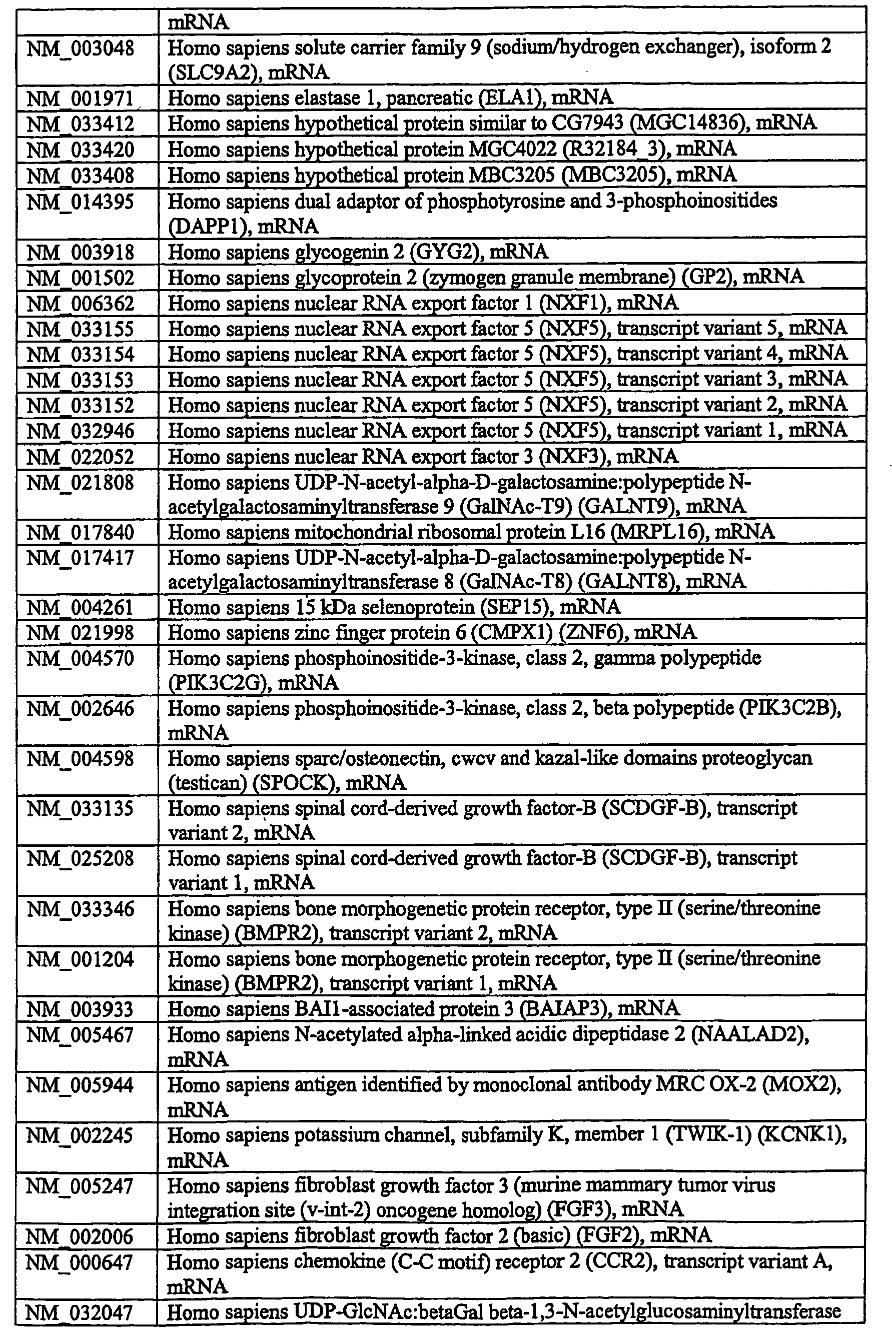

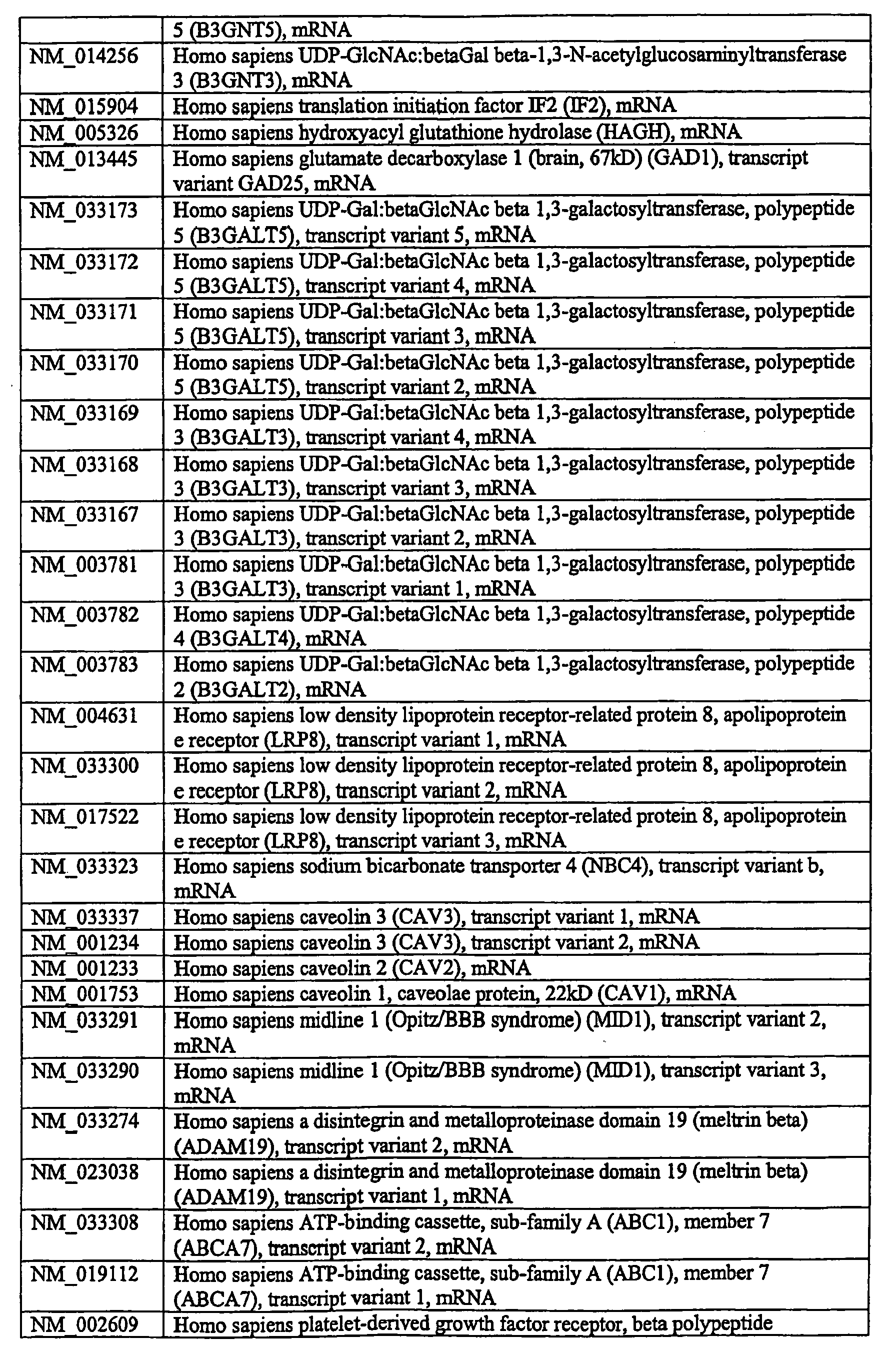

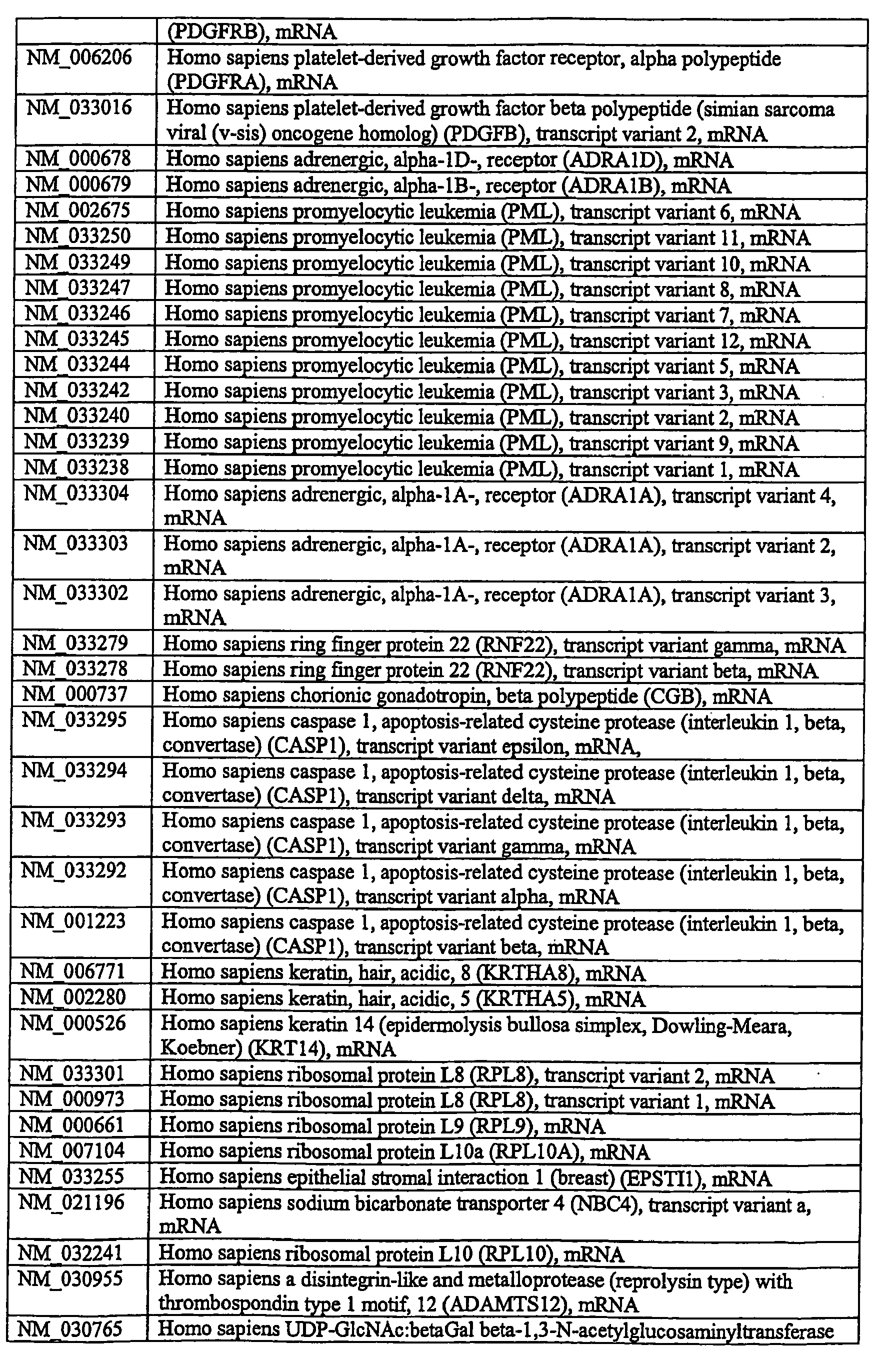

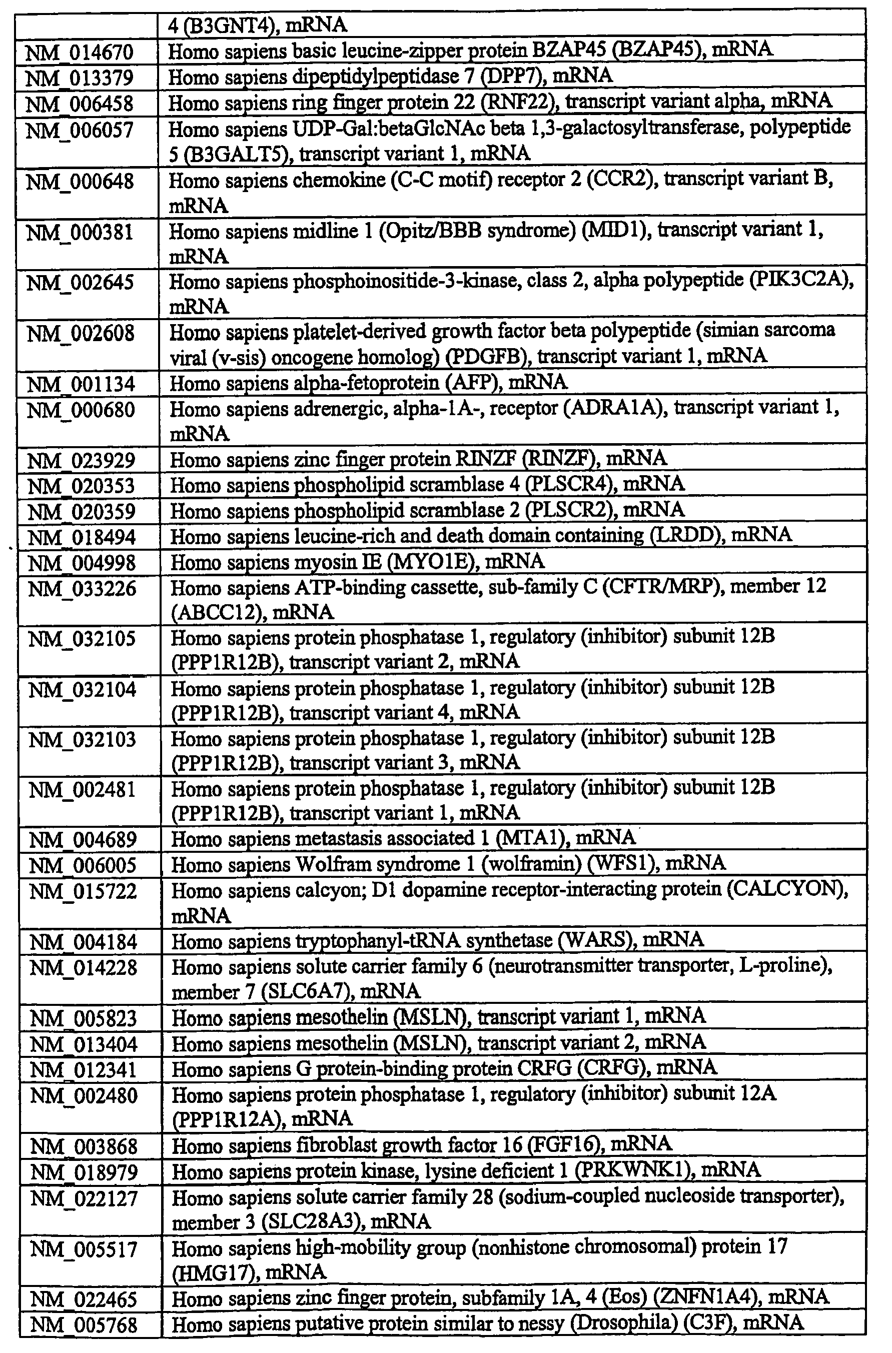

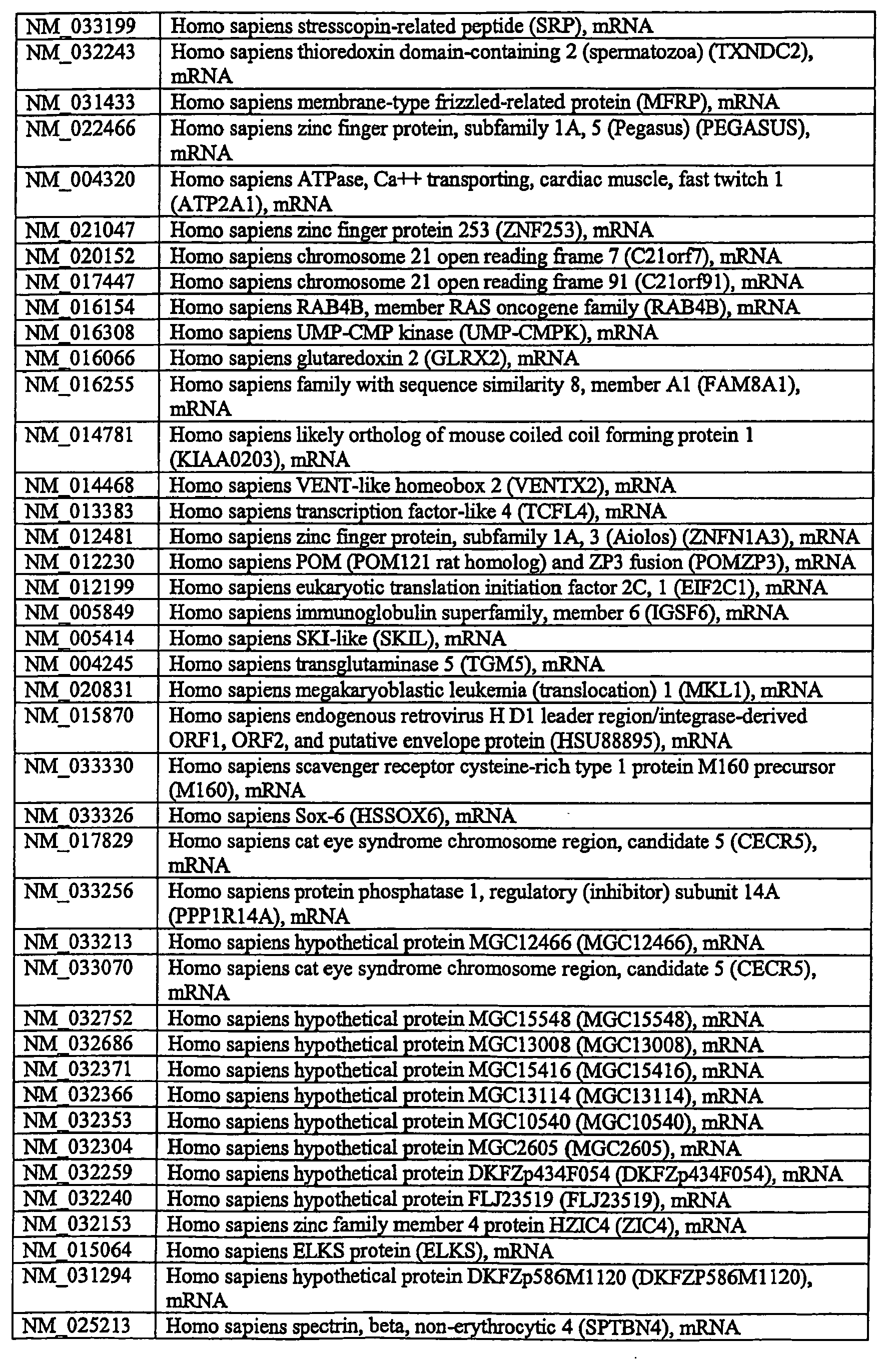

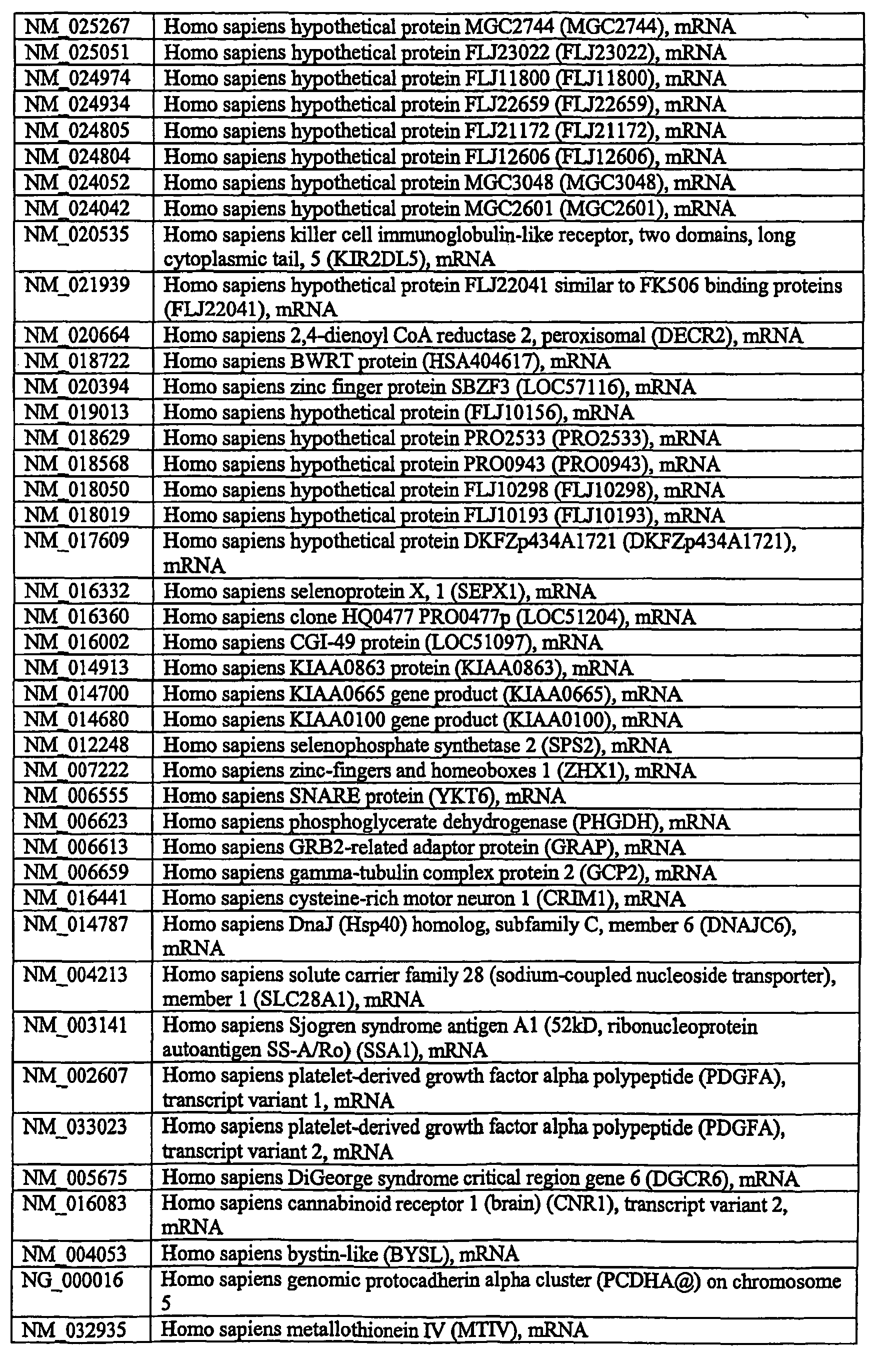

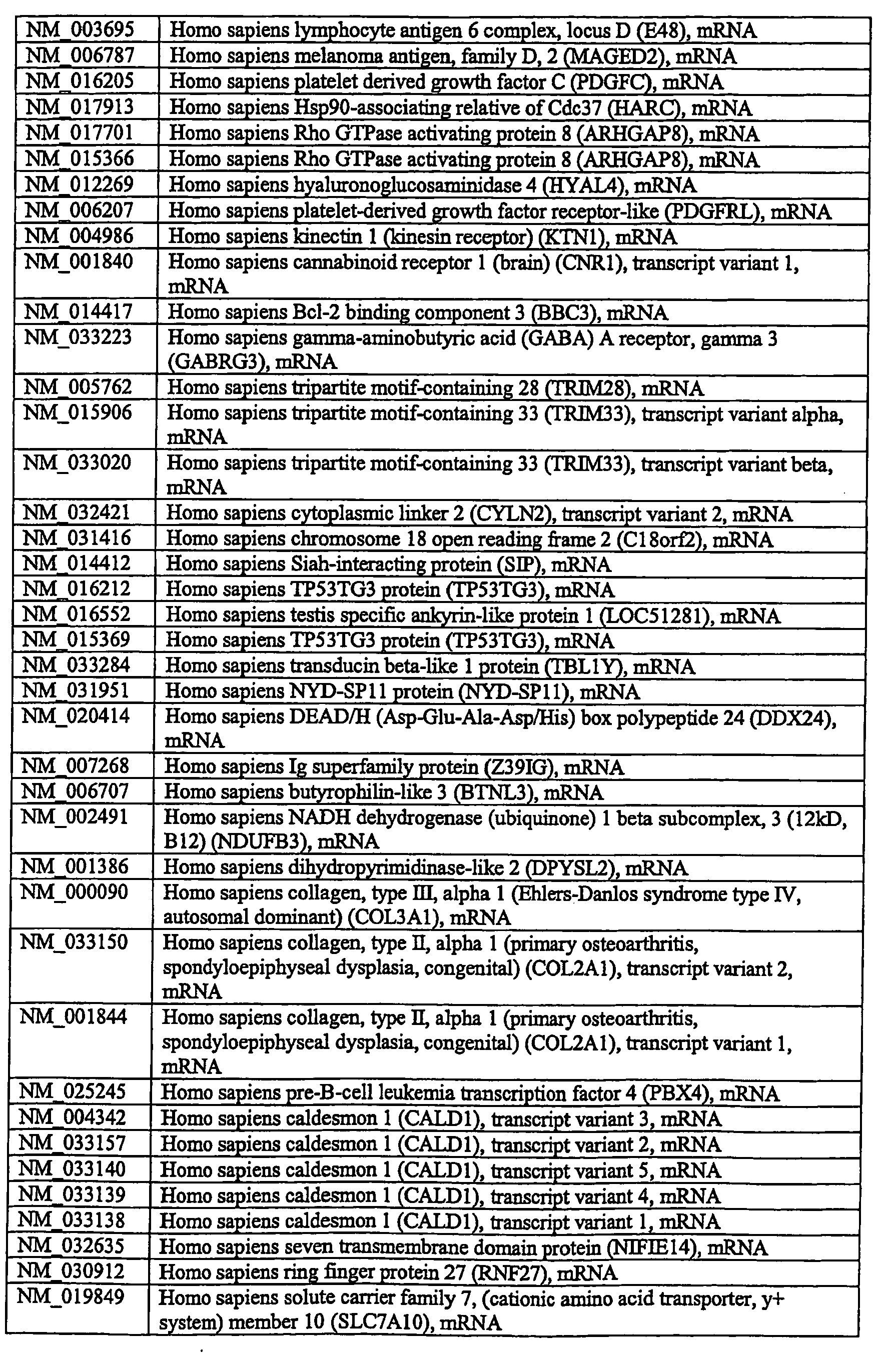

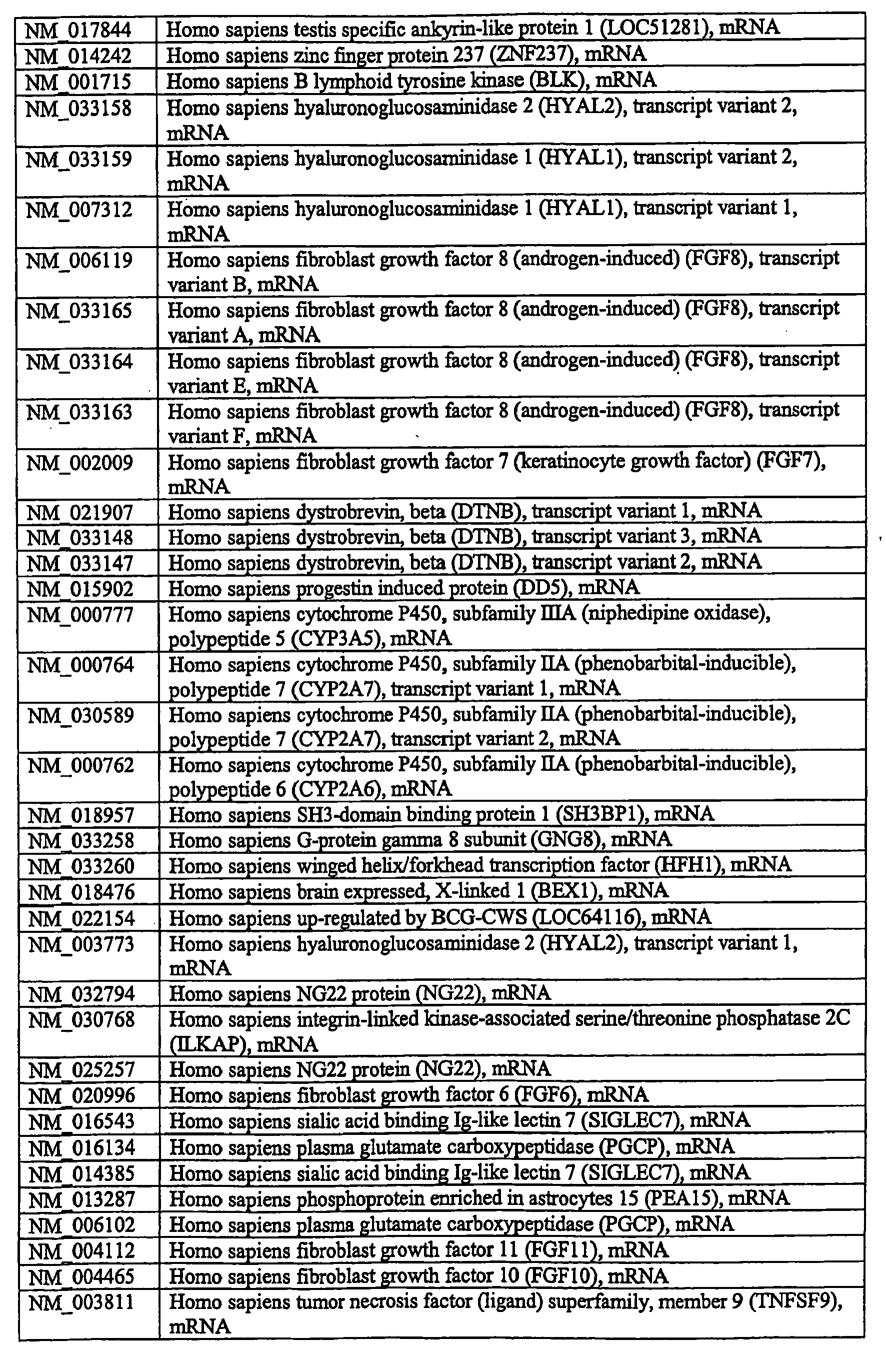

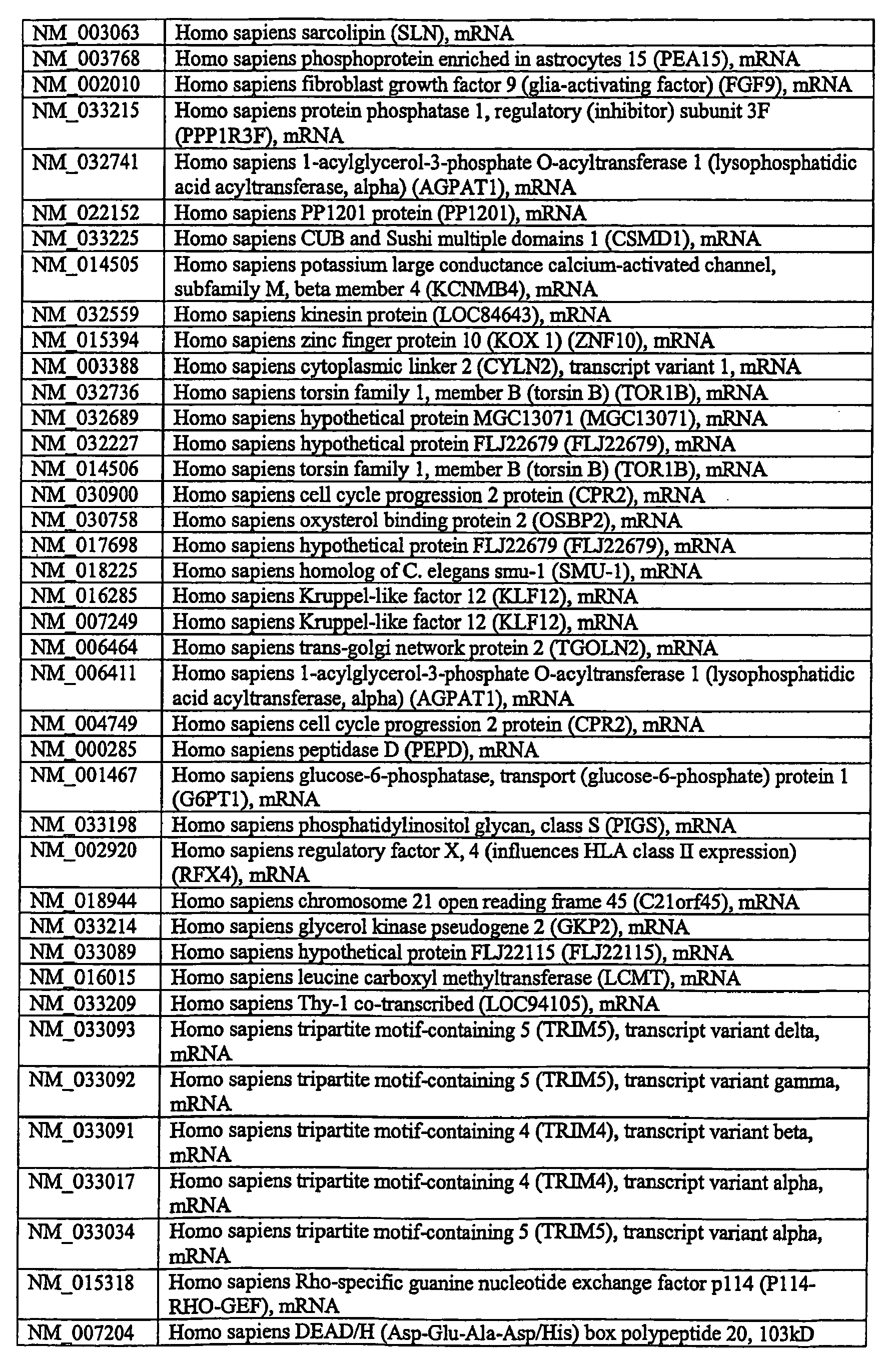

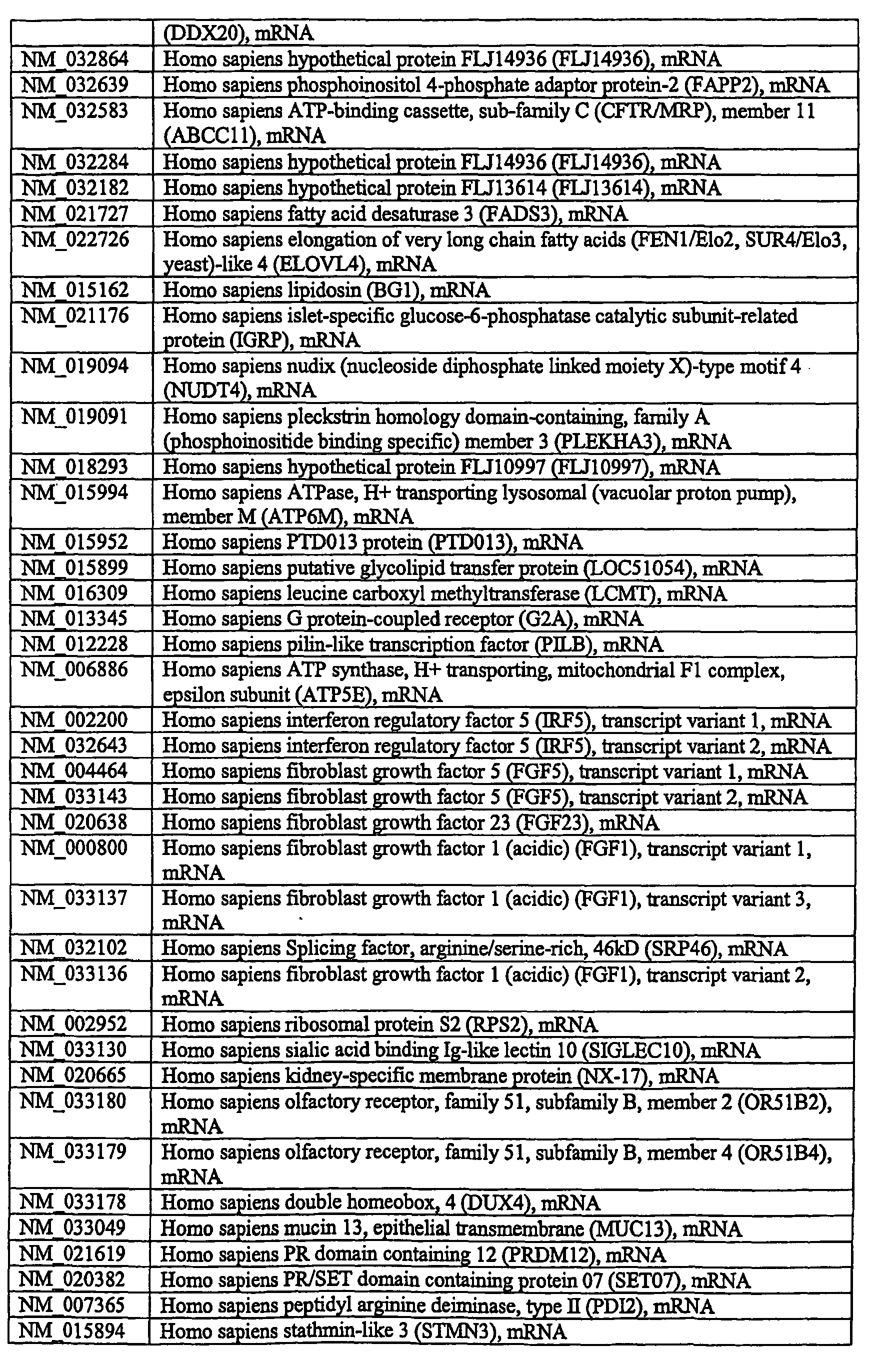

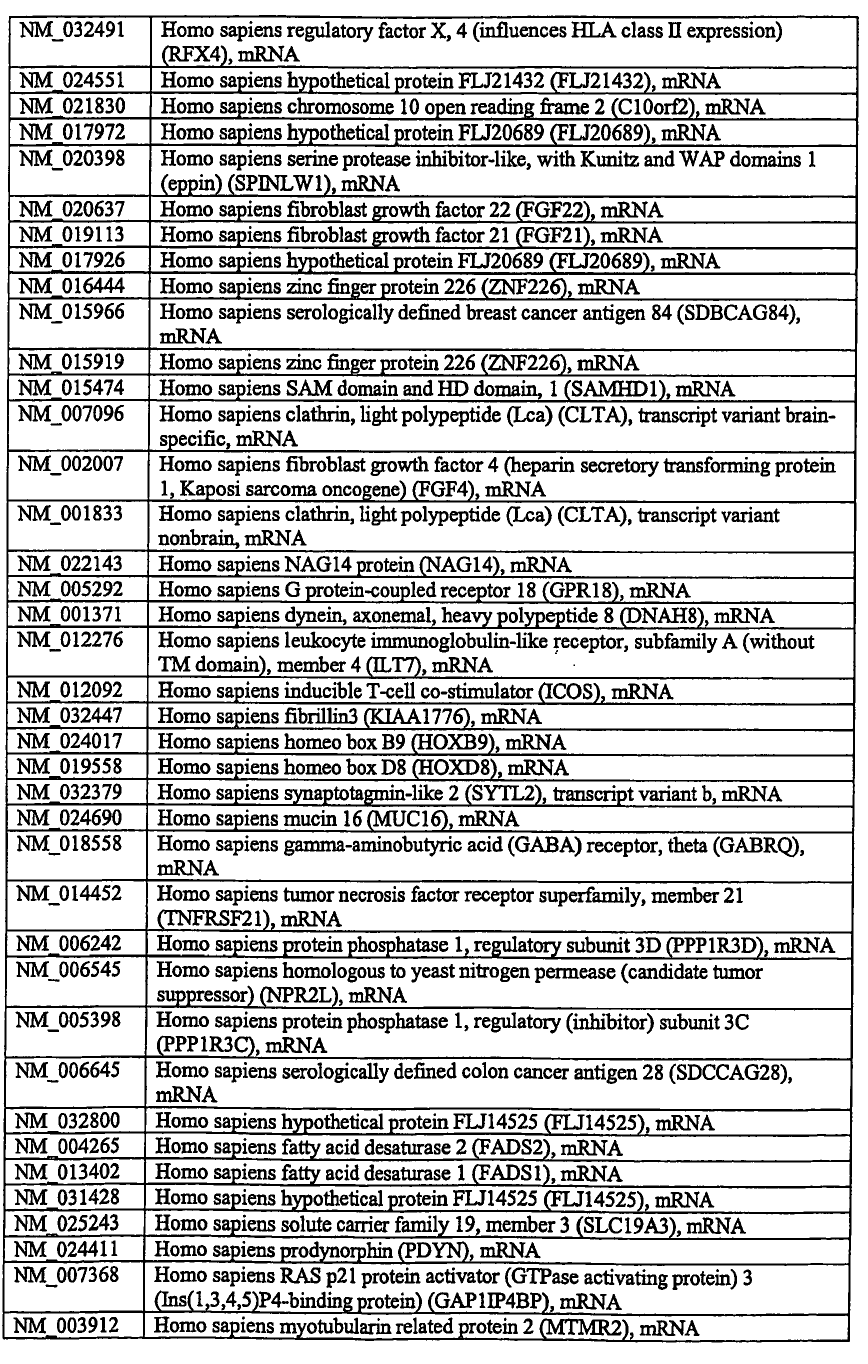

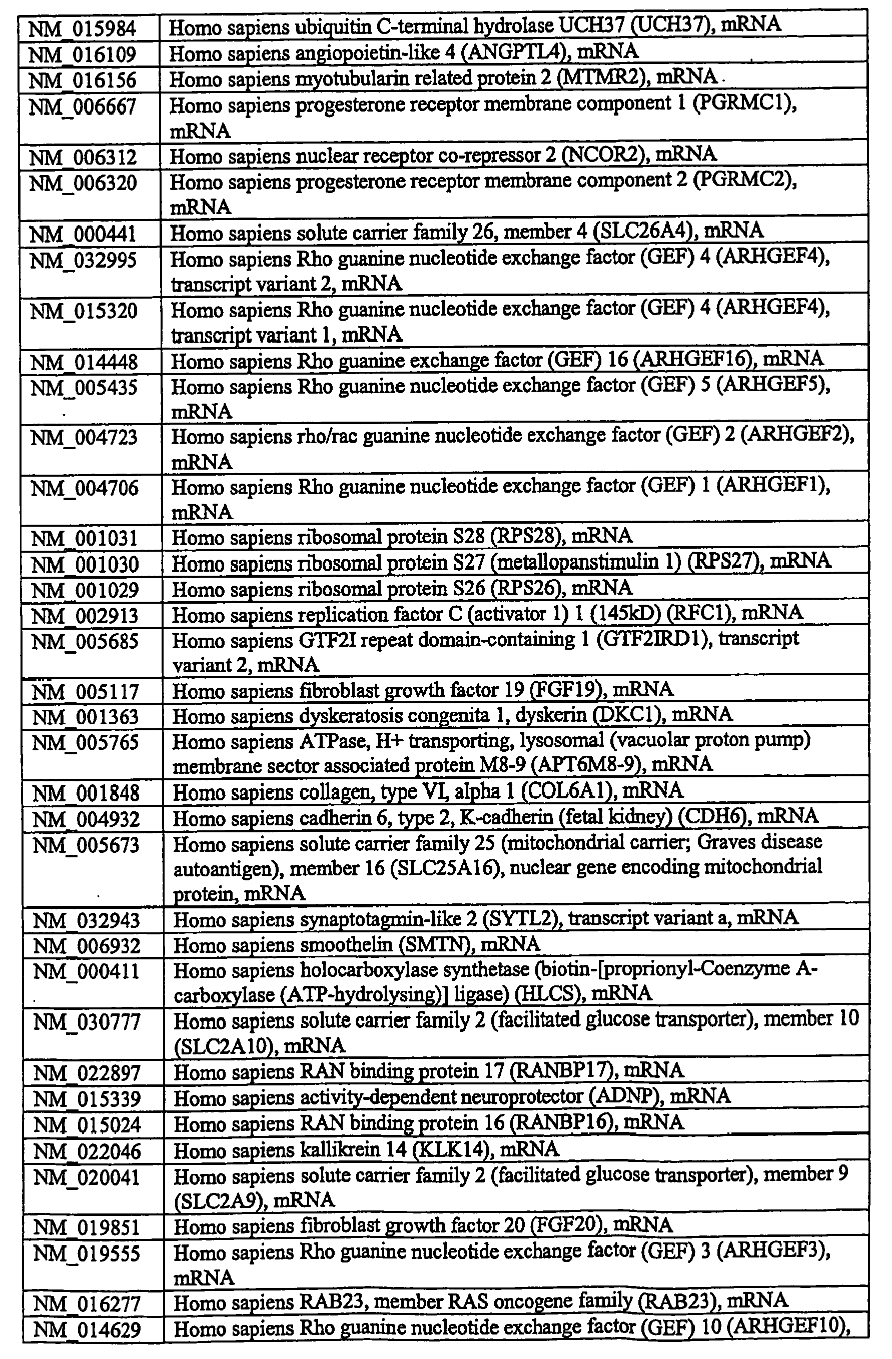

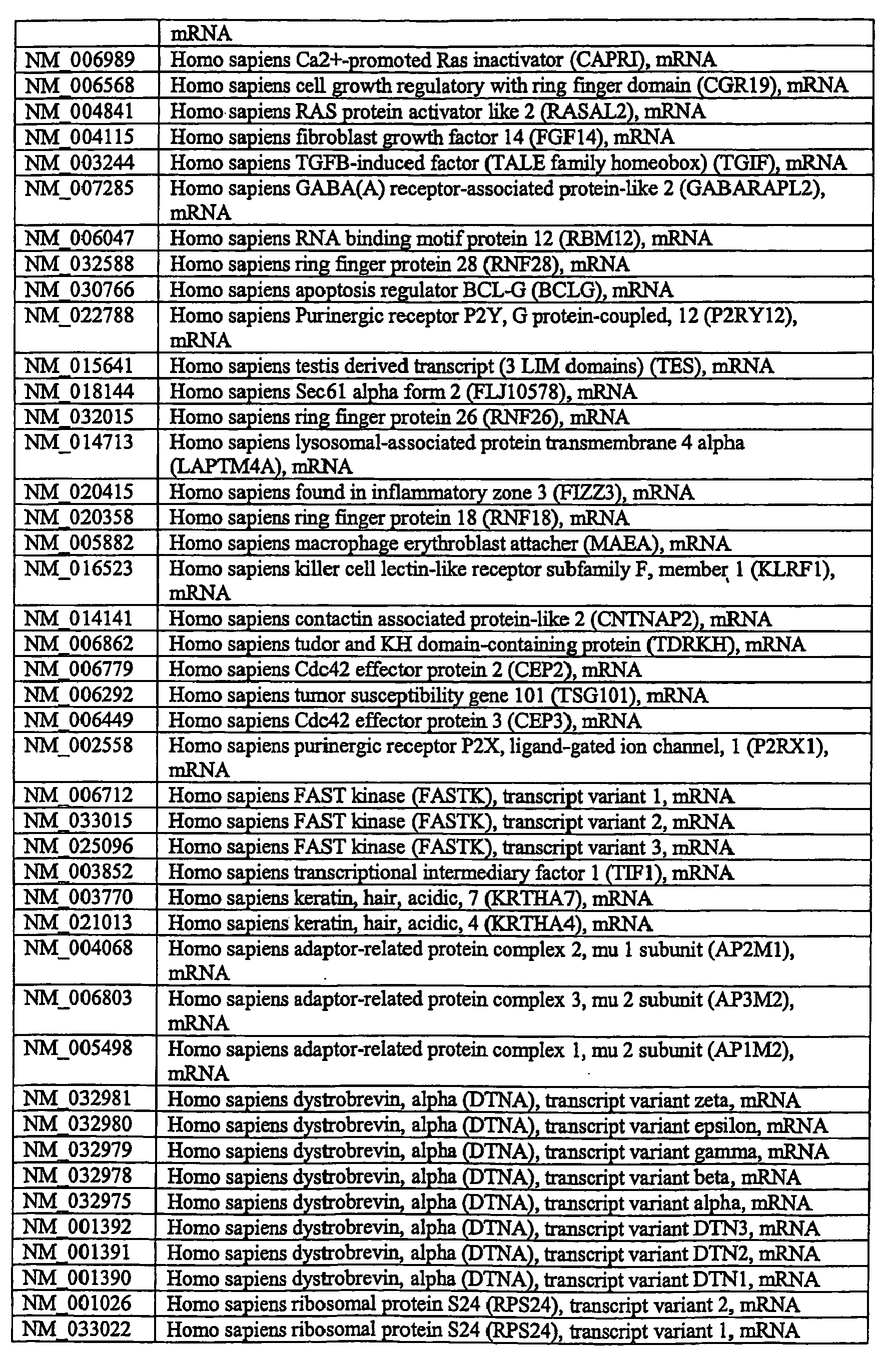

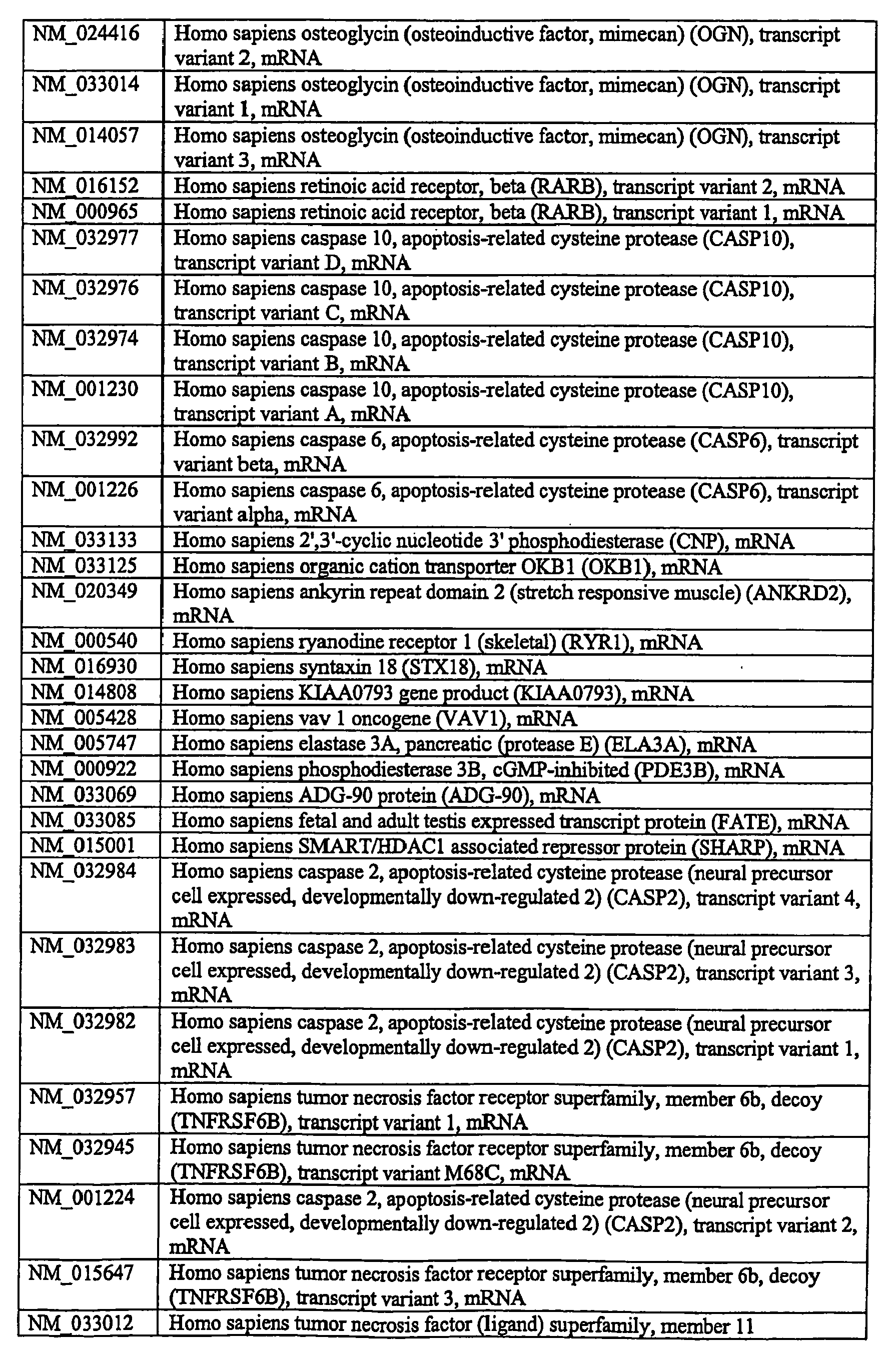

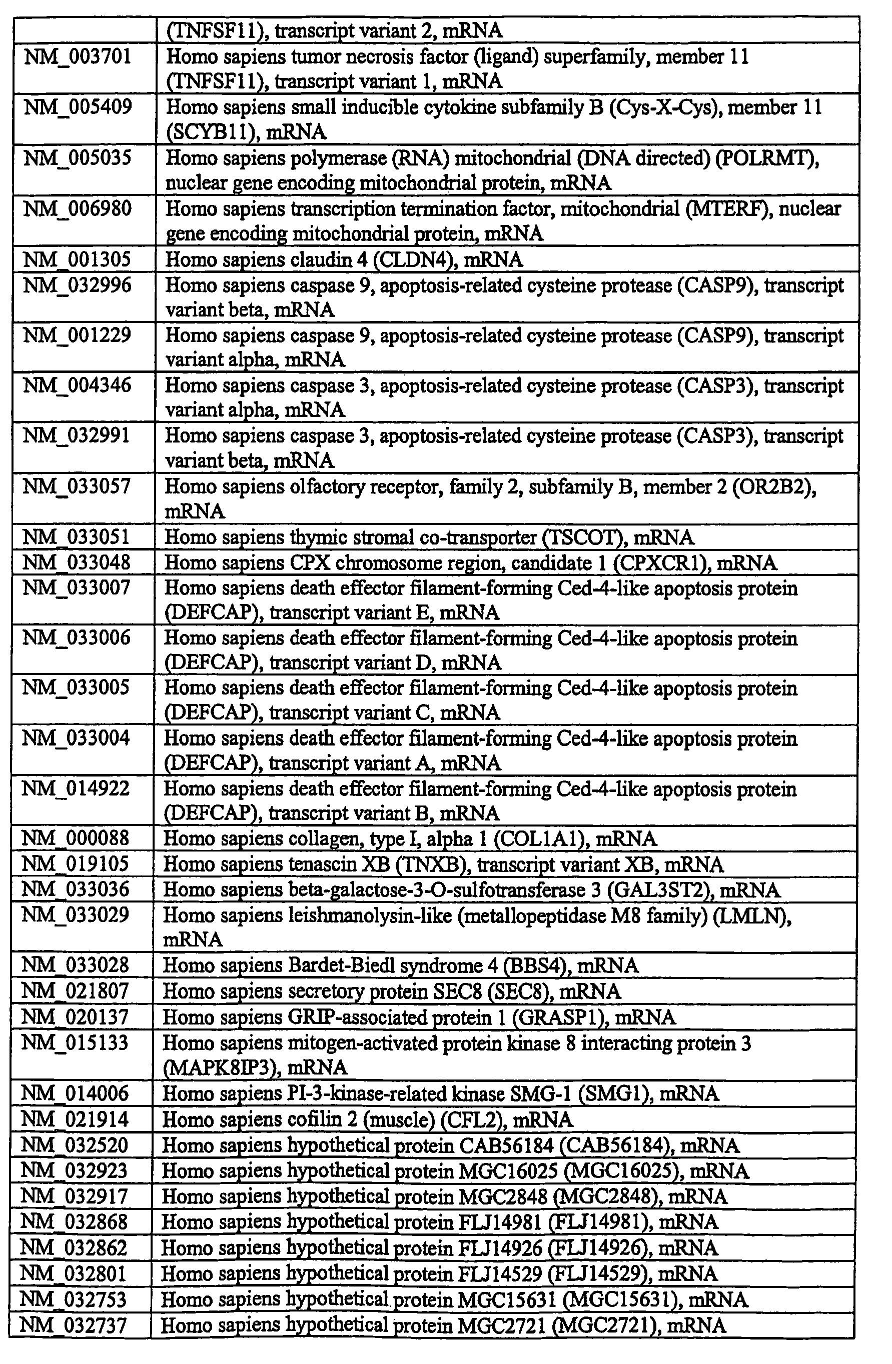

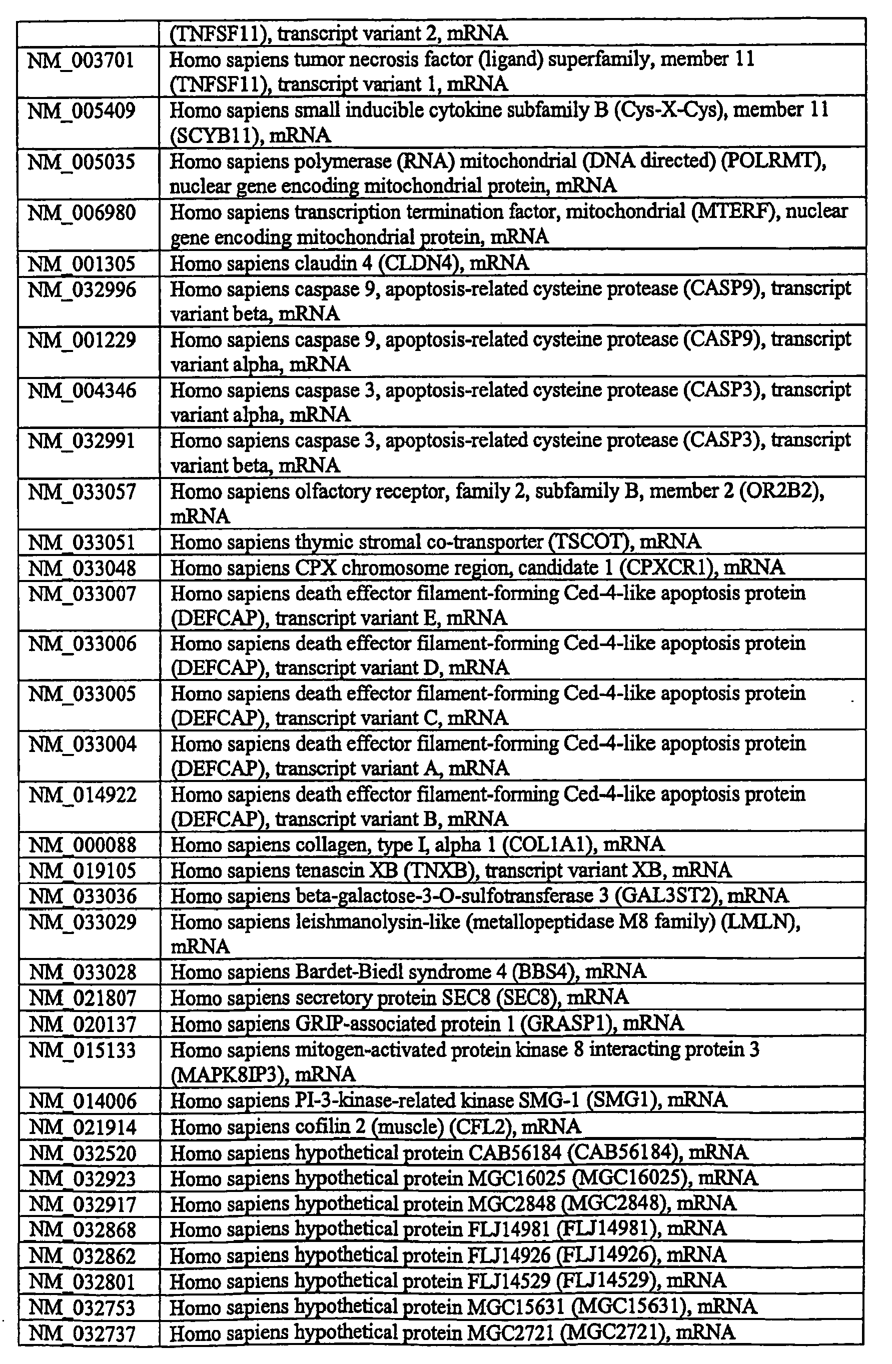

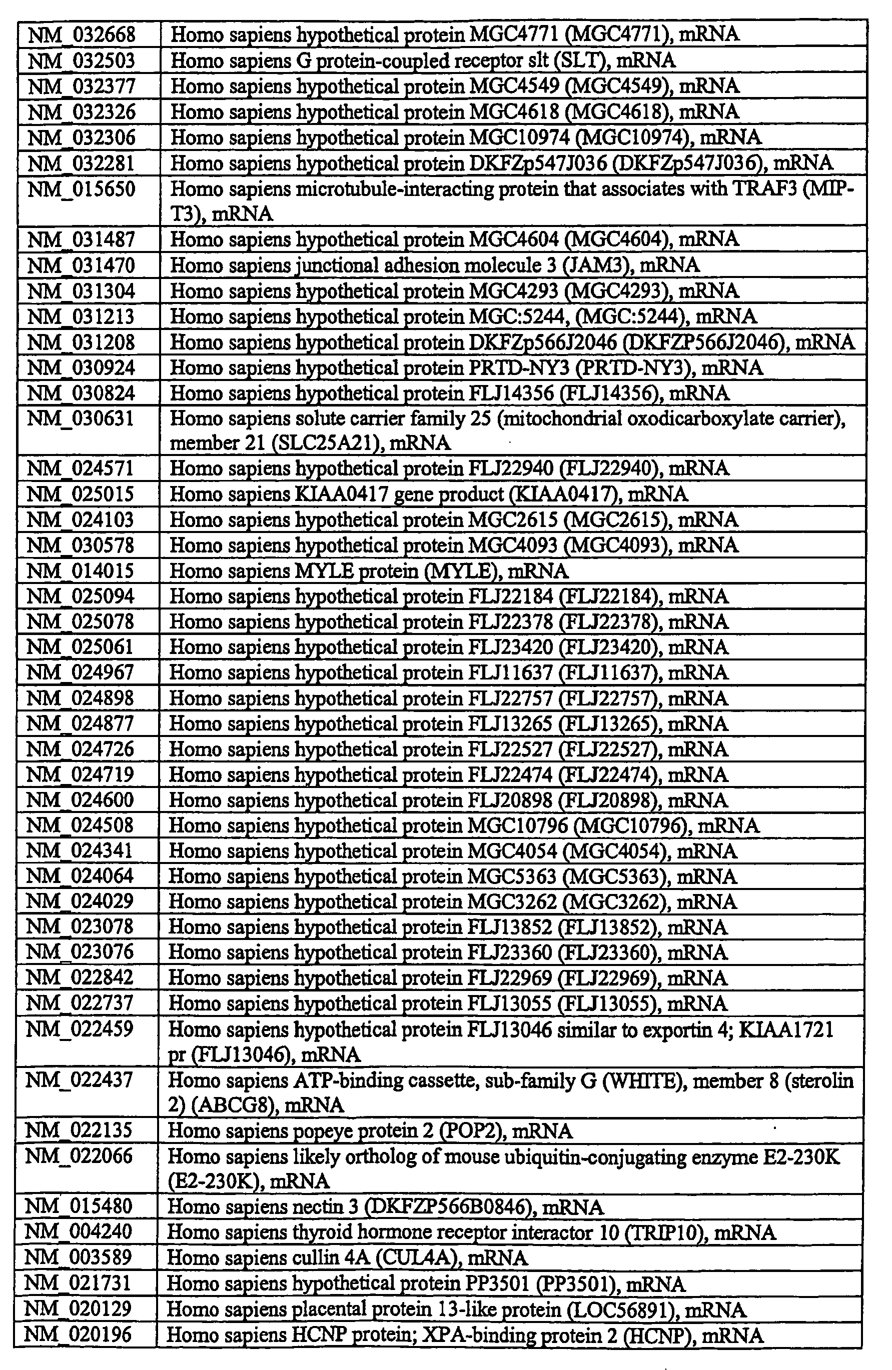

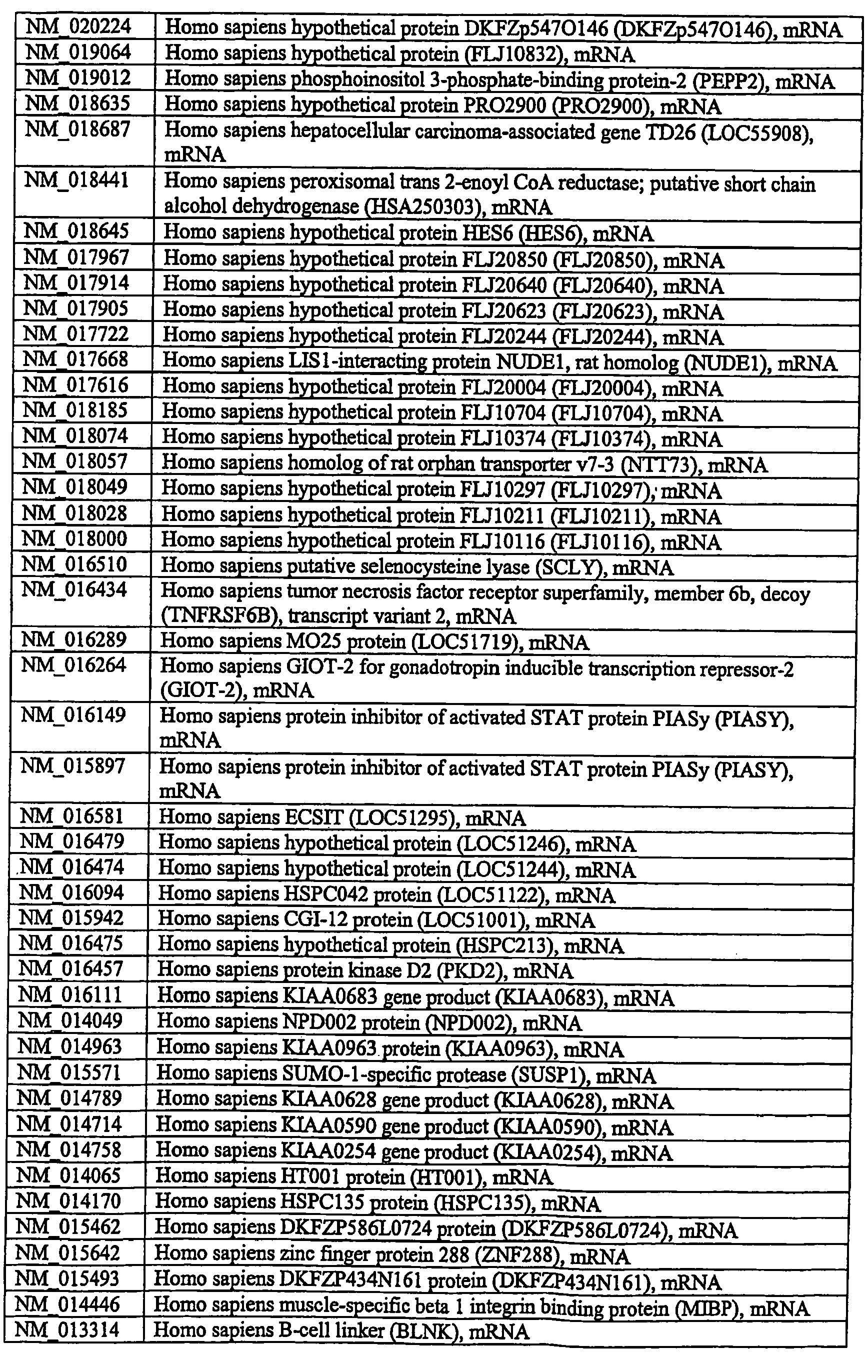

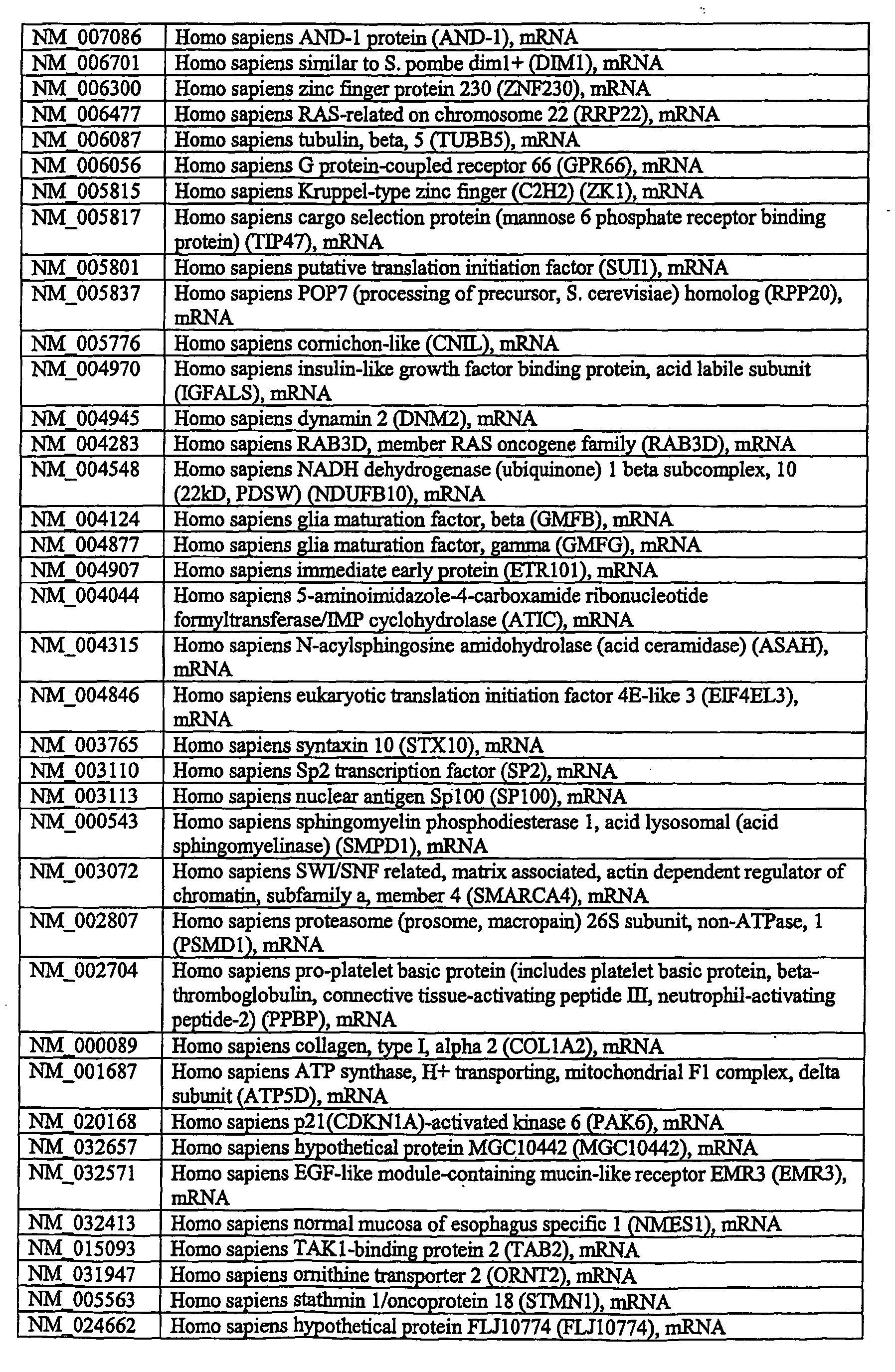

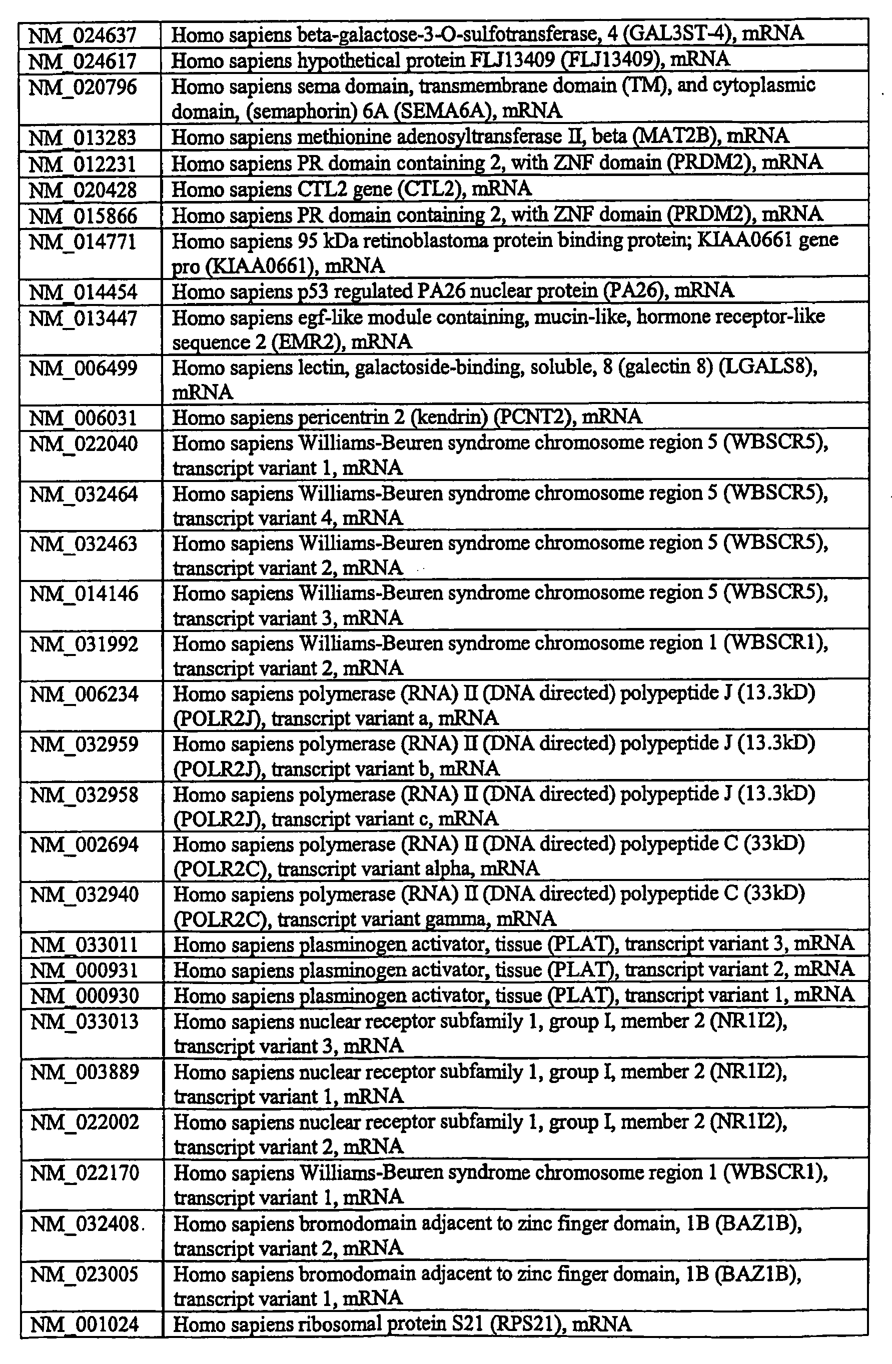

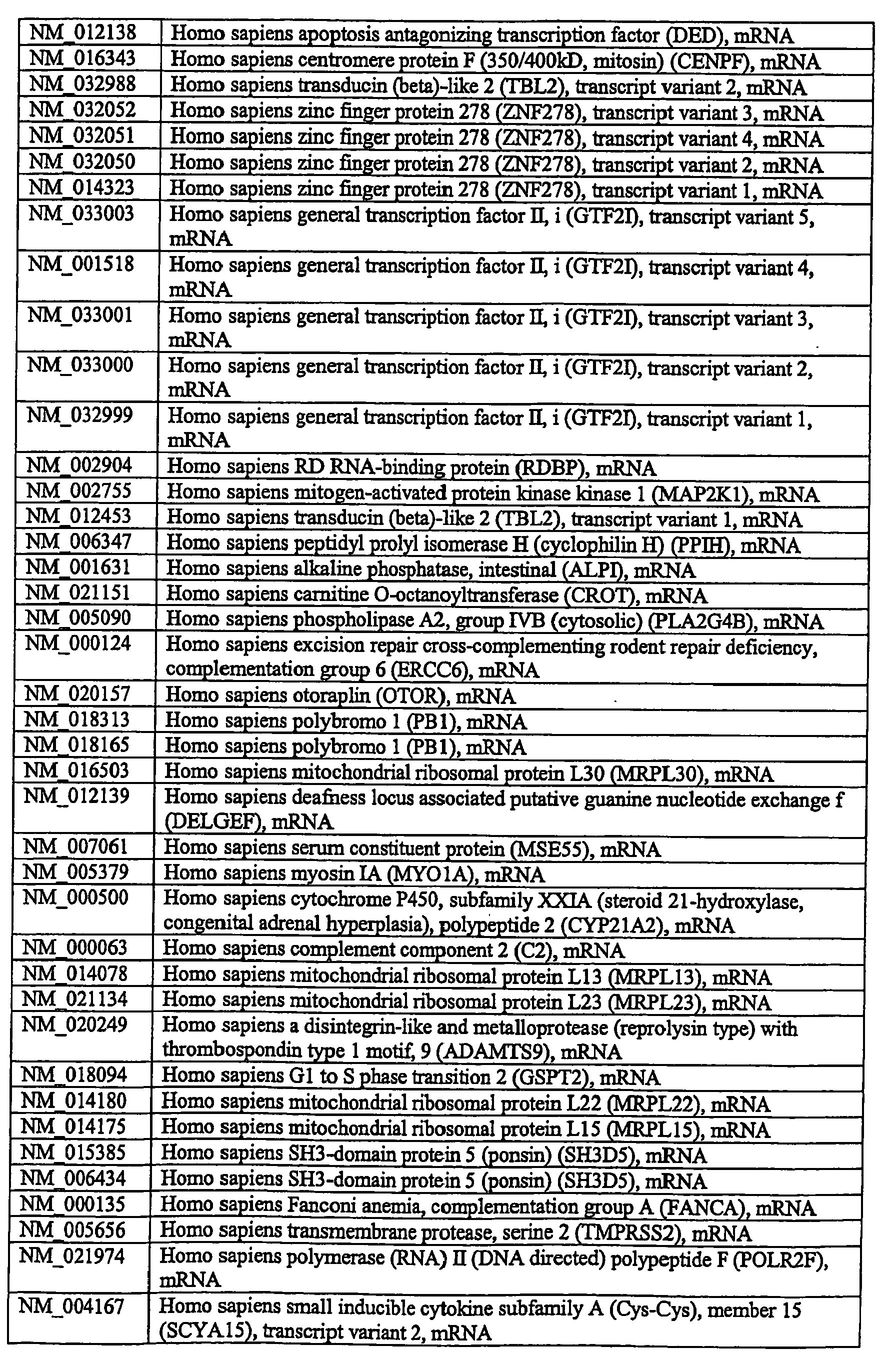

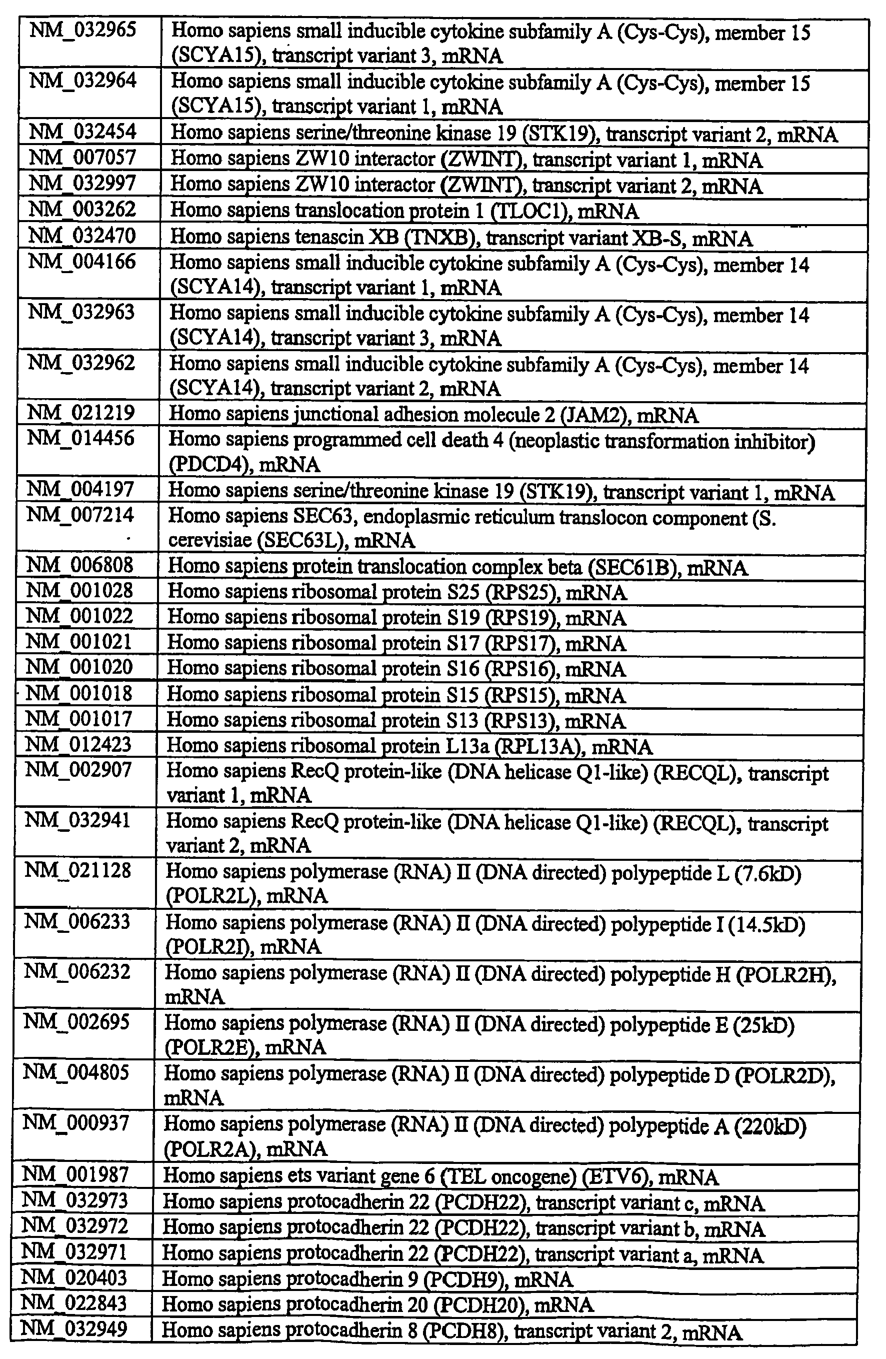

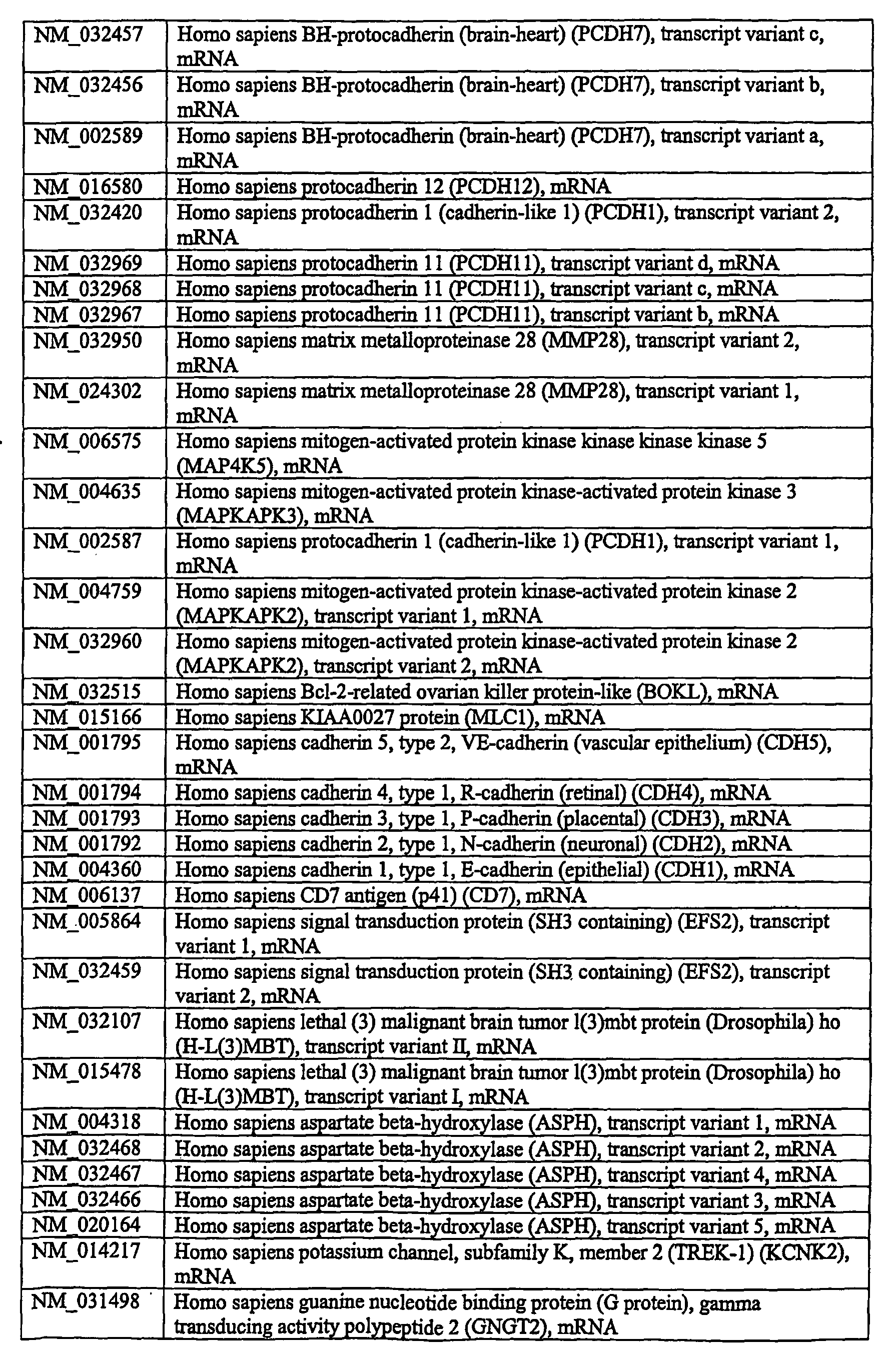

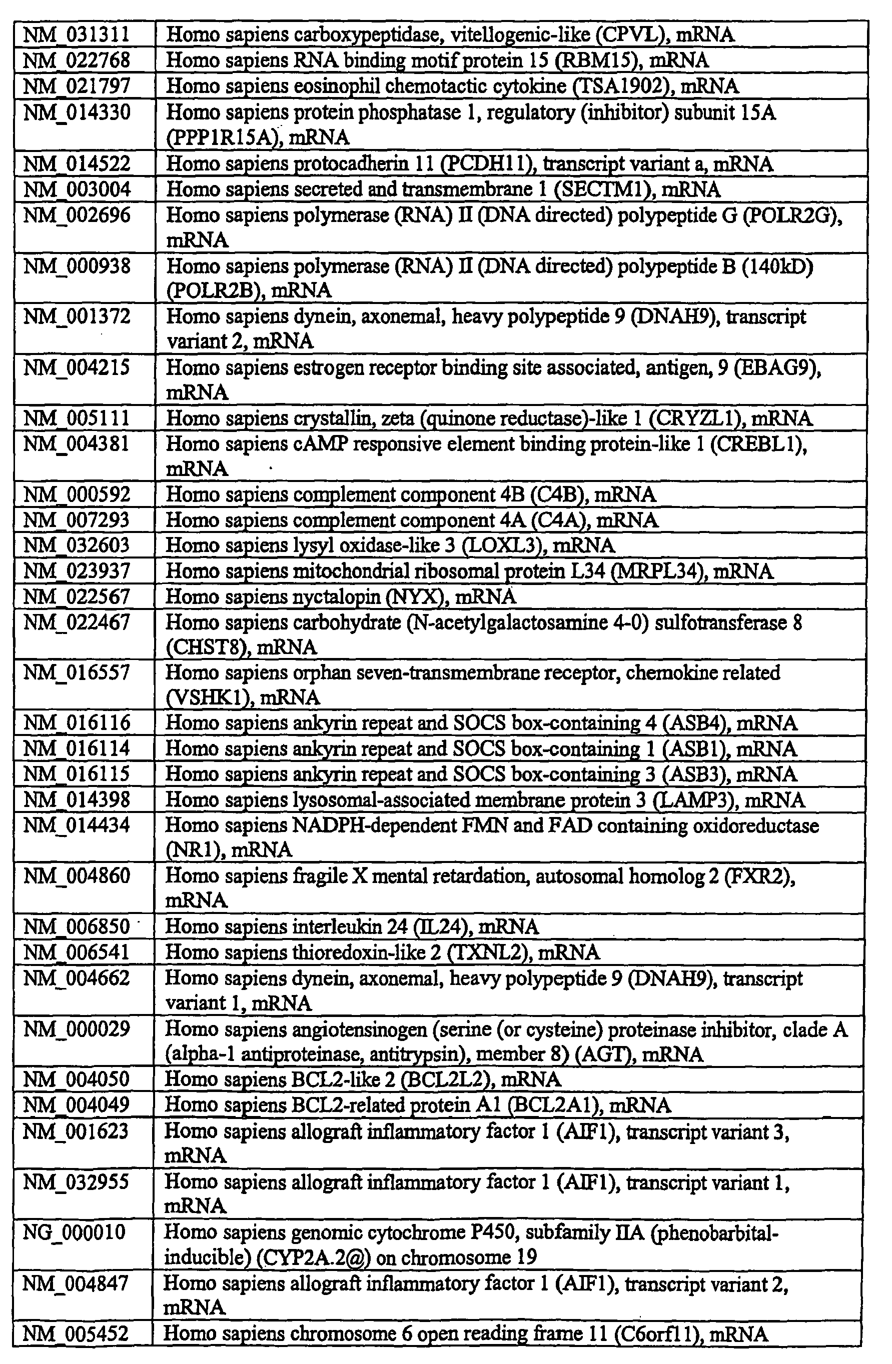

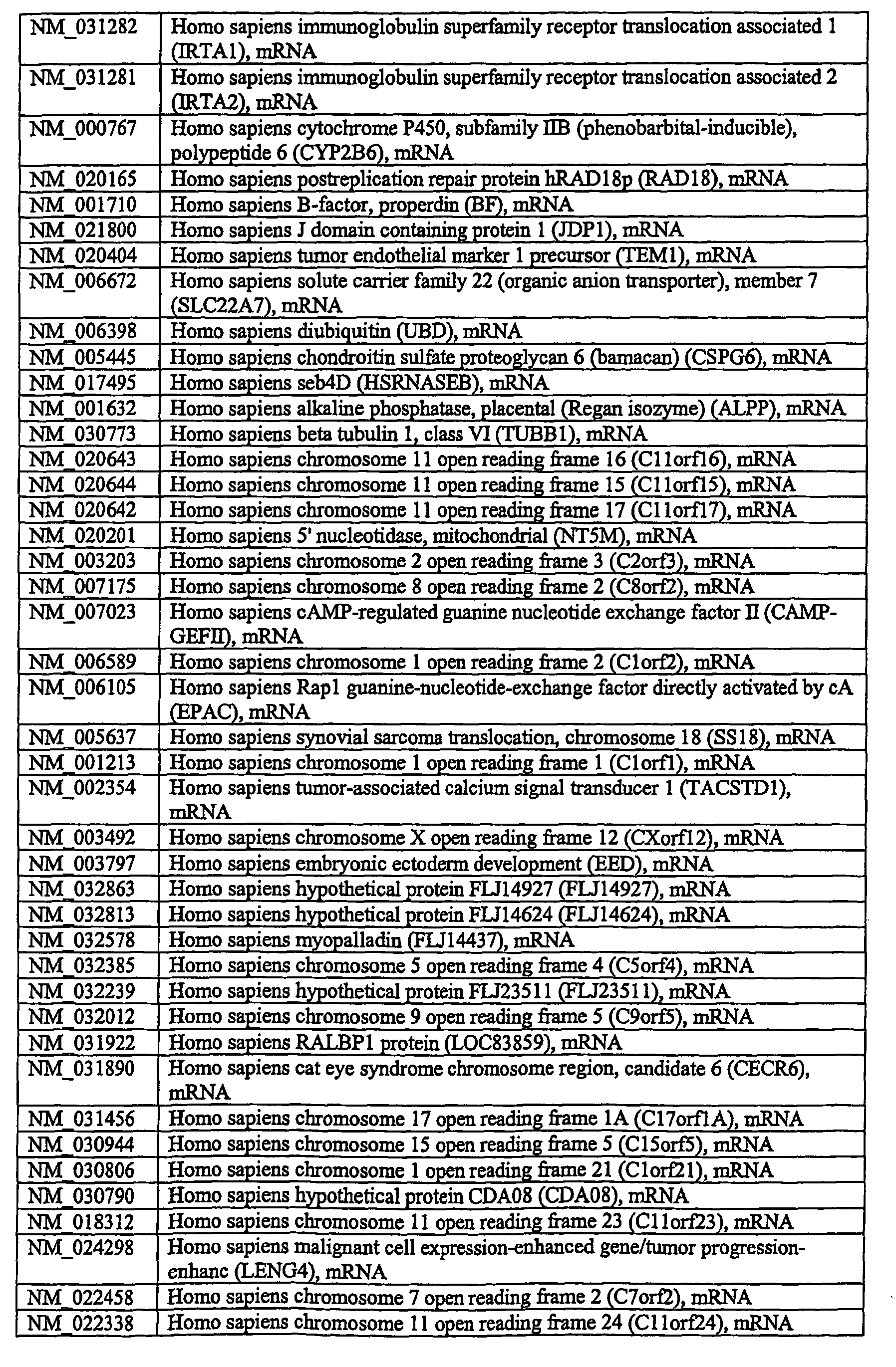

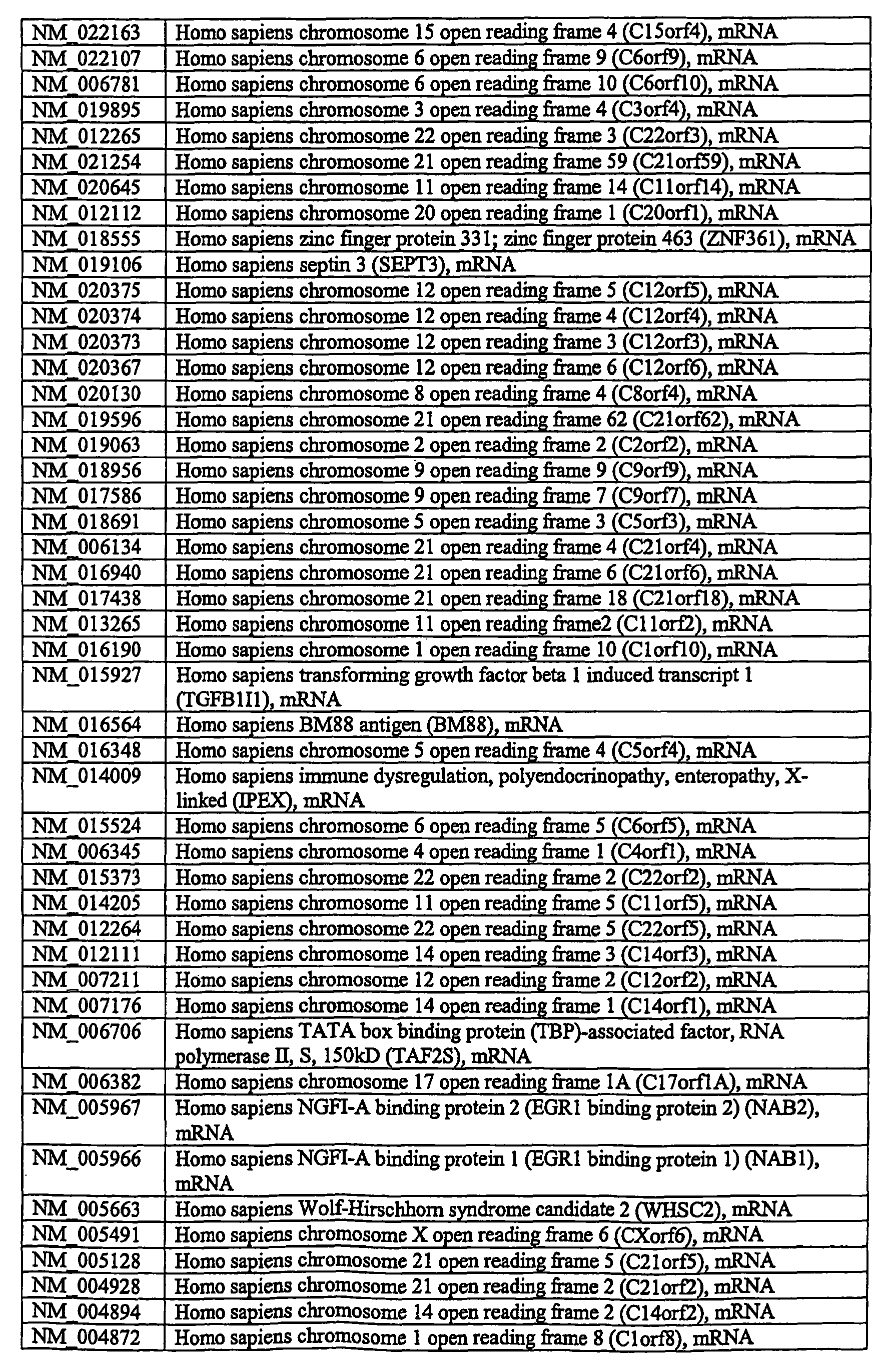

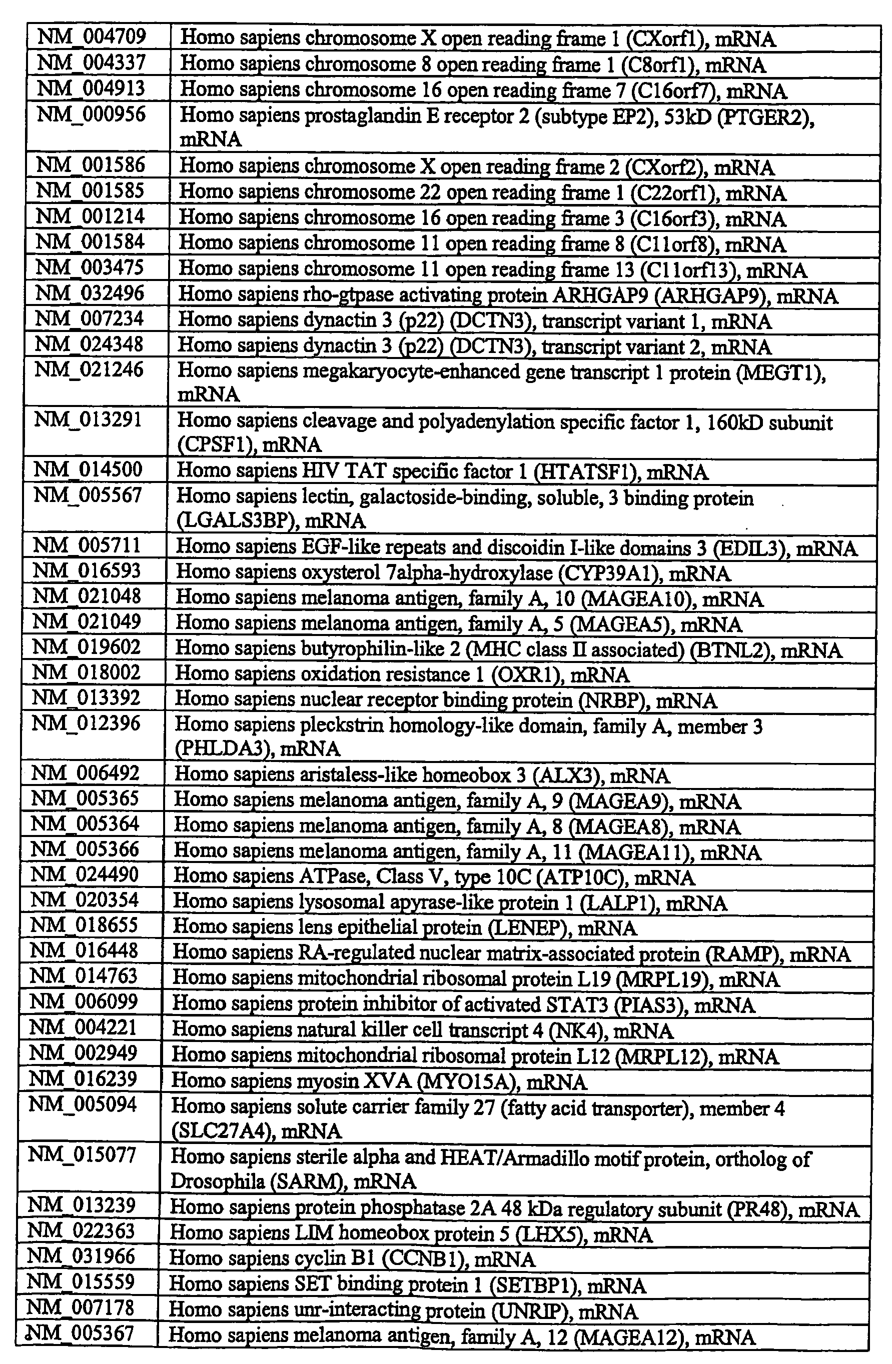

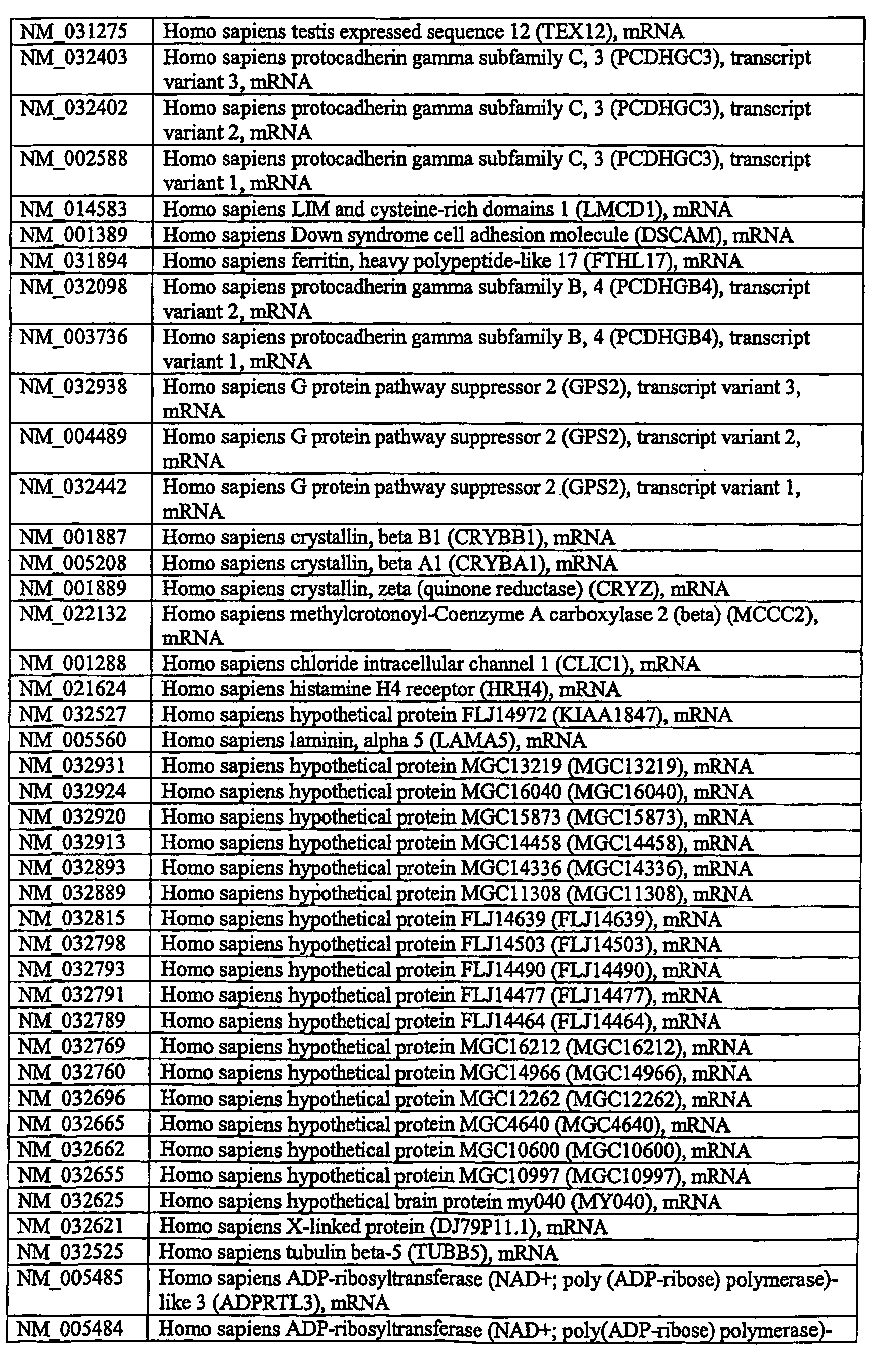

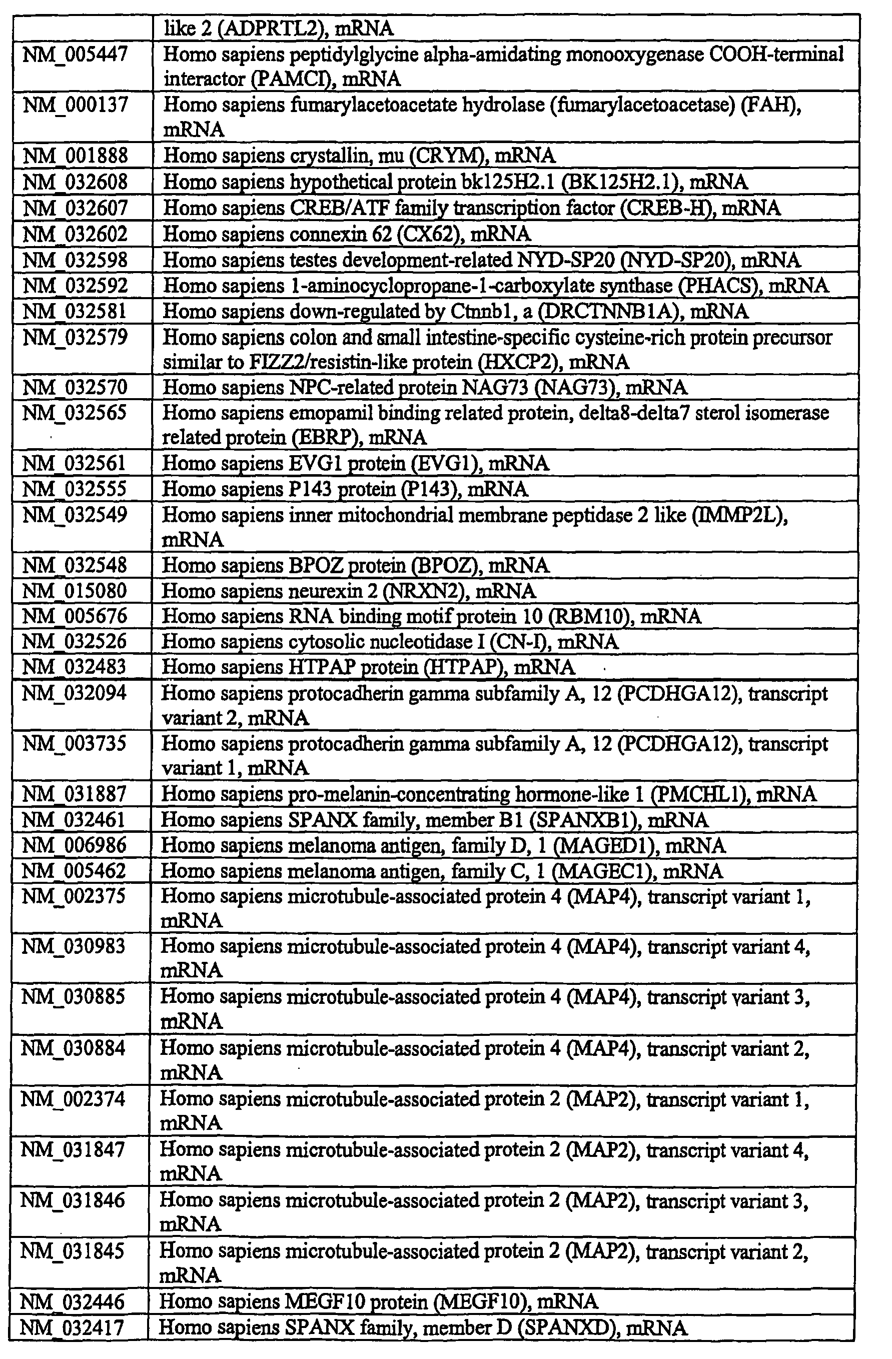

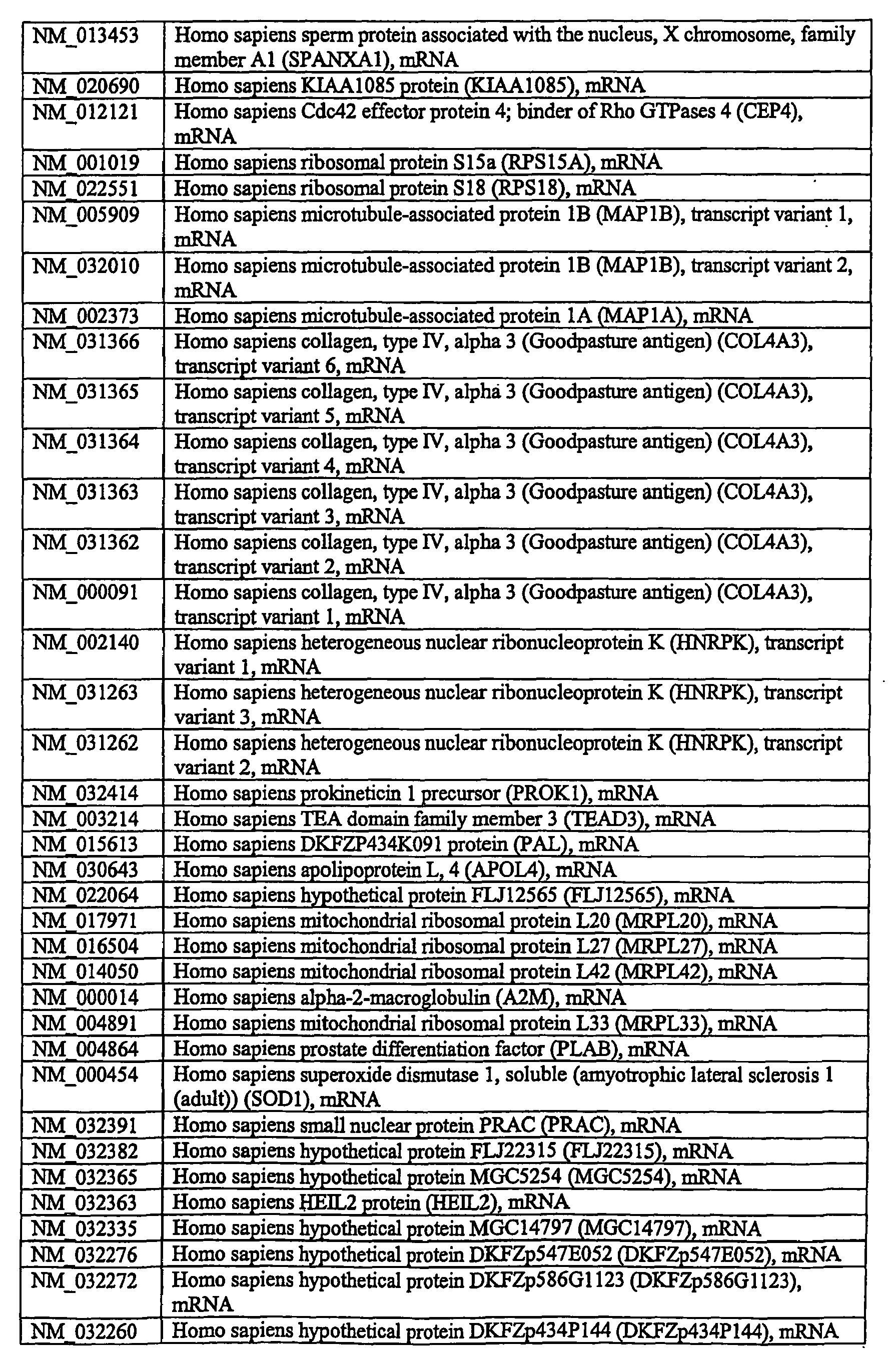

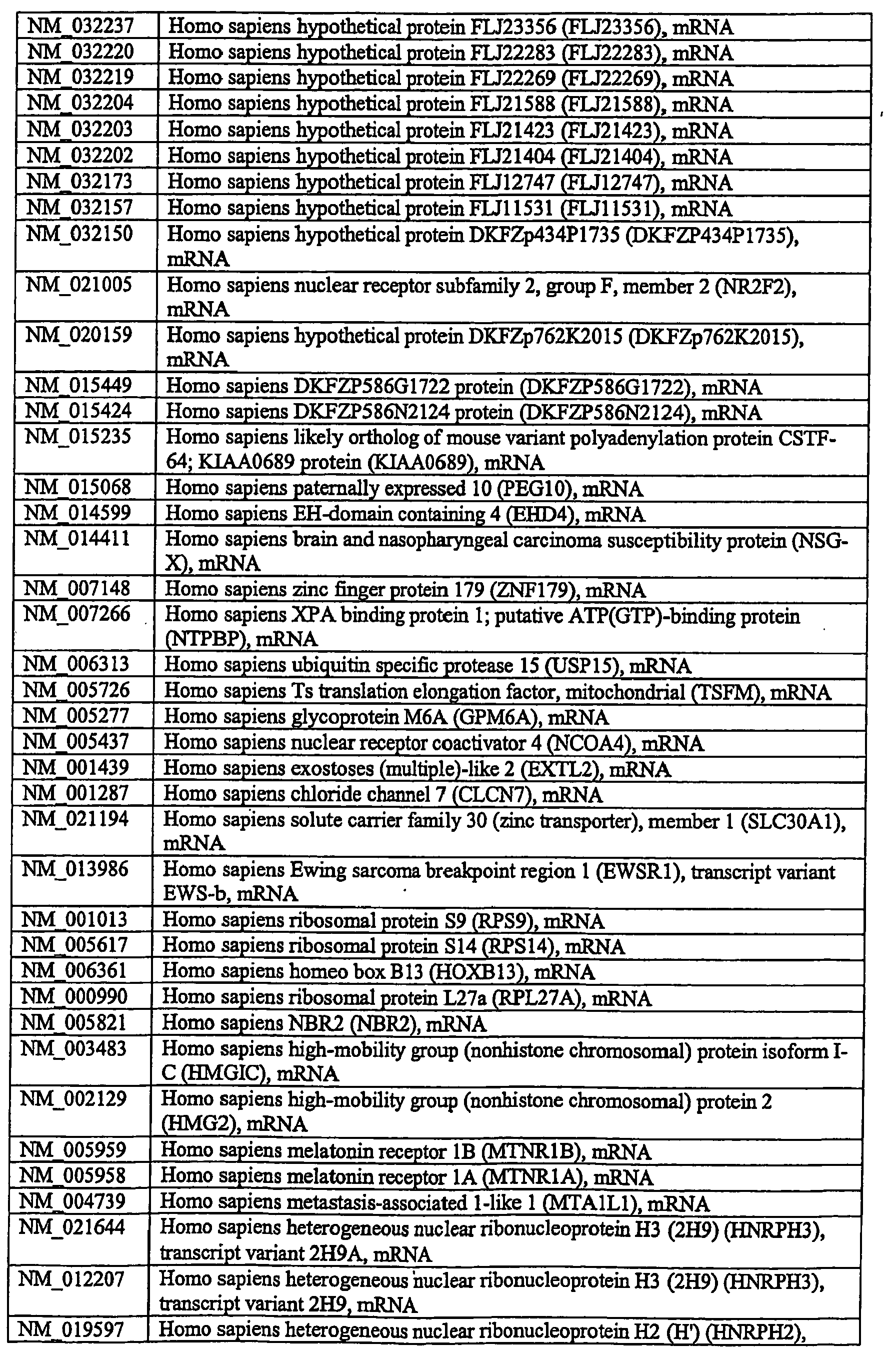

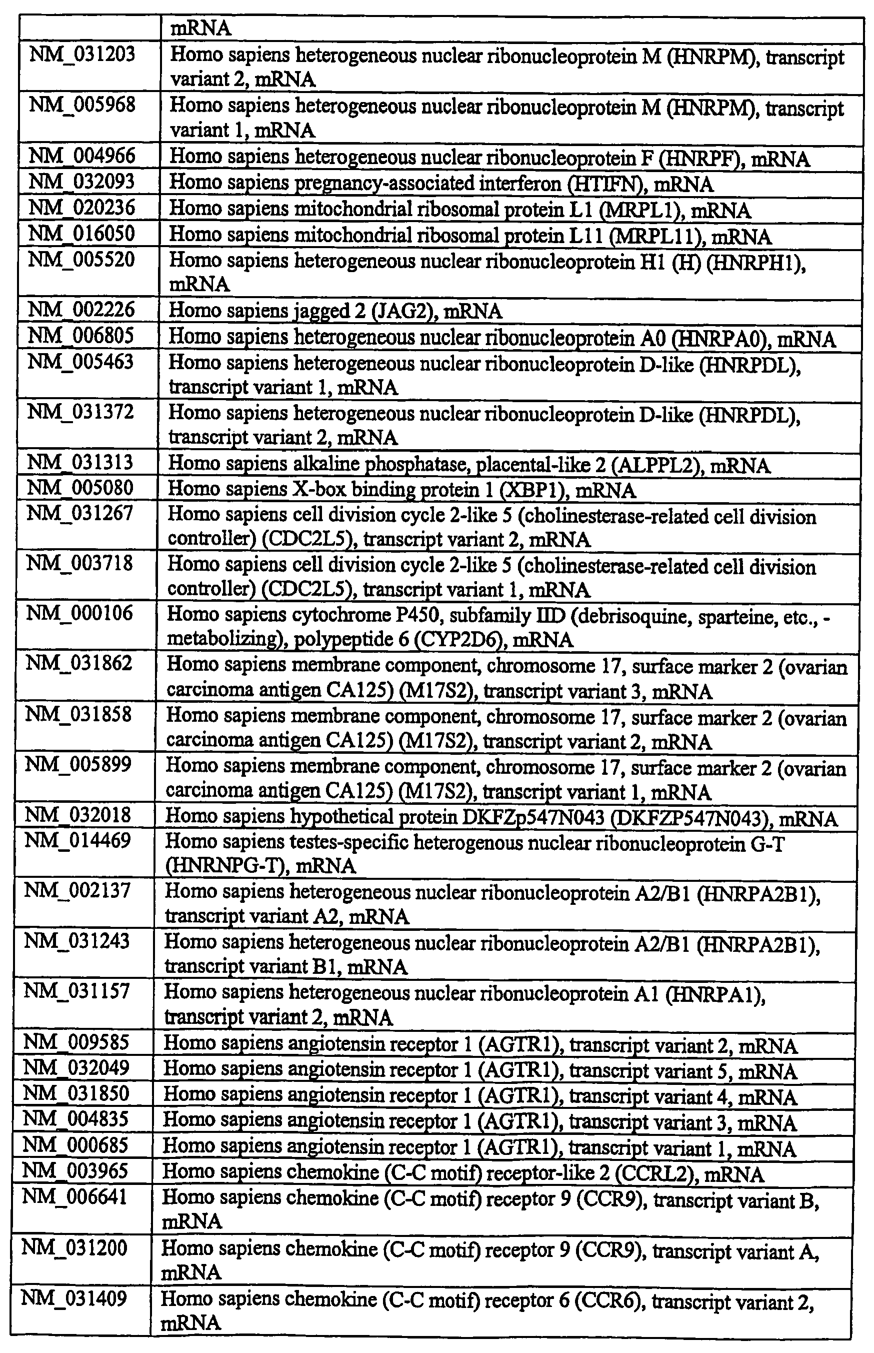

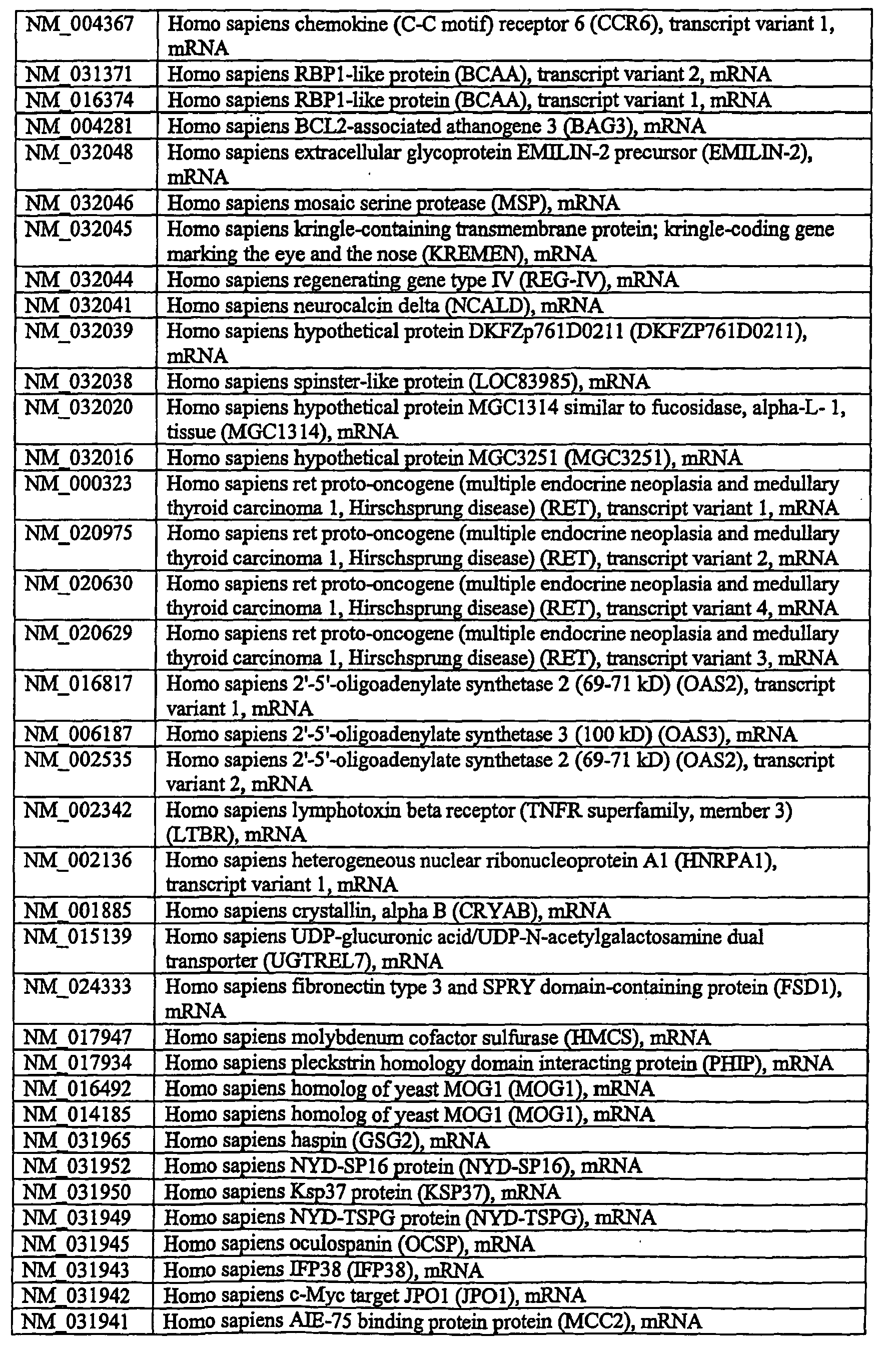

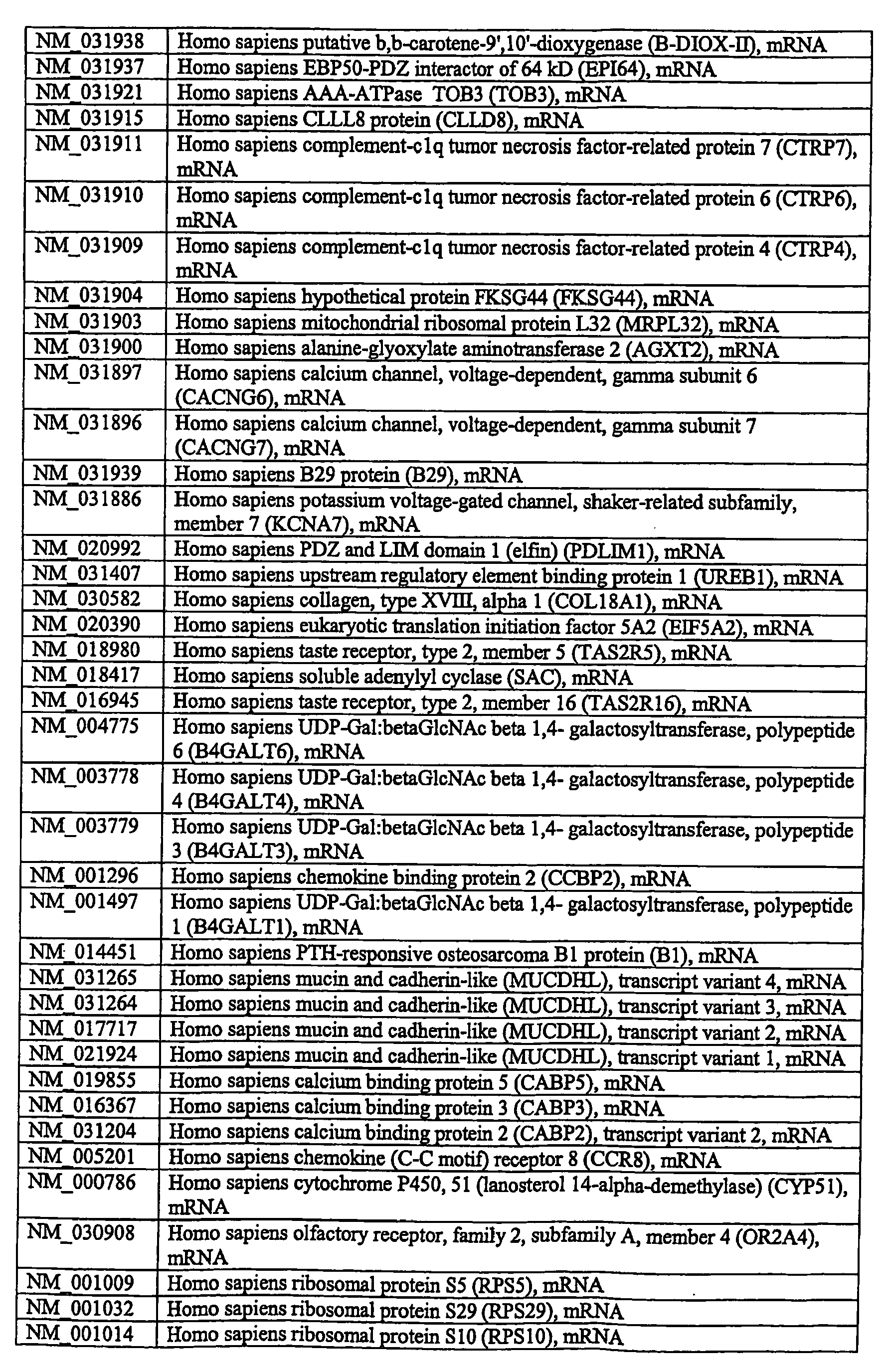

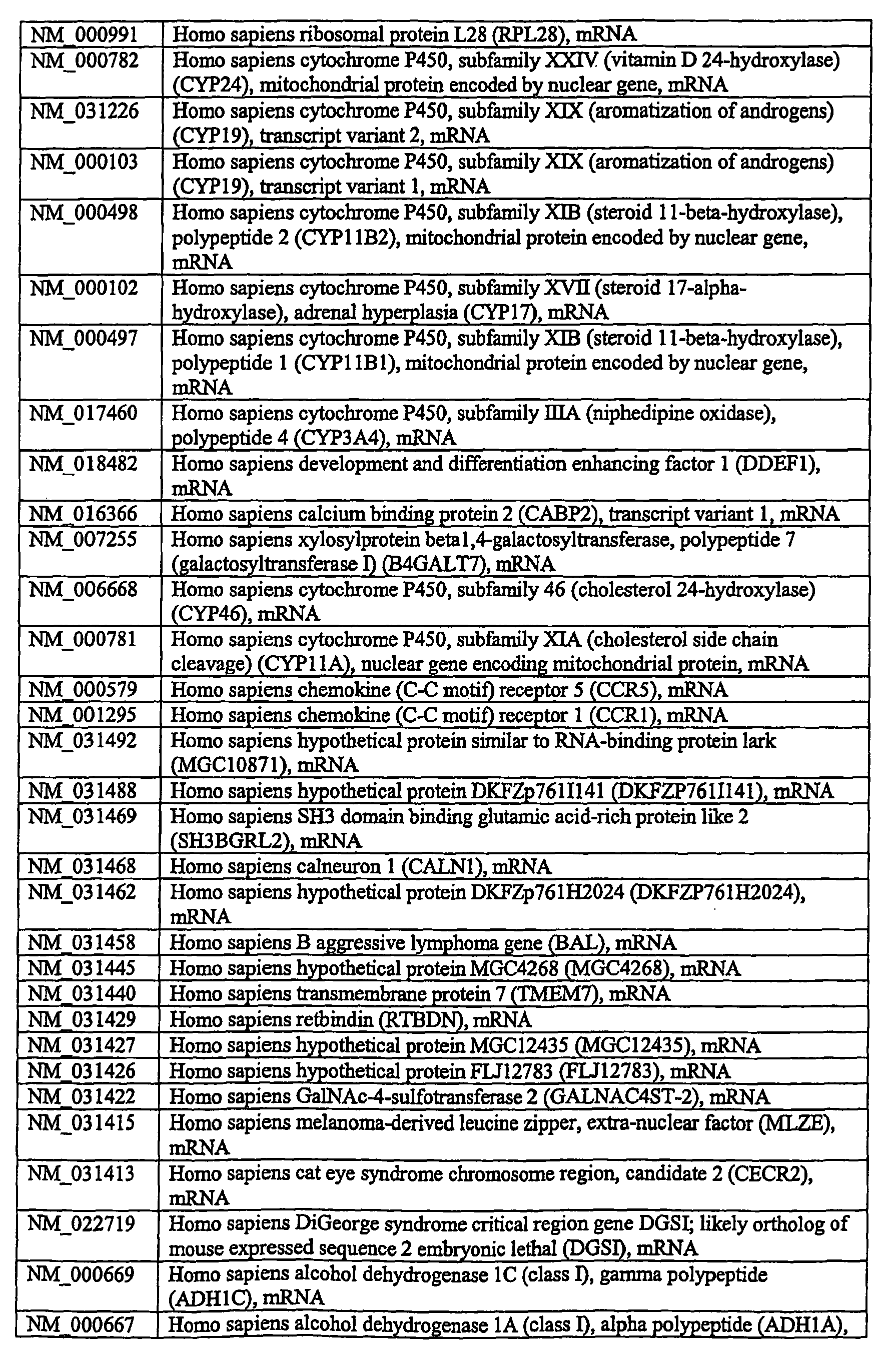

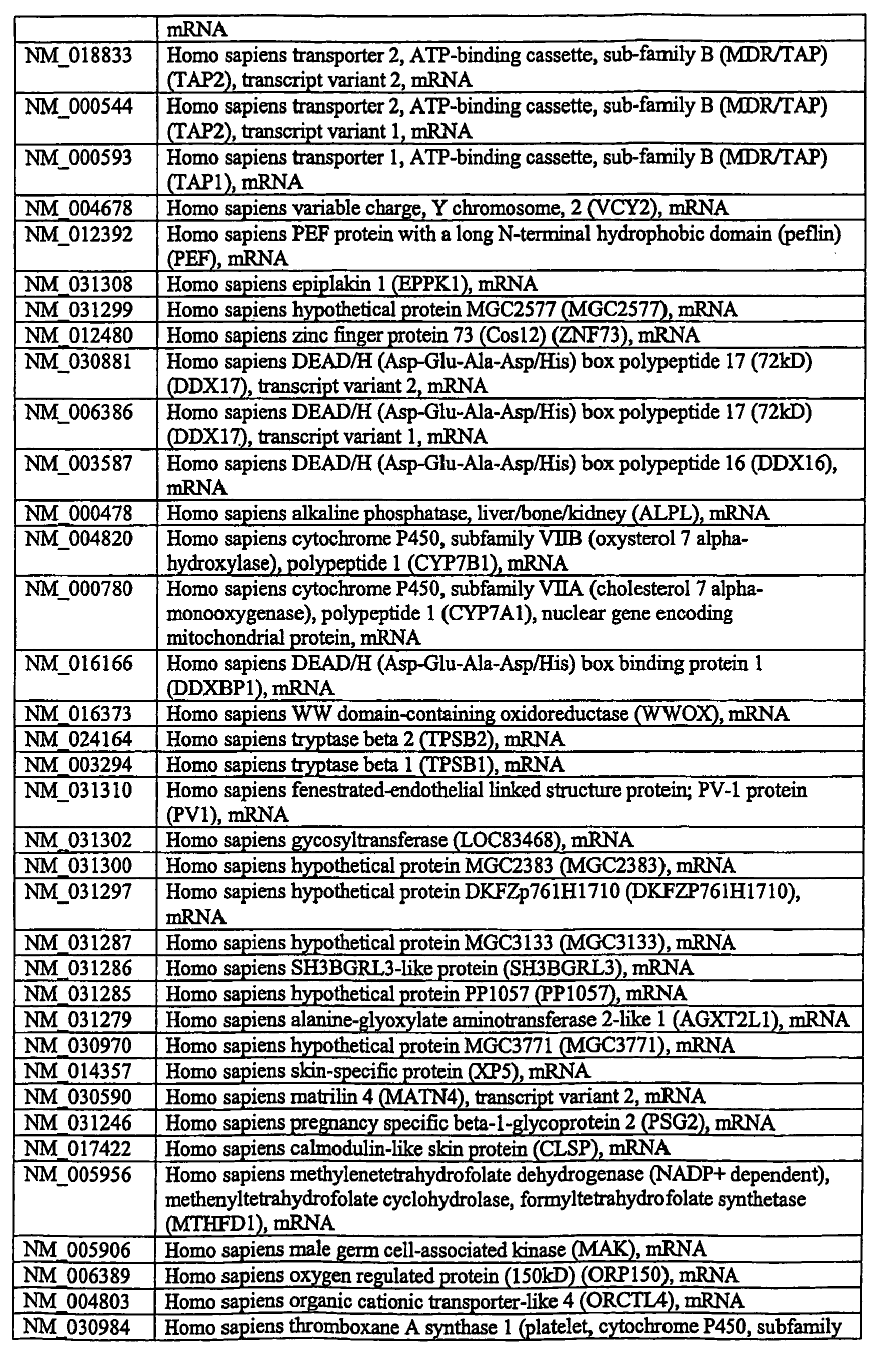

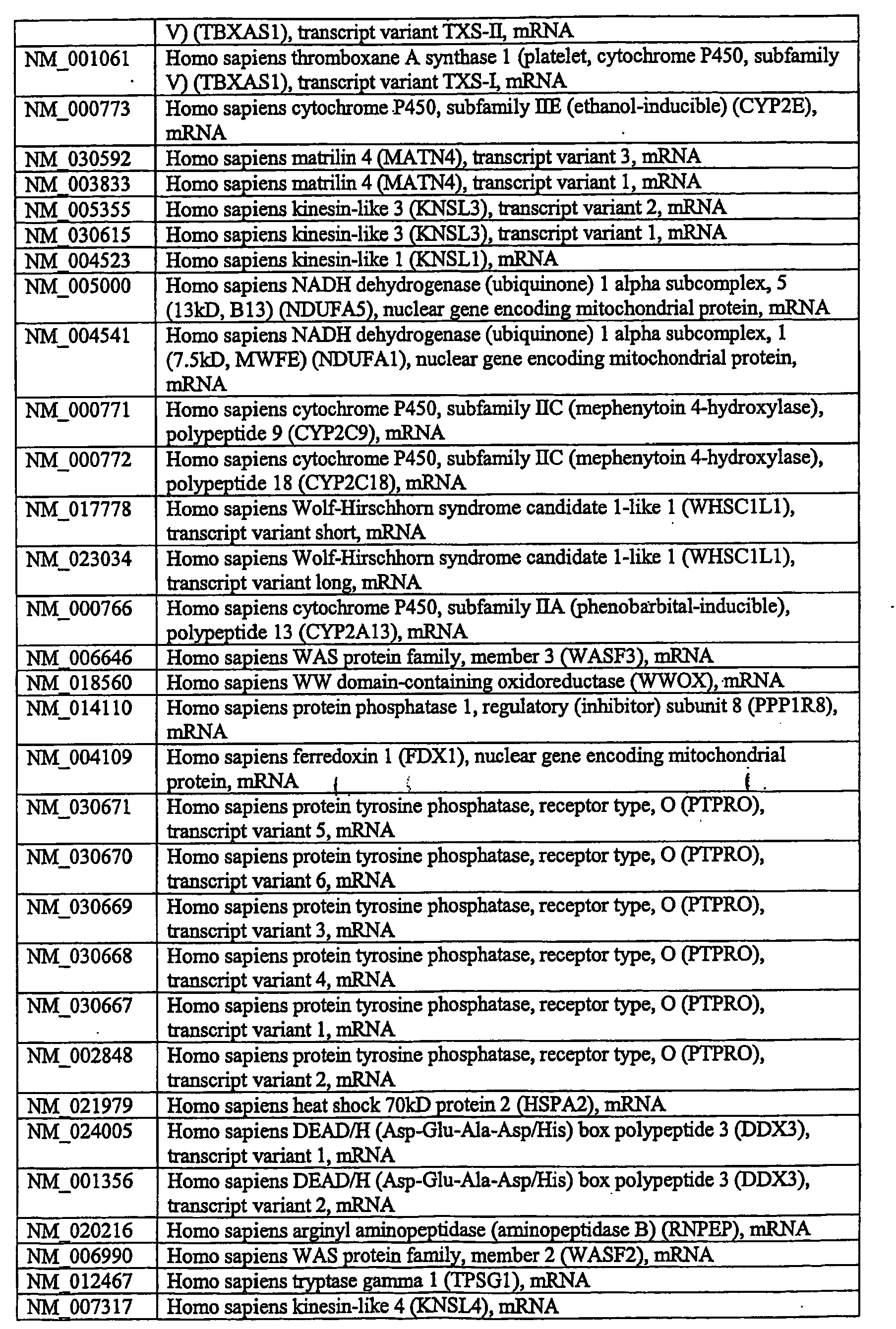

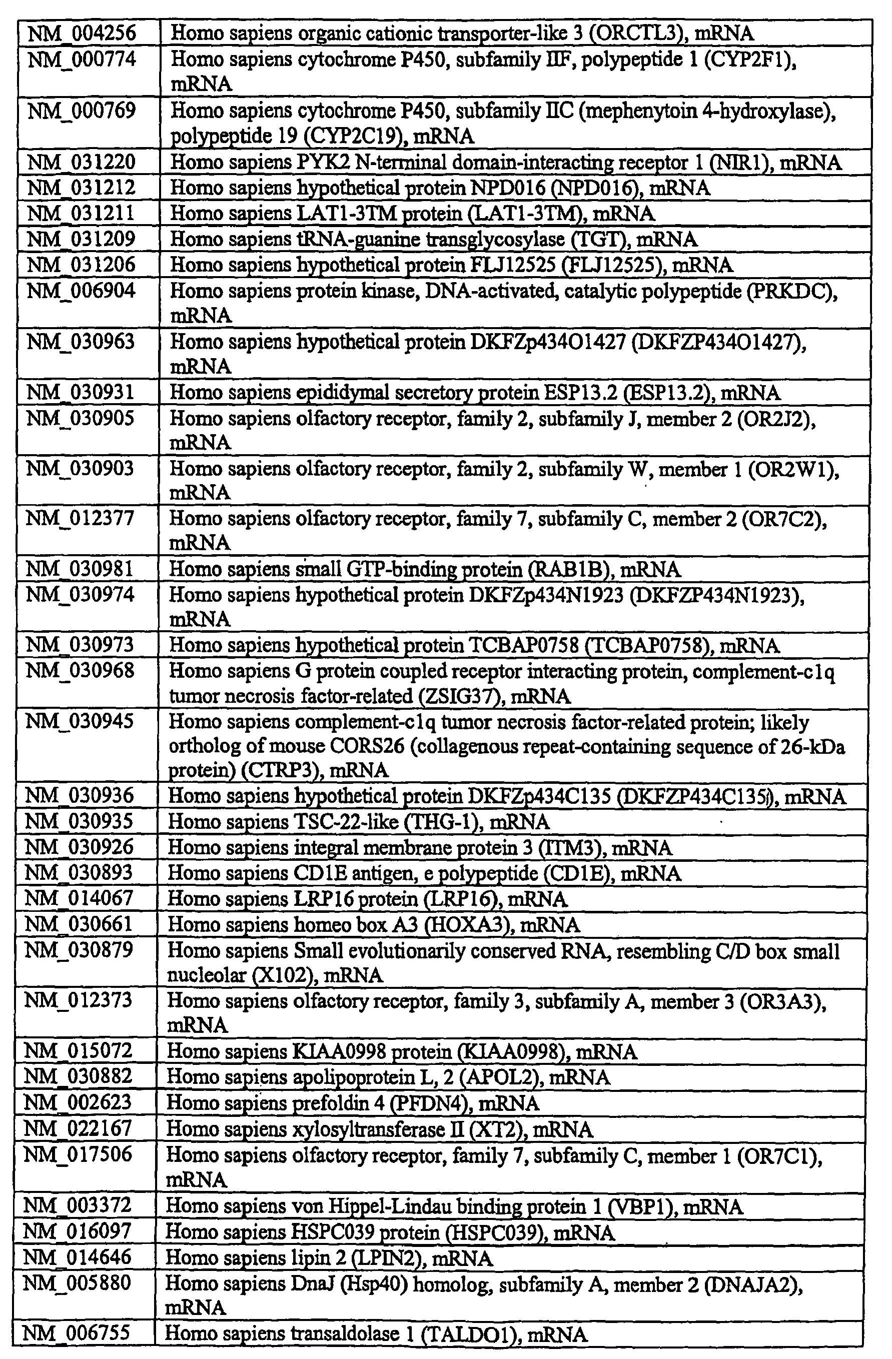

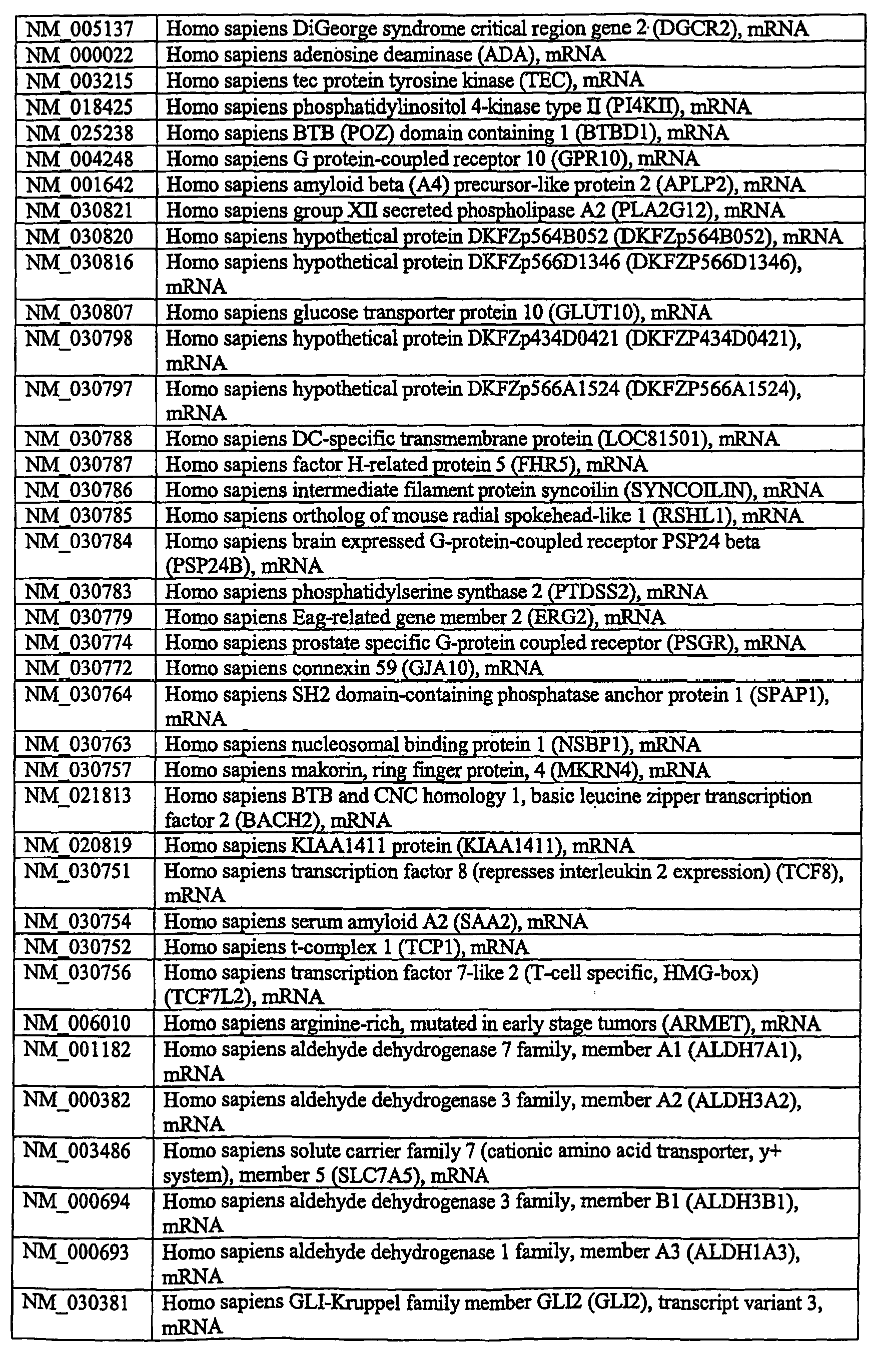

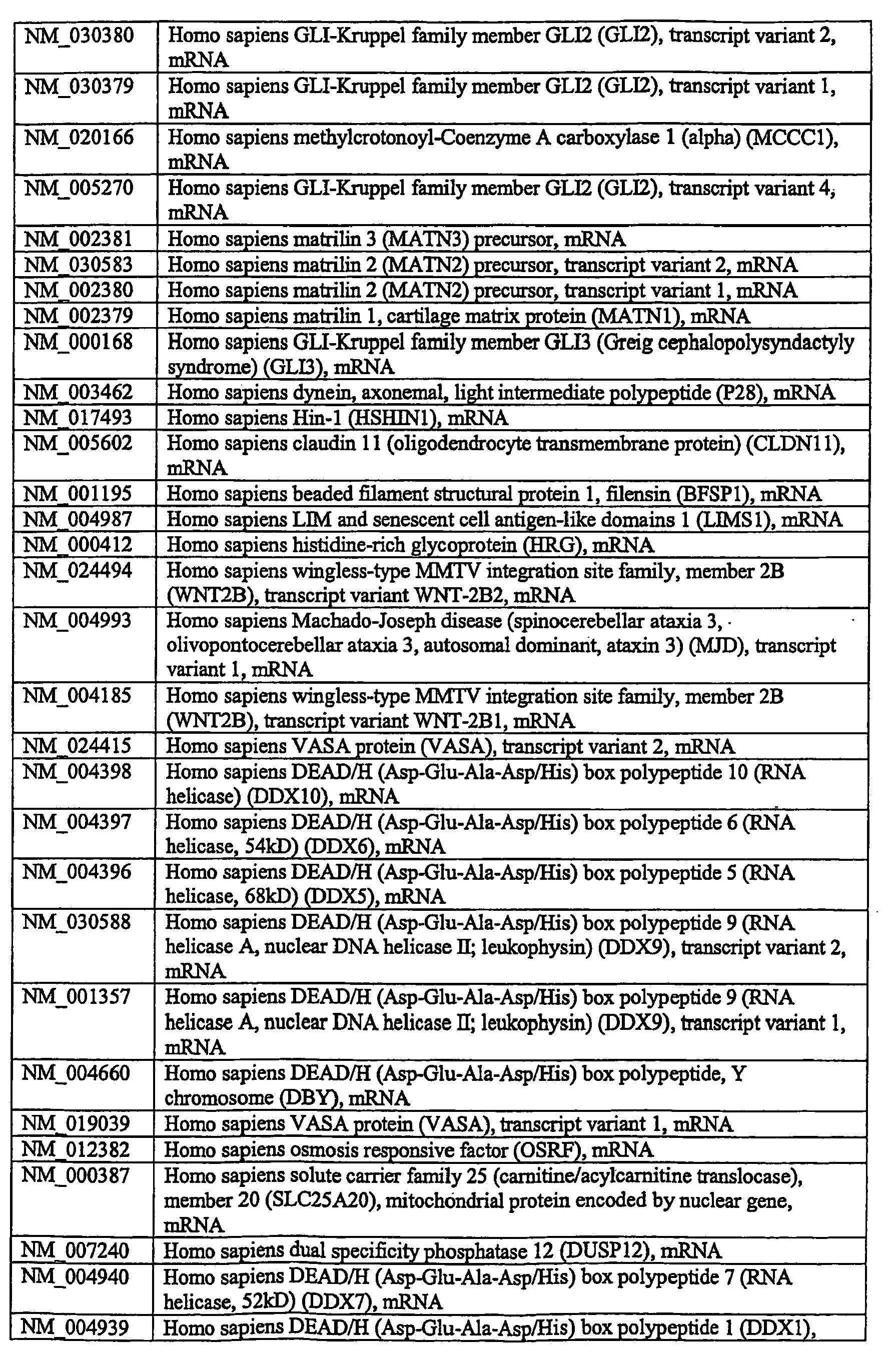

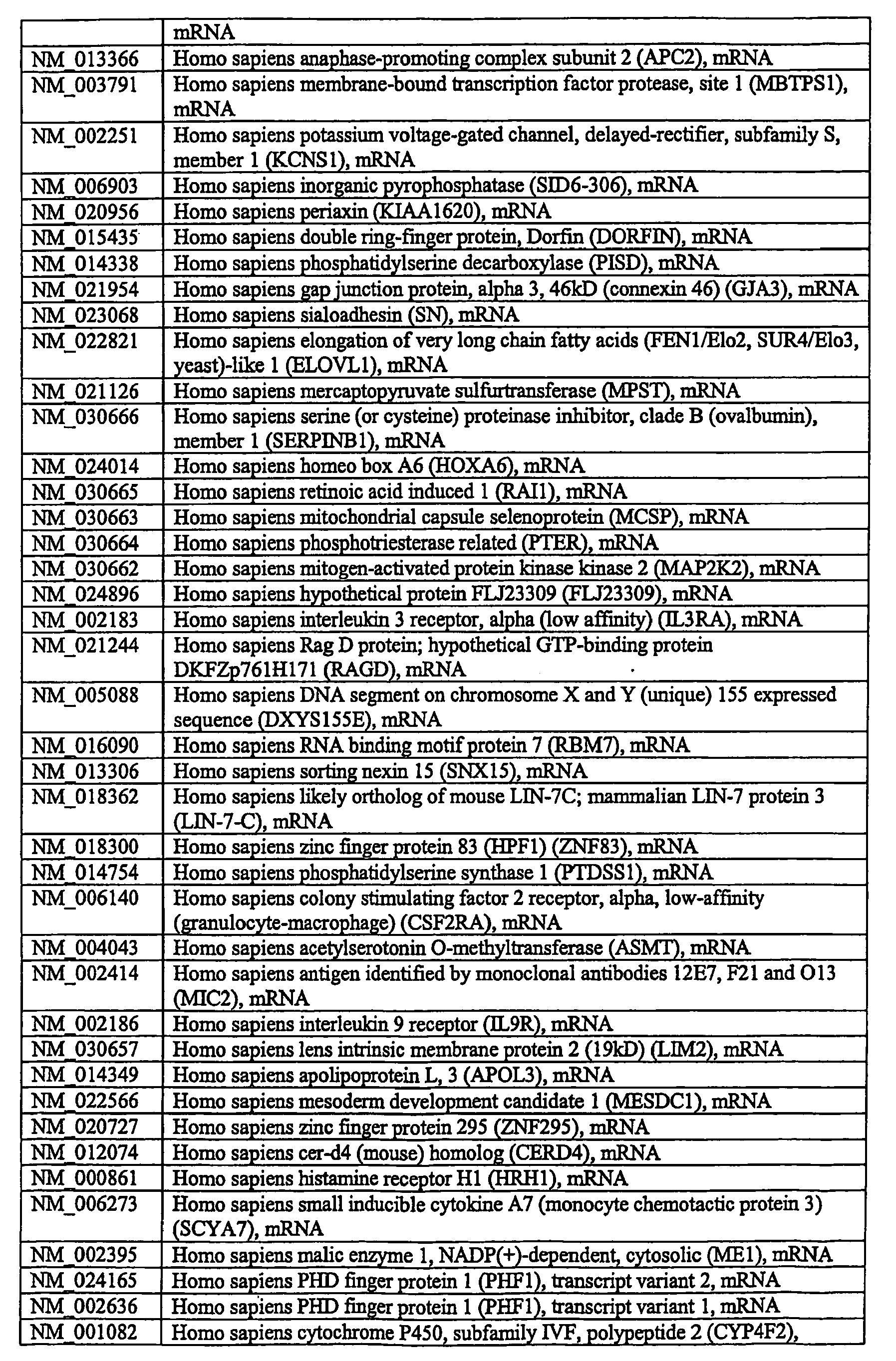

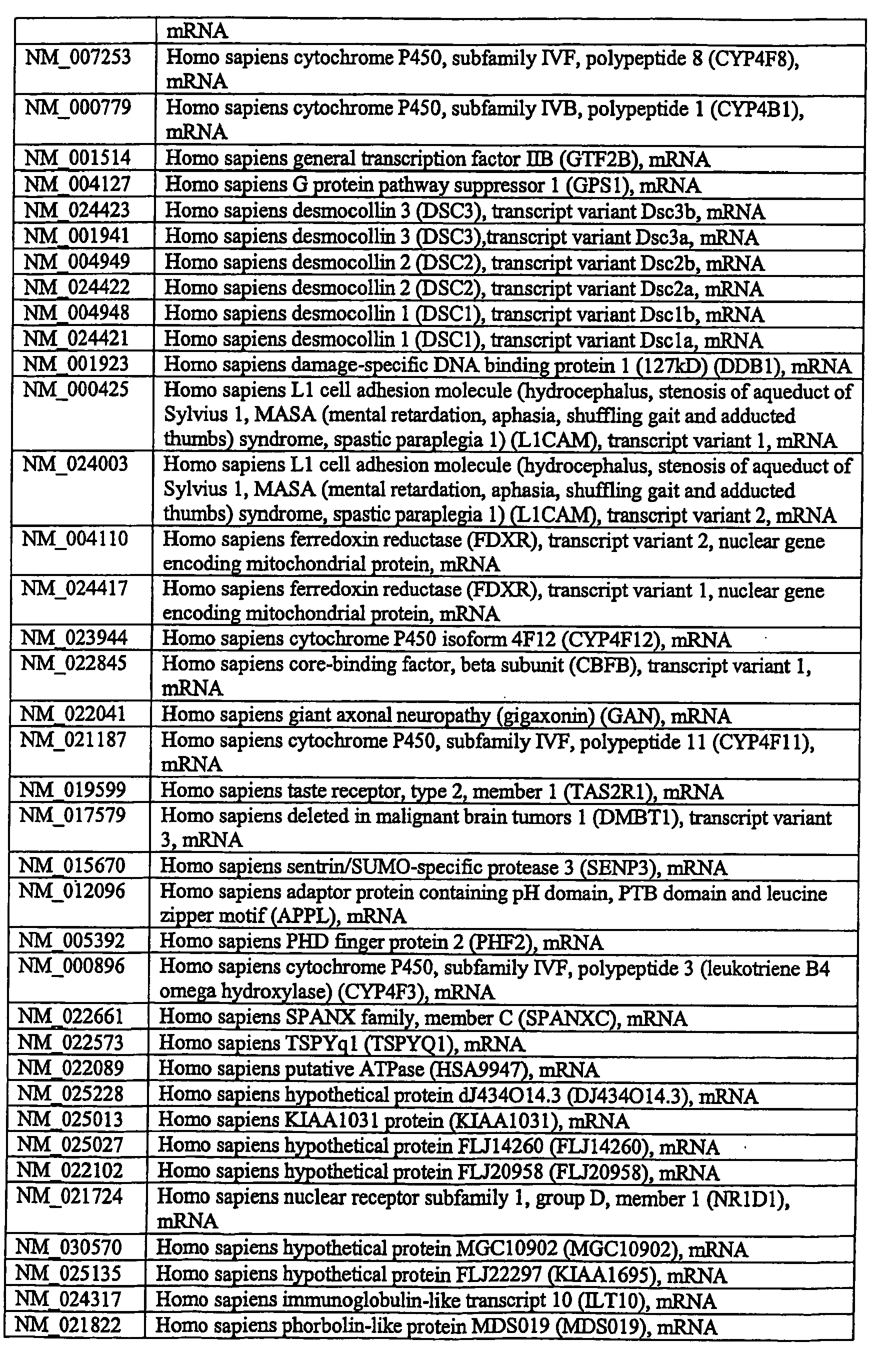

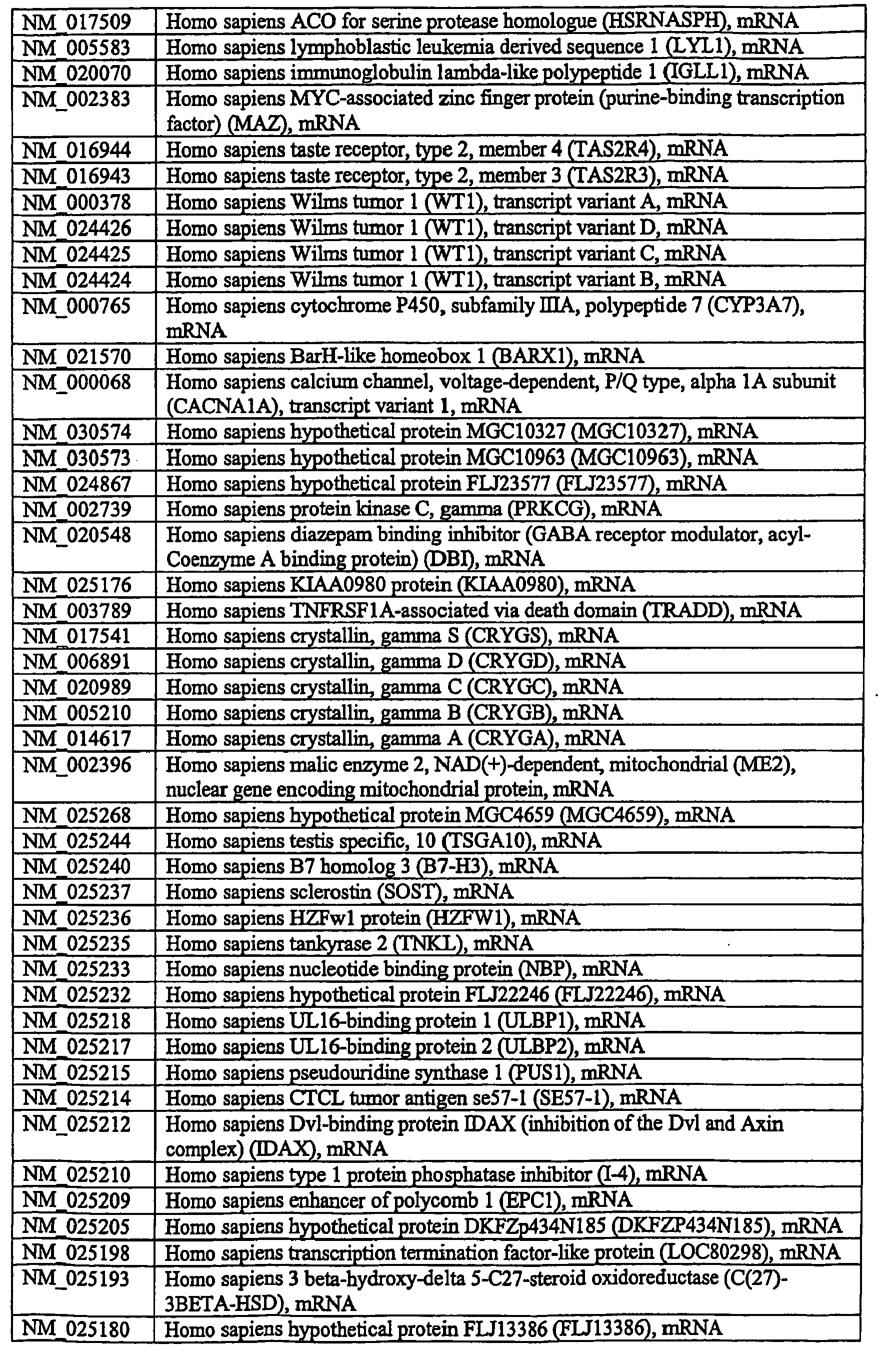

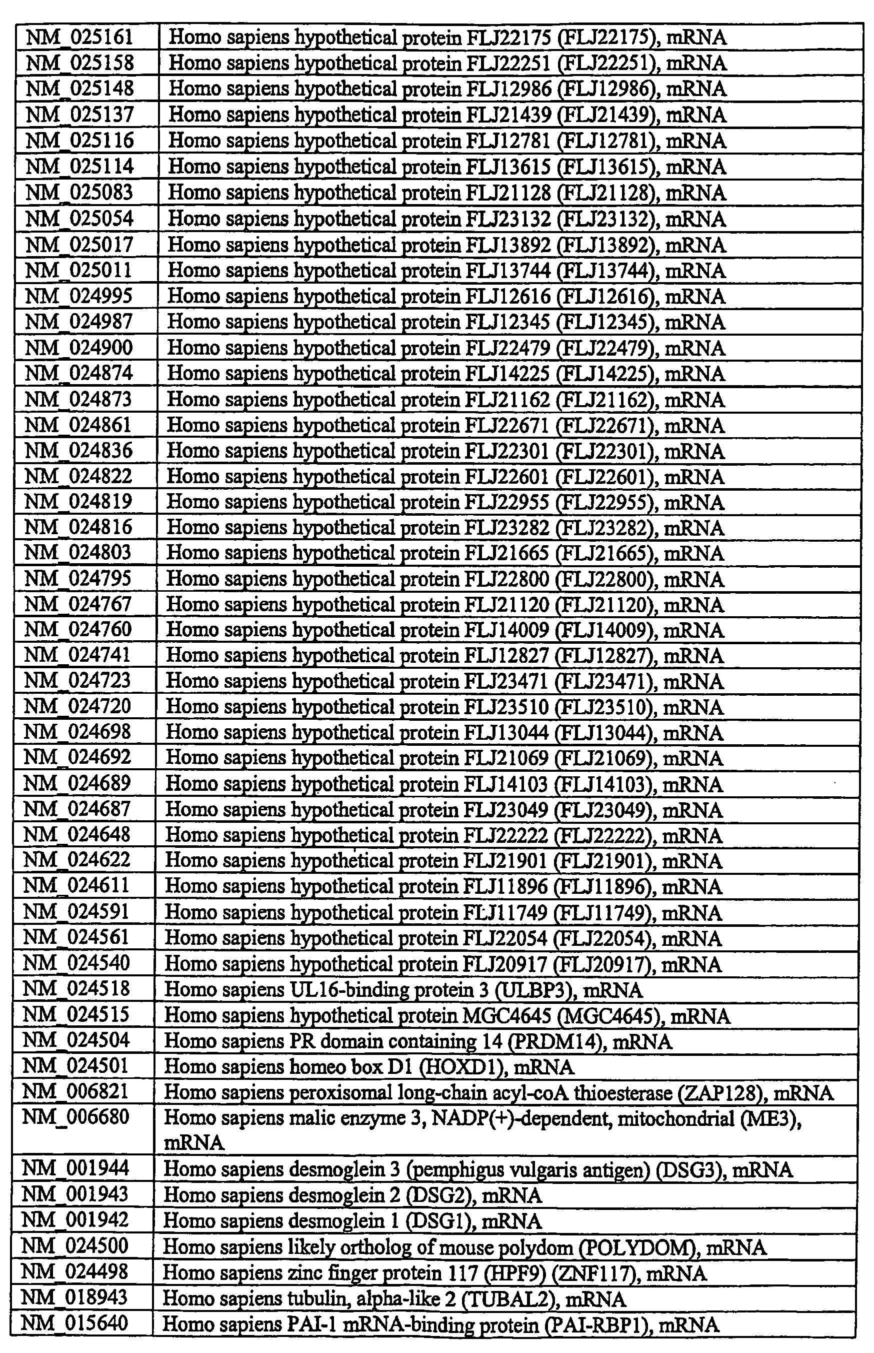

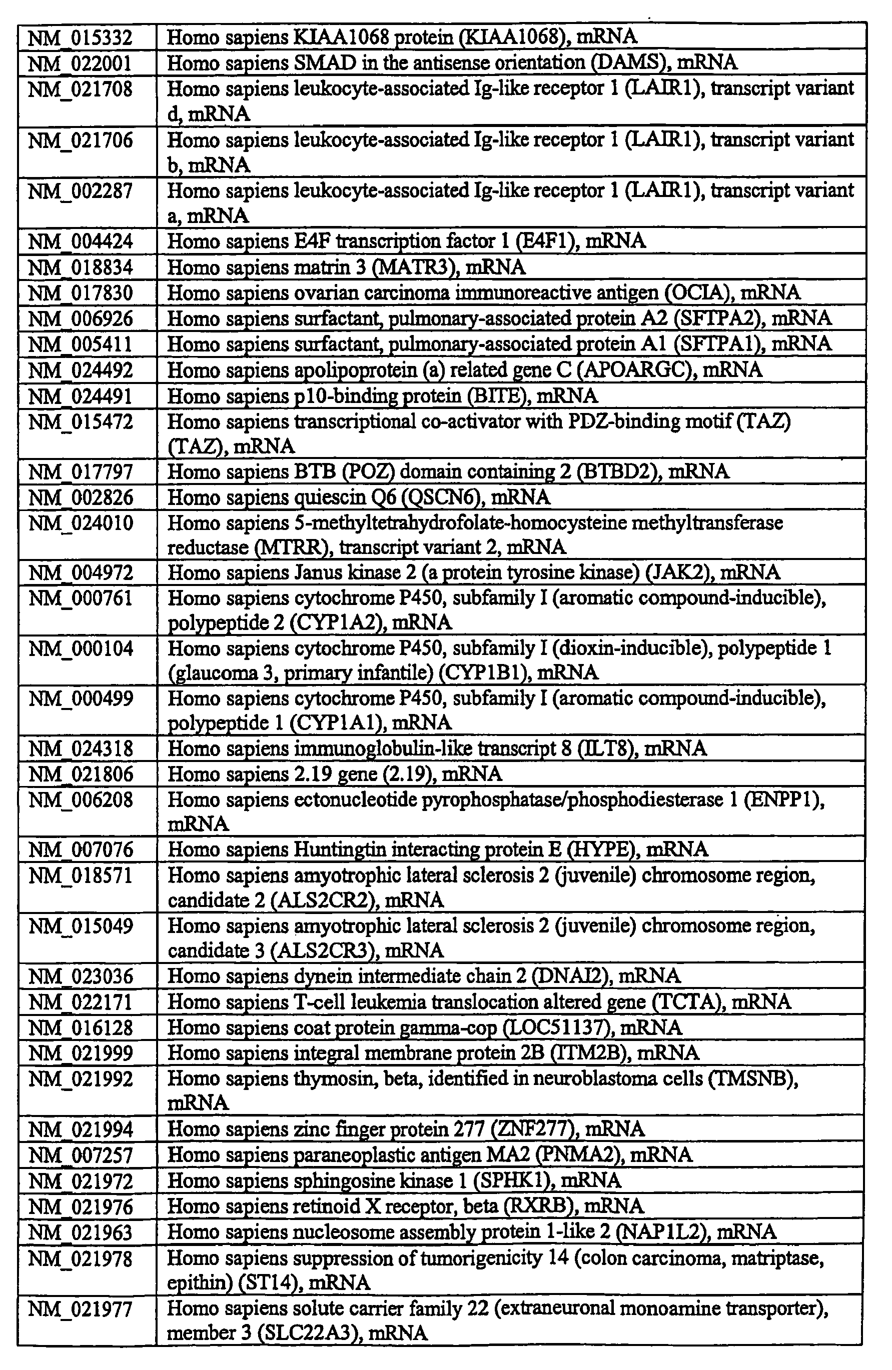

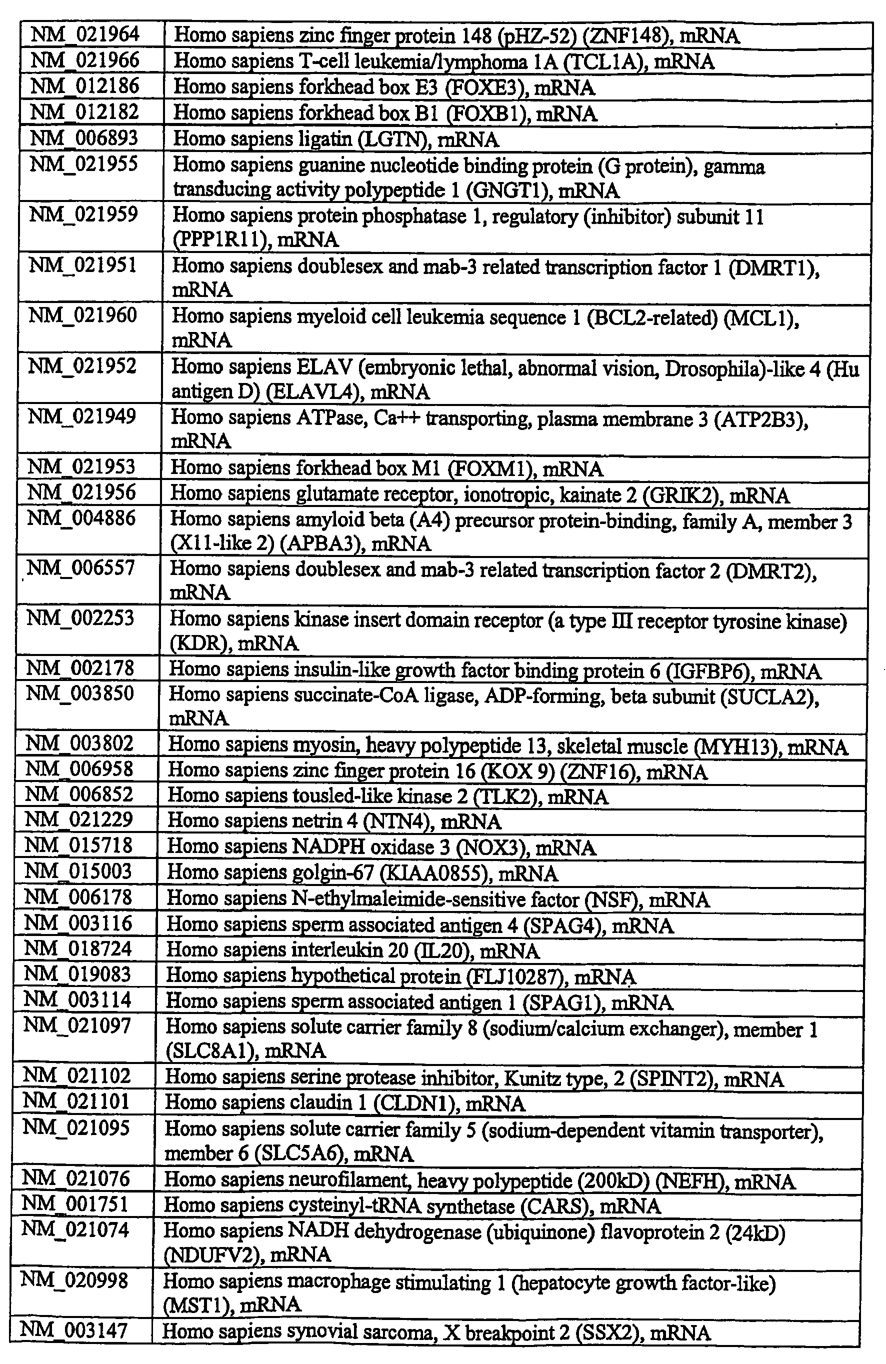

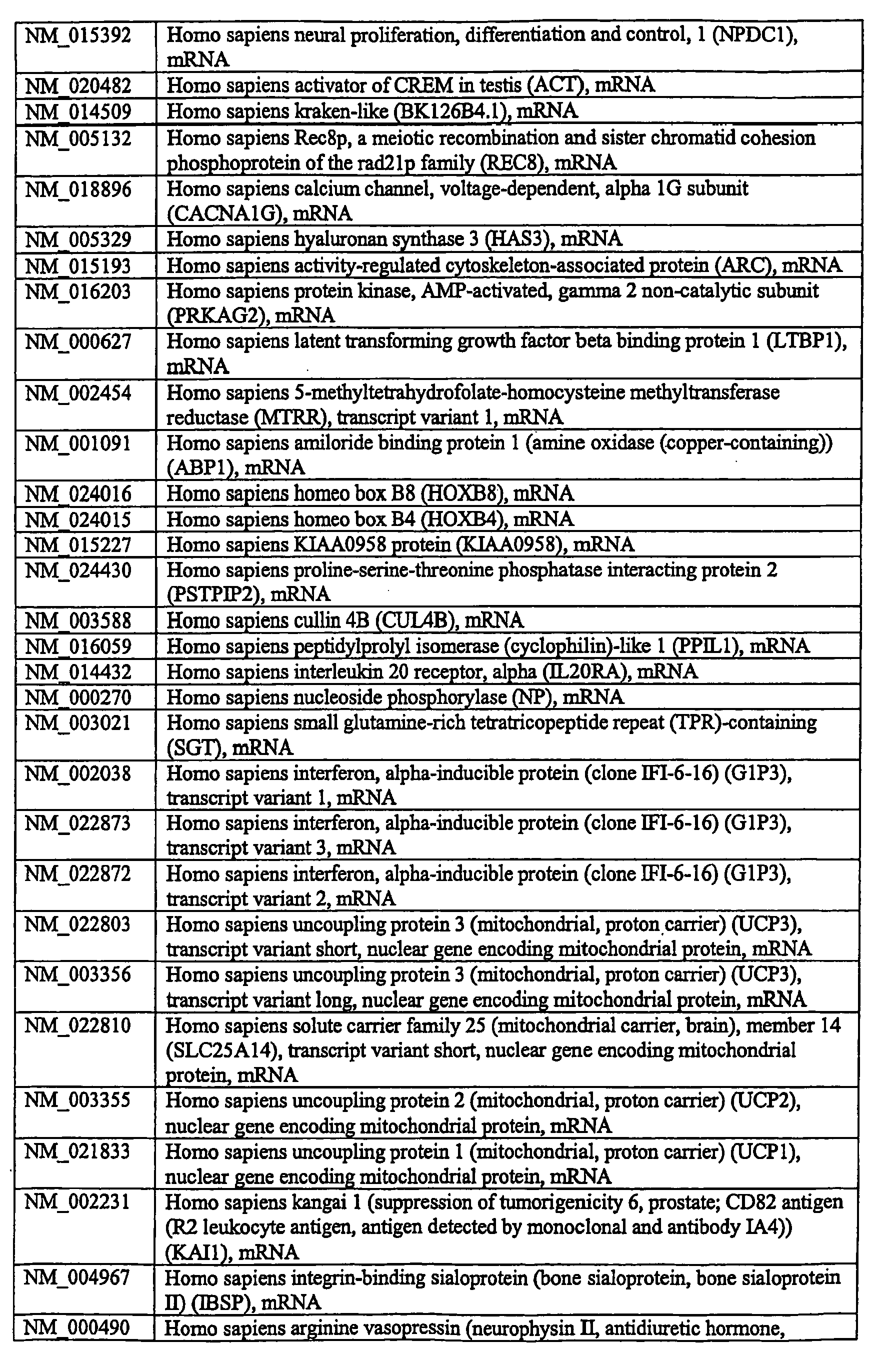

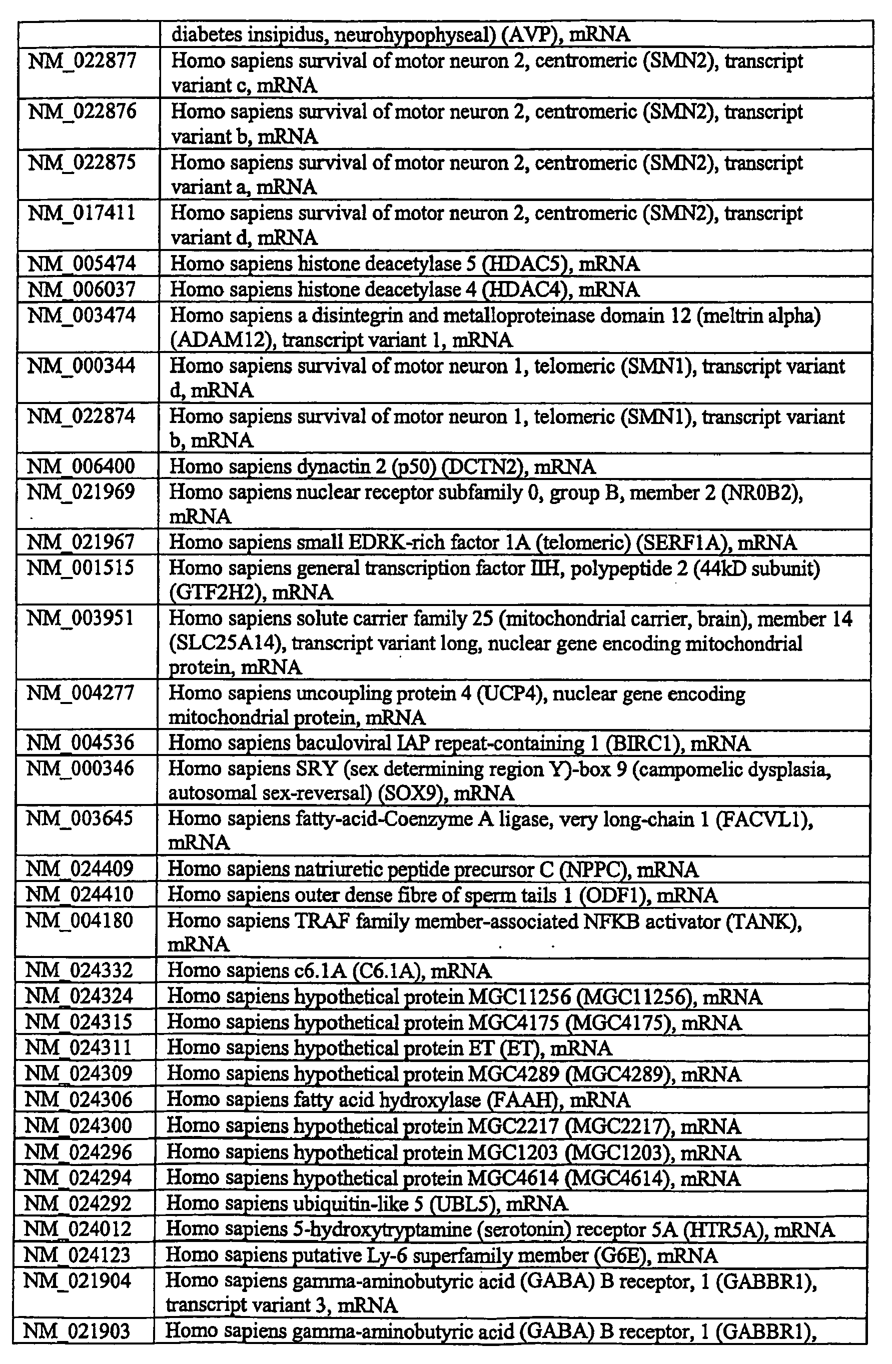

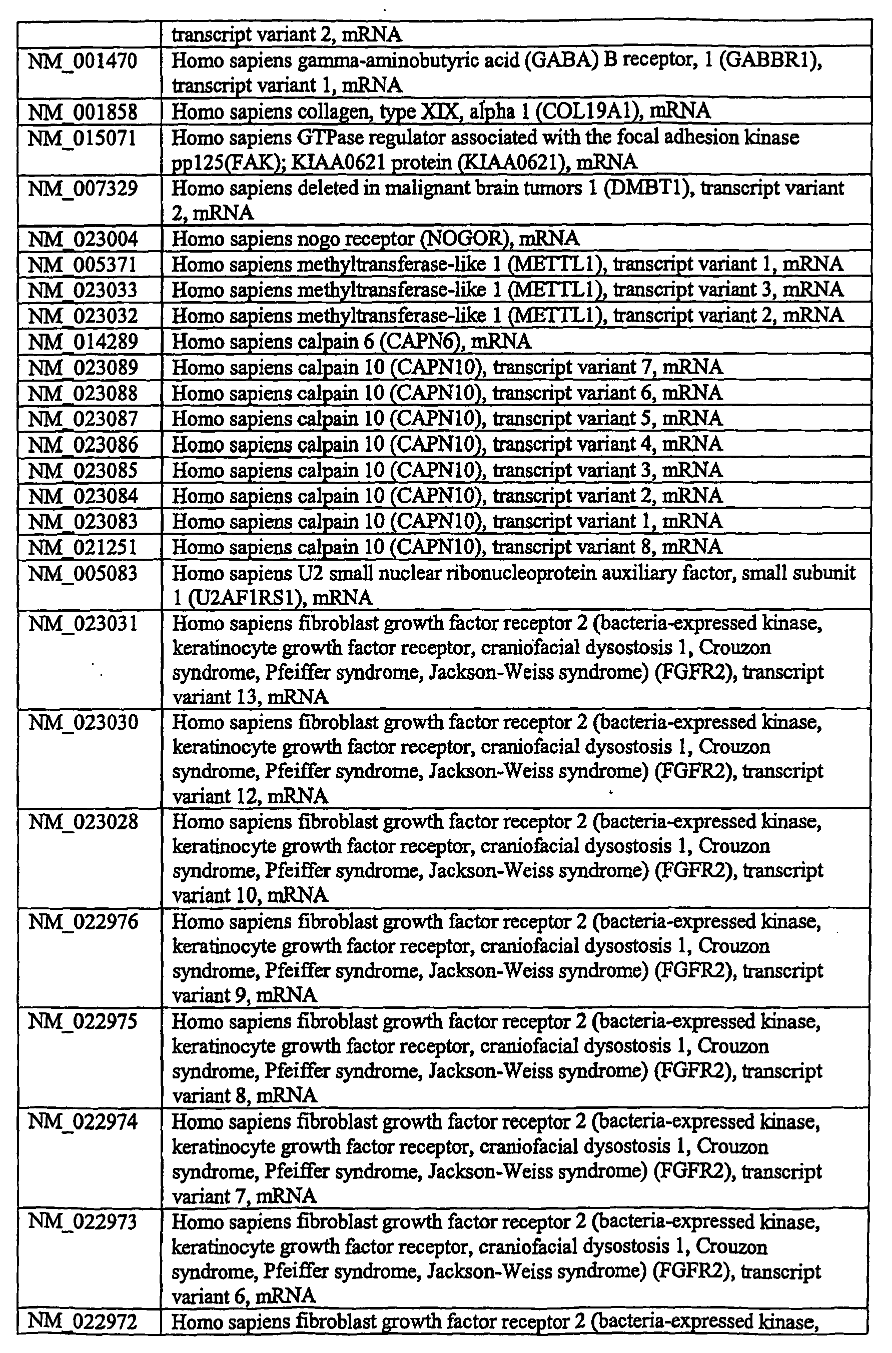

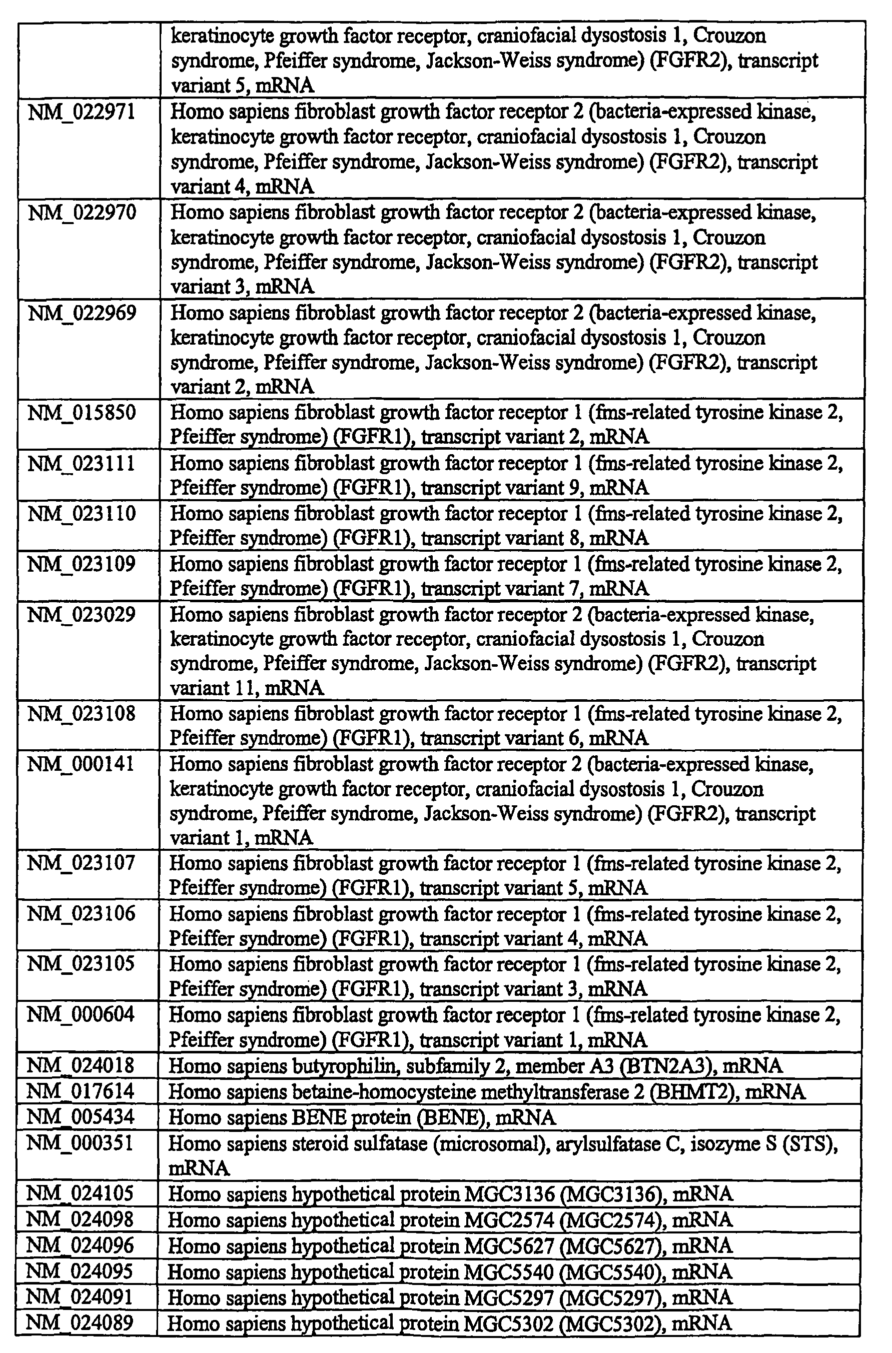

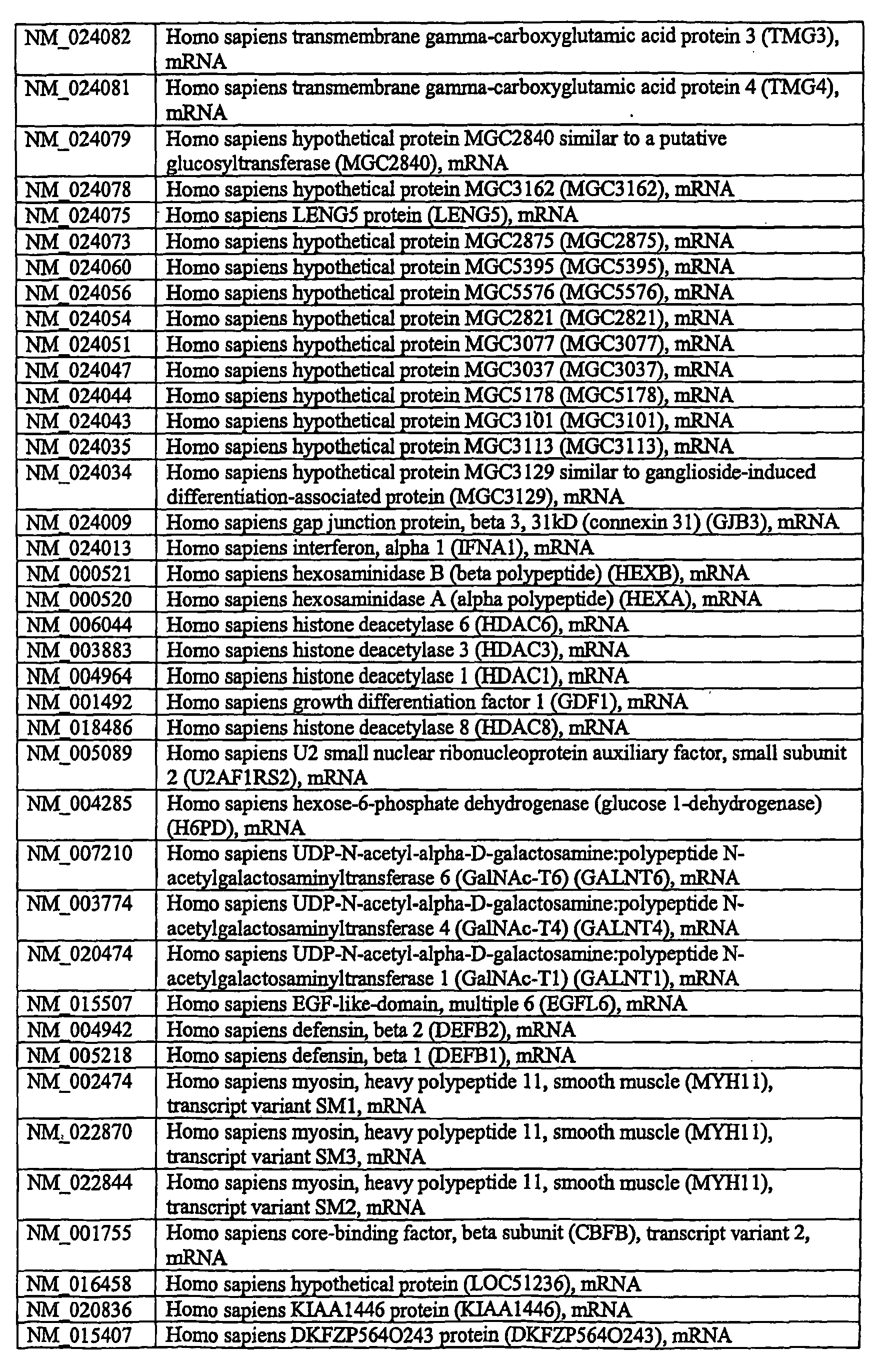

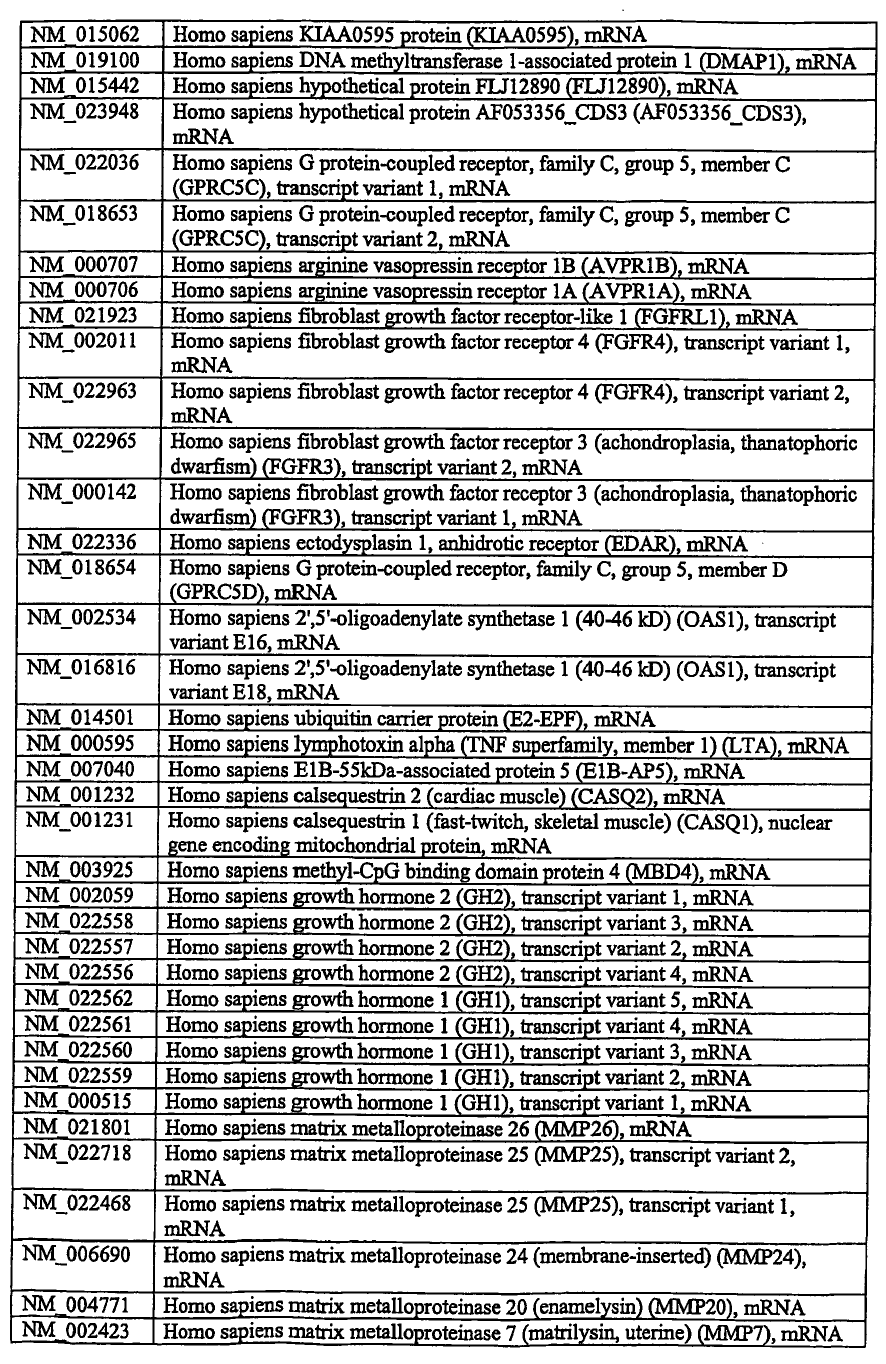

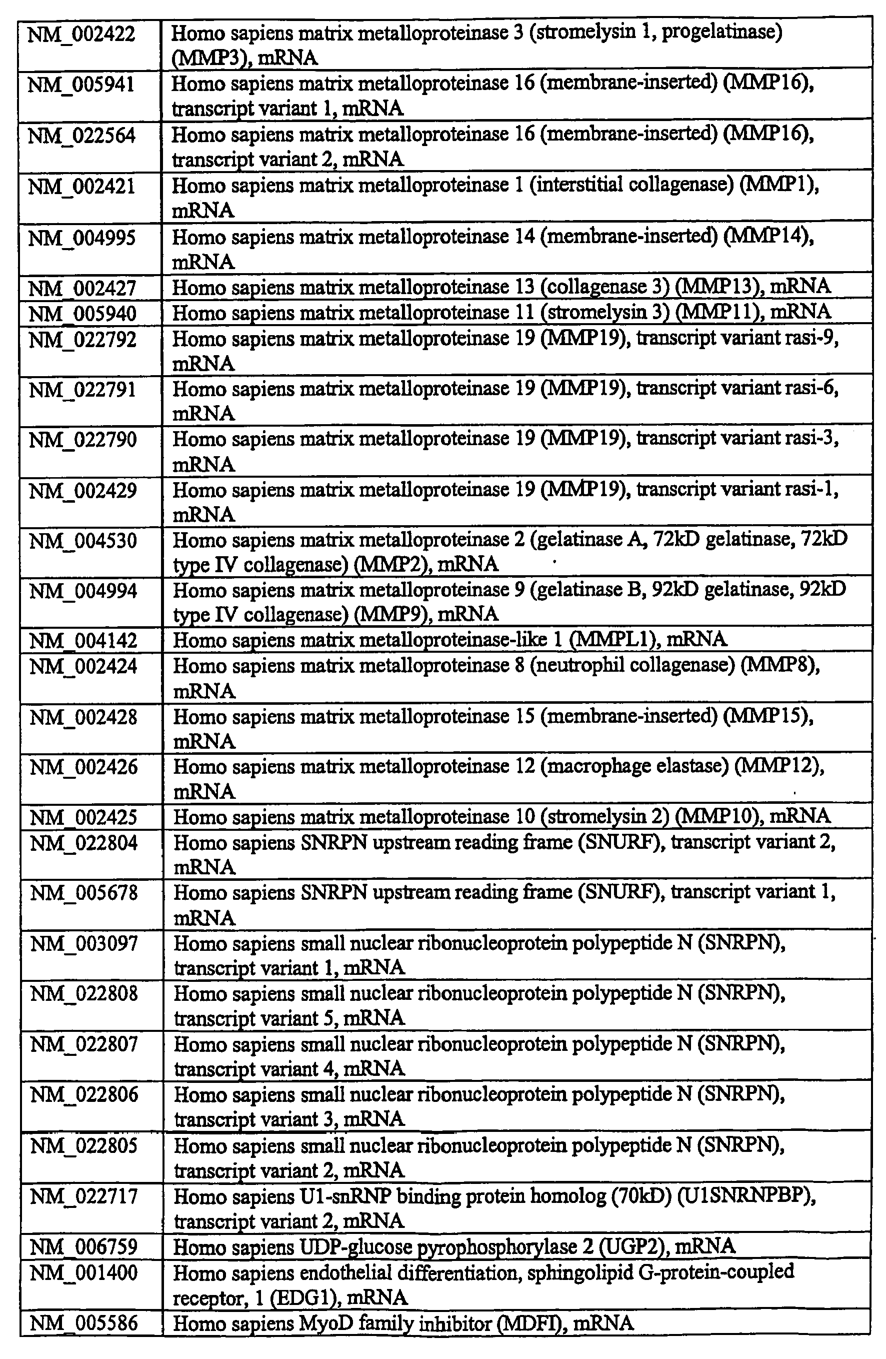

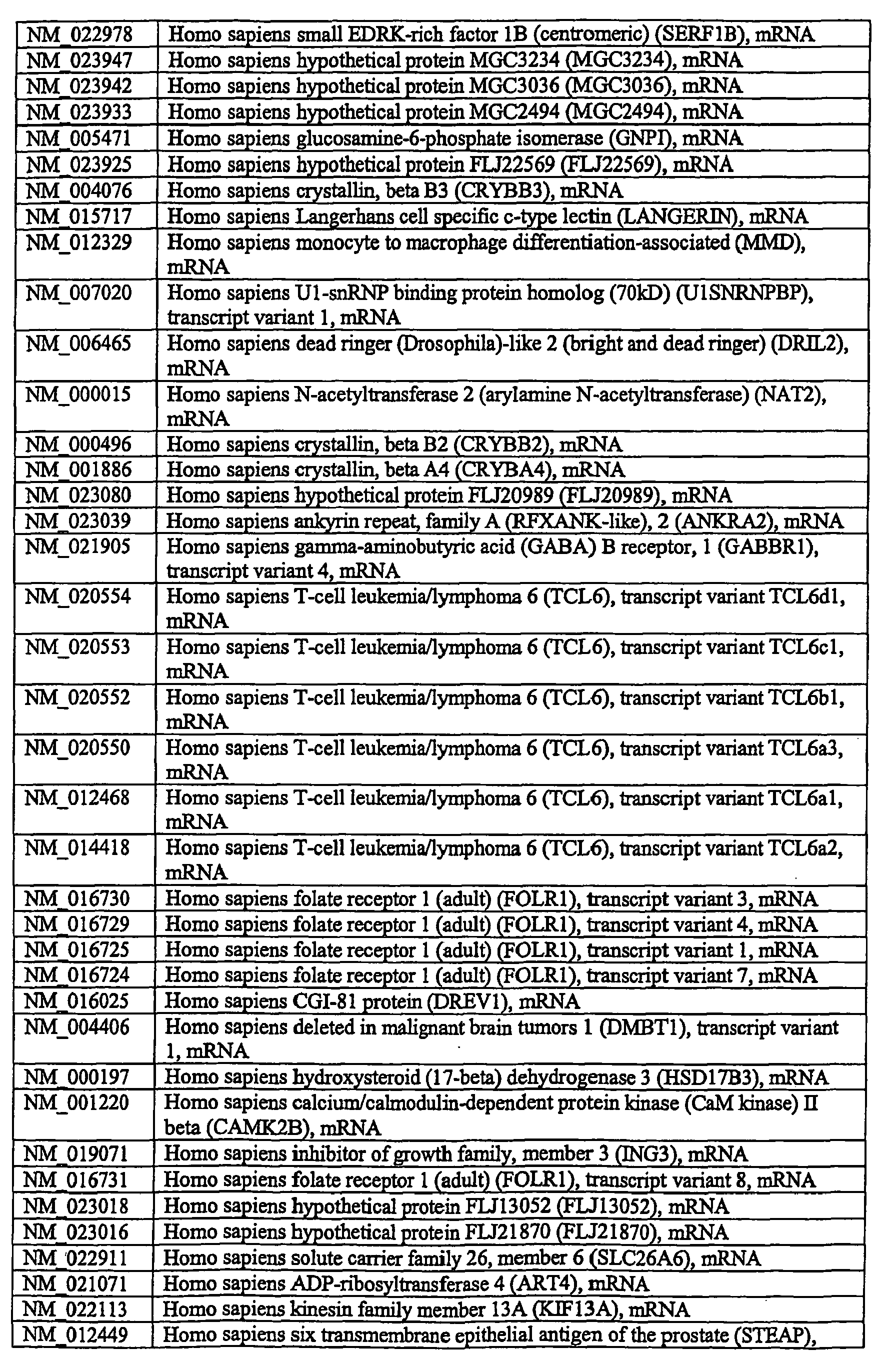

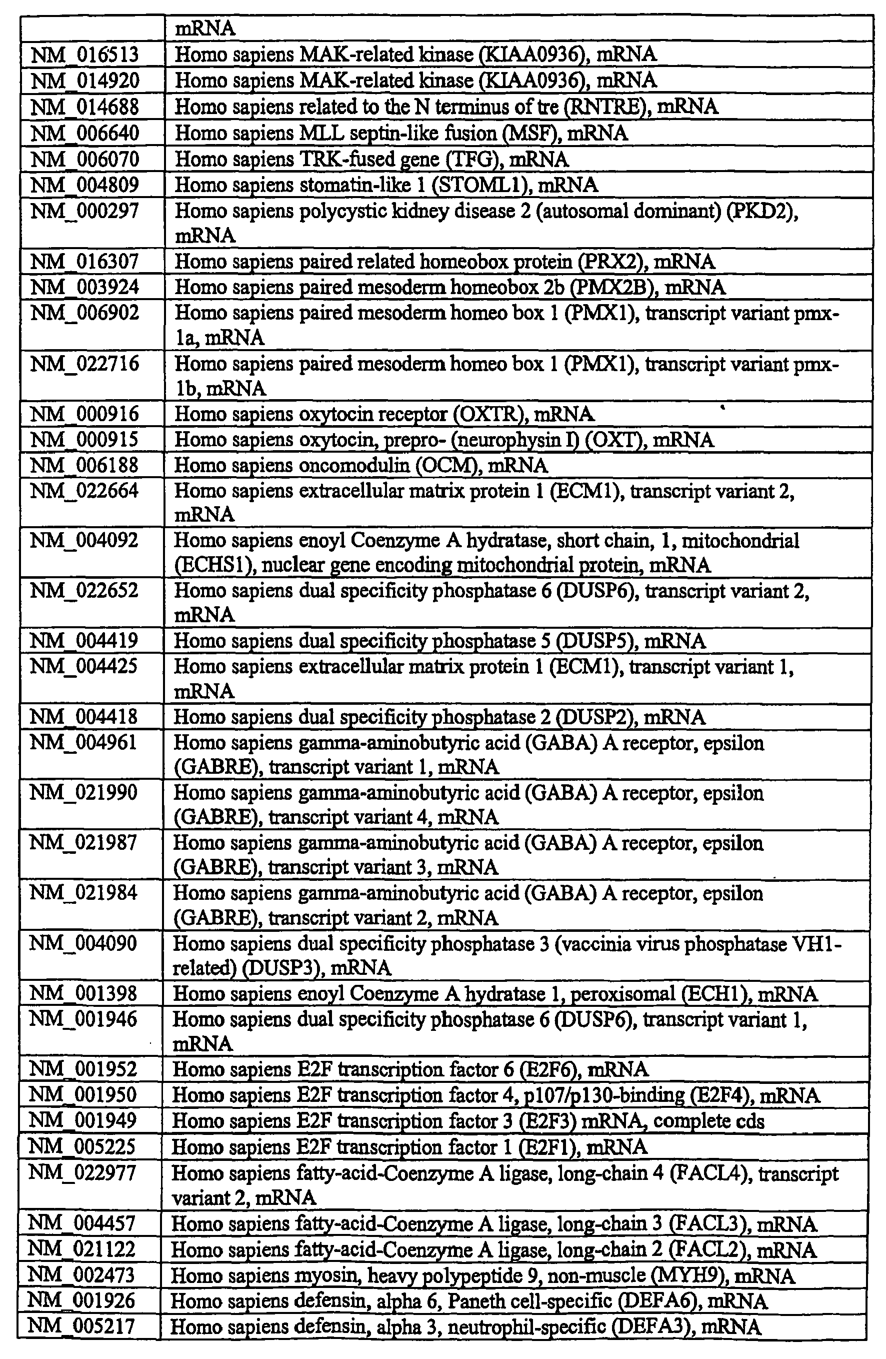

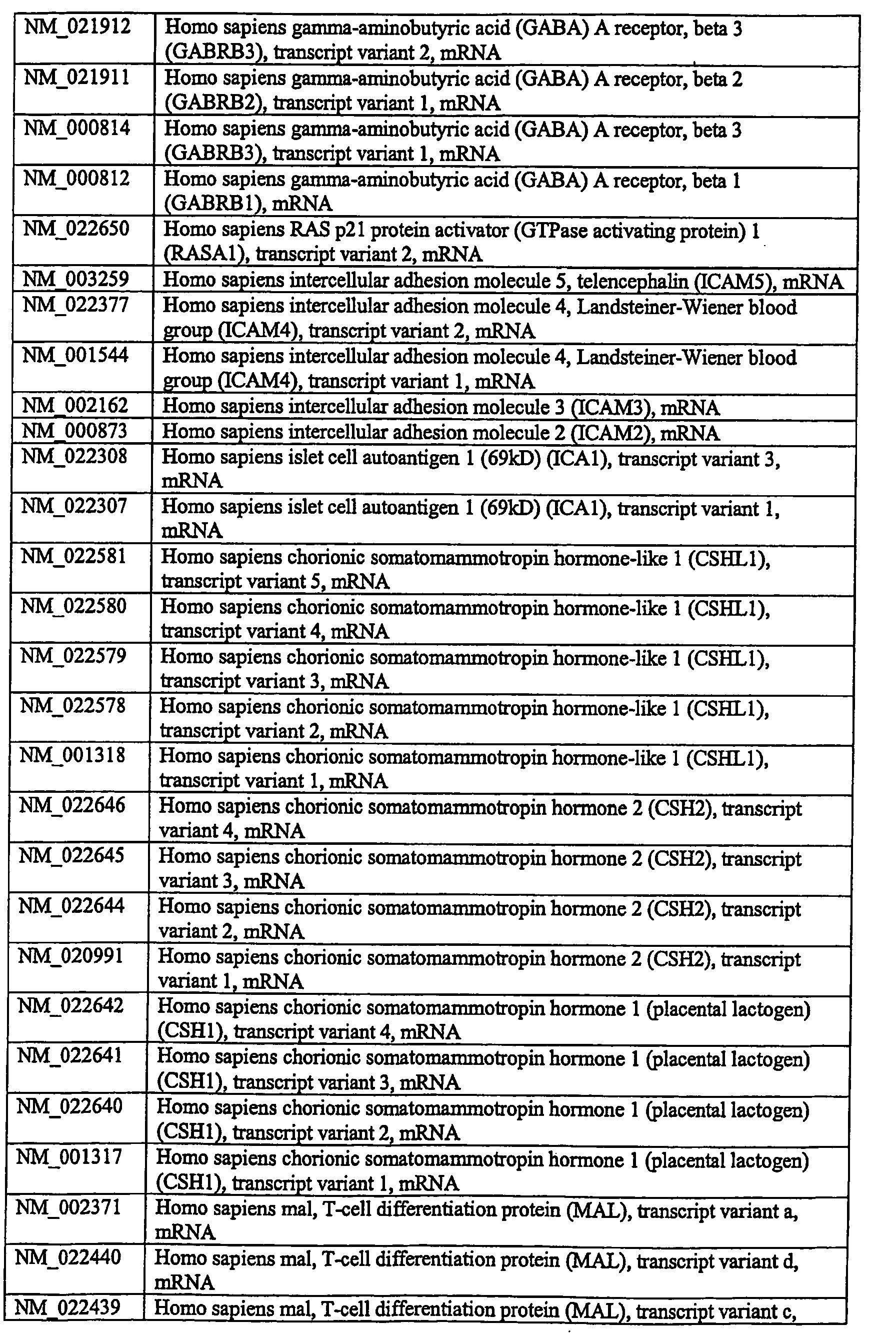

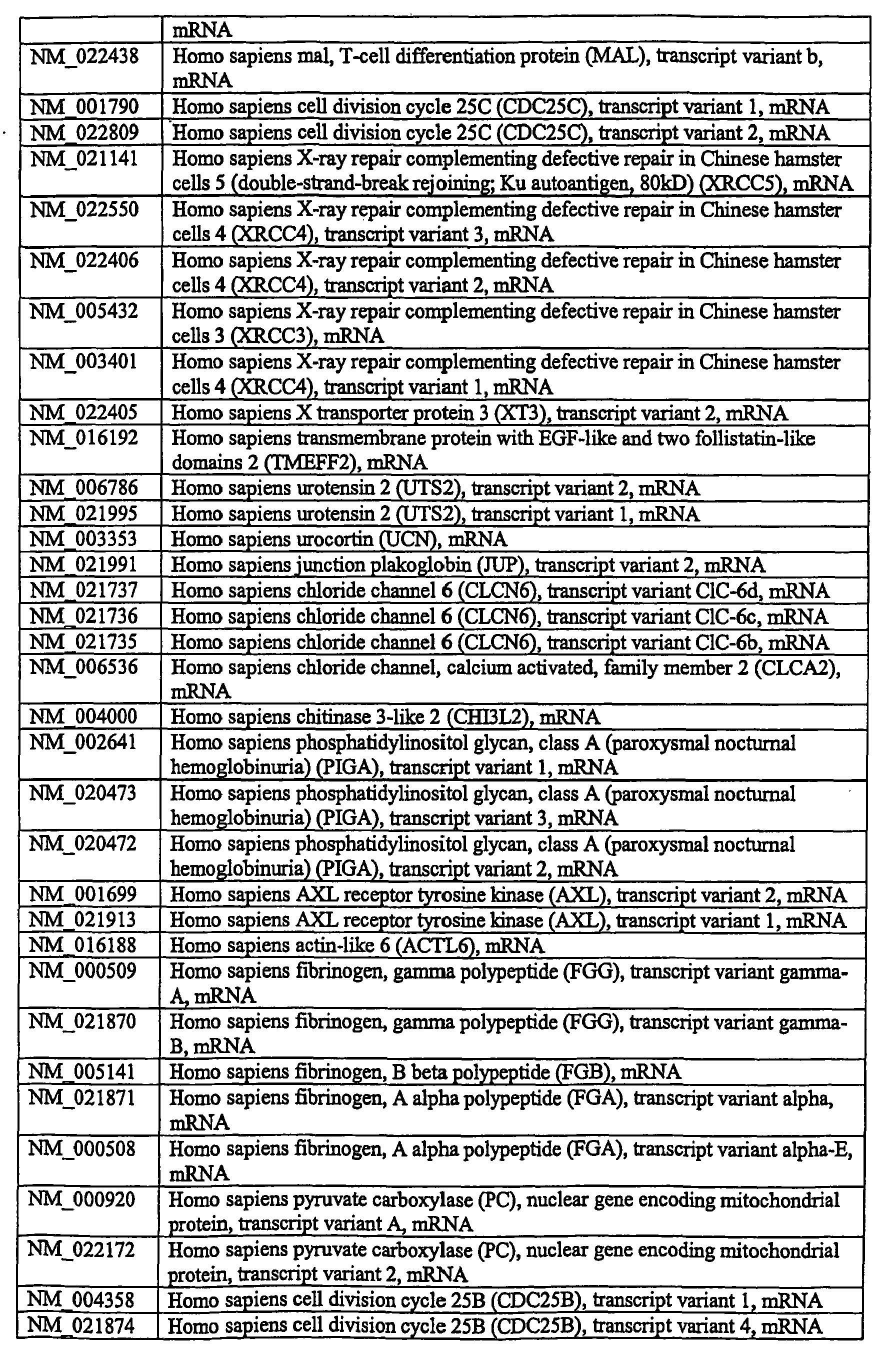

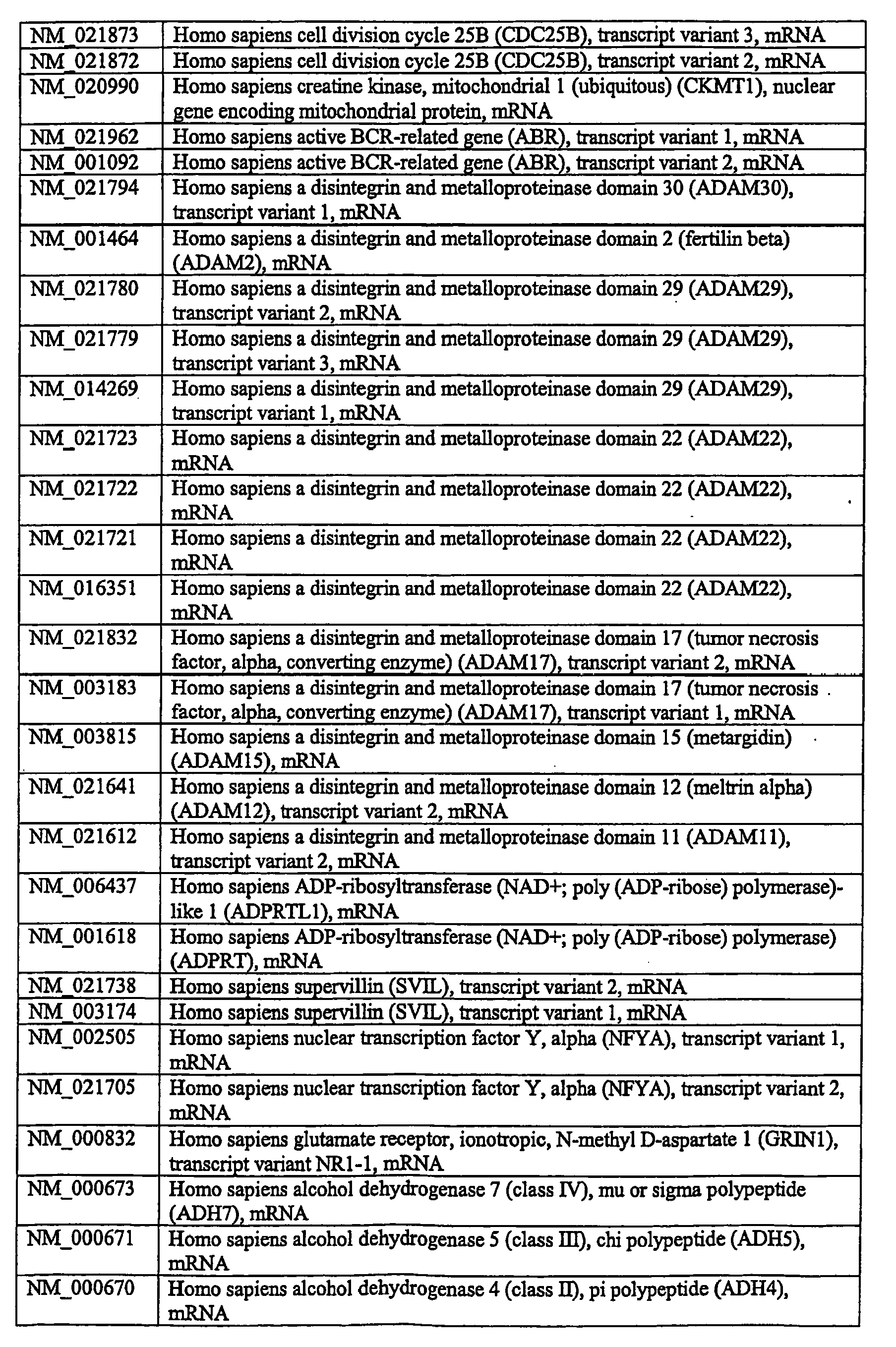

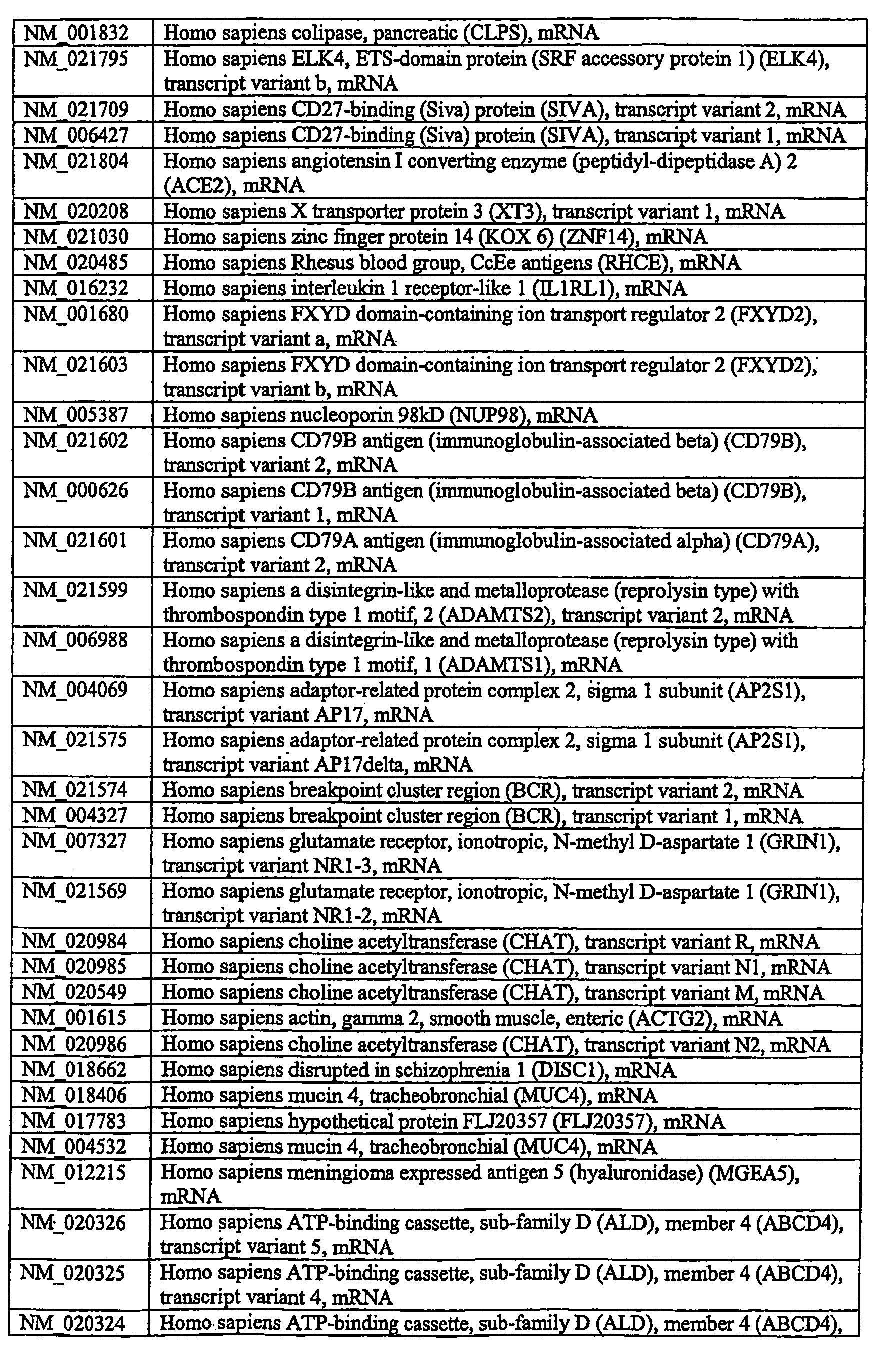

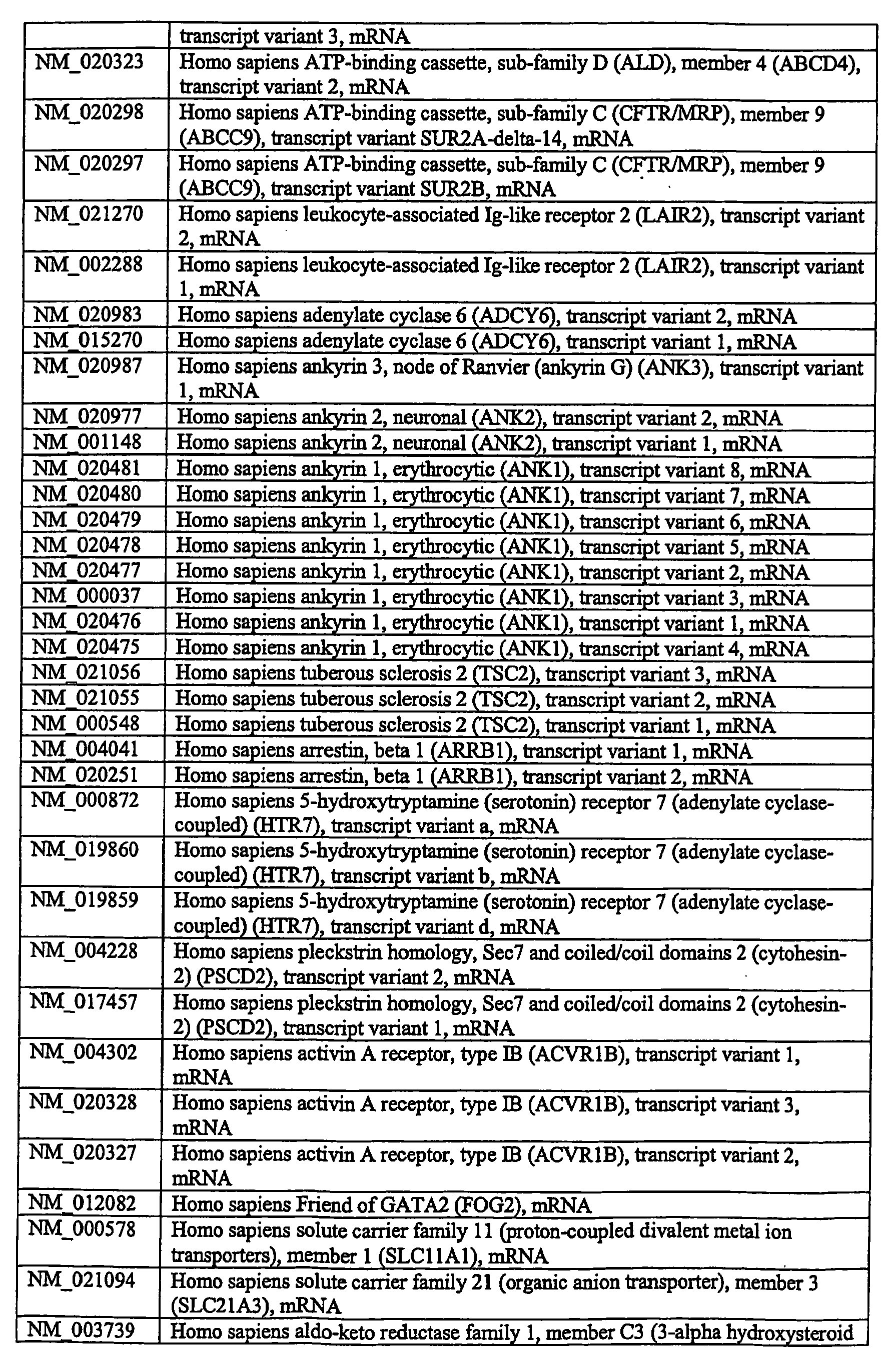

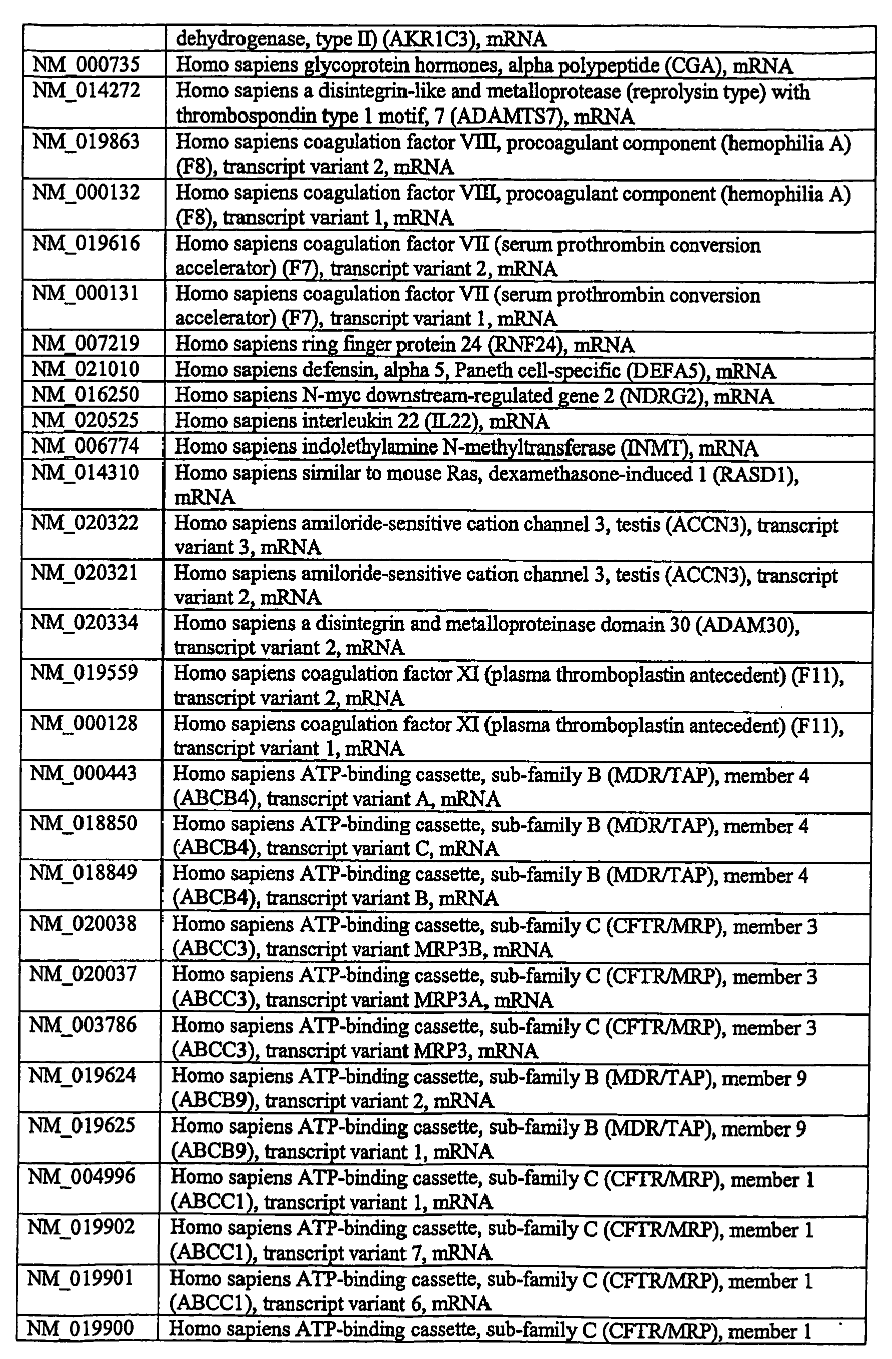

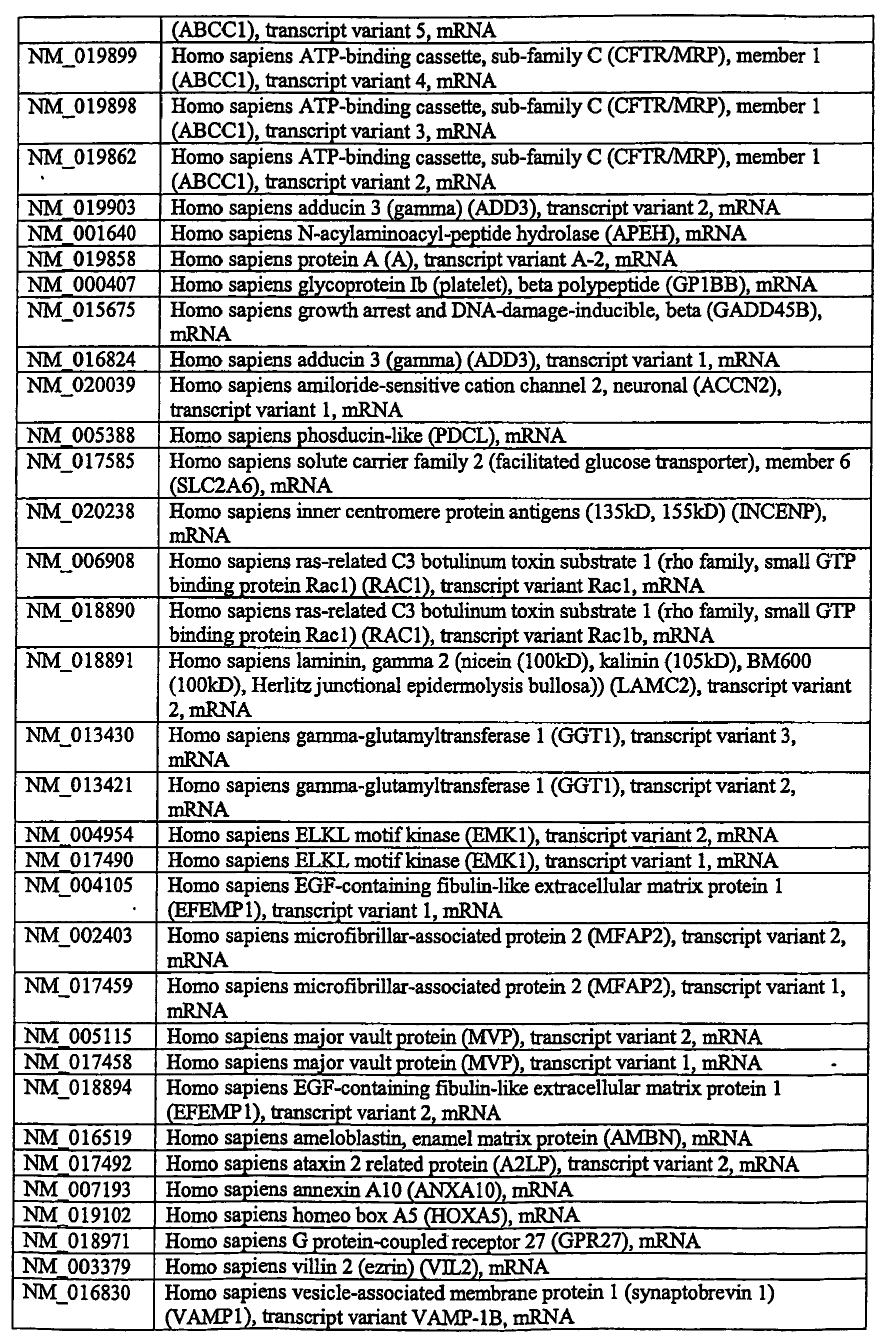

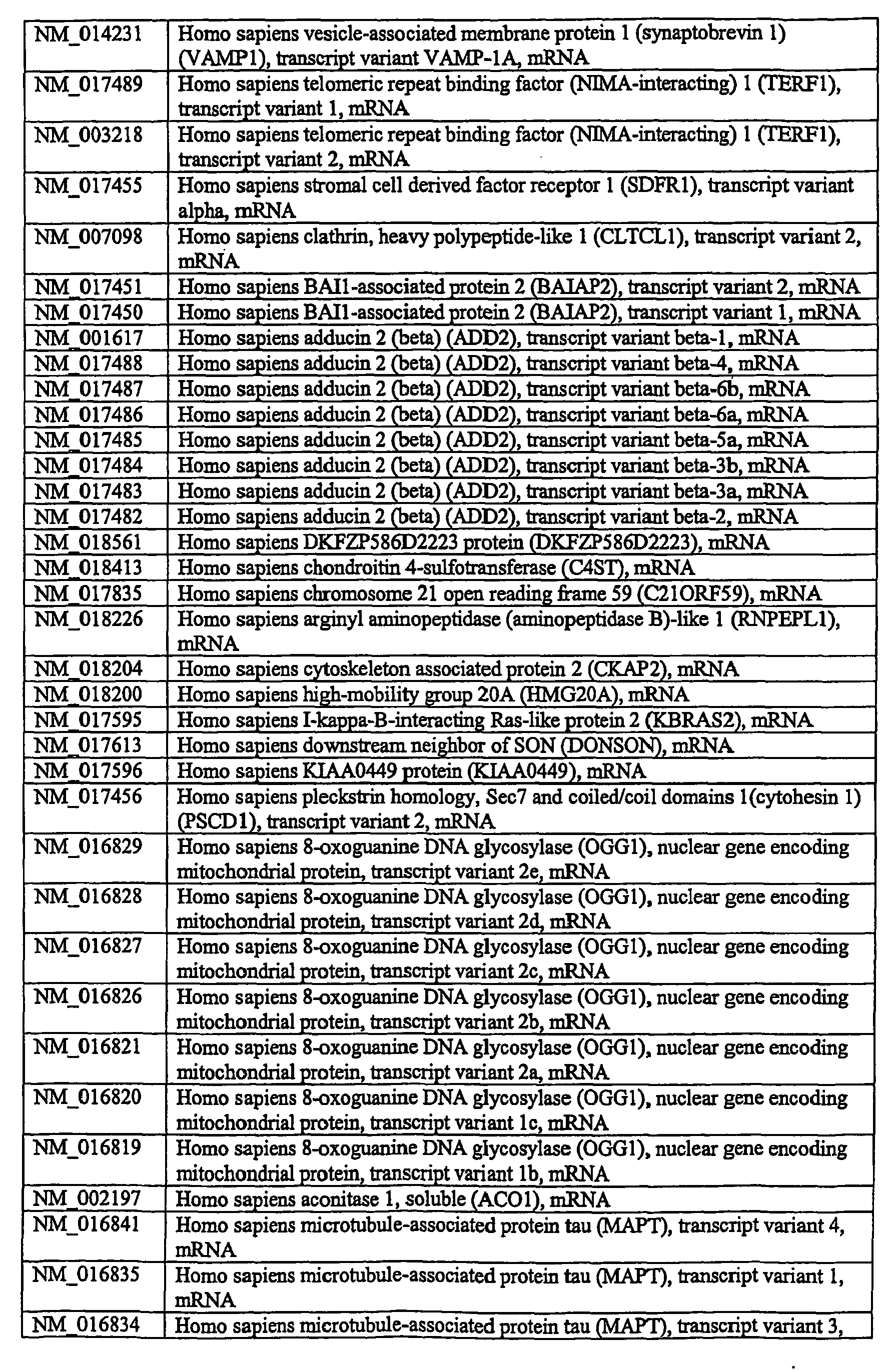

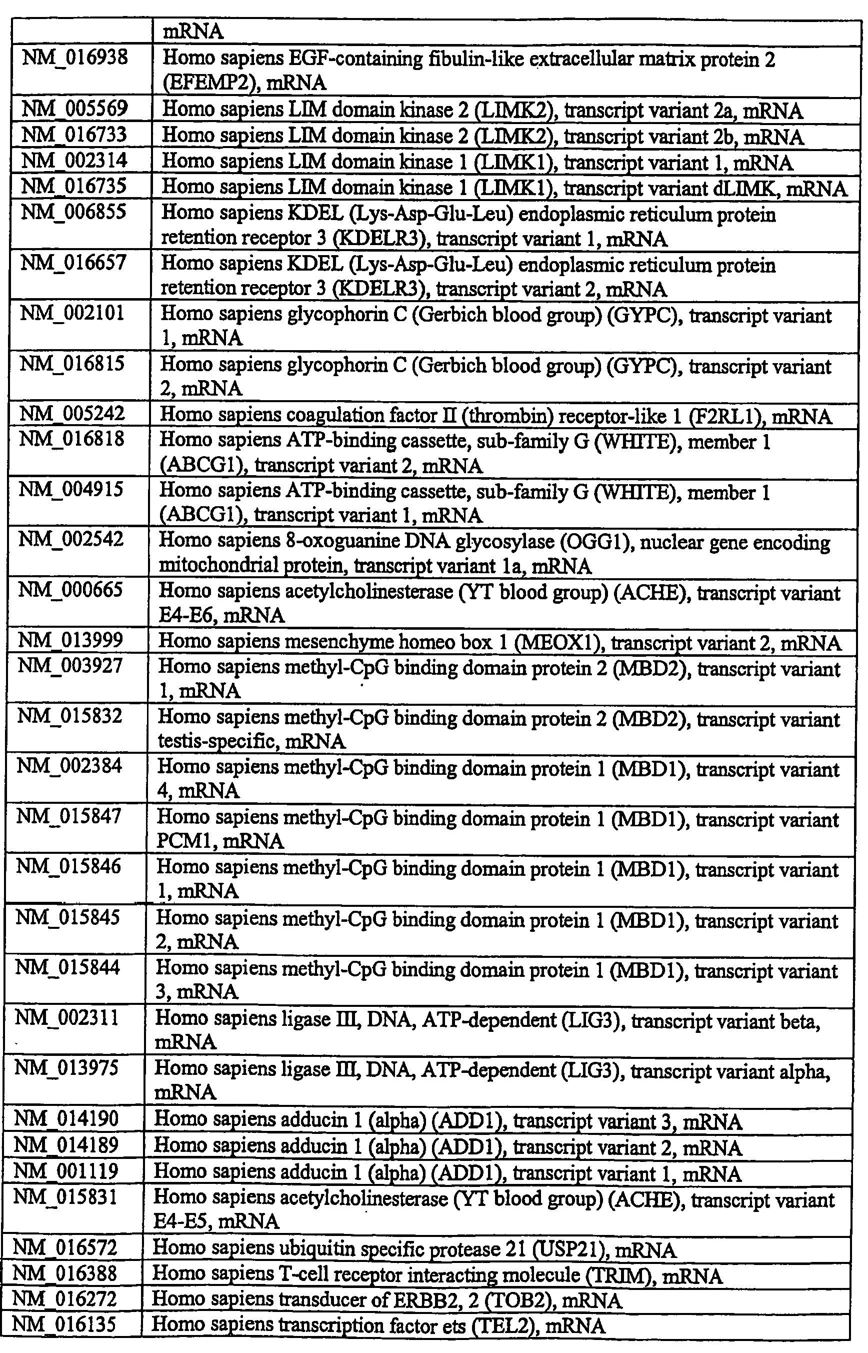

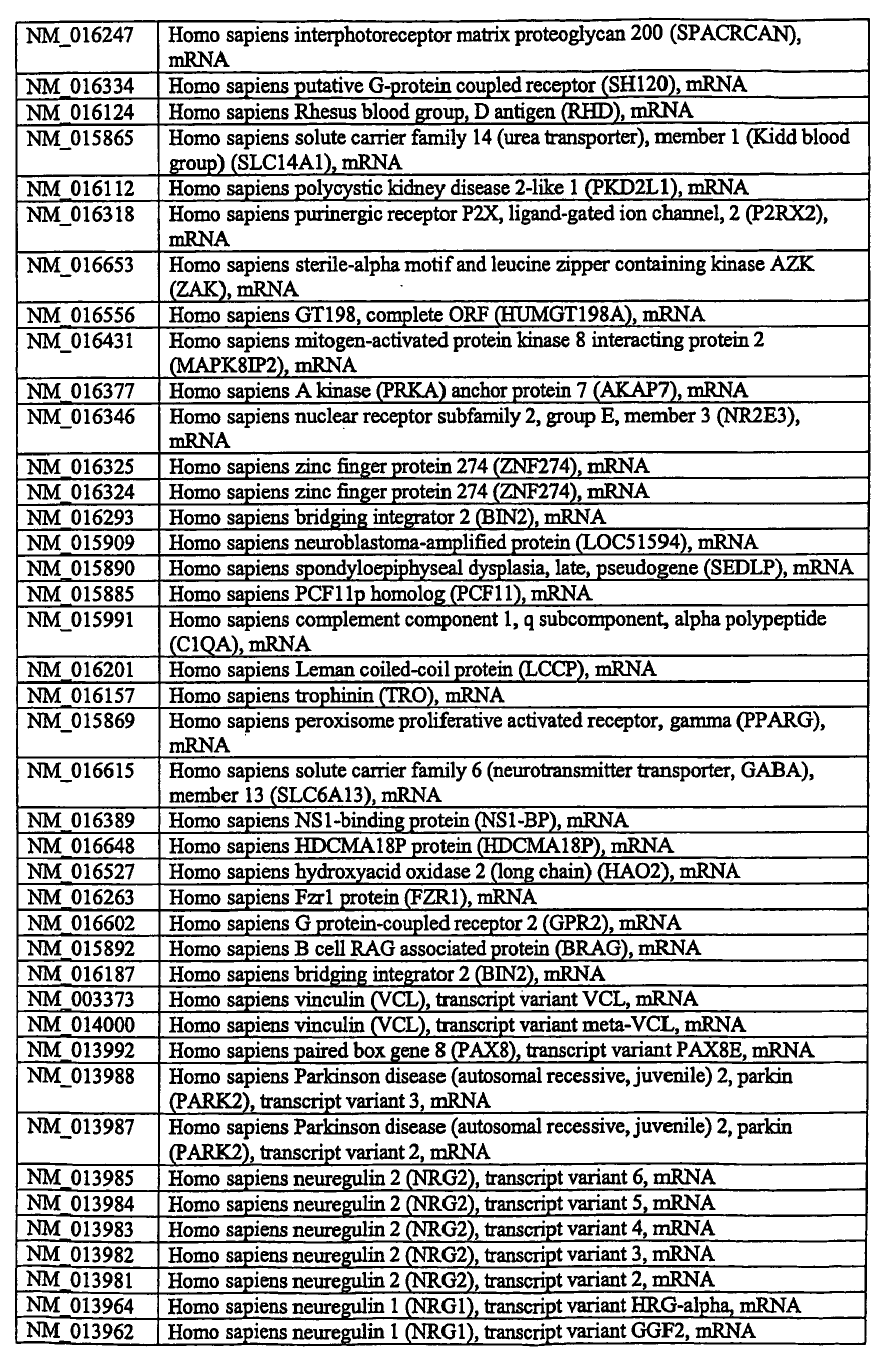

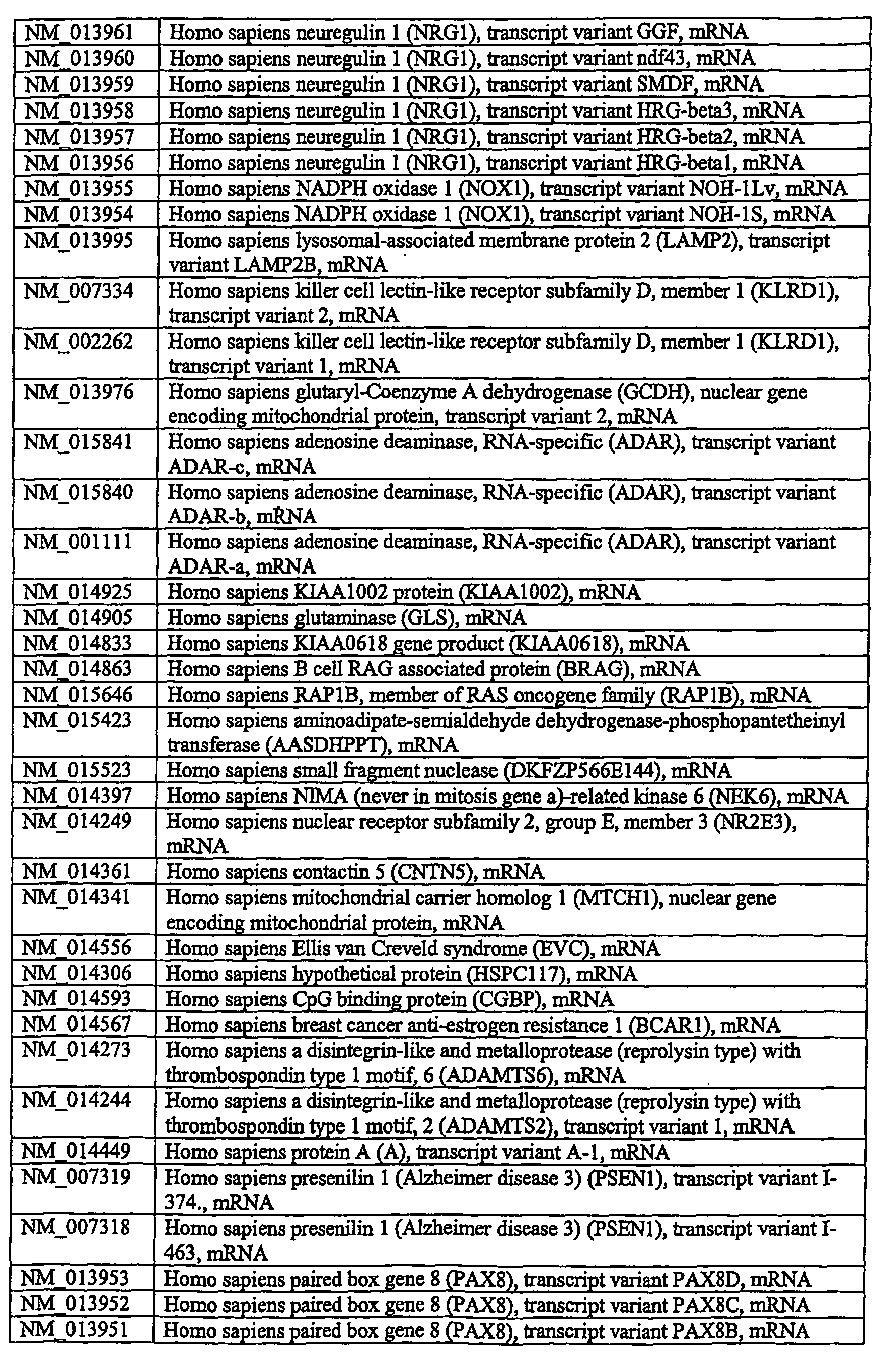

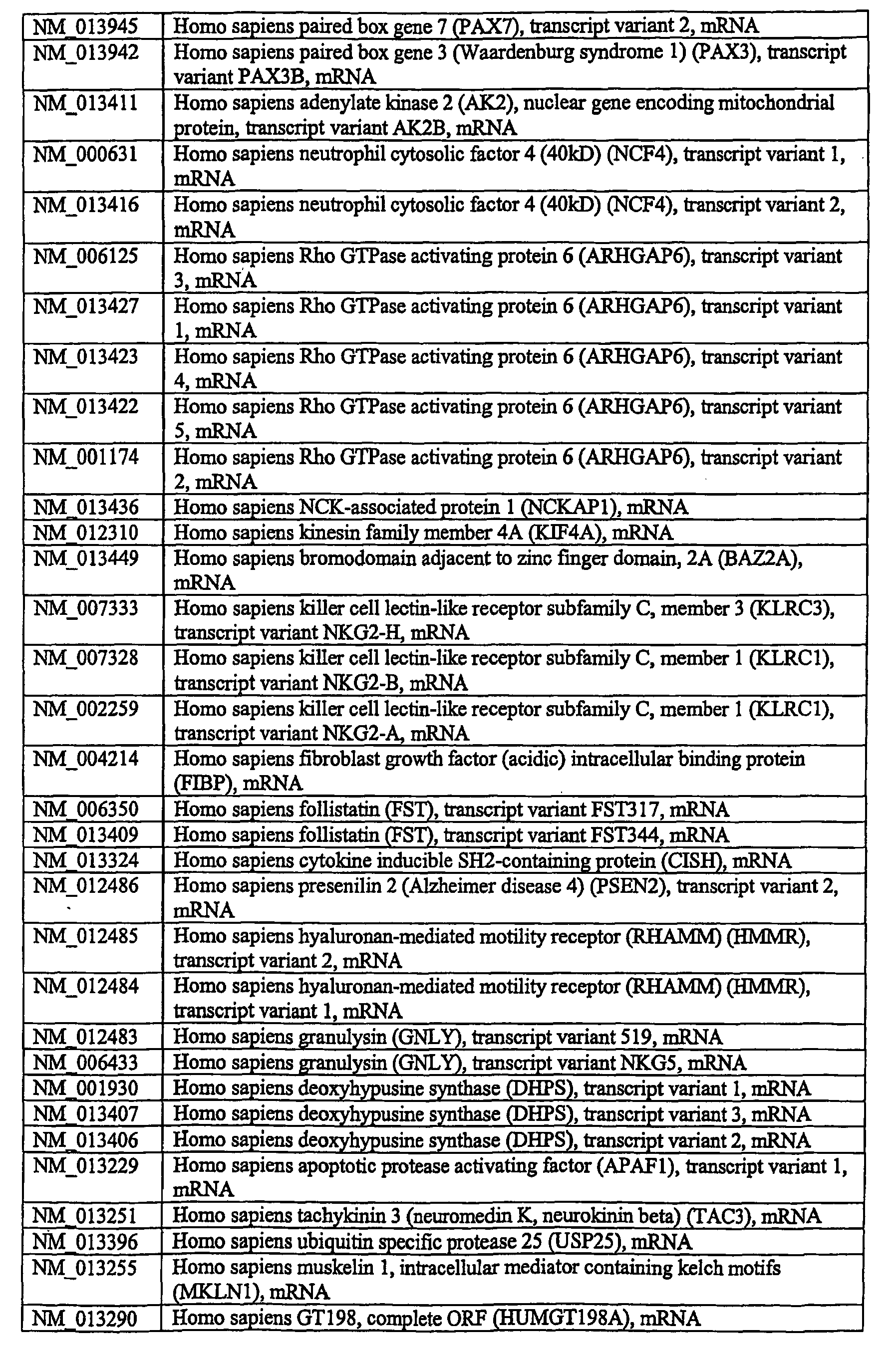

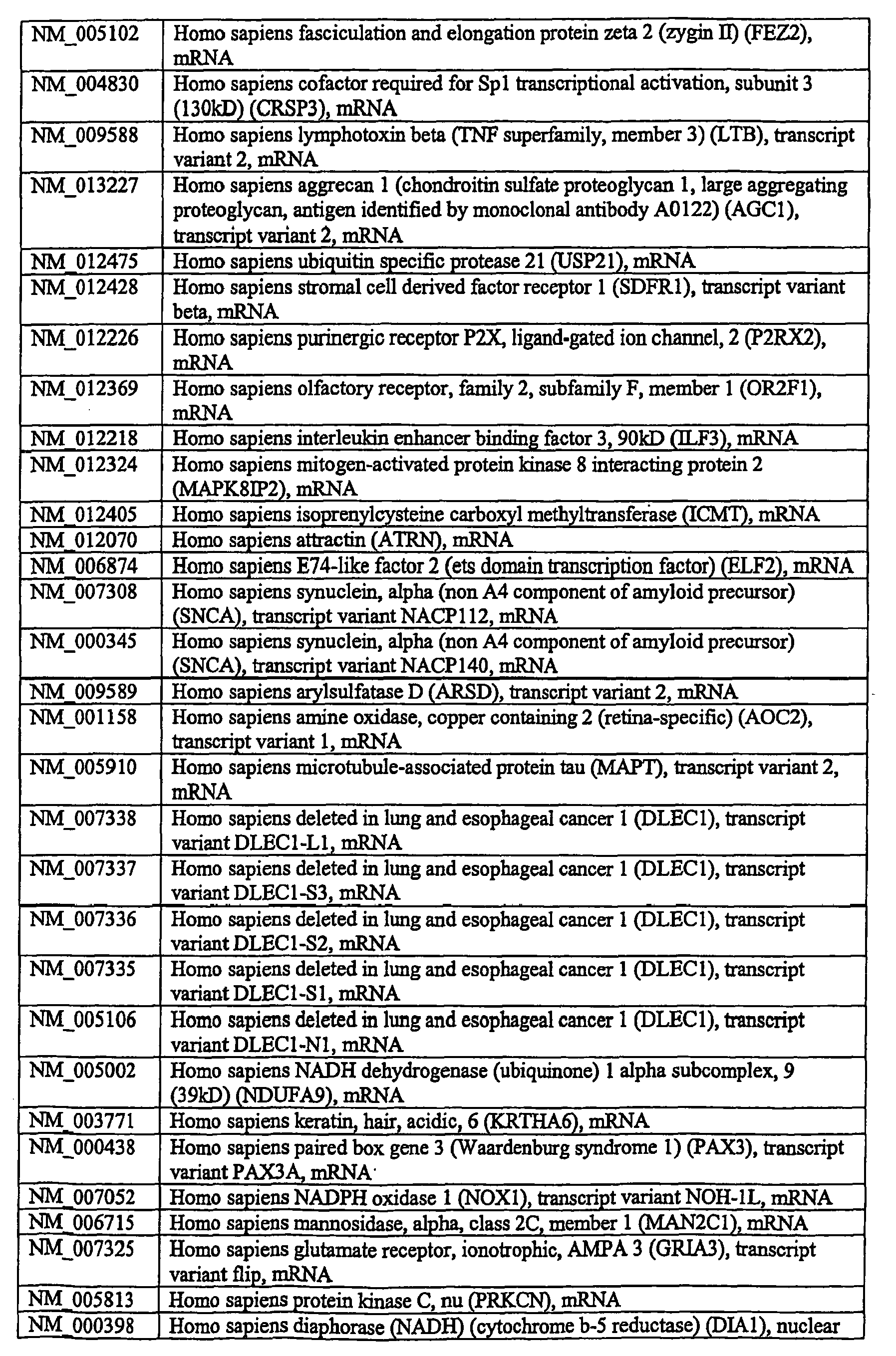

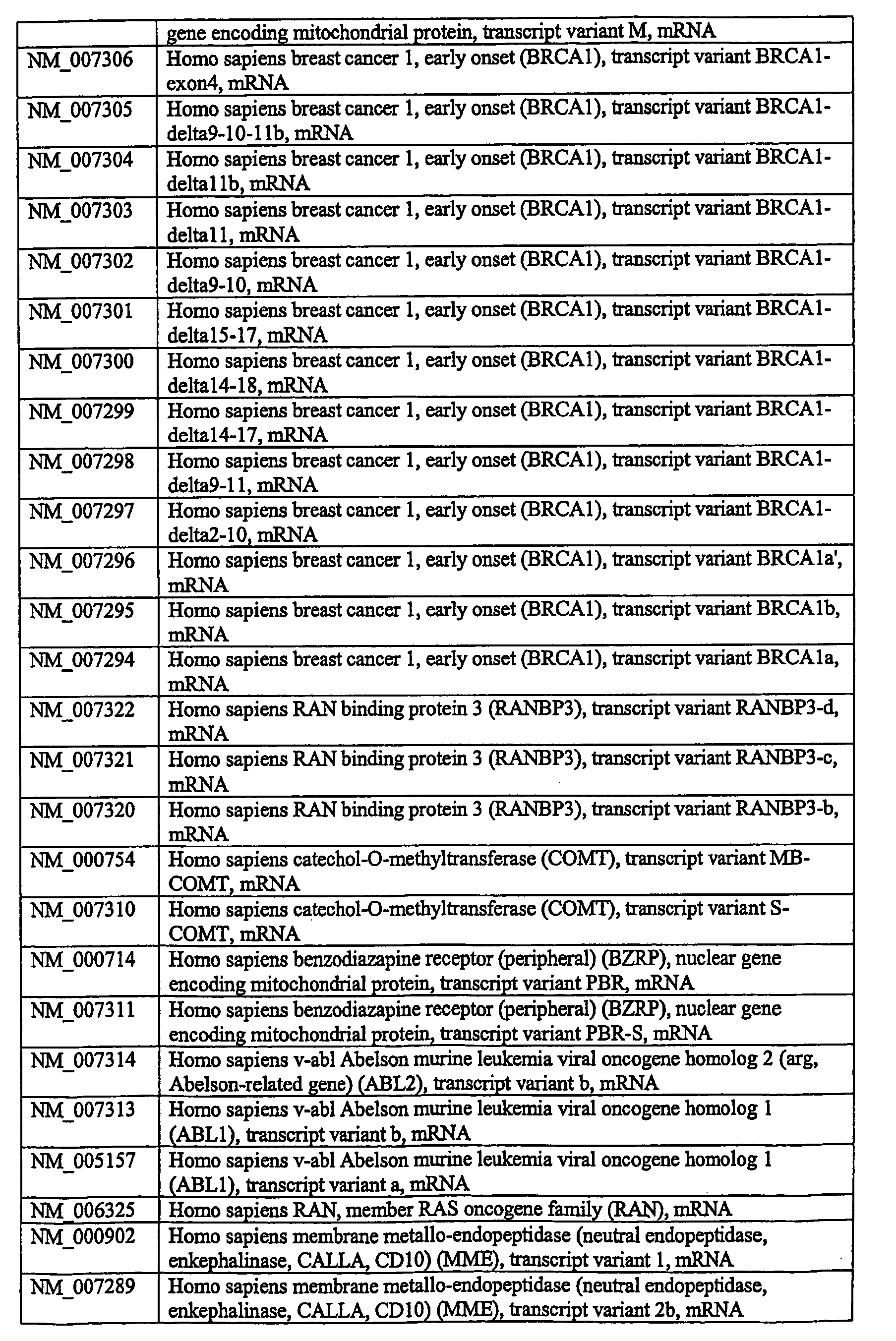

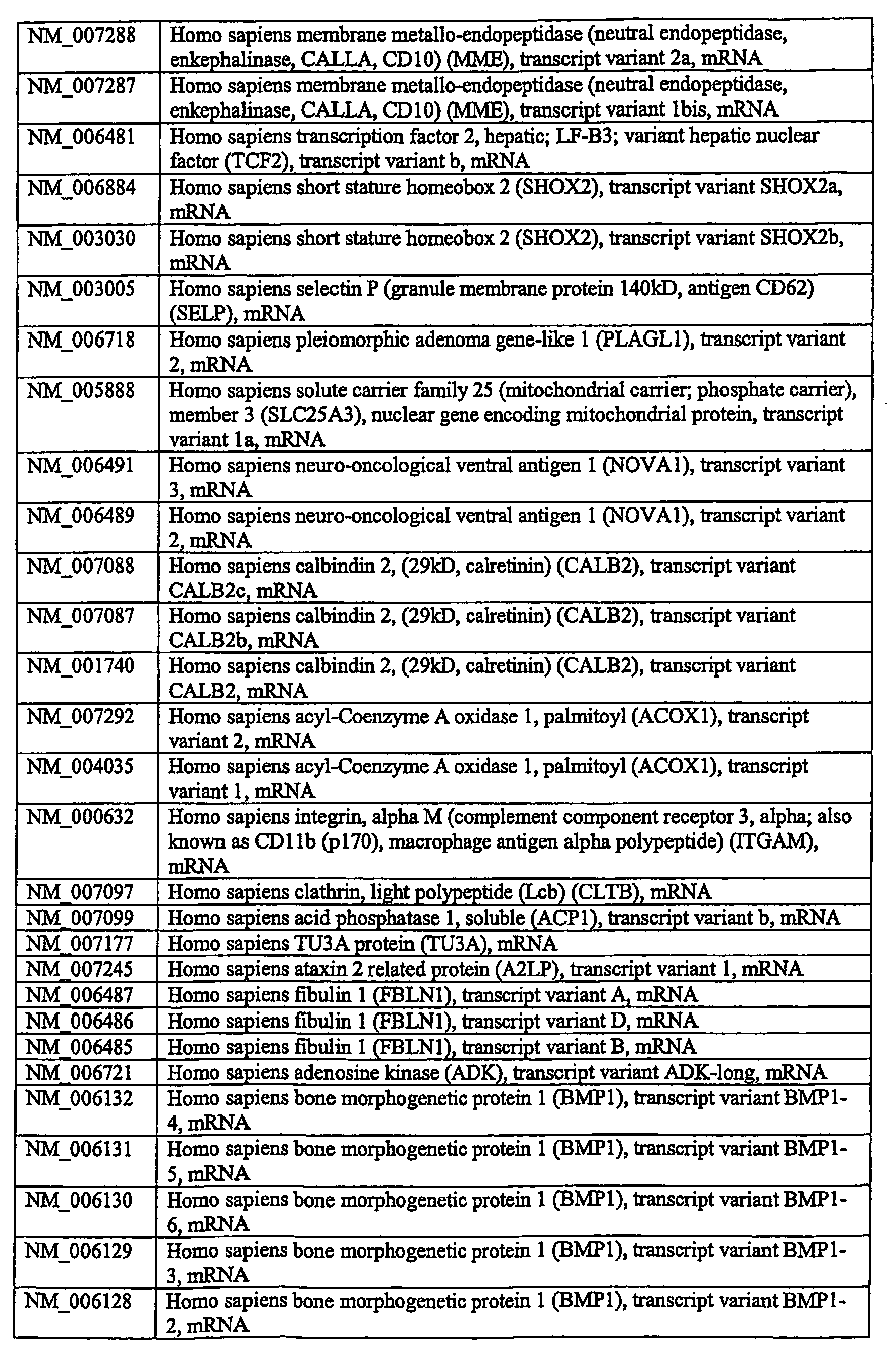

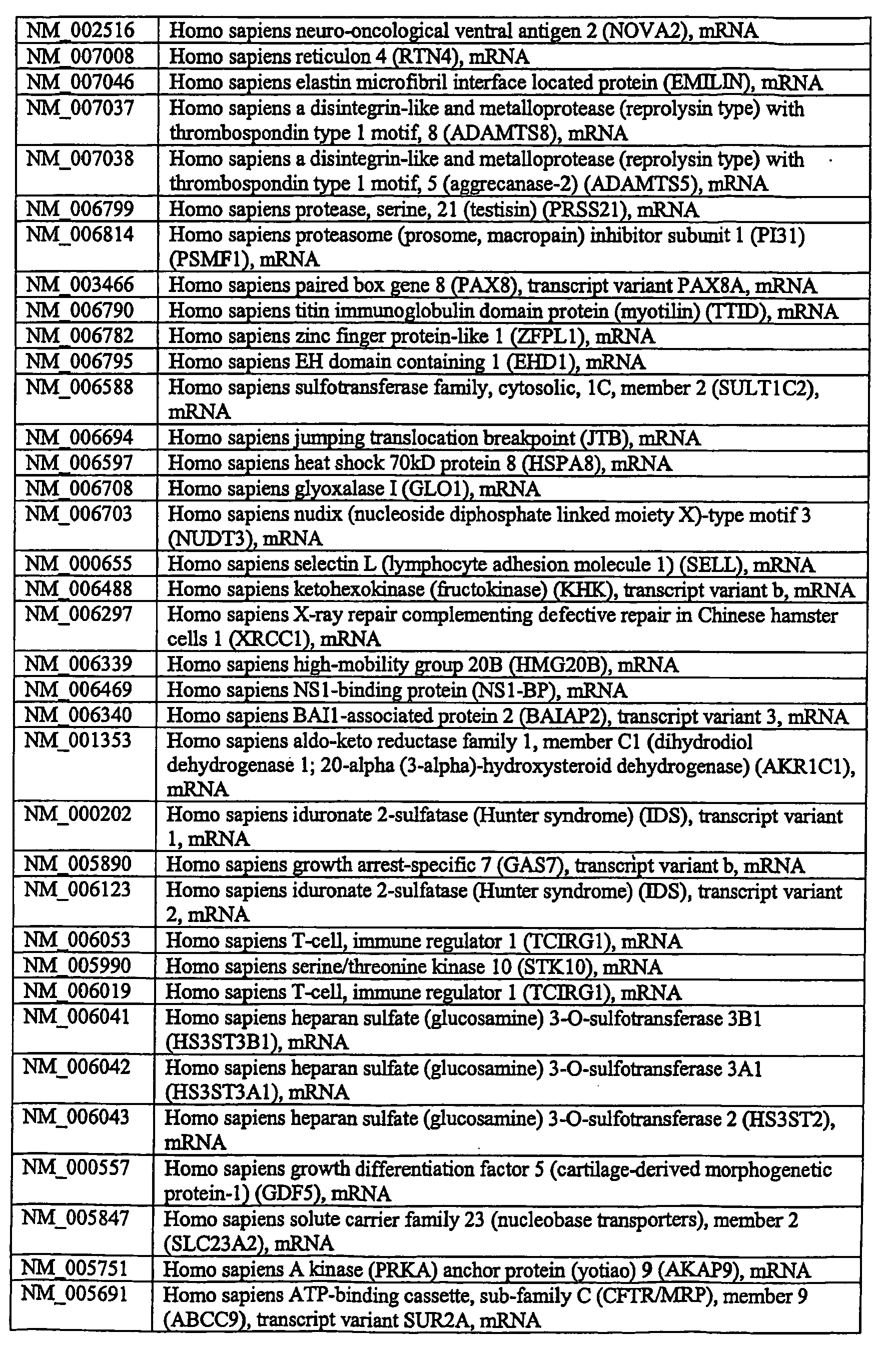

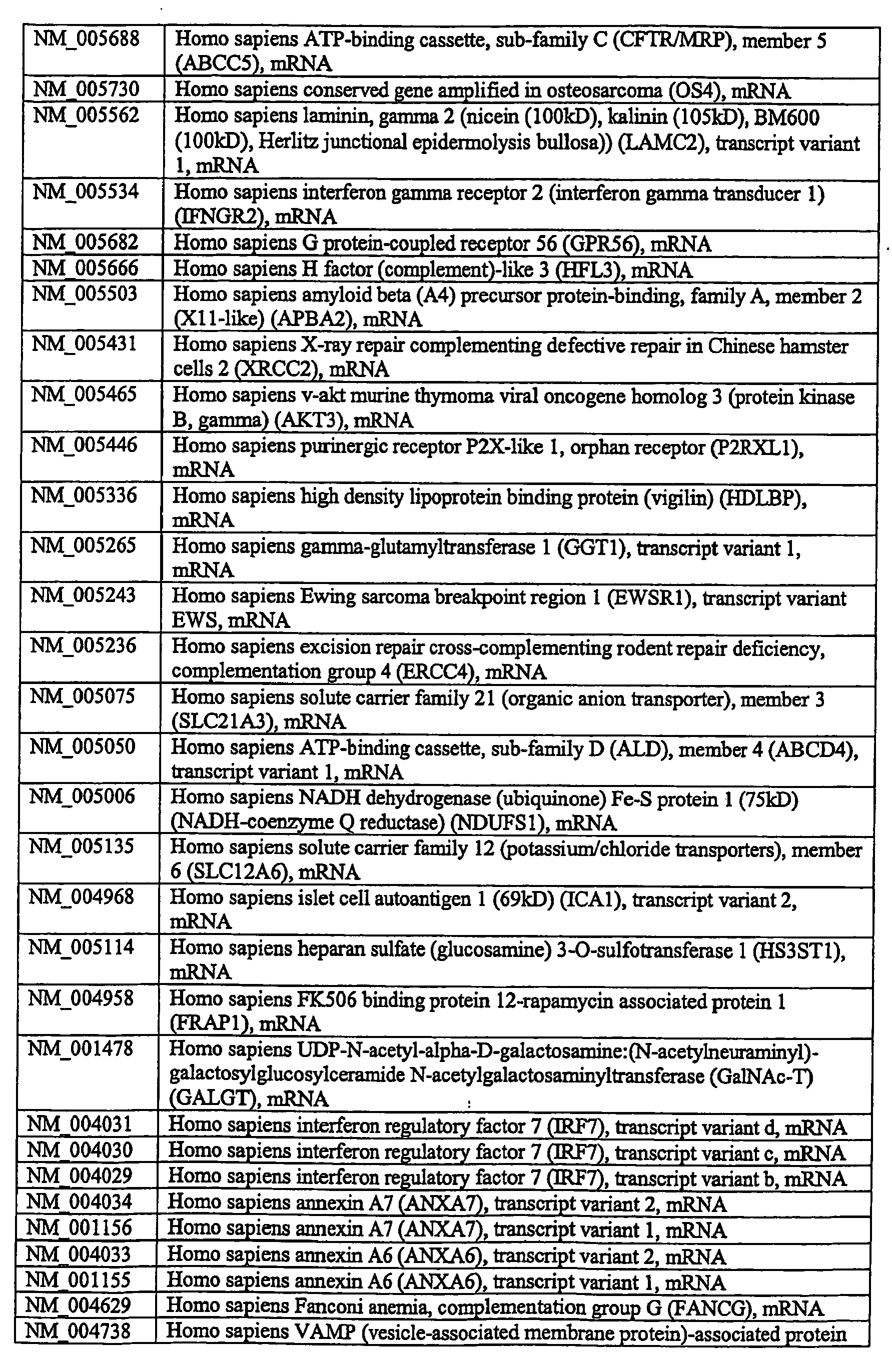

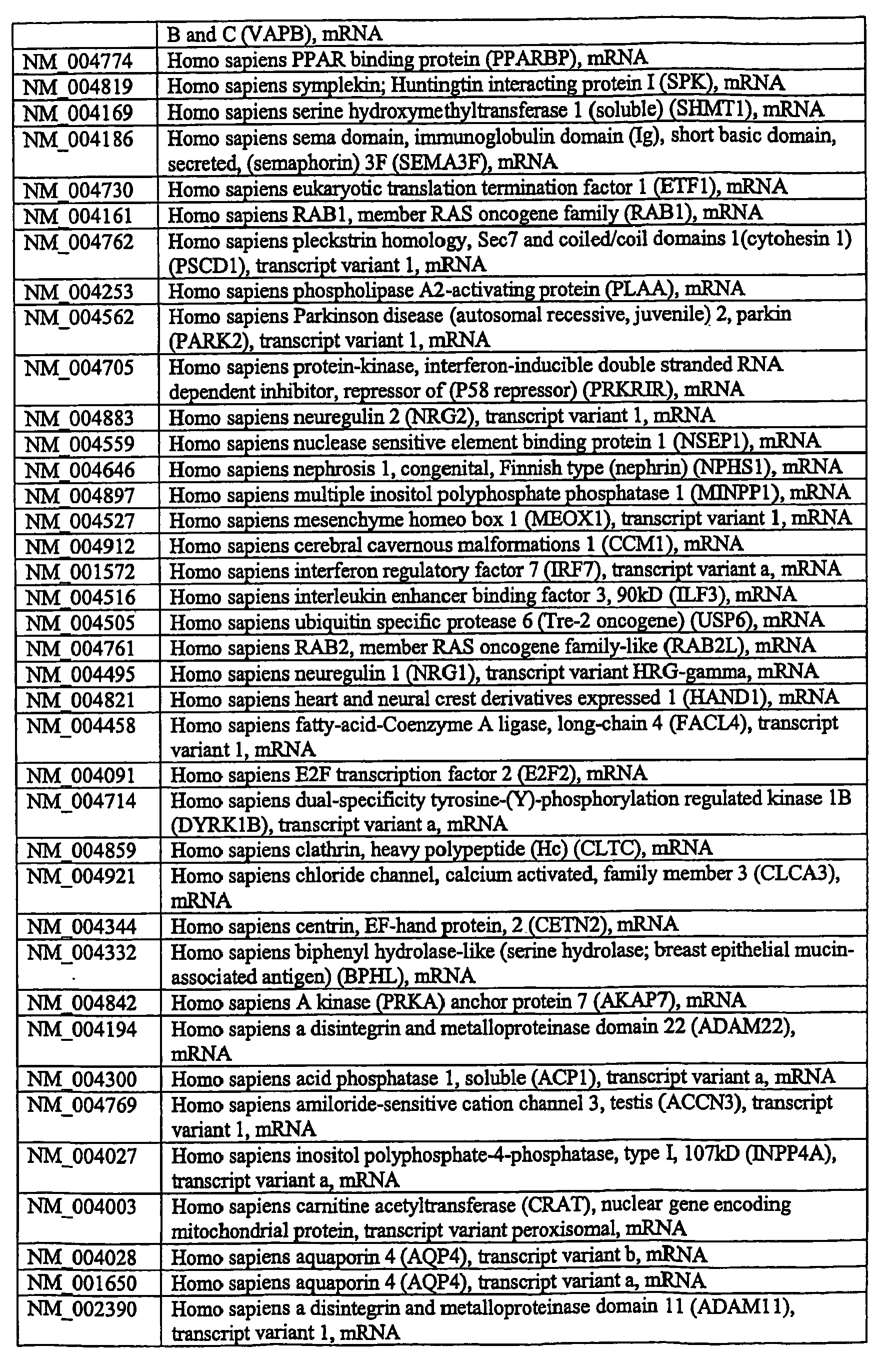

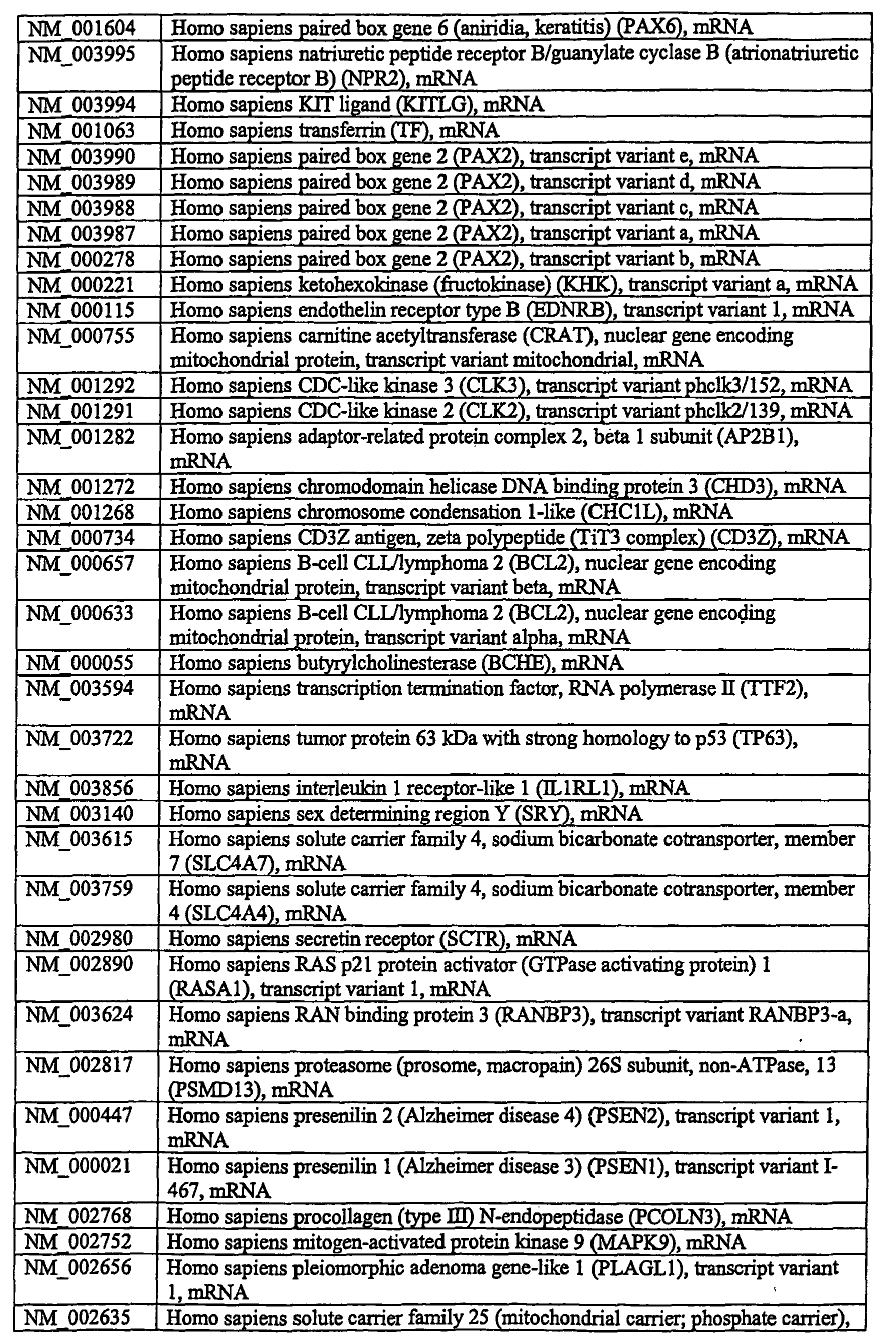

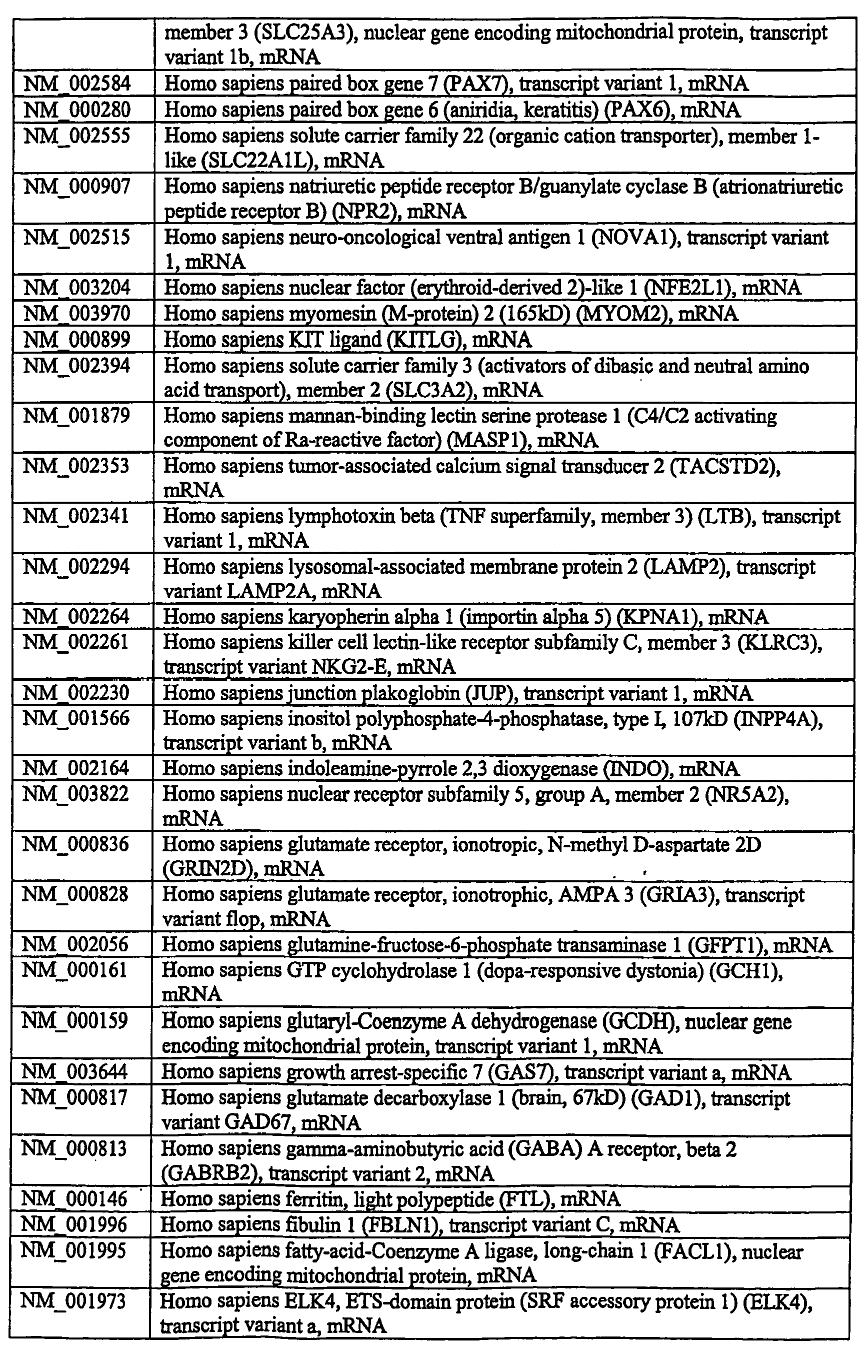

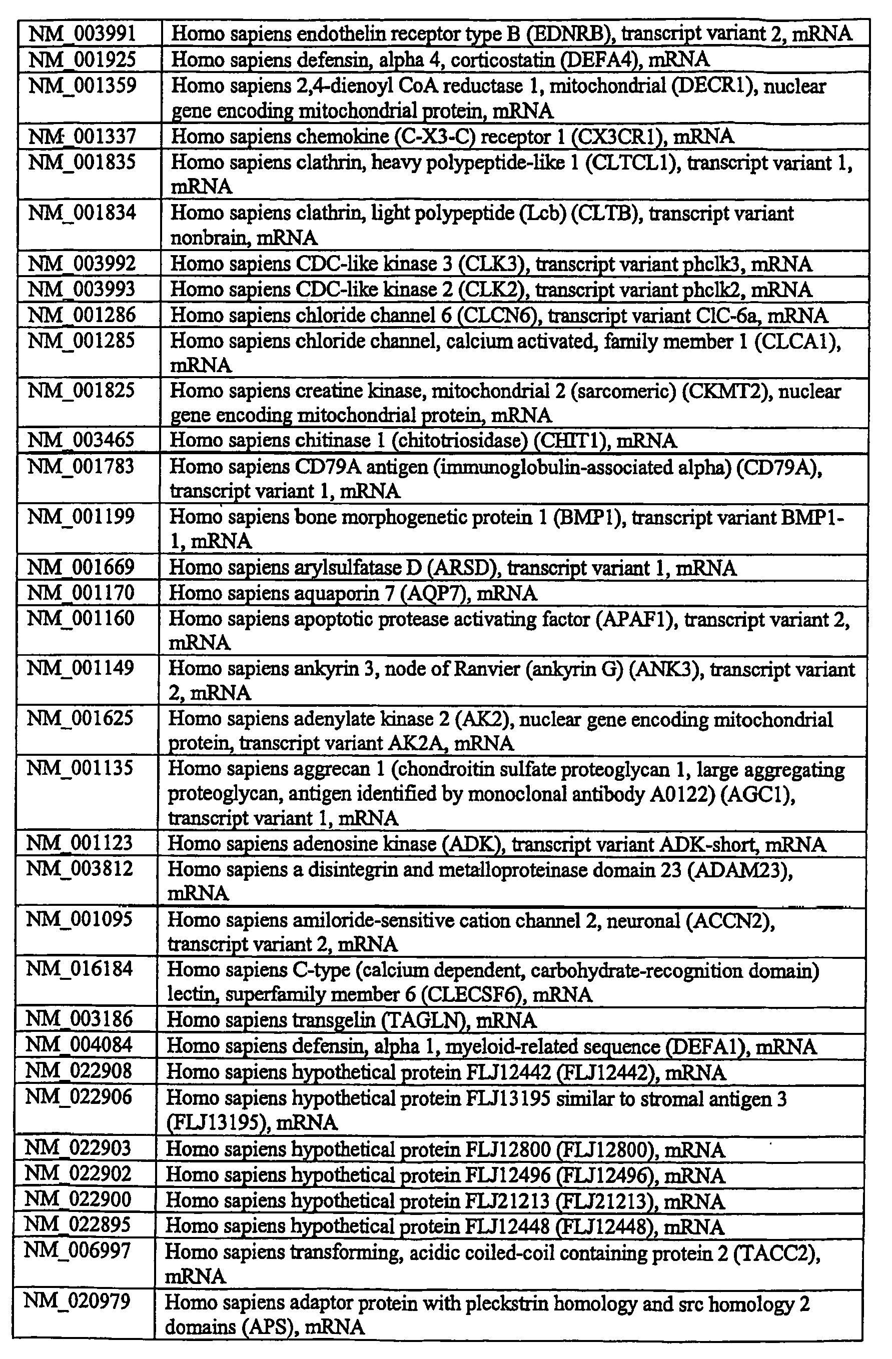

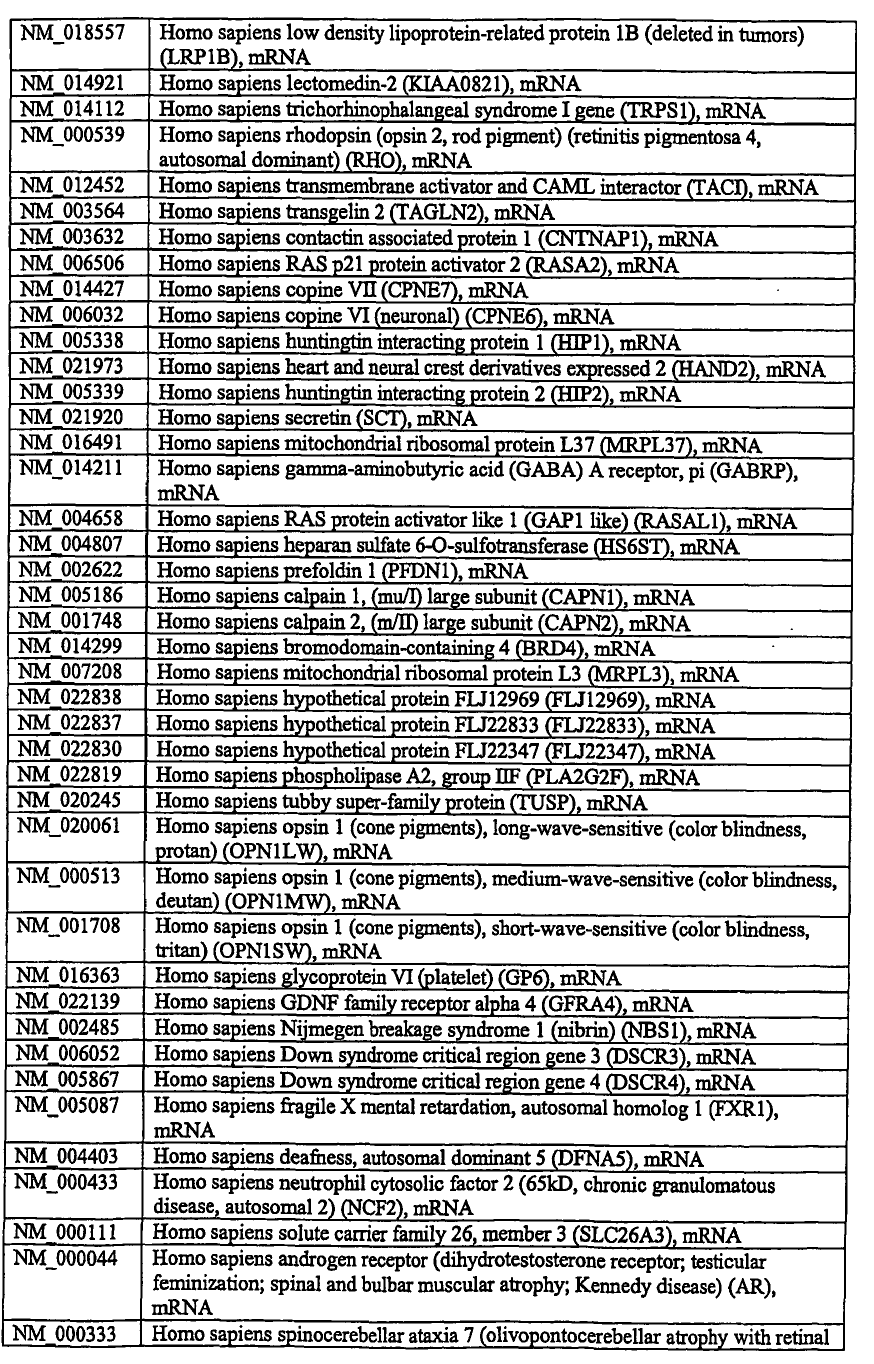

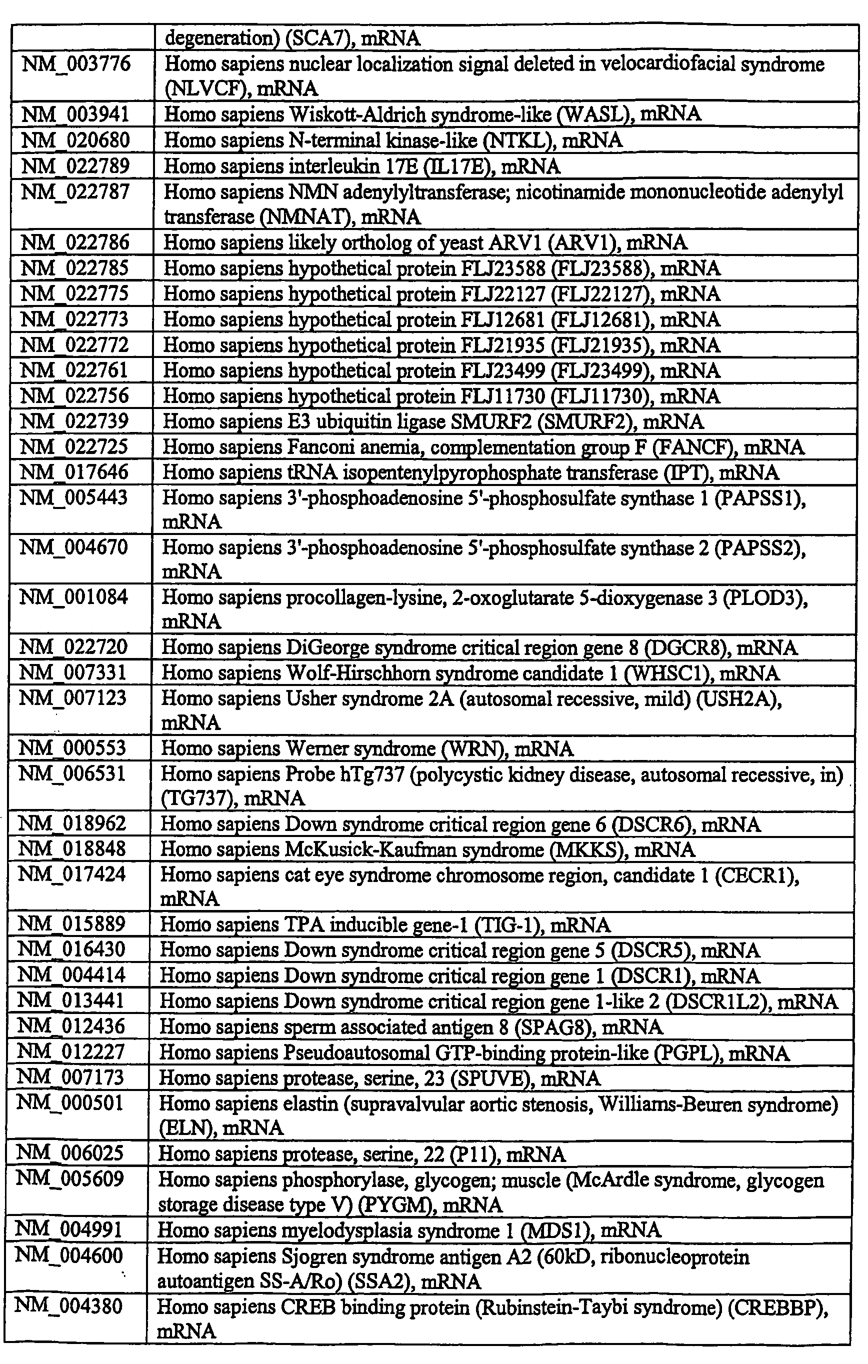

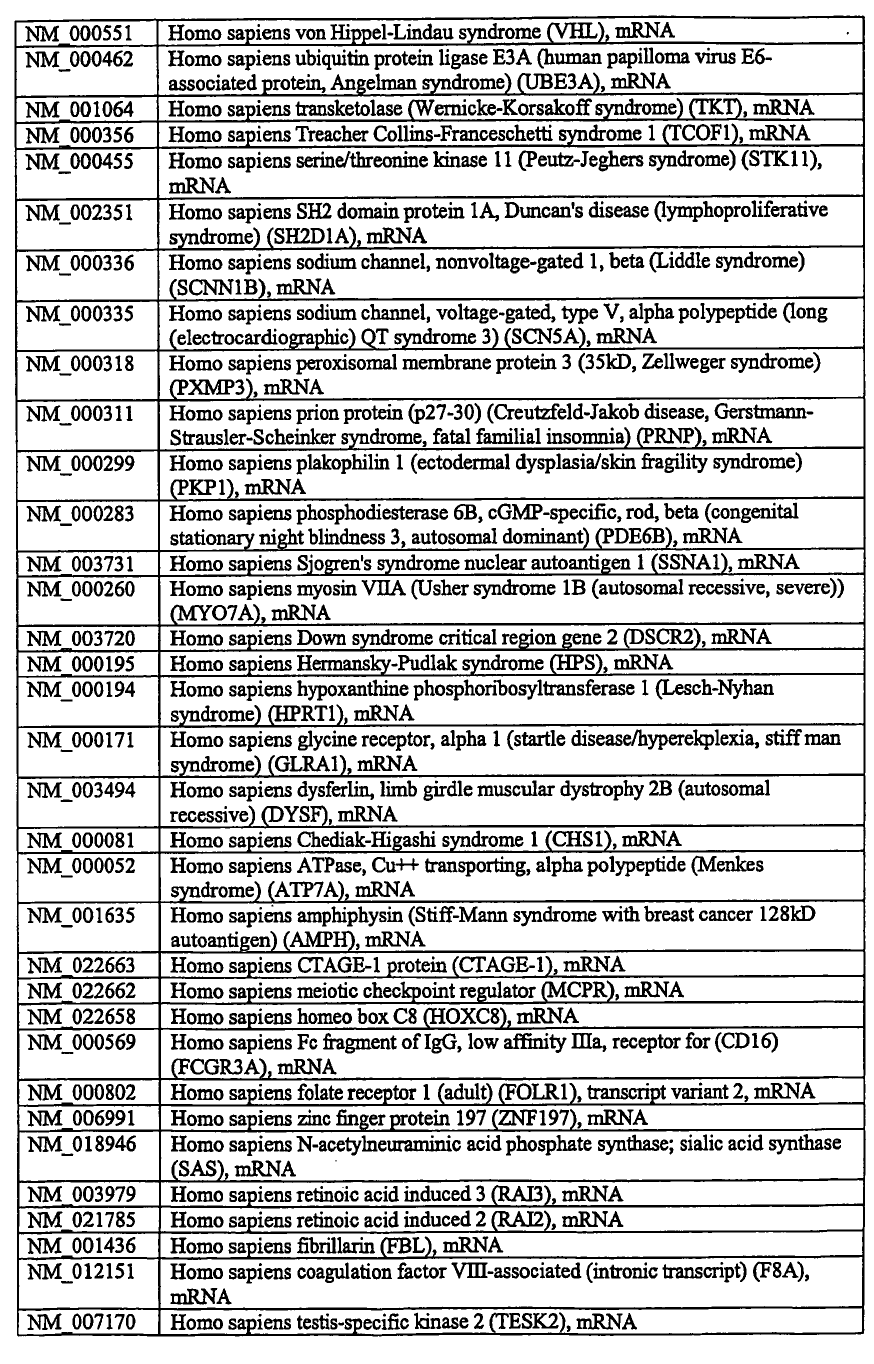

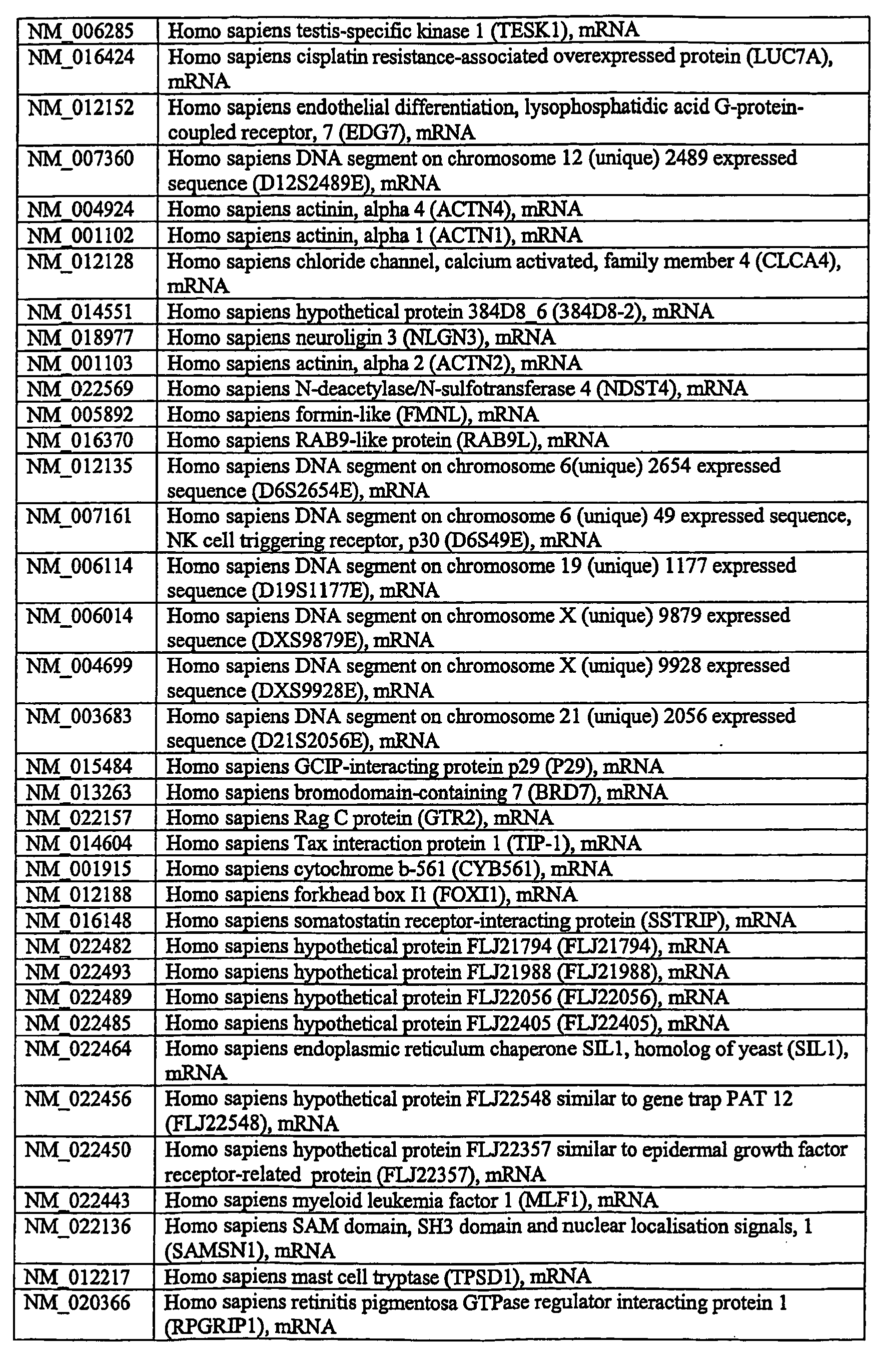

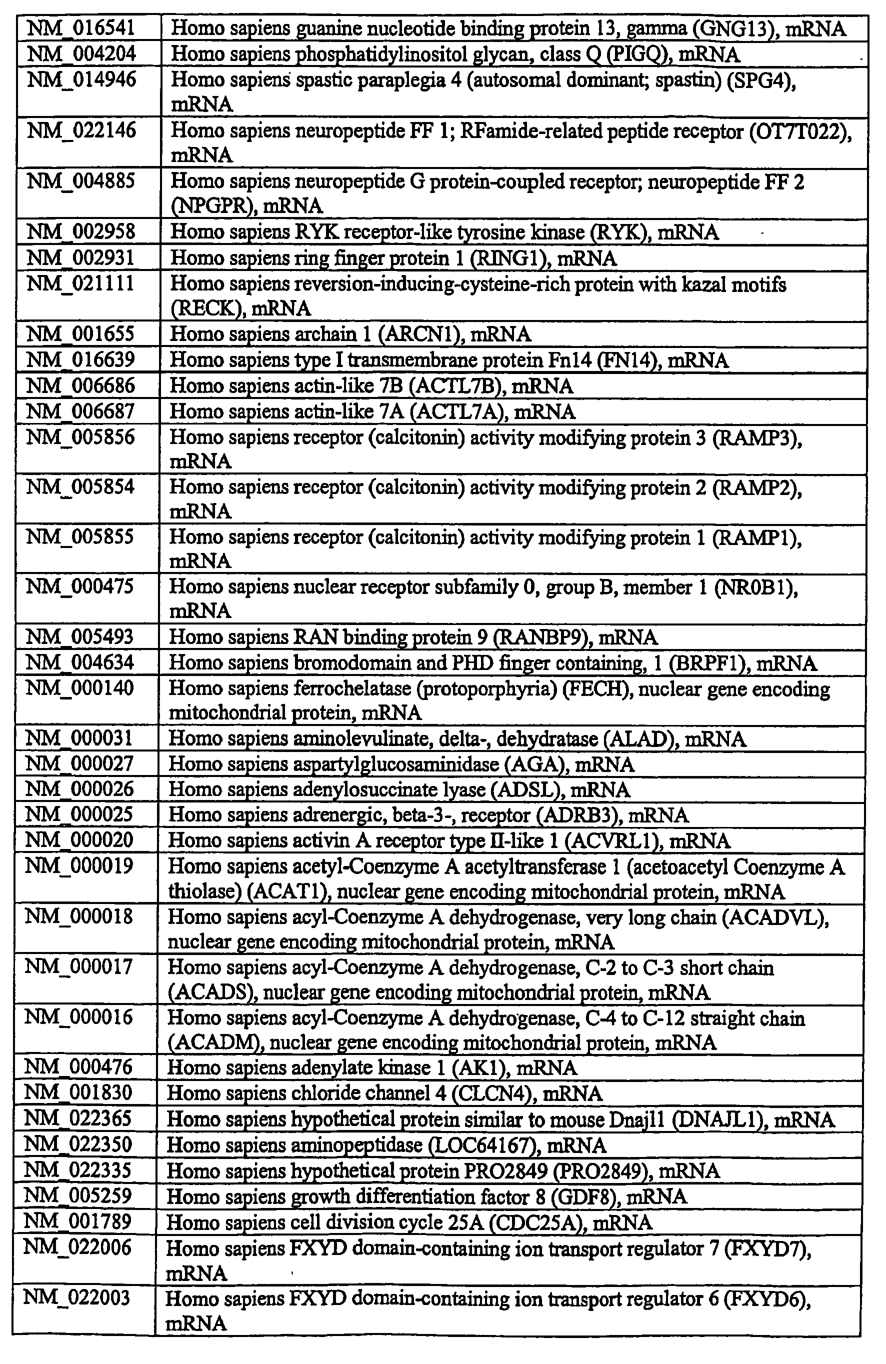

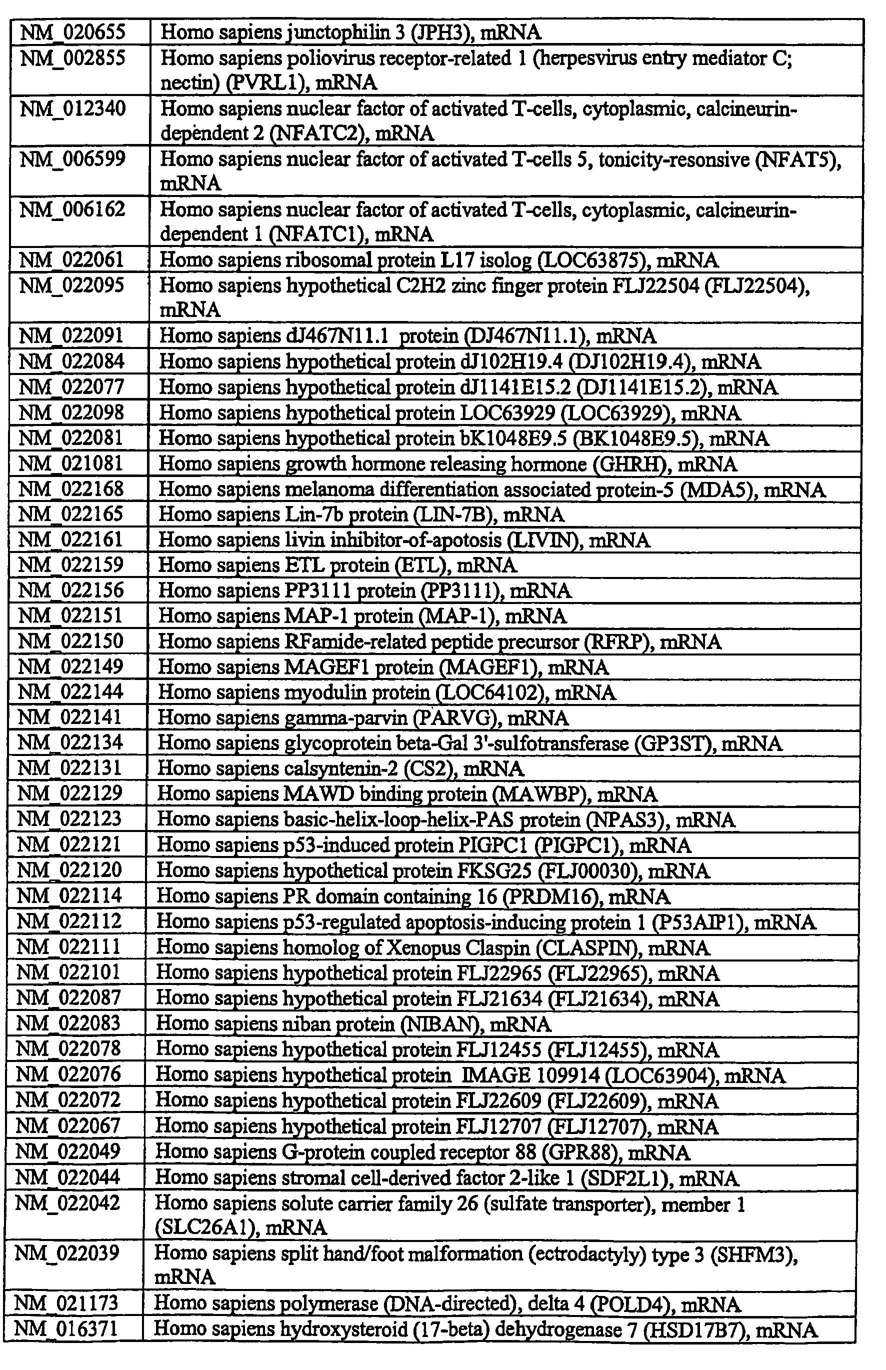

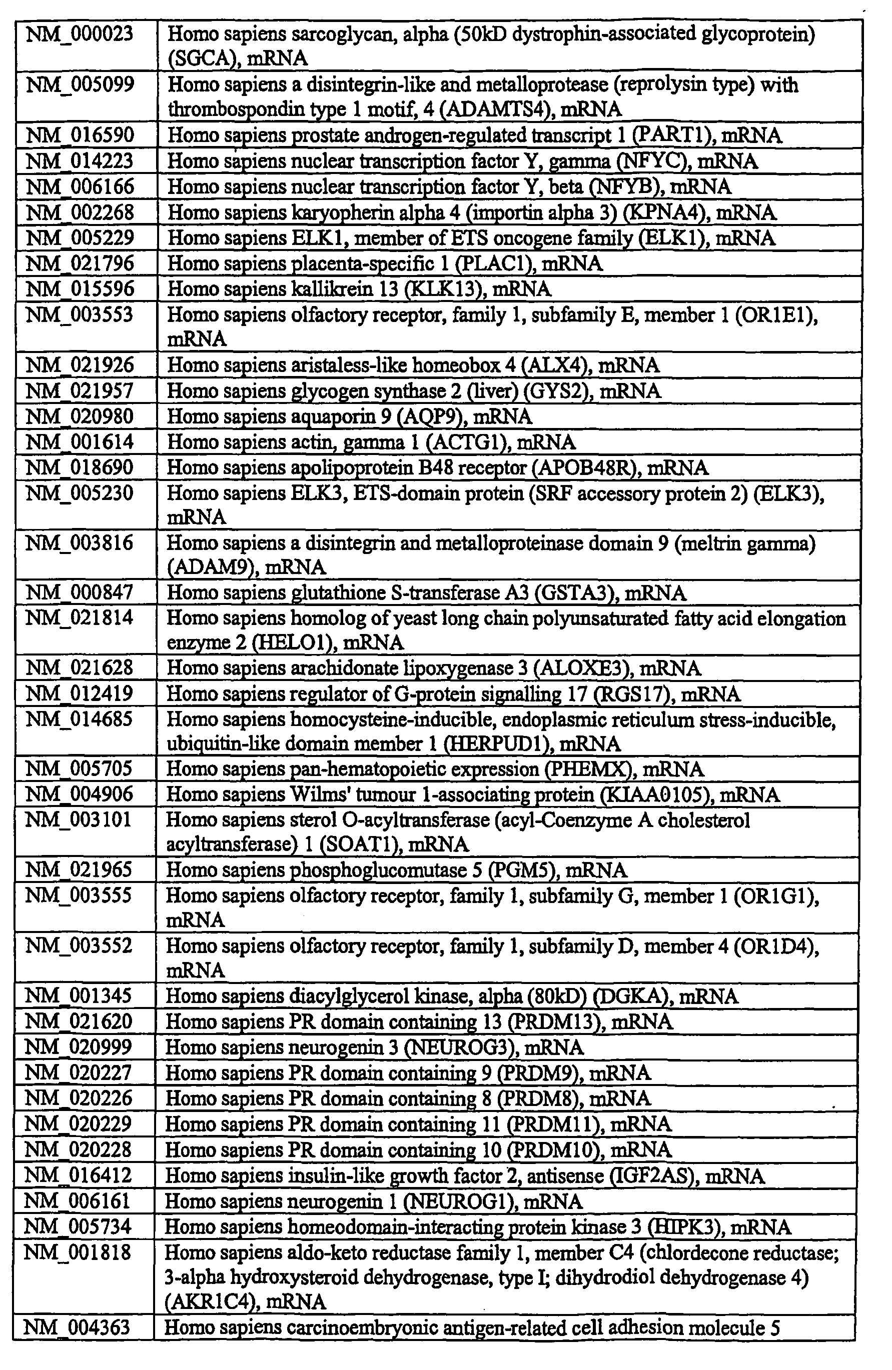

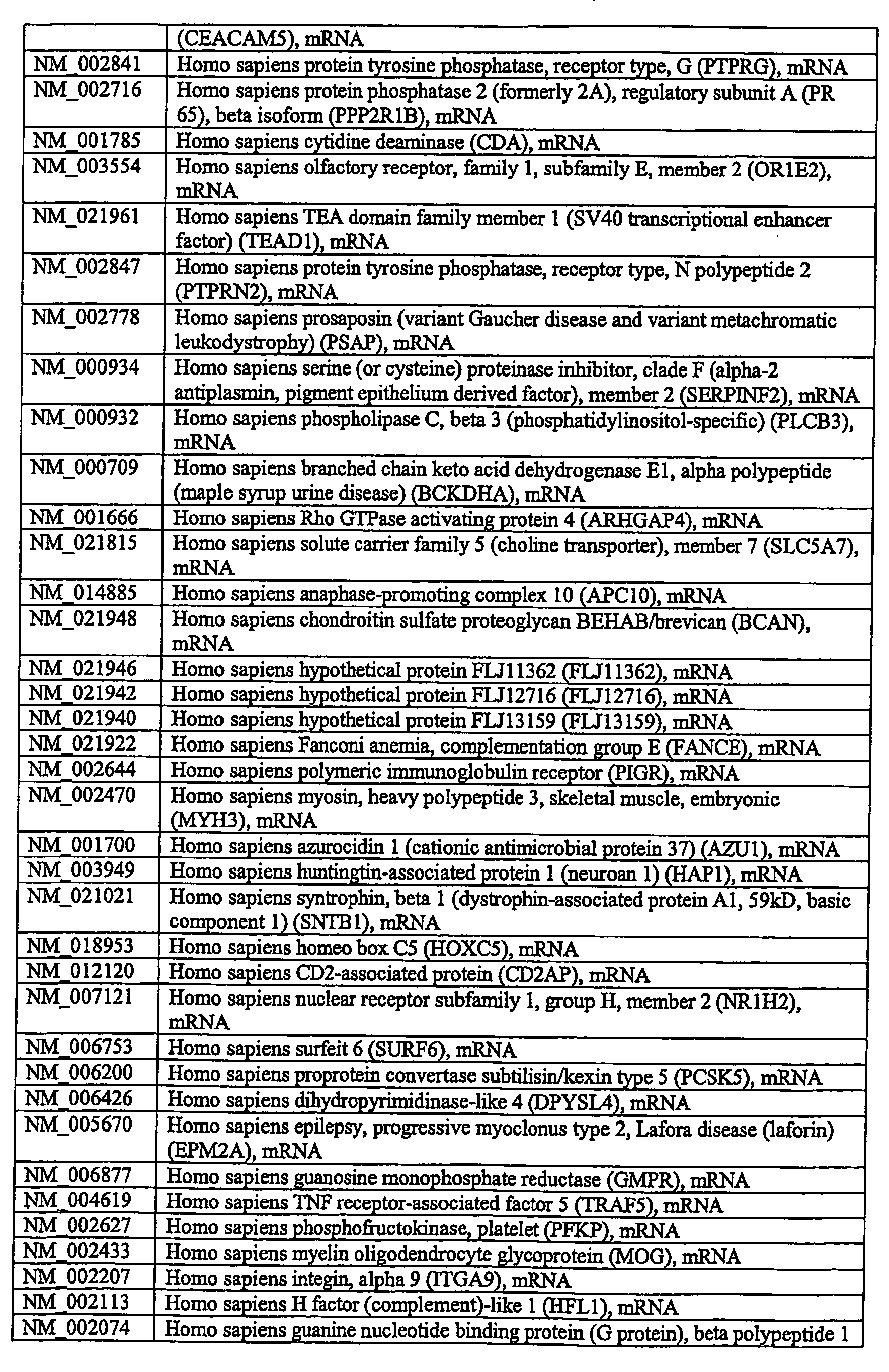

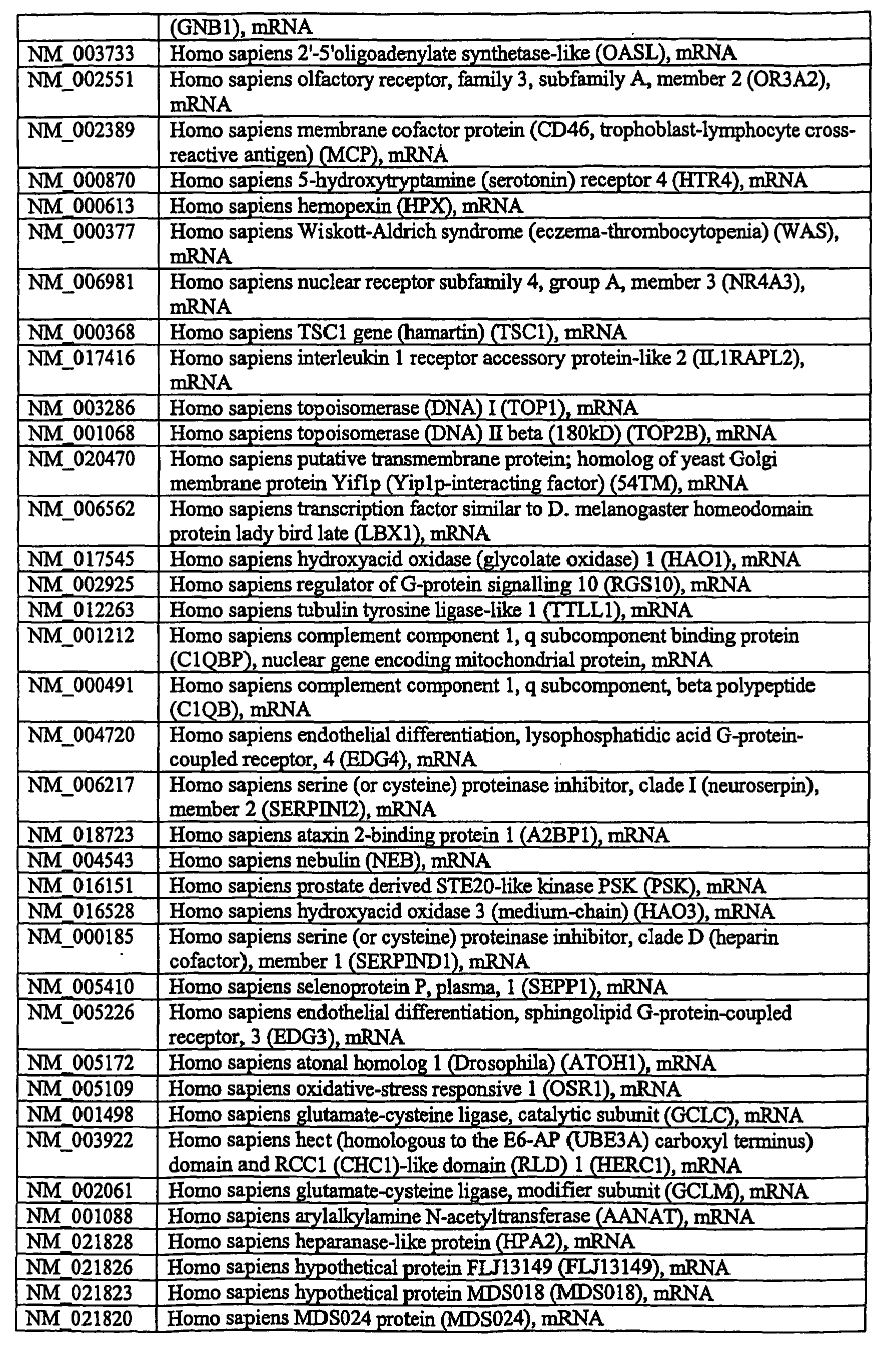

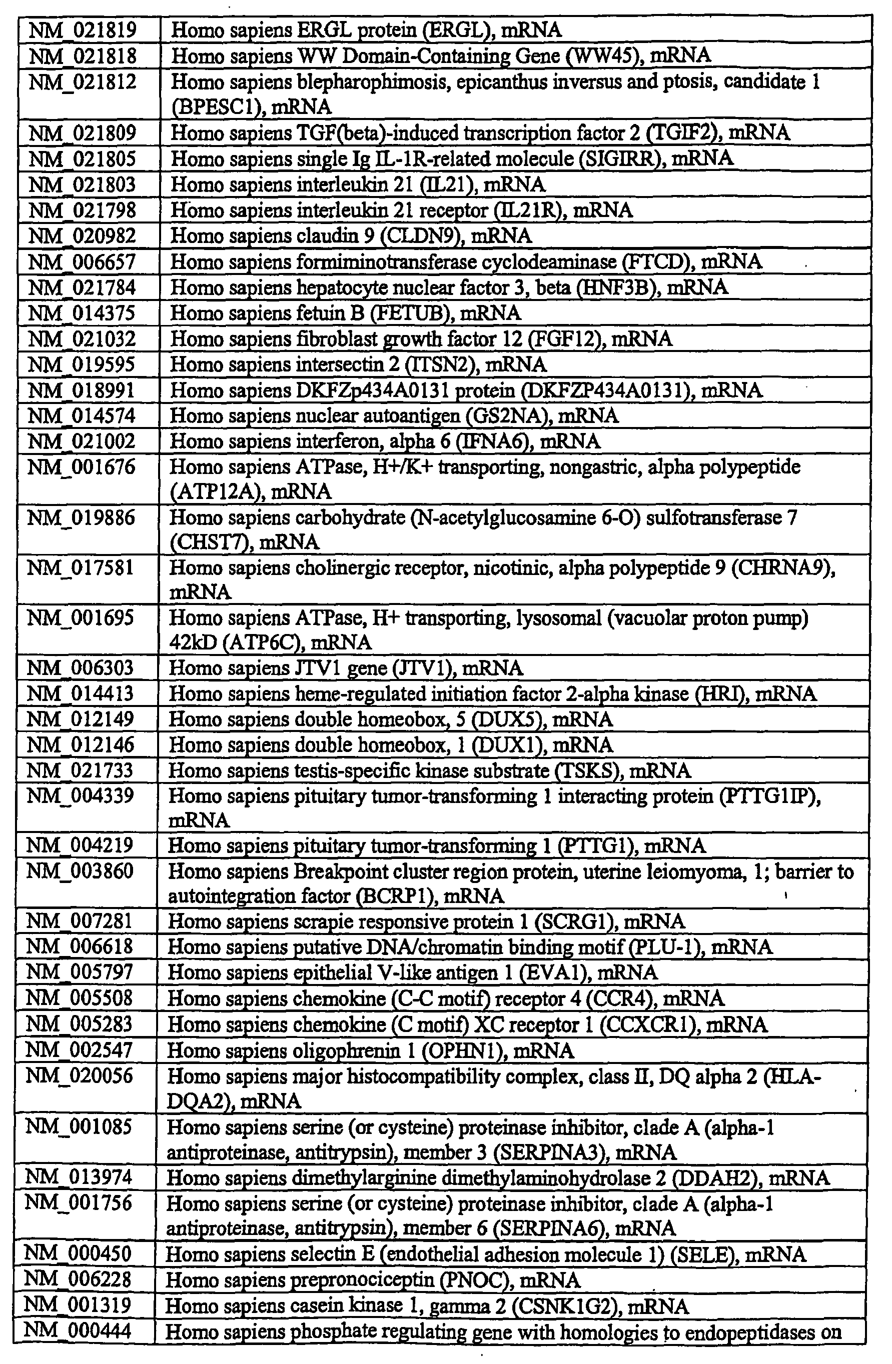

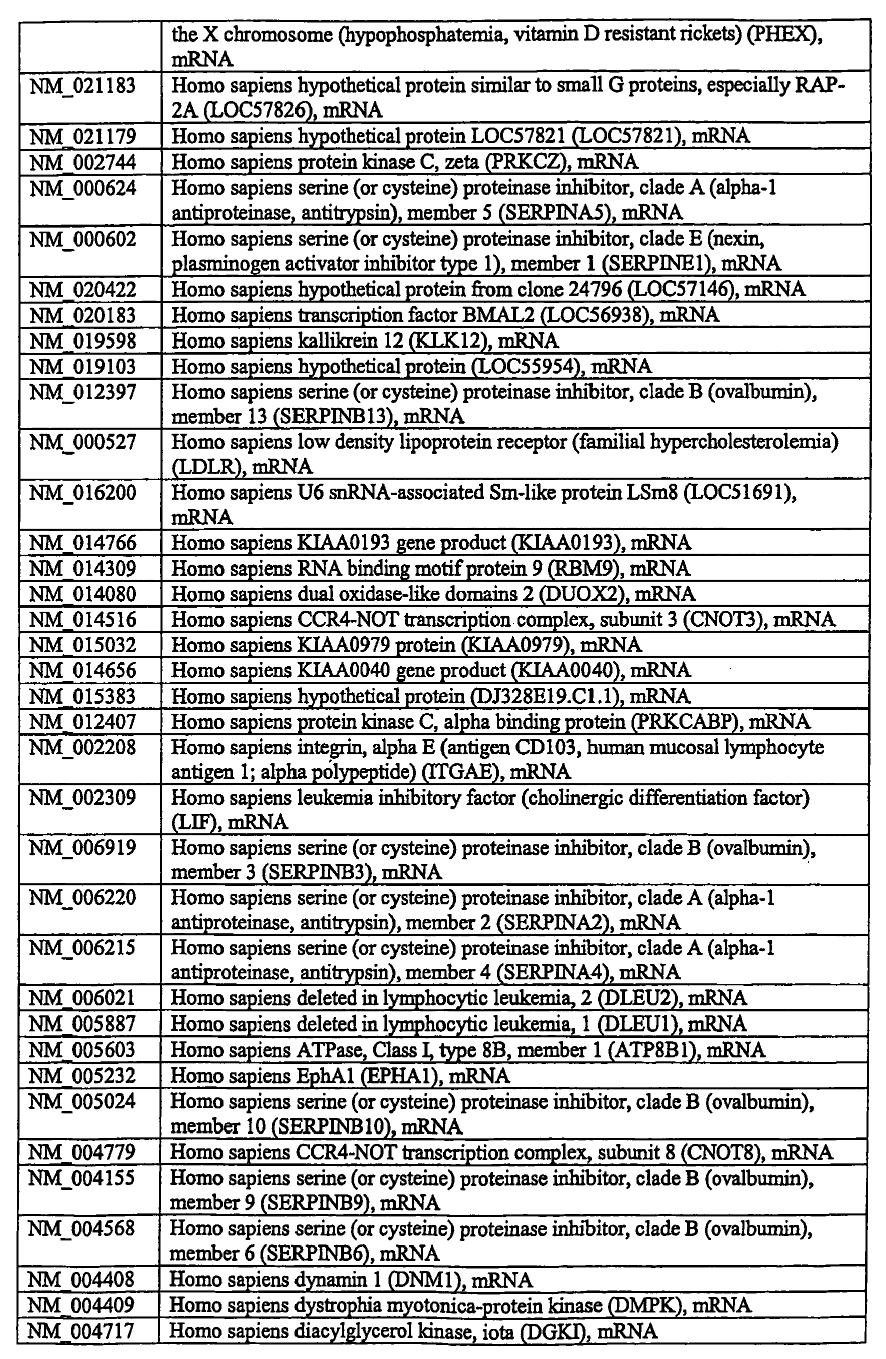

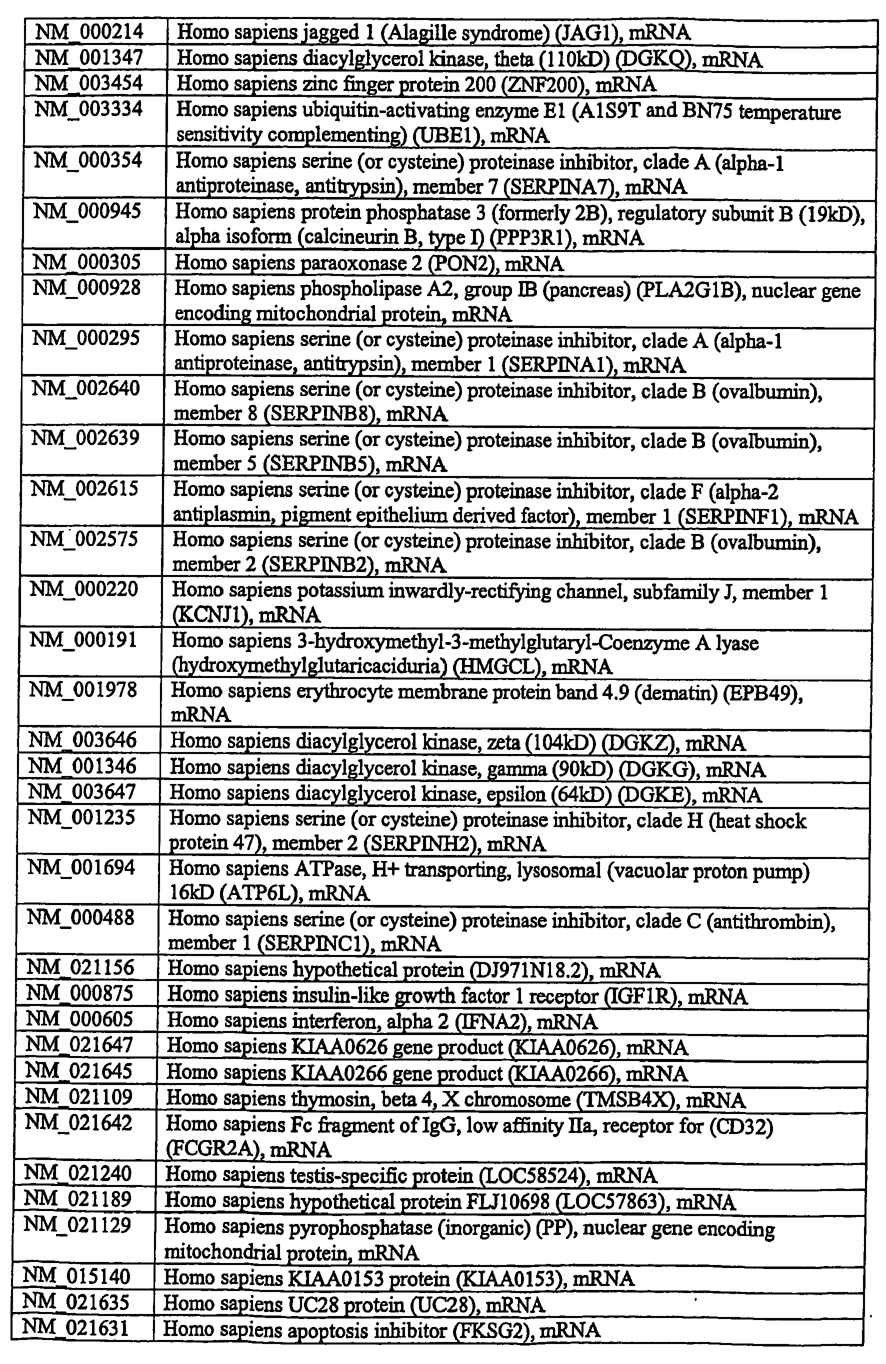

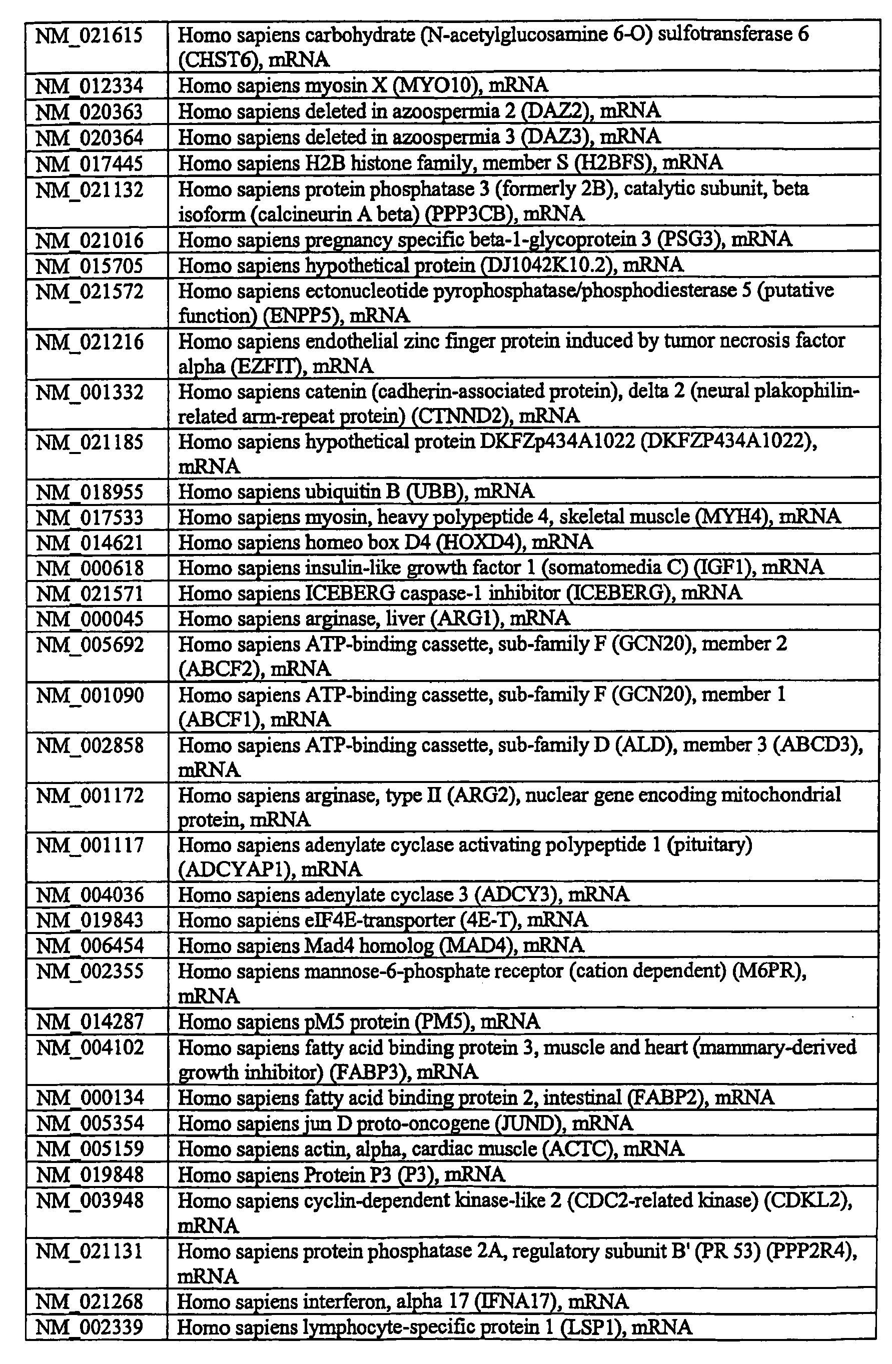

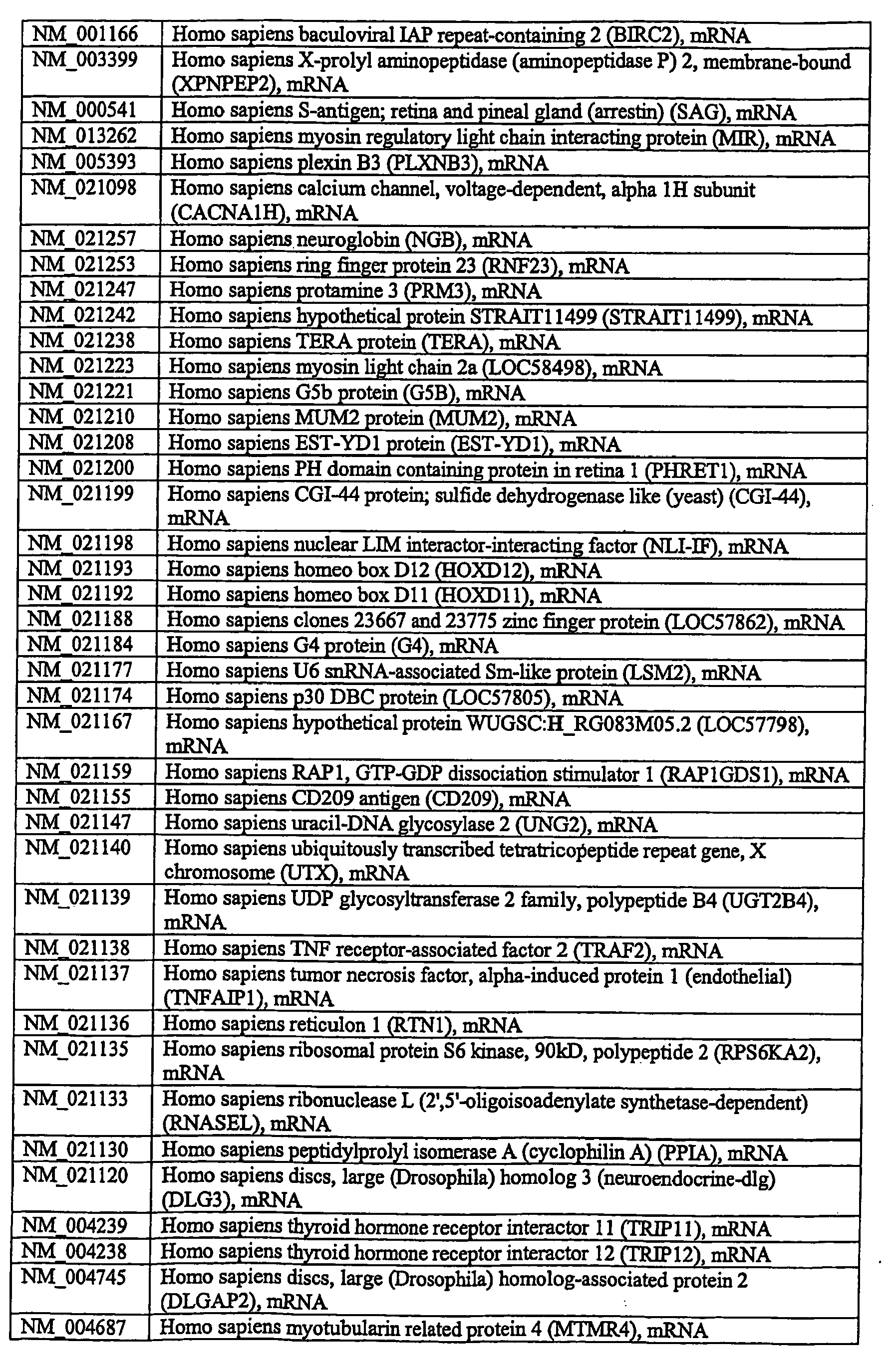

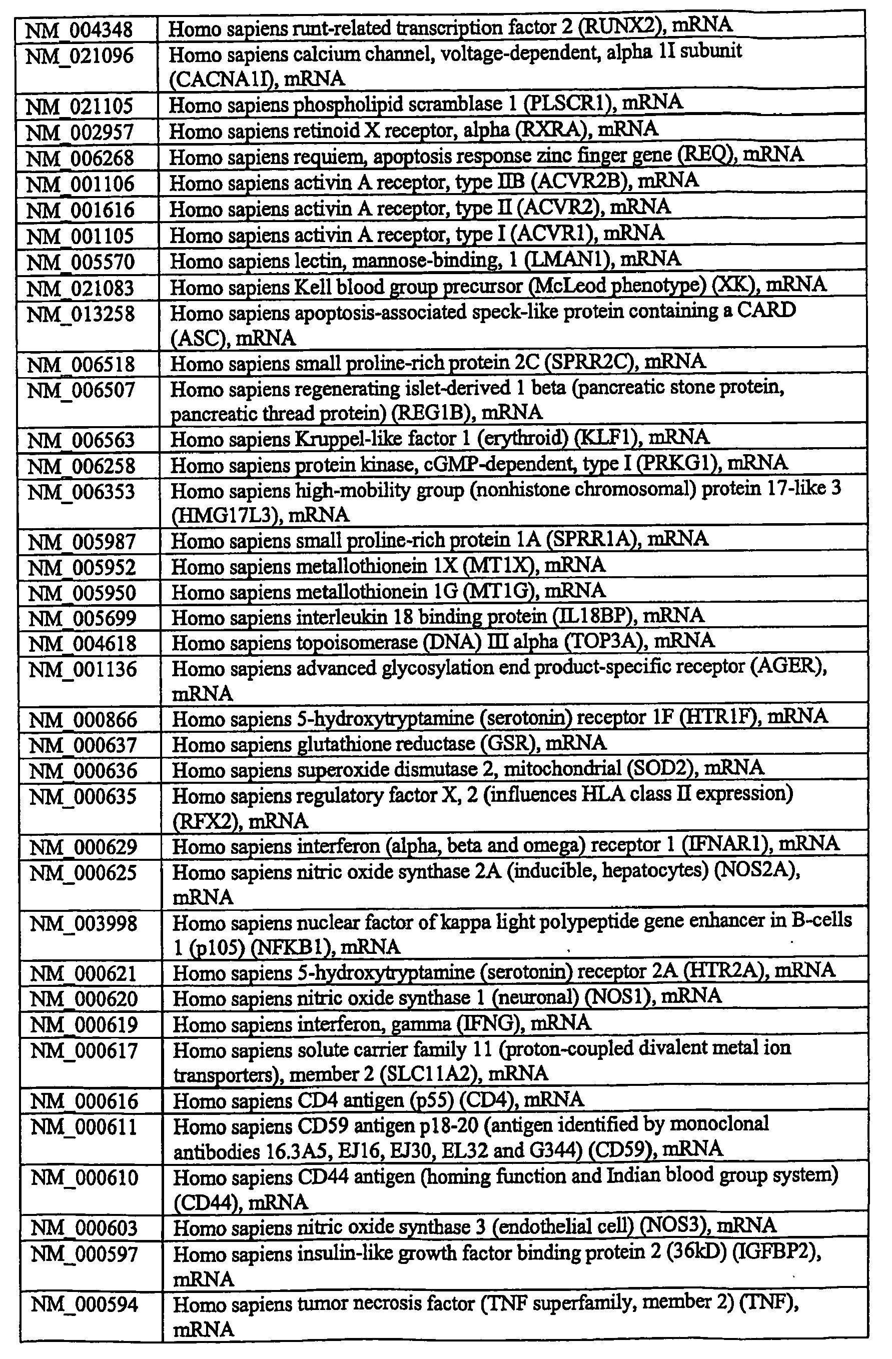

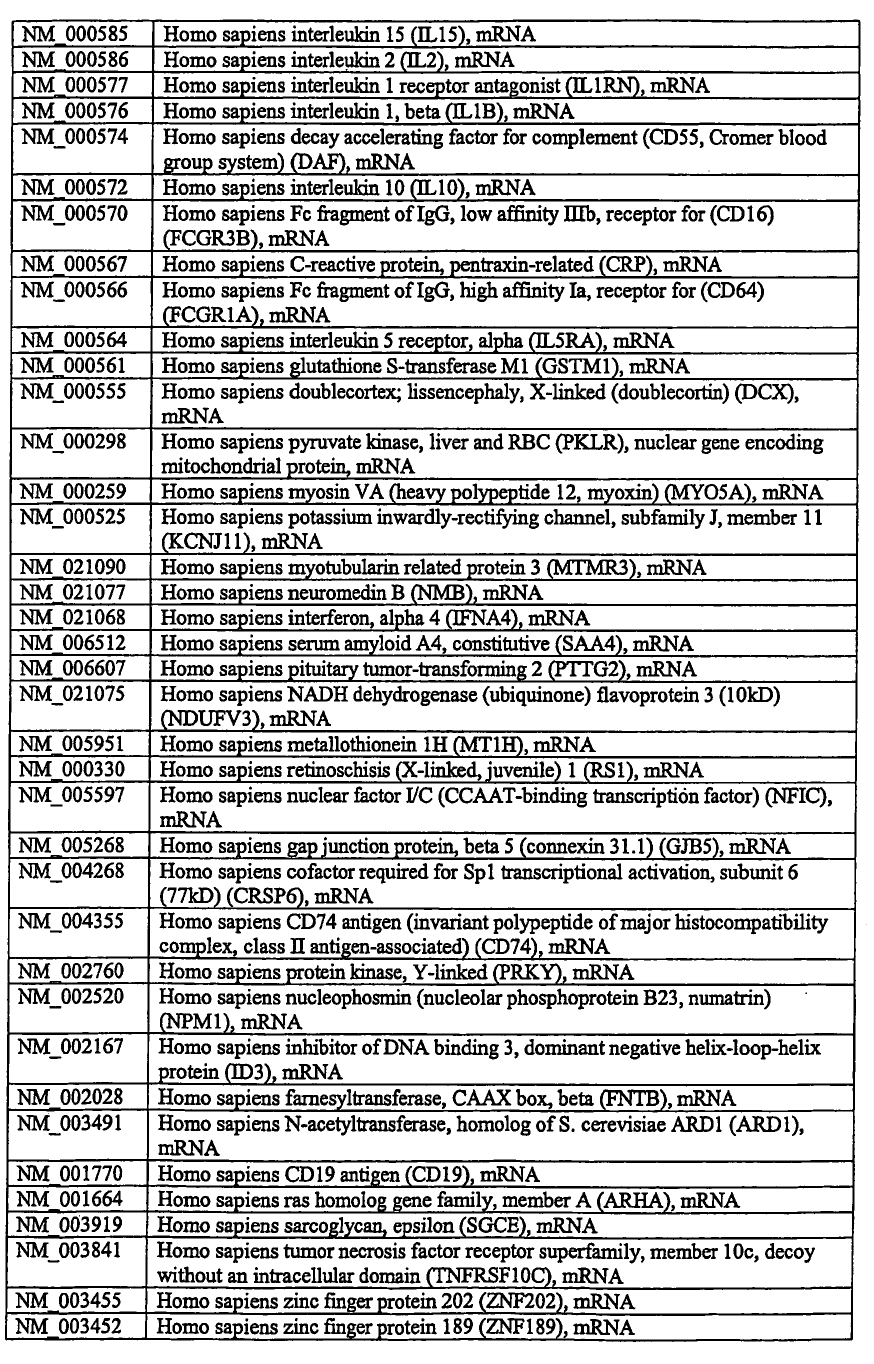

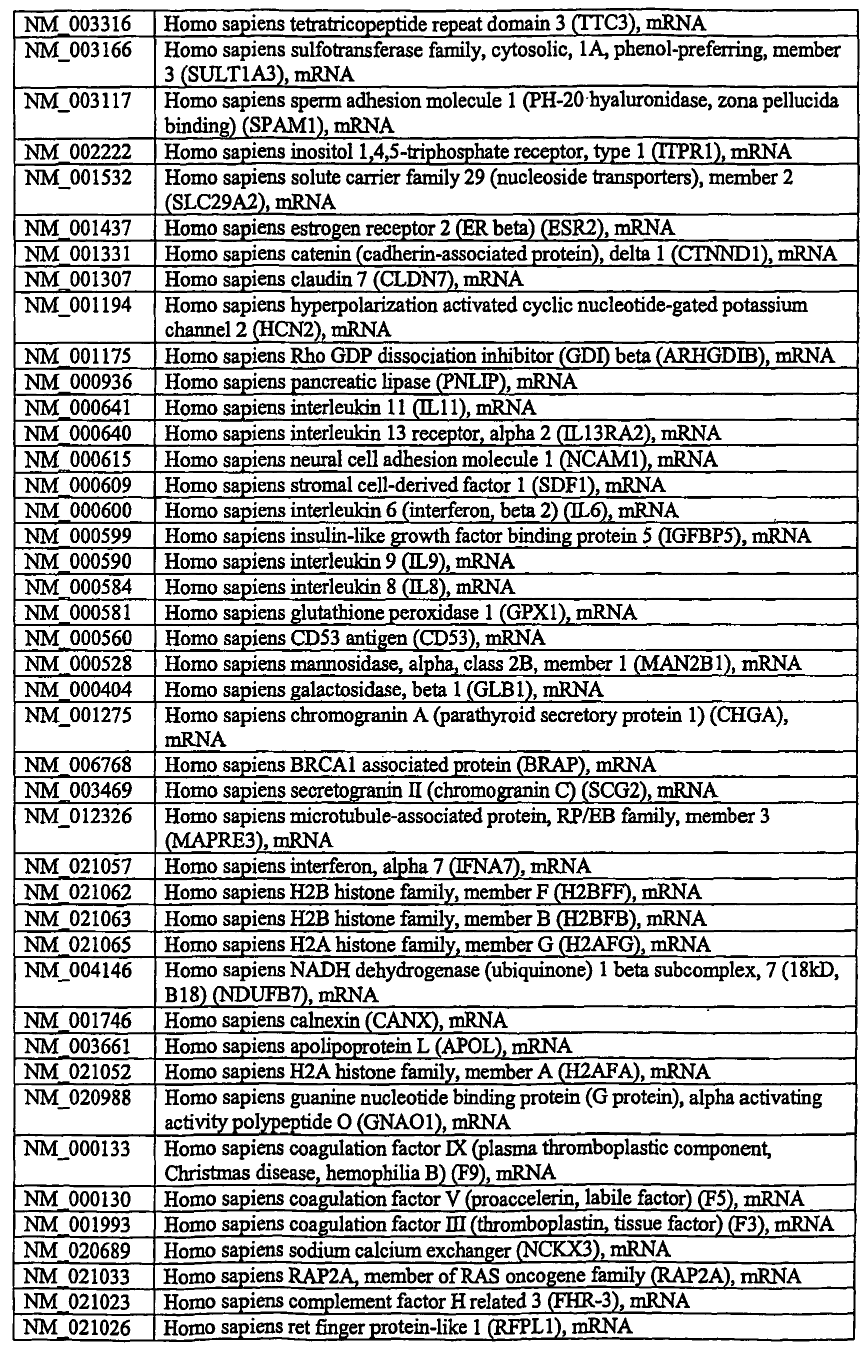

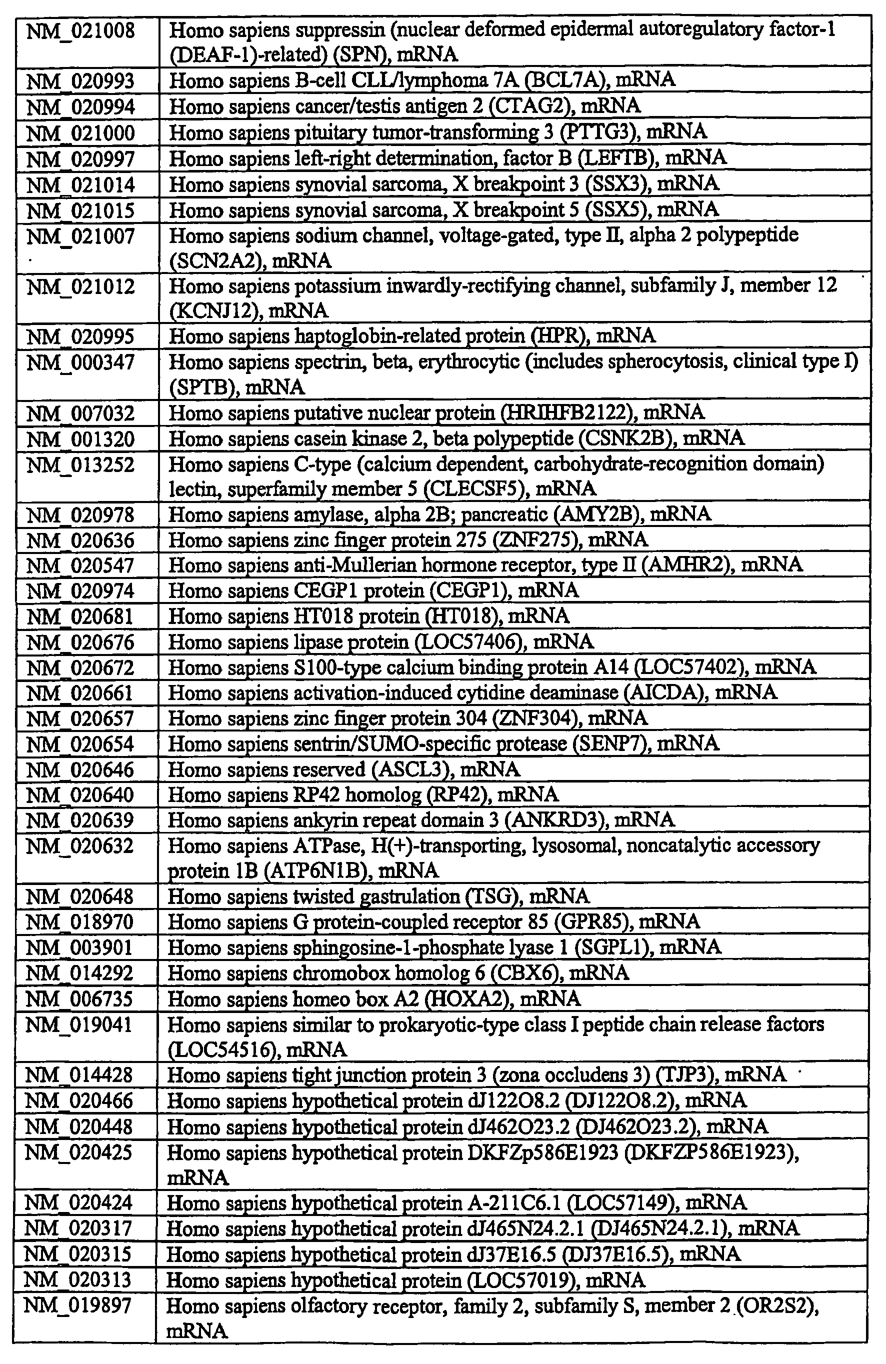

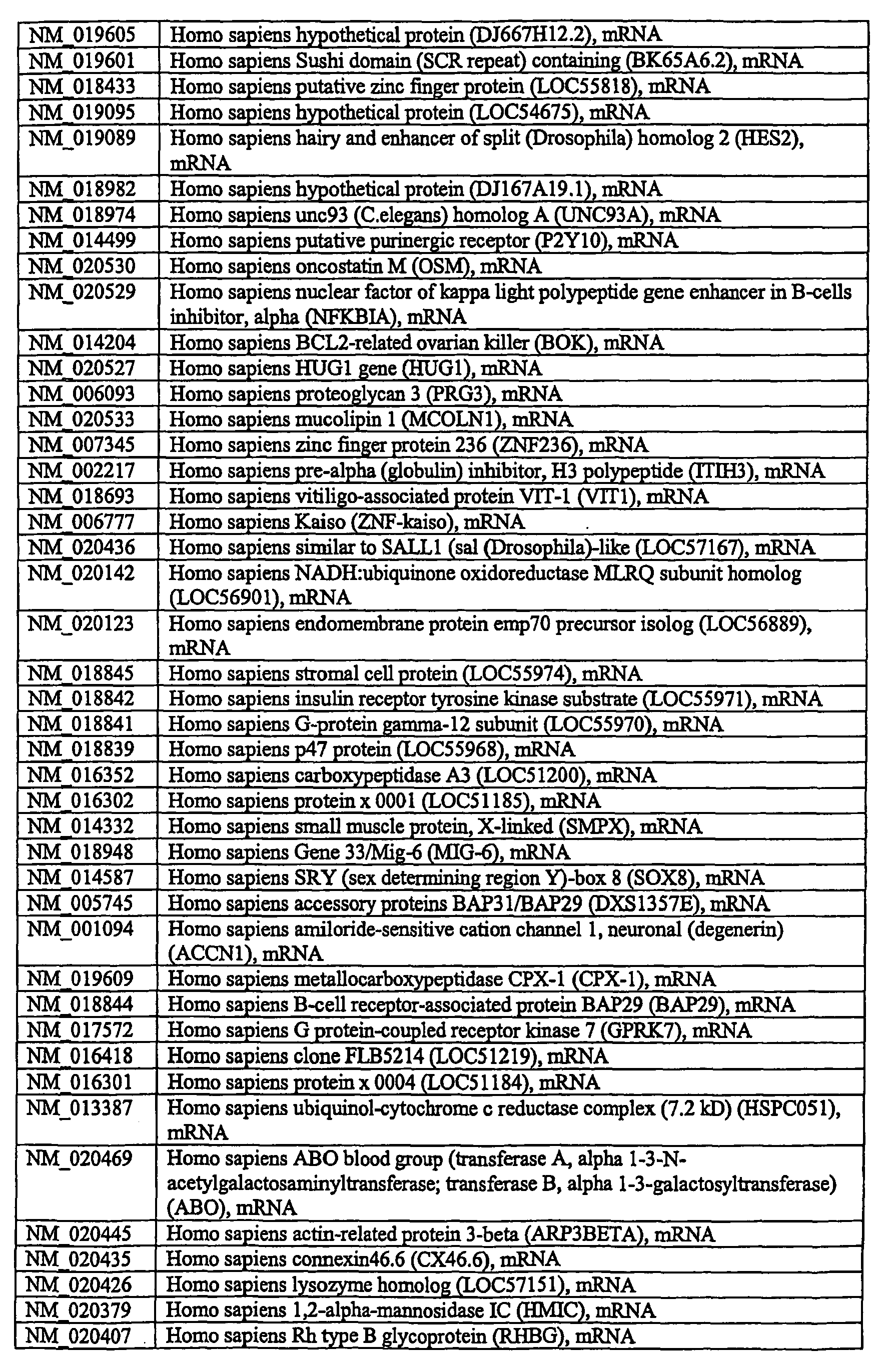

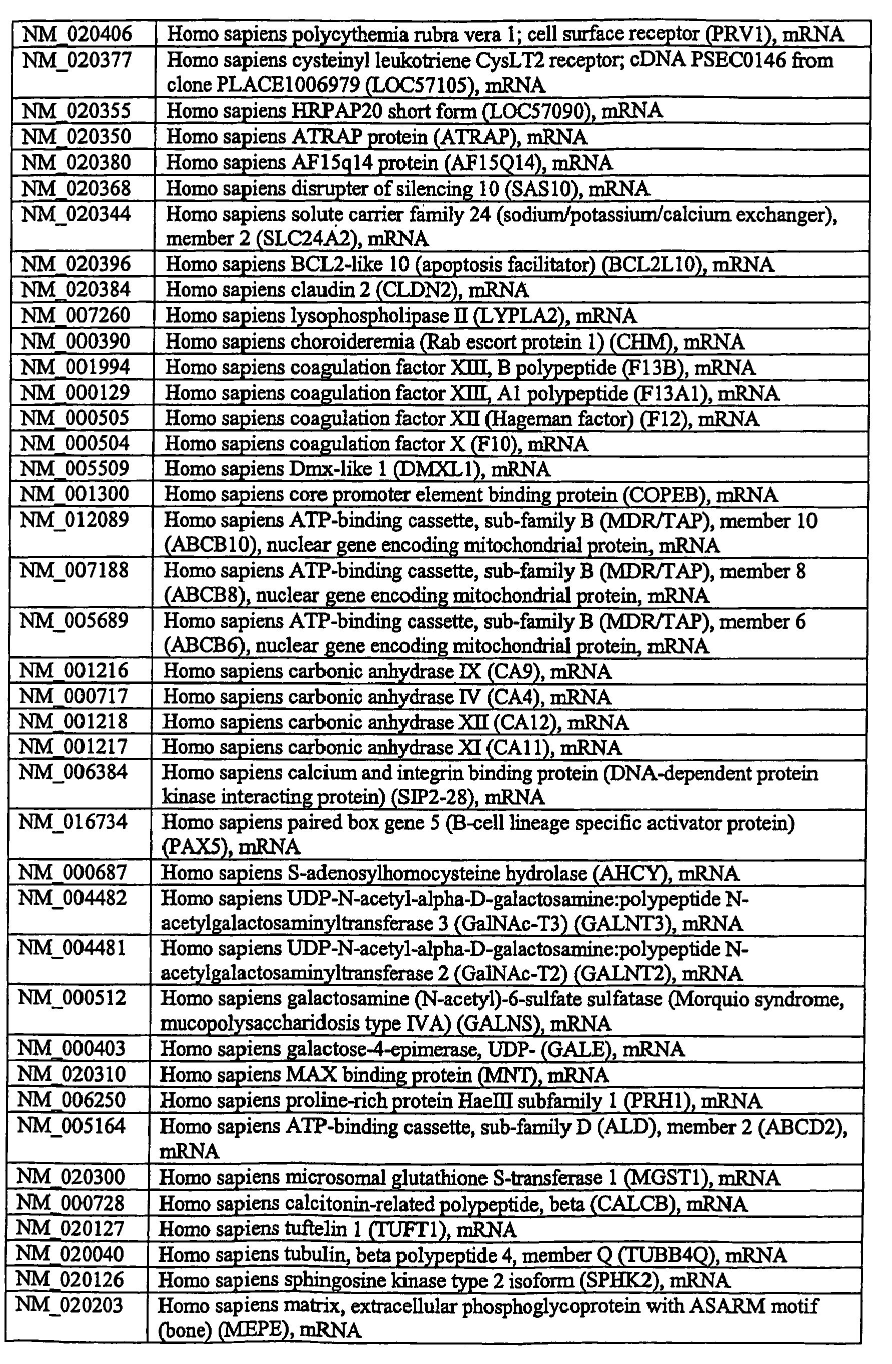

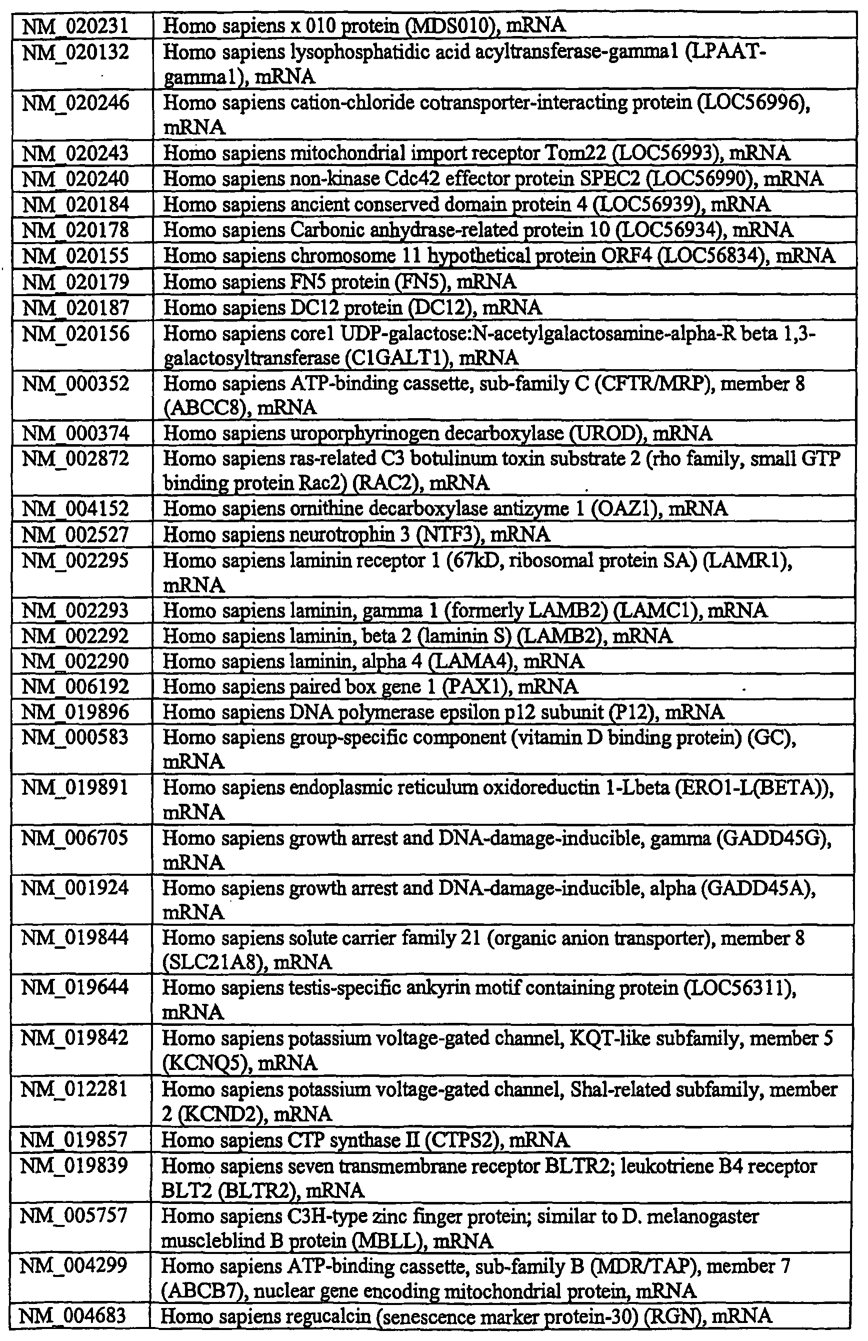

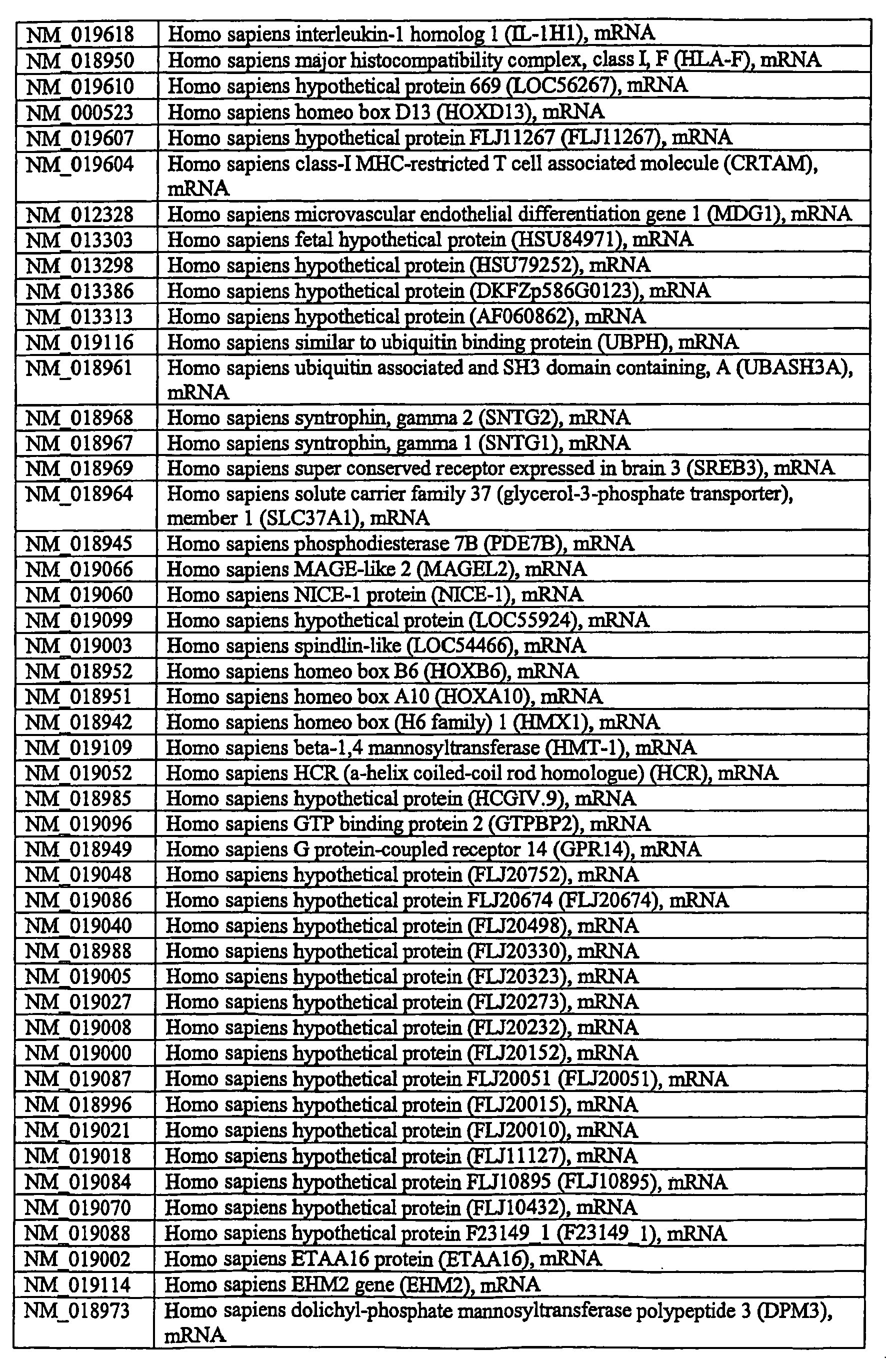

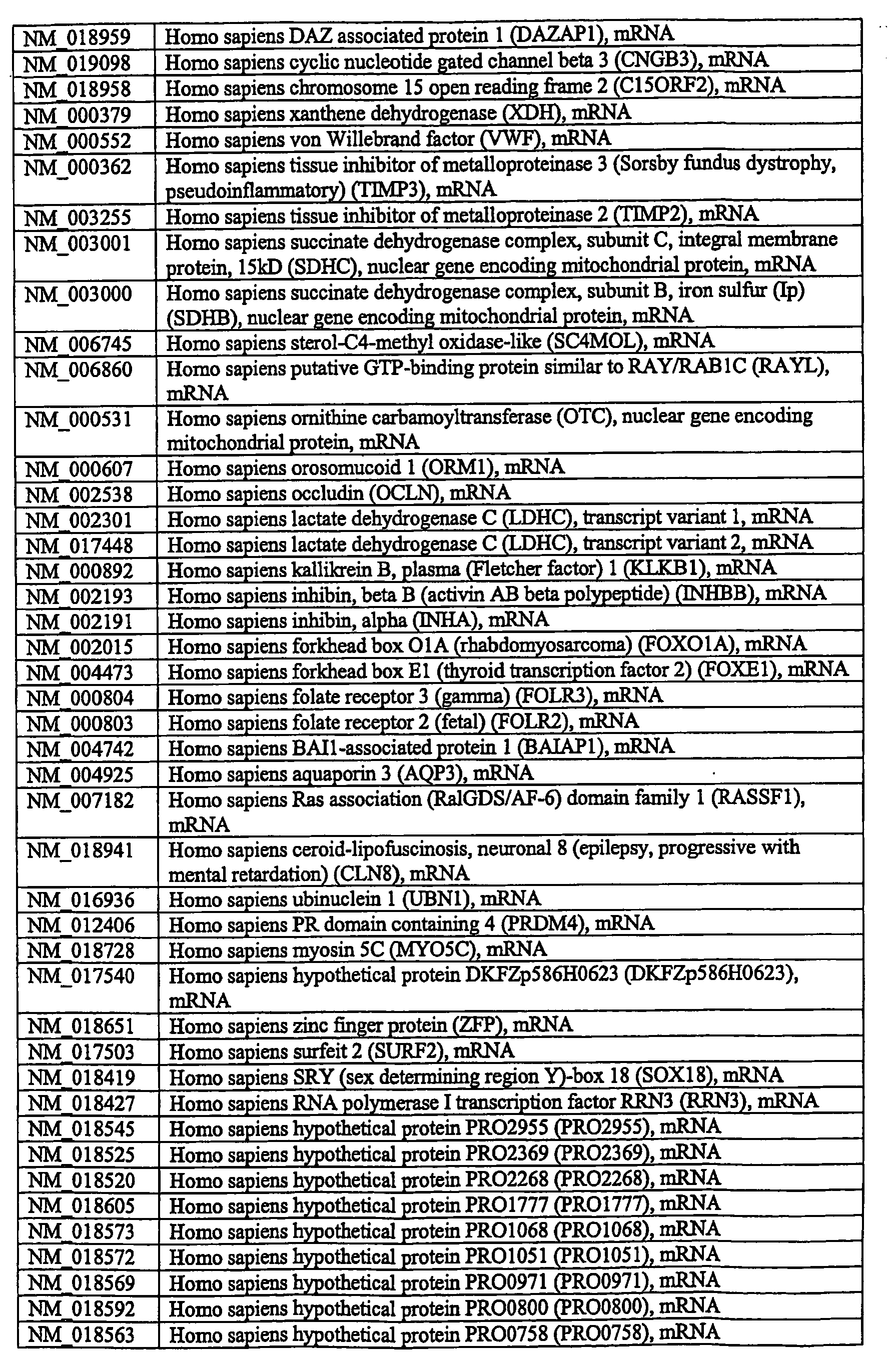

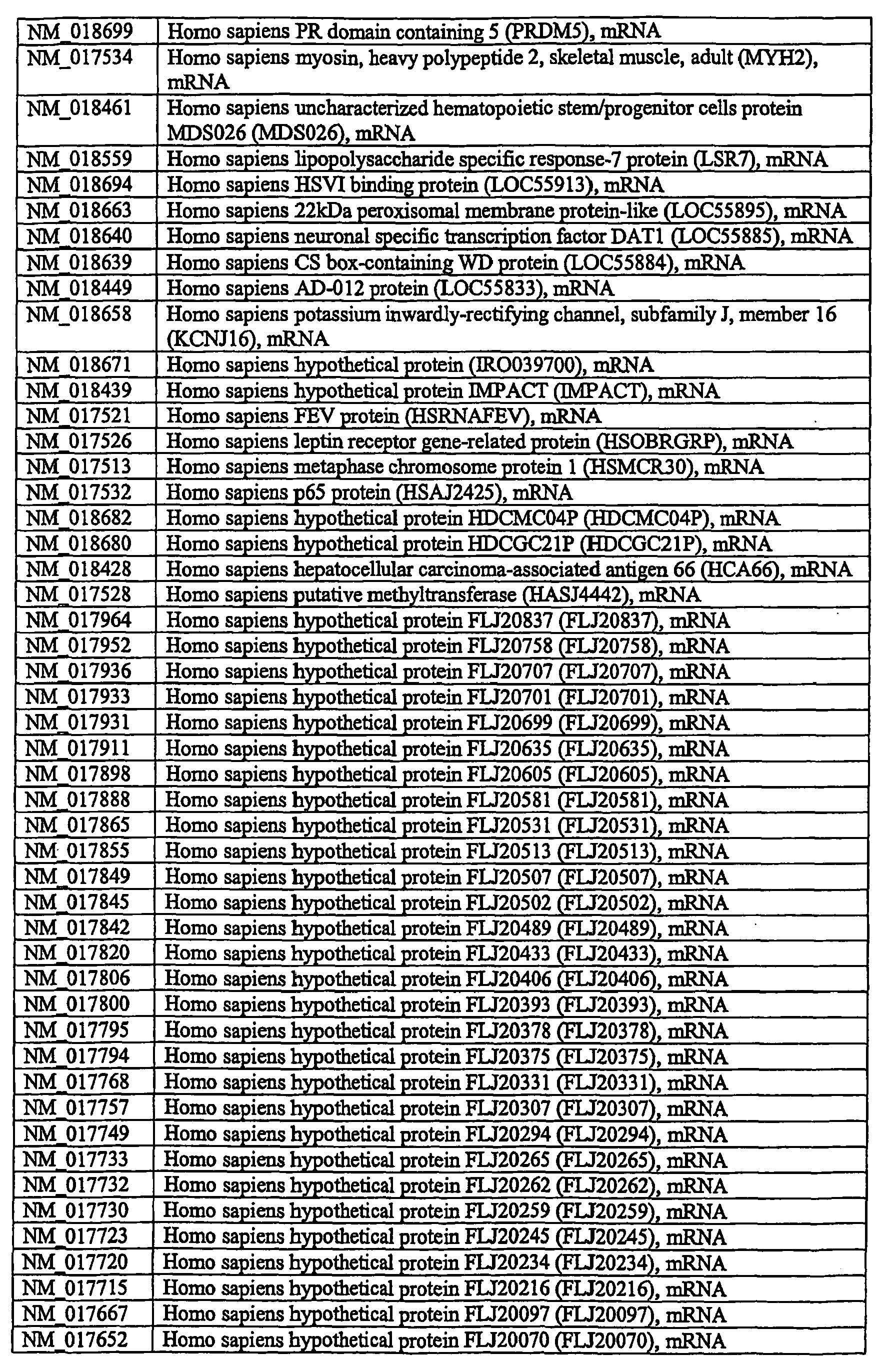

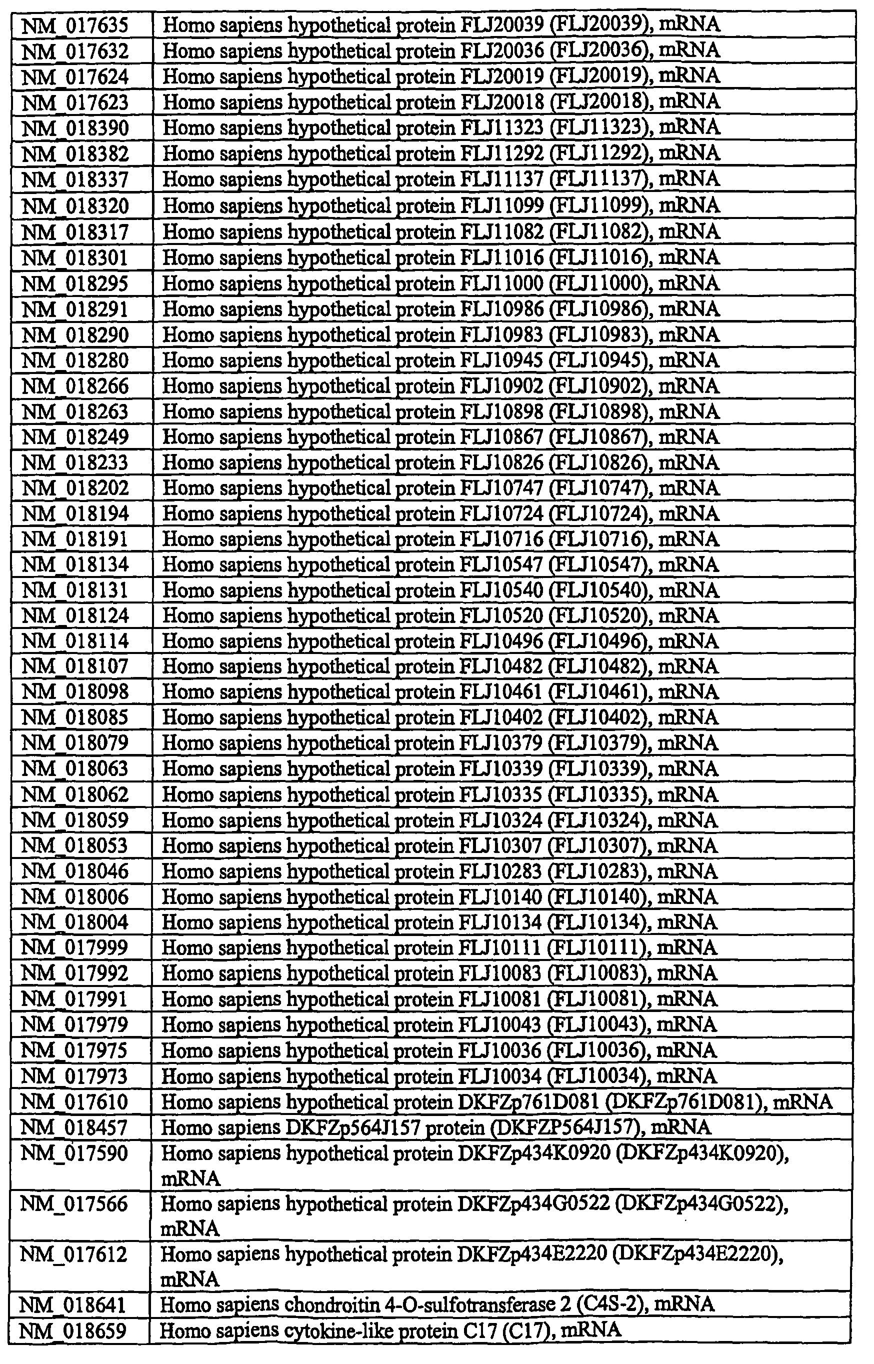

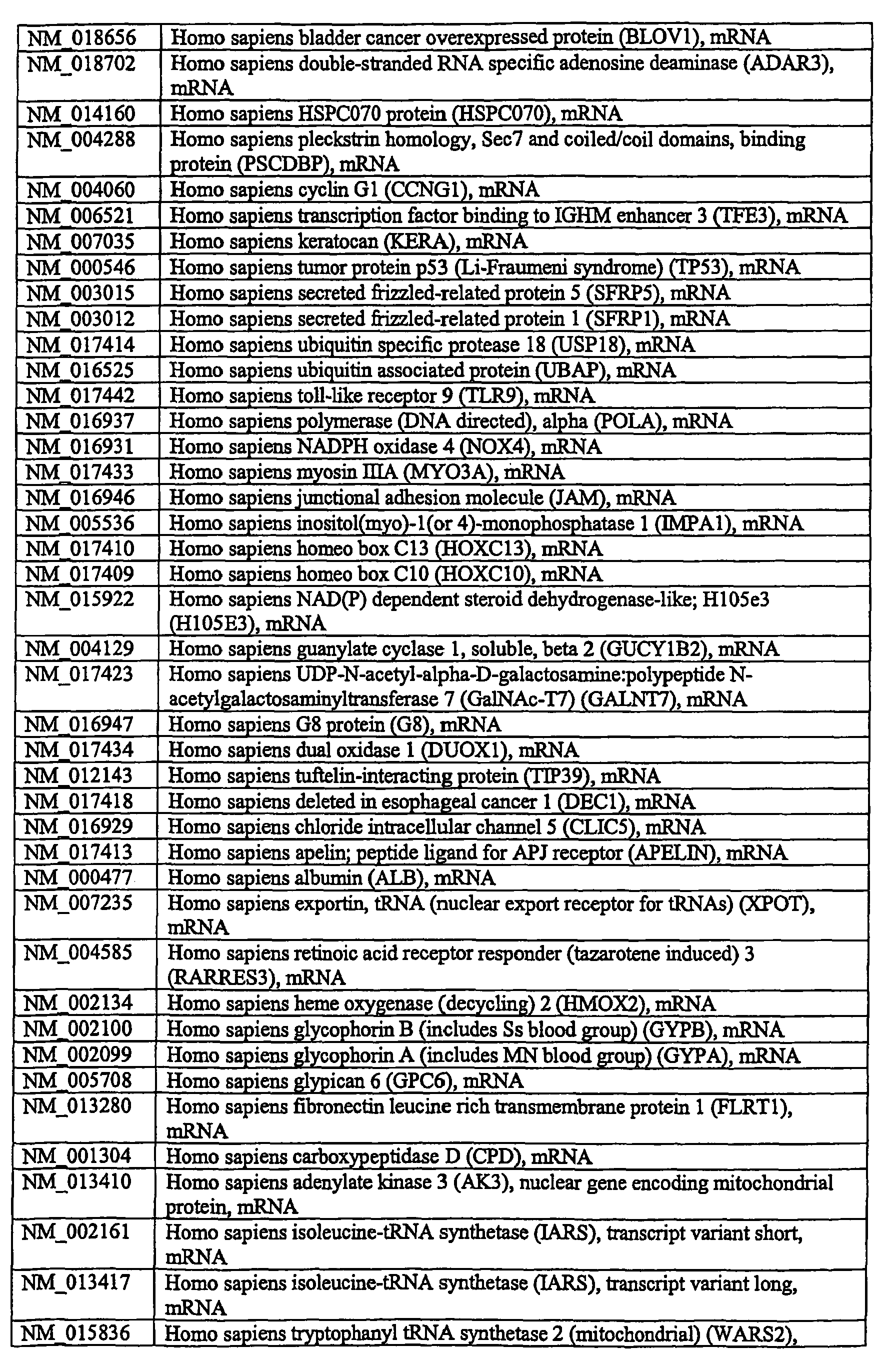

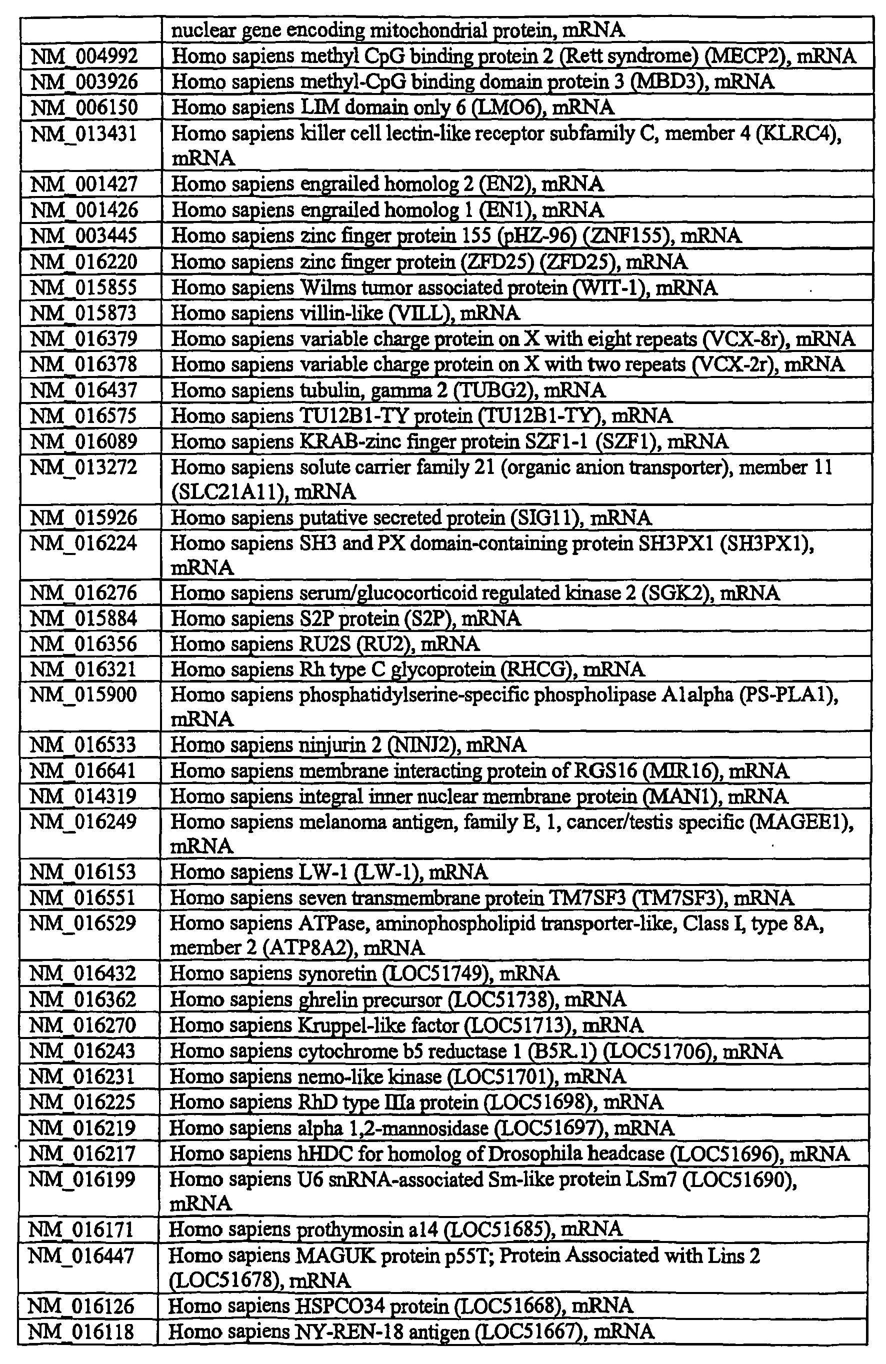

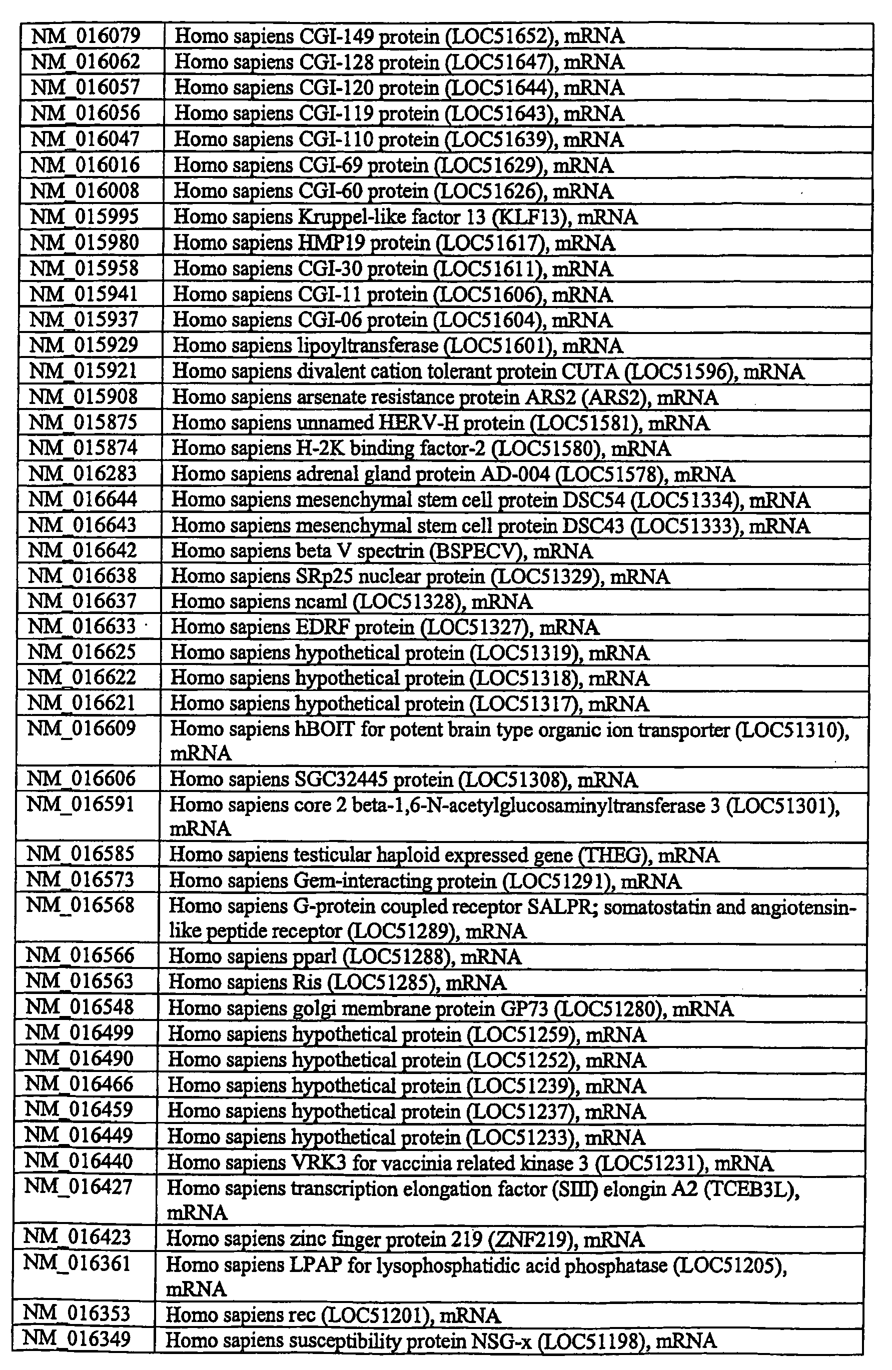

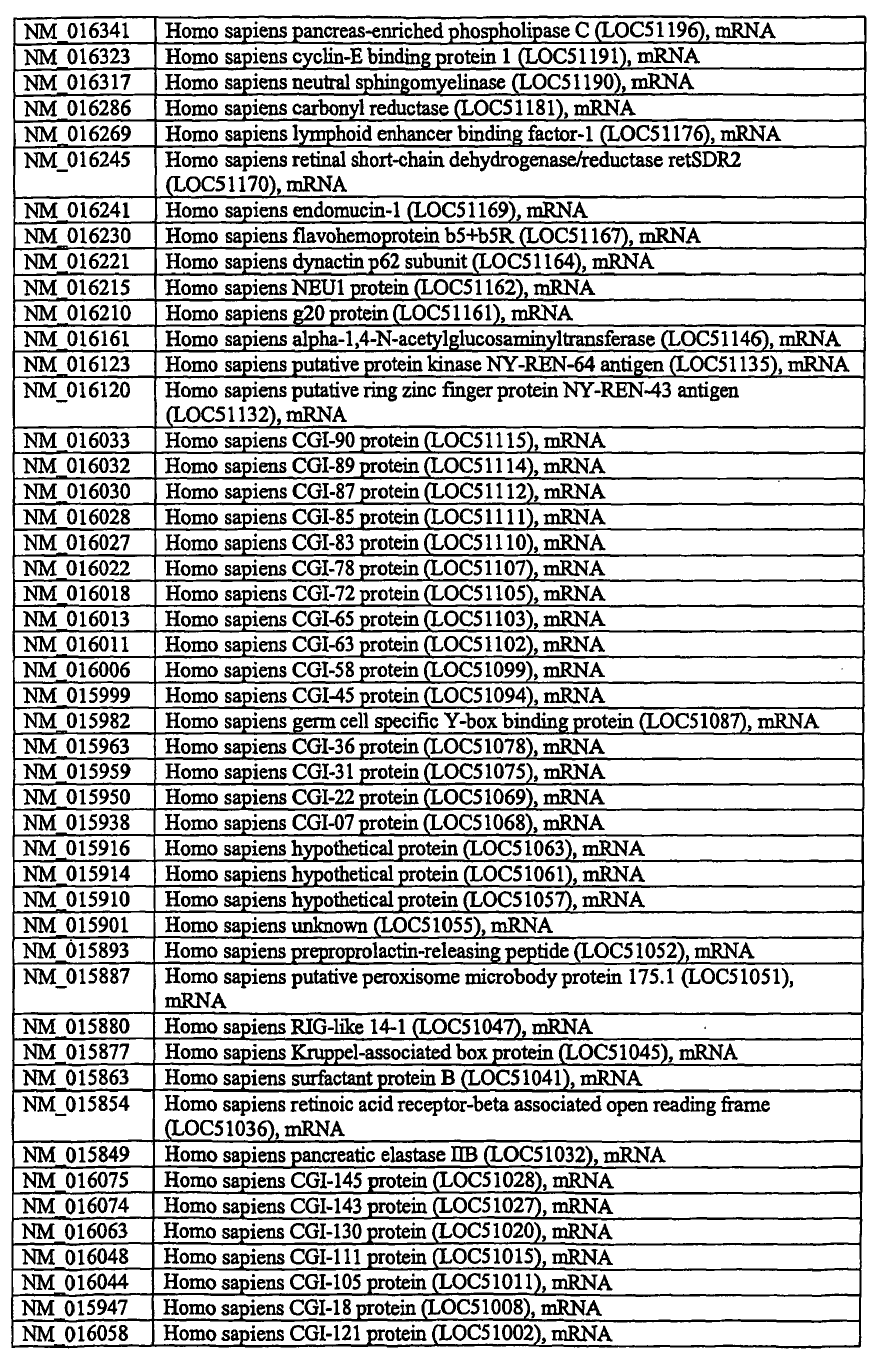

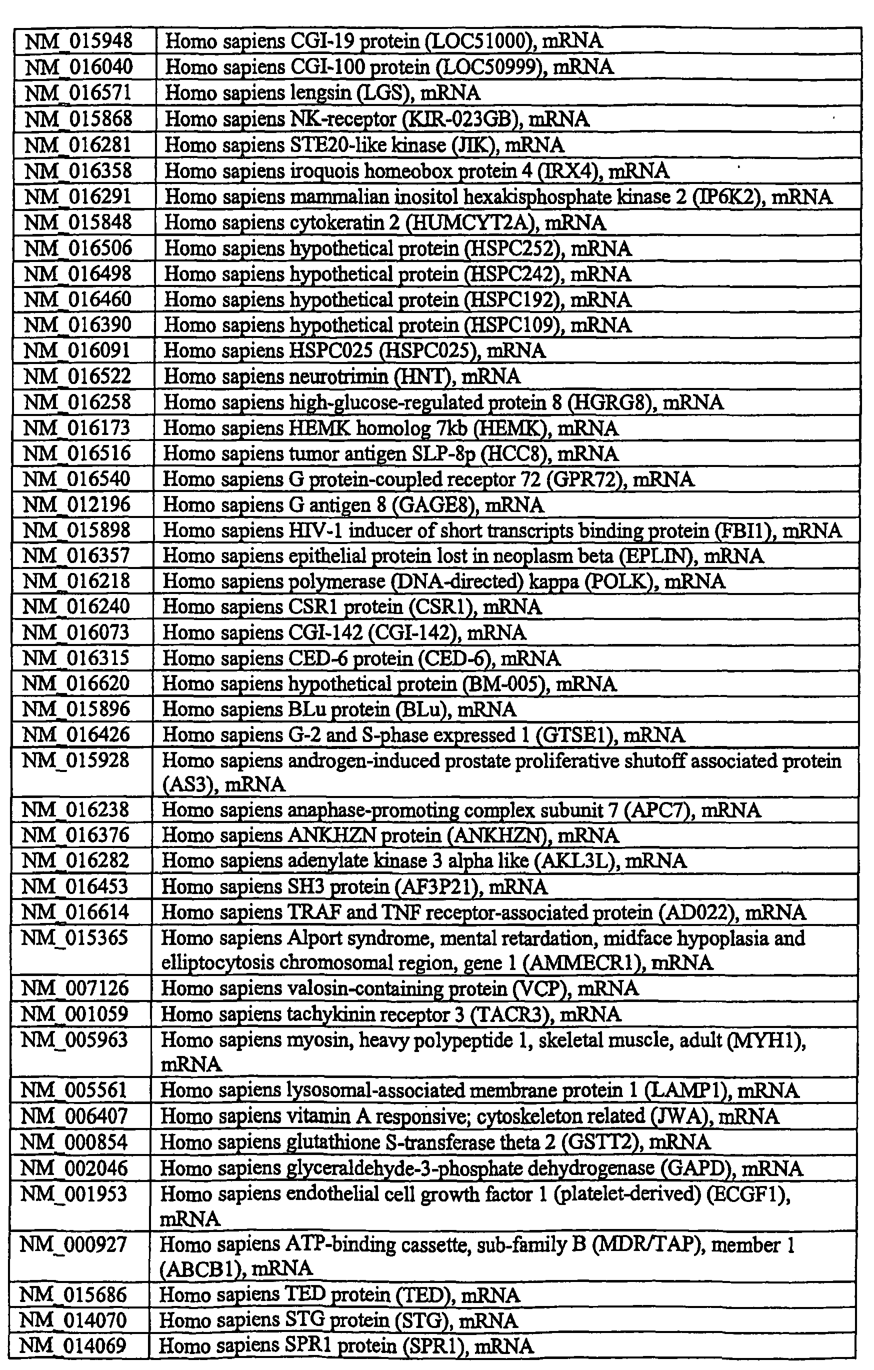

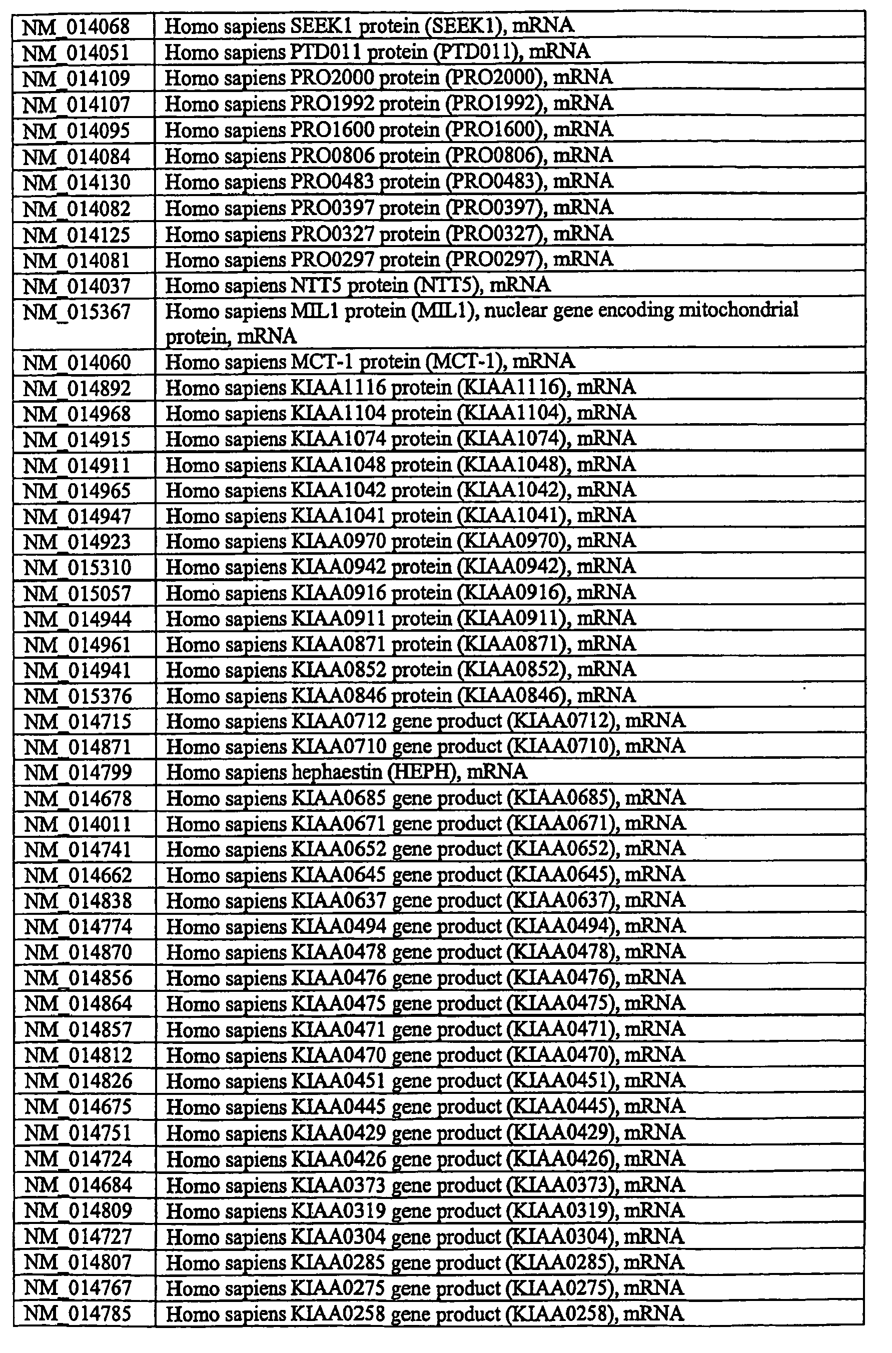

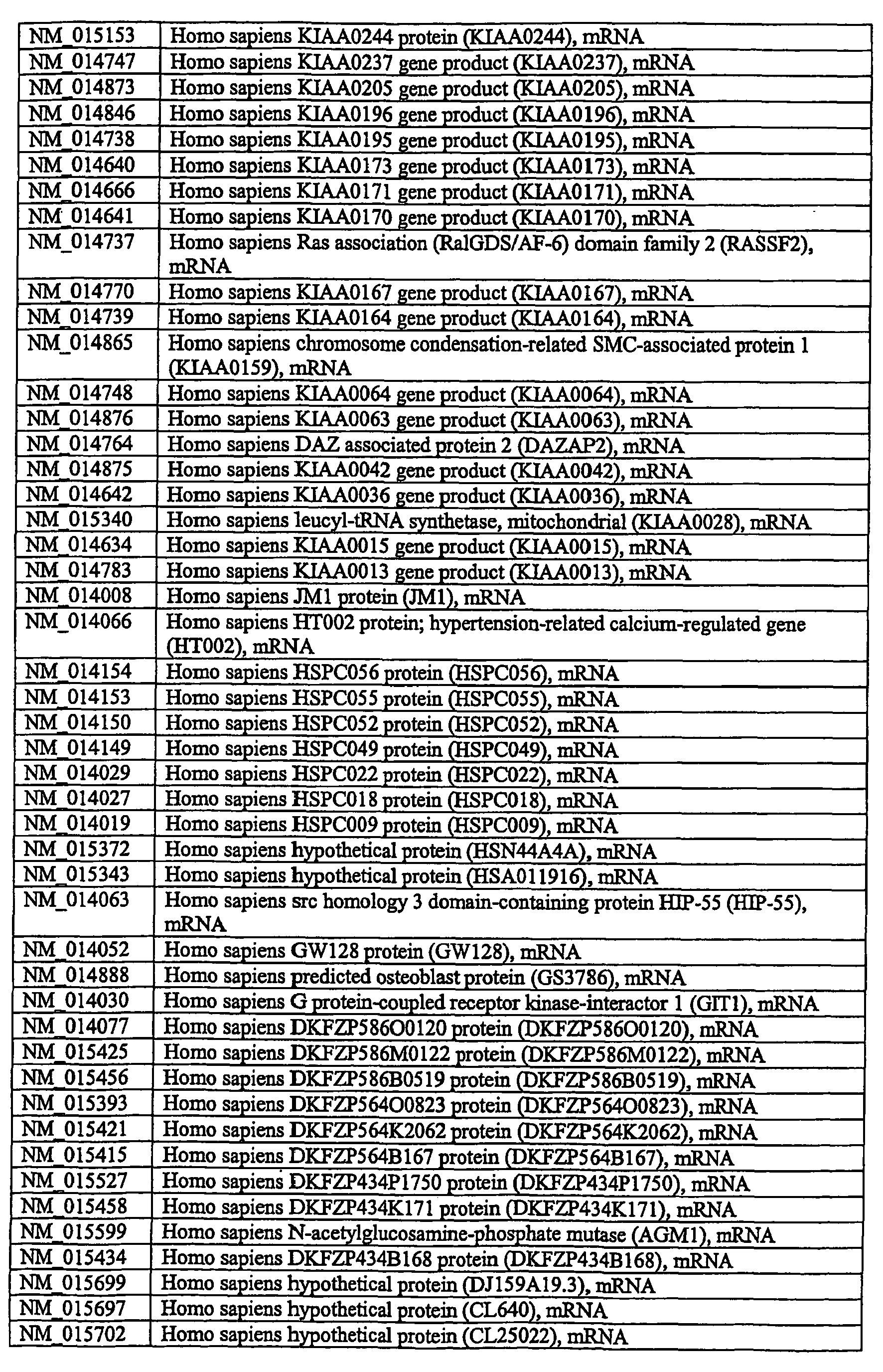

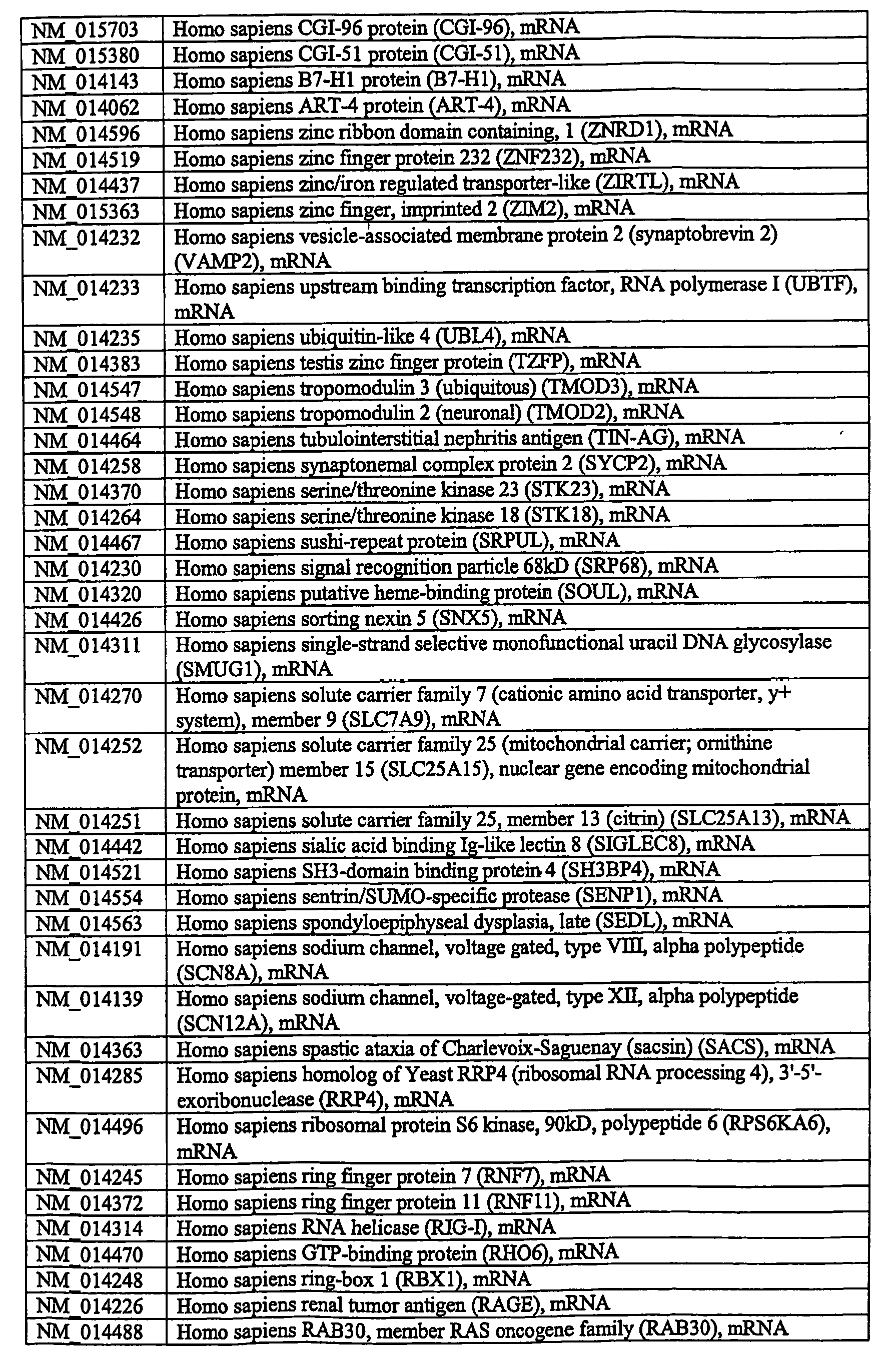

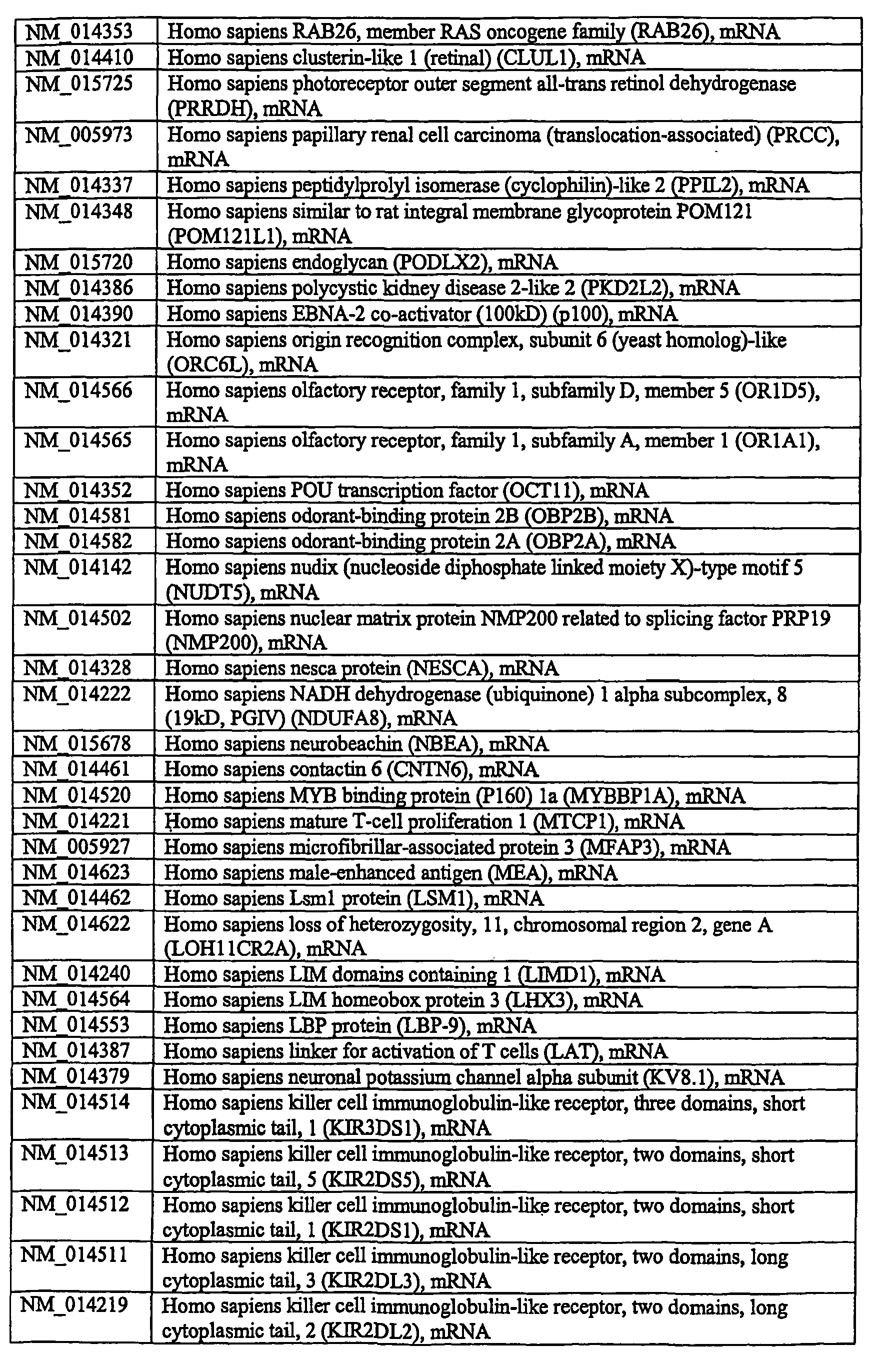

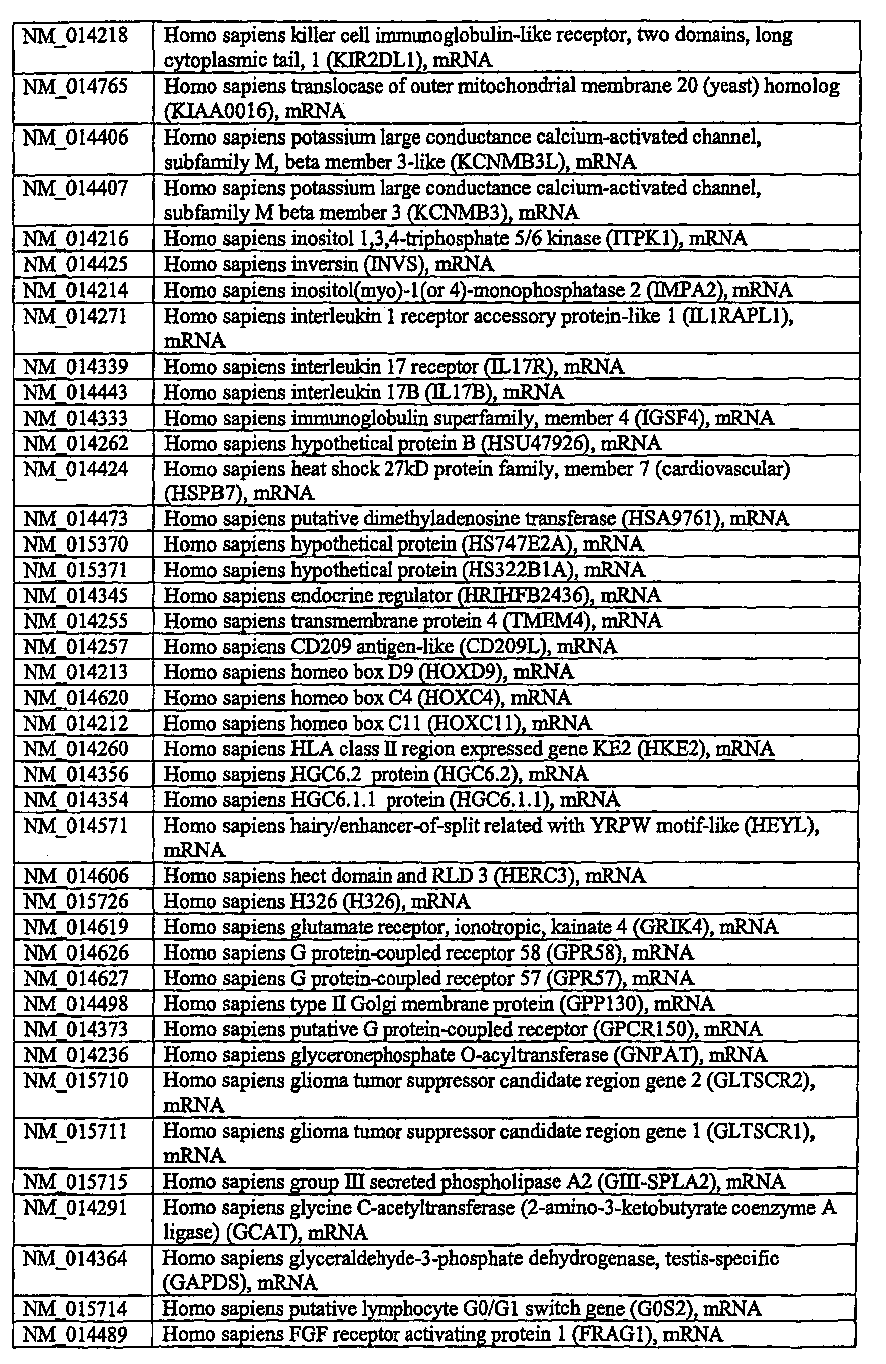

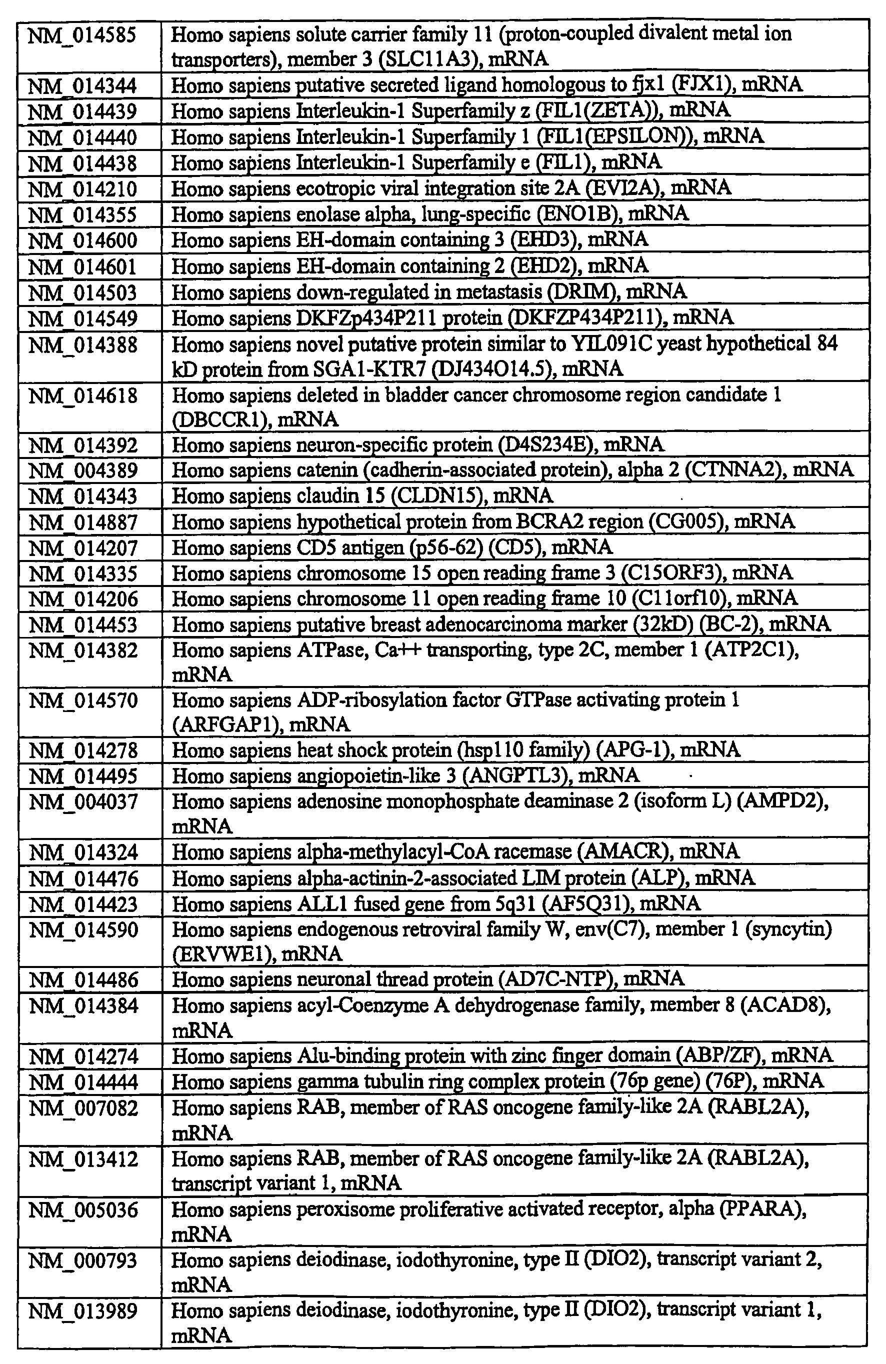

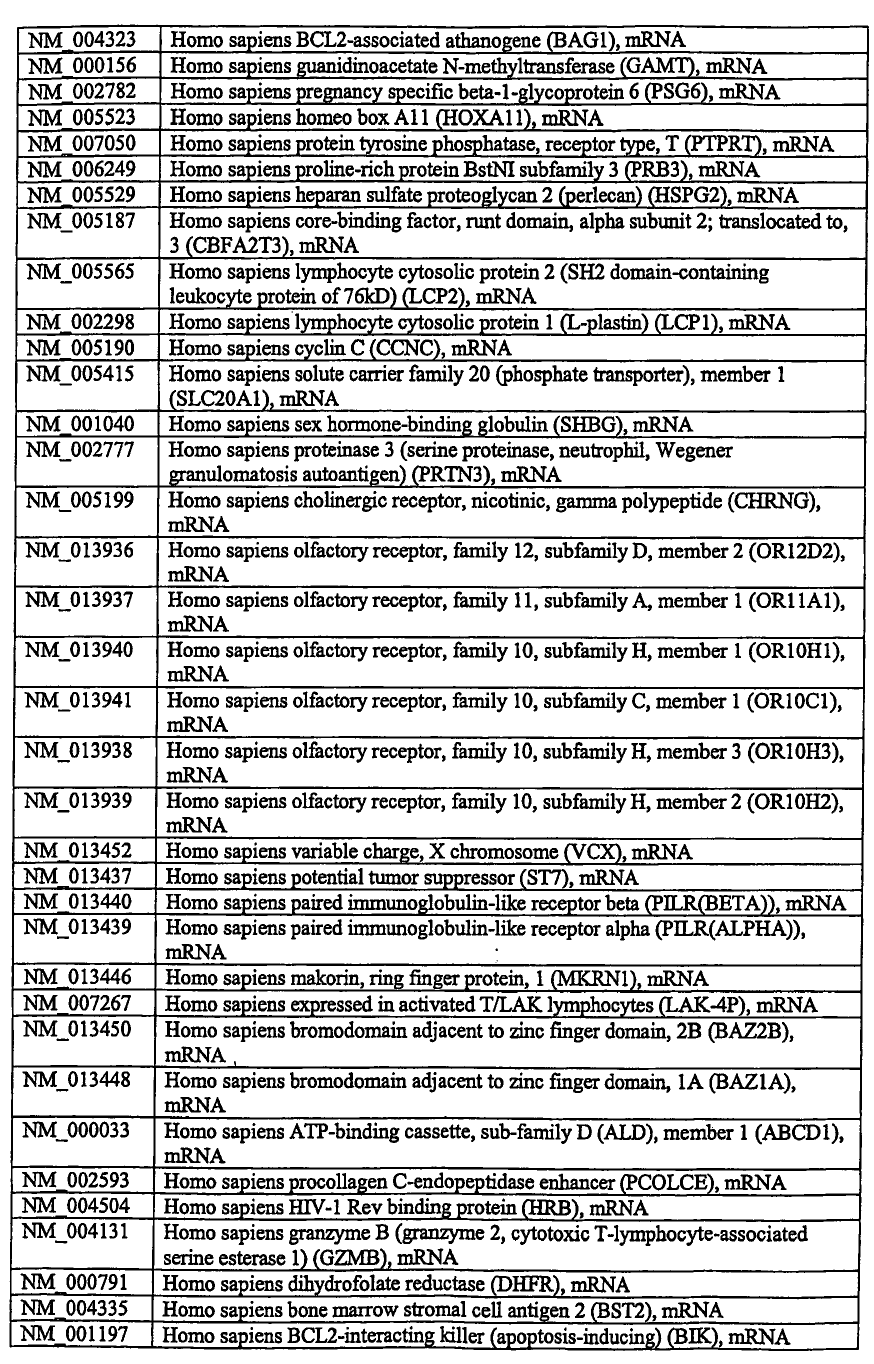

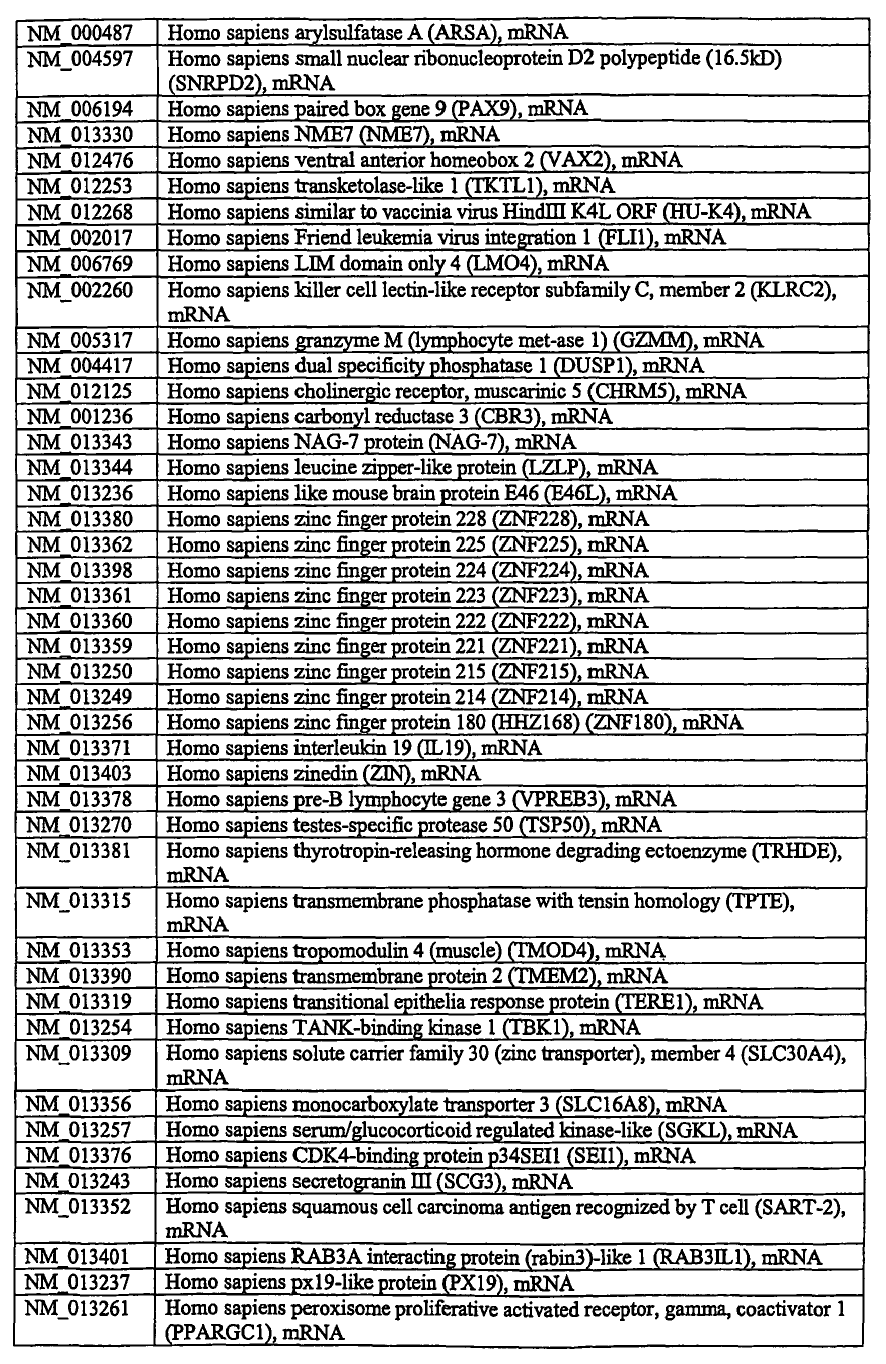

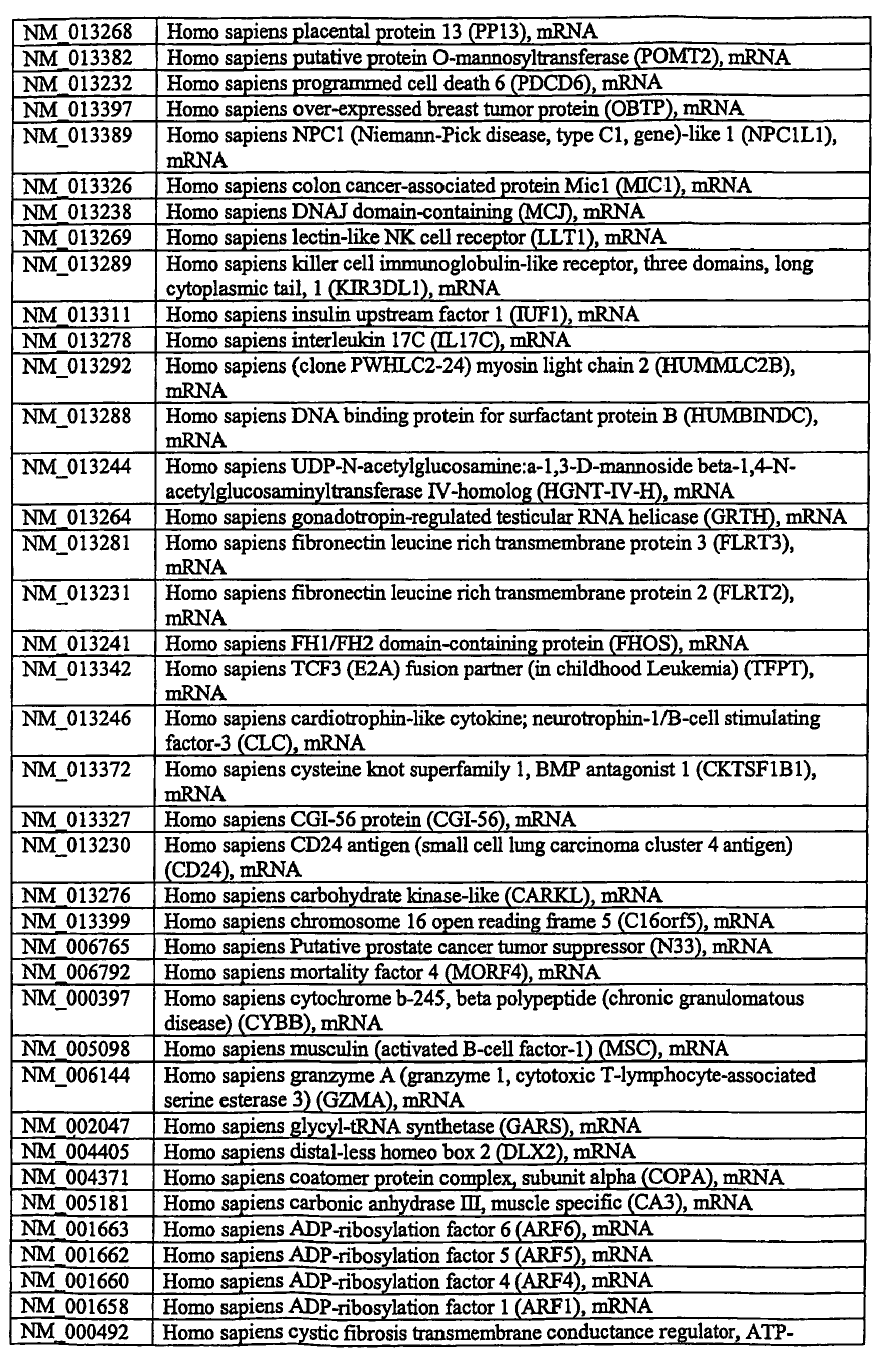

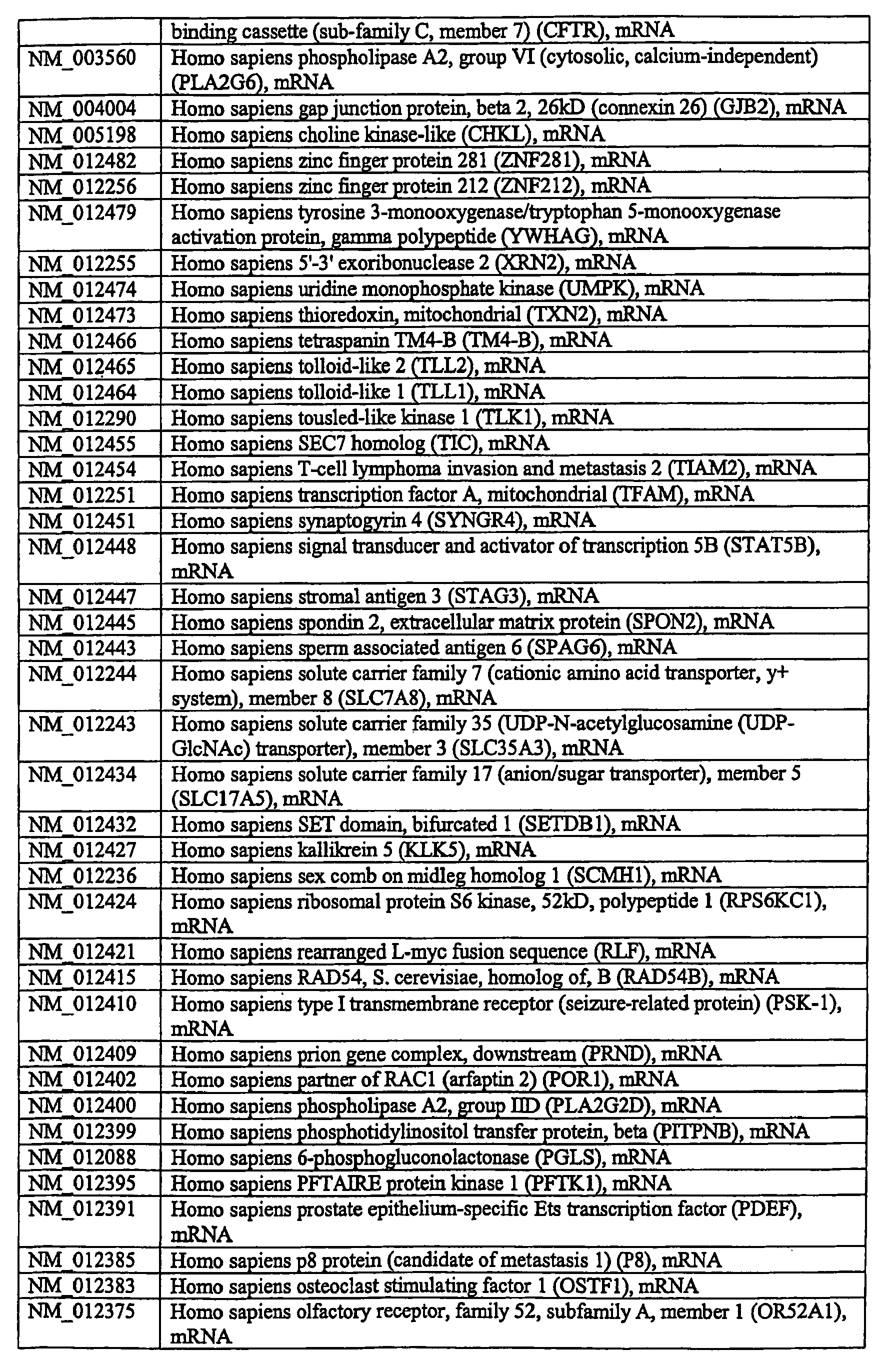

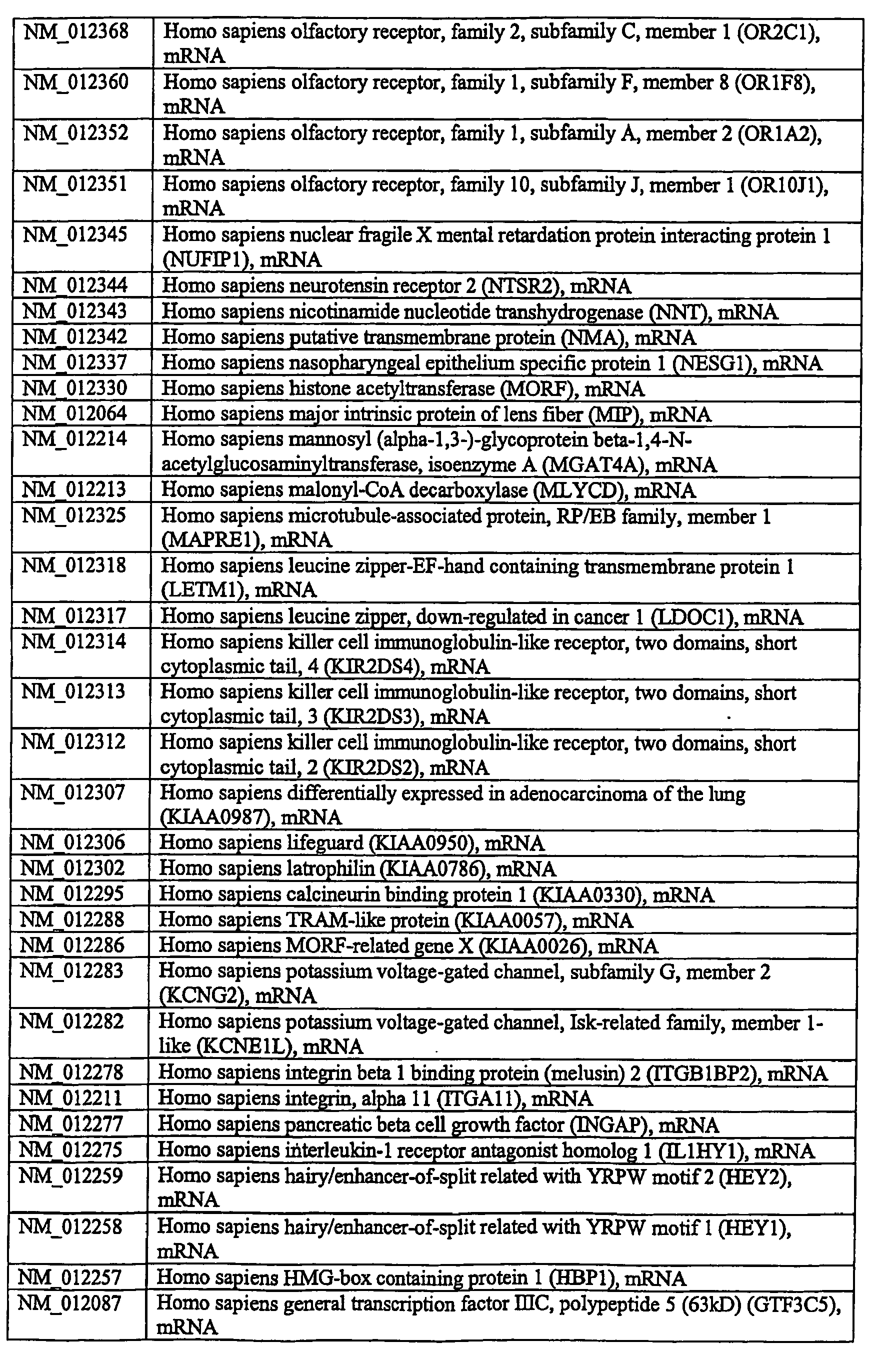

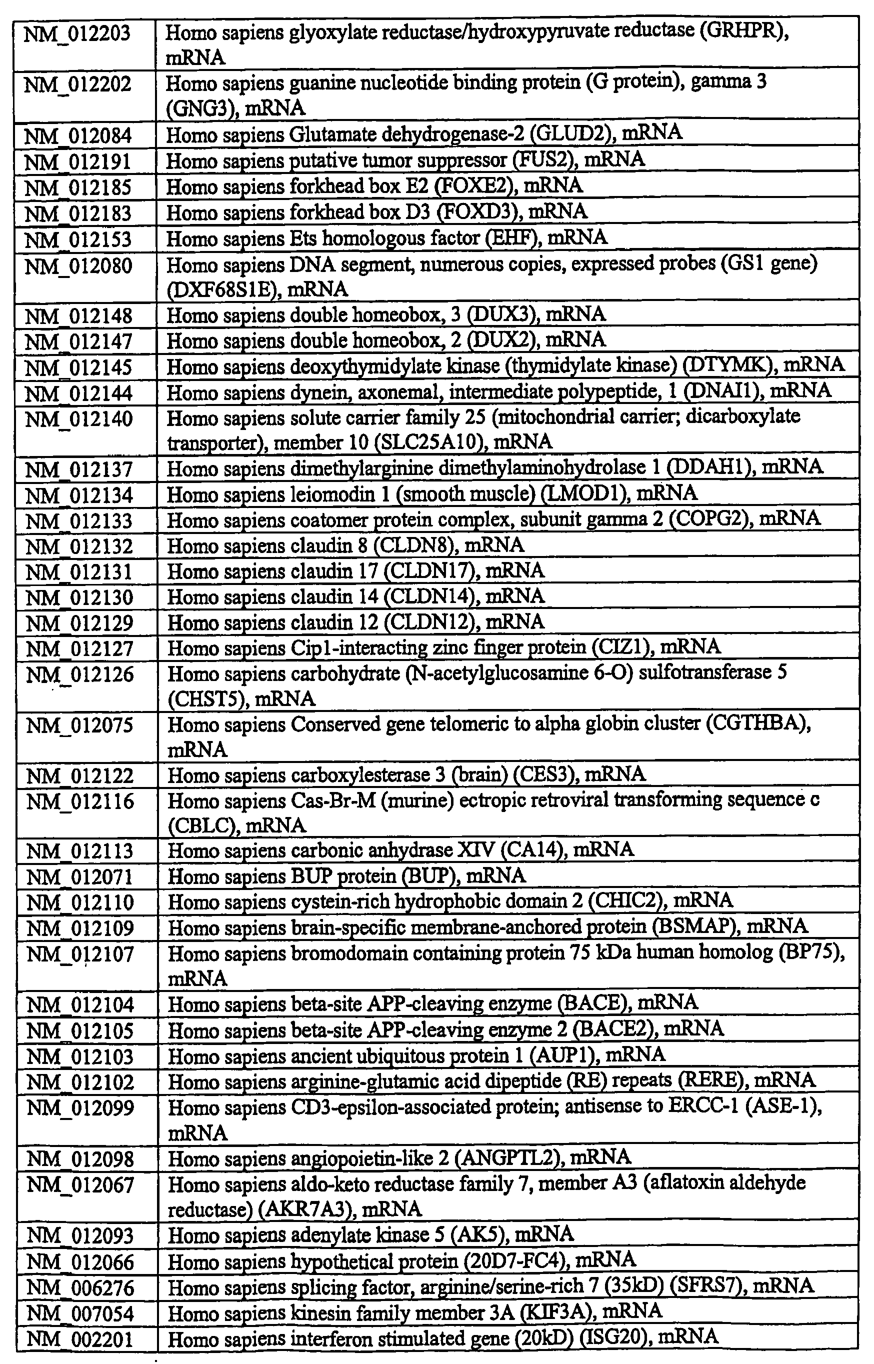

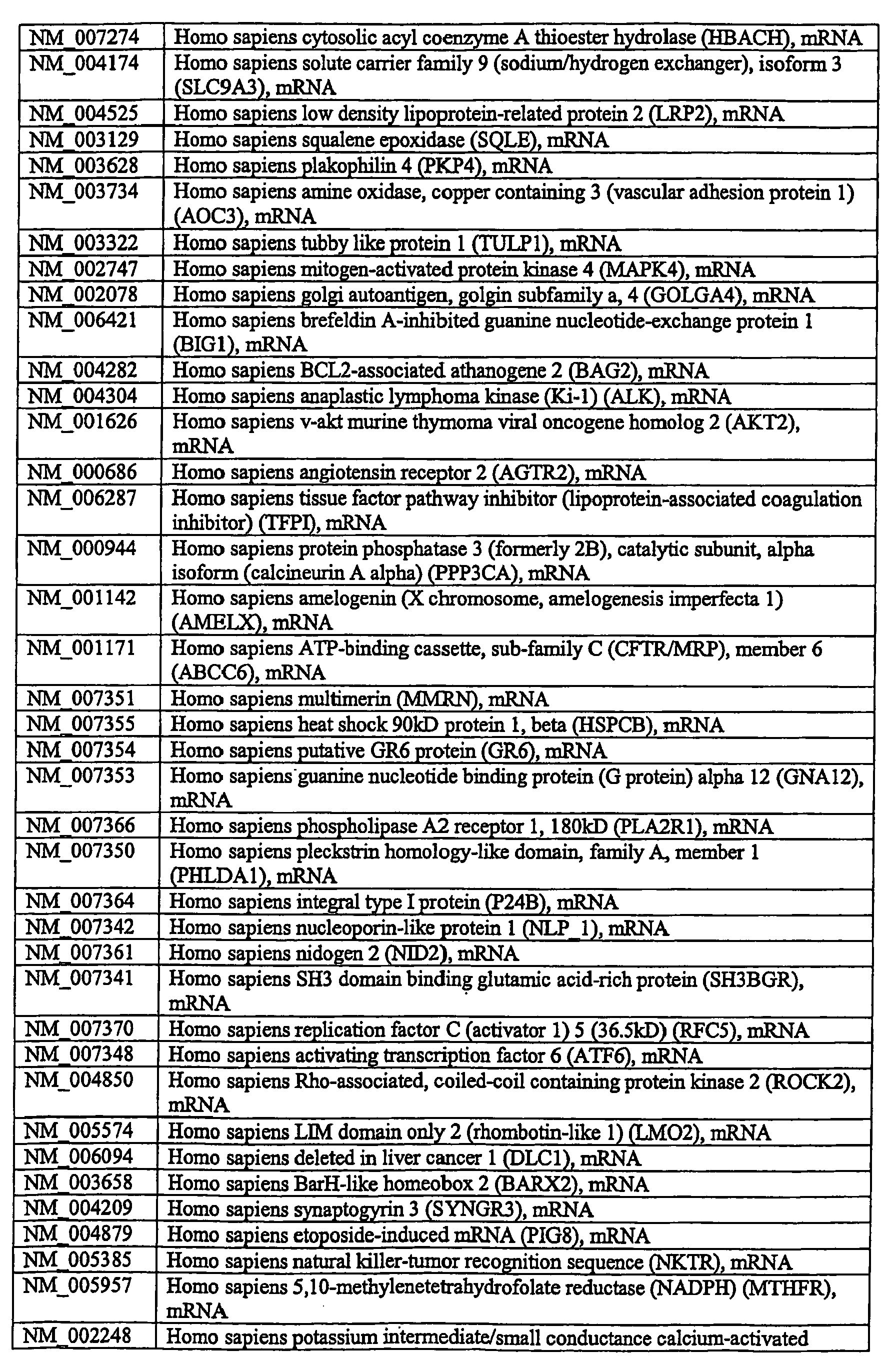

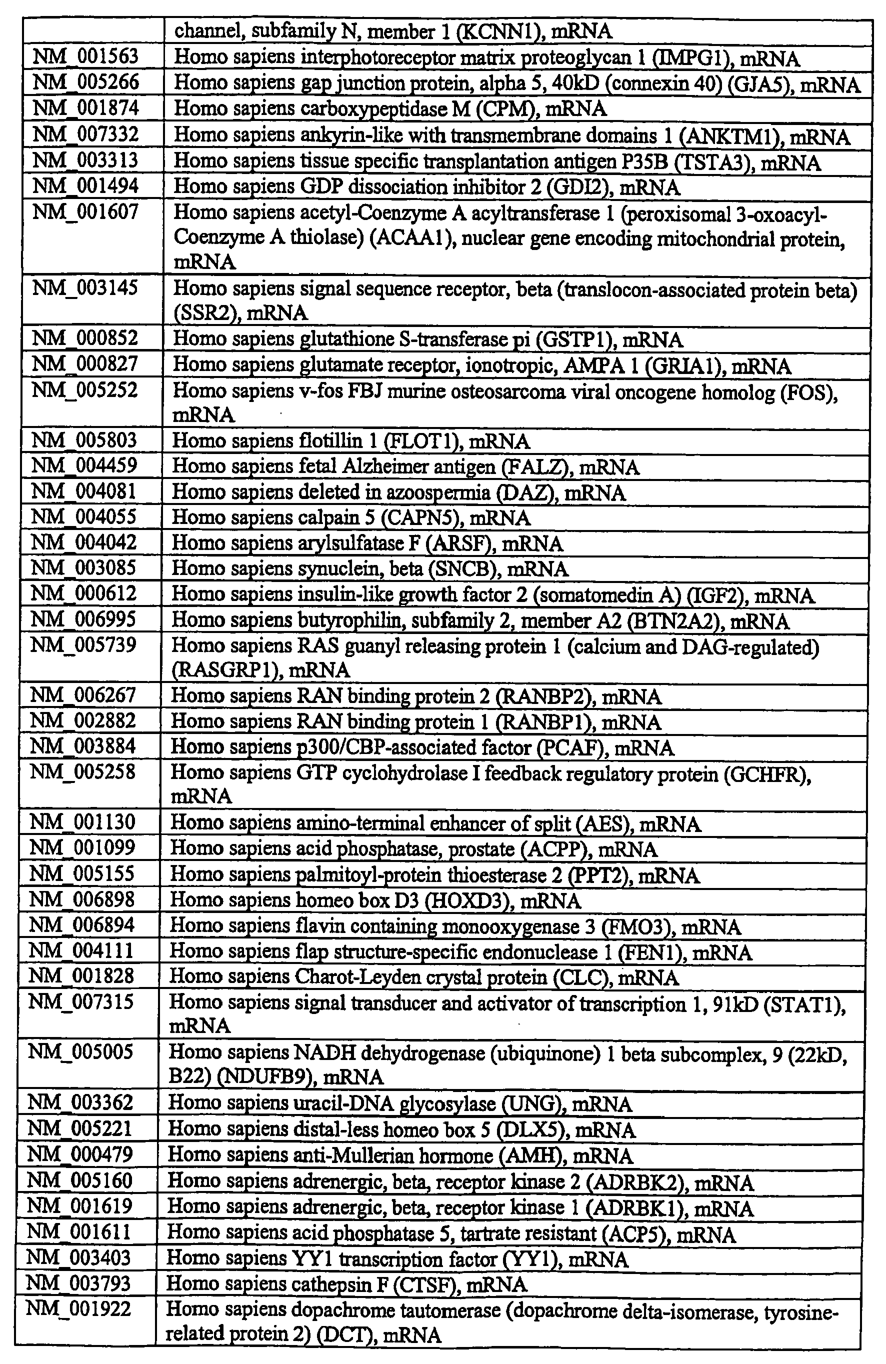

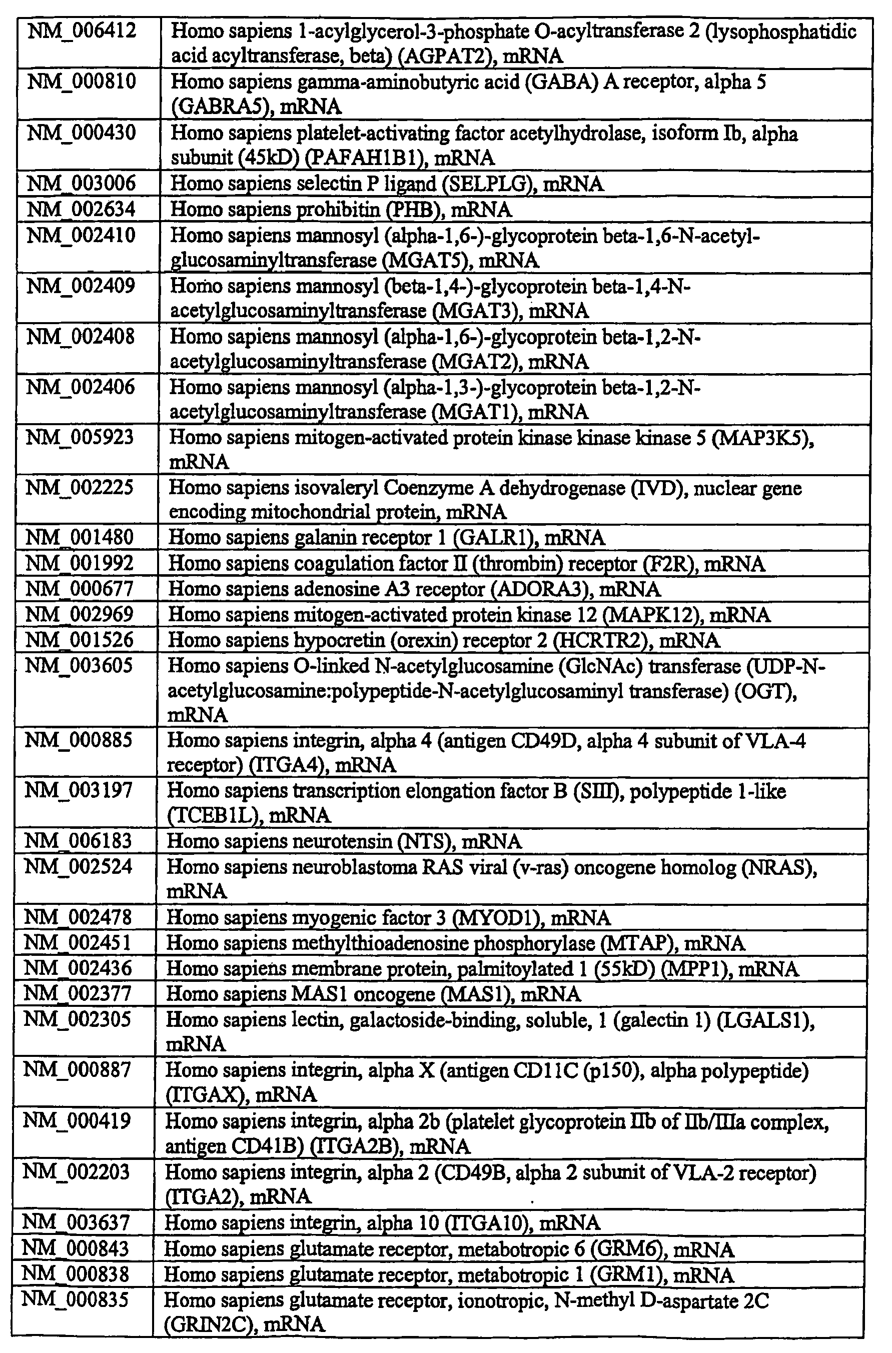

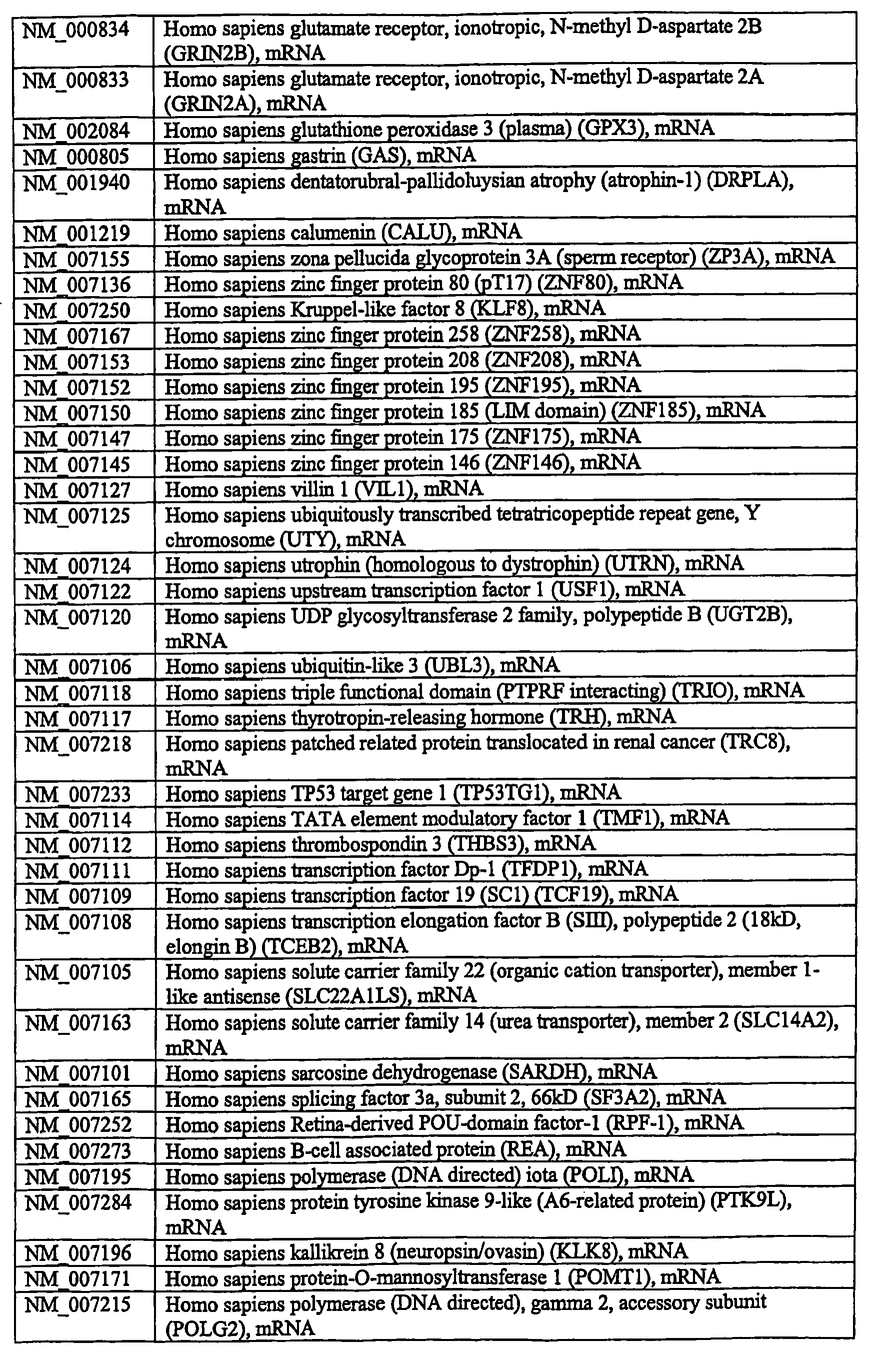

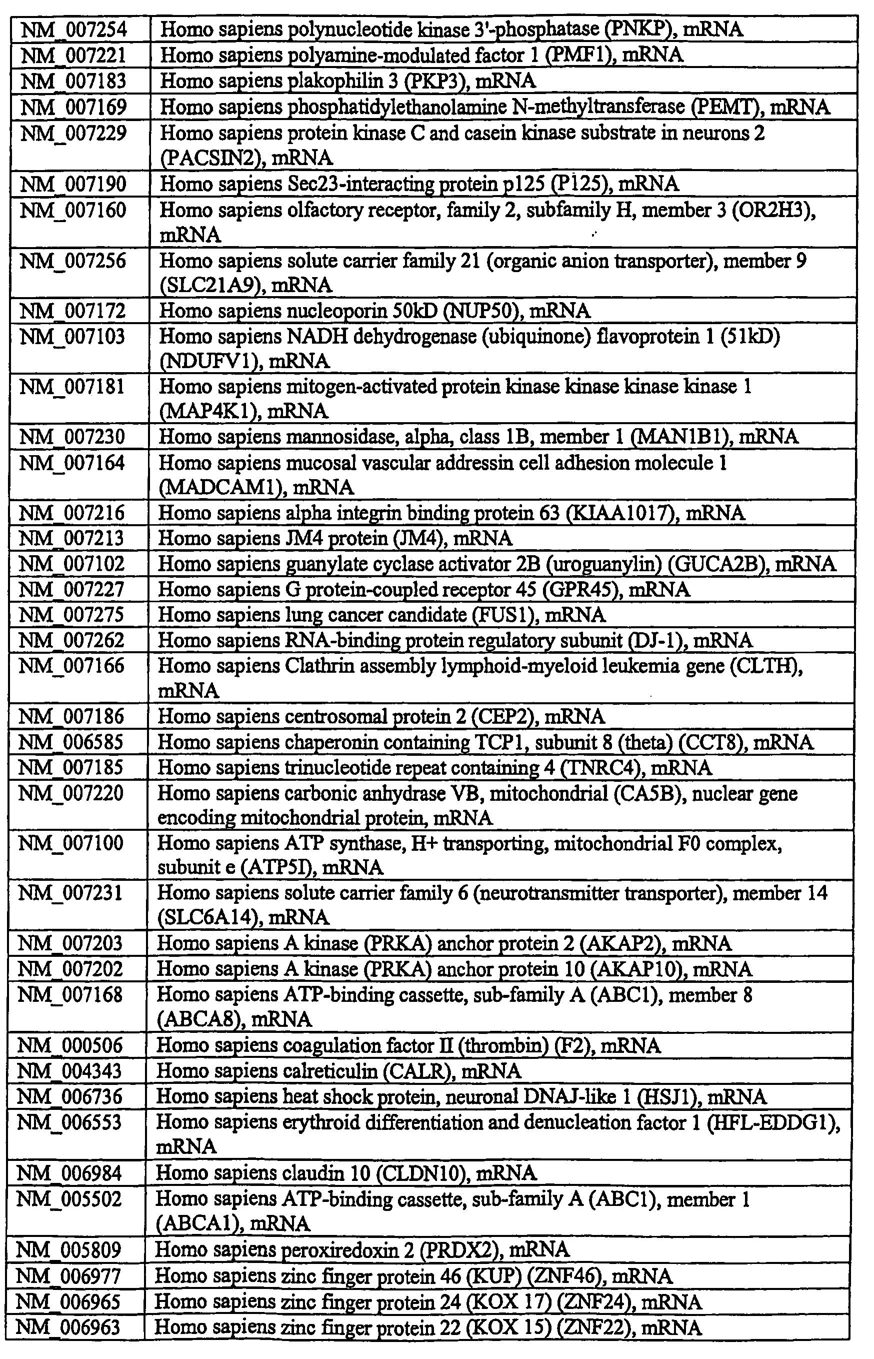

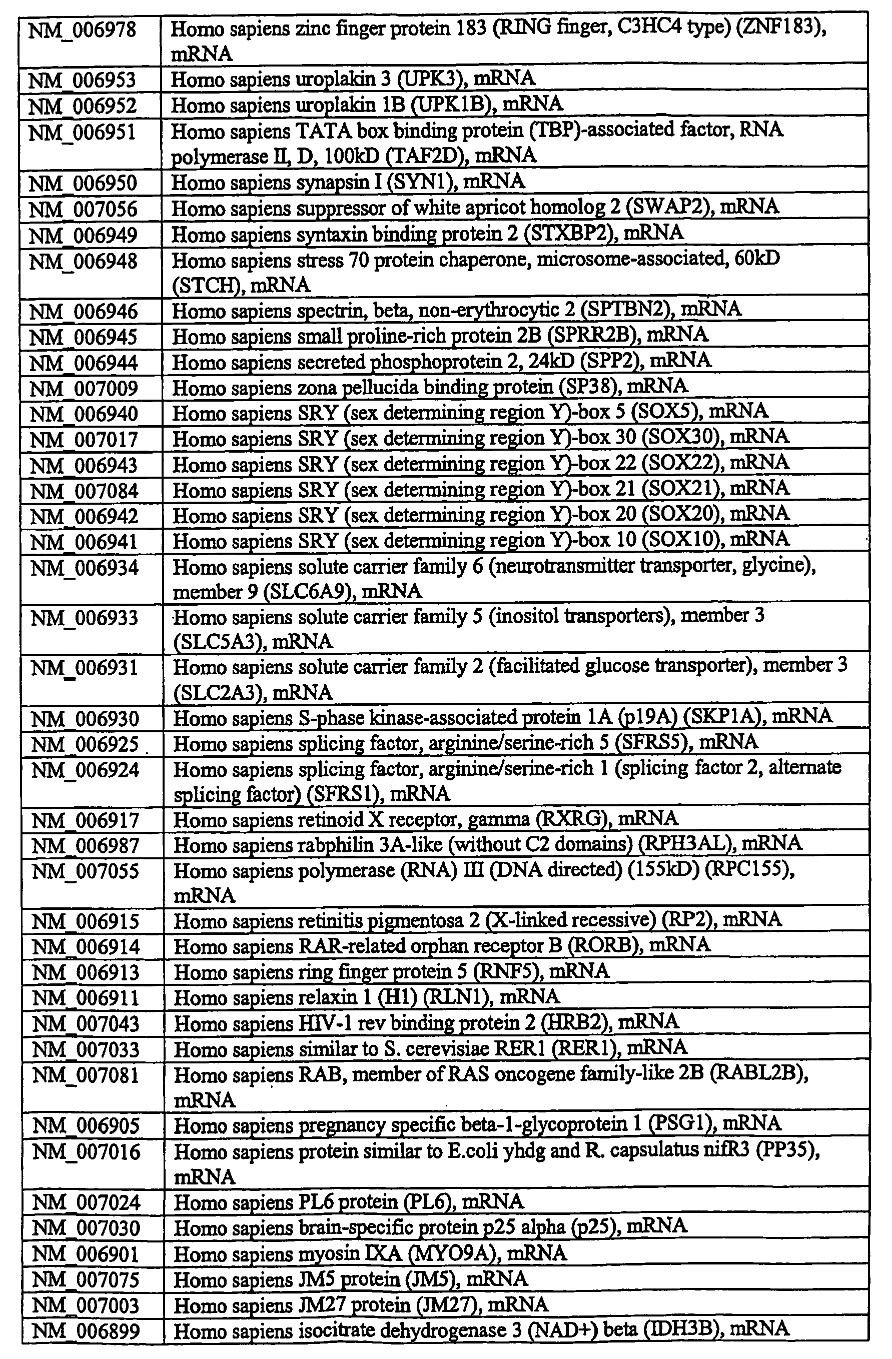

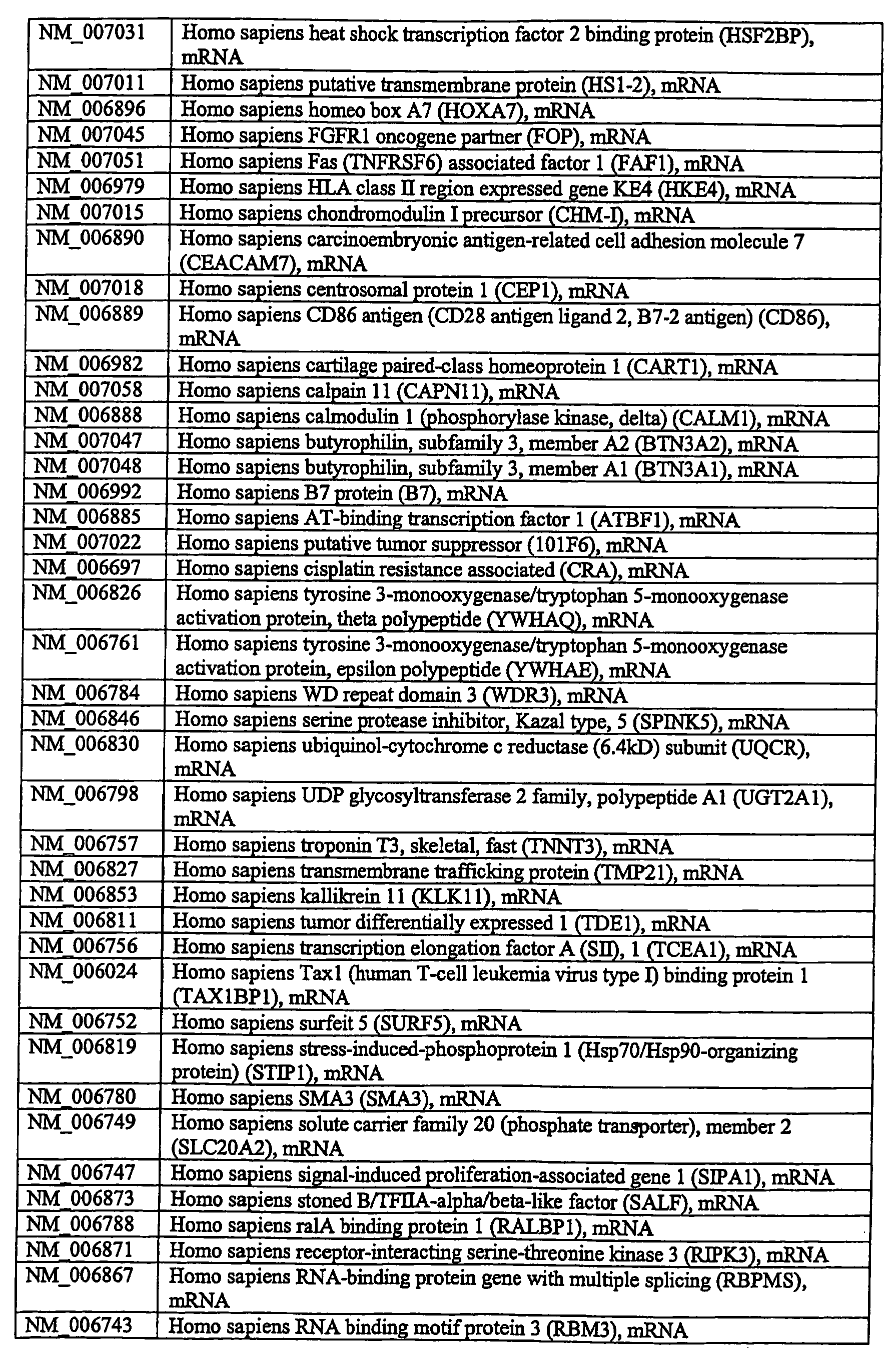

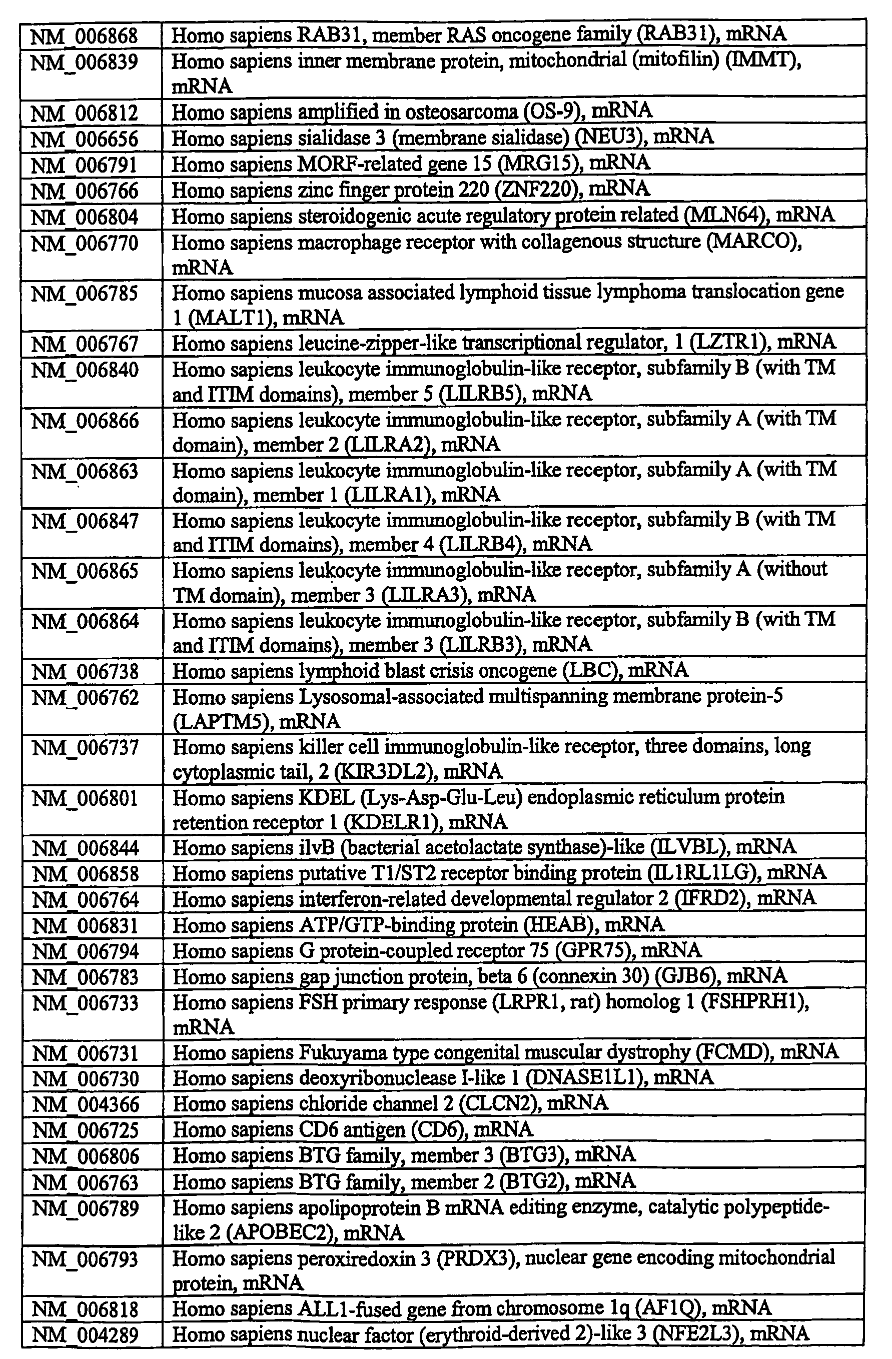

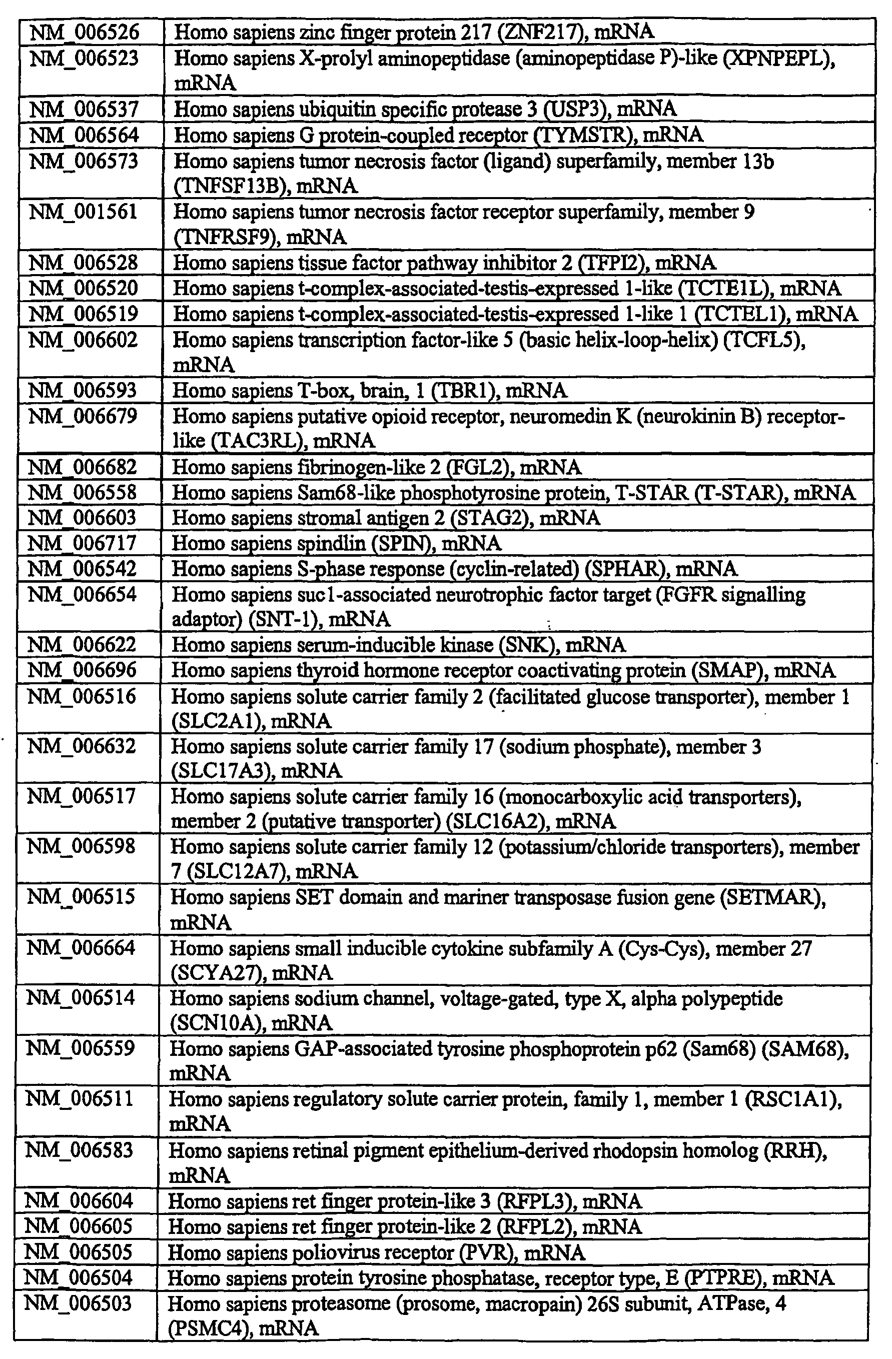

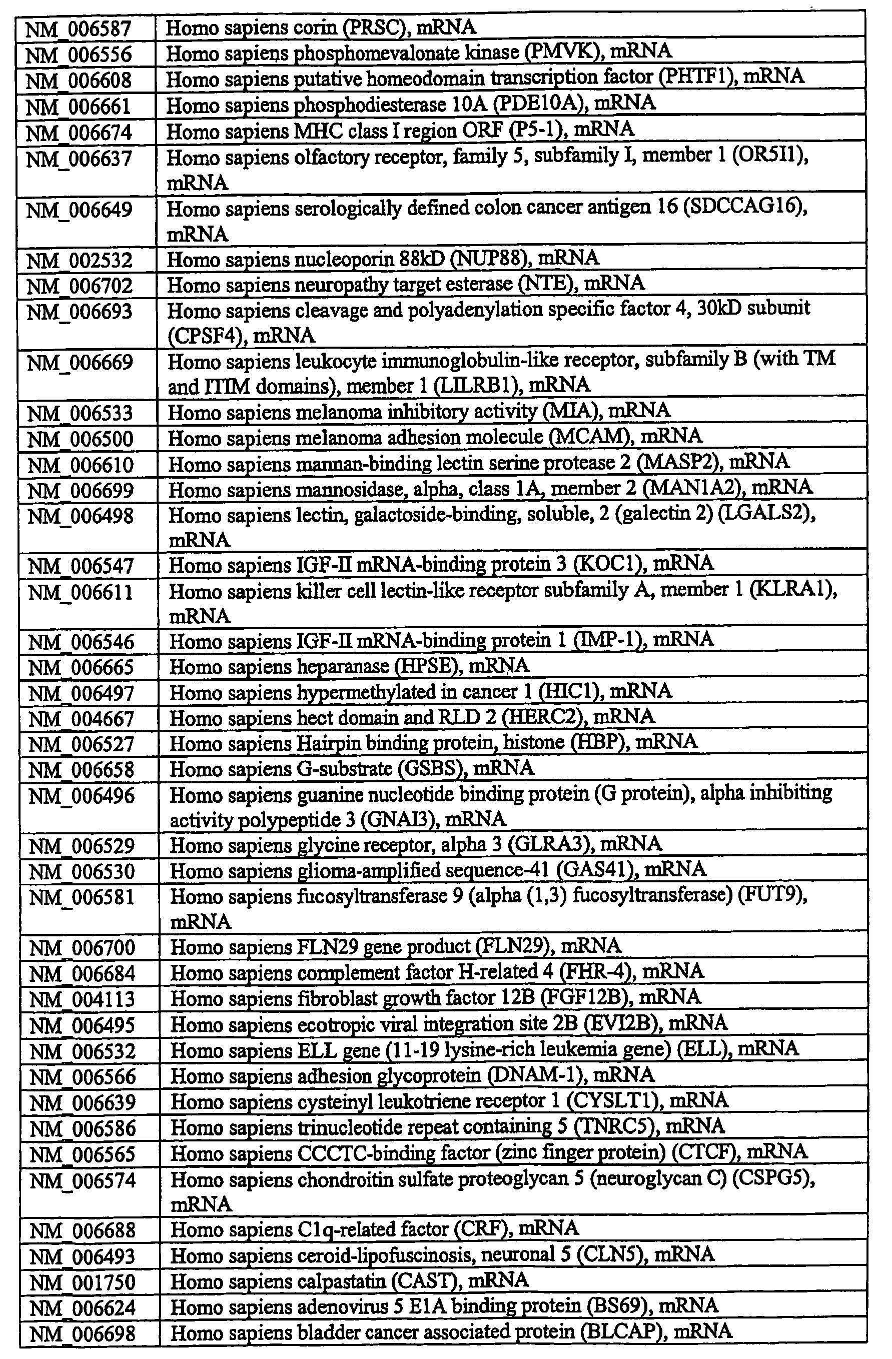

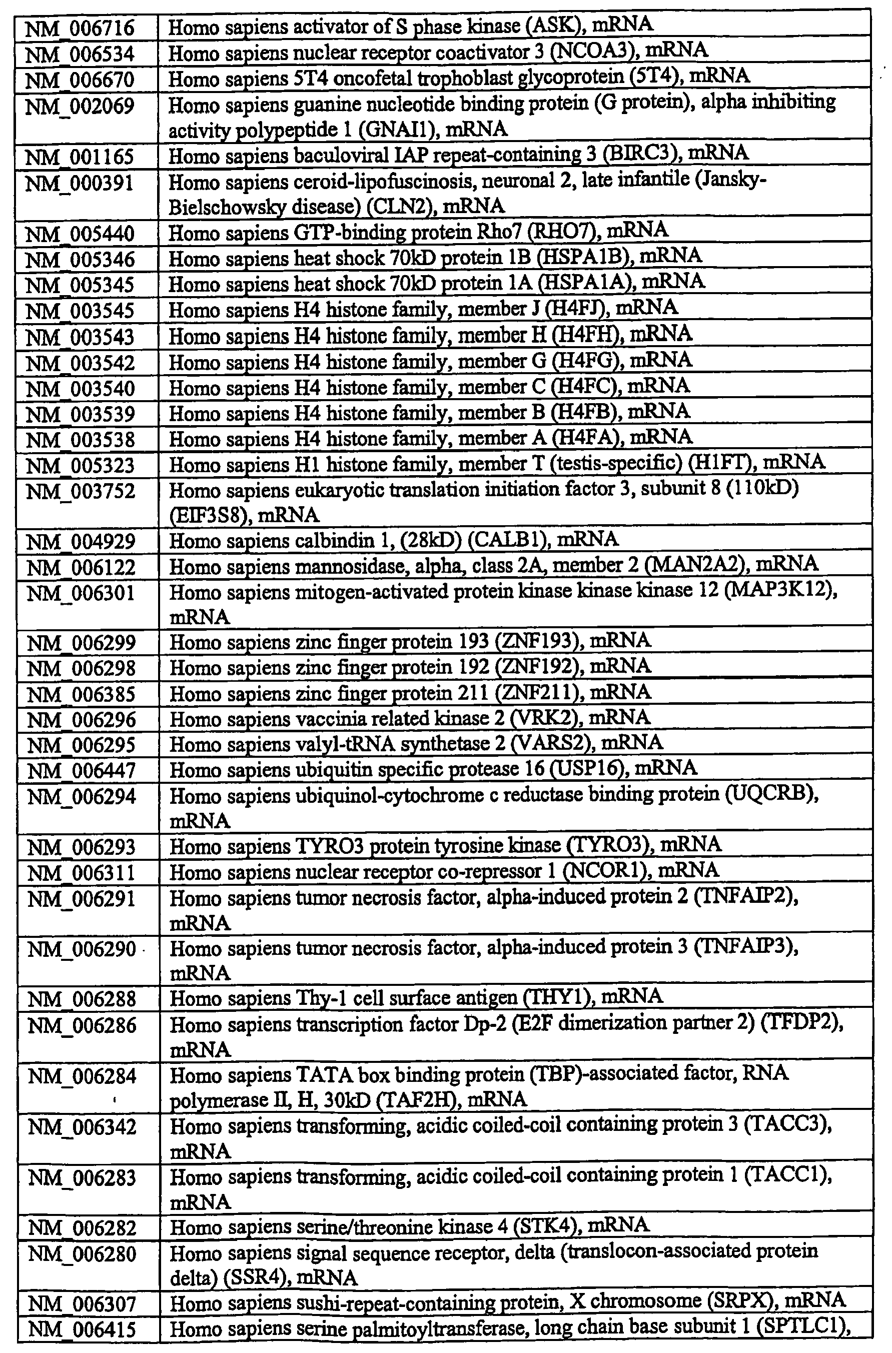

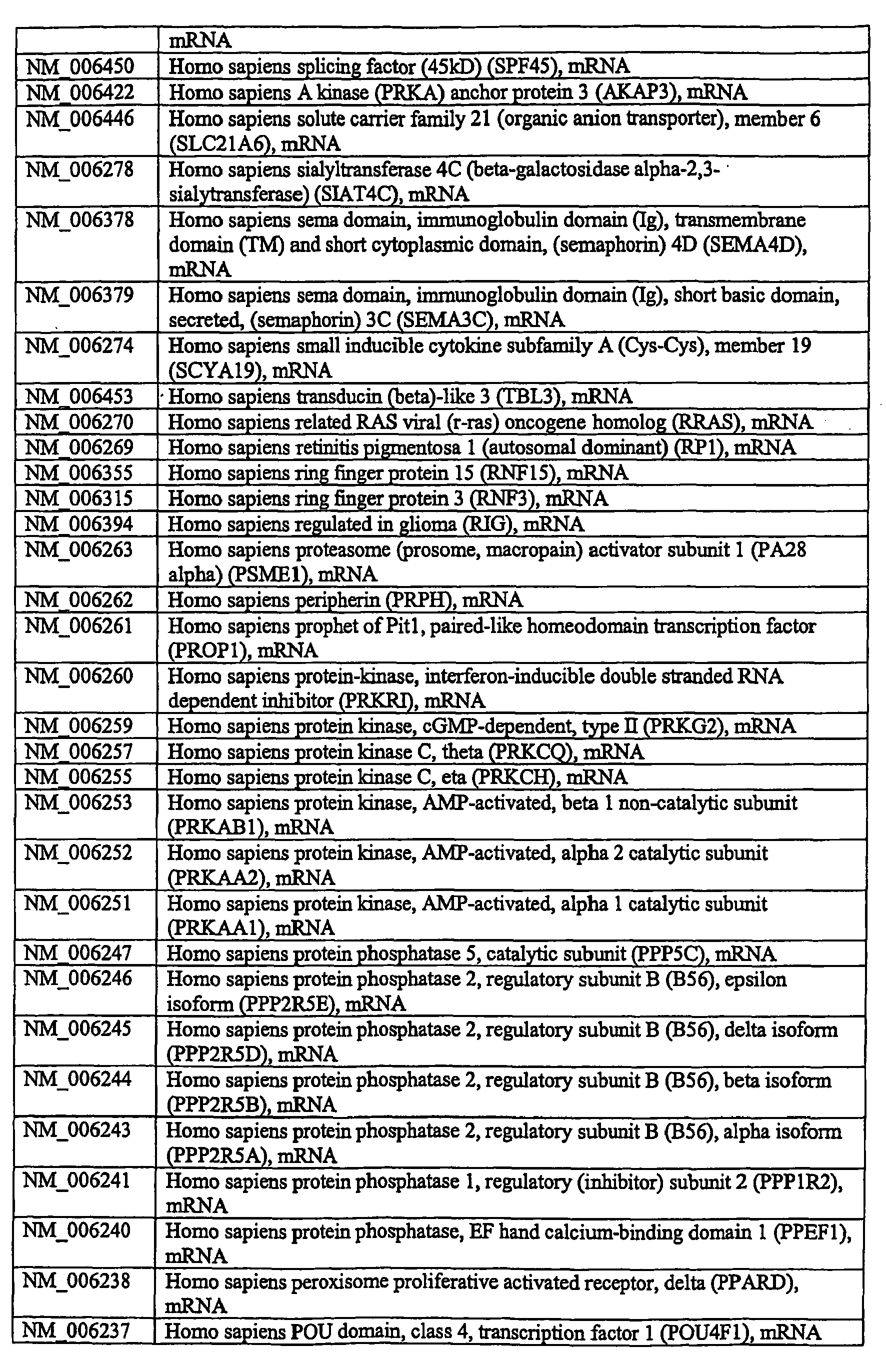

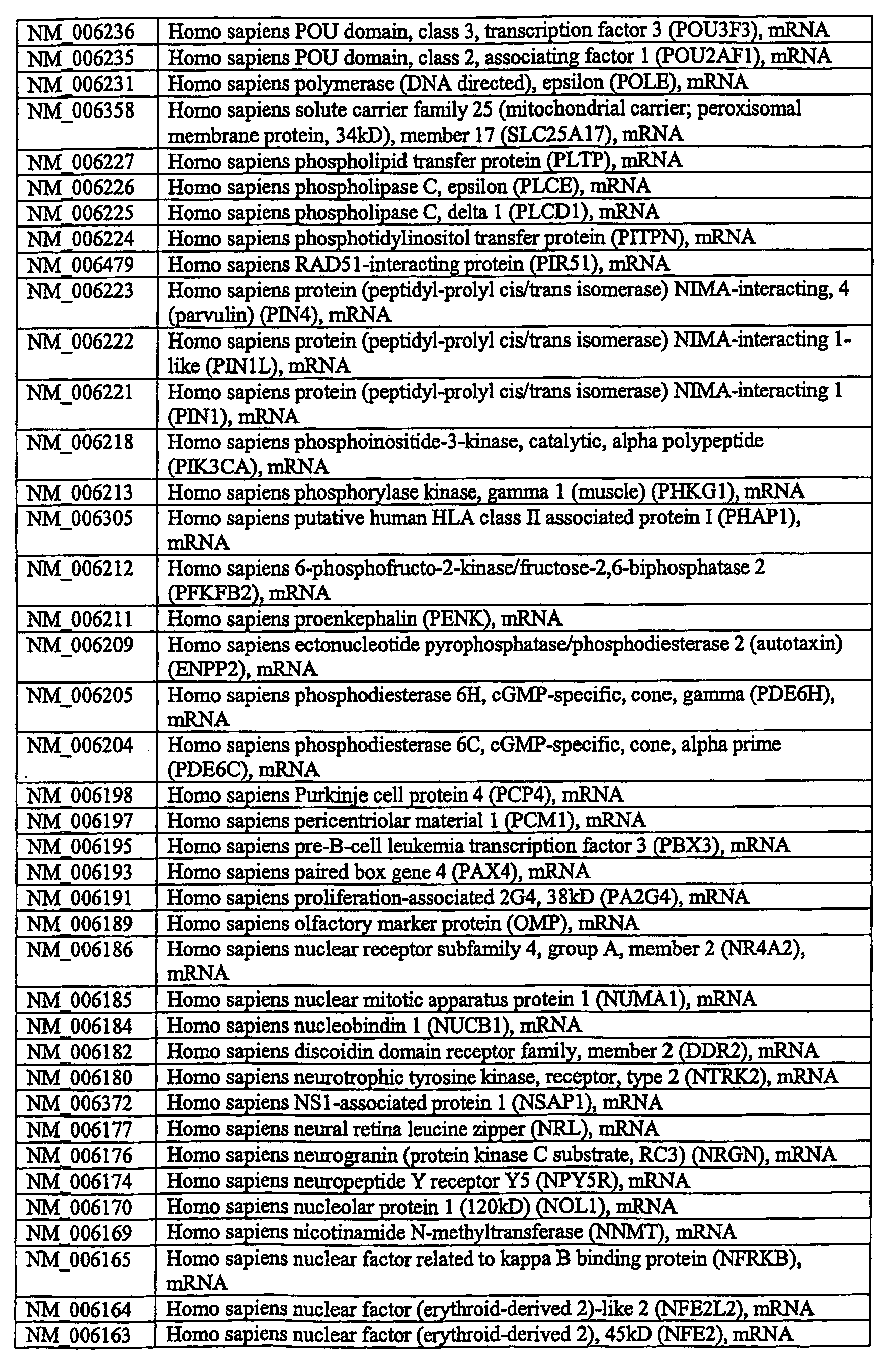

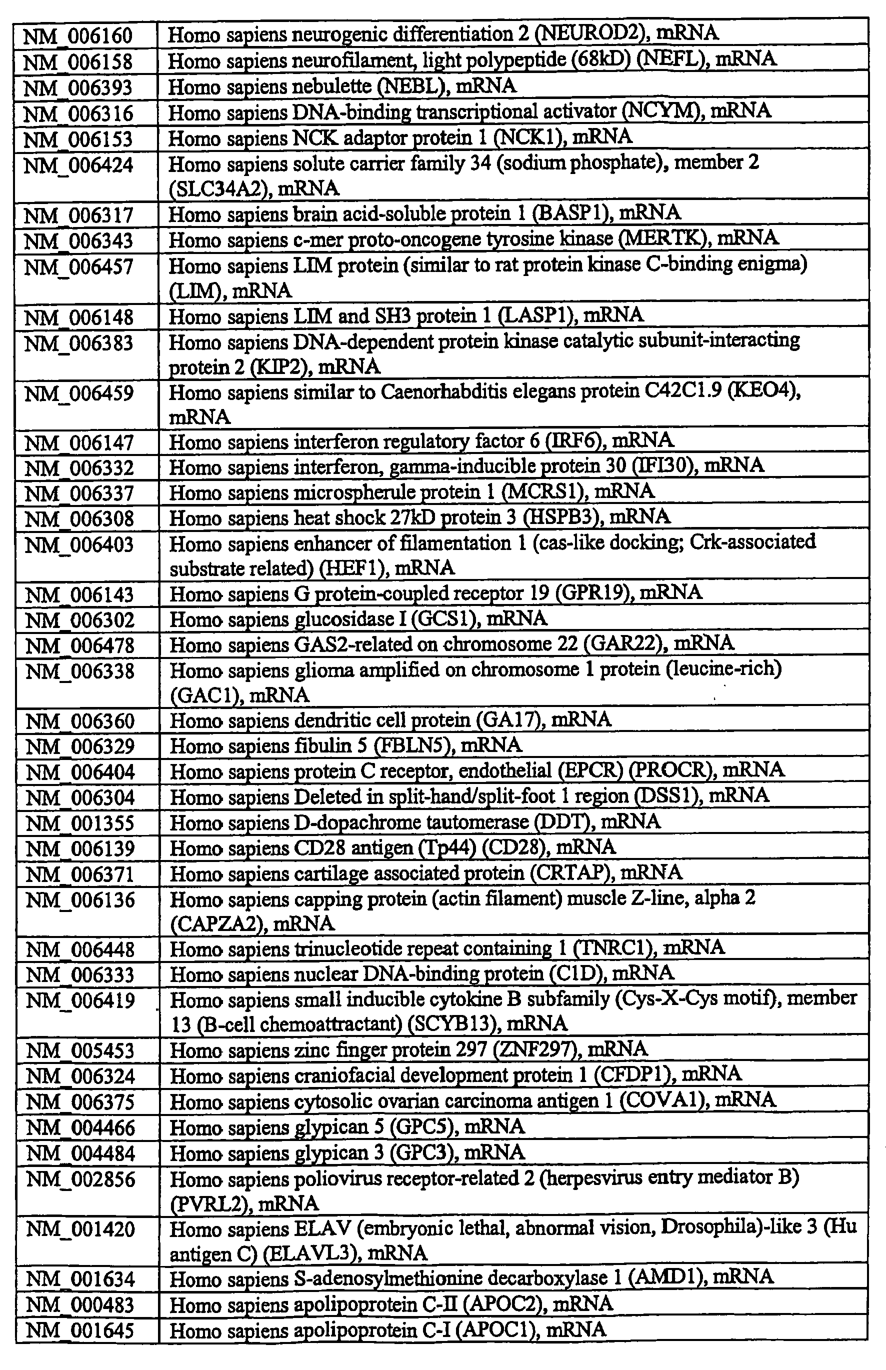

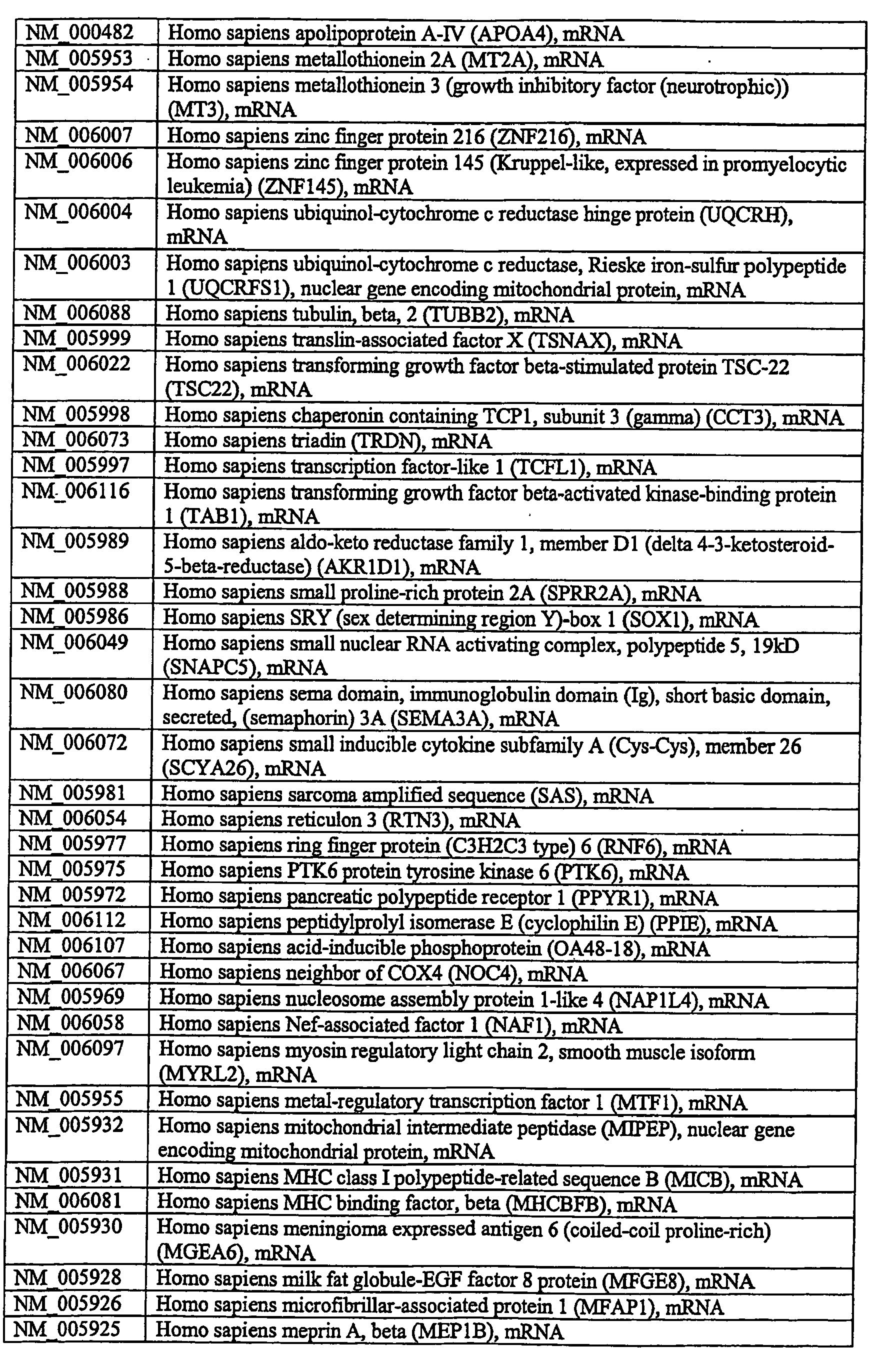

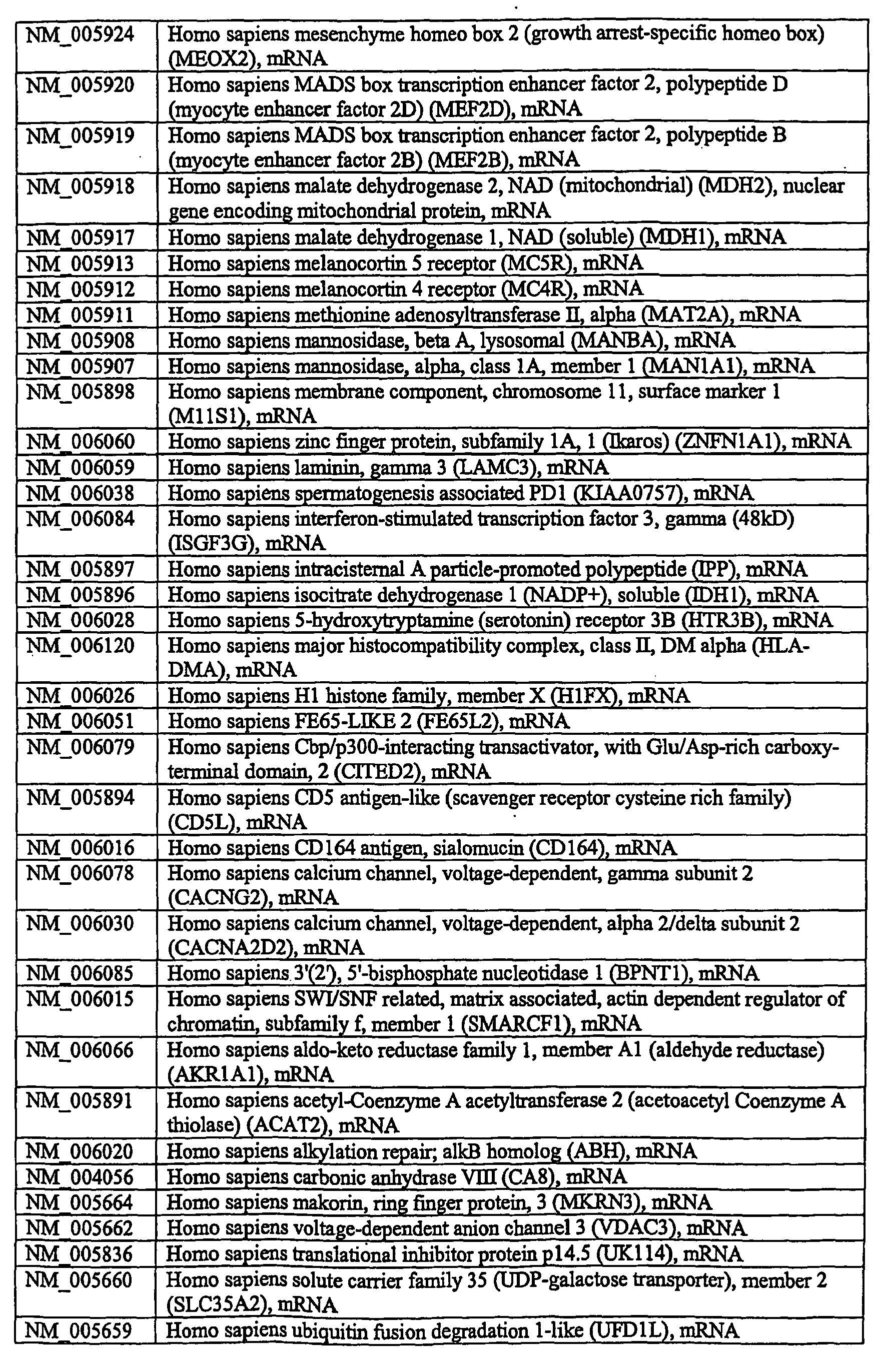

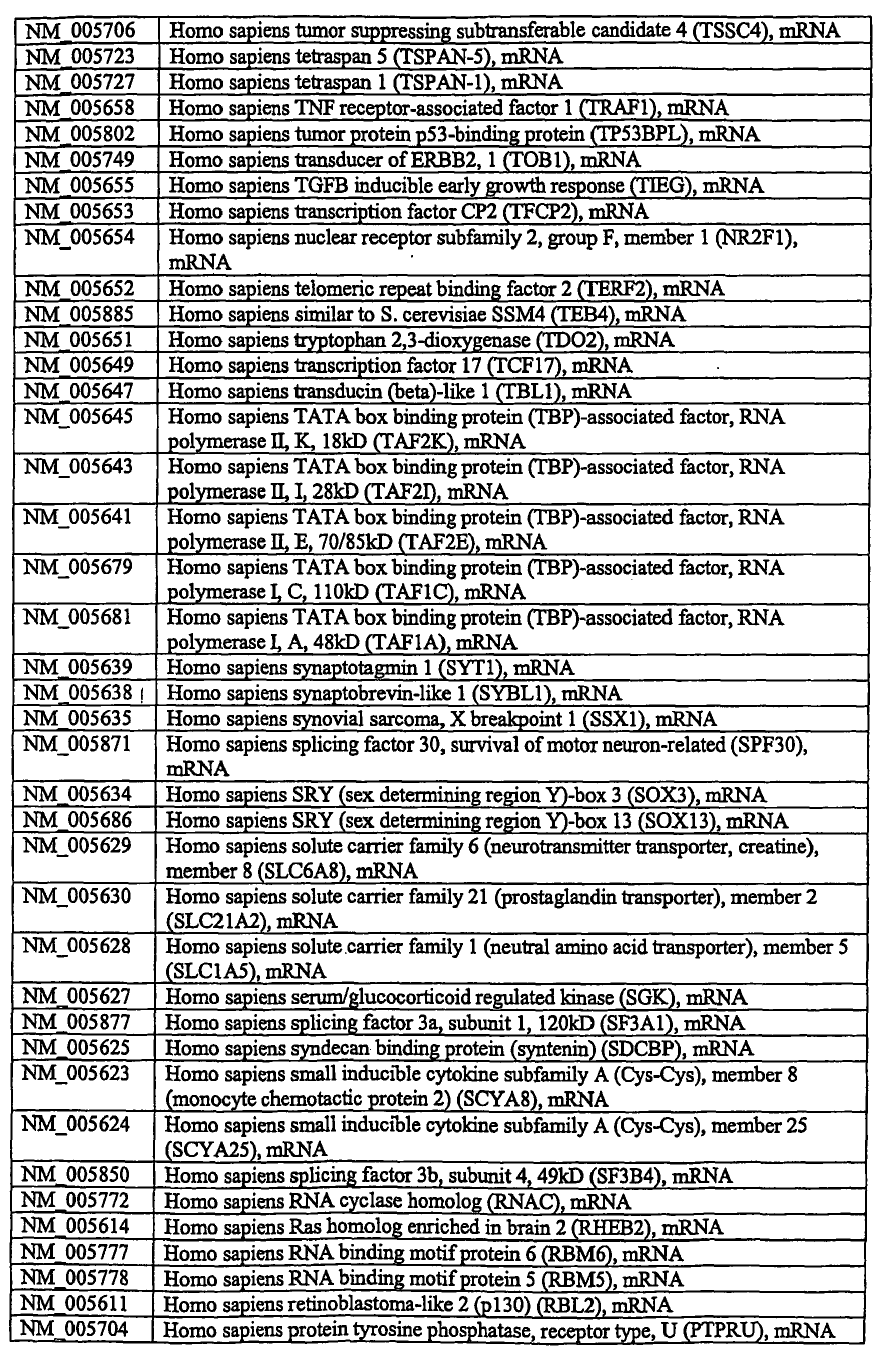

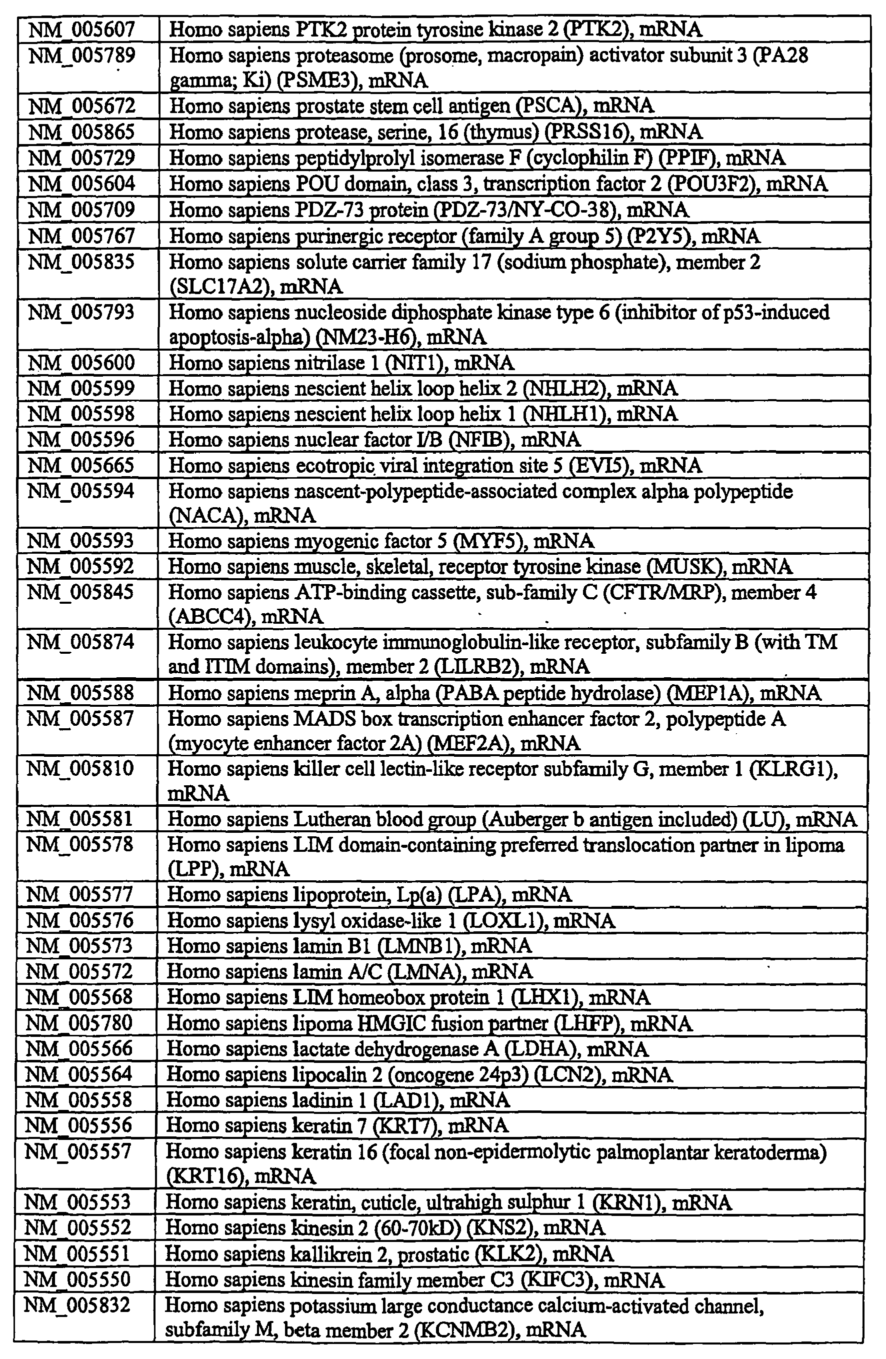

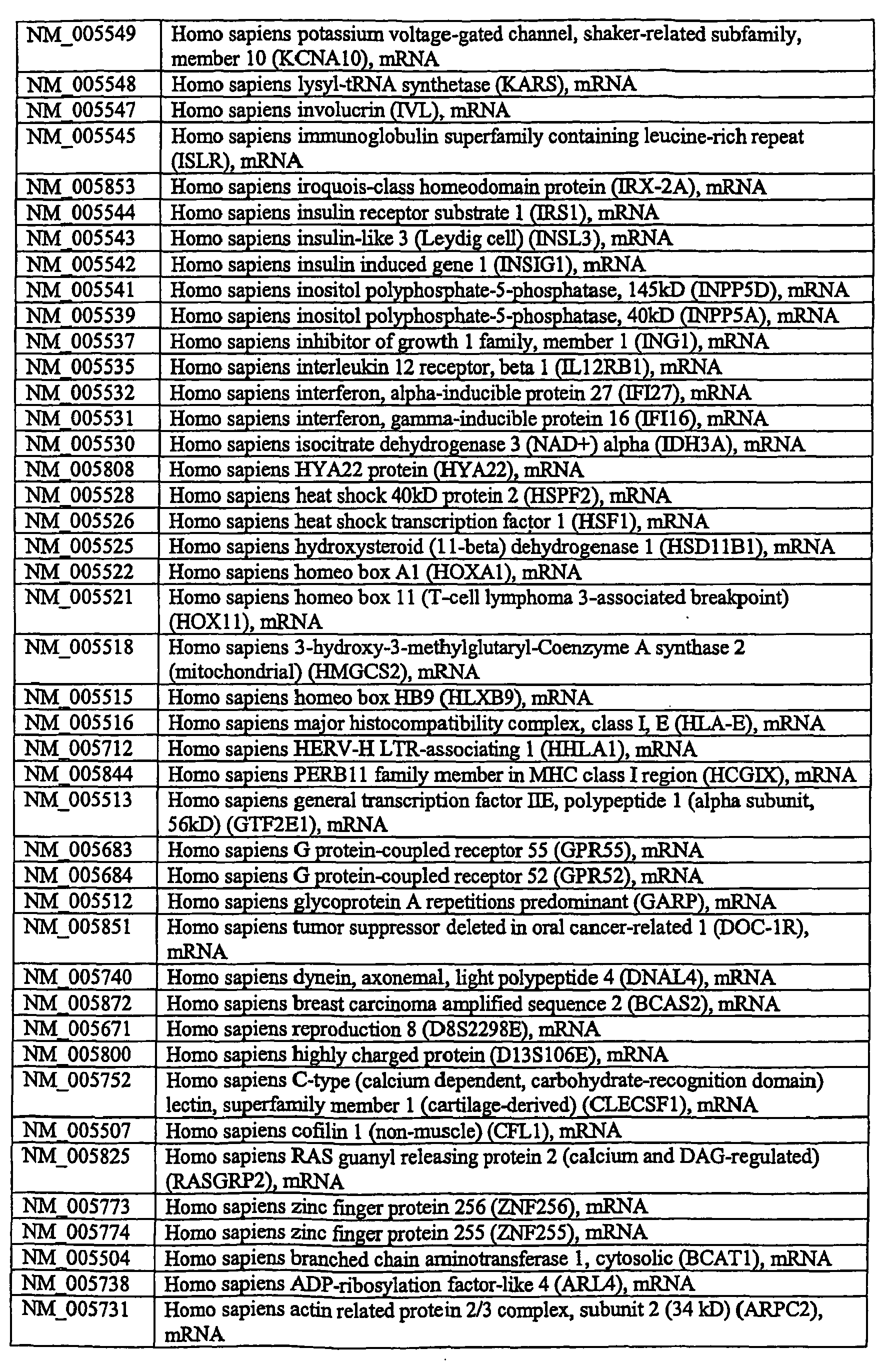

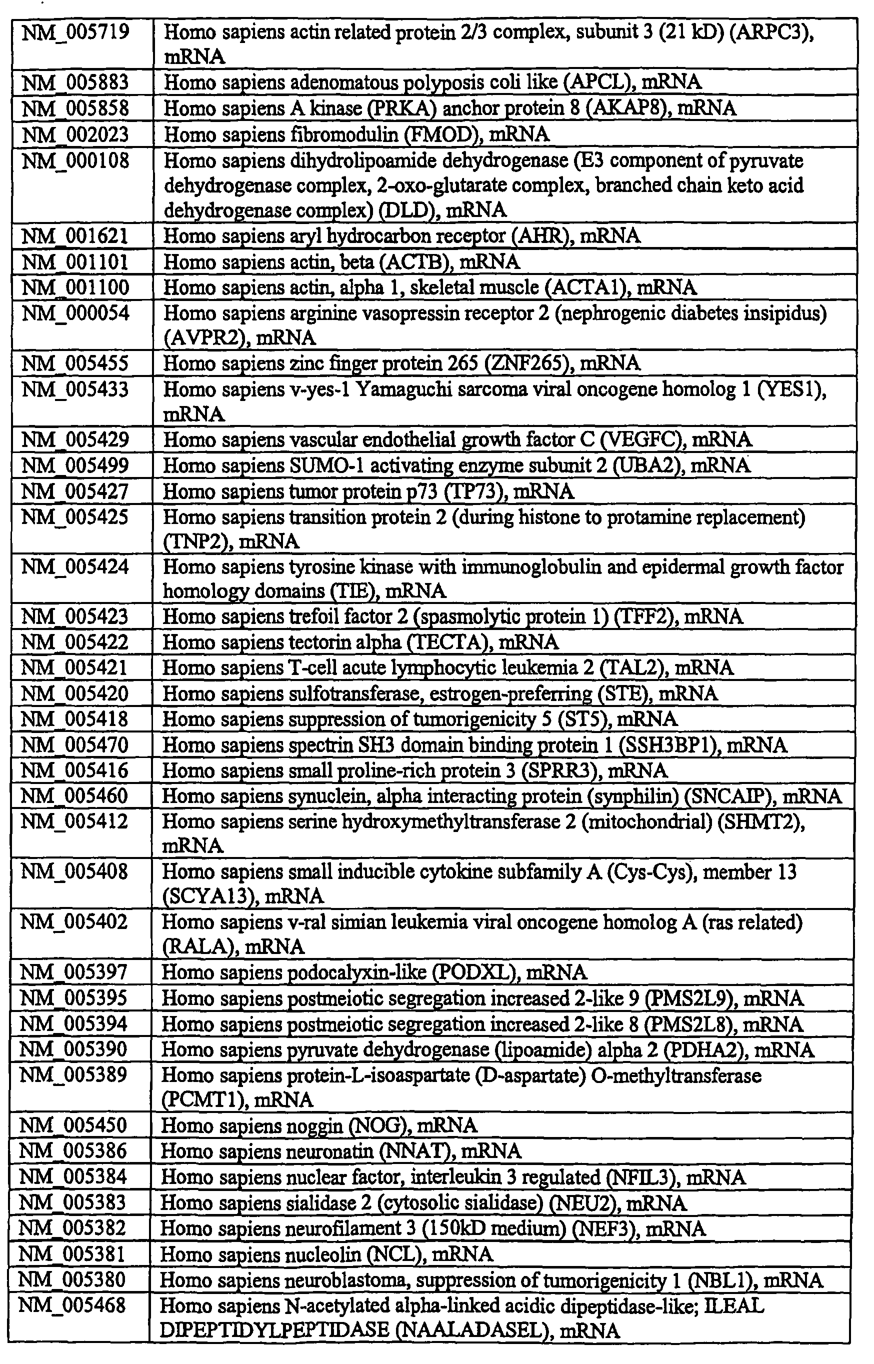

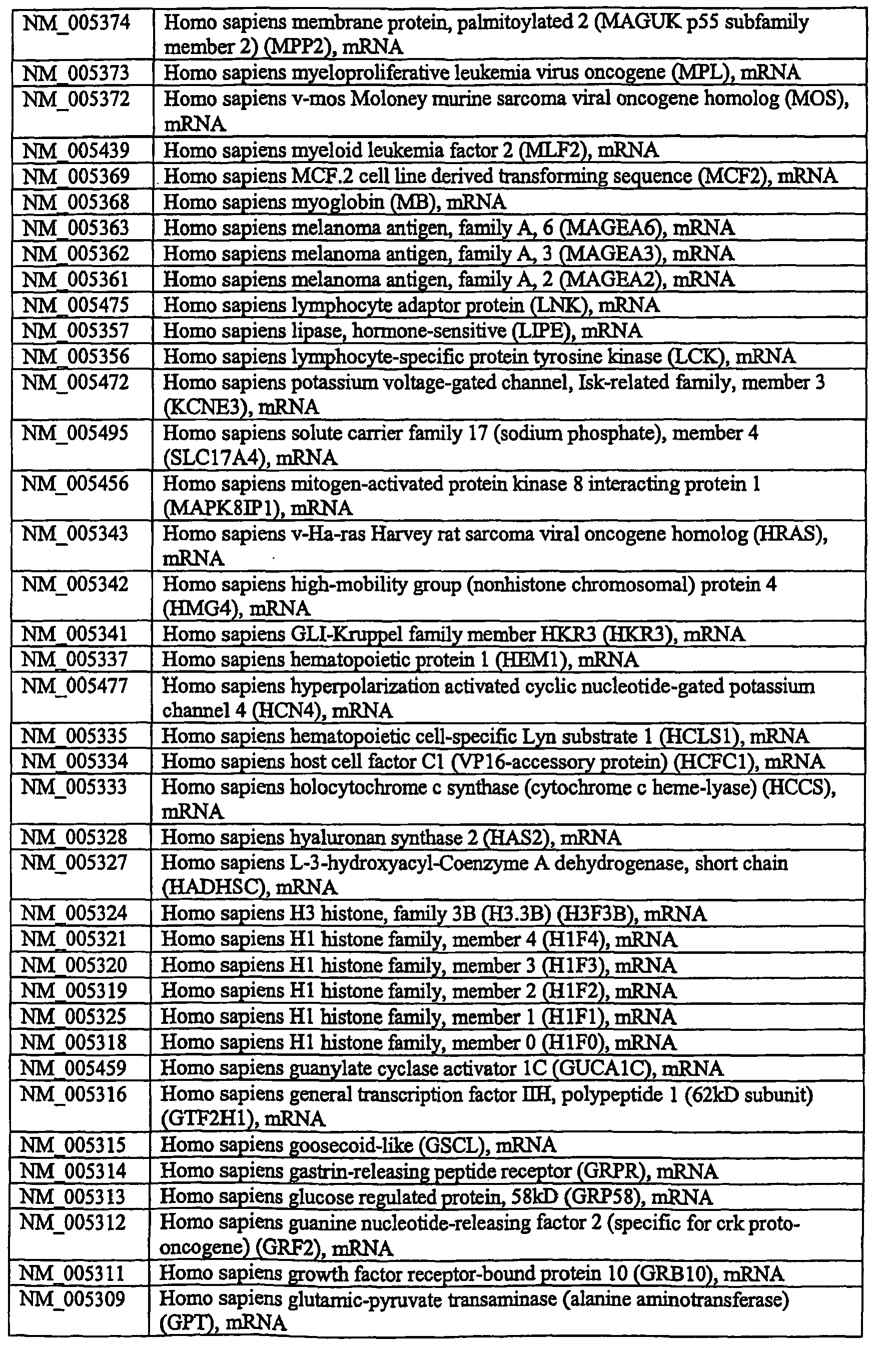

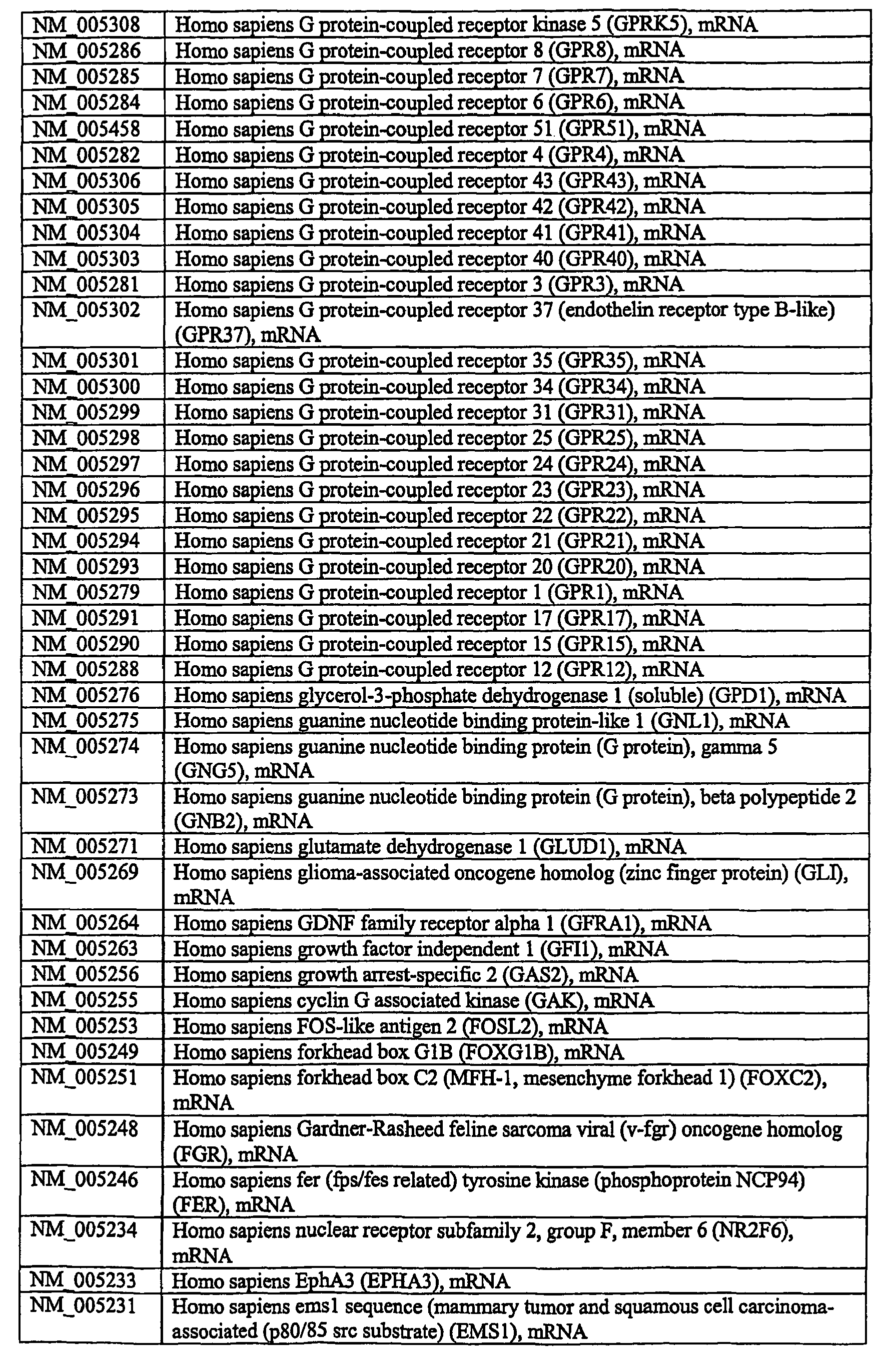

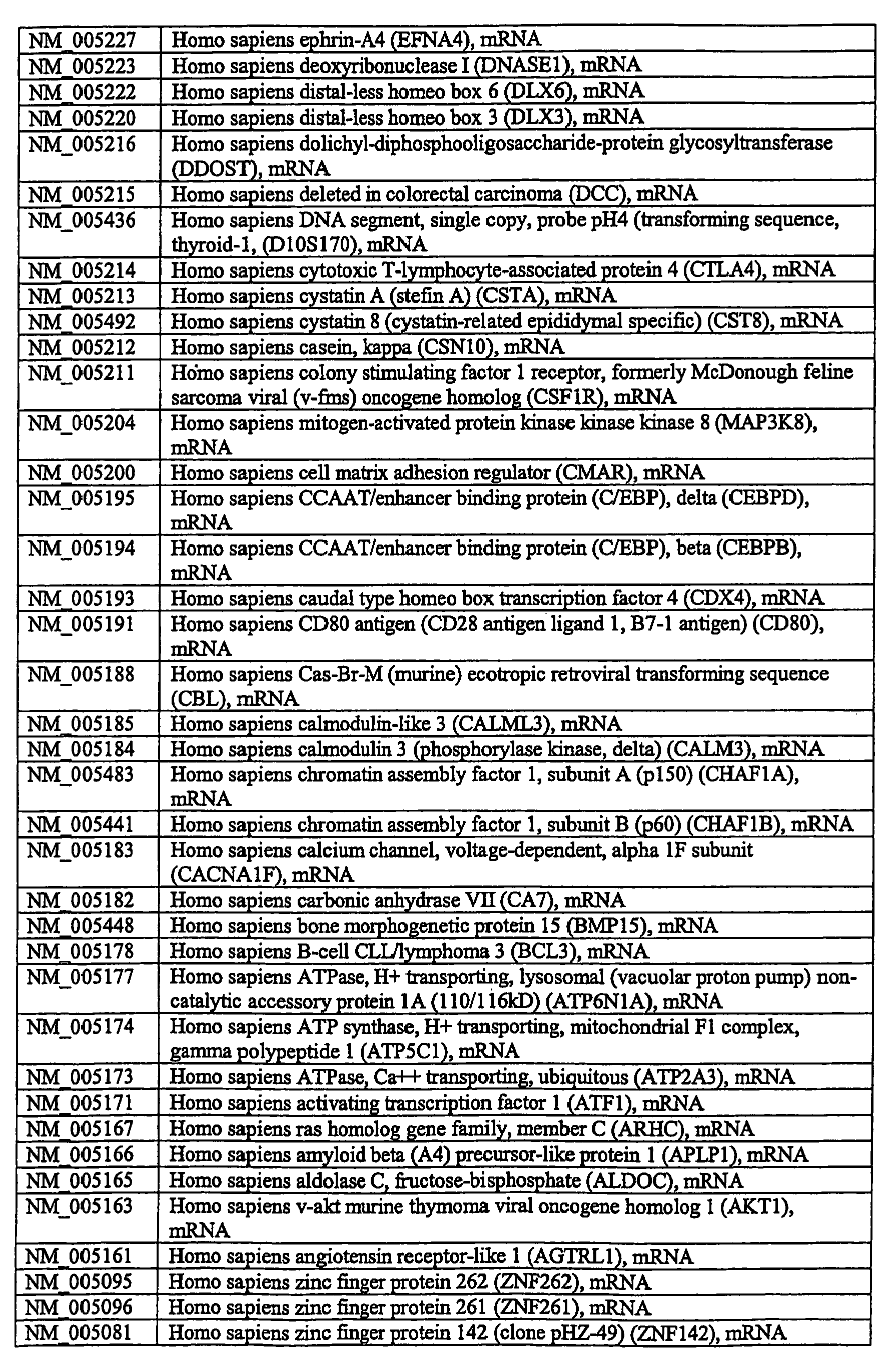

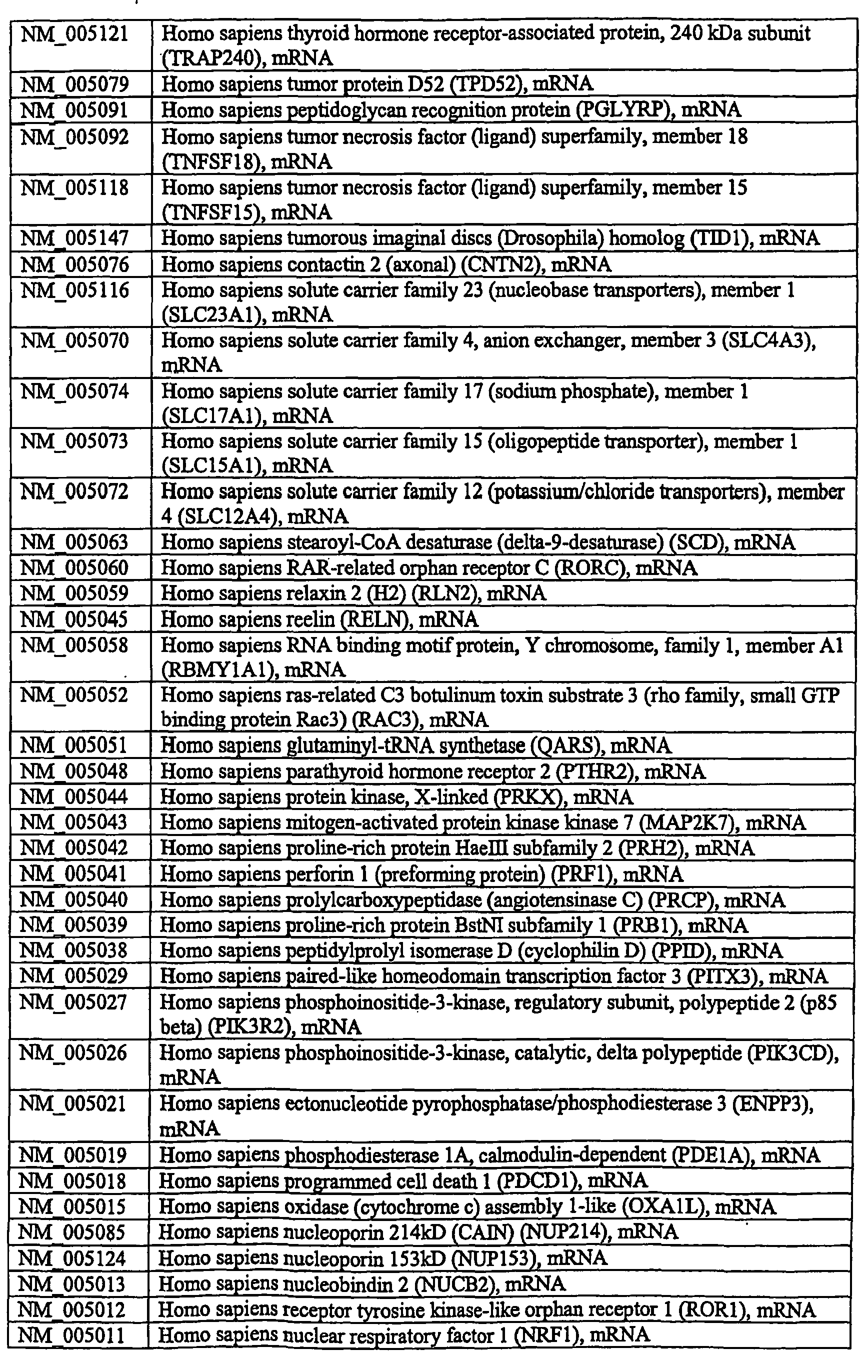

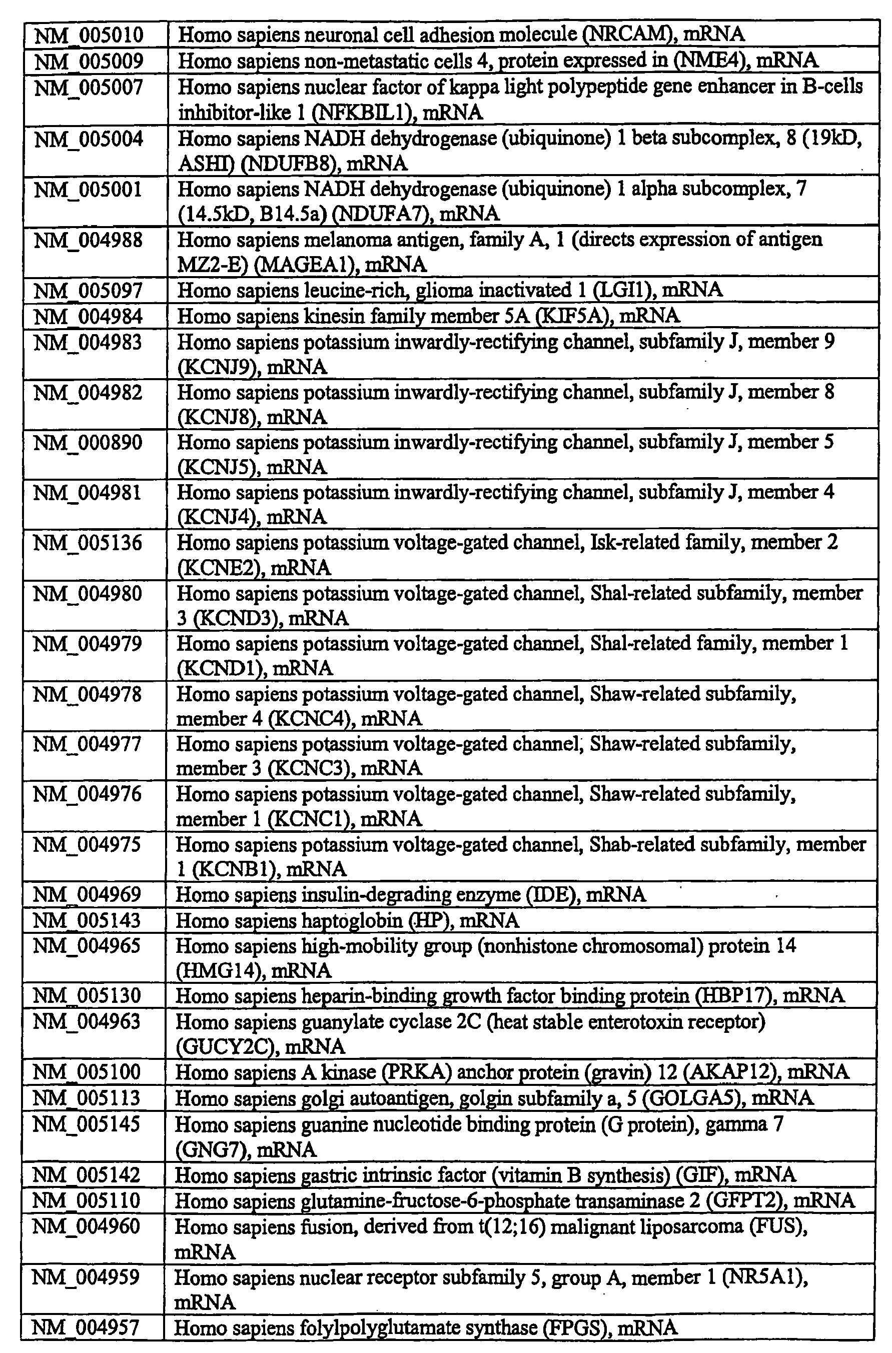

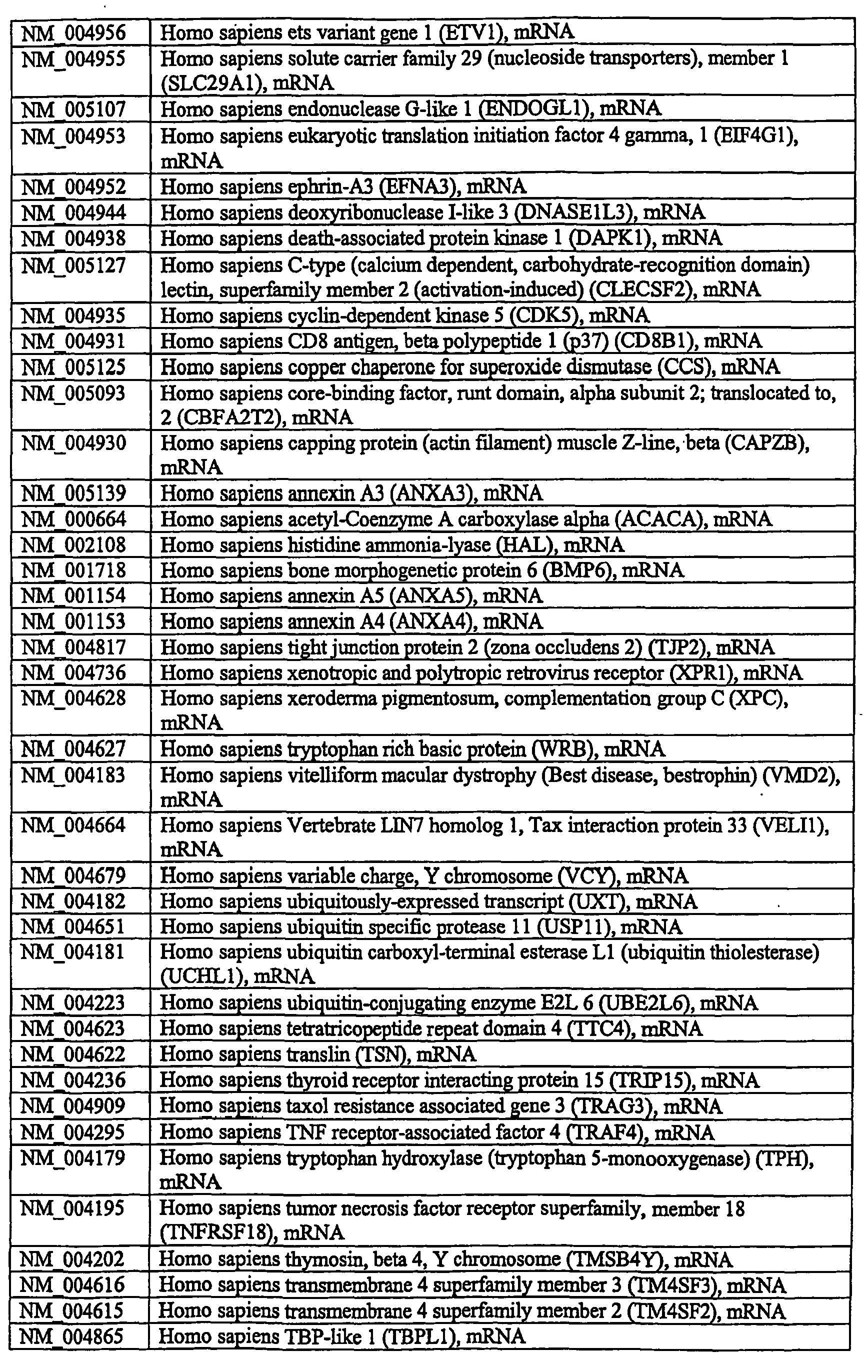

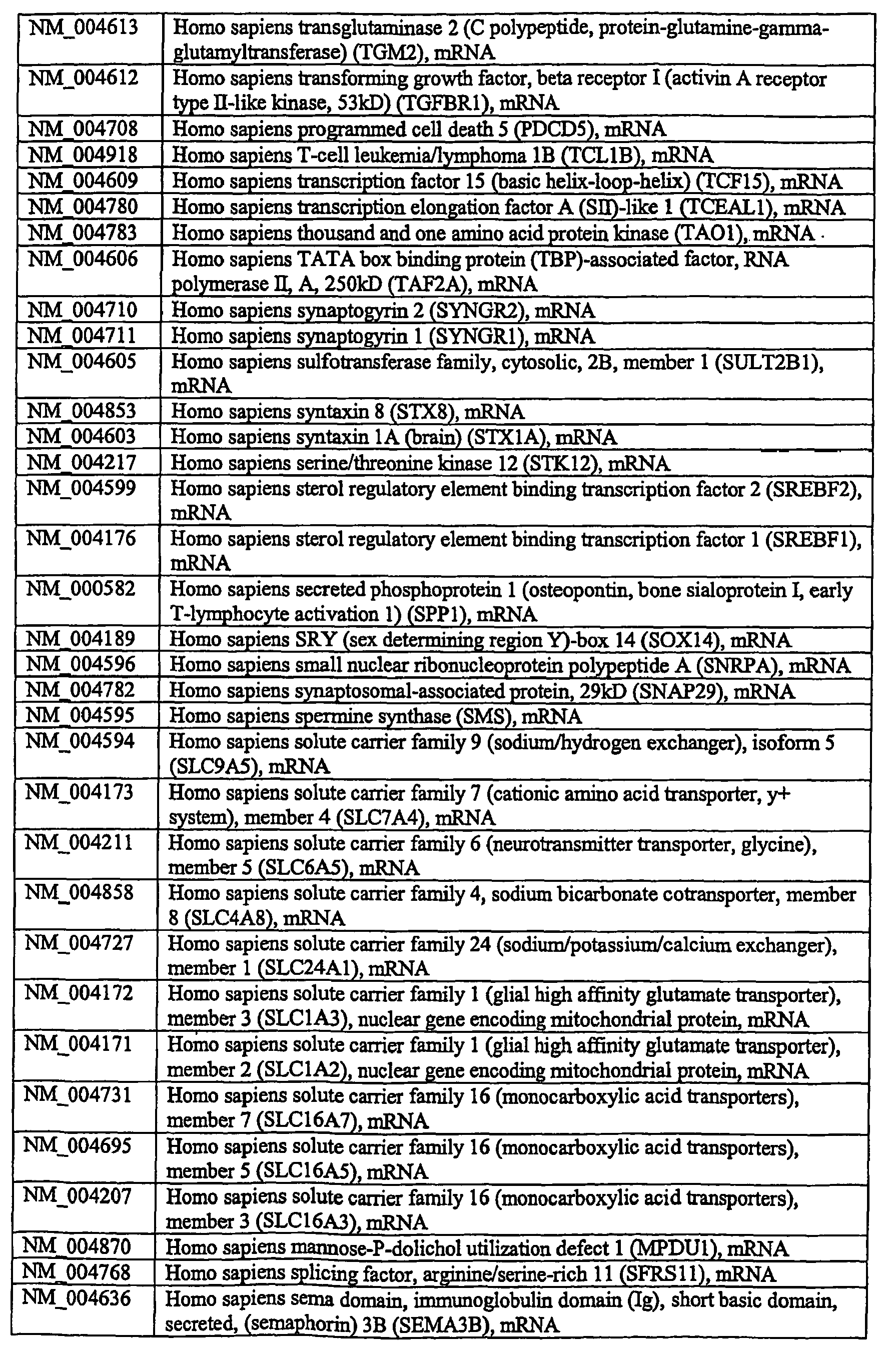

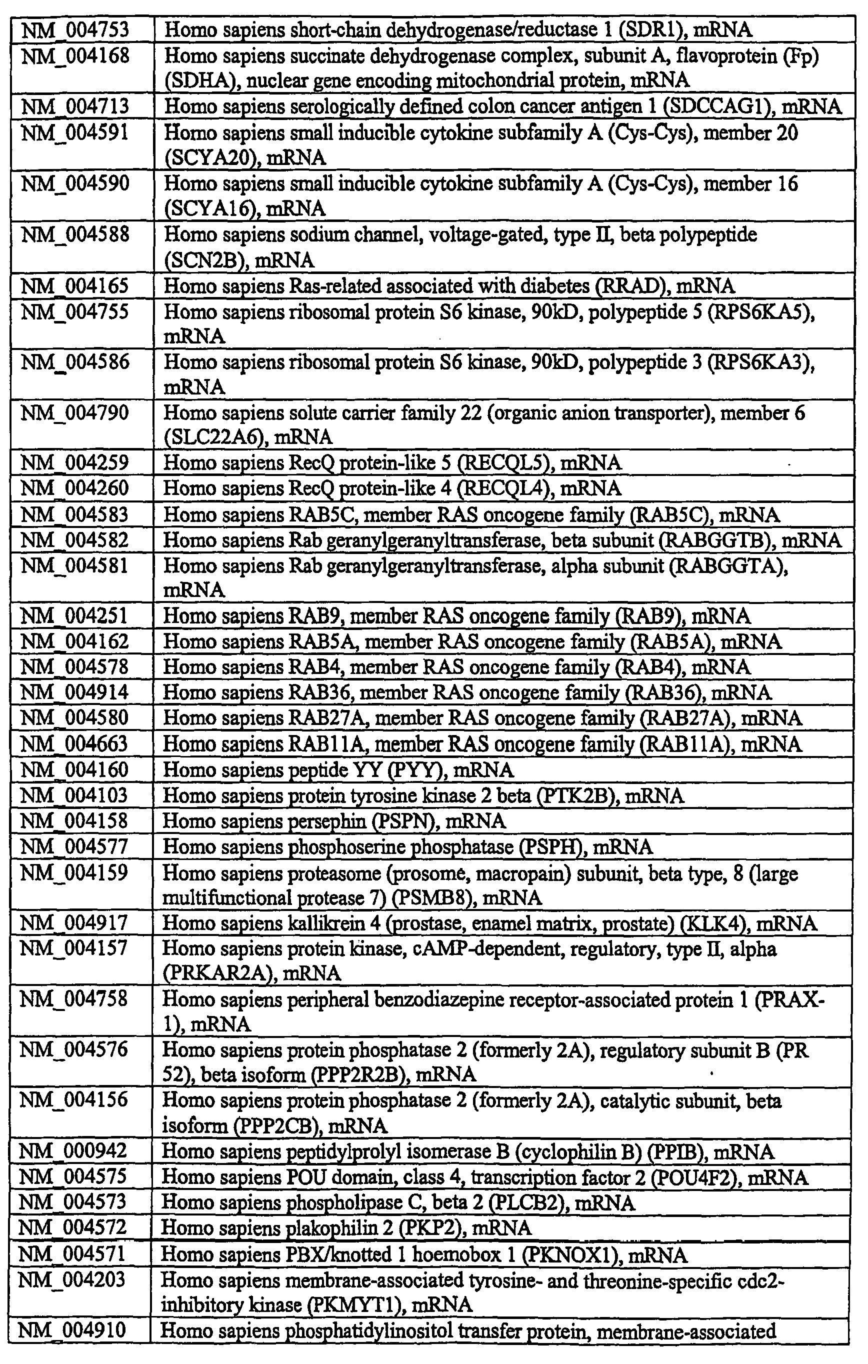

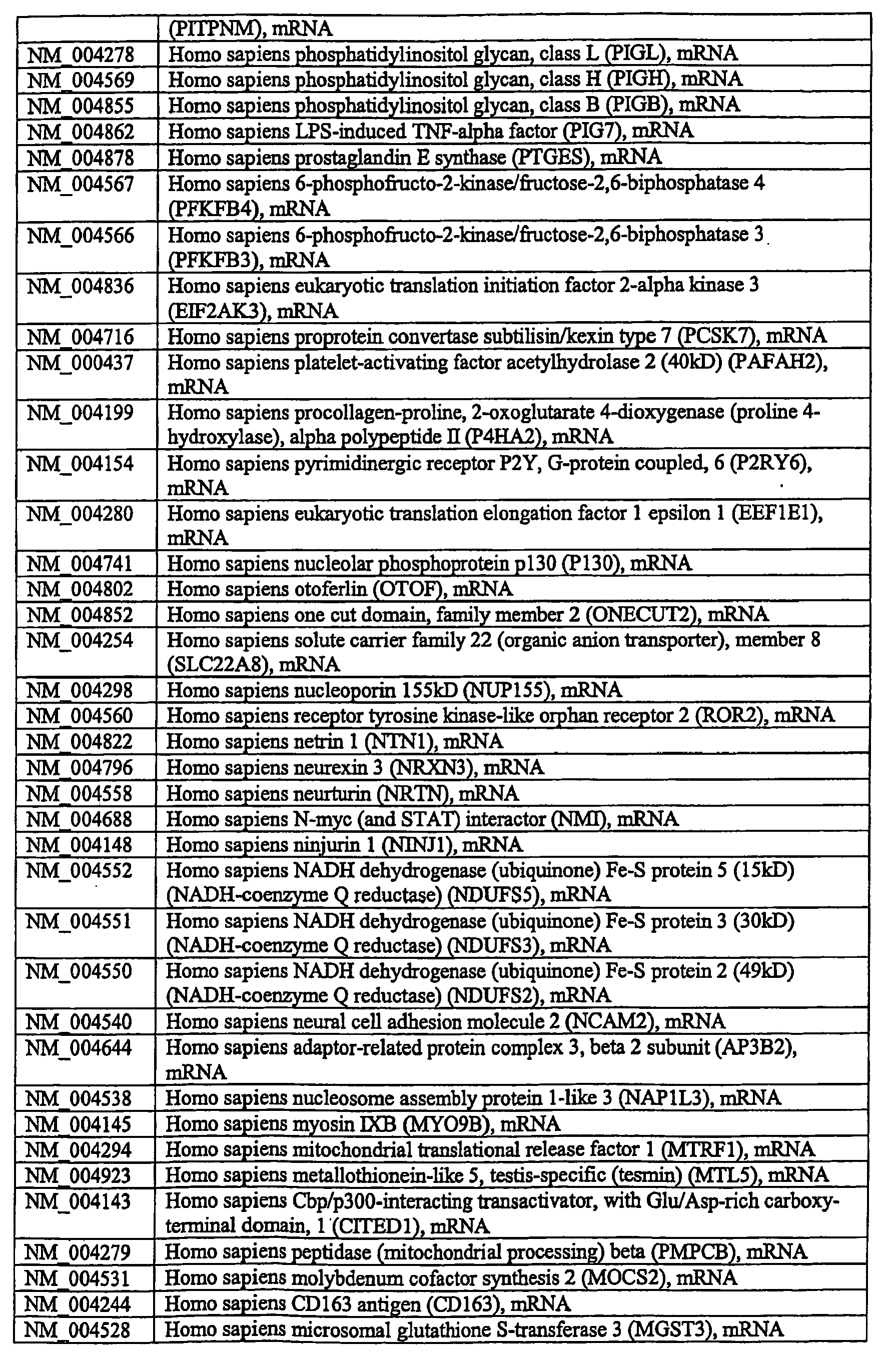

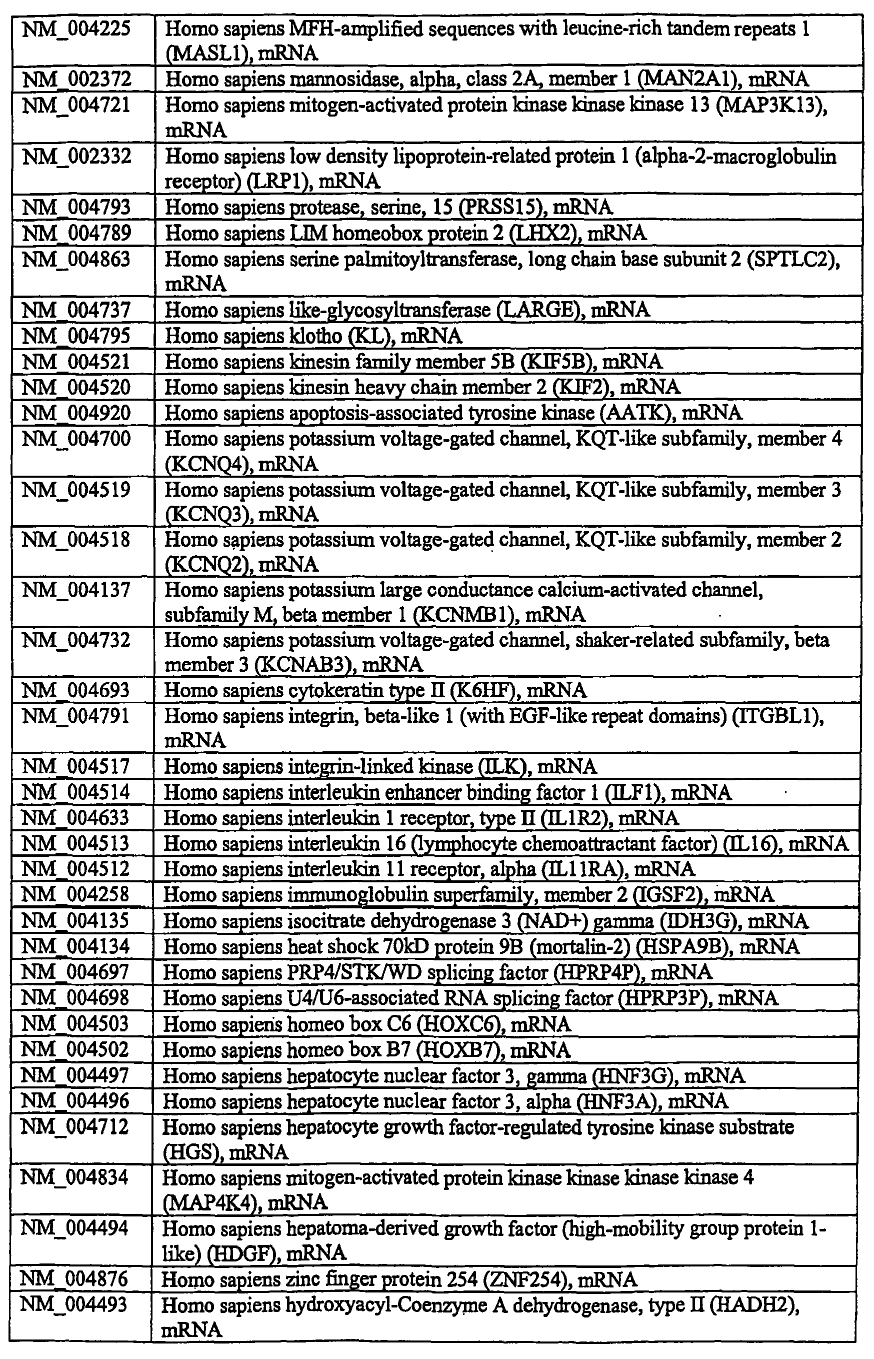

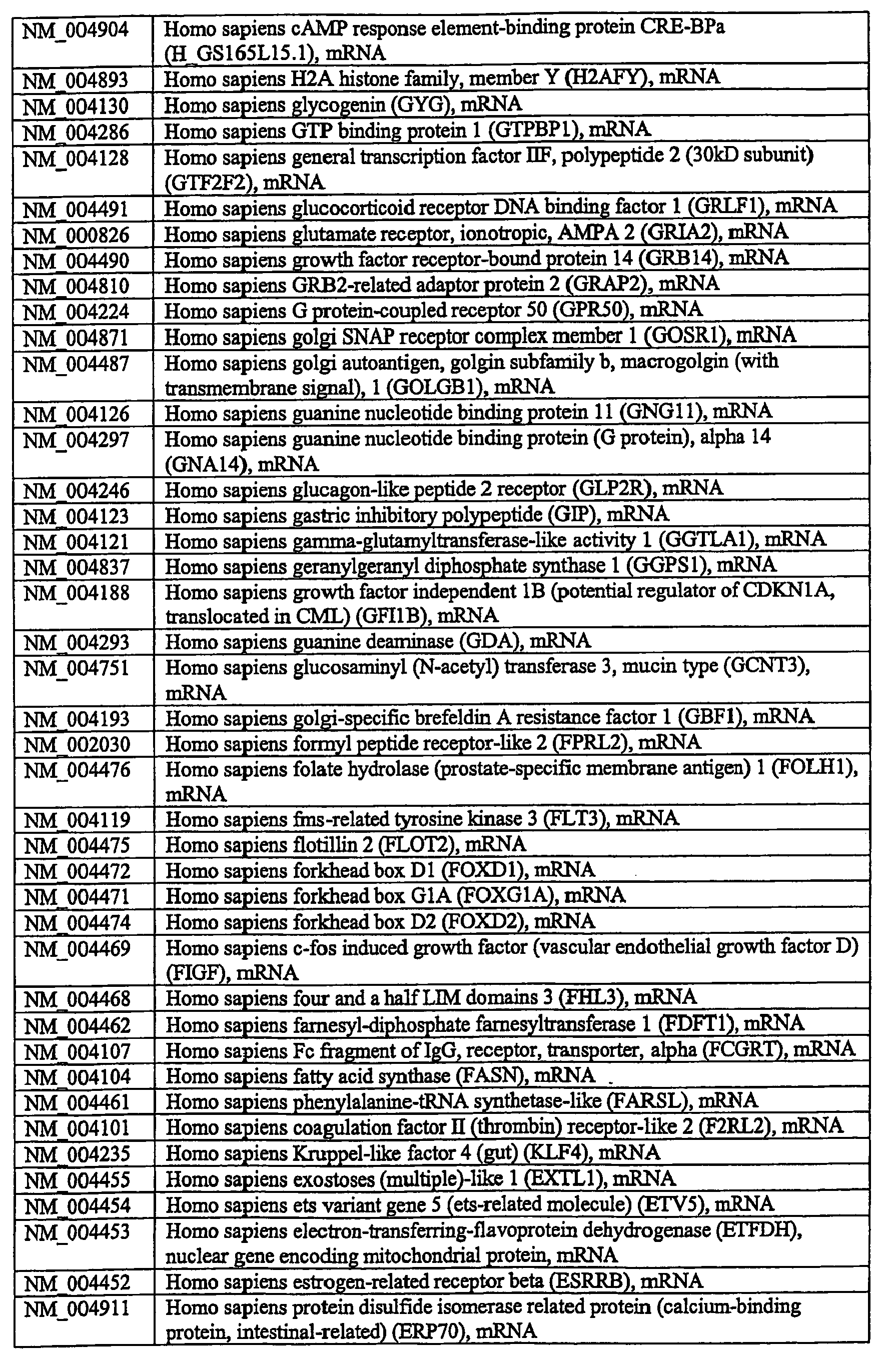

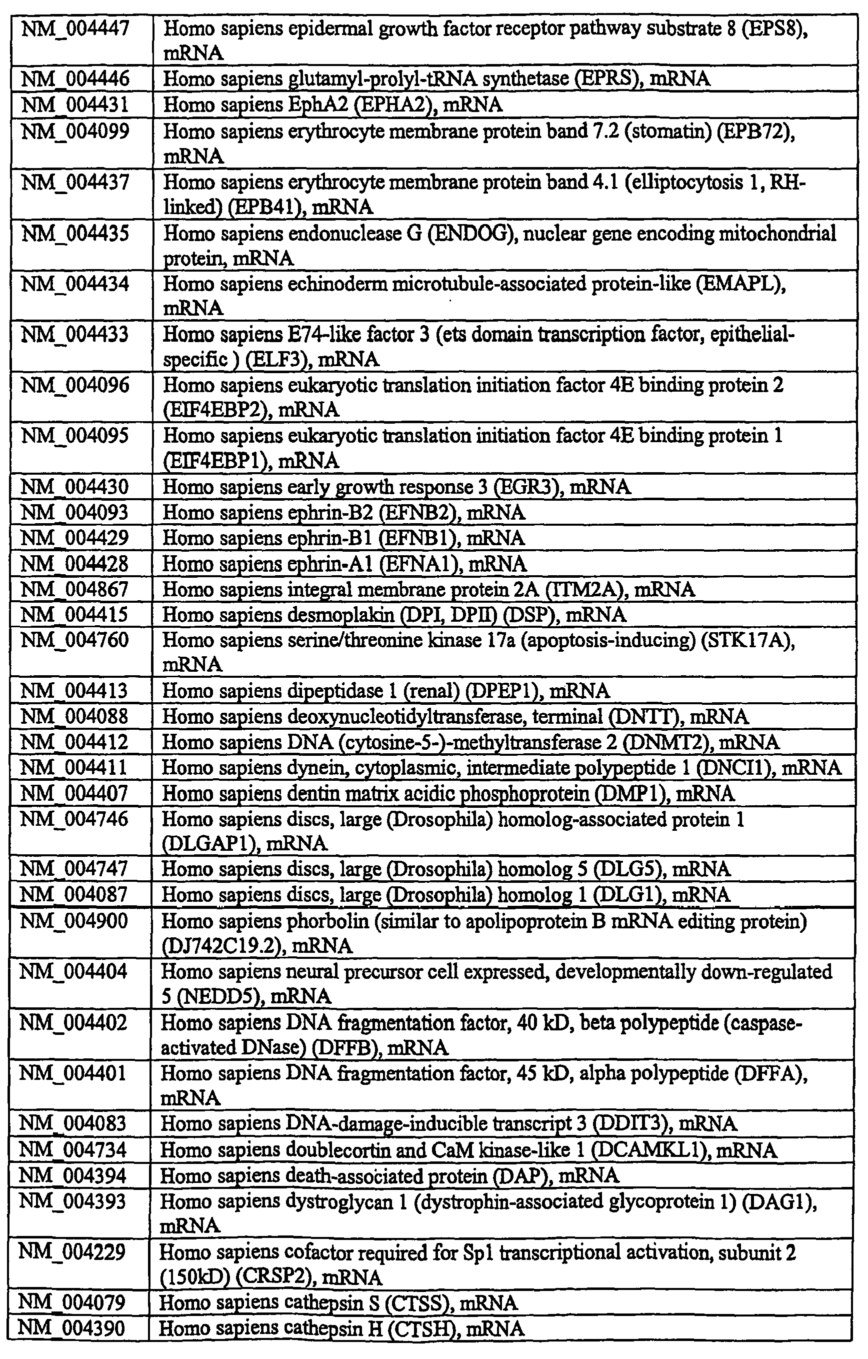

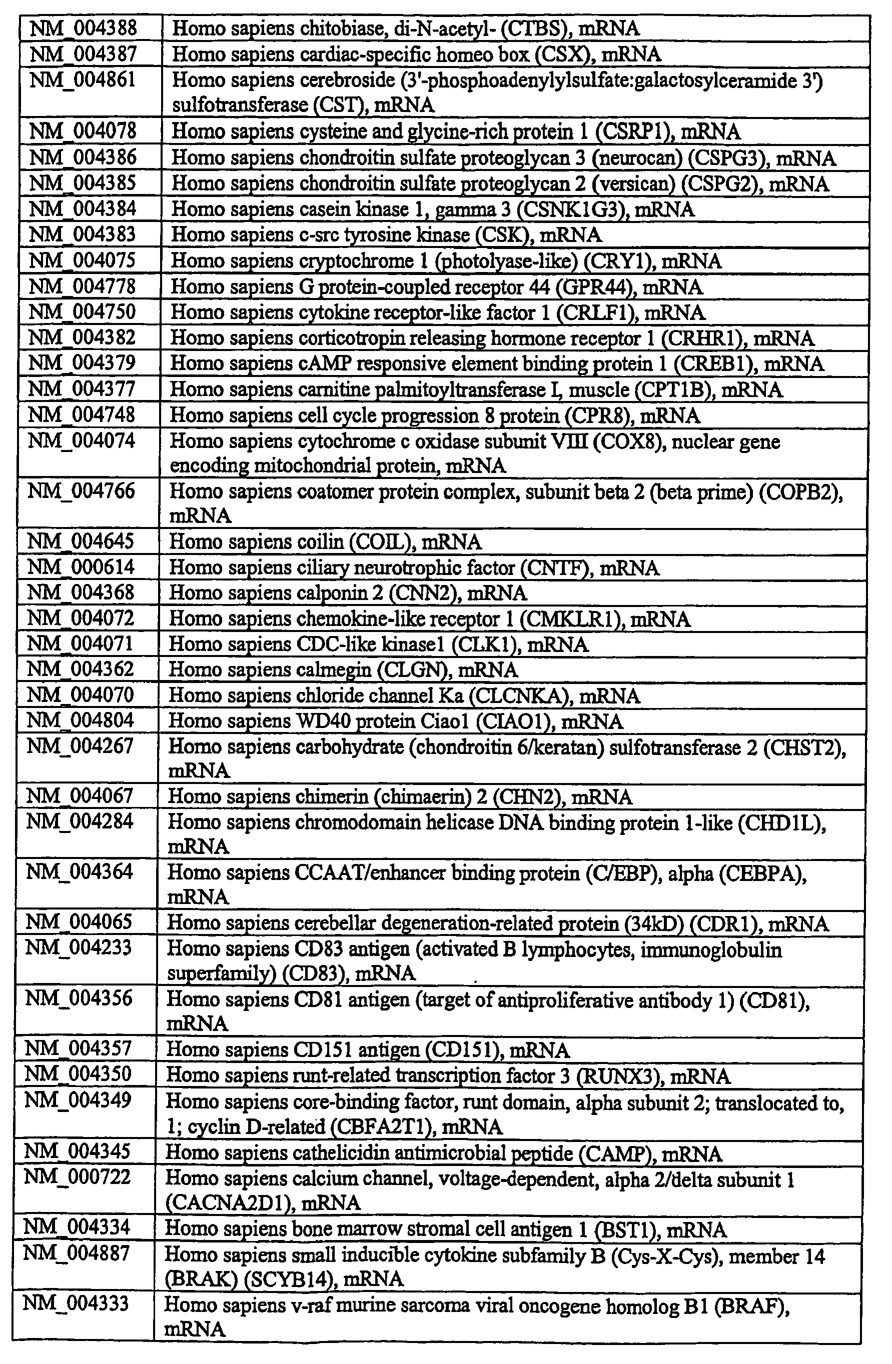

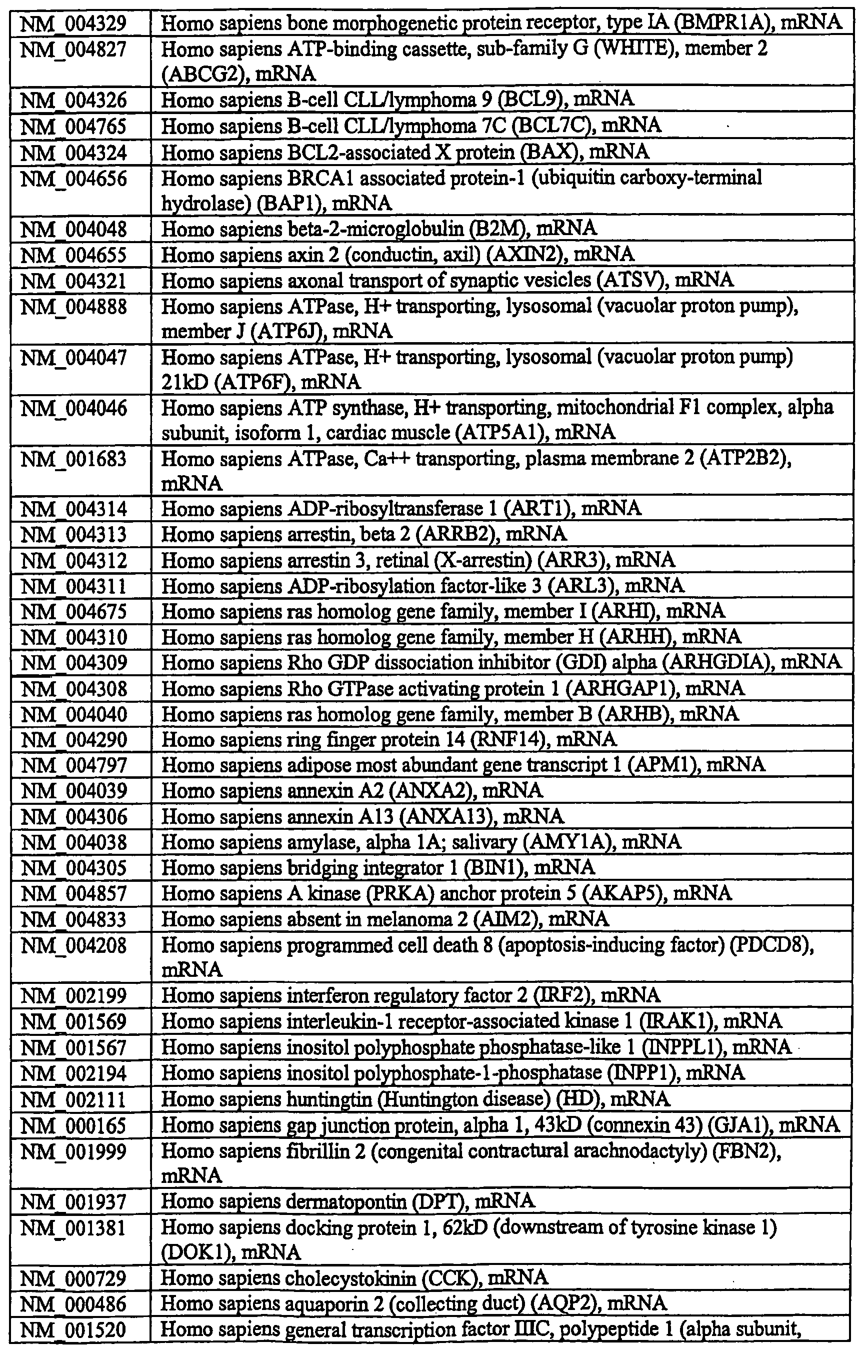

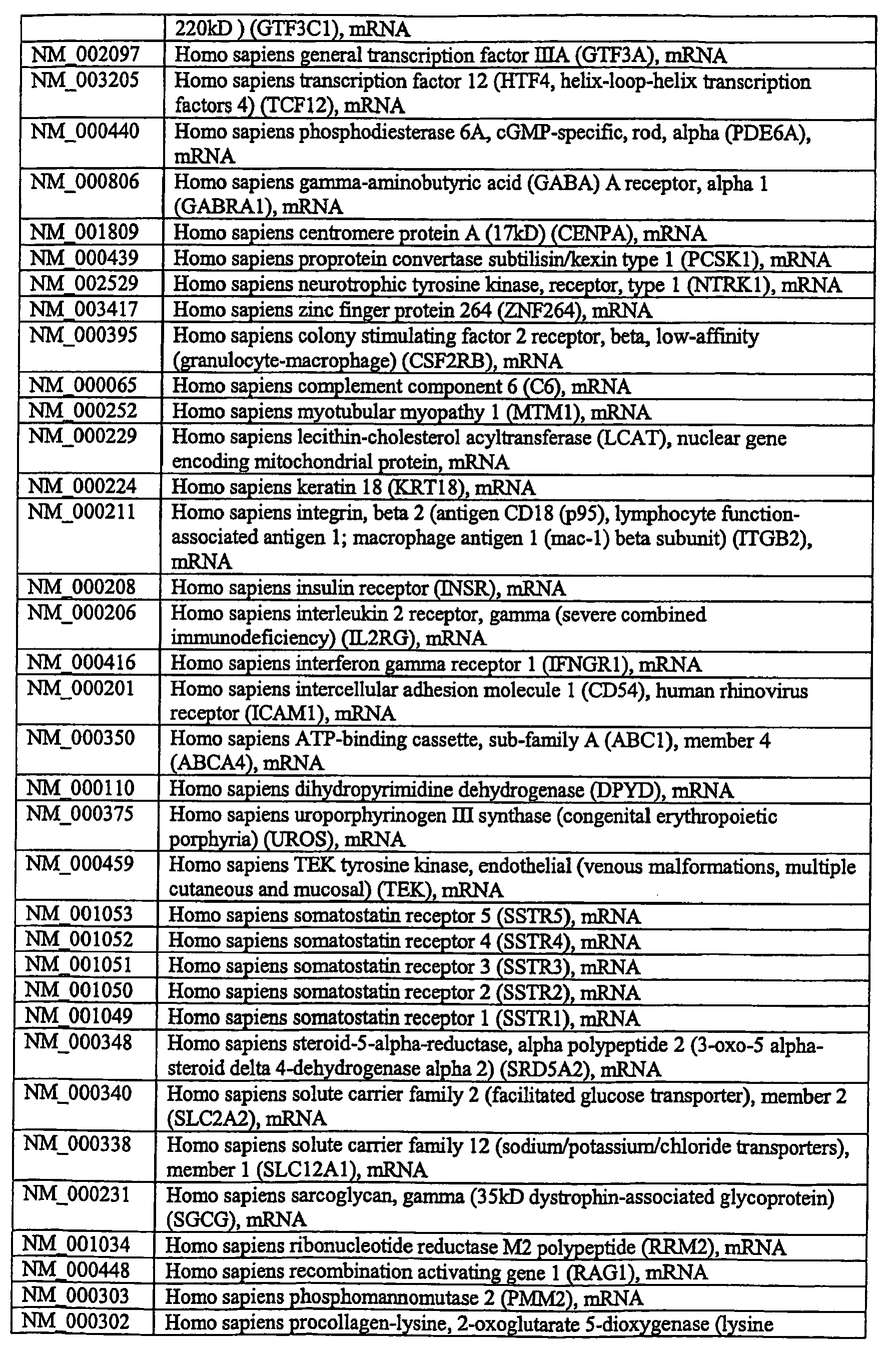

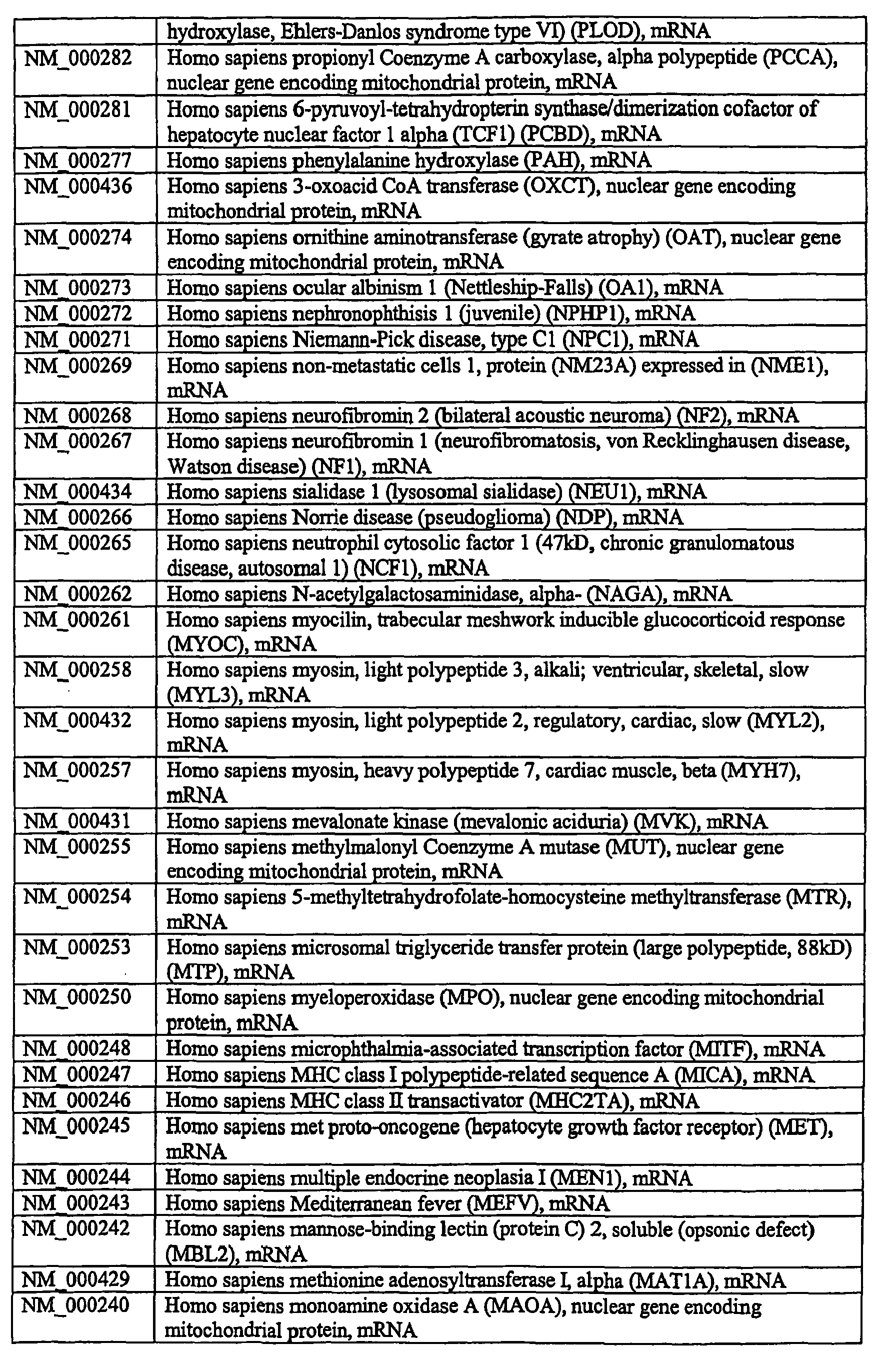

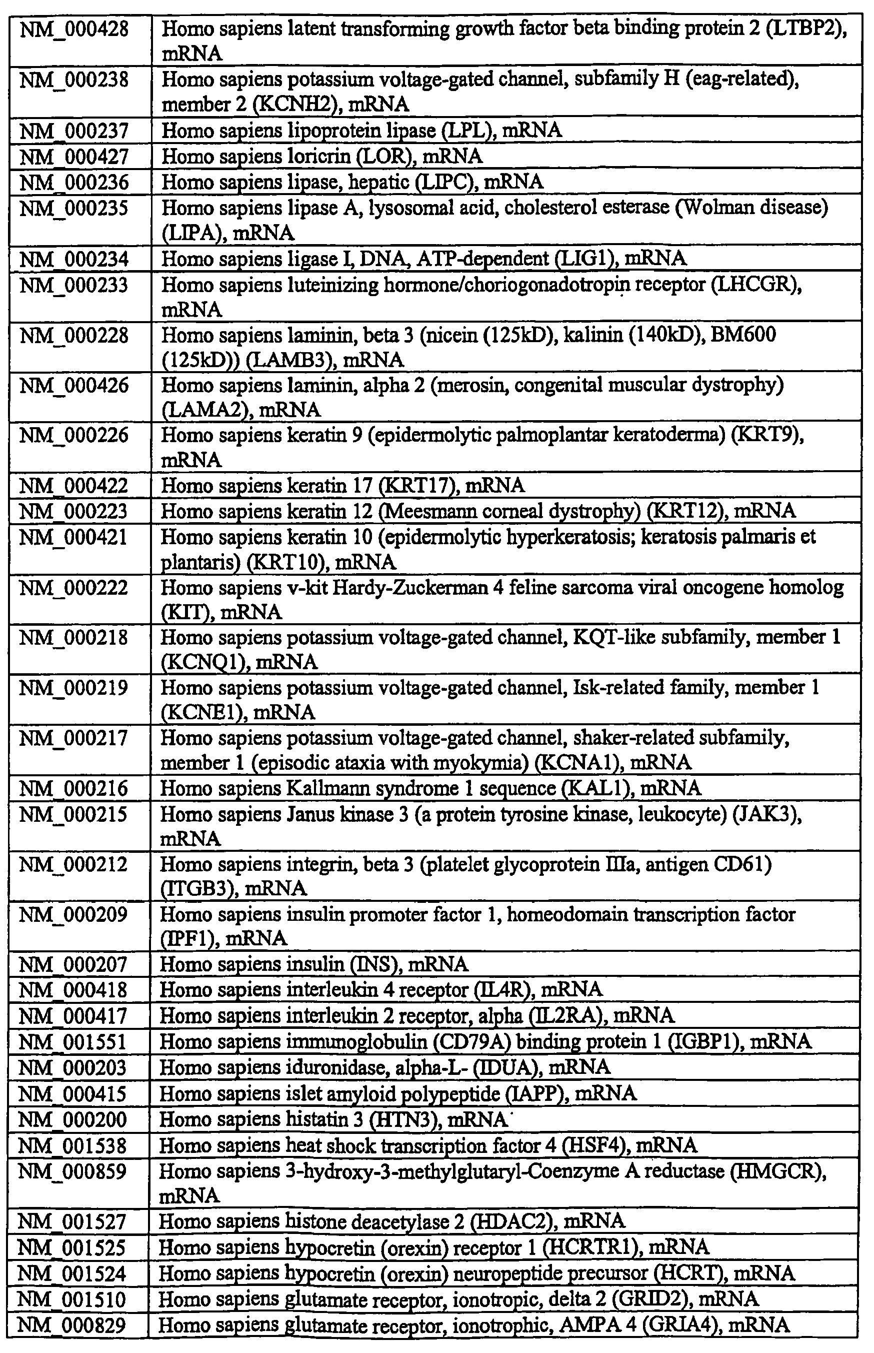

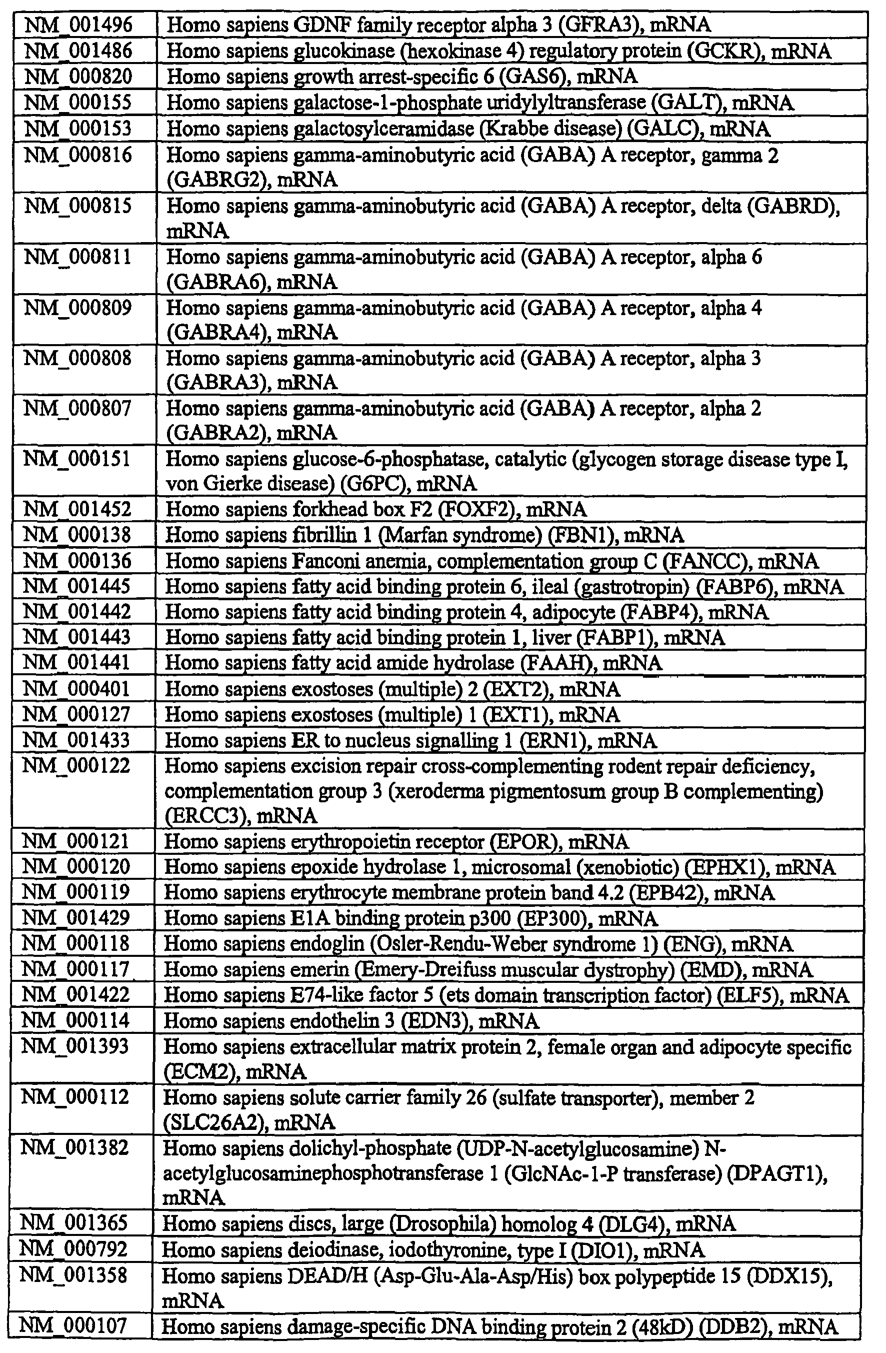

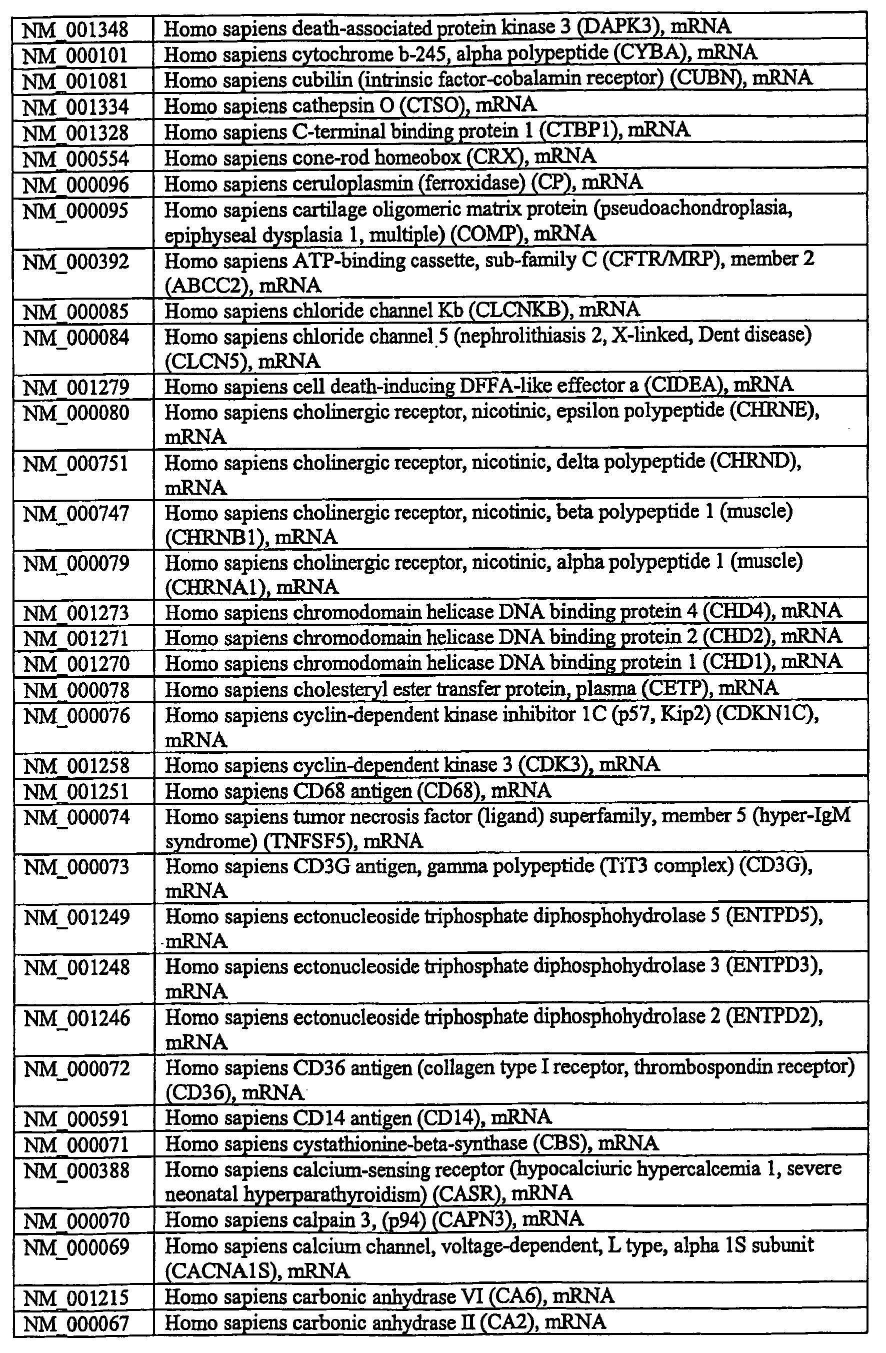

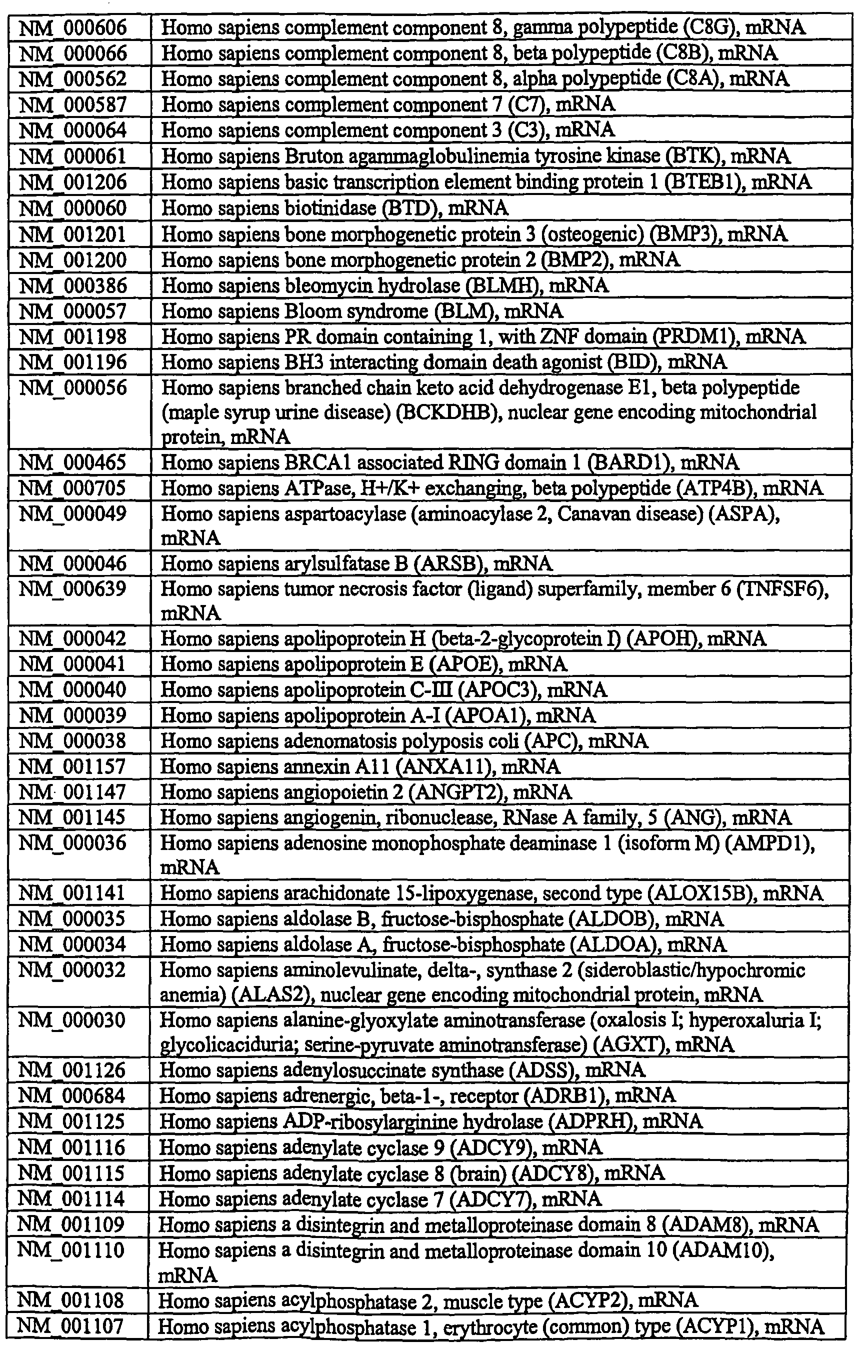

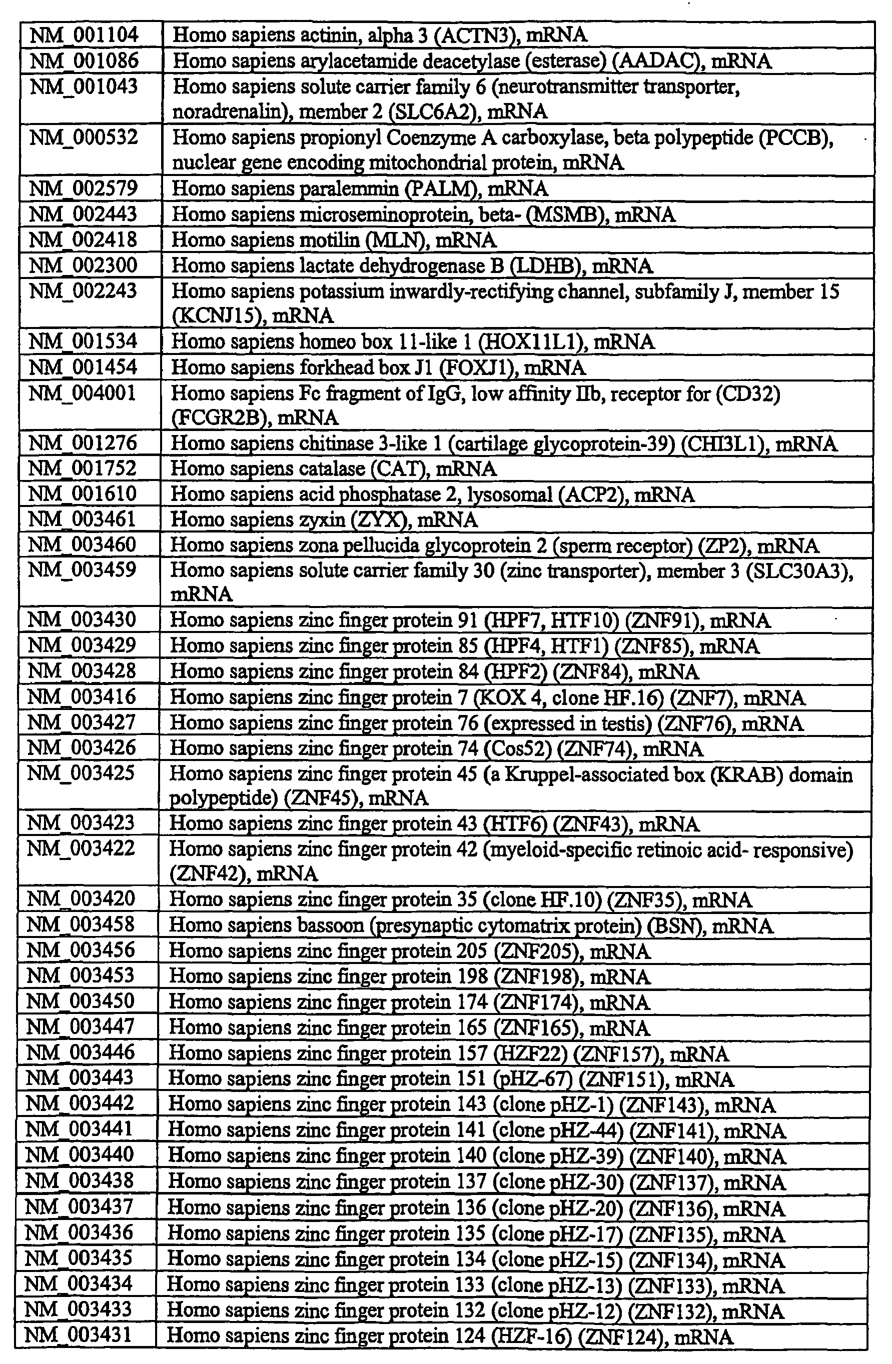

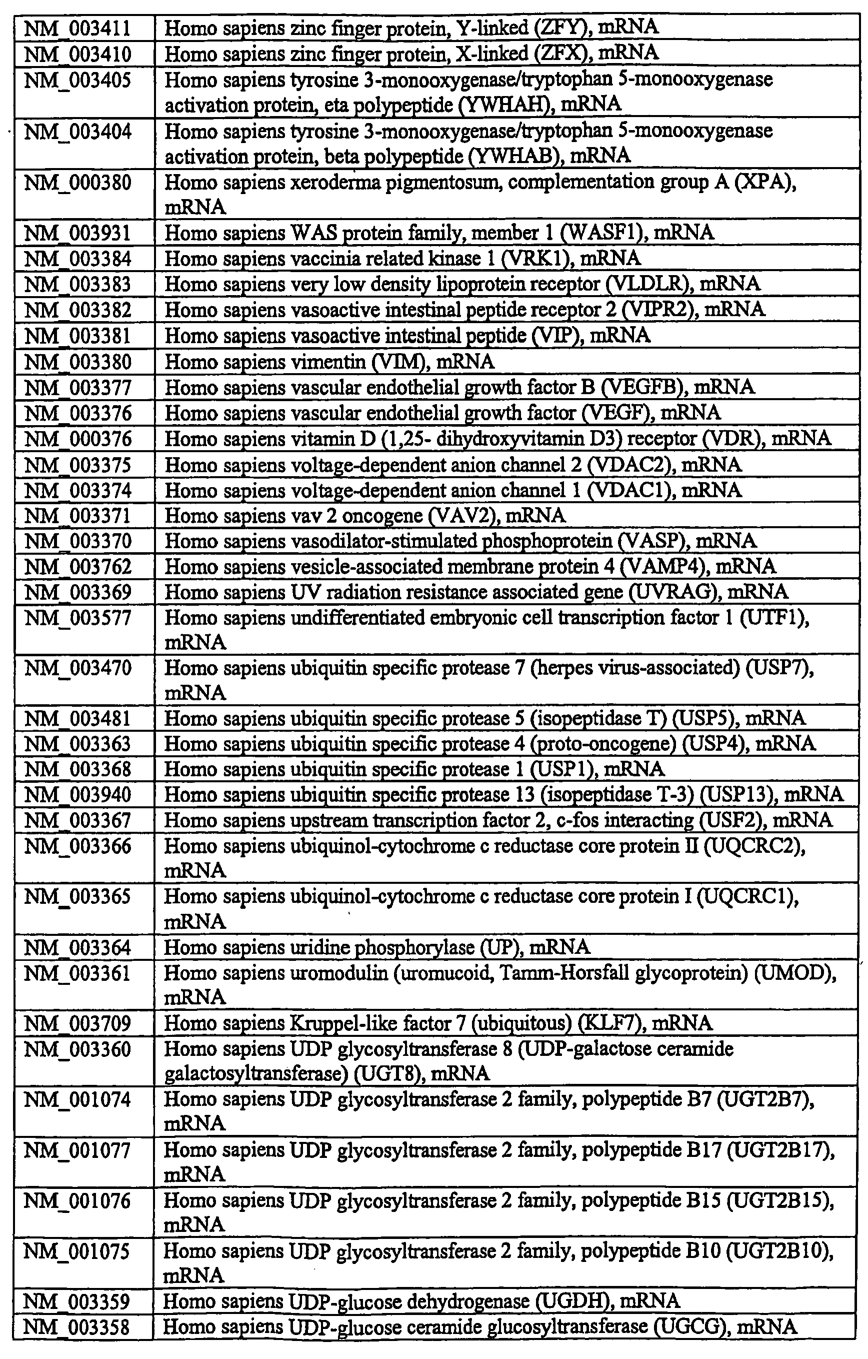

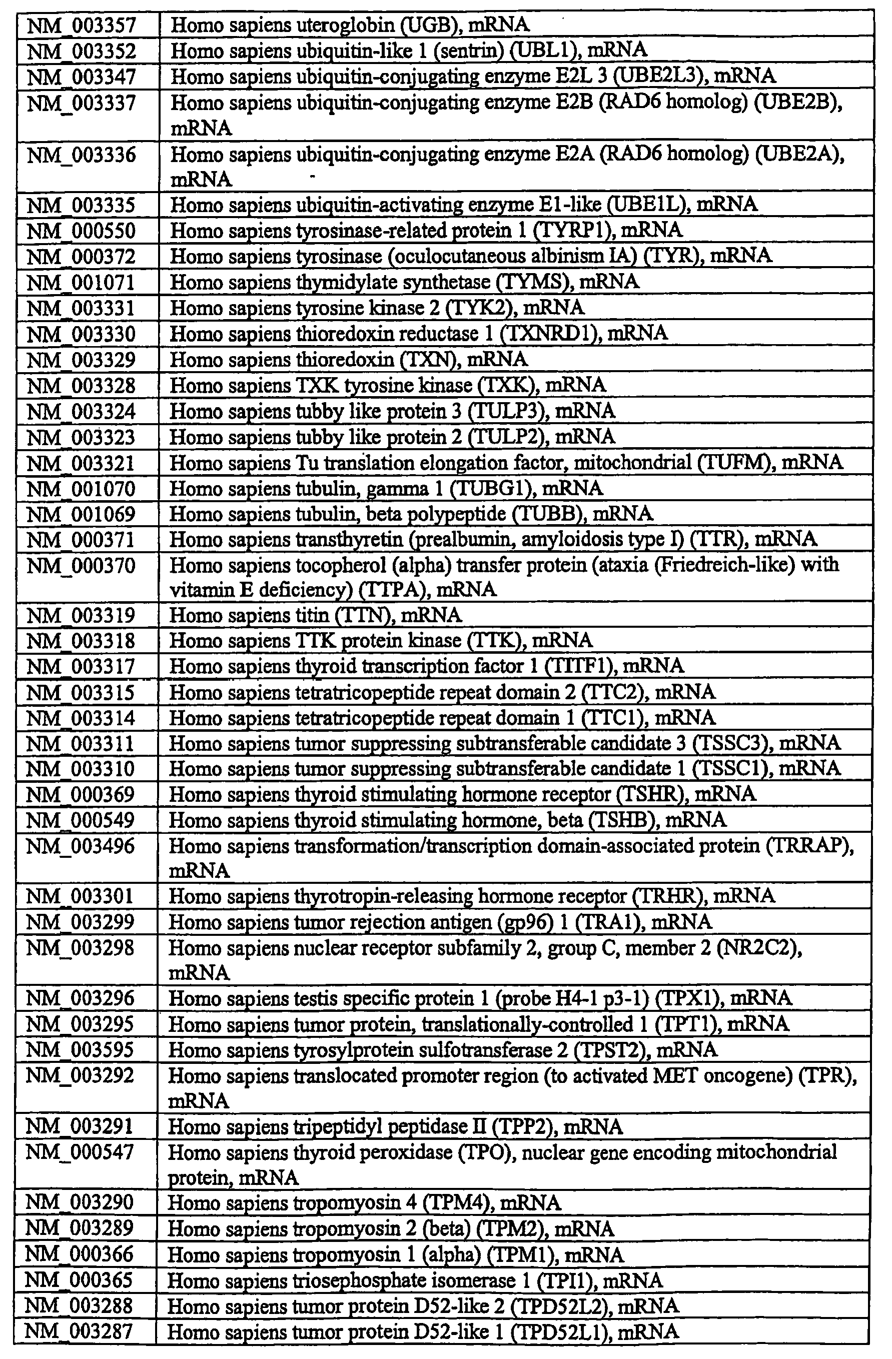

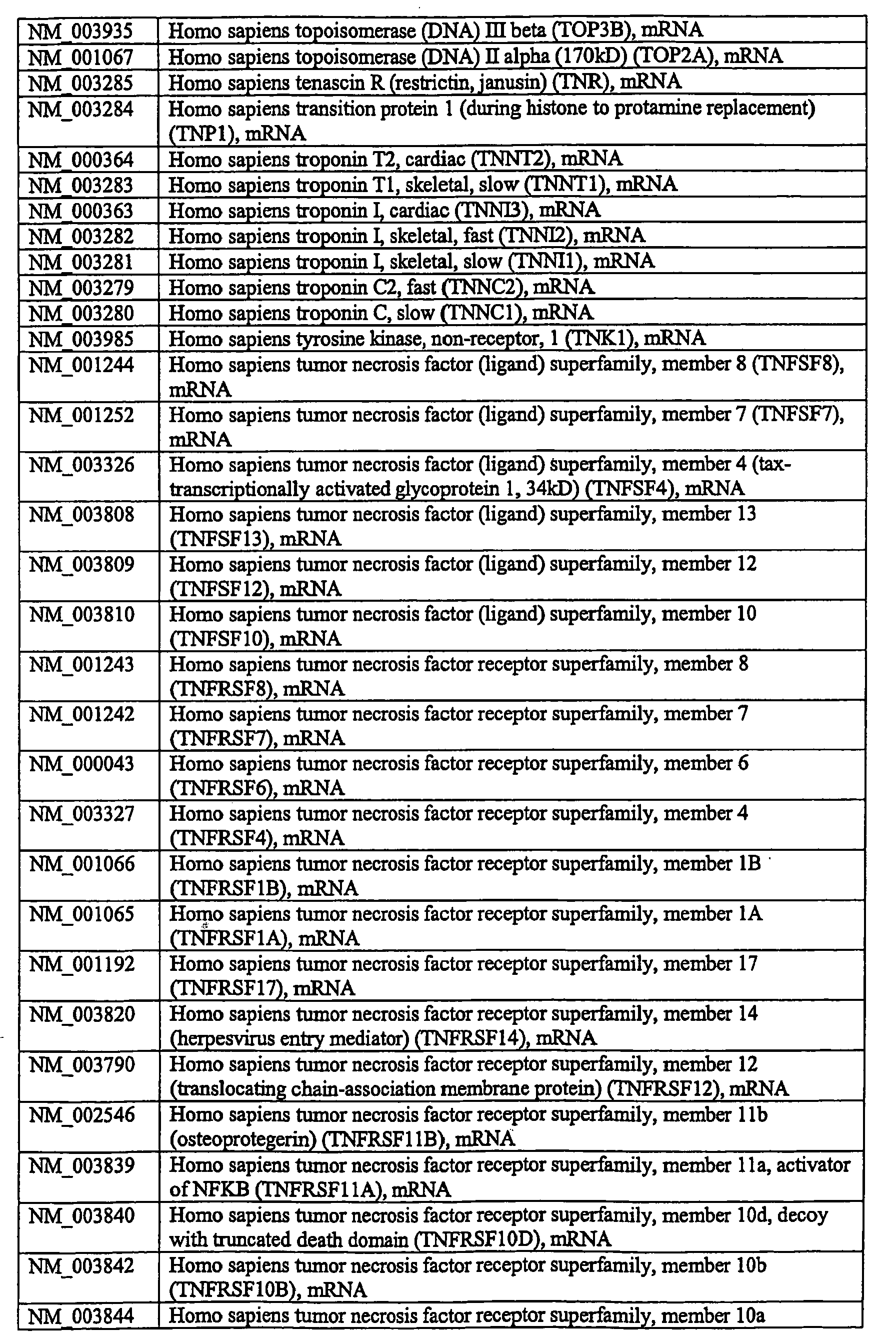

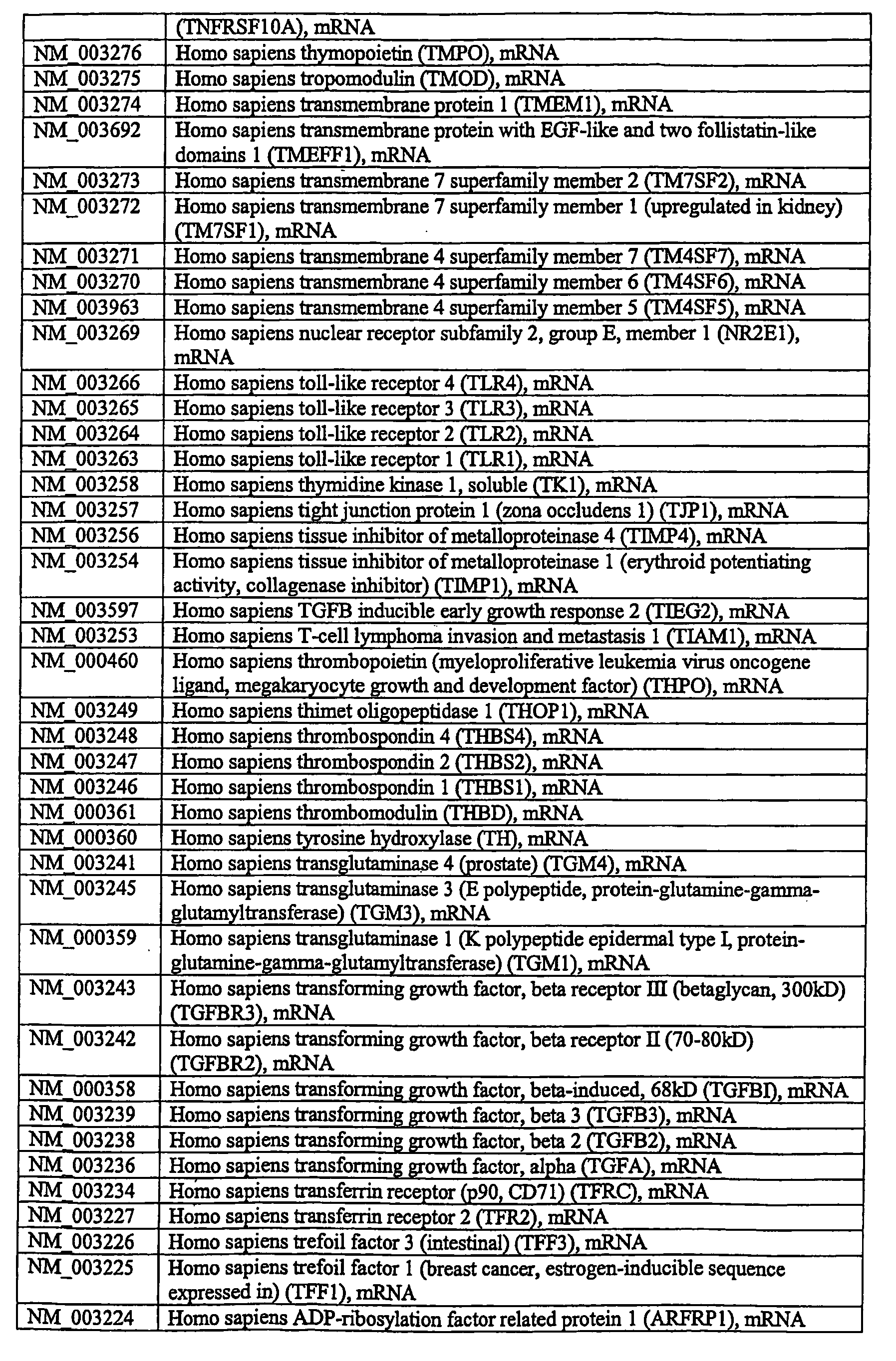

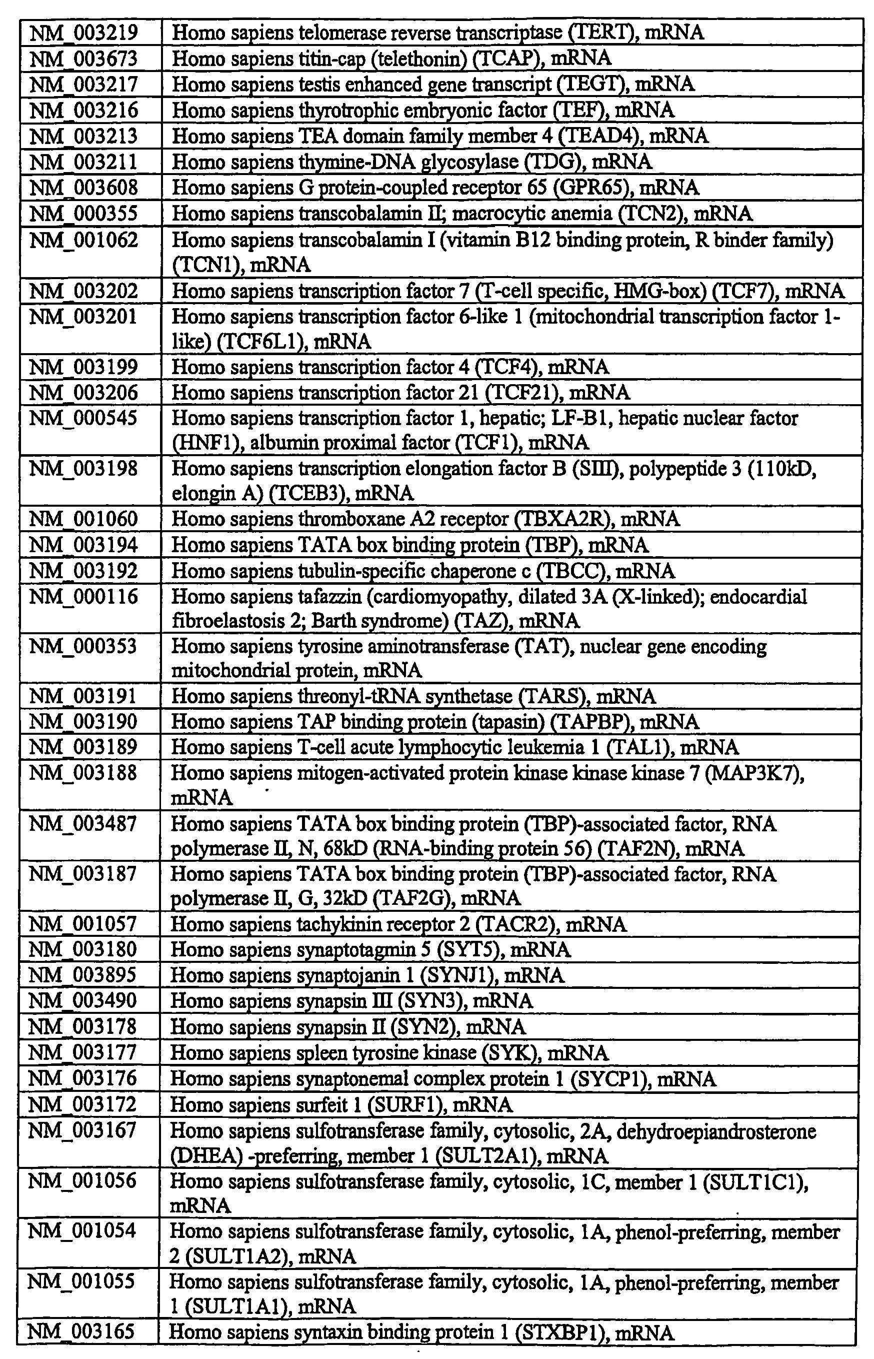

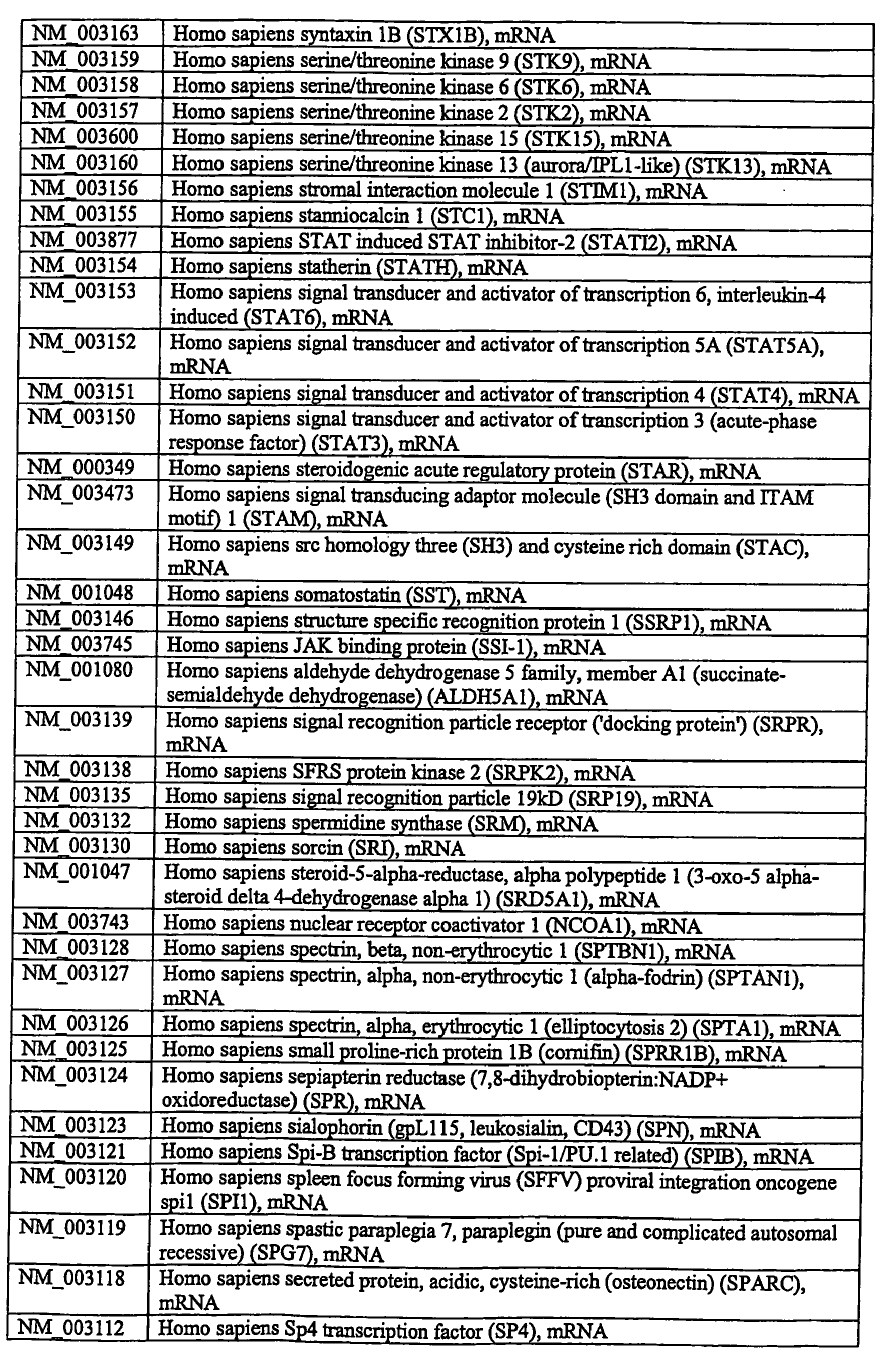

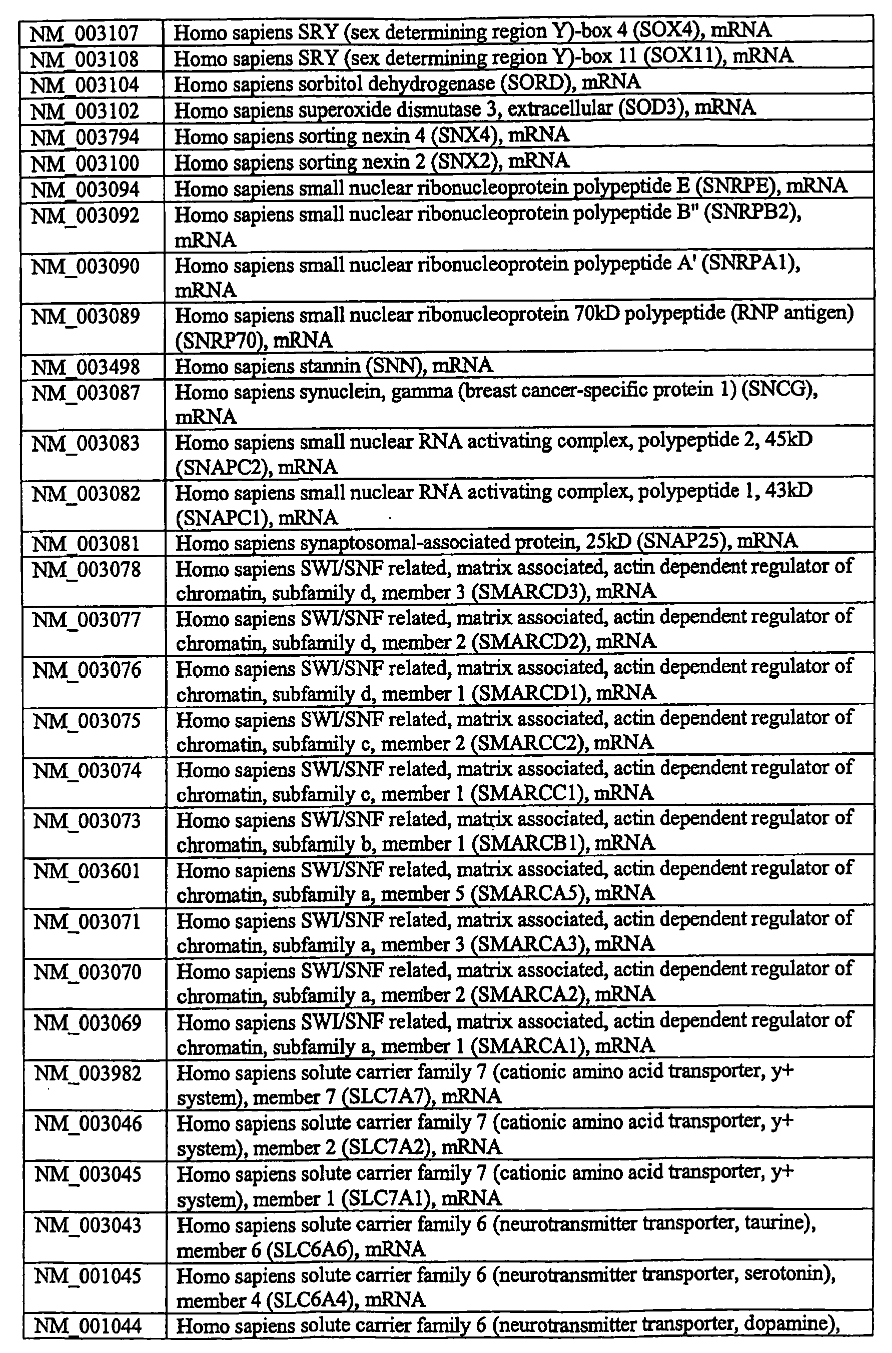

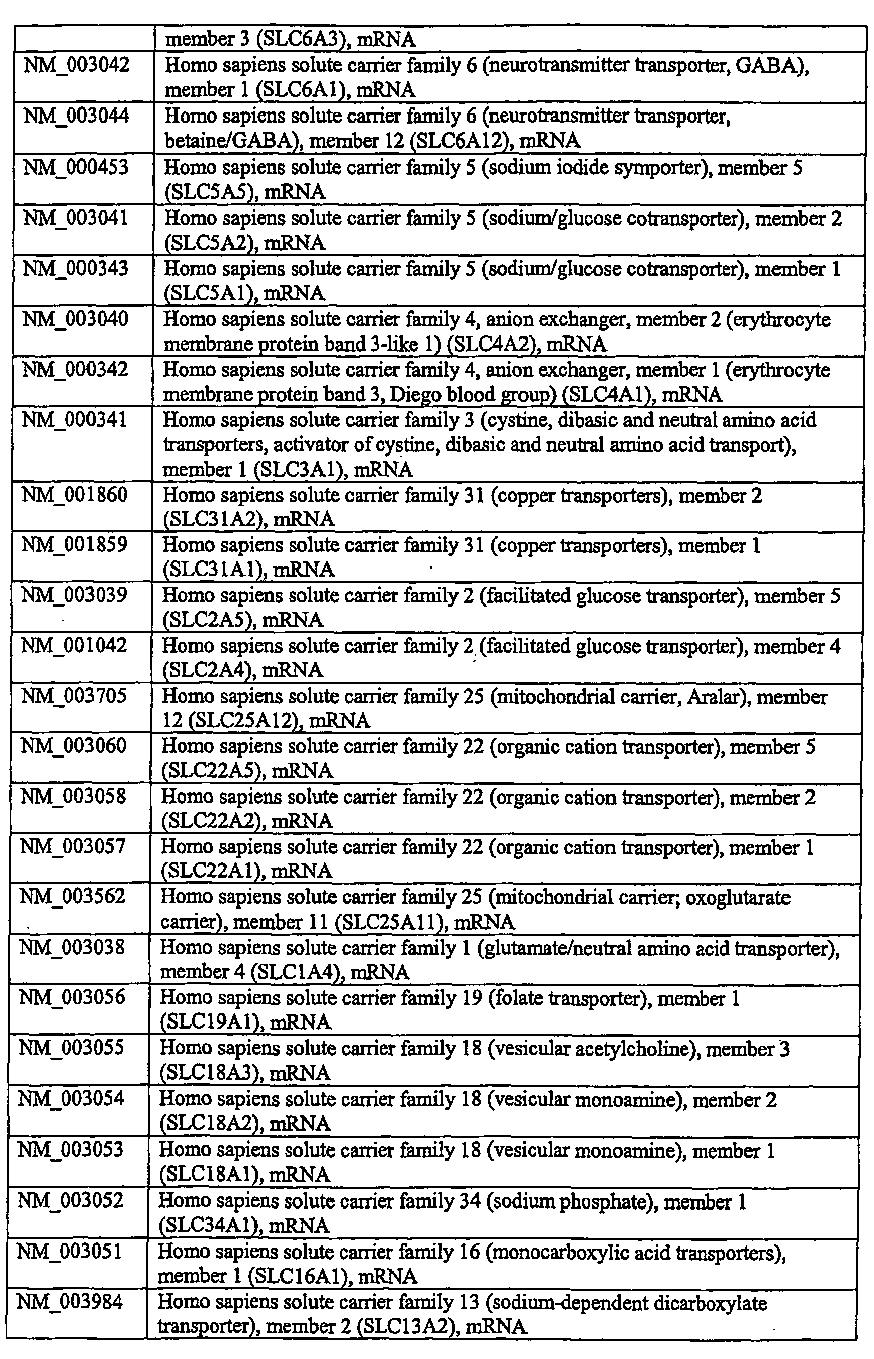

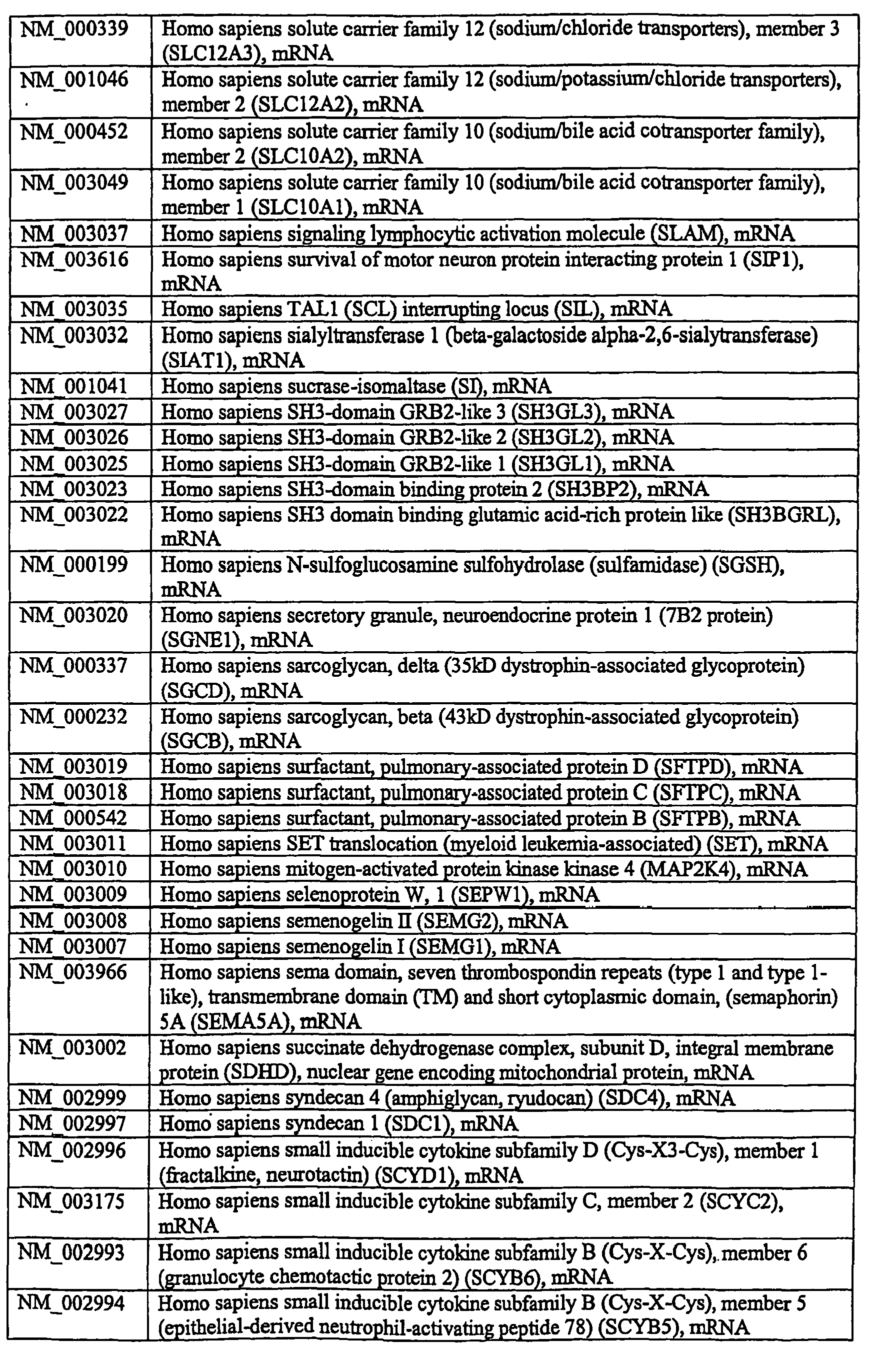

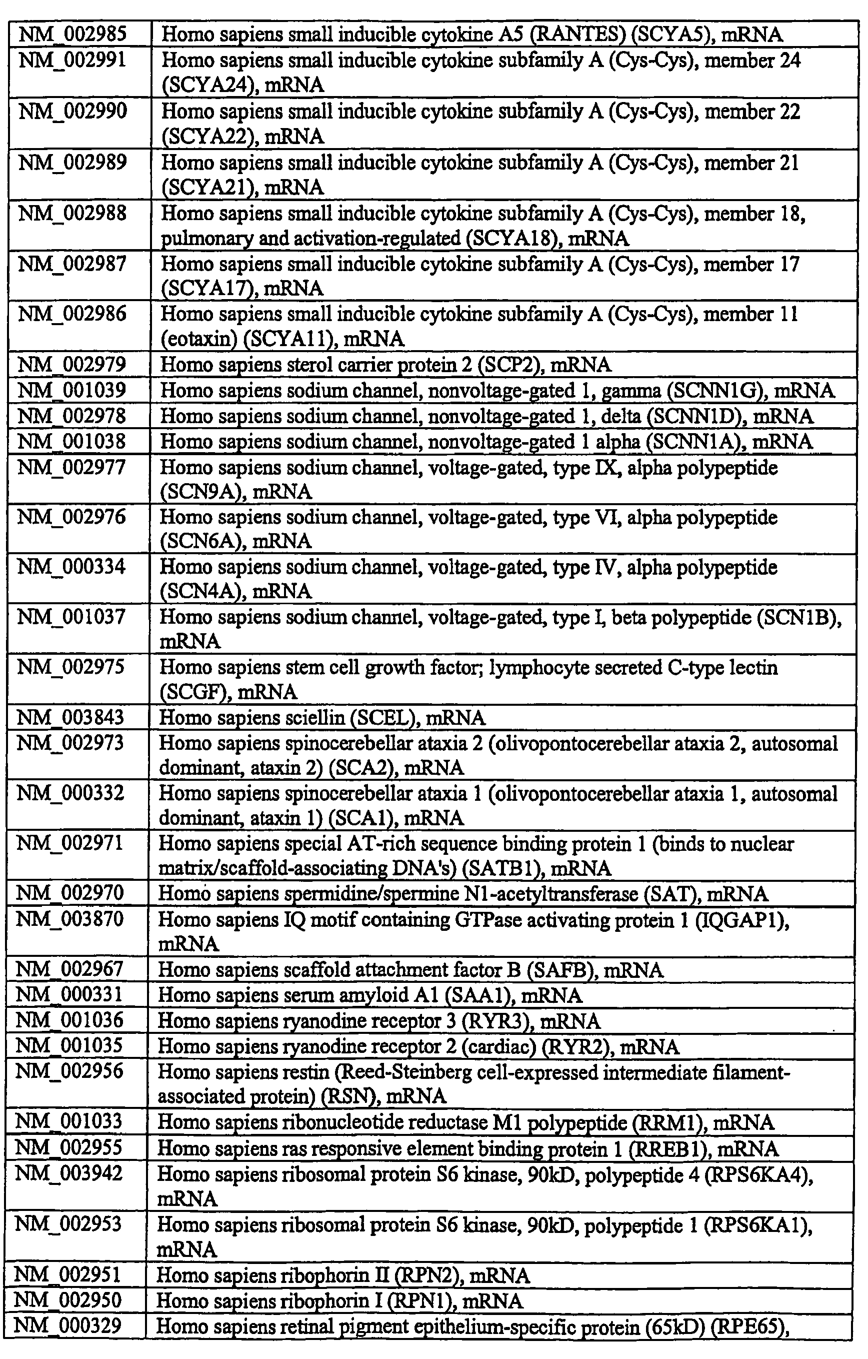

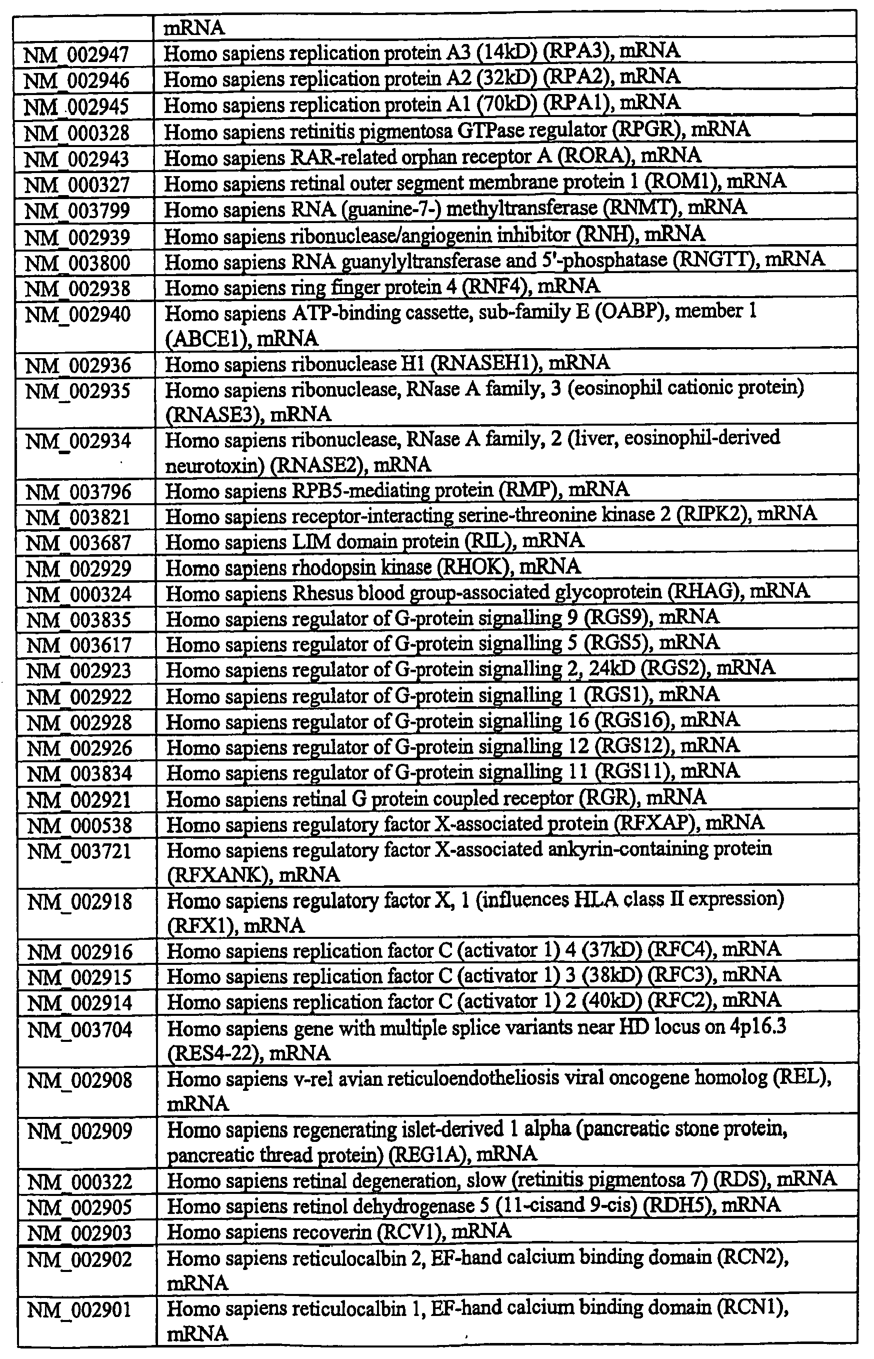

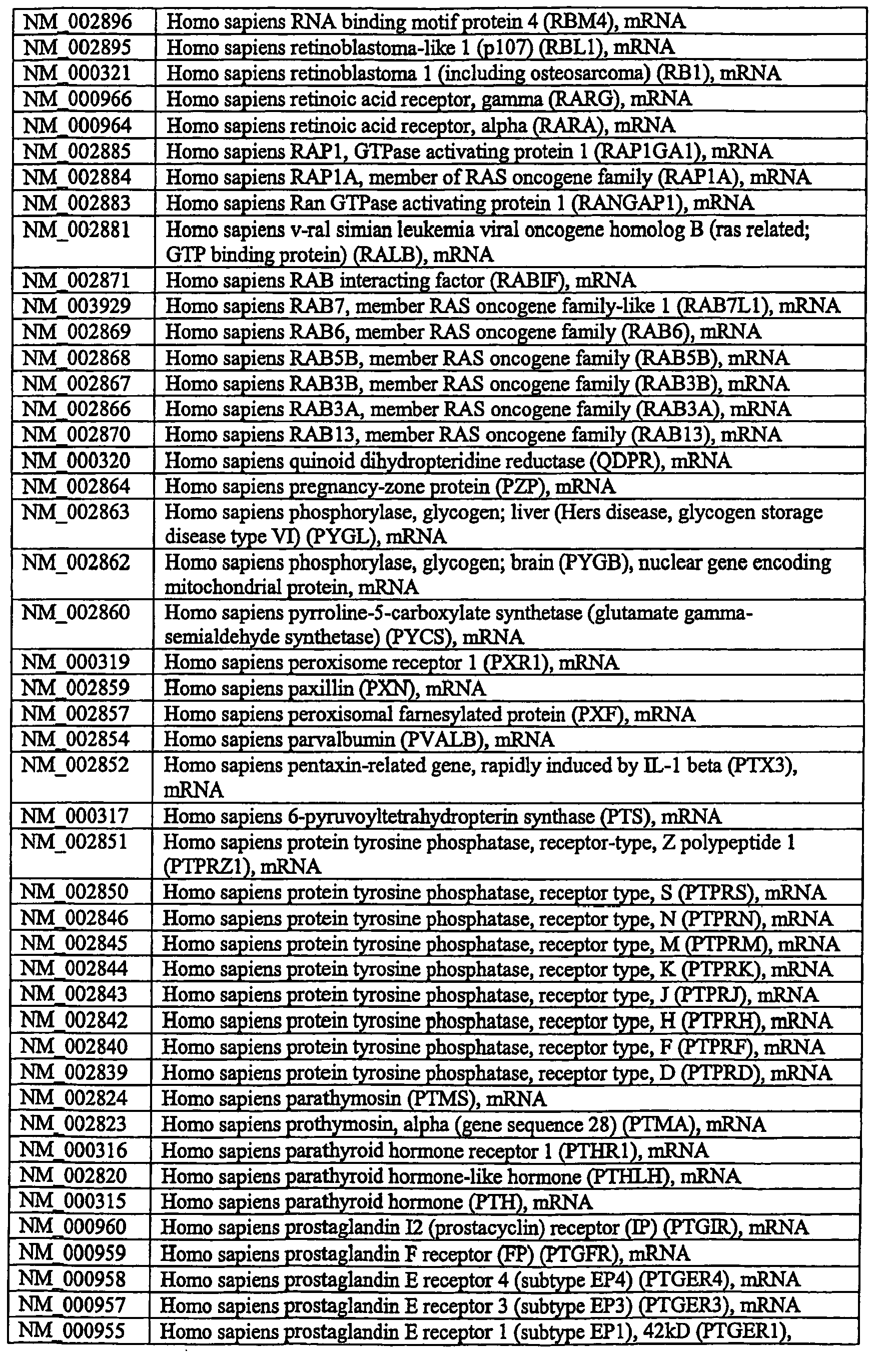

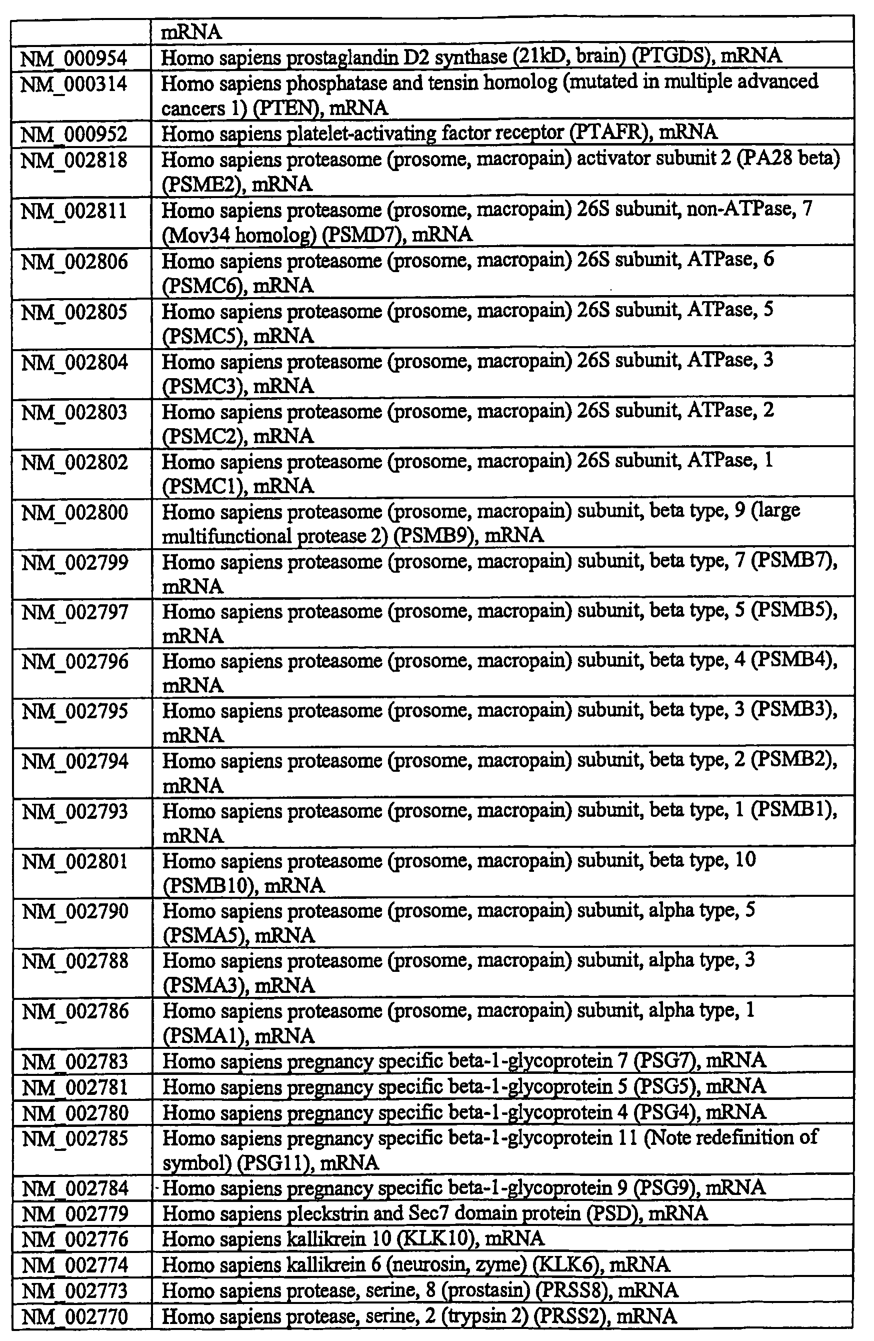

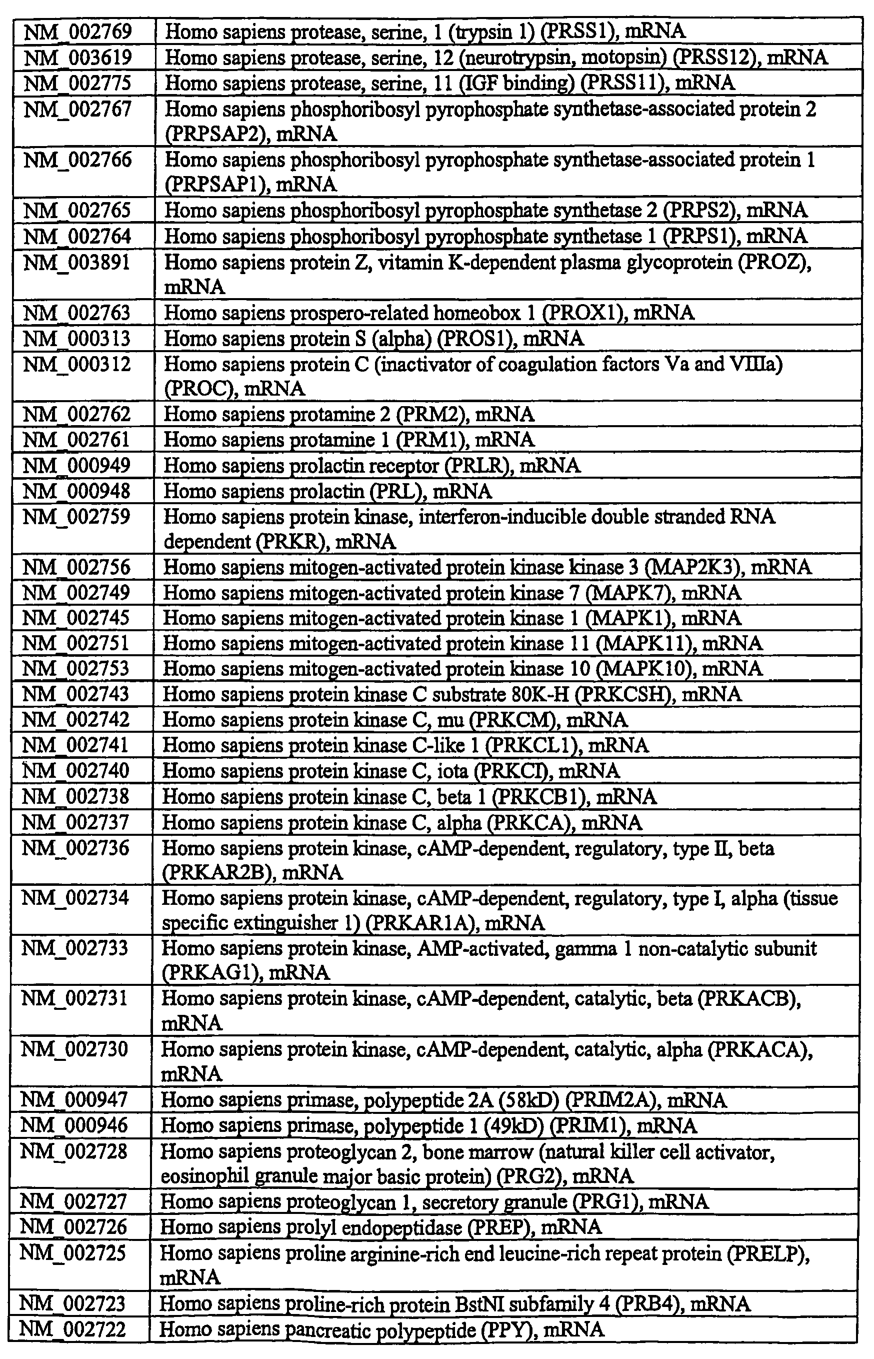

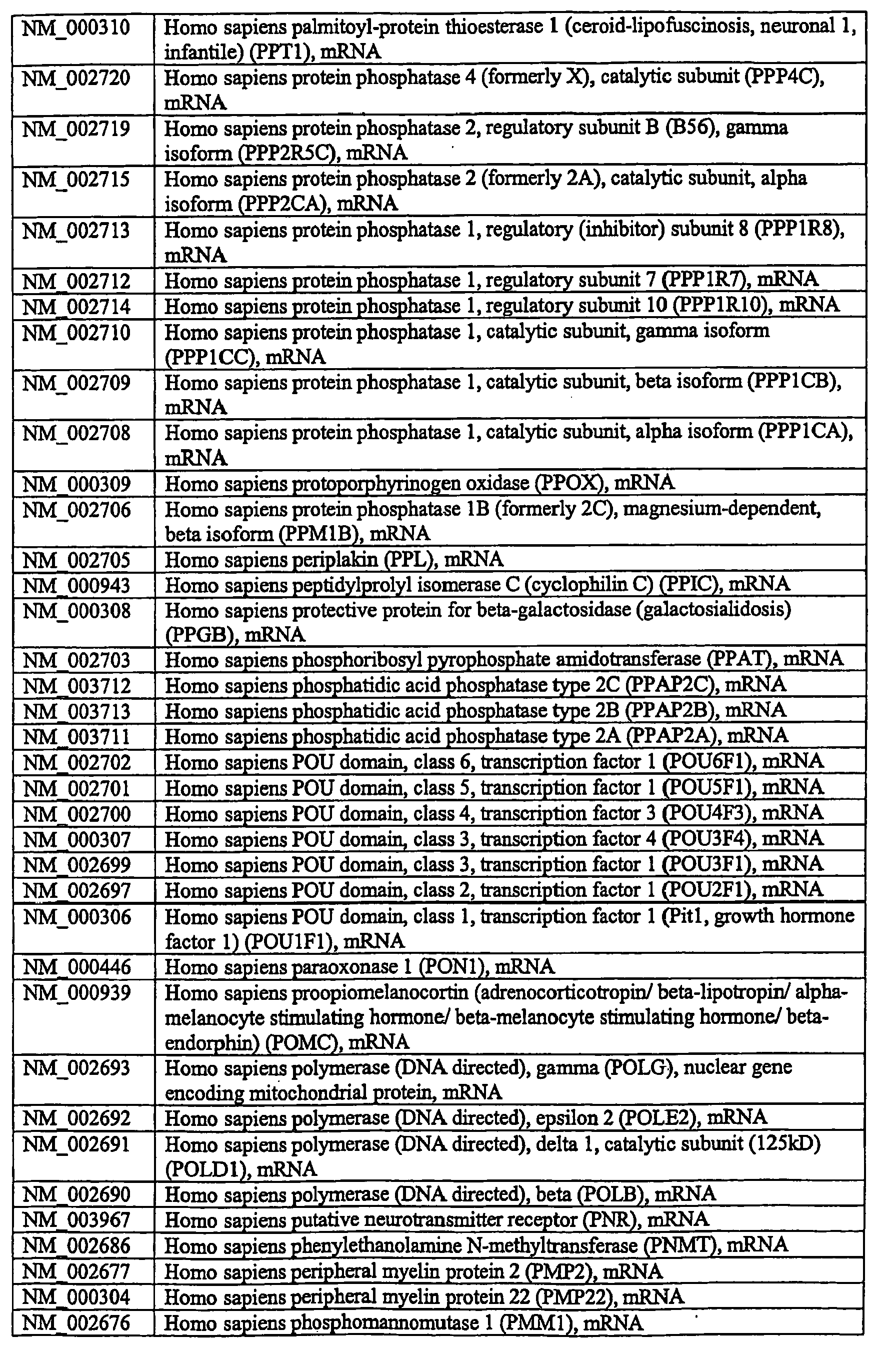

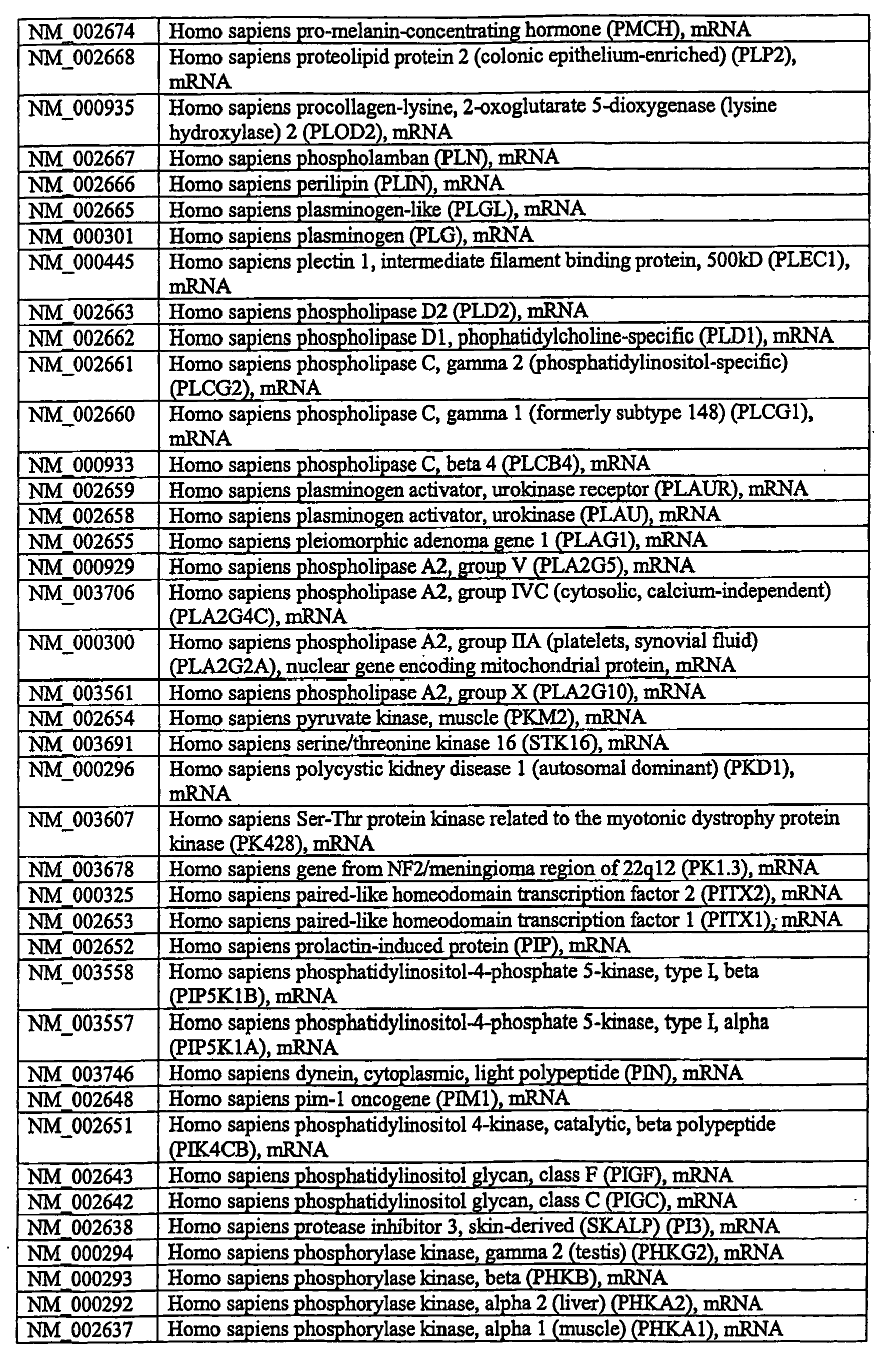

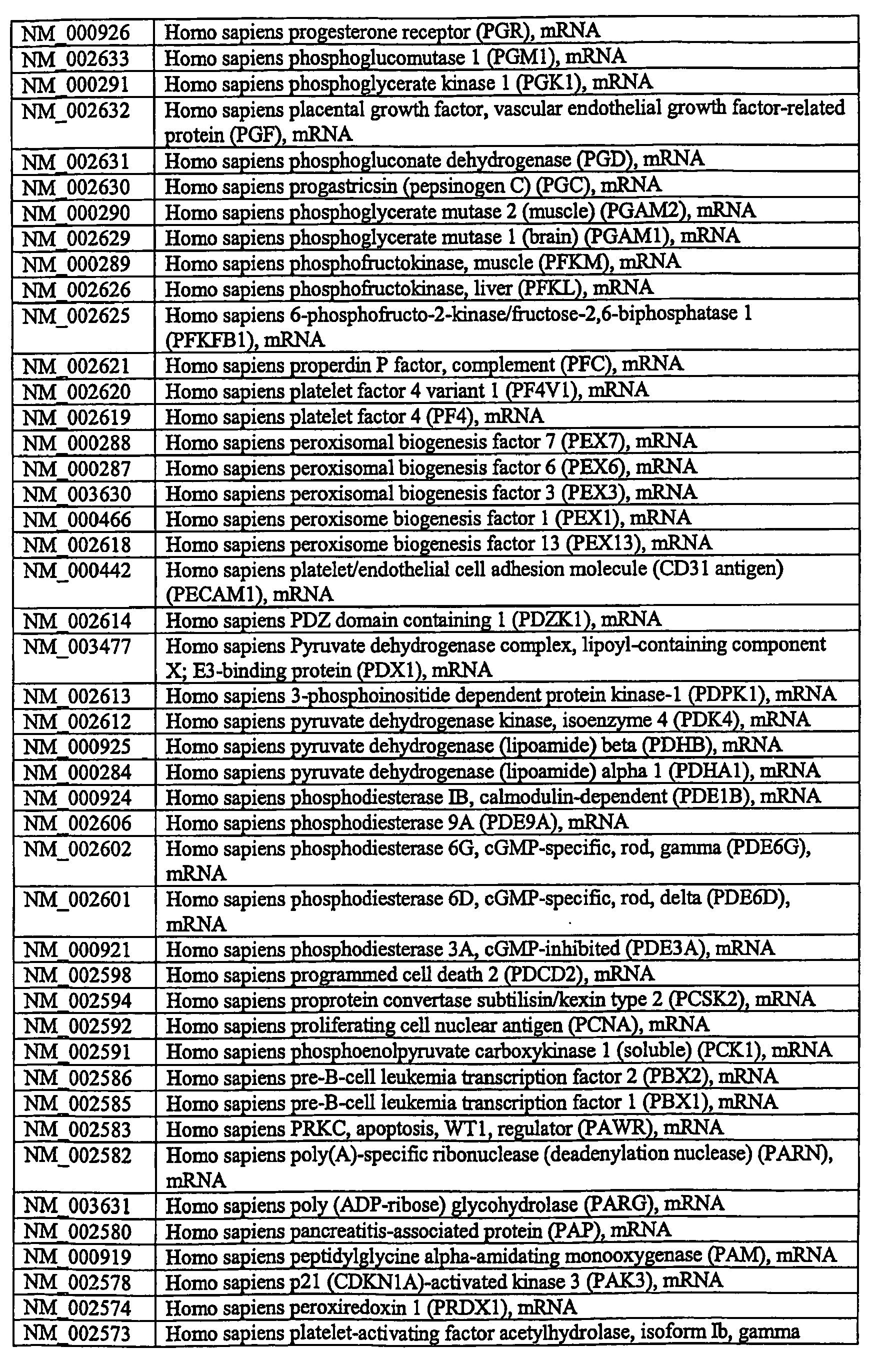

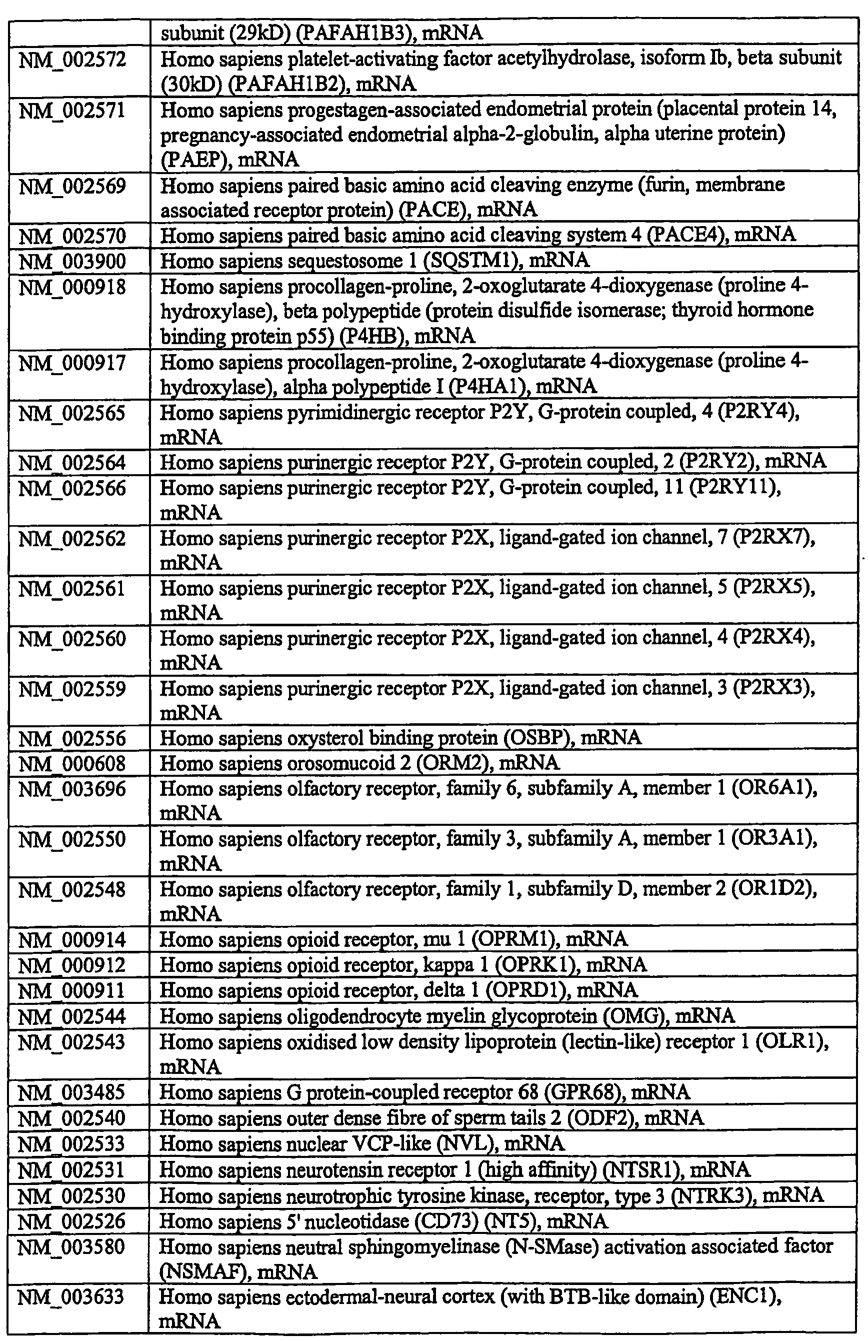

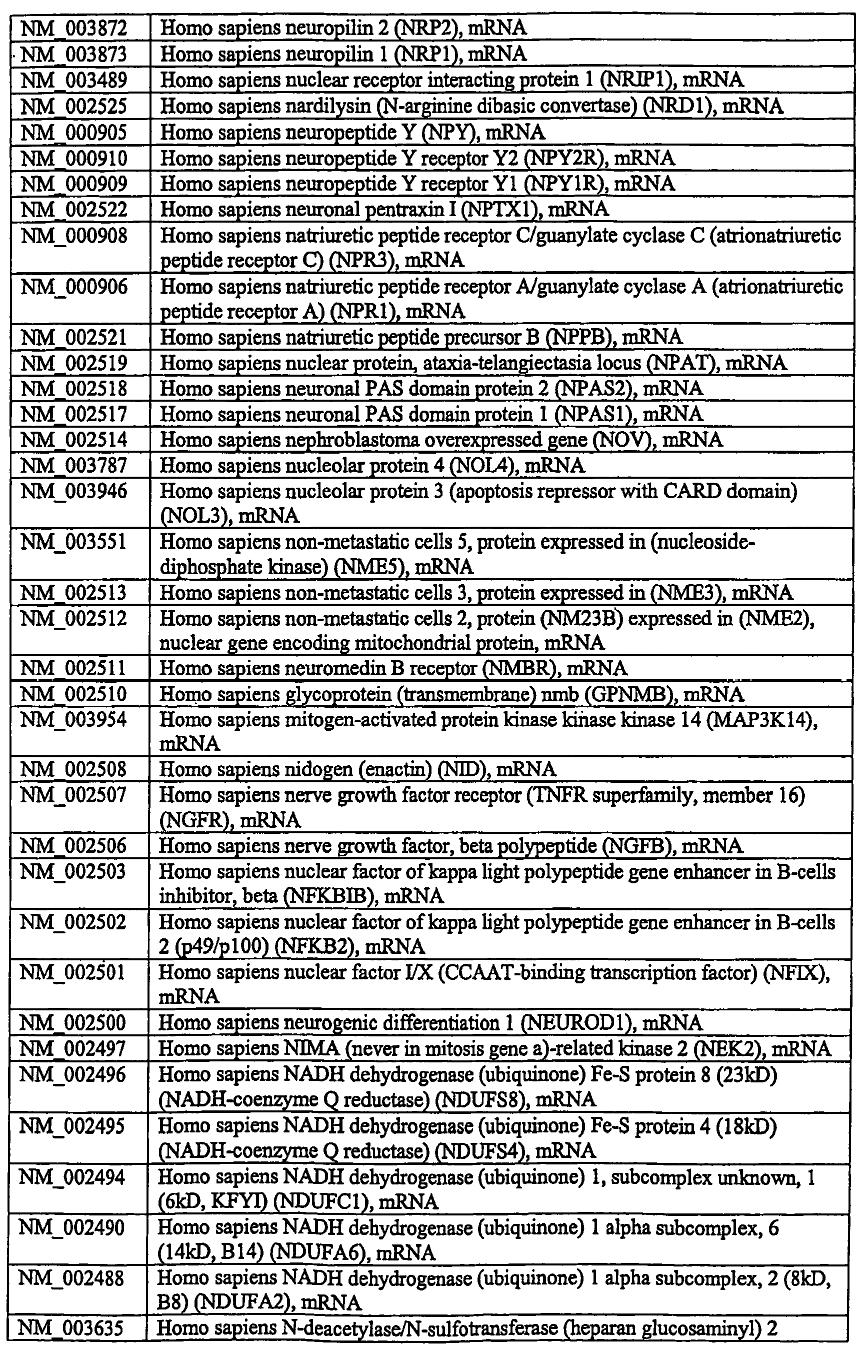

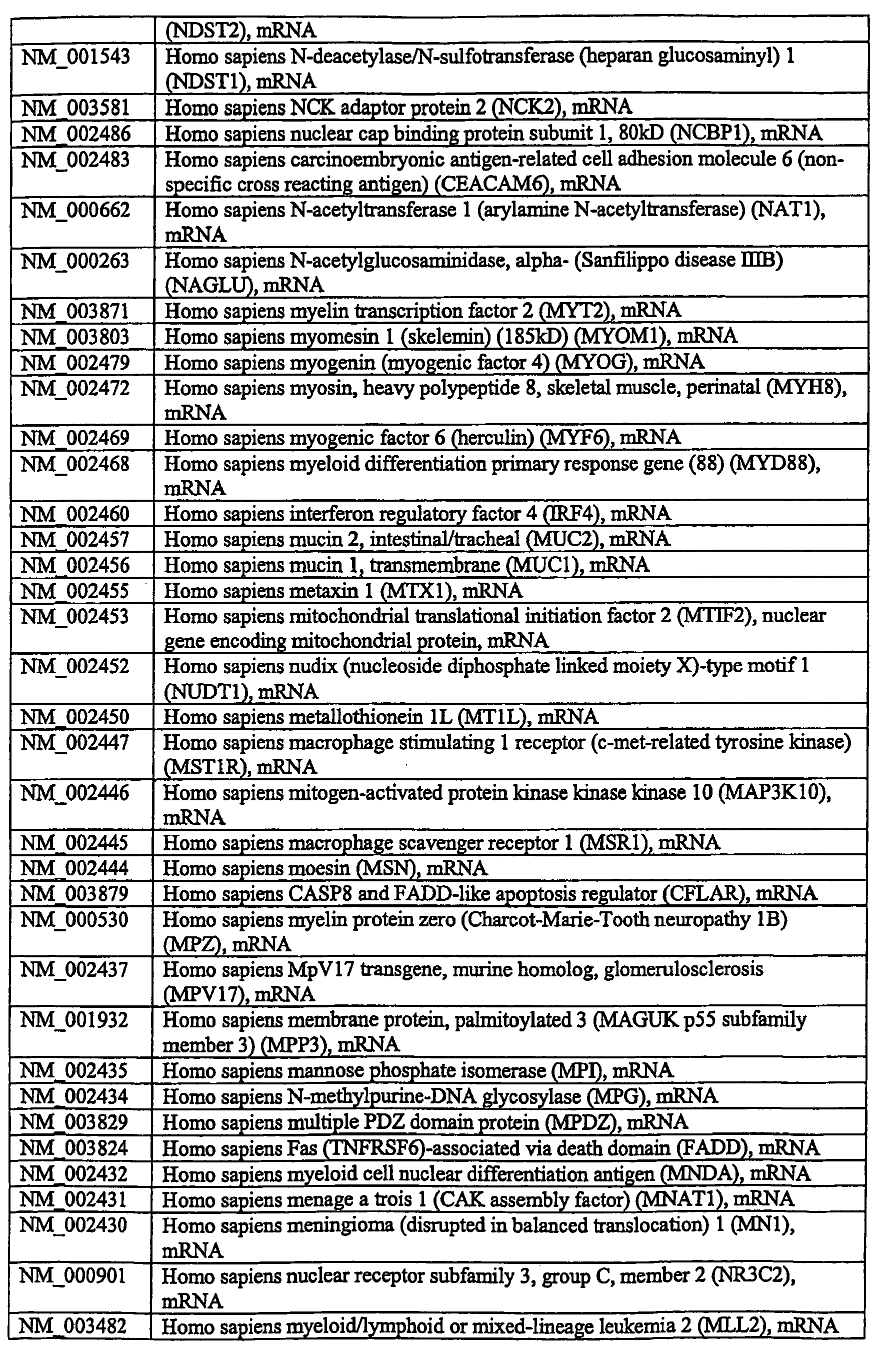

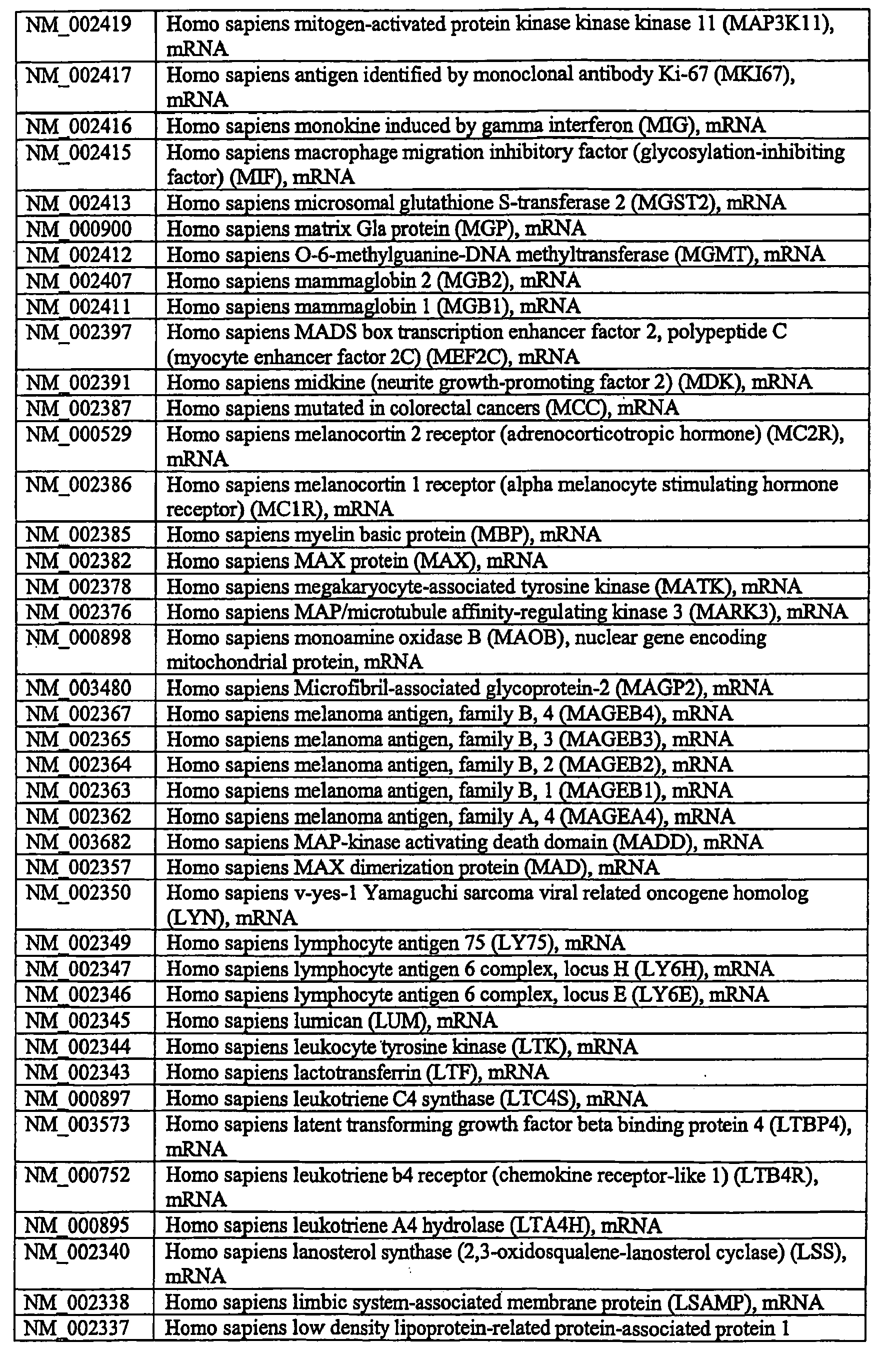

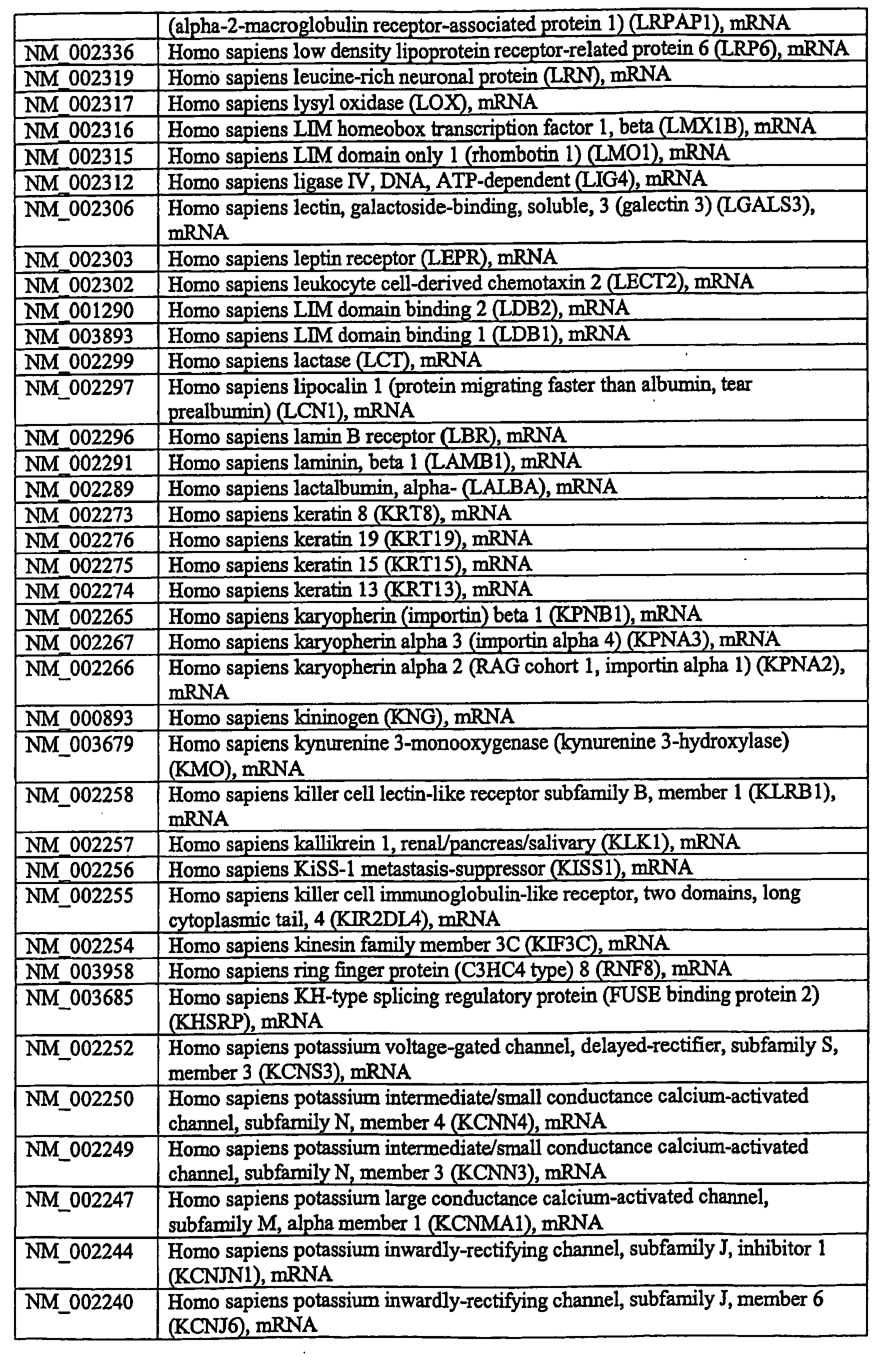

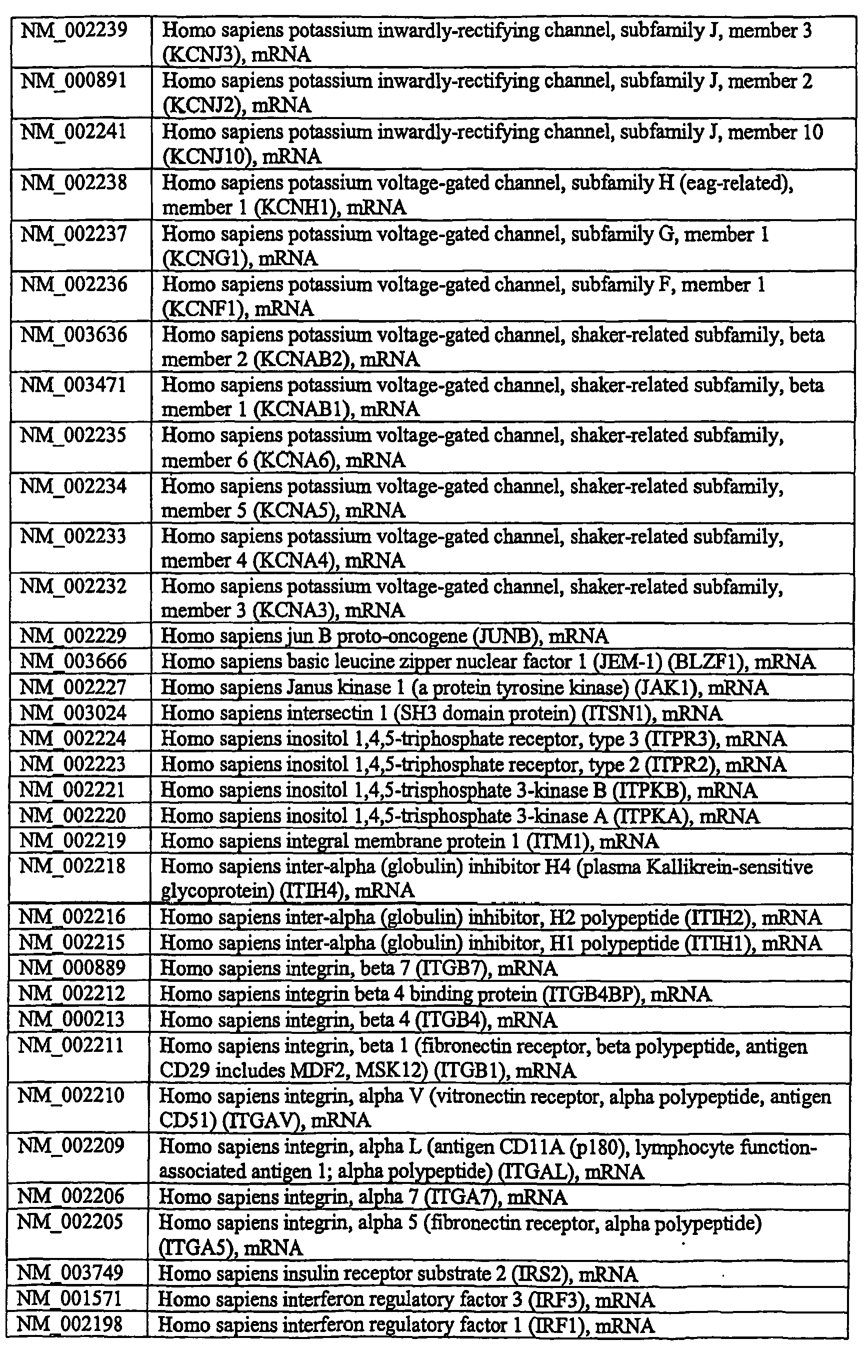

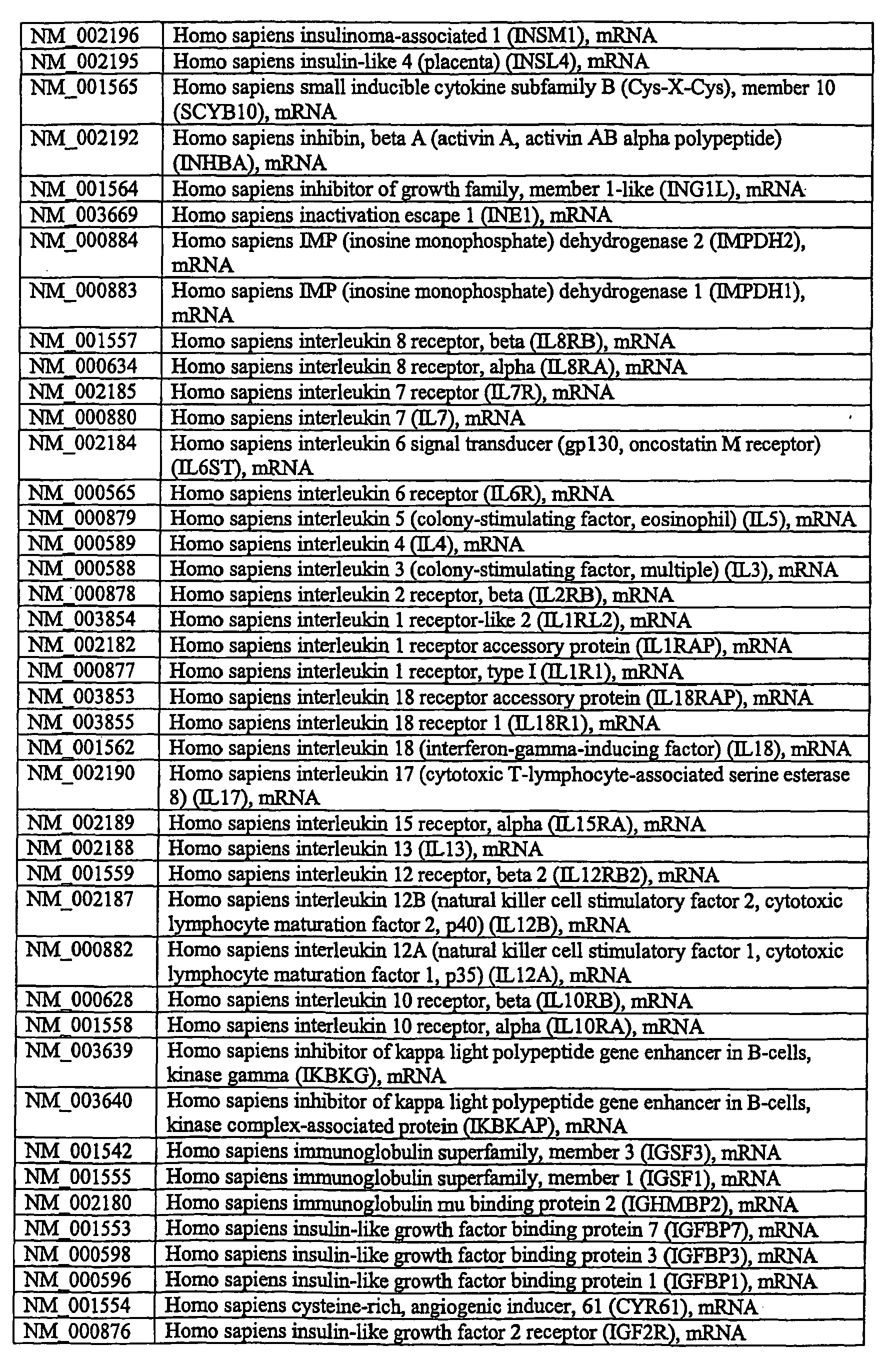

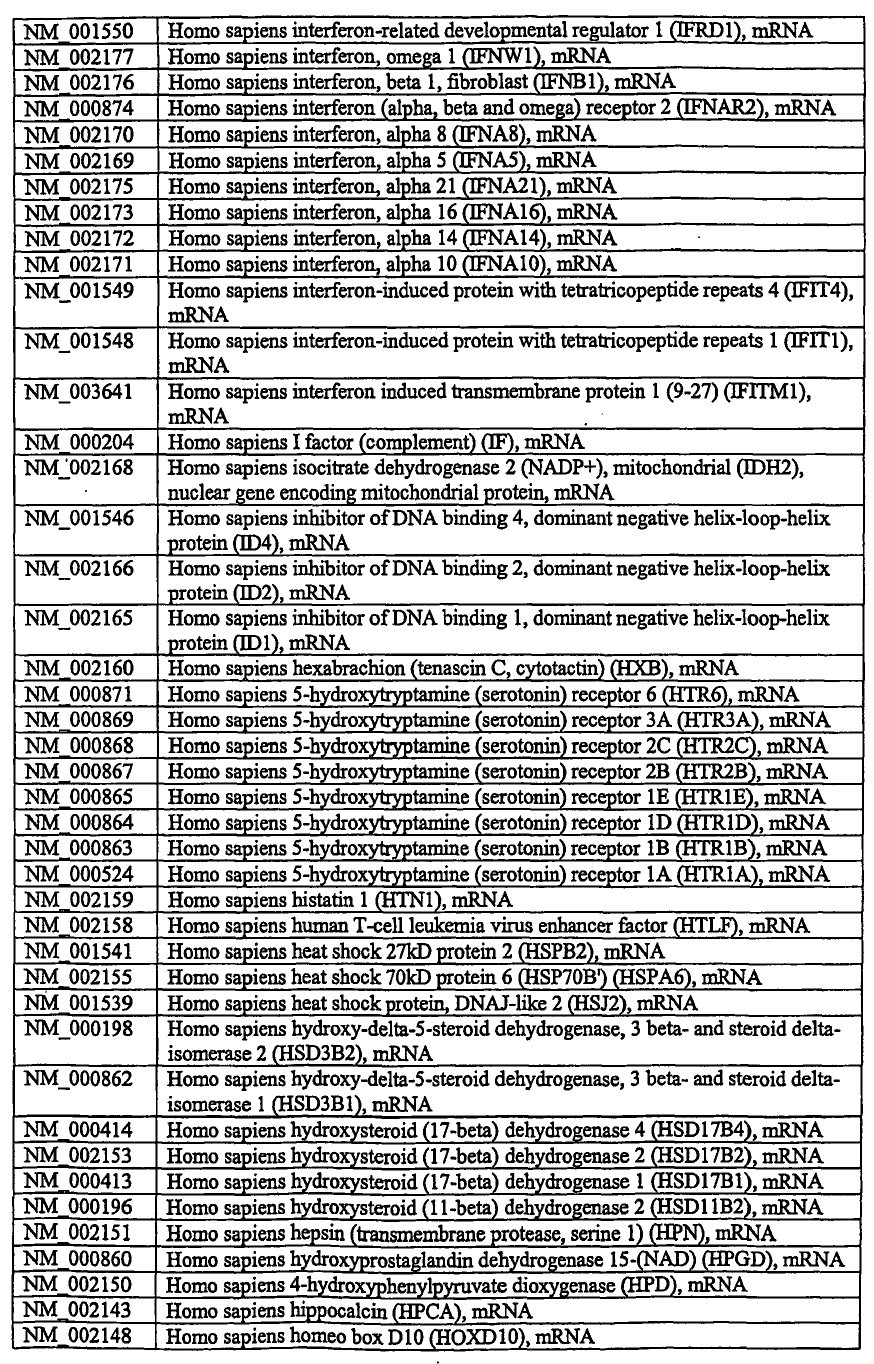

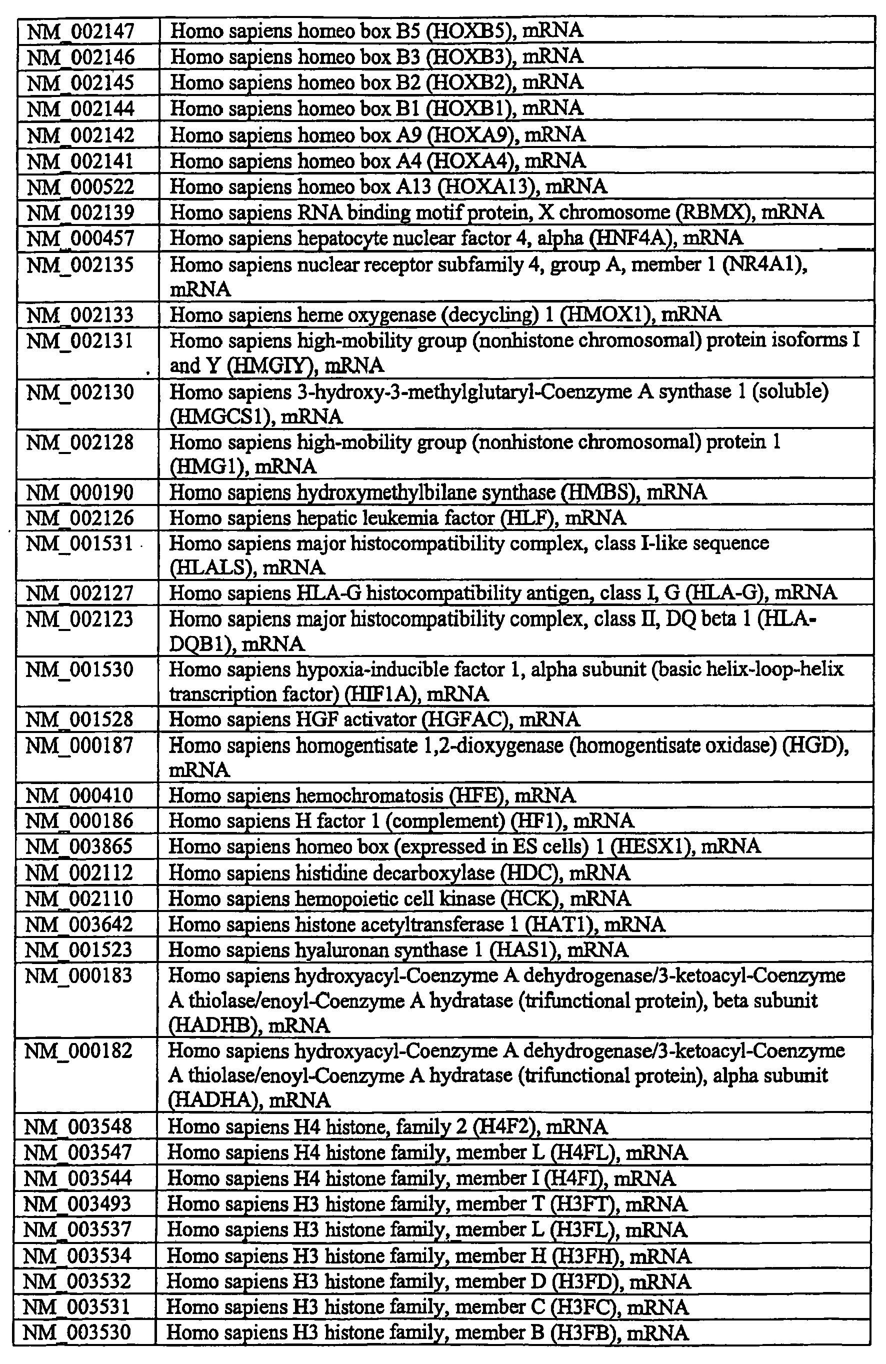

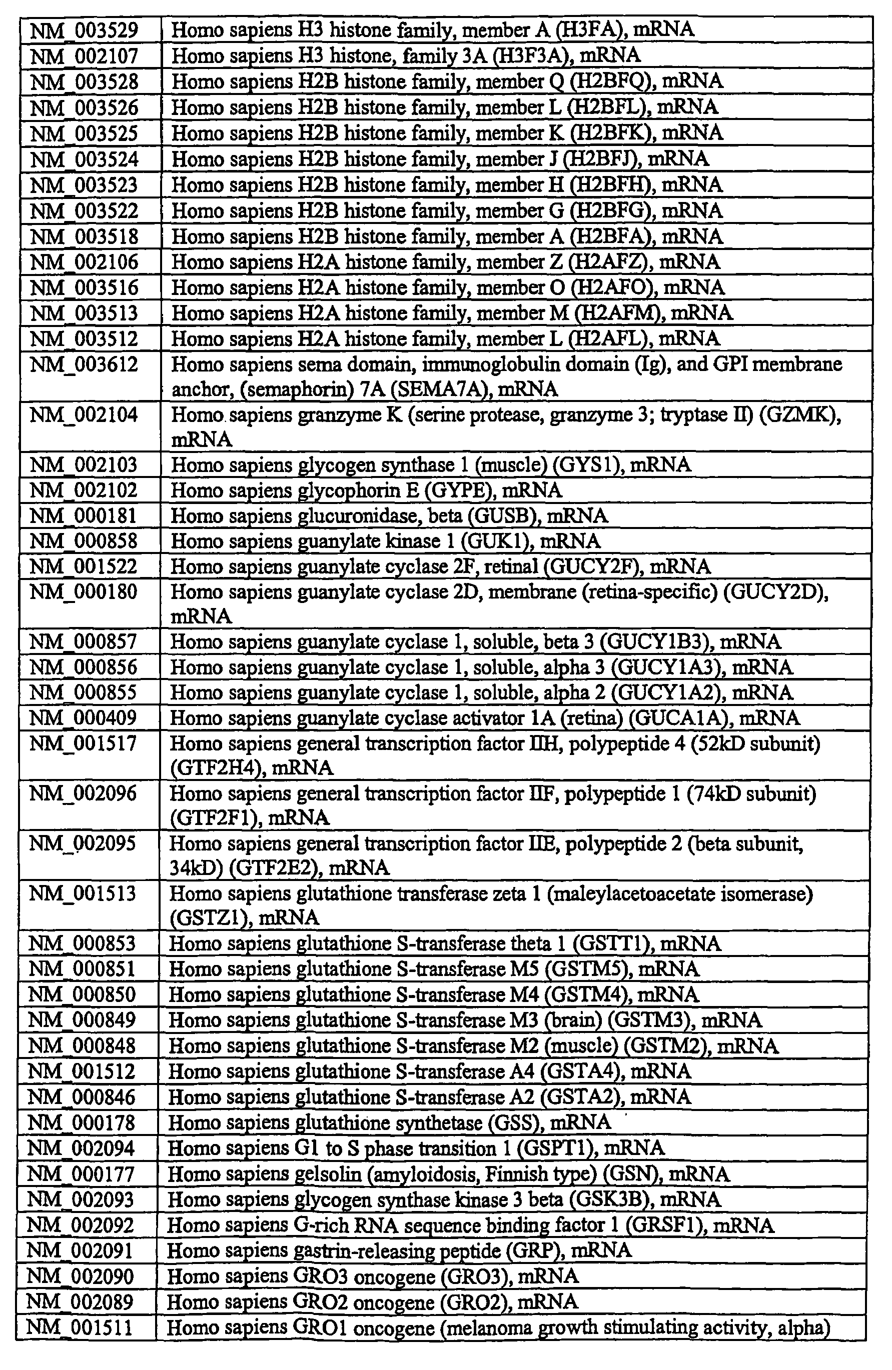

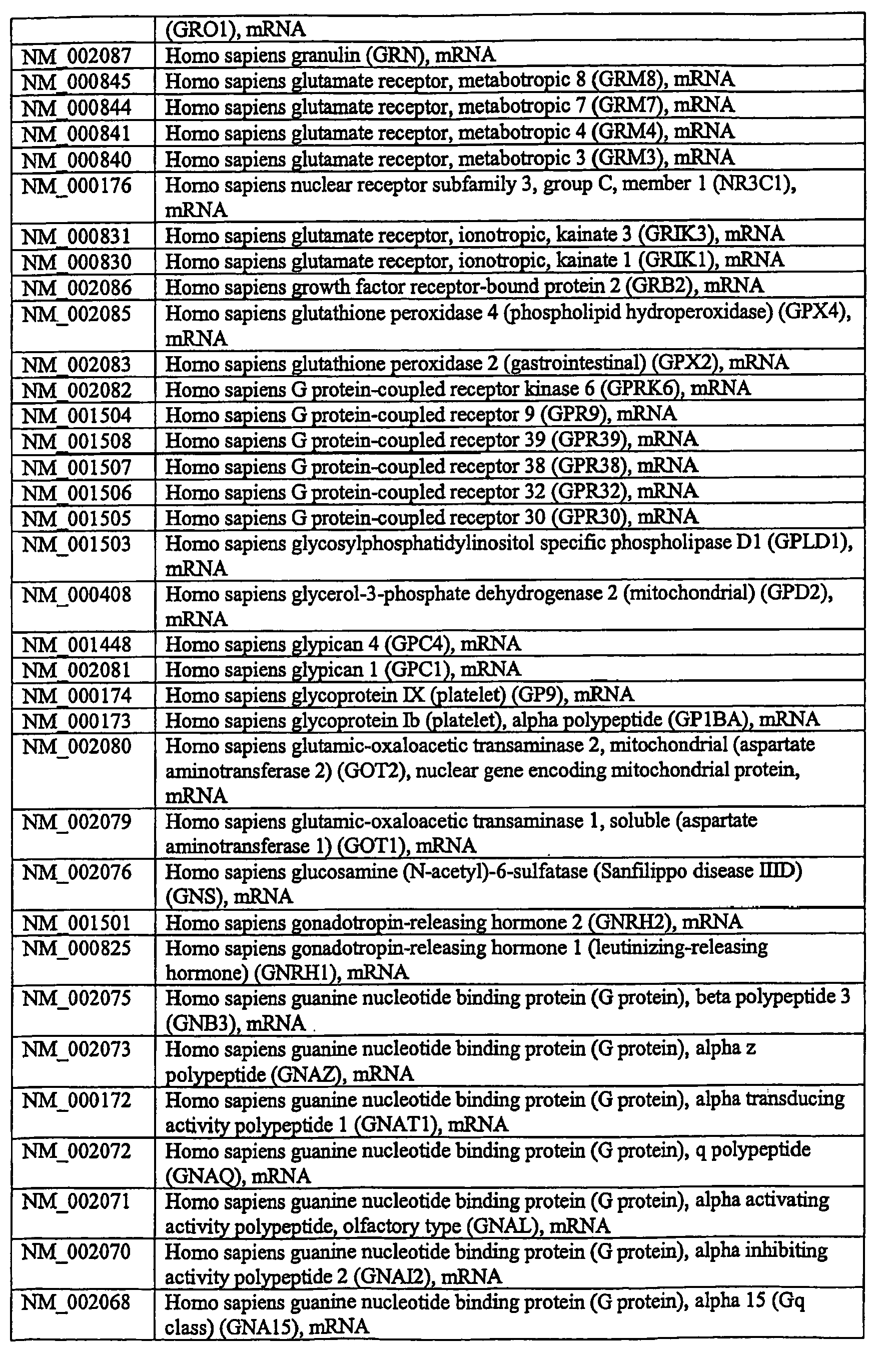

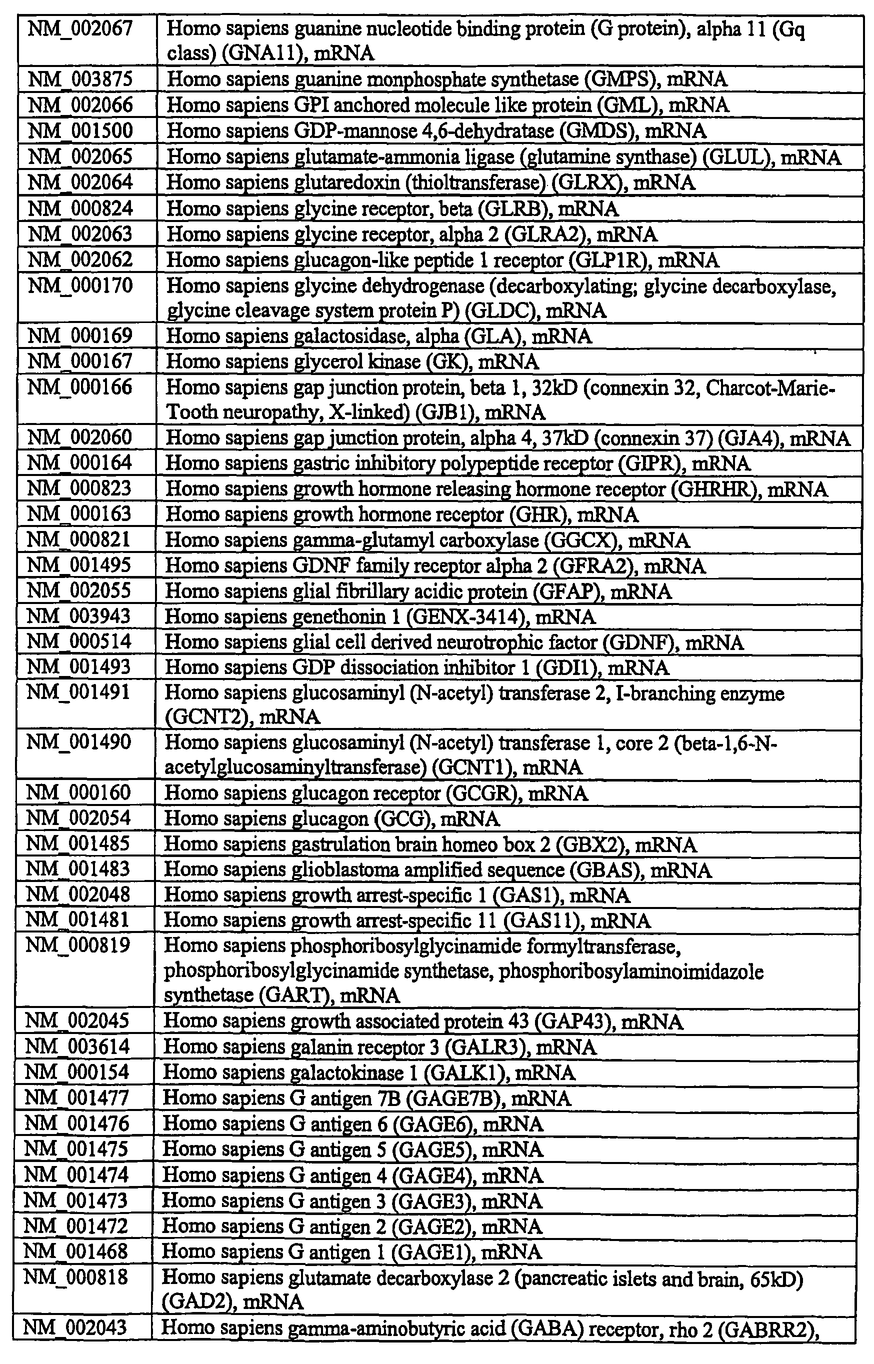

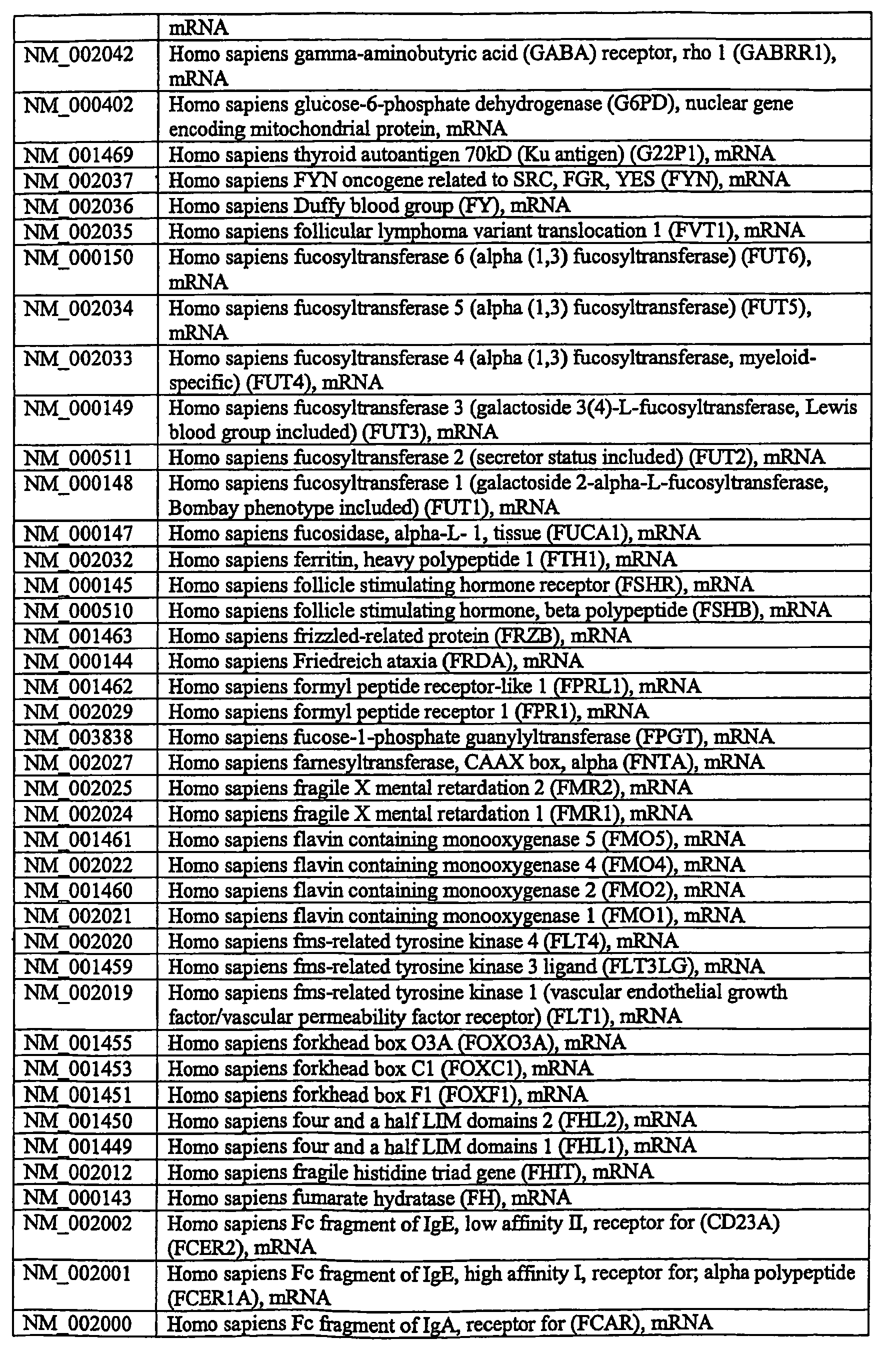

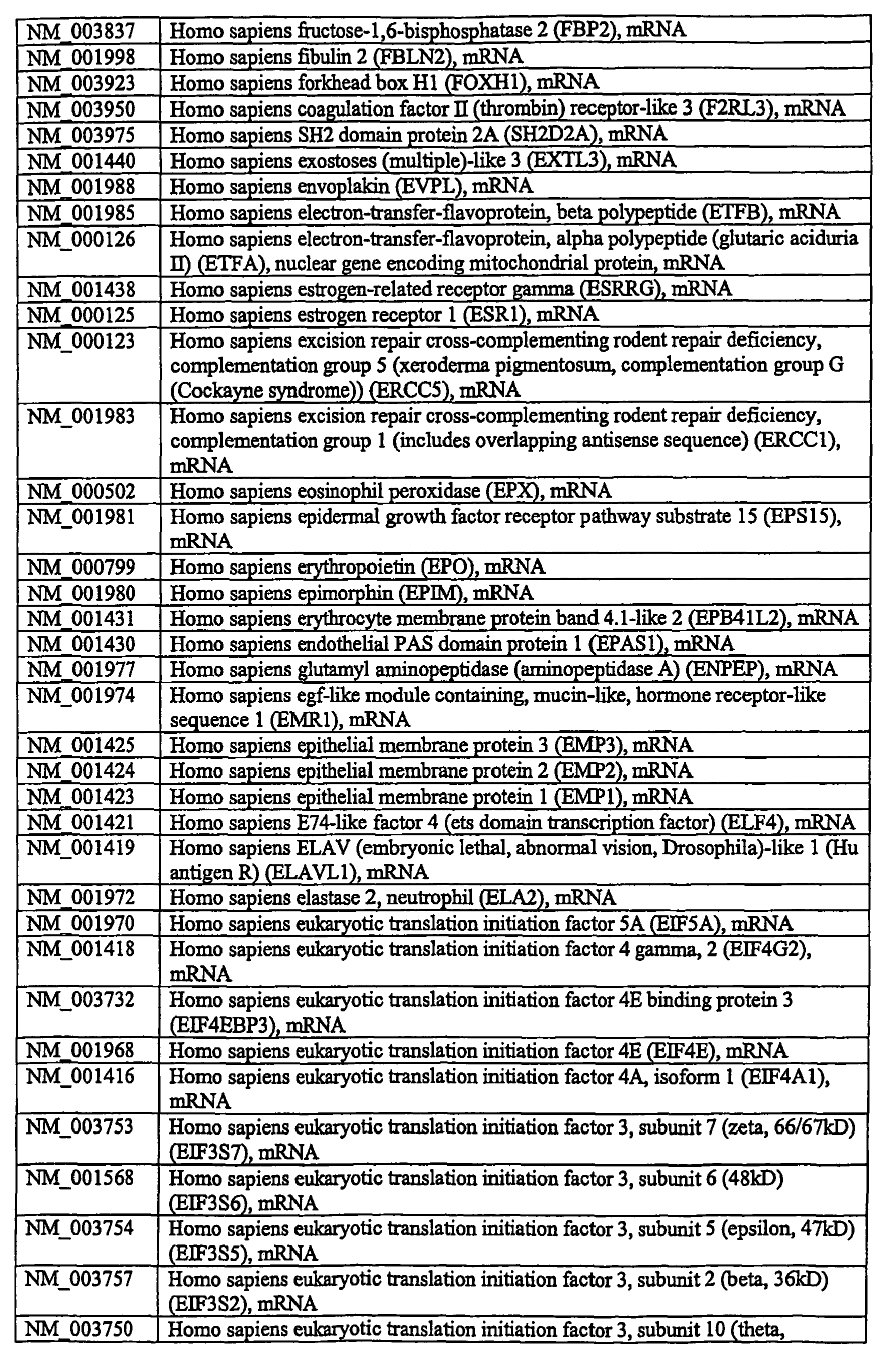

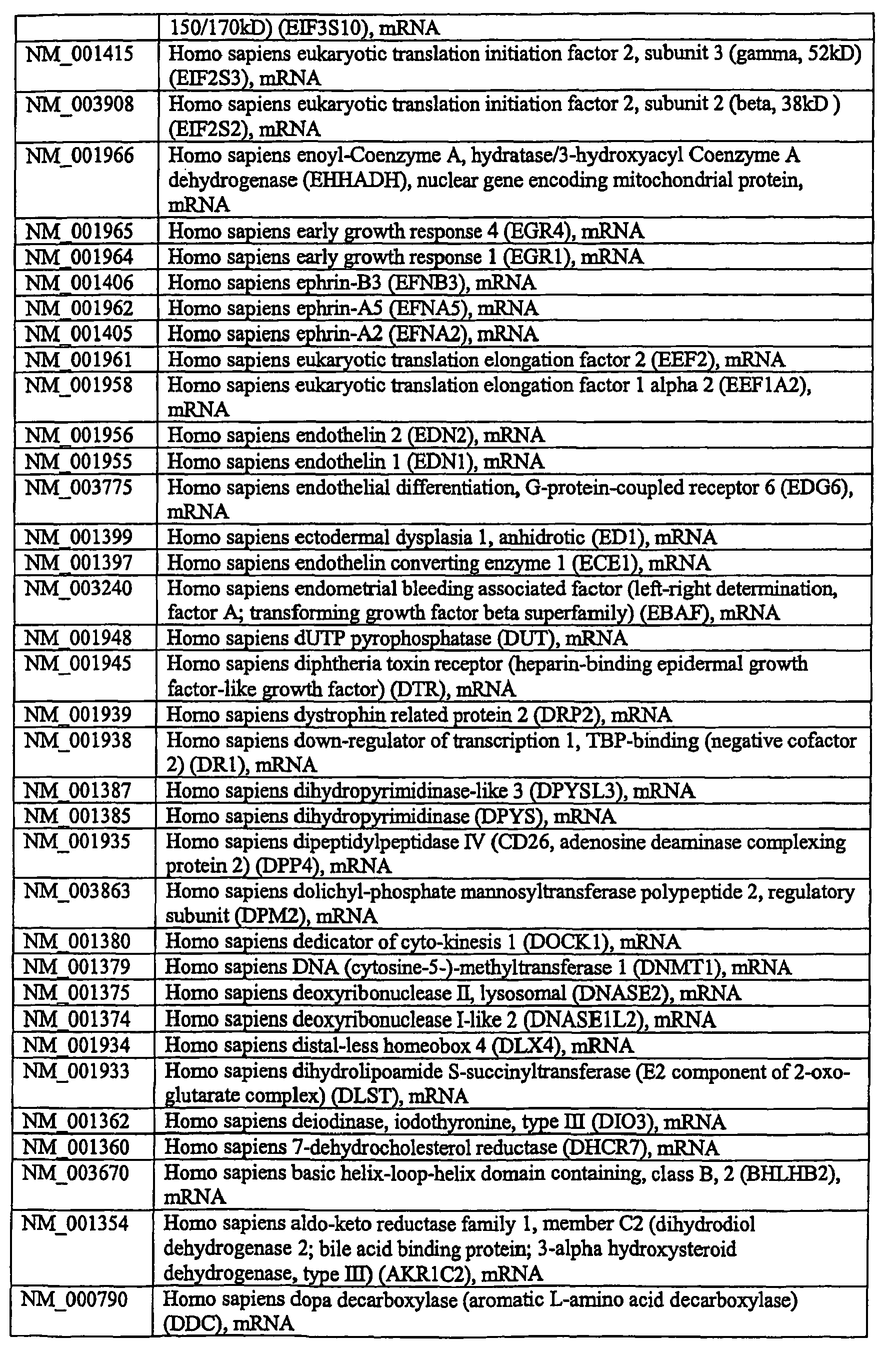

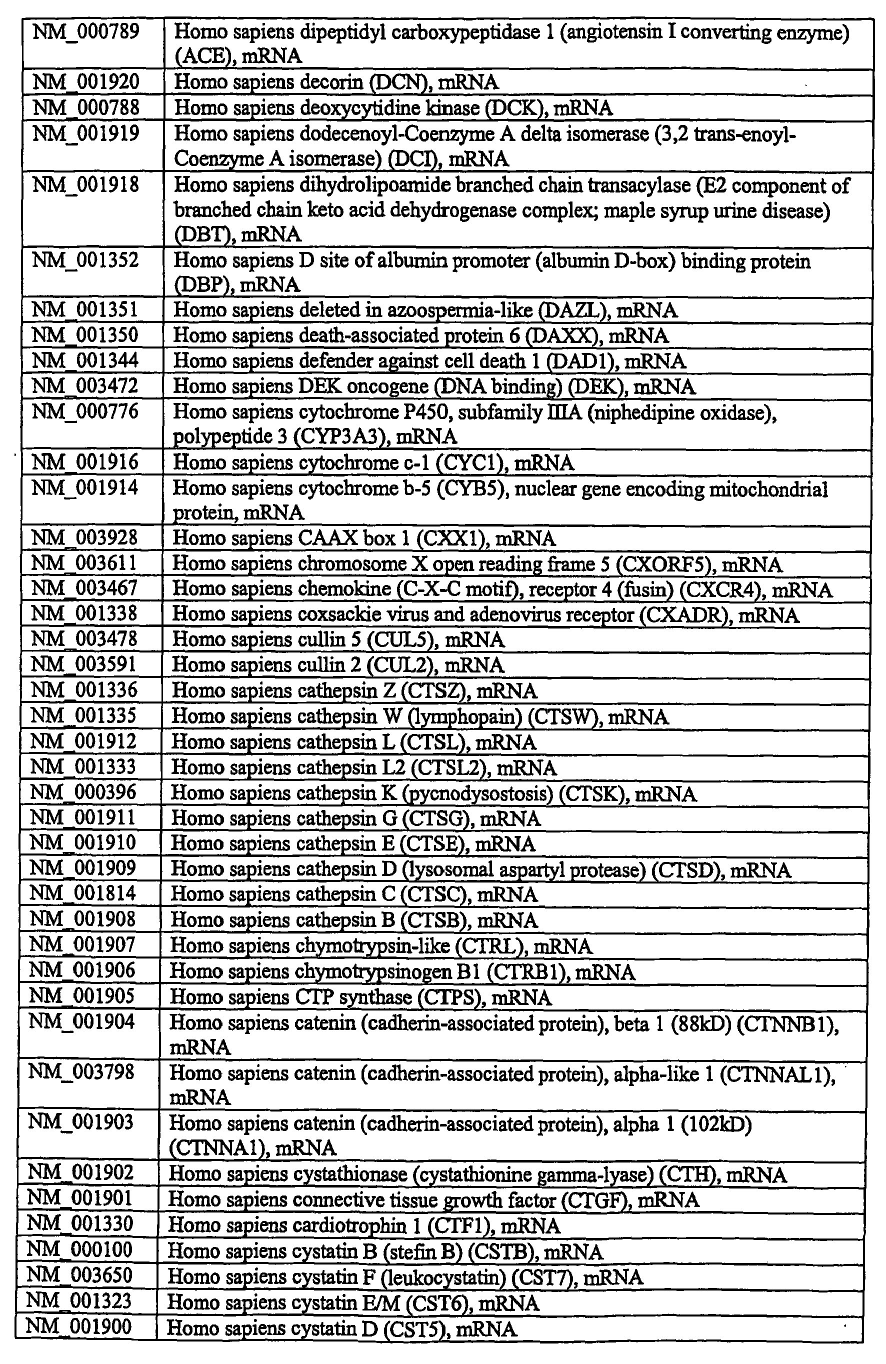

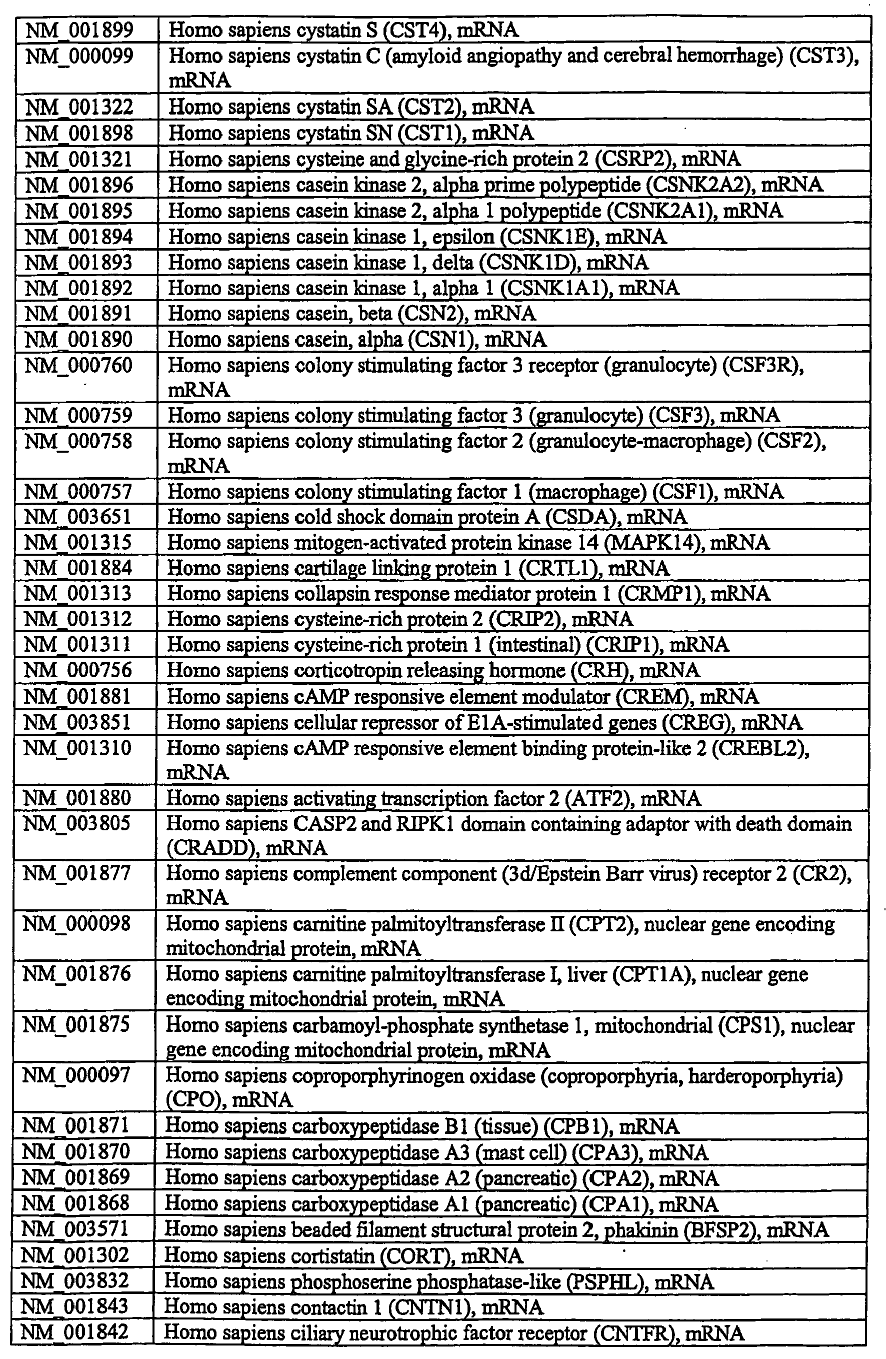

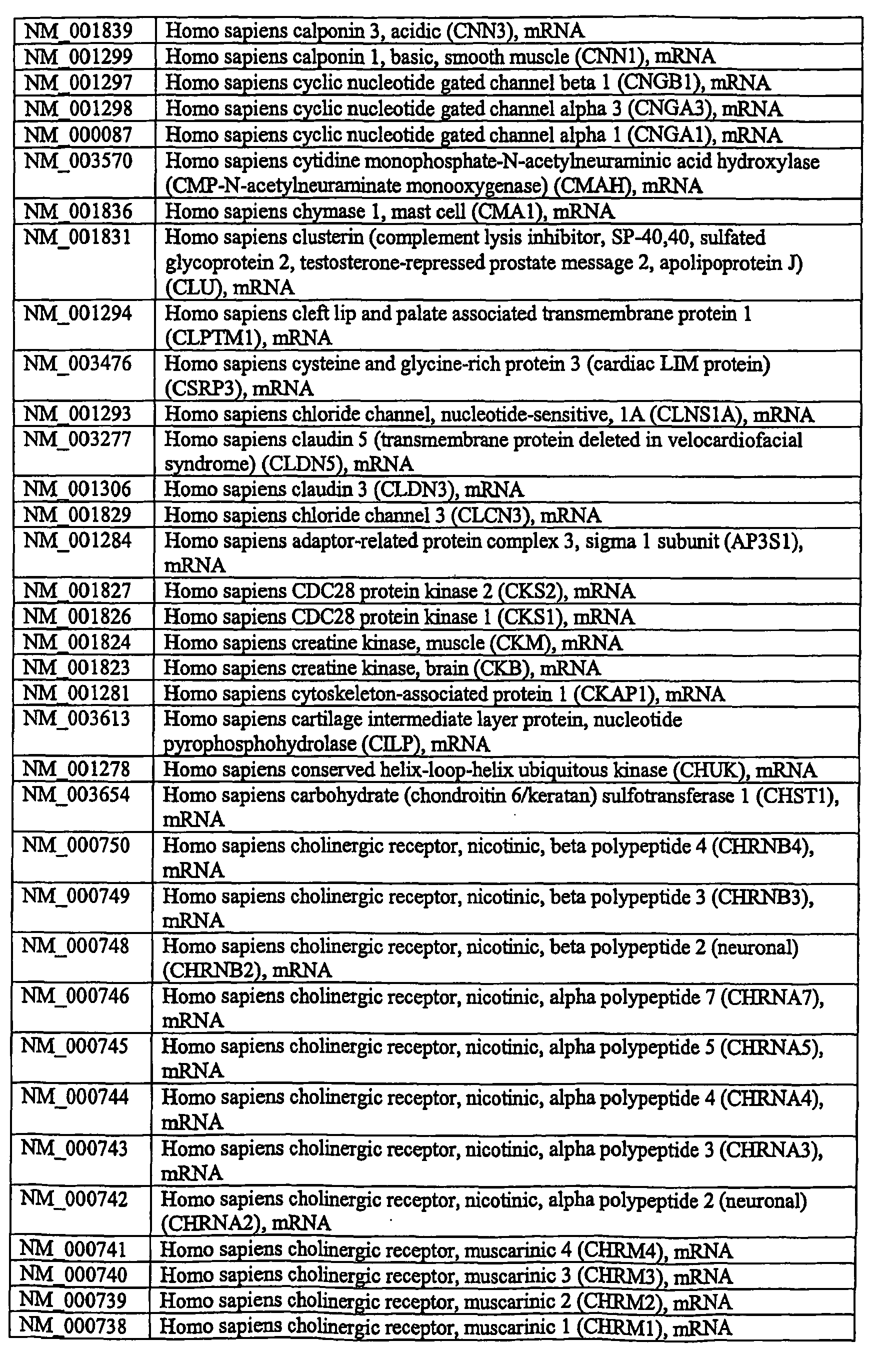

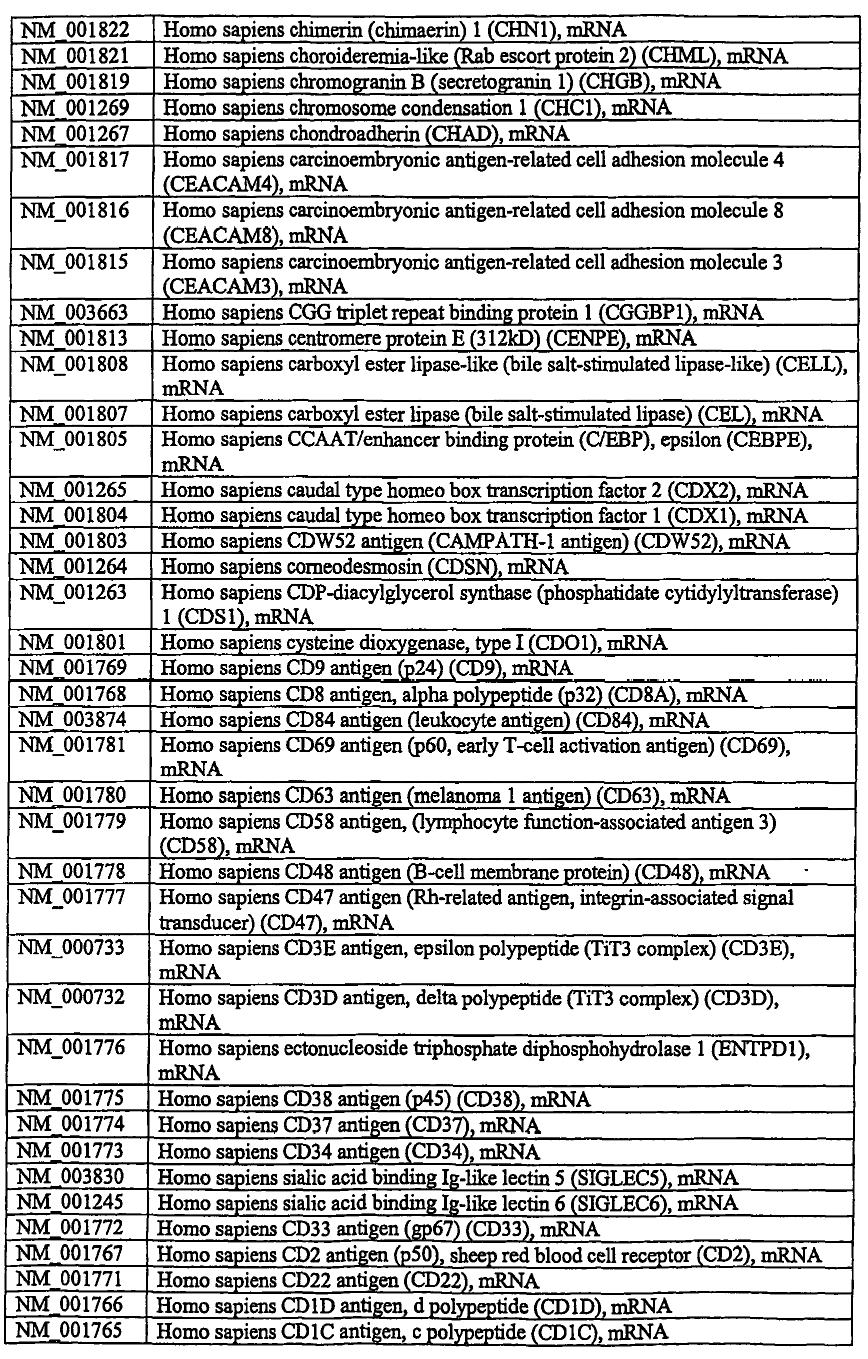

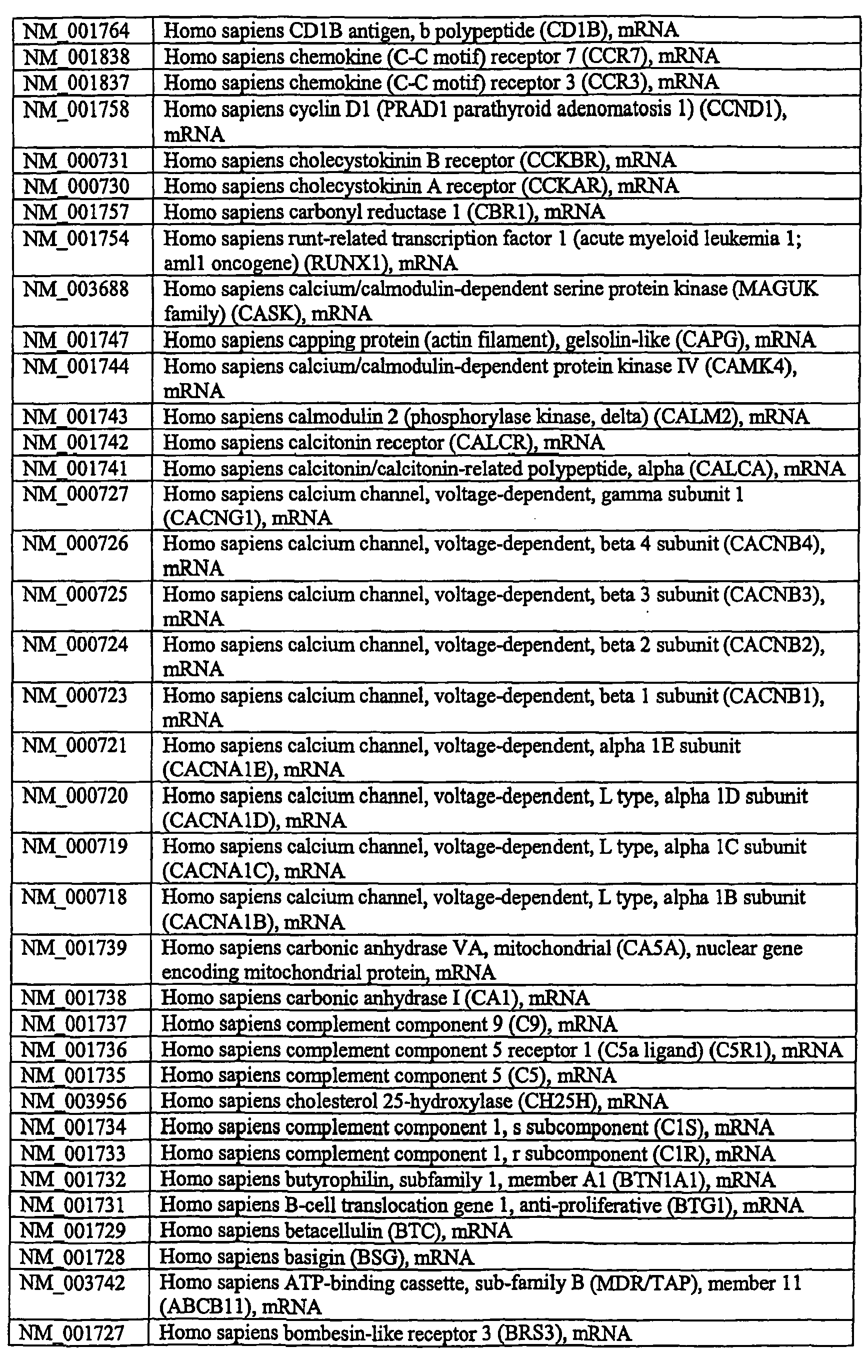

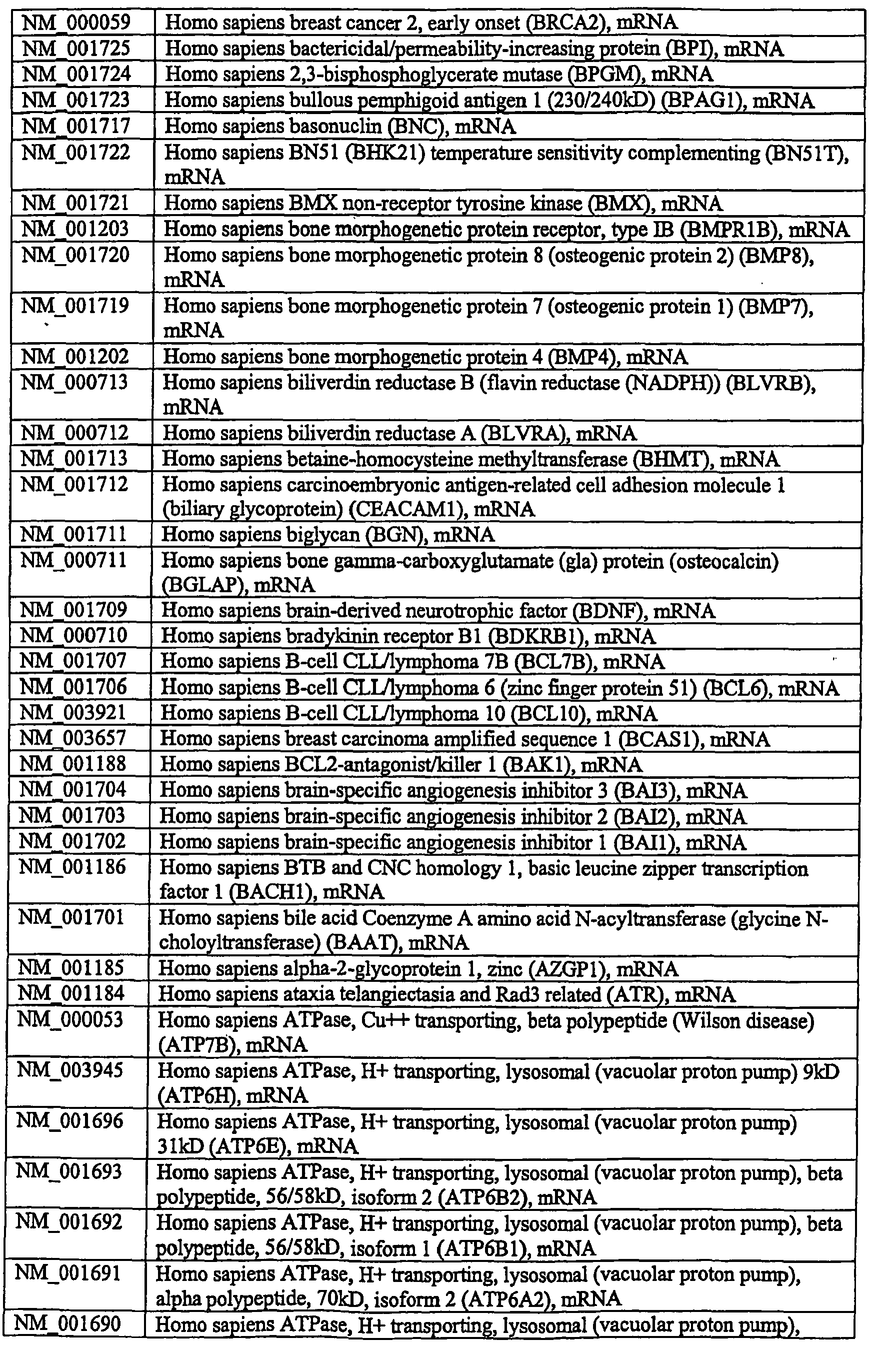

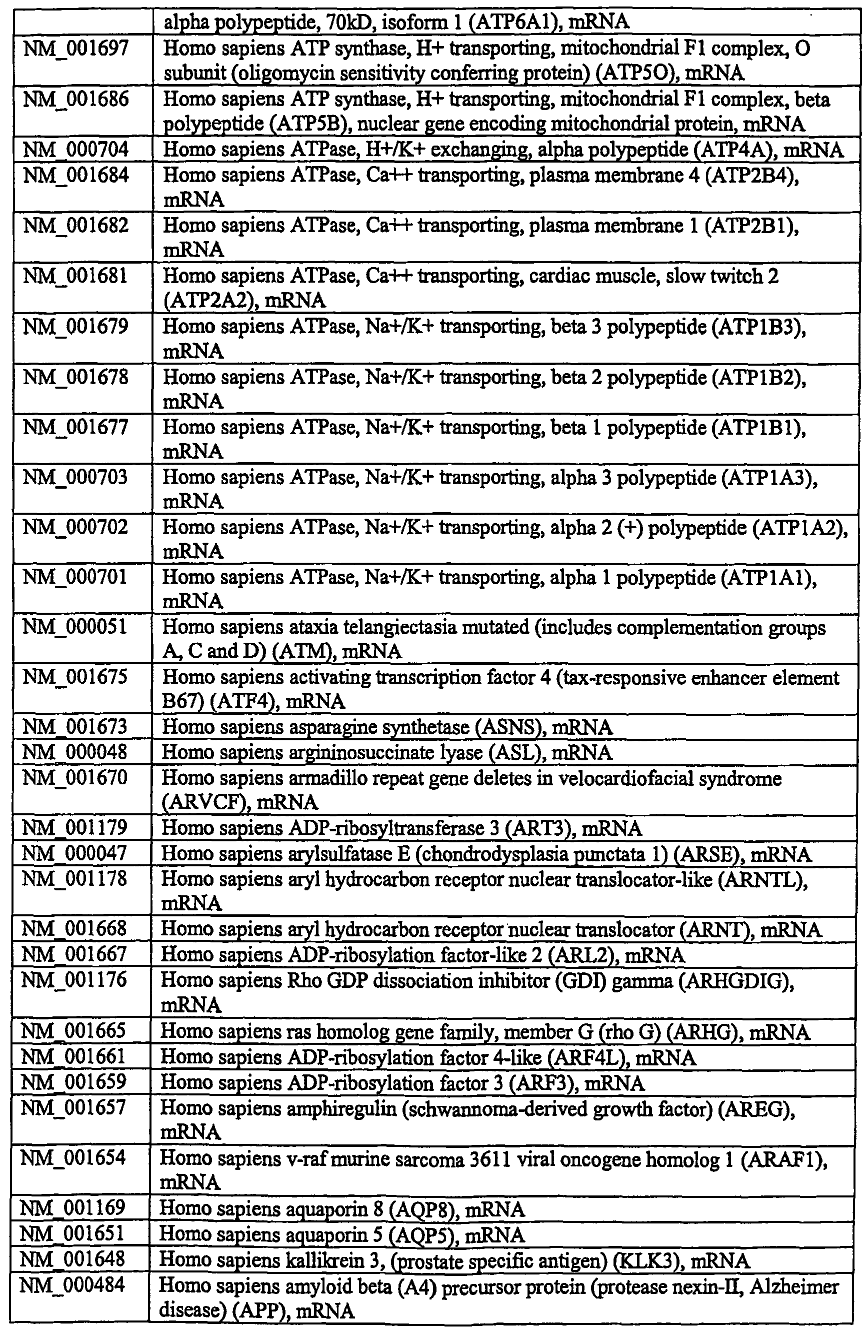

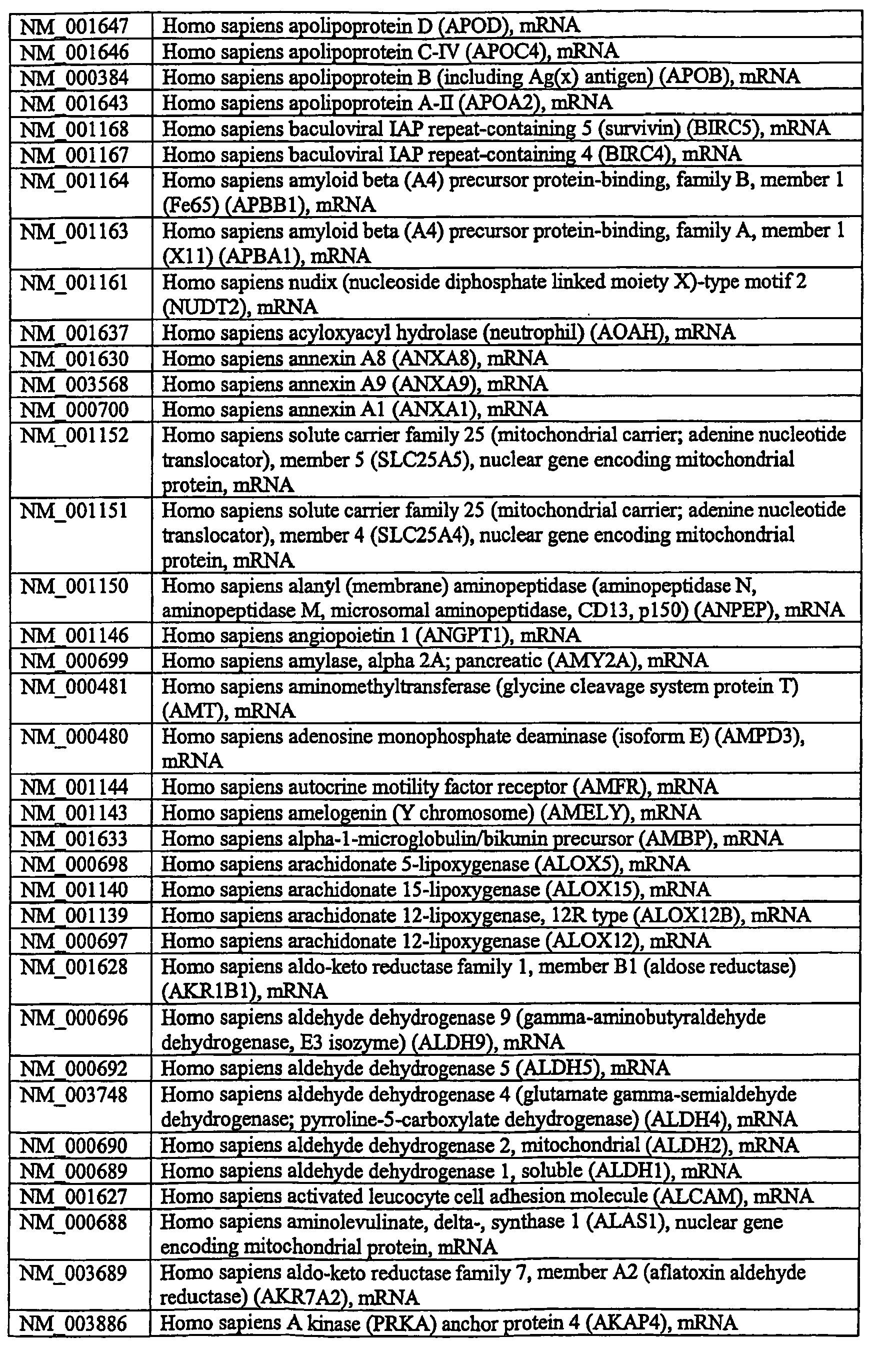

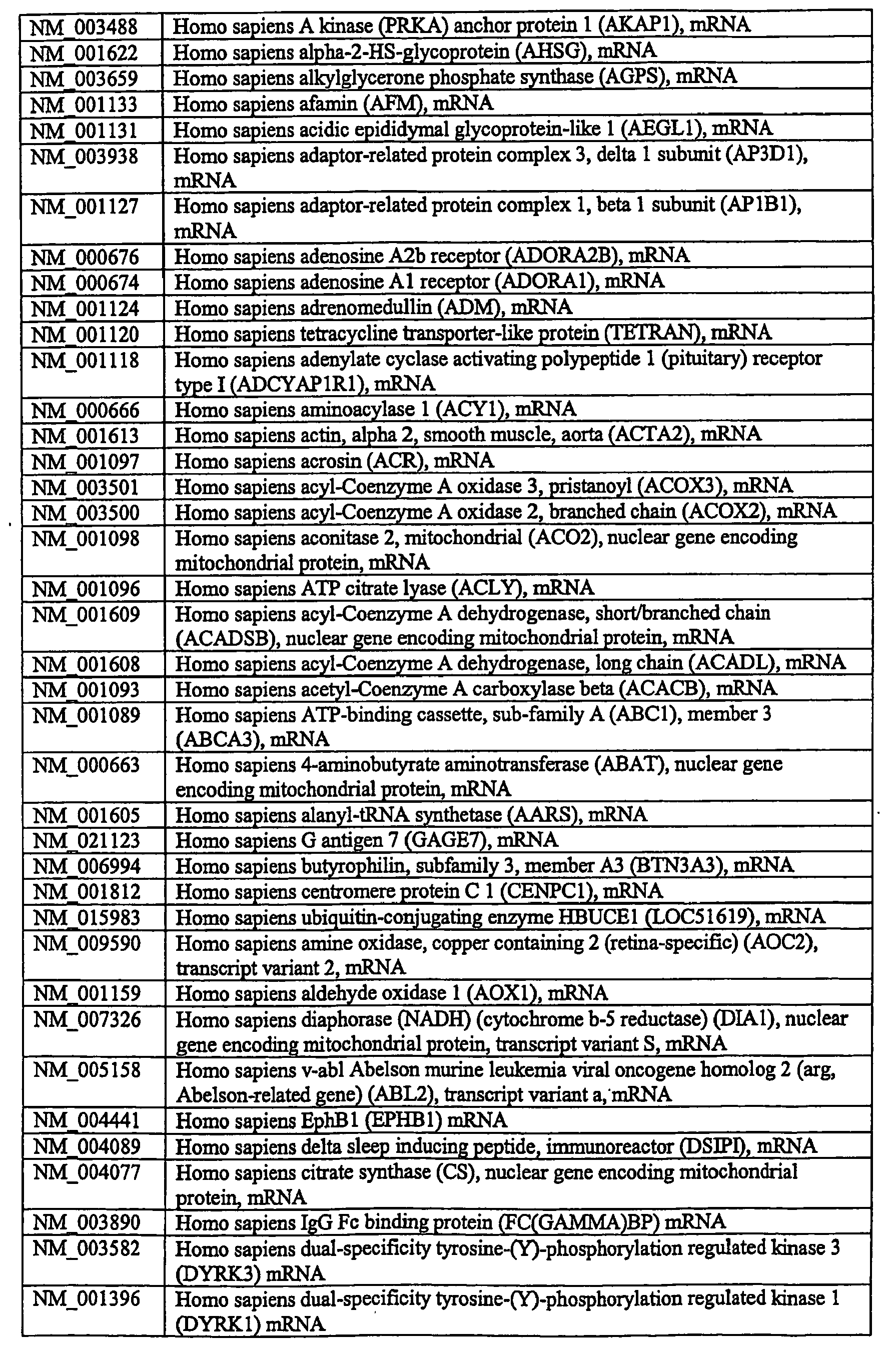

## Claims

1. A double stranded short interfering nucleic acid (siNA) molecule that directs cleavage of more than one Respiratory Syncytial Virus (RSV) RNA transcript having shared sequence homology via RNA interference (RNAi), wherein:
a. the double stranded siNA molecule comprises a first and a second strand and each strand of said siNA molecule is about 18 to about 23 nucleotides in length;
b. the first strand of said siNA molecule comprises nucleotide sequence having sufficient complementarity to said homologous RSV RNA transcripts for the siNA molecule to direct cleavage of the homologous RSV RNA transcripts via RNA interference;
c. the second strand of said siNA molecule comprises nucleotide sequence that is complementary to the first strand; and
d. the siNA comprises one or more nucleotides that are chemically modified.

2. The double stranded siNA molecule of claim 1, wherein said RSV RNA transcripts are derived from one or more strains of RSV.

3. The double stranded siNA molecule of claim 1, wherein said siNA molecule is chemically synthesized.

4. The double stranded siNA molecule of claim 3, wherein said siNA molecule comprises no ribonucleotides.

5. The double stranded siNA molecule of claim 3, wherein said siNA molecule comprises one or more ribonucleotides.

6. The double stranded siNA molecule of claim 1, wherein a 3'-end of one or both strands of the siNA molecule comprises one or more nucleotide overhangs.

7. The double stranded siNA molecule of claim 6, wherein said overhangs are 1-4 nucleotides.

8. The double stranded siNA molecule of claim 7, wherein said overhangs are 2 nucleotides.

9. The double stranded siNA molecule of claim 6, wherein said overhangs comprise 2'-deoxynucleotides.

10. The double stranded siNA molecule of claim 3 wherein each strand of said double stranded siNA molecule independently comprises at least one nucleotide sugar modification.

11. The double stranded siNA molecule of claim 10, wherein the sugar modification is selected from the group consisting of 2'-H, 2'-O-alkyl, and 2'-deoxy-2'-fluoro.

12. The double stranded siNA molecule of claim 11, wherein the 2'-O-alkyl modification is selected from the group consisting of 2'-O-methyl, 2'-O-allyl and 2'-methoxyethyl.

13. The double stranded siNA molecule of claim 11, wherein the 2'-deoxy-2'-fluoro modification is present on one or more of the pyrimidine nucleotides when present in the double stranded siNA molecule.

14. The double stranded siNA molecule of claim 13, wherein all the pyrimidine nucleotides in the double stranded siNA molecule comprise 2'-deoxy-2'-fluoro modification.

15. The double stranded siNA molecule of claim 10, wherein the sugar modification is present at the 5'-end or the 3'-end or both the 5' and 3' ends of the second strand of said double stranded siNA molecule.

16. The double stranded siNA molecule of claim 10, wherein the sugar modification is present at the 3'-end of the first strand of said double stranded siNA molecule.


### Annex to the application documents - subsequently filed sequences listing

SEQUENCE LISTING

<110> Sirna Therapeutics, Inc.
McSwiggen, James
Beigelman, Leonid
Chowrira, Bharat
Pavco, Pamela
Fosnaugh, Kathy
Jamison, Sharon
Usman, Nassim
Thompson, James

<120> RNA Interference Mediated Inhibition of Gene Expression Using
Short Interfering Nucleic Acid (siNA)

<130> 400/104 (MBHB 03-198)

<140> PCT/US 03/05028
<141> 2003-02-20

<150> US 60/363,124
<151> 2002-03-11

<150> US 60/358,580
<151> 2002-02-20

<150> US 60/386,782
<151> 2002-06-06

<150> US 60/406,784
<151> 2002-08-29

<150> US 60/408,378
<151> 2002-09-05

<150> US 60/409,293
<151> 2002-09-09

<150> US 60/440,129
<151> 2003-01-15

<160> 932

<170> PatentIn version 3.2

<210> 1
<211> 23
<212> RNA
<213> Artificial Sequence

<220>
<223> Description of Artificial Sequence: Target Sequence/siNA sense region

<400> 1
cauuccuccu ggaaauucaa ccu 23

<210> 2
<211> 23
<212> RNA
<213> Artificial Sequence

<220>

<223>  Description of Artificial Sequence:  Target Sequence/siNA sense region

<400>  2
uuccucucau gaugcuggug uuu                                              23


<210>  3
<211>  23
<212>  RNA
<213>  Artificial Sequence

<220>
<223>  Description of Artificial Sequence:  Target Sequence/siNA sense region

<400>  3
cacgauagcu gaaaacauuc gcu                                              23


<210>  4
<211>  23
<212>  RNA
<213>  Artificial Sequence

<220>
<223>  Description of Artificial Sequence:  Target Sequence/siNA sense region

<400>  4
aaaaugcagc ugaugaaucc aaa                                              23


<210>  5
<211>  23
<212>  RNA
<213>  Artificial Sequence

<220>
<223>  Description of Artificial Sequence:  Target Sequence/siNA sense region

<400>  5
aguucgagaa ggucaucagc aug                                              23


<210>  6
<211>  23
<212>  RNA
<213>  Artificial Sequence

<220>
<223>  Description of Artificial Sequence:  Target Sequence/siNA sense region

<400>  6
gaccaggugu cuagaggcaa cag                                              23


<210>  7
<211>  23
<212>  RNA
<213>  Artificial Sequence

<220>
<223>  Description of Artificial Sequence:  Target Sequence/siNA sense region

<400>  7

ggaccaagcu uaaggagagg aga                                          23

<210>  8
<211>  23
<212>  RNA
<213>  Artificial Sequence

<220>
<223>  Description of Artificial Sequence:  Target Sequence/siNA sense region

<400>  8
gucgguugac cuucugaaca uga                                          23

<210>  9
<211>  23
<212>  RNA
<213>  Artificial Sequence

<220>
<223>  Description of Artificial Sequence:  Target Sequence/siNA sense region

<400>  9
ugaccaucaa uaaggaagaa gcc                                          23

<210>  10
<211>  23
<212>  RNA
<213>  Artificial Sequence

<220>
<223>  Description of Artificial Sequence:  Target Sequence/siNA sense region

<400>  10
ccaucaauaa ggaagaagcc cuu                                          23

<210>  11
<211>  23
<212>  RNA
<213>  Artificial Sequence

<220>
<223>  Description of Artificial Sequence:  Target Sequence/siNA sense region

<400>  11
cugaccauca auaaggaaga agc                                          23

<210>  12
<211>  23
<212>  RNA
<213>  Artificial Sequence

<220>
<223>  Description of Artificial Sequence:  Target Sequence/siNA sense region

<400>  12
caauaaggaa gaagcccuuc agc                                          23

<210>  13
<211>  23
<212>  RNA
<213>  Artificial Sequence

<220>
<223>  Description of Artificial Sequence:  Target Sequence/siNA sense region

<400>  13
uggauuuaag cagaguucaa aag                                              23


<210>  14
<211>  23
<212>  RNA
<213>  Artificial Sequence

<220>
<223>  Description of Artificial Sequence:  Target Sequence/siNA sense region

<400>  14
gcagaguuca aaagcccuuc agc                                              23


<210>  15
<211>  23
<212>  RNA
<213>  Artificial Sequence

<220>
<223>  Description of Artificial Sequence:  Target Sequence/siNA sense region

<400>  15
agcagaguuc aaaagcccuu cag                                              23


<210>  16
<211>  23
<212>  RNA
<213>  Artificial Sequence

<220>
<223>  Description of Artificial Sequence:  Target Sequence/siNA sense region

<400>  16
ggauuuaagc agaguucaaa agc                                              23


<210>  17
<211>  23
<212>  RNA
<213>  Artificial Sequence

<220>
<223>  Description of Artificial Sequence:  Target Sequence/siNA sense region

<400>  17
aaugggugag guuaccaacc agu                                              23


<210>  18
<211>  23
<212>  RNA

<213>  Artificial Sequence

<220>
<223>  Description of Artificial Sequence:  Target Sequence/siNA sense region

<400>  18
ucaccuugga cauggaagac ugu                                                23


<210>  19
<211>  23
<212>  RNA
<213>  Artificial Sequence

<220>
<223>  Description of Artificial Sequence:  Target Sequence/siNA sense region

<400>  19
uaugggaccu gcuaagugug gaa                                                23


<210>  20
<211>  23
<212>  RNA
<213>  Artificial Sequence

<220>
<223>  Description of Artificial Sequence:  Target Sequence/siNA sense region

<400>  20
ccuaacauug gugcaaagau ugc                                                23


<210>  21
<211>  23
<212>  RNA
<213>  Artificial Sequence

<220>
<223>  Description of Artificial Sequence:  Target Sequence/siNA sense region

<400>  21
uggcugucuc ugaagacucu gcu                                                23


<210>  22
<211>  23
<212>  RNA
<213>  Artificial Sequence

<220>
<223>  Description of Artificial Sequence:  Target Sequence/siNA sense region

<400>  22
cuuuacgugg ccuguuucaa cac                                                23


<210>  23
<211>  23
<212>  RNA
<213>  Artificial Sequence

<220>

<223>  Description of Artificial Sequence:  Target Sequence/siNA sense region

<400>  23
aguuuggauc agggaguugg aag                                              23


<210>  24
<211>  23
<212>  RNA
<213>  Artificial Sequence

<220>
<223>  Description of Artificial Sequence:  Target Sequence/siNA sense region

<400>  24
cagggaugau caacagggua gug                                              23


<210>  25
<211>  23
<212>  RNA
<213>  Artificial Sequence

<220>
<223>  Description of Artificial Sequence:  Target Sequence/siNA sense region

<400>  25
gcuguagugg gguucuaggc auc                                              23


<210>  26
<211>  23
<212>  RNA
<213>  Artificial Sequence

<220>
<223>  Description of Artificial Sequence:  Target Sequence/siNA sense region

<400>  26
acacacaaac cuucugccuu uga                                              23


<210>  27
<211>  23
<212>  RNA
<213>  Artificial Sequence

<220>
<223>  Description of Artificial Sequence:  Target Sequence/siNA sense region

<400>  27
ucacauuguu ugcugcuauu gga                                              23


<210>  28
<211>  23
<212>  RNA
<213>  Artificial Sequence

<220>
<223>  Description of Artificial Sequence:  Target Sequence/siNA sense region

<400>  28

gaacacuucc ucuccaaaau gcc                                                23

<210>  29
<211>  23
<212>  RNA
<213>  Artificial Sequence

<220>
<223>  Description of Artificial Sequence:  Target Sequence/siNA sense region

<400>  29
cuggacacug agacugaggg ugu                                                23

<210>  30
<211>  23
<212>  RNA
<213>  Artificial Sequence

<220>
<223>  Description of Artificial Sequence:  Target Sequence/siNA sense region

<400>  30
ccaucaagcu agcagacuuu gga                                                23

<210>  31
<211>  23
<212>  RNA
<213>  Artificial Sequence

<220>
<223>  Description of Artificial Sequence:  Target Sequence/siNA sense region

<400>  31
cacucaccuu cuagucuugg cca                                                23

<210>  32
<211>  23
<212>  RNA
<213>  Artificial Sequence

<220>
<223>  Description of Artificial Sequence:  Target Sequence/siNA sense region

<400>  32
acacguuaga uuugccguac caa                                                23

<210>  33
<211>  23
<212>  RNA
<213>  Artificial Sequence

<220>
<223>  Description of Artificial Sequence:  Target Sequence/siNA sense region

<400>  33
uauggucaca ggagagaagg caa                                                23

<210>  34
<211>  23
<212>  RNA
<213>  Artificial Sequence

<220>
<223>  Description of Artificial Sequence:  Target Sequence/siNA sense region

<400>  34
ugagaaguug ggcuaucaau gga                                               23


<210>  35
<211>  23
<212>  RNA
<213>  Artificial Sequence

<220>
<223>  Description of Artificial Sequence:  Target Sequence/siNA sense region

<400>  35
guuucagggg acaugaguuu ucc                                               23


<210>  36
<211>  23
<212>  RNA
<213>  Artificial Sequence

<220>
<223>  Description of Artificial Sequence:  Target Sequence/siNA sense region

<400>  36
uaagggugau ggauuggagu uca                                               23


<210>  37
<211>  19
<212>  RNA
<213>  Artificial Sequence

<220>
<223>  Description of Artificial Sequence:  Target Sequence/siNA sense region

<400>  37
uaaccucgua cuggugccu                                                    19


<210>  38
<211>  19
<212>  RNA
<213>  Artificial Sequence

<220>
<223>  Description of Artificial Sequence:  Target Sequence/siNA sense region

<400>  38
accucguacu ggugccucc                                                    19


<210>  39
<211>  19
<212>  RNA

<213>  Artificial Sequence

<220>
<223>  Description of Artificial Sequence:  Target Sequence/siNA sense region

<400>  39
auuggggauc uuggaguuu                                                    19


<210>  40
<211>  19
<212>  RNA
<213>  Artificial Sequence

<220>
<223>  Description of Artificial Sequence:  Target Sequence/siNA sense region

<400>  40
ugauugggga ucuuggagu                                                    19


<210>  41
<211>  19
<212>  RNA
<213>  Artificial Sequence

<220>
<223>  Description of Artificial Sequence:  Target Sequence/siNA sense region

<400>  41
gaaaucacag gguuuuugc                                                    19


<210>  42
<211>  19
<212>  RNA
<213>  Artificial Sequence

<220>
<223>  Description of Artificial Sequence:  Target Sequence/siNA sense region

<400>  42
aggaaaucac aggguuuuu                                                    19


<210>  43
<211>  19
<212>  RNA
<213>  Artificial Sequence

<220>
<223>  Description of Artificial Sequence:  Target Sequence/siNA sense region

<400>  43
acugccagaa acugaccaa                                                    19


<210>  44
<211>  19
<212>  RNA
<213>  Artificial Sequence

<220>

<223>  Description of Artificial Sequence:  Target Sequence/siNA sense region

<400>  44
gaacugccag aaacugacc                                                    19


<210>  45
<211>  19
<212>  RNA
<213>  Artificial Sequence

<220>
<223>  Description of Artificial Sequence:  Target Sequence/siNA sense region

<400>  45
aggcaccagu acgagguua                                                    19


<210>  46
<211>  19
<212>  RNA
<213>  Artificial Sequence

<220>
<223>  Description of Artificial Sequence:  Target Sequence/siNA sense region

<400>  46
guuccgugag uugaucauc                                                    19


<210>  47
<211>  19
<212>  RNA
<213>  Artificial Sequence

<220>
<223>  Description of Artificial Sequence:  Target Sequence/siNA sense region

<400>  47
ccaaguccua cagacucca                                                    19


<210>  48
<211>  23
<212>  RNA
<213>  Artificial Sequence

<220>
<223>  Description of Artificial Sequence:  Target Sequence/siNA sense region

<400>  48
aggugaaugg cucaaggaac ucu                                               23


<210>  49
<211>  23
<212>  RNA
<213>  Artificial Sequence

<220>
<223>  Description of Artificial Sequence:  Target Sequence/siNA sense region

<400>  49

aaggaacugu gcaagaugac caa                                          23

<210>  50
<211>  23
<212>  RNA
<213>  Artificial Sequence

<220>
<223>  Description of Artificial Sequence:  Target Sequence/siNA sense region

<400>  50
gaaagcugcu caaccaucuc cuu                                          23

<210>  51
<211>  23
<212>  RNA
<213>  Artificial Sequence

<220>
<223>  Description of Artificial Sequence:  Target Sequence/siNA sense region

<400>  51
cucaaccauc uccuuccaca gug                                          23

<210>  52
<211>  23
<212>  RNA
<213>  Artificial Sequence

<220>
<223>  Description of Artificial Sequence:  Target Sequence/siNA sense region

<400>  52
uacaugcgac ugagacagcu caa                                          23

<210>  53
<211>  23
<212>  RNA
<213>  Artificial Sequence

<220>
<223>  Description of Artificial Sequence:  Target Sequence/siNA sense region

<400>  53
gcacauccug acuucuguga gcu                                          23

<210>  54
<211>  23
<212>  RNA
<213>  Artificial Sequence

<220>
<223>  Description of Artificial Sequence:  Target Sequence/siNA sense region

<400>  54
acgaugauga ugauggagac gau                                          23

<210> 55
<211> 23
<212> RNA
<213> Artificial Sequence

<220>
<223> Description of Artificial Sequence: Target Sequence/siNA sense region

<400> 55
ugacaauuuc ugugccauug cua 23


<210> 56
<211> 23
<212> RNA
<213> Artificial Sequence

<220>
<223> Description of Artificial Sequence: Target Sequence/siNA sense region

<400> 56
aacugaguuu aaaaggcacc cag 23


<210> 57
<211> 23
<212> RNA
<213> Artificial Sequence

<220>
<223> Description of Artificial Sequence: Target Sequence/siNA sense region

<400> 57
aacaaccaca aaauacaaca aga 23


<210> 58
<211> 23
<212> RNA
<213> Artificial Sequence

<220>
<223> Description of Artificial Sequence: Target Sequence/siNA sense region

<400> 58
agccuggaaa gaaucaaaac cuu 23


<210> 59
<211> 23
<212> RNA
<213> Artificial Sequence

<220>
<223> Description of Artificial Sequence: Target Sequence/siNA sense region

<400> 59
aagcaaggag ggccucugau ggu 23


<210> 60
<211> 23
<212> RNA

<213>  Artificial Sequence

<220>
<223>  Description of Artificial Sequence:  Target Sequence/siNA sense region

<400>  60
ucguguaagg aguggaccau cau                                                23


<210>  61
<211>  23
<212>  RNA
<213>  Artificial Sequence

<220>
<223>  Description of Artificial Sequence:  Target Sequence/siNA sense region

<400>  61
uuacggagua uugcuguggg aaa                                                23


<210>  62
<211>  23
<212>  RNA
<213>  Artificial Sequence

<220>
<223>  Description of Artificial Sequence:  Target Sequence/siNA sense region

<400>  62
uagcaggccu aagacaugug agg                                                23


<210>  63
<211>  23
<212>  RNA
<213>  Artificial Sequence

<220>
<223>  Description of Artificial Sequence:  Target Sequence/siNA sense region

<400>  63
agcaaaaagc aagggagaaa aga                                                23


<210>  64
<211>  23
<212>  RNA
<213>  Artificial Sequence

<220>
<223>  Description of Artificial Sequence:  Target Sequence/siNA sense region

<400>  64
agcaacugag aagccaagac uga                                                23


<210>  65
<211>  23
<212>  RNA
<213>  Artificial Sequence

<220>

<223>  Description of Artificial Sequence:  Target Sequence/siNA sense region

<400>  65
   gacauggacc uaucuggguc cuu                                              23

<210>  66
<211>  23
<212>  RNA
<213>  Artificial Sequence

<220>
<223>  Description of Artificial Sequence:  Target Sequence/siNA sense region

<400>  66
uagggaggac cuuaucugug cgu                                              23

<210>  67
<211>  23
<212>  RNA
<213>  Artificial Sequence

<220>
<223>  Description of Artificial Sequence:  Target Sequence/siNA sense region

<400>  67
aagcauccau guguggacuc aag                                              23

<210>  68
<211>  23
<212>  RNA
<213>  Artificial Sequence

<220>
<223>  Description of Artificial Sequence:  Target Sequence/siNA sense region

<400>  68
ugaagaggga aagcugacau cug                                              23

<210>  69
<211>  23
<212>  RNA
<213>  Artificial Sequence

<220>
<223>  Description of Artificial Sequence:  Target Sequence/siNA sense region

<400>  69
gaggaagaag gaaggagagg cuu                                              23

<210>  70
<211>  23
<212>  RNA
<213>  Artificial Sequence

<220>
<223>  Description of Artificial Sequence:  Target Sequence/siNA sense region

<400>  70

gagaggacug agccuacgga aag                                          23

<210>  71
<211>  23
<212>  RNA
<213>  Artificial Sequence

<220>
<223>  Description of Artificial Sequence:  Target Sequence/siNA sense region

<400>  71
uuugcugugg ugacacaugg uac                                          23

<210>  72
<211>  19
<212>  RNA
<213>  Artificial Sequence

<220>
<223>  Description of Artificial Sequence:  Target Sequence/siNA sense region

<400>  72
ccgcagugag caccaugga                                               19

<210>  73
<211>  19
<212>  RNA
<213>  Artificial Sequence

<220>
<223>  Description of Artificial Sequence:  Target Sequence/siNA sense region

<400>  73
agccgcagug agcaccaug                                               19

<210>  74
<211>  19
<212>  RNA
<213>  Artificial Sequence

<220>
<223>  Description of Artificial Sequence:  Target Sequence/siNA sense region

<400>  74
cauggugcuc acugcggcu                                               19

<210>  75
<211>  19
<212>  RNA
<213>  Artificial Sequence

<220>
<223>  Description of Artificial Sequence:  Target Sequence/siNA sense region

<400>  75
uggggucguc aaagacguu                                               19

<210>  76
<211>  19
<212>  RNA
<213>  Artificial Sequence

<220>
<223>  Description of Artificial Sequence:  Target Sequence/siNA sense region

<400>  76
ggugcuugga ucuggcgcu                                                    19


<210>  77
<211>  19
<212>  RNA
<213>  Artificial Sequence

<220>
<223>  Description of Artificial Sequence:  Target Sequence/siNA sense region

<400>  77
gaucuuuggg agccuggca                                                    19


<210>  78
<211>  19
<212>  RNA
<213>  Artificial Sequence

<220>
<223>  Description of Artificial Sequence:  Target Sequence/siNA sense region

<400>  78
gaauggcuca gugaccugu                                                    19


<210>  79
<211>  19
<212>  RNA
<213>  Artificial Sequence

<220>
<223>  Description of Artificial Sequence:  Target Sequence/siNA sense region

<400>  79
caccuucaaa gggacaccu                                                    19


<210>  80
<211>  23
<212>  RNA
<213>  Artificial Sequence

<220>
<223>  Description of Artificial Sequence:  Target Sequence/siNA sense region

<400>  80
gaaccauuuu guggacgaau acg                                               23


<210>  81
<211>  23
<212>  RNA

<213>  Artificial Sequence

<220>
<223>  Description of Artificial Sequence:  Target Sequence/siNA sense region

<400>  81
gccuguugga cauccuggau acc                                                23


<210>  82
<211>  23
<212>  RNA
<213>  Artificial Sequence

<220>
<223>  Description of Artificial Sequence:  Target Sequence/siNA sense region

<400>  82
gaggaugccu ucuacacguu ggu                                                23


<210>  83
<211>  23
<212>  RNA
<213>  Artificial Sequence

<220>
<223>  Description of Artificial Sequence:  Target Sequence/siNA sense region

<400>  83
cugaacccuc cugaugagag ugg                                                23


<210>  84
<211>  23
<212>  RNA
<213>  Artificial Sequence

<220>
<223>  Description of Artificial Sequence:  Target Sequence/siNA sense region

<400>  84
ucagcuuggc caauccgugc ggu                                                23


<210>  85
<211>  23
<212>  RNA
<213>  Artificial Sequence

<220>
<223>  Description of Artificial Sequence:  Target Sequence/siNA sense region

<400>  85
gguugcggag ggugggccug gga                                                23


<210>  86
<211>  23
<212>  RNA
<213>  Artificial Sequence

<220>

<223>  Description of Artificial Sequence:  Target Sequence/siNA sense region

<400>  86
gccugccgcc uuccaccguu cau                                              23


<210>  87
<211>  23
<212>  RNA
<213>  Artificial Sequence

<220>
<223>  Description of Artificial Sequence:  Target Sequence/siNA sense region

<400>  87
gcacccacug ccaccgcgaa gag                                              23


<210>  88
<211>  23
<212>  RNA
<213>  Artificial Sequence

<220>
<223>  Description of Artificial Sequence:  Target Sequence/siNA sense region

<400>  88
gcgcggcgcg auucccugag cug                                              23


<210>  89
<211>  23
<212>  RNA
<213>  Artificial Sequence

<220>
<223>  Description of Artificial Sequence:  Target Sequence/siNA sense region

<400>  89
gcucccuuua uaagccgacu cgc                                              23


<210>  90
<211>  23
<212>  RNA
<213>  Artificial Sequence

<220>
<223>  Description of Artificial Sequence:  Target Sequence/siNA sense region

<400>  90
ccuuccaccg uucauucuag agc                                              23


<210>  91
<211>  23
<212>  RNA
<213>  Artificial Sequence

<220>
<223>  Description of Artificial Sequence:  Target Sequence/siNA sense region

<400>  91

uuccaccguu cauucuagag caa                                          23

<210>  92
<211>  23
<212>  RNA
<213>  Artificial Sequence

<220>
<223>  Description of Artificial Sequence:  Target Sequence/siNA sense region

<400>  92
gcgaagaguu gggcucuguc agc                                          23

<210>  93
<211>  23
<212>  RNA
<213>  Artificial Sequence

<220>
<223>  Description of Artificial Sequence:  Target Sequence/siNA sense region

<400>  93
uggaagagcc aacuguguga gau                                          23

<210>  94
<211>  23
<212>  RNA
<213>  Artificial Sequence

<220>
<223>  Description of Artificial Sequence:  Target Sequence/siNA sense region

<400>  94
agagggagga gaaggaguuc cuc                                          23

<210>  95
<211>  23
<212>  RNA
<213>  Artificial Sequence

<220>
<223>  Description of Artificial Sequence:  Target Sequence/siNA sense region

<400>  95
agggaguaca gcaaacugaa ggc                                          23

<210>  96
<211>  23
<212>  RNA
<213>  Artificial Sequence

<220>
<223>  Description of Artificial Sequence:  Target Sequence/siNA sense region

<400>  96
gucauggagu gcauugagua ggg                                          23

<210> 97
<211> 23
<212> RNA
<213> Artificial Sequence

<220>
<223> Description of Artificial Sequence: Target Sequence/siNA sense region

<400> 97
uaaccucaac uccugccaca aug 23


<210> 98
<211> 23
<212> RNA
<213> Artificial Sequence

<220>
<223> Description of Artificial Sequence: Target Sequence/siNA sense region

<400> 98
aacuccuguc uugcauugca cua 23


<210> 99
<211> 23
<212> RNA
<213> Artificial Sequence

<220>
<223> Description of Artificial Sequence: Target Sequence/siNA sense region

<400> 99
ucuugcacuu gucacaaaca gug 23


<210> 100
<211> 23
<212> RNA
<213> Artificial Sequence

<220>
<223> Description of Artificial Sequence: Target Sequence/siNA sense region

<400> 100
aacacagcua caacuggagc auu 23


<210> 101
<211> 23
<212> RNA
<213> Artificial Sequence

<220>
<223> Description of Artificial Sequence: Target Sequence/siNA sense region

<400> 101
uguccacuua ccugaggagc aag 23


<210> 102
<211> 23
<212> RNA

<213>  Artificial Sequence

<220>
<223>  Description of Artificial Sequence:  Target Sequence/siNA sense region

<400>  102
uuugagcaug gaagaggauu cug                                              23


<210>  103
<211>  23
<212>  RNA
<213>  Artificial Sequence

<220>
<223>  Description of Artificial Sequence:  Target Sequence/siNA sense region

<400>  103
augguucuug ccucagaaga gcu                                              23


<210>  104
<211>  23
<212>  RNA
<213>  Artificial Sequence

<220>
<223>  Description of Artificial Sequence:  Target Sequence/siNA sense region

<400>  104
ucugaaggcu caaaccagac aag                                              23


<210>  105
<211>  23
<212>  RNA
<213>  Artificial Sequence

<220>
<223>  Description of Artificial Sequence:  Target Sequence/siNA sense region

<400>  105
ugaccuugga gcaucucauc ugu                                              23


<210>  106
<211>  23
<212>  RNA
<213>  Artificial Sequence

<220>
<223>  Description of Artificial Sequence:  Target Sequence/siNA sense region

<400>  106
ucaccuguuu ccuguaugga gga                                              23


<210>  107
<211>  23
<212>  RNA
<213>  Artificial Sequence

<220>

<223>  Description of Artificial Sequence:  Target Sequence/siNA sense region

<400>  107
gacaacacag caggaaucag uca                                              23


<210>  108
<211>  23
<212>  RNA
<213>  Artificial Sequence

<220>
<223>  Description of Artificial Sequence:  Target Sequence/siNA sense region

<400>  108
acaagacagg guguugauga ugc                                              23


<210>  109
<211>  23
<212>  RNA
<213>  Artificial Sequence

<220>
<223>  Description of Artificial Sequence:  Target Sequence/siNA sense region

<400>  109
uuuccucgaa gugccaguau ucc                                              23


<210>  110
<211>  23
<212>  RNA
<213>  Artificial Sequence

<220>
<223>  Description of Artificial Sequence:  Target Sequence/siNA sense region

<400>  110
   auuucugucu ugggguuuuu ggu                                               23


<210>  111
<211>  23
<212>  RNA
<213>  Artificial Sequence

<220>
<223>  Description of Artificial Sequence:  Target Sequence/siNA sense region

<400>  111
guuuuuggug caugcaguug auu                                              23


<210>  112
<211>  23
<212>  RNA
<213>  Artificial Sequence

<220>
<223>  Description of Artificial Sequence:  Target Sequence/siNA sense region

<400>  112

accagaccua cugccagaga acc 23

<210> 113
<211> 23
<212> RNA
<213> Artificial Sequence

<220>
<223> Description of Artificial Sequence: Target Sequence/siNA sense region

<400> 113
aucacacagg guuccugaca gaa 23

<210> 114
<211> 23
<212> RNA
<213> Artificial Sequence

<220>
<223> Description of Artificial Sequence: Target Sequence/siNA sense region

<400> 114
uuggcucuag ucacuggcau cuc 23

<210> 115
<211> 23
<212> RNA
<213> Artificial Sequence

<220>
<223> Description of Artificial Sequence: Target Sequence/siNA sense region

<400> 115
acuguggagu ugacucggug uuc 23

<210> 116
<211> 23
<212> RNA
<213> Artificial Sequence

<220>
<223> Description of Artificial Sequence: Target Sequence/siNA sense region

<400> 116
gccuacuuug cucaguacca cga 23

<210> 117
<211> 23
<212> RNA
<213> Artificial Sequence

<220>
<223> Description of Artificial Sequence: Target Sequence/siNA sense region

<400> 117
ugucugucuu ugugggaggg uaa 23

<210> 118
<211> 23
<212> RNA
<213> Artificial Sequence

<220>
<223> Description of Artificial Sequence: Target Sequence/siNA sense region

<400> 118
aaaagggucu ucuuggcagc uua 23


<210> 119
<211> 23
<212> RNA
<213> Artificial Sequence

<220>
<223> Description of Artificial Sequence: Target Sequence/siNA sense region

<400> 119
ggacucuaag cuggagcucu ugg 23


<210> 120
<211> 23
<212> RNA
<213> Artificial Sequence

<220>
<223> Description of Artificial Sequence: Target Sequence/siNA sense region

<400> 120
aacagcuugg aacaccaugu ccu 23


<210> 121
<211> 23
<212> RNA
<213> Artificial Sequence

<220>
<223> Description of Artificial Sequence: Target Sequence/siNA sense region

<400> 121
uuuucccagc ugacucagaa cac 23


<210> 122
<211> 23
<212> RNA
<213> Artificial Sequence

<220>
<223> Description of Artificial Sequence: Target Sequence/siNA sense region

<400> 122
caaugucaac agauccgacu uug 23


<210> 123
<211> 23
<212> RNA

<213>  Artificial Sequence

<220>
<223>  Description of Artificial Sequence:  Target Sequence/siNA sense region

<400>  123
cuuuggccuc ugauacagac agc                                                23


<210>  124
<211>  23
<212>  RNA
<213>  Artificial Sequence

<220>
<223>  Description of Artificial Sequence:  Target Sequence/siNA sense region

<400>  124
cagugacgag gaugaugagg acu                                                23


<210>  125
<211>  23
<212>  RNA
<213>  Artificial Sequence

<220>
<223>  Description of Artificial Sequence:  Target Sequence/siNA sense region

<400>  125
uuggucuguu auugccaagc acu                                                23


<210>  126
<211>  23
<212>  RNA
<213>  Artificial Sequence

<220>
<223>  Description of Artificial Sequence:  Target Sequence/siNA sense region

<400>  126
aucugcagga gucuucaaaa gcc                                                23


<210>  127
<211>  23
<212>  RNA
<213>  Artificial Sequence

<220>
<223>  Description of Artificial Sequence:  Target Sequence/siNA sense region

<400>  127
aggugcuacc aacacagaac cac                                                23


<210>  128
<211>  23
<212>  RNA
<213>  Artificial Sequence

<220>

<223>  Description of Artificial Sequence:  Target Sequence/siNA sense region

<400>  128
caagaggguc aaguuggaca gug                                              23


<210>  129
<211>  23
<212>  RNA
<213>  Artificial Sequence

<220>
<223>  Description of Artificial Sequence:  Target Sequence/siNA sense region

<400>  129
aagcagagga gcaaaagcuc auu                                              23


<210>  130
<211>  23
<212>  RNA
<213>  Artificial Sequence

<220>
<223>  Description of Artificial Sequence:  Target Sequence/siNA sense region

<400>  130
uacggaacuc uugugcguaa gga                                              23


<210>  131
<211>  23
<212>  RNA
<213>  Artificial Sequence

<220>
<223>  Description of Artificial Sequence:  Target Sequence/siNA sense region

<400>  131
acaaccuugg cugagucuug aga                                              23


<210>  132
<211>  23
<212>  RNA
<213>  Artificial Sequence

<220>
<223>  Description of Artificial Sequence:  Target Sequence/siNA sense region

<400>  132
ucguucagug ucucuccaaa agc                                              23


<210>  133
<211>  23
<212>  RNA
<213>  Artificial Sequence

<220>
<223>  Description of Artificial Sequence:  Target Sequence/siNA sense region

<400>  133

guuuugcaga uagccuugag caa                                              23

<210>  134
<211>  23
<212>  RNA
<213>  Artificial Sequence

<220>
<223>  Description of Artificial Sequence:  Target Sequence/siNA sense region

<400>  134
auuccugcug cuuucauuga cag                                              23

<210>  135
<211>  23
<212>  RNA
<213>  Artificial Sequence

<220>
<223>  Description of Artificial Sequence:  Target Sequence/siNA sense region

<400>  135
uugacugcca uguguucauc auc                                              23

<210>  136
<211>  23
<212>  RNA
<213>  Artificial Sequence

<220>
<223>  Description of Artificial Sequence:  Target Sequence/siNA sense region

<400>  136
cccugcagua ccucuaccug cag                                              23

<210>  137
<211>  23
<212>  RNA
<213>  Artificial Sequence

<220>
<223>  Description of Artificial Sequence:  Target Sequence/siNA sense region

<400>  137
accgucuccu acugcaccag aac                                              23

<210>  138
<211>  23
<212>  RNA
<213>  Artificial Sequence

<220>
<223>  Description of Artificial Sequence:  Target Sequence/siNA sense region

<400>  138
acuggagccu ggaagaccag cuu                                              23

<210>  139
<211>  23
<212>  RNA
<213>  Artificial Sequence

<220>
<223>  Description of Artificial Sequence:  Target Sequence/siNA sense region

<400>  139
uggugacuca gaaggcucag gug                                              23


<210>  140
<211>  23
<212>  RNA
<213>  Artificial Sequence

<220>
<223>  Description of Artificial Sequence:  Target Sequence/siNA sense region

<400>  140
uuugcacgua uaugccgaga ucu                                              23


<210>  141
<211>  23
<212>  RNA
<213>  Artificial Sequence

<220>
<223>  Description of Artificial Sequence:  Target Sequence/siNA sense region

<400>  141
agccauauug gagaugcugu ugu                                              23


<210>  142
<211>  23
<212>  RNA
<213>  Artificial Sequence

<220>
<223>  Description of Artificial Sequence:  Target Sequence/siNA sense region

<400>  142
aaauugcgga uaugggacac uua                                              23


<210>  143
<211>  23
<212>  RNA
<213>  Artificial Sequence

<220>
<223>  Description of Artificial Sequence:  Target Sequence/siNA sense region

<400>  143
caaaagccac uccacucucu uca                                              23


<210>  144
<211>  23
<212>  RNA

<213>  Artificial Sequence

<220>
<223>  Description of Artificial Sequence:  Target Sequence/siNA sense region

<400>  144
uucaggaccu ccacaugaua gga                                              23


<210>  145
<211>  23
<212>  RNA
<213>  Artificial Sequence

<220>
<223>  Description of Artificial Sequence:  Target Sequence/siNA sense region

<400>  145
cuaaaggcug agguugcuga uga                                              23


<210>  146
<211>  23
<212>  RNA
<213>  Artificial Sequence

<220>
<223>  Description of Artificial Sequence:  Target Sequence/siNA sense region

<400>  146
ggaaacaacc uuccaacaac cuu                                              23


<210>  147
<211>  23
<212>  RNA
<213>  Artificial Sequence

<220>
<223>  Description of Artificial Sequence:  Target Sequence/siNA sense region

<400>  147
caaaggacug augaccaaac acc                                              23


<210>  148
<211>  23
<212>  RNA
<213>  Artificial Sequence

<220>
<223>  Description of Artificial Sequence:  Target Sequence/siNA sense region

<400>  148
aaggaugugg ugauucagga uga                                              23


<210>  149
<211>  23
<212>  RNA
<213>  Artificial Sequence

<220>

<223>  Description of Artificial Sequence:  Target Sequence/siNA sense region

<400>  149
aucucguuuc ucuuggacaa gca                                              23


<210>  150
<211>  23
<212>  RNA
<213>  Artificial Sequence

<220>
<223>  Description of Artificial Sequence:  Target Sequence/siNA sense region

<400>  150
gaggugagcu acaaacacau gcg                                              23


<210>  151
<211>  23
<212>  RNA
<213>  Artificial Sequence

<220>
<223>  Description of Artificial Sequence:  Target Sequence/siNA sense region

<400>  151
guaguggaag acuggcugag ccu                                              23


<210>  152
<211>  23
<212>  RNA
<213>  Artificial Sequence

<220>
<223>  Description of Artificial Sequence:  Target Sequence/siNA sense region

<400>  152
cuccucuagc cuguuuguug ugg                                              23


<210>  153
<211>  23
<212>  RNA
<213>  Artificial Sequence

<220>
<223>  Description of Artificial Sequence:  Target Sequence/siNA sense region

<400>  153
uauccgacau gaagccagug acu                                              23


<210>  154
<211>  23
<212>  RNA
<213>  Artificial Sequence

<220>
<223>  Description of Artificial Sequence:  Target Sequence/siNA sense region

<400>  154

ugcugaugga caagaggaaa gac                                              23

<210>  155
<211>  23
<212>  RNA
<213>  Artificial Sequence

<220>
<223>  Description of Artificial Sequence:  Target Sequence/siNA sense region

<400>  155
agguguggau aaggcuuagg ugc                                              23

<210>  156
<211>  23
<212>  RNA
<213>  Artificial Sequence

<220>
<223>  Description of Artificial Sequence:  Target Sequence/siNA sense region

<400>  156
aaguccgaga gucaggguca cuc                                              23

<210>  157
<211>  23
<212>  RNA
<213>  Artificial Sequence

<220>
<223>  Description of Artificial Sequence:  Target Sequence/siNA sense region

<400>  157
aaaacacggc augugaacau ucu                                              23

<210>  158
<211>  23
<212>  RNA
<213>  Artificial Sequence

<220>
<223>  Description of Artificial Sequence:  Target Sequence/siNA sense region

<400>  158
ccucuacaaa caccugcaug ucc                                              23

<210>  159
<211>  23
<212>  RNA
<213>  Artificial Sequence

<220>
<223>  Description of Artificial Sequence:  Target Sequence/siNA sense region

<400>  159
ucucacauca acaaccgaga uca                                              23

<210> 160
<211> 23
<212> RNA
<213> Artificial Sequence

<220>
<223> Description of Artificial Sequence: Target Sequence/siNA sense region

<400> 160
caaggaagcc aggaauacag guu 23


<210> 161
<211> 23
<212> RNA
<213> Artificial Sequence

<220>
<223> Description of Artificial Sequence: Target Sequence/siNA sense region

<400> 161
gagaggagca cagauaccac caa 23


<210> 162
<211> 23
<212> RNA
<213> Artificial Sequence

<220>
<223> Description of Artificial Sequence: Target Sequence/siNA sense region

<400> 162
gauggcuucu augaggcuga gcu 23


<210> 163
<211> 23
<212> RNA
<213> Artificial Sequence

<220>
<223> Description of Artificial Sequence: Target Sequence/siNA sense region

<400> 163
ugugugacaa ggugcagaaa gag 23


<210> 164
<211> 23
<212> RNA
<213> Artificial Sequence

<220>
<223> Description of Artificial Sequence: Target Sequence/siNA sense region

<400> 164
uccuccagcu ucugguacuc ucc 23


<210> 165
<211> 23
<212> RNA

<213>  Artificial Sequence

<220>
<223>  Description of Artificial Sequence:  Target Sequence/siNA sense region

<400>  165
gauaugcugu ggugcuuaau gcc                                                23


<210>  166
<211>  23
<212>  RNA
<213>  Artificial Sequence

<220>
<223>  Description of Artificial Sequence:  Target Sequence/siNA sense region

<400>  166
acugcuggac augagaugga gag                                                23


<210>  167
<211>  23
<212>  RNA
<213>  Artificial Sequence

<220>
<223>  Description of Artificial Sequence:  Target Sequence/siNA sense region

<400>  167
uagaggcuac agggguuagc cug                                                23


<210>  168
<211>  23
<212>  RNA
<213>  Artificial Sequence

<220>
<223>  Description of Artificial Sequence:  Target Sequence/siNA sense region

<400>  168
cugaccuacc ucaaagggca guu                                                23


<210>  169
<211>  23
<212>  RNA
<213>  Artificial Sequence

<220>
<223>  Description of Artificial Sequence:  Target Sequence/siNA sense region

<400>  169
cugcgcacgu gggaagcccu ggc                                                23


<210>  170
<211>  23
<212>  RNA
<213>  Artificial Sequence

<220>

<223>  Description of Artificial Sequence:  Target Sequence/siNA sense region

<400>  170
ugcagaggcu gugcgagcgc ggc                                                23


<210>  171
<211>  23
<212>  RNA
<213>  Artificial Sequence

<220>
<223>  Description of Artificial Sequence:  Target Sequence/siNA sense region

<400>  171
cgucugggau gcgaacgggc cug                                                23


<210>  172
<211>  23
<212>  RNA
<213>  Artificial Sequence

<220>
<223>  Description of Artificial Sequence:  Target Sequence/siNA sense region

<400>  172
cuugggaacc acgcgcagug ccc                                                23


<210>  173
<211>  23
<212>  RNA
<213>  Artificial Sequence

<220>
<223>  Description of Artificial Sequence:  Target Sequence/siNA sense region

<400>  173
ugccaccacg ccgugcgcau cag                                                23


<210>  174
<211>  23
<212>  RNA
<213>  Artificial Sequence

<220>
<223>  Description of Artificial Sequence:  Target Sequence/siNA sense region

<400>  174
guggagacca ucuuucuggg uuc                                                23


<210>  175
<211>  23
<212>  RNA
<213>  Artificial Sequence

<220>
<223>  Description of Artificial Sequence:  Target Sequence/siNA sense region

<400>  175

agugucugga gcaaguugca aag                                          23


<210>  176
<211>  23
<212>  RNA
<213>  Artificial Sequence

<220>
<223>  Description of Artificial Sequence:  Target Sequence/siNA sense region

<400>  176
aucagagcca gucucaccuu caa                                          23


<210>  177
<211>  23
<212>  RNA
<213>  Artificial Sequence

<220>
<223>  Description of Artificial Sequence:  Target Sequence/siNA sense region

<400>  177
ugaaguguca cagccuguuu cug                                          23


<210>  178
<211>  23
<212>  RNA
<213>  Artificial Sequence

<220>
<223>  Description of Artificial Sequence:  Target Sequence/siNA sense region

<400>  178
agggauaaca cacugcaagu gga                                          23


<210>  179
<211>  23
<212>  RNA
<213>  Artificial Sequence

<220>
<223>  Description of Artificial Sequence:  Target Sequence/siNA sense region

<400>  179
ccauagcaac acucugagau ggc                                          23


<210>  180
<211>  23
<212>  RNA
<213>  Artificial Sequence

<220>
<223>  Description of Artificial Sequence:  Target Sequence/siNA sense region

<400>  180
gaaccugcuu uaguggggga uag                                          23

<210> 181
<211> 23
<212> RNA
<213> Artificial Sequence

<220>
<223> Description of Artificial Sequence: Target Sequence/siNA sense region

<400> 181
uagcacuuuu gggaggcaga gau 23


<210> 182
<211> 23
<212> RNA
<213> Artificial Sequence

<220>
<223> Description of Artificial Sequence: Target Sequence/siNA sense region

<400> 182
aaggacacca ugagcacuga aag 23


<210> 183
<211> 23
<212> RNA
<213> Artificial Sequence

<220>
<223> Description of Artificial Sequence: Target Sequence/siNA sense region

<400> 183
uuguuccuca gccucuucuc cuu 23


<210> 184
<211> 23
<212> RNA
<213> Artificial Sequence

<220>
<223> Description of Artificial Sequence: Target Sequence/siNA sense region

<400> 184
cuccuaccag accaagguca acc 23


<210> 185
<211> 23
<212> RNA
<213> Artificial Sequence

<220>
<223> Description of Artificial Sequence: Target Sequence/siNA sense region

<400> 185
uuaggccuuc cucucuccag aug 23


<210> 186
<211> 21
<212> RNA

<213>  Artificial Sequence

<220>
<223>  Description of Artificial Sequence:  siNA sense region


<220>
<221>  misc_feature
<222>  (1)..(8)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (14)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  186
uuccuccugg aaauucaacn n                                                21


<210>  187
<211>  21
<212>  RNA
<213>  Artificial Sequence

<220>
<223>  Description of Artificial Sequence:  siNA sense region


<220>
<221>  misc_feature
<222>  (1)..(6)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (8)..(8)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  187
ccucucauga ugcuggugun n                                                21


<210>  188
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (7)..(8)
<223>  2'-deoxy-2'-fluoro

<220>
<221> misc_feature
<222> (14)..(14)
<223> 2'-deoxy-2'-fluoro

<220>
<221> misc_feature
<222> (16)..(18)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 188
cgauagcuga aaacauucgn n 21


<210> 189
<211> 21
<212> RNA
<213> Artificial Sequence

<220>
<223> Description of Artificial Sequence: siNA sense region


<220>
<221> misc_feature
<222> (1)..(1)
<223> 5'-3 attached terminal deoxyabasic moeity

<220>
<221> misc_feature
<222> (3)..(3)
<223> 2'-deoxy-2'-fluoro

<220>
<221> misc_feature
<222> (5)..(5)
<223> 2'-deoxy-2'-fluoro

<220>
<221> misc_feature
<222> (8)..(9)
<223> 2'-deoxy-2'-fluoro

<220>
<221> misc_feature
<222> (12)..(12)
<223> 2'-deoxy-2'-fluoro

<220>
<221> misc_feature
<222> (16)..(18)
<223> 2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  189
aaugcagcug augaauccan n                                                21


<210>  190
<211>  21
<212>  RNA
<213>  Artificial Sequence

<220>
<223>  Description of Artificial Sequence:  siNA antisense region


<220>
<221>  misc_feature
<222>  (2)..(3)
<223>  2'-deoxy-2'-fluoro

<220>
<221>  misc_feature
<222>  (7)..(11)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  190
guugaauuuc caggaggaan n                                                21


<210>  191
<211>  21
<212>  RNA
<213>  Artificial Sequence

<220>
<223>  Description of Artificial Sequence:  siNA antisense region


<220>
<221>  misc_feature
<222>  (2)..(2)
<223>  2'-deoxy-2'-fluoro

<220>

<221>  misc_feature
<222>  (4)..(5)
<223>  2'-deoxy-2'-fluoro

<220>
<221>  misc_feature
<222>  (8)..(8)
<223>  2'-deoxy-2'-fluoro

<220>
<221>  misc_feature
<222>  (10)..(11)
<223>  2'-deoxy-2'-fluoro

<220>
<221>  misc_feature
<222>  (13)..(13)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  191
acaccagcau caugagaggn n                                                21


<210>  192
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (5)..(5)
<223>  2'-deoxy-2'-fluoro

<220>
<221>  misc_feature
<222>  (7)..(11)
<223>  2'-deoxy-2'-fluoro

<220>
<221>  misc_feature
<222>  (14)..(15)
<223>  2'-deoxy-2'-fluoro

<220>

<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  192
cgaauguuuu cagcuaucgn n                                                21


<210>  193
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (5)..(7)
<223>  2'-deoxy-2'-fluoro

<220>
<221>  misc_feature
<222>  (9)..(10)
<223>  2'-deoxy-2'-fluoro

<220>
<221>  misc_feature
<222>  (13)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>

<221>  misc_feature
<222>  (20)..(20)
<223>  Phosphorothioate 3'-Internucleotide Linkage

<400>  193
uggauucauc agcugcauun n                                                21


<210>  194
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  194
uuccuccugg aaauucaacn n                                                21


<210>  195
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  195
ccucucauga ugcuggugun n                                                21


<210>  196
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  196
cgauagcuga aaacauucgn n                                                21


<210>  197

<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  197
aaugcagcug augaauccan n                                                21


<210>  198
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  198
guugaauuuc caggaggaan n                                                21


<210>  199
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  199
acaccagcau caugagaggn n                                                21


<210>  200
<211>  21
<212>  RNA
<213>  Artificial Sequence

<220>
<223>  Description of Artificial Sequence:  siNA antisense region


<220>

<221>  misc_feature
<222>  (20)..(21)
<223>  n stands for thymidine

<400>  200
cgaauguuuu cagcuaucgn n                                                21


<210>  201
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  201
uggauucauc agcugcauun n                                                21


<210>  202
<211>  21
<212>  RNA
<213>  Artificial Sequence

<220>
<223>  Description of Artificial Sequence:  siNA sense region


<220>
<221>  misc_feature
<222>  (1)..(3)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (11)..(12)
<223>  2'-deoxy-2'-fluoro

<220>
<221>  misc_feature
<222>  (14)..(15)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)

<223>  n stands for thymidine

<220>
<221>  misc_feature
<222>  (21)..(21)
<223>  3'-3 attached terminal deoxyabasic moeity

<400>  202
uucgagaagg ucaucagcan n                                                21


<210>  203
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(6)
<223>  2'-deoxy-2'-fluoro

<220>
<221>  misc_feature
<222>  (8)..(10)
<223>  2'-deoxy-2'-fluoro

<220>
<221>  misc_feature
<222>  (16)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  203
ccaggugucu agaggcaacn n                                                21

<210>  204
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(3)
<223>  2'-deoxy-2'-fluoro

<220>
<221>  misc_feature
<222>  (7)..(9)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  204
accaagcuua aggagaggan n                                                21


<210>  205
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (8)..(13)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  205
cgguugaccu ucugaacaun n                                                21


<210>  206
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(4)
<223>  2'-deoxy-2'-fluoro

<220>
<221>  misc_feature
<222>  (7)..(7)
<223>  2'-deoxy-2'-fluoro

<220>
<221>  misc_feature
<222>  (10)..(16)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  206
ugcugaugac cuucucgaan n                                                21


<210>  207
<211>  21
<212>  RNA
<213>  Artificial Sequence

<220>
<223>  Description of Artificial Sequence:  siNA antisense region


<220>
<221>  misc_feature
<222>  (2)..(3)
<223>  2'-deoxy-2'-fluoro

<220>
<221>  misc_feature
<222>  (5)..(9)
<223>  2'-deoxy-2'-fluoro

<220>
<221>  misc_feature
<222>  (13)..(13)
<223>  2'-deoxy-2'-fluoro

<220>
<221>  misc_feature
<222>  (15)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  207
guugccucua gacaccuggn n                                                21


<210>  208
<211>  21
<212>  RNA
<213>  Artificial Sequence

<220>
<223>  Description of Artificial Sequence:  siNA antisense region


<220>
<221>  misc_feature

<222>  (1)..(10)
<223>  2'-deoxy-2'-fluoro

<220>
<221>  misc_feature
<222>  (14)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  208
uccucuccuu aagcuuggun n                                                21


<210>  209
<211>  21
<212>  RNA
<213>  Artificial Sequence

<220>
<223>  Description of Artificial Sequence:  siNA antisense region


<220>
<221>  misc_feature
<222>  (2)..(2)
<223>  2'-deoxy-2'-fluoro

<220>
<221>  misc_feature
<222>  (4)..(6)
<223>  2'-deoxy-2'-fluoro

<220>
<221>  misc_feature
<222>  (13)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature

<222>  (20)..(20)
<223>  Phosphorothioate 3'-Internucleotide Linkage

<400>  209
auguucagaa ggucaaccgn n                                                21


<210>  210
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  210
uucgagaagg ucaucagcan n                                                21


<210>  211
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  211
ccaggugucu agaggcaacn n                                                21


<210>  212
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  212
accaagcuua aggagaggan n                                                21


<210>  213
<211>  21

<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  213
cgguugaccu ucugaacaun n                                                21


<210>  214
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  214
ugcugaugac cuucucgaan n                                                21


<210>  215
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  215
guugccucua gacaccuggn n                                                21


<210>  216
<211>  21
<212>  RNA
<213>  Artificial Sequence

<220>
<223>  Description of Artificial Sequence:  siNA antisense region


<220>
<221>  misc_feature

<222>  (20)..(21)
<223>  n stands for thymidine

<400>  216
uccucuccuu aagcuuggun n                                                21


<210>  217
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  217
auguucagaa ggucaaccgn n                                                21


<210>  218
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  218
accaucaaua aggaagaagn n                                                21


<210>  219
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  219
aucaauaagg aagaagcccn n                                                21


<210>  220
<211>  21

<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  220
gaccaucaau aaggaagaan n                                                21


<210>  221
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  221
auaaggaaga agcccuucan n                                                21


<210>  222
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  222
cuucuuccuu auugauggun n                                                21


<210>  223
<211>  21
<212>  RNA
<213>  Artificial Sequence

<220>
<223>  Description of Artificial Sequence:  siNA antisense region


<220>
<221>  misc_feature

<222>  (20)..(21)
<223>  n stands for thymidine

<400>  223
gggcuucuuc cuuauugaun n                                                21


<210>  224
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  224
uucuuccuua uugauggucn n                                                21


<210>  225
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  225
ugaagggcuu cuuccuuaun n                                                21


<210>  226
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  226
gauuuaagca gaguucaaan n                                                21


<210>  227
<211>  21

<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  227
agaguucaaa agcccuucan n                                                21


<210>  228
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  228
cagaguucaa aagcccuucn n                                                21


<210>  229
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  229
auuuaagcag aguucaaaan n                                                21


<210>  230
<211>  21
<212>  RNA
<213>  Artificial Sequence

<220>
<223>  Description of Artificial Sequence:  siNA antisense region


<220>
<221>  misc_feature

<222>  (20)..(21)
<223>  n stands for thymidine

<400>  230
uuugaacucu gcuuaaaucn n                                                21


<210>  231
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  231
ugaagggcuu uugaacucun n                                                21


<210>  232
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  232
gaagggcuuu ugaacucugn n                                                21


<210>  233
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  233
uuuugaacuc ugcuuaaaun n                                                21


<210>  234
<211>  21

<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (10)..(11)
<223>  2'-deoxy-2'-fluoro

<220>
<221>  misc_feature
<222>  (13)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  234
ugggugaggu uaccaaccan n                                                21


<210>  235
<211>  21
<212>  RNA
<213>  Artificial Sequence

<220>
<223>  Description of Artificial Sequence:  siNA sense region


<220>
<221>  misc_feature
<222>  (1)..(1)

<223>  5'-3 attached terminal deoxyabasic moeity

<220>
<221>  misc_feature
<222>  (2)..(5)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  235
accuuggaca uggaagacun n                                                21


<210>  236
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(8)
<223>  2'-deoxy-2'-fluoro

<220>
<221>  misc_feature
<222>  (10)..(11)

<223>  2'-deoxy-2'-fluoro

<220>
<221>  misc_feature
<222>  (15)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  236
ugggaccugc uaaguguggn n                                                21


<210>  237
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (4)..(5)
<223>  2'-deoxy-2'-fluoro

<220>
<221>  misc_feature
<222>  (8)..(8)
<223>  2'-deoxy-2'-fluoro

<220>
<221>  misc_feature
<222>  (11)..(14)
<223>  2'-deoxy-2'-fluoro

<220>
<221>  misc_feature
<222>  (16)..(18)
<223>  2'-deoxy-2'-fluoro

<220>
<221>  misc_feature
<222>  (20)..(21)

<223>  n stands for thymidine

<220>
<221>  misc_feature
<222>  (20)..(20)
<223>  Phosphorothioate 3'-Internucleotide Linkage

<400>  237
ugguugguaa ccucacccan n                                                21


<210>  238
<211>  21
<212>  RNA
<213>  Artificial Sequence

<220>
<223>  Description of Artificial Sequence:  siNA antisense region


<220>
<221>  misc_feature
<222>  (3)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(14)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  238
agucuuccau guccaaggun n                                                21


<210>  239
<211>  21
<212>  RNA
<213>  Artificial Sequence

<220>
<223>  Description of Artificial Sequence:  siNA antisense region

<220>
<221>  misc_feature
<222>  (1)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (6)..(8)
<223>  2'-deoxy-2'-fluoro

<220>
<221>  misc_feature
<222>  (11)..(11)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  239
ccacacuuag cagguccan n                                                21


<210>  240
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  240
ugggugaggu uaccaaccan n                                               21


<210>  241
<211>  21
<212>  RNA
<213>  Artificial Sequence

<220>

<223>  Description of Artificial Sequence:  siNA sense region

<220>
<221>  misc_feature
<222>  (20)..(21)
<223>  n stands for thymidine

<400>  241
accuuggaca uggaagacun n                                                21

<210>  242
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  242
uaacauuggu gcaaagauun n                                                21

<210>  243
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  243
ugggaccugc uaaguguggn n                                                21

<210>  244
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  244

ugguugguaa ccucacccan n                                              21


<210>  245
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  245
agucuuccau guccaaggun n                                              21


<210>  246
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  246
aaucuuugca ccaauguuan n                                              21


<210>  247
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  247
ccacacuuag caggucccan n                                              21


<210>  248
<211>  21
<212>  RNA
<213>  Artificial Sequence

<220>

<223>  Description of Artificial Sequence:  siNA sense region

<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (6)..(7)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  248
uaacauuggu gcaaagauun n                                                21

<210>  249
<211>  21
<212>  RNA
<213>  Artificial Sequence

<220>
<223>  Description of Artificial Sequence:  siNA antisense region

<220>
<221>  misc_feature

<222>  (3)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(12)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  249
aaucuuugca ccaauguuan n                                                21


<210>  250
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (2)..(3)
<223>  2'-deoxy

<220>
<221>  misc_feature

<222>  (4)..(4)
<223>  2'-deoxy-2'-fluoro

<220>
<221>  misc_feature
<222>  (5)..(5)
<223>  2'-deoxy

<220>
<221>  misc_feature
<222>  (6)..(7)
<223>  2'-deoxy-2'-fluoro

<220>
<221>  misc_feature
<222>  (8)..(9)
<223>  2'-deoxy

<220>
<221>  misc_feature
<222>  (10)..(10)
<223>  2'-deoxy-2'-fluoro

<220>
<221>  misc_feature
<222>  (11)..(11)
<223>  2'-deoxy

<220>
<221>  misc_feature
<222>  (12)..(12)
<223>  2'-deoxy-2'-fluoro

<220>
<221>  misc_feature
<222>  (13)..(17)
<223>  2'-deoxy

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  250
uaacauuggu gcaaagauun n                                                21


<210>  251
<211>  21
<212>  RNA
<213>  Artificial Sequence

<220>

<223>  Description of Artificial Sequence:  siNA antisense region

<220>
<221>  misc_feature
<222>  (1)..(2)
<223>  2'-deoxy

<220>
<221>  misc_feature
<222>  (3)..(7)
<223>  2'-deoxy-2'-fluoro

<220>
<221>  misc_feature
<222>  (8)..(8)
<223>  2'-deoxy

<220>
<221>  misc_feature
<222>  (9)..(9)
<223>  2'-deoxy-2'-fluoro

<220>
<221>  misc_feature
<222>  (10)..(10)
<223>  2'-deoxy

<220>
<221>  misc_feature
<222>  (11)..(12)
<223>  2'-deoxy-2'-fluoro

<220>
<221>  misc_feature
<222>  (13)..(14)
<223>  2'-deoxy

<220>
<221>  misc_feature
<222>  (15)..(15)
<223>  2'-deoxy-2'-fluoro

<220>
<221>  misc_feature
<222>  (16)..(16)
<223>  2'-deoxy

<220>
<221>  misc_feature
<222>  (17)..(18)
<223>  2'-deoxy-2'-fluoro

<220>
<221>  misc_feature
<222>  (19)..(19)
<223>  2'-deoxy

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  251
aaucuuugca ccaauguuan n                                                21


<210>  252
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (8)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (13)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  252
uuagaaacgu gguuacaaun n                                                21

<210>  253
<211>  21
<212>  RNA
<213>  Artificial Sequence

<220>
<223>  Description of Artificial Sequence:  inverted control/siNA antisense

<220>
<221>  misc_feature
<222>  (2)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (8)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  253
auuguaacca cguuucuaan n                                                21

<210>  254
<211>  21
<212>  RNA
<213>  Artificial Sequence

<220>
<223>  Description of Artificial Sequence:  inverted control/siNA sense

<220>
<221>  misc_feature

<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(7)
<223>  2'-deoxy

<220>
<221>  misc_feature
<222>  (8)..(8)
<223>  2'-deoxy-2'-fluoro

<220>
<221>  misc_feature
<222>  (9)..(9)
<223>  2'-deoxy

<220>
<221>  misc_feature
<222>  (10)..(10)
<223>  2'-deoxy-2'-fluoro

<220>
<221>  misc_feature
<222>  (11)..(12)
<223>  2'-deoxy

<220>
<221>  misc_feature
<222>  (13)..(14)
<223>  2'-deoxy-2'-fluoro

<220>
<221>  misc_feature
<222>  (15)..(15)
<223>  2'-deoxy

<220>
<221>  misc_feature
<222>  (16)..(16)
<223>  2'-deoxy-2'-fluoro

<220>
<221>  misc_feature
<222>  (17)..(18)
<223>  2'-deoxy

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  254
uuagaaacgu gguuacaaun n                                                21


<210>  255
<211>  21
<212>  RNA
<213>  Artificial Sequence

<220>
<223>  Description of Artificial Sequence:  inverted control/siNA antisense


<220>
<221>  misc_feature
<222>  (1)..(1)
<223>  2'-deoxy

<220>
<221>  misc_feature
<222>  (2)..(3)
<223>  2'-deoxy-2'-fluoro

<220>
<221>  misc_feature
<222>  (4)..(4)
<223>  2'-deoxy

<220>
<221>  misc_feature
<222>  (5)..(5)
<223>  2'-deoxy-2'-fluoro

<220>
<221>  misc_feature
<222>  (6)..(7)
<223>  2'-deoxy

<220>
<221>  misc_feature
<222>  (8)..(9)
<223>  2'-deoxy-2'-fluoro

<220>
<221>  misc_feature
<222>  (10)..(10)
<223>  2'-deoxy

<220>
<221>  misc_feature
<222>  (11)..(11)
<223>  2'-deoxy-2'-fluoro

<220>
<221>  misc_feature
<222>  (12)..(12)
<223>  2'-deoxy

<220>
<221>  misc_feature
<222>  (13)..(17)
<223>  2'-deoxy-2'-fluoro

<220>
<221>  misc_feature
<222>  (18)..(19)
<223>  2'-deoxy

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  255
auuguaacca cguuucuaan n                                                21


<210>  256
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(3)
<223>  2'-deoxy-2'-fluoro

<220>
<221>  misc_feature
<222>  (5)..(9)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  256
gcugucucug aagacucugn n                                                21


<210>  257
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (6)..(6)
<223>  2'-deoxy-2'-fluoro

<220>
<221>  misc_feature
<222>  (9)..(11)
<223>  2'-deoxy-2'-fluoro

<220>
<221>  misc_feature
<222>  (13)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  257
uuacguggcc uguuucaacn n                                                21


<210>  258

<211>  21
<212>  RNA
<213>  Artificial Sequence

<220>
<223>  Description of Artificial Sequence:  siNA sense region


<220>
<221>  misc_feature
<222>  (1)..(3)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (7)..(8)
<223>  2'-deoxy-2'-fluoro

<220>
<221>  misc_feature
<222>  (15)..(16)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  258
uuuggaucag ggaguuggan n                                                21


<210>  259
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (6)..(10)
<223>  2'-deoxy-2'-fluoro

<220>
<221>  misc_feature

<222>  (16)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  259
cagagucuuc agagacagcn n                                                21


<210>  260
<211>  21
<212>  RNA
<213>  Artificial Sequence

<220>
<223>  Description of Artificial Sequence:  siNA antisense region


<220>
<221>  misc_feature
<222>  (2)..(3)
<223>  2'-deoxy-2'-fluoro

<220>
<221>  misc_feature
<222>  (8)..(8)
<223>  2'-deoxy-2'-fluoro

<220>
<221>  misc_feature
<222>  (12)..(13)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature

<222>  (20)..(20)
<223>  Phosphorothioate 3'-Internucleotide Linkage

<400>  260
guugaaacag gccacguaan n                                                21


<210>  261
<211>  21
<212>  RNA
<213>  Artificial Sequence

<220>
<223>  Description of Artificial Sequence:  siNA antisense region


<220>
<221>  misc_feature
<222>  (1)..(3)
<223>  2'-deoxy-2'-fluoro

<220>
<221>  misc_feature
<222>  (6)..(11)
<223>  2'-deoxy-2'-fluoro

<220>
<221>  misc_feature
<222>  (14)..(16)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  261
uccaacuccc ugauccaaan n                                                21


<210>  262
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  262
gcugucucug aagacucugn n                                                21

<210>  263
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  263
gggaugauca acaggguagn n                                                21


<210>  264
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  264
uuacguggcc uguuucaacn n                                                21


<210>  265
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  265
uuuggaucag ggaguuggan n                                                21


<210>  266
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  266
cagagucuuc agagacagcn n                                                21


<210>  267
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  267
cuacccuguu gaucaucccn n                                                21


<210>  268
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  268
guugaaacag gccacguaan n                                                21


<210>  269
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  269
uccaacuccc ugauccaaan n                                                21

<210>  270
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (8)..(9)
<223>  2'-deoxy-2'-fluoro

<220>
<221>  misc_feature
<222>  (12)..(12)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  270
gggaugauca acaggguagn n                                                21


<210>  271
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>

<221>  misc_feature
<222>  (4)..(7)
<223>  2'-deoxy-2'-fluoro

<220>
<221>  misc_feature
<222>  (9)..(10)
<223>  2'-deoxy-2'-fluoro

<220>
<221>  misc_feature
<222>  (13)..(14)
<223>  2'-deoxy-2'-fluoro

<220>
<221>  misc_feature
<222>  (16)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  271
cuacccuguu gaucaucccn n                                                21


<210>  272
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (8)..(8)
<223>  2'-deoxy-2'-fluoro

<220>
<221>  misc_feature
<222>  (11)..(12)
<223>  2'-deoxy-2'-fluoro

<220>

<221>  misc_feature
<222>  (15)..(15)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  272
gaugggacaa cuaguagggn n                                                21


<210>  273
<211>  21
<212>  RNA
<213>  Artificial Sequence

<220>
<223>  Description of Artificial Sequence:  inverted control/siNA antisense


<220>
<221>  misc_feature
<222>  (1)..(4)
<223>  2'-deoxy-2'-fluoro

<220>
<221>  misc_feature
<222>  (6)..(7)
<223>  2'-deoxy-2'-fluoro

<220>
<221>  misc_feature
<222>  (10)..(11)
<223>  2'-deoxy-2'-fluoro

<220>
<221>  misc_feature
<222>  (13)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  273

cccuacuagu ugucccaucn n                                                21

<210>  274
<211>  21
<212>  RNA
<213>  Artificial Sequence

<220>
<223>  Description of Artificial Sequence:  siNA sense region


<220>
<221>  misc_feature
<222>  (1)..(4)
<223>  2'-deoxy

<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (6)..(7)
<223>  2'-deoxy

<220>
<221>  misc_feature
<222>  (8)..(9)
<223>  2'-deoxy-2'-fluoro

<220>
<221>  misc_feature
<222>  (10)..(11)
<223>  2'-deoxy

<220>
<221>  misc_feature
<222>  (12)..(12)
<223>  2'-deoxy-2'-fluoro

<220>
<221>  misc_feature
<222>  (13)..(16)
<223>  2'-deoxy

<220>
<221>  misc_feature
<222>  (17)..(17)
<223>  2'-deoxy-2'-fluoro

<220>
<221>  misc_feature
<222>  (18)..(19)
<223>  2'-deoxy

<220>

<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  274
gggaugauca acaggguagn n                                                21


<210>  275
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (3)..(3)
<223>  2'-deoxy

<220>
<221>  misc_feature
<222>  (4)..(7)
<223>  2'-deoxy-2'-fluoro

<220>
<221>  misc_feature
<222>  (8)..(8)
<223>  2'-deoxy

<220>
<221>  misc_feature
<222>  (9)..(10)
<223>  2'-deoxy-2'-fluoro

<220>
<221>  misc_feature
<222>  (11)..(12)
<223>  2'-deoxy

<220>
<221>  misc_feature
<222>  (13)..(14)
<223>  2'-deoxy-2'-fluoro

<220>
<221>  misc_feature
<222>  (15)..(15)
<223>  2'-deoxy

<220>

<221>  misc_feature
<222>  (16)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  275
cuacccuguu gaucaucccn n                                                21


<210>  276
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (4)..(7)
<223>  2'-deoxy

<220>
<221>  misc_feature
<222>  (8)..(8)
<223>  2'-deoxy-2'-fluoro

<220>
<221>  misc_feature
<222>  (9)..(10)
<223>  2'-deoxy

<220>
<221>  misc_feature
<222>  (11)..(12)
<223>  2'-deoxy-2'-fluoro

<220>

<221>  misc_feature
<222>  (13)..(14)
<223>  2'-deoxy

<220>
<221>  misc_feature
<222>  (15)..(15)
<223>  2'-deoxy-2'-fluoro

<220>
<221>  misc_feature
<222>  (16)..(19)
<223>  2'-deoxy

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  276
gaugggacaa cuaguagggn n                                                21


<210>  277
<211>  21
<212>  RNA
<213>  Artificial Sequence

<220>
<223>  Description of Artificial Sequence:  inverted control/siNA antisense


<220>
<221>  misc_feature
<222>  (1)..(4)
<223>  2'-deoxy-2'-fluoro

<220>
<221>  misc_feature
<222>  (5)..(5)
<223>  2'-deoxy

<220>
<221>  misc_feature
<222>  (6)..(7)
<223>  2'-deoxy-2'-fluoro

<220>
<221>  misc_feature
<222>  (8)..(9)
<223>  2'-deoxy

<220>
<221>  misc_feature
<222>  (10)..(11)
<223>  2'-deoxy-2'-fluoro

<220>

<221>  misc_feature
<222>  (12)..(12)
<223>  2'-deoxy

<220>
<221>  misc_feature
<222>  (13)..(16)
<223>  2'-deoxy-2'-fluoro

<220>
<221>  misc_feature
<222>  (17)..(17)
<223>  2'-deoxy

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  277
cccuacuagu ugucccaucn n                                                21


<210>  278
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (6)..(6)
<223>  2'-deoxy-2'-fluoro

<220>

<221>  misc_feature
<222>  (11)..(14)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  278
uguagugggg uucuaggcan n                                                21


<210>  279
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (8)..(13)
<223>  2'-deoxy-2'-fluoro

<220>
<221>  misc_feature
<222>  (15)..(19)
<223>  2'-deoxy-2'-fluoro

<220>
<221>  misc_feature
<222>  (20)..(21)
<223>  n stands for thymidine

<220>

<221>  misc_feature
<222>  (21)..(21)
<223>  3'-3 attached terminal deoxyabasic moeity

<400>  279
acacaaaccu ucugccuuun n                                                21


<210>  280
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(9)
<223>  2'-deoxy-2'-fluoro

<220>
<221>  misc_feature
<222>  (11)..(12)
<223>  2'-deoxy-2'-fluoro

<220>
<221>  misc_feature
<222>  (14)..(15)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  280

acauuguuug cugcuauugn n                                                21

<210>  281
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(5)
<223>  2'-deoxy-2'-fluoro

<220>
<221>  misc_feature
<222>  (10)..(13)
<223>  2'-deoxy-2'-fluoro

<220>
<221>  misc_feature
<222>  (15)..(16)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  281
ugccuagaac cccacuacan n                                                21


<210>  282
<211>  21
<212>  RNA
<213>  Artificial Sequence

<220>
<223>  Description of Artificial Sequence:  siNA antisense region


<220>
<221>  misc_feature
<222>  (6)..(6)

<223>  2'-deoxy-2'-fluoro

<220>
<221>  misc_feature
<222>  (13)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  282
aaaggcagaa gguuugugun n                                                21


<210>  283
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (4)..(4)
<223>  2'-deoxy-2'-fluoro

<220>
<221>  misc_feature
<222>  (7)..(7)
<223>  2'-deoxy-2'-fluoro

<220>
<221>  misc_feature
<222>  (10)..(10)
<223>  2'-deoxy-2'-fluoro

<220>
<221>  misc_feature
<222>  (14)..(14)

<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  283
caauagcagc aaacaaugun n                                                21


<210>  284
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  284
   acacuuccuc uccaaaaugn n                                               21


<210>  285
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  285
   uguagugggg uucuaggcan n                                               21


<210>  286

<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  286
acacaaaccu ucugccuuun n                                                21


<210>  287
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  287
acauuguuug cugcuauugn n                                                21


<210>  288
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  288
cauuuuggag aggaagugun n                                                21


<210>  289
<211>  21
<212>  RNA
<213>  Artificial Sequence

<220>
<223>  Description of Artificial Sequence:  siNA antisense region


<220>

<221>  misc_feature
<222>  (20)..(21)
<223>  n stands for thymidine

<400>  289
ugccuagaac cccacuacan n                                                21


<210>  290
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  290
aaaggcagaa gguuugugun n                                                21


<210>  291
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  291
caauagcagc aaacaaugun n                                                21


<210>  292
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221> misc_feature
<222> (4)..(13)
<223> 2'-deoxy-2'-fluoro

<220>
<221> misc_feature
<222> (18)..(18)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 292
acacuuccuc uccaaaaugn n 21


<210> 293
<211> 21
<212> RNA
<213> Artificial Sequence

<220>
<223> Description of Artificial Sequence: siNA antisense region


<220>
<221> misc_feature
<222> (1)..(1)
<223> 2'-deoxy-2'-fluoro

<220>
<221> misc_feature
<222> (2)..(2)
<223> 2'-deoxy-2'-fluoro

<220>
<221> misc_feature
<222> (3)..(6)
<223> 2'-deoxy-2'-fluoro

<220>
<221> misc_feature
<222> (4)..(13)
<223> 2'-deoxy-2'-fluoro

<220>
<221> misc_feature
<222> (17)..(17)
<223> 2'-deoxy-2'-fluoro

<220>
<221> misc_feature
<222> (18)..(18)
<223> 2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  293
cauuuuggag aggaagugun n                                                21


<210>  294
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (7)..(16)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  294
guaaaaccuc uccuucacan n                                                21


<210>  295

<211>  21
<212>  RNA
<213>  Artificial Sequence

<220>
<223>  Description of Artificial Sequence:  inverted control/siNA antisense


<220>
<221>  misc_feature
<222>  (1)..(1)
<223>  2'-deoxy-2'-fluoro

<220>
<221>  misc_feature
<222>  (3)..(3)
<223>  2'-deoxy-2'-fluoro

<220>
<221>  misc_feature
<222>  (14)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  295
ugugaaggag agguuuuacn n                                                21


<210>  296
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  296
ggacacugag acugagggun n                                                21


<210>  297
<211>  21
<212>  RNA

<213>  Artificial Sequence

<220>
<223>  Description of Artificial Sequence:  siNA sense region


<220>
<221>  misc_feature
<222>  (20)..(21)
<223>  n stands for thymidine

<400>  297
aucaagcuag cagacuuugn n                                                21


<210>  298
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  298
cucaccuucu agucuuggcn n                                                21


<210>  299
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  299
acguuagauu ugccguaccn n                                                21


<210>  300
<211>  21
<212>  RNA
<213>  Artificial Sequence

<220>
<223>  Description of Artificial Sequence:  siNA antisense region


<220>
<221>  misc_feature
<222>  (20)..(21)

<223>  n stands for thymidine

<400>  300
acccucaguc ucaguguccn n                                                21


<210>  301
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  301
caaagucugc uagcuugaun n                                                21


<210>  302
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  302
gccaagacua gaaggugagn n                                                21


<210>  303
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  303
gguacggcaa aucuaacgun n                                                21


<210>  304
<211>  21
<212>  RNA

<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  304
uggucacagg agagaaggcn n                                                21


<210>  305
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (6)..(7)
<223>  2'-deoxy-2'-fluoro

<220>
<221> misc_feature
<222> (11)..(12)
<223> 2'-deoxy-2'-fluoro

<220>
<221> misc_feature
<222> (14)..(15)
<223> 2'-deoxy-2'-fluoro

<220>
<221> misc_feature
<222> (18)..(18)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 305
agaaguuggg cuaucaaugn n                                                21


<210> 306
<211> 21
<212> RNA
<213> Artificial Sequence

<220>
<223> Description of Artificial Sequence: siNA sense region


<220>
<221> misc_feature
<222> (1)..(3)
<223> 2'-deoxy-2'-fluoro

<220>
<221> misc_feature
<222> (1)..(1)
<223> 5'-3 attached terminal deoxyabasic moeity

<220>
<221> misc_feature
<222> (10)..(10)
<223> 2'-deoxy-2'-fluoro

<220>
<221> misc_feature
<222> (12)..(12)
<223> 2'-deoxy-2'-fluoro

<220>
<221> misc_feature
<222> (16)..(19)
<223> 2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  306
uucaggggac augaguuuun n                                                21


<210>  307
<211>  21
<212>  RNA
<213>  Artificial Sequence

<220>
<223>  Description of Artificial Sequence:  siNA antisense region


<220>
<221>  misc_feature
<222>  (2)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  307
gccuucucuc cugugaccan n                                                21


<210>  308
<211>  21
<212>  RNA
<213>  Artificial Sequence

<220>
<223>  Description of Artificial Sequence:  siNA antisense region


<220>

<221>  misc_feature
<222>  (1)..(1)
<223>  2'-deoxy-2'-fluoro

<220>
<221>  misc_feature
<222>  (3)..(4)
<223>  2'-deoxy-2'-fluoro

<220>
<221>  misc_feature
<222>  (7)..(7)
<223>  2'-deoxy-2'-fluoro

<220>
<221>  misc_feature
<222>  (10)..(12)
<223>  2'-deoxy-2'-fluoro

<220>
<221>  misc_feature
<222>  (15)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  308
cauugauagc ccaacuucun n                                                21


<210>  309
<211>  21
<212>  RNA
<213>  Artificial Sequence

<220>
<223>  Description of Artificial Sequence:  siNA antisense region


<220>
<221>  misc_feature
<222>  (5)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(16)
<223>  2'-deoxy-2'-fluoro

<220>

<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  309
aaaacucaug uccccugaan n                                                21


<210>  310
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  310
uggucacagg agagaaggcn n                                                21


<210>  311
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  311
agaaguuggg cuaucaaugn n                                                21


<210>  312
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  312
agggugaugg auuggaguun n                                                21

<210>  313
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  313
uucaggggac augaguuuun n                                                21

<210>  314
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  314
gccuucucuc cugugaccan n                                                21

<210>  315
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  315
cauugauagc ccaacuucun n                                                21

<210>  316
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  316
aacuccaauc caucacccun n                                                21

<210>  317
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  317
aaaacucaug uccccugaan n                                                21

<210>  318
<211>  21
<212>  RNA
<213>  Artificial Sequence

<220>
<223>  Description of Artificial Sequence:  siNA sense region

<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (8)..(8)
<223>  2'-deoxy-2'-fluoro

<220>
<221>  misc_feature
<222>  (12)..(13)
<223>  2'-deoxy-2'-fluoro

<220>
<221>  misc_feature
<222>  (18)..(19)

<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  318
agggugaugg auuggaguun n                                                21


<210>  319
<211>  21
<212>  RNA
<213>  Artificial Sequence

<220>
<223>  Description of Artificial Sequence:  siNA antisense region


<220>
<221>  misc_feature
<222>  (3)..(6)
<223>  2'-deoxy-2'-fluoro

<220>
<221>  misc_feature
<222>  (9)..(11)
<223>  2'-deoxy-2'-fluoro

<220>
<221>  misc_feature
<222>  (13)..(14)
<223>  2'-deoxy-2'-fluoro

<220>
<221>  misc_feature
<222>  (16)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  319
aacuccaauc caucacccun n                                                21


<210>  320
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (7)..(8)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(15)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  320
uugagguuag guagugggan n                                                21


<210>  321
<211>  21
<212>  RNA
<213>  Artificial Sequence

<220>
<223>  Description of Artificial Sequence:  inverted control/siNA antisense


<220>
<221>  misc_feature
<222>  (1)..(4)
<223>  2'-deoxy-2'-fluoro

<220>
<221>  misc_feature
<222>  (6)..(7)
<223>  2'-deoxy-2'-fluoro

<220>
<221>  misc_feature
<222>  (9)..(11)
<223>  2'-deoxy-2'-fluoro

<220>
<221>  misc_feature
<222>  (14)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  321
ucccacuacc uaaccucaan n                                                21


<210>  322
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  322
uaaccucgua cuggugccuc c                                                21


<210>  323
<211>  21
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature

<222>  (21)..(21)
<223>  3'-3 attached terminal deoxyabasic moeity

<400>  323
ggaggcacca guacgagguu a                                                21


<210>  324
<211>  21
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  324
aaacuccaag auccccaauc a                                                21


<210>  325
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  325
ugauugggga ucuuggaguu u                                                21


<210>  326
<211>  21
<212>  RNA
<213>  Artificial Sequence

<220>
<223>  Description of Artificial Sequence:  siNA antisense region


<220>

<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  326
gcaaaaaccc ugugauuucc u                                                21


<210>  327
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  327
aggaaaucac aggguuuuug c                                                21


<210>  328
<211>  21
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  328
uuggucaguu ucuggcaguu c                                                21


<210>  329
<211>  21
<212>  RNA
<213>  Artificial Sequence

<220>
<223> Description of Artificial Sequence: siNA sense region

<220>
<221> misc_feature
<222> (1)..(1)
<223> 5'-3 attached terminal deoxyabasic moeity

<220>
<221> misc_feature
<222> (21)..(21)
<223> 3'-3 attached terminal deoxyabasic moeity

<400> 329
gaacugccag aaacugacca a 21

<210> 330
<211> 21
<212> RNA
<213> Artificial Sequence

<220>
<223> Description of Artificial Sequence: inverted control/siNA sense

<220>
<221> misc_feature
<222> (1)..(1)
<223> 5'-3 attached terminal deoxyabasic moeity

<220>
<221> misc_feature
<222> (21)..(21)
<223> 3'-3 attached terminal deoxyabasic moeity

<400> 330
ccuccguggu caugcuccaa u 21

<210> 331
<211> 21
<212> RNA
<213> Artificial Sequence

<220>
<223> Description of Artificial Sequence: inverted control/siNA sense

<220>
<221> misc_feature
<222> (1)..(1)
<223> 5'-3 attached terminal deoxyabasic moeity

<220>
<221> misc_feature
<222> (21)..(21)
<223> 3'-3 attached terminal deoxyabasic moeity

<400> 331
auuggagcau gaccacggag g 21

<210>  332
<211>  23
<212>  RNA
<213>  Artificial Sequence

<220>
<223>  Description of Artificial Sequence:  siNA sense region

<400>  332
uaaccucgua cuggugccuc cuu                                              23


<210>  333
<211>  23
<212>  RNA
<213>  Artificial Sequence

<220>
<223>  Description of Artificial Sequence:  siNA antisense region

<400>  333
ggaggcacca guacgagguu auu                                              23


<210>  334
<211>  23
<212>  RNA
<213>  Artificial Sequence

<220>
<223>  Description of Artificial Sequence:  siNA antisense region

<400>  334
aaacuccaag auccccaauc auu                                              23


<210>  335
<211>  23
<212>  RNA
<213>  Artificial Sequence

<220>
<223>  Description of Artificial Sequence:  siNA sense region

<400>  335
ugauugggga ucuuggaguu uuu                                              23


<210>  336
<211>  23
<212>  RNA
<213>  Artificial Sequence

<220>
<223>  Description of Artificial Sequence:  siNA antisense region

<400>  336
gcaaaaaccc ugugauuucc uuu                                              23


<210>  337

<211>  23
<212>  RNA
<213>  Artificial Sequence

<220>
<223>  Description of Artificial Sequence:  siNA sense region

<400>  337
   aggaaaucac aggguuuuug cuu                                                23


<210>  338
<211>  23
<212>  RNA
<213>  Artificial Sequence

<220>
<223>  Description of Artificial Sequence:  siNA antisense region

<400>  338
uuggucaguu ucuggcaguu cuu                                               23


<210>  339
<211>  23
<212>  RNA
<213>  Artificial Sequence

<220>
<223>  Description of Artificial Sequence:  siNA sense region

<400>  339
gaacugccag aaacugacca auu                                               23


<210>  340
<211>  23
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  340
uaaccucgua cuggugccuc cuu                                               23


<210>  341
<211>  23
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  341
ggaggcacca guacgagguu auu                                              23


<210>  342
<211>  23
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  342
aaacuccaag auccccaauc auu                                              23


<210>  343
<211>  23
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  343
ugauugggga ucuuggaguu uuu                                              23

<210>  344
<211>  23
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  344
gcaaaaaccc ugugauuucc uuu                                              23


<210>  345
<211>  23
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  345
aggaaaucac aggguuuuug cuu                                              23


<210>  346
<211>  23
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature

<222>  (21)..(21)
<223>  3'-3 attached terminal deoxyabasic moeity

<400>  346
uuggucaguu ucuggcaguu cuu                                              23


<210>  347
<211>  23
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  347
gaacugccag aaacugacca auu                                              23


<210>  348
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (7)..(8)
<223>  2'-deoxy-2'-fluoro

<220>
<221>  misc_feature
<222>  (14)..(15)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  348
gaacugccag aaacugaccn n                                                21


<210>  349
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (7)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (15)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  349
aggaaaucac aggguuuuun n                                                21


<210>  350
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (11)..(12)
<223>  2'-deoxy-2'-fluoro

<220>
<221>  misc_feature
<222>  (15)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  350
guuccgugag uugaucaucn n                                                21


<210>  351
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy-2'-fluoro

<220>

<221>  misc_feature
<222>  (1)..(1)
<223>  5'-3 attached terminal deoxyabasic moeity

<220>
<221>  misc_feature
<222>  (6)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  351
ccaaguccua cagacuccan n                                                21


<210>  352
<211>  21
<212>  RNA
<213>  Artificial Sequence

<220>
<223>  Description of Artificial Sequence:  siNA antisense region


<220>
<221>  misc_feature
<222>  (3)..(4)
<223>  2'-deoxy-2'-fluoro

<220>
<221>  misc_feature
<222>  (7)..(11)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(19)
<223>  2'-deoxy-2'-fluoro

<220>

<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  352
ggucaguuuc uggcaguucn n                                                21


<210>  353
<211>  21
<212>  RNA
<213>  Artificial Sequence

<220>
<223>  Description of Artificial Sequence:  siNA antisense region


<220>
<221>  misc_feature
<222>  (6)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (14)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  353
aaaaacccug ugauuuccun n                                                21


<210>  354
<211>  21
<212>  RNA
<213>  Artificial Sequence

<220>
<223>  Description of Artificial Sequence:  siNA antisense region


<220>
<221>  misc_feature
<222>  (3)..(3)

<223>  2'-deoxy-2'-fluoro

<220>
<221>  misc_feature
<222>  (6)..(7)
<223>  2'-deoxy-2'-fluoro

<220>
<221>  misc_feature
<222>  (10)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  354
gaugaucaac ucacggaacn n                                                21


<210>  355
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (6)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (15)..(17)

<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  355
uggagucugu aggacuuggn n                                                21


<210>  356
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  356
gaacugccag aaacugaccn n                                                21


<210>  357
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  357
aggaaaucac aggguuuuun n                                                21


<210>  358
<211>  21
<212>  RNA
<213>  Artificial Sequence

<220>
<223>  Description of Artificial Sequence:  siNA sense region


<220>
<221>  misc_feature

<222>  (20)..(21)
<223>  n stands for thymidine

<400>  358
guuccgugag uugaucaucn n                                                21


<210>  359
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  359
ccaaguccua cagacuccan n                                                21


<210>  360
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  360
ggucaguuuc uggcaguucn n                                                21


<210>  361
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  361
aaaaacccug ugauuuccun n                                                21


<210>  362
<211>  21

<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  362
gaugaucaac ucacggaacn n                                                21


<210>  363
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  363
uggagucugu aggacuuggn n                                                21


<210>  364
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  5'-3 attached terminal deoxyabasic moeity

<220>
<221>  misc_feature
<222>  (2)..(5)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(14)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  364
accucagaca uccugaaccn n                                                21


<210>  365
<211>  21
<212>  RNA
<213>  Artificial Sequence

<220>
<223>  Description of Artificial Sequence:  inverted control/siNA antisense


<220>
<221>  misc_feature
<222>  (3)..(5)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(14)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  365
gguucaggau gucugaggun n                                                21


<210>  366

<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (6)..(6)
<223>  2'-deoxy-2'-fluoro

<220>
<221>  misc_feature
<222>  (9)..(11)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  366
gugaauggcu caaggaacun n                                                21


<210>  367
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature

<222>  (5)..(6)
<223>  2'-deoxy-2'-fluoro

<220>
<221>  misc_feature
<222>  (8)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (15)..(15)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  367
ggaacugugc aagaugaccn n                                                21


<210>  368
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (7)..(9)
<223>  2'-deoxy-2'-fluoro

<220>
<221>  misc_feature

<222>  (12)..(13)
<223>  2'-deoxy-2'-fluoro

<220>
<221>  misc_feature
<222>  (15)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  368
aagcugcuca accaucuccn n                                                21


<210>  369
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (7)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature

<222>  (21)..(21)
<223>  3'-3 attached terminal deoxyabasic moeity

<400>  369
caaccaucuc cuuccacagn n                                                21


<210>  370
<211>  21
<212>  RNA
<213>  Artificial Sequence

<220>
<223>  Description of Artificial Sequence:  siNA antisense region


<220>
<221>  misc_feature
<222>  (3)..(8)
<223>  2'-deoxy-2'-fluoro

<220>
<221>  misc_feature
<222>  (12)..(13)
<223>  2'-deoxy-2'-fluoro

<220>
<221>  misc_feature
<222>  (15)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  370
aguuccuuga gccauucacn n                                                21


<210>  371
<211>  21
<212>  RNA
<213>  Artificial Sequence

<220>
<223>  Description of Artificial Sequence:  siNA antisense region


<220>
<221>  misc_feature
<222>  (3)..(4)
<223>  2'-deoxy-2'-fluoro

<220>
<221>  misc_feature
<222>  (6)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(13)
<223>  2'-deoxy-2'-fluoro

<220>
<221>  misc_feature
<222>  (16)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  371
ggucaucuug cacaguuccn n                                                21


<210>  372
<211>  21
<212>  RNA
<213>  Artificial Sequence

<220>
<223>  Description of Artificial Sequence:  siNA antisense region


<220>
<221>  misc_feature
<222>  (6)..(6)
<223>  2'-deoxy-2'-fluoro

<220>
<221>  misc_feature
<222>  (9)..(10)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(19)
<223>  2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400> 372
ggagaugguu gagcagcuun n                                                21


<210> 373
<211> 21
<212> RNA
<213> Artificial Sequence

<220>
<223> Description of Artificial Sequence: siNA antisense region


<220>
<221> misc_feature
<222> (1)..(2)
<223> 2'-deoxy-2'-fluoro

<220>
<221> misc_feature
<222> (4)..(4)
<223> 2'-deoxy-2'-fluoro

<220>
<221> misc_feature
<222> (14)..(14)
<223> 2'-deoxy-2'-fluoro

<220>
<221> misc_feature
<222> (17)..(18)
<223> 2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400> 373
cuguggaagg agaugguugn n                                                21


<210> 374
<211> 21
<212> RNA
<213> Artificial Sequence

<220>
<223>  Description of Artificial Sequence:  siNA sense region


<220>
<221>  misc_feature
<222>  (20)..(21)
<223>  n stands for thymidine

<400>  374
gugaauggcu caaggaacun n                                                21


<210>  375
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  375
ggaacugugc aagaugaccn n                                                21


<210>  376
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  376
aagcugcuca accaucuccn n                                                21


<210>  377
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  377
caaccaucuc cuuccacagn n                                                21

<210>  378
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  378
aguuccuuga gccauucacn n                                                21

<210>  379
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  379
ggucaucuug cacaguuccn n                                                21

<210>  380
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  380
ggagaugguu gagcagcuun n                                                21

<210>  381
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  381
cuguggaagg agaugguugn n                                                21


<210>  382
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  382
caugcgacug agacagcucn n                                                21


<210>  383
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  383
acauccugac uucugugagn n                                                21


<210>  384
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  384
gaugaugaug auggagacgn n                                                21

<210>  385
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  385
acaauuucug ugccauugcn n                                                21

<210>  386
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  386
gagcugucuc agucgcaugn n                                                21

<210>  387
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  387
cucacagaag ucaggaugun n                                                21

<210>  388
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  388
cgucuccauc aucaucaucn n                                                21


<210>  389
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  389
gcaauggcac agaaauugun n                                                21


<210>  390
<211>  21
<212>  RNA
<213>  Artificial Sequence

<220>
<223>  Description of Artificial Sequence:  siNA sense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  390
cugaguuuaa aaggcacccn n                                                21


<210>  391
<211>  21
<212>  RNA
<213>  Artificial Sequence

<220>
<223>  Description of Artificial Sequence:  siNA sense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  391
caaccacaaa auacaacaan n                                                21


<210>  392
<211>  21
<212>  RNA
<213>  Artificial Sequence

<220>
<223>  Description of Artificial Sequence:  siNA sense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  392
ccuggaaaga aucaaaaccn n                                                21


<210>  393
<211>  21
<212>  RNA
<213>  Artificial Sequence

<220>
<223>  Description of Artificial Sequence:  siNA sense region


<220>
<221>  misc_feature

<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  393
gcaaggaggg ccucugaugn n                                                21


<210>  394
<211>  21
<212>  RNA
<213>  Artificial Sequence

<220>
<223>  Description of Artificial Sequence:  siNA antisense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  394
gggugccuuu uaaacucagn n                                                21


<210>  395
<211>  21
<212>  RNA
<213>  Artificial Sequence

<220>
<223>  Description of Artificial Sequence:  siNA antisense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  395
uuguuguauu uugugguugn n                                                21


<210>  396
<211>  21
<212>  RNA
<213>  Artificial Sequence

<220>
<223>  Description of Artificial Sequence:  siNA antisense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  396
gguuuugauu cuuuccaggn n                                                21


<210>  397
<211>  21
<212>  RNA
<213>  Artificial Sequence

<220>
<223>  Description of Artificial Sequence:  siNA antisense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  397

caucagaggc ccuccuugcn n                                                21

<210>  398
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy-2'-fluoro

<220>
<221>  misc_feature
<222>  (1)..(4)
<223>  Phosphorothioate 3'-Internucleotide Linkage

<220>
<221>  misc_feature
<222>  (6)..(8)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(19)
<223>  2'-deoxy-2'-fluoro

<220>
<221>  misc_feature
<222>  (17)..(20)
<223>  Phosphorothioate 3'-Internucleotide Linkage

<220>
<221>  misc_feature
<222>  (20)..(21)
<223>  n stands for thymidine

<400>  398
cugaguuuaa aaggcacccn n                                                21


<210>  399
<211>  21
<212>  RNA
<213>  Artificial Sequence

<220>
<223>  Description of Artificial Sequence:  siNA sense region


<220>
<221>  misc_feature
<222>  (1)..(1)

<223>  2'-deoxy-2'-fluoro

<220>
<221>  misc_feature
<222>  (1)..(4)
<223>  Phosphorothioate 3'-Internucleotide Linkage

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (17)..(20)
<223>  Phosphorothioate 3'-Internucleotide Linkage

<220>
<221>  misc_feature
<222>  (20)..(21)
<223>  n stands for thymidine

<400>  399
caaccacaaa auacaacaan n                                                21


<210>  400
<211>  21
<212>  RNA
<213>  Artificial Sequence

<220>
<223>  Description of Artificial Sequence:  siNA sense region


<220>
<221>  misc_feature
<222>  (1)..(3)
<223>  2'-deoxy-2'-fluoro

<220>
<221>  misc_feature
<222>  (1)..(4)

<223>  Phosphorothioate 3'-Internucleotide Linkage

<220>
<221>  misc_feature
<222>  (12)..(13)
<223>  2'-deoxy-2'-fluoro

<220>
<221>  misc_feature
<222>  (17)..(20)
<223>  Phosphorothioate 3'-Internucleotide Linkage

<220>
<221>  misc_feature
<222>  (18)..(19)
<223>  2'-deoxy-2'-fluoro

<220>
<221>  misc_feature
<222>  (20)..(21)
<223>  n stands for thymidine

<400>  400
ccuggaaaga aucaaaaccn n                                                21


<210>  401
<211>  21
<212>  RNA
<213>  Artificial Sequence

<220>
<223>  Description of Artificial Sequence:  siNA sense region


<220>
<221>  misc_feature
<222>  (1)..(4)
<223>  Phosphorothioate 3'-Internucleotide Linkage

<220>
<221>  misc_feature
<222>  (2)..(2)
<223>  2'-deoxy-2'-fluoro

<220>
<221>  misc_feature
<222>  (11)..(15)
<223>  2'-deoxy-2'-fluoro

<220>
<221>  misc_feature
<222>  (17)..(20)
<223>  Phosphorothioate 3'-Internucleotide Linkage

<220>
<221>  misc_feature
<222>  (18)..(18)
<223>  2'-deoxy-2'-fluoro

<220>
<221>  misc_feature
<222>  (20)..(21)

<223>  n stands for thymidine

<400>  401
gcaaggaggg ccucugaugn n                                                21


<210>  402
<211>  21
<212>  RNA
<213>  Artificial Sequence

<220>
<223>  Description of Artificial Sequence:  siNA antisense region


<220>
<221>  misc_feature
<222>  (1)..(20)
<223>  Phosphorothioate 3'-Internucleotide Linkage

<220>
<221>  misc_feature
<222>  (20)..(21)
<223>  n stands for thymidine

<400>  402
gggugccuuu uaaacucagn n                                                21


<210>  403
<211>  21
<212>  RNA
<213>  Artificial Sequence

<220>
<223>  Description of Artificial Sequence:  siNA antisense region


<220>
<221>  misc_feature
<222>  (1)..(20)
<223>  Phosphorothioate 3'-Internucleotide Linkage

<220>
<221>  misc_feature
<222>  (20)..(21)
<223>  n stands for thymidine

<400>  403
uuguuguauu uugugguugn n                                                21


<210>  404
<211>  21
<212>  RNA
<213>  Artificial Sequence

<220>
<223>  Description of Artificial Sequence:  siNA antisense region


<220>
<221>  misc_feature

<222>  (1)..(20)
<223>  Phosphorothioate 3'-Internucleotide Linkage

<220>
<221>  misc_feature
<222>  (20)..(21)
<223>  n stands for thymidine

<400>  404
gguuuugauu cuuuccaggn n                                                21


<210>  405
<211>  21
<212>  RNA
<213>  Artificial Sequence

<220>
<223>  Description of Artificial Sequence:  siNA antisense region


<220>
<221>  misc_feature
<222>  (1)..(20)
<223>  Phosphorothioate 3'-Internucleotide Linkage

<220>
<221>  misc_feature
<222>  (20)..(21)
<223>  n stands for thymidine

<400>  405
caucagaggc ccuccuugcn n                                                21


<210>  406
<211>  21
<212>  RNA
<213>  Artificial Sequence

<220>
<223>  Description of Artificial Sequence:  siNA sense region

<400>  406
caaccacaaa auacaacaag a                                                21


<210>  407
<211>  21
<212>  RNA
<213>  Artificial Sequence

<220>
<223>  Description of Artificial Sequence:  siNA antisense region

<400>  407
uuguuguauu uugugguugu u                                                21


<210>  408
<211>  21
<212>  RNA
<213>  Artificial Sequence

<220>
<223>  Description of Artificial Sequence:  siNA sense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  408
aacaacauaa aacaccaacn n                                                21


<210>  409
<211>  21
<212>  RNA
<213>  Artificial Sequence

<220>
<223>  Description of Artificial Sequence:  siNA antisense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  409
guugguguuu uauguuguun n                                                21


<210>  410
<211>  21
<212>  RNA
<213>  Artificial Sequence

<220>
<223>  Description of Artificial Sequence:  siNA sense region


<220>
<221>  misc_feature

<222>  (1)..(4)
<223>  Phosphorothioate 3'-Internucleotide Linkage

<220>
<221>  misc_feature
<222>  (3)..(3)
<223>  2'-deoxy-2'-fluoro

<220>
<221>  misc_feature
<222>  (6)..(6)
<223>  2'-deoxy-2'-fluoro

<220>
<221>  misc_feature
<222>  (8)..(8)
<223>  2'-deoxy-2'-fluoro

<220>
<221>  misc_feature
<222>  (13)..(13)
<223>  2'-deoxy-2'-fluoro

<220>
<221>  misc_feature
<222>  (15)..(16)
<223>  2'-deoxy-2'-fluoro

<220>
<221>  misc_feature
<222>  (17)..(20)
<223>  Phosphorothioate 3'-Internucleotide Linkage

<220>
<221>  misc_feature
<222>  (19)..(19)
<223>  2'-deoxy-2'-fluoro

<220>
<221>  misc_feature
<222>  (20)..(21)
<223>  n stands for thymidine

<400>  410
aacaacauaa aacaccaacn n                                                21


<210>  411
<211>  21
<212>  RNA
<213>  Artificial Sequence

<220>
<223>  Description of Artificial Sequence:  siNA antisense region


<220>
<221>  misc_feature
<222>  (1)..(20)
<223>  Phosphorothioate 3'-Internucleotide Linkage

<220>
<221>  misc_feature

<222>  (20)..(21)
<223>  n stands for thymidine

<220>
<221>  misc_feature
<222>  (20)..(20)
<223>  Phosphorothioate 3'-Internucleotide Linkage

<400>  411
guugguguuu uauguuguun n                                                21


<210>  412
<211>  21
<212>  RNA
<213>  Artificial Sequence

<220>
<223>  Description of Artificial Sequence:  siNA sense region

<400>  412
agaacaacau aaaacaccaa c                                                21


<210>  413
<211>  21
<212>  RNA
<213>  Artificial Sequence

<220>
<223>  Description of Artificial Sequence:  siNA antisense region

<400>  413
uuguuggugu uuuauguugu u                                                21


<210>  414
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  414
caaccacaaa auacaacaan n                                                21


<210>  415
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  415
uuguuguauu uugugguugn n                                                21


<210>  416
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  416
agaacaacau aaaacaccan n                                                21


<210>  417
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  417
uuguuggugu uuuauguugn n                                                21


<210>  418
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<400>  418
uuguuguauu uugugguugn n                                                21


<210>  419
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for 5-Nitroindole universal base

<400>  419
uuguuguauu uugugguugn n                                                21


<210>  420
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for 3-Nitropyrole universal base

<400>  420
uuguuguauu uugugguugn n                                                21

<210>  421
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  421
caaccacaaa auacaacaan n                                                21


<210>  422
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<400>  422
caaccacaaa auacaacaan n                                                21


<210>  423
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>

<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(20)
<223>  n stands for thymidine

<220>
<221>  misc_feature
<222>  (21)..(21)
<223>  n stands for 3',3'-inverted thymidine

<400>  423
uuguuguauu uugugguugn n                                                21


<210>  424
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>

<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(20)
<223>  n stands for thymidine

<220>
<221>  misc_feature
<222>  (21)..(21)
<223>  n stands for glyceryl

<400>  424
uuguuguauu uugugguugn n                                                21


<210>  425
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>

<221>  misc_feature
<222>  (20)..(20)
<223>  n stands for thymidine

<220>
<221>  misc_feature
<222>  (21)..(21)
<223>  n stands for 3'-O-methyl uridine

<400>  425
uuguuguauu uugugguugn u                                                21


<210>  426
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(20)
<223>  n stands for thymidine

<220>
<221>  misc_feature
<222>  (21)..(21)
<223>  n stands for L-thymidine

<400>  426

uuguuguauu uugugguugn n                                                21

<210>  427
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(20)
<223>  n stands for thymidine

<220>
<221>  misc_feature
<222>  (21)..(2)
<223>  n stands for L-uridine

<400>  427
uuguuguauu uugugguugn u                                                21


<210>  428
<211>  21
<212>  RNA
<213>  Artificial Sequence

<220>
<223>  Description of Artificial Sequence:  siNA antisense region

<220>
<221> misc_feature
<222> (1)..(2)
<223> 2'-deoxy-2'-fluoro

<220>
<221> misc_feature
<222> (4)..(5)
<223> 2'-deoxy-2'-fluoro

<220>
<221> misc_feature
<222> (7)..(7)
<223> 2'-deoxy-2'-fluoro

<220>
<221> misc_feature
<222> (9)..(12)
<223> 2'-deoxy-2'-fluoro

<220>
<221> misc_feature
<222> (14)..(14)
<223> 2'-deoxy-2'-fluoro

<220>
<221> misc_feature
<222> (17)..(18)
<223> 2'-deoxy-2'-fluoro

<220>
<221> misc_feature
<222> (20)..(20)
<223> n stands for thymidine

<220>
<221> misc_feature
<222> (21)..(21)
<223> n stands for 3',3'-inverted thymidine

<400> 428
uuguuguauu uugugguugn d 21


<210> 429
<211> 21
<212> RNA
<213> Artificial Sequence

<220>
<223> Description of Artificial Sequence: siNA antisense region


<220>
<221> misc_feature
<222> (1)..(2)
<223> 2'-deoxy-2'-fluoro

<220>
<221> misc_feature
<222> (4)..(5)

<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(20)
<223>  n stands for 3',3'-inverted thymidine

<220>
<221>  misc_feature
<222>  (21)..(21)
<223>  n stands for thymidine

<400>  429
uuguuguauu uugugguugn n                                                21


<210>  430
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)

<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  430
uuguuguauu uugugguugn n                                                21


<210>  431
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (11)..(11)
<223>  2'-deoxy-2'-fluoro

<220>
<221>  misc_feature
<222>  (15)..(16)
<223>  2'-deoxy-2'-fluoro

<220>
<221>  misc_feature
<222>  (18)..(19)

<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  431
guguaaggag uggaccaucn n                                                21


<210>  432
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(10)
<223>  2'-deoxy-2'-fluoro

<220>
<221>  misc_feature
<222>  (12)..(13)
<223>  2'-deoxy-2'-fluoro

<220>
<221>  misc_feature
<222>  (15)..(15)
<223>  2'-deoxy-2'-fluoro

<220>
<221>  misc_feature
<222>  (20)..(21)
<223>  n stands for thymidine

<220>
<221>  misc_feature
<222>  (21)..(21)

<223>  3'-3 attached terminal deoxyabasic moeity

<400>  432
acggaguauu gcugugggan n                                                21


<210>  433
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (6)..(8)
<223>  2'-deoxy-2'-fluoro

<220>
<221>  misc_feature
<222>  (13)..(13)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  433
gcaggccuaa gacaugugan n                                                21


<210>  434
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (8)..(8)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  434
caaaaagcaa gggagaaaan n                                                21


<210>  435
<211>  21
<212>  RNA
<213>  Artificial Sequence

<220>
<223>  Description of Artificial Sequence:  siNA antisense region


<220>
<221>  misc_feature
<222>  (3)..(3)
<223>  2'-deoxy-2'-fluoro

<220>
<221>  misc_feature
<222>  (6)..(8)
<223>  2'-deoxy-2'-fluoro

<220>
<221>  misc_feature
<222>  (10)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  435
gaugguccac uccuuacacn n                                                21


<210>  436
<211>  21
<212>  RNA
<213>  Artificial Sequence

<220>
<223>  Description of Artificial Sequence:  siNA antisense region


<220>
<221>  misc_feature
<222>  (1)..(4)
<223>  2'-deoxy-2'-fluoro

<220>
<221>  misc_feature
<222>  (6)..(6)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  436
ucccacagca auacuccgun n                                                21


<210>  437
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (6)..(6)
<223>  2'-deoxy-2'-fluoro

<220>
<221>  misc_feature
<222>  (8)..(11)
<223>  2'-deoxy-2'-fluoro

<220>
<221>  misc_feature
<222>  (15)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  437
ucacaugucu uaggccugcn n                                                21


<210>  438

<211>  21
<212>  RNA
<213>  Artificial Sequence

<220>
<223>  Description of Artificial Sequence:  siNA antisense region


<220>
<221>  misc_feature
<222>  (1)..(11)
<223>  2'-deoxy-2'-fluoro

<220>
<221>  misc_feature
<222>  (13)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  438
uuuucucccu ugcuuuugn n                                                21


<210>  439
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature

<222>  (12)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  439
caaccacaaa auacaacaan n                                                21


<210>  440
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature

<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<220>
<221>  misc_feature
<222>  (20)..(21)

<400>  440
uuguuguauu uugugguugn n                                                21


<210>  441
<211>  21
<212>  RNA
<213>  Artificial Sequence

<220>
<223>  Description of Artificial Sequence:  inverted control/siNA sense


<220>
<221>  misc_feature
<222>  (20)..(21)
<223>  n stands for thymidine

<400>  441
aacaacauaa aacaccaacn n                                                21


<210>  442
<211>  21
<212>  RNA
<213>  Artificial Sequence

<220>
<223>  Description of Artificial Sequence:  inverted control/siNA antisense


<220>
<221>  misc_feature
<222>  (20)..(21)
<223>  n stands for thymidine

<400>  442
guugguguuu uauguuguun n                                                21


<210>  443
<211>  21
<212>  RNA
<213>  Artificial Sequence

<220>

<223>  Description of Artificial Sequence:  inverted control/siNA sense

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (6)..(6)
<223>  2'-deoxy-2'-fluoro

<220>
<221>  misc_feature
<222>  (8)..(8)
<223>  2'-deoxy-2'-fluoro

<220>
<221>  misc_feature
<222>  (13)..(13)
<223>  2'-deoxy-2'-fluoro

<220>
<221>  misc_feature
<222>  (15)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  443
aacaacauaa aacaccaacn n                                                21

<210>  444
<211>  21
<212>  RNA
<213>  Artificial Sequence

<220>
<223>  Description of Artificial Sequence:  inverted control/siNA antisense

<220>
<221>  misc_feature

<222>  (2)..(3)
<223>  2'-deoxy-2'-fluoro

<220>
<221>  misc_feature
<222>  (6)..(6)
<223>  2'-deoxy-2'-fluoro

<220>
<221>  misc_feature
<222>  (8)..(11)
<223>  2'-deoxy-2'-fluoro

<220>
<221>  misc_feature
<222>  (13)..(13)
<223>  2'-deoxy-2'-fluoro

<220>
<221>  misc_feature
<222>  (15)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  444
guugguguuu uauguuguun n                                                21


<210>  445
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy

<220>
<221>  misc_feature
<222>  (3)..(3)
<223>  2'-deoxy-2'-fluoro

<220>
<221>  misc_feature

<222>  (4)..(5)
<223>  2'-deoxy

<220>
<221>  misc_feature
<222>  (6)..(6)
<223>  2'-deoxy-2'-fluoro

<220>
<221>  misc_feature
<222>  (7)..(7)
<223>  2'-deoxy

<220>
<221>  misc_feature
<222>  (8)..(8)
<223>  2'-deoxy-2'-fluoro

<220>
<221>  misc_feature
<222>  (9)..(12)
<223>  2'-deoxy

<220>
<221>  misc_feature
<222>  (13)..(13)
<223>  2'-deoxy-2'-fluoro

<220>
<221>  misc_feature
<222>  (14)..(14)
<223>  2'-deoxy

<220>
<221>  misc_feature
<222>  (15)..(16)
<223>  2'-deoxy-2'-fluoro

<220>
<221>  misc_feature
<222>  (17)..(18)
<223>  2'-deoxy

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  445
aacaacauaa aacaccaacn n                                                21


<210>  446

<211>  21
<212>  RNA
<213>  Artificial Sequence

<220>
<223>  Description of Artificial Sequence:  inverted control/siNA antisense


<220>
<221>  misc_feature
<222>  (2)..(3)
<223>  2'-deoxy-2'-fluoro

<220>
<221>  misc_feature
<222>  (6)..(6)
<223>  2'-deoxy-2'-fluoro

<220>
<221>  misc_feature
<222>  (8)..(11)
<223>  2'-deoxy-2'-fluoro

<220>
<221>  misc_feature
<222>  (13)..(13)
<223>  2'-deoxy-2'-fluoro

<220>
<221>  misc_feature
<222>  (15)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  446
guugguguuu uauguuguun n                                                21


<210>  447
<211>  21
<212>  RNA
<213>  Artificial Sequence

<220>
<223>  Description of Artificial Sequence:  siNA sense region


<220>
<221>  misc_feature

<222>  (20)..(21)
<223>  n stands for thymidine

<400>  447
cugaguuuaa aaggcacccn n                                                21


<210>  448
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  448
gcaaggaggg ccucugaugn n                                                21


<210>  449
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  449
ccuggaaaga aucaaaaccn n                                                21


<210>  450
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  450
gggugccuuu uaaacucagn n                                                21


<210>  451
<211>  21

<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  451
caucagaggc ccuccuugcn n                                                21


<210>  452
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  452
gguuuugauu cuuuccaggn n                                                21


<210>  453
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(8)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  453
cugaguuuaa aaggcacccn n                                                21


<210>  454
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (11)..(15)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  454
gcaaggaggg ccucugaugn n                                                21


<210>  455

<211>  21
<212>  RNA
<213>  Artificial Sequence

<220>
<223>  Description of Artificial Sequence:  siNA sense region


<220>
<221>  misc_feature
<222>  (1)..(3)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (12)..(13)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  455
ccuggaaaga aucaaaaccn n                                                21


<210>  456
<211>  21
<212>  RNA
<213>  Artificial Sequence

<220>
<223>  Description of Artificial Sequence:  siNA antisense region


<220>
<221>  misc_feature
<222>  (4)..(4)
<223>  2'-deoxy-2'-fluoro

<220>
<221>  misc_feature
<222>  (6)..(11)
<223>  2'-deoxy-2'-fluoro

<220>
<221>  misc_feature

<222>  (15)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  456
gggugccuuu uaaacucagn n                                                21


<210>  457
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(4)
<223>  2'-deoxy-2'-fluoro

<220>
<221>  misc_feature
<222>  (10)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  457
caucagaggc ccuccuugcn n                                                21


<210>  458
<211>  21
<212>  RNA

<213>  Artificial Sequence

<220>
<223>  Description of Artificial Sequence:  siNA antisense region


<220>
<221>  misc_feature
<222>  (3)..(6)
<223>  2'-deoxy-2'-fluoro

<220>
<221>  misc_feature
<222>  (9)..(16)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  458
gguuuugauu cuuuccaggn n                                                21


<210>  459
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(5)
<223>  2'-deoxy

<220>
<221>  misc_feature
<222>  (6)..(8)
<223>  2'-deoxy-2'-fluoro

<220>
<221>  misc_feature
<222>  (9)..(14)
<223>  2'-deoxy

<220>
<221> misc_feature
<222> (15)..(15)
<223> 2'-deoxy-2'-fluoro

<220>
<221> misc_feature
<222> (16)..(16)
<223> 2'-deoxy

<220>
<221> misc_feature
<222> (17)..(19)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 459
cugaguuuaa aaggcacccn n                                                21


<210> 460
<211> 21
<212> RNA
<213> Artificial Sequence

<220>
<223> Description of Artificial Sequence: siNA sense region


<220>
<221> misc_feature
<222> (1)..(1)
<223> 2'-deoxy

<220>
<221> misc_feature
<222> (1)..(1)
<223> 5'-3 attached terminal deoxyabasic moeity

<220>
<221> misc_feature
<222> (2)..(2)
<223> 2'-deoxy-2'-fluoro

<220>
<221> misc_feature
<222> (3)..(10)
<223> 2'-deoxy

<220>
<221> misc_feature
<222> (11)..(15)
<223> 2'-deoxy-2'-fluoro

<220>
<221>  misc_feature
<222>  (16)..(17)
<223>  2'-deoxy

<220>
<221>  misc_feature
<222>  (18)..(18)
<223>  2'-deoxy-2'-fluoro

<220>
<221>  misc_feature
<222>  (19)..(19)
<223>  2'-deoxy

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  460
gcaaggaggg ccucugaugn n                                                21


<210>  461
<211>  21
<212>  RNA
<213>  Artificial Sequence

<220>
<223>  Description of Artificial Sequence:  siNA sense region


<220>
<221>  misc_feature
<222>  (1)..(3)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(11)
<223>  2'-deoxy

<220>
<221>  misc_feature
<222>  (12)..(13)
<223>  2'-deoxy-2'-fluoro

<220>
<221>  misc_feature
<222>  (14)..(17)
<223>  2'-deoxy

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  461
ccuggaaaga aucaaaaccn n                                                21


<210>  462
<211>  21
<212>  RNA
<213>  Artificial Sequence

<220>
<223>  Description of Artificial Sequence:  siNA antisense region


<220>
<221>  misc_feature
<222>  (4)..(4)
<223>  2'-deoxy-2'-fluoro

<220>
<221>  misc_feature
<222>  (6)..(11)
<223>  2'-deoxy-2'-fluoro

<220>
<221>  misc_feature
<222>  (15)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  462
gggugccuuu uaaacucagn n                                                21


<210>  463
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(4)
<223>  2'-deoxy-2'-fluoro

<220>
<221>  misc_feature
<222>  (10)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  463
caucagaggc ccuccuugcn n                                                21


<210>  464
<211>  21
<212>  RNA
<213>  Artificial Sequence

<220>
<223>  Description of Artificial Sequence:  siNA antisense region


<220>
<221>  misc_feature
<222>  (3)..(6)
<223>  2'-deoxy-2'-fluoro

<220>
<221>  misc_feature
<222>  (9)..(16)
<223>  2'-deoxy-2'-fluoro

<220>
<221>  misc_feature
<222>  (20)..(21)
<223>  n stands for thymidine

<220>

<221>  misc_feature
<222>  (20)..(20)
<223>  Phosphorothioate 3'-Internucleotide Linkage

<400>  464
gguuuugauu cuuuccaggn n                                                21


<210>  465
<211>  21
<212>  RNA
<213>  Artificial Sequence

<220>
<223>  Description of Artificial Sequence:  inverted control/siNA sense


<220>
<221>  misc_feature
<222>  (20)..(21)
<223>  n stands for thymidine

<400>  465
cccacggaaa auuugagucn n                                                21


<210>  466
<211>  21
<212>  RNA
<213>  Artificial Sequence

<220>
<223>  Description of Artificial Sequence:  inverted control/siNA sense


<220>
<221>  misc_feature
<222>  (20)..(21)
<223>  n stands for thymidine

<400>  466
guagucuccg ggaggaacgn n                                                21


<210>  467
<211>  21
<212>  RNA
<213>  Artificial Sequence

<220>
<223>  Description of Artificial Sequence:  inverted control/siNA sense


<220>
<221>  misc_feature
<222>  (20)..(21)
<223>  n stands for thymidine

<400>  467
ccaaaacuaa gaaagguccn n                                                21


<210>  468

<211>  21
<212>  RNA
<213>  Artificial Sequence

<220>
<223>  Description of Artificial Sequence:  inverted control/siNA antisense


<220>
<221>  misc_feature
<222>  (20)..(21)
<223>  n stands for thymidine

<400>  468
gacucaaauu uuccgugggn n                                                21


<210>  469
<211>  21
<212>  RNA
<213>  Artificial Sequence

<220>
<223>  Description of Artificial Sequence:  inverted control/siNA antisense


<220>
<221>  misc_feature
<222>  (20)..(21)
<223>  n stands for thymidine

<400>  469
cguuccuccc ggagacuacn n                                                21


<210>  470
<211>  21
<212>  RNA
<213>  Artificial Sequence

<220>
<223>  Description of Artificial Sequence:  inverted control/siNA antisense


<220>
<221>  misc_feature
<222>  (20)..(21)
<223>  n stands for thymidine

<400>  470
ggaccuuucu uaguuuuggn n                                                21


<210>  471
<211>  21
<212>  RNA
<213>  Artificial Sequence

<220>
<223>  Description of Artificial Sequence:  inverted control/siNA sense


<220>

<221>  misc_feature
<222>  (1)..(3)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (12)..(14)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  471
cccacggaaa auuugagucn n                                                21


<210>  472
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (5)..(9)
<223>  2'-deoxy-2'-fluoro

<220>

<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  472
guagucuccg ggaggaacgn n                                                21


<210>  473
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (7)..(8)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  473
ccaaaacuaa gaaagguccn n                                                21


<210>  474
<211>  21

<212>  RNA
<213>  Artificial Sequence

<220>
<223>  Description of Artificial Sequence:  inverted control/siNA antisense


<220>
<221>  misc_feature
<222>  (3)..(5)
<223>  2'-deoxy-2'-fluoro

<220>
<221>  misc_feature
<222>  (9)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  474
gacucaaauu uuccgugggn n                                                21


<210>  475
<211>  21
<212>  RNA
<213>  Artificial Sequence

<220>
<223>  Description of Artificial Sequence:  inverted control/siNA antisense


<220>
<221>  misc_feature
<222>  (1)..(1)
<223>  2'-deoxy-2'-fluoro

<220>
<221>  misc_feature
<222>  (3)..(10)
<223>  2'-deoxy-2'-fluoro

<220>
<221>  misc_feature
<222>  (16)..(17)
<223>  2'-deoxy-2'-fluoro

<220>
<221>  misc_feature
<222>  (19)..(19)

<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  475
cguuccuccc ggagacuacn n                                                21


<210>  476
<211>  21
<212>  RNA
<213>  Artificial Sequence

<220>
<223>  Description of Artificial Sequence:  inverted control/siNA antisense


<220>
<221>  misc_feature
<222>  (4)..(11)
<223>  2'-deoxy-2'-fluoro

<220>
<221>  misc_feature
<222>  (14)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  476
ggaccuuucu uaguuuuggn n                                                21


<210>  477
<211>  21
<212>  RNA
<213>  Artificial Sequence

<220>
<223>  Description of Artificial Sequence:  inverted control/siNA sense


<220>
<221>  misc_feature
<222>  (1)..(3)
<223>  2'-deoxy-2'-fluoro

<220>
<221> misc_feature
<222> (1)..(1)
<223> 5'-3 attached terminal deoxyabasic moeity

<220>
<221> misc_feature
<222> (4)..(4)
<223> 2'-deoxy

<220>
<221> misc_feature
<222> (5)..(5)
<223> 2'-deoxy-2'-fluoro

<220>
<221> misc_feature
<222> (6)..(11)
<223> 2'-deoxy

<220>
<221> misc_feature
<222> (12)..(14)
<223> 2'-deoxy-2'-fluoro

<220>
<221> misc_feature
<222> (15)..(17)
<223> 2'-deoxy

<220>
<221> misc_feature
<222> (18)..(19)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 477
cccacggaaa auuugagucn n 21


<210> 478
<211> 21
<212> RNA
<213> Artificial Sequence

<220>
<223> Description of Artificial Sequence: inverted control/siNA sense


<220>
<221> misc_feature
<222> (1)..(1)
<223> 2'-deoxy

<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (3)..(4)
<223>  2'-deoxy

<220>
<221>  misc_feature
<222>  (5)..(9)
<223>  2'-deoxy-2'-fluoro

<220>
<221>  misc_feature
<222>  (10)..(17)
<223>  2'-deoxy

<220>
<221>  misc_feature
<222>  (18)..(18)
<223>  2'-deoxy-2'-fluoro

<220>
<221>  misc_feature
<222>  (19)..(19)
<223>  2'-deoxy

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  478
guagucuccg ggaggaacgn n                                                21


<210>  479
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(6)
<223>  2'-deoxy

<220>
<221>  misc_feature
<222>  (7)..(8)
<223>  2'-deoxy-2'-fluoro

<220>
<221>  misc_feature
<222>  (9)..(16)
<223>  2'-deoxy

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  479
ccaaaacuaa gaaagguccn n                                                21


<210>  480
<211>  21
<212>  RNA
<213>  Artificial Sequence

<220>
<223>  Description of Artificial Sequence:  inverted control/siNA antisense


<220>
<221>  misc_feature
<222>  (3)..(5)
<223>  2'-deoxy-2'-fluoro

<220>
<221>  misc_feature
<222>  (9)..(14)
<223>  2'-deoxy-2'-fluoro

<220>
<221>  misc_feature
<222>  (16)..(16)
<223>  2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400> 480
gacucaaauu uuccgugggn n 21


<210> 481
<211> 21
<212> RNA
<213> Artificial Sequence

<220>
<223> Description of Artificial Sequence: inverted control/siNA antisense


<220>
<221> misc_feature
<222> (1)..(1)
<223> 2'-deoxy-2'-fluoro

<220>
<221> misc_feature
<222> (3)..(10)
<223> 2'-deoxy-2'-fluoro

<220>
<221> misc_feature
<222> (16)..(17)
<223> 2'-deoxy-2'-fluoro

<220>
<221> misc_feature
<222> (19)..(19)
<223> 2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400> 481
cguuccuccc ggagacuacn n 21


<210> 482
<211> 21
<212> RNA
<213> Artificial Sequence

<220>

<223>  Description of Artificial Sequence:  inverted control/siNA antisense

<220>
<221>  misc_feature
<222>  (4)..(11)
<223>  2'-deoxy-2'-fluoro

<220>
<221>  misc_feature
<222>  (14)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  482
ggaccuuucu uaguuuuggn n                                                21

<210>  483
<211>  21
<212>  RNA
<213>  Artificial Sequence

<220>
<223>  Description of Artificial Sequence:  siNA sense region

<220>
<221>  misc_feature
<222>  (1)..(1)
<223>  5'-3 attached terminal deoxyabasic moeity

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  483
cugaguuuaa aaggcacccn n                                                21

<210>  484
<211>  21
<212>  RNA
<213>  Artificial Sequence

<220>
<223>  Description of Artificial Sequence:  siNA sense region

<220>
<221>  misc_feature
<222>  (1)..(1)
<223>  5'-3 attached terminal deoxyabasic moeity

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  484
gcaaggaggg ccucugaugn n                                                21


<210>  485
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  485
ccuggaaaga aucaaaaccn n                                                21


<210>  486
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<220>

<221>  misc_feature
<222>  (20)..(20)
<223>  Phosphorothioate 3'-Internucleotide Linkage

<400>  486
gggugccuuu uaaacucagn n                                                21


<210>  487
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  487
caucagaggc ccuccuugcn n                                                21


<210>  488
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  488
gguuuugauu cuuuccaggn n                                                21


<210>  489
<211>  21
<212>  RNA
<213>  Artificial Sequence

<220>
<223>  Description of Artificial Sequence:  inverted control/siNA sense

<220>
<221>  misc_feature
<222>  (1)..(1)
<223>  5'-3 attached terminal deoxyabasic moeity

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  489
cccacggaaa auuugagucn n                                                21


<210>  490
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  5'-3 attached terminal deoxyabasic moeity

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  490
guagucuccg ggaggaacgn n                                                21


<210>  491
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  5'-3 attached terminal deoxyabasic moeity

<220>
<221>  misc_feature

<222>  (20)..(21)
<223>  n stands for thymidine

<220>
<221>  misc_feature
<222>  (21)..(21)
<223>  3'-3 attached terminal deoxyabasic moeity

<400>  491
ccaaaacuaa gaaagguccn n                                                21


<210>  492
<211>  21
<212>  RNA
<213>  Artificial Sequence

<220>
<223>  Description of Artificial Sequence:  inverted control/siNA antisense


<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  492
gacucaaauu uuccgugggn n                                                21


<210>  493
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  493
cguuccuccc ggagacuacn n                                                21


<210>  494
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  494
ggaccuuucu uaguuuuggn n                                                21


<210>  495
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (3)..(3)
<223>  2'-deoxy

<220>
<221>  misc_feature
<222>  (4)..(5)
<223>  2'-deoxy-2'-fluoro

<220>
<221>  misc_feature
<222>  (6)..(6)
<223>  2'-deoxy

<220>
<221>  misc_feature
<222>  (7)..(7)
<223>  2'-deoxy-2'-fluoro

<220>
<221>  misc_feature
<222>  (8)..(8)
<223>  2'-deoxy

<220>
<221>  misc_feature
<222>  (9)..(12)
<223>  2'-deoxy-2'-fluoro

<220>

<221>  misc_feature
<222>  (13)..(13)
<223>  2'-deoxy

<220>
<221>  misc_feature
<222>  (14)..(14)
<223>  2'-deoxy-2'-fluoro

<220>
<221>  misc_feature
<222>  (15)..(16)
<223>  2'-deoxy

<220>
<221>  misc_feature
<222>  (17)..(18)
<223>  2'-deoxy-2'-fluoro

<220>
<221>  misc_feature
<222>  (19)..(19)
<223>  2'-deoxy

<220>
<221>  misc_feature
<222>  (20)..(21)
<223>  n stands for 5-bromo-deoxy-uridine

<220>
<221>  misc_feature
<222>  (20)..(20)
<223>  Phosphorothioate 3'-Internucleotide Linkage

<400>  495
uuguuguauu uugugguugn n                                                21


<210>  496
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (2)..(2)
<223>  2'-deoxy

<220>
<221>  misc_feature
<222>  (3)..(4)
<223>  2'-deoxy-2'-fluoro

<220>

<221>  misc_feature
<222>  (5)..(9)
<223>  2'-deoxy

<220>
<221>  misc_feature
<222>  (10)..(17)
<223>  2'-deoxy-2'-fluoro

<220>
<221>  misc_feature
<222>  (18)..(18)
<223>  2'-deoxy

<220>
<221>  misc_feature
<222>  (19)..(19)
<223>  2'-deoxy-2'-fluoro

<220>
<221>  misc_feature
<222>  (20)..(21)
<223>  n stands for 5-bromo-deoxy-uridine

<220>
<221>  misc_feature
<222>  (20)..(20)
<223>  Phosphorothioate 3'-Internucleotide Linkage

<400>  496
caucagaggc ccuccuugcn n                                                21


<210>  497
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (3)..(3)
<223>  2'-deoxy

<220>
<221>  misc_feature
<222>  (4)..(5)
<223>  2'-deoxy-2'-fluoro

<220>
<221>  misc_feature
<222>  (6)..(6)
<223>  2'-deoxy

<220>

<221>  misc_feature
<222>  (7)..(7)
<223>  2'-deoxy-2'-fluoro

<220>
<221>  misc_feature
<222>  (8)..(8)
<223>  2'-deoxy

<220>
<221>  misc_feature
<222>  (9)..(12)
<223>  2'-deoxy-2'-fluoro

<220>
<221>  misc_feature
<222>  (13)..(13)
<223>  2'-deoxy

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (19)..(19)
<223>  2'-deoxy

<220>
<221>  misc_feature
<222>  (20)..(20)
<223>  n stands for 5-bromo-deoxy-uridine

<220>
<221>  misc_feature
<222>  (20)..(20)
<223>  Phosphorothioate 3'-Internucleotide Linkage

<220>
<221>  misc_feature
<222>  (21)..(21)
<223>  n stands for thymidine

<400>  497
uuguuguauu uugugguugn n                                                21


<210>  498
<211>  21
<212>  RNA
<213>  Artificial Sequence

<220>
<223>  Description of Artificial Sequence:  siNA antisense region


<220>

<221>  misc_feature
<222>  (1)..(1)
<223>  2'-deoxy-2'-fluoro

<220>
<221>  misc_feature
<222>  (2)..(2)
<223>  2'-deoxy

<220>
<221>  misc_feature
<222>  (3)..(4)
<223>  2'-deoxy-2'-fluoro

<220>
<221>  misc_feature
<222>  (5)..(9)
<223>  2'-deoxy

<220>
<221>  misc_feature
<222>  (10)..(17)
<223>  2'-deoxy-2'-fluoro

<220>
<221>  misc_feature
<222>  (18)..(18)
<223>  2'-deoxy

<220>
<221>  misc_feature
<222>  (19)..(19)
<223>  2'-deoxy-2'-fluoro

<220>
<221>  misc_feature
<222>  (20)..(20)
<223>  n stands for 5-bromo-deoxy-uridine

<220>
<221>  misc_feature
<222>  (20)..(20)
<223>  Phosphorothioate 3'-Internucleotide Linkage

<220>
<221>  misc_feature
<222>  (21)..(21)
<223>  n stands for thymidine

<400>  498
caucagaggc ccuccuugcn n                                                21


<210>  499
<211>  21
<212>  RNA
<213>  Artificial Sequence

<220>
<223>  Description of Artificial Sequence:  siNA sense region


<220>

<221>  misc_feature
<222>  (1)..(1)
<223>  5'-3 attached terminal deoxyabasic moeity

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  499
caaccacaaa auacaacaan n                                                21


<210>  500
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  500
aacaacauaa aacaccaacn n                                                21


<210>  501
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<220>
<221>  misc_feature
<222>  (20)..(20)

<223>  Phosphorothioate 3'-Internucleotide Linkage

<400>  501
uuguuguauu uugugguugn n                                                21


<210>  502
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  502
guugguguuu uauguuguun n                                                21


<210>  503
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (12)..(13)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  503
caacugagaa gccaagacun n                                                21


<210>  504
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (7)..(9)
<223>  2'-deoxy-2'-fluoro

<220>
<221>  misc_feature
<222>  (11)..(13)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  504
cauggaccua ucuggguccn n                                                21

<210>  505
<211>  21
<212>  RNA
<213>  Artificial Sequence

<220>
<223>  Description of Artificial Sequence:  siNA sense region

<220>
<221>  misc_feature
<222>  (1)..(1)
<223>  5'-3 attached terminal deoxyabasic moeity

<220>
<221>  misc_feature
<222>  (8)..(11)
<223>  2'-deoxy-2'-fluoro

<220>
<221>  misc_feature
<222>  (13)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  505
gggaggaccu uaucugugcn n                                                21

<210>  506
<211>  21
<212>  RNA
<213>  Artificial Sequence

<220>
<223>  Description of Artificial Sequence:  siNA sense region

<220>
<221>  misc_feature

<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (4)..(6)
<223>  2'-deoxy-2'-fluoro

<220>
<221>  misc_feature
<222>  (8)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (16)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  506
gcauccaugu guggacucan n                                                21


<210>  507
<211>  21
<212>  RNA
<213>  Artificial Sequence

<220>
<223>  Description of Artificial Sequence:  siNA antisense region


<220>
<221>  misc_feature
<222>  (3)..(6)
<223>  2'-deoxy-2'-fluoro

<220>
<221>  misc_feature

<222>  (9)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  507
agucuuggcu ucucaguugn n                                                21


<210>  508
<211>  21
<212>  RNA
<213>  Artificial Sequence

<220>
<223>  Description of Artificial Sequence:  siNA antisense region


<220>
<221>  misc_feature
<222>  (4)..(6)
<223>  2'-deoxy-2'-fluoro

<220>
<221>  misc_feature
<222>  (10)..(10)
<223>  2'-deoxy-2'-fluoro

<220>
<221>  misc_feature
<222>  (14)..(16)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  508
ggacccagau agguccaugn n                                                21

<210>  509
<211>  21
<212>  RNA
<213>  Artificial Sequence

<220>
<223>  Description of Artificial Sequence:  siNA antisense region


<220>
<221>  misc_feature
<222>  (2)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (8)..(8)
<223>  2'-deoxy-2'-fluoro

<220>
<221>  misc_feature
<222>  (13)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  509
gcacagauaa gguccucccn n                                                21


<210>  510
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (5)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(13)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  510
ugaguccaca cauggaugcn n                                                21


<210>  511
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  511
caacugagaa gccaagacun n                                                21


<210>  512
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  512
cauggaccua ucuggguccn n                                                21


<210>  513
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  513
gggaggaccu uaucugugcn n                                                21


<210>  514
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  514
gcauccaugu guggacucan n                                                21


<210>  515
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  515
agucuuggcu ucucaguugn n                                                21

<210>  516
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  516
ggacccagau agguccaugn n                                                21

<210>  517
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  517
gcacagauaa gguccucccn n                                                21

<210>  518
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  518
ugaguccaca cauggaugcn n                                                21

<210>  519
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  519
aagagggaaa gcugacaucn n                                                21


<210>  520
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  520
ggaagaagga aggagaggcn n                                                21


<210>  521
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  521
gaggacugag ccuacggaan n                                                21


<210>  522
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  522
ugcuguggug acacauggun n                                                21

<210>  523
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  523
gaugucagcu uucccucuun n                                                21

<210>  524
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  524
gccucuccuu ccuucuuccn n                                                21

<210>  525
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  525
uuccguaggc ucaguccucn n                                                21

<210>  526
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  526
accauguguc accacagcan n                                                21


<210>  527
<211>  21
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  527
uccauggugc ucacugcggc u                                                21


<210>  528
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  528
agccgcagug agcaccaugg a                                                21


<210>  529
<211>  21
<212>  RNA
<213>  Artificial Sequence

<220>
<223>  Description of Artificial Sequence:  inverted control/siNA sense

<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  529
agguaccacg agugacgccg a                                                21

<210>  530
<211>  21
<212>  RNA
<213>  Artificial Sequence

<220>
<223>  Description of Artificial Sequence:  inverted control/siNA sense

<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  530
ucggcgucac ucgugguacc u                                                21

<210>  531
<211>  23
<212>  RNA
<213>  Artificial Sequence

<220>
<223>  Description of Artificial Sequence:  siNA antisense region

<400>  531
uccauggugc ucacugcggc uuu                                              23

<210>  532
<211>  23
<212>  RNA
<213>  Artificial Sequence

<220>
<223>  Description of Artificial Sequence:  siNA sense region

<400>  532

agccgcagug agcaccaugg auu                                              23


<210>  533
<211>  23
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  533
uccauggugc ucacugcggc uuu                                              23


<210>  534
<211>  23
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  534
agccgcagug agcaccaugg auu                                              23


<210>  535
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  535
uggggucguc aaagacguun n                                                21

<210>  536
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  536
aacgucuuug acgaccccan n                                                21

<210>  537
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  537
uugcagaaac ugcuggggun n                                                21

<210>  538
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  538
accccagcag uuucugcaan n                                                21

<210>  539
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  539
ggugcuugga ucuggcgcun n                                                21


<210>  540
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  540
agcgccagau ccaagcaccn n                                                21


<210>  541
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  541
ucgcggucua gguucguggn n                                                21


<210>  542
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  542
ccacgaaccu agaccgcgan n                                                21

<210>  543
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  543
gaucuuuggg agccuggcan n                                                21

<210>  544
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  544
ugccaggcuc ccaaagaucn n                                                21

<210>  545
<211>  21
<212>  RNA
<213>  Artificial Sequence

<220>
<223>  Description of Artificial Sequence:  inverted control/siNA sense

<220>
<221>  misc_feature
<222>  (20)..(21)
<223>  n stands for thymidine

<400>  545
acgguccgag gguuucuagn n                                                21

<210>  546
<211>  21
<212>  RNA
<213>  Artificial Sequence

<220>
<223>  Description of Artificial Sequence:  inverted control/siNA antisense


<220>
<221>  misc_feature
<222>  (20)..(21)
<223>  n stands for thymidine

<400>  546
cuagaaaccc ucggaccgun n                                                21


<210>  547
<211>  21
<212>  RNA
<213>  Artificial Sequence

<220>
<223>  Description of Artificial Sequence:  siNA sense region


<220>
<221>  misc_feature
<222>  (1)..(4)
<223>  Phosphorothioate 3'-Internucleotide Linkage

<220>
<221>  misc_feature
<222>  (3)..(3)
<223>  2'-deoxy-2'-fluoro

<220>
<221>  misc_feature
<222>  (5)..(7)
<223>  2'-deoxy-2'-fluoro

<220>
<221>  misc_feature
<222>  (11)..(13)
<223>  2'-deoxy-2'-fluoro

<220>
<221>  misc_feature
<222>  (16)..(16)
<223>  2'-deoxy-2'-fluoro

<220>
<221>  misc_feature
<222>  (17)..(20)
<223>  Phosphorothioate 3'-Internucleotide Linkage

<220>
<221>  misc_feature
<222>  (18)..(19)
<223>  2'-deoxy-2'-fluoro

<220>
<221>  misc_feature
<222>  (20)..(21)
<223>  n stands for thymidine

<400>  547

ggugcuugga ucuggcgcun n                                              21


<210>  548
<211>  21
<212>  RNA
<213>  Artificial Sequence

<220>
<223>  Description of Artificial Sequence:  siNA antisense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  548
agcgccagau ccaagcaccn n                                              21


<210>  549
<211>  21
<212>  RNA
<213>  Artificial Sequence

<220>
<223>  Description of Artificial Sequence:  siNA sense region


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  549
ggugcuugga ucuggcgcun n                                              21


<210>  550
<211>  21
<212>  RNA
<213>  Artificial Sequence

<220>
<223>  Description of Artificial Sequence:  siNA sense region


<220>
<221>  misc_feature
<222>  (1)..(4)
<223>  Phosphorothioate 3'-Internucleotide Linkage

<220>
<221>  misc_feature
<222>  (3)..(3)
<223>  2'-deoxy-2'-fluoro

<220>
<221>  misc_feature
<222>  (5)..(7)
<223>  2'-deoxy-2'-fluoro

<220>
<221>  misc_feature
<222>  (11)..(13)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  550
ggugcuugga ucuggcgcun n                                                21


<210>  551
<211>  21
<212>  RNA
<213>  Artificial Sequence

<220>
<223>  Description of Artificial Sequence:  siNA antisense region


<220>
<221>  misc_feature
<222>  (1)..(20)
<223>  Phosphorothioate 3'-Internucleotide Linkage

<220>
<221>  misc_feature
<222>  (20)..(21)
<223>  n stands for thymidine

<400>  551
agcgccagau ccaagcaccn n                                                21


<210>  552
<211>  21
<212>  RNA
<213>  Artificial Sequence

<220>
<223>  Description of Artificial Sequence:  siNA antisense region


<220>
<221>  misc_feature
<222>  (1)..(10)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  552
agcgccagau ccaagcaccn n                                                21


<210>  553
<211>  21
<212>  RNA
<213>  Artificial Sequence

<220>
<223>  Description of Artificial Sequence:  siNA antisense region


<220>
<221>  misc_feature
<222>  (1)..(14)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  553
agcgccagau ccaagcaccn n                                                21

<210>  554
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy-2'-fluoro

<220>
<221>  misc_feature
<222>  (1)..(4)
<223>  Phosphorothioate 3'-Internucleotide Linkage

<220>
<221>  misc_feature
<222>  (4)..(4)
<223>  2'-deoxy-2'-fluoro

<220>
<221>  misc_feature
<222>  (7)..(9)
<223>  2'-deoxy-2'-fluoro

<220>
<221>  misc_feature
<222>  (13)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (17)..(20)
<223>  Phosphorothioate 3'-Internucleotide Linkage

<220>
<221>  misc_feature
<222>  (20)..(21)
<223>  n stands for thymidine

<400>  554
ucgcggucua gguucguggn n                                                21


<210>  555
<211>  21
<212>  RNA
<213>  Artificial Sequence

<220>
<223>  Description of Artificial Sequence:  inverted control/siNA sense

<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  555
ucgcggucua gguucguggn n                                                21


<210>  556
<211>  21
<212>  RNA
<213>  Artificial Sequence

<220>
<223>  Description of Artificial Sequence:  inverted control/siNA sense


<220>
<221>  misc_feature
<222>  (1)..(4)
<223>  Phosphorothioate 3'-Internucleotide Linkage

<220>
<221>  misc_feature
<222>  (1)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (7)..(9)
<223>  2'-deoxy-2'-fluoro

<220>
<221>  misc_feature
<222>  (13)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  556
ucgcggucua gguucguggn n                                                21


<210>  557
<211>  21
<212>  RNA
<213>  Artificial Sequence

<220>
<223>  Description of Artificial Sequence:  inverted control/siNA antisense


<220>
<221>  misc_feature
<222>  (1)..(5)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  557
ccacgaaccu agaccgcgan n                                                21


<210>  558
<211>  21
<212>  RNA
<213>  Artificial Sequence

<220>
<223>  Description of Artificial Sequence:  inverted control/siNA antisense


<220>
<221>  misc_feature
<222>  (1)..(20)
<223>  Phosphorothioate 3'-Internucleotide Linkage

<220>
<221>  misc_feature
<222>  (20)..(21)
<223>  n stands for thymidine

<400>  558
ccacgaaccu agaccgcgan n                                                21


<210>  559
<211>  21

<212>  RNA
<213>  Artificial Sequence

<220>
<223>  Description of Artificial Sequence:  inverted control/siNA antisense


<220>
<221>  misc_feature
<222>  (1)..(10)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  559
ccacgaaccu agaccgcgan n                                                21


<210>  560
<211>  21
<212>  RNA
<213>  Artificial Sequence

<220>
<223>  Description of Artificial Sequence:  inverted control/siNA antisense


<220>
<221>  misc_feature
<222>  (1)..(14)
<223>  Phosphorothioate 3'-Internucleotide Linkage

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  560
ccacgaaccu agaccgcgan n                                                21


<210>  561
<211>  21
<212>  RNA
<213>  Artificial Sequence

<220>
<223>  Description of Artificial Sequence:  siNA sense region

<220>
<221> misc_feature
<222> (1)..(1)
<223> 2'-deoxy-2'-fluoro

<220>
<221> misc_feature
<222> (1)..(1)
<223> 5'-3 attached terminal deoxyabasic moeity

<220>
<221> misc_feature
<222> (6)..(7)
<223> 2'-deoxy-2'-fluoro

<220>
<221> misc_feature
<222> (9)..(10)
<223> 2'-deoxy-2'-fluoro

<220>
<221> misc_feature
<222> (16)..(16)
<223> 2'-deoxy-2'-fluoro

<220>
<221> misc_feature
<222> (18)..(19)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 561
uggggucguc aaagacguun n 21


<210> 562
<211> 21
<212> RNA
<213> Artificial Sequence

<220>
<223> Description of Artificial Sequence: siNA antisense region


<220>
<221> misc_feature
<222> (3)..(3)
<223> 2'-deoxy-2'-fluoro

<220>
<221> misc_feature
<222> (5)..(9)
<223> 2'-deoxy-2'-fluoro

<220>
<221>  misc_feature
<222>  (12)..(12)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  562
aacgucuuug acgaccccan n                                                21


<210>  563
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (10)..(11)
<223>  2'-deoxy-2'-fluoro

<220>
<221>  misc_feature
<222>  (13)..(14)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  563
uugcagaaac ugcuggggun n                                                21


<210>  564
<211>  21
<212>  RNA
<213>  Artificial Sequence

<220>
<223>  Description of Artificial Sequence:  inverted control/siNA antisense


<220>
<221>  misc_feature
<222>  (2)..(5)
<223>  2'-deoxy-2'-fluoro

<220>
<221>  misc_feature
<222>  (8)..(8)
<223>  2'-deoxy-2'-fluoro

<220>
<221>  misc_feature
<222>  (11)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  564
accccagcag uuucugcaan n                                                21


<210>  565
<211>  21
<212>  RNA
<213>  Artificial Sequence

<220>

<223>  Description of Artificial Sequence:  siNA sense region

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (5)..(7)
<223>  2'-deoxy-2'-fluoro

<220>
<221>  misc_feature
<222>  (11)..(13)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  565
ggugcuugga ucuggcgcun n                                                21


<210>  566
<211>  21
<212>  RNA
<213>  Artificial Sequence

<220>
<223>  Description of Artificial Sequence:  siNA antisense region


<220>
<221>  misc_feature
<222>  (3)..(3)
<223>  2'-deoxy-2'-fluoro

<220>
<221>  misc_feature

<222>  (5)..(6)
<223>  2'-deoxy-2'-fluoro

<220>
<221>  misc_feature
<222>  (10)..(12)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  566
agcgccagau ccaagcaccn n                                                21


<210>  567
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (7)..(9)
<223>  2'-deoxy-2'-fluoro

<220>
<221>  misc_feature

<222>  (13)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  567
ucgcggucua gguucguggn n                                                21


<210>  568
<211>  21
<212>  RNA
<213>  Artificial Sequence

<220>
<223>  Description of Artificial Sequence:  inverted control/siNA antisense


<220>
<221>  misc_feature
<222>  (1)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (8)..(10)
<223>  2'-deoxy-2'-fluoro

<220>
<221>  misc_feature
<222>  (14)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature

<222>  (20)..(20)
<223>  Phosphorothioate 3'-Internucleotide Linkage

<400>  568
ccacgaaccu agaccgcgan n                                                21


<210>  569
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(7)
<223>  2'-deoxy-2'-fluoro

<220>
<221>  misc_feature
<222>  (9)..(10)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  569
uggggucguc aaagacguun n                                                21


<210>  570
<211>  21
<212>  RNA

<213>  Artificial Sequence

<220>
<223>  Description of Artificial Sequence:  inverted control/siNA sense


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (10)..(11)
<223>  2'-deoxy-2'-fluoro

<220>
<221>  misc_feature
<222>  (13)..(14)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  570
uugcagaaac ugcuggggun n                                                21


<210>  571
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (5)..(7)
<223>  2'-deoxy-2'-fluoro

<220>
<221>  misc_feature
<222>  (11)..(13)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  571
ggugcuugga ucuggcgcun n                                                21


<210>  572
<211>  21
<212>  RNA
<213>  Artificial Sequence

<220>
<223>  Description of Artificial Sequence:  inverted control/siNA sense


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221> misc_feature
<222> (7)..(9)
<223> 2'-deoxy-2'-fluoro

<220>
<221> misc_feature
<222> (13)..(15)
<223> 2'-deoxy-2'-fluoro

<220>
<221> misc_feature
<222> (17)..(17)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 572
ucgcggucua gguucguggn n 21


<210> 573
<211> 21
<212> RNA
<213> Artificial Sequence

<220>
<223> Description of Artificial Sequence: siNA sense region


<220>
<221> misc_feature
<222> (1)..(2)
<223> 2'-deoxy

<220>
<221> misc_feature
<222> (1)..(1)
<223> 5'-3 attached terminal deoxyabasic moeity

<220>
<221> misc_feature
<222> (3)..(3)
<223> 2'-deoxy-2'-fluoro

<220>
<221> misc_feature
<222> (4)..(4)
<223> 2'-deoxy

<220>
<221> misc_feature
<222> (5)..(7)
<223> 2'-deoxy-2'-fluoro

<220>
<221>  misc_feature
<222>  (8)..(10)
<223>  2'-deoxy

<220>
<221>  misc_feature
<222>  (11)..(13)
<223>  2'-deoxy-2'-fluoro

<220>
<221>  misc_feature
<222>  (14)..(15)
<223>  2'-deoxy

<220>
<221>  misc_feature
<222>  (16)..(16)
<223>  2'-deoxy-2'-fluoro

<220>
<221>  misc_feature
<222>  (17)..(17)
<223>  2'-deoxy

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  573
ggugcuugga ucuggcgcun n                                                21


<210>  574
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy

<220>
<221>  misc_feature
<222>  (1)..(1)
<223>  5'-3 attached terminal deoxyabasic moeity

<220>
<221> misc_feature
<222> (3)..(3)
<223> 2'-deoxy-2'-fluoro

<220>
<221> misc_feature
<222> (4)..(4)
<223> 2'-deoxy

<220>
<221> misc_feature
<222> (5)..(9)
<223> 2'-deoxy-2'-fluoro

<220>
<221> misc_feature
<222> (10)..(11)
<223> 2'-deoxy

<220>
<221> misc_feature
<222> (12)..(12)
<223> 2'-deoxy-2'-fluoro

<220>
<221> misc_feature
<222> (13)..(14)
<223> 2'-deoxy

<220>
<221> misc_feature
<222> (15)..(18)
<223> 2'-deoxy-2'-fluoro

<220>
<221> misc_feature
<222> (19)..(19)
<223> 2'-deoxy

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 574
aacgucuuug acgaccccan n                                                21


<210> 575
<211> 21
<212> RNA
<213> Artificial Sequence

<220>
<223> Description of Artificial Sequence: siNA antisense region

<220>
<221>  misc_feature
<222>  (3)..(3)
<223>  2'-deoxy-2'-fluoro

<220>
<221>  misc_feature
<222>  (5)..(6)
<223>  2'-deoxy-2'-fluoro

<220>
<221>  misc_feature
<222>  (10)..(12)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  575
agcgccagau ccaagcaccn n                                                21


<210>  576
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (6)..(7)
<223>  2'-deoxy-2'-fluoro

<220>
<221>  misc_feature
<222>  (9)..(10)
<223>  2'-deoxy-2'-fluoro

<220>
<221> misc_feature
<222> (16)..(16)
<223> 2'-deoxy-2'-fluoro

<220>
<221> misc_feature
<222> (18)..(19)
<223> 2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400> 576
uggggucguc aaagacguun n                                                21


<210> 577
<211> 21
<212> RNA
<213> Artificial Sequence

<220>
<223> Description of Artificial Sequence: siNA sense region


<220>
<221> misc_feature
<222> (1)..(1)
<223> 5'-3 attached terminal deoxyabasic moeity

<220>
<221> misc_feature
<222> (4)..(4)
<223> 2'-deoxy-2'-fluoro

<220>
<221> misc_feature
<222> (7)..(9)
<223> 2'-deoxy-2'-fluoro

<220>
<221> misc_feature
<222> (12)..(12)
<223> 2'-deoxy-2'-fluoro

<220>
<221> misc_feature
<222> (15)..(17)
<223> 2'-deoxy-2'-fluoro

<220>
<221> misc_feature
<222> (19)..(19)
<223> 2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  577
gaauggcuca gugaccugun n                                                21


<210>  578
<211>  21
<212>  RNA
<213>  Artificial Sequence

<220>
<223>  Description of Artificial Sequence:  siNA antisense region


<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (5)..(9)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  578
aacgucuuug acgaccccan n                                                21

<210>  579
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(7)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  579
caccuucaaa gggacaccun n                                                21


<210>  580
<211>  21
<212>  RNA
<213>  Artificial Sequence

<220>
<223>  Description of Artificial Sequence:  siNA antisense region


<220>
<221>  misc_feature
<222>  (2)..(2)
<223>  2'-deoxy-2'-fluoro

<220>

<221>  misc_feature
<222>  (6)..(7)
<223>  2'-deoxy-2'-fluoro

<220>
<221>  misc_feature
<222>  (9)..(10)
<223>  2'-deoxy-2'-fluoro

<220>
<221>  misc_feature
<222>  (14)..(15)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  580
acaggucacu gagccauucn n                                                21


<210>  581
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (6)..(7)
<223>  2'-deoxy-2'-fluoro

<220>
<221>  misc_feature
<222>  (9)..(10)
<223>  2'-deoxy-2'-fluoro

<220>
<221>  misc_feature
<222>  (16)..(16)
<223>  2'-deoxy-2'-fluoro

<220>

<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  581
uggggucguc aaagacguun n                                                21


<210>  582
<211>  21
<212>  RNA
<213>  Artificial Sequence

<220>
<223>  Description of Artificial Sequence:  siNA antisense region


<220>
<221>  misc_feature
<222>  (4)..(4)
<223>  2'-deoxy-2'-fluoro

<220>
<221>  misc_feature
<222>  (6)..(12)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  582
agguguccu uugaaggugn n                                                 21


<210>  583
<211>  21
<212>  RNA
<213>  Artificial Sequence

<220>
<223>  Description of Artificial Sequence:  siNA sense region

<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (2)..(5)
<223>  2'-deoxy

<220>
<221>  misc_feature
<222>  (6)..(7)
<223>  2'-deoxy-2'-fluoro

<220>
<221>  misc_feature
<222>  (8)..(8)
<223>  2'-deoxy

<220>
<221>  misc_feature
<222>  (9)..(10)
<223>  2'-deoxy-2'-fluoro

<220>
<221>  misc_feature
<222>  (11)..(15)
<223>  2'-deoxy

<220>
<221>  misc_feature
<222>  (16)..(16)
<223>  2'-deoxy-2'-fluoro

<220>
<221>  misc_feature
<222>  (17)..(17)
<223>  2'-deoxy

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  583

uggggucguc aaagacguun n                                              21

<210>  584
<211>  21
<212>  RNA
<213>  Artificial Sequence

<220>
<223>  Description of Artificial Sequence:  siNA antisense region

<220>
<221>  misc_feature
<222>  (3)..(3)
<223>  2'-deoxy-2'-fluoro

<220>
<221>  misc_feature
<222>  (5)..(9)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  584
aacgucuuug acgaccccan n                                              21

<210>  585
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  585
accauuuugu ggacgaauan n                                              21

<210>  586
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  586
cuguuggaca uccuggauan n                                                21


<210>  587
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  587
ggaugccuuc uacacguugn n                                                21


<210>  588
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  588
gaacccuccu gaugagagun n                                                21


<210>  589
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  589
uauucgucca caaaauggun n                                                21


<210>  590
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  590
uauccaggau guccaacagn n                                                21


<210>  591
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  591
caacguguag aaggcauccn n                                                21


<210>  592
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  592
acucucauca ggaggguucn n                                                21

<210> 593
<211> 21
<212> RNA
<213> Artificial Sequence

<220>
<223> Description of Artificial Sequence: siNA sense region

<400> 593
agcuuggcca auccgugcgg u 21


<210> 594
<211> 21
<212> RNA
<213> Artificial Sequence

<220>
<223> Description of Artificial Sequence: siNA sense region

<400> 594
uugcggaggg ugggccuggg a 21


<210> 595
<211> 21
<212> RNA
<213> Artificial Sequence

<220>
<223> Description of Artificial Sequence: siNA sense region

<400> 595
cugccgccuu ccaccguuca u 21


<210> 596
<211> 21
<212> RNA
<213> Artificial Sequence

<220>
<223> Description of Artificial Sequence: siNA sense region

<400> 596
acccacugcc accgcgaaga g 21


<210> 597
<211> 21
<212> RNA
<213> Artificial Sequence

<220>
<223> Description of Artificial Sequence: siNA sense region

<400> 597
gcggcgcgau ucccugagcu g 21


<210> 598
<211> 21

<212>  RNA
<213>  Artificial Sequence

<220>
<223>  Description of Artificial Sequence:  siNA antisense region

<400>  598
cgcacggauu ggccaagcug a                                                21


<210>  599
<211>  21
<212>  RNA
<213>  Artificial Sequence

<220>
<223>  Description of Artificial Sequence:  siNA antisense region

<400>  599
ccaggcccac ccuccgcaac c                                                21


<210>  600
<211>  21
<212>  RNA
<213>  Artificial Sequence

<220>
<223>  Description of Artificial Sequence:  siNA antisense region

<400>  600
gaacggugga aggcggcagg c                                                21


<210>  601
<211>  21
<212>  RNA
<213>  Artificial Sequence

<220>
<223>  Description of Artificial Sequence:  siNA antisense region

<400>  601
cuucgcggug gcagugggug c                                                21


<210>  602
<211>  21
<212>  RNA
<213>  Artificial Sequence

<220>
<223>  Description of Artificial Sequence:  siNA antisense region

<400>  602
gcucagggaa ucgcgccgcg c                                                21


<210>  603
<211>  21
<212>  RNA
<213>  Artificial Sequence

<220>
<223>  Description of Artificial Sequence:  siNA sense region


<220>
<221>  misc_feature
<222>  (1)..(7)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (9)..(9)
<223>  2'-deoxy-2'-fluoro

<220>
<221>  misc_feature
<222>  (13)..(14)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  603
ucccuuuaua agccgacucn n                                                21


<210>  604
<211>  21
<212>  RNA
<213>  Artificial Sequence

<220>
<223>  Description of Artificial Sequence:  siNA sense region


<220>
<221>  misc_feature
<222>  (1)..(4)
<223>  2'-deoxy-2'-fluoro

<220>
<221>  misc_feature
<222>  (1)..(1)
<223>  5'-3 attached terminal deoxyabasic moeity

<220>

<221>  misc_feature
<222>  (6)..(7)
<223>  2'-deoxy-2'-fluoro

<220>
<221>  misc_feature
<222>  (9)..(11)
<223>  2'-deoxy-2'-fluoro

<220>
<221>  misc_feature
<222>  (13)..(16)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  604
uuccaccguu cauucuagan n                                                21


<210>  605
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (7)..(9)
<223>  2'-deoxy-2'-fluoro

<220>
<221>  misc_feature
<222>  (11)..(14)
<223>  2'-deoxy-2'-fluoro

<220>

<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  605
ccaccguuca uucuagagcn n                                                21


<210>  606
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (7)..(8)
<223>  2'-deoxy-2'-fluoro

<220>
<221>  misc_feature
<222>  (12)..(15)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3-3 attached terminal deoxyabasic moeity

<400>  606
gaagaguugg gcucugucan n                                                21


<210>  607
<211>  21

<212>  RNA
<213>  Artificial Sequence

<220>
<223>  Description of Artificial Sequence:  siNA antisense region


<220>
<221>  misc_feature
<222>  (4)..(5)
<223>  2'-deoxy-2'-fluoro

<220>
<221>  misc_feature
<222>  (8)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  607
gagucggcuu auaaagggan n                                                21


<210>  608
<211>  21
<212>  RNA
<213>  Artificial Sequence

<220>
<223>  Description of Artificial Sequence:  siNA antisense region


<220>
<221>  misc_feature
<222>  (1)..(3)
<223>  2'-deoxy-2'-fluoro

<220>
<221>  misc_feature
<222>  (8)..(8)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(15)

<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  608
ucuagaauga acgguggaan n                                                21


<210>  609
<211>  21
<212>  RNA
<213>  Artificial Sequence

<220>
<223>  Description of Artificial Sequence:  siNA antisense region


<220>
<221>  misc_feature
<222>  (2)..(5)
<223>  2'-deoxy-2'-fluoro

<220>
<221>  misc_feature
<222>  (10)..(10)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  609
gcucuagaau gaacggugg n n                                                21


<210>  610
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (4)..(4)
<223>  2'-deoxy-2'-fluoro

<220>
<221>  misc_feature
<222>  (9)..(11)
<223>  2'-deoxy-2'-fluoro

<220>
<221>  misc_feature
<222>  (14)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  610
ugacagagcc caacucuucn n                                                21


<210>  611
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (7)..(8)
<223>  2'-deoxy-2'-fluoro

<220>
<221>  misc_feature
<222>  (11)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  611
gaagagccaa cugugugagn n                                                21


<210>  612
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (16)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  612
agggaggaga aggaguuccn n                                                21


<210>  613
<211>  21
<212>  RNA
<213>  Artificial Sequence

<220>

<223>  Description of Artificial Sequence:  siNA sense region

<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (10)..(10)
<223>  2'-deoxy-2'-fluoro

<220>
<221>  misc_feature
<222>  (14)..(15)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  613
ggaguacagc aaacugaagn n                                                21

<210>  614
<211>  21
<212>  RNA
<213>  Artificial Sequence

<220>
<223>  Description of Artificial Sequence:  siNA sense region

<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature

<222>  (3)..(3)
<223>  2'-deoxy-2'-fluoro

<220>
<221>  misc_feature
<222>  (8)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(13)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  614
cauggagugc auugaguagn n                                                21


<210>  615
<211>  21
<212>  RNA
<213>  Artificial Sequence

<220>
<223>  Description of Artificial Sequence:  siNA antisense region


<220>
<221>  misc_feature
<222>  (1)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (7)..(7)
<223>  2'-deoxy-2'-fluoro

<220>
<221>  misc_feature

<222>  (10)..(11)
<223>  2'-deoxy-2'-fluoro

<220>
<221>  misc_feature
<222>  (14)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  615
cucacacagu uggcucuucn n                                                21


<210>  616
<211>  21
<212>  RNA
<213>  Artificial Sequence

<220>
<223>  Description of Artificial Sequence:  siNA antisense region


<220>
<221>  misc_feature
<222>  (5)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  616
ggaacuccuu cuccucccun n                                                21


<210>  617
<211>  21
<212>  RNA
<213>  Artificial Sequence

<220>
<223>  Description of Artificial Sequence:  siNA antisense region


<220>
<221>  misc_feature
<222>  (1)..(4)
<223>  2'-deoxy-2'-fluoro

<220>
<221> misc_feature
<222> (7)..(9)
<223> 2'-deoxy-2'-fluoro

<220>
<221> misc_feature
<222> (11)..(12)
<223> 2'-deoxy-2'-fluoro

<220>
<221> misc_feature
<222> (14)..(14)
<223> 2'-deoxy-2'-fluoro

<220>
<221> misc_feature
<222> (16)..(19)
<223> 2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400> 617
cuucaguuug cuguacuccn n                                                21


<210> 618
<211> 21
<212> RNA
<213> Artificial Sequence

<220>
<223> Description of Artificial Sequence: siNA antisense region


<220>
<221> misc_feature
<222> (1)..(2)
<223> 2'-deoxy-2'-fluoro

<220>
<221> misc_feature
<222> (4)..(6)
<223> 2'-deoxy-2'-fluoro

<220>
<221> misc_feature
<222> (9)..(9)
<223> 2'-deoxy-2'-fluoro

<220>
<221> misc_feature
<222> (11)..(11)
<223> 2'-deoxy-2'-fluoro

<220>
<221> misc_feature
<222> (13)..(16)
<223> 2'-deoxy-2'-fluoro

<220>
<221> misc_feature
<222> (18)..(18)
<223> 2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400> 618
cuacucaaug cacuccaugn n 21


<210> 619
<211> 21
<212> RNA
<213> Artificial Sequence

<220>
<223> Description of Artificial Sequence: siNA sense region


<220>
<221> misc_feature
<222> (1)..(1)
<223> 5'-3 attached terminal deoxyabasic moeity

<220>
<221> misc_feature
<222> (2)..(5)
<223> 2'-deoxy-2'-fluoro

<220>
<221> misc_feature
<222> (8)..(12)
<223> 2'-deoxy-2'-fluoro

<220>
<221> misc_feature
<222> (14)..(15)
<223> 2'-deoxy-2'-fluoro

<220>
<221> misc_feature
<222> (17)..(17)
<223> 2'-deoxy-2'-fluoro

<220>
<221> misc_feature
<222> (20)..(21)
<223> n stands for thymidine

<220>
<221>  misc_feature
<222>  (21)..(21)
<223>  3'-3 attached terminal deoxyabasic moeity

<400>  619
accucaacuc cugccacaan n                                                21


<210>  620
<211>  21
<212>  RNA
<213>  Artificial Sequence

<220>
<223>  Description of Artificial Sequence:  siNA sense region


<220>
<221>  misc_feature
<222>  (1)..(5)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (7)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  620
cuccugucuu gcauugcacn n                                                21

<210>  621
<211>  21
<212>  RNA
<213>  Artificial Sequence

<220>
<223>  Description of Artificial Sequence:  siNA sense region

<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (6)..(8)
<223>  2'-deoxy-2'-fluoro

<220>
<221>  misc_feature
<222>  (10)..(11)
<223>  2'-deoxy-2'-fluoro

<220>
<221>  misc_feature
<222>  (13)..(13)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  621
uugcacuugu cacaaacagn n                                                21

<210>  622
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (6)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(13)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  622
cacagcuaca acuggagcan n                                                21


<210>  623
<211>  21
<212>  RNA
<213>  Artificial Sequence

<220>
<223>  Description of Artificial Sequence:  siNA antisense region

<220>
<221>  misc_feature
<222>  (1)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (7)..(7)
<223>  2'-deoxy-2'-fluoro

<220>
<221>  misc_feature
<222>  (13)..(14)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  623
uuguggcagg aguugaggun n                                                21

<210>  624
<211>  21
<212>  RNA
<213>  Artificial Sequence

<220>
<223>  Description of Artificial Sequence:  siNA antisense region

<220>
<221>  misc_feature
<222>  (2)..(2)
<223>  2'-deoxy-2'-fluoro

<220>
<221>  misc_feature
<222>  (4)..(4)
<223>  2'-deoxy-2'-fluoro

<220>

<221>  misc_feature
<222>  (7)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (14)..(14)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  624
gugcaaugca agacaggagn n                                                21


<210>  625
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(6)
<223>  2'-deoxy-2'-fluoro

<220>
<221>  misc_feature
<222>  (8)..(8)
<223>  2'-deoxy-2'-fluoro

<220>
<221>  misc_feature
<222>  (11)..(11)
<223>  2'-deoxy-2'-fluoro

<220>
<221>  misc_feature
<222>  (15)..(15)
<223>  2'-deoxy-2'-fluoro

<220>

<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  625
cuguuuguga caagugcaan n                                                21


<210>  626
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(6)
<223>  2'-deoxy-2'-fluoro

<220>
<221>  misc_feature
<222>  (9)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(16)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>

<221>  misc_feature
<222>  (20)..(20)
<223>  Phosphorothioate 3'-Internucleotide Linkage

<400>  626
ugcuccaguu guagcugugn n                                                21


<210>  627
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  627
accucaacuc cugccacaan n                                                21


<210>  628
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  628
cuccugucuu gcauugcacn n                                                21


<210>  629
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  629
uugcacuugu cacaaacagn n                                                21


<210>  630

<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  630
cacagcuaca acuggagcan n                                                21


<210>  631
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  631
uuguggcagg aguugaggun n                                                21


<210>  632
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  632
gugcaaugca agacaggagn n                                                21


<210>  633
<211>  21
<212>  RNA
<213>  Artificial Sequence

<220>
<223>  Description of Artificial Sequence:  siNA antisense region


<220>

<221>  misc_feature
<222>  (20)..(21)
<223>  n stands for thymidine

<400>  633
cuguuuguga caagugcaan n                                                21


<210>  634
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  634
ugcuccaguu guagcugugn n                                                21


<210>  635
<211>  21
<212>  RNA
<213>  Artificial Sequence

<220>
<223>  Description of Artificial Sequence:  siNA sense region


<220>
<221>  misc_feature
<222>  (1)..(3)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (5)..(7)
<223>  2'-deoxy-2'-fluoro

<220>
<221>  misc_feature
<222>  (9)..(11)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)

<223>  n stands for thymidine

<220>
<221>  misc_feature
<222>  (21)..(21)
<223>  3'-3 attached terminal deoxyabasic moeity

<400>  635
uccacuuacc ugaggagcan n                                                21


<210>  636
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  636
ugagcaugga agaggauucn n                                                21


<210>  637
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (3)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  637
gguucuugcc ucagaagagn n                                                21


<210>  638
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (7)..(9)
<223>  2'-deoxy-2'-fluoro

<220>
<221>  misc_feature
<222>  (13)..(14)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  638
ugaaggcuca aaccagacan n                                                21


<210>  639
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(8)
<223>  2'-deoxy-2'-fluoro

<220>
<221>  misc_feature
<222>  (12)..(12)
<223>  2'-deoxy-2'-fluoro

<220>
<221>  misc_feature
<222>  (16)..(16)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  639
ugcuccucag guaaguggan n                                                21


<210>  640

<211>  21
<212>  RNA
<213>  Artificial Sequence

<220>
<223>  Description of Artificial Sequence:  siNA antisense region


<220>
<221>  misc_feature
<222>  (4)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (16)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  640
gaauccucuu ccaugcucan n                                                21


<210>  641
<211>  21
<212>  RNA
<213>  Artificial Sequence

<220>
<223>  Description of Artificial Sequence:  siNA antisense region


<220>
<221>  misc_feature
<222>  (1)..(7)
<223>  2'-deoxy-2'-fluoro

<220>
<221>  misc_feature
<222>  (12)..(12)
<223>  2'-deoxy-2'-fluoro

<220>
<221>  misc_feature
<222>  (18)..(19)
<223>  2'-deoxy-2'-fluoro

<220>
<221>  misc_feature

<222>  (20)..(21)
<223>  n stands for thymidine

<220>
<221>  misc_feature
<222>  (20)..(20)
<223>  Phosphorothioate 3'-Internucleotide Linkage

<400>  641
cucuucugag gcaagaaccn n                                                21


<210>  642
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(5)
<223>  2'-deoxy-2'-fluoro

<220>
<221>  misc_feature
<222>  (8)..(10)
<223>  2'-deoxy-2'-fluoro

<220>
<221>  misc_feature
<222>  (14)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  642
ugucugguuu gagccuucan n                                                21


<210>  643
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  643
uccacuuacc ugaggagcan n                                                21


<210>  644
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  644
ugagcaugga agaggauucn n                                                21


<210>  645
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  645
gguucuugcc ucagaagagn n                                                21


<210>  646
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  646
ugaaggcuca aaccagacan n                                                21

<210>  647
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  647
ugcuccucag guaaguggan n                                                 21


<210>  648
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  648
gaauccucuu ccaugcucan n                                                 21


<210>  649
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  649
cucuucugag gcaagaaccn n                                                 21


<210>  650
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  650
ugucugguuu gagccuucan n                                                21


<210>  651
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  651
accuuggagc aucucaucun n                                                21


<210>  652
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  652
accuguuucc uguauggagn n                                                21


<210>  653
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  653
caacacagca ggaaucagun n                                                21

<210> 654
<211> 21
<212> RNA
<213> Artificial Sequence

<220>
<223> Description of Artificial Sequence: siNA antisense region


<220>
<221> misc_feature
<222> (20)..(21)
<223> n stands for thymidine

<400> 654
agaugagaug cuccaaggun n 21


<210> 655
<211> 21
<212> RNA
<213> Artificial Sequence

<220>
<223> Description of Artificial Sequence: siNA antisense region


<220>
<221> misc_feature
<222> (20)..(21)
<223> n stands for thymidine

<400> 655
cuccauacag gaaacaggun n 21


<210> 656
<211> 21
<212> RNA
<213> Artificial Sequence

<220>
<223> Description of Artificial Sequence: siNA antisense region


<220>
<221> misc_feature
<222> (20)..(21)
<223> n stands for thymidine

<400> 656
acugauuccu gcuguguugn n 21


<210> 657
<211> 21
<212> RNA
<213> Artificial Sequence

<220>
<223> Description of Artificial Sequence: siNA sense region

<220>
<221>  misc_feature
<222>  (20)..(21)
<223>  n stands for thymidine

<400>  657
aagacagggu guugaugaun n                                                21


<210>  658
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  658
uccucgaagu gccaguauun n                                                21


<210>  659
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  659
uucugucuug ggguuuuugn n                                                21


<210>  660
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  660
uuuuggugca ugcaguugan n                                                21

<210>  661
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  661
aucaucaaca cccugucuun n                                                21


<210>  662
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  662
aauacuggca cuucgaggan n                                                21


<210>  663
<211>  21
<212>  RNA
<213>  Artificial Sequence

<220>
<223>  Description of Artificial Sequence:  siNA antisense region


<220>
<221>  misc_feature
<222>  (20)..(21)
<223>  n is a, c, g, or u

<400>  663
caaaaacccc aagacagaan n                                                21


<210>  664
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  664
ucaacugcau gcaccaaaan n                                                21


<210>  665
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (5)..(7)
<223>  2'-deoxy-2'-fluoro

<220>
<221>  misc_feature
<222>  (9)..(10)
<223>  2'-deoxy-2'-fluoro

<220>
<221>  misc_feature
<222>  (12)..(13)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  665
cagaccuacu gccagagaan n                                                21


<210>  666
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (10)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  666
cacacagggu uccugacagn n                                                21


<210>  667
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>

<221>  misc_feature
<222>  (3)..(6)
<223>  2'-deoxy-2'-fluoro

<220>
<221>  misc_feature
<222>  (9)..(10)
<223>  2'-deoxy-2'-fluoro

<220>
<221>  misc_feature
<222>  (12)..(13)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  667
ggcucuaguc acuggcaucn n                                                21


<210>  668
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>

<221>  misc_feature
<222>  (8)..(9)
<223>  2'-deoxy-2'-fluoro

<220>
<221>  misc_feature
<222>  (12)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  668
uguggaguug acucggugun n                                                21


<210>  669
<211>  21
<212>  RNA
<213>  Artificial Sequence

<220>
<223>  Description of Artificial Sequence:  siNA antisense region


<220>
<221>  misc_feature
<222>  (1)..(6)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(12)
<223>  2'-deoxy-2'-fluoro

<220>
<221>  misc_feature
<222>  (16)..(18)
<223>  2'-deoxy-2'-fluoro

<220>

<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  669
uucucuggca guaggucugn n                                                21


<210>  670
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (11)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  670
cugucaggaa cccugugugn n                                                21


<210>  671
<211>  21

<212>  RNA
<213>  Artificial Sequence

<220>
<223>  Description of Artificial Sequence:  siNA antisense region


<220>
<221>  misc_feature
<222>  (3)..(3)
<223>  2'-deoxy-2'-fluoro

<220>
<221>  misc_feature
<222>  (5)..(6)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(13)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  671
gaugccagug acuagagccn n                                                21


<210>  672
<211>  21
<212>  RNA
<213>  Artificial Sequence

<220>
<223>  Description of Artificial Sequence:  siNA antisense region


<220>
<221>  misc_feature
<222>  (2)..(2)
<223>  2'-deoxy-2'-fluoro

<220>
<221>  misc_feature
<222>  (4)..(5)

<223>  2'-deoxy-2'-fluoro

<220>
<221>  misc_feature
<222>  (9)..(10)
<223>  2'-deoxy-2'-fluoro

<220>
<221>  misc_feature
<222>  (13)..(16)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  672
acaccgaguc aacuccacan n                                                21


<210>  673
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  673
cuacuuugcu caguaccacn n                                                21


<210>  674
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  674
ucugucuuug ugggagggun n                                                21

<210>  675
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  675
aagggucuuc uuggcagcun n                                                21

<210>  676
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  676
acucuaagcu ggagcucuun n                                                21

<210>  677
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  677
gugguacuga gcaaaguagn n                                                21

<210>  678
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  678
acccucccac aaagacagan n                                                21


<210>  679
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  679
agcugccaag aagacccuun n                                                21


<210>  680
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  680
aagagcucca gcuuagagun n                                                21


<210>  681
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  681
cagcuuggaa caccaugucn n                                                21

<210>  682
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  682
uucccagcug acucagaacn n                                                21

<210>  683
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  683
augucaacag auccgacuun n                                                21

<210>  684
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  684
uuggccucug auacagacan n                                                21

<210>  685
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  685
gacauggugu uccaagcugn n                                                21

<210>  686
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  686
guucugaguc agcugggaan n                                                21

<210>  687
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  687
aagucggauc uguugacaun n                                                21

<210>  688
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  688
ugucuguauc agaggccaan n                                                21

<210>  689
<211>  21
<212>  RNA
<213>  Artificial Sequence

<220>
<223>  Description of Artificial Sequence:  siNA sense region

<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (5)..(5)
<223>  2'-deoxy-2'-fluoro

<220>
<221>  misc_feature
<222>  (11)..(11)
<223>  2'-deoxy-2'-fluoro

<220>
<221>  misc_feature
<222>  (14)..(14)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  689
gugacgagga ugaugaggan n                                                21

<210>  690
<211>  21
<212>  RNA
<213>  Artificial Sequence

<220>
<223>  Description of Artificial Sequence:  siNA sense region

<220>
<221>  misc_feature

<222>  (1)..(1)
<223>  5'-3 attached terminal deoxyabasic moeity

<220>
<221>  misc_feature
<222>  (3)..(5)
<223>  2'-deoxy-2'-fluoro

<220>
<221>  misc_feature
<222>  (7)..(8)
<223>  2'-deoxy-2'-fluoro

<220>
<221>  misc_feature
<222>  (10)..(11)
<223>  2'-deoxy-2'-fluoro

<220>
<221>  misc_feature
<222>  (13)..(14)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  690
ggucuguuau ugccaagcan n                                                21


<210>  691
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature

<222>  (4)..(4)
<223>  2'-deoxy-2'-fluoro

<220>
<221>  misc_feature
<222>  (10)..(14)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  691
cugcaggagu cuucaaaagn n                                                21


<210>  692
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(8)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(13)
<223>  2'-deoxy-2'-fluoro

<220>
<221>  misc_feature

<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  692
gugcuaccaa cacagaaccn n                                                21


<210>  693
<211>  21
<212>  RNA
<213>  Artificial Sequence

<220>
<223>  Description of Artificial Sequence:  siNA antisense region


<220>
<221>  misc_feature
<222>  (1)..(5)
<223>  2'-deoxy-2'-fluoro

<220>
<221>  misc_feature
<222>  (7)..(8)
<223>  2'-deoxy-2'-fluoro

<220>
<221>  misc_feature
<222>  (10)..(14)
<223>  2'-deoxy-2'-fluoro

<220>
<221>  misc_feature
<222>  (16)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  693
uccucaucau ccucgucacn n                                                21

<210>  694
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(5)
<223>  2'-deoxy-2'-fluoro

<220>
<221>  misc_feature
<222>  (8)..(8)
<223>  2'-deoxy-2'-fluoro

<220>
<221>  misc_feature
<222>  (11)..(11)
<223>  2'-deoxy-2'-fluoro

<220>
<221>  misc_feature
<222>  (14)..(14)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  694
ugcuuggcaa uaacagaccn n                                                21


<210>  695
<211>  21
<212>  RNA
<213>  Artificial Sequence

<220>
<223>  Description of Artificial Sequence:  siNA antisense region

<220>
<221>  misc_feature
<222>  (1)..(5)
<223>  2'-deoxy-2'-fluoro

<220>
<221>  misc_feature
<222>  (11)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  695
cuuuugaaga cuccugcagn n                                                21


<210>  696
<211>  21
<212>  RNA
<213>  Artificial Sequence

<220>
<223>  Description of Artificial Sequence:  siNA antisense region


<220>
<221>  misc_feature
<222>  (3)..(6)
<223>  2'-deoxy-2'-fluoro

<220>
<221>  misc_feature
<222>  (8)..(8)
<223>  2'-deoxy-2'-fluoro

<220>
<221>  misc_feature
<222>  (10)..(11)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  696
gguucugugu ugguagcacn n                                                21


<210>  697
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  697
gugacgagga ugaugaggan n                                                21


<210>  698
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  698
ggucuguuau ugccaagcan n                                                21


<210>  699
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  699
cugcaggagu cuucaaaagn n                                                21


<210>  700
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  700
gugcuaccaa cacagaaccn n                                                21


<210>  701
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  701
uccucaucau ccucgucacn n                                                21


<210>  702
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  702
ugcuuggcaa uaacagaccn n                                                21

<210>  703
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  703
cuuuugaaga cuccugcagn n                                                  21


<210>  704
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  704
gguucugugu ugguagcacn n                                                  21


<210>  705
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (7)..(8)
<223>  2'-deoxy-2'-fluoro

<220>
<221>  misc_feature
<222>  (12)..(13)
<223>  2'-deoxy-2'-fluoro

<220>

<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  705
agagggucaa guuggacagn n                                                21


<210>  706
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (10)..(10)
<223>  2'-deoxy-2'-fluoro

<220>
<221>  misc_feature
<222>  (16)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  706
gcagaggagc aaaagcucan n                                                21


<210>  707
<211>  21

<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  707
cggaacucuu gugcguaagn n                                                21


<210>  708
<211>  21
<212>  RNA
<213>  Artificial Sequence

<220>
<223>  Description of Artificial Sequence:  siNA sense region


<220>
<221>  misc_feature
<222>  (1)..(1)

<223>  5'-3 attached terminal deoxyabasic moeity

<220>
<221>  misc_feature
<222>  (3)..(6)
<223>  2'-deoxy-2'-fluoro

<220>
<221>  misc_feature
<222>  (9)..(10)
<223>  2'-deoxy-2'-fluoro

<220>
<221>  misc_feature
<222>  (14)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  708
aaccuuggcu gagucuugan n                                                21


<210>  709
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(6)
<223>  2'-deoxy-2'-fluoro

<220>
<221>  misc_feature
<222>  (9)..(11)
<223>  2'-deoxy-2'-fluoro

<220>
<221>  misc_feature
<222>  (14)..(19)
<223>  2'-deoxy-2'-fluoro

<220>
<221>  misc_feature
<222>  (20)..(21)

<223>  n stands for thymidine

<220>
<221>  misc_feature
<222>  (20)..(20)
<223>  Phosphorothioate 3'-Internucleotide Linkage

<400>  709
cuguccaacu ugacccucun n                                                21


<210>  710
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (5)..(9)
<223>  2'-deoxy-2'-fluoro

<220>
<221>  misc_feature
<222>  (11)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  710
ugagcuuuug cuccucugcn n                                                21


<210>  711
<211>  21
<212>  RNA
<213>  Artificial Sequence

<220>
<223>  Description of Artificial Sequence:  siNA antisense region

<220>
<221>  misc_feature
<222>  (1)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  711
cuuacgcaca agaguuccgn n                                                21


<210>  712
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (7)..(9)
<223>  2'-deoxy-2'-fluoro

<220>
<221>  misc_feature
<222>  (12)..(13)
<223>  2'-deoxy-2'-fluoro

<220>
<221> misc_feature
<222> (18)..(19)
<223> 2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400> 712
ucaagacuca gccaagguun n 21


<210> 713
<211> 21
<212> RNA
<213> Artificial Sequence

<220>
<223> Description of Artificial Sequence: siNA sense region


<220>
<221> misc_feature
<222> (20)..(21)
<223> n stands for thymidine

<400> 713
agagggucaa guuggacagn n 21


<210> 714
<211> 21
<212> RNA
<213> Artificial Sequence

<220>
<223> Description of Artificial Sequence: siNA sense region


<220>
<221> misc_feature
<222> (20)..(21)
<223> n stands for thymidine

<400> 714
gcagaggagc aaaagcucan n 21


<210> 715
<211> 21
<212> RNA
<213> Artificial Sequence

<220>
<223> Description of Artificial Sequence: siNA sense region

<220>
<221>  misc_feature
<222>  (20)..(21)
<223>  n stands for thymidine

<400>  715
cggaacucuu gugcguaagn n                                                21


<210>  716
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  716
aaccuuggcu gagucuugan n                                                21


<210>  717
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  717
cuguccaacu ugacccucun n                                                21


<210>  718
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  718
ugagcuuuug cuccucugcn n                                                21

<210>  719
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  719
cuuacgcaca agaguuccgn n                                                21


<210>  720
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  720
ucaagacuca gccaagguun n                                                21


<210>  721
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy

<220>
<221>  misc_feature
<222>  (1)..(1)
<223>  5'-3 attached terminal deoxyabasic moeity

<220>
<221>  misc_feature
<222>  (3)..(6)
<223>  2'-deoxy-2'-fluoro

<220>
<221>  misc_feature

<222>  (7)..(8)
<223>  2'-deoxy

<220>
<221>  misc_feature
<222>  (9)..(10)
<223>  2'-deoxy-2'-fluoro

<220>
<221>  misc_feature
<222>  (11)..(13)
<223>  2'-deoxy

<220>
<221>  misc_feature
<222>  (14)..(17)
<223>  2'-deoxy-2'-fluoro

<220>
<221>  misc_feature
<222>  (18)..(19)
<223>  2'-deoxy

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  721
aaccuuggcu gagucuugan n                                                21


<210>  722
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (3)..(6)
<223>  2'-deoxy

<220>
<221>  misc_feature
<222>  (7)..(9)
<223>  2'-deoxy-2'-fluoro

<220>
<221>  misc_feature

<222>  (10)..(11)
<223>  2'-deoxy

<220>
<221>  misc_feature
<222>  (12)..(13)
<223>  2'-deoxy-2'-fluoro

<220>
<221>  misc_feature
<222>  (14)..(17)
<223>  2'-deoxy

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  722
ucaagacuca gccaagguun n                                                21


<210>  723
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  5'-3 attached terminal deoxyabasic moeity

<220>
<221>  misc_feature
<222>  (3)..(6)
<223>  2'-deoxy-2'-fluoro

<220>
<221>  misc_feature
<222>  (10)..(11)
<223>  2'-deoxy-2'-fluoro

<220>
<221>  misc_feature
<222>  (14)..(17)
<223>  2'-deoxy-2'-fluoro

<220>
<221>  misc_feature

<222>  (20)..(21)
<223>  n stands for thymidine

<220>
<221>  misc_feature
<222>  (21)..(21)
<223>  3'-3 attached terminal deoxyabasic moeity

<400>  723
aguucugagu cgguuccaan n                                                21


<210>  724
<211>  21
<212>  RNA
<213>  Artificial Sequence

<220>
<223>  Description of Artificial Sequence:  inverted control/siNA antisense


<220>
<221>  misc_feature
<222>  (1)..(2)
<223>  2'-deoxy-2'-fluoro

<220>
<221>  misc_feature
<222>  (7)..(8)
<223>  2'-deoxy-2'-fluoro

<220>
<221>  misc_feature
<222>  (11)..(13)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  724
uuggaaccga cucagaacun n                                                21


<210>  725
<211>  21
<212>  RNA
<213>  Artificial Sequence

<220>
<223>  Description of Artificial Sequence:  inverted control/siNA sense

<220>
<221>  misc_feature
<222>  (1)..(2)
<223>  2'-deoxy

<220>
<221>  misc_feature
<222>  (1)..(1)
<223>  5'-3 attached terminal deoxyabasic moeity

<220>
<221>  misc_feature
<222>  (3)..(6)
<223>  2'-deoxy-2'-fluoro

<220>
<221>  misc_feature
<222>  (7)..(9)
<223>  2'-deoxy

<220>
<221>  misc_feature
<222>  (10)..(11)
<223>  2'-deoxy-2'-fluoro

<220>
<221>  misc_feature
<222>  (12)..(13)
<223>  2'-deoxy

<220>
<221>  misc_feature
<222>  (14)..(17)
<223>  2'-deoxy-2'-fluoro

<220>
<221>  misc_feature
<222>  (18)..(19)
<223>  2'-deoxy

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  725
aguucugagu cgguuccaan n                                                21


<210>  726
<211>  21
<212>  RNA
<213>  Artificial Sequence

<220>
<223>  Description of Artificial Sequence:  inverted control/siNA antisense

<220>
<221>  misc_feature
<222>  (1)..(2)
<223>  2'-deoxy-2'-fluoro

<220>
<221>  misc_feature
<222>  (3)..(6)
<223>  2'-deoxy

<220>
<221>  misc_feature
<222>  (7)..(8)
<223>  2'-deoxy-2'-fluoro

<220>
<221>  misc_feature
<222>  (9)..(10)
<223>  2'-deoxy

<220>
<221>  misc_feature
<222>  (11)..(13)
<223>  2'-deoxy-2'-fluoro

<220>
<221>  misc_feature
<222>  (14)..(17)
<223>  2'-deoxy

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  726
uuggaaccga cucagaacun n                                                21


<210>  727
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(4)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(15)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  727
guucaguguc ucuccaaaan n                                                21


<210>  728
<211>  21
<212>  RNA
<213>  Artificial Sequence

<220>
<223>  Description of Artificial Sequence:  siNA sense region


<220>
<221>  misc_feature
<222>  (1)..(3)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(15)
<223>  2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 728
uuugcagaua gccuugagcn n 21


<210> 729
<211> 21
<212> RNA
<213> Artificial Sequence

<220>
<223> Description of Artificial Sequence: siNA sense region


<220>
<221> misc_feature
<222> (1)..(4)
<223> 2'-deoxy-2'-fluoro

<220>
<221> misc_feature
<222> (1)..(1)
<223> 5'-3 attached terminal deoxyabasic moeity

<220>
<221> misc_feature
<222> (6)..(7)
<223> 2'-deoxy-2'-fluoro

<220>
<221> misc_feature
<222> (9)..(13)
<223> 2'-deoxy-2'-fluoro

<220>
<221> misc_feature
<222> (15)..(16)
<223> 2'-deoxy-2'-fluoro

<220>
<221> misc_feature
<222> (19)..(19)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400>  729
uccugcugcu uucauugacn n                                                21

<210>  730
<211>  21
<212>  RNA
<213>  Artificial Sequence

<220>
<223>  Description of Artificial Sequence:  siNA sense region

<220>
<221>  misc_feature
<222>  (1)..(1)
<223>  5'-3 attached terminal deoxyabasic moeity

<220>
<221>  misc_feature
<222>  (3)..(4)
<223>  2'-deoxy-2'-fluoro

<220>
<221>  misc_feature
<222>  (6)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(15)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  730
gacugccaug uguucaucan n                                                21

<210>  731
<211>  21
<212>  RNA
<213>  Artificial Sequence

<220>
<223>  Description of Artificial Sequence:  siNA antisense region


<220>
<221>  misc_feature
<222>  (1)..(4)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(15)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  731
uuuuggagag acacugaacn n                                                21


<210>  732
<211>  21
<212>  RNA
<213>  Artificial Sequence

<220>
<223>  Description of Artificial Sequence:  siNA antisense region


<220>
<221>  misc_feature
<222>  (2)..(4)
<223>  2'-deoxy-2'-fluoro

<220>
<221>  misc_feature
<222>  (9)..(10)
<223>  2'-deoxy-2'-fluoro

<220>

<221>  misc_feature
<222>  (12)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  732
gcucaaggcu aucugcaaan n                                                21


<210>  733
<211>  21
<212>  RNA
<213>  Artificial Sequence

<220>
<223>  Description of Artificial Sequence:  siNA antisense region


<220>
<221>  misc_feature
<222>  (2)..(3)
<223>  2'-deoxy-2'-fluoro

<220>
<221>  misc_feature
<222>  (6)..(6)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(15)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  733

gucaaugaaa gcagcaggan n                                                21

<210>  734
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (4)..(4)
<223>  2'-deoxy-2'-fluoro

<220>
<221>  misc_feature
<222>  (8)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(15)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  734
ugaugaacac auggcagucn n                                                21


<210>  735
<211>  21

<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  735
cugcaguacc ucuaccugcn n                                                 21


<210>  736
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  736
cgucuccuac ugcaccagan n                                                 21


<210>  737
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  737
uggagccugg aagaccagcn n                                                 21


<210>  738
<211>  21
<212>  RNA
<213>  Artificial Sequence

<220>
<223>  Description of Artificial Sequence:  siNA sense region


<220>
<221>  misc_feature

<222>  (20)..(21)
<223>  n stands for thymidine

<400>  738
gugacucaga aggcucaggn n                                                21


<210>  739
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  739
gcagguagag guacugcagn n                                                21


<210>  740
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  740
ucuggugcag uaggagacgn n                                                21


<210>  741
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  741
gcuggucuuc caggcuccan n                                                21


<210>  742
<211>  21

<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  742
ccugagccuu cugagucacn n                                                21


<210>  743
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(14)

<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  743
ugcacguaua ugccgagaun n                                                21


<210>  744
<211>  21
<212>  RNA
<213>  Artificial Sequence

<220>
<223>  Description of Artificial Sequence:  siNA sense region


<220>
<221>  misc_feature
<222>  (1)..(2)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (6)..(7)
<223>  2'-deoxy-2'-fluoro

<220>
<221>  misc_feature
<222>  (13)..(13)
<223>  2'-deoxy-2'-fluoro

<220>
<221>  misc_feature
<222>  (15)..(16)
<223>  2'-deoxy-2'-fluoro

<220>
<221>  misc_feature
<222>  (18)..(19)

<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  744
ccauauugga gaugcuguun n                                                21


<210>  745
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)

<223>  n stands for thymidine

<220>
<221>  misc_feature
<222>  (21)..(21)
<223>  3'-3 attached terminal deoxyabasic moeity

<400>  745
auugcggaua ugggacacun n                                                21


<210>  746
<211>  21
<212>  RNA
<213>  Artificial Sequence

<220>
<223>  Description of Artificial Sequence:  siNA antisense region


<220>
<221>  misc_feature
<222>  (2)..(5)
<223>  2'-deoxy-2'-fluoro

<220>
<221>  misc_feature
<222>  (8)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)

<223>  Phosphorothioate 3'-Internucleotide Linkage

<400>  746
aucucggcau auacgugcan n                                                21


<210>  747
<211>  21
<212>  RNA
<213>  Artificial Sequence

<220>
<223>  Description of Artificial Sequence:  siNA antisense region


<220>
<221>  misc_feature
<222>  (3)..(3)
<223>  2'-deoxy-2'-fluoro

<220>
<221>  misc_feature
<222>  (6)..(6)
<223>  2'-deoxy-2'-fluoro

<220>
<221>  misc_feature
<222>  (8)..(12)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  747
aacagcaucu ccaauauggn n                                                21


<210>  748
<211>  21
<212>  RNA
<213>  Artificial Sequence

<220>
<223>  Description of Artificial Sequence:  siNA antisense region

<220>
<221>  misc_feature
<222>  (3)..(3)
<223>  2'-deoxy-2'-fluoro

<220>
<221>  misc_feature
<222>  (5)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  748
agugucccau auccgcaaun n                                                21


<210>  749
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  749
ugcacguaua ugccgagaun n                                                21


<210>  750

<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  750
ccauauugga gaugcuguun n                                                 21


<210>  751
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  751
aaagccacuc cacucucuun n                                                 21


<210>  752
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  752
auugcggaua ugggacacun n                                                 21


<210>  753
<211>  21
<212>  RNA
<213>  Artificial Sequence

<220>
<223>  Description of Artificial Sequence:  siNA antisense region


<220>

<221>  misc_feature
<222>  (20)..(21)
<223>  n stands for thymidine

<400>  753
aucucggcau auacgugcan n                                                21


<210>  754
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  754
aacagcaucu ccaauauggn n                                                21


<210>  755
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  755
aagagagugg aguggcuuun n                                                21


<210>  756
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  756
agugucccau auccgcaaun n                                                21


<210>  757

<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (5)..(6)
<223>  2'-deoxy-2'-fluoro

<220>
<221>  misc_feature
<222>  (8)..(11)
<223>  2'-deoxy-2'-fluoro

<220>
<221>  misc_feature
<222>  (13)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  757
aaagccacuc cacucucuun n                                                21


<210>  758
<211>  21
<212>  RNA
<213>  Artificial Sequence

<220>
<223>  Description of Artificial Sequence:  siNA antisense region


<220>
<221>  misc_feature
<222>  (8)..(8)
<223>  2'-deoxy-2'-fluoro

<220>
<221>  misc_feature
<222>  (13)..(13)
<223>  2'-deoxy-2'-fluoro

<220>
<221>  misc_feature

<222>  (16)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  758
aagagagugg aguggcuuun n                                                21


<210>  759
<211>  21
<212>  RNA
<213>  Artificial Sequence

<220>
<223>  Description of Artificial Sequence:  inverted control/siNA sense


<220>
<221>  misc_feature
<222>  (1)..(7)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (9)..(12)
<223>  2'-deoxy-2'-fluoro

<220>
<221>  misc_feature
<222>  (14)..(15)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  759
uucucucacc ucaccgaaan n                                                21


<210>  760
<211>  21
<212>  RNA

<213>  Artificial Sequence

<220>
<223>  Description of Artificial Sequence:  inverted control/siNA antisense


<220>
<221>  misc_feature
<222>  (1)..(4)
<223>  2'-deoxy-2'-fluoro

<220>
<221>  misc_feature
<222>  (7)..(7)
<223>  2'-deoxy-2'-fluoro

<220>
<221>  misc_feature
<222>  (12)..(12)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  760
uuucggugag gugagagaan n                                                21


<210>  761
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  761
caggaccucc acaugauagn n                                                21


<210>  762
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (4)..(6)
<223>  2'-deoxy-2'-fluoro

<220>
<221>  misc_feature
<222>  (9)..(15)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  762
agauuugacc uuccugacan n                                                21


<210>  763
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (5)..(5)
<223>  2'-deoxy-2'-fluoro

<220>
<221>  misc_feature
<222>  (8)..(9)
<223>  2'-deoxy-2'-fluoro

<220>
<221>  misc_feature
<222>  (13)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  763
ugaguagcug gauuacaggn n                                                21


<210>  764
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (3)..(6)
<223>  2'-deoxy-2'-fluoro

<220>
<221>  misc_feature
<222>  (10)..(14)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  764
ggucucaaac uccugaccun n                                                21


<210>  765
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>

<221>  misc_feature
<222>  (9)..(9)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  765
cuaucaugug gagguccugn n                                                21


<210>  766
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(4)
<223>  2'-deoxy-2'-fluoro

<220>
<221>  misc_feature
<222>  (12)..(13)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  766

ugucaggaag gucaaaucun n                                                21

<210>  767
<211>  21
<212>  RNA
<213>  Artificial Sequence

<220>
<223>  Description of Artificial Sequence:  siNA antisense region

<220>
<221>  misc_feature
<222>  (1)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (8)..(10)
<223>  2'-deoxy-2'-fluoro

<220>
<221>  misc_feature
<222>  (13)..(14)
<223>  2'-deoxy-2'-fluoro

<220>
<221>  misc_feature
<222>  (16)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  767
ccuguaaucc agcuacucan n                                                21

<210>  768
<211>  21
<212>  RNA
<213>  Artificial Sequence

<220>
<223>  Description of Artificial Sequence:  siNA antisense region

<220>
<221>  misc_feature
<222>  (4)..(5)

<223>  2'-deoxy-2'-fluoro

<220>
<221>  misc_feature
<222>  (11)..(13)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  768
aggucaggag uuugagaccn n                                                21


<210>  769
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (6)..(7)
<223>  2'-deoxy-2'-fluoro

<220>
<221>  misc_feature
<222>  (12)..(13)
<223>  2'-deoxy-2'-fluoro

<220>
<221>  misc_feature
<222>  (15)..(16)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)

<223>  n stands for thymidine

<220>
<221>  misc_feature
<222>  (21)..(21)
<223>  3'-3 attached terminal deoxyabasic moeity

<400>  769
aaaggcugag guugcugaun n                                                21


<210>  770
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (7)..(12)
<223>  2'-deoxy-2'-fluoro

<220>
<221>  misc_feature
<222>  (15)..(15)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  770
aaacaaccuu ccaacaaccn n                                                21


<210>  771
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (6)..(7)
<223>  2'-deoxy-2'-fluoro

<220>
<221>  misc_feature
<222>  (10)..(10)
<223>  2'-deoxy-2'-fluoro

<220>
<221>  misc_feature
<222>  (13)..(14)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  771
aaggacugau gaccaaacan n                                                21


<210>  772
<211>  21
<212>  RNA
<213>  Artificial Sequence

<220>
<223>  Description of Artificial Sequence:  siNA antisense region


<220>
<221>  misc_feature
<222>  (2)..(3)
<223>  2'-deoxy-2'-fluoro

<220>
<221>  misc_feature
<222>  (6)..(6)
<223>  2'-deoxy-2'-fluoro

<220>
<221>  misc_feature
<222>  (9)..(12)
<223>  2'-deoxy-2'-fluoro

<220>
<221>  misc_feature
<222>  (15)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  772
aucagcaacc ucagccuuun n                                                21


<210>  773
<211>  21
<212>  RNA
<213>  Artificial Sequence

<220>
<223>  Description of Artificial Sequence:  siNA antisense region


<220>
<221>  misc_feature
<222>  (3)..(4)
<223>  2'-deoxy-2'-fluoro

<220>
<221>  misc_feature
<222>  (6)..(7)
<223>  2'-deoxy-2'-fluoro

<220>
<221>  misc_feature
<222>  (14)..(15)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  773
gguuguugga agguuguuun n                                                21

<210>  774
<211>  21
<212>  RNA
<213>  Artificial Sequence

<220>
<223>  Description of Artificial Sequence:  siNA antisense region

<220>
<221>  misc_feature
<222>  (1)..(1)
<223>  2'-deoxy-2'-fluoro

<220>
<221>  misc_feature
<222>  (3)..(5)
<223>  2'-deoxy-2'-fluoro

<220>
<221>  misc_feature
<222>  (8)..(9)
<223>  2'-deoxy-2'-fluoro

<220>
<221>  misc_feature
<222>  (11)..(12)
<223>  2'-deoxy-2'-fluoro

<220>
<221>  misc_feature
<222>  (15)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  774
uguuugguca ucaguccuun n                                                21

<210>  775
<211>  21
<212>  RNA
<213>  Artificial Sequence

<220>
<223>  Description of Artificial Sequence:  siNA sense region

<220>
<221>  misc_feature

<222>  (20)..(21)
<223>  n stands for thymidine

<400>  775
aaaggcugag guugcugaun n                                                21


<210>  776
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  776
aaacaaccuu ccaacaaccn n                                                21


<210>  777
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  777
ggauguggug auucaggaun n                                                21


<210>  778
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  778
aaggacugau gaccaaacan n                                                21


<210>  779
<211>  21

<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  779
aucagcaacc ucagccuuun n                                                21


<210>  780
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  780
gguuguugga agguuguuun n                                                21


<210>  781
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  781
auccugaauc accacauccn n                                                21


<210>  782
<211>  21
<212>  RNA
<213>  Artificial Sequence

<220>
<223>  Description of Artificial Sequence:  siNA antisense region


<220>
<221>  misc_feature

<222>  (20)..(21)
<223>  n stands for thymidine

<400>  782
uguuugguca ucaguccuun n                                                21


<210>  783
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (6)..(6)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(14)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  783
ggauguggug auucaggaun n                                                21


<210>  784
<211>  21
<212>  RNA

<213>  Artificial Sequence

<220>
<223>  Description of Artificial Sequence:  siNA antisense region


<220>
<221>  misc_feature
<222>  (2)..(5)
<223>  2'-deoxy-2'-fluoro

<220>
<221>  misc_feature
<222>  (9)..(10)
<223>  2'-deoxy-2'-fluoro

<220>
<221>  misc_feature
<222>  (12)..(13)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  784
auccugaauc accacauccn n                                                21


<210>  785
<211>  21
<212>  RNA
<213>  Artificial Sequence

<220>
<223>  Description of Artificial Sequence:  siNA sense region


<220>
<221>  misc_feature
<222>  (1)..(3)
<223>  2'-deoxy

<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (5)..(5)
<223>  2'-deoxy

<220>
<221>  misc_feature
<222>  (6)..(6)
<223>  2'-deoxy-2'-fluoro

<220>
<221>  misc_feature
<222>  (7)..(8)
<223>  2'-deoxy

<220>
<221>  misc_feature
<222>  (9)..(9)
<223>  2'-deoxy-2'-fluoro

<220>
<221>  misc_feature
<222>  (10)..(11)
<223>  2'-deoxy

<220>
<221>  misc_feature
<222>  (12)..(14)
<223>  2'-deoxy-2'-fluoro

<220>
<221>  misc_feature
<222>  (15)..(18)
<223>  2'-deoxy

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  785
ggauguggug auucaggaun n                                                21


<210>  786
<211>  21
<212>  RNA

<213>  Artificial Sequence

<220>
<223>  Description of Artificial Sequence:  siNA antisense region


<220>
<221>  misc_feature
<222>  (1)..(1)
<223>  2'-deoxy

<220>
<221>  misc_feature
<222>  (2)..(5)
<223>  2'-deoxy-2'-fluoro

<220>
<221>  misc_feature
<222>  (6)..(8)
<223>  2'-deoxy

<220>
<221>  misc_feature
<222>  (9)..(10)
<223>  2'-deoxy-2'-fluoro

<220>
<221>  misc_feature
<222>  (11)..(11)
<223>  2'-deoxy

<220>
<221>  misc_feature
<222>  (12)..(13)
<223>  2'-deoxy-2'-fluoro

<220>
<221>  misc_feature
<222>  (14)..(14)
<223>  2'-deoxy

<220>
<221>  misc_feature
<222>  (15)..(15)
<223>  2'-deoxy-2'-fluoro

<220>
<221>  misc_feature
<222>  (16)..(16)
<223>  2'-deoxy

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature

<222>  (20)..(20)
<223>  Phosphorothioate 3'-Internucleotide Linkage

<400>  786
auccugaauc accacauccn n                                                21


<210>  787
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(8)
<223>  2'-deoxy-2'-fluoro

<220>
<221>  misc_feature
<222>  (11)..(11)
<223>  2'-deoxy-2'-fluoro

<220>
<221>  misc_feature
<222>  (14)..(14)
<223>  2'-deoxy-2'-fluoro

<220>
<221>  misc_feature
<222>  (16)..(16)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  787
uaggacuuag ugguguaggn n                                                21


<210>  788
<211>  21
<212>  RNA

<213>  Artificial Sequence

<220>
<223>  Description of Artificial Sequence:  inverted control/siNA antisense


<220>
<221>  misc_feature
<222>  (1)..(3)
<223>  2'-deoxy-2'-fluoro

<220>
<221>  misc_feature
<222>  (5)..(5)
<223>  2'-deoxy-2'-fluoro

<220>
<221>  misc_feature
<222>  (7)..(8)
<223>  2'-deoxy-2'-fluoro

<220>
<221>  misc_feature
<222>  (10)..(11)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  788
ccuacaccac uaaguccuan n                                                21


<210>  789
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (2)..(5)
<223>  2'-deoxy

<220>
<221>  misc_feature
<222>  (6)..(8)
<223>  2'-deoxy-2'-fluoro

<220>
<221>  misc_feature
<222>  (9)..(10)
<223>  2'-deoxy

<220>
<221>  misc_feature
<222>  (11)..(11)
<223>  2'-deoxy-2'-fluoro

<220>
<221>  misc_feature
<222>  (12)..(13)
<223>  2'-deoxy

<220>
<221>  misc_feature
<222>  (14)..(14)
<223>  2'-deoxy-2'-fluoro

<220>
<221>  misc_feature
<222>  (15)..(15)
<223>  2'-deoxy

<220>
<221>  misc_feature
<222>  (16)..(16)
<223>  2'-deoxy-2'-fluoro

<220>
<221>  misc_feature
<222>  (17)..(19)
<223>  2'-deoxy

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  789
uaggacuuag ugguguaggn n                                                21


<210>  790
<211>  21
<212>  RNA

<213>  Artificial Sequence

<220>
<223>  Description of Artificial Sequence:  inverted control/siNA antisense


<220>
<221>  misc_feature
<222>  (1)..(3)
<223>  2'-deoxy-2'-fluoro

<220>
<221>  misc_feature
<222>  (4)..(4)
<223>  2'-deoxy

<220>
<221>  misc_feature
<222>  (5)..(5)
<223>  2'-deoxy-2'-fluoro

<220>
<221>  misc_feature
<222>  (6)..(6)
<223>  2'-deoxy

<220>
<221>  misc_feature
<222>  (7)..(8)
<223>  2'-deoxy-2'-fluoro

<220>
<221>  misc_feature
<222>  (9)..(9)
<223>  2'-deoxy

<220>
<221>  misc_feature
<222>  (10)..(11)
<223>  2'-deoxy-2'-fluoro

<220>
<221>  misc_feature
<222>  (12)..(14)
<223>  2'-deoxy

<220>
<221>  misc_feature
<222>  (15)..(18)
<223>  2'-deoxy-2'-fluoro

<220>
<221>  misc_feature
<222>  (19)..(19)
<223>  2'-deoxy

<220>
<221>  misc_feature
<222>  (20)..(21)
<223>  n stands for thymidine

<220>
<221>  misc_feature

<222>  (20)..(20)
<223>  Phosphorothioate 3'-Internucleotide Linkage

<400>  790
ccuacaccac uaaguccuan n                                                21


<210>  791
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  791
cucguuucuc uuggacaagn n                                                21


<210>  792
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  792
ggugagcuac aaacacaugn n                                                21


<210>  793
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  793
aguggaagac uggcugagcn n                                                21


<210>  794
<211>  21

<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  794
ccucuagccu guuuguugun n                                                21


<210>  795
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  795
cuuguccaag agaaacgagn n                                                21


<210>  796
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  796
cauguguuug uagcucaccn n                                                21


<210>  797
<211>  21
<212>  RNA
<213>  Artificial Sequence

<220>
<223>  Description of Artificial Sequence:  siNA antisense region


<220>
<221>  misc_feature

<222>  (20)..(21)
<223>  n stands for thymidine

<400>  797
gcucagccag ucuuccacun n                                                21


<210>  798
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  798
acaacaaaca ggcuagaggn n                                                21


<210>  799
<211>  21
<212>  RNA
<213>  Artificial Sequence

<220>
<223>  Description of Artificial Sequence:  siNA sense region


<220>
<221>  misc_feature
<222>  (1)..(3)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(6)
<223>  2'-deoxy-2'-fluoro

<220>
<221>  misc_feature
<222>  (8)..(8)
<223>  2'-deoxy-2'-fluoro

<220>
<221>  misc_feature
<222>  (13)..(14)
<223>  2'-deoxy-2'-fluoro

<220>
<221>  misc_feature
<222>  (17)..(17)
<223>  2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 799
uccgacauga agccagugan n                                                21


<210> 800
<211> 21
<212> RNA
<213> Artificial Sequence

<220>
<223> Description of Artificial Sequence: siNA sense region


<220>
<221> misc_feature
<222> (1)..(2)
<223> 2'-deoxy-2'-fluoro

<220>
<221> misc_feature
<222> (1)..(1)
<223> 5'-3 attached terminal deoxyabasic moeity

<220>
<221> misc_feature
<222> (5)..(5)
<223> 2'-deoxy-2'-fluoro

<220>
<221> misc_feature
<222> (9)..(9)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 800
cugauggaca agaggaaagn n                                                21


<210> 801
<211> 21
<212> RNA
<213> Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (8)..(8)
<223>  2'-deoxy-2'-fluoro

<220>
<221>  misc_feature
<222>  (13)..(15)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  801
guguggauaa ggcuuaggun n                                                21


<210>  802
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>

<221>  misc_feature
<222>  (4)..(5)
<223>  2'-deoxy-2'-fluoro

<220>
<221>  misc_feature
<222>  (8)..(11)
<223>  2'-deoxy-2'-fluoro

<220>
<221>  misc_feature
<222>  (13)..(13)
<223>  2'-deoxy-2'-fluoro

<220>
<221>  misc_feature
<222>  (15)..(16)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  802
ucacuggcuu caugucggan n                                                21


<210>  803
<211>  21
<212>  RNA
<213>  Artificial Sequence

<220>
<223>  Description of Artificial Sequence:  siNA antisense region


<220>
<221>  misc_feature
<222>  (1)..(10)
<223>  2'-deoxy-2'-fluoro

<220>
<221>  misc_feature
<222>  (12)..(14)
<223>  2'-deoxy-2'-fluoro

<220>
<221>  misc_feature
<222>  (16)..(17)
<223>  2'-deoxy-2'-fluoro

<220>
<221>  misc_feature
<222>  (20)..(21)
<223>  n stands for thymidine

<220>

<221>  misc_feature
<222>  (20)..(20)
<223>  Phosphorothioate 3'-Internucleotide Linkage

<400>  803
cuuuccucuu guccaucagn n                                                21


<210>  804
<211>  21
<212>  RNA
<213>  Artificial Sequence

<220>
<223>  Description of Artificial Sequence:  siNA antisense region


<220>
<221>  misc_feature
<222>  (2)..(4)
<223>  2'-deoxy-2'-fluoro

<220>
<221>  misc_feature
<222>  (8)..(11)
<223>  2'-deoxy-2'-fluoro

<220>
<221>  misc_feature
<222>  (13)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  804
accuaagccu uauccacacn n                                                21


<210>  805
<211>  21
<212>  RNA
<213>  Artificial Sequence

<220>
<223>  Description of Artificial Sequence:  siNA sense region

<220>
<221> misc_feature
<222> (20)..(21)
<223> n stands for thymidine

<400> 805
uccgacauga agccagugan n 21

<210> 806
<211> 21
<212> RNA
<213> Artificial Sequence

<220>
<223> Description of Artificial Sequence: siNA sense region

<220>
<221> misc_feature
<222> (20)..(21)
<223> n stands for thymidine

<400> 806
guccgagagu cagggucacn n 21

<210> 807
<211> 21
<212> RNA
<213> Artificial Sequence

<220>
<223> Description of Artificial Sequence: siNA sense region

<220>
<221> misc_feature
<222> (20)..(21)
<223> n stands for thymidine

<400> 807
cugauggaca agaggaaagn n 21

<210> 808
<211> 21
<212> RNA
<213> Artificial Sequence

<220>
<223> Description of Artificial Sequence: siNA sense region

<220>
<221> misc_feature
<222> (20)..(21)
<223> n stands for thymidine

<400> 808
guguggauaa ggcuuaggun n 21

<210>  809
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  809
ucacuggcuu caugucggan n                                                21


<210>  810
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  810
gugacccuga cucucggacn n                                                21


<210>  811
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  811
cuuuccucuu guccaucagn n                                                21


<210>  812
<211>  21
<212>  RNA
<213>  Artificial Sequence

<220>
<223>  Description of Artificial Sequence:  siNA antisense region

<220>
<221> misc_feature
<222> (20)..(21)
<223> n stands for thymidine

<400> 812
accuaagccu uauccacacn n 21


<210> 813
<211> 21
<212> RNA
<213> Artificial Sequence

<220>
<223> Description of Artificial Sequence: siNA sense region


<220>
<221> misc_feature
<222> (1)..(1)
<223> 5'-3 attached terminal deoxyabasic moeity

<220>
<221> misc_feature
<222> (2)..(4)
<223> 2'-deoxy-2'-fluoro

<220>
<221> misc_feature
<222> (10)..(11)
<223> 2'-deoxy-2'-fluoro

<220>
<221> misc_feature
<222> (16)..(17)
<223> 2'-deoxy-2'-fluoro

<220>
<221> misc_feature
<222> (19)..(19)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 813
guccgagagu cagggucacn n 21


<210> 814
<211> 21
<212> RNA
<213> Artificial Sequence

<220>
<223>  Description of Artificial Sequence:  siNA antisense region


<220>
<221>  misc_feature
<222>  (2)..(2)
<223>  2'-deoxy-2'-fluoro

<220>
<221>  misc_feature
<222>  (5)..(8)
<223>  2'-deoxy-2'-fluoro

<220>
<221>  misc_feature
<222>  (11)..(15)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  814
gugacccuga cucucggacn n                                                21


<210>  815
<211>  21
<212>  RNA
<213>  Artificial Sequence

<220>
<223>  Description of Artificial Sequence:  inverted control/siNA sense


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(4)
<223>  2'-deoxy-2'-fluoro

<220>
<221> misc_feature
<222> (9)..(10)
<223> 2'-deoxy-2'-fluoro

<220>
<221> misc_feature
<222> (16)..(18)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 815
cacugggacu gagagccugn n                                                21


<210> 816
<211> 21
<212> RNA
<213> Artificial Sequence

<220>
<223> Description of Artificial Sequence: inverted control/siNA antisense


<220>
<221> misc_feature
<222> (1)..(1)
<223> 2'-deoxy-2'-fluoro

<220>
<221> misc_feature
<222> (5)..(9)
<223> 2'-deoxy-2'-fluoro

<220>
<221> misc_feature
<222> (12)..(15)
<223> 2'-deoxy-2'-fluoro

<220>
<221> misc_feature
<222> (18)..(18)
<223> 2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400>  816
caggcucuca gucccagugn n                                                21

<210>  817
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  817
aacacggcau gugaacauun n                                                21

<210>  818
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  818
ucuacaaaca ccugcaugun n                                                21

<210>  819
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  819
ucacaucaac aaccgagaun n                                                21

<210>  820
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  820
aggaagccag gaauacaggn n                                                21


<210>  821
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  821
aauguucaca ugccguguun n                                                21


<210>  822
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  822
acaugcaggu guuuguagan n                                                21


<210>  823
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  823
aucucgguug uugaugugan n                                                21


<210>  824
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  824
ccuguauucc uggcuuccun n                                                21


<210>  825
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  825
gaggagcaca gauaccaccn n                                                21


<210>  826
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  826
uggcuucuau gaggcugagn n                                                21


<210>  827
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  827
ugugacaagg ugcagaaagn n                                                21


<210>  828
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  828
cuccagcuuc ugguacucun n                                                21


<210>  829
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  829
ggugguaucu gugcuccucn n                                                21


<210>  830
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  830
cucagccuca uagaagccan n                                                21


<210>  831
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  831
cuuucugcac cuugucacan n                                                21


<210>  832
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  832
agaguaccag aagcuggagn n                                                21


<210>  833
<211>  21
<212>  RNA
<213>  Artificial Sequence

<220>
<223>  Description of Artificial Sequence:  siNA sense region


<220>
<221>  misc_feature
<222>  (1)..(4)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (7)..(11)
<223>  2'-deoxy-2'-fluoro

<220>
<221> misc_feature
<222> (14)..(14)
<223> 2'-deoxy-2'-fluoro

<220>
<221> misc_feature
<222> (16)..(19)
<223> 2'-deoxy-2'-fluoro

<220>
<221> misc_feature
<222> (20)..(21)
<223> n stands for thymidine

<220>
<221> misc_feature
<222> (21)..(21)
<223> 3'-3 attached terminal deoxyabasic moeity

<400> 833
cuccagcuuc ugguacucun n                                                21


<210> 834
<211> 21
<212> RNA
<213> Artificial Sequence

<220>
<223> Description of Artificial Sequence: siNA antisense region


<220>
<221> misc_feature
<222> (5)..(5)
<223> 2'-deoxy-2'-fluoro

<220>
<221> misc_feature
<222> (7)..(8)
<223> 2'-deoxy-2'-fluoro

<220>
<221> misc_feature
<222> (14)..(15)
<223> 2'-deoxy-2'-fluoro

<220>
<221> misc_feature
<222> (20)..(21)
<223> n stands for thymidine

<220>
<221> misc_feature
<222> (20)..(20)
<223> Phosphorothioate 3'-Internucleotide Linkage

<400> 834
agaguaccag aagcuggagn n                                                21

<210>  835
<211>  21
<212>  RNA
<213>  Artificial Sequence

<220>
<223>  Description of Artificial Sequence:  inverted control/siNA sense


<220>
<221>  misc_feature
<222>  (1)..(4)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(6)
<223>  2'-deoxy-2'-fluoro

<220>
<221>  misc_feature
<222>  (9)..(13)
<223>  2'-deoxy-2'-fluoro

<220>
<221>  misc_feature
<222>  (16)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  835
ucucaugguc uucgaccucn n                                                21


<210>  836
<211>  21
<212>  RNA
<213>  Artificial Sequence

<220>
<223>  Description of Artificial Sequence:  inverted control/siNA antisense


<220>
<221>  misc_feature
<222>  (5)..(6)
<223>  2'-deoxy-2'-fluoro

<220>

<221>  misc_feature
<222>  (12)..(13)
<223>  2'-deoxy-2'-fluoro

<220>
<221>  misc_feature
<222>  (15)..(15)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  836
gaggucgaag accaugagan n                                                21


<210>  837
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(3)
<223>  2'-deoxy-2'-fluoro

<220>
<221>  misc_feature
<222>  (5)..(6)
<223>  2'-deoxy-2'-fluoro

<220>
<221>  misc_feature
<222>  (8)..(8)
<223>  2'-deoxy-2'-fluoro

<220>
<221>  misc_feature
<222>  (11)..(11)
<223>  2'-deoxy-2'-fluoro

<220>

<221>  misc_feature
<222>  (13)..(15)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  837
uaugcugugg ugcuuaaugn n                                                21


<210>  838
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(4)
<223>  2'-deoxy-2'-fluoro

<220>
<221>  misc_feature
<222>  (8)..(8)
<223>  2'-deoxy-2'-fluoro

<220>
<221>  misc_feature
<222>  (10)..(10)
<223>  2'-deoxy-2'-fluoro

<220>
<221>  misc_feature
<222>  (15)..(15)
<223>  2'-deoxy-2'-fluoro

<220>

<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  838
ugcuggacau gagauggagn n                                                21


<210>  839
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (5)..(6)
<223>  2'-deoxy-2'-fluoro

<220>
<221>  misc_feature
<222>  (8)..(8)
<223>  2'-deoxy-2'-fluoro

<220>
<221>  misc_feature
<222>  (14)..(15)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  839
gaggcuacag ggguuagccn n                                                21


<210>  840
<211>  21

<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (3)..(5)
<223>  2'-deoxy-2'-fluoro

<220>
<221>  misc_feature
<222>  (7)..(10)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  840
gaccuaccuc aaagggcagn n                                                21


<210>  841
<211>  21
<212>  RNA
<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(4)
<223>  2'-deoxy-2'-fluoro

<220>
<221>  misc_feature
<222>  (8)..(8)

<223>  2'-deoxy-2'-fluoro

<220>
<221>  misc_feature
<222>  (10)..(11)
<223>  2'-deoxy-2'-fluoro

<220>
<221>  misc_feature
<222>  (13)..(13)
<223>  2'-deoxy-2'-fluoro

<220>
<221>  misc_feature
<222>  (16)..(16)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  841
cauuaagcac cacagcauan n                                                21


<210>  842
<211>  21
<212>  RNA
<213>  Artificial Sequence

<220>
<223>  Description of Artificial Sequence:  siNA antisense region


<220>
<221>  misc_feature
<222>  (1)..(4)
<223>  2'-deoxy-2'-fluoro

<220>
<221>  misc_feature
<222>  (6)..(9)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(15)

<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  842
cuccaucuca uguccagcan n                                                21


<210>  843
<211>  21
<212>  RNA
<213>  Artificial Sequence

<220>
<223>  Description of Artificial Sequence:  siNA antisense region


<220>
<221>  misc_feature
<222>  (3)..(4)
<223>  2'-deoxy-2'-fluoro

<220>
<221>  misc_feature
<222>  (7)..(11)
<223>  2'-deoxy-2'-fluoro

<220>
<221>  misc_feature
<222>  (13)..(13)
<223>  2'-deoxy-2'-fluoro

<220>
<221>  misc_feature
<222>  (16)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  843
ggcuaacccc uguagccucn n                                                21

<210>  844
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(9)
<223>  2'-deoxy-2'-fluoro

<220>
<221>  misc_feature
<222>  (14)..(14)
<223>  2'-deoxy-2'-fluoro

<220>
<221>  misc_feature
<222>  (18)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  844
cugcccuuug agguaggucn n                                                21


<210>  845
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  845
uaugcugugg ugcuuaaugn n                                                21


<210>  846

<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  846
ugcuggacau gagauggagn n                                                21


<210>  847
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  847
gaggcuacag ggguuagccn n                                                21


<210>  848
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  848
gaccuaccuc aaagggcagn n                                                21


<210>  849
<211>  21
<212>  RNA
<213>  Artificial Sequence

<220>
<223>  Description of Artificial Sequence:  siNA antisense region


<220>

<221>  misc_feature
<222>  (20)..(21)
<223>  n stands for thymidine

<400>  849
cauuaagcac cacagcauan n                                                21


<210>  850
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  850
cuccaucuca uguccagcan n                                                21


<210>  851
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  851
ggcuaacccc uguagccucn n                                                21


<210>  852
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  852
cugcccuuug agguaggucn n                                                21


<210>  853

<211>  21
<212>  RNA
<213>  Artificial Sequence

<220>
<223>  Description of Artificial Sequence:  siNA sense region

<400>  853
gcgcacgugg gaagcccugg c                                                21


<210>  854
<211>  21
<212>  RNA
<213>  Artificial Sequence

<220>
<223>  Description of Artificial Sequence:  siNA sense region

<400>  854
cagaggcugu gcgagcgcgg c                                                21


<210>  855
<211>  21
<212>  RNA
<213>  Artificial Sequence

<220>
<223>  Description of Artificial Sequence:  siNA sense region

<400>  855
ucugggaugc gaacgggccu g                                                21


<210>  856
<211>  21
<212>  RNA
<213>  Artificial Sequence

<220>
<223>  Description of Artificial Sequence:  siNA sense region

<400>  856
ugggaaccac gcgcagugcc c                                                21


<210>  857
<211>  21
<212>  RNA
<213>  Artificial Sequence

<220>
<223>  Description of Artificial Sequence:  siNA sense region

<400>  857
ccaccacgcc gugcgcauca g                                                21


<210>  858
<211>  21
<212>  RNA
<213>  Artificial Sequence

<220>
<223>  Description of Artificial Sequence:  siNA antisense region

<400>  858
cagggcuucc cacgugcgca g                                                21


<210>  859
<211>  21
<212>  RNA
<213>  Artificial Sequence

<220>
<223>  Description of Artificial Sequence:  siNA antisense region

<400>  859
cgcgcucgca cagccucugc a                                                21


<210>  860
<211>  21
<212>  RNA
<213>  Artificial Sequence

<220>
<223>  Description of Artificial Sequence:  siNA antisense region

<400>  860
ggcccguucg caucccagac g                                                21


<210>  861
<211>  21
<212>  RNA
<213>  Artificial Sequence

<220>
<223>  Description of Artificial Sequence:  siNA antisense region

<400>  861
gcacugcgcg ugguucccaa g                                                21


<210>  862
<211>  21
<212>  RNA
<213>  Artificial Sequence

<220>
<223>  Description of Artificial Sequence:  siNA antisense region

<400>  862
gaugcgcacg gcgugguggc a                                                21


<210>  863
<211>  21
<212>  RNA
<213>  Artificial Sequence

<220>
<223>  Description of Artificial Sequence:  siNA sense region

<220>
<221>  misc_feature
<222>  (1)..(1)
<223>  5'-3 attached terminal deoxyabasic moeity

<220>
<221>  misc_feature
<222>  (6)..(7)
<223>  2'-deoxy-2'-fluoro

<220>
<221>  misc_feature
<222>  (9)..(15)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  863
ggagaccauc uuucugggun n                                                21


<210>  864
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (3)..(5)
<223>  2'-deoxy-2'-fluoro

<220>
<221>  misc_feature
<222>  (10)..(10)

<223>  2'-deoxy-2'-fluoro

<220>
<221>  misc_feature
<222>  (14)..(15)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  864
ugucuggagc aaguugcaan n                                                21


<210>  865
<211>  21
<212>  RNA
<213>  Artificial Sequence

<220>
<223>  Description of Artificial Sequence:  siNA sense region


<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (6)..(7)
<223>  2'-deoxy-2'-fluoro

<220>
<221>  misc_feature
<222>  (10)..(13)
<223>  2'-deoxy-2'-fluoro

<220>
<221>  misc_feature
<222>  (15)..(19)
<223>  2'-deoxy-2'-fluoro

<220>
<221>  misc_feature
<222>  (20)..(21)

<223>  n stands for thymidine

<220>
<221>  misc_feature
<222>  (21)..(21)
<223>  3'-3 attached terminal deoxyabasic moeity

<400>  865
cagagccagu cucaccuucn n                                                21


<210>  866
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (6)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (12)..(14)
<223>  2'-deoxy-2'-fluoro

<220>
<221>  misc_feature
<222>  (16)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  866
aagugucaca gccuguuucn n                                                21

<210>  867
<211>  21
<212>  RNA
<213>  Artificial Sequence

<220>
<223>  Description of Artificial Sequence:  siNA antisense region


<220>
<221>  misc_feature
<222>  (2)..(4)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  867
acccagaaag auggucuccn n                                                21


<210>  868
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (4)..(4)
<223>  2'-deoxy-2'-fluoro

<220>
<221>  misc_feature
<222>  (7)..(9)
<223>  2'-deoxy-2'-fluoro

<220>
<221>  misc_feature
<222>  (11)..(14)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  868
uugcaacuug cuccagacan n                                                21


<210>  869
<211>  21
<212>  RNA
<213>  Artificial Sequence

<220>
<223>  Description of Artificial Sequence:  siNA antisense region


<220>
<221>  misc_feature
<222>  (6)..(6)
<223>  2'-deoxy-2'-fluoro

<220>
<221>  misc_feature
<222>  (11)..(12)
<223>  2'-deoxy-2'-fluoro

<220>
<221>  misc_feature
<222>  (15)..(18)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  869
gaaggugaga cuggcucugn n                                                21


<210>  870

<211>  21
<212>  RNA
<213>  Artificial Sequence

<220>
<223>  Description of Artificial Sequence:  siNA antisense region


<220>
<221>  misc_feature
<222>  (5)..(5)
<223>  2'-deoxy-2'-fluoro

<220>
<221>  misc_feature
<222>  (9)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  870
gaaacaggcu gugacacuun n                                                21


<210>  871
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature

<222>  (4)..(4)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (11)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  871
ggauaacaca cugcaagugn n                                                21


<210>  872
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature

<222>  (5)..(5)
<223>  2'-deoxy-2'-fluoro

<220>
<221>  misc_feature
<222>  (8)..(8)
<223>  2'-deoxy-2'-fluoro

<220>
<221>  misc_feature
<222>  (10)..(13)
<223>  2'-deoxy-2'-fluoro

<220>
<221>  misc_feature
<222>  (18)..(18)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  872
auagcaacac ucugagaugn n                                                21


<210>  873
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(4)
<223>  2'-deoxy-2'-fluoro

<220>
<221>  misc_feature
<222>  (6)..(9)
<223>  2'-deoxy-2'-fluoro

<220>
<221>  misc_feature
<222>  (12)..(12)
<223>  2'-deoxy-2'-fluoro

<220>
<221>  misc_feature

<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  873
accugcuuua gugggggaun n                                                21


<210>  874
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(2)
<223>  2'-deoxy-2'-fluoro

<220>
<221>  misc_feature
<222>  (4)..(8)
<223>  2'-deoxy-2'-fluoro

<220>
<221>  misc_feature
<222>  (15)..(15)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  874
gcacuuuugg gaggcagagn n                                                21


<210>  875
<211>  21
<212>  RNA

<213>  Artificial Sequence

<220>
<223>  Description of Artificial Sequence:  siNA antisense region


<220>
<221>  misc_feature
<222>  (1)..(1)
<223>  2'-deoxy-2'-fluoro

<220>
<221>  misc_feature
<222>  (3)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (10)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(15)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  875
cacuugcagu guguuauccn n                                                21


<210>  876
<211>  21
<212>  RNA
<213>  Artificial Sequence

<220>
<223>  Description of Artificial Sequence:  siNA antisense region

<220>
<221>  misc_feature
<222>  (1)..(1)
<223>  2'-deoxy-2'-fluoro

<220>
<221>  misc_feature
<222>  (3)..(6)
<223>  2'-deoxy-2'-fluoro

<220>
<221>  misc_feature
<222>  (11)..(11)
<223>  2'-deoxy-2'-fluoro

<220>
<221>  misc_feature
<222>  (13)..(14)
<223>  2'-deoxy-2'-fluoro

<220>
<221>  misc_feature
<222>  (16)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  876
caucucagag uguugcuaun n                                                21


<210>  877
<211>  21
<212>  RNA
<213>  Artificial Sequence

<220>
<223>  Description of Artificial Sequence:  siNA antisense region


<220>
<221>  misc_feature
<222>  (2)..(7)
<223>  2'-deoxy-2'-fluoro

<220>
<221>  misc_feature
<222>  (9)..(10)
<223>  2'-deoxy-2'-fluoro

<220>
<221>  misc_feature
<222>  (15)..(15)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  877
aucccccacu aaagcaggun n                                                21


<210>  878
<211>  21
<212>  RNA
<213>  Artificial Sequence

<220>
<223>  Description of Artificial Sequence:  siNA antisense region


<220>
<221>  misc_feature
<222>  (1)..(4)
<223>  2'-deoxy-2'-fluoro

<220>
<221>  misc_feature
<222>  (6)..(11)
<223>  2'-deoxy-2'-fluoro

<220>
<221>  misc_feature
<222>  (17)..(17)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  878
cucugccucc caaaagugcn n                                                21

<210>  879
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  879
ggauaacaca cugcaagugn n                                                21

<210>  880
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  880
auagcaacac ucugagaugn n                                                21

<210>  881
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  881
accugcuuua gugggggaun n                                                21

<210>  882
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  882
gcacuuuugg gaggcagagn n                                                21


<210>  883
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  883
cacuugcagu guguuauccn n                                                21


<210>  884
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  884
caucucagag uguugcuaun n                                                21


<210>  885
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  885
aucccccacu aaagcaggun n                                                21


<210>  886
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  886
cucugccucc caaaagugcn n                                                21


<210>  887
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (4)..(4)
<223>  2'-deoxy-2'-fluoro

<220>
<221>  misc_feature
<222>  (6)..(7)
<223>  2'-deoxy-2'-fluoro

<220>
<221>  misc_feature
<222>  (9)..(9)
<223>  2'-deoxy-2'-fluoro

<220>
<221>  misc_feature
<222>  (13)..(13)
<223>  2'-deoxy-2'-fluoro

<220>
<221>  misc_feature
<222>  (15)..(16)
<223>  2'-deoxy-2'-fluoro

<220>

<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  887
ggacaccaug agcacugaan n                                                21


<210>  888
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (2)..(7)
<223>  2'-deoxy-2'-fluoro

<220>
<221>  misc_feature
<222>  (10)..(19)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  888
guuccucagc cucuucuccn n                                                21


<210>  889
<211>  21
<212>  RNA
<213>  Artificial Sequence

<220>
<223>  Description of Artificial Sequence:  siNA sense region


<220>
<221>  misc_feature
<222>  (1)..(3)

<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (5)..(6)
<223>  2'-deoxy-2'-fluoro

<220>
<221>  misc_feature
<222>  (10)..(11)
<223>  2'-deoxy-2'-fluoro

<220>
<221>  misc_feature
<222>  (16)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  889
ccuaccagac caaggucaan n                                                21


<210>  890
<211>  21
<212>  RNA
<213>  Artificial Sequence

<220>
<223>  Description of Artificial Sequence:  siNA sense region


<220>
<221>  misc_feature
<222>  (1)..(1)
<223>  5'-3 attached terminal deoxyabasic moeity

<220>
<221>  misc_feature
<222>  (4)..(16)
<223>  2'-deoxy-2'-fluoro

<220>
<221>  misc_feature
<222>  (20)..(21)
<223>  n stands for thymidine

<220>
<221>  misc_feature
<222>  (21)..(21)

<223>  3'-3 attached terminal deoxyabasic moeity

<400>  890
aggccuuccu cucuccagan n                                                21


<210>  891
<211>  21
<212>  RNA
<213>  Artificial Sequence

<220>
<223>  Description of Artificial Sequence:  siNA antisense region


<220>
<221>  misc_feature
<222>  (1)..(3)
<223>  2'-deoxy-2'-fluoro

<220>
<221>  misc_feature
<222>  (6)..(6)
<223>  2'-deoxy-2'-fluoro

<220>
<221>  misc_feature
<222>  (8)..(10)
<223>  2'-deoxy-2'-fluoro

<220>
<221>  misc_feature
<222>  (12)..(12)
<223>  2'-deoxy-2'-fluoro

<220>
<221>  misc_feature
<222>  (15)..(15)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  891
uucagugcuc augguguccn n                                                21


<210>  892
<211>  21
<212>  RNA
<213>  Artificial Sequence

<220>
<223>  Description of Artificial Sequence:  siNA antisense region


<220>
<221>  misc_feature
<222>  (11)..(12)
<223>  2'-deoxy-2'-fluoro

<220>
<221>  misc_feature
<222>  (19)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  892
ggagaagagg cugaggaacn n                                                21


<210>  893
<211>  21
<212>  RNA
<213>  Artificial Sequence

<220>
<223>  Description of Artificial Sequence:  siNA antisense region


<220>
<221>  misc_feature
<222>  (1)..(2)
<223>  2'-deoxy-2'-fluoro

<220>
<221>  misc_feature
<222>  (5)..(8)
<223>  2'-deoxy-2'-fluoro

<220>
<221>  misc_feature
<222>  (11)..(13)
<223>  2'-deoxy-2'-fluoro

<220>
<221>  misc_feature
<222>  (16)..(16)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  893
uugaccuugg ucugguaggn n                                                21


<210>  894
<211>  21
<212>  RNA
<213>  Artificial Sequence

<220>
<223>  Description of Artificial Sequence:  siNA antisense region


<220>
<221>  misc_feature
<222>  (1)..(3)
<223>  2'-deoxy-2'-fluoro

<220>
<221>  misc_feature
<222>  (17)..(19)
<223>  2'-deoxy-2'-fluoro

<220>
<221>  misc_feature
<222>  (20)..(21)
<223>  n stands for thymidine

<220>
<221>  misc_feature
<222>  (20)..(20)
<223>  Phosphorothioate 3'-Internucleotide Linkage

<400>  894
ucuggagaga ggaaggccun n                                                21


<210>  895
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  895
ggacaccaug agcacugaan n                                                21


<210>  896
<211>  21
<212>  RNA

<213>  Artificial Sequence

<220>
<223>  Description of Artificial Sequence:  siNA sense region


<220>
<221>  misc_feature
<222>  (20)..(21)
<223>  n stands for thymidine

<400>  896
guuccucagc cucuucuccn n                                                21


<210>  897
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  897
ccuaccagac caaggucaan n                                                21


<210>  898
<211>  21
<212>  RNA
<213>  Artificial Sequence

<220>
<223>  Description of Artificial Sequence:  siNA sense region


<220>
<221>  misc_feature
<222>  (20)..(21)
<223>  n stands for thymidine

<400>  898
aggccuuccu cucuccagan n                                                21


<210>  899
<211>  21
<212>  RNA
<213>  Artificial Sequence

<220>
<223>  Description of Artificial Sequence:  siNA antisense region


<220>
<221>  misc_feature
<222>  (20)..(21)

<223>  n stands for thymidine

<400>  899
uucagugcuc augguguccn n                                                21


<210>  900
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  900
ggagaagagg cugaggaacn n                                                21


<210>  901
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  901
uugaccuugg ucugguaggn n                                                21


<210>  902
<211>  21
<212>  RNA
<213>  Artificial Sequence

<220>
<223>  Description of Artificial Sequence:  siNA antisense region


<220>
<221>  misc_feature
<222>  (20)..(21)
<223>  n stands for thymidine

<400>  902
ucuggagaga ggaaggccun n                                                21


<210>  903
<211>  21
<212>  RNA

<213>  Artificial Sequence

<220>
<223>  Description of Artificial Sequence:  siNA sense region

<220>
<221>  misc_feature
<222>  (1)..(4)
<223>  Phosphorothioate 3'-Internucleotide Linkage

<220>
<221>  misc_feature
<222>  (17)..(20)
<223>  Phosphorothioate 3'-Internucleotide Linkage

<220>
<221>  misc_feature
<222>  (1)..(19)
<223>  n stands for any nucleotide wherein any pyrimidine nucleotide present is
2'-O-methyl or 2'-Fluoro

<220>
<221>  misc_feature
<222>  (20)..(21)
<223>  n stands for any nucleotide

<400>  903
nnnnnnnnnn nnnnnnnnnn n                                                21


<210>  904
<211>  21
<212>  RNA
<213>  Artificial Sequence

<220>
<223>  Description of Artificial Sequence:  siNA antisense region

<220>
<221>  misc_feature
<222>  (1)..(4)
<223>  Phosphorothioate 3'-Internucleotide Linkage

<220>
<221>  misc_feature
<222>  (20)..(20)
<223>  Phosphorothioate 3'-Internucleotide Linkage

<220>
<221>  misc_feature
<222>  (1)..(19)
<223>  n stands for any nucleotide wherein any pyrimidine nucleotide present is
2'-Fluoro

<220>
<221>  misc_feature
<222>  (20)..(21)
<223>  n stands for any nucleotide

<220>
<221>  misc_feature
<222>  (21)..(21)
<223>  3'-3 attached terminal glyceryl moeity

<400>  904
nnnnnnnnnn nnnnnnnnnn n                                                21


<210>  905
<211>  21
<212>  RNA
<213>  Artificial Sequence

<220>
<223>  Description of Artificial Sequence:  siNA sense region

<220>
<221>  misc_feature
<222>  (1)..(19)
<223>  n stands for any nucleotide wherein any pyrimidine nucleotide present is
2'-O-methyl or 2'-Fluoro

<220>
<221>  misc_feature
<222>  (20)..(21)
<223>  n stands for any nucleotide

<400>  905
nnnnnnnnnn nnnnnnnnnn n                                                21


<210>  906
<211>  21
<212>  RNA
<213>  Artificial Sequence

<220>
<223>  Description of Artificial Sequence:  siNA antisense region

<220>
<221>  misc_feature
<222>  (1)..(19)
<223>  n stands for any nucleotide wherein any pyrimidine nucleotide present is
2'-Fluoro

<220>
<221>  misc_feature
<222>  (20)..(21)
<223>  n stands for any nucleotide

<220>
<221>  misc_feature
<222>  (21)..(21)
<223>  3'-3 attached terminal glyceryl moeity

<400>  906
nnnnnnnnnn nnnnnnnnnn n                                                21


<210>  907
<211>  21
<212>  RNA
<213>  Artificial Sequence

<220>
<223>  Description of Artificial Sequence:  siNA sense region

<220>
<221>  misc_feature
<222>  (1)..(19)
<223>  n stands for any nucleotide wherein any pyrimidine nucleotide present is
2'-O-methyl or 2'-Fluoro

<220>
<221>  misc_feature
<222>  (20)..(21)
<223>  n stands for any nucleotide

<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  907
nnnnnnnnnn nnnnnnnnnn n                                                21


<210>  908
<211>  21
<212>  RNA
<213>  Artificial Sequence

<220>
<223>  Description of Artificial Sequence:  siNA antisense region

<220>
<221>  misc_feature
<222>  (1)..(19)
<223>  n stands for any nucleotide wherein any pyrimidine nucleotide present is
2'-Fluoro

<220>
<221>  misc_feature
<222>  (20)..(20)
<223>  Phosphorothioate 3'-Internucleotide Linkage

<220>
<221>  misc_feature
<222>  (20)..(21)
<223>  n stands for any nucleotide

<220>
<221>  misc_feature
<222>  (21)..(21)
<223>  3'-3 attached terminal glyceryl moeity

<400>  908
nnnnnnnnnn nnnnnnnnnn n                                                21


<210>  909
<211>  21
<212>  RNA
<213>  Artificial Sequence

<220>
<223>  Description of Artificial Sequence:  siNA sense region

<220>
<221>  misc_feature
<222>  (1)..(19)
<223>  n stands for any nucleotide wherein any pyrimidine nucleotide present is
2'-Fluoro
and any purine nucleotide present is 2'-Deoxy

<220>
<221>  misc_feature
<222>  (20)..(21)
<223>  n stands for any nucleotide

<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  909
nnnnnnnnnn nnnnnnnnnn n                                                    21


<210>  910
<211>  21
<212>  RNA
<213>  Artificial Sequence

<220>
<223>  Description of Artificial Sequence:  siNA antisense region

<220>
<221>  misc_feature
<222>  (1)..(19)
<223>  n stands for any nucleotide wherein any pyrimidine nucleotide present is
2'-Fluoro
and any purine nucleotide present is 2'-O-methyl

<220>
<221>  misc_feature
<222>  (20)..(20)
<223>  Phosphorothioate 3'-Internucleotide Linkage

<220>
<221>  misc_feature
<222>  (20)..(21)
<223>  n stands for any nucleotide

<220>
<221>  misc_feature
<222>  (21)..(21)
<223>  3'-3 attached terminal glyceryl moeity

<400>  910
nnnnnnnnnn nnnnnnnnnn n                                                    21

<210>  911
<211>  21
<212>  RNA
<213>  Artificial Sequence

<220>
<223>  Description of Artificial Sequence:  siNA sense region

<220>
<221>  misc_feature
<222>  (1)..(19)
<223>  n stands for any nucleotide wherein any pyrimidine nucleotide present is
2'-Fluoro

<220>
<221>  misc_feature
<222>  (20)..(21)
<223>  n stands for any nucleotide

<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<400>  911
nnnnnnnnnn nnnnnnnnnn n                                                21


<210>  912
<211>  21
<212>  RNA
<213>  Artificial Sequence

<220>
<223>  Description of Artificial Sequence:  siNA antisense region

<220>
<221>  misc_feature
<222>  (1)..(19)
<223>  n stands for any nucleotide wherein any pyrimidine nucleotide present is
2'-Fluoro
and any purine nucleotide present is 2'-O-methyl

<220>
<221>  misc_feature
<222>  (20)..(21)
<223>  n stands for any nucleotide

<220>
<221>  misc_feature
<222>  (21)..(21)
<223>  3'-3 attached terminal glyceryl moeity

<400>  912
nnnnnnnnnn nnnnnnnnnn n                                                21

<210>  913
<211>  21
<212>  RNA
<213>  Artificial Sequence

<220>
<223>  Description of Artificial Sequence:  siNA antisense region

<220>
<221>  misc_feature
<222>  (1)..(19)
<223>  n stands for any nucleotide wherein any pyrimidine nucleotide present is
2'-Fluoro
and any purine nucleotide present is 2'-Deoxy

<220>
<221>  misc_feature
<222>  (20)..(20)
<223>  Phosphorothioate 3'-Internucleotide Linkage

<220>
<221>  misc_feature
<222>  (20)..(21)
<223>  n stands for any nucleotide

<220>
<221>  misc_feature
<222>  (21)..(21)
<223>  3'-3 attached terminal glyceryl moeity

<400>  913
nnnnnnnnnn nnnnnnnnnn n                                                21


<210>  914
<211>  21
<212>  RNA
<213>  Artificial Sequence

<220>
<223>  Description of Artificial Sequence:  siNA sense region

<220>

<221>  misc_feature

<222>  (1)..(4)

<223>  Phosphorothioate 3'-Internucleotide Linkage

<220>

<221>  misc_feature

<222>  (17)..(20)

<223>  Phosphorothioate 3'-Internucleotide Linkage

<220>

<221>  misc_feature

<222>  (1)..(3)

<223>  2'-O-Methyl or 2'-deoxy-2'-fluoro

<220>

<221>  misc_feature

<222>  (5)..(10)

<223>  2'-O-Methyl or 2'-deoxy-2'-fluoro

<220>

<221>  misc_feature

<222>  (12)..(15)

<223>  2'-O-Methyl or 2'-deoxy-2'-fluoro

<220>

<221>  misc_feature

<222>  (17)..(18)

<223>  2'-O-Methyl or 2'-deoxy-2'-fluoro

<220>

<221>  misc_feature

<222>  (20)..(21)

<223>  n stands for thymidine

<400>  914
uuugucuucc auuccauugn n                                                21


<210>  915
<211>  21
<212>  RNA
<213>  Artificial Sequence

<220>
<223>  Description of Artificial Sequence:  siNA antisense region

<220>

<221>  misc_feature

<222>  (1)..(4)

<223>  Phosphorothioate 3'-Internucleotide Linkage

<220>

<221>  misc_feature

<222>  (20)..(20)

<223>  Phosphorothioate 3'-Internucleotide Linkage

<220>

<221>  misc_feature

<222>  (1)..(1)

<223>  2'-O-Methyl or 2'-deoxy-2'-fluoro

<220>

<221>  misc_feature

<222>  (4)..(4)

<223>  2'-O-Methyl or 2'-deoxy-2'-fluoro

<220>

<221>  misc_feature

<222>  (9)..(9)

<223>  2'-O-Methyl or 2'-deoxy-2'-fluoro

<220>

<221>  misc_feature

<222>  (16)..(16)

<223>  2'-O-Methyl or 2'-deoxy-2'-fluoro

<220>

<221>  misc_feature

<222>  (20)..(21)

<223>  n stands for thymidine

<220>
<221>  misc_feature
<222>  (21)..(21)
<223>  3'-3 attached terminal glyceryl moeity

<400>  915
caauggaaug gaagacaaan n                                                21


<210>  916
<211>  21
<212>  RNA
<213>  Artificial Sequence

<220>
<223>  Description of Artificial Sequence:  siNA sense region

<220>

<221>  misc_feature

<222>  (1)..(3)

<223>  2'-O-Methyl or 2'-deoxy-2'-fluoro

<220>

<221>  misc_feature

<222>  (5)..(10)

<223>  2'-O-Methyl or 2'-deoxy-2'-fluoro

<220>

<221>  misc_feature

<222>  (12)..(15)

<223>  2'-O-Methyl or 2'-deoxy-2'-fluoro

<220>

<221>  misc_feature

<222>  (17)..(18)

<223>  2'-O-Methyl or 2'-deoxy-2'-fluoro

<220>

<221>  misc_feature

<222>  (20)..(21)

<223>  n stands for thymidine

<400>  916
uuugucuucc auuccauugn n                                                21


<210>  917
<211>  21
<212>  RNA
<213>  Artificial Sequence

<220>
<223>  Description of Artificial Sequence:  siNA antisense region

<220>

<221>  misc_feature

<222>  (1)..(1)

<223>  2'-O-Methyl or 2'-deoxy-2'-fluoro

<220>

<221>  misc_feature

<222>  (4)..(4)

<223>  2'-O-Methyl or 2'-deoxy-2'-fluoro

<220>

<221>  misc_feature

<222>  (9)..(9)

<223>  2'-O-Methyl or 2'-deoxy-2'-fluoro

<220>

<221>  misc_feature

<222>  (16)..(16)

<223>  2'-O-Methyl or 2'-deoxy-2'-fluoro

<220>

<221>  misc_feature

<222>  (20)..(21)

<223>  n stands for thymidine

<220>
<221>  misc_feature
<222>  (21)..(21)
<223>  3'-3 attached terminal glyceryl moeity

<400>  917
caauggaaug gaagacaaan n                                                21


<210>  918
<211>  21
<212>  RNA
<213>  Artificial Sequence

<220>
<223>  Description of Artificial Sequence:  siNA sense region

<220>

<221>  misc_feature

<222>  (1)..(3)

<223>  2'-O-Methyl or 2'-deoxy-2'-fluoro

<220>

<221>  misc_feature

<222>  (5)..(10)

<223>  2'-O-Methyl or 2'-deoxy-2'-fluoro

<220>

<221>  misc_feature

<222>  (12)..(15)

<223>  2'-O-Methyl or 2'-deoxy-2'-fluoro

<220>

<221>  misc_feature

<222>  (17)..(18)

<223>  2'-O-Methyl or 2'-deoxy-2'-fluoro

<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<220>

<221>  misc_feature

<222>  (20)..(21)

<223>  n stands for thymidine

<400>  918
uuugucuucc auuccauugn n                                                21


<210>  919
<211>  21
<212>  RNA
<213>  Artificial Sequence

<220>
<223>  Description of Artificial Sequence:  siNA antisense region

<220>

<221>  misc_feature

<222>  (1)..(1)

<223>  2'-O-Methyl or 2'-deoxy-2'-fluoro

<220>

<221>  misc_feature

<222>  (4)..(4)

<223>  2'-O-Methyl or 2'-deoxy-2'-fluoro

<220>

<221>  misc_feature

<222>  (9)..(9)

<223>  2'-O-Methyl or 2'-deoxy-2'-fluoro

<220>

<221>  misc_feature

<222>  (16)..(16)

<223>  2'-O-Methyl or 2'-deoxy-2'-fluoro

<220>

<221>  misc_feature

<222>  (20)..(20)

<223>  Phosphorothioate 3'-Internucleotide Linkage

<220>

<221>  misc_feature

<222>  (20)..(21)

<223>  n stands for thymidine

<220>
<221>  misc_feature
<222>  (21)..(21)
<223>  3'-3 attached terminal glyceryl moeity

<400>  919
caauggaaug gaagacaaan n                                                21


<210>  920
<211>  21
<212>  RNA
<213>  Artificial Sequence

<220>
<223>  Description of Artificial Sequence:  siNA sense region

<220>

<221>  misc_feature

<222>  (4)..(4)

<223>  2'-deoxy

<220>

<221>  misc_feature

<222>  (11)..(11)

<223>  2'-deoxy

<220>

<221>  misc_feature

<222>  (16)..(16)

<223>  2'-deoxy

<220>

<221>  misc_feature

<222>  (19)..(19)

<223>  2'-deoxy

<220>

<221>  misc_feature

<222>  (1)..(3)

<223>  2'-deoxy-2'-fluoro

<220>

<221>  misc_feature

<222>  (5)..(10)

<223>  2'-deoxy-2'-fluoro

<220>

<221>  misc_feature

<222>  (12)..(15)

<223>  2'-deoxy-2'-fluoro

<220>

<221>  misc_feature

<222>  (17)..(18)

<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<220>

<221>  misc_feature

<222>  (20)..(21)

<223>  n stands for thymidine

<400>  920
uuugucuucc auuccauugn n                                                    21

<210> 921
<211> 21
<212> RNA
<213> Artificial Sequence

<220>
<223> Description of Artificial Sequence: siNA antisense region

<220>

<221> misc_feature

<222> (2)..(3)

<223> 2'-O-methyl

<220>

<221> misc_feature

<222> (5)..(8)

<223> 2'-O-methyl

<220>

<221> misc_feature

<222> (10)..(15)

<223> 2'-O-methyl

<220>

<221> misc_feature

<222> (17)..(19)

<223> 2'-O-methyl

<220>

<221> misc_feature

<222> (1)..(1)

<223> 2'-O-Methyl or 2'-deoxy-2'-fluoro

<220>

<221> misc_feature

<222> (4)..(4)

<223> 2'-O-Methyl or 2'-deoxy-2'-fluoro

<220>

<221> misc_feature

<222> (9)..(9)

<223>  2'-O-Methyl or 2'-deoxy-2'-fluoro

<220>

<221>  misc_feature

<222>  (16)..(16)

<223>  2'-O-Methyl or 2'-deoxy-2'-fluoro

<220>

<221>  misc_feature

<222>  (20)..(20)

<223>  Phosphorothioate 3'-Internucleotide Linkage

<220>

<221>  misc_feature

<222>  (20)..(21)

<223>  n stands for thymidine

<220>
<221>  misc_feature
<222>  (21)..(21)
<223>  3'-3 attached terminal glyceryl moeity

<400>  921
caauggaaug gaagacaaan n                                                21


<210>  922
<211>  21
<212>  RNA
<213>  Artificial Sequence

<220>
<223>  Description of Artificial Sequence:  siNA sense region

<220>

<221>  misc_feature

<222>  (1)..(3)

<223>  2'-deoxy-2'-fluoro

<220>

<221>  misc_feature

<222>  (5)..(10)

<223>  2'-deoxy-2'-fluoro

<220>

<221>  misc_feature

<222>  (12)..(15)

<223>  2'-deoxy-2'-fluoro

<220>

<221>  misc_feature

<222>  (17)..(18)

<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (21)..(21)
<223>  3'-3 attached terminal deoxyabasic moeity

<220>

<221>  misc_feature

<222>  (20)..(21)

<223>  n stands for thymidine

<400>  922
uuugucuucc auuccauugn n                                                21


<210>  923
<211>  21
<212>  RNA
<213>  Artificial Sequence

<220>
<223>  Description of Artificial Sequence:  siNA antisense region

<220>

<221>  misc_feature

<222>  (2)..(3)

<223>  2'-O-methyl

<220>

<221>  misc_feature

<222>  (5)..(8)

<223>  2'-O-methyl

<220>

<221>  misc_feature

<222>  (10)..(15)

<223>  2'-O-methyl

<220>

<221>  misc_feature

<222>  (17)..(19)

<223>  2'-O-methyl

<220>

<221>  misc_feature

<222>  (1)..(1)

<223>  2'-O-Methyl or 2'-deoxy-2'-fluoro

<220>

<221>  misc_feature

<222>  (4)..(4)

<223>  2'-O-Methyl or 2'-deoxy-2'-fluoro

<220>

<221>  misc_feature

<222>  (9)..(9)

<223>  2'-O-Methyl or 2'-deoxy-2'-fluoro

<220>

<221>  misc_feature

<222>  (16)..(16)

<223>  2'-O-Methyl or 2'-deoxy-2'-fluoro

<220>

<221>  misc_feature

<222>  (20)..(21)

<223>  n stands for thymidine

<220>
<221>  misc_feature
<222>  (21)..(21)
<223>  3'-3 attached terminal glyceryl moeity

<400>  923
caauggaaug gaagacaaan n                                                21


<210>  924
<211>  21
<212>  RNA

<213>  Artificial Sequence

<220>
<223>  Description of Artificial Sequence:  siNA antisense region

<220>

<221>  misc_feature

<222>  (2)..(3)

<223>  2'-deoxy

<220>

<221>  misc_feature

<222>  (5)..(8)

<223>  2'-deoxy

<220>

<221>  misc_feature

<222>  (10)..(15)

<223>  2'-deoxy

<220>

<221>  misc_feature

<222>  (17)..(19)

<223>  2'-deoxy

<220>

<221>  misc_feature

<222>  (1)..(1)

<223>  2'-deoxy-2'-fluoro

<220>

<221>  misc_feature

<222>  (4)..(4)

<223>  2'-deoxy-2'-fluoro

<220>

<221>  misc_feature

<222>  (9)..(9)

<223>  2'-deoxy-2'-fluoro

<220>

<221>  misc_feature

<222>  (16)..(16)

<223>  2'-deoxy-2'-fluoro

<220>

<221>  misc_feature

<222>  (20)..(20)

<223>  Phosphorothioate 3'-Internucleotide Linkage

<220>

<221>  misc_feature

<222>  (20)..(21)

<223>  n stands for thymidine

<220>
<221>  misc_feature
<222>  (21)..(21)
<223>  3'-3 attached terminal glyceryl moeity

<400>  924
caauggaaug gaagacaaan n                                                21


<210>  925
<211>  21
<212>  RNA
<213>  Artificial Sequence

<220>
<223>  Description of Artificial Sequence:  siNA sense region

<220>
<221>  misc_feature
<222>  (1)..(1)
<223>  2'-deoxy-2'-fluoro

<220>
<221>  misc_feature
<222>  (1)..(1)
<223>  5'-3 attached terminal deoxyabasic moeity

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (12)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(17)
<223>  2'-deoxy-2'-fluoro

<220>
<221>  misc_feature
<222>  (20)..(21)
<223>  n stands for thymidine

<220>
<221>  misc_feature
<222>  (21)..(21)
<223>  3'-3 attached terminal deoxyabasic moeity

<400>  925
caaccacaaa auacaacaan n                                                21


<210>  926
<211>  21
<212>  RNA
<213>  Artificial Sequence

<220>
<223>  Description of Artificial Sequence:  siNA antisense region

<220>
<221>  misc_feature
<222>  (20)..(21)
<223>  n stands for thymidine

<400>  926
ucgaaguauu ccgcguacgn n                                                21


<210>  927
<211>  21
<212>  RNA
<213>  Artificial Sequence

<220>
<223>  Description of Artificial Sequence:  siNA antisense region

<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)

<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (21)..(21)
<223>  n stands for thymidine

<220>
<221>  misc_feature
<222>  (20)..(20)
<223>  n stands for 3-deoxy thymidine

<400>  927
uuguuguauu uugugguugn n                                                21


<210>  928
<211>  21
<212>  RNA
<213>  Artificial Sequence

<220>
<223>  Description of Artificial Sequence:  siNA antisense region

<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (21)..(21)
<223>  n stands for thymidine

<220>
<221>  misc_feature
<222>  (20)..(20)
<223>  n stands for inverted thymidine

<400>  928
uuguuguauu uugugguugn n                                                21

<210>  929
<211>  21
<212>  RNA
<213>  Artificial Sequence

<220>
<223>  Description of Artificial Sequence:  siNA antisense region

<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(20)
<223>  n stands for thymidine

<220>
<221>  misc_feature
<222>  (21)..(21)
<223>  n stands for 3-deoxy thymidine

<400>  929
uuguuguauu uugugguugn n                                                21


<210>  930
<211>  21
<212>  RNA
<213>  Artificial Sequence

<220>
<223>  Description of Artificial Sequence:  siNA antisense region

<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(20)
<223>  n stands for thymidine

<220>
<221>  misc_feature
<222>  (21)..(21)
<223>  n stands for glyceryl moiety

<400>  930
uuguuguauu uugugguugn n                                                21


<210>  931
<211>  21

<212>  RNA
<213>  Artificial Sequence

<220>
<223>  Description of Artificial Sequence:  siNA antisense region

<220>
<221>  misc_feature
<222>  (1)..(2)
<223>  2'-deoxy-2'-fluoro

<220>
<221>  misc_feature
<222>  (4)..(5)
<223>  2'-deoxy-2'-fluoro

<220>
<221>  misc_feature
<222>  (7)..(7)
<223>  2'-deoxy-2'-fluoro

<220>
<221>  misc_feature
<222>  (9)..(12)
<223>  2'-deoxy-2'-fluoro

<220>
<221>  misc_feature
<222>  (14)..(14)
<223>  2'-deoxy-2'-fluoro

<220>
<221>  misc_feature
<222>  (17)..(18)
<223>  2'-deoxy-2'-fluoro

<220>
<221>  misc_feature
<222>  (20)..(20)
<223>  n stands for thymidine

<220>
<221>  misc_feature
<222>  (21)..(21)
<223>  n stands for L-thymidine

<400>  931
uuguuguauu uugugguugn n                                                21


<210>  932
<211>  21
<212>  RNA
<213>  Artificial Sequence

<220>
<223>  Description of Artificial Sequence:  siNA sense region

<220>
<221>  misc_feature
<222>  (20)..(21)
<223>  n stands for thymidine

<400>  932
cguacgcgga auacuucgan n                                                21